# EUROPEAN PATENT SPECIFICATION

(11) **EP 3 305 785 B1**
(45) Date of publication and mention of the grant of the patent: **25.08.2021**
(21) Application number: 16803266.2
(22) Date of filing: 27.05.2016
(51) Int. Cl.: C07D 471/04, A61K 31/519, A61K 31/5377, A61K 31/5386, A61K 31/541, A61K 31/55, A61K 31/551, A61K 31/553, A61P 9/10, A61P 11/00, A61P 19/02, A61P 25/28, A61P 29/00, A61P 35/00, A61P 43/00, C07D 519/00

(54) **PYRIDO[3,4-D]PYRIMIDINE DERIVATIVE AND PHARMACEUTICALLY ACCEPTABLE SALT THEREOF**
PYRIDO[3,4-D]PYRIMIDIN-DERIVAT UND PHARMAZEUTISCH UNBEDENKLICHES SALZ DAVON
DÉRIVÉ DE PYRIDO[3,4-D]PYRIMIDINE ET SEL PHARMACEUTIQUEMENT ACCEPTABLE DE CELUI-CI

(30) Priority: 29.05.2015 JP 2015110684
(43) Date of publication of application: 11.04.2018
(73) Proprietor: Teijin Pharma Limited, Tokyo 100-0013 (JP)
(72) Inventor: MIZUNO, Tsuyoshi, Tokyo 100-0013 (JP); SHIMADA, Tomohiro, Tokyo 100-0013 (JP); UNOKI, Gen, Tokyo 100-0013 (JP); EBISAWA, Masaru, Tokyo 100-0013 (JP); TAKEUCHI, Susumu, Tokyo 100-0013 (JP); MINAMIZONO, Kunio, Tokyo 100-0013 (JP); SASAKI, Kosuke, Tokyo 100-0013 (JP); YOKOSAKA, Takuya, Tokyo 100-0013 (JP); IGARASHI, Junji, Tokyo 100-0013 (JP); MARUYAMA, Akinobu, Tokyo 100-0013 (JP); TAKAHASHI, Hiroshi, Tokyo 100-0013 (JP); HORIE, Kyohei, Tokyo 100-0013 (JP); SAKAI, Yuri, Tokyo 100-0013 (JP)
(74) Representative: Hoffmann Eitle
(86) International application number: PCT/JP2016/065770
(87) International publication number: WO 2016/194831

(56) References cited:
- WO-A1-2011/101417
- WO-A1-2014/037750
- JP-A- 2005 519 909
- JP-A- 2007 530 425
- INNOCENTI, P. ET AL.: 'Expanding the scope of fused pyrimidines as kinase inhibitor scaffolds: synthesis and modification of pyrido[3,4-d]pyrimidines' ORGANIC & BIOMOLECULAR CHEMISTRY vol. 13, 21 January 2015, ISSN 1477-0520 pages 893 - 904, XP055332115

## Description

### Technical Field

The present invention relates to a pyrido[3,4-d]pyrimidine derivative and a pharmaceutically acceptable salt thereof. In particular, the present invention relates to a compound that exhibits an inhibitory activity against cyclin-dependent kinase 4 and/or cyclin-dependent kinase 6 (hereinafter referred to as "CDK4/6") and that is useful for the prevention or treatment of rheumatoid arthritis, arteriosclerosis, pulmonary fibrosis, cerebral infarction, or cancer.

### Background Art

Cell growth, which is a process involving proliferation and division of cells, occurs in response to various stimuli.

Pathological conditions caused by hyperproliferation of cells, such as cancer, are characterized by uncontrollable cell cycle progression and thus excessive progression of the cell cycle, for example, resulting from abnormality in genes or proteins that directly or indirectly regulate the cell cycle progression. Substances that regulate hyperproliferation of cells through control of the cell cycle can be used for the treatment of various pathological conditions characterized by uncontrollable or unwanted cell growth.

Cell cycle progression is a complicated process involving highly regulated transition of phases and multiple checkpoints.

Cyclin-dependent kinases and associated serine/threonine protein kinases are important intracellular enzymes that play essential roles in the regulation of division and proliferation of cells. Catalytic subunits of cyclin-dependent kinases are activated by regulatory subunits known as cyclins, and multiple cyclins have been identified in mammals (NPL 1).

The retinoblastoma (Rb) protein is a checkpoint protein for transition from the G1 phase to the S phase in the cell cycle. The Rb protein associates with the E2F transcription factor family and inhibits the activity thereof in the absence of appropriate growth stimulation (NPLs 2 and 3). A cell stimulated by a mitogen enters the S phase through synthesis of cyclin D, which is a CDK4/6 activator. The cyclin D-bound CDK 4/6 inactivates the Rb protein through phosphorylation. The phosphorylation of the Rb protein releases E2F in order to indirective the transcription of a gene necessary for the S phase. The complete inactivation of the Rb protein requires phosphorylation of both cyclin D-CDK4/6 and cyclin E-CDK2. The phosphorylation of the Rb protein by CDK4/6 at a specific site is essential in the phosphorylation of cyclin E-CDK2 (NPL 4). Thus, cyclin D-CDK4/6 is an important enzyme complex which controls the transition from the G1 phase to the S phase.

CDK2 forms a complex with cyclin E and also forms a complex with cyclin A. CDK2 also acts on steps subsequent to the S phase and is responsible for DNA replication. The inhibition of CDK2 probably leads to the expression of genotoxicity (NPL 5).

Cyclin D has a molecular mechanism that positively regulates the activity of CDK4/6. In contrast, p16 encoded by the INK4a gene negatively regulates the activity of CDK4/6 (NPL 6).

CDK inhibitors can be used for the treatment of various diseases caused by abnormal cell growth, such as cancer, cardiovascular disorder, renal disease, specific infections, and autoimmune diseases. CDK inhibitors is also expected to be effective for the treatment of diseases including but not limited to rheumatoid arthritis, arteriosclerosis, pulmonary fibrosis, cerebral infarction, and cancer. The inhibition of cell cycle progression and cell growth through CDK inhibition is expected to be effective for such a disease on the basis of the technical findings described below.

Rheumatoid arthritis involves the formation of pannus through hyperproliferation of synovial cells. This hyperproliferation can be reduced by the introduction of p16 into an affected area of a model animal or the administration of a CDK4/6 inhibitor to the animal (NPLs 7 to 9). A CDK4-cyclin D complex regulates the production of MMP3 in synovial cells derived from a patient with rheumatoid arthritis. The negative regulation of the activity of CDK4/6 inhibits not only the proliferation but also production of MMP3 (NPL 10).

Thus, CDK4/6 inhibitors are expected to exhibit both an inhibitory effect on proliferation of synovial cells and a cartilage protective effect in rheumatoid arthritis.

A pathway for the regulation of cell growth including genes responsible for the checkpoints in the G1 and S phases of the cell cycle is associated with plaque progression, stenosis, and restenosis after angiogenesis. The overexpression of the CDK inhibitory protein p21 inhibits angiogenesis and subsequent growth of vascular smooth muscle and intimal hyperplasia (NPLs 11 and 12).

Abnormal regulation of the cell cycle is also associated with polycystic kidney disease, which is characterized by growth of cysts filled with fluid in the renal tubule. A small-molecule CDK inhibitor is effective for the treatment of the disease (NPL 13).

The induction of expression of the cell cycle inhibitory protein p21 with an adenoviral vector is effective in a murine pulmonary fibrosis model (NPL 14).

The level of cyclin D1/CDK4 is known to increase in a rat cerebral infarction model in association with neuronal death caused by local ischemia. The neuronal death is reduced by administering flavopiridol, which is a nonselective CDK inhibitor (NPL 15).

The cyclin D-CDK4/6-INK4a-Rb pathway is frequently detected in human cancer caused by abnormality of any factors contributing to growth of cancer cells, such as loss of functional p16INK4a, overexpression of cyclin D1, overexpression of CDK4, or loss of functional Rb (NPLs 16 to 18). Such abnormality promotes the cell cycle progression from the G1 phase to the S phase, and this pathway certainly plays an important role in oncogenic transformation or abnormal growth of cancer cells.

CDK4/6 inhibitors may be effective, particularly for tumors involving abnormality in genes that activate the CDK4/6 kinase activity, such as cancers involving the translocation of cyclin D, cancers involving the amplification of cyclin D, cancers involving the amplification or overexpression of CDK4 or CDK6, and cancers involving the inactivation of p16. CDK4/6 inhibitors may be effective for the treatment of cancers involving genetic abnormality in the upstream regulator of cyclin D, the amount of which increases due to defects in the upstream regulator.

In fact, many compounds that inhibit the CDK4/6 activity have been synthesized and disclosed in the art, and such compounds have been clinically tested for the treatment of cancers, such as breast cancer (NPL 19).

Most acute and severe radiotherapeutic and chemotherapeutic toxicities are caused by the effects on stem cells and progenitor cells. A CDK4/6 inhibitor causes temporary cell cycle arrest to hematopoietic stem and progenitor cells, and protects them from radiotherapeutic or chemotherapeutic cytotoxicity. After the treatment with the inhibitor, hematopoietic stem and progenitor cells (HSPCs) return from the temporary dormancy and then function normally. Thus, the chemotherapeutic resistance with use of a CDK4/6 inhibitor is expected to provide a significant protection of bone marrow (NPL 20).

Hence, CDK4/6 inhibitors are expected to be effective for the treatment of rheumatoid arthritis, arteriosclerosis, pulmonary fibrosis, cerebral infarction, or cancer, and the protection of bone marrow, in particular, for the treatment of rheumatoid arthritis or cancer and the protection of bone marrow.

PTL 1 and NPL 21 disclose CDK4 inhibitors, PTLs 2 and 3 and NPLs 22 to 24 disclose CDK4/6-containing CDK inhibitors, and NPL 25 discloses CDK4/FLT3 inhibitors.

Pyrido[3,4-d]pyrimidine derivatives exhibit an inhibitory effect on Mps1 (also known as TTK) (PTL 4). This inhibitory effect is completely different from the CDK4/6 inhibitory effect disclosed in the present invention.

NPL 26 and NPL 27 disclose that a plurality of pyrido[3,4-d]pyrimidine derivatives exhibit a CDK2 inhibitory activity, which is completely different from the superior CDK4/6 inhibitory effect exhibited by the present invention.

### List of Citations

### Patent Literature

[PTL 1] WO2003/062236
[PTL 2] WO2010/020675
[PTL 3] WO2010/075074
[PTL 4] WO2014/037750

### Non-patent Literature

[NPL 1] Johnson D. G. and Walker C.L., Annual Review of Pharmacology and Toxicology 1999; 39: p.295-312
[NPL 2] Ortega et al., Biochimica et Biophysica Acta-Reviews on Cancer 2002; 1602 (1): p.73-87
[NPL 3] Shapiro, Journal of Clinical Oncology 2006; 24 (11): p.1770-1783
[NPL 4] Lundberg et al., Molecular and Cellular Biology 1998; 18 (2): p.753-761
[NPL 5] Andrew J. Olaharski, PLoS Computational Biology 2009; 5 (7): e1000446
[NPL 6] Kamb et al., Science 1994; 264 (5157): p.436-440
[NPL 7] Taniguchi, K et al., Nature Medicine, Vol.5, p.760-767 (1999)
[NPL 8] Sekine, C et al., Journal of immunology 2008, 180: p.1954-1961
[NPL 9] Hosoya, T et al., Annnl Rheumatic Diseases 2014, Aug 27 Epub ahead of print
[NPL 10] Nonomura Y et al., Arthritis & Rheumatology 2006, Jul; 54 (7): p.2074-83
[NPL 11] Chang M.W. et al., Journal of Clinical Investigation, 1995, 96: p.2260
[NPL 12] Yang Z-Y. et al., Proceedings of the National Academy of Sciences (USA) 1996, 93: p.9905
[NPL 13] Bukanov N.O. et al., Nature, 2006, 4444: p.949-952
[NPL 14] American Journal Physiology: Lung Cellular and Molecular Physiology, 2004, Vol. 286, p.L727-L733
[NPL 15] Proceedings of the National Academy of Sciences of the United States of America, 2000, Vol.97, p.10254-10259
[NPL 16] Science, Vol. 254, p.1138-1146 (1991)
[NPL 17] Cancer Research, 1993, Vol. 53, p.5535-5541
[NPL 18] Current Opinion in Cell Biology, 1996, Vol.8, p.805-814
[NPL 19] Guha M, Nature Biotechnology 2013, Mar; 31 (3): p.187
[NPL 20] Journal of Clinical Investigation 2010; 120 (7): p.2528-2536 Soren M. Johnson
[NPL 21] Journal of Medicinal Chemistry, 2005, 48, p.2371-2387
[NPL 22] Journal of Medicinal Chemistry, 2000, 43, p.4606-4616
[NPL 23] Journal of Medicinal Chemistry, 2005, 48, p.2388-2406
[NPL 24] Journal of Medicinal Chemistry, 2010, 53, p.7938-7957
[NPL 25] Journal of Medicinal Chemistry, 2014, 57, p.3430-3449
[NPL 26] Organic & Biomolecular Chemistry, 2015, 13, p.893-904
[NPL 27] Rapid Discovery of Pyrido[3,4-d]pyrimidine Inhibitors of Monopolar Spindle Kinase 1 (MPS1) Using a Structure-Based Hybridization Approach, Paolo Innocenti et al, J. Med. Chem., Article ASAP,Publication Date (Web): April 7, 2016, DOI: 10.1021/acs.jmedchem.5b01811.

### Summary of Invention

### Problem to be Solved by the Invention

An object of the present invention is to provide a compound exhibiting a superior CDK4/6 inhibitory activity.

### Means to Solve the Problem

The present inventors have conducted extensive studies for solving the problems described above and have found that a novel pyrido[3,4-d]pyrimidine derivative represented by Formula (I) exhibits a CDK4/6 inhibitory activity. The present invention has been accomplished on the basis of this finding.

The present invention includes the following aspects:
Aspect (1): A compound represented by Formula (I), or a pharmaceutically acceptable salt thererof:

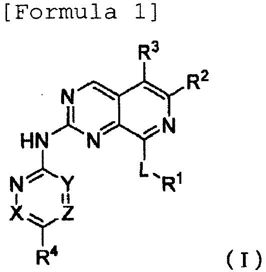

   [wherein
   L represents -NR⁵-, -O-, or -S-;
   R⁵ represents a hydrogen atom or a C₁₋₆ alkyl group substituted with zero to two -OH groups, zero to two C₁₋₈ alkoxy groups, and zero to six fluorine atoms;
   R¹ represents a C₁₋₃ alkyl, C₃₋₁₂ cycloalkyl, (C₃₋₁₂ cycloalkyl)-C₁₋₆ alkyl, 4- to 12-membered heterocyclyl, (4- to 12-membered heterocyclyl) -C₁₋₆ alkyl, C₆₋₁₀ aryl, (C₆₋₁₀ aryl)-C₁₋₆ alkyl, 5- to 10-membered heteroaryl, (5-to 10-membered heteroaryl)-C₁₋₆ alkyl, C₁₋₈ alkylsulfonyl, or C₁₋₈ acyl group;
   each of the heteroatom-containing groups represented by R¹ contains one to four heteroatoms selected from oxygen, sulfur, and nitrogen atoms;
   R¹ is optionally substituted with one to six substituents selected from the group consisting of a halogen atom, =O, -OH, -CN, -COOH, -COOR⁶, -R⁷, a C₃₋₆ cycloalkyl group substituted with zero to two -OH groups, zero to two C₁₋₈ alkoxy groups, and zero to six fluorine atoms, a 3- to 10-membered heterocyclyl group substituted with zero to two -OH groups, zero to two C₁₋₈ alkoxy groups, and zero to six fluorine atoms, a C₁₋₈ acyl group substituted with zero to two -OH groups, zero to two C₁₋₈ alkoxy groups, and zero to six fluorine atoms, and a C₁₋₈ alkoxy group substituted with zero to two -OH groups, zero to two C₁₋₈ alkoxy groups, and zero to six fluorine atoms;
   R⁶ and R⁷ each independently represent a C₁₋₆ alkyl group substituted with zero to two -OH groups, zero to two C₁₋₈ alkoxy groups, and zero to six fluorine atoms;
   R² represents a C₁₋₈ alkyl, C₃₋₈ cycloalkyl, 4- to 6-membered heterocyclyl, or C₁₋₈ acyl group, -COOR⁸, or-CONR⁹R¹⁰;
   each of the C₁₋₈ alkyl and C₃₋₈ cycloalkyl groups represented by R² is substituted with zero or one -OH group, zero to two C₁₋₈ alkoxy groups substituted with zero or one -OH group, zero or one C₁₋₄ alkoxy group, and zero to three fluorine atoms, and zero to five fluorine atoms;
   R² is neither an unsubstituted C₁₋₈ alkyl, nor unsubstituted C₃₋₈ cycloalkyl, nor trifluoromethyl group; R⁸, R⁹, and R¹⁰ each independently represent a hydrogen atom or a C₁₋₈ alkyl group;
   the 4- to 6-membered heterocyclyl group represented by R² is optionally substituted with one to four substituents selected from the group consisting of a fluorine atom,-OH, and C₁₋₄ alkyl and C₁₋₄ alkoxy groups;
   each of the C₁₋₈ acyl group, -COOR⁸, and -CONR⁹R¹⁰ represented by R² is optionally substituted with one to four substituents selected from the group consisting of a fluorine atom, -OH, and a C₁₋₄ alkoxy group;
   R⁹ and R¹⁰ of -CONR⁹R¹⁰ represented by R² are optionally bonded via a single bond or -0- to form a ring including the nitrogen atom bonded to R⁹ and R¹⁰;
   the heterocyclyl group represented by R² having a 4- or 5-membered ring contains one oxygen heteroatom, and the heterocyclyl group having a 6-membered ring contains one or two oxygen heteroatoms;
   R³ represents a hydrogen atom, a C₁₋₈ alkyl group, or a halogen atom;
   X represents CR¹¹ or a nitrogen atom;
   Y represents CR¹² or a nitrogen atom;
   Z represents CR¹³ or a nitrogen atom;
   R¹¹ to R¹³ each independently represent a hydrogen, fluorine, chlorine atom, a C₁₋₆ alkyl, or C₁₋₆ alkoxy group;
   R⁴ represents -A¹-A²-A³;
   A¹ represents a single bond, a C₁₋₈ alkylene, C₂₋₈ alkenylene, or C₂₋₈ alkynylene group;
   one or two sp³ carbon atoms at any positions of A¹ are optionally replaced with one or two structures selected from the group consisting of -O-, -NR¹⁴-, -C(=O)-, -C(=O)-O-, -O-C(=O)-, -O-C(=O)-O-, -C(=O)-NR¹⁵-, -O-C(=O)-NR¹⁶-,-NR¹⁷-C(=O) -, -NR¹⁸-C (=O) -O-, -NR¹⁹-C (=O) -NR²⁰-, -S (=O) p-,-S (=O)₂-NR²¹-, -NR²²-S (=O)₂-, and -NR²³-S (=O)₂-NR²⁴-, and a structure of -O-O-, -O-NR¹⁴-, -NR¹⁴⁻O-, -O-CH₂-O-, -O-CH₂-NR¹⁴-, or -NR¹⁴-CH₂-O- is not formed in the case of replacement of two sp³ carbon atoms;
   A² represents a single bond, a C₁₋₇ alkylene, C₃₋₁₂ cycloalkylene, C₃₋₁₂ cycloalkylidene, 4- to 12-membered heterocyclylene, 4- to 12-membered heterocyclylidene, C₆₋₁₀ arylene, or 5- to 10-membered heteroarylene group;
   A³ represents a halogen atom, -CN, -NO₂, -R²⁵, -OR²⁶,-NR²⁷R²⁸, -C(=O)R²⁹, -C(=O)-OR³⁰, -O-C(=O)R³¹,-NR³²R³³, -C (=O) -NR³⁴R³⁵, -NR³⁶-C (=O)R³⁷, -NR³⁸-C (=O) -OR³⁹,-S (=O)₂-R⁴⁰, -S (=O)₂-NR⁴¹R⁴², or -NR⁴³-S (=O)₂R⁴⁴;
   A³ represents -R²⁵, if the A¹ end on the A² side has a structure selected from the group consisting of -O-,-NR¹⁴-, -C(=O)-, -C(=O)-O-, -O-C(=O)-, -O-C(=O)-O-, -C(=O)-NR¹⁵-, -O-C(=O)-NR¹⁶-, -NR¹⁷⁻C(=O) -, -NR¹⁸-C (=O) -O-, -NR¹⁹-C (=O) -NR²⁰-, -S(=O)ₚ-, -S(=O)₂-NR²¹-, -NR²²-S (=O) ₂-, and-NR²³-S (=O)₂-NR²⁴- and A² is a single bond;
   R¹⁴, R³², R³⁴, R³⁶, R³⁸, R⁴¹, and R⁴³ each independently represent a hydrogen atom, a C₁₋₈ alkyl, C₁₋₈ acyl, C₁₋₈ alkylsulfonyl, 4- to 12-membered heterocyclyl, C₃₋₁₂ cycloalkyl, C₆₋₁₀ aryl, 5- to 10-membered heteroaryl, (4-to 12-membered heterocyclyl) -C₁₋₃ alkyl, (C₃₋₁₂ cycloalkyl)-C₁₋₃ alkyl, (C₆₋₁₀ aryl)-C₁₋₃ alkyl, or (5- to 10-membered heteroaryl)-C₁₋₃ alkyl group;
   R¹⁵ to R³¹, R³³, R³⁵, R³⁷, R³⁹, R⁴⁰, R⁴², and R⁴⁴ each independently represent a hydrogen atom or a C₁₋₈ alkyl, 4- to 12-membered heterocyclyl, C₃₋₁₂ cycloalkyl, C₆₋₁₀ aryl, 5- to 10-membered heteroaryl, (4- to 12-membered heterocyclyl)-C₁₋₃ alkyl, (C₃₋₁₂ cycloalkyl)-C₁₋₃ alkyl, (C₆₋₁₀ aryl)-C₁₋₃ alkyl, or (5- to 10-membered heteroaryl) -C₁₋₃ alkyl group;
   A¹, A², A³, and R¹⁴ to R⁴⁴ in A¹, A², and A³ are each optionally substituted with one to four substituents selected from the group consisting of -OH, =O, -COOH,-SO₃H, -PO₃H, -CN, -NO₂, a halogen atom, a C₁₋₈ alkyl group substituted with zero to two -OH groups, zero to two -OR⁴⁵ groups, and zero to six fluorine atoms, a C₃₋₁₂ cycloalkyl group substituted with zero to two -OH groups, zero to two -OR⁴⁶ groups, and zero to six fluorine atoms, a C₁₋₈ alkoxy group substituted with zero to two -OH groups, zero to two -OR⁴⁷ groups, and zero to six fluorine atoms, and a 4- to 12-membered heterocyclyl group substituted with zero to two -OH groups, zero to two -OR⁴⁹ groups, and zero to six fluorine atoms;
   R¹⁴ to R⁴⁴ are optionally bonded in A¹, A², or A³ or between A¹ and A², between A¹ and A³, or between A² and A³ via a single bond, -0-, -NR⁵⁰-, or -S(=O)ₚ- to form a ring;
   R¹¹ or R¹³ is optionally bonded to A¹, A², or A³ via a single bond, -O-, -NR⁵¹-, or -S(=O)ₚ- to form a ring;
   R⁴⁵ to R⁵¹ each represent a hydrogen atom or a C₁₋₄ alkyl group substituted with zero or one -OH group and zero to six fluorine atoms;
   p represents an integer of 0 to 2; and
   each of the heteroatom-containing groups represented by A¹, A², and A³ contains one to four heteroatoms selected from oxygen, sulfur, and nitrogen atoms].
Aspect (2): The compound or pharmaceutically acceptable salt thereof according to Aspect (1), wherein L represents -NH-.
Aspect (3): The compound or pharmaceutically acceptable salt thereof according to Aspect (1) or (2), wherein R¹ represents a C₁₋₈ alkyl, C₃₋₁₂ cycloalkyl, (C₃₋₁₂ cycloalkyl)-C₁₋₆ alkyl, 4- to 12-membered heterocyclyl, or (4- to 12-membered heterocyclyl)-C₁₋₆ alkyl group.
Aspect (4): The compound or pharmaceutically acceptable salt thereof according to any one of Aspects (1) to (3), wherein R² is a C₁₋₈ alkyl group substituted with one to four fluorine atoms.
Aspect (5): The compound or pharmaceutically acceptable salt thereof according to any one of Aspects (1) to (3), wherein R² is a C₁₋₈ alkyl group substituted with zero or one -OH group and zero to two C₁₋₈ alkoxy groups substituted with zero or one -OH group, zero or one C₁₋₄ alkoxy group, and zero to three fluorine atoms.
Aspect (6): The compound or pharmaceutically acceptable salt thereof according to any one of Aspects (1) to (3), wherein R² is a 4- to 6-membered heterocyclyl group optionally substituted with one to four substituents selected from the group consisting of a fluorine atom,-OH, and C₁₋₄ alkyl and C₁₋₄ alkoxy groups.
Aspect (7): The compound or pharmaceutically acceptable salt thereof according to any one of Aspects (1) to (3), wherein R² is -COOR , -CONR⁹R¹⁰, or a C₁₋₈ acyl group, optionally each group being substituted with one to four substituents selected from the group consisting of a fluorine atom, -OH, and a C₁₋₈ alkoxy group.
Aspect (8): The compound or pharmaceutically acceptable salt thereof according to any one of Aspects (1) to (7), wherein X represents CR¹¹, Y represents CR¹², and Z represents CR¹³.
Aspect (9): The compound or pharmaceutically acceptable salt thereof according to any one of Aspects (1) to (7), wherein X represents a nitrogen atom, Y represents CR¹², and Z represents CR¹³.
Aspect (10): The compound or pharmaceutically acceptable salt thereof according to any one of Aspects (1) to (7), wherein X represents CR¹¹, Y represents a nitrogen atom, and Z represents CR¹³.
Aspect (11): The compound or pharmaceutically acceptable salt thereof according to any one of Aspects (1) to (7), wherein X represents CR¹¹, Y represents CR¹², and Z represents a nitrogen atom.
Aspect (12): The compound or pharmaceutically acceptable salt thereof according to any one of Aspects (1) to (11), wherein A¹ is a single bond.
Aspect (13): The compound or pharmaceutically acceptable salt thereof according to any one of Aspects (1) to (11), wherein A¹ represents a C₁₋₈ alkylene group, and no sp³ carbon atom in A¹ is replaced with another structure.
Aspect (14): The compound or pharmaceutically acceptable salt thereof according to any one of Aspects (1) to (11), wherein A¹ represents a C₁₋₈ alkylene group, and one sp³ carbon atom at any position of A¹ is replaced with -O-.
Aspect (15): The compound or pharmaceutically acceptable salt thereof according to any one of Aspects (1) to (11), wherein A¹ represents a C₁₋₈ alkylene group, and one sp³ carbon atom at any position of A¹ is replaced with -NR¹⁴-.
Aspect (16): The compound or pharmaceutically acceptable salt thereof according to any one of Aspects (1) to (11), wherein A¹ represents a C₁₋₈ alkylene group, one sp³ carbon atom at any position of A¹ is replaced with -NR¹⁴-, and one sp³ carbon atom at any other position of A¹ is optionally replaced with -O-.
Aspect (17): The compound or pharmaceutically acceptable salt thereof according to any one of Aspects (1) to (16), wherein A² represents a 4- to 12-membered heterocyclylene group; and A² is optionally substituted with one to four substituents selected from the group consisting of -OH,-COOH, -SO₃H, -PO₃H, -CN, -NO₂, a halogen atom, a C₁₋₈ alkyl group optionally substituted with zero to two -OH groups, zero to two -OR⁴⁵ groups, and zero to six fluorine atoms, a C₃₋₁₂ cycloalkyl group optionally substituted with zero to two -OH groups, zero to two -OR⁴⁶ groups, and zero to six fluorine atoms, a C₁₋₈ alkoxy group optionally substituted with zero to two -OH groups, zero to two -OR⁴⁷ groups, and zero to six fluorine atoms, and a 4- to 12-membered heterocyclyl group substituted with zero to two -OH groups, zero to two -OR⁴⁹ groups, and zero to six fluorine atoms.
Aspect (18): The compound or pharmaceutically acceptable salt thereof according to any one of Aspects (1) to (16), wherein A² represents a 4- to 12-membered heterocyclylene group substituted with =O; and A² is optionally substituted with one to four substituents selected from the group consisting of -OH, =O, -COOH, -SO₃H, -PO₃H, -CN, -NO₂, a halogen atom, a C₁₋₈ alkyl group substituted with zero to two -OH groups, zero to two -OR⁴⁵ groups, and zero to six fluorine atoms, a C₃₋₁₂ cycloalkyl group substituted with zero to two -OH groups, zero to two -OR⁴⁶ groups, and zero to six fluorine atoms, a C₁₋₈ alkoxy group substituted with zero to two -OH groups, zero to two -OR⁴⁷ groups, and zero to six fluorine atoms, and a 4- to 12-membered heterocyclyl group substituted with zero to two -OH groups, zero to two -OR⁴⁹ groups, and zero to six fluorine atoms.
Aspect (19): The compound or pharmaceutically acceptable salt thereof according to any one of Aspects (1) to (18), wherein X represents CR¹¹, Y represents CR¹², Z represents CR¹³, and R¹¹ or R¹³ is bonded to A¹, A², or A³ via a single bond, -O-, -NR⁵¹-, or -S(=O)ₚ- to form a ring.
Aspect (20): The compound or pharmaceutically acceptable salt thereof according to any one of Aspects (1) to (19), wherein A³ is a hydrogen atom.
Aspect (21): The compound or pharmaceutically acceptable salt thereof according to any one of Aspects (1) to (19), wherein A³ is a halogen atom, -CN, -R²⁵, -OR²⁶, -NR²⁷R²⁸,-C(=O)R²⁹, or -C (=O) -OR³⁰, and R²⁵ to R³⁰ each independently represent a hydrogen atom, an optionally substituted C₁₋₈ alkyl group, an optionally substituted 4- to 12-membered heterocyclyl group, an optionally substituted C₃₋₁₂ cycloalkyl group, an optionally substituted (4- to 12-membered heterocyclyl)-C₁₋₃ alkyl group, or an optionally substituted (C₃₋₁₂ cycloalkyl) -C₁₋₃ alkyl group.
Aspect (22): The compound or pharmaceutically acceptable salt thereof according to any one of Aspects (1) to (21), wherein R³ is a hydrogen atom.
Aspect (23): The compound or pharmaceutically acceptable salt thereof according to any one of Aspects (1) to (21), wherein R³ represents a C₁₋₄ alkyl group, a fluorine atom, or a chlorine atom.
Aspect (24):
   The compound, or pharmaceutically acceptable salt thereof, selected from; 6-(difluoromethyl)-N8-isopropyl-N2-(5-piperazin-1-yl-2-pyridyl)pyrido[3,4-d]pyrimidine-2,8-diamine (1R)-1-[8-(isopropylamino)-2-[(5-piperazin-1-yl-2-pyridyl)amino]pyrido[3,4-d]pyrimidin-6-yl]ethanol 1-[2-[(5-piperazin-1-yl-2-pyridyl)amino]-8-(tetrahydrofuran-3-ylamino)pyrido[3,4-d]pyrimidin-6-yl]ethanol 1-[2-[(5-piperazin-1-yl-2-pyridyl)amino]-8-(tetrahydropyran-3-ylamino)pyrido[3,4-d]pyrimidin-6-yl]ethanol N8-isopropyl-6-[(1R)-1-methoxyethyl]-N2-(6-piperazin-1-ylpyridazin-3-yl)pyrido[3,4-d]pyrimidine-2,8-diamine N8-isopropyl-6-[(1R)-1-methoxyethyl]-N2-[5-(piperazin-1-ylmethyl)-2-pyridyl]pyrido[3,4-d]pyrimidine-2,8-diamine 1-[6-[[6-[(1R)-1-hydroxyethyl]-8-(isopropylamino)pyrido[3,4-d]pyrimidin-2-yl]amino]-3-pyridyl]piperazin-2-one 1-[6-[[5-chloro-6-[(1R)-1-hydroxyethyl]-8-(isopropylamino)pyrido[3,4-d]pyrimidin-2-yl]amino]-3-pyridyl]piperazin-2-one (1R)-1-[2-[(6-piperazin-1-ylpyridazin-3-yl)amino]-8-(tetrahydropyran-4-ylamino)pyrido[3,4-d]pyrimidin-6-yl]ethanol (1R)-1-[2-[(6-piperazin-1-ylpyridazin-3-yl)amino]-8-[[(3S)-tetrahydropyran-3-yl]amino]pyrido[3,4-d]pyrimidin-6-yl]ethanol (1R)-1-[2-[(6-piperazin-1-ylpyridazin-3-yl)amino]-8-[[(3R)-tetrahydropyran-3-yl]amino]pyrido[3,4-d]pyrimidin-6-yl]ethanol (1R)-1-[2-[[5-(piperazin-1-ylmethyl)-2-pyridyl]amino]-8-(tetrahydropyran-4-ylamino)pyrido[3,4-d]pyrimidin-6-yl]ethanol (1R)-1-[2-[[5-(piperazin-1-ylmethyl)-2-pyridyl]amino]-8-[[(3S)-tetrahydropyran-3-yl]amino]pyrido[3,4-d]pyrimidin-6-yl]ethanol (1R)-1-[2-[[5-(piperazin-1-ylmethyl)-2-pyridyl]aminol-8-[[(3R)-tetrahydropyran-3-yl]amino]pyrido[3,4-d]pyrimidin-6-yl]ethanol 1-[6-[[6-[(1R)-1-hydroxyethyl]-8-(isopropylamino)pyrido[3,4-d]pyrimidin-2-yl]amino]pyridazin-3-yl]piperidin-4-ol (1R)-1-[8-(isopropylamino)-2-[(6-piperazin-1-ylpyridazin-3-yl)amino]pyrido[3,4-d]pyrimidin-6-yl]ethanol 1-[[6-[[6-[(1R)-1-hydroxyethyl]-8-(isopropylamino)pyrido[3,4-d]pyrimidin-2-yl]amino]-3-pyridyl]methyl]piperazin-2-one 6-[(1R)-1-methoxyethyl]-N2-[5-(piperazin-1-ylmethyl)-2-pyridyl]-N8-[(3S)-tetrahydropyran-3-yl]pyrido[3,4-d]pyrimidine-2,8-diamine 6-[(1R)-1-methoxyethyl]-N2-(6-piperazin-1-ylpyridazin-3-yl)-N8-[(3S)-tetrahydropyran-3-yl]pyrido[3,4-d]pyrimidine-2,8-diamine 6-[(1R)-1-methoxyethyl]-N2-[5-(piperazin-1-ylmethyl)-2-pyridyl]-N8-(tetrahydropyran-4-ylmethyl)pyrido[3,4-d]pyrimidine-2,8-diamine N8-isopropyl-6-[(1R)-1-methoxyethyl]-N2-(5-piperazin-1-ylpyrazin-2-yl)pyrido[3,4-d]pyrimidine-2,8-diamine N8-isopropyl-6-[(1R)-1-methoxyethyl]-N2-[6-[(2S)-2-methylpiperazin-1-yl]pyridazin-3-yl]pyrido[3,4-d]pyrimidine-2,8-diamine N8-isopropyl-6-[(1R)-1-methoxyethyl]-N2-[6-[(2R)-2-methylpiperazin-1-yl]pyridazin-3-yl]pyrido[3,4-d]pyrimidine-2,8-diamine (1R)-1-[2-[[6-(4,7-diazaspiro[2.5]octan-7-yl)pyridazin-3-yl]amino]-8-(isopropylamino)pyrido[3,4-d]pyrimidin-6-yl]ethanol (1R)-1-[2-[[5-(4,7-diazaspiro[2.5]octan-7-ylmethyl)-2-pyridyl]amino]-8-(isopropylamino)pyrido[3,4-d]pyrimidin-6-yl]ethanol 2-[1-[[6-[[6-[(1R)-1-hydroxyethyl]-8-(isopropylamino)pyrido[3,4-d]pyrimidin-2-yl]amino]-3-pyridyl]methyl]-4-piperidyl]propan-2-ol (1R)-1-[2-[[5-[[4-(2-hydroxyethyl)piperazin-1-yl]methyl]-2-pyridyl]amino]-8-(isopropylamino)pyrido[3,4-d]pyrimidin-6-yl]ethanol (1R)-1-[2-[[5-[2-(dimethylamino)ethoxy]-2-pyridyl]amino]-8-[[(3S)-tetrahydropyran-3-yl]amino]pyrido[3,4-d]pyrimidin-6-yl]ethanol (1R)-1-[2-[[6-(4-methylpiperazin-1-yl)pyridazin-3-yl]amino]-8-[[(3S)-tetrahydropyran-3-yl]amino]pyrido[3,4-d]pyrimidin-6-yl]ethanol 2-hydroxy-1-[4-[6-[[6-[(1R)-1-hydroxyethyl]-8-(isopropylamino)pyrido[3,4-d]pyrimidin-2-yl]amino]pyridazin-3-yl]piperazin-1-yl]ethanone 1-[6-[[8-(isopropylamino)-6-[(2S)-tetrahydrofuran-2-yl]pyrido[3,4-d]pyrimidin-2-yl]amino]-3-pyridyl]piperazin-2-one (1R)-1-[8-(isopropylamino)-2-(5,6,7,8-tetrahydro-1,6-naphthyridin-2-ylamino)pyrido[3,4-d]pyrimidin-6-yl]ethanol 2-[4-[[6-[[6-[(1R)-1-hydroxyethyl]-8-(isopropylamino)pyrido[3,4-d]pyrimidin-2-yl]amino]-3-pyridyl]methyl]piperazin-1-yl]-2-methyl-propan-1-ol 4-[6-[[6-[(1R)-1-hydroxyethyl]-8-(isopropylamino)pyrido[3,4-d]pyrimidin-2-yl]amino]-3-pyridyl]-1-[(2S)-2-hydroxypropyl]-1,4-diazepan-5-one 4-[6-[[6-[(1R)-1-hydroxyethyl]-8-(isopropylamino)pyrido[3,4-d]pyrimidin-2-yl]amino]-3-pyridyl]-1-[(2R)-2-hydroxypropyl]-1,4-diazepan-5-one N8-isopropyl-N2-[5-(piperazin-1-ylmethyl)-2-pyridyl]-6-[(2S)-tetrahydrofuran-2-yl]pyrido[3,4-d]pyrimidine-2,8-diamine 1-[6-[[6-[(1R)-1-hydroxyethyl]-8-(isopropylamino)pyrido[3,4-d]pyrimidin-2-yl]amino]-2-methyl-3-pyridyl]piperazin-2-one 1-[6-[[8-(isopropylamino)-6-[(3S)-tetrahydrofuran-3-yl]pyrido[3,4-d]pyrimidin-2-yl]amino]-3-pyridyl]piperazin-2-one (1R)-1-[2-(5,6,7,8-tetrahydro-1,6-naphthyridin-2-ylamino)-8-[[(3S)-tetrahydropyran-3-yl]amino]pyrido[3,4-d]pyrimidin-6-yl]ethanol 1-[6-[[8-(isopropylamino)-6-(3-methyloxetan-3-yl)pyrido[3,4-d]pyrimidin-2-yl]amino]-3-pyridyl]piperazin-2-one (1R)-1-[2-[[5-[4-(dimethylamino)cyclohexoxy]-2-pyridyl]amino]-8-[[(3S)-tetrahydropyran-3-yl]amino]pyrido[3,4-d]pyrimidin-6-yl]ethanol 6-[(1R)-1-methoxyethyl]-N2-[5-(piperazin-1-ylmethyl)-2-pyridyl]-N8-propyl-pyrido[3,4-d]pyrimidine-2,8-diamine 6-[(1R)-1-methoxyethyl]-N2-(6-piperazin-1-ylpyridazin-3-yl)-N8-propyl-pyrido[3,4-d]pyrimidine-2,8-diamine 1-[[6-[[6-(difluoromethyl)-8-[(4-methylcyclohexyl)amino]pyrido[3,4-d]pyrimidin-2-yl]amino]-3-pyridyl]methyl]piperidine-4-carboxylic acid (1R)-1-[8-(ethylamino)-2-[[5-[[4-(2-hydroxyethyl)piperazin-1-yl]methyl]-2-pyridyl]amino]pyrido[3,4-d]pyrimidin-6-yl]ethanol (1R)-1-[2-[[5-[[4-(2-hydroxyethyl)piperazin-1-yl]methyl]-2-pyridyl]amino]-8-(propylamino)pyrido[3,4-d]pyrimidin-6-yl]ethanol N8-isopropyl-6-(3-methyloxetan-3-yl)-N2-(6-piperazin-1-ylpyridazin-3-yl)pyrido[3,4-d]pyrimidine-2,8-diamine N8-isopropyl-6-(3-methyloxetan-3-yl)-N2-[5-(piperazin-1-ylmethyl)-2-pyridyl]pyrido[3,4-d]pyrimidine-2,8-diamine 6-(3-methyloxetan-3-yl)-N2-[5-(piperazin-1-ylmethyl)-2-pyridyl]-N8-[(3S)-tetrahydropyran-3-yl]pyrido[3,4-d]pyrimidine-2,8-diamine 4-[6-[[6-[(1R)-1-hydroxyethyl]-8-[isopropyl(methyl)amino]pyrido[3,4-d]pyrimidin-2-yl]amino]-3-pyridyl]-1,4-diazepan-5-one (1R)-1-[8-(isopropylamino)-2-[(6-methyl-5-piperazin-1-yl-2-pyridyl)amino]pyrido[3,4-d]pyrimidin-6-yl]ethanol (1R)-1-[2-[[6-(2-hydroxyethyl)-7,8-dihydro-5H-1,6-naphthyridin-2-yl]amino]-8-(isopropylamino)pyrido[3,4-d]pyrimidin-6-yl]ethanol (1R)-1-[8-(isopropylamino)-2-[[6-[2-(methylamino)ethyl]-7,8-dihydro-5H-1,6-naphthyridin-2-yl]amino]pyrido[3,4-d]pyrimidin-6-yl]ethanol N2-(6-piperazin-1-ylpyridazin-3-yl)-6-[(3S)-tetrahydrofuran-3-yl]-N8-[(3S)-tetrahydropyran-3-yl]pyrido[3,4-d]pyrimidine-2,8-diamine N2-[5-(piperazin-1-ylmethyl)-2-pyridyl]-6-[(3R)-tetrahydrofuran-3-yl]-N8-[(3S)-tetrahydropyran-3-yl]pyrido[3,4-d]pyrimidine-2,8-diamine (1R)-1-[2-[[6-[2-(dimethylamino)ethyl]-7,8-dihydro-5H-1,6-naphthyridin-2-yl]amino]-8-(isopropylamino)pyrido[3,4-d]pyrimidin-6-yl]ethanol (2S)-1-[4-[[6-[[8-(ethylamino)-6-[(1R)-1-hydroxyethyl]pyrido[3,4-d]pyrimidin-2-yl]amino]-3-pyridyl]methyl]piperazin-1-yl]propan-2-ol (2R)-1-[4-[[6-[[8-(ethylamino)-6-[(1R)-1-hydroxyethyl]pyrido[3,4-d]pyrimidin-2-yl]amino]-3-pyridyl]methyl]piperazin-1-yl]propan-2-ol (1R)-1-[8-(isopropylamino)-2-[[5-[(2R)-2-methylpiperazin-1-yl]-2-pyridyl]amino]pyrido[3,4-d]pyrimidin-6-yl]ethanol (1R)-1-[8-(isopropylamino)-2-[[5-[(2S)-2-methylpiperazin-1-yl]-2-pyridyl]amino]pyrido[3,4-d]pyrimidin-6-yl]ethanol N8-isopropyl-N2-(5-piperazin-1-yl-2-pyridyl)-6-[(2S)-tetrahydrofuran-2-yl]pyrido[3,4-d]pyrimidine-2,8-diamine (1R)-1-[8-(cyclobutylamino)-2-[[5-[[4-(2-hydroxyethyl)piperazin-1-yl]methyl]-2-pyridyl]amino]pyrido[3,4-d]pyrimidin-6-yl]ethanol (1R)-1-[8-(cyclopropylmethylamino)-2-[[5-[[4-(2-hydroxyethyl)piperazin-1-yl]methyl]-2-pyridyl]amino]pyrido[3,4-d]pyrimidin-6-yl]ethanol 6-(3-methyloxetan-3-yl)-N2-(5-piperazin-1-yl-2-pyridyl)-N8-propyl-pyrido[3,4-d]pyrimidine-2,8-diamine 6-(3-methyloxetan-3-yl)-N2-[5-(piperazin-1-ylmethyl)-2-pyridyl]-N8-propyl-pyrido[3,4-d]pyrimidine-2,8-diamine N2-(5-piperazin-1-yl-2-pyridyl)-N8-propyl-6-tetrahydrofuran-3-yl-pyrido[3,4-d]pyrimidine-2,8-diamine N2-[5-(piperazin-1-ylmethyl)-2-pyridyl]-N8-propyl-6-tetrahydrofuran-3-yl-pyrido[3,4-d]pyrimidine-2,8-diamine N8-isopropyl-6-(3-methyloxetan-3-yl)-N2-(5-piperazin-1-yl-2-pyridyl)pyrido[3,4-d]pyrimidine-2,8-diamine N8-isopropyl-N2-(5-piperazin-1-yl-2-pyridyl)-6-tetrahydrofuran-3-yl-pyrido[3,4-d]pyrimidine-2,8-diamine 2-[4-[[6-[[8-(isopropylamino)-6-tetrahydrofuran-3-yl-pyrido[3,4-d]pyrimidin-2-yl]amino]-3-pyridyl]methyl]piperazin-1-yl]ethanol 2-[4-[[6-[[6-tetrahydrofuran-3-yl-8-[[(3S)-tetrahydropyran-3-yl]amino]pyrido[3,4-d]pyrimidin-2-yl]amino]-3-pyridyl]methyl]piperazin-1-yl]ethanol (1R)-1-[2-[[5-[[4-(hydroxymethyl)-1-piperidyl]methyl]-2-pyridyl]amino]-8-(isopropylamino)pyrido[3,4-d]pyrimidin-6-yl]ethanol 1-[[6-[[6-[([R)-1-hydroxyethyl]-8-(isopropylamino)pyrido[3,4-d]pyrimidin-2-yl]amino]-3-pyridyl]methyl]piperidin-4-ol 1-[[6-[[8-(tert-butylamino)-6-[(1R)-1-hydroxyethyl]pyrido[3,4-d]pyrimidin-2-yl]amino]-3-pyridyl]methyl]piperidin-4-ol (1R)-1-[8-(tert-butylamino)-2-[[5-[[4-(hydroxymethyl)-1-piperidyl]methyl]-2-pyridyl]amino]pyrido[3,4-d]pyrimidin-6-yl]ethanol 1-[[6-[[6-[(1R)-1-hydroxyethyl]-8-(isobutylamino)pyrido[3,4-d]pyrimidin-2-yl]amino]-3-pyridyl]methyl]piperidin-4-ol (1R)-1-[2-[[5-[[4-(hydroxymethyl)-1-piperidyl]methyl]-2-pyridyl]amino]-8-(isobutylamino)pyrido[3,4-d]pyrimidin-6-yl]ethanol 1-[6-[[6-[(1R)-1-hydroxypropyl]-8-(isopropylamino)pyrido[3,4-d]pyrimidin-2-yl]amino]-3-pyridyl]piperazin-2-one (1R)-1-[2-[[5-[[4-(2-hydroxyethyl)piperazin-1-yl]methy1]-6-methyl-2-pyridyl]amino]-8-(propylamino)pyrido[3,4-d]pyrimidin-6-yl]ethanol
Aspect (25): A pharmaceutical composition comprising the compound or pharmaceutically acceptable salt thereof according to any one of Aspects (1) to (24) and a pharmaceutically acceptable carrier.
Aspect (26): A pharmaceutical composition exhibiting a CDK4/6 inhibitory activity, comprising the compound or pharmaceutically acceptable salt thereof according to any one of Aspects (1) to (24) as an active ingredient.
Aspect (27): A drug for prevention or treatment of rheumatoid arthritis, arteriosclerosis, pulmonary fibrosis, cerebral infarction, or cancer, the drug comprising the compound or pharmaceutically acceptable salt thereof according to any one of Aspects (1) to (24) as an active ingredient.

### Advantageous Effects of Invention

The compound of the present invention exhibits a superior CDK4/6 inhibitory activity and is useful as a drug for prevention or treatment of rheumatoid arthritis, arteriosclerosis, pulmonary fibrosis, cerebral infarction, or cancer.

### Brief Description of Drawings

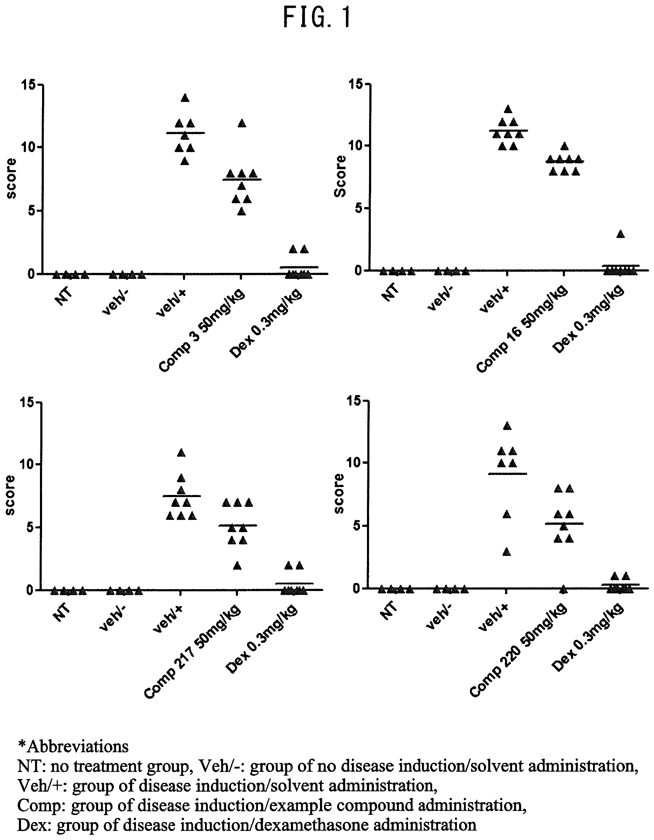

Fig. 1 is graphs showing the results (scores) obtained through administration of the compound of the present invention to mice.

### Description of Embodiments

Now will be described the structures (groups) of the compound of the present invention represented by Formula (I). The description of "groups" with parentheses is as follows: For example, the term "(cycloalkyl)-alkyl" refers to a cycloalkyl group bonded to an alkyl group such that the alkyl group is bonded to a structure other than the cycloalkyl group. Similarly, the term "(heterocyclyl)-alkyl" refers to a heterocyclyl group bonded to an alkyl group such that the alkyl group is bonded to a structure other than the heterocyclyl group.

It must be noted that, as used herein and the annexed claims, the singular form "a", "an" or "the" may include plural referents unless the context clearly dictates otherwise.

As used herein, "C₃₋₆ cycloalkyl group substituted with zero to two -OH groups, zero to two C₁₋₈ alkoxy groups, and zero to six fluorine atoms" refers to the case where the C₃₋₆ cycloalkyl group is substituted with the following substituents: zero to two -OH groups, zero to two C₁₋₈ alkoxy groups, and zero to six fluorine atoms. Examples of the substituted C₃₋₆ cycloalkyl group include a C₃₋₆ cycloalkyl group substituted with two -OH groups, one C₁₋₈ alkoxy group, and three fluorine atoms; a C₃₋₆ cycloalkyl group substituted with two C₁₋₈ alkoxy groups and four fluorine atoms; and a C₃₋₆ cycloalkyl group substituted with one -OH group, and the like. The C₃₋₆ cycloalkyl group is not substituted in the case where the number of all the substituents is zero.

As used herein, "C₁₋₈" refers to a group having one to eight carbon atoms, and "C₁₋₆" refers to a group having one to six carbon atoms. Similarly, "5- to 10-membered" refers to a structure having 5 to 10 carbon atoms, and "5- or 6-membered" refers to a structure having five or six carbon atoms.

Non-limiting examples of the groups described in this specification are as follows:
The term "alkyl" as used herein refers to a monovalent group obtained by removal of one hydrogen atom from an alkane at any carbon atom.

The term "alkylene" as used herein refers to a divalent group obtained by removal of two hydrogen atoms from an alkane at any two different carbon atoms.

The term "alkane" as used herein refers to a saturated aliphatic hydrocarbon.

The term "C₁₋₈ alkyl" as used herein refers to a linear or branched hydrocarbon group having one to eight carbon atoms. Examples of the C₁₋₈ alkyl include methyl, ethyl, n-propyl, isopropyl, n-butyl, isobutyl, sec-butyl, tert-butyl, n-pentyl, neopentyl, isopentyl, 1,2-dimethylpropyl, n-hexyl, isohexyl, 1,1-dimethylbutyl, 2,2-dimethylbutyl, 1-ethylbutyl, 2-ethylbutyl, isoheptyl, n-octyl, isooctyl, and the like.

The alkane of "C₁₋₈ alkylene" as used herein refers to a linear or branched hydrocarbon having one to eight carbon atoms. Examples of the alkane include methane, ethane, propane, n-butane, 2-methylpropane, n-pentane, 2,2-dimethylpropane, n-hexane, 2-methylpentane, 3-methylpentane, 2,2-dimethylbutane, 2,3-dimethylbutane, n-heptane, 2,2-dimethylhexane, 2,3-dimethylhexane, n-octane, 2-methylheptane, and the like.

The term "cycloalkyl" as used herein refers to a monovalent group obtained by removal of one hydrogen atom from a cycloalkane at any carbon atom.

The term "cycloalkylene" as used herein refers to a divalent group obtained by removal of two hydrogen atoms from a cycloalkane at any two different carbon atoms.

The term "cycloalkylidene" refers to a divalent group obtained by removal of two hydrogen atoms from a cycloalkane at any one carbon atom.

The term "cycloalkane" as used herein refers to an alicyclic hydrocarbon.

The cycloalkane of "C₃₋₁₂ cycloalkyl, " "C₃₋₁₂ cycloalkylene," or "C₃₋₁₂ cycloalkylidene" as used herein refers to a monocyclic or polycyclic 3- to 12- membered aliphatic hydrocarbon ring. Specific examples of the cycloalkane include cyclopropane, cyclobutane, cyclopentane, cyclohexane, cycloheptane, cyclooctane, spiro[3.3]heptane, bicyclo[1.1.1]pentane, bicyclo[2.2.2]octane, adamantane, and the like.

The term "heterocyclyl" as used herein refers to a monovalent group obtained by removal of one hydrogen atom from a heterocycle at any carbon or nitrogen atom.

The term "heterocyclylene" as used herein refers to a divalent group obtained by removal of two hydrogen atoms from a heterocycle at any two different carbon or nitrogen atoms.

The term "heterocyclylidene" as used herein refers to a divalent group obtained by removal of two hydrogen atoms from a heterocycle at any one carbon atom.

The term "heterocycle" as used herein refers to a ring containing a heteroatom selected from sulfur, nitrogen, and oxygen atoms.

The heterocycle of "4- to 12-membered heterocyclyl," "4- to 12-membered heterocyclylene," or "4- to 12-membered heterocyclylidene" as used herein refers to "4-to 12-membered heterocycloalkane," "4- to 12-membered heterocycloalkane" having an unsaturated bond, a 4- to 12-membered ring composed of a heterocycloalkane and a heteroarene or arene bonded to a portion of the heterocycloalkane, a 4- to 12-membered ring composed of a cycloalkane and a heteroarene bonded to a portion of the cycloalkane, a 4- to 12-membered ring containing a heteroatom and having a spiro structure, or a 4- to 12-membered ring containing a heteroatom and having a cross-linked structure. The term "4- to 12-membered heterocycloalkane" refers to a 4- to 12-membered cyclic heteroalkane; i.e., a monocyclic or polycyclic aliphatic hydrocarbon ring containing one to four heteroatoms selected from sulfur, nitrogen, and oxygen atoms. Specific examples of the "4- to 12-membered heterocycloalkane" include aziridine, thiirane, azetidine, oxetane, thietane, tetrahydrofuran, tetrahydropyran, 1,4-dioxane, piperidine, piperazine, pyrrolidine, imidazolidine, pyrazolidine, morpholine, thiomorpholine, tetrahydrothiopyran, tetrahydrothiophene, 1,4-diazepane, oxepane, and the like. A compound having a "spiro structure" is composed of two cyclic structures (cycloalkanes or heterocycloalkanes) that are bonded to one common carbon atom. Examples of the compound include 2-azaspiro[3.3]heptane, 1,6-diazaspiro[3.3]heptane, 2,6-diazaspiro[3.3]heptane, 2,6-diazaspiro[3.4]octane, 2,7-diazaspiro[3.5]nonane, 1,7-diazaspiro[4.5]decane, 2,8-diazaspiro[4.5]decane, 4,7-diazaspiro[2.5]octane, and the like. A compound having a "cross-linked structure" is composed of two cyclic structures (cycloalkanes and heterocycloalkanes) that are bonded to two or more common carbon, nitrogen, or oxygen atoms. Examples of the compound include 2,5-diazabicyclo[2.2.2]octane, 3,8-diazabicyclo[3.2.1]octane, 1,4-diazabicyclo[3.2.2]nonane, octahydropyrrolo[3,4-b]pyrrole, and the like.

The term "aryl" as used herein refers to a monovalent group obtained by removal of one hydrogen atom from an arene at any carbon atom.

The term "arylene" as used herein refers to a divalent group obtained by removal of two hydrogen atoms from an arene at any two different carbon atoms.

The term "arene" as used herein refers to an aromatic hydrocarbon.

The arene of "C₆₋₁₀ aryl" or "C₆₋₁₀ arylene" as used herein refers to an aromatic hydrocarbon ring having six to ten carbon atoms. Specific examples of the arene include benzene, naphthalene, and the like.

The term "heteroaryl" as used herein refers to a monovalent group obtained by removal of one hydrogen atom from a heteroarene at any carbon or nitrogen atom.

The term "heteroarylene" as used herein refers to a divalent group obtained by removal of two hydrogen atoms from a heteroarene at any two different carbon or nitrogen atoms.

The term "heteroarene" as used herein refers to an aromatic heterocyclic ring containing a heteroatom selected from sulfur, nitrogen, and oxygen atoms.

The heteroarene of "5- to 10-membered heteroaryl" or "5- to 10-membered heteroarylene" as used herein refers to a 5- to 10-membered aromatic heterocyclic ring containing one to four heteroatoms selected from sulfur, nitrogen, and oxygen atoms. Specific examples of the heteroarene include furan, thiophene, pyrrole, imidazole, pyrazole, triazole, tetrazole, thiazole, oxazole, isoxazole, oxadiazole, thiadiazole, isothiazole, pyridine, pyridazine, pyrazine, pyrimidine, quinolone, isoquinolone, benzofuran, benzothiophene, indole, indazole, benzimidazole, and the like.

The term " (4- to 12-membered heterocyclyl)-C₁₋₆ alkyl" as used herein refers to a 4- to 12-membered heterocyclyl group bonded to a C₁₋₆ alkyl group such that the C₁₋₆ alkyl group is bonded to a structure other than the 4- to 12-membered heterocyclyl group. Specific examples of the (4- to 12-membered heterocyclyl)-C₁₋₆ alkyl include groups prepared by bonding of any of the above-exemplified 4- to 12-membered heterocyclyl groups to any of the above-exemplified C₁₋₆ alkyl groups.

The term " (C₆₋₁₀ aryl) -C₁₋₆ alkyl" as used herein refers to a C₆₋₁₀ aryl group bonded to a C₁₋₆ alkyl group such that the C₁₋₆ alkyl group is bonded to a structure other than the C₆₋₁₀ aryl group. Specific examples of the (C₆₋₁₆ aryl)-C₁₋₆ alkyl include groups prepared by bonding of any of the above-exemplified C₆₋₁₀ aryl groups to any of the above-exemplified C₁₋₆ alkyl groups.

The term "(5- to 10-membered heteroaryl)-C₁₋₆ alkyl" as used herein refers to a 5- to 10-membered heteroaryl group bonded to a C₁₋₆ alkyl group such that the C₁₋₆ alkyl group is bonded to a structure other than the 5- to 10-membered heteroaryl group. Specific examples of the (5-to 10-membered heteroaryl)-C₁₋₆ alkyl include groups prepared by bonding of any of the above-exemplified 5- to 10-membered heteroaryl groups to any of the above-exemplified C₁₋₆ alkyl groups.

The term "C₁₋₈ alkylsulfonyl" as used herein refers to a C₁₋₈ alkyl group bonded to a sulfonyl (-S(=O)₂-) group such that the sulfonyl group is bonded to a structure other than the C₁₋₈ alkyl group.

The term "C₁₋₈ acyl" as used herein refers to a C₁₋₇ alkyl group bonded to a carbonyl (-CO-) group such that the carbonyl group is bonded to a structure other than the C₁₋₇ alkyl group.

The term "halogen" as used herein refers to a fluorine, chlorine, bromine, or iodine atom.

The term "C₁₋₈ alkoxy" as used herein refers to a linear, branched, or cyclic alkoxy group having one to eight carbon atoms. Specific examples of the C₁₋₈ alkoxy include methoxy, ethoxy, n-propoxy, isopropoxy, n-butoxy, isobutoxy, sec-butoxy, tert-butoxy, n-pentyloxy, neopentyloxy, tert-pentyloxy, 2-methylbutoxy, n-hexyloxy, isohexyloxy, cyclopropyloxy, cyclobutyloxy, cyclopentyloxy, cyclohexyloxy, cycloheptyloxy, cyclooctyloxy, spiro[3.3]heptyloxy, bicyclo[2.2.2]octyloxy, and the like.

The term "alkenyl" as used herein refers to a monovalent group obtained by removal of one hydrogen atom from an alkene at any carbon atom.

The term "alkenylene" as used herein refers to a divalent group obtained by removal of two hydrogen atoms from an alkene at any two different carbon atoms.

The term "alkene" as used herein refers to an unsaturated aliphatic hydrocarbon having one double bond.

The term "C₂₋₈ alkenyl" as used herein refers to a chain aliphatic hydrocarbon having one double bond. Examples of the C₂₋₈ alkenyl include ethenyl (or vinyl), propenyl (or allyl), butenyl, and the like.

The term "alkynyl" as used herein refers to a monovalent group obtained by one hydrogen atom from an alkyne at any carbon atom.

The term "alkynylene" as used herein refers to a divalent group obtained by removal of two hydrogen atoms from an alkyne at any two different carbon atoms.

The term "alkyne" as used herein refers to an unsaturated aliphatic hydrocarbon having one triple bond.

The term "C₂₋₄ alkynyl" as used herein refers to a chain hydrocarbon group having one triple bond. Examples of the C₂₋₄ alkynyl include ethynyl, propynyl, butynyl, and the like.

L is preferably -NR⁵-.

The "C₁₋₆ alkyl" of R⁵ is preferably methyl or ethyl.

R⁵ is preferably a hydrogen atom or a methyl group.

The "C₁₋₈ alkyl" of R¹ is preferably methyl, ethyl, n-propyl, isopropyl, n-butyl, isobutyl, sec-butyl, tert-butyl, n-pentyl, neopentyl, isopentyl, 1,2-dimethylpropyl, n-hexyl, isohexyl, 1,1-dimethylbutyl, 2,2-dimethylbutyl, 1-ethylbutyl, 2-ethylbutyl, isoheptyl, n-octyl, or isooctyl.

The "C₃₋₁₂ cycloalkyl" of R¹ is preferably cyclopropyl, cyclobutyl, cyclopentyl, cyclohexyl, spiro[3.3]heptyl, bicyclo[1.1.1]pentane, bicyclo[2.2.2]octyl, or adamantyl.

The "(C₃₋₁₂ cycloalkyl)-C₁₋₆ alkyl" of R¹ is preferably cyclopropylmethyl, cyclobutylmethyl, cyclopentylmethyl, cyclohexylmethyl, or cyclopentylethyl.

The heterocycle of "4- to 12-membered heterocyclyl" in R¹ is preferably azetidine, oxetane, thietane, tetrahydrofuran, 1,4-dioxane, morpholine, thiomorpholine, tetrahydropyran, tetrahydrothiophene, or oxepane.

The " (4- to 12-membered heterocyclyl) -C₁₋₆ alkyl" of R¹ is preferably (tetrahydrofuranyl)methyl, (tetrahydropyranyl)methyl, (tetrahydrofuranyl)ethyl, or (tetrahydropyranyl)ethyl.

The "C₆₋₁₀ aryl" of R¹ is preferably phenyl.

The " (C₆₋₁₀ aryl)-C₁₋₆ alkyl" of R¹ is preferably phenylmethyl or phenylethyl.

The "5- to 10-membered heteroaryl" of R¹ is preferably furanyl, pyrazolyl, or thienyl.

The "halogen" in the substituent of R¹ is preferably a fluorine or chlorine atom.

The "-COOR⁶" in the substituent of R¹ is preferably-COOH or -COOCH₃.

The "R⁷" in the substituent of R¹ is preferably ethyl, n-propyl, isopropyl, n-butyl, isobutyl, sec-butyl, tert-butyl, n-pentyl, neopentyl, isopentyl, 1,1-dimethyl-2-methoxyethyl, 1-methyl-2-methoxyethyl, 1-methyl-2-hydroxyethyl, 2,2,2-trifluoroethyl, hydroxymethyl, or 1-methyl-2,2,2-trifluoroethyl.

The "C₃₋₆ cycloalkyl optionally substituted with a substituent selected from the group consisting of one or two -OH groups, one or two C₁₋₈ alkoxy groups, and one to six fluorine atoms" in the substituent of R¹ is preferably cyclopentyl, cyclohexyl, 4-methoxycyclohexyl, or 4-isopropoxycyclohexyl.

The 3- to 10-membered heterocyclyl optionally substituted with a substituent selected from the group consisting of one or two -OH groups, one or two C₁₋₆ alkoxy groups, and one to six fluorine atoms in the substituent of R¹ is preferably tetrahydrofuranyl, tetrahydropyranyl, or 2,2-dimethyltetrahydropyranyl.

R¹ preferably has any of the following structures:

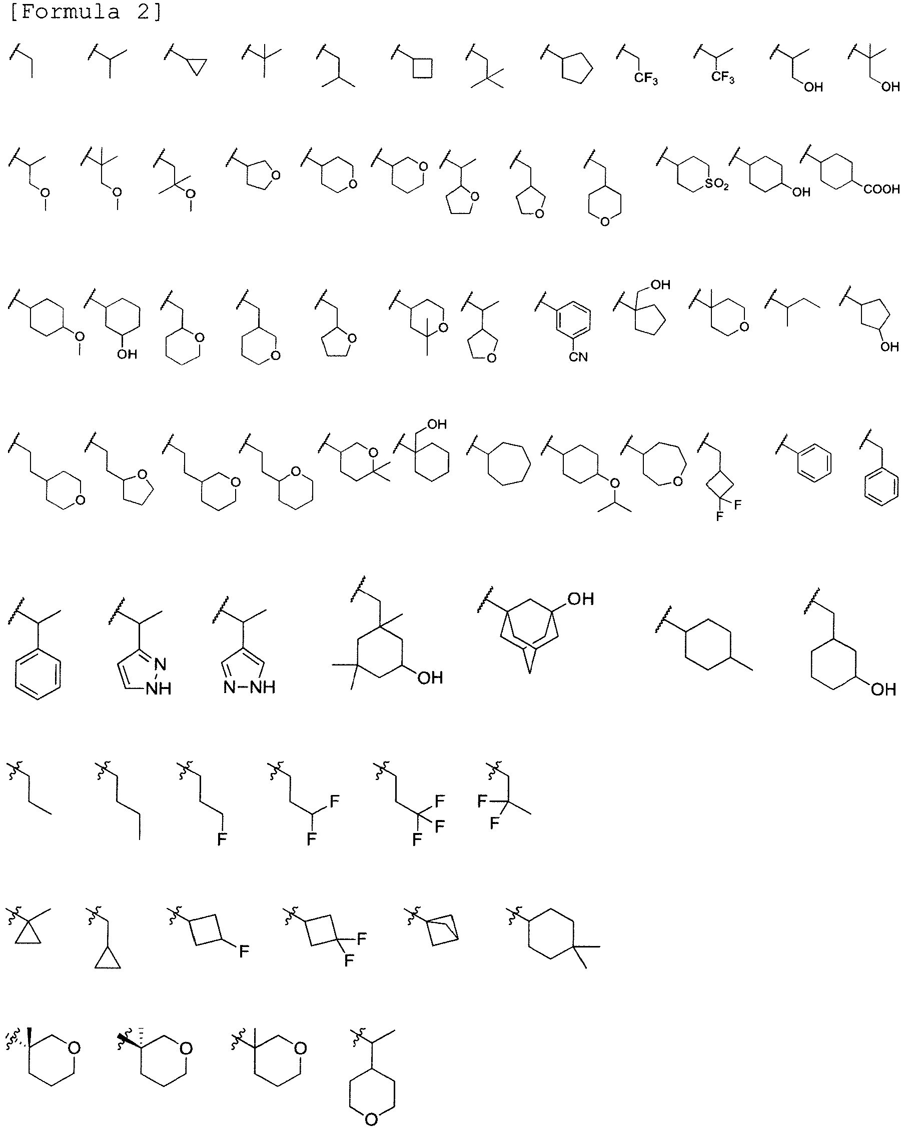

The "C₁₋₈ alkyl" of R² is preferably methyl, ethyl, or n-propyl, and the substituent is preferably a hydroxy, methoxy, or ethoxy group or a fluorine atom. The "4- to 6-membered heterocyclyl" of R² is preferably oxetane or tetrahydrofuranyl.

The "C₁₋₈ acyl" of R² is preferably acetyl.

The "-COOR⁸" of R² is preferably -COOH or -COOCH₃.

The "-CONR⁹R¹⁰" of R² is preferably -CON (CH₃)₂.

R⁹ and R¹⁰ of -CONR⁹R¹⁰ of R² may be bonded via a single bond or -O- to form a ring including the nitrogen atom bonded to R⁹ and R¹⁰. Examples of such a ring include the following structures:

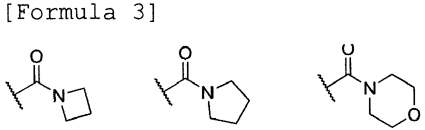

R² preferably has any of the following structures:

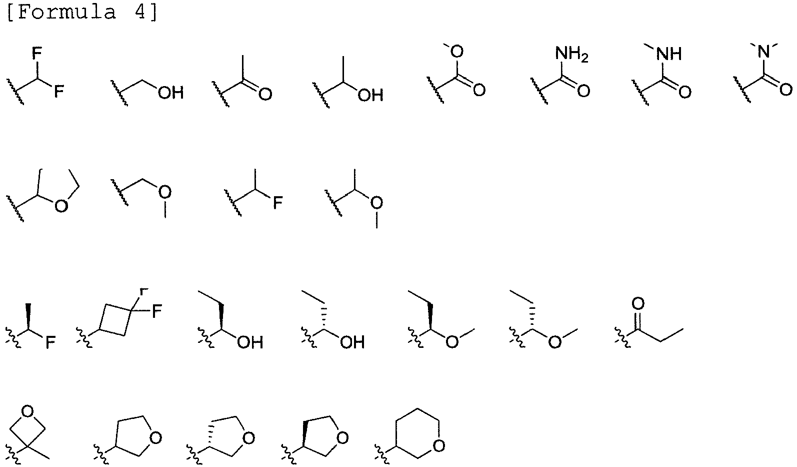

The "C₁₋₈ alkyl" of R³ is preferably methyl.

The "halogen" of R³ is preferably a fluorine or chlorine atom.

R³ is preferably a hydrogen, fluorine, or chlorine atom or a methyl group.

X, Y, and Z preferably correspond to any of the following combinations: X, Y, and Z are each CH; X is a nitrogen atom and Y and Z are each CH; Y is a nitrogen atom and X and Z are each CH; and Z is a nitrogen atom and X and Y are each CH.

The "C₁₋₃ alkylene" of A¹ is preferably methylene, ethylene, or n-propylene. The structure obtained by replacement of one or two sp³ carbon atoms at any positions of A¹ is preferably -O-, -OCH₂-, -OCH₂CH₂-, -OCH₂CH₂CH₂-, -CH₂O-, -CH₂OCH₂-,-CH₂OCH₂CH₂-, -CH₂CO-, -COCH₂-, -CH₂CH₂CO-, -COCH₂CH₂-,-CH₂COCH₂-, -CH₂COCH₂CH₂-, -NR¹⁴-, -NR¹⁴CH₂-, -CH₂NR¹⁴-,-NR¹⁴CH₂CH₂-, -CH₂NR¹⁴CH₂-, or -CH₂CH₂NR¹⁴-.

The "C₁₋₇ alkylene" of A² is preferably methylene, ethylene, or n-propylene.

The "C₃₋₁₂ cycloalkylene" of A² is preferably cyclopropylene, cyclobutylene, cyclopentylene, or cyclohexylene.

The heterocycle of "4- to 12-membered heterocyclylene" of A² is preferably piperidine, piperazine, pyrrolidine, morpholine, tetrahydrofuran, tetrahydropyran, 1,4-diazepane, oxepane, 2-azaspiro[3.3]heptane, 1,6-diazaspiro[3.3]heptane, 2,6-diazaspiro[3.3]heptane, 2,6-diazaspiro[3.4]octane, 2,5-diazabicyclo[2.2.2]octane, 3,8-diazabicyclo[3.2.1]octane, 2,7-diazaspiro[3.5]nonane, 1,7-diazaspiro[4.5]decane, 2,8-diazaspiro[4.5]decane, 4,7-diazaspiro[2.5]octane, 1,4-diazabicyclo[3.2.2]nonane, or octahydropyrrolo[3,4-b] pyrrole.

The heterocycle of "4- to 12-membered heterocyclylidene" of A² is preferably oxetane, tetrahydrofuran, tetrahydropyran, pyrrolidine, piperidine, piperazine, morpholine, or oxepane.

The "C₆₋₁₀ arylene" of A² is preferably phenylene.

The heteroarene of "5- to 10-membered heteroarylene" of A² is preferably furan, thiophene, pyrrole, imidazole, pyrazole, triazole, tetrazole, thiazole, oxazole, isoxazole, oxadiazole, thiadiazole, isothiazole, pyridine, pyridazine, pyrazine, pyrimidine, quinolone, isoquinoline, benzofuran, benzothiophene, indole, indazole, or benzimidazole.

The "halogen" of A³ is preferably a fluorine or chlorine atom.

The "-R²⁵" of A³ is a hydrogen atom or a methyl, ethyl, n-propyl, isopropyl, n-butyl, isobutyl, or tert-butyl group. The -R²⁵ substituted with a substituent is preferably a hydroxymethyl, 1-hydroxyethyl, 2-hydroxyethyl, 2-hydroxy-2-propyl, 2-hydroxy-1-propyl, 1-hydroxy-2-propyl, 1-hydroxy-2-methyl-2-propyl, 2-hydroxy-2-methyl-1-propyl, trifluoromethyl, 2,2,2-trifluoroethyl, carboxymethyl, 1-carboxyethyl, 2-carboxyethyl, 2-carboxy-2-propyl, or cyanomethyl group.

The "-OR²⁶" of A³ is preferably -OH, methoxy, ethoxy, or isopropoxy.

The "-NR²⁷R²⁸" of A³ is preferably amino, dimethylamino, methylamino, pyrrolidin-1-yl, piperidin-1-yl, piperazin-1-yl, or morpholin-1-yl.

The "-C(=O)R²⁹" of A³ is preferably acetyl. The-C(=O)R²⁹ substituted with a substituent is preferably hydroxyacetyl.

The "-C(=O)-OR³⁰" of A³ is preferably -COOH, methoxycarbonyl, ethoxycarbonyl, or isopropoxycarbonyl.

The "-C(=O)-NR³⁴R³⁵" of A³ is preferably aminocarbonyl (or carbamoyl), (methylamino)carbonyl, (dimethylamino)carbonyl, (pyrrolidin-1-yl)carbonyl, (piperidin-1-yl)carbonyl, (morpholin-1-yl)carbonyl, or (piperazin-1-yl)carbonyl.

The "-S(=O)₂-R⁴⁰" of A³ is preferably methanesulfonyl or ethylsulfonyl.

R¹⁴ to R⁴⁴ in A¹, A², and A³ may be bonded in A¹, A², or A³ or between A¹ and A², between A¹ and A³, or between A² and A³ via a single bond, -O-, -NR⁵⁰-, or -S(=O)ₚ- to form a ring. Examples of such a ring include the following structures:

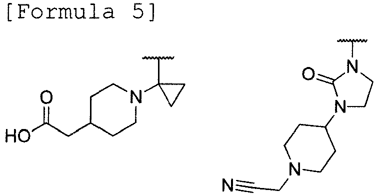

R¹¹ or R¹³ may be bonded to A¹, A², or A³ via a single bond, -O-, -NR⁵¹-, or -S(=O)ₚ- to form a ring. Examples of such a ring include the following structures:

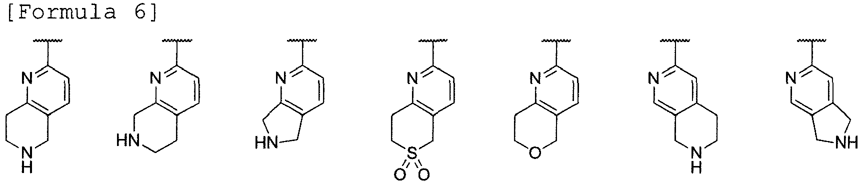

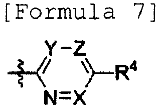

Preferred examples of the aforementioned entire structure are as follows:

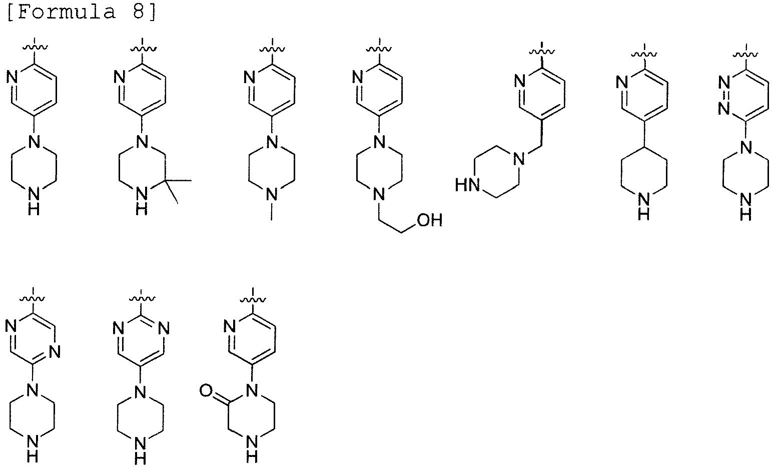

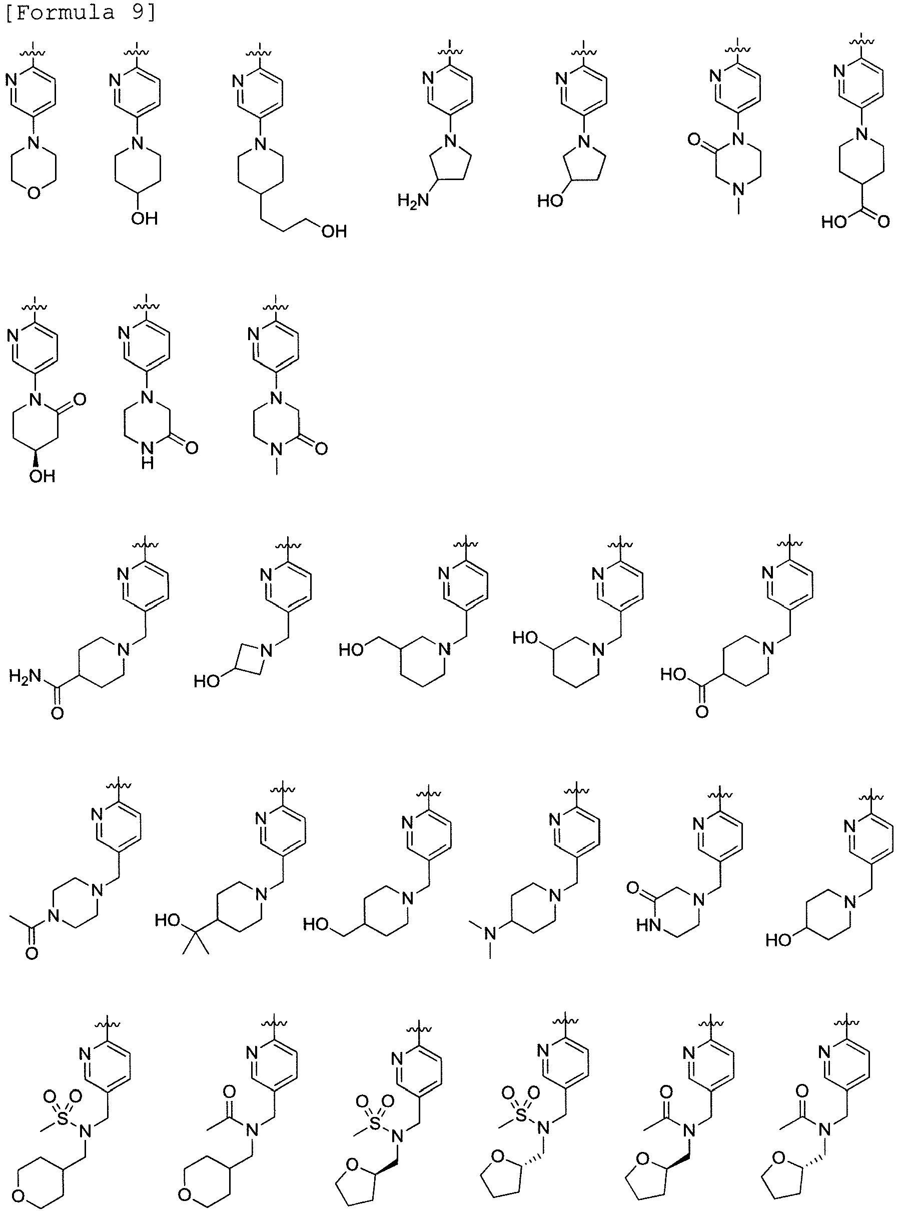

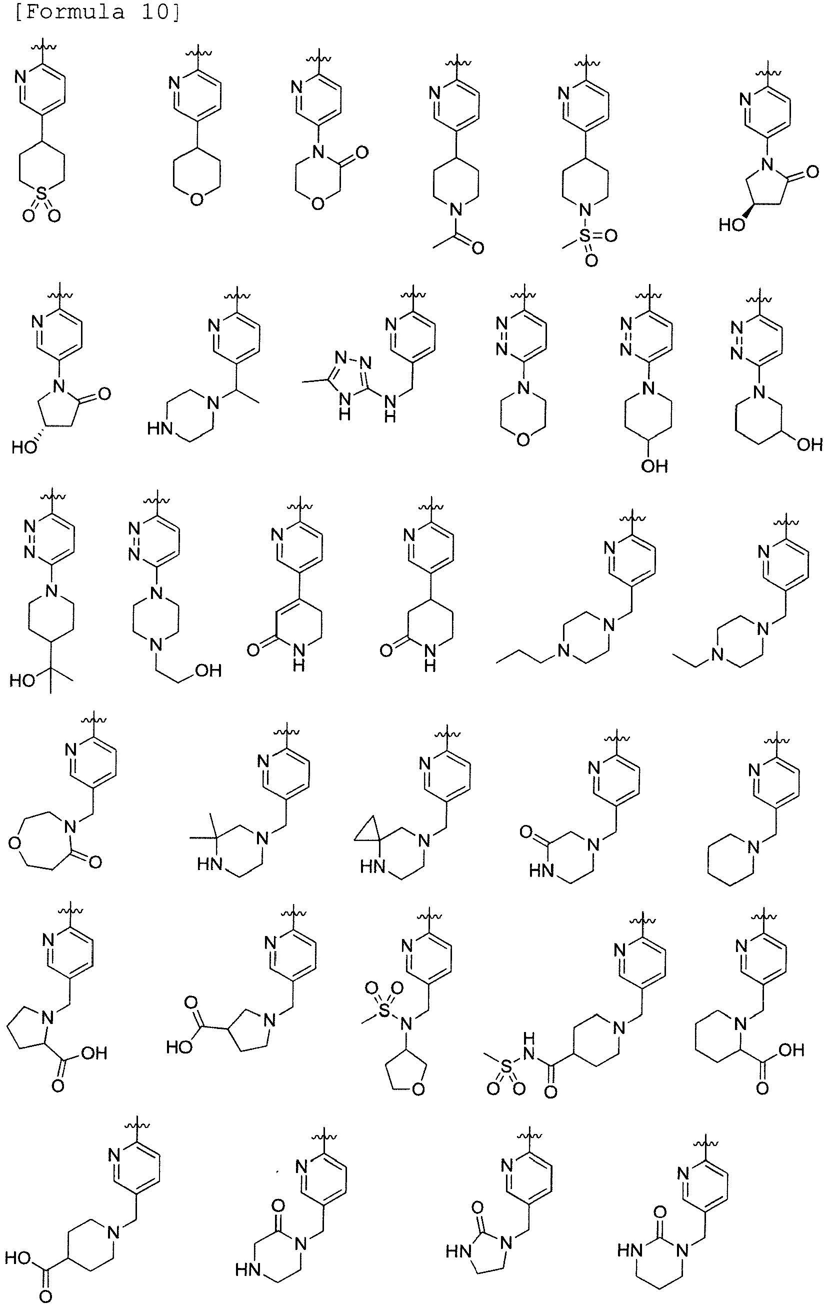

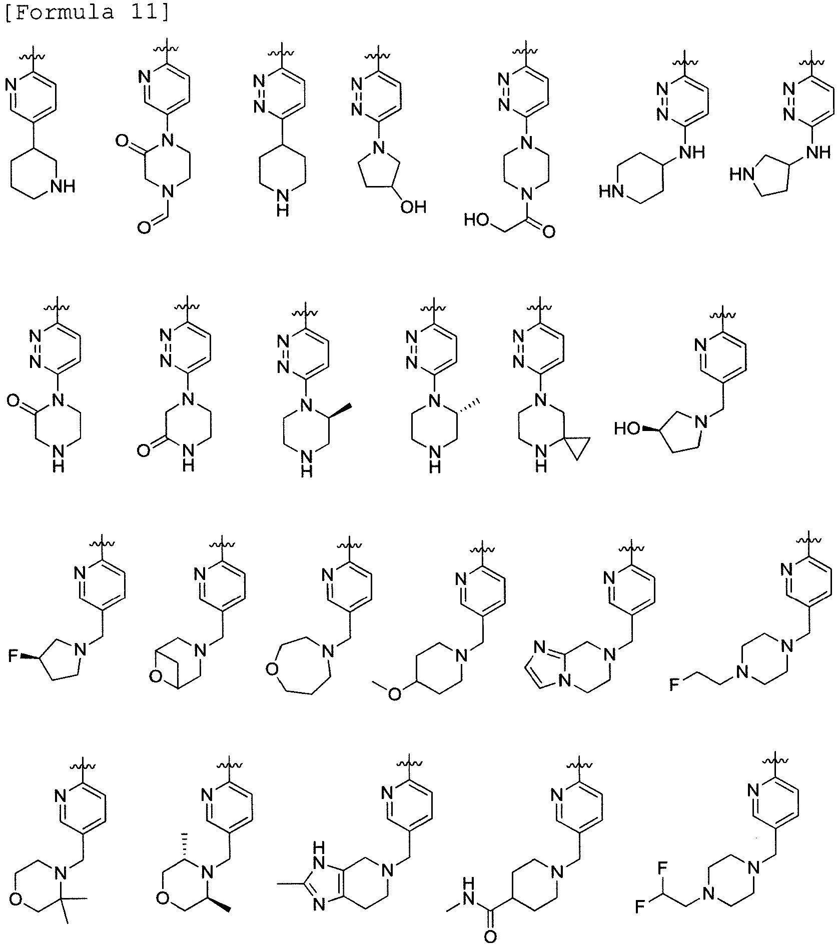

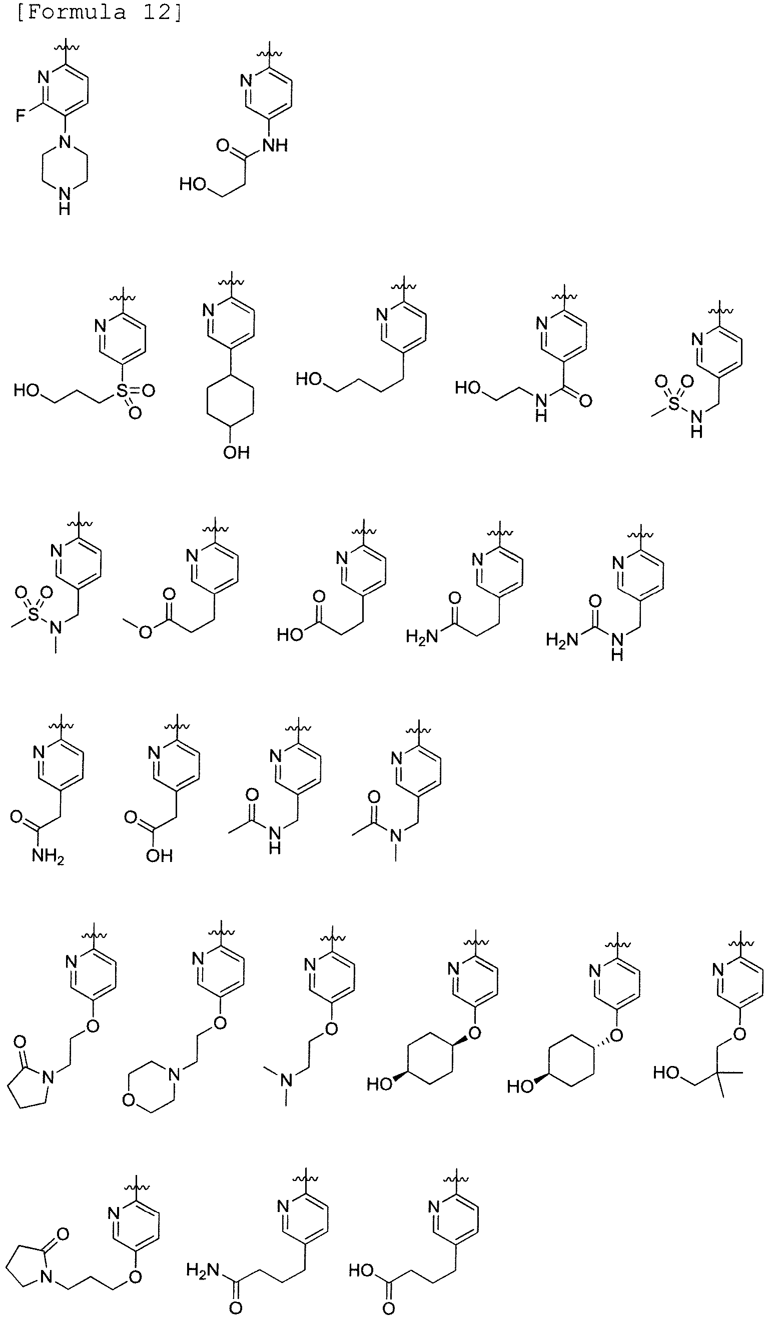

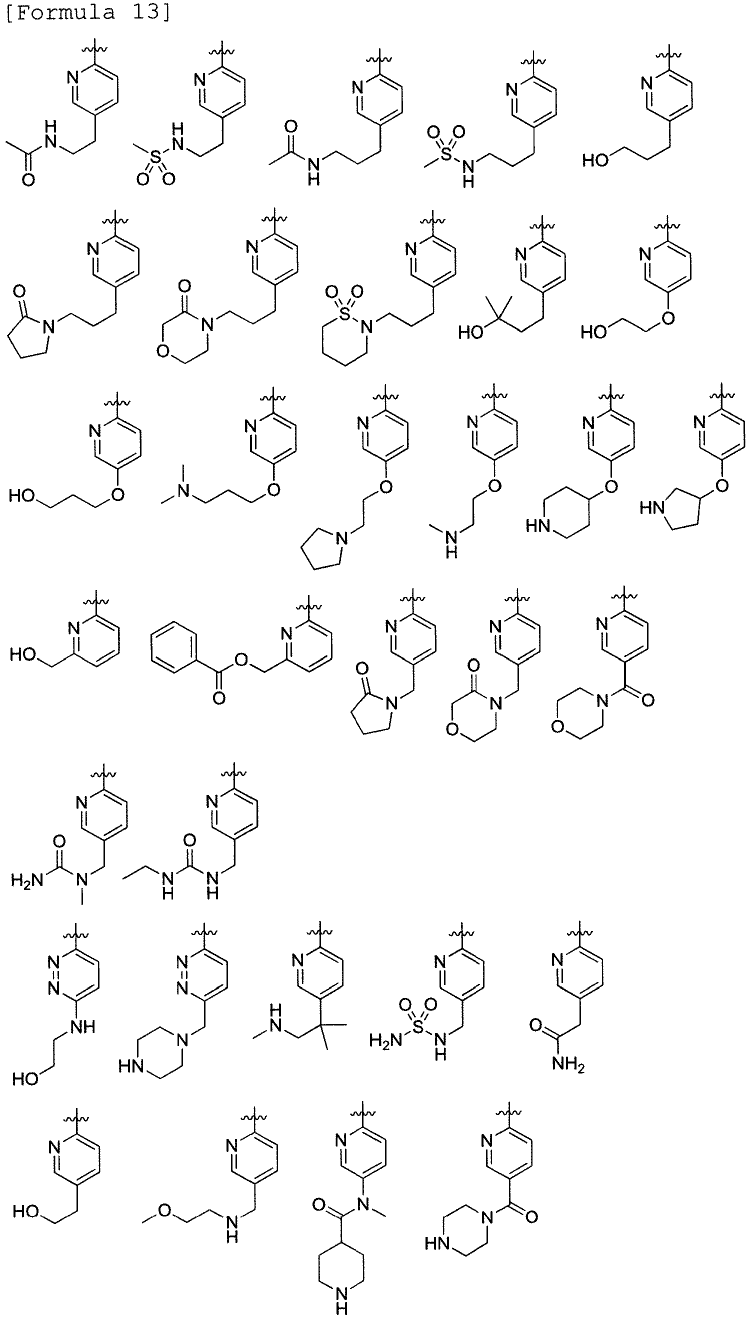

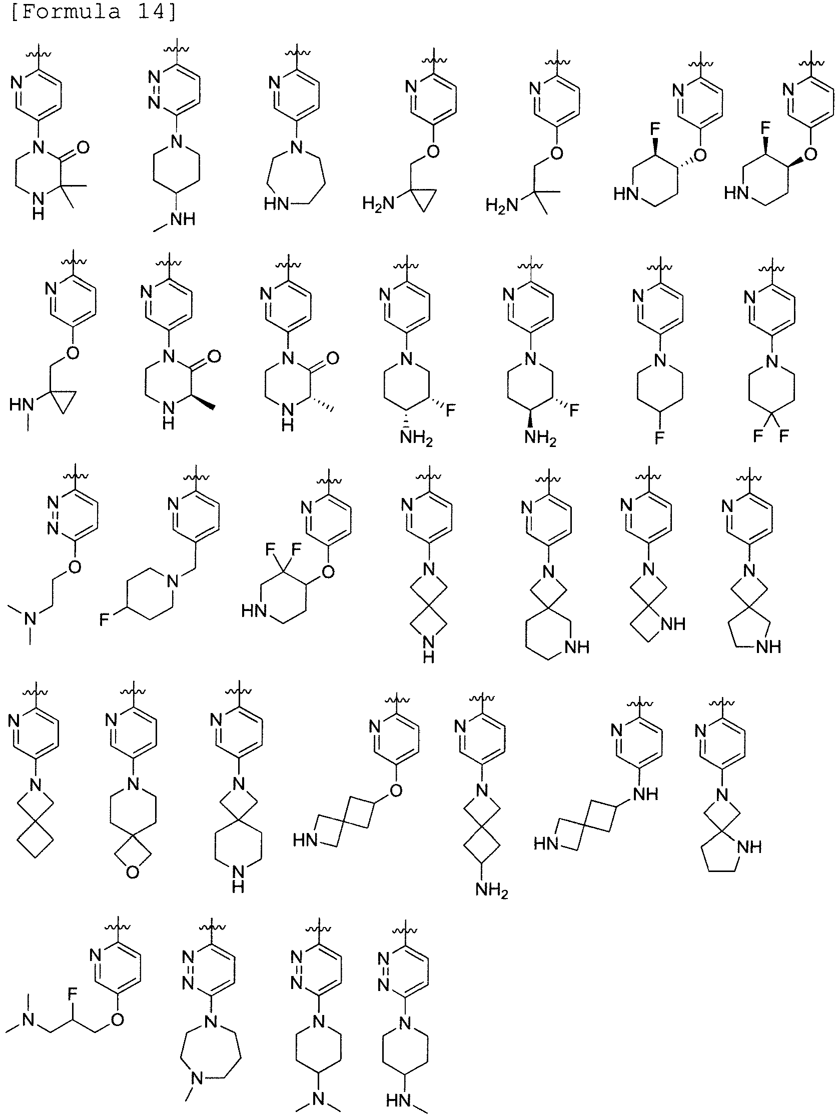

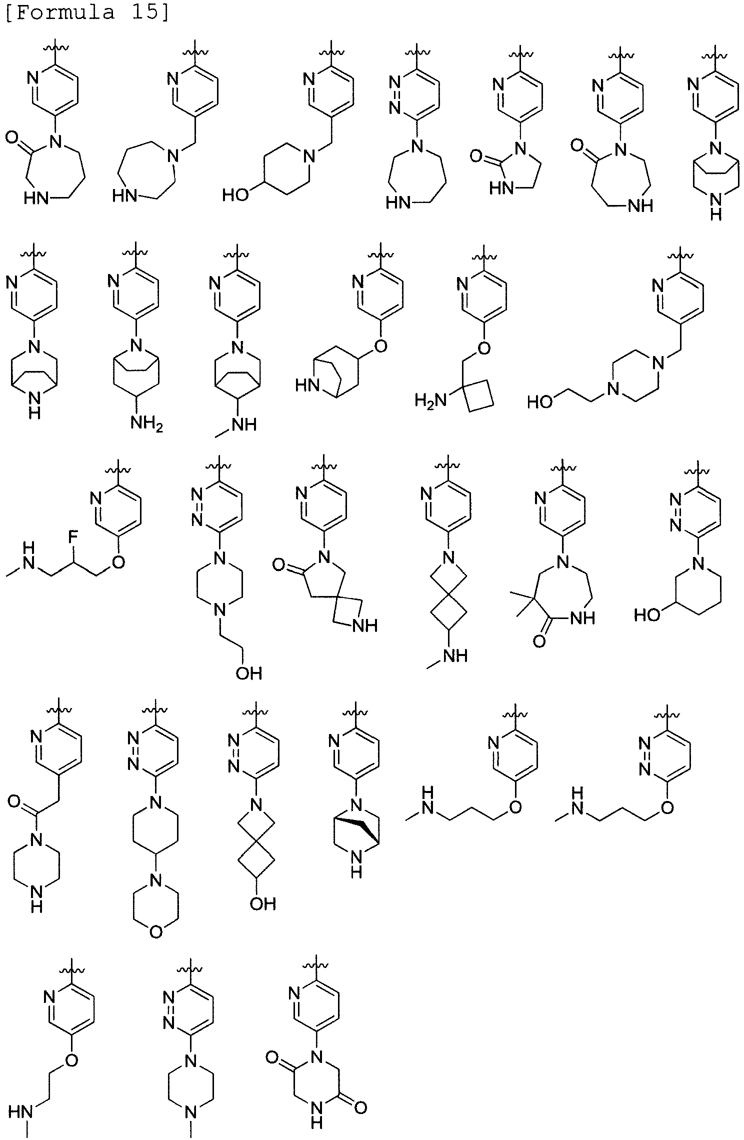

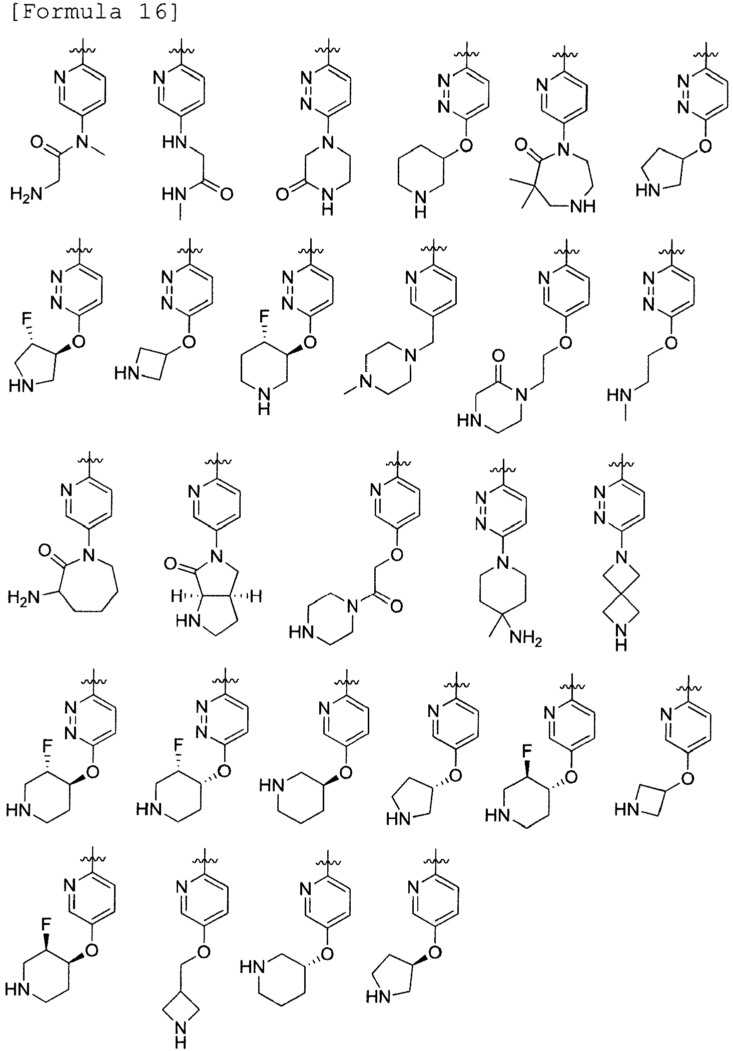

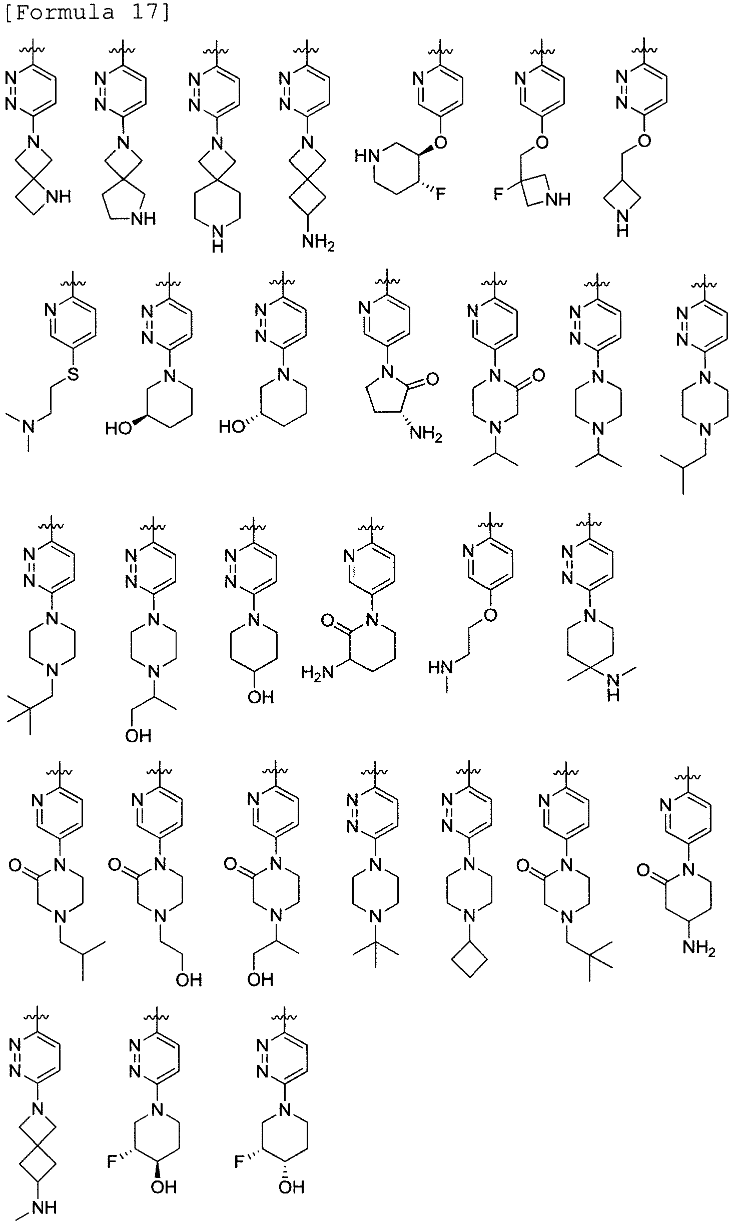

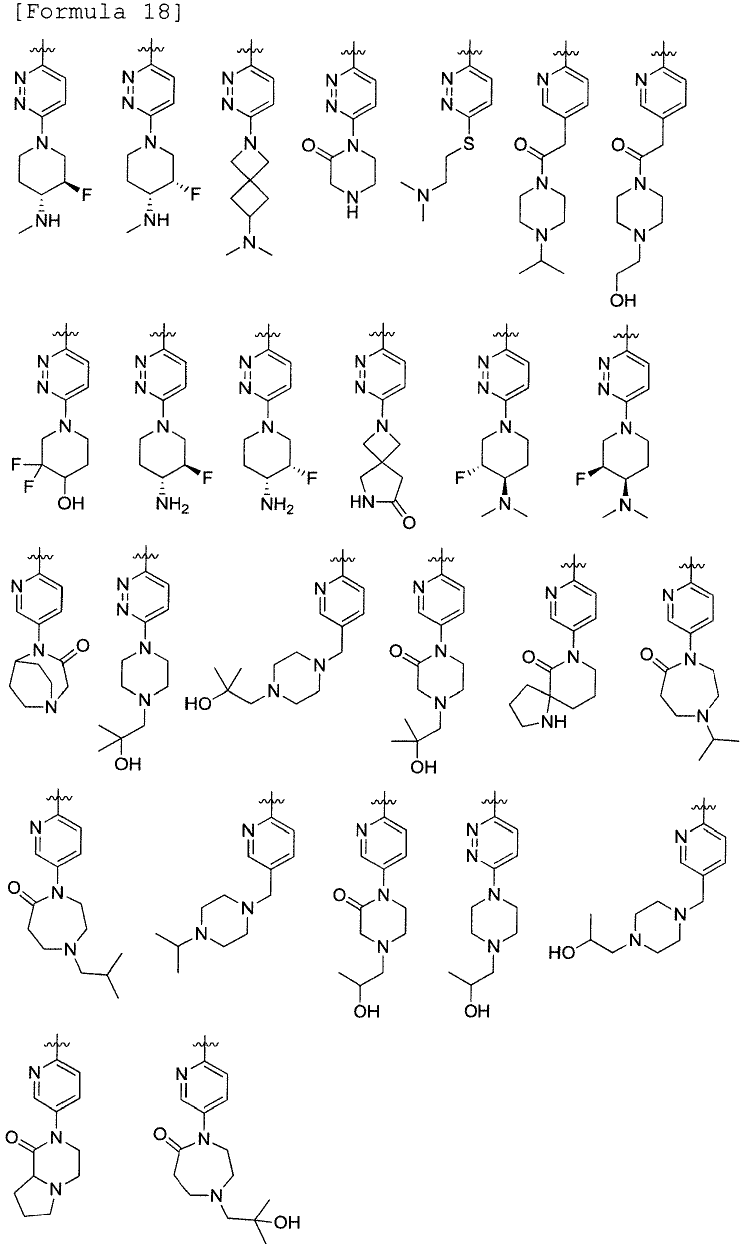

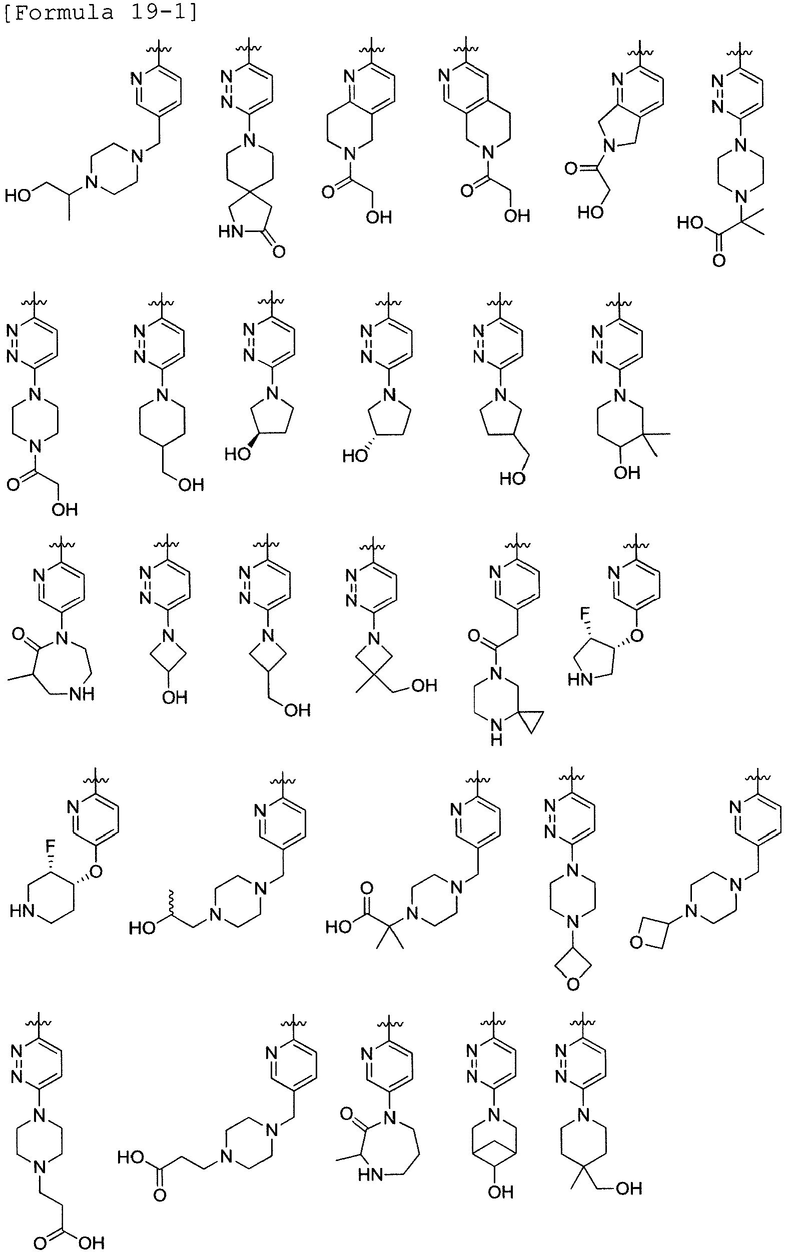

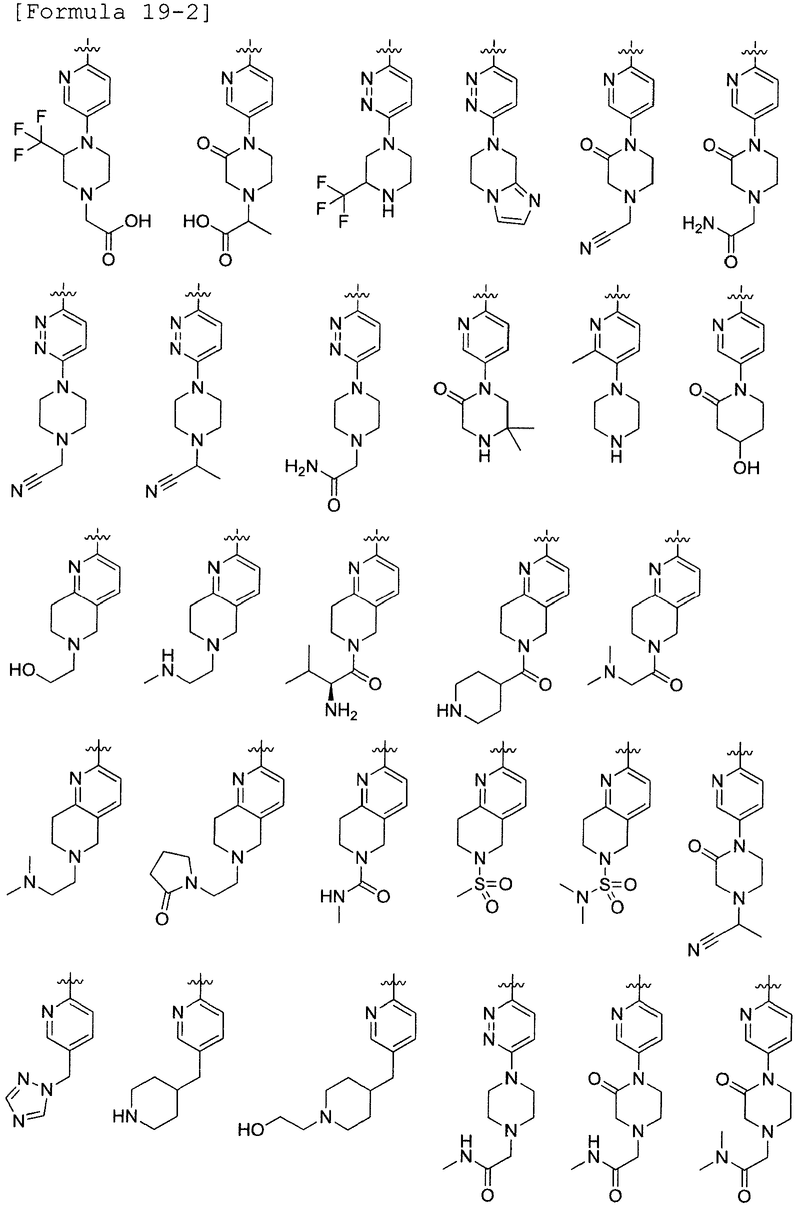

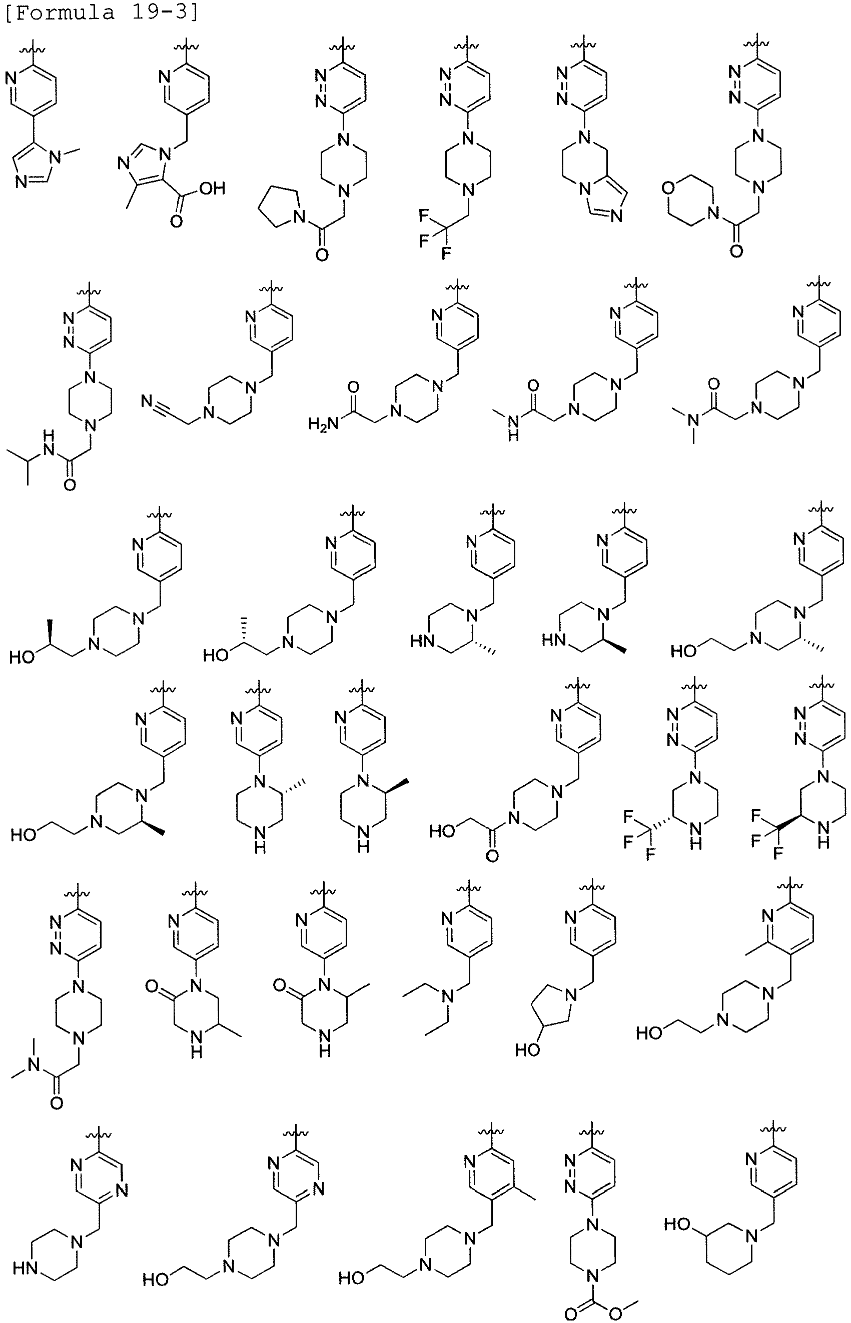

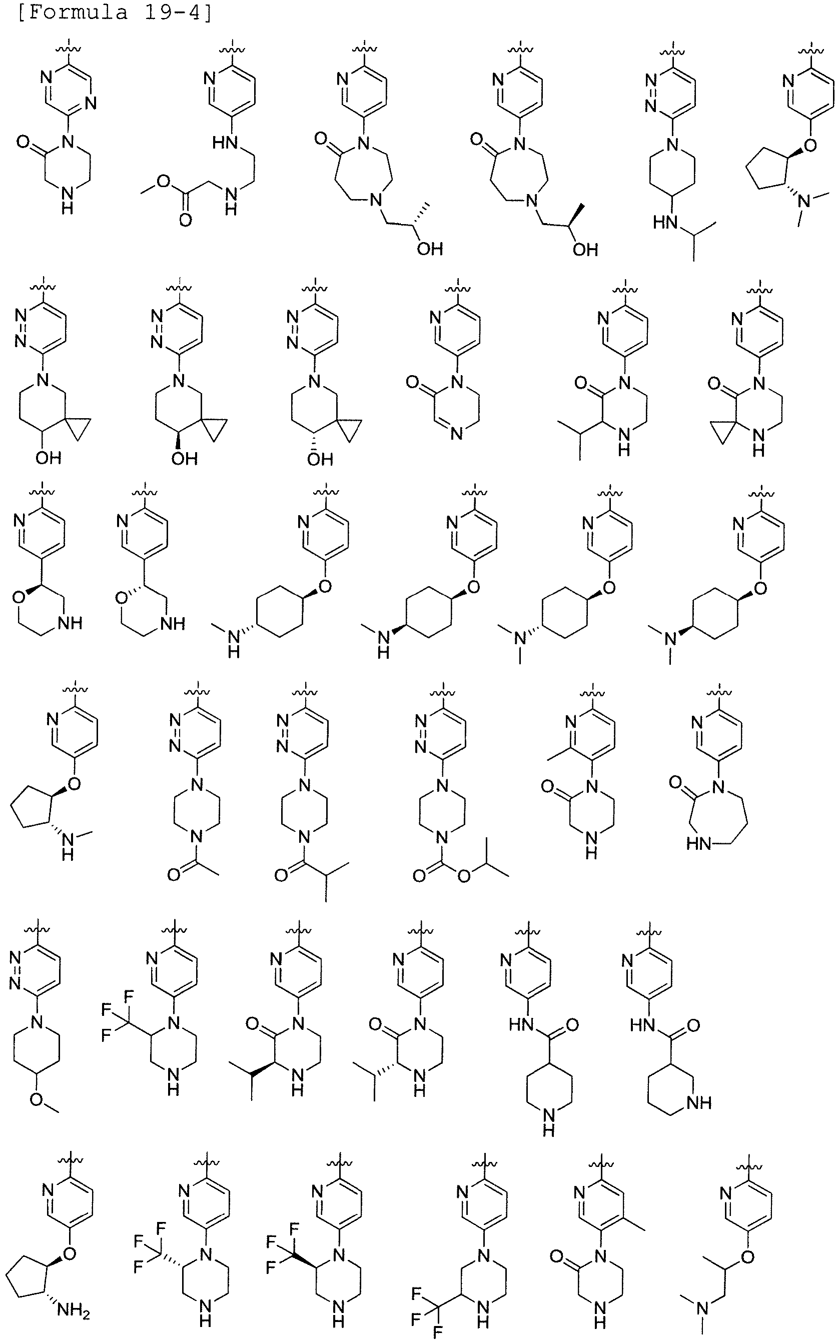

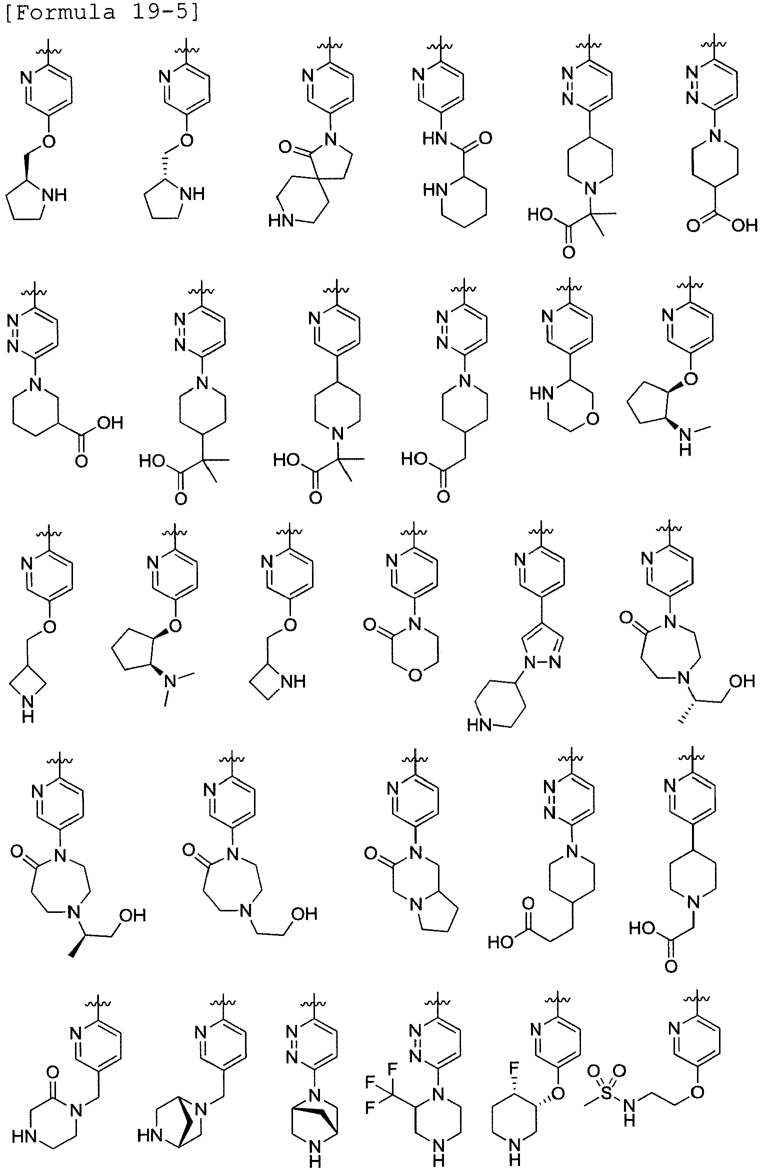

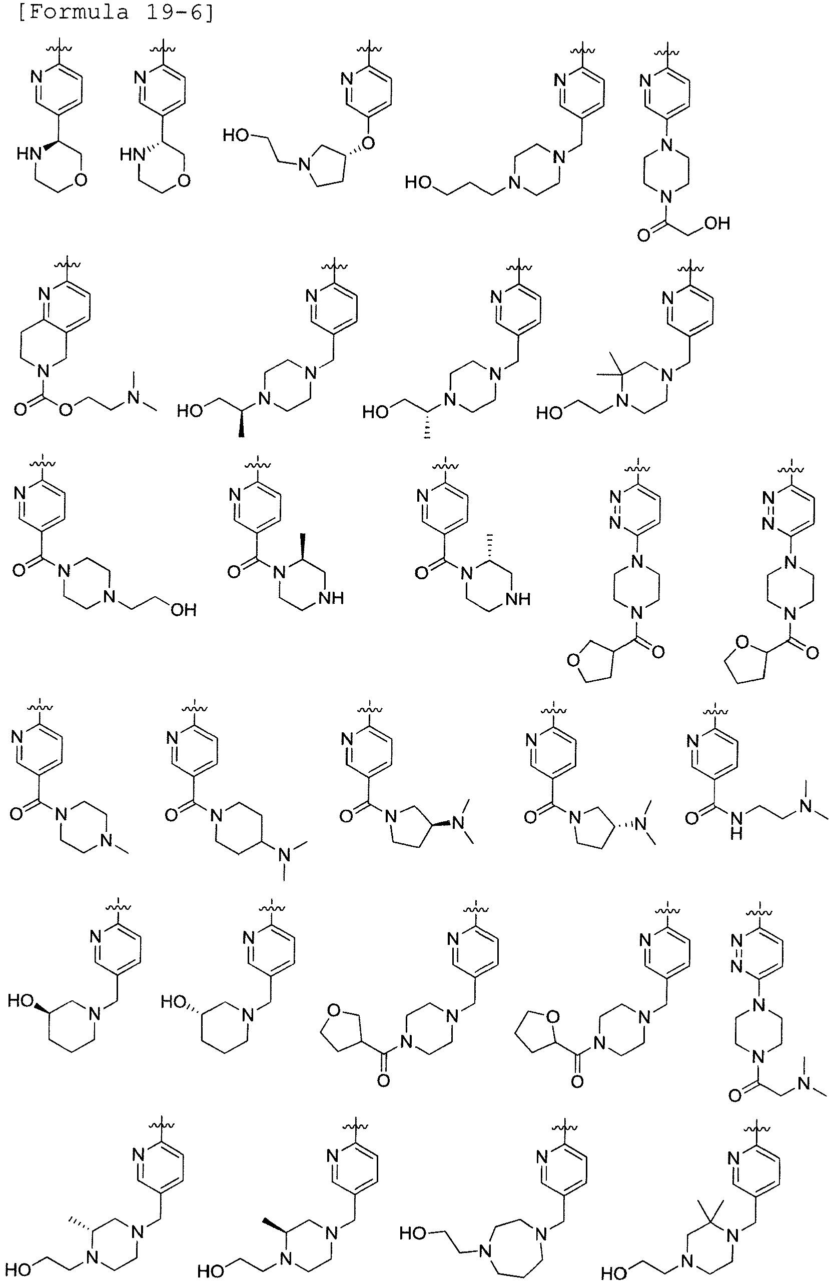

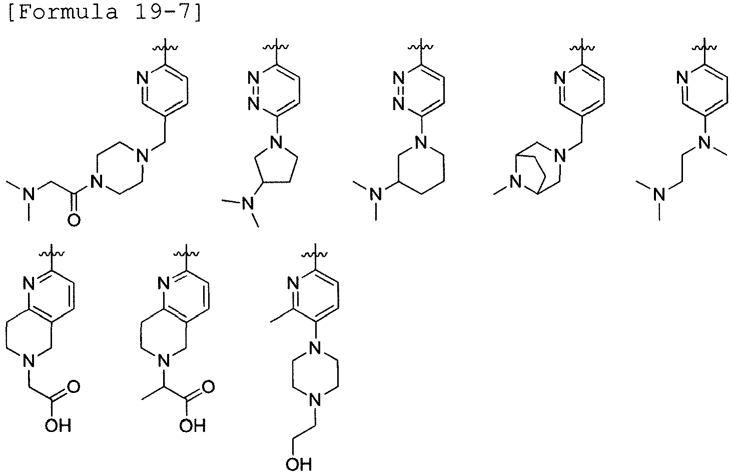

A preferred compound represented by Formula (I) is composed of a combination of a group selected from the above-defined ones and a preferred group, or a combination of preferred groups.

The compound of the present invention represented by Formula (I) may optionally be formed into a pharmaceutically acceptable salt. Examples of the salt include salts with inorganic acids, such as hydrochloric acid, hydrobromic acid, hydroiodic acid, sulfuric acid, nitric acid, phosphoric acid, carbonic acid, and the like; salts with organic acids, such as formic acid, acetic acid, propionic acid, trifluoroacetic acid, phthalic acid, oxalic acid, malonic acid, succinic acid, fumaric acid, maleic acid, lactic acid, malic acid, tartaric acid, citric acid, benzoic acid, methanesulfonic acid, ethanesulfonic acid, benzenesulfonic acid, p-toluenesulfonic acid, and the like; salts with amino acids, such as lysine, arginine, ornithine, glutamic acid, aspartic acid, and the like; salts with alkali metals, such as sodium, potassium, lithium, and the like; salts with alkaline earth metals, such as calcium magnesium, and the like; salts with metals, such as aluminum, zinc, iron, and the like; salts with organic bases, such as methylamine, ethylamine, t-octylamine, diethylamine, trimethylamine, triethylamine, ethylenediamine, piperidine, piperazine, pyridine, picoline, ethanolamine, diethanolamine, triethanolamine, cyclohexylamine, dicyclohexylamine, N-methylglucamine, tris(hydroxymethyl)aminomethane, N,N'-dibenzylethylenediamine, and the like; and ammonium salts and the like.

The present invention also encompasses compounds prepared through replacement of one or more atoms of the compound represented by Formula (I) with stable isotopes or radioisotopes.

The present invention also encompasses stereoisomers, racemates, and all acceptable optical isomers of the compound represented by Formula (I).

Tautomers of the compound of the present invention may be generated depending on the combination of substituents. The present invention also encompasses such tautomers.

Now will be described a typical process for synthesizing the compound of the present invention represented by Formula (I).

The compound of the present invention can be synthesized by the process described below. R¹, R³, R⁴, and R⁷ shown in the following reaction schemes are as defined in Formula (I). The reagents or solvents and the like shown in the reaction schemes are for illustrative purposes only as described below. Each substituent may optionally be protected with an appropriate protective group or deprotected in an appropriate step (reference: PROTECTIVE GROUPS in ORGANIC SYNTHESIS, 4TH EDITION, John Wiley & Sons, Inc.). The abbreviations of substituents, reagents, and solvents described below and in tables are as follows:
Me: methyl
Et: ethyl
Ph: phenyl
Boc: tert-butoxycarbonyl
Cbz: benzyloxycarbonyl
THF: tetrahydrofuran
DMF: N,N-dimethylformamide
NMP: N-methylpyrrolidone
TFA: trifluoroacetic acid
TBS: tert-butyldimethylsilyl
BINAP: 2,2'-bis(diphenylphosphino)-1,1'-binaphthyl
TBDPS: tert-butyldiphenylsilyl
DIPEA: N,N-Diisopropylethylamine
LAH: Lithium aluminium hydride
DMAP: 4-Dimethylaminopyridine
Ac: acetyl
Ms: mesyl
WSC: water-soluble carbodiimide (1-Ethyl-3-(3-dimethylaminopropyl)carbodiimide)
m-CPBA: m-chloroperoxybenzoic acid
DAST: diethylaminosulfur trifluoride
dba: dibenzylideneacetone
DIBAL-H: diisobutylaluminium hydride

### 1) Synthesis of compound I-e

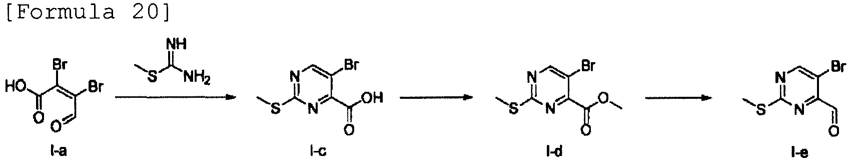

Compound I-e, which is a known compound, can be synthesized by any process known to those skilled in the art; for example, the aforementioned process.

### 2) Synthesis of compound I-f from compound I-e

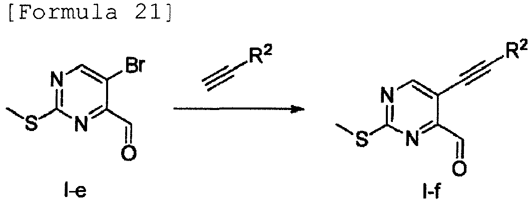

Compound I-e is reacted with a terminal alkyne derivative represented by the formula R²-C=CH in an appropriate organic solvent (e.g., THF or DMF) in the presence of an appropriate palladium catalyst (e.g., tetrakis(triphenylphosphin)palladium), appropriate copper catalyst (e.g., copper iodide (I)) and appropriate base (e.g., triethylamine) at a temperature of 0°C to the reflux temperature of the solvent, to yield compound I-f.

### 3) Synthesis of compound I-h from compound I-f

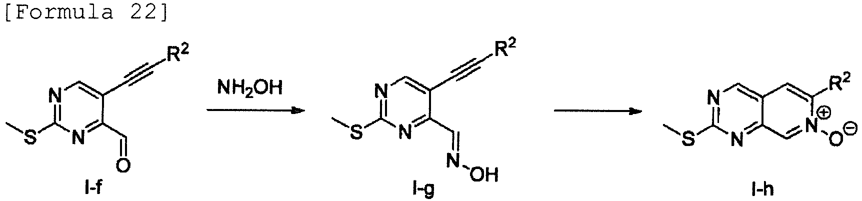

Compound I-f is reacted with hydroxylamine or a salt thereof in an appropriate organic solvent (e.g., ethanol) in the presence or absence of an appropriate base (e.g., sodium acetate) at a temperature of 0°C to the reflux temperature of the solvent. The resultant hydroxyimine compound is reacted with an appropriate acid or base (e.g., silver triflate or potassium carbonate) to yield compound I-h.

### 4) Synthesis of compound I-i from compound I-h

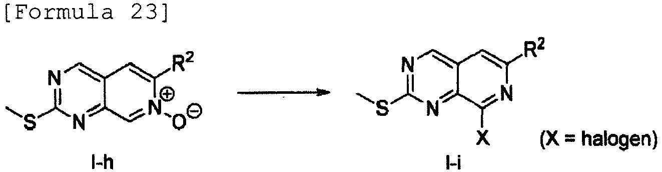

Compound I-h is reacted with an appropriate halogenating agent (e.g., thionyl chloride) in an appropriate organic solvent (e.g., dichloromethane) or under solvent-free conditions at a temperature of 0°C to 140°C, to yield compound I-i.

### 5) Synthesis of compound I-j from compound I-i

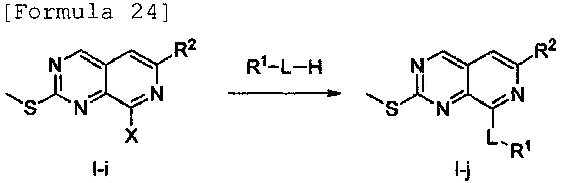

Compound I-i is reacted with an amine, alcohol, or thiol derivative represented by the formula R¹-L-H in an appropriate organic solvent (e.g., THF or 1,4-dioxane) or under solvent-free conditions in the presence or absence of an appropriate base (e.g., triethylamine, potassium carbonate, or sodium hydride) at a temperature of 0°C to the reflux temperature of the solvent, to yield compound I-j.

In this step, R² may be modified by any process known to those skilled in the art in view of the intended structure of the compound.

### 6) Synthesis of compound I-k from compound I-j

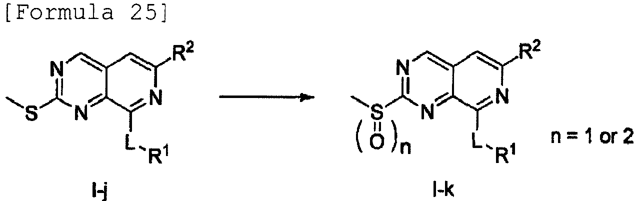

Compound I-j is reacted with an appropriate oxidant (e.g., Oxone (R) or m-chloroperbenzoic acid) in an appropriate organic solvent (e.g., dichloromethane or water) at a temperature of 0°C to the reflux temperature of the solvent, to yield compound I-k.

### 7) Synthesis of compound I-l from compound I-k

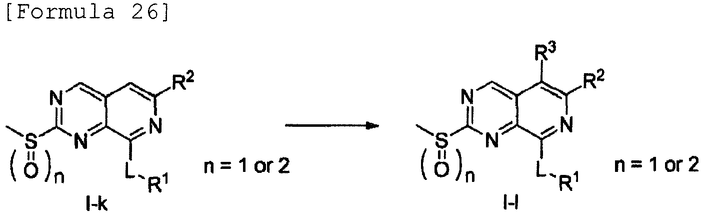

Compound I-k is reacted with an appropriate halogenating agent (e.g., N-chlorosuccinimide) in an appropriate organic solvent (e.g., dichloromethane or 1,2-dichloroethane) at a temperature of 0°C to the reflux temperature of the solvent, to yield compound I-l.

In this step, R³ may be modified by any process known to those skilled in the art in view of the intended structure of the compound.

### 8) Synthesis of compound I-m from compound I-l

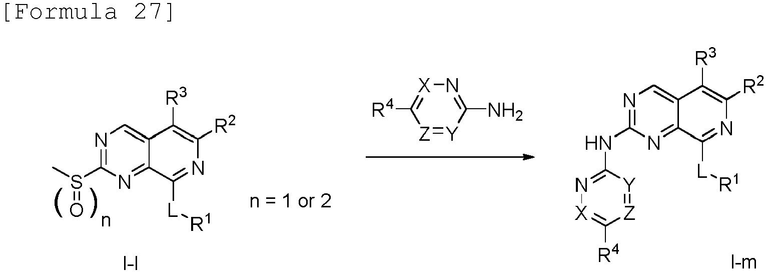

Compound I-l is reacted with an amine derivative represented by the formula R⁴-(nitrogen-containing heteroaryl with X, Y, and Z)-NH₂ of the above scheme in an appropriate organic solvent (e.g., NMP, THF, or toluene) or under solvent-free conditions in the presence or absence of an appropriate base (e.g., sodium hydride, triethylamine, or N,N-diisopropyl-N-ethylamine) at a temperature of 0°C to the reflux temperature of the solvent, to yield compound I-m.

If L, R¹, R², or R⁴ of compound I-m is protected with an appropriate protective group, deprotection can be performed by any process known to those skilled in the art. For example, deprotection can be performed through reaction of the compound with an appropriate deprotecting reagent (e.g., TFA or hydrogen chloride for a Boc protective group, lithium hydroxide for a benzoyl protective group, or hydrogen in the presence of Pd/C for a Cbz protective group) in an appropriate organic solvent (e.g., dichloromethane, methanol, or THF) or under solvent-free conditions at a temperature of 0°C to the reflux temperature of the solvent (reference: Green's Protective Groups in Organic Synthesis, 4th edition, John Wiley & Sons Inc.).

If compound I-m is protected with two or more protective groups, deprotection may be performed in an appropriate order depending on the structure of compound I-m.

In each of the reactions 9) to 13) described below, L, R¹, R², or R⁴ of compound I-m is appropriately protected depending on the corresponding reaction conditions. After completion of the reaction, deprotection can be performed by an appropriate process.

### 9) Synthesis of compound I-n from compound I-m

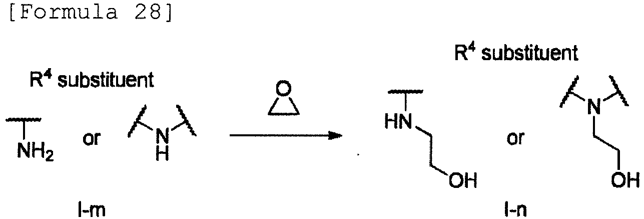

Compound I-m in which R⁴ has a primary or secondary amine structure is reacted with an optionally substituted epoxide in an appropriate organic solvent (e.g., dichloromethane, NMP, or THF) in the presence or absence of an appropriate acid (e.g., boron trifluoride-diethyl ether complex) or an appropriate base (e.g., potassium carbonate or triethylamine) at a temperature of 0°C to the reflux temperature of the solvent, to yield compound I-n.

### 10) Synthesis of compound I-o from compound I-m

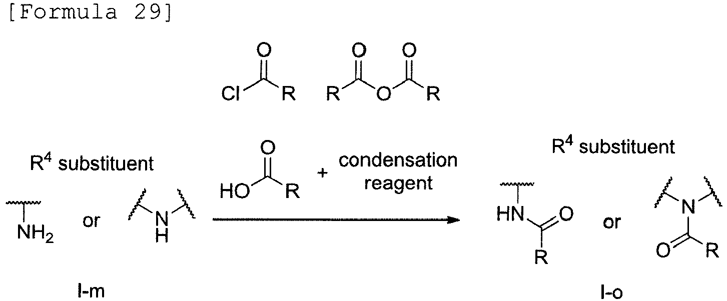

Compound I-m in which R⁴ has a primary or secondary amine structure is reacted with a carboxylic acid chloride, a carboxylic anhydride, or a carboxylic acid and a condensation reagent in an appropriate organic solvent (e.g., NMP, THF, or pyridine) in the presence or absence of an appropriate base (e.g., triethylamine or N,N-diisopropyl-N-ethylamine) at a temperature of 0°C to the reflux temperature of the solvent, to yield compound I-o.

### 11) Synthesis of compound I-p from compound I-m

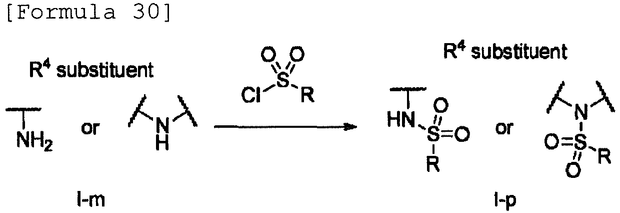

Compound I-m in which R⁴ has a primary or secondary amine structure is reacted with sulfonic acid chloride in an appropriate organic solvent (e.g., NMP, THF, or pyridine) in the presence or absence of an appropriate base (e.g., triethylamine or N,N-diisopropyl-N-ethylamine) at a temperature of 0°C to the reflux temperature of the solvent, to yield compound I-p.

### 12) Synthesis of compound I-q from compound I-m

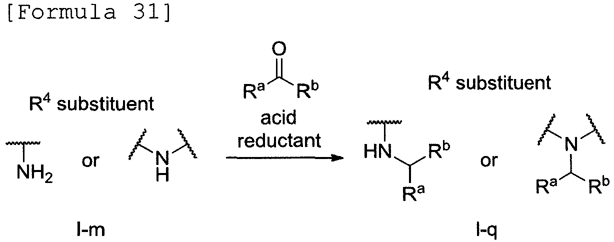

Compound I-m in which R⁴ has a primary or secondary amine structure is reacted with an optionally substituted ketone or aldehyde and an appropriate reductant (e.g., sodium triacetoxyborohydride or sodium cyanoborohydride) in an appropriate organic solvent (e.g., NMP or methanol) in the presence of an appropriate acid (e.g., acetic acid) at a temperature of room temperature to the reflux temperature of the solvent, to yield compound I-q.

### 13) Synthesis of compound I-r from compound I-m

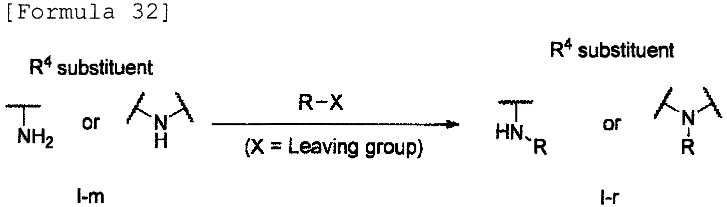

Compound I-m in which R⁴ has a primary or secondary amine structure is reacted with a compound having a leaving group (e.g., a halogen atom or a sulfonyloxy group) in an appropriate organic solvent (e.g., NMP, THF, or pyridine) in the presence or absence of an appropriate base (e.g., triethylamine or N,N-diisopropyl-N-ethylamine) at a temperature of 0°C to the reflux temperature of the solvent, to yield compound I-r.

### 14) Synthesis of compound I-s from compound I-m

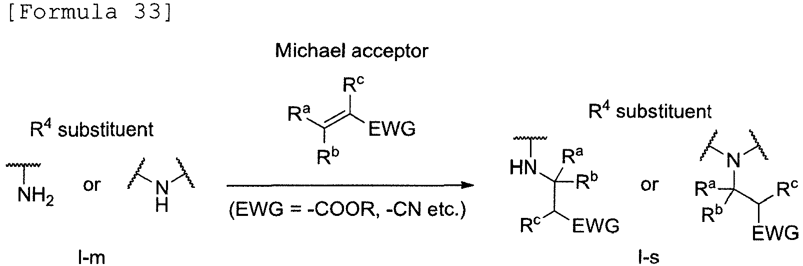

Compound I-m in which R⁴ has a primary or secondary amine structure is reacted with a compound having a structure of Michael acceptor in an appropriate organic solvent (e.g., methanol, THF) at a temperature of 0°C to the reflux temperature of the solvent to yield compound I-s.

The compound of the present invention exhibits a CDK4/6 inhibitory activity and thus is useful for the prevention or treatment of a disease associated with CDK4/6. Specifically, the compound is useful for the treatment of rheumatoid arthritis, arteriosclerosis, pulmonary fibrosis, cerebral infarction, or cancer and the protection of bone marrow. In particular, the compound is effective for the treatment of rheumatoid arthritis or cancer and the protection of bone marrow.

The compound of the present invention preferably exhibits selectivity for the CDK4/6 inhibitory activity compared to the inhibitory activity against another cyclin-dependent kinase, such as CDK2 inhibitory activity. Such selectivity of the compound is expected to reduce the expression of genotoxicity because the inhibition of CDK2 is also involved in DNA replication. Preferably, the compound of the present invention selectively inhibits CDK4 rather than CDK2.

The active ingredient of the present invention may be provided in any preparation form, such as a solid, semisolid, or liquid form, and the like. The active ingredient may be provided in any dosage form, such as an oral form or a parenteral form (e.g., an injection, a transdermal agent, an eye drop, a suppository, a nasal agent, or an inhalant, and the like).

A drug containing the active ingredient of the present invention is prepared with a common additive used for drug preparation. Examples of the additive for solid drugs include excipients, such as lactose, sucrose, glucose, cornstarch, potato starch, crystalline cellulose, light silicic anhydride, synthetic aluminum silicate, magnesium aluminometasilicate, calcium hydrogen phosphate, and the like; binders, such as crystalline cellulose, carboxymethyl cellulose, hydroxypropyl cellulose, sodium carboxymethyl cellulose, poly(vinylpyrrolidone), and the like; disintegrants, such as starch, sodium carboxymethyl cellulose, calcium carboxymethyl cellulose, croscarmellose sodium, sodium carboxymethyl starch, and the like; lubricants, such as talc stearic acid, and the like; coating agents, such as hydroxymethyl propyl cellulose, hydroxypropyl methyl cellulose phthalate, ethyl cellulose, and the like; and colorants. Examples of the additive for semisolid drugs include bases, such as white vaseline, and the like. Examples of the additive for liquid drugs include solvents, such as ethanol, and the like; solubilizers, such as ethanol, and the like; preservatives, such as paraoxybenzoic acid esters, and the like; isotonic agents, such as glucose, and the like; buffers, such as citric acid, and the like; antioxidants, such as L-ascorbic acid, and the like; chelators, such as EDTA, and the like; suspending agents and emulsifiers, such as polysorbate 80, and the like; and the like.

The dose of the active ingredient of the present invention is typically about 1 to 1,000 mg/day. The active ingredient is typically administered once to three times a day.

### Examples

The present invention will now be described in detail by way of Examples, which should not be construed as limiting the invention. The structure of an isolated novel compound was determined by ¹H-NMR and/or mass spectrometry with a single quadrupole instrumentation equipped with an electron spray source, and other appropriate analytical methods. Chemical shifts (δ: ppm) and coupling constants (J: Hz) are shown for the ¹H-NMR spectra (400 MHz, DMSO-d₆, CD₃OD or CDCl₃) . Abbreviations are as follows: s (singlet), d (doublet), t (triplet), q (quartet), brs (broad singlet), and m (multiplet). For the results of mass spectrometry, measurements are represented by (M+H)⁺; i.e., a value corresponding to a proton (H⁺) attached to the molecular mass (M) of a compound.

### Reference Example 1

### Synthesis of 5-bromo-2-(methylthio)pyrimidine-4-carboxylic acid

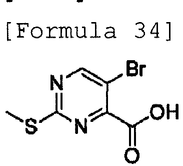

Mucobromic acid (300 g, 1.16 mol) was added to an aqueous solution (2.5 L) of 2-methyl-2-pseudothiourea sulfate (324 g, 1.16 mol) at room temperature. The resultant suspension was cooled to 0°C with stir, and triethylamine (486 mL, 3.49 mol) was added dropwise thereto over four hours. The resultant reaction mixture was stirred overnight, and the completion of the reaction was confirmed by silica gel TLC. The reaction mixture was then acidified with concentrated hydrochloric acid (about 250 mL). The resultant yellow solid was collected by filtration and washed twice with water (500 mL) and then twice with diethyl ether (500 mL). The solid was dried under reduced pressure to yield the title compound (160 g, 55%).

### Reference Example 2

### Synthesis of methyl 5-bromo-2-methylthiopyrimidine-4-carboxylate

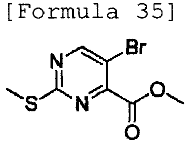

A solution of 5-bromo-2-(methylthio)pyrimidine-4-carboxylic acid (110 g, 0.44 mol) in methanol (1.1 L) was cooled to 0°C with stir, and thionyl chloride (50 mL, 0.66 mol) was added dropwise thereto. The resultant reaction mixture was slowly heated, and the reaction was allowed to proceed under reflux for four hours. The completion of the reaction was confirmed by LC/MS and TLC, and the reaction mixture was cooled to room temperature. The volatiles were removed through evaporation under reduced pressure, and the residue was dissolved in ethyl acetate (1 L). The resultant solution was washed three times with 10% aqueous sodium carbonate solution (200 mL) and then twice with saturated brine (200 mL). The resultant organic phase was dried over anhydrous magnesium sulfate, and solid was separated by filtration. The filtrate was then concentrated under reduced pressure, and the resultant crude product was purified by silica gel column chromatography to yield the title compound (88 g, 75%).

### Reference Example 3

### Synthesis of mixture of 5-bromo-2-methylthiopyrimidine-4-carbaldehyde and (5-bromo-2-methylthiopyrimidin-4-yl)methoxymethanol

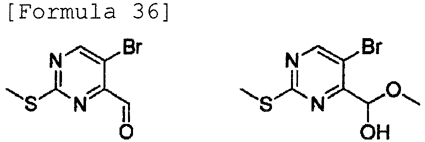

A solution (375 mL) of methyl 5-bromo-2-methylsulfanylpyrimidine-4-carboxylate (25 g, 95 mmol) in THF was cooled to -78°C and stirred under a nitrogen atmosphere. DIBAL-H (84 mL, 143 mmol, 1.7M toluene solution) was added dropwise to the THF solution, and the mixture was stirred at -78°C for four hours. The completion of the reaction was confirmed by TLC, and the reaction was quenched through dropwise addition of methanol at -78°C. The resultant reaction mixture was allowed to warm slowly to 0°C and diluted with ethyl acetate, and the mixture was filtrated through celite. The filtrate was washed twice with saturated brine (200 mL), and the resultant organic phase was dried over anhydrous magnesium sulfate. The resultant solid was separated by filtration, and the filtrate was concentrated to yield the title compound mixture (25 g, crude product). The crude product was used for the subsequent reaction without further purification.

### Reference Example 4

### Synthesis of tert-butyl 4-(6-nitropyridin-3-yl)piperazine-1-carboxylate

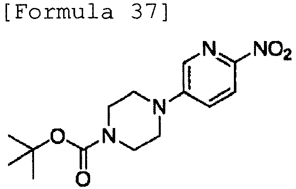

A mixture of 5-Bromo-2-nitropyridine (203 g, 1.37 mol), piperazine (153 g, 1.77 mol), tetrabutylammonium iodide (25.2 g, 0.068 mol), and potassium carbonate (207 g, 1.50 mol) in dimethyl sulfoxide (2.6 L) was stirred at 80°C overnight. The resultant reaction mixture was cooled to room temperature, and the mixture was poured into water (7 L). The resultant solid was collected by filtration, and the solid was washed with dichloromethane (1 L × 2) and dried. The filtrate was extracted with chloroform (2 L × 7). The resultant organic phase was washed with water (2 L) and then with saturated brine (2 L), and the organic phase was concentrated under reduced pressure to yield solid. The resultant solid products were combined together and used for the subsequent reaction without further purification.

The solid product (490 g) was dissolved in THF (2 L) and water (500 mL), and sodium hydrogen carbonate (119 g, 1.42 mol) was added to the solution. To the resultant suspension was added di-tert-butyl dicarboxylate (262 g, 1.2 mol), and the mixture was stirred at room temperature for three hours. The reaction mixture was concentrated under reduced pressure, and the residue was diluted with water (1 L) and extracted with dichloromethane (1 L × 3). The resultant organic phases were combined together and then washed with water (1 L). The aqueous phase was extracted with dichloromethane (300 mL). The resultant organic phases were combined together and dried over anhydrous magnesium sulfate. The solid was separated by filtration, and the filtrate was concentrated under reduced pressure. The resultant solid was suspended in ethyl acetate (2 L) and heated to 60°C, and the solid was separated by filtration at 60°C. The solid was dried under reduced pressure to yield the title compound (191 g, 62%)
APCI-MS (M+H)⁺ 309.1, C₁₄H₂₀N₄O₄=308.15
¹H-NMR δ(400 MHz, CDCl₃): 8.16 (d, J=9 Hz, 1H), 8.11 (d, J=3 Hz, 1H), 7.19 (dd, J=9.3 Hz, 1H), 3.64-3.61 (m, 4H), 3.45-3.42 (m, 4H), 1.47 (s, 9H) .

### Reference Example 5

### Synthesis of tert-butyl 4-(6-aminopyridin-3-yl)piperazine-1-carboxylate

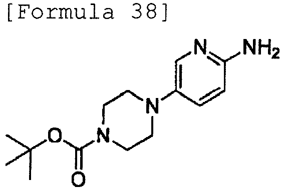

The tert-butyl 4-(6-nitropyridin-3-yl)piperazine-1-carboxylate synthesized in Reference Example 4 (83 g, 269 mmol) was dissolved in methanol (1.3 L) in Parr Shaker and Raney nickel (15 g, 50% aqueous suspension) was added thereto. The resultant reaction mixture was stirred under a hydrogen atmosphere (50 psi) for five hours. The reaction mixture was filtered through a Celite pad to separate solid,and the filtrate was concentrated under reduced pressure. The resultant solid was suspended in diethyl ether (120 mL) and stirred for four hours. Heptane was added to the suspension and cooled at 0°C for 45 minutes. The resultant solid was separated by filtration and dried under reduced pressure to yield the title compound (62.5 g, 83%).
ESI-MS (M+H)⁺ 279, C₁₄H₂₂N₄O₂=278.17

Intermediates A-1 to A-44 were each synthesized by the process of Reference Example 4 and/or 5 with the corresponding halopyridine derivatives and amine derivatives. Appropriate protection or deprotection was performed as needed.

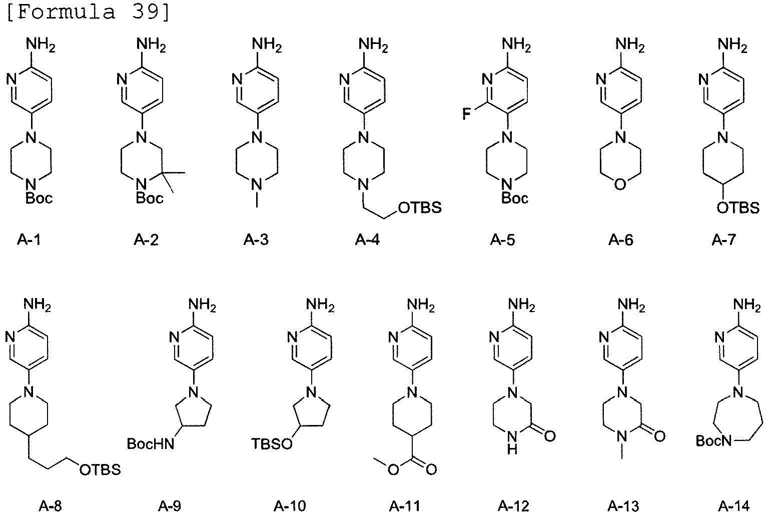

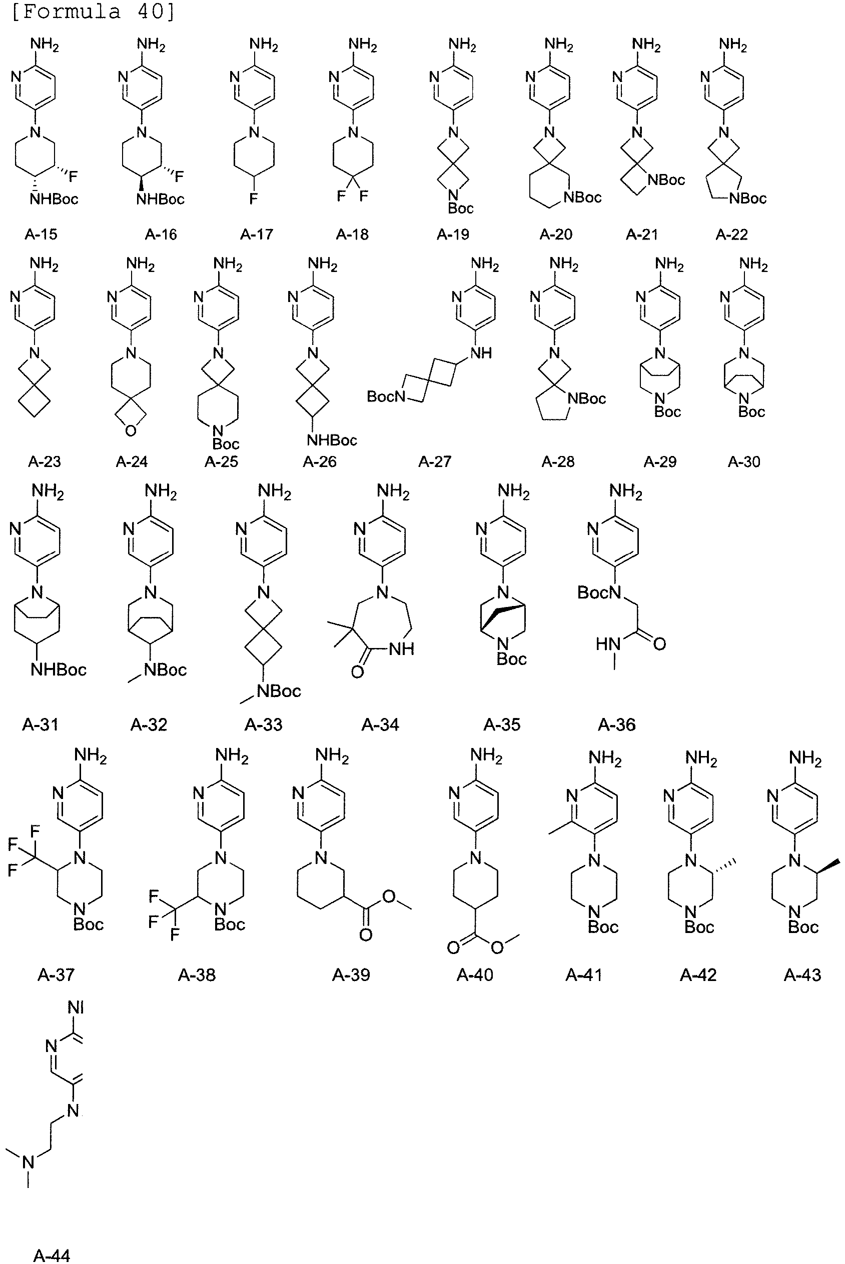

### Reference Example 6

### Synthesis of 6-aminopyridine-3-carbaldehyde

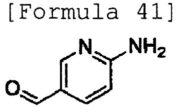

6-Aminopyridine-3-carbonitrile (1.9 g, 16 mmol) was dissolved in THF (160 mL) and cooled to -78°C with stir. Diisobutylaluminium hydride (106.5 mL, 1.5M toluene solution) was slowly added dropwise to the solution at - 78°C and the mixture was allowed to warm to 20°C with stir, followed by further stirring for two hours. The reaction was quenched by addition of ice water (100 mL) to the resultant reaction mixture, and the mixture was extracted three times with dichloromethane (50 mL). The resultant organic phases were combined together and then washed once with brine (100 mL) and dried over anhydrous sodium sulfate. The solid was separated by filtration, and the filtrate was concentrated under reduced pressure. The residue was roughly purified by silica gel column chromatography to yield a crude product of the title compound (1.7 g). The crude product was used for the subsequent reaction without further purification.

### Reference Example 7

### Synthesis of tert-butyl 4-[(6-aminopyridin-3-yl)methyl]piperazine-1-carboxylate

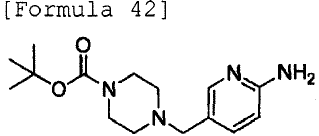

The crude 6-aminopyridine-3-carbaldehyde synthesized in Reference Example 6 (1.7 g, 13.9 mmol) and tert-butyl piperazine-1-carboxylate (3.2 g, 17.2 mmol) were dissolved in dichloromethane (50 mL) and stirred at room temperature for eight hours. To the resultant mixture was added sodium triacetoxyborohydride (8.84 g, 40.9 mmol) and stirred at room temperature for two hours. The reaction was monitored by LC/MS. After completion of the reaction, the reaction was quenched through addition of saturated aqueous sodium carbonate solution (50 mL), and the reaction mixture was extracted three times with ethyl acetate (50 mL). The resultant organic phases were combined together, and the mixture was washed once with brine (100 mL) and dried over anhydrous sodium sulfate. The resultant solid was separated by filtration, and then the filtrate was concentrated under reduced pressure. The residue was roughly purified by silica gel column chromatography to yield the title compound (3.3 g, 81%).

### Reference Example 8

### Synthesis of di-tert-butyl (5-methylpyridin-2-yl)imidodicarbonate

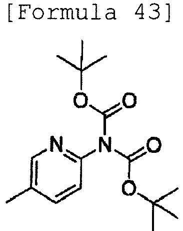

In reference to the process disclosed in WO2010/141406, 5-methylpyridine-2-amine (20 g, 185 mmol) and di-tert-butyl dicarbonate (101 g, 462 mmol) were dissolved in THF (160 mL) and 4-N,N-dimethylaminopyridine (3.6 g, 29.7 mmol) was added to the solution. The resultant reaction mixture was stirred at room temperature for three days. The reaction mixture was concentrated under reduced pressure, and the residue was dissolved in ethyl acetate and washed with water. The resultant organic phase was washed with saturated brine and dried over anhydrous sodium sulfate. The solid was separated by filtration, and the filtrate was concentrated. The resultant solid was dissolved in ethyl acetate (50 mL) and heptane (50 mL) was added thereto. The solid was collected by filtration and dried under reduced pressure, to yield the title compound (25.1 g, 44%). The filtrate was concentrated, and the residue was purified by silica gel column chromatography to yield the title compound (17.9 g, 31%).

### Reference Example 9

### Synthesis of di-tert-butyl [5-(bromomethyl)pyridin-2-yl]imidodicarbonate

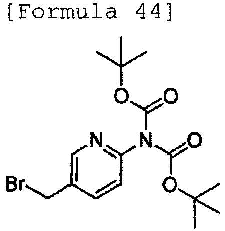

The di-tert-butyl (5-methylpyridin-2-yl)imidodicarbonate synthesized in Reference Example 8 (17.2 g, 55.8 mmol), N-bromosuccinimide (12.17 g, 68.4 mmol), and benzoyl peroxide (1.5 g, 8.1 mmol) were dissolved in carbon tetrachloride (100 mL) and the reaction was stirred at 80°C for six hours. The reaction mixture was cooled to room temperature, and the resultant solid was separated by filtration. The filtrate was then concentrated under reduced pressure, and the residue was purified by silica gel column chromatography to yield a mixture of the title compound, di-tert-butyl [5-(dibromomethyl)pyridin-2-yl]imidodicarbonate, and di-tert-butyl (5-methylpyridin-2-yl)imidodicarbonate (14.5 g, 60.3 : 4.4 : 35.3, determined by the ¹H-NMR spectrum). The mixture was used for the subsequent reaction without further purification.

### Reference Example 10

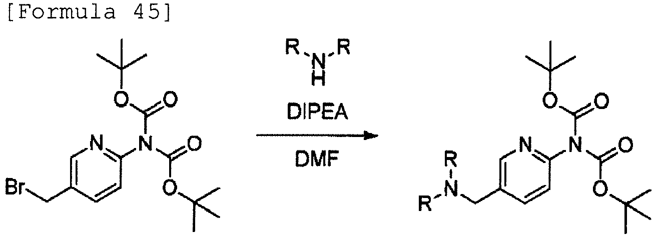

Di-tert-butyl [5-(bromomethyl)pyridin-2-yl]imidodicarbonate (1 equivalent) was dissolved in DMF and an appropriate amine derivative (1.5 equivalents) and N,N-diisopropyl-N-ethylamine (3 equivalents) was added to the solution at room temperature. The reaction mixture was stirred at room temperature for several hours, and the mixture was then diluted with ethyl acetate and washed with saturated brine. The resultant organic phase was dried over anhydrous sodium sulfate, and the solid was separated by filtration. The filtrate was then concentrated under reduced pressure, and the residue was purified by silica gel column chromatography to yield a target amine derivative.

### Reference Example 11

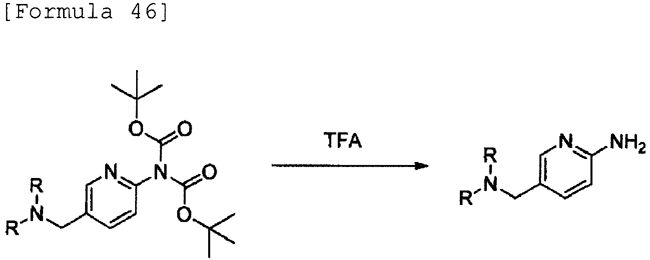

To the compound synthesized in Reference Example 10 was added an excess amount of trifluoroacetic acid and stirred at room temperature for several hours. The reaction mixture was concentrated under reduced pressure, and the resultant TFA salt of the target product was dissolved in methanol and applied onto a strong cation exchange resin (SCX). The SCX column was washed with methanol and the target product was eluted with ammonia (2 mol/L, methanol solution). The eluate was concentrated under reduced pressure to yield a target 2-aminopyridine derivative. The resultant product was used for the subsequent reaction without further purification.

In the case of the presence of a primary or secondary amino group in the compound besides the aminopyridine structure, the crude product was dissolved in THF and reacted with di-tert-butyl dicarbonate at room temperature. After completion of the reaction, the solvent was removed through evaporation, and the residue was roughly purified by silica gel column chromatography to yield a 2-aminopyridine derivative having a primary or secondary amino group protected with a Boc group.

Intermediates B-1 to B-68 were each synthesized by any of the processes of Reference Example 6 and/or 7 or Reference Examples 8 to 11 or a combination of the processes with the corresponding aldehyde or alkyl halide derivatives and amine derivatives. Appropriate protection or deprotection was performed as needed.

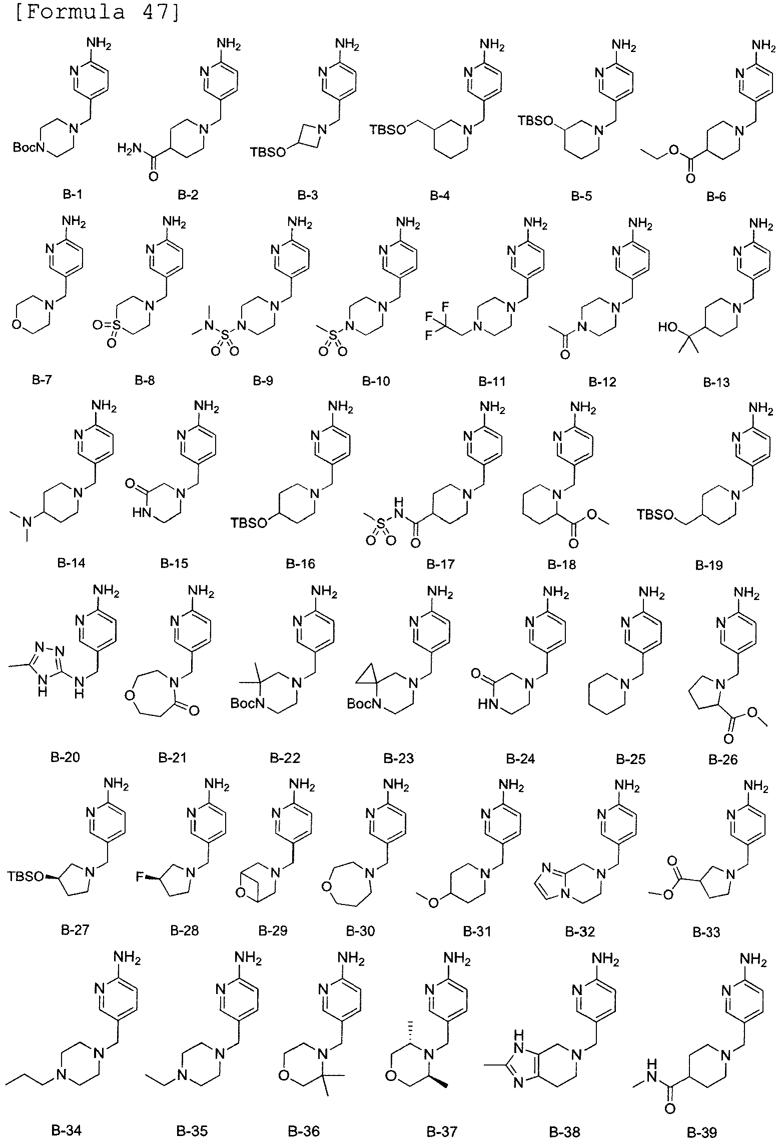

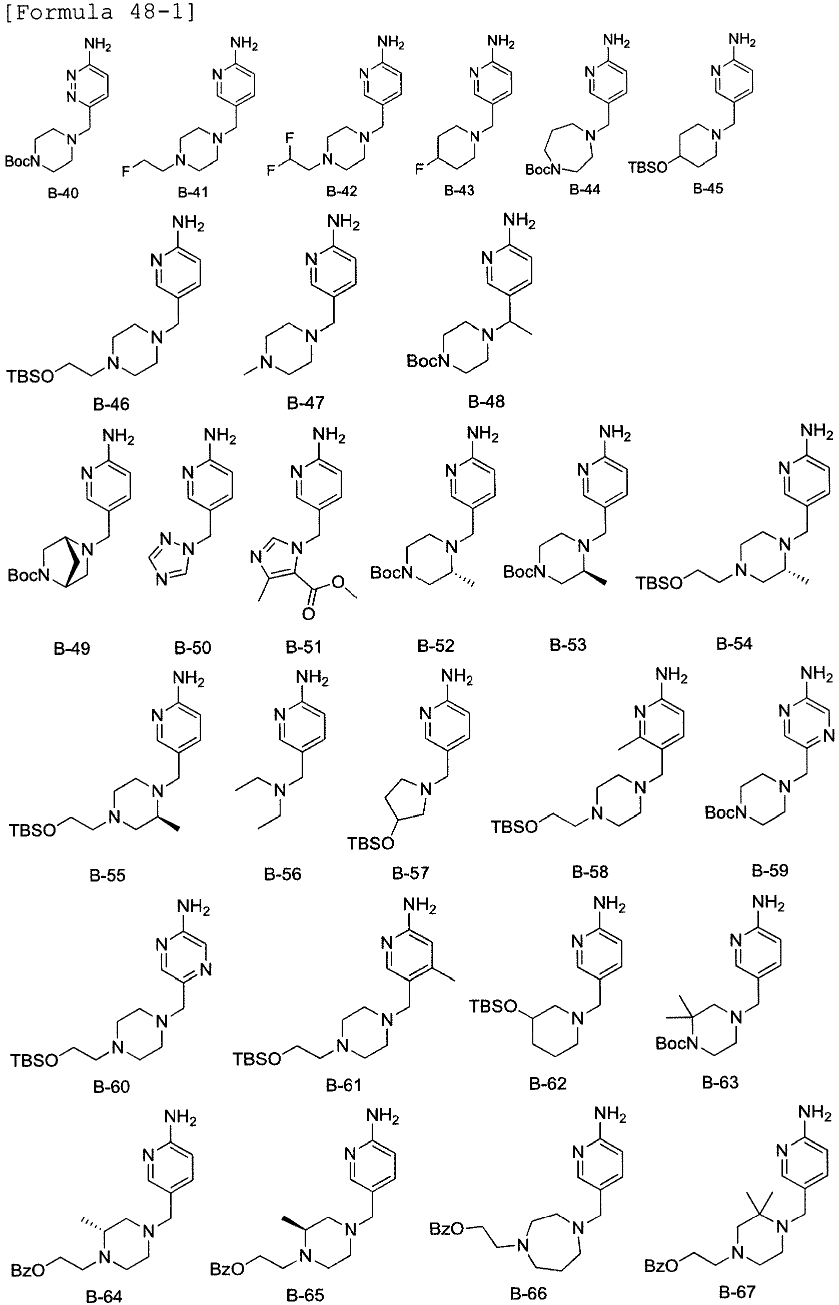

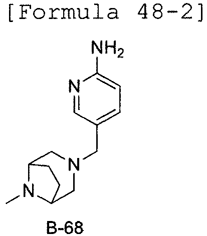

### Reference Example 12

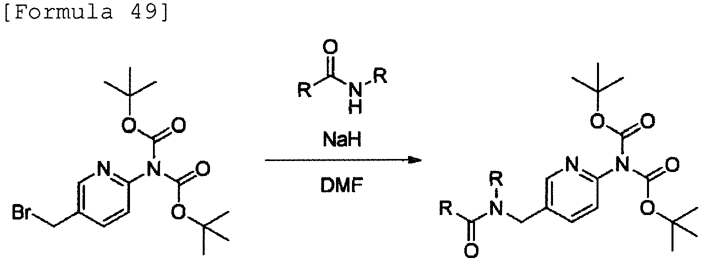

An appropriate amide derivative (1 equivalent) was dissolved in DMF, and sodium hydride (1 equivalent) was gradually added thereto at 0°C and the mixture was stirred at room temperature for several minutes. The resultant reaction mixture was cooled to 0°C and di-tert-butyl [5-(bromomethyl)pyridin-2-yl]imidodicarbonate (1.5 equivalents) was gradually added to the mixture. The reaction mixture was stirred at room temperature for several hours and then water was added to the mixture to stop the reaction. The mixture was extracted with ethyl acetate and washed with saturated brine. The resultant organic phase was dried over anhydrous sodium sulfate, and the solid was separated by filtration. The filtrate was concentrated under reduced pressure, and the residue was purified by silica gel column chromatography to yield a target amide derivative.

The following intermediates C-1 to C-5 were synthesized by the process of Reference Example 12 and 11 with the corresponding alkyl halide derivatives, amide derivatives, or urea derivatives. Appropriate protection or deprotection was performed as needed.

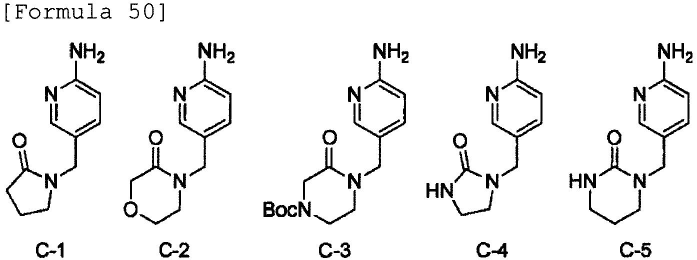

### Reference Example 13

### Synthesis of tert-butyl 4-(6-nitropyridin-3-yl)-3-oxopiperazine-1-carboxylate

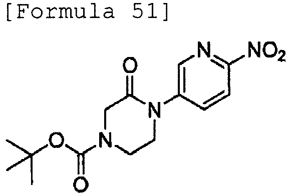

In reference to the process disclosed in WO2012/031004, 2-nitro-5-bromopyridine (1.01 g, 5.0 mmol), tert-butyl 2-oxo-4-piperazinecarboxylate (1.00 g, 5.0 mmol, and cesium carbonate (3.26 g, 10.0 mmol) were suspended in 1,4-dioxane, and the suspension was bubbled with nitrogen gas for 30 minutes. To the suspension was added Xantphos (246 mg, 0.43 mmol) and tris(dibenzylideneacetone)dipalladium (229 mg, 0.25 mmol), and the mixture was stirred under reflux for two hours. The resultant reaction mixture was cooled to room temperature, and water and ethyl acetate were then added to the mixture, followed by filtration with Celite. The organic phase was separated from the filtrate, and the aqueous phase was extracted with ethyl acetate. The resultant organic phases were combined together and dried over anhydrous sodium sulfate, and the resultant solid was separated by filtration. The filtrate was concentrated under reduced pressure, and the residue was purified by silica gel column chromatography to yield the title compound (1.08 g, 67%).
¹H-NMR (CDCl₃)δ: 8.67 (1H, d, J=2.4 Hz), 8.32 (1H, d, J=8.8 Hz), 8.15 (1H, dd, J=8.8, 2.4 Hz), 4.33 (2H, s), 3.93-3.83 (4H, m), 1.51 (9H, s).

### Reference Example 14

### Synthesis of tert-butyl 4-(6-aminopyridin-3-yl)-3-oxopiperazine-1-carboxylate

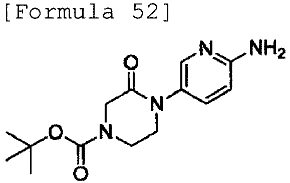

The compound synthesized in Reference Example 13 (1.08 g, 3.34 mmol) was dissolved in ethanol (45 mL) and THF (22 mL). Palladium-carbon (108 mg) was added to the solution, and the mixture was stirred under a hydrogen atmosphere for 24 hours. The resultant reaction mixture was filtered through Celite, and the filtrate was concentrated under reduced pressure. The residue was purified by silica gel column chromatography to yield the title compound (0.928 g, 95%).
¹H-NMR (CDCl₃)δ: 7.99 (1H, d, J=2.4 Hz), 7.38 (1H, dd, J=8.8, 2.4 Hz), 6.53 (1H, d, J=8.8 Hz), 4.50 (2H, brs), 4.24 (2H, s), 3.78 (2H, t, J=5.1 Hz), 3.67 (2H, t, J=5.4 Hz), 1.50 (9H, s).

Intermediates D-1 to D-41 were each synthesized by the process of Reference Example 13 and/or 14 with the corresponding halopyridine derivatives and amide derivatives. Appropriate protection or deprotection was performed as needed.

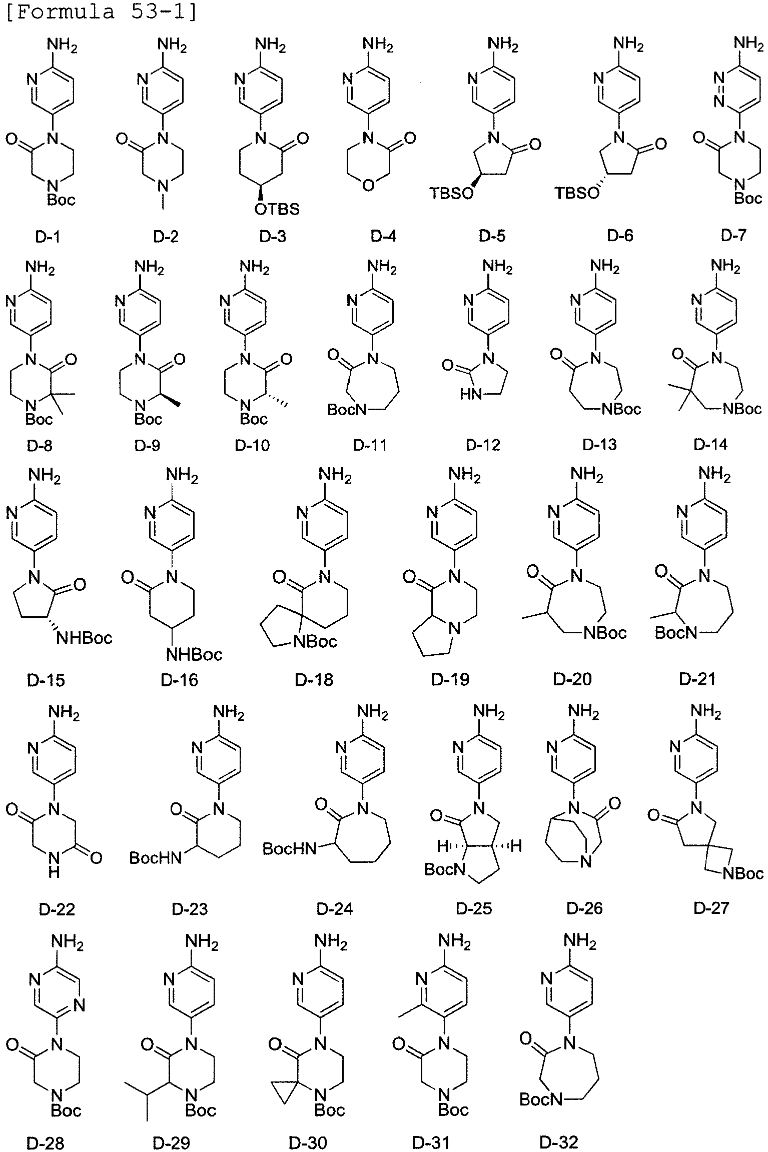

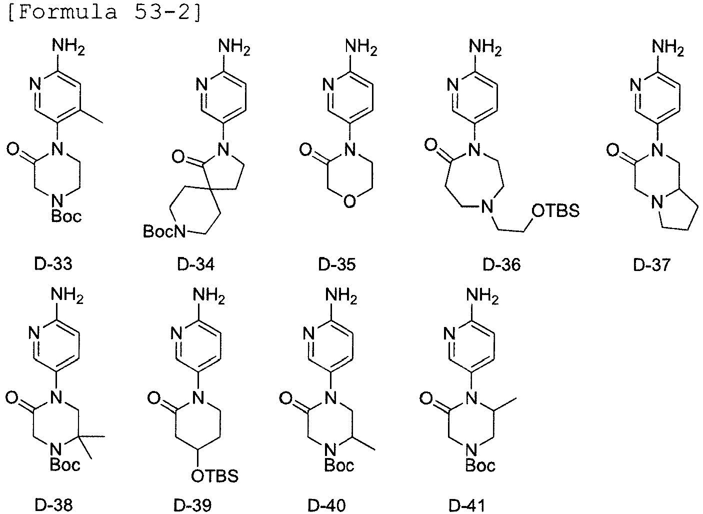

### Reference Example 15

### Synthesis of dimethyl-[2-(6-nitropyridin-3-yloxy)ethyl]amine

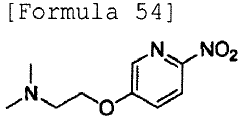

2-Dimethylaminoethanol (0.32 mL, 3.17 mmol) was dissolved in DMF (4 mL) and cesium carbonate (1.03 g, 3.17 mmol) was added thereto, and the resultant suspension was stirred at room temperature for 10 minutes. 5-Fluoro-2-nitropyridine (0.30 g, 2.11 mmol) was added to the suspension at room temperature, and the mixture then was stirred at 80°C for 16 hours. The reaction was monitored by LC/MS. After completion of the reaction, the reaction was quenched through addition of ice water, and the reaction mixture was extracted with ethyl acetate. The resultant organic phase was dried over anhydrous sodium sulfate, and the solid was separated by filtration. The filtrate was then concentrated under reduced pressure, and the residue was purified by silica gel column chromatography to yield the title compound (0.40 g, 90%).

### Reference Example 16

### Synthesis of 5-(2-dimethylaminoethoxy)pyridin-2-ylamine

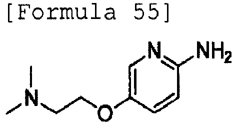

Dimethyl-[2-(6-nitropyridin-3-yloxy)ethyl]amine synthesized in Reference Example 15 (0.40 g, 1.90 mmol) was dissolved in THF (5 mL) and ethanol (5 mL), and palladium-carbon (80 mg) was added to the solution. The mixture was stirred under a hydrogen atmosphere overnight. The resultant reaction mixture was filtered through Celite, and the filtrate was concentrated under reduced pressure. The resultant crude product was washed with a solvent mixture of ethyl acetate and hexane (1:9) to yield the title compound (0.28 g, 82%).

Intermediates E-1 to E-61 were each synthesized by the process of Reference Examples 15 and/or 16 with the corresponding halopyridine derivatives, alcohol derivatives, or thiol derivatives. Appropriate protection or deprotection was performed as needed.

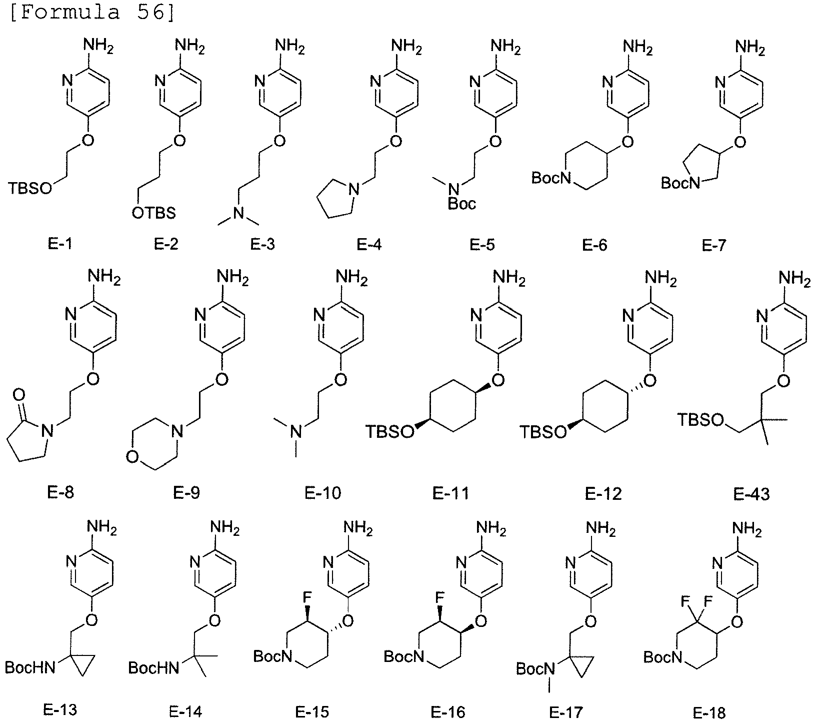

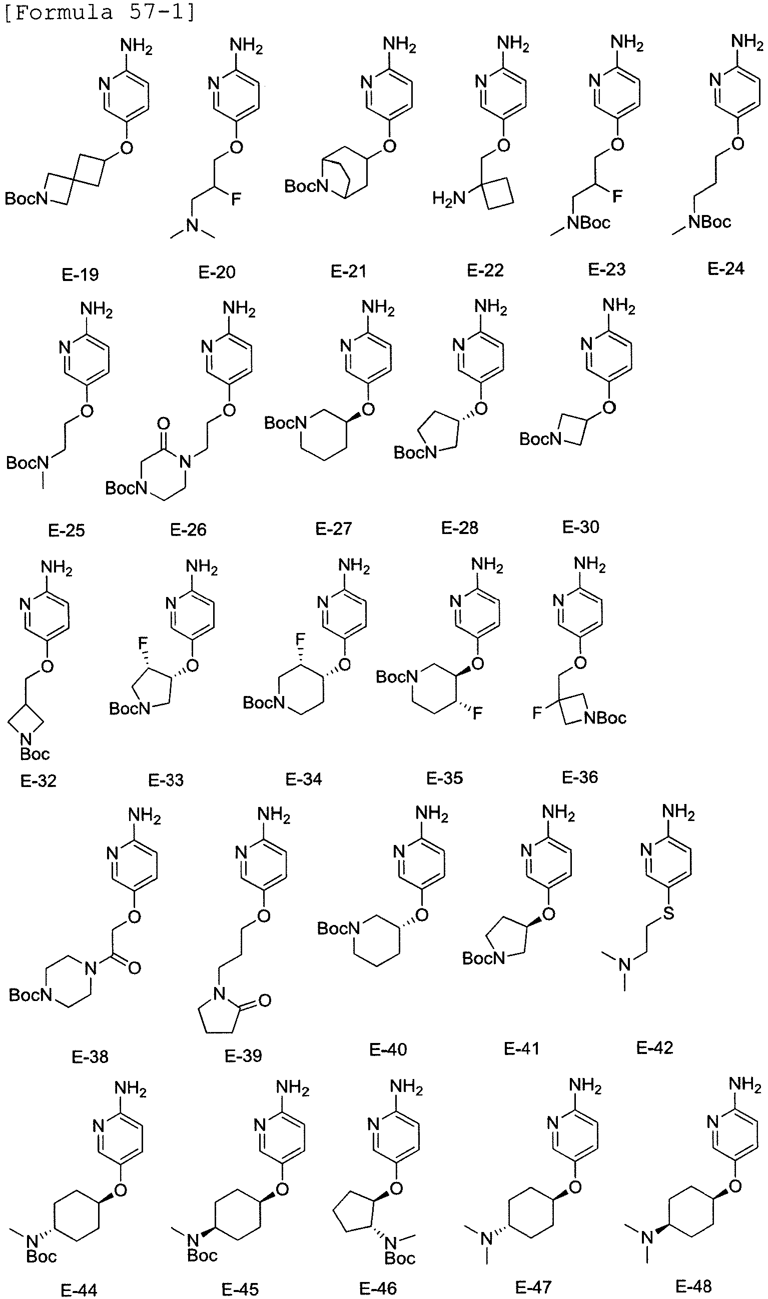

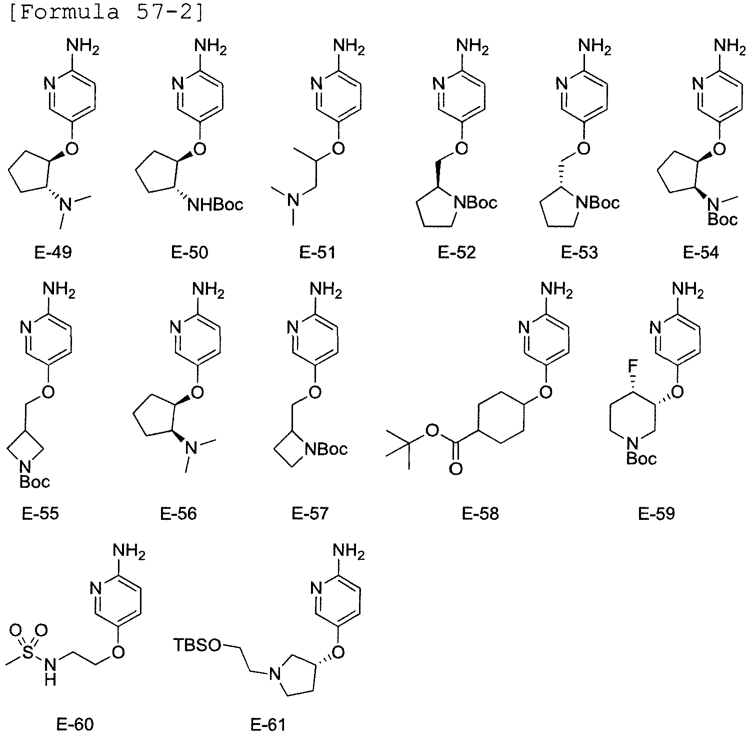

### Reference Example 17

### Synthesis of tert-butyl 4-(6-chloropyridazin-3-yl)piperazine-1-carboxylate

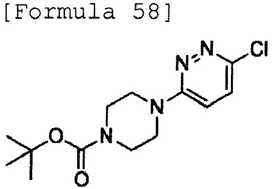

A solution of 3,6-dichloropyridazine (5.01 g, 33.6 mmol) and tert-butyl piperazine-1-carboxylate (6.88g, 37.0 mmol) in DMF (50 mL) was added triethylamine (11.7 mL, 50.4 mmol) and stirred at 80°C overnight. The resultant reaction mixture was cooled to room temperature, and water was added to the mixture. The mixture was extracted three times with a solvent mixture of dichloromethane and methanol (95:5) (50 mL). The resultant organic phases were combined together and dried over anhydrous magnesium sulfate. The resultant solid was separated by filtration, and the filtrate was then concentrated under reduced pressure. The resultant crude product was washed with diethyl ether to yield the title compound (7.0 g, 70%).

### Reference Example 18

### Synthesis of tert-butyl 4-(6-((diphenylmethylene)amino)pyridazin-3-yl)piperazine-1-carboxylate

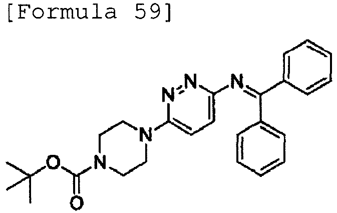

Tert-butyl 4-(6-chloropyridazin-3-yl)piperazine-1-carboxylate synthesized in Reference Example 17 (59.8 mg, 0.20 mmol, benzophenone imine (43.5 mg, 0.24 mmol), tris(dibenzylideneacetone)dipalladium (9.2 mg, 0.010 mmol), BINAP (12.5 mg, 0.020 mmol), and cesium carbonate (130.3 mg, 0.40 mmol) were suspended in toluene (1.0 mL), and the suspension was stirred at 100°C overnight. The resultant reaction mixture was cooled to room temperature and then filtered through Celite, and the Celite was washed with ethyl acetate. The filtrate was washed with saturated brine and dried over anhydrous magnesium sulfate, and the solid was separated by filtration. The filtrate was concentrated under reduced pressure, and the residue was purified by silica gel column chromatography to yield the title compound (67 mg, 76%).

### Reference Example 19

### Synthesis of tert-butyl 4-(6-aminopyridazin-3-yl)piperazine-1-carboxylate

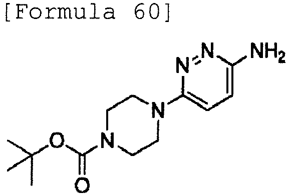

Tert-butyl 4-(6-((diphenylmethylene)amino)pyridazin-3-yl)piperazine-1-carboxylate synthesized in Reference Example 18 (67 mg, 0.151 mmol) was dissolved in THF (0.76 mL). To the solution was added an aqueous citric acid solution (0.378 mL, 0.755 mmol, 2 mol/L) and the mixture was stirred at room temperature overnight. The resultant reaction mixture was neutralized with saturated aqueous sodium hydrogen carbonate solution (5 mL), and the mixture was extracted twice with ethyl acetate (5 mL). The resultant organic phases were combined together and dried over anhydrous magnesium sulfate, and the resultant solid was separated by filtration. The filtrate was concentrated under reduced pressure, and the resultant crude product was washed with tert-butyl methyl ether (5 mL) to yield the title compound (30 mg, 71%).

Intermediates F-1 to F-77 were each synthesized by any of the processes of Reference Examples 17 to 19 or a combination of the processes with the corresponding haloheteroaryl derivatives and amine derivatives. Appropriate protection or deprotection was performed as needed.

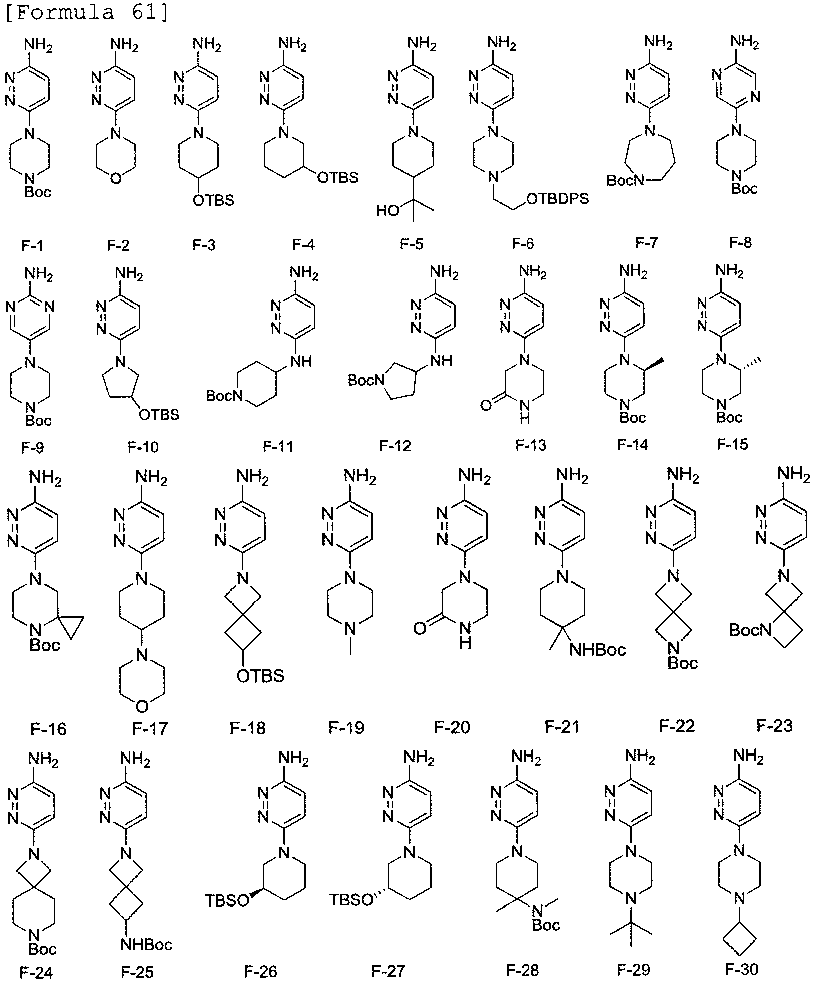

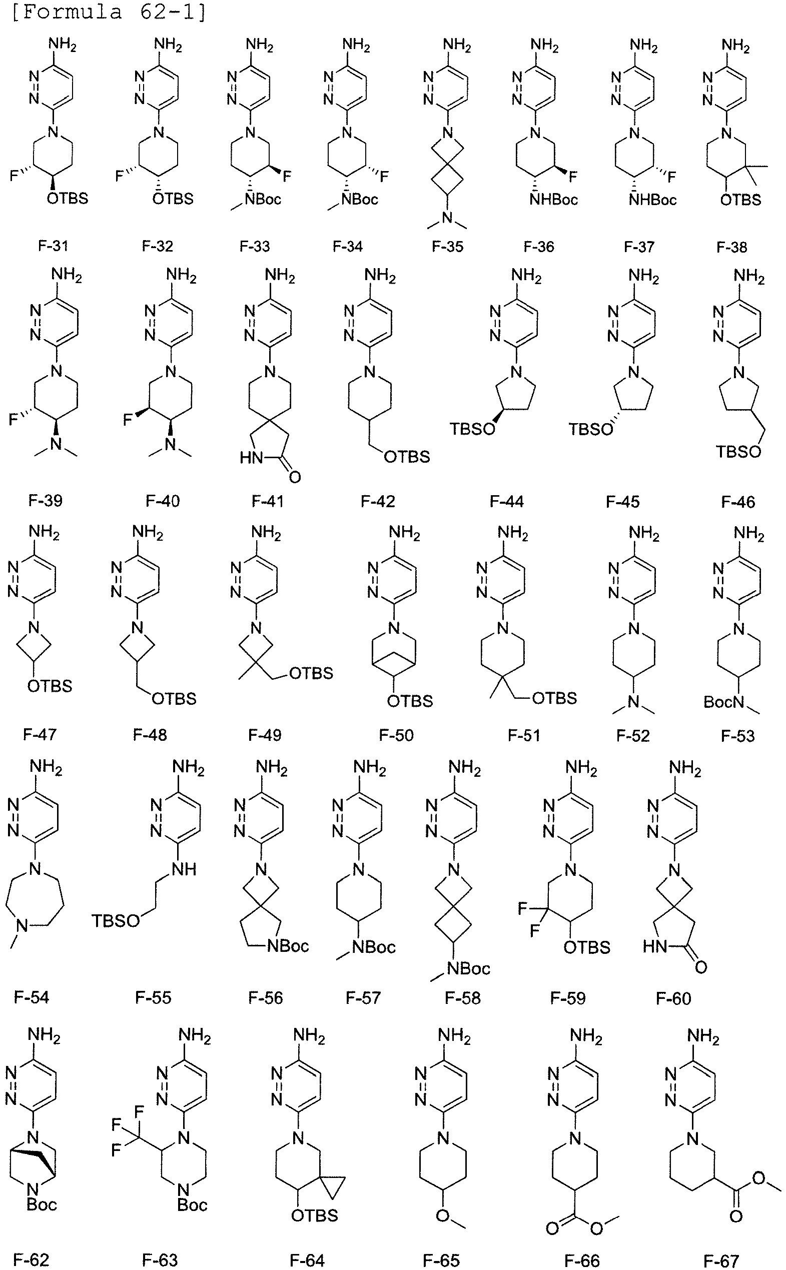

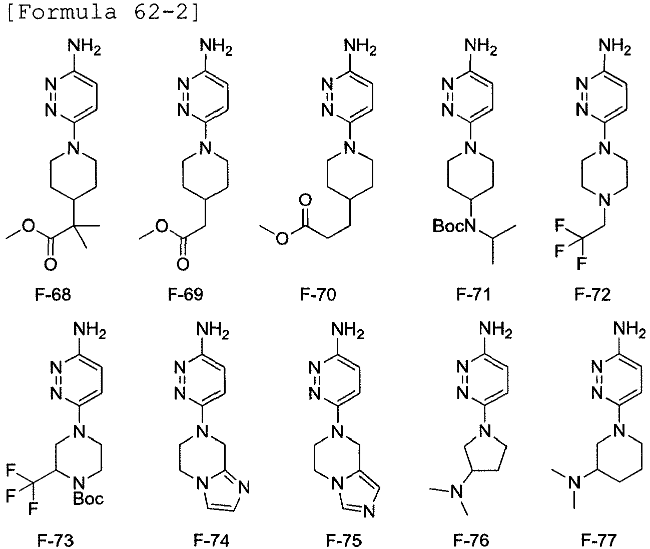

Intermediates G-1 to G-12 were each synthesized by any of the processes of Reference Examples 15, 18, and 19 or a combination of the processes with the corresponding halopyridazine, alcohol, or thiol derivative. Appropriate protection or deprotection was performed as needed.

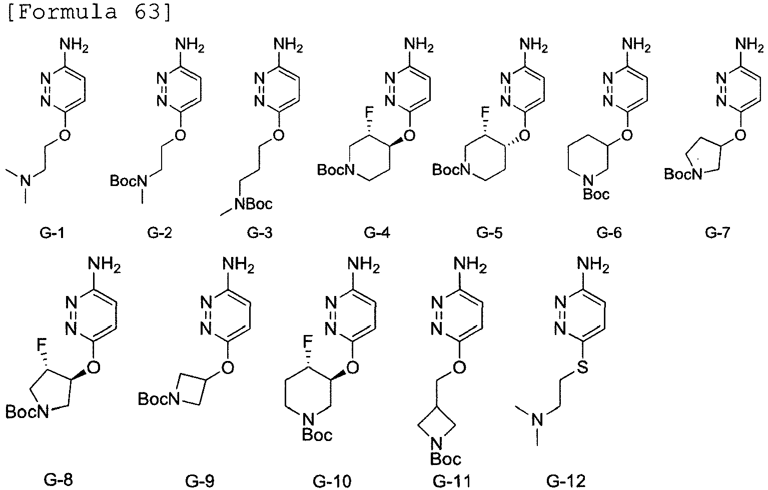

### Reference Example 20

### Synthesis of tert-butyl 4-(6-nitropyridin-3-yl)piperidin-3-ene-1-carboxylate

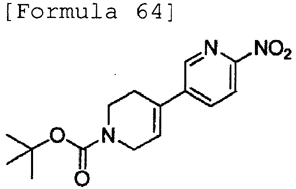

3-Bromo-6-nitropyridine was reacted with tert-butyl 4-(4,4,5,5-tetramethyl-1,3,2-dioxaborolan-2-yl)-5,6-dihydropyridine-1(2H)-carboxylate under heating in the presence of a palladium catalyst by using the process described in J. Med. Chem. 2010, 53, p.7938-7957, to yield the title compound.

### Reference Example 21

### Synthesis of tert-butyl 4-(6-aminopyridin-3-yl)piperidine-1-carboxylate

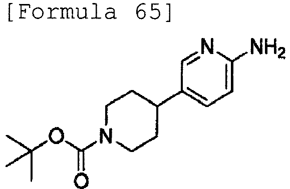

Tert-butyl 4-(6-nitropyridin-3-yl)piperidin-3-ene-1-carboxylate synthesized in Reference Example 20 was reduced under a hydrogen atmosphere in the presence of palladium-carbon by using the process described in J. Med. Chem. 2010, 53, p.7938-7957 to yield the title compound.

Intermediates H-1 to H-12 were each synthesized by the process of Reference Example 20 and/or 21 with the corresponding haloheteroaryl or boric acid derivative. Appropriate protection or deprotection was performed as needed.

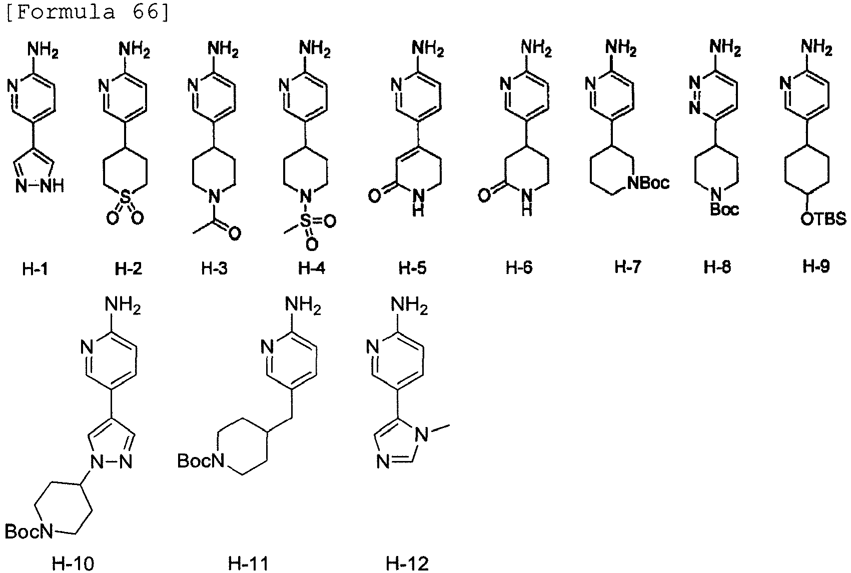

### Reference Example 22

Intermediate I-1 was synthesized through the reaction of tert-butyl chlorosulfonylcarbamate with tert-butyl N-[5-(aminoethyl)-2-pyridyl]-N-tert-butoxycarbonylcarbamate synthesized by any of the processes of Reference Examples 8 to 10 or a combination of the processes, and then the removal of the Boc groups under acidic conditions.

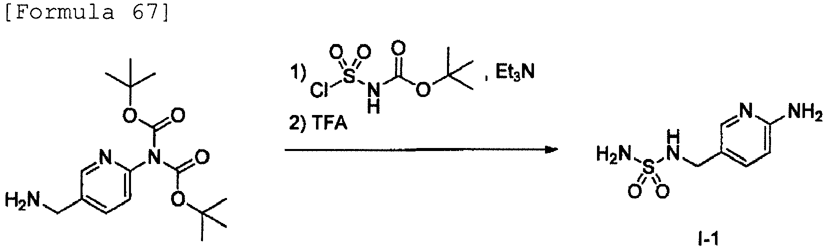

### Reference Example 23

Intermediate J-1 was synthesized through the reaction of potassium isocyanate with tert-butyl N-tert-butoxycarbonyl-N-[5-(N-methylaminoethyl)-2-pyridyl]carbamate synthesized as in Reference Example 8 to 10, and the removal of the Boc groups under acidic conditions.

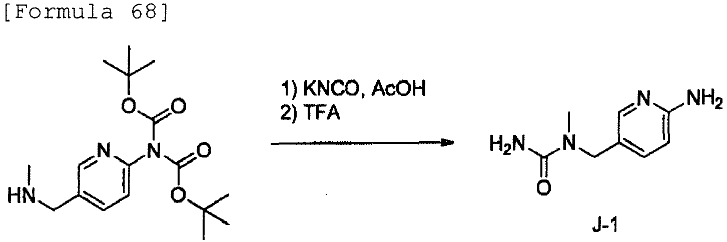

Intermediate J-2 was synthesized through hydrogen reduction of the nitro group of 5-amino-2-nitropyridine in the presence of palladium hydroxide/activated carbon by the process of Reference Example 23.

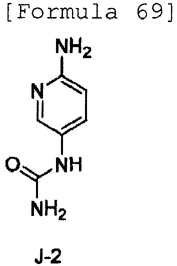

### Reference Example 24

Intermediate K-1 was synthesized through the reaction of isocyanatoethane with tert-butyl N-[5-(aminoethyl)-2-pyridyl]-N-tert-butoxycarbonylcarbamate synthesized by any of the processes of Reference Examples 8 to 10 or a combination of the processes, and the removal of the Boc groups under acidic conditions.

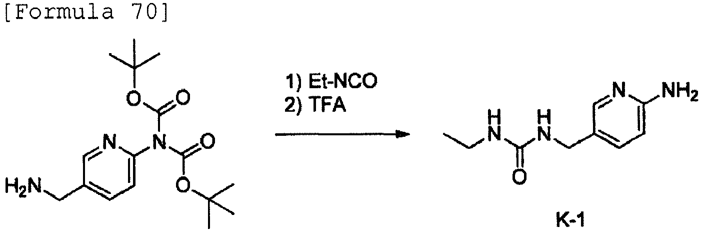

### Reference Example 25

Intermediate L-1 was synthesized through the reaction of 2-methoxyethyl bromide with tert-butyl N-[5-(aminoethyl)-2-pyridyl]-N-tert-butoxycarbonylcarbamate synthesized by any of the processes of Reference Examples 8 to 10 or a combination of the processes, the removal of the Boc groups under acidic conditions, and the selective protection of the secondary amino moiety with a Boc group as in Reference Example 11.

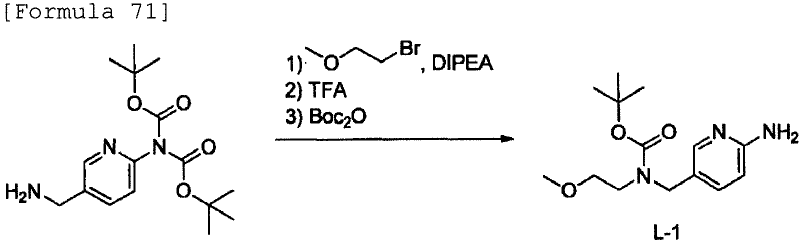

### Reference Example 26

Intermediate M-1 was synthesized through the esterification of the carboxylic acid moiety of 2-(6-chloropyridin-3-yl)acetic acid, dimethylation of the carbonyl group at the α-position, reduction of the ester moiety with LAH, oxidation of the resultant alcohol moiety, reductive amination with methylamine, protection with a Boc group, amination of the 2-chloropyridine moiety in the presence of a Pd catalyst, and deprotection.

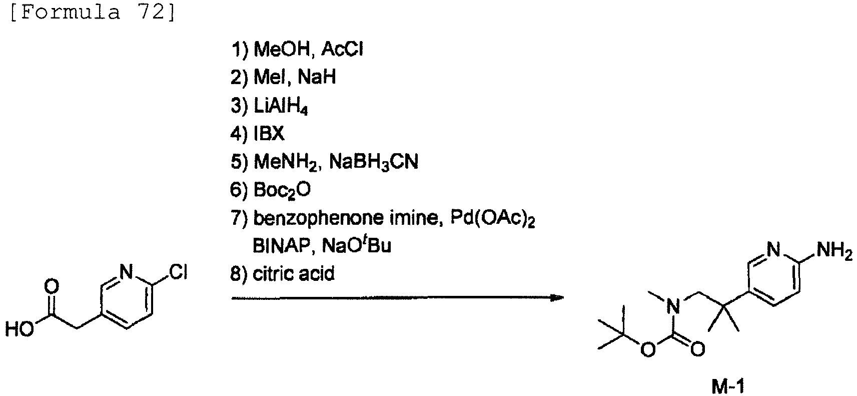

### Reference Example 27

Intermediate N-1 was synthesized through the reaction of 5-bromo-2-nitropyridine with tert-butyl cyanoacetate under basic conditions, removal of the tert-butyl group and decarboxylation under acidic conditions, and reduction of the cyano group.

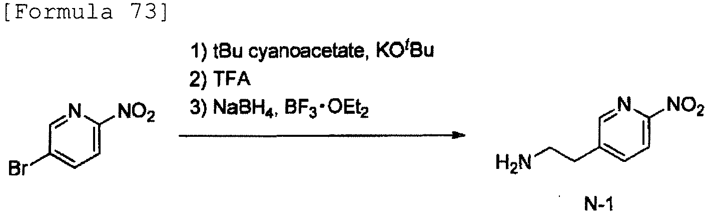

### Reference Example 28

Intermediate 0-1 was synthesized through the reaction of an alkyne derivative with imide derivative, subsequent reaction with 5-bromo-2-nitropyridine under Sonogashira coupling reaction conditions, and reduction with hydrogen in the presence of palladium hydroxide/activated carbon, involving protection and deprotection.

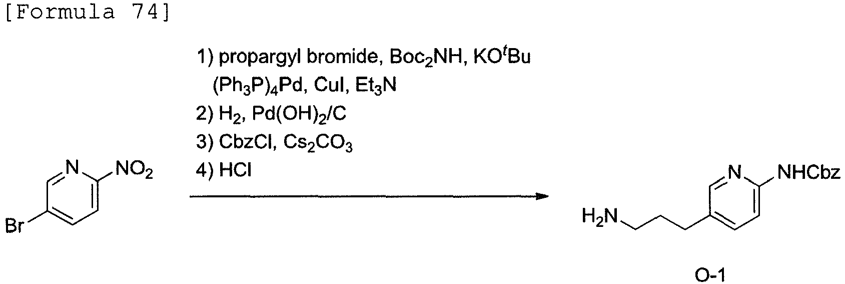

### Reference Example 29

Intermediate P-1 was synthesized through acylation of 2-(6-nitropyridin-3-yl)ethylamine and reduction with hydrogen in the presence of palladium hydroxide/activated carbon.

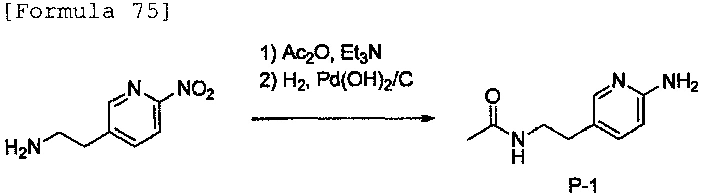

Intermediates P-2 to P-17 were each synthesized by the process of Reference Example 29 with the corresponding amine derivative synthesized by, for example, any of the processes of Reference Examples 8 to 10 and the corresponding acylating agent, involving appropriate deprotection as needed. Appropriate acylation conditions were selected depending on the structure to be introduced. For example, an acid chloride or combination of a carboxylic acid and a condensing agent was used as the acylating agent in place of an acid anhydride.

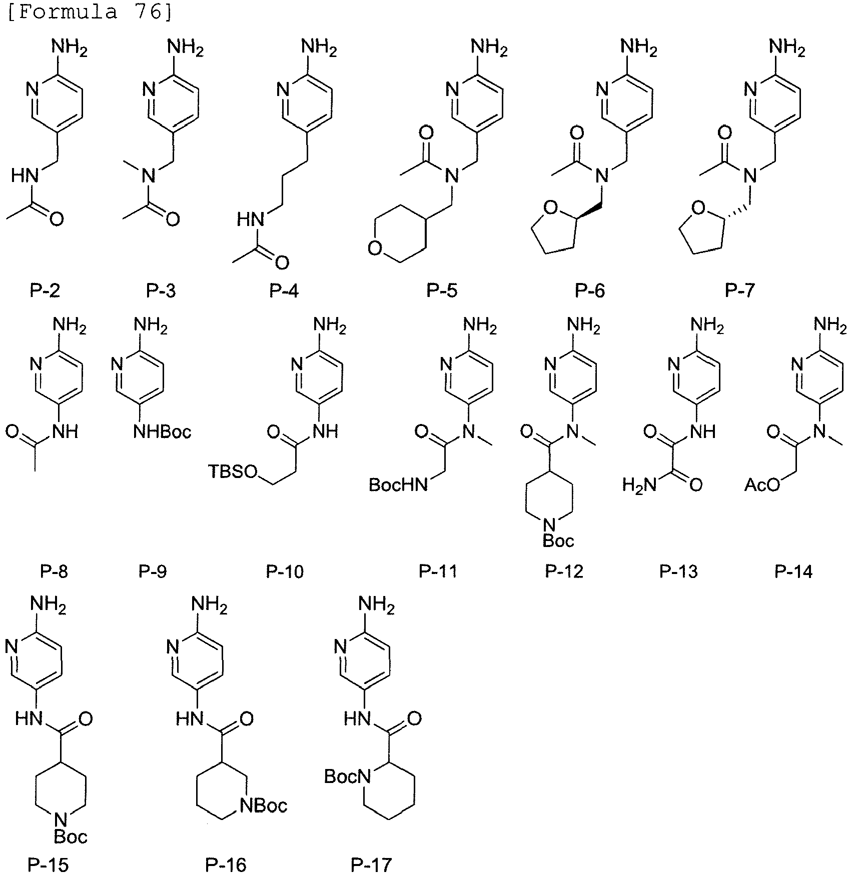

### Reference Example 30

Intermediate Q-1 was synthesized by mesylation of 2-(6-nitropyridin-3-yl)ethylamine and reduction with hydrogen in the presence of palladium hydroxide/activated carbon.

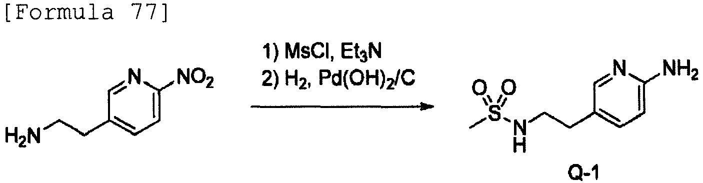

Intermediates Q-2 to Q-9 were each synthesized by the process of Reference Example 29 with the corresponding amine derivative synthesized by, for example, any of the processes of Reference Examples 8 to 10, involving appropriate deprotection as needed.

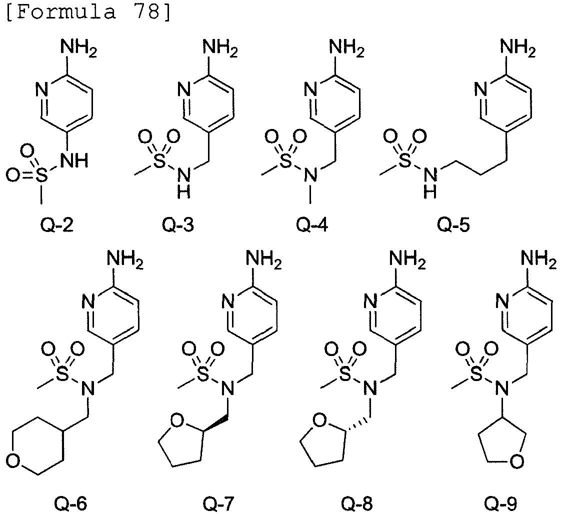

### Reference Example 31

Intermediate R-1 was synthesized through the reaction of 5-bromo-2-nitropyridine with an alkyne derivative under Sonogashira coupling reaction conditions, protection and reduction with hydrogen in the presence of palladium hydroxide/activated carbon, involving protection and deprotection.

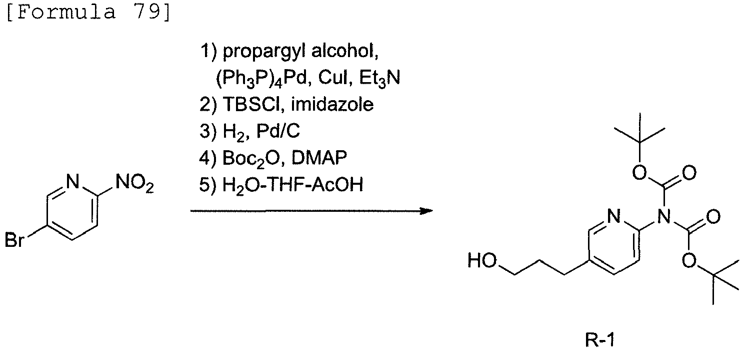

Intermediates R-2 to R-6 were each synthesized by the process of Reference Example 31; i.e., by the reactions 1) to 3) in Reference Example 31 with a halopyridine derivative and the corresponding terminal alkyne derivative.

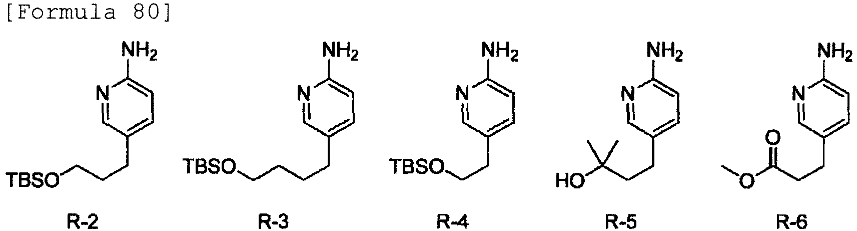

### Reference Example 32

Intermediate S-1 was synthesized through the mesylation of intermediate R-1 synthesized in Reference Example 31, reaction with an amide derivative under basic conditions and subsequent deprotection.

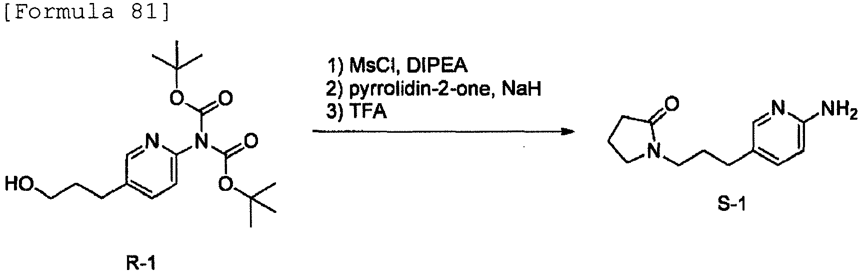

Intermediates S-2 and S-3 were each synthesized by the process of Reference Example 32 with the corresponding alcohol derivative, amide derivative, or sulfonamide derivative.

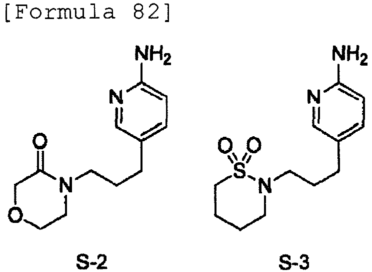

### Reference Example 33

Intermediate T-1 was synthesized through basic hydrolysis of methyl 6-((tert-butoxycarbonyl)amino)nicotinate, condensation with morpholine, and deprotection.

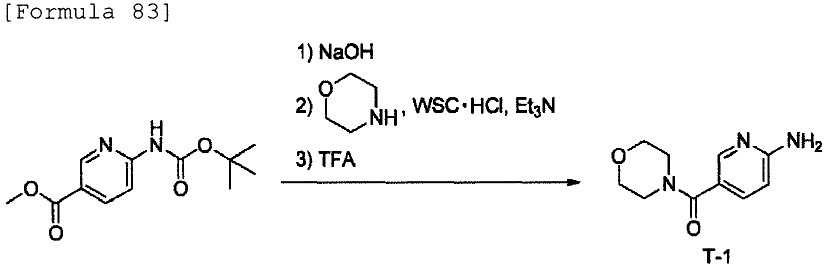

Intermediates T-2 to T-17 were each synthesized by the process of Reference Example 33 with the corresponding ester derivative synthesized by, for example, the process of Reference Example 31, or the corresponding carboxylic acid derivative and amine derivative. Appropriate protection and deprotection were performed as needed.

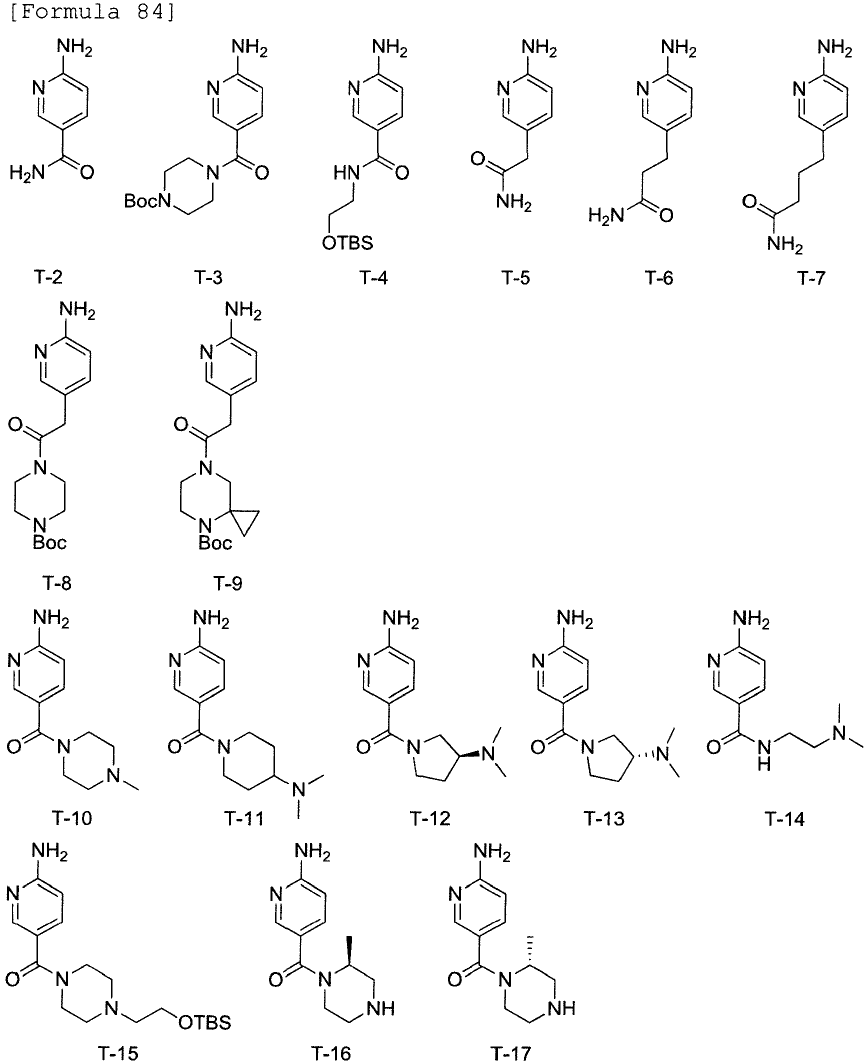

### Reference Example 34

Intermediate U-1 was synthesized by oxidation of 5-((3-((tert-butyldimethylsilyl)oxy)propyl)thio)-2-nitropyridine synthesized by the process of Reference Example 15 with m-chloroperbenzoic acid and reduction with hydrogen in the presence of palladium hydroxide/activated carbon.

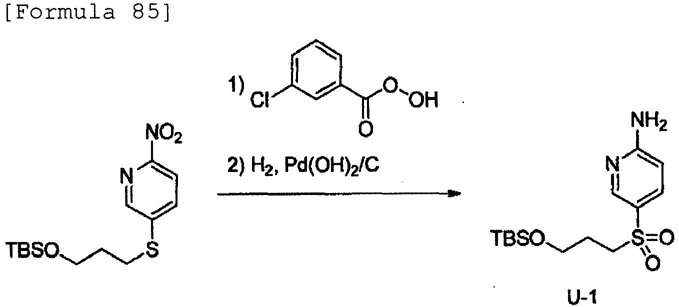

### Reference Example 35

Intermediate V-1 was synthesized through the reaction of 5-amino-2-nitropyridine with sodium azide and orthoformate and subsequent reduction with hydrogen in the presence of palladium hydroxide/activated carbon.

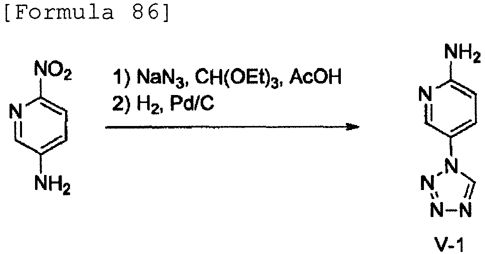

### Reference Example 36

Intermediate W-1 was synthesized through the reaction of tert-butyl 2-chloro-7,8-dihydro-1,6-naphthyridine-6(5H)-carboxylate with benzophenone imine and tert-butoxysodium in the presence of a Pd catalyst and deprotection.

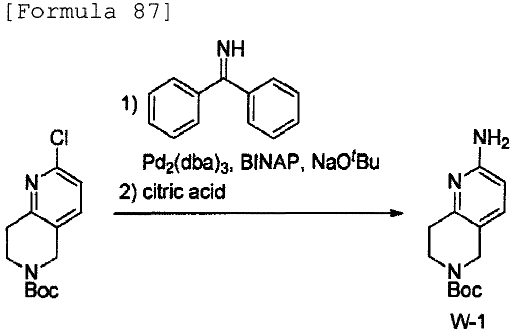

Intermediates W-2 to W-4 were each synthesized by the process of Reference Example 36 with the corresponding halopyridine derivative.

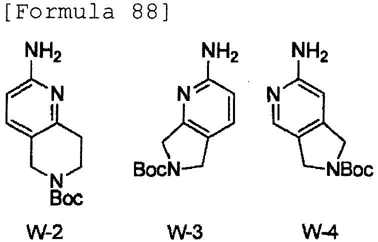

### Example 1

### Synthesis of 3-(4-formyl-2-methylthiopyrimidin-5-yl)-2-propynyl benzoate

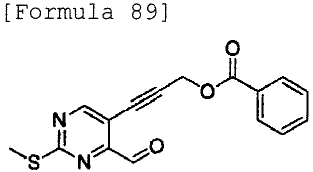

A solution of Pd(PhCN)₂Cl₂ (2.4 g, 6.4 mmol), copper iodide (0.82 g, 4.3 mmol), and [(t-Bu)₃P]HBF₄ (4 g, 13.9 mmol) in 1,4-dioxane (55 mL) was degassed and purged with argon, and diisopropylamine (18.5 mL, 128.8 mmol) was added to the solution at room temperature. The resultant reaction mixture was stirred at room temperature for five minutes. A solution of a mixture (25 g, crude product) of 5-bromo-2-methylsulfanylpyrimidine-4-carbaldehyde and (5-bromo-2-methylsulfanylpyrimidin-4-yl)methoxymethanol described in Reference Example 3 and propargyl benzoate (20 g, 128.8 mmol) in 1,4-dioxane (55 mL) was slowly added dropwise to the reaction mixture, and the reaction mixture was then stirred at room temperature for five hours. The reaction was monitored by LC/MS. After completion of the reaction, the reaction mixture was diluted with ethyl acetate (1 L). The mixture was subjected to suction filtration through Celite, and the Celite was washed with ethyl acetate. The filtrate was concentrated under reduced pressure, and the resultant crude product was directly used for the subsequent reaction.

### Example 2

### Synthesis of 6-((benzoyloxy)methyl)-2-(methylthio)pyrido[3,4-d]pyrimidine 7-oxide

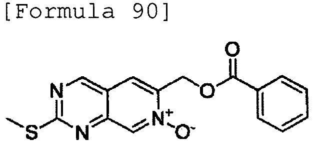

The crude product of 3-(4-formyl-2-methylthiopyrimidin-5-yl)-2-propynyl benzoate synthesized in Example 1 was dissolved in ethanol (500 mL), and hydroxylamine hydrochloride (8.3 g, 120 mmol) and sodium acetate (10 g, 120 mmol) were added to the solution at room temperature. The resultant reaction mixture was stirred at room temperature for six hours, and then diluted with ethanol (1 L). Potassium carbonate (27.8 g, 200 mmol) was added to the mixture, and the mixture was then stirred at 50°C for three hours. The reaction was monitored by LC/MS. After completion of the reaction, the reaction mixture was subjected to suction filtration through Celite, and the Celite was washed with ethyl acetate. The filtrate was dried over anhydrous sodium sulfate, and solid was separated by filtration. The filtrate was concentrated under reduced pressure, and the resultant crude product was purified by silica gel column chromatography to yield the title compound (5.0 g, 16%).

### Example 3

### Synthesis of 8-chloro-2-methylthiopyrido[3,4-d]pyrimidin-6-yl benzoate

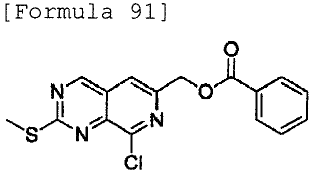

The 6-((benzoyloxy)methyl)-2-(methylthio)pyrido[3,4-d]pyrimidine 7-oxide synthesized in Example 2 (5.0 g, 15.3 mmol) was dissolved in dichloromethane (60 mL) and cooled to 0°C. Thionyl chloride (25 mL, 343 mmol) was added dropwise to the solution at 0°C, and the mixture was stirred at room temperature for 16 hours. The reaction was monitored by TLC. After completion of the reaction, the reaction mixture was concentrated under reduced pressure, followed by azeotropic distillation twice with toluene (20 mL), to remove thionyl chloride. The residue was roughly purified by neutral alumina column chromatography to yield the title compound (2.75 g, 52%).

### Example 4

### Synthesis of (R)-1-(2-(methylthio)-8-(((S)-tetrahydro-2H-pyran-3-yl)amino)pyrido[3,4-d]pyrimidin-6-yl)ethyl benzoate

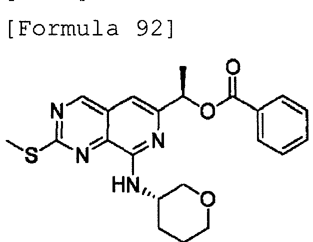

A mixture of (R)-1-(8-chloro-2-(methylthio)pyrido[3,4-d]pyrimidin-6-yl)ethyl benzoate synthesized by the process described in Example 3 (360 mg, 1.0 mmol), (S)-tetrahydro-2H-pyran-3-amine hydrochloride (206 mg, 1.5 mmol), and potassium carbonate (415 mg, 3.0 mmol) in 1,4-dioxane (4.0 mL) was stirred at 100°C overnight. The reaction was monitored by TLC. After completion of the reaction, the reaction mixture was cooled to room temperature. The reaction mixture was diluted with water, and the mixture was extracted twice with ethyl acetate (10 mL). The resultant organic phase was washed with brine and dried over anhydrous magnesium sulfate. The solid was separated by filtration, and the filtrate was concentrated under reduced pressure. The resultant crude product was purified by silica gel column chromatography to yield the title compound (232 mg, 55%). ¹H-NMR (CDCl₃)δ: 8.97 (1H, s), 8.17-8.14 (2H, m), 7.62-7.57 (1H, m), 7.51-7.46 (2H, m), 6.87 (1H, s), 6.65 (1H, d, J=7.8 Hz), 6.10 (1H, q, J=6.7 Hz), 4.39-4.31 (1H, m), 4.08-4.03 (1H, m), 3.82-3.76 (1H, m), 3.70-3.64 (1H, m), 3.56-3.51 (1H, m), 2.65 (3H, s), 2.09-2.02 (1H, m), 1.89-1.78 (2H, m), 1.76-1.65 (4H, m)
LC/MS: (M+H)⁺=425.2, C₂₂H₂₄N₄O₃S=424.16

### Example 5

### Synthesis of 2-methylthio-8-(propan-2-yl)aminopyrido[3,4-d]pyrimidin-6-ylmethanol

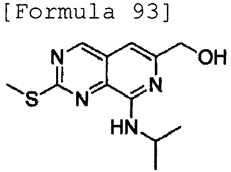

The 8-isopropylamino-2-methylthiopyrido[3,4-d]pyrimidine-6-yl benzoate synthesized by the process described in Example 4 (3.7 g, 10.0 mmol) was dissolved in methanol (20 mL) and THF (20 mL), and an aqueous solution (10 mL) of lithium hydroxide (0.96 g, 40 mmol) was added dropwise to the solution at room temperature. The resultant reaction mixture was stirred at room temperature for one hour. The reaction was monitored by LC/MS. After completion of the reaction, hydrochloric acid (2 mol/L) was added dropwise to the reaction mixture, to adjust the pH of the mixture to 7. The resultant solid was separated by filtration and dried under reduced pressure to yield the title compound (2.55 g, 96%).

### Example 6

### Synthesis of 2-methylthio-8-(propan-2-yl)aminopyrido[3,4-d]pyrimidine-6-carbaldehyde

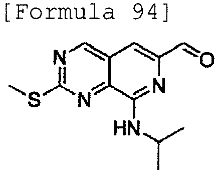

The 2-methylthio-8-(propan-2-yl)aminopyrido[3,4-d]pyrimidin-6-ylmethanol synthesized in Example 5 (3.1 g, 11.7 mmol) was dissolved in dichloromethane (30 mL) and the solution was stirred at 0°C. Dess-Martin Periodinane (15 g, 35.2 mmol) was gradually added to the solution at 0°C, and the reaction mixture was stirred at room temperature for three hours. The reaction was monitored by LC/MS. After completion of the reaction, the reaction was quenched by addition of an aqueous sodium thiosulfate solution for reduction of excess reagent. The aqueous phase was extracted three times with dichloromethane (50 mL). The resultant organic phases were combined together and dried over anhydrous sodium sulfate. The solid was separated by filtration, and the filtrate was then concentrated under reduced pressure. The residue was purified by silica gel column chromatography to yield the title compound (2.9 g, 94%).

### Example 7

### Synthesis of 6-difluoromethyl-2-methylthio-N-(propan-2-yl)pyrido[3,4-d]pyrimidine-8-amine

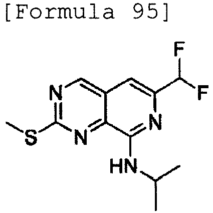

The 2-methylthio-8-(propan-2-yl)aminopyrido[3,4-d]pyrimidine-6-carbaldehyde synthesized in Example 6 (2.9 g, 11.1 mmol) was dissolved in dichloromethane (30 mL) and the solution was stirred at 0°C. DAST (7.1 g, 44.2 mmol) was gradually added to the solution at 0°C, and the reaction mixture was stirred at room temperature for three hours. The reaction was monitored by LC/MS. After completion of the reaction, the reaction was quenched by addition of saturated aqueous sodium carbonate solution (20 mL). The aqueous phase was extracted three times with dichloromethane (50 mL). The resultant organic phases were combined together and dried over anhydrous sodium sulfate. The solid was separated by filtration, and the filtrate was then concentrated under reduced pressure. The residue was purified by silica gel column chromatography to yield the title compound (2.37 g, 75%).

Compounds Int-1 to Int-8 were synthesized by the process described in Example 4 or Examples 5 to 7 in an appropriate order depending on the substituents.

**[Table 1-1]**

| Compound No. | Structure | NMR | (M+H)⁺ | Exact Mass |
|---|---|---|---|---|
| Int-1 | 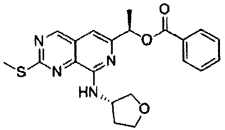 | 1H-NMR (CDCl3) δ: 8.99 (1H, s), 8.17-8.13 (2H, m), 7.63-7.56 (1H, m), 7.52-7.45 (2H, m), 6.90 (1H, s), 6.59 (1H, d, J = 6.3 Hz), 6.11 (1H, q, J = 6.7 Hz), 4.84-4.74 (1H, m), 4.11-4.00 (2H, m), 3.93-3.79 (2H, m), 2.64 (3H, s), 2.47-2.35 (1H, m), 2.06-1.95 (1H, m), 1.73 (3H, d, J = 6.8 Hz). | | |
| Int-2 | 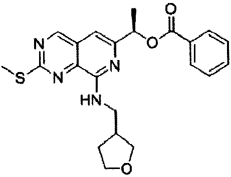 | 1H-NMR (CDCl3) δ: 8.98 (1H, s), 8.18-8.12 (2H, m), 7.63-7.56 (1H, m), 7.52-7.44 (2H, m), 6.87 (1H, s), 6.76-6.68 (1H, br m), 6.15-6.06 (1H, m), 3.98-3.55 (6H, m), 2.80-2.62 (4H, m), 2.12-2.01 (1H, m), 1.79-1.69 (4H, m). | | |
| Int-3 | 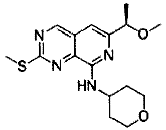 | 1H-NMR (CDCl3) δ: 9.01 (1H, s), 6.87 (1H, s), 6.42 (1H, d, J = 7.3 Hz), 4.39-4.27 (2H, m), 4.07-4.00 (2H, m), 3.66-3.57 (2H, m), 3.40 (3H, s), 2.66 (3H, s), 2.18-2.09 (2H, m), 1.73-1.59 (2H, m), 1.48 (3H, d, J = 6.8 Hz). | | |
| Int-4 | 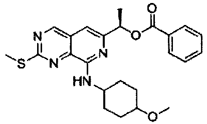 | | 453.3 | 452.19 |

**[Table 1-2]**

| Compound No. | Structure | NMR | (M+H)⁺ | Exact Mass |
|---|---|---|---|---|
| Int-5 | 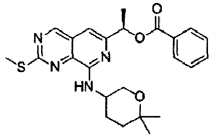 | | 453.3 | 452.19 |
| Int-6 | 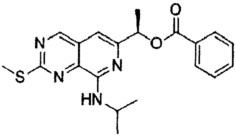 | | 383.10 | 382.15 |
| Int-7 | 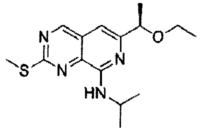 | | 307.15 | 306.15 |
| Int-8 | 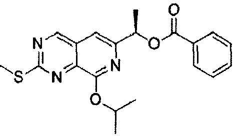 | | 384.10 | 383.13 |

### Example 8

### Synthesis of (R)-1-(2- (methylsulfonyl)-8-(((S)-tetrahydro-2H-pyran-3-yl)amino)pyrido[3,4-d]pyrimidin-6-yl)ethyl benzoate

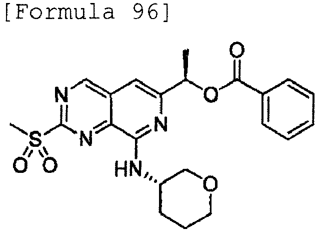

The (R)-1-(2-(methylthio)-8-(((S)-tetrahydro-2H-pyran-3-yl)amino)pyrido[3,4-d]pyrimidin-6-yl)ethyl benzoate synthesized in Example 4 (232 mg, 0.55 mmol) and Oxone (R) (672 mg, 1.09 mmol) were added to THF (2.7 mL) and water (2.7 mL) and the reaction mixture was stirred at room temperature overnight. The reaction was monitored by LC/MS. After completion of the reaction, saturated aqueous sodium hydrogen carbonate solution was slowly added to the reaction mixture, and the aqueous phase was extracted three times with ethyl acetate. The resultant organic phases were combined together and washed with saturated brine, and then dried over anhydrous magnesium sulfate. The solid was separated by filtration, and the filtrate was then concentrated under reduced pressure. The residue was purified by silica gel column chromatography to yield a crude product of the title compound (245 mg, 98%).
¹H-NMR (CDCl₃)δ: 9.30 (1H, s), 8.16 (2H, d, J=7.3 Hz), 7.65-7.60 (1H, m), 7.53-7.48 (2H, m), 7.02 (1H, s), 6.87 (1H, d, J=7.8 Hz), 6.13 (1H, q, J=6.7 Hz), 4.45-4.36 (1H, m), 4.08-4.04 (1H, m), 3.85-3.80 (1H, m), 3.67-3.60 (1H, m), 3.52-3.47 (1H, m), 3.41 (3H, s), 2.14-2.07 (1H, m), 1.90-1.74 (6H, m).
LC/MS: (M+H)⁺=457.2, C₂₂H₂₄N₄O₅S=456.15

### Example 9

### Synthesis of (R)-1-(8-(1-methoxy-2-methylpropan-2-ylamino)-2-(methylsulfinyl)pyrido[3,4-d]pyrimidin-6-yl)ethyl benzoate

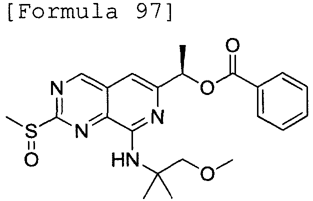

(R)-1-(8-(1-methoxy-2-methylpropan-2-ylamino)-2-(methylthio)pyrido[3,4-d]pyrimidin-6-yl)ethyl benzoate synthesized by the process described in Example 7 (1.9 g, 4.46 mmol) was dissolved in dichloromethane (30 mL) and the solution was stirred at 0°C. m-CPBA (0.767 g, 4.46 mmol) was gradually added to the solution at 0°C, and the reaction mixture was stirred at room temperature overnight. The reaction was monitored by LC/MS. After completion of the reaction, the reaction was quenched by addition of an aqueous sodium thiosulfate solution for reduction of excess reagent. The aqueous phase was extracted three times with dichloromethane (30 mL). The resultant organic phases were combined together and washed once with saturated aqueous sodium hydrogen carbonate solution (50 mL) and once with saturated brine (50 mL). The organic phase was dried over anhydrous sodium sulfate, and the solid was separated by filtration. The filtrate was then concentrated under reduced pressure, and the residue was purified by silica gel column chromatography to yield the title compound (1.9 g, 96%).

Compounds Int-9 to Int-16 were synthesized by the process described in Example 8 or 9.

**[Table 2]**

| Compound No. | Structure | (M+H)⁺ | Exact Mass |
|---|---|---|---|
| Int-9 | 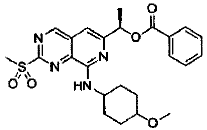 | 485.3 | 484.18 |
| Int-10 | 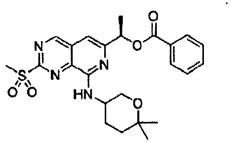 | 485.3 | 484.18 |
| Int-11 | 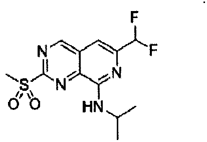 | 317.10 | 316.08 |
| Int-12 | 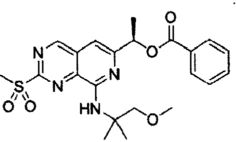 | 459.15 | 458.16 |
| Int-13 | 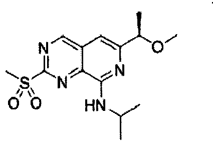 | 325.10 | 324.13 |
| Int-14 | 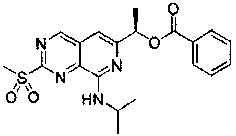 | 415.10 | 414.14 |
| Int-15 | 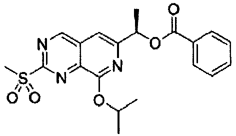 | 416.05 | 415.12 |
| | | 374.05 | |
| Int-16 | 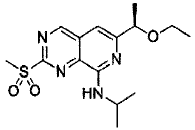 | 339.15 | 338.14 |

### Example 10

### Synthesis of (R)-1-(5-chloro-2-(methylsulfonyl)-8-(((S)-tetrahydro-2H-pyran-3-yl)amino)pyrido[3,4-d]pyrimidin-6-yl)ethyl benzoate

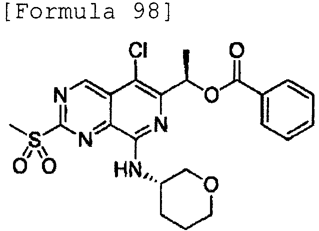

A mixture of (R)-1-(2-(methylsulfonyl)-8-(((S)-tetrahydro-2H-pyran-3-yl)amino)pyrido[3,4-d]pyrimidin-6-yl)ethyl benzoate synthesized in Example 8 (268 mg, 0.587 mmol) and N-chlorosuccinimide (96 mg, 0.72 mmol) in 1,2-dichloroethane (2.9 mL) was stirred at 65°C overnight. The reaction was monitored by LC/MS. After completion of the reaction, the reaction mixture was cooled to room temperature. The reaction mixture was directly purified by silica gel column chromatography to yield the title compound (255 mg, 89%).
¹H-NMR (CDCl₃)δ : 9.70 (1H, s), 8.11-8.06 (2H, m), 7.60-7.53 (1H, m), 7.48-7.42 (2H, m), 6.90 (1H, d, J=7.8 Hz), 6.46 (1H, q, J=6.7 Hz), 4.28-4.18 (1H, m), 3.82 (1H, dd, J=11.5, 3.2 Hz), 3.76-3.69 (1H, m), 3.65-3.56 (1H, m), 3.45-3.37 (4H, m), 2.09-2.00 (1H, m), 1.88-1.61 (6H, m).

Compound Int-17 was synthesized by the process described in Example 10.

**[Table 3]**

| Compound No. | Structure | (M+H) ⁺ | Exact Mass |
|---|---|---|---|
| Int-17 | 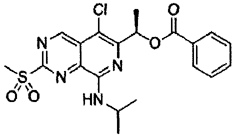 | 449.10 | 448.10 |

### Example 11

### Synthesis of tert-butyl 4-[6-(6-difluoromethyl-8-isopropylaminopyrido[3,4-d]pyrimidin-2-ylamino)pyridin-3-yl]piperazine-1-carboxylate

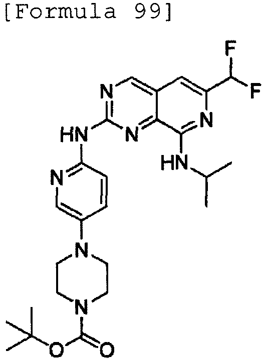

The tert-butyl 4-(6-aminopyridin-3-yl)piperazine-1-carboxylate synthesized in Reference Example 5 (88 mg, 0.316 mmol) was dissolved in THF (3.5 mL), and sodium hydride (22.8 mg, 0.57 mmol, 60%) was added to the solution at 0°C and stirred for 10 minutes. To the suspension was added a solution of the (6-difluoromethyl-2-methanesulfonylpyrido[3,4-d]pyrimidine-8-yl)isopropylamine synthesized in Example 8 (Int-11, 100 mg, 0.316 mmol) in THF (3.5 mL) at room temperature and the reaction mixture was stirred at 35°C for one hour. The reaction was monitored by TLC and LC/MS. After completion of the reaction, the reaction was quenched by addition of ice water (10 mL). The aqueous phase was extracted twice with ethyl acetate (25 mL). The resultant organic phases were combined together and washed with saturated brine, and the mixture was dried over anhydrous sodium sulfate. The solid was separated by filtration, and the filtrate was then concentrated under reduced pressure. The residue was purified by silica gel column chromatography to yield the title compound (56.7 mg, 35%).

### Example 12

### Synthesis of 6-difluoromethyl-8-isopropyl-2-(5-piperazin-1-ylpyridin-2-yl)pyrido[3,4-d]pyrimidine-2,8-diamine (compound 3)

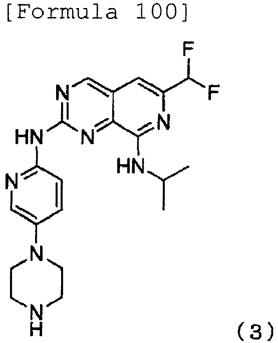

The tert-butyl 4-[6-(6-difluoromethyl-8-isopropylaminopyrido[3,4-d]pyrimidin-2-ylamino)pyridin-3-yl]piperazine-1-carboxylate synthesized in Example 11 (195 mg, 0.378 mmol) was dissolved in dichloromethane (5 mL) and stirred at 0°C. Hydrogen chloride (0.4 mL, 4 mol/L, 1,4-dioxane solution) was added dropwise to the solution and stirred at room temperature for 30 minutes. The reaction was monitored by LC/MS. After completion of the reaction, the reaction mixture was concentrated under reduced pressure. The resultant crude product was purified by fractionation HPLC (acetonitrile/water/TFA) to yield a TFA salt of the title compound (114 mg, purity: 99% or more). The TFA salts obtained by multiple reactions were combined and used in the next step.

The TFA salt (200 mg) was dissolved in methanol (0.625 mL) and dichloromethane (1.875 mL) and applied onto a strong cation exchange resin (SCX) column. The SCX column was washed with a solvent mixture of methanol and dichloromethane (1:3). The target compound was subsequently eluted from the SCX column with a solvent mixture of methanol and dichloromethane (1:3) containing 2.5% ammonia (2 mol/L, methanol solution). The eluate was concentrated under reduced pressure to yield the title compound (105 mg, purity: > 99%).

### Example 13

### Synthesis of tert-butyl (R)-4-(6-(6-(benzoyloxy)ethyl-8-(1-methoxy-2-methylpropan-2-ylamino)pyrido[3,4-d]pyrimidin-2-ylamino)pyridin-3-yl)piperazine-1-carboxylate

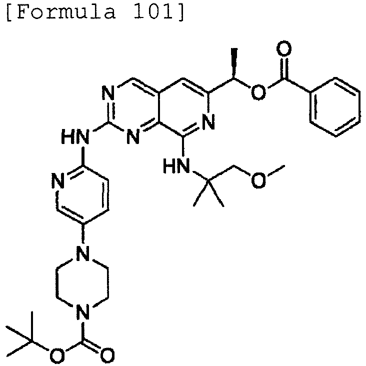

The (R)-1-(8-(1-methoxy-2-methylpropan-2-ylamino)-2-(methylsulfinyl)pyrido[3,4-d]pyrimidin-6-yl) ethyl benzoate synthesized in Example 9 (1.9 g, 4.3 mmol) and the tert-butyl 4-(6-aminopyridin-3-yl)piperazine-1-carboxylate synthesized in Reference Example 5 (3.59 g, 12.9 mmol) were suspended in toluene (30 mL) and the reaction mixture was stirred at 120°C overnight. The reaction was monitored by LC/MS. The resultant reaction mixture was cooled to room temperature, and the solvent was removed through evaporation under reduced pressure. The residue was purified by silica gel column chromatography to yield the title compound (850 mg, 30%).

### Example 14

### Synthesis of (R)-1-(8-(1-methoxy-2-methylpropan-2-ylamino)-2-(5-(piperazin-1-yl)pyridin-2-ylamino)pyrido[3,4-d]pyrimidin-6-yl)ethanol (compound 195)

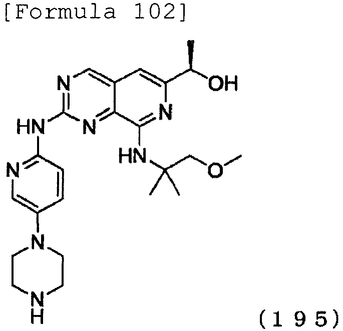

The tert-butyl (R)-4-(6-(6-(benzoyloxy)ethyl-8-(1-methoxy-2-methylpropan-2-ylamino)pyrido[3,4-d]pyrimidin-2-ylamino)pyridin-3-yl)piperazine-1-carboxylate synthesized in Example 13 (850 mg, 1.3 mmol) was dissolved in THF (15 mL) and methanol (15 mL), and lithium hydroxide (124 mg, 5.2 mmol) was added to the solution. The resultant reaction mixture was stirred at room temperature overnight, and the reaction was monitored by LC/MS. After completion of the reaction, hydrogen chloride (4 mol/L, methanol solution) was added dropwise to the reaction mixture, to adjust the pH of the mixture to 7. The reaction mixture was concentrated under reduced pressure to yield a crude product. The crude product was used for the subsequent reaction without purification.

The crude product was dissolved in hydrogen chloride (20 mL, 4 mol/L, methanol solution) and stirred at room temperature for four hours. The reaction was monitored by LC/MS. After completion of the reaction, the reaction mixture was concentrated under reduced pressure. The residue was dissolved in methanol (30 mL), and concentrated aqueous ammonia (25%) was added dropwise to the solution, to adjust the pH of the solution to 10 or higher. Saturated brine (100 mL) was added to the solution, and the mixture was extracted three times with a solvent mixture of dichloromethane and methanol (9:1) (30 mL). The resultant organic phases were combined together and washed once with saturated brine (50 mL). The organic phase was dried over anhydrous sodium sulfate, and the solid was separated by filtration. The filtrate was concentrated under reduced pressure to yield a crude product of the title compound. The crude product was then washed with methanol to yield the title compound (470 mg, 80%).

### Example 15

### Synthesis of (S)-1-(4-(6-((6-((R)-1-hydroxyethyl)-8-(isopropylamino)pyrido[3,4-d]pyrimidin-2-yl)amino)pyridazin-3-yl)piperazin-1-yl)propan-2-ol (compound 676)

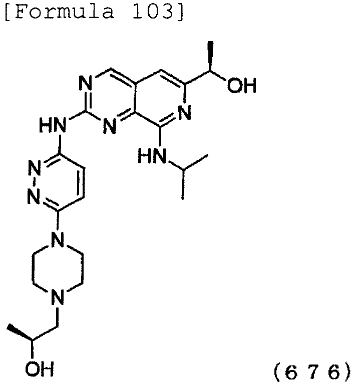

(R)-1-(8-(isopropylamino)-2-((6-(piperazin-1-yl)pyridazin-3-yl)amino)pyrido[3,4-d]pyrimidin-6-yl)ethanol synthesized by the process described in Example 14 (compound 261, 25 mg, 0.061 mmol) was dissolved in methanol (0.31 mL), and (S)-propylene oxide (3.5 mg, 0.061 mmol) was added to the solution. The resultant reaction mixture was stirred at 55°C overnight, and the reaction was monitored by LC/MS. After completion of the reaction, the reaction mixture was concentrated under reduced pressure. The resultant crude product was purified by fractionation HPLC (acetonitrile/water/TFA) and applied onto a strong cation exchange resin (SCX) column. The SCX column was washed with methanol, and the target product was then eluted with ammonia (2 mol/L, methanol solution). The eluate was concentrated under reduced pressure to yield the title compound (19 mg).

### Example 16

### Synthesis of (R)-1-(8-(isopropylamino)-2-((6-(4-(oxetan-3-yl)piperazin-1-yl)pyridazin-3-yl)amino)pyrido[3,4-d]pyrimidin-6-yl)ethanol (compound 682)

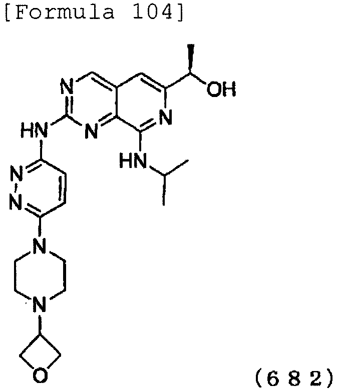

(R)-1-(8-(isopropylamino)-2-((6-(piperazin-1-yl)pyridazin-3-yl)amino)pyrido[3,4-d]pyrimidin-6-yl)ethanol synthesized by the process described in Example 14 (compound 261, 16.4 mg, 0.040 mmol) was dissolved in acetic acid (2.8 µL) and 1,2-dichloroethane (0.4 mL). To the mixture was added 3-oxetanone (2.8 µL, 0.048 mmol) and sodium triacetoxyborohydride (12.7 mg, 0.060 mmol). The resultant reaction mixture was stirred at 55°C for two hours, and the reaction was monitored by LC/MS. After completion of the reaction, the reaction mixture was cooled to room temperature, and the reaction was quenched by addition of water. The reaction mixture was extracted with ethyl acetate, and the organic layer was concentrated under reduced pressure. The resultant crude product was then purified by amine-modified column chromatography (ethyl acetate/methanol) to yield the title compound (7.5 mg).

### Example 17

### Synthesis of (R)-3-(4-(6-((8-(isopropylamino)-6-(1-methoxyethyl)pyrido[3,4-d]pyrimidin-2-yl)amino)pyridazin-3-yl)piperazin-1-yl)propanoic acid (compound 684)

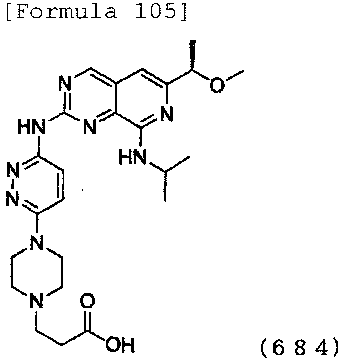

(R)-N8-isopropyl-6-(1-methoxyethyl)-N2-(6-(piperazin-1-yl)pyridazin-3-yl)pyrido[3,4-d]pyrimidine-2,8-diamine synthesized by the process described in Example 14 (compound 217, 29.6 mg, 0.07 mmol) was dissolved in methanol (0.35 mL), and methyl acrylate (6.3 µL, 0.07 mmol) was added to the solution. The resultant reaction mixture was stirred at 55°C for two hours, and the reaction was monitored by LC/MS. After completion of the reaction, the reaction mixture was concentrated under reduced pressure, and the resultant crude product was roughly purified by silica gel column chromatography (ethyl acetate/heptane). The crude product was then dissolved in THF (0.56 mL) and methanol (0.56 mL), and 4M aqueous lithium hydroxide solution (0.028 mL, 0.112 mmol) was added to the solution. The resultant reaction mixture was stirred at room temperature overnight, and the reaction was monitored by LC/MS. After completion of the reaction, the reaction mixture was acidified with 2M aqueous hydrochloric acid solution and then adsorbed onto a strong cation exchange resin (SCX) column. The SCX column was washed with water and dichloromethane, and the target product was then eluted with ammonia (2 mol/L, methanol solution). The eluate was concentrated under reduced pressure to yield the title compound (27.5 mg).

### Example 18

### Synthesis of (R)-2-(4-(6-((8-(isopropylamino)-6-(1-methoxyethyl)pyrido[3,4-d]pyrimidin-2-yl)amino)pyridazin-3-yl)piperazin-1-yl)-2-methylpropanoic acid (compound 678)

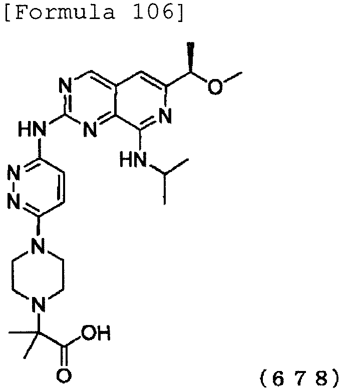

(R)-N8-isopropyl-6-(1-methoxyethyl)-N2-(6-(piperazin-1-yl)pyridazin-3-yl)pyrido[3,4-d]pyrimidine-2,8-diamine synthesized by the process described in Example 14 (compound 217, 42.3 mg, 0.10 mmol) was dissolved in acetonitrile (0.2 mL). To the mixture was added tert-butyl 2-bromo-2-methylpropanoate (22.4 µL, 0.12 mmol) and potassium carbonate (16.6 mg). The resultant reaction mixture was stirred at 85°C overnight, and the reaction was monitored by LC/MS. After completion of the reaction, the reaction mixture was cooled to room temperature, and the reaction was quenched by addition of water. The reaction mixture was extracted with ethyl acetate, and the resultant crude product was briefly purified by silica gel column chromatography (ethyl acetate/heptane). The crude product was then dissolved in dichloromethane (1 mL), and trifluoroacetic acid (1 mL) was added to the solution. The resultant reaction mixture was stirred at room temperature for 24 hours, and the reaction was monitored by LC/MS. After completion of the reaction, the reaction mixture was concentrated under reduced pressure, and the resultant crude product was adsorbed onto a strong cation exchange resin (SCX) column. The SCX column was washed with methanol, and the target product was then eluted with ammonia (2 mol/L, methanol solution). The eluate was concentrated under reduced pressure to yield the title compound (8.5 mg).

### Example 19

Compounds 1 to 1239 were synthesized by the processes described in Examples 11 to 18.

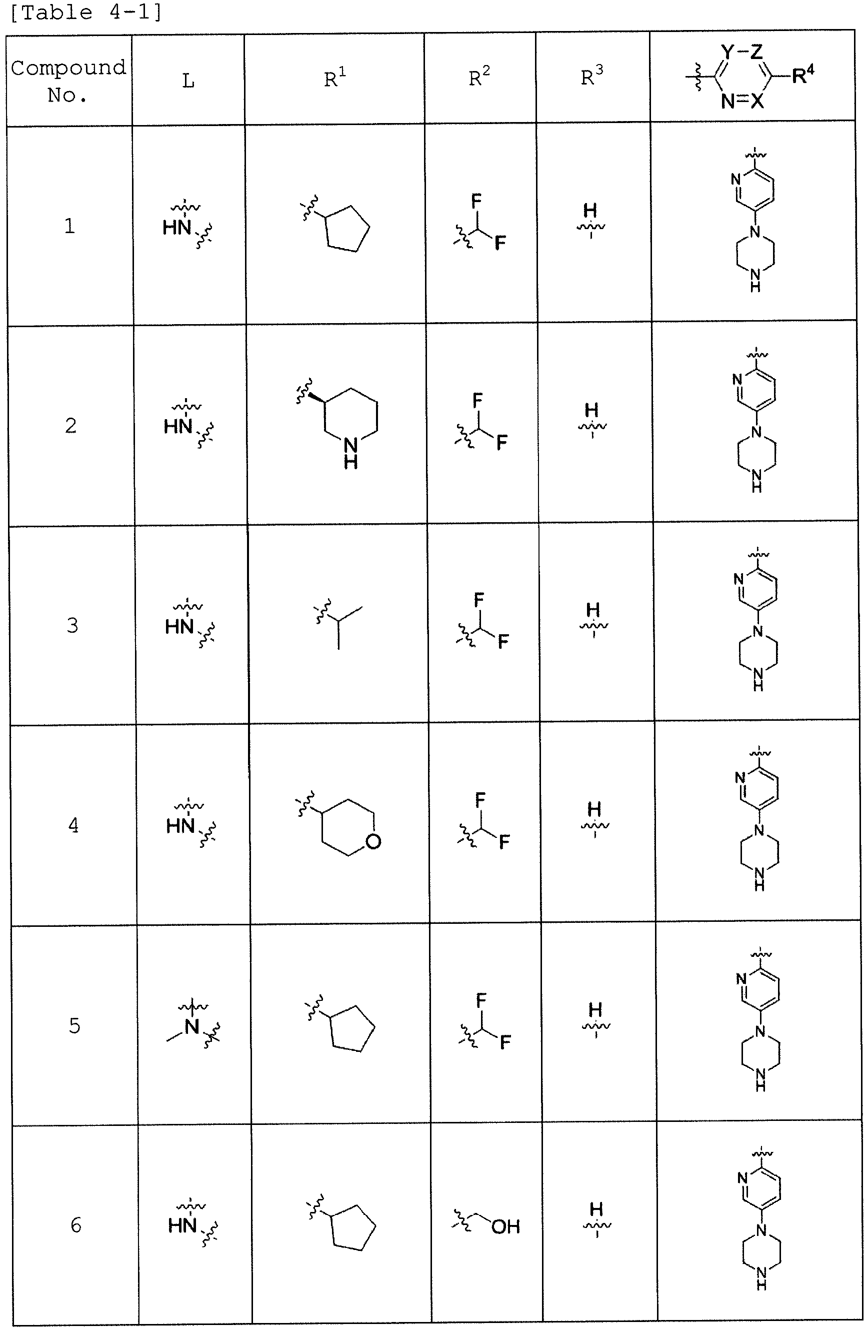

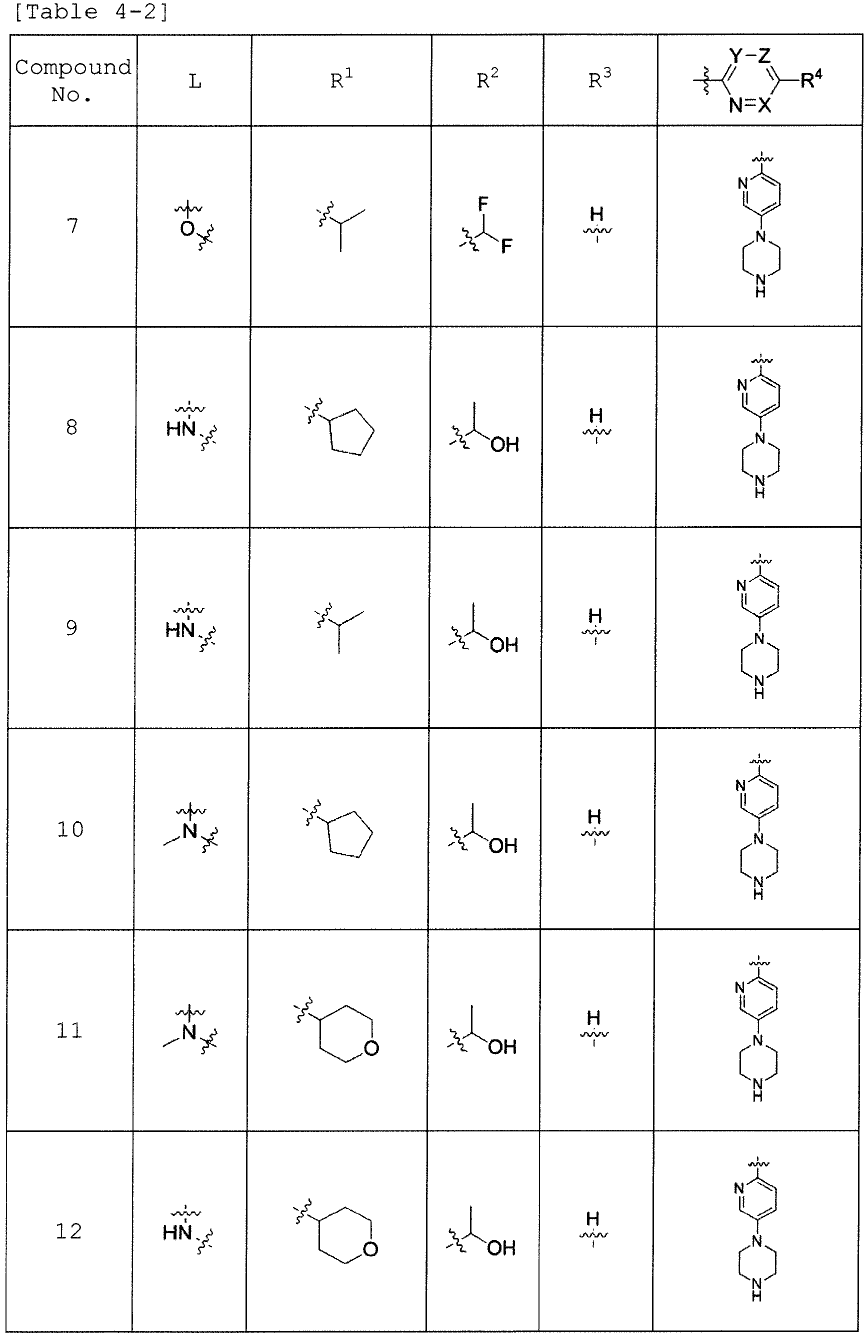

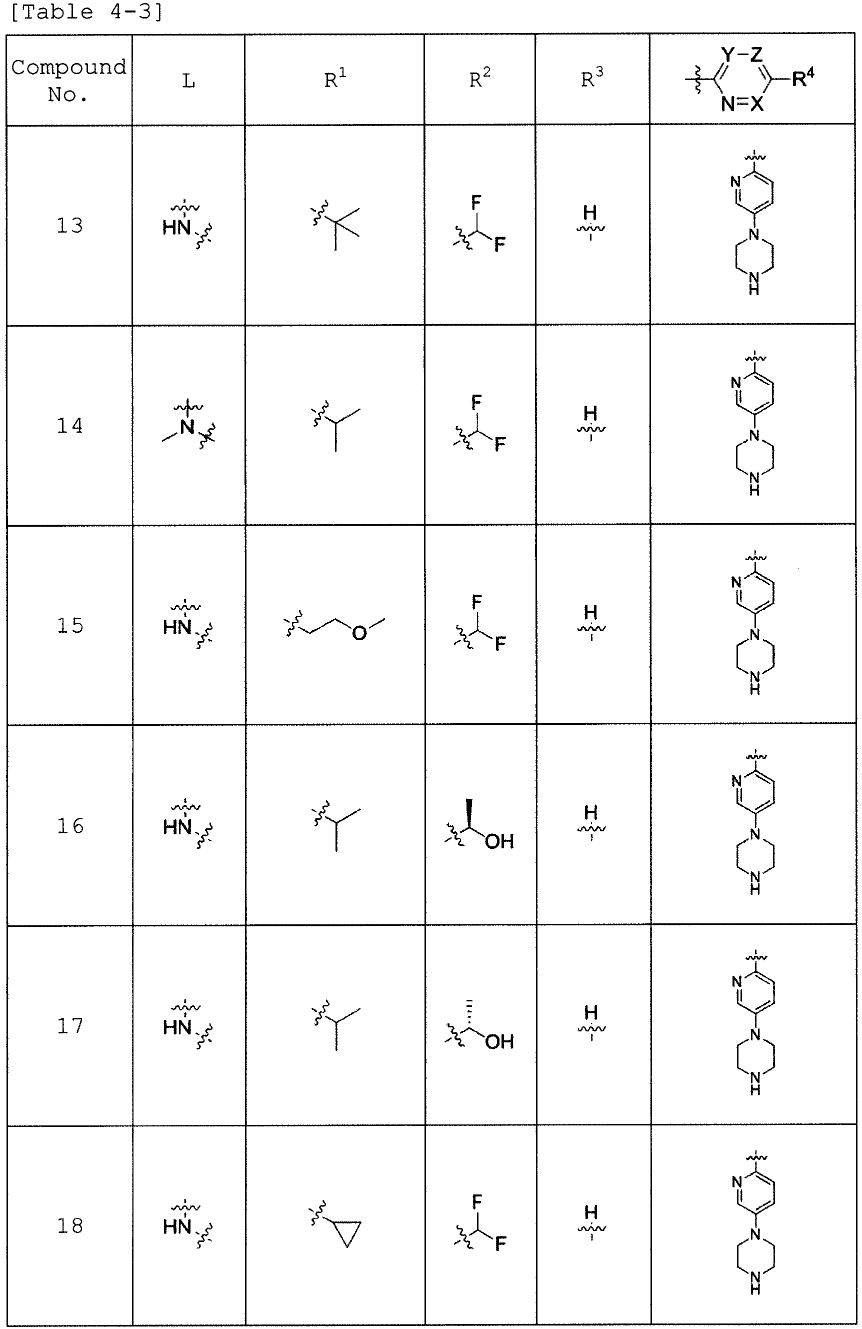

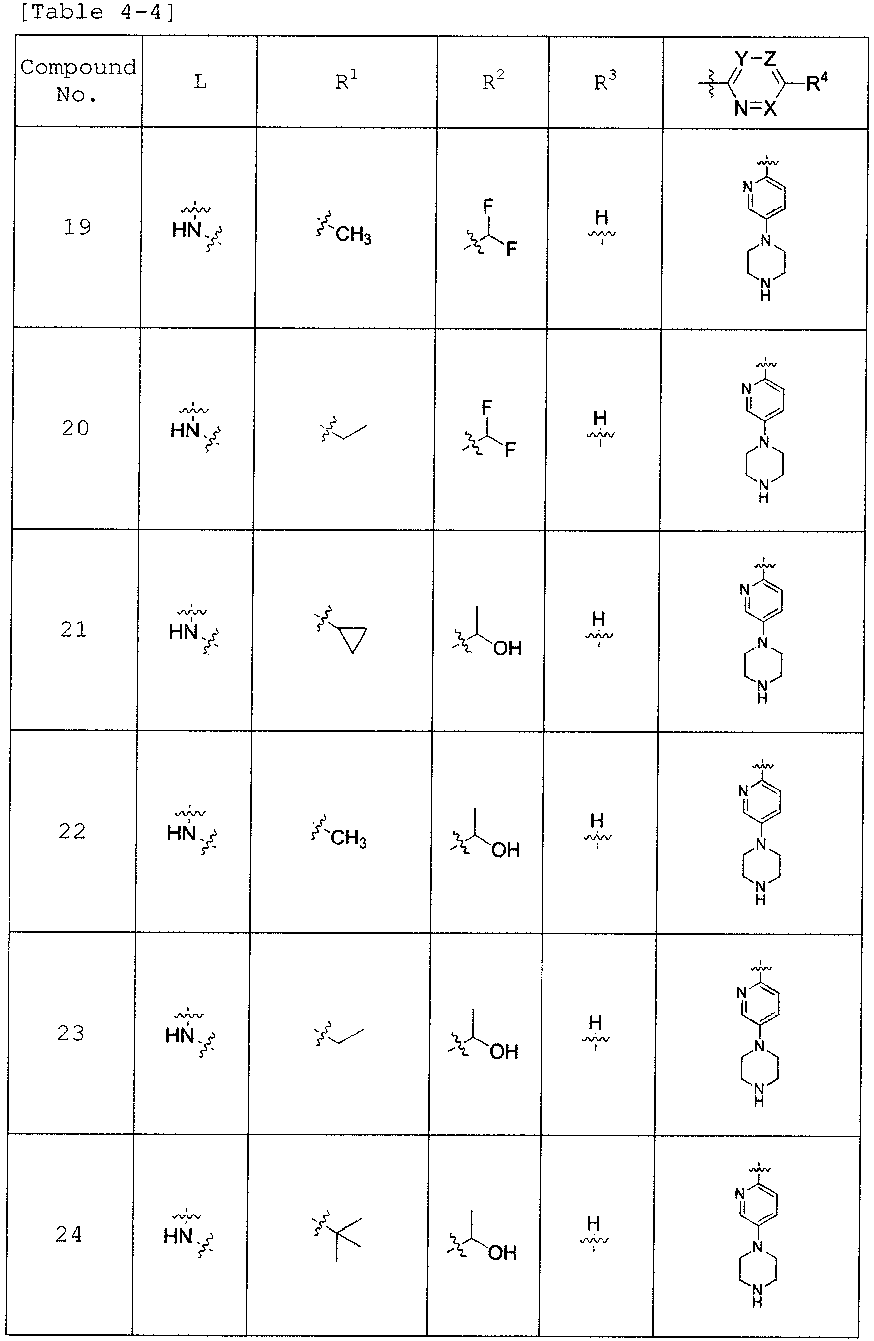

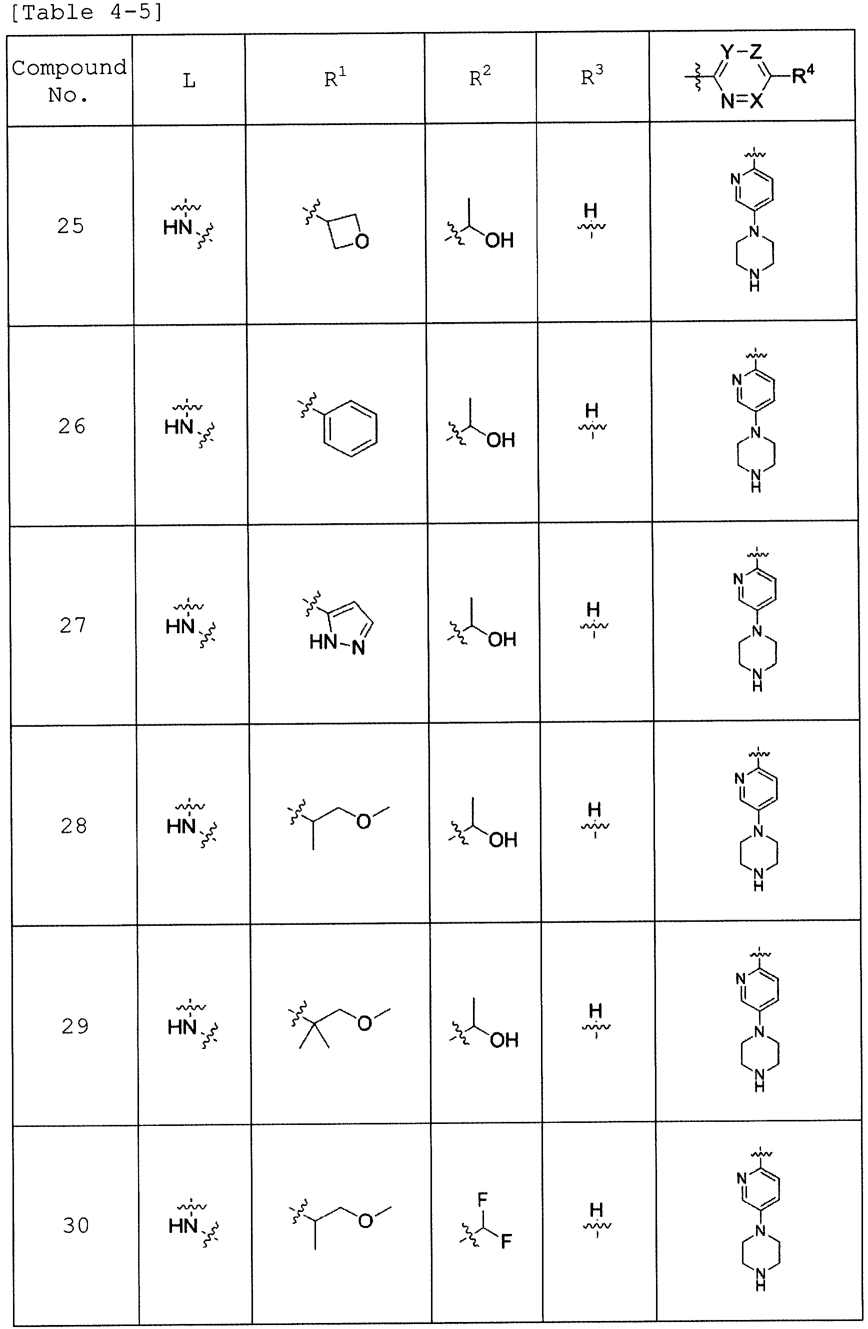

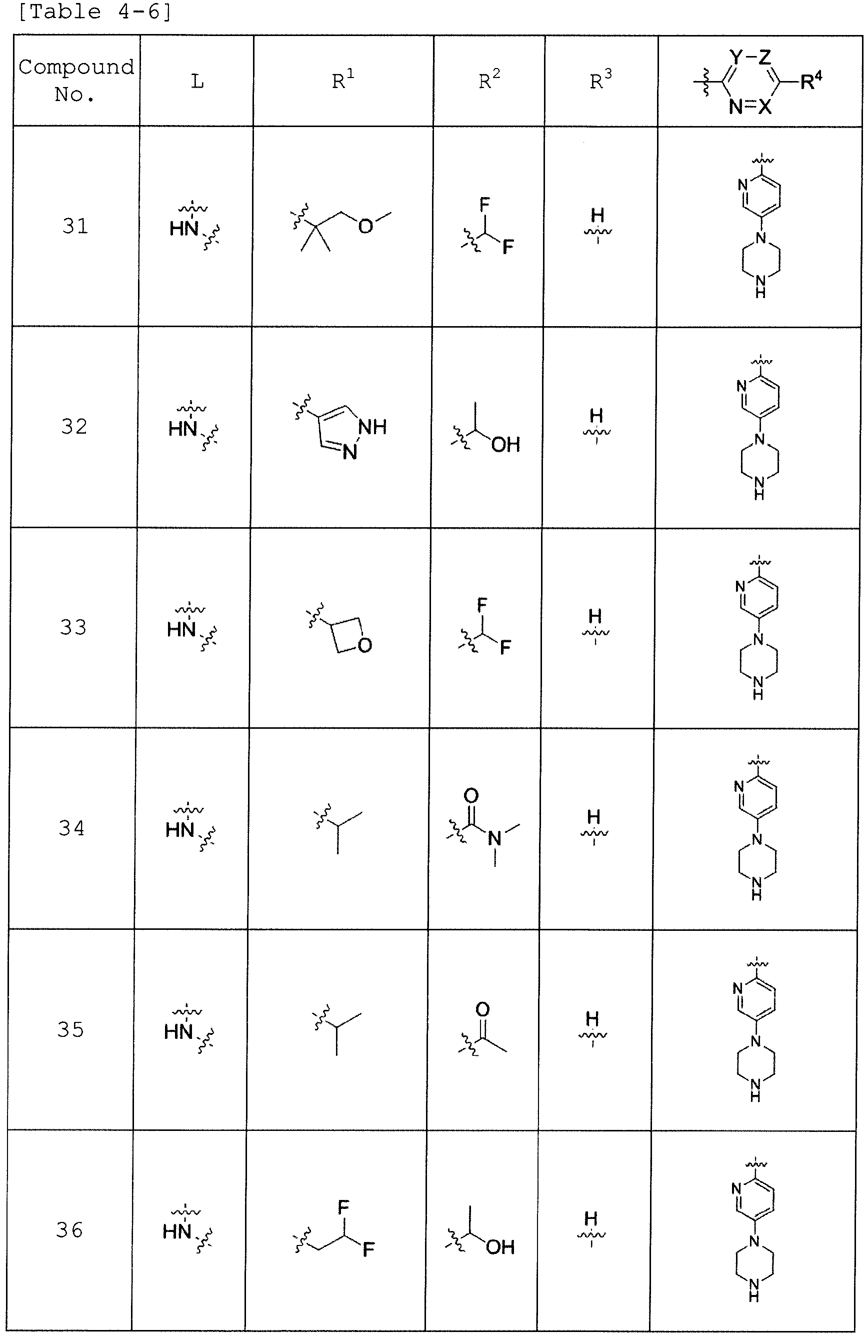

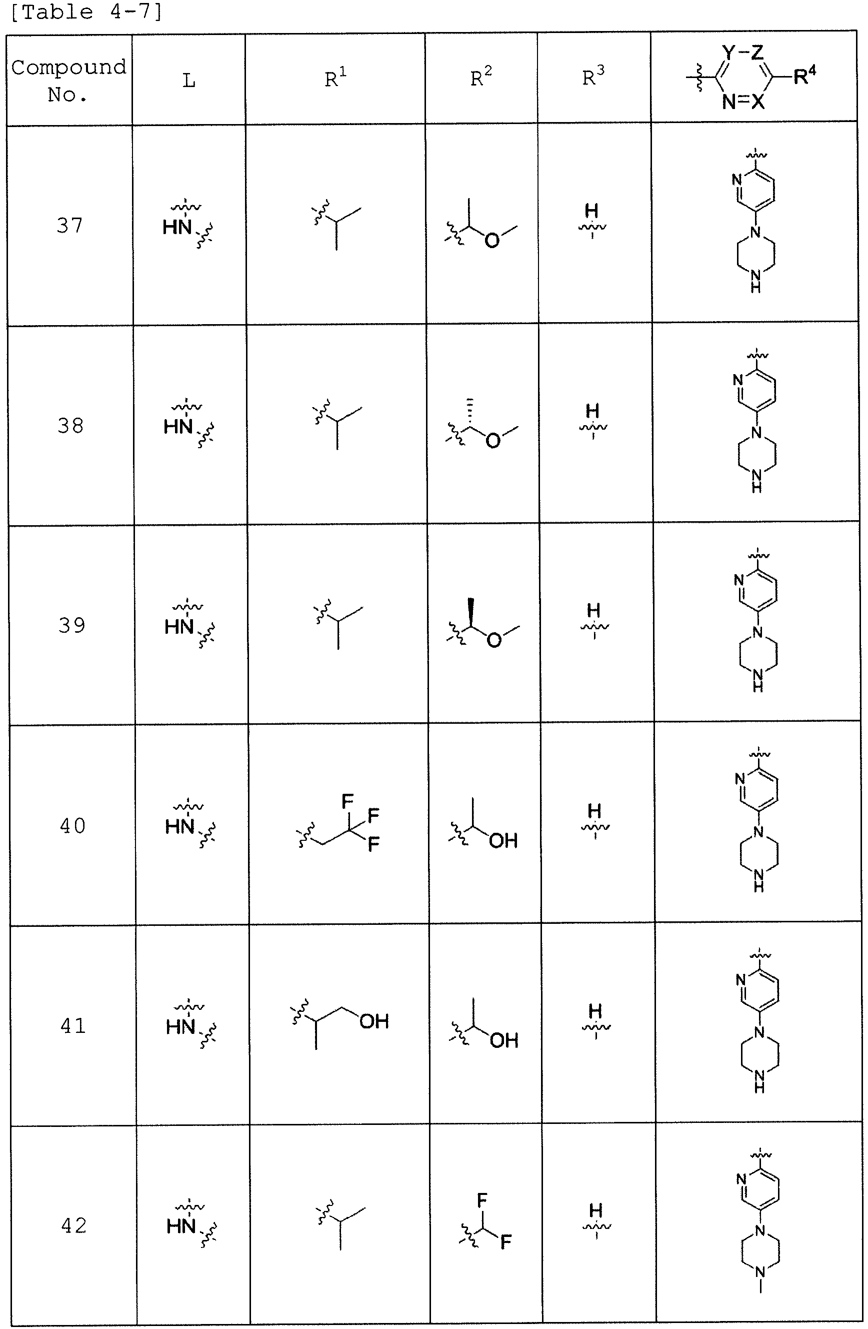

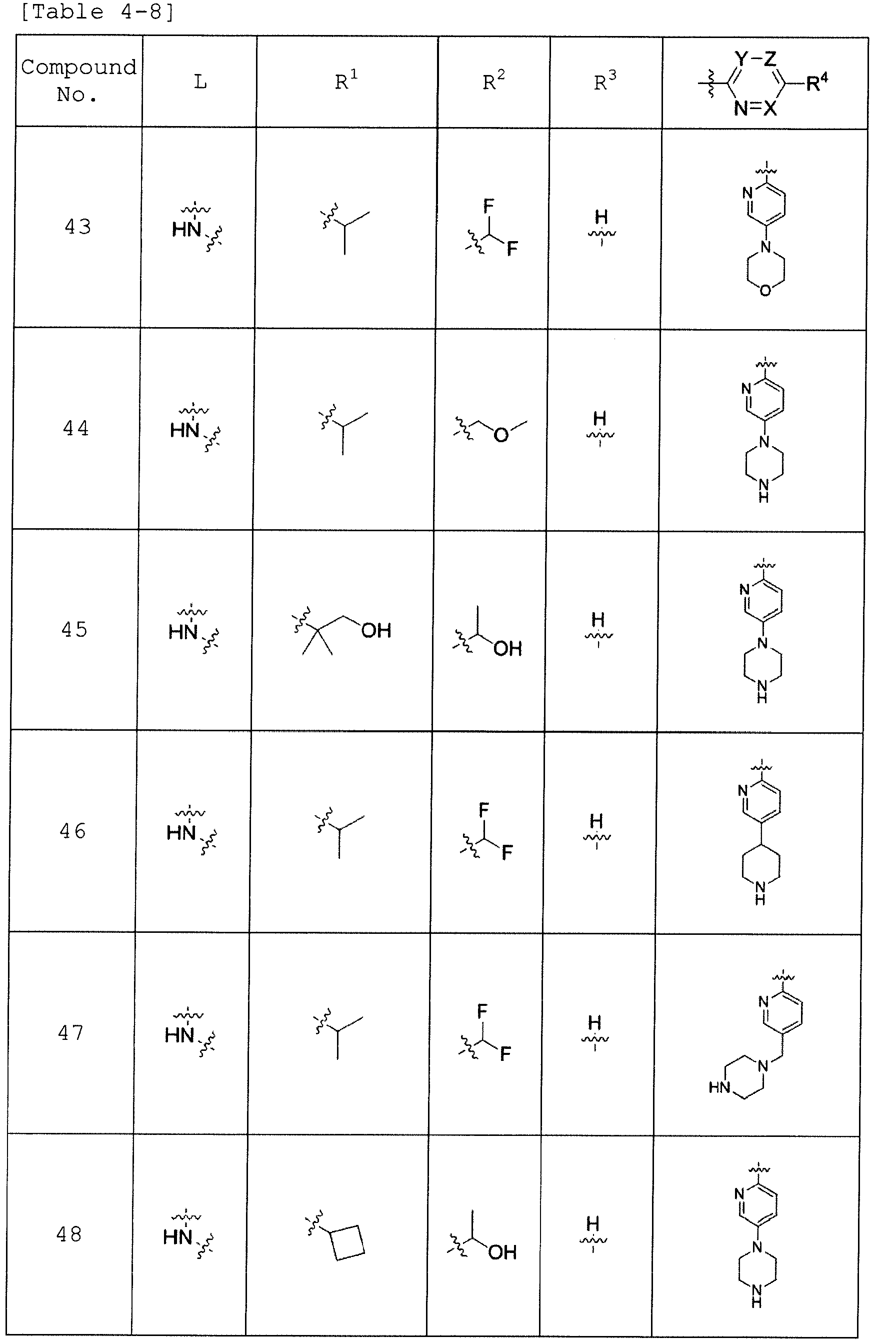

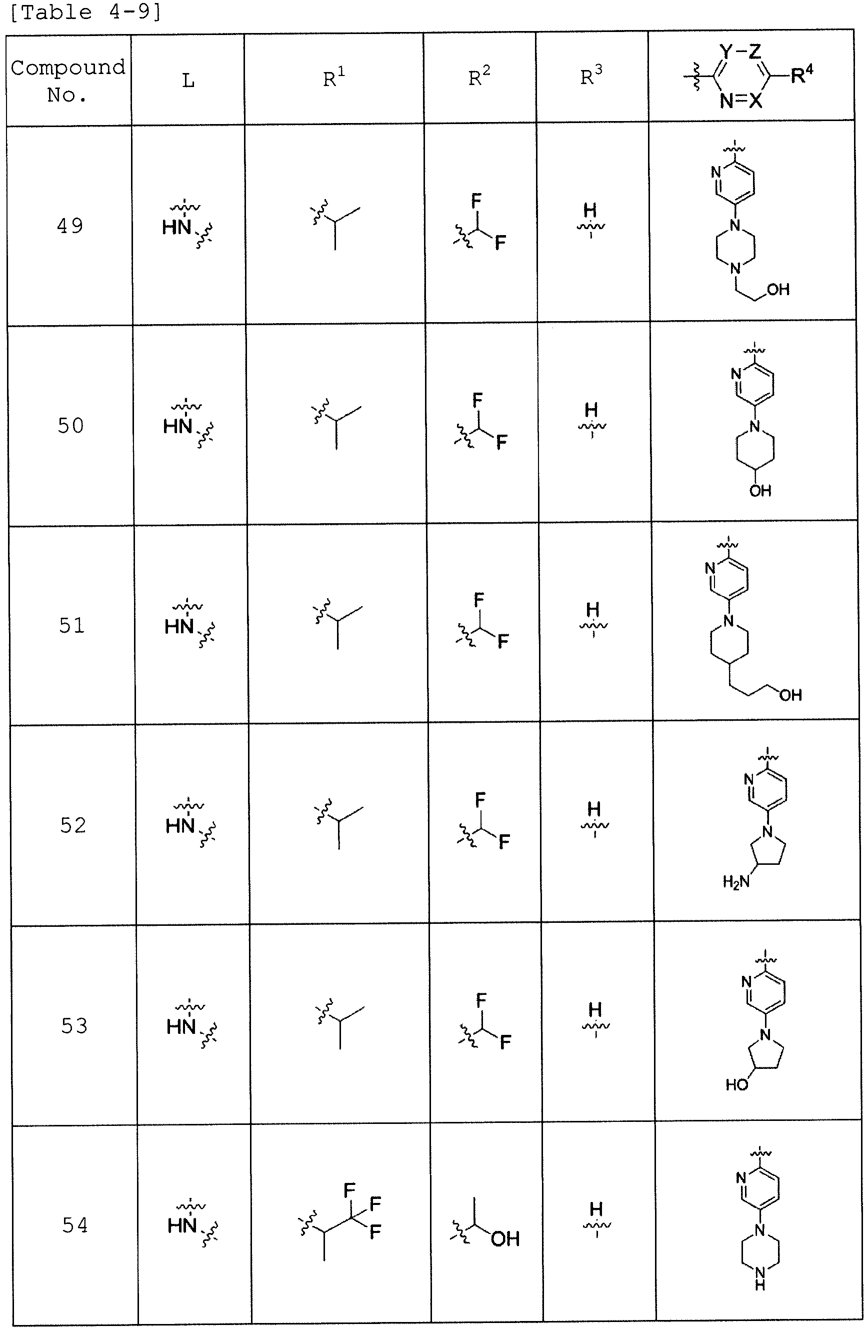

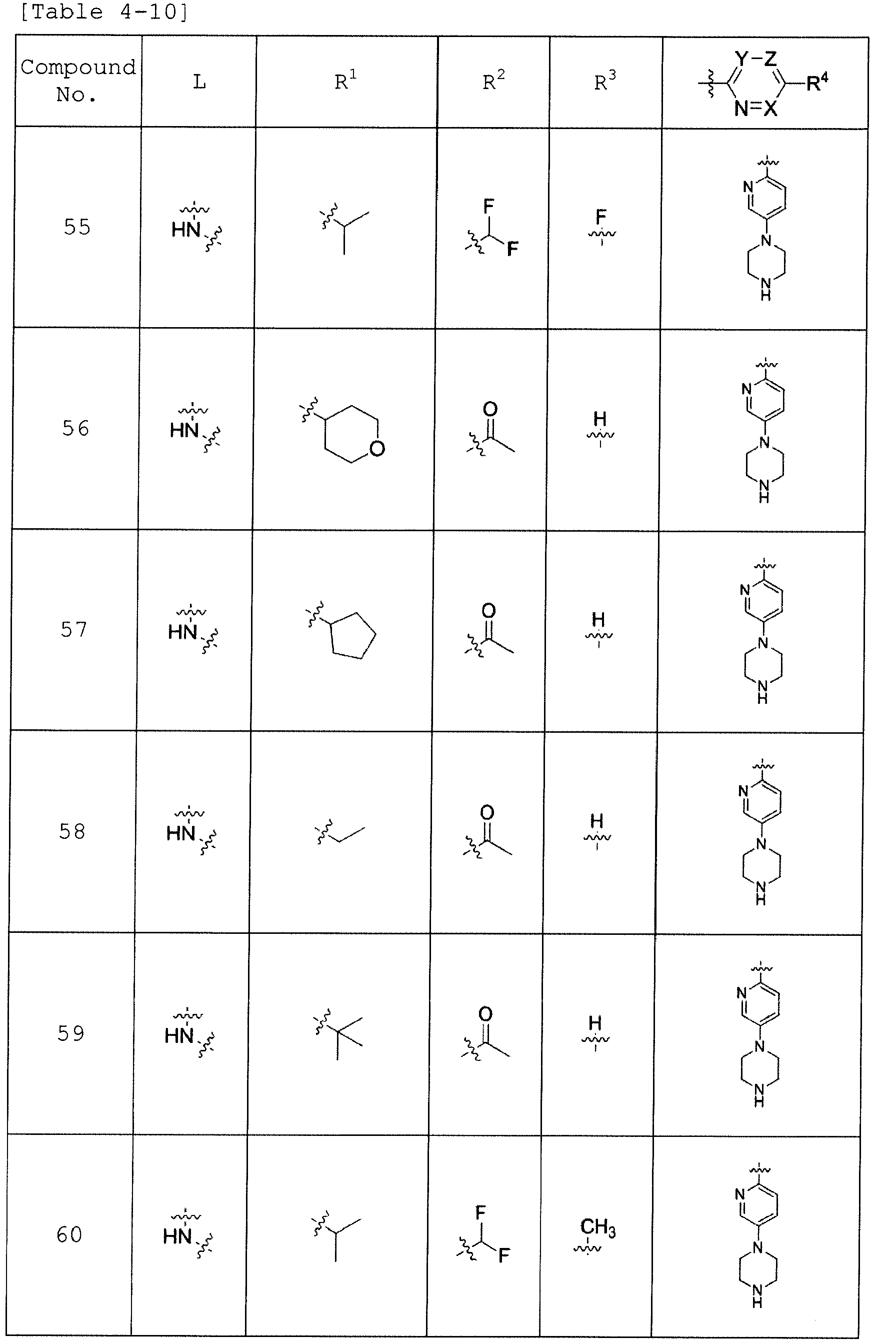

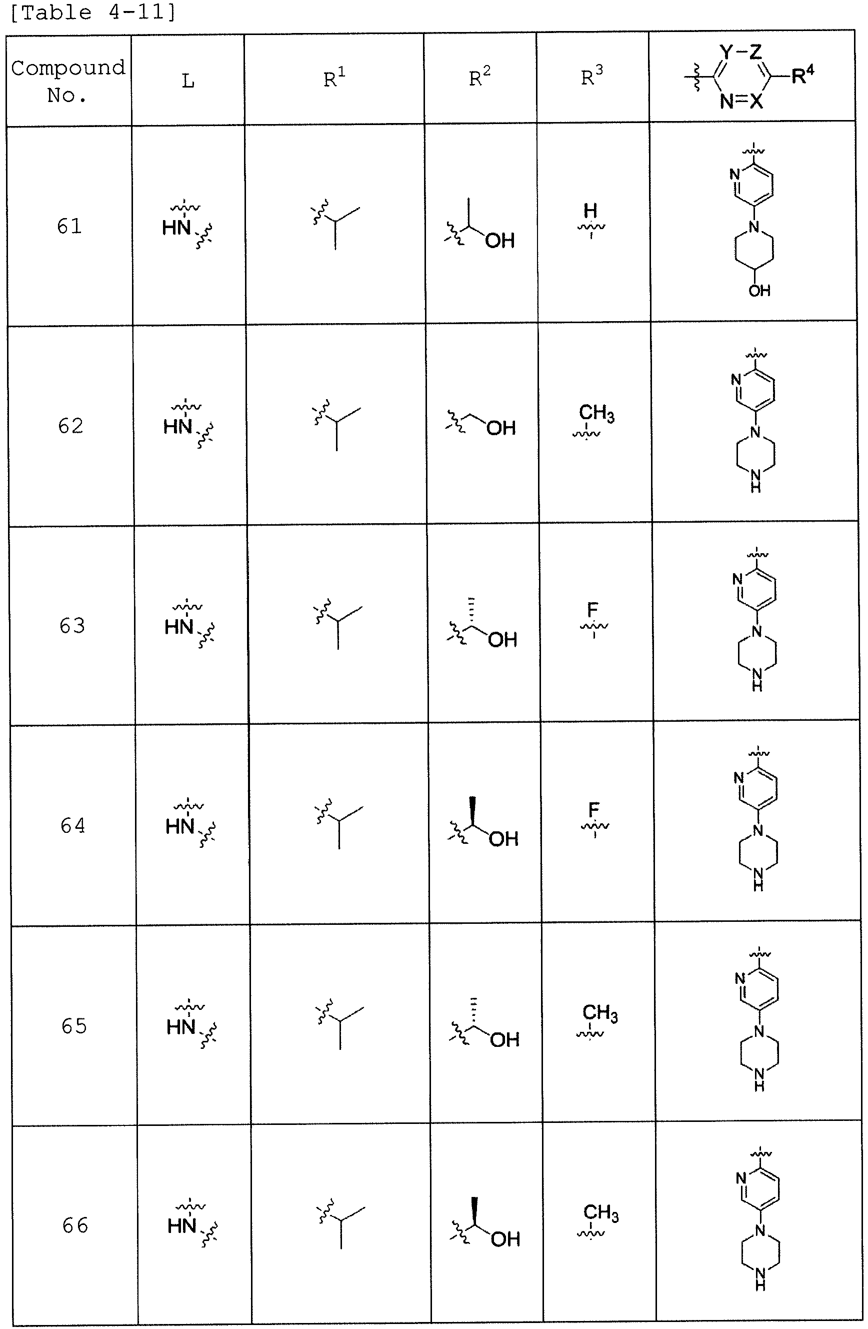

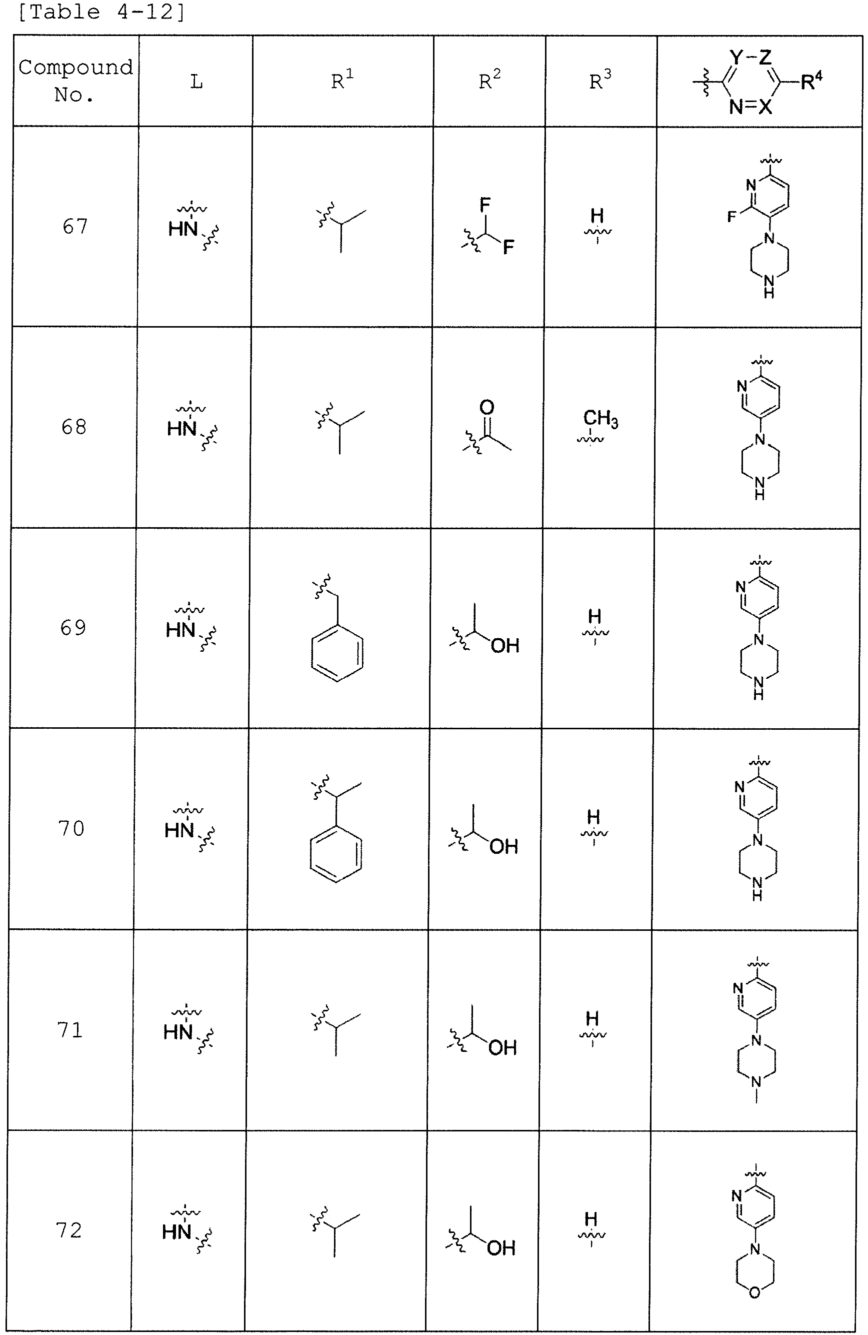

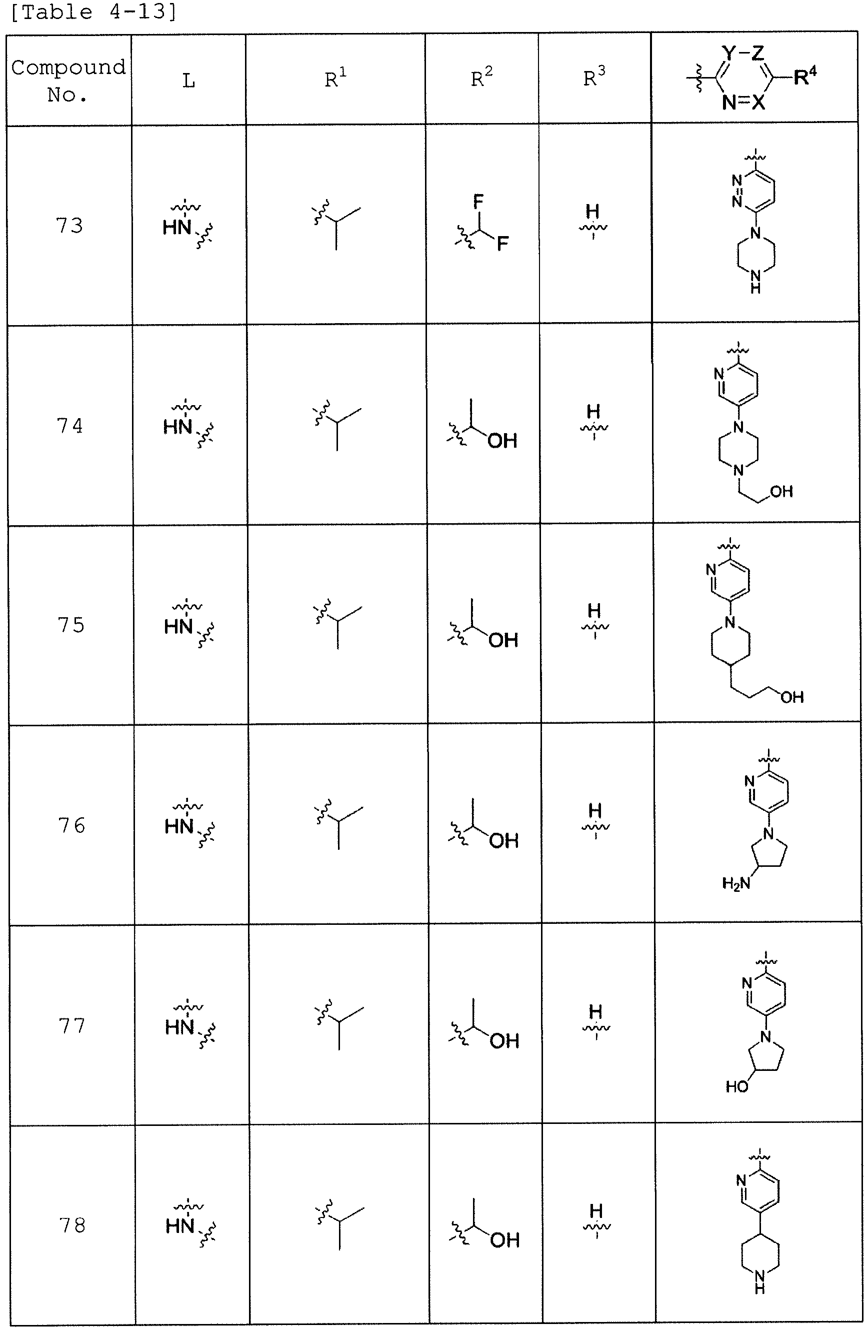

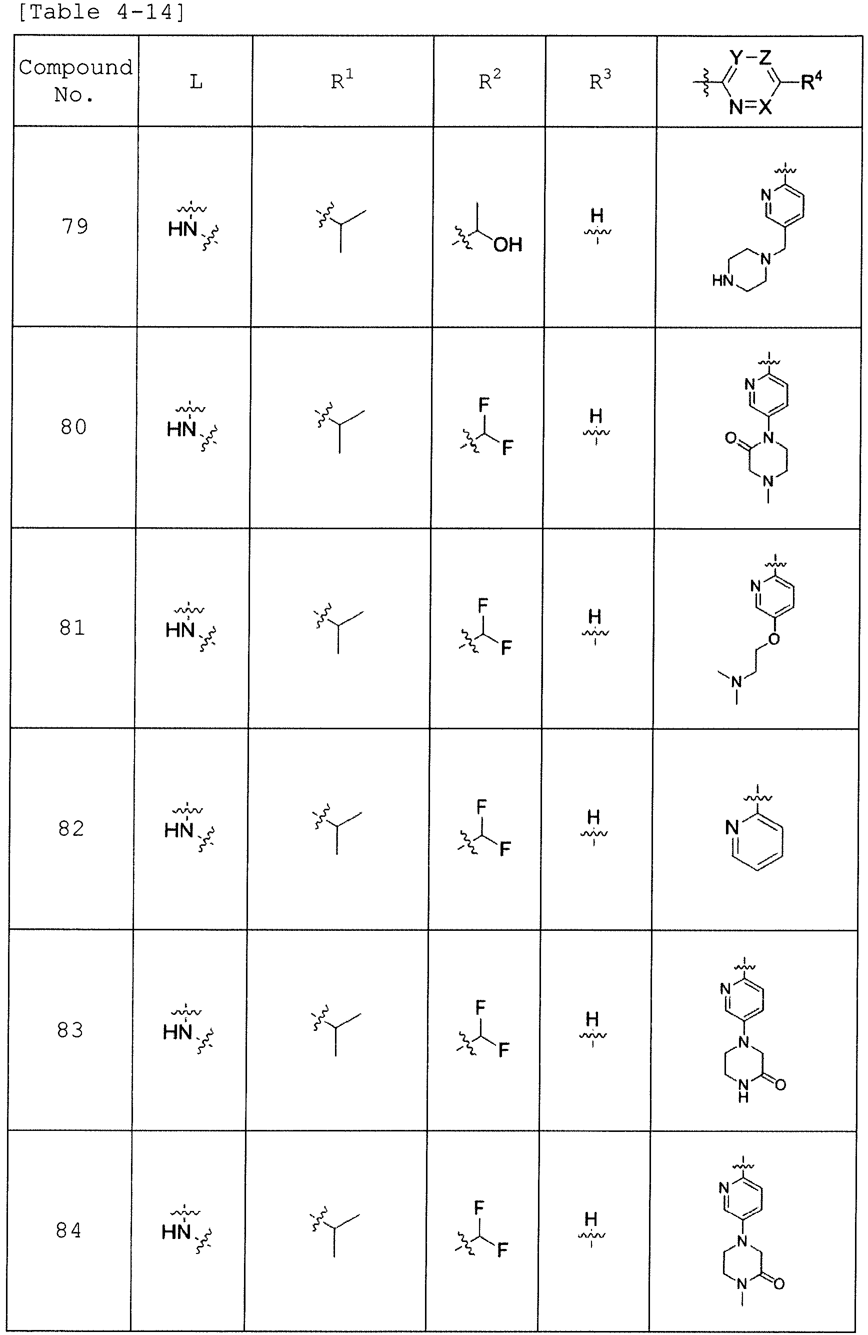

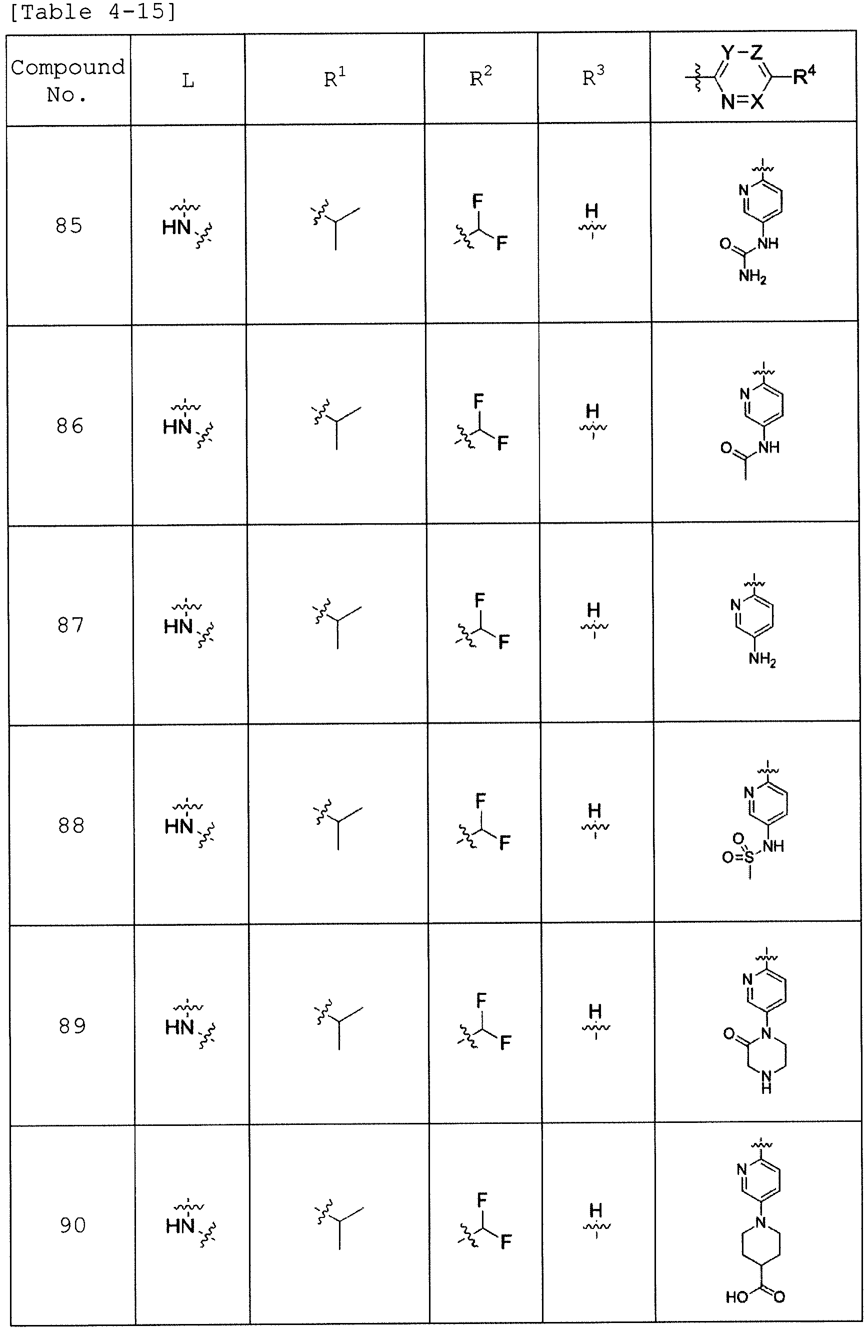

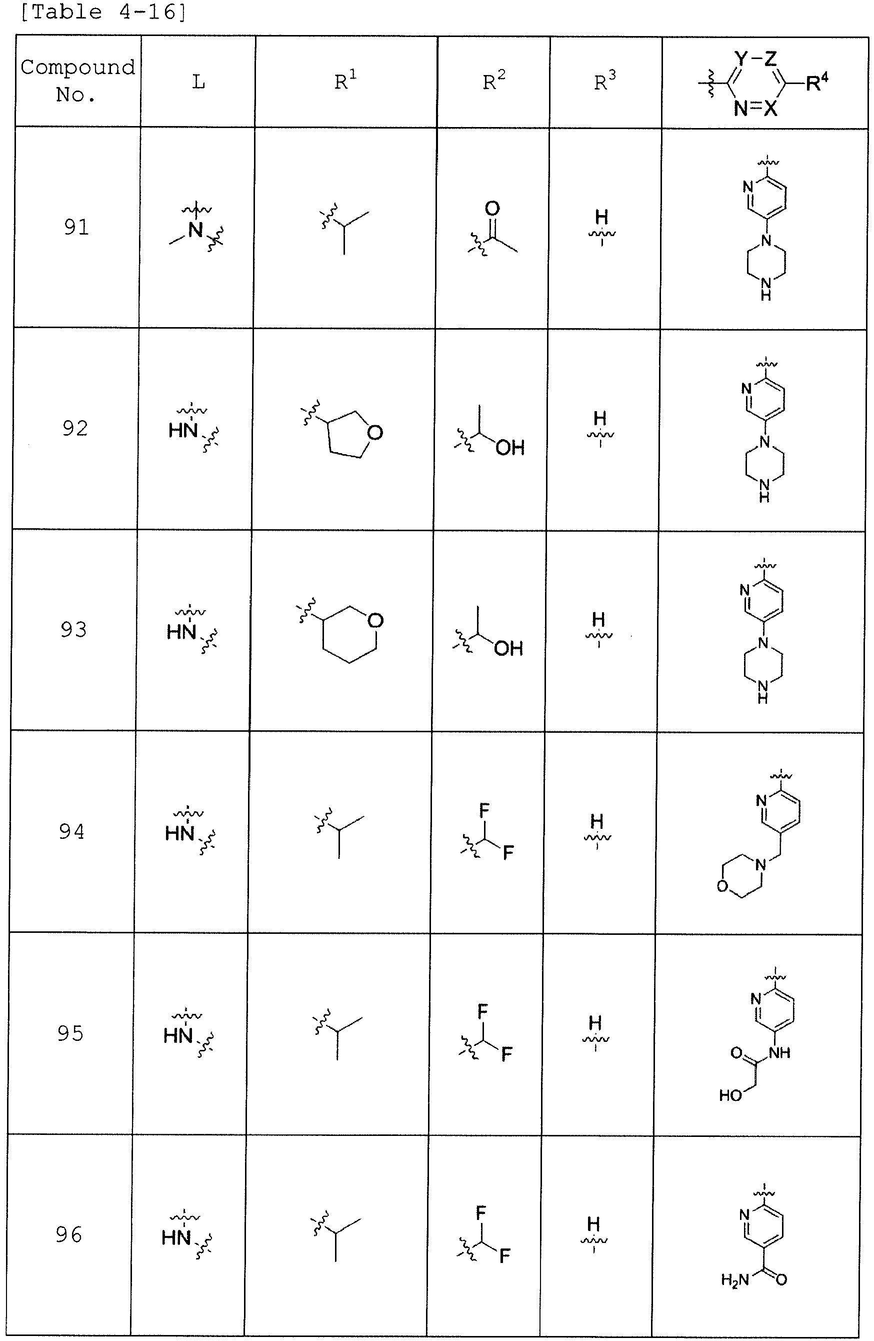

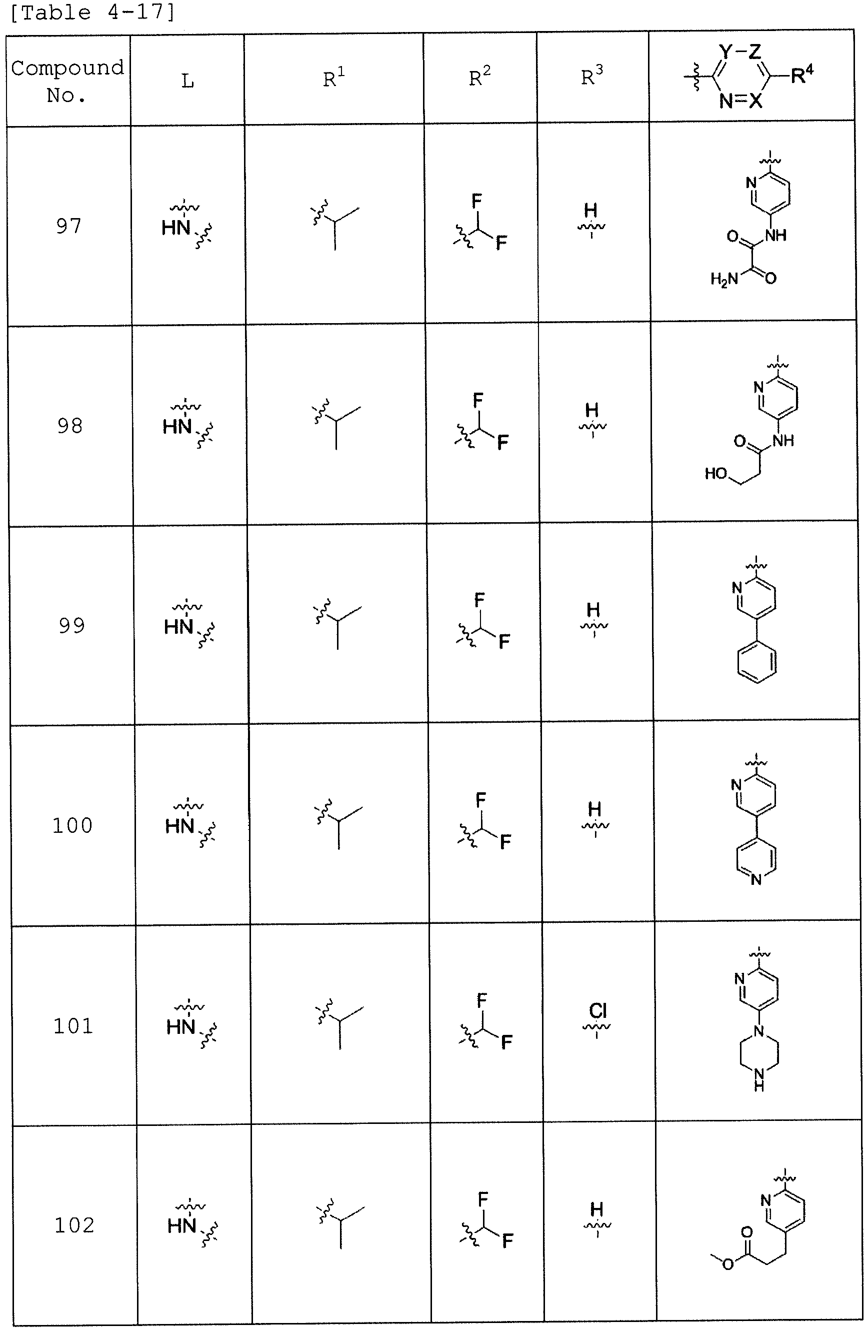

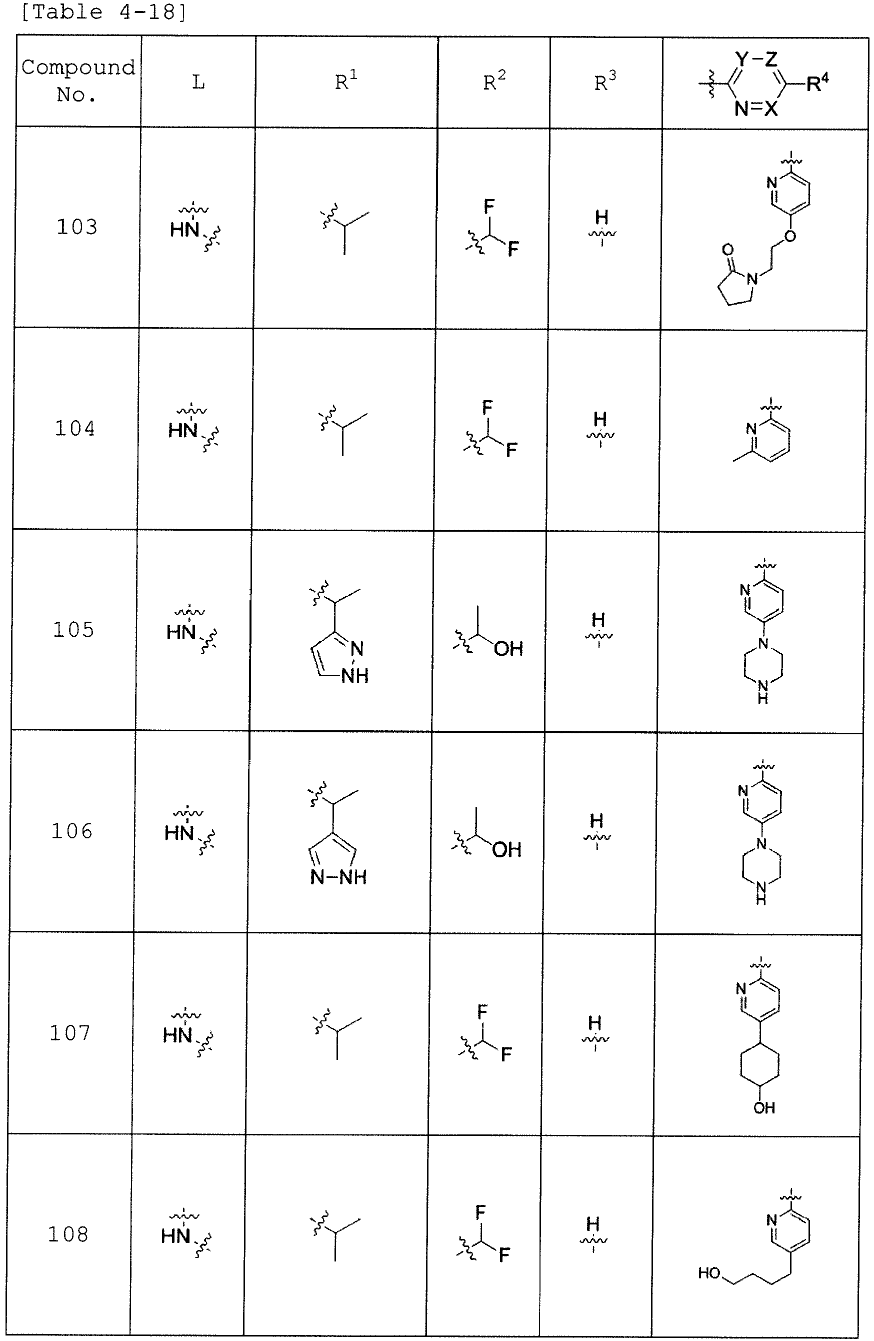

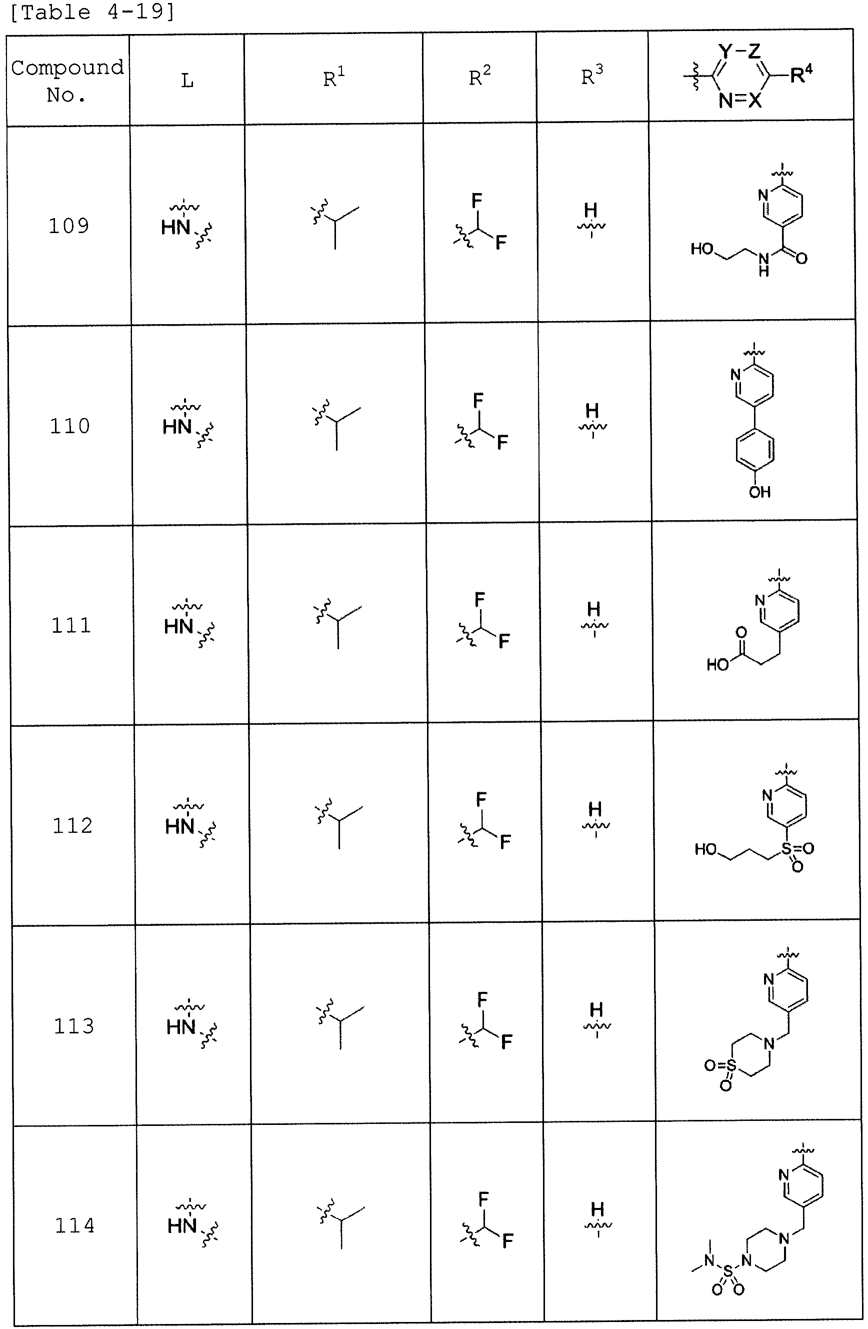

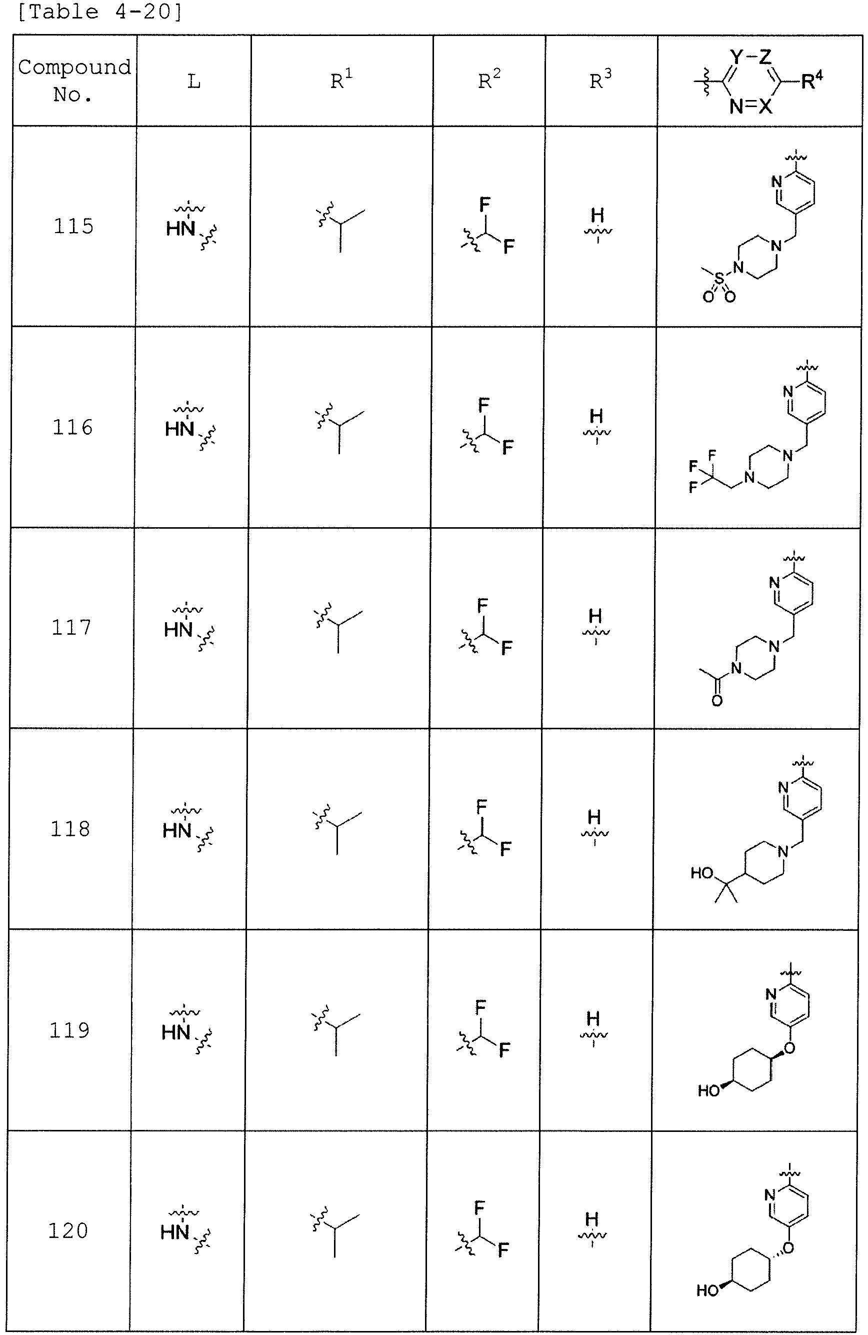

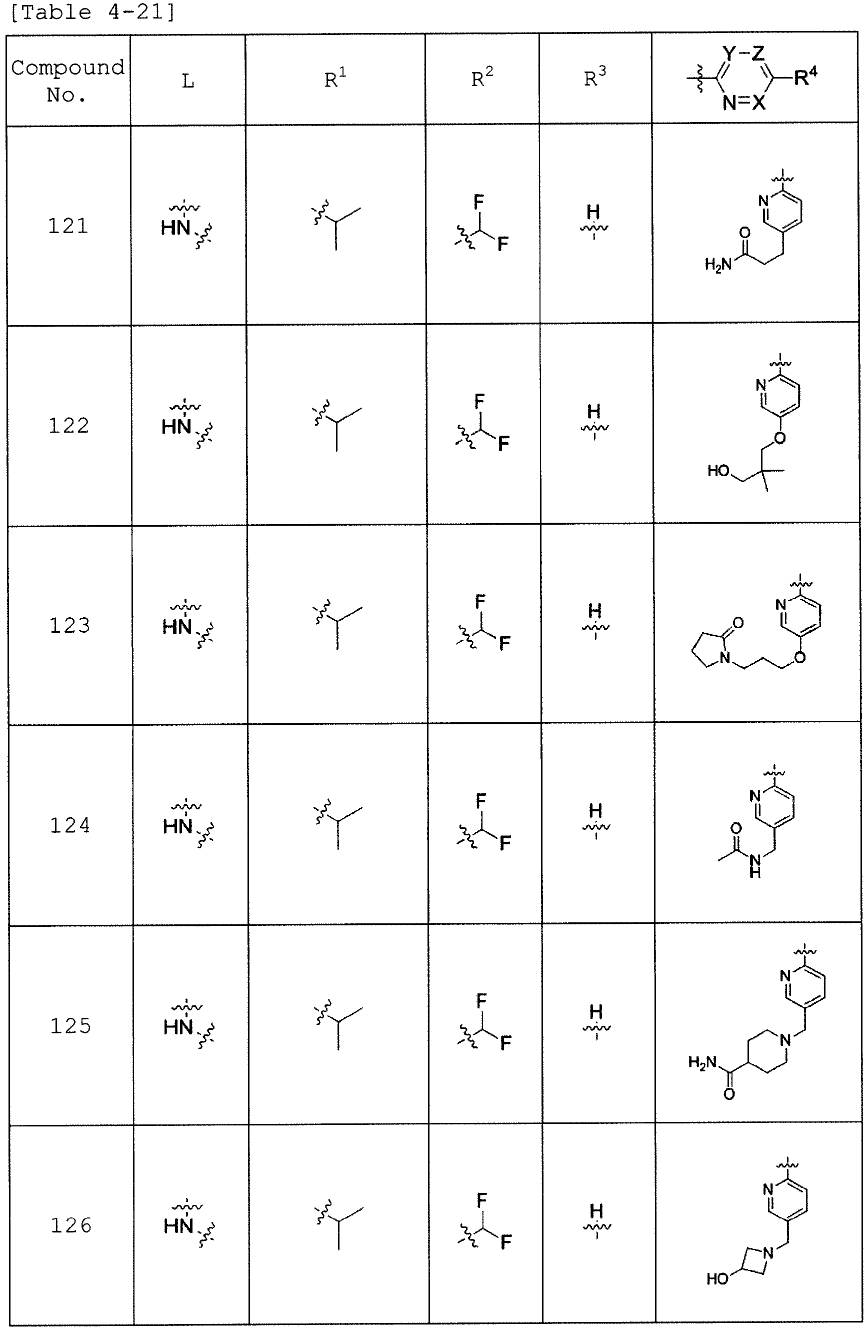

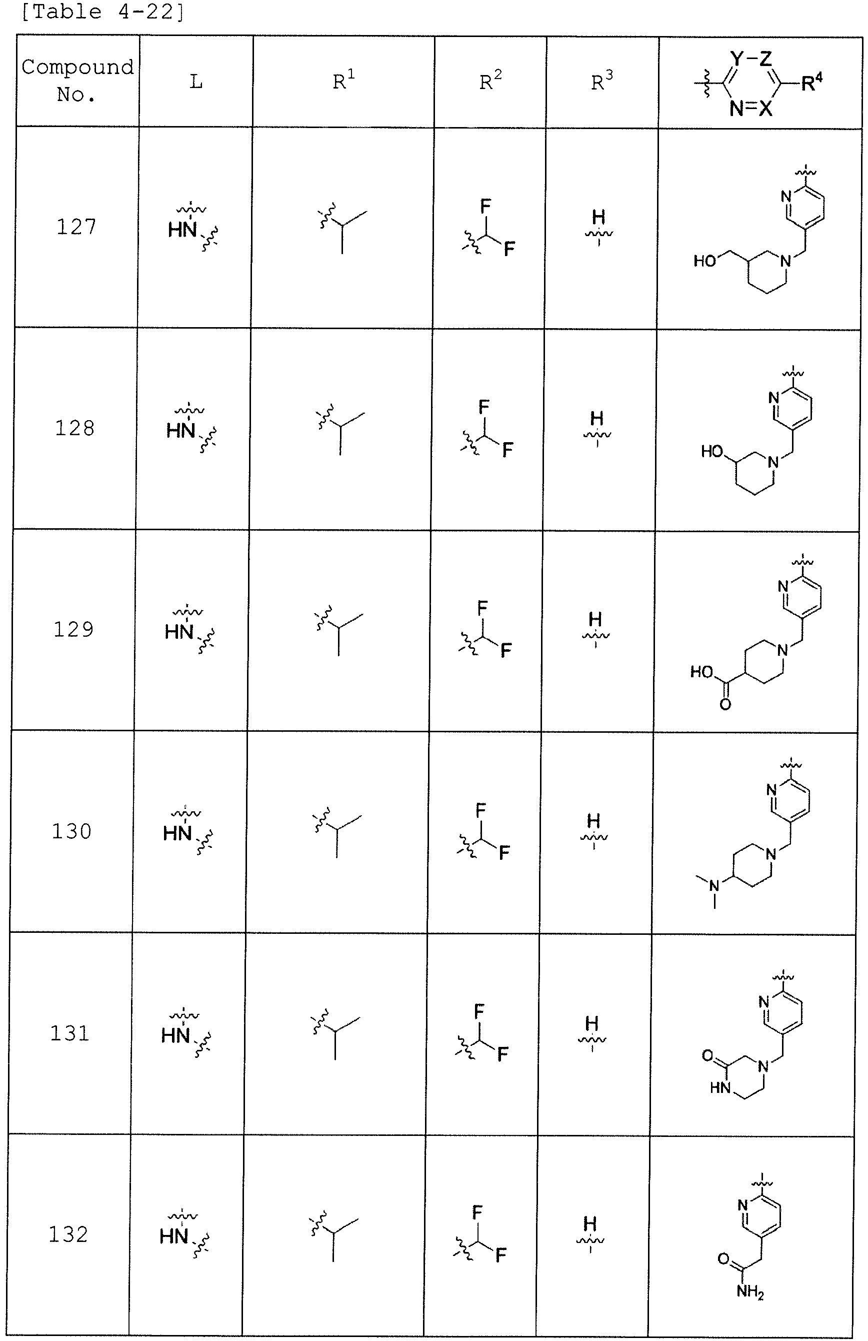

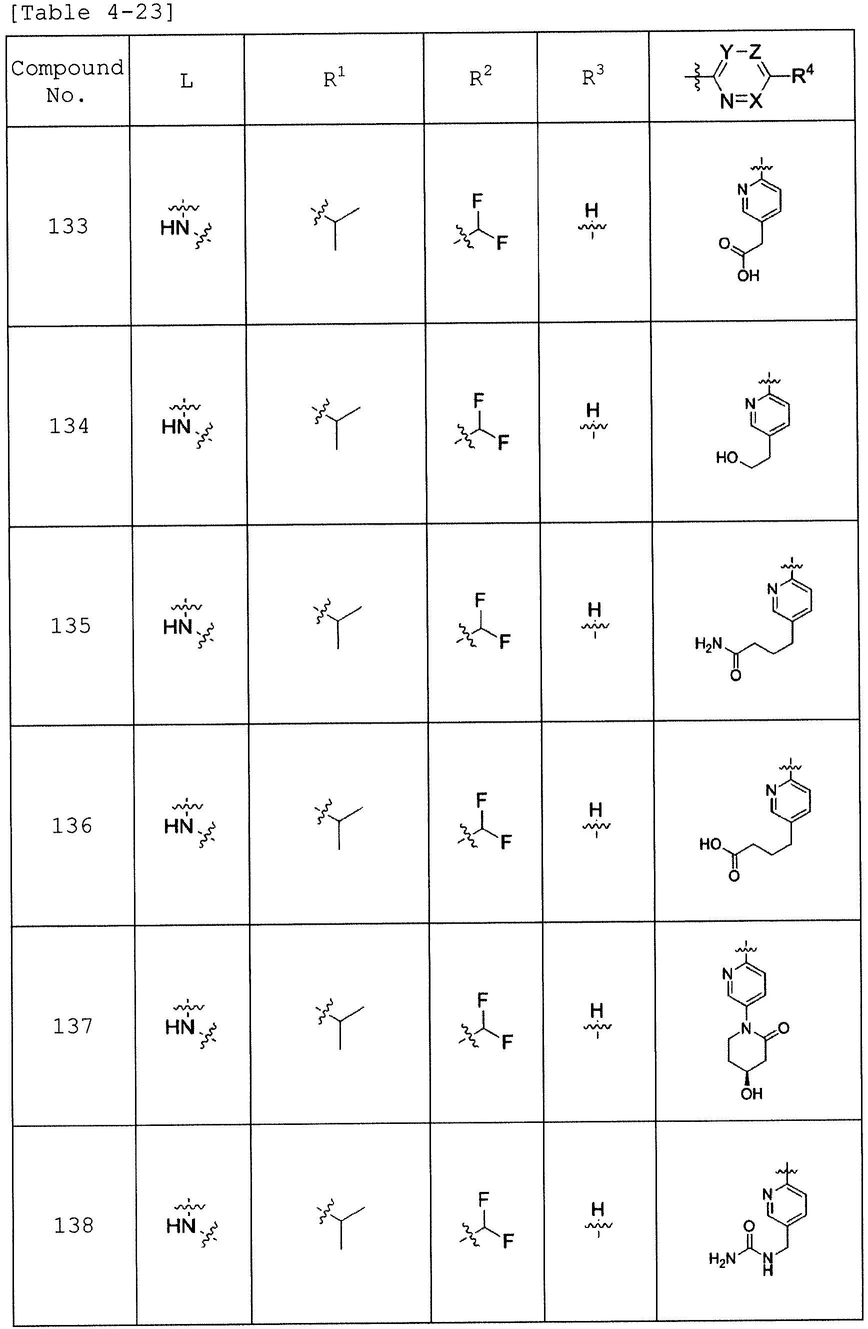

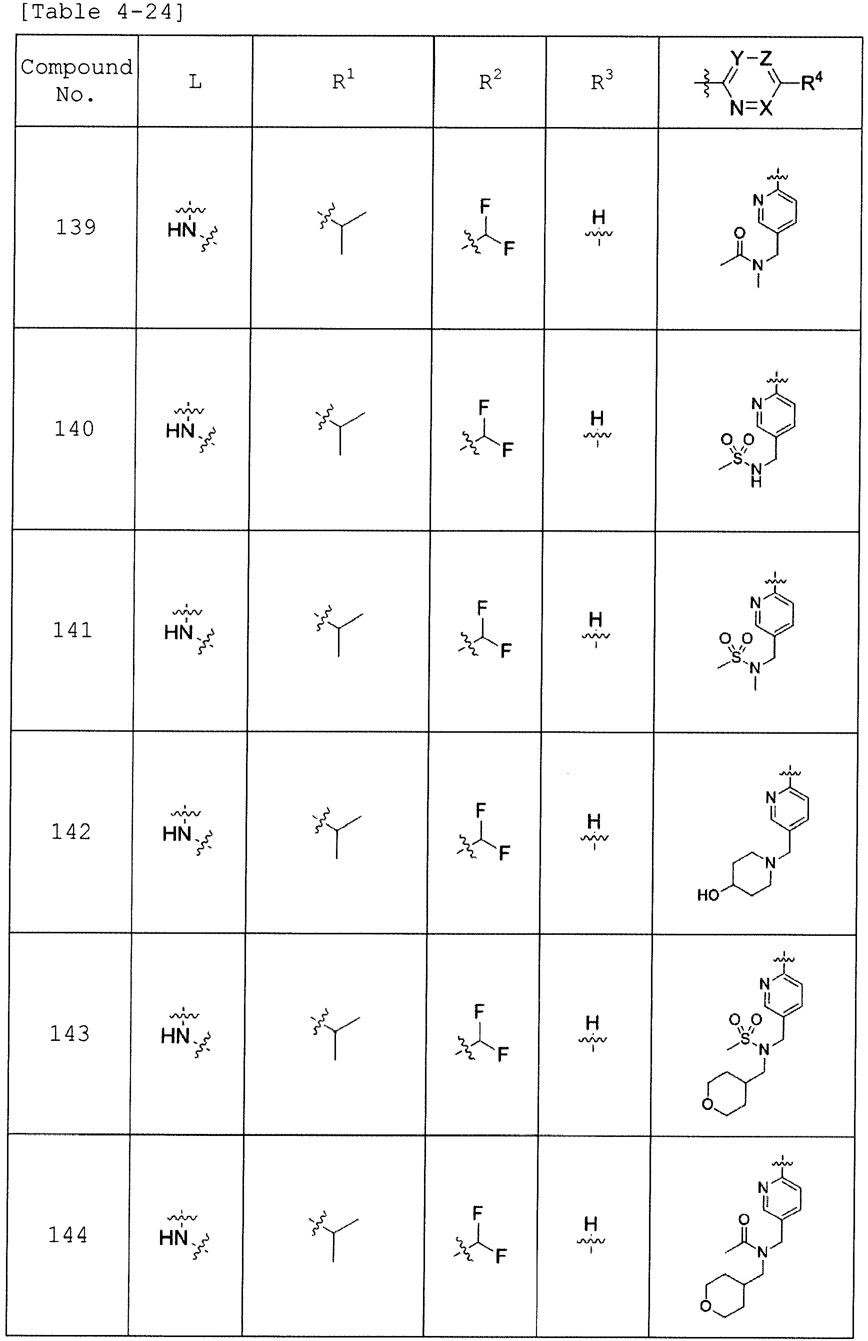

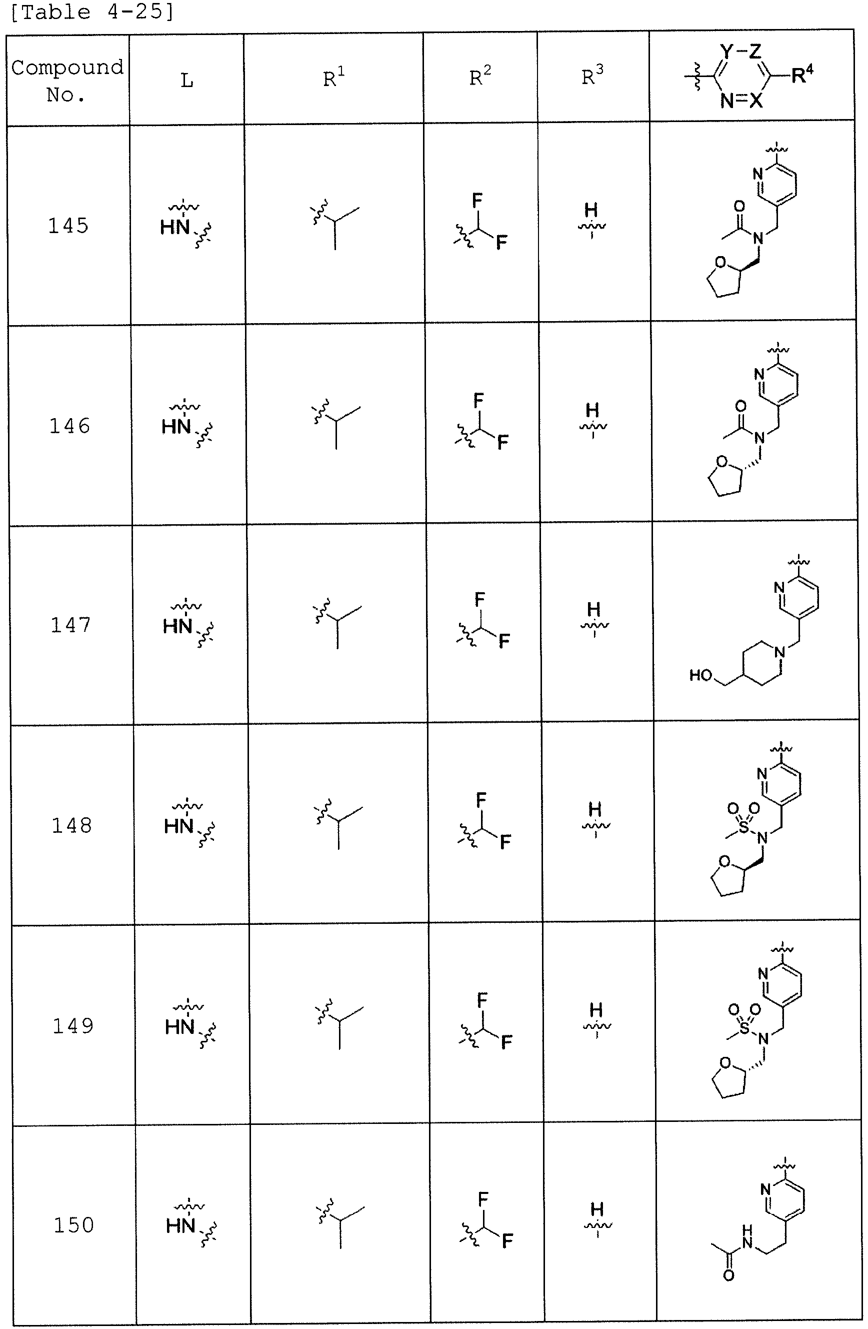

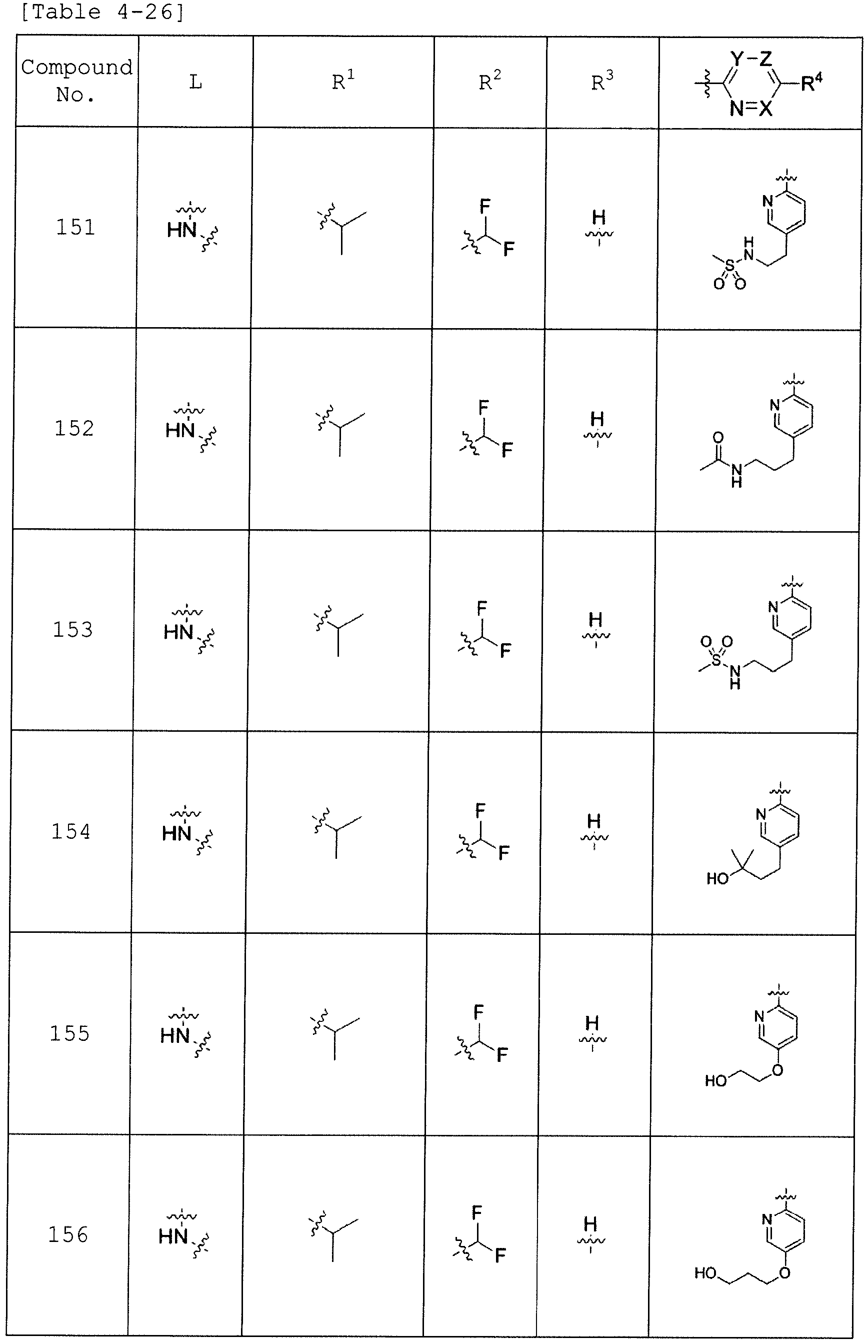

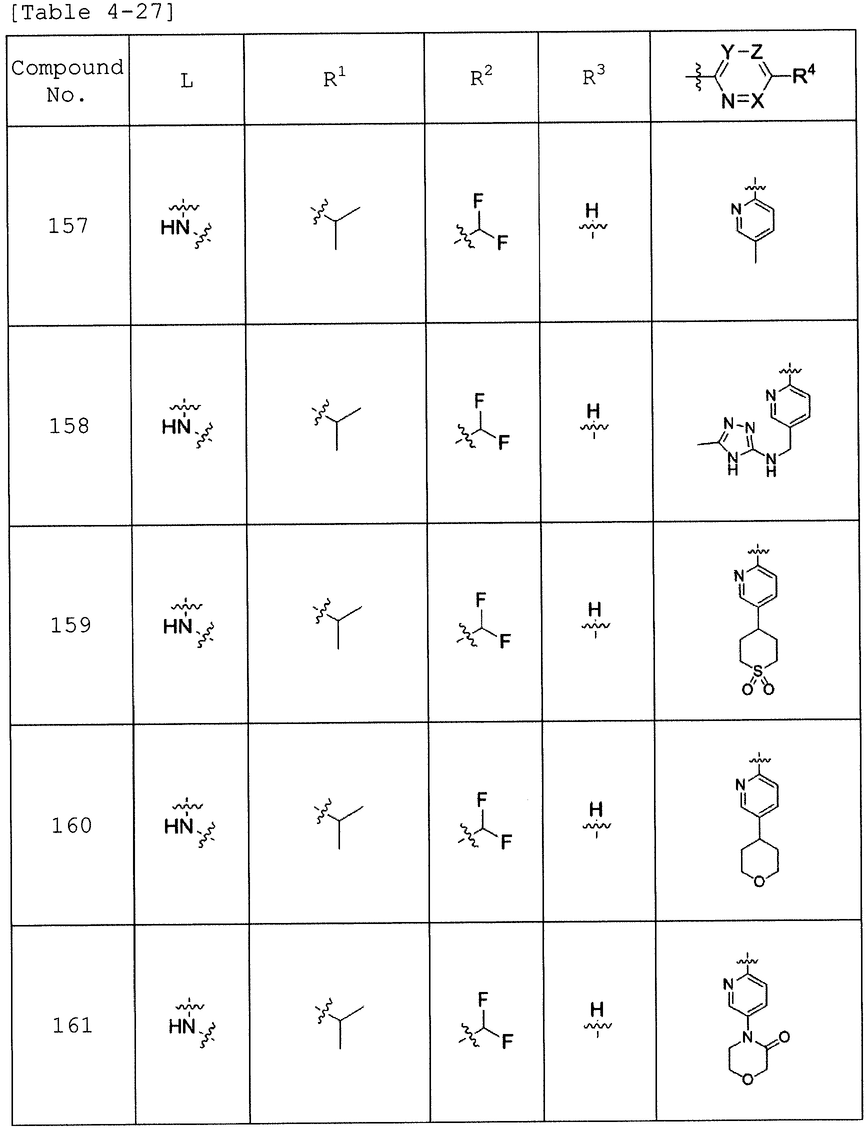

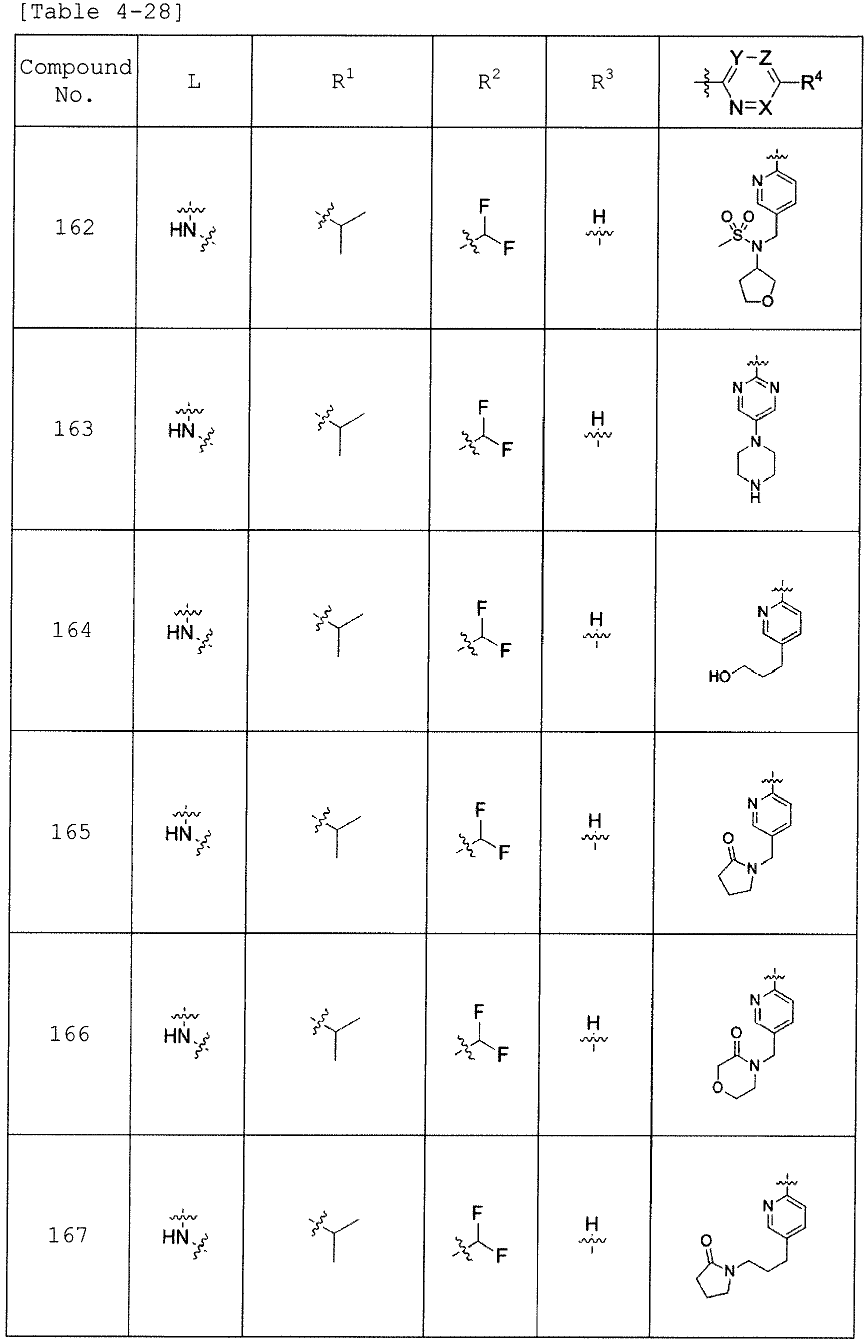

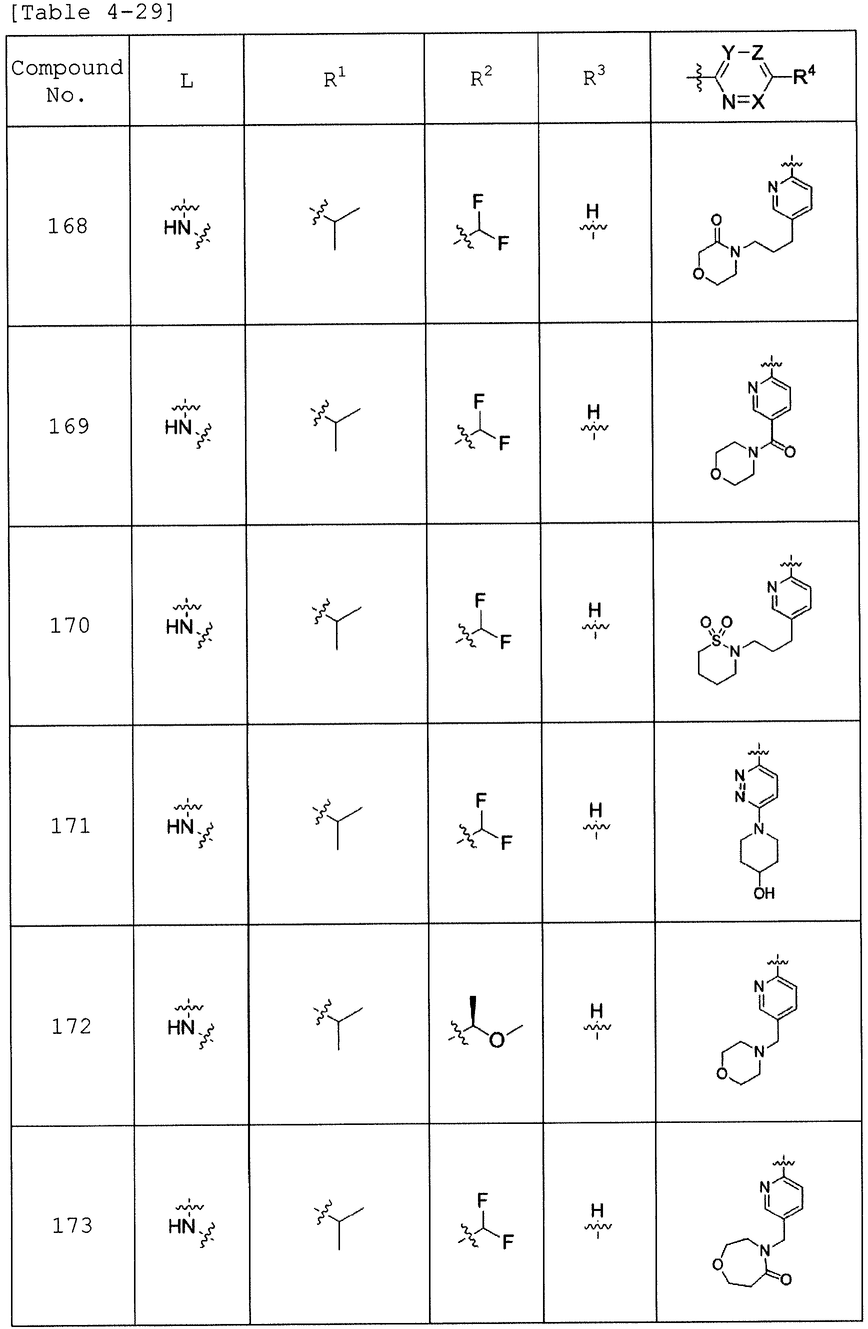

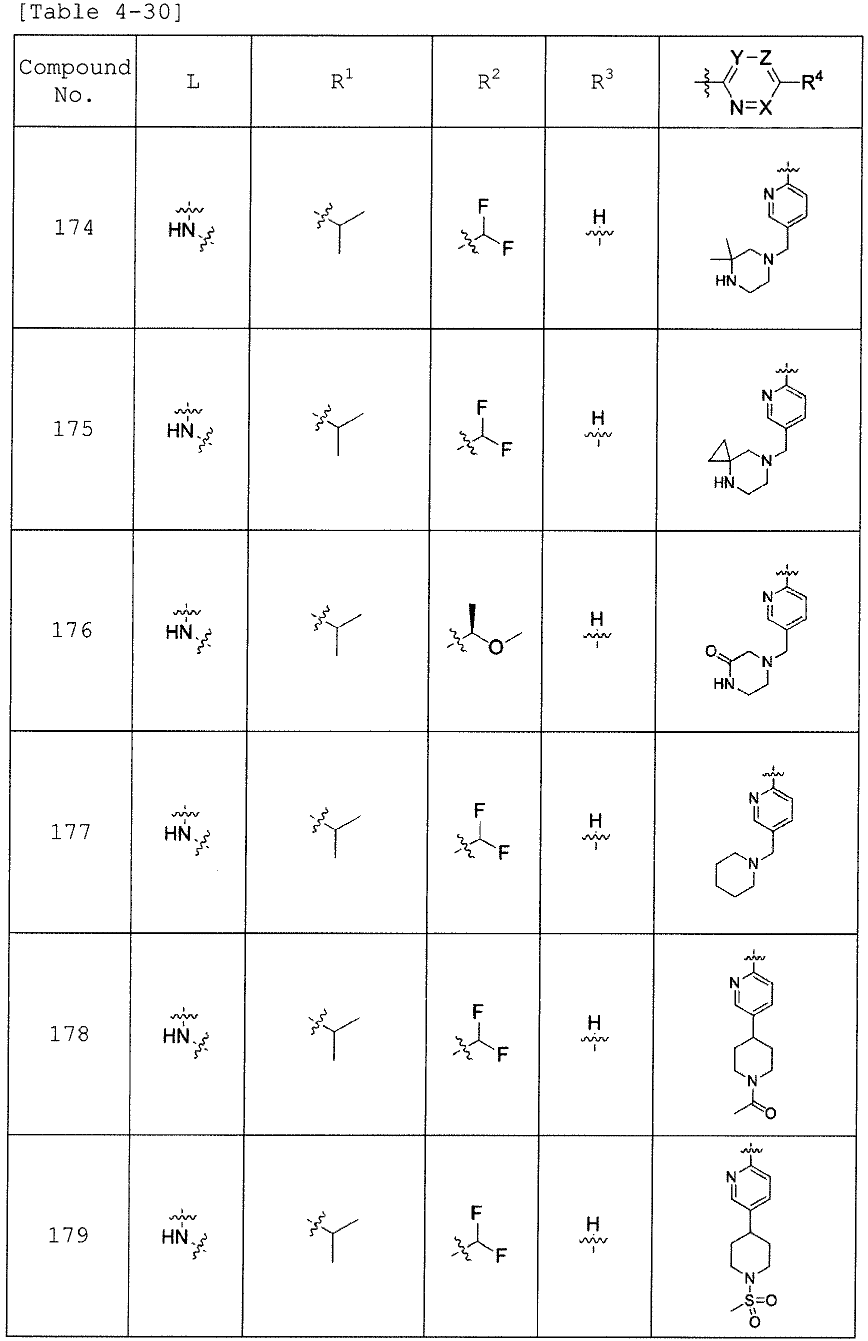

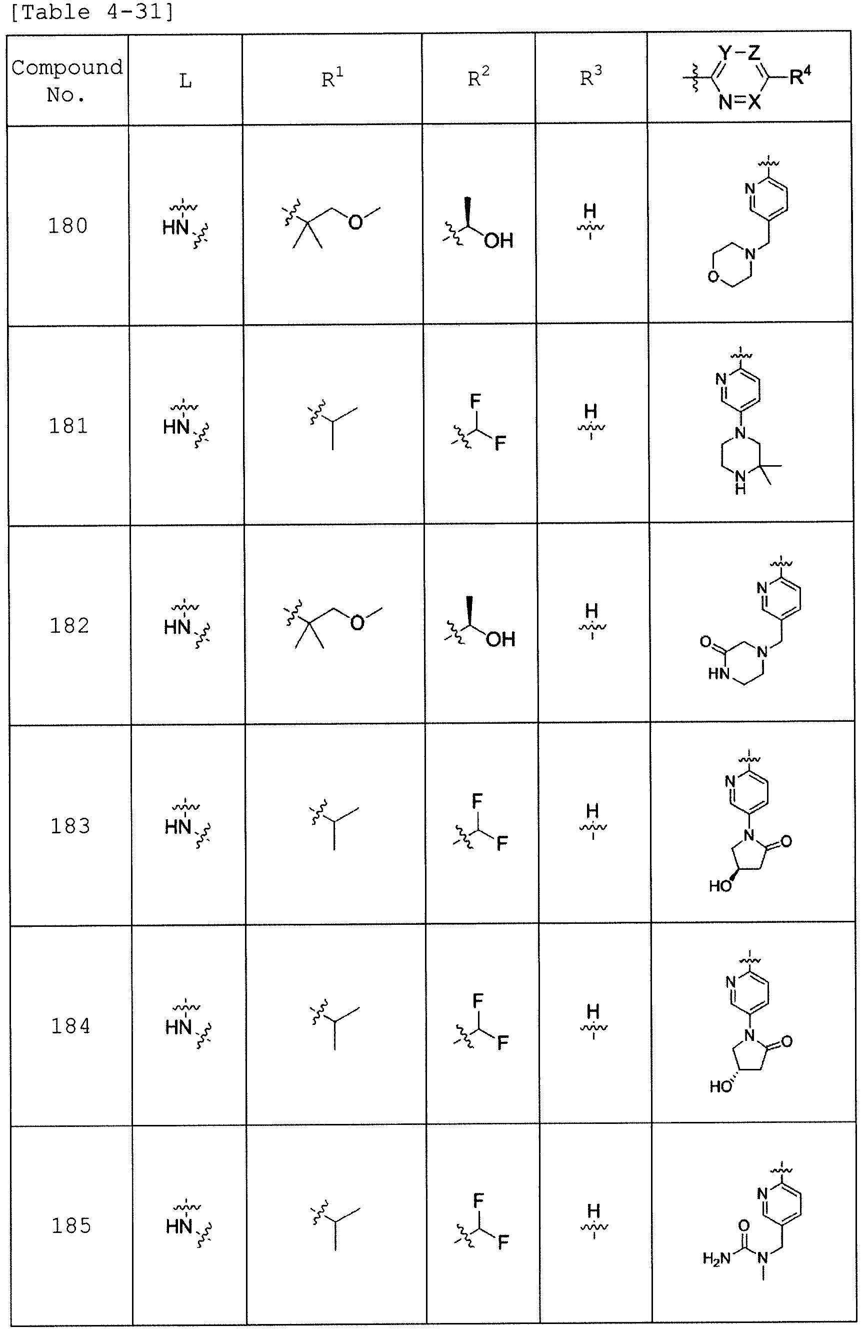

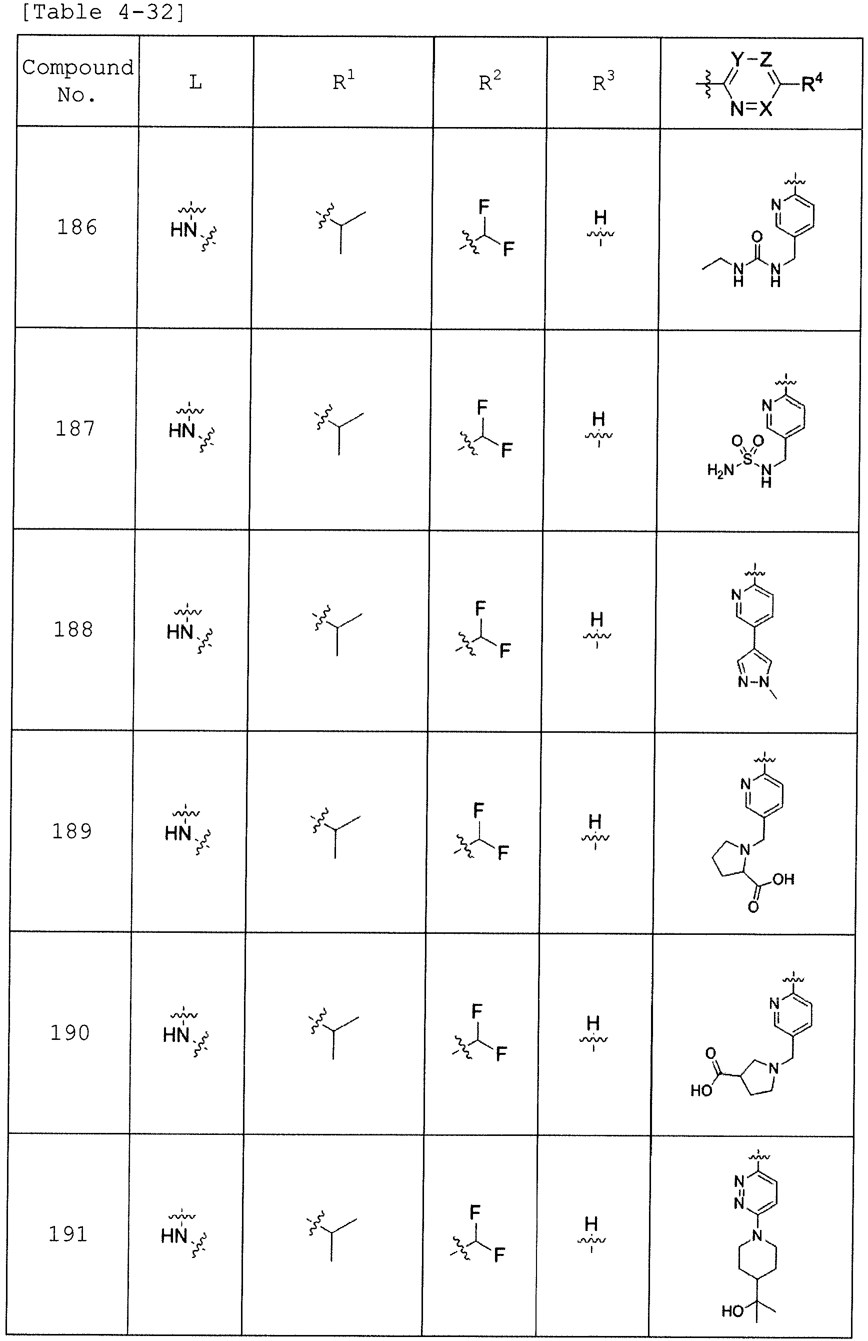

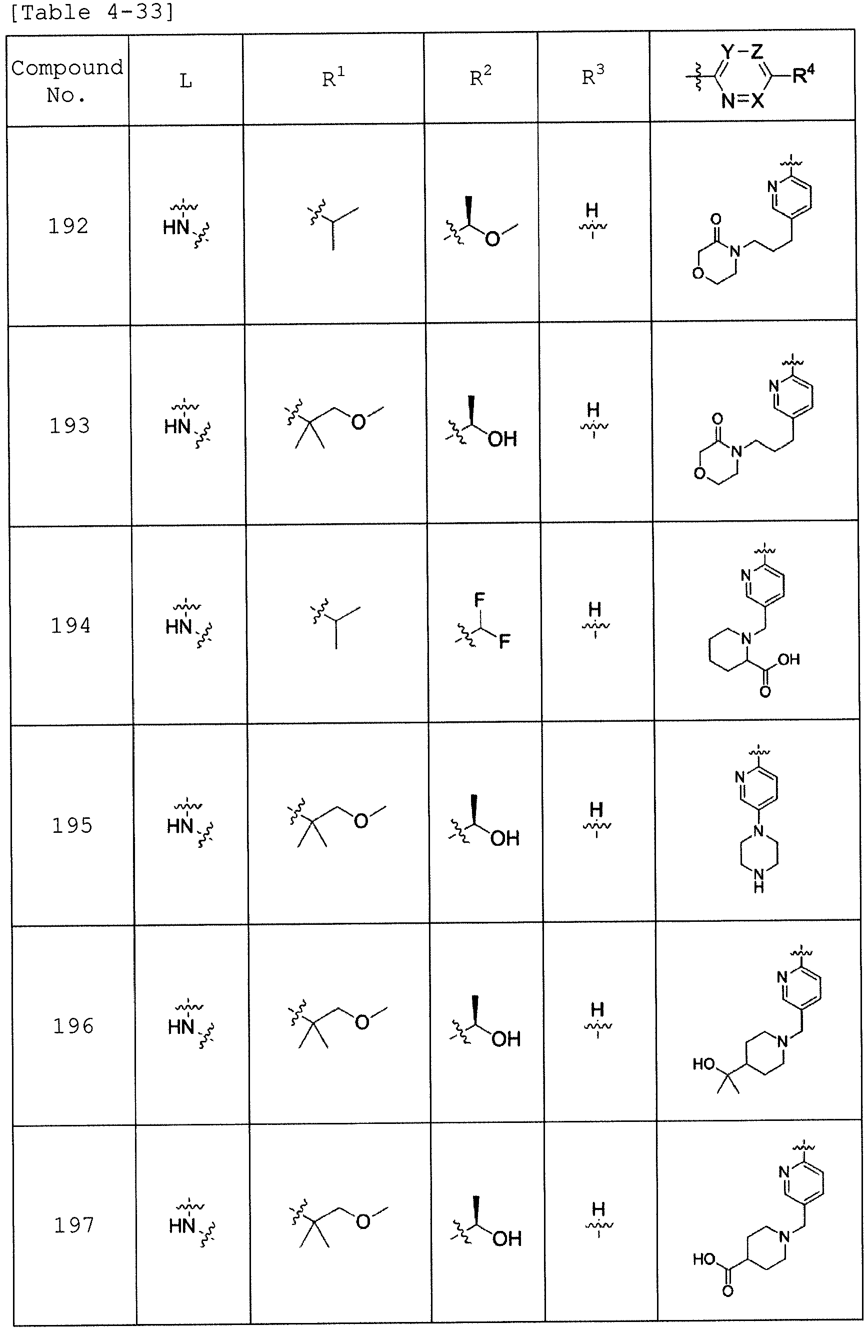

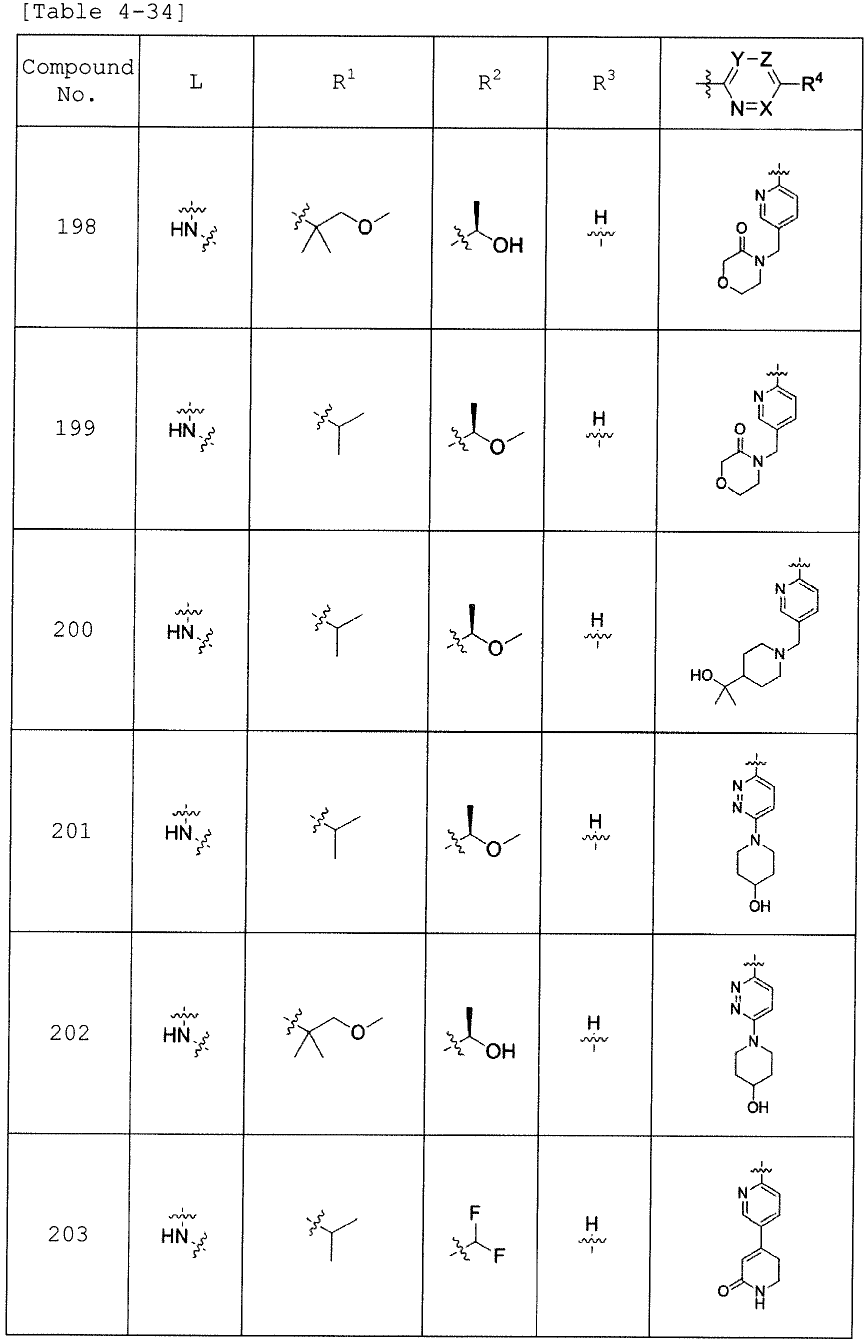

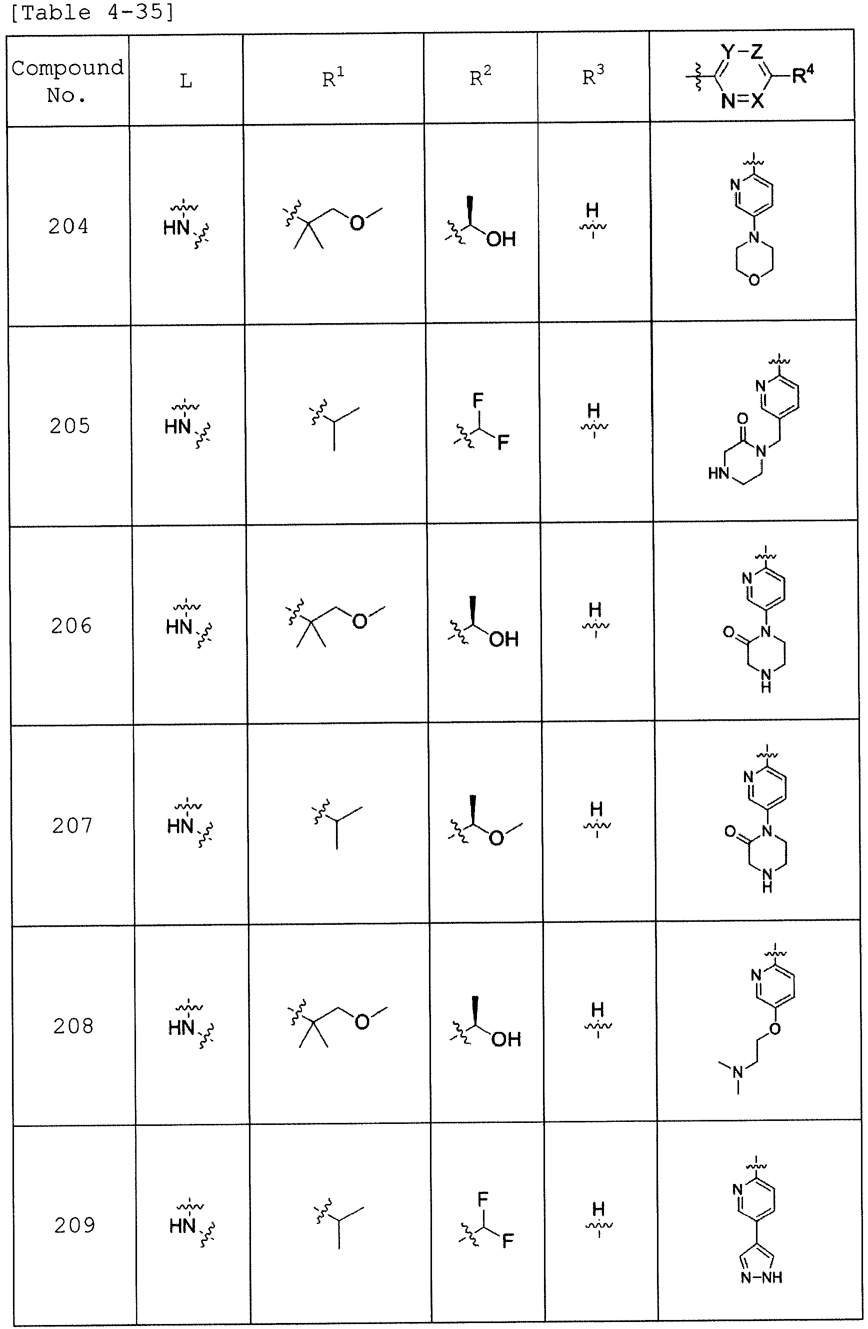

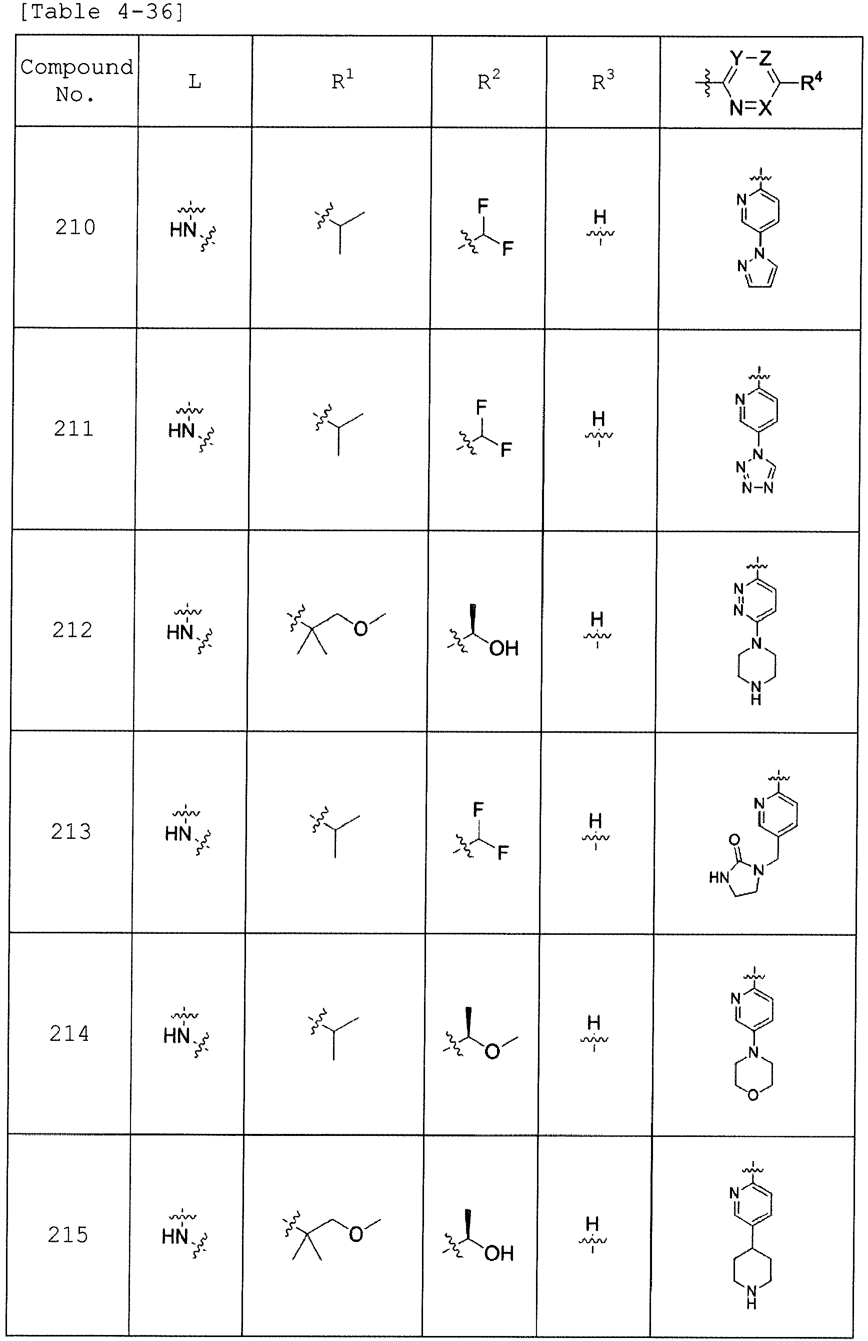

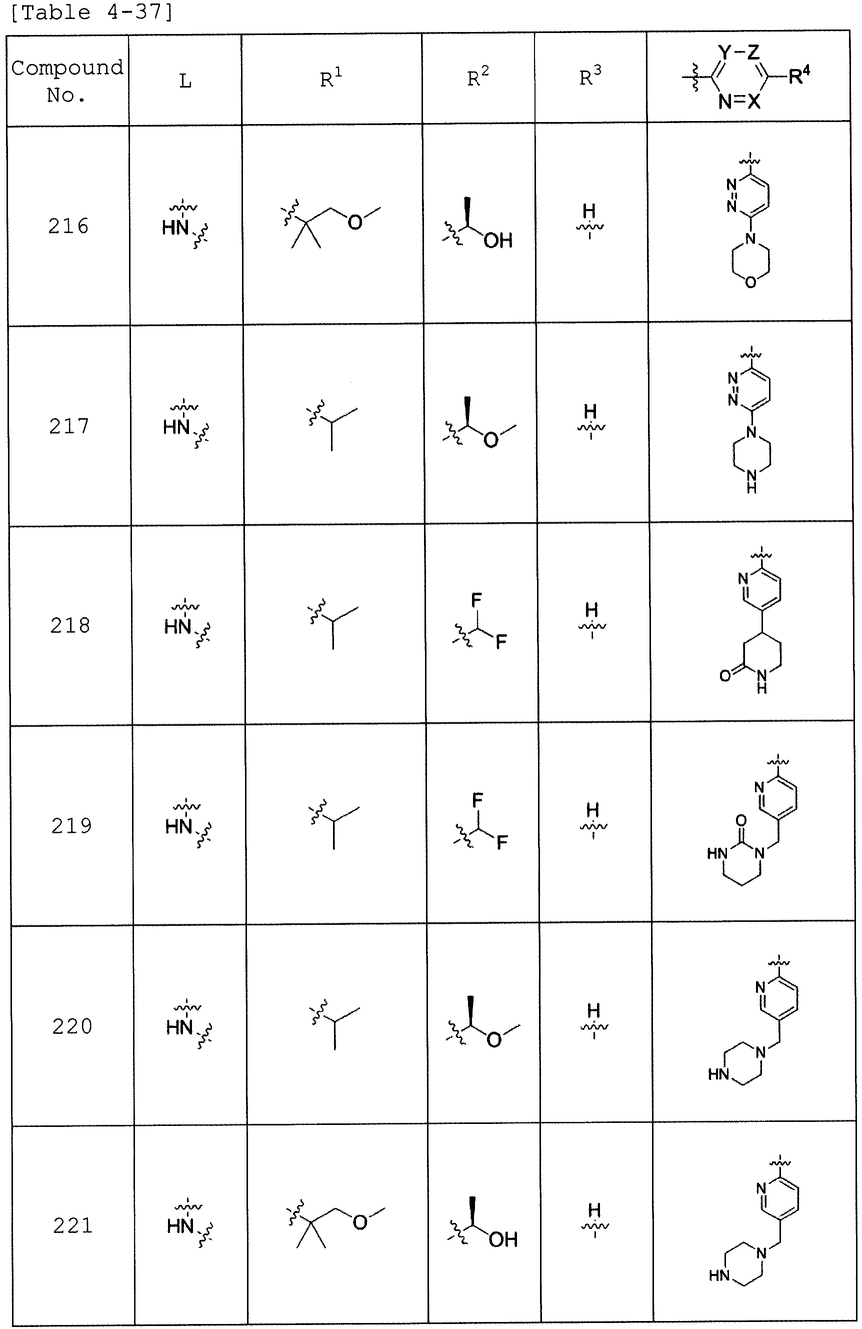

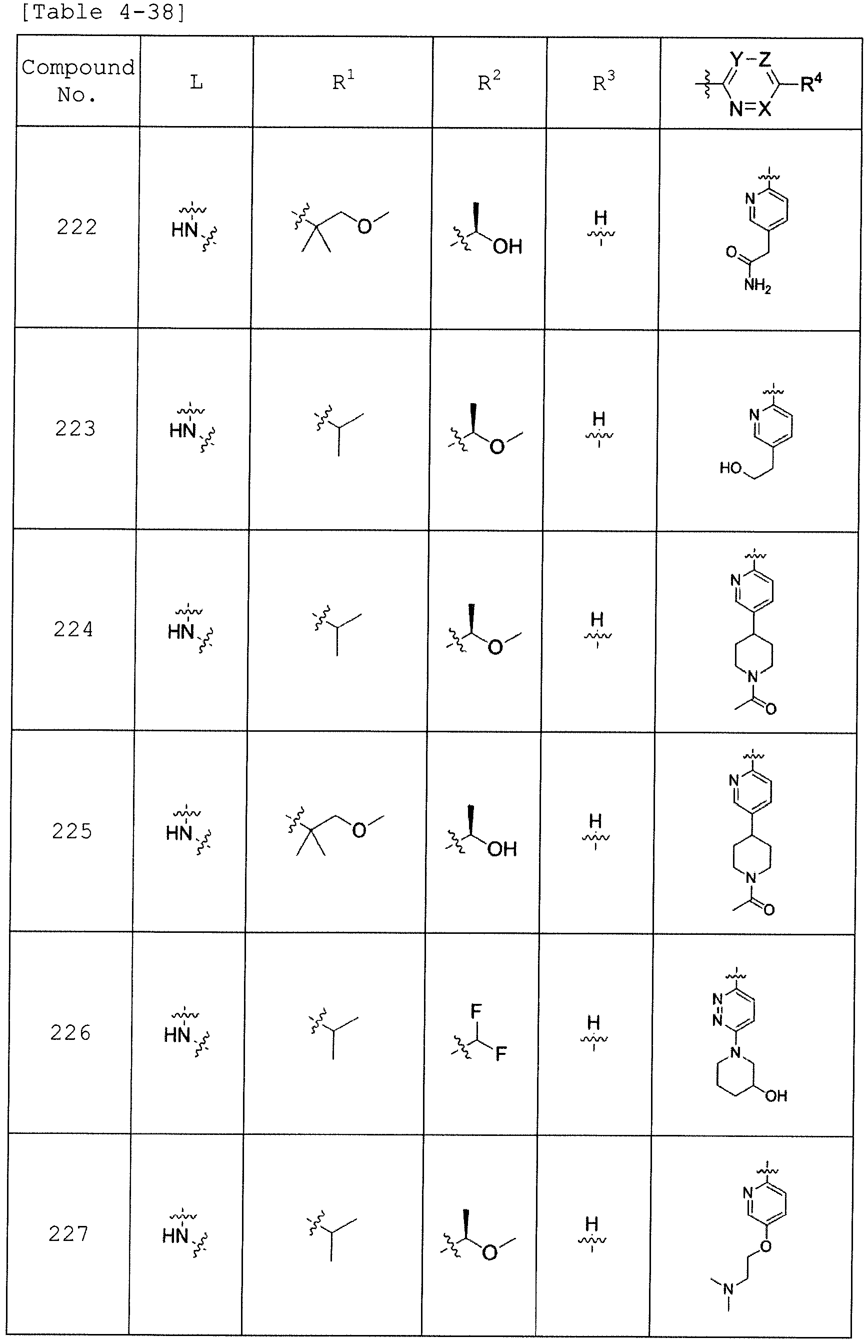

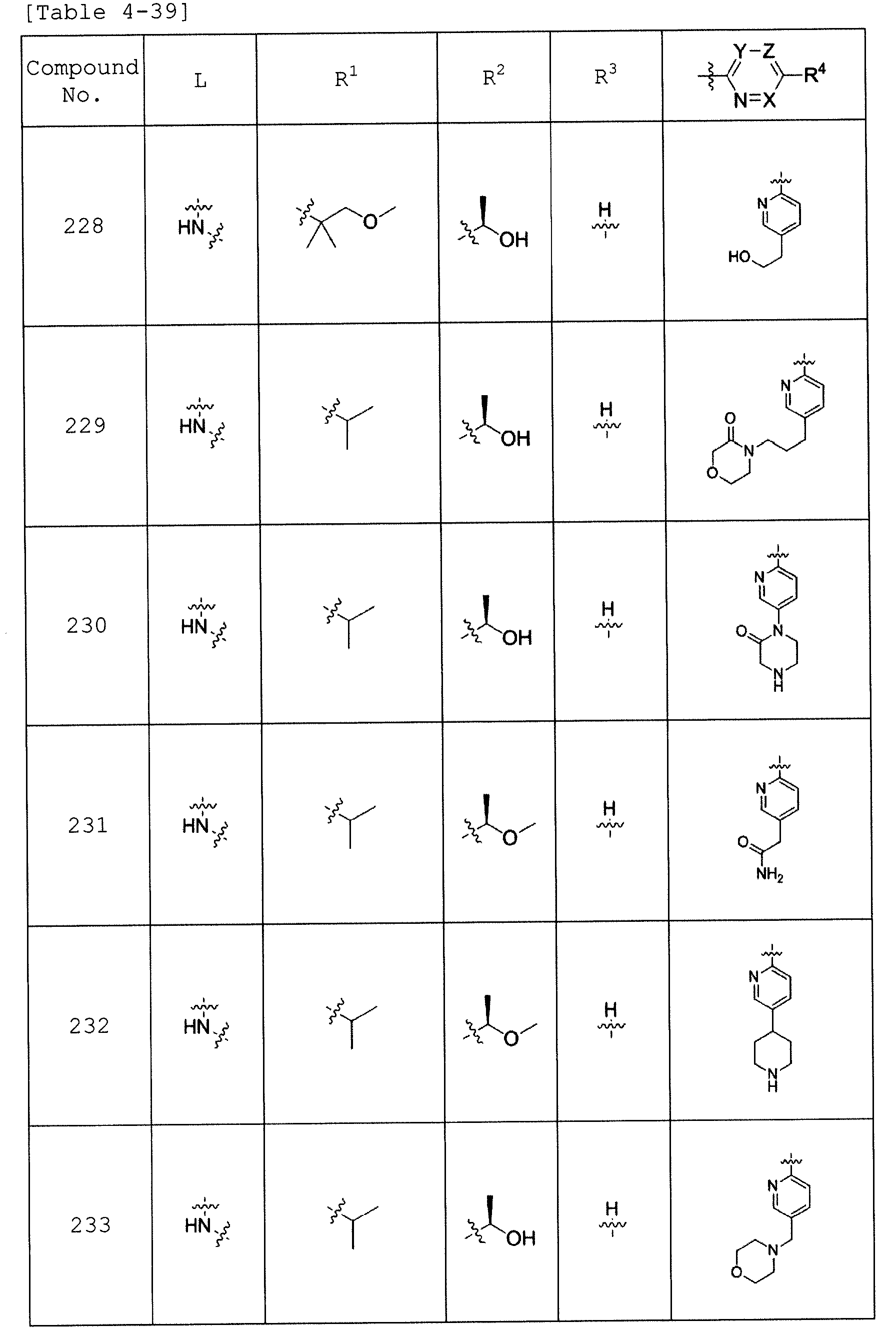

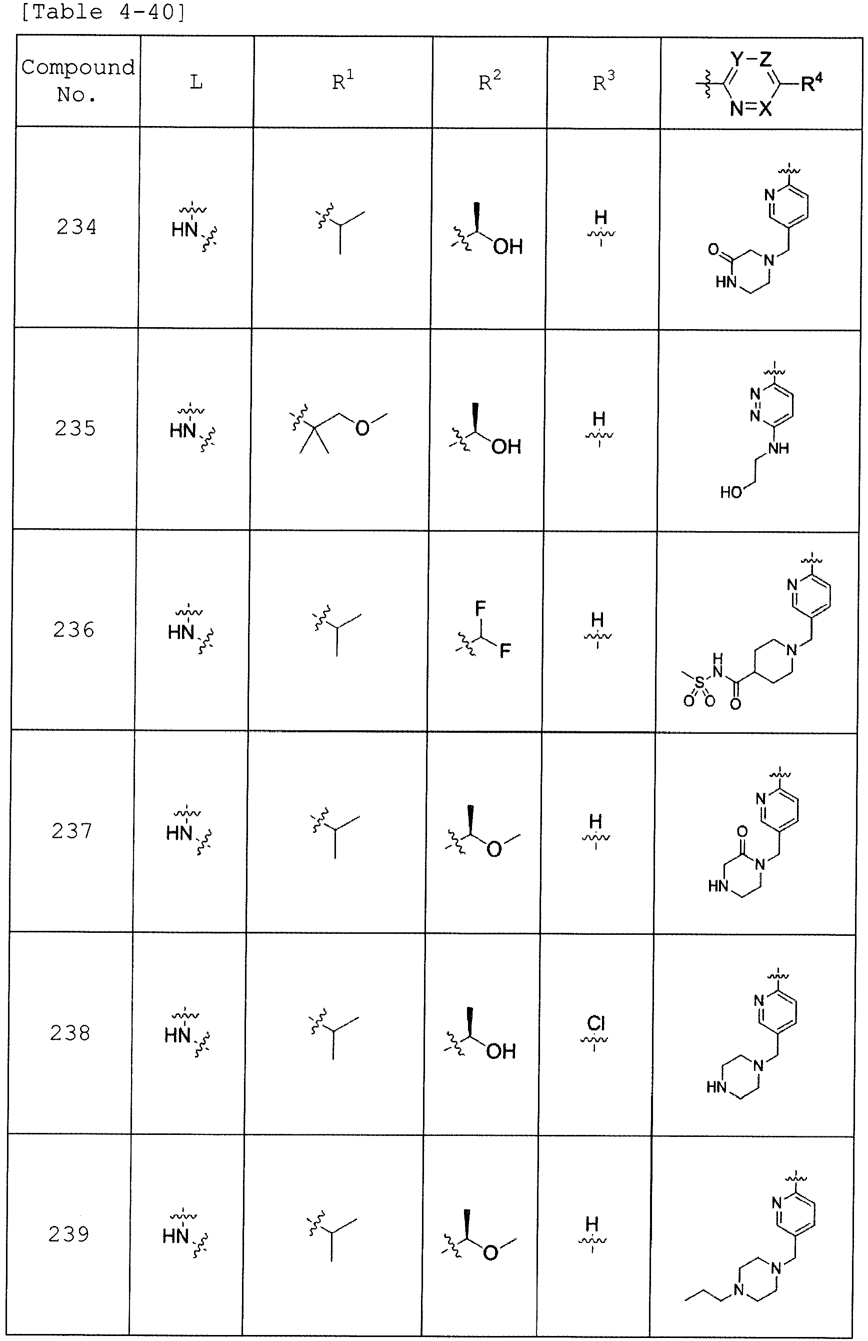

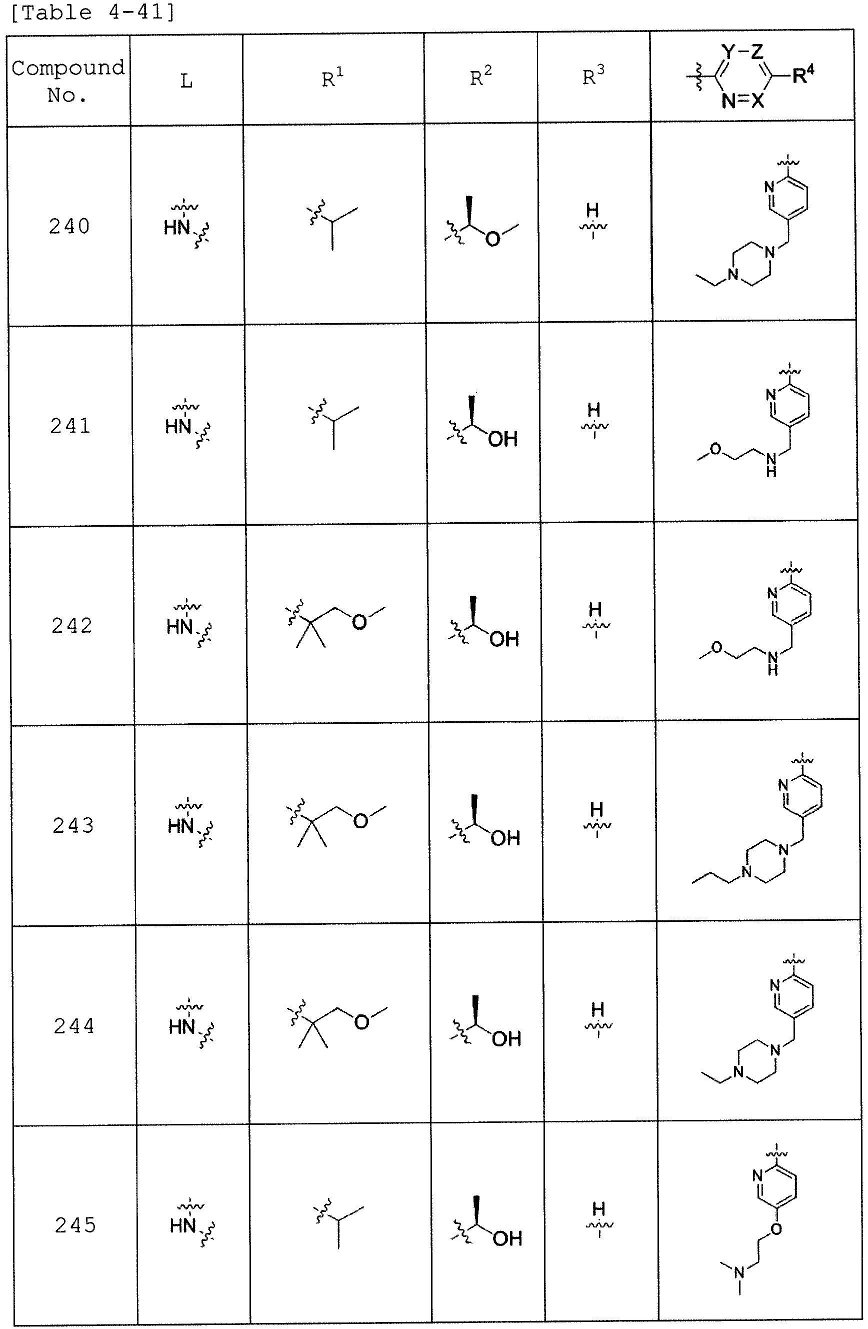

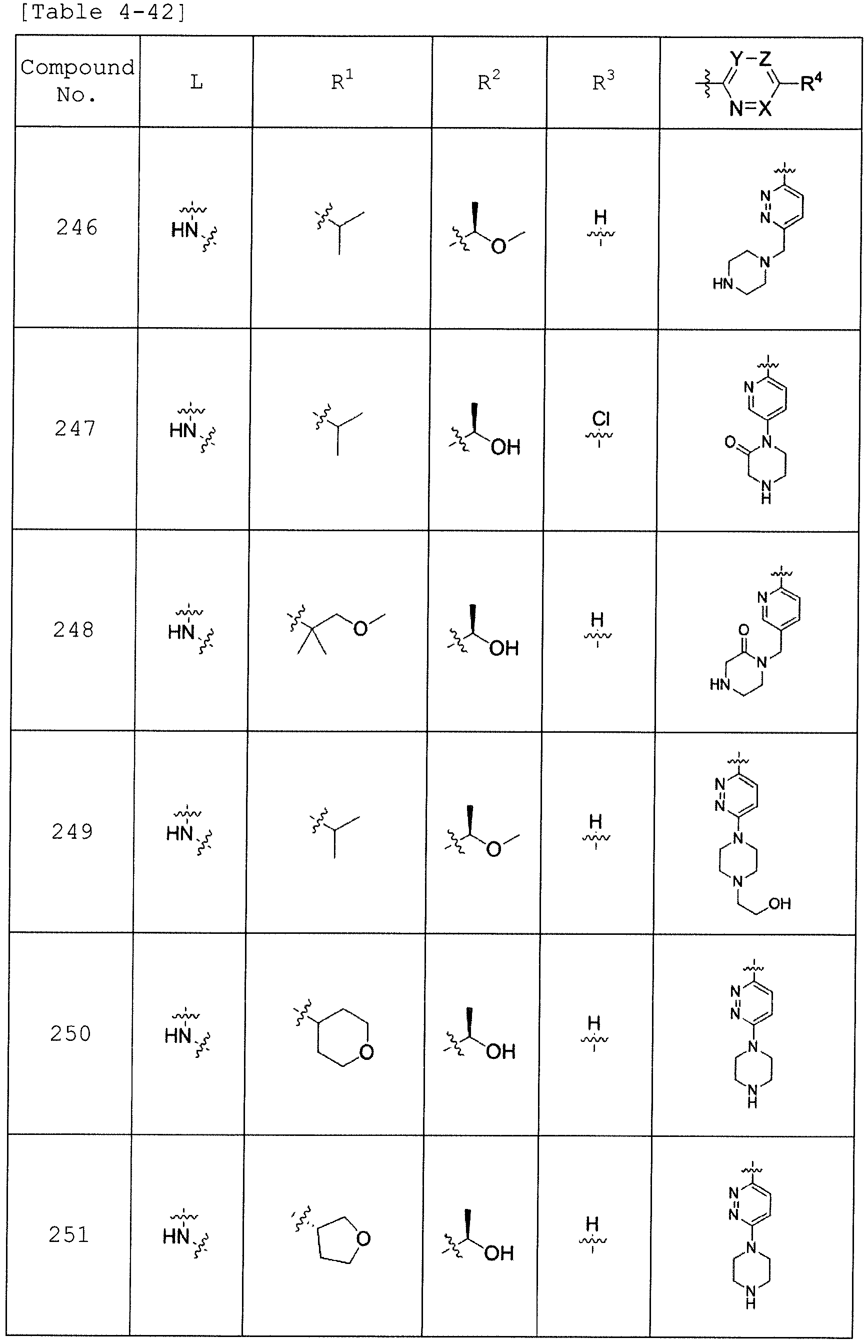

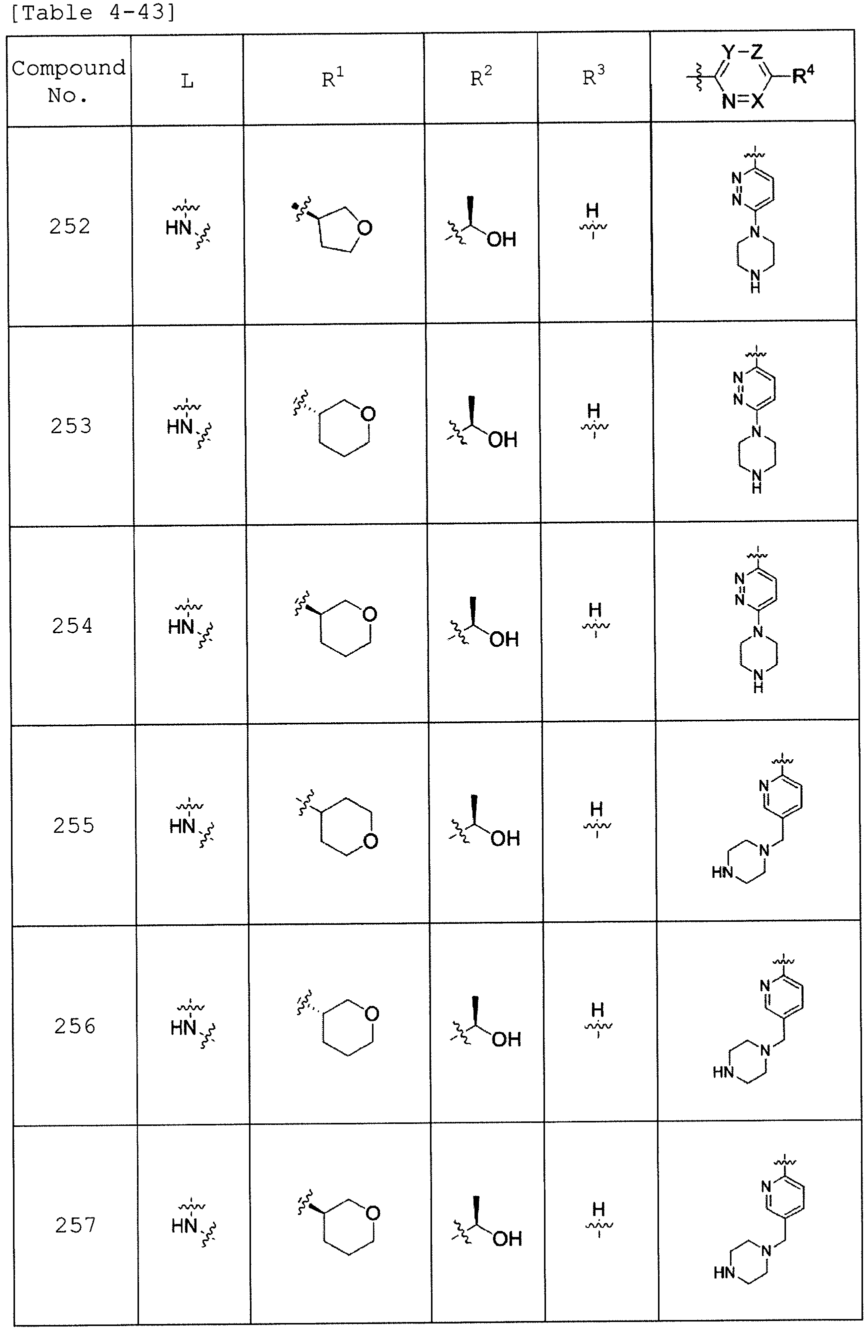

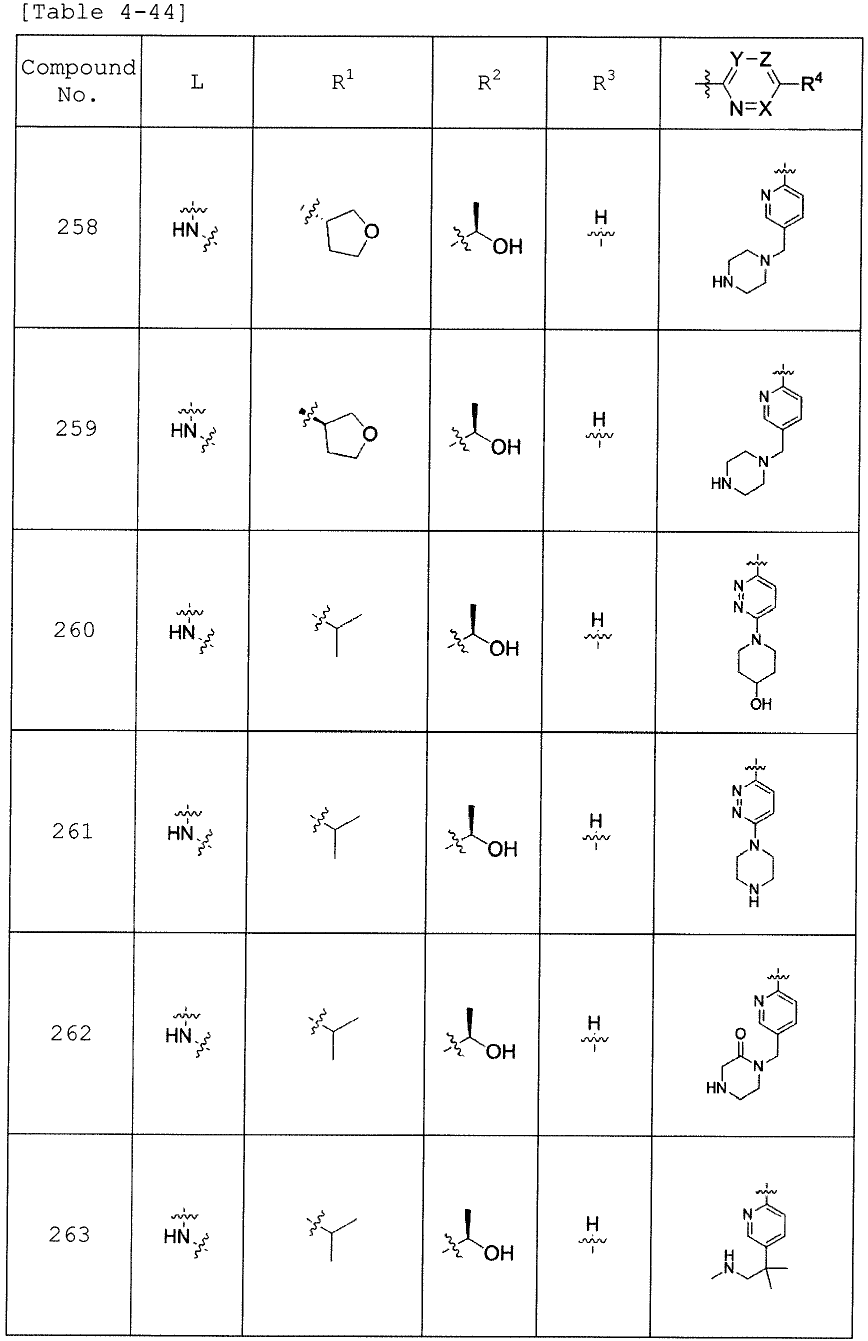

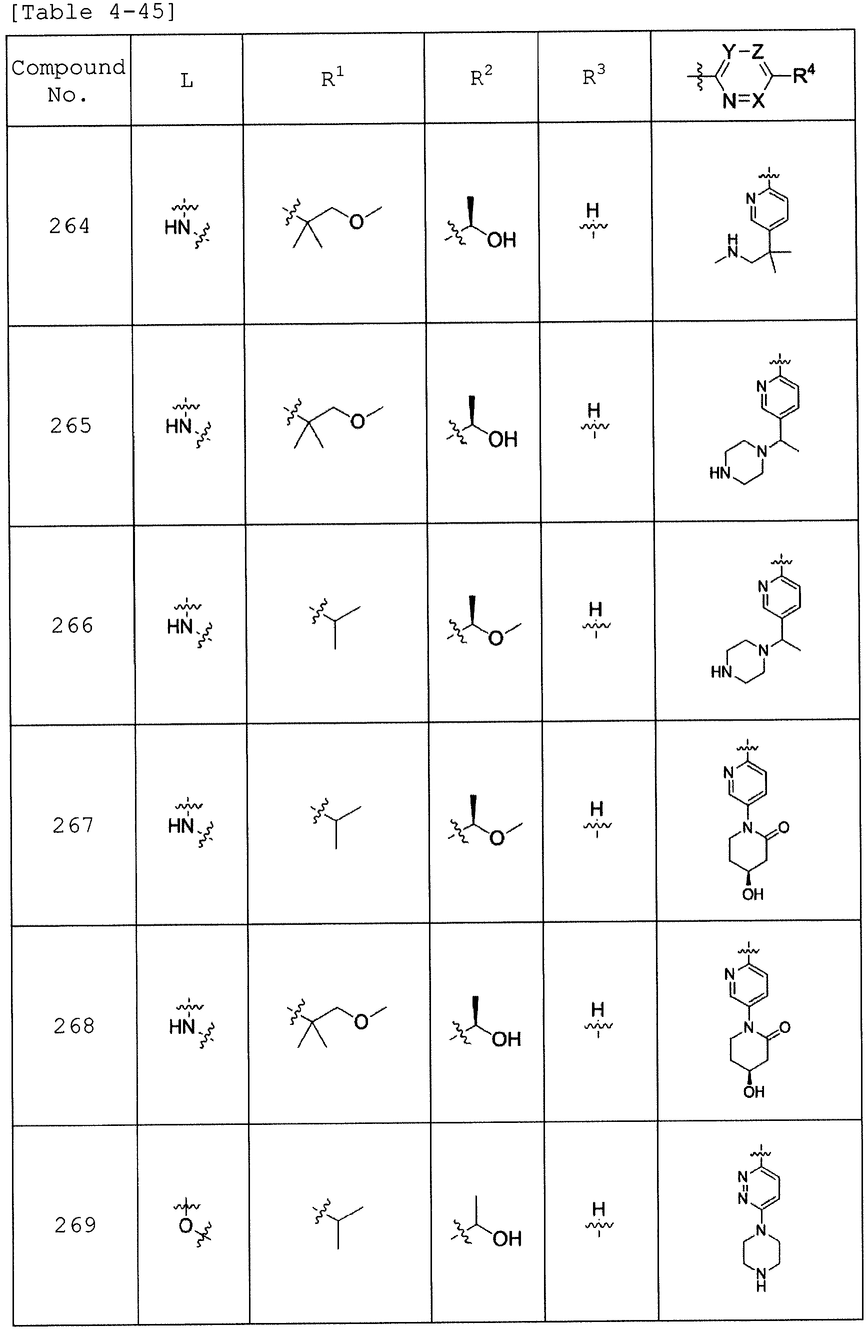

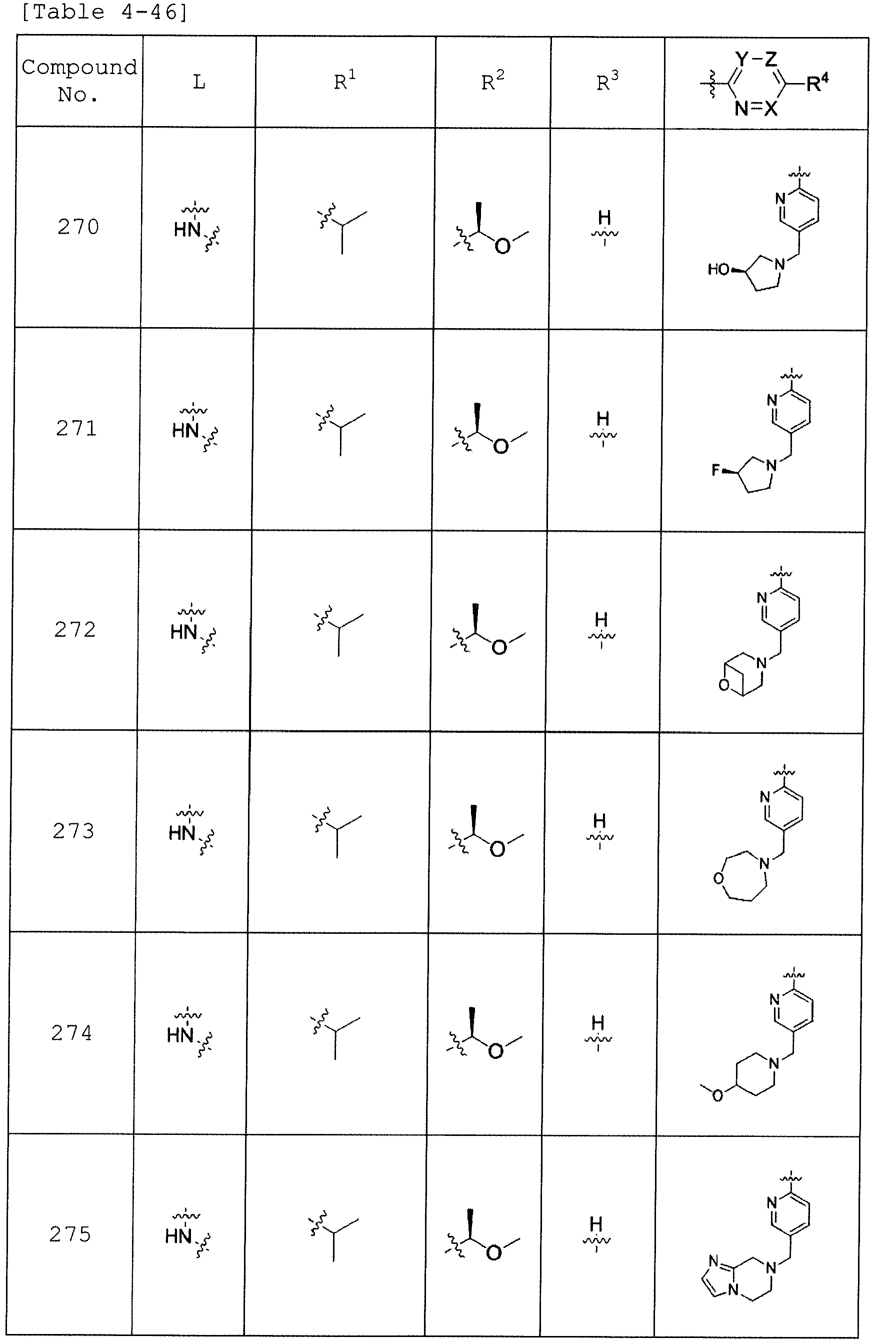

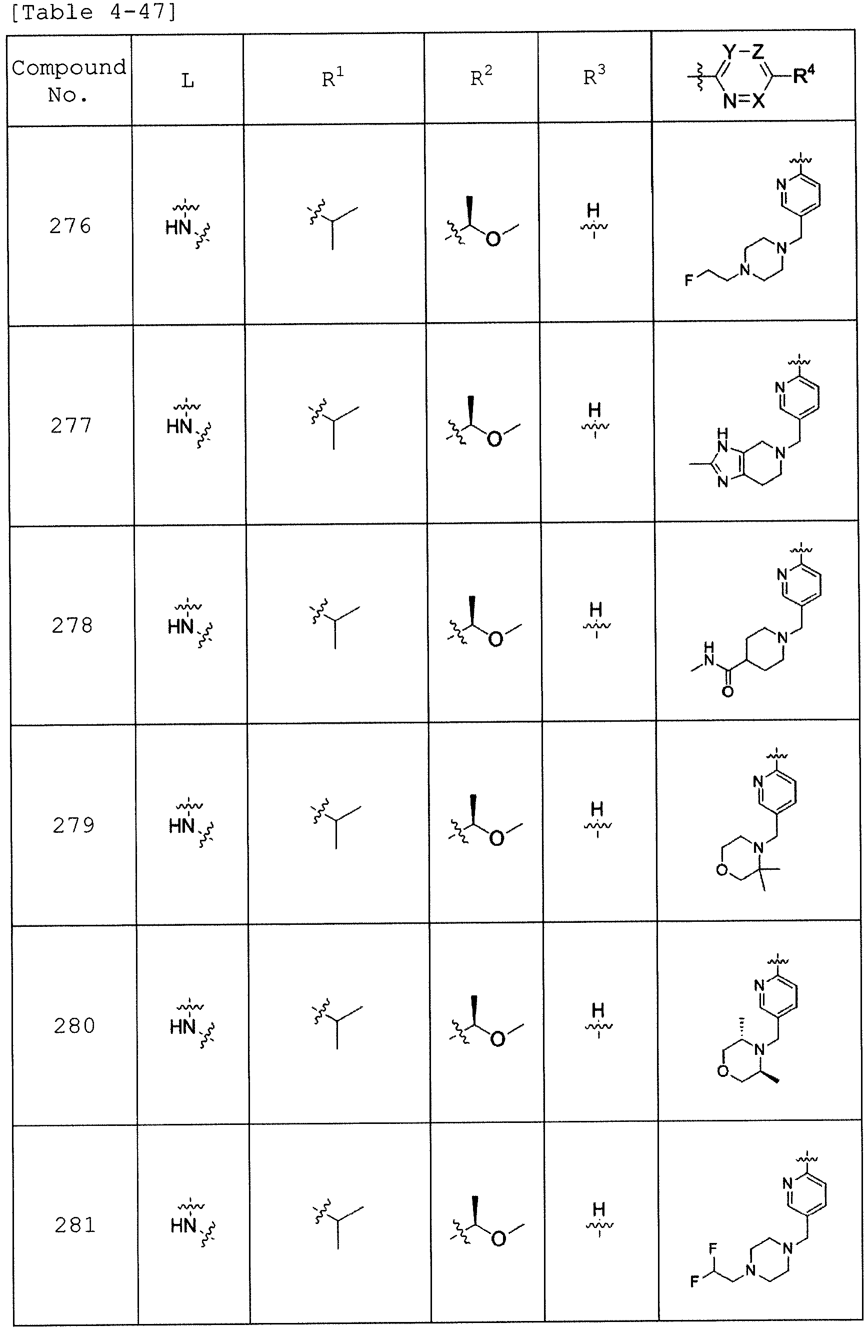

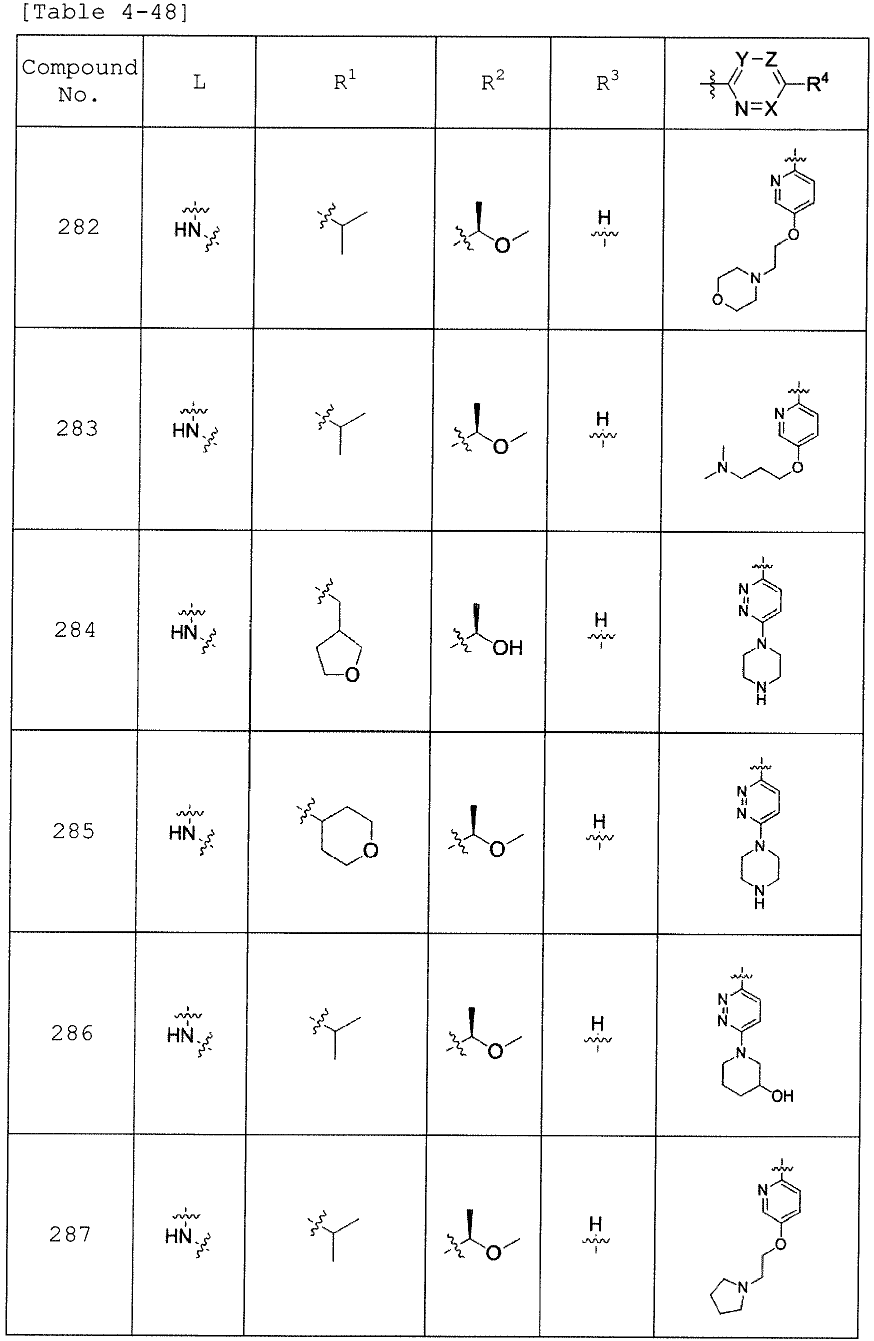

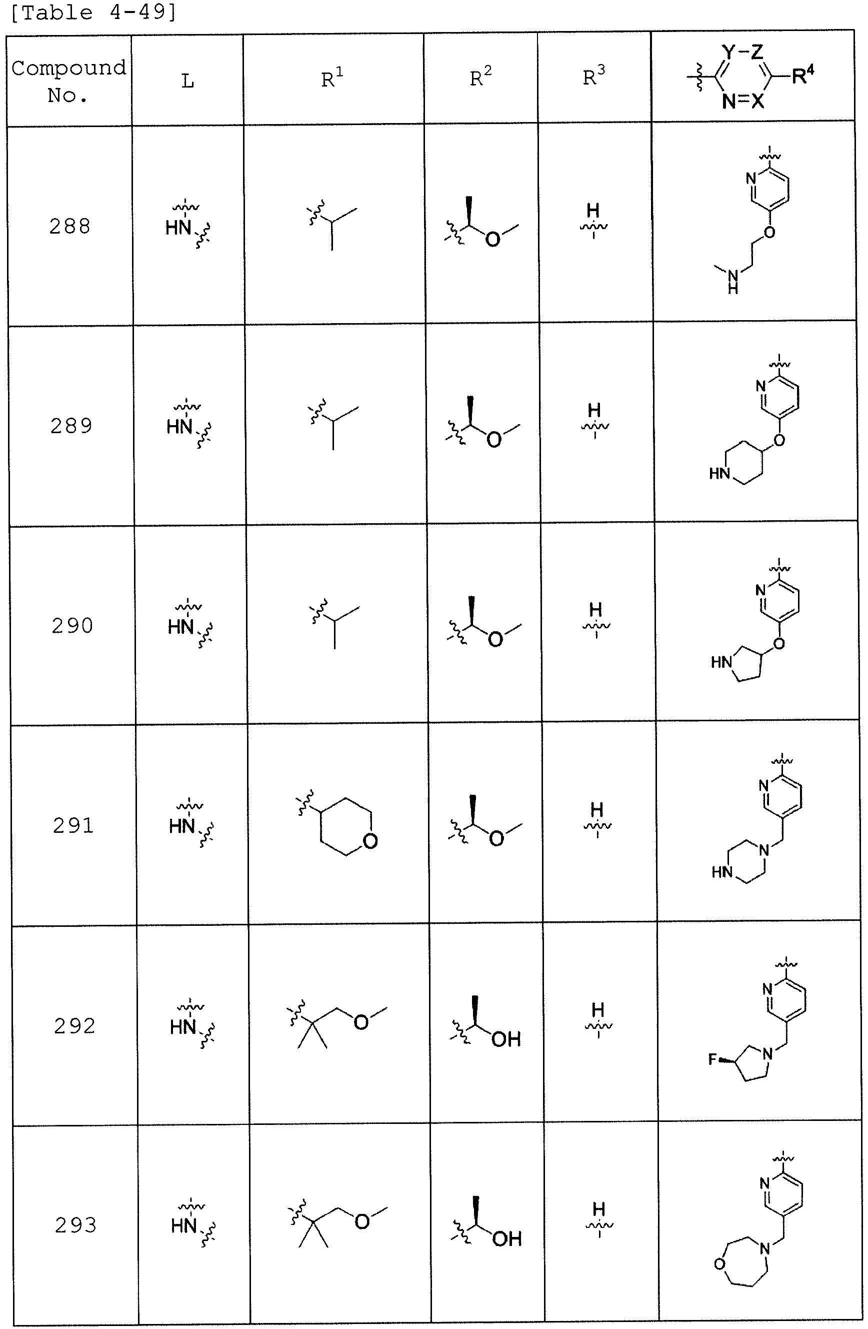

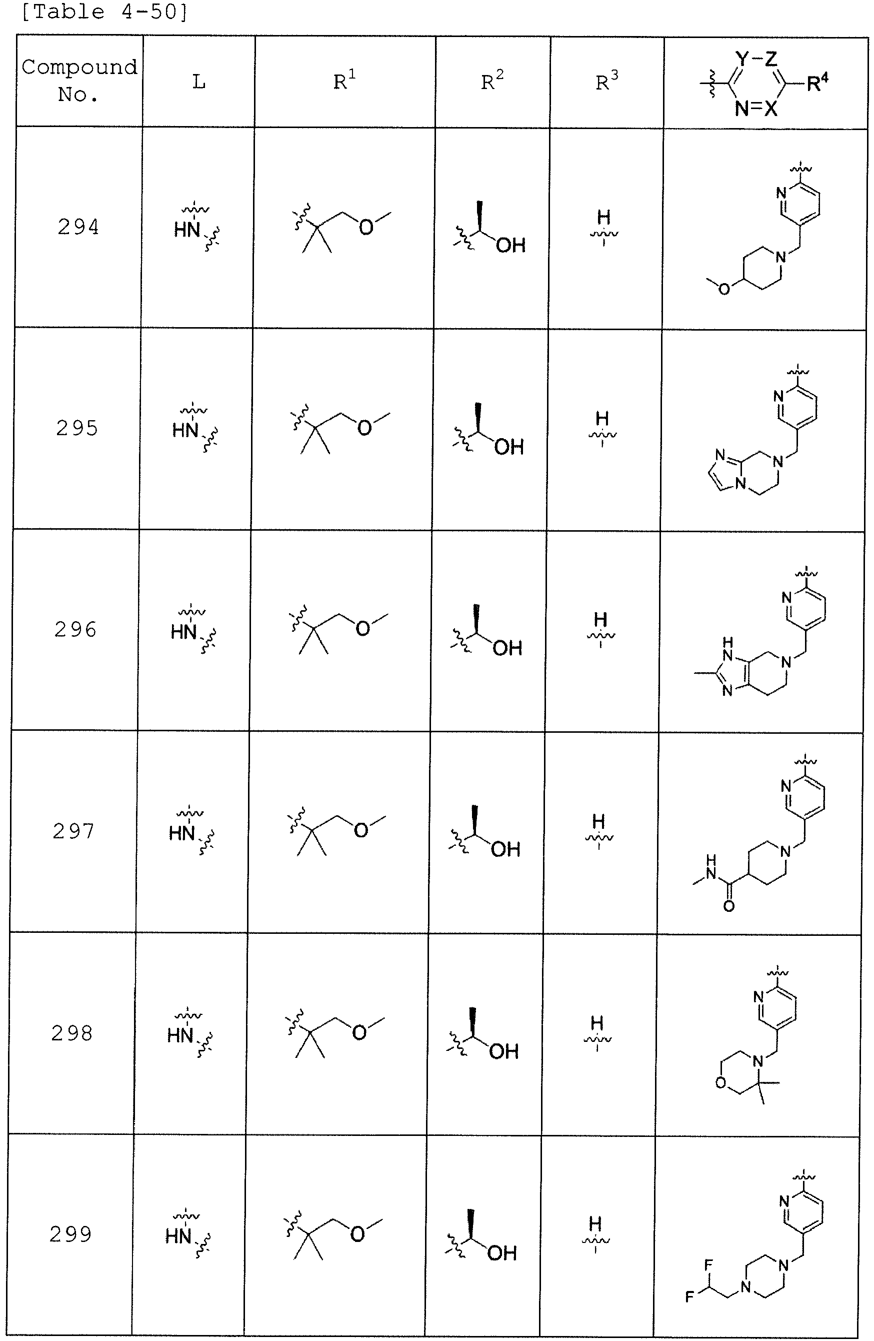

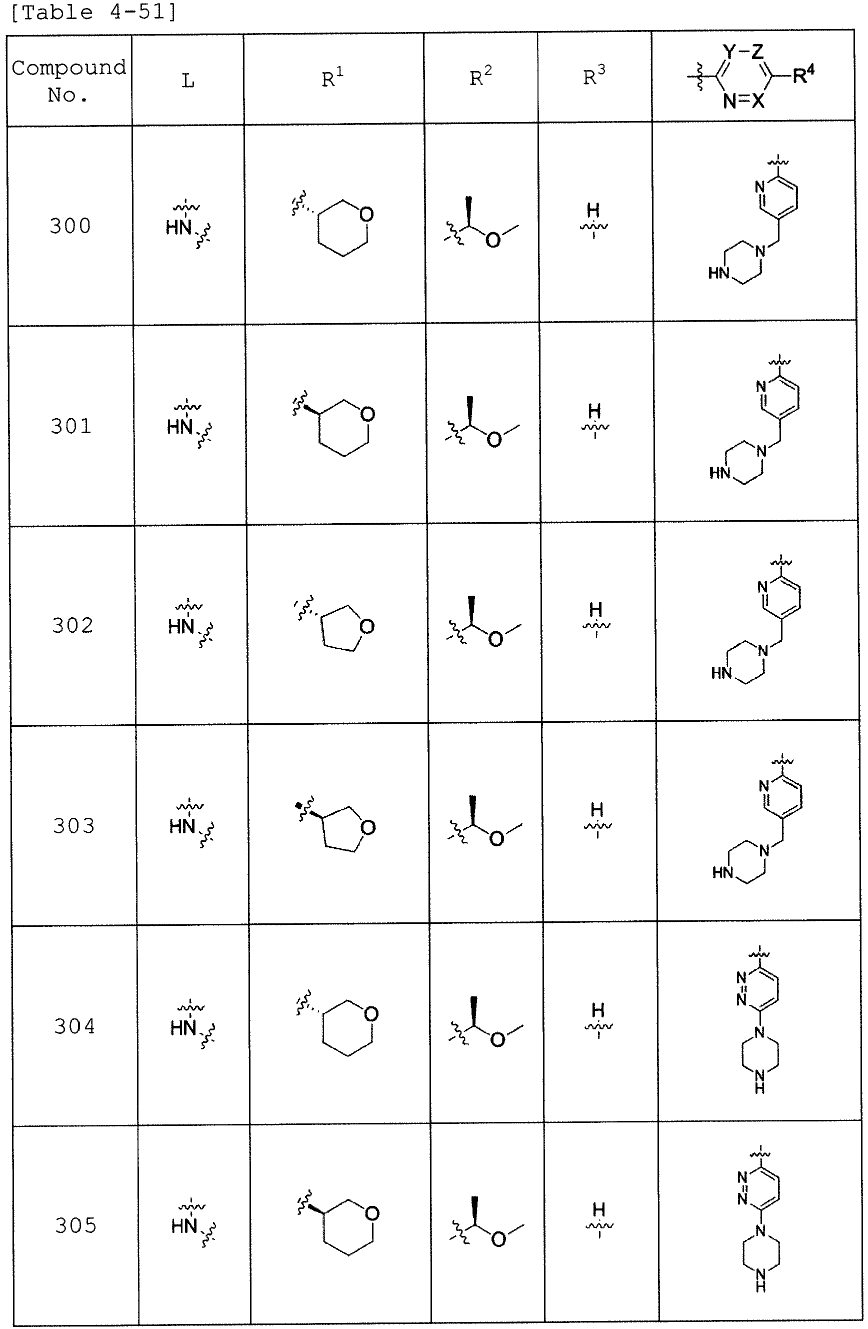

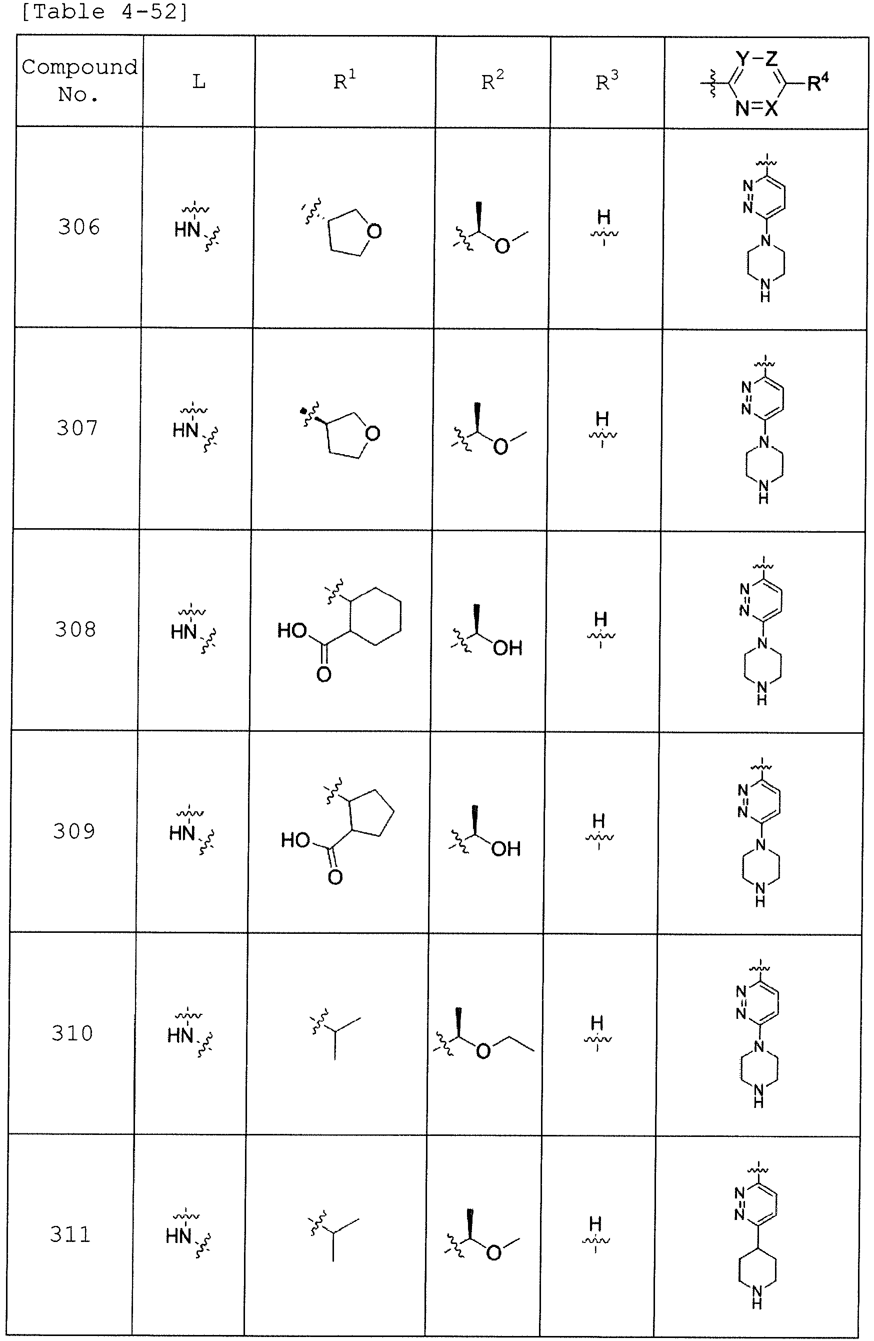

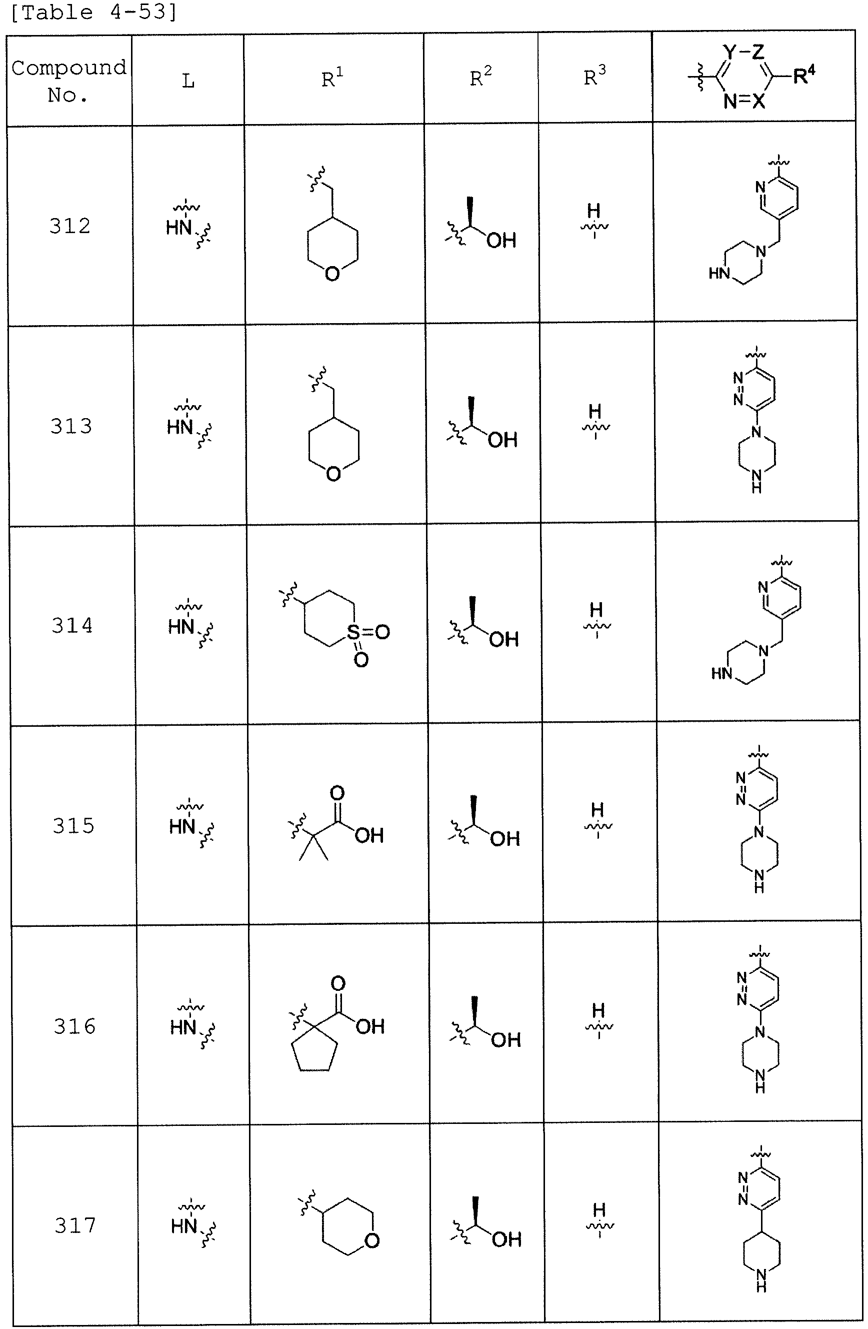

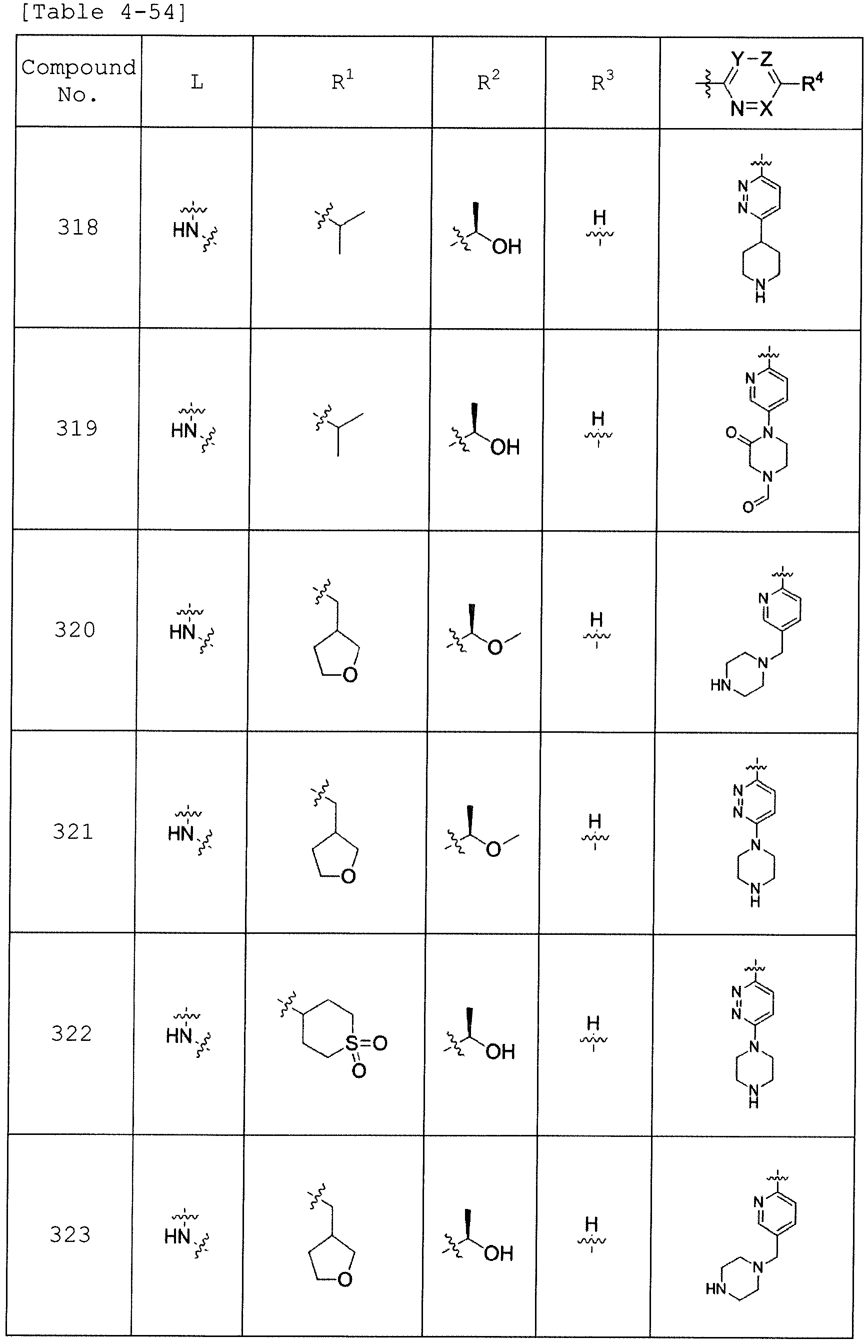

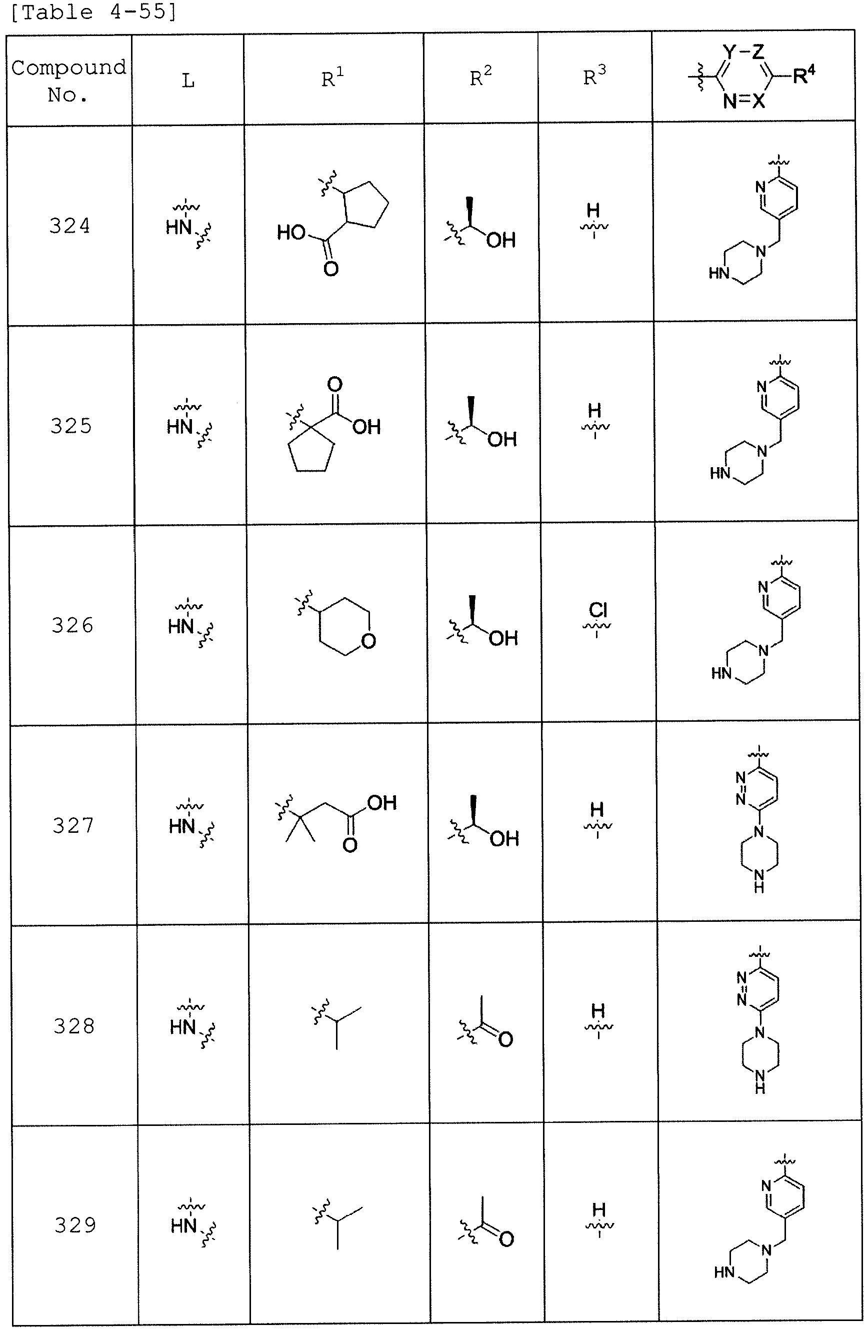

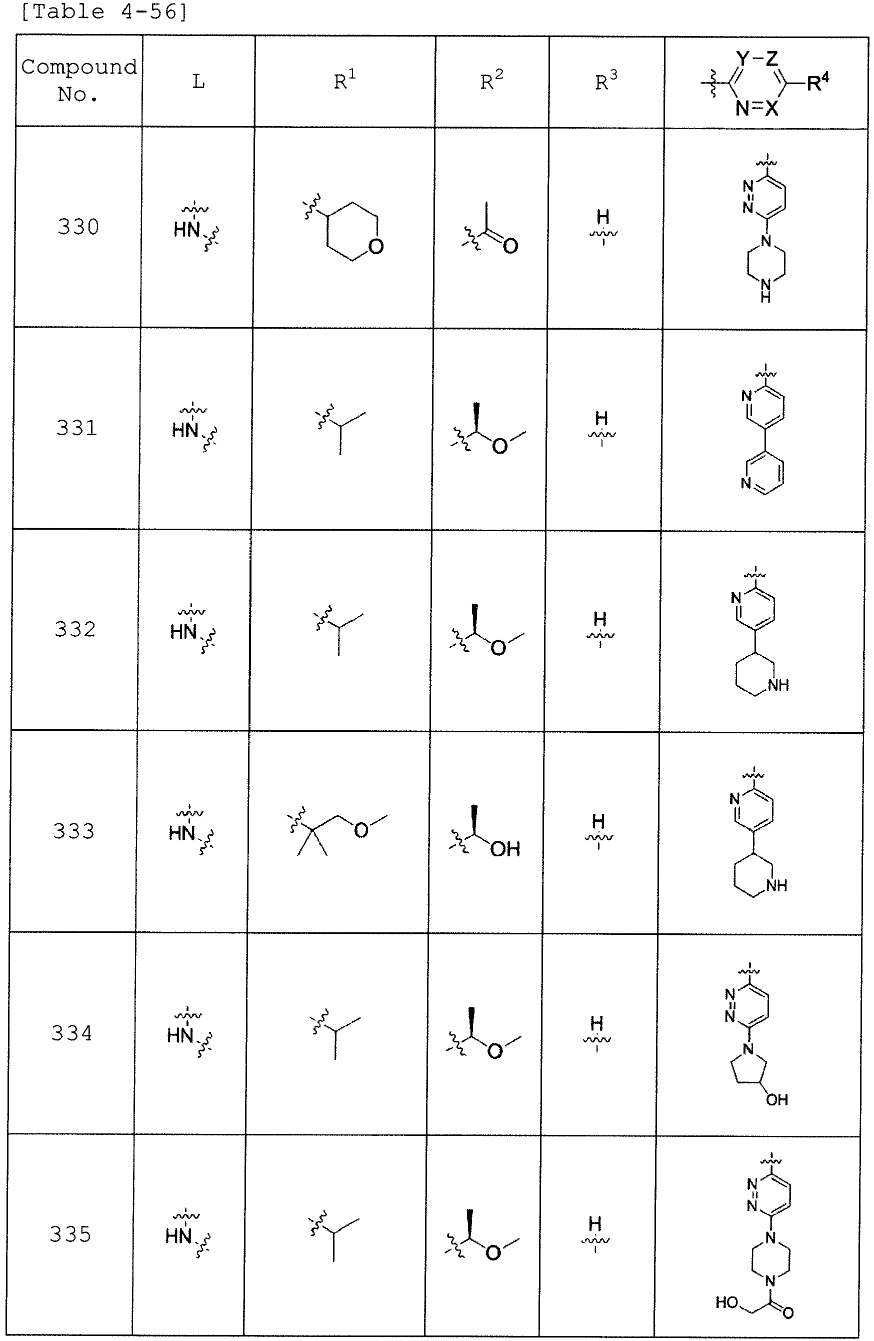

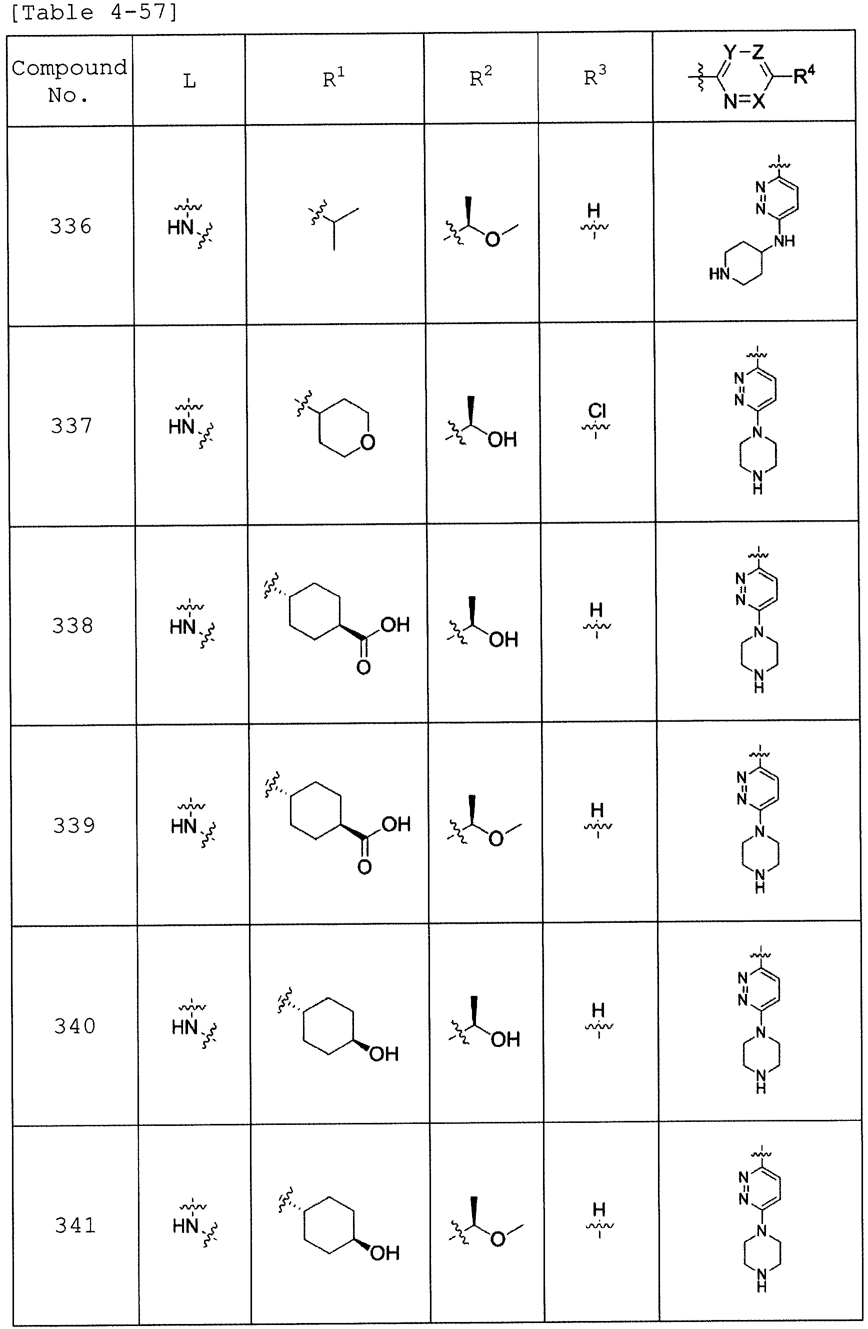

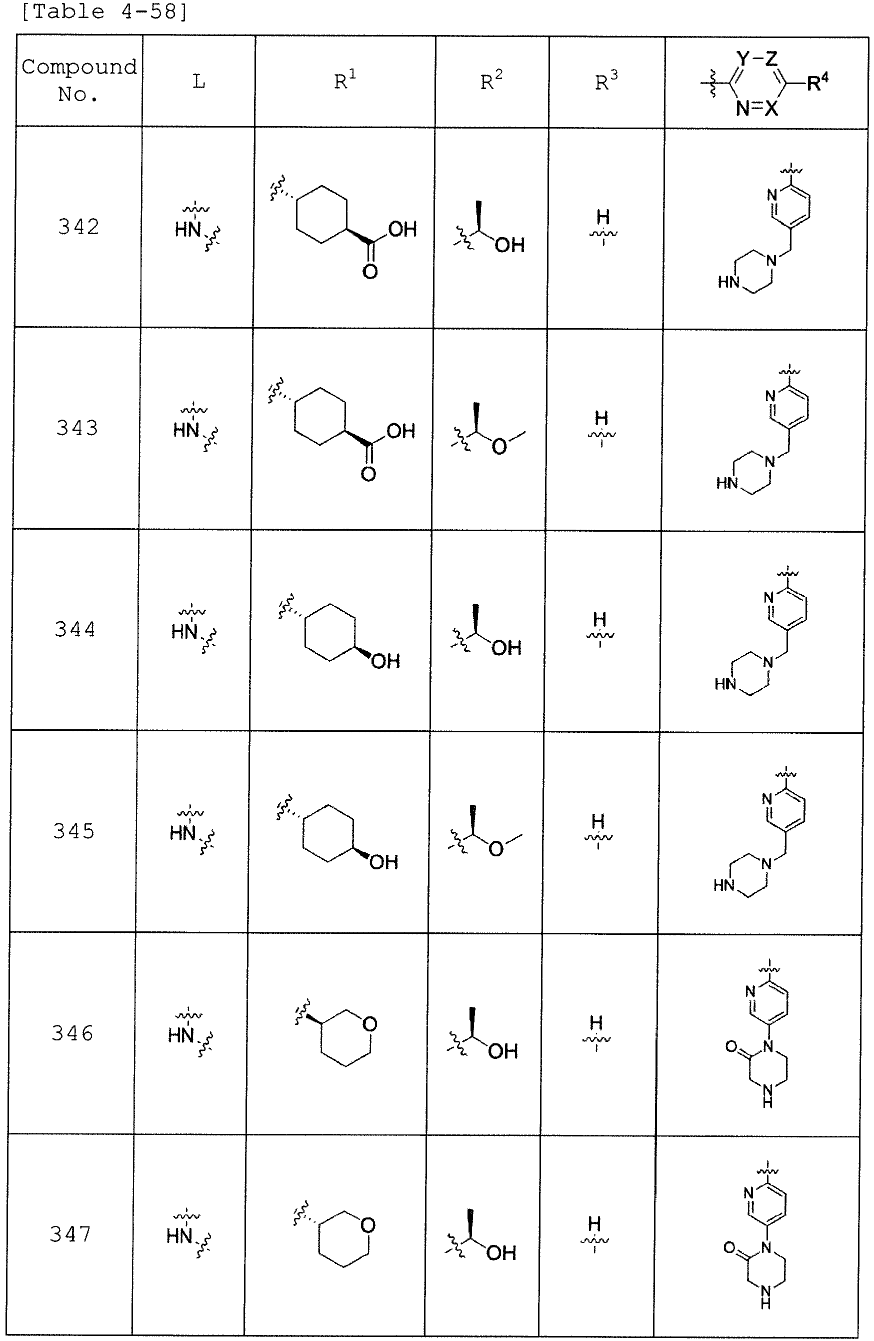

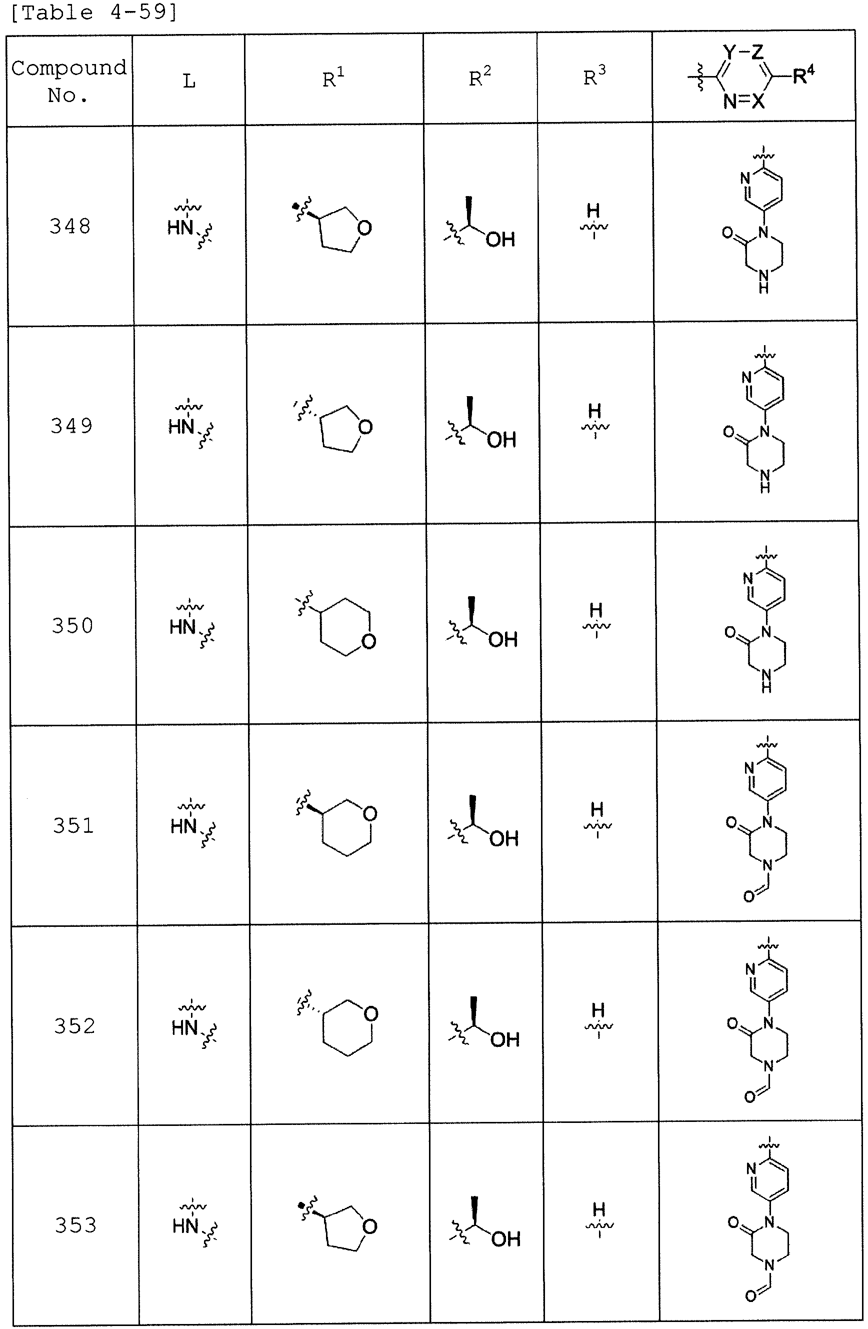

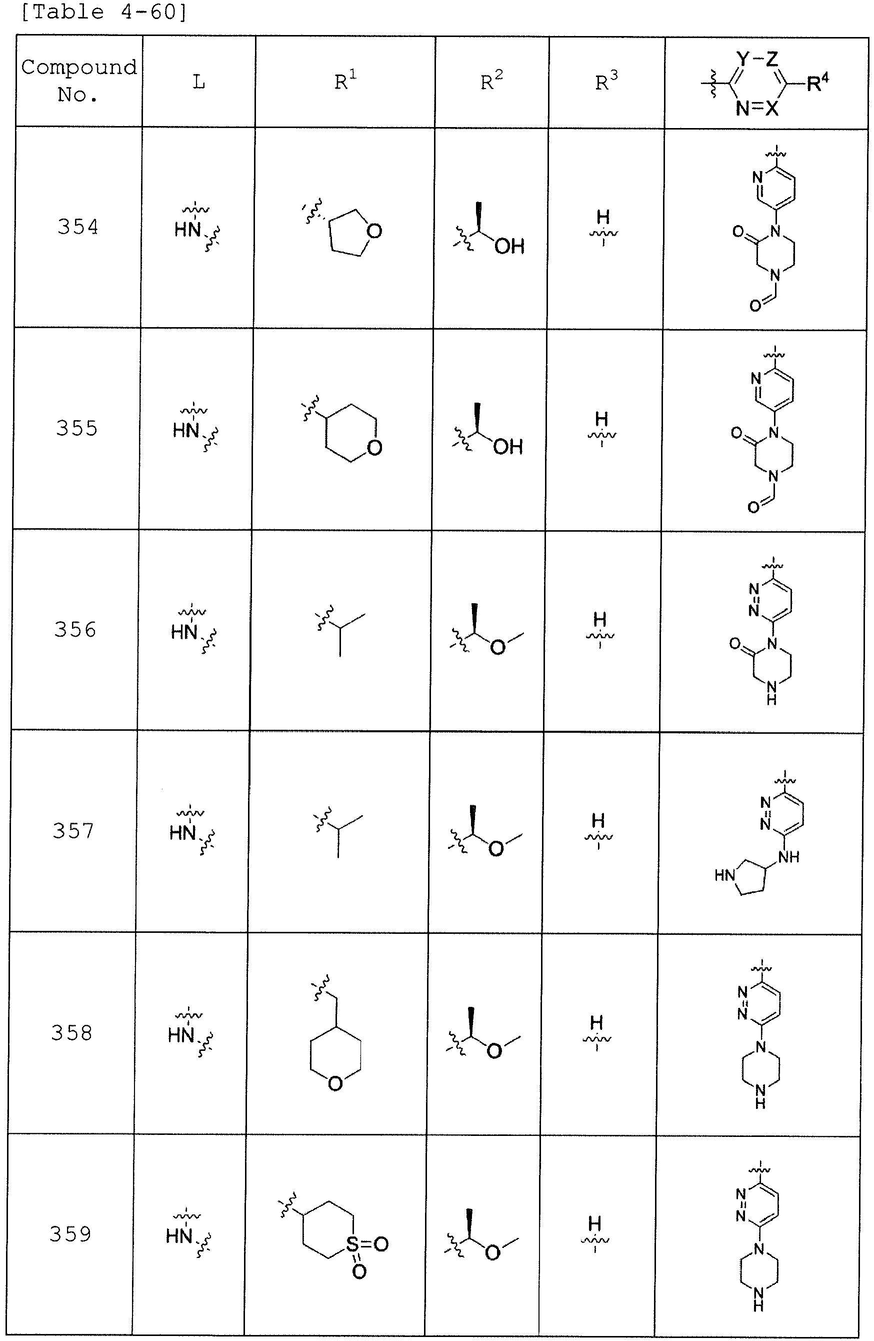

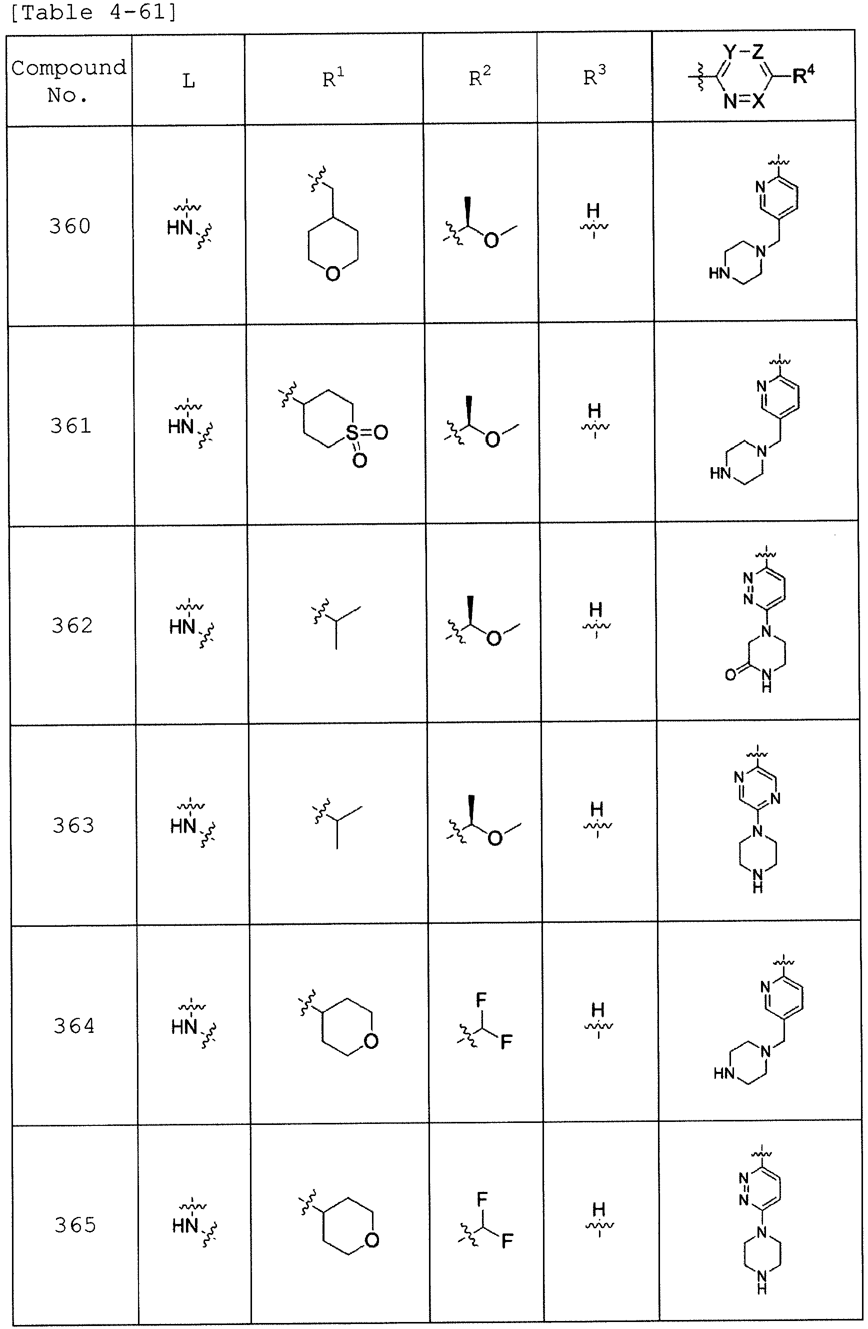

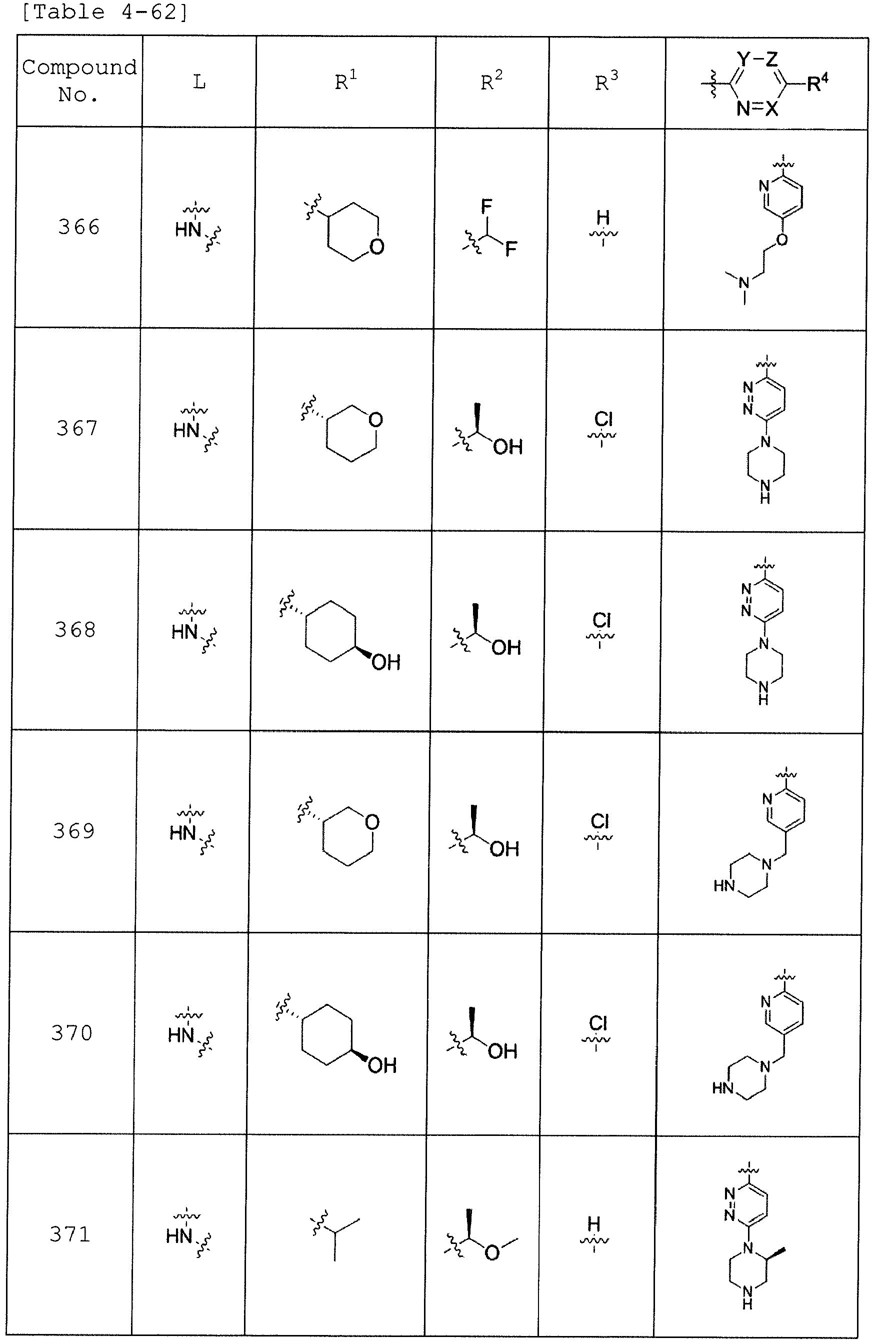

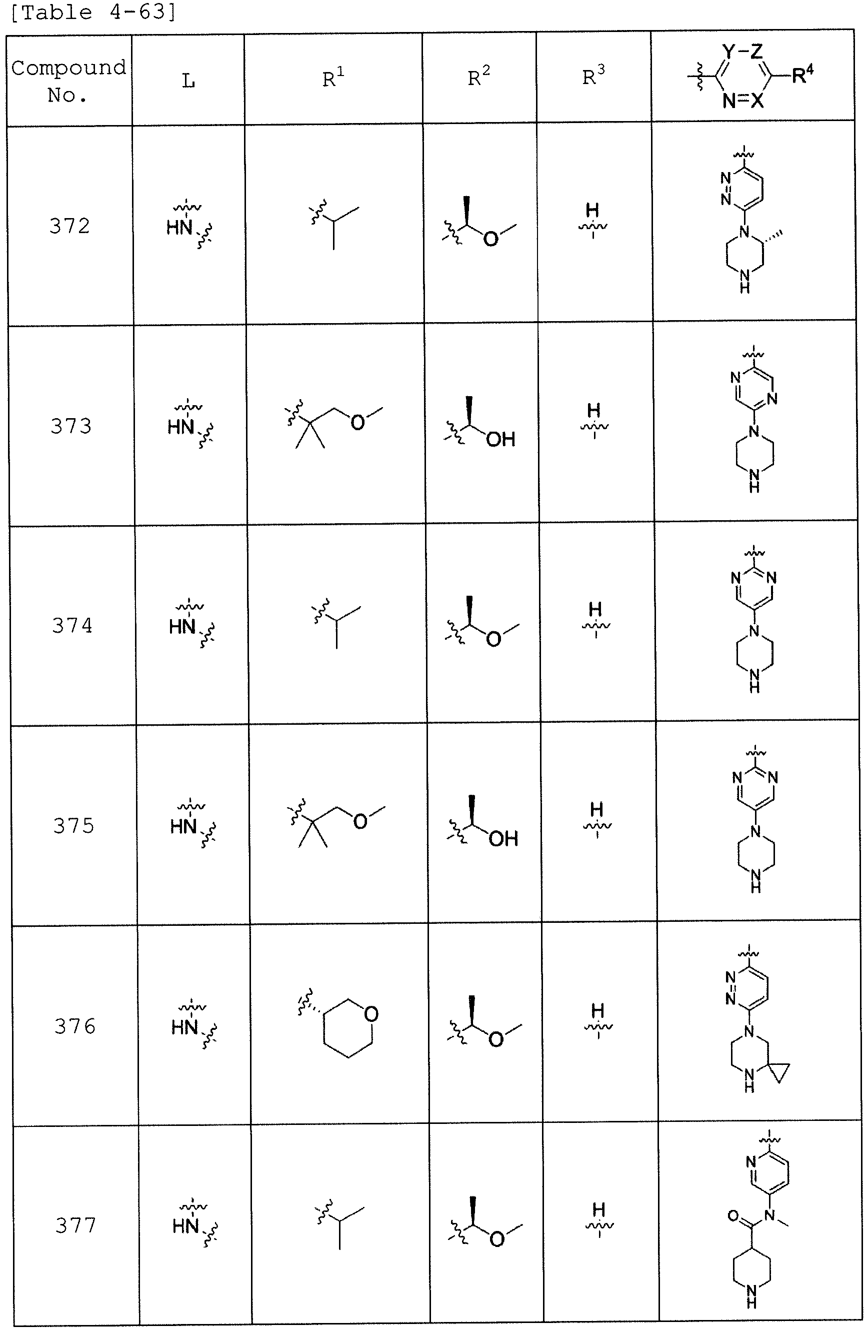

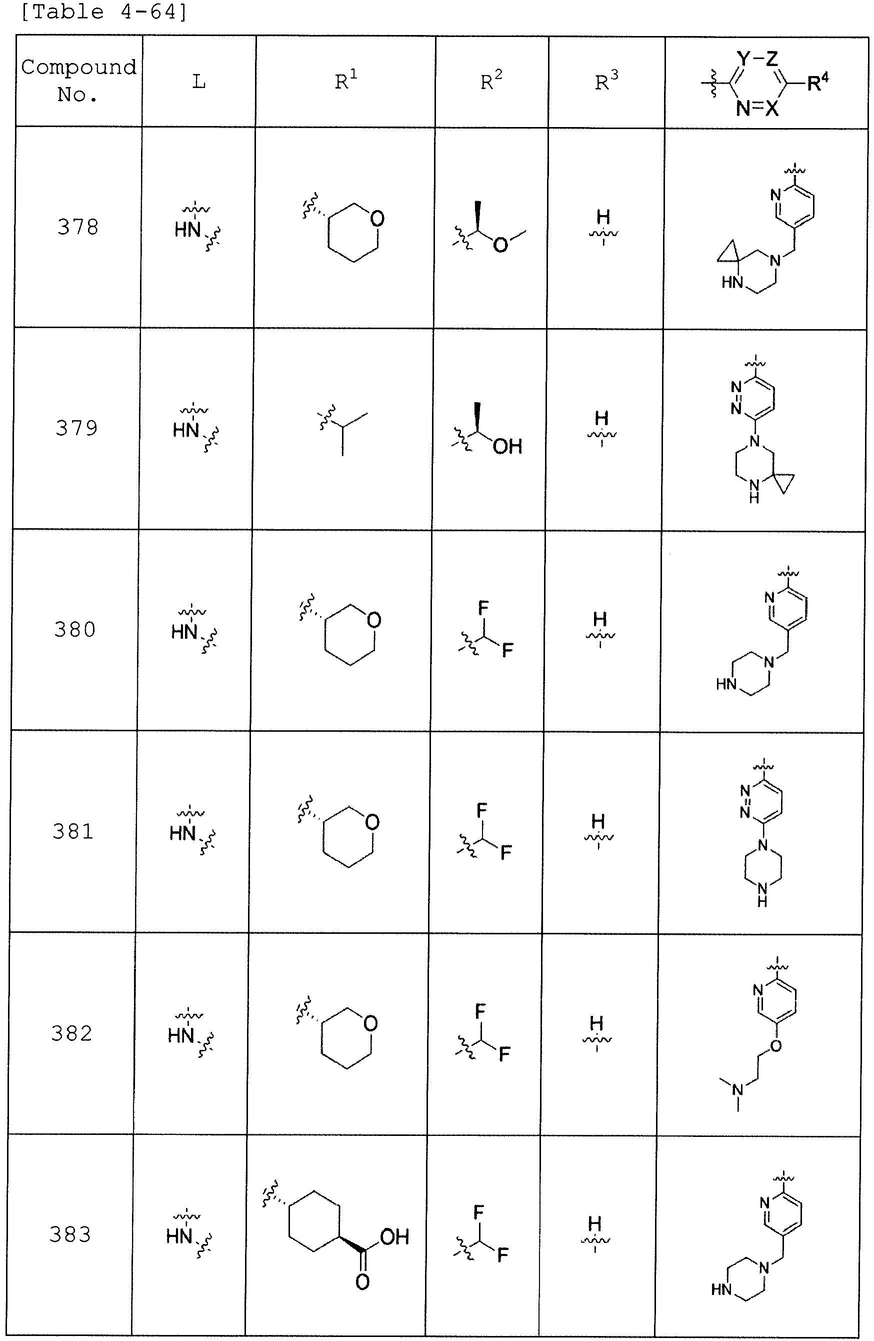

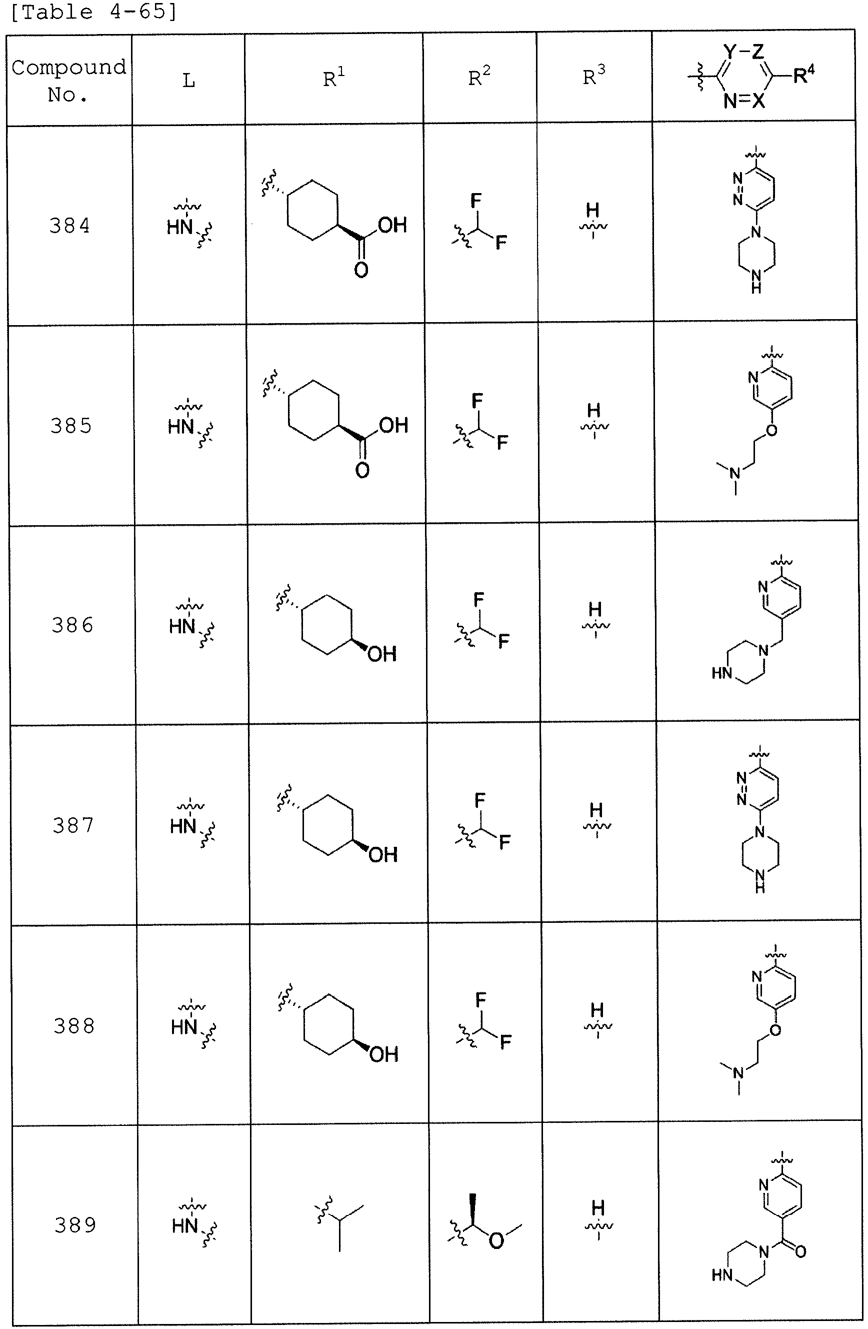

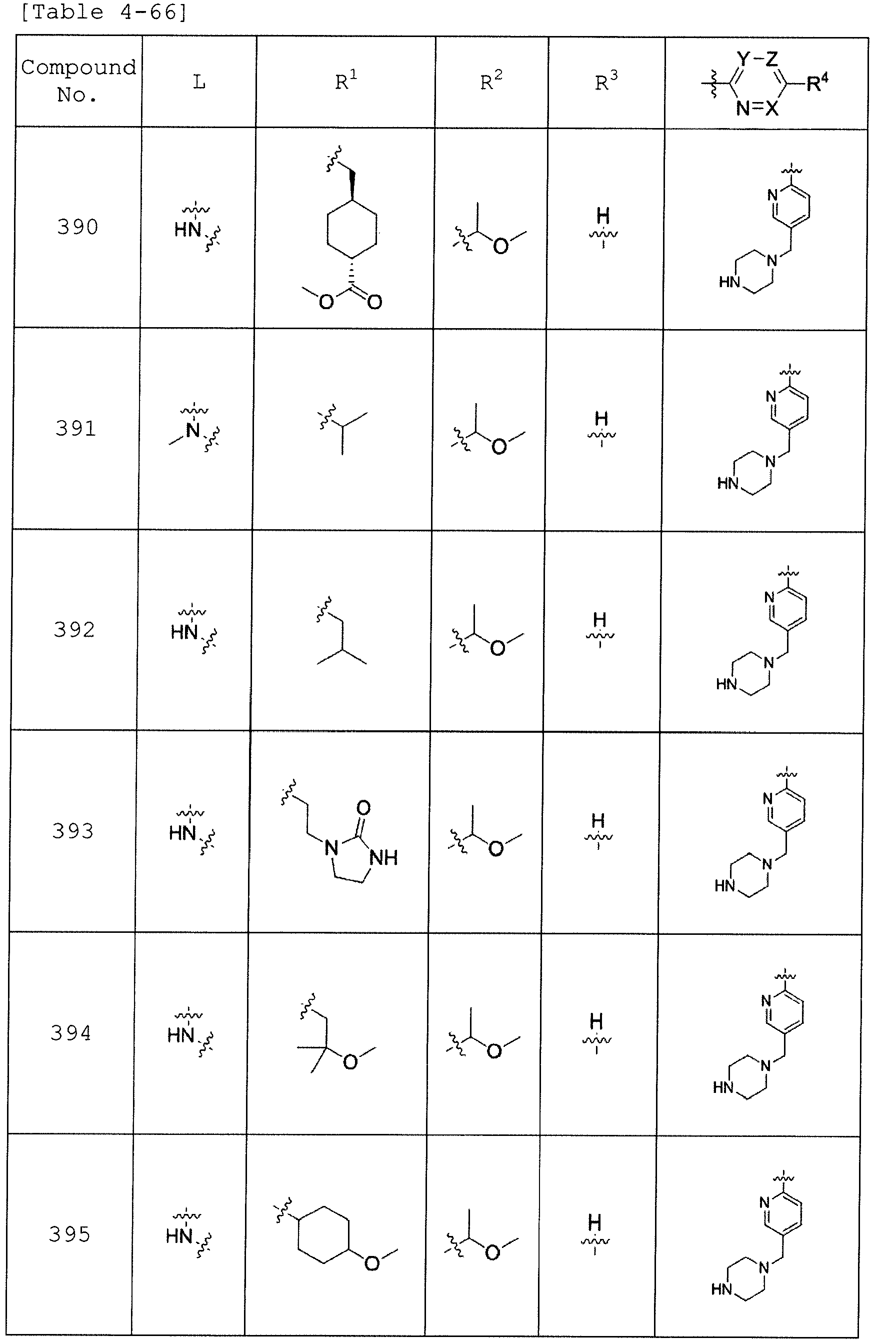

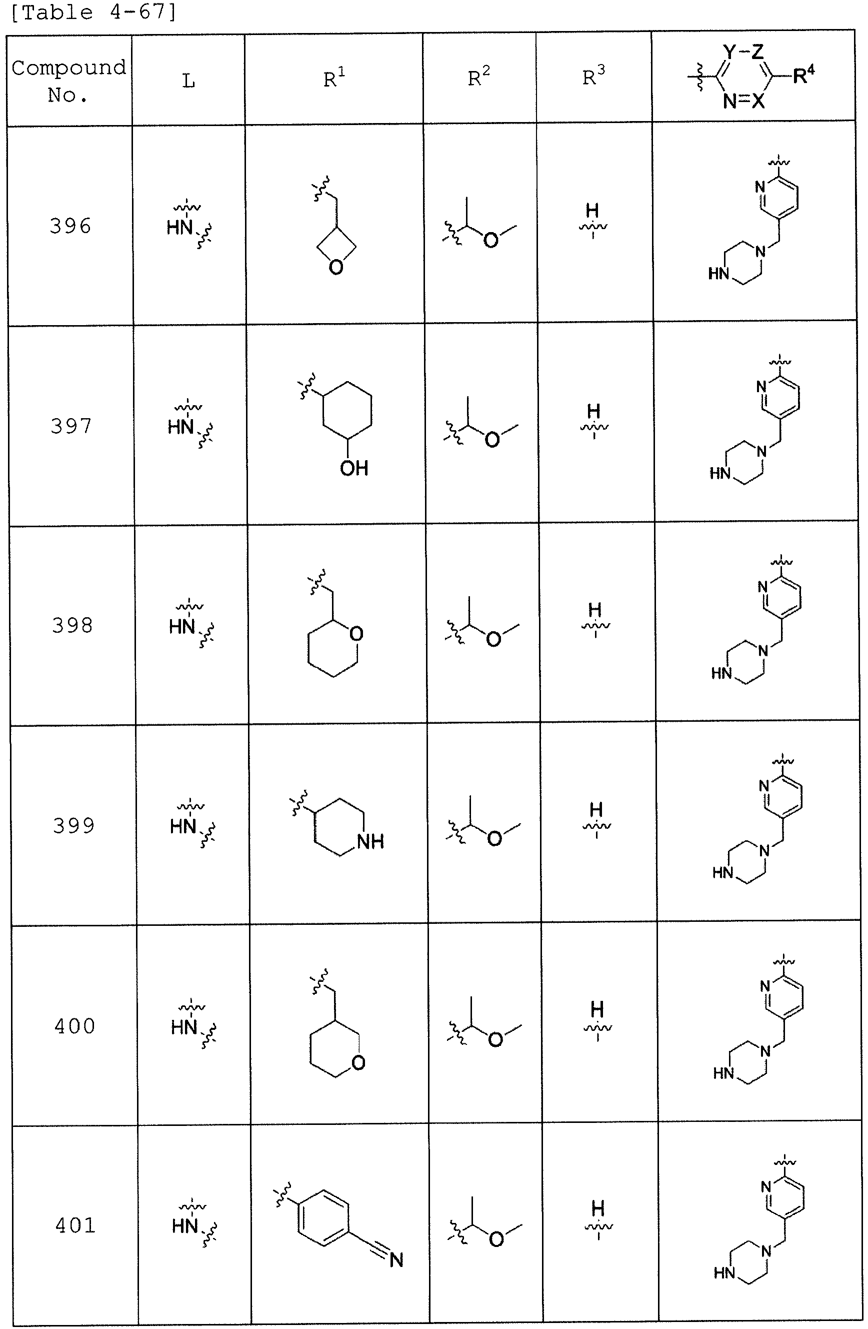

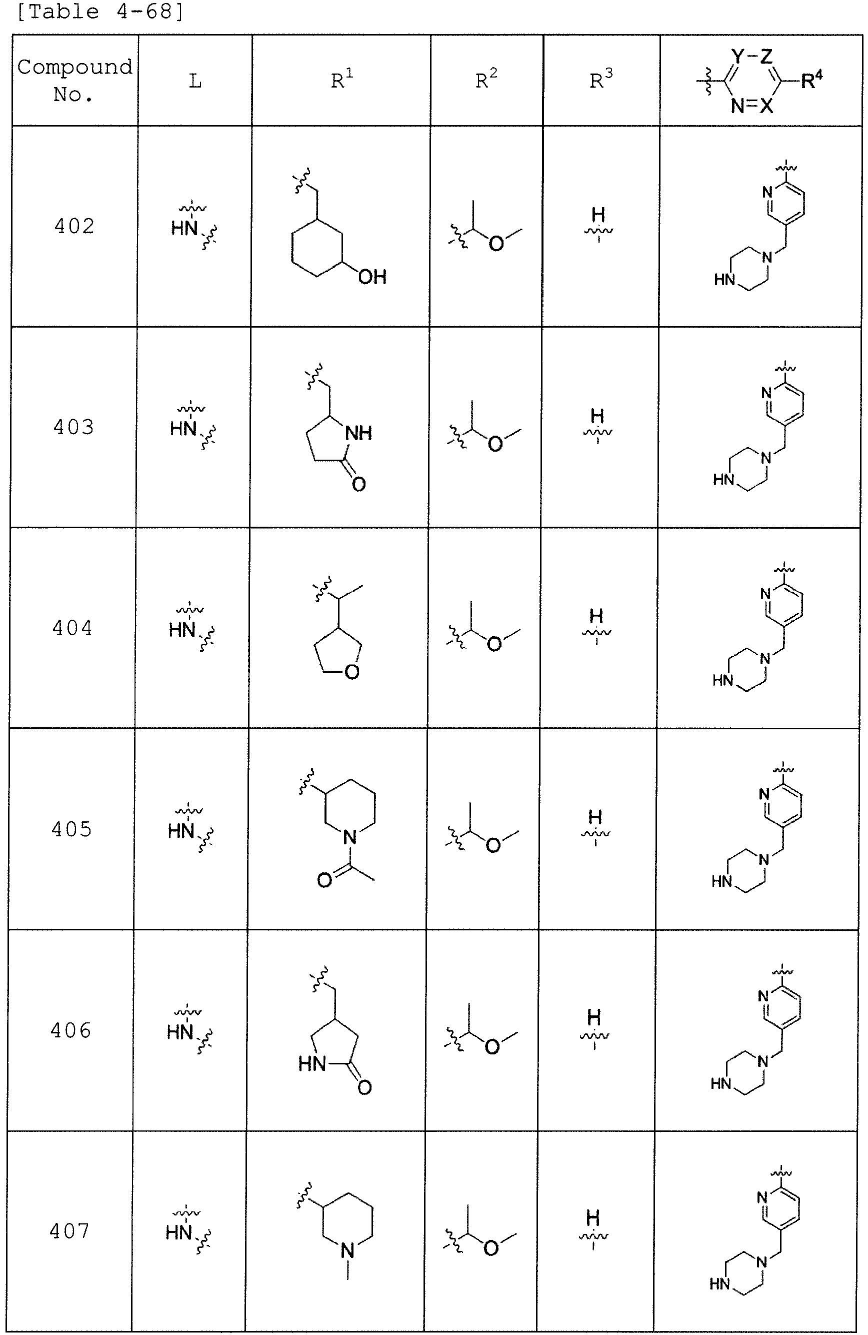

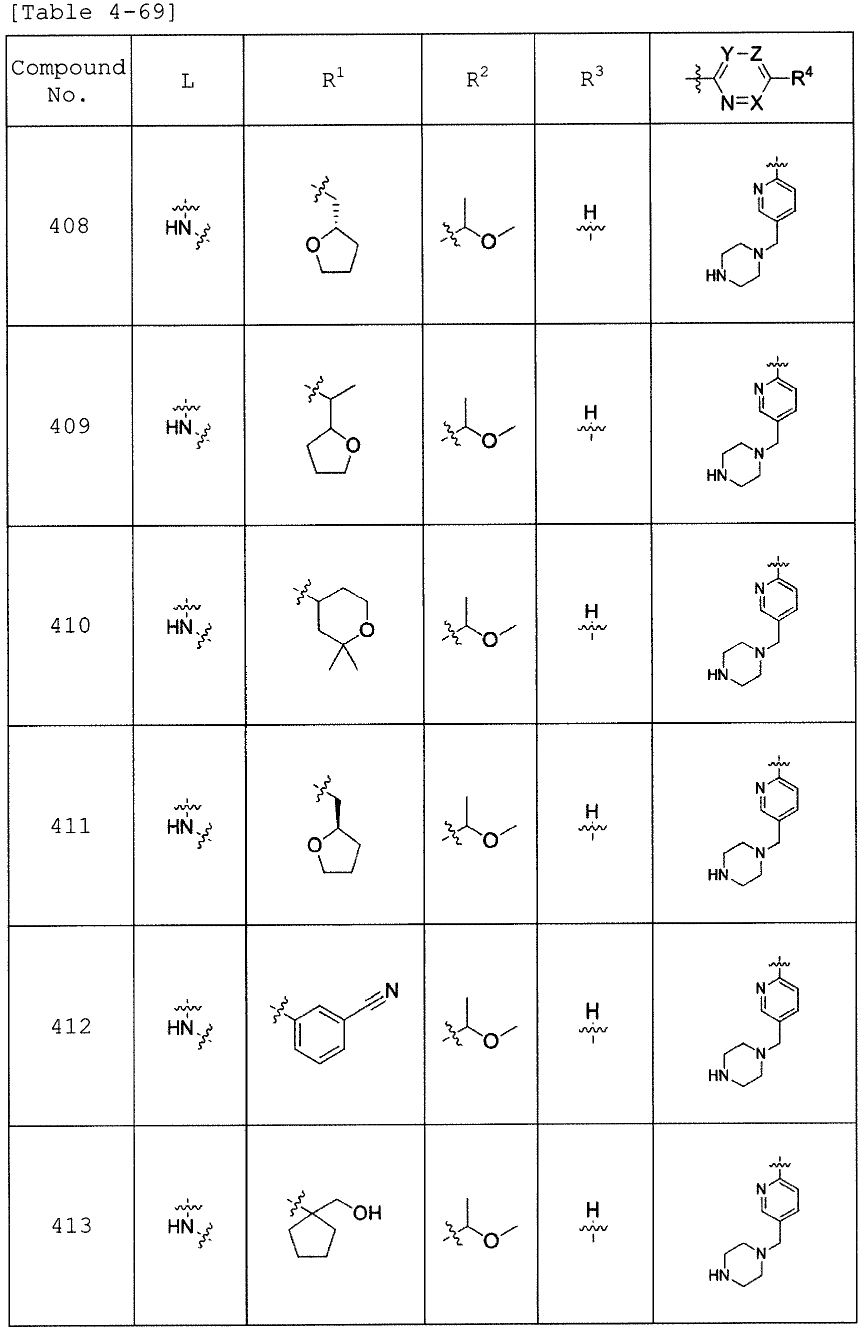

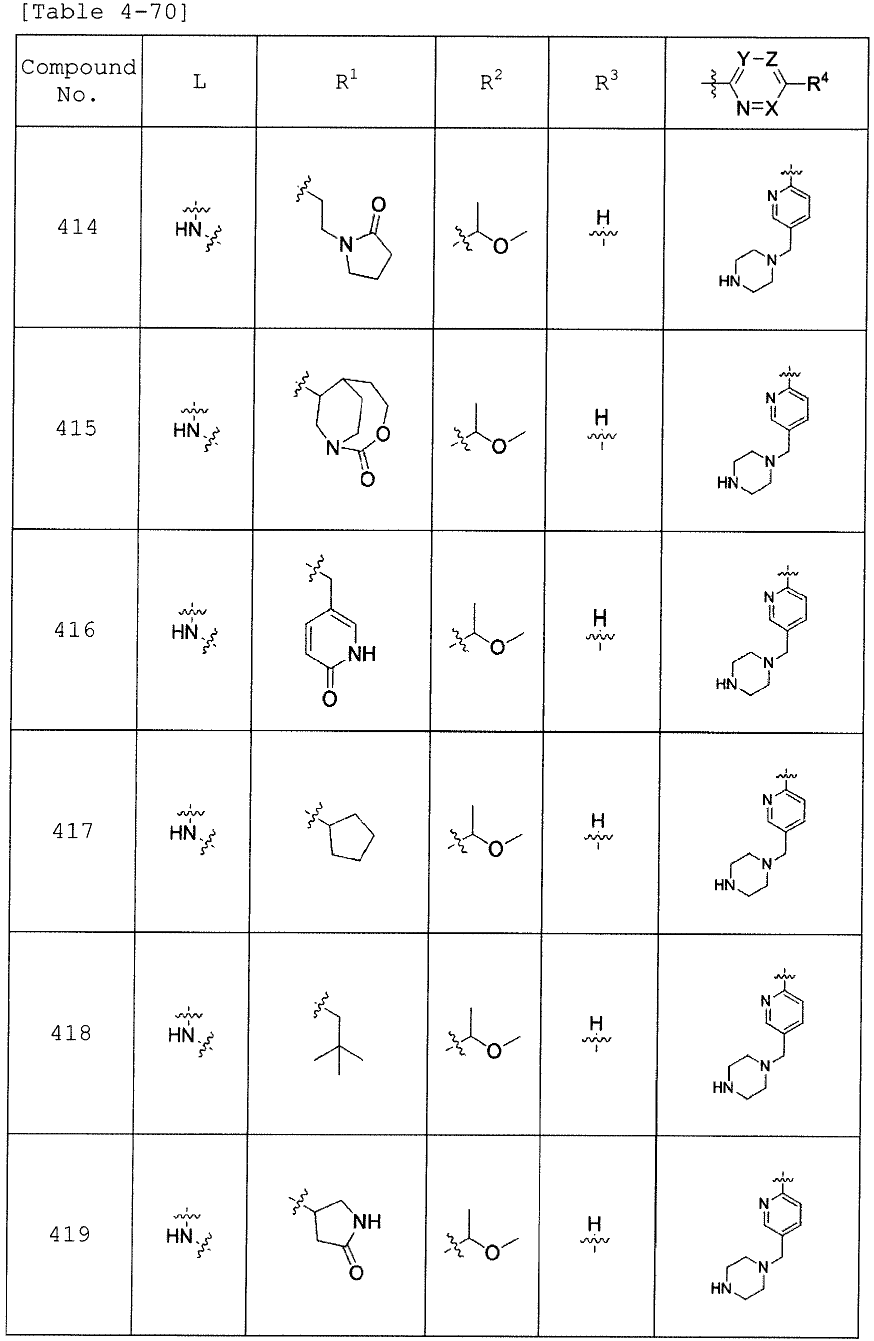

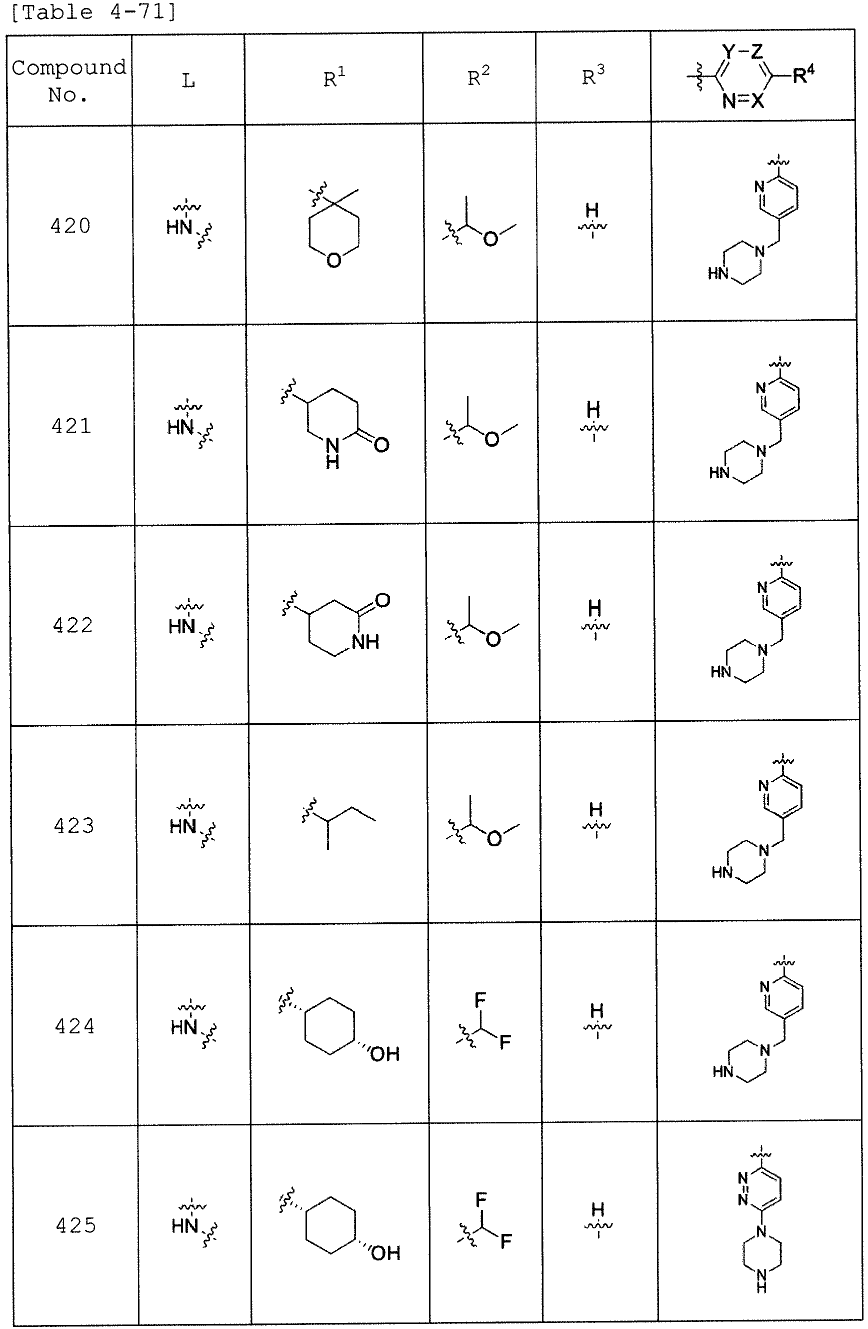

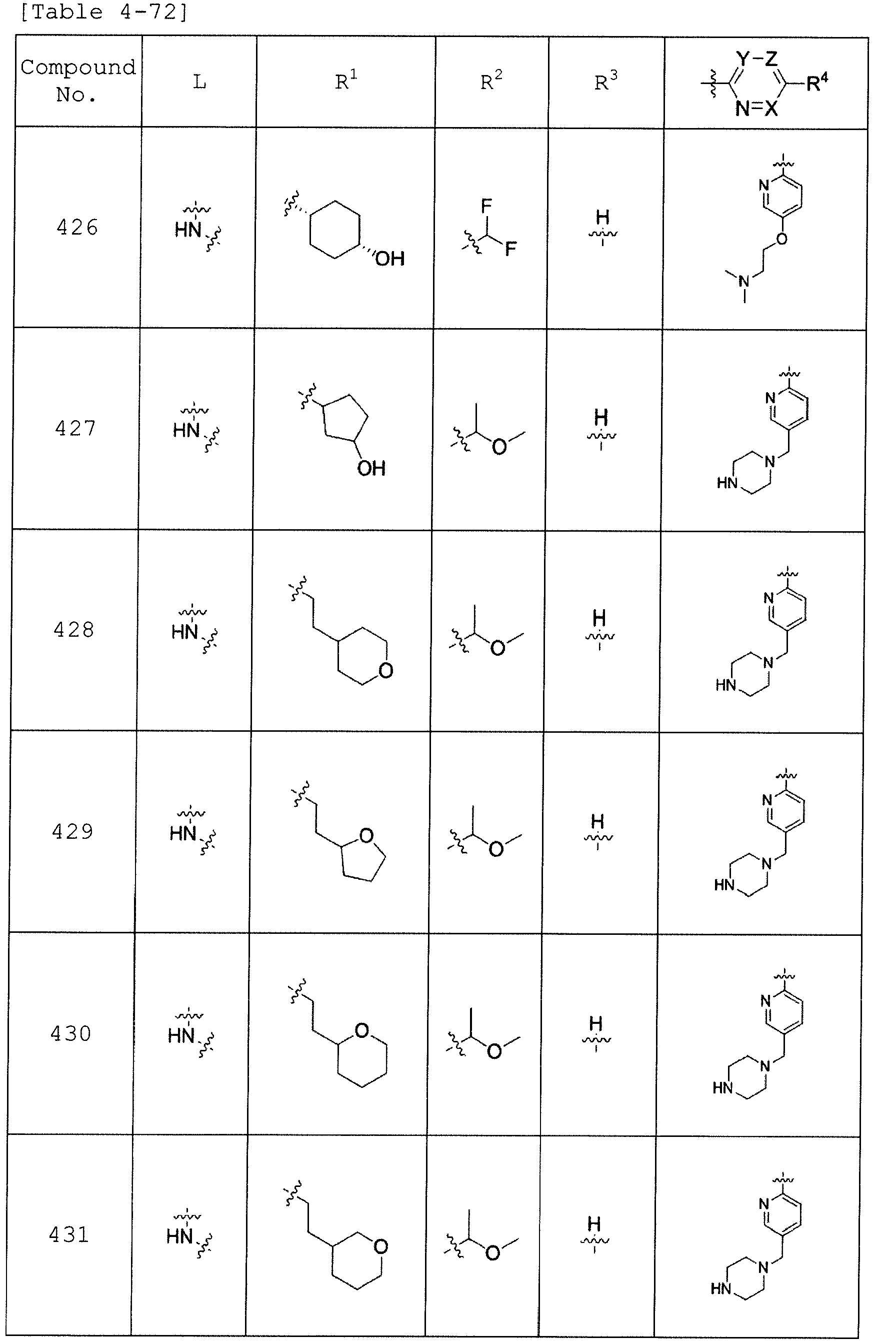

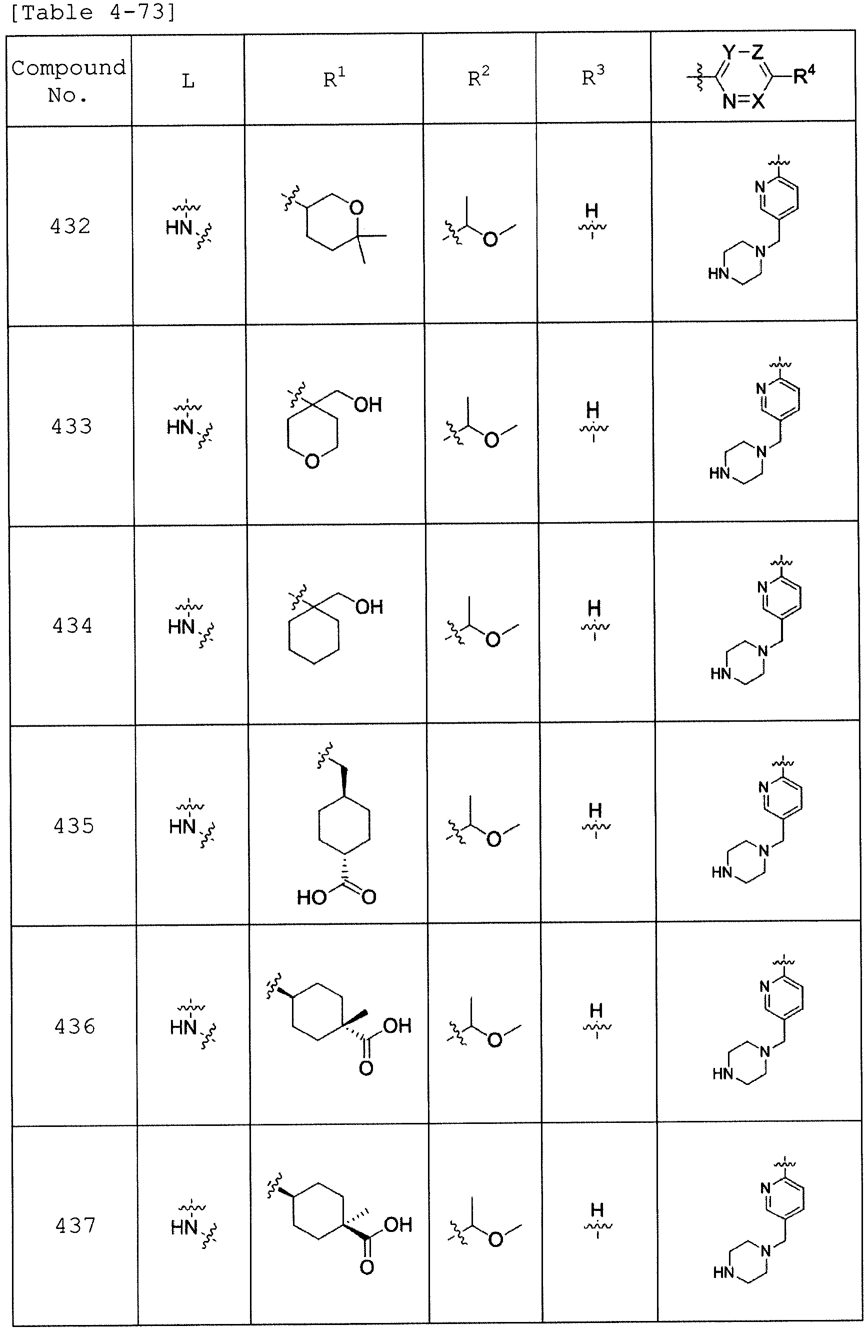

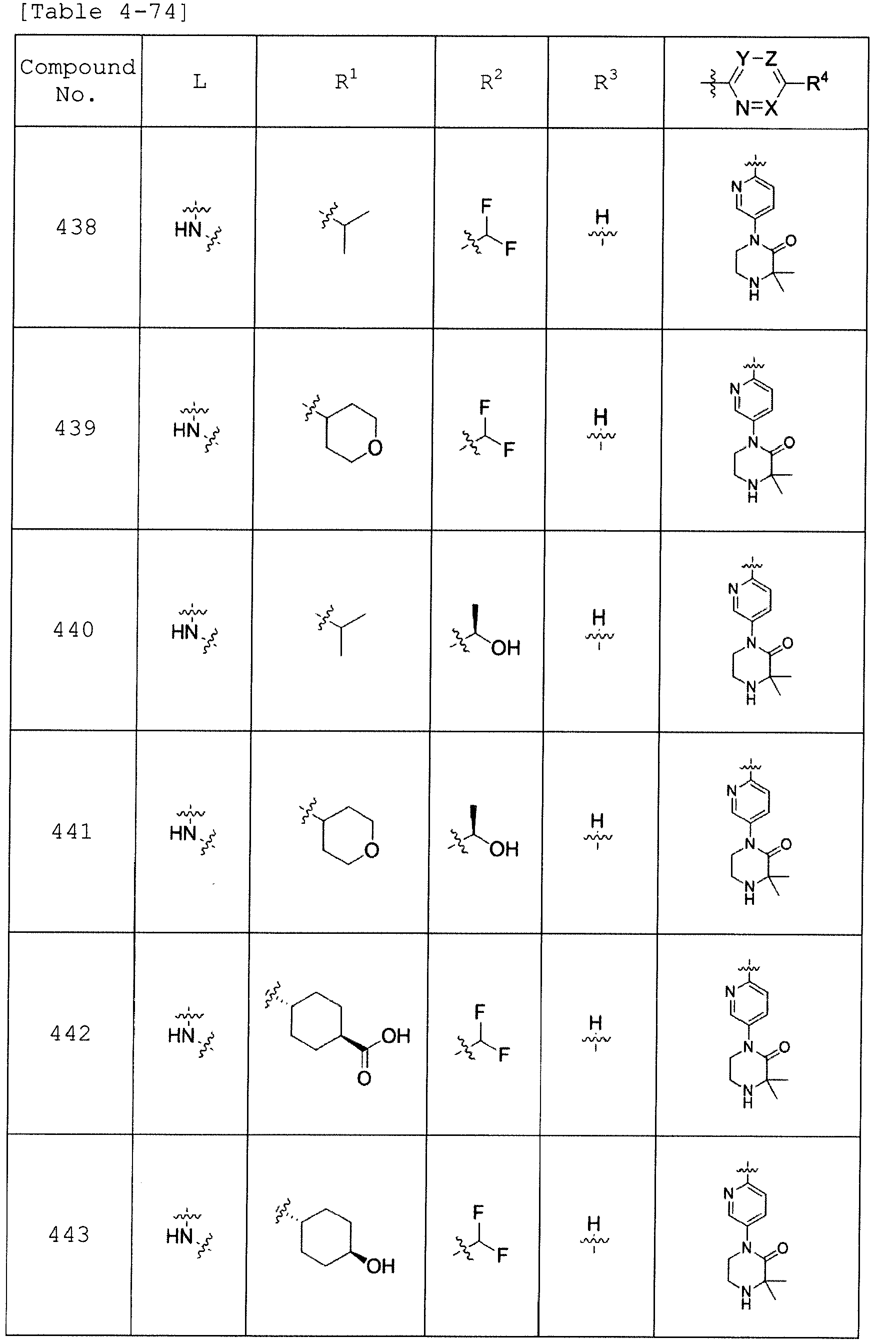

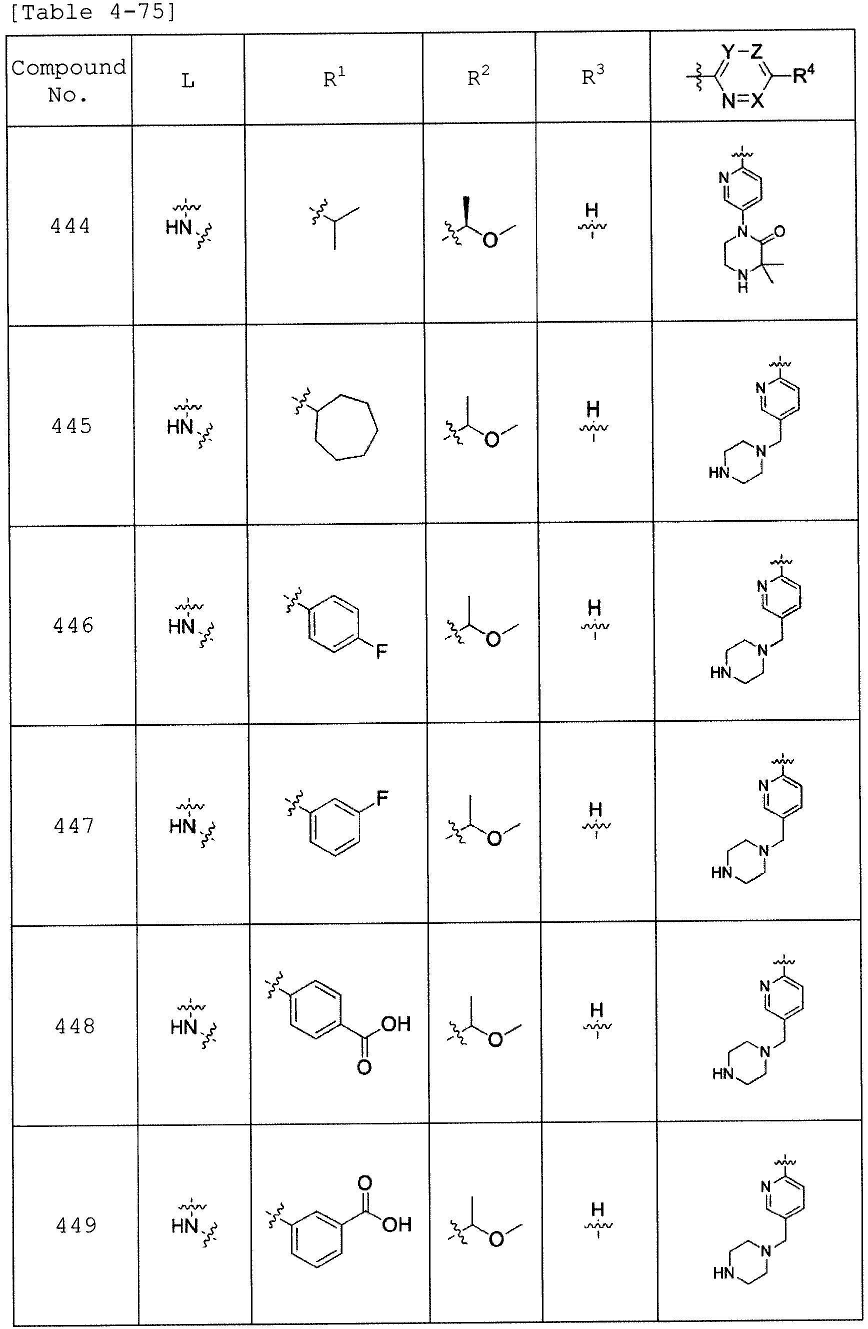

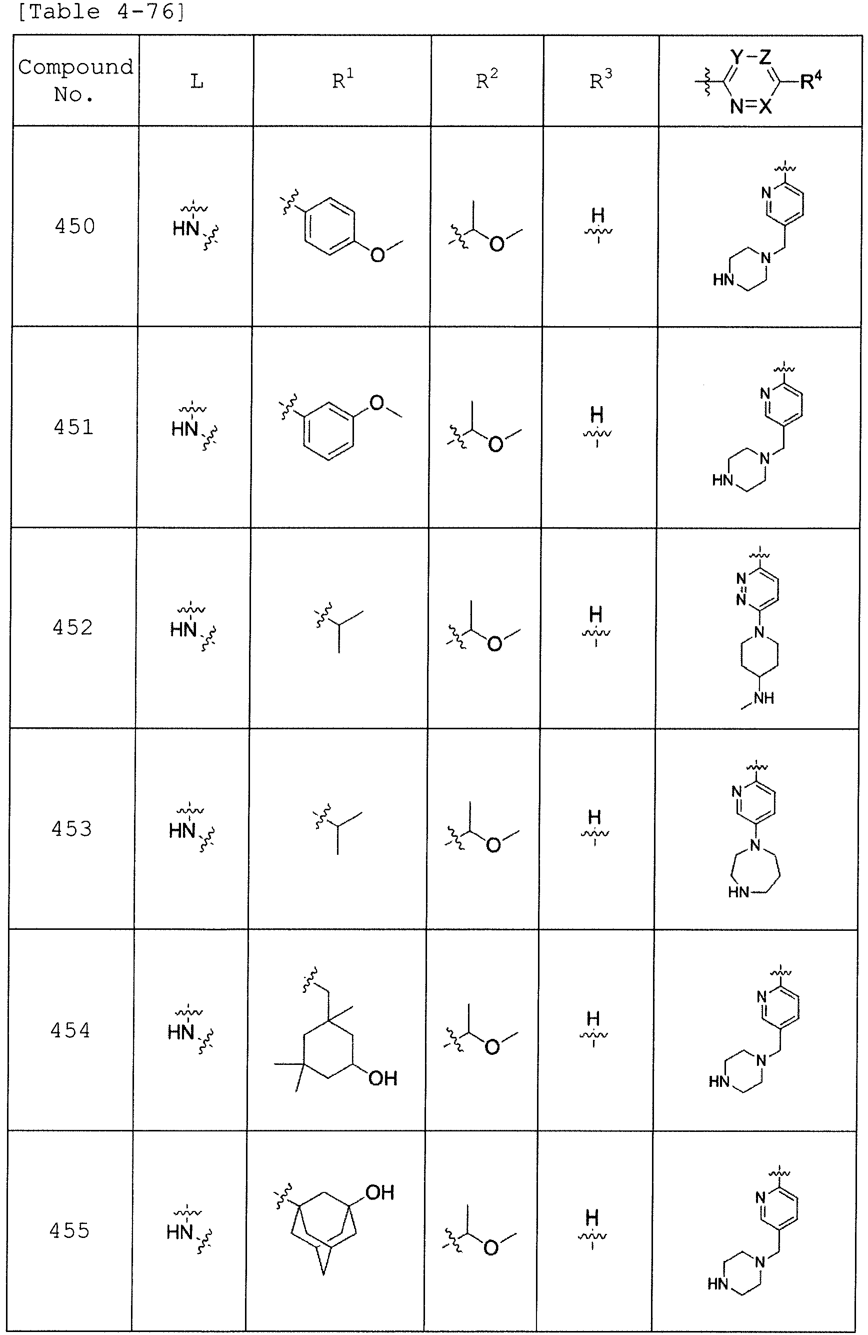

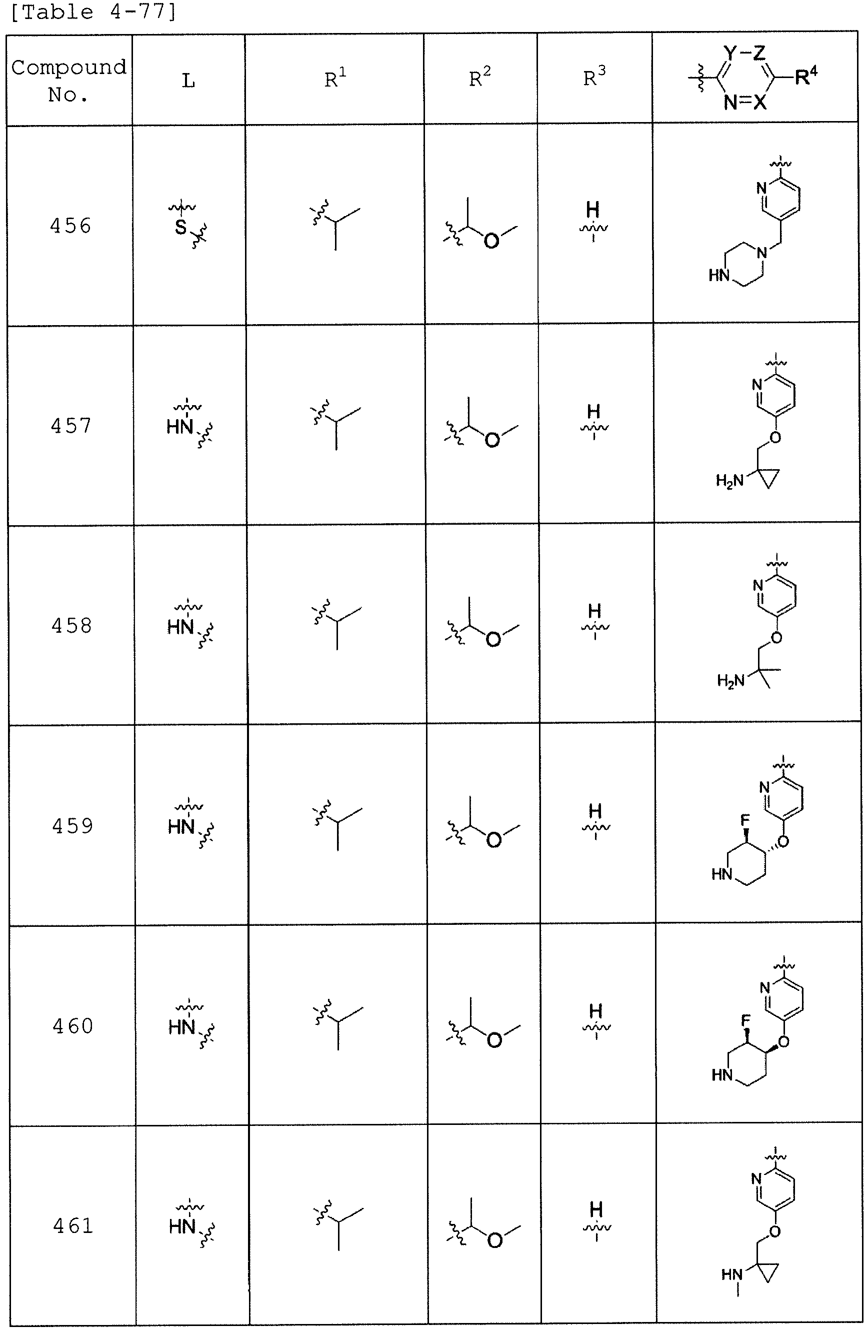

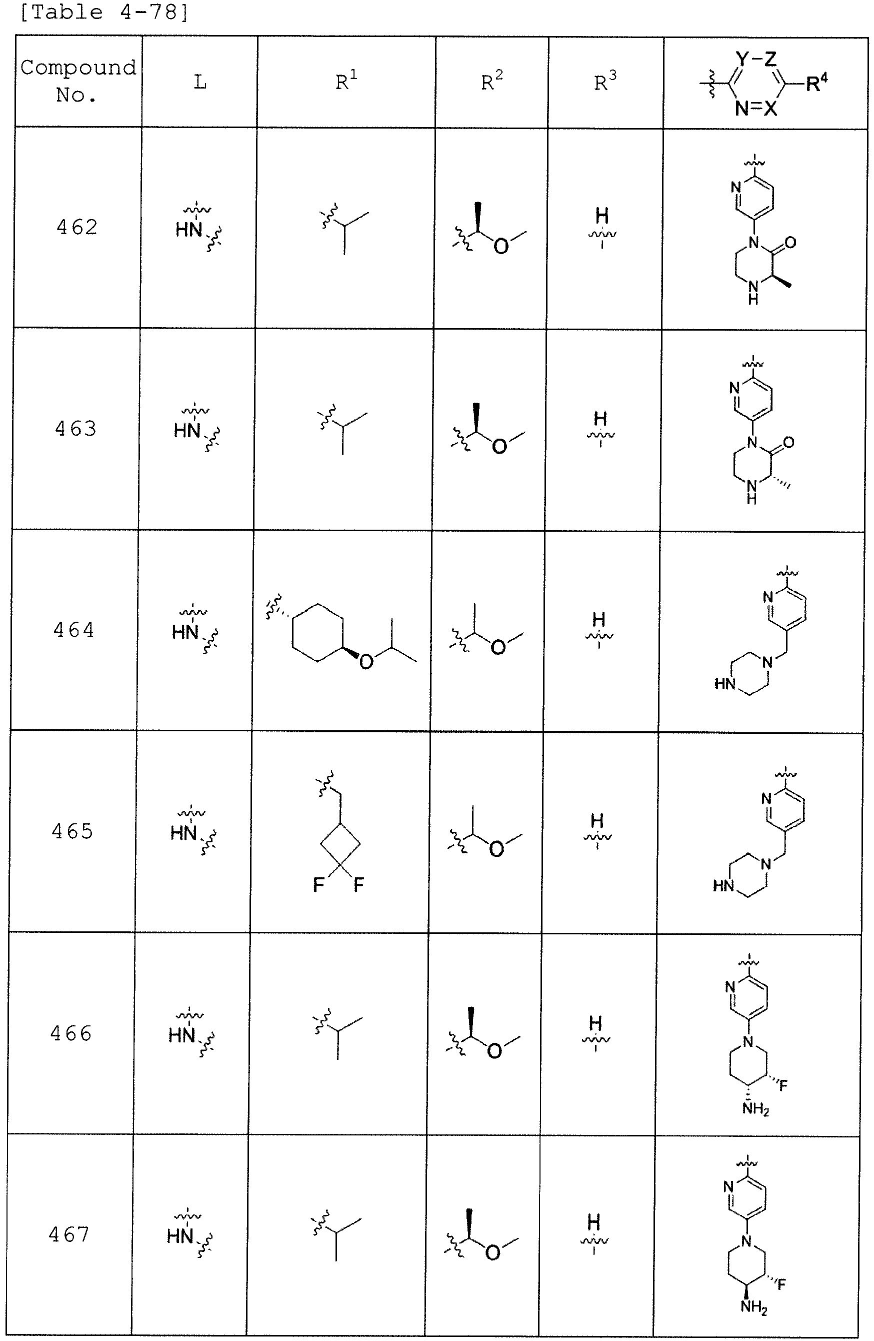

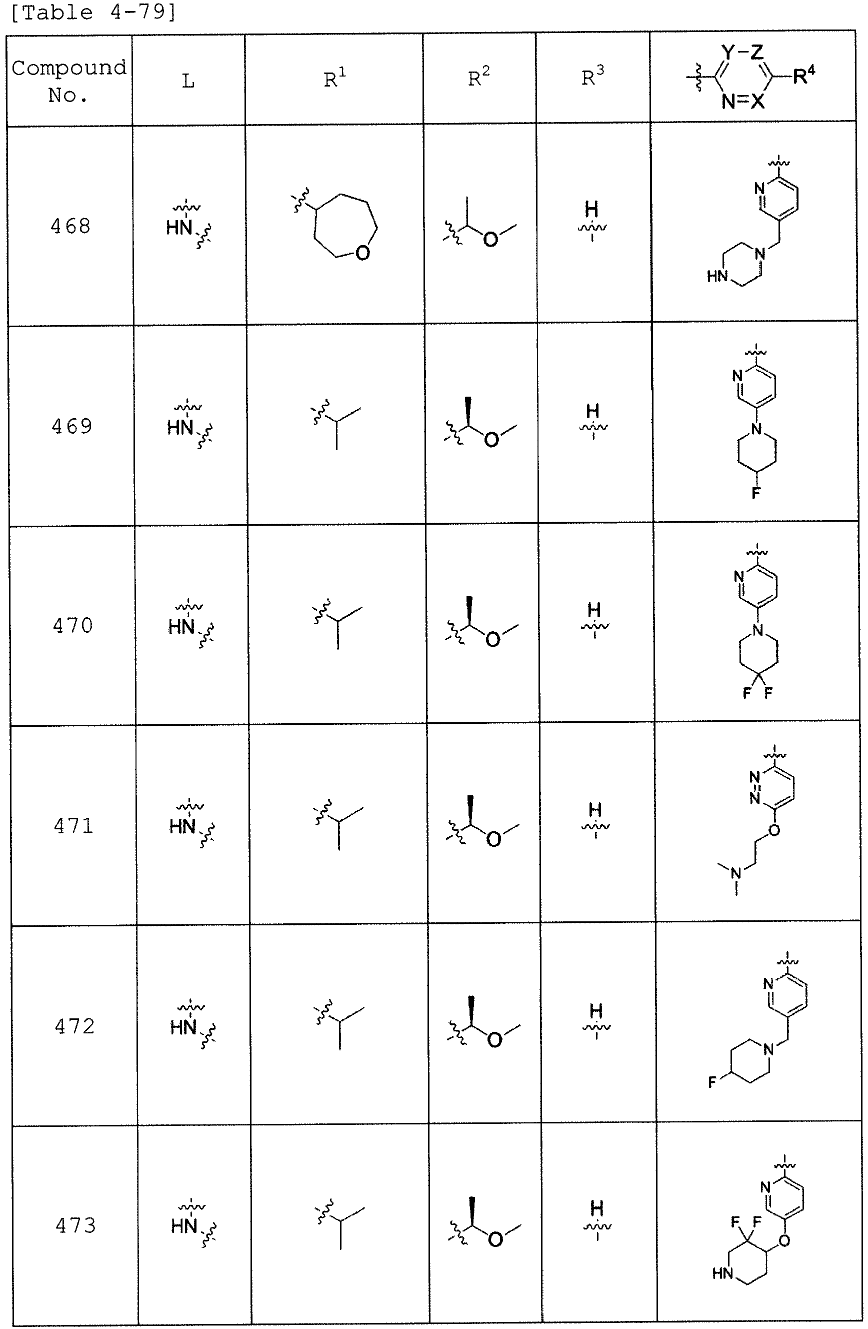

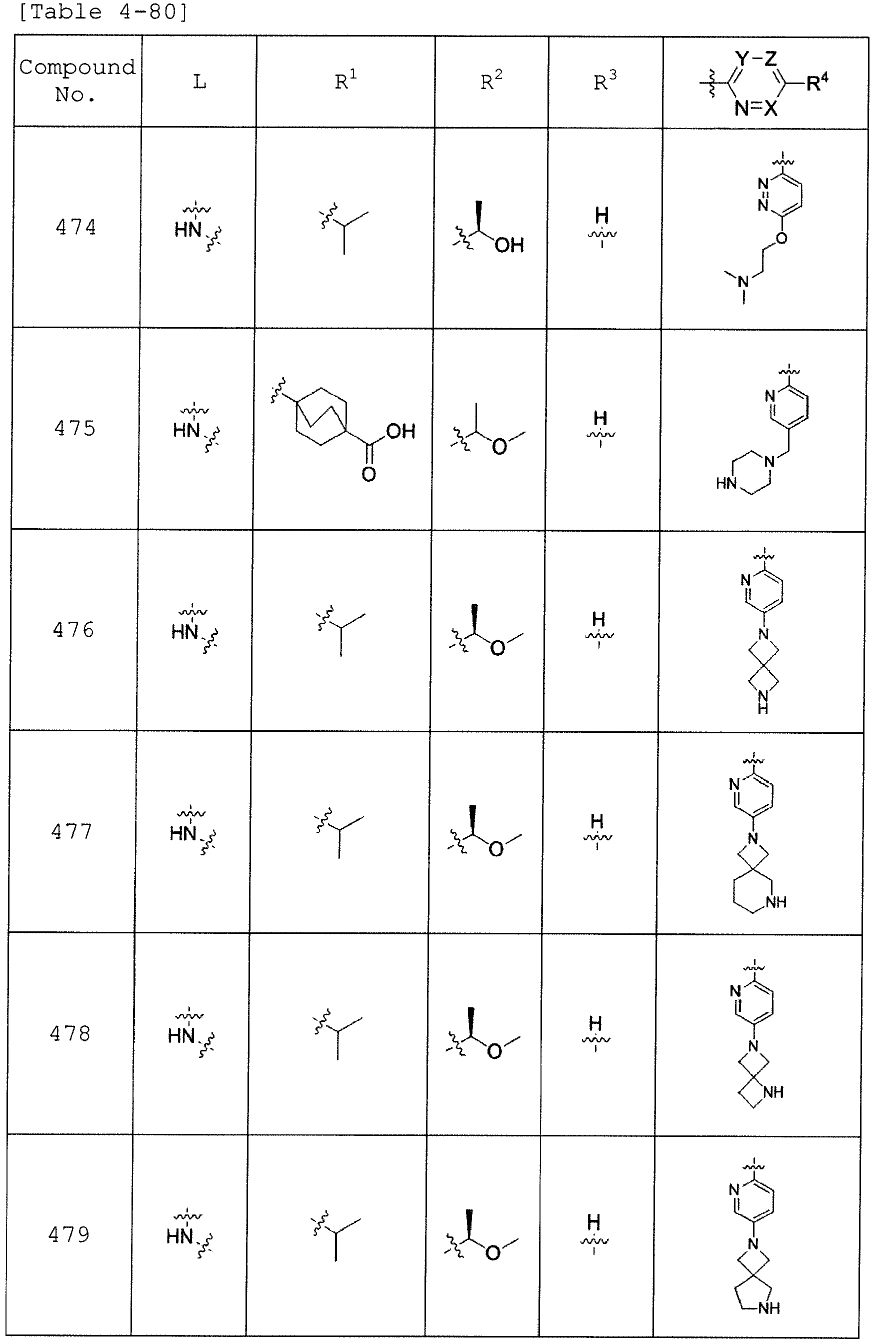

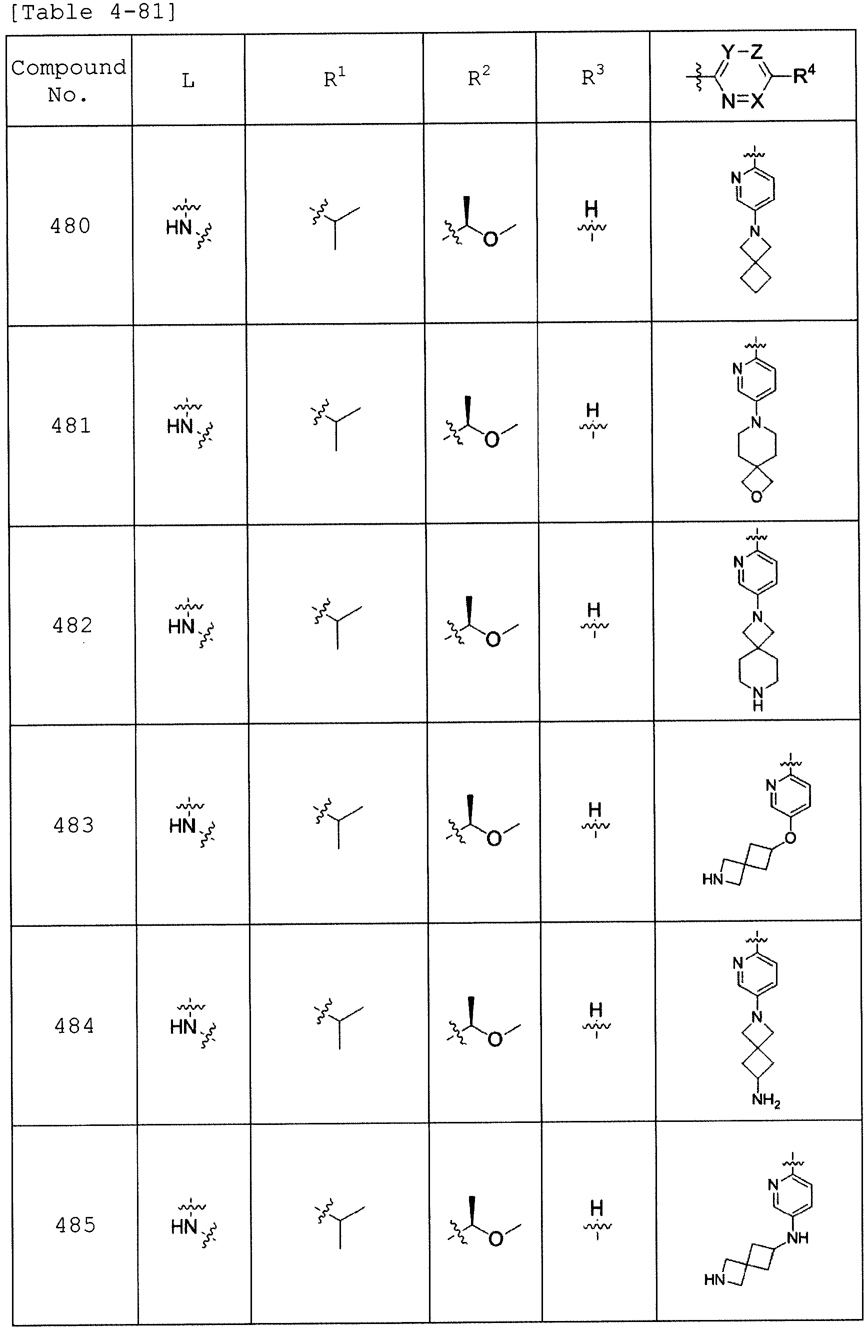

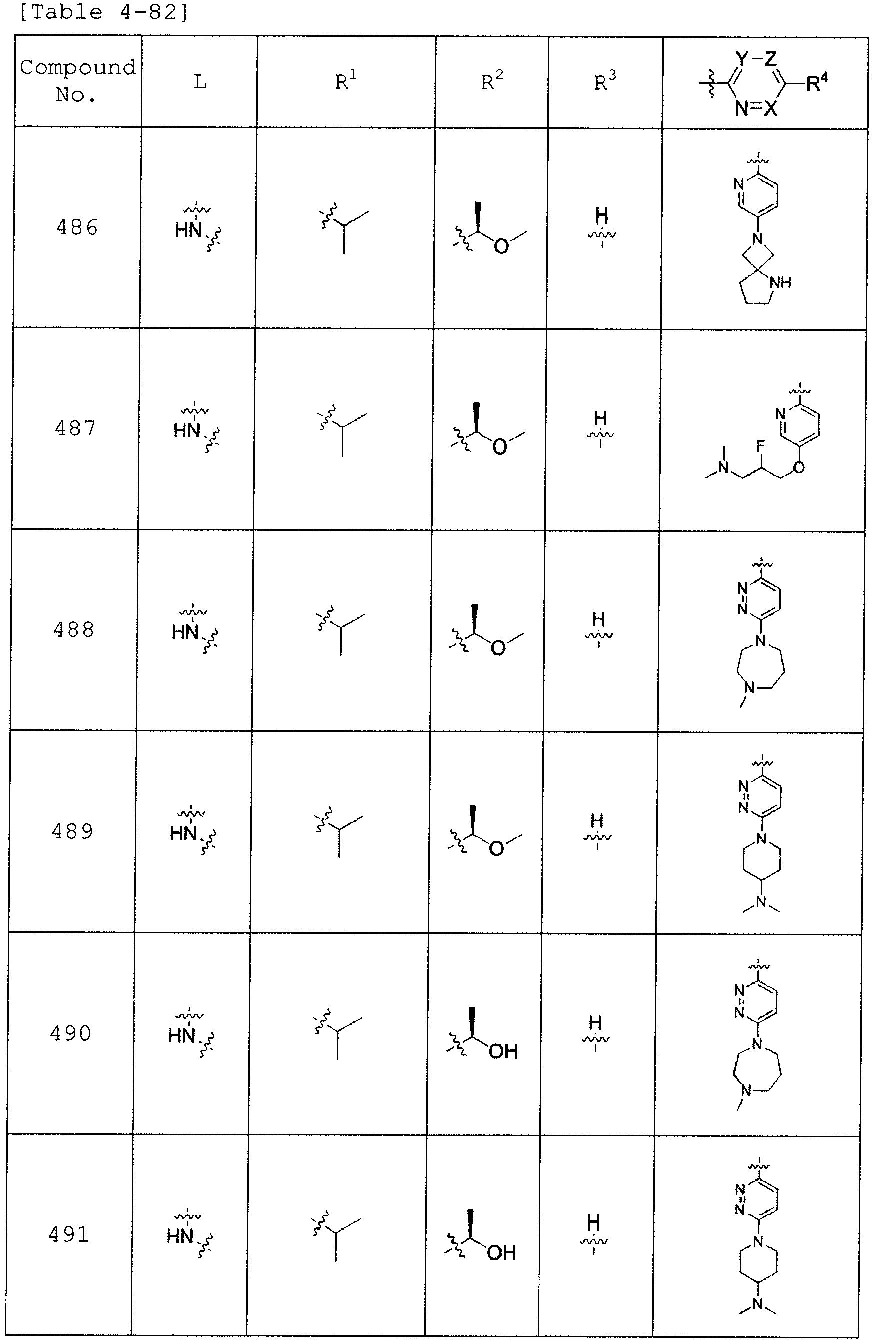

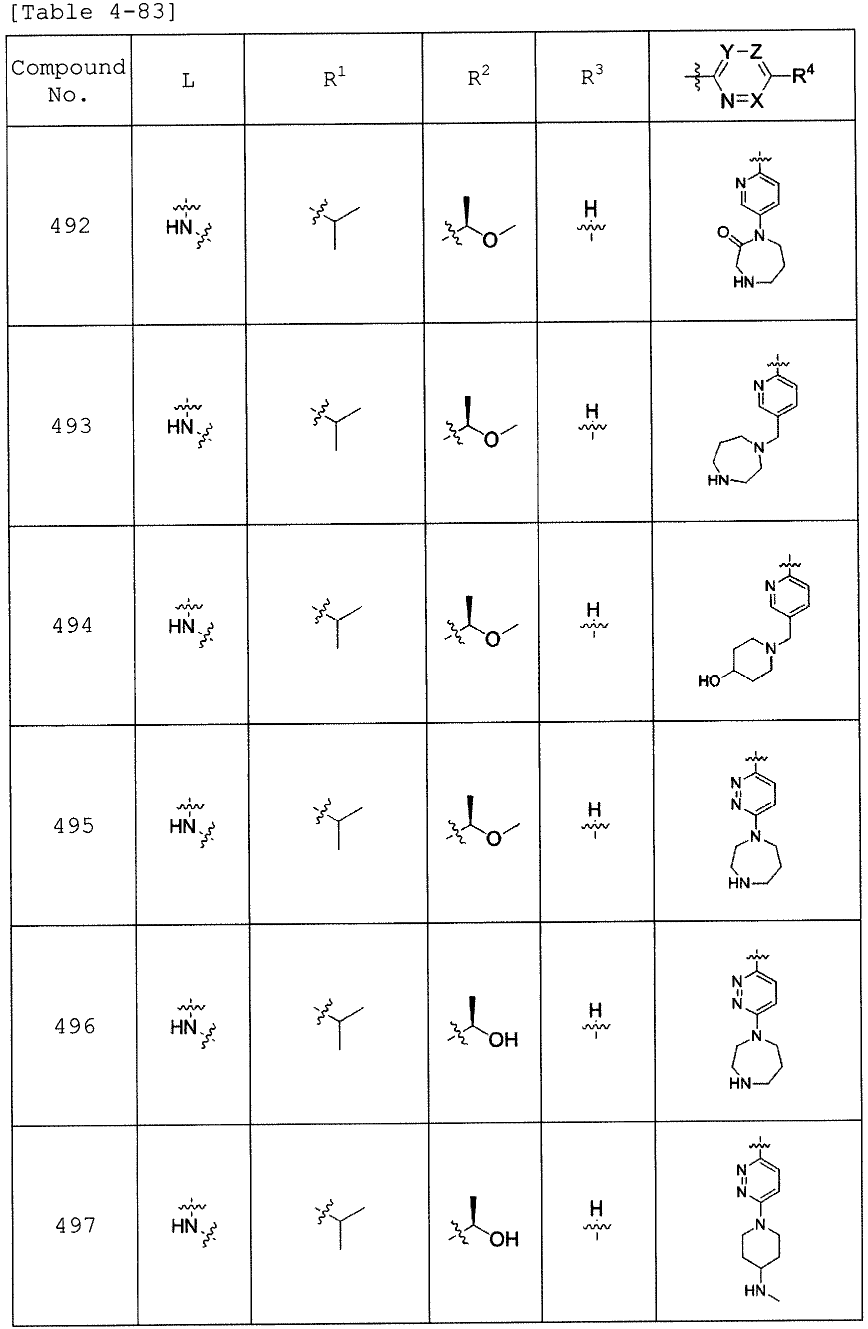

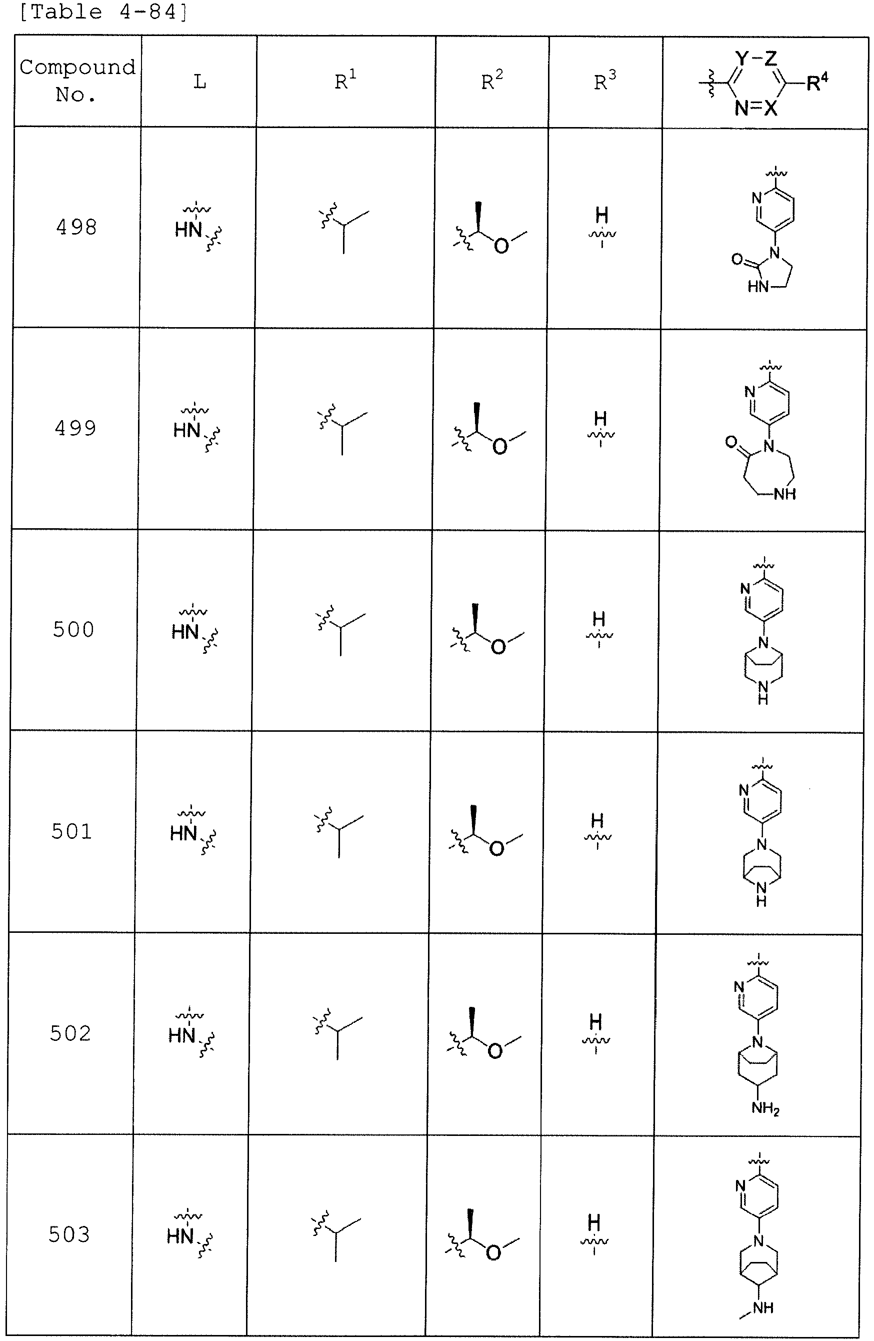

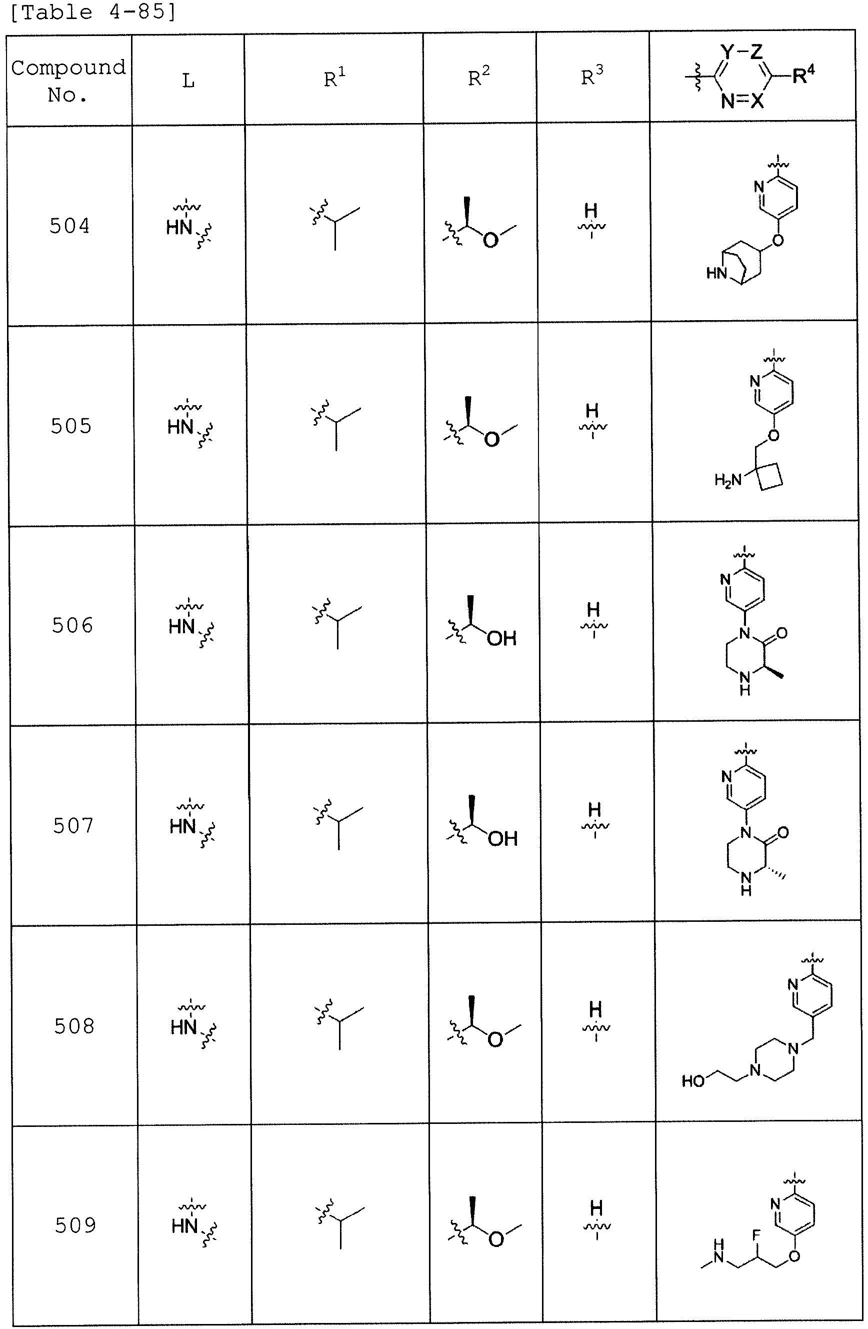

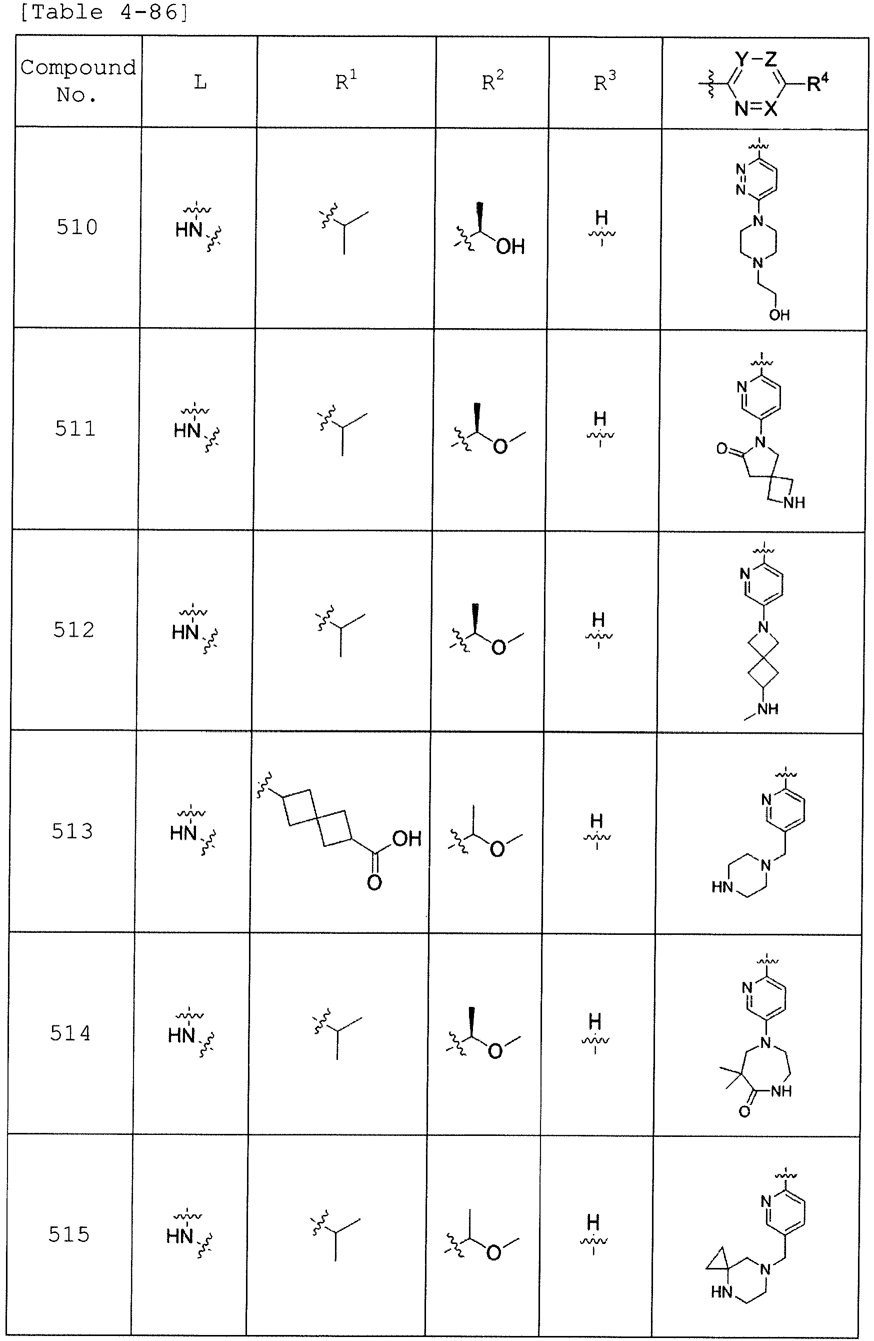

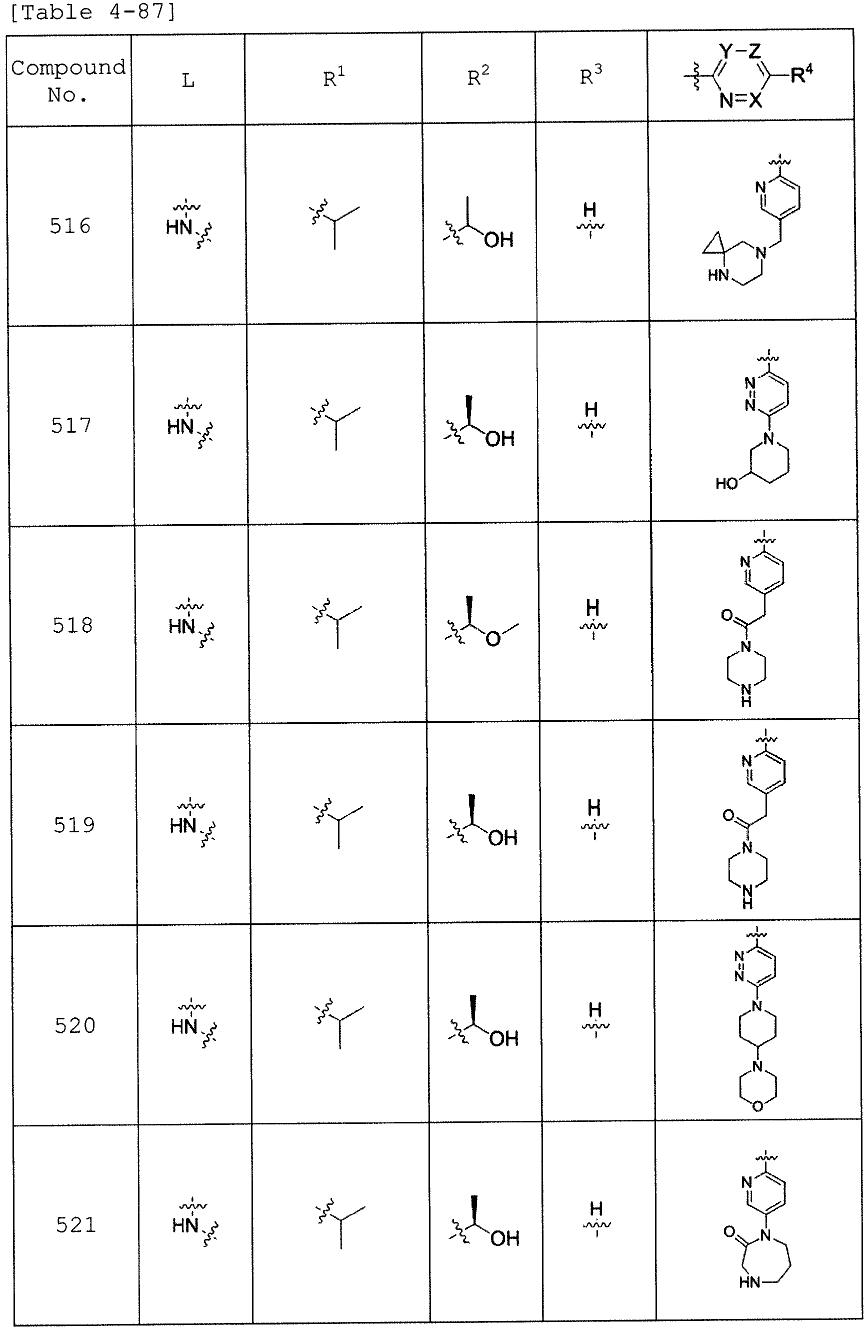

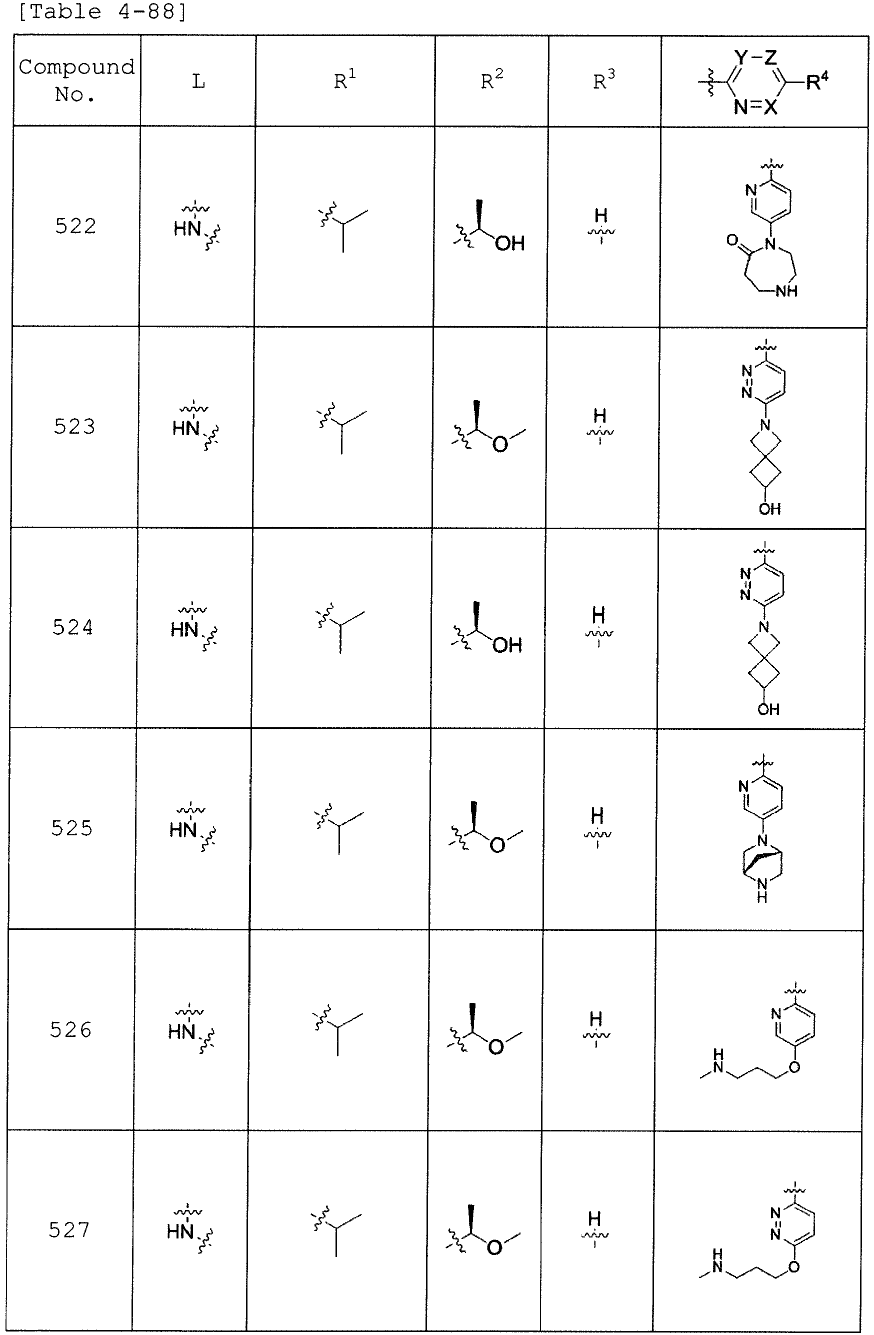

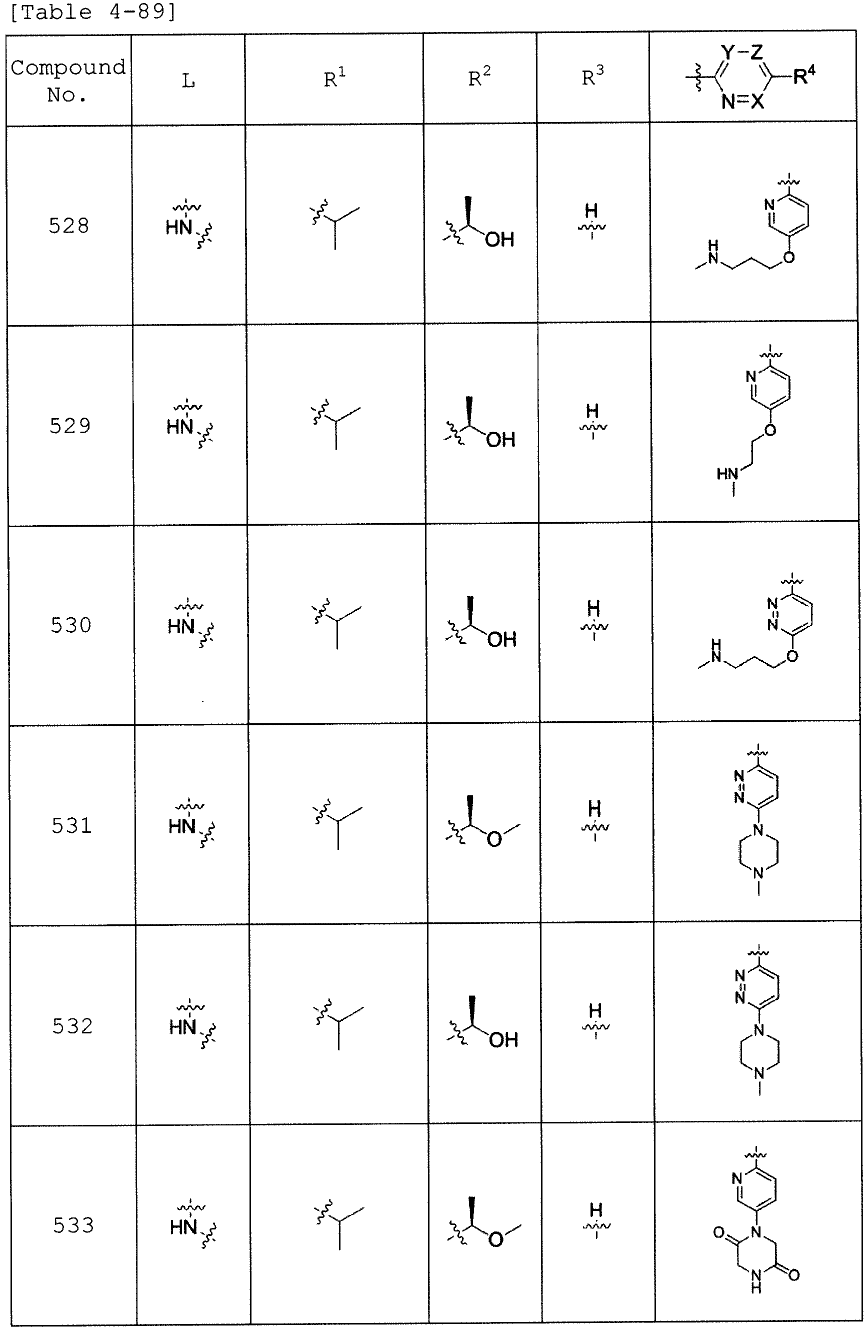

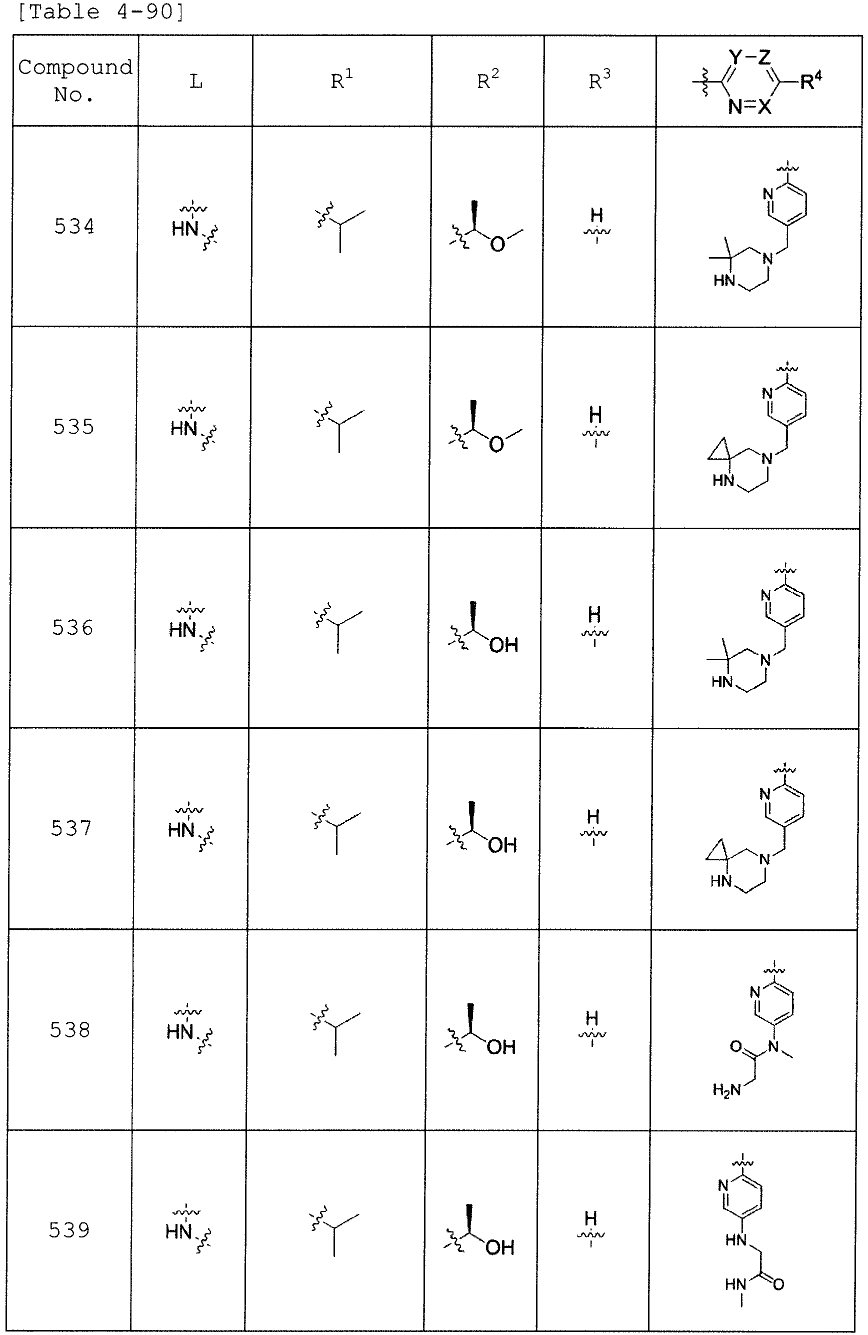

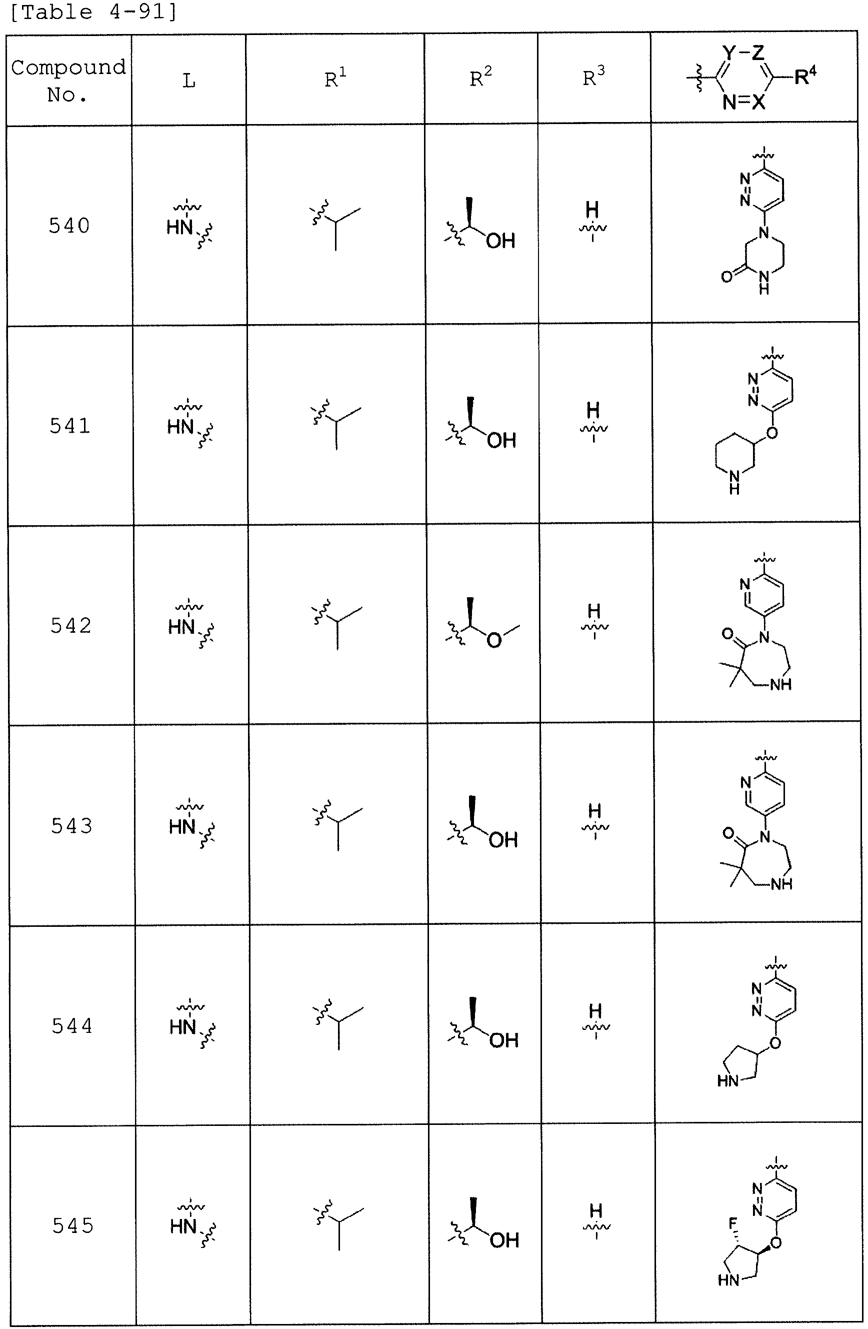

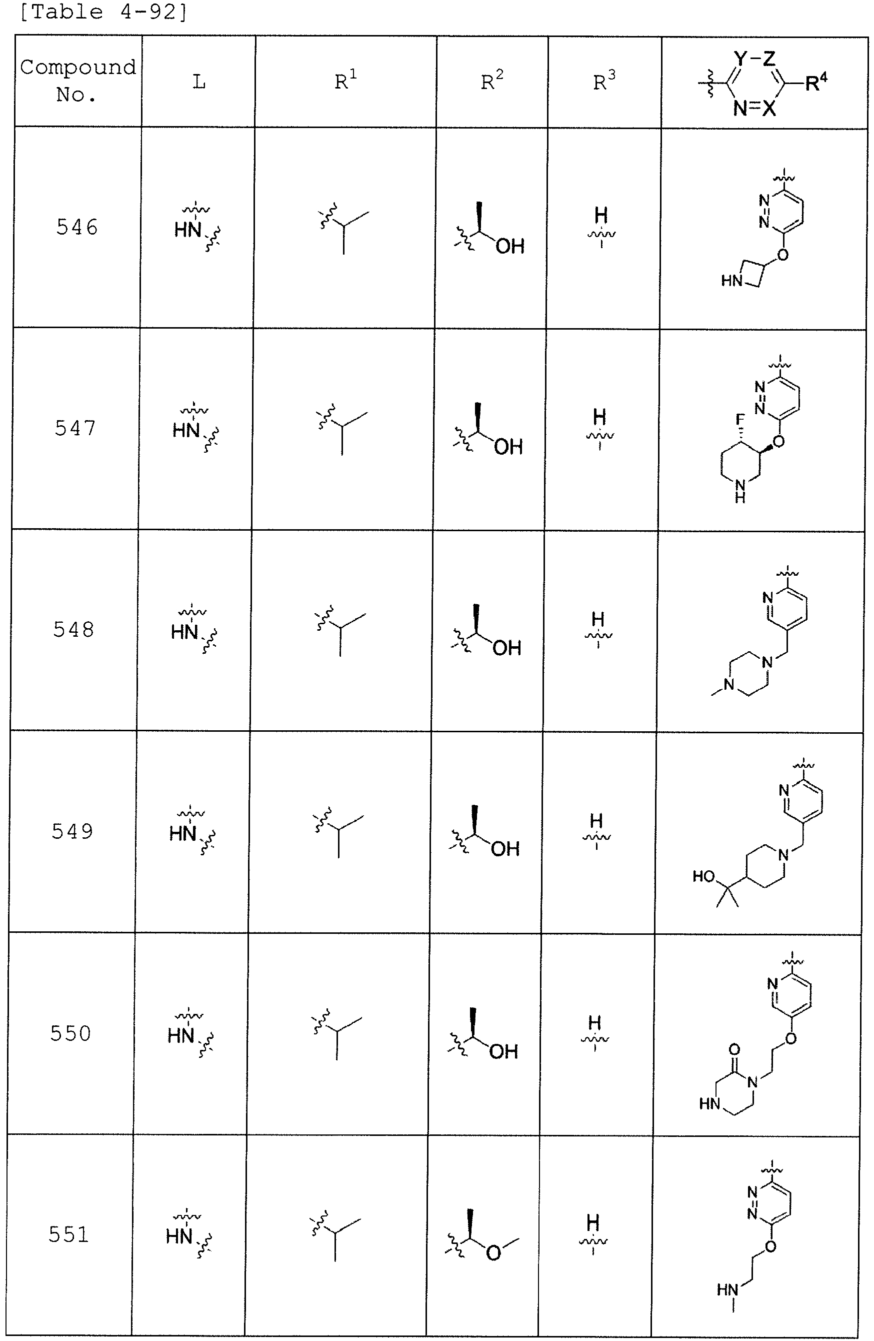

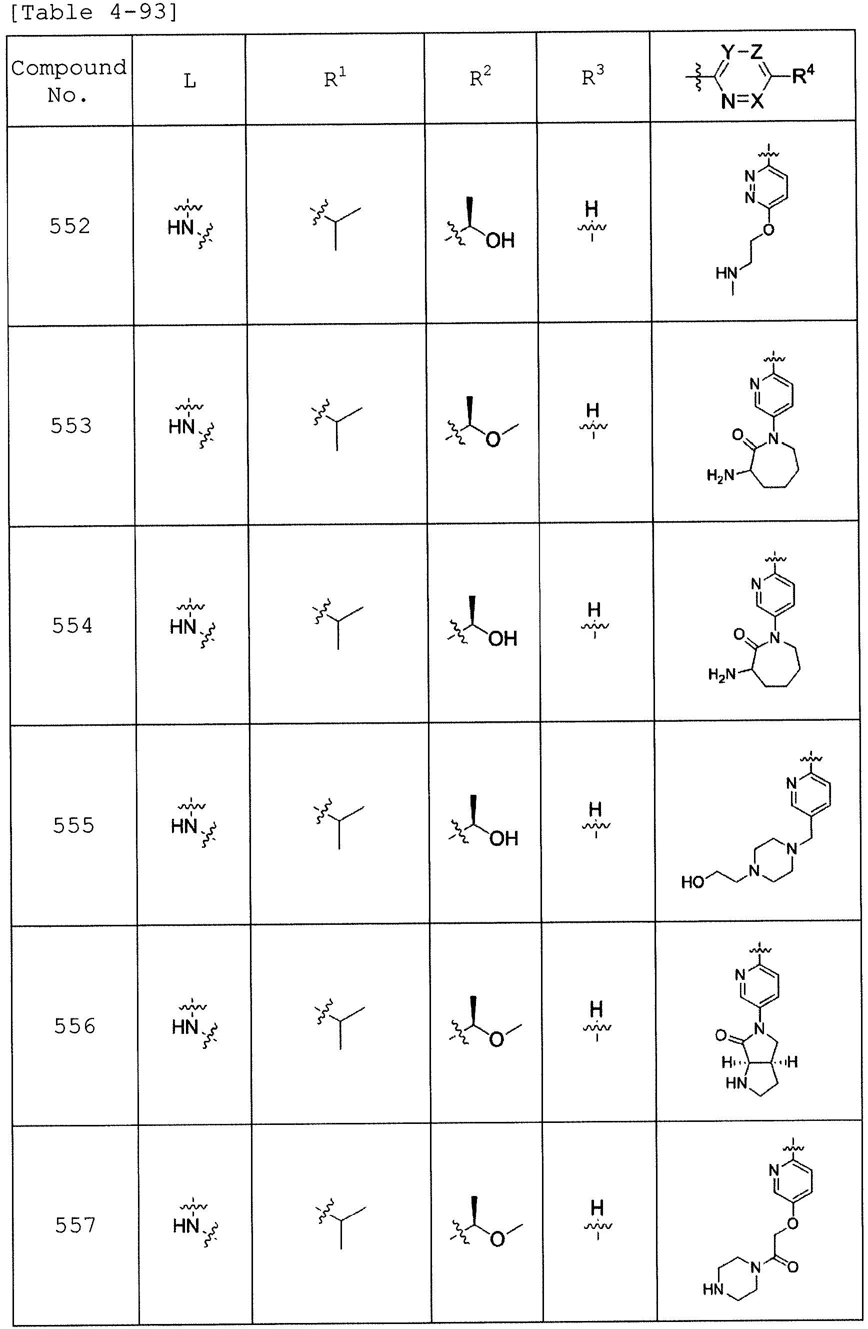

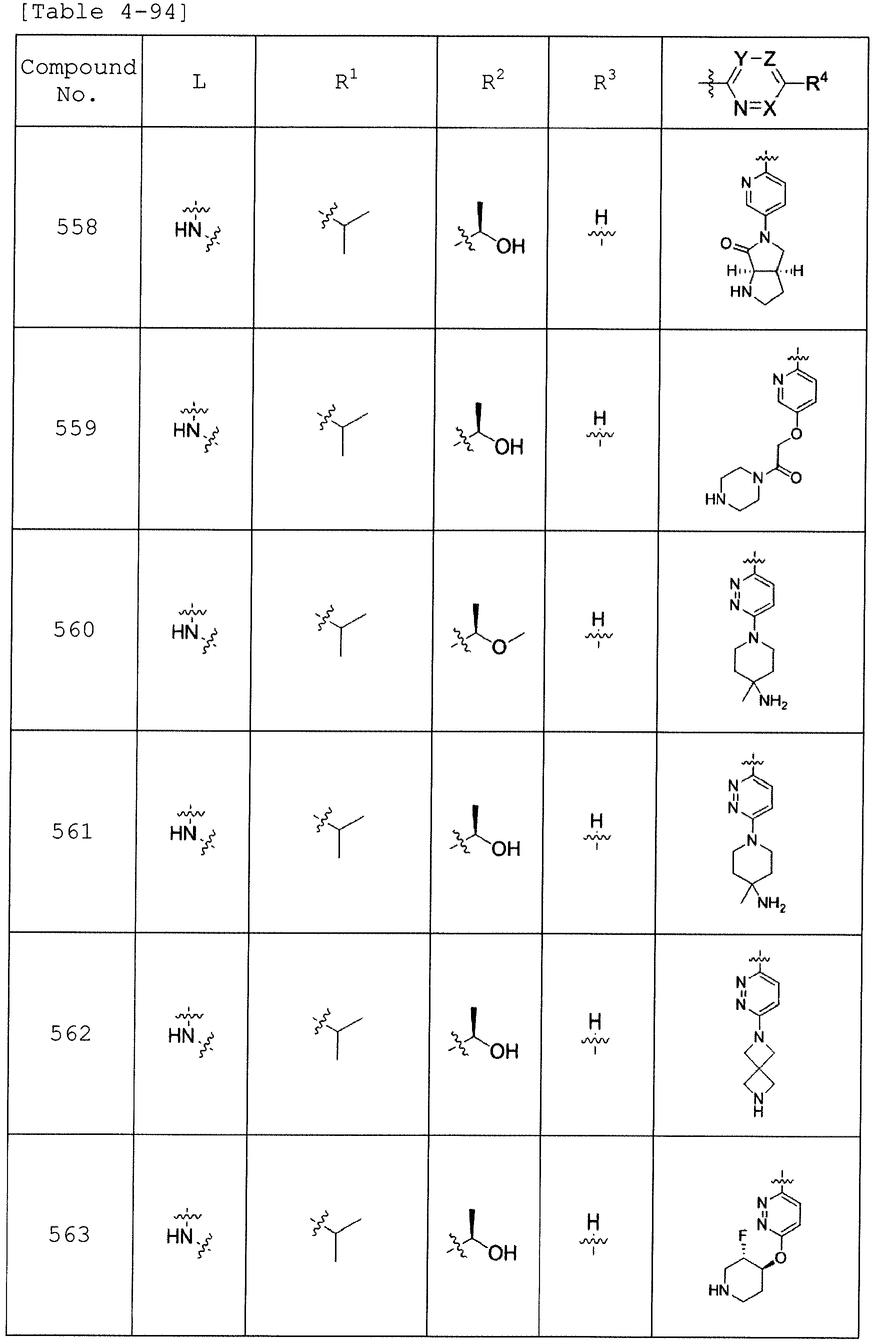

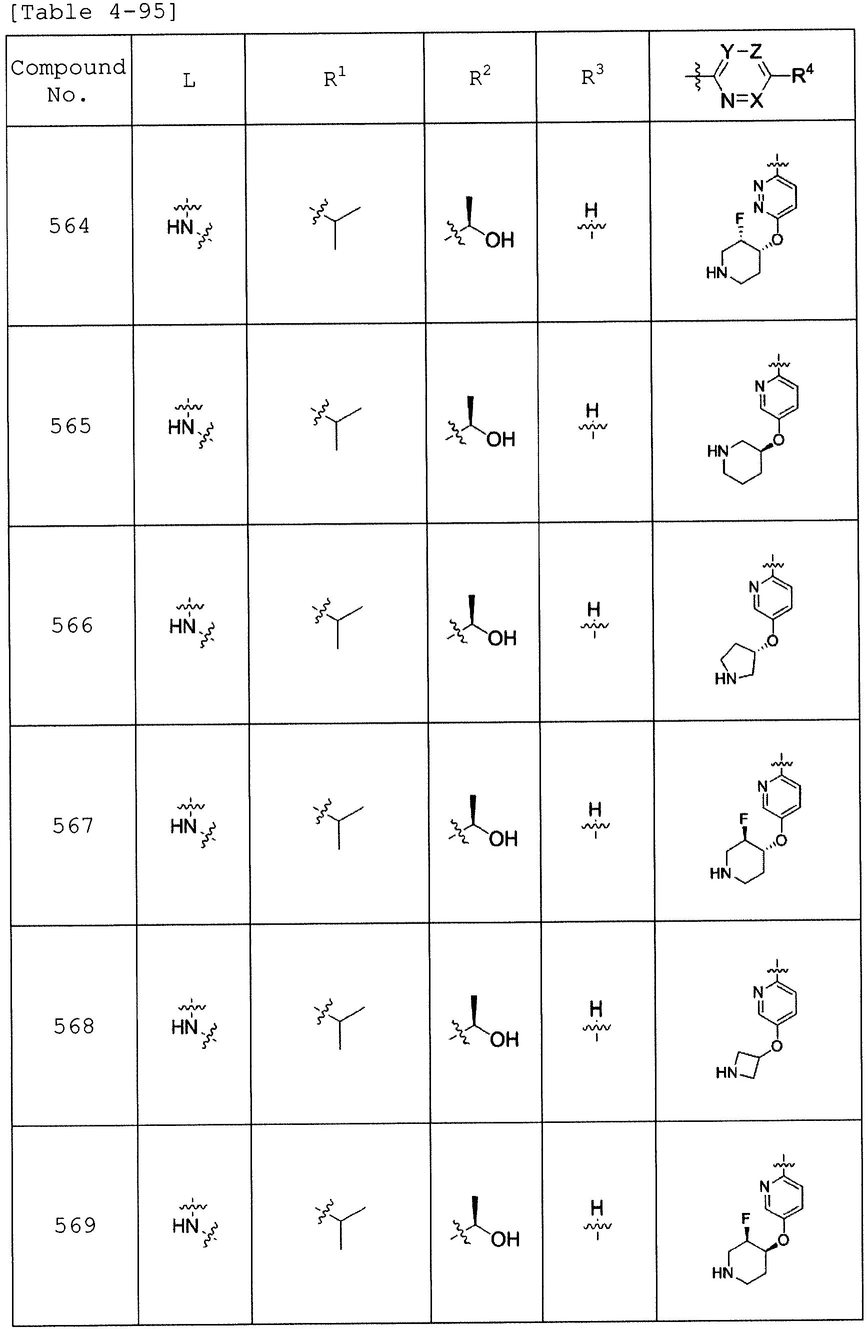

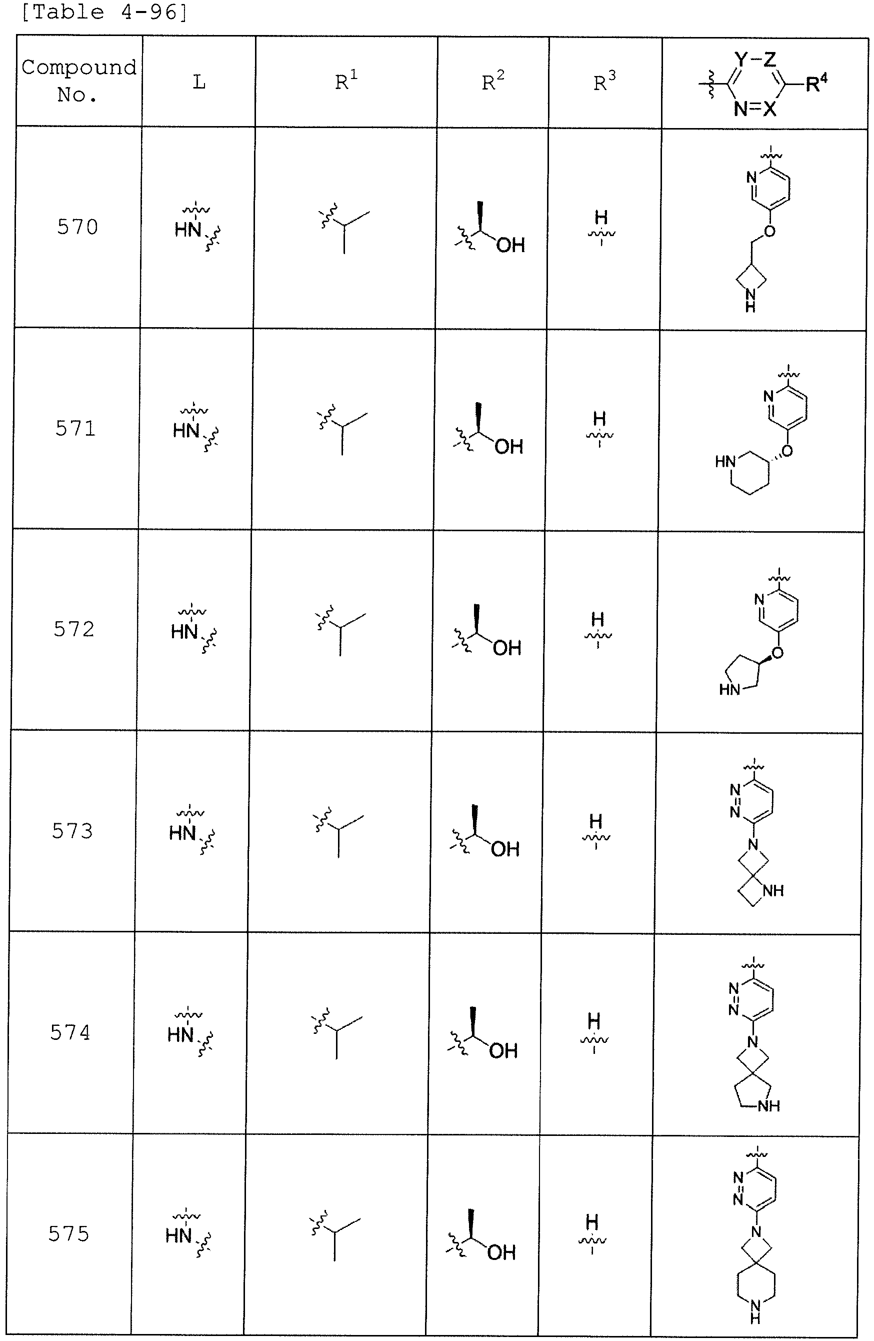

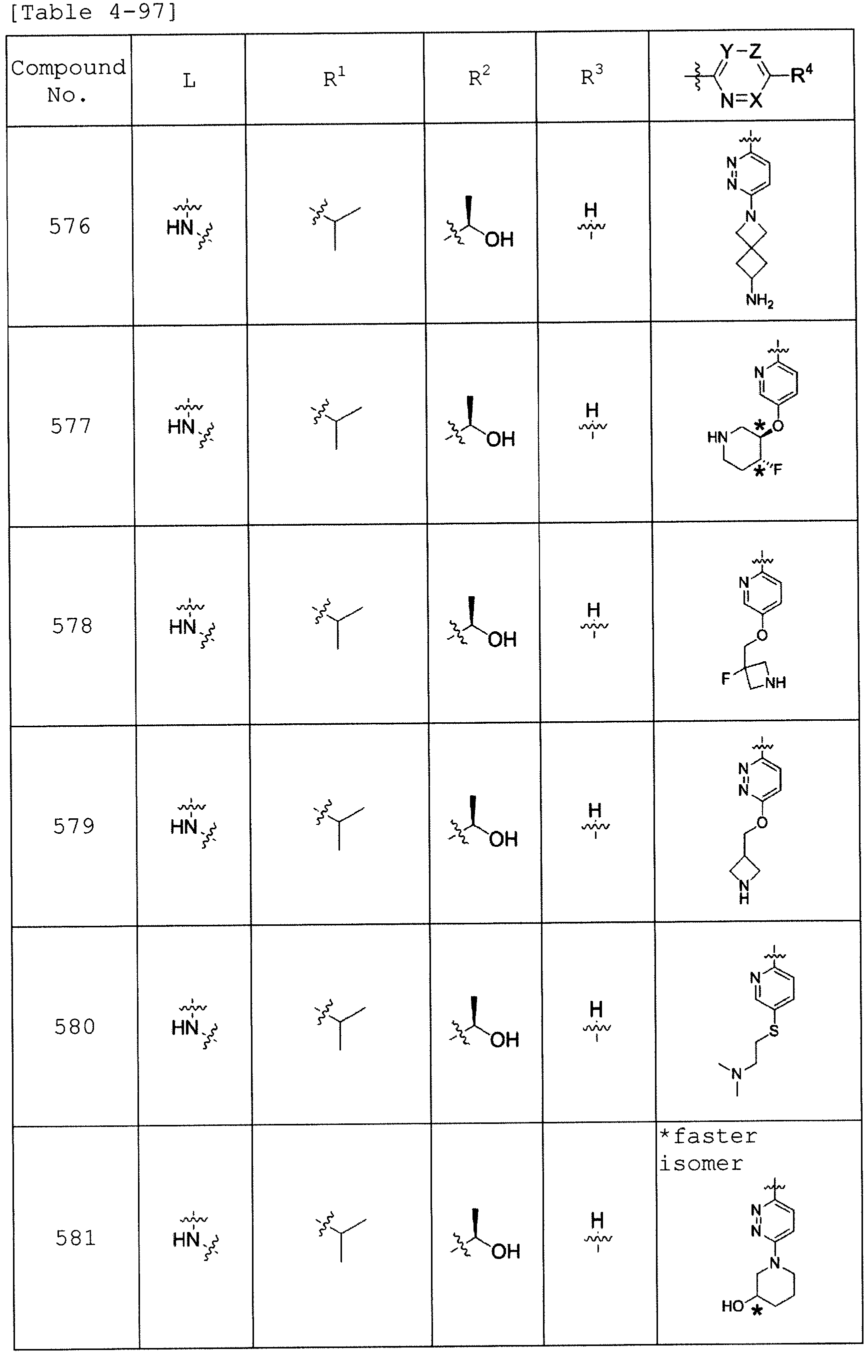

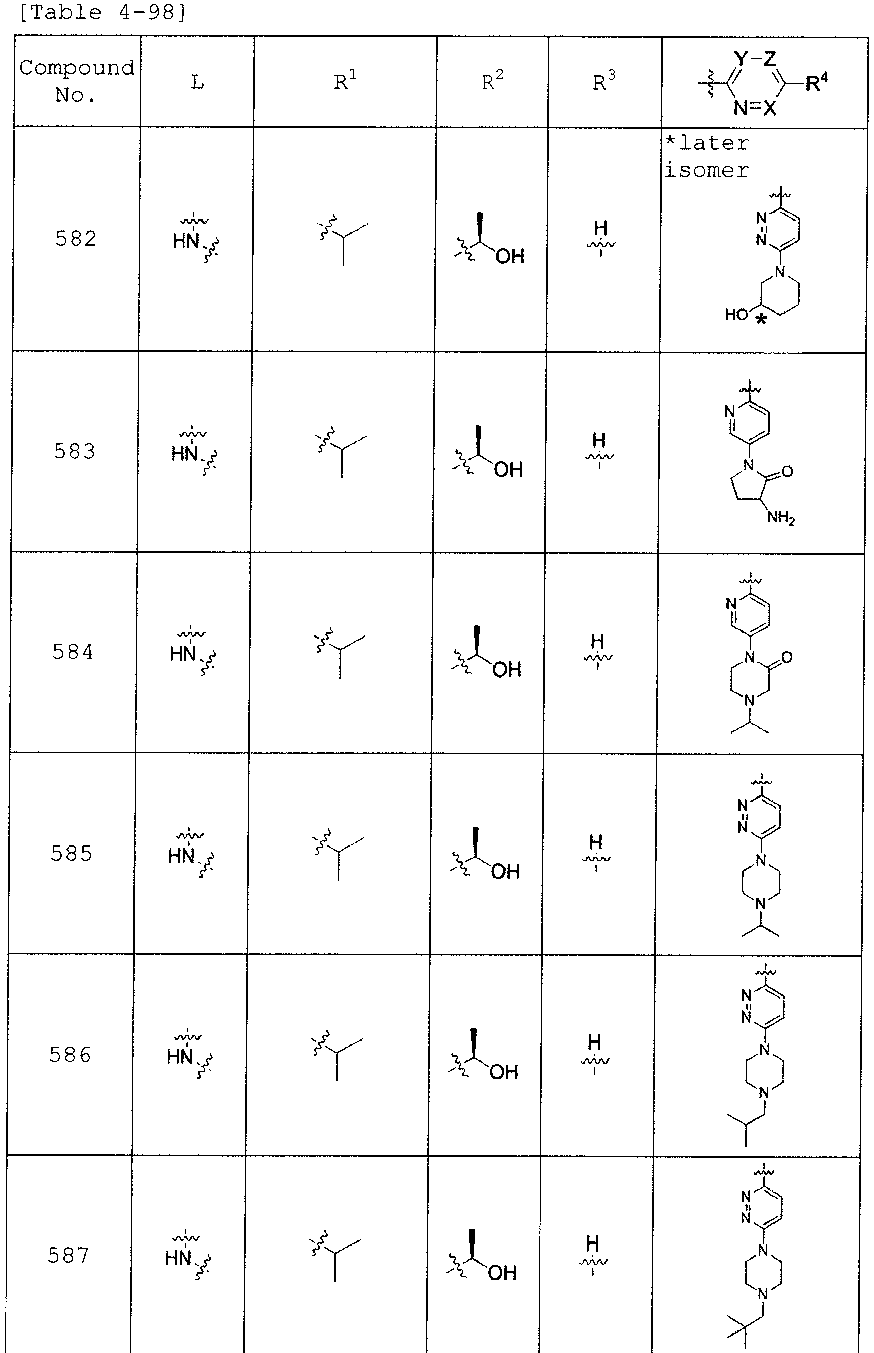

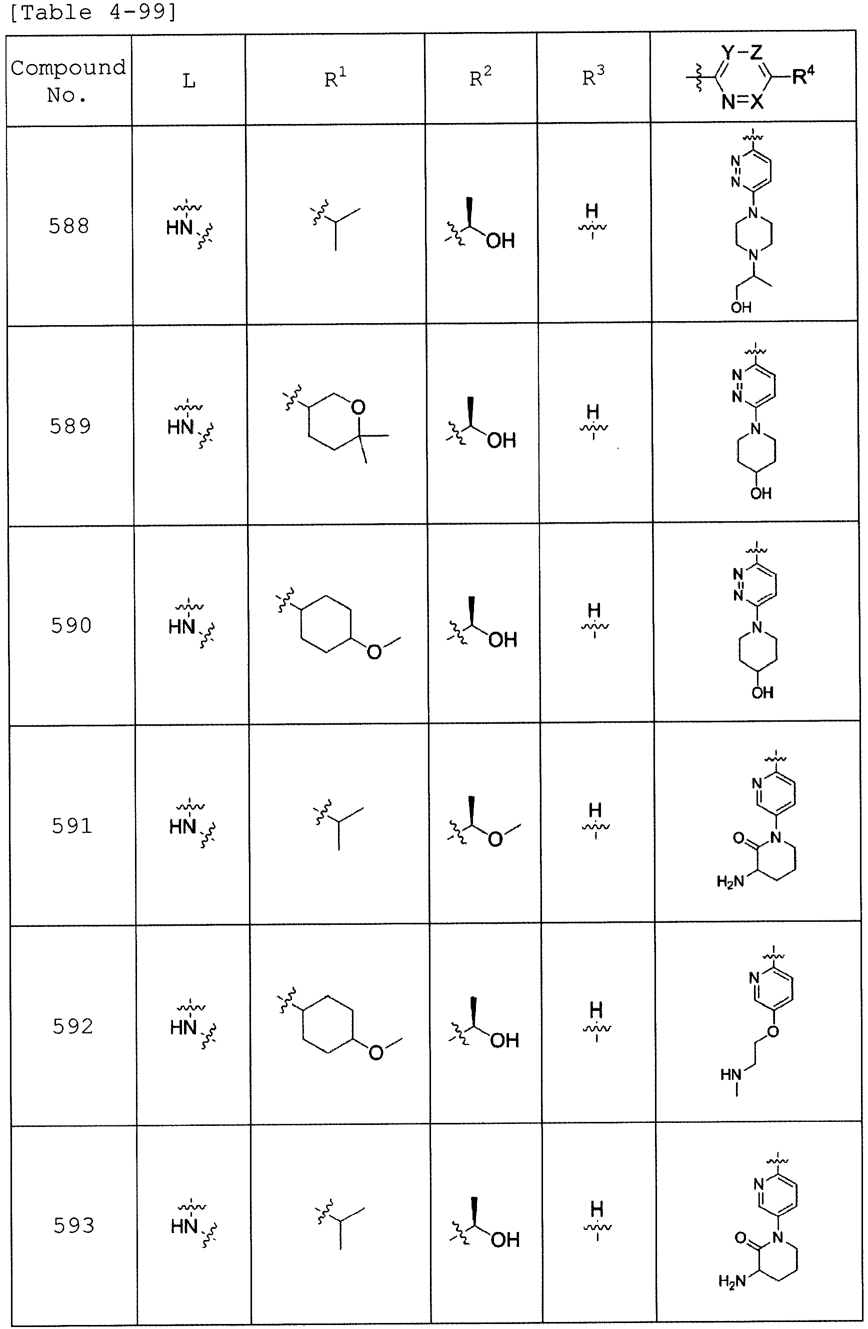

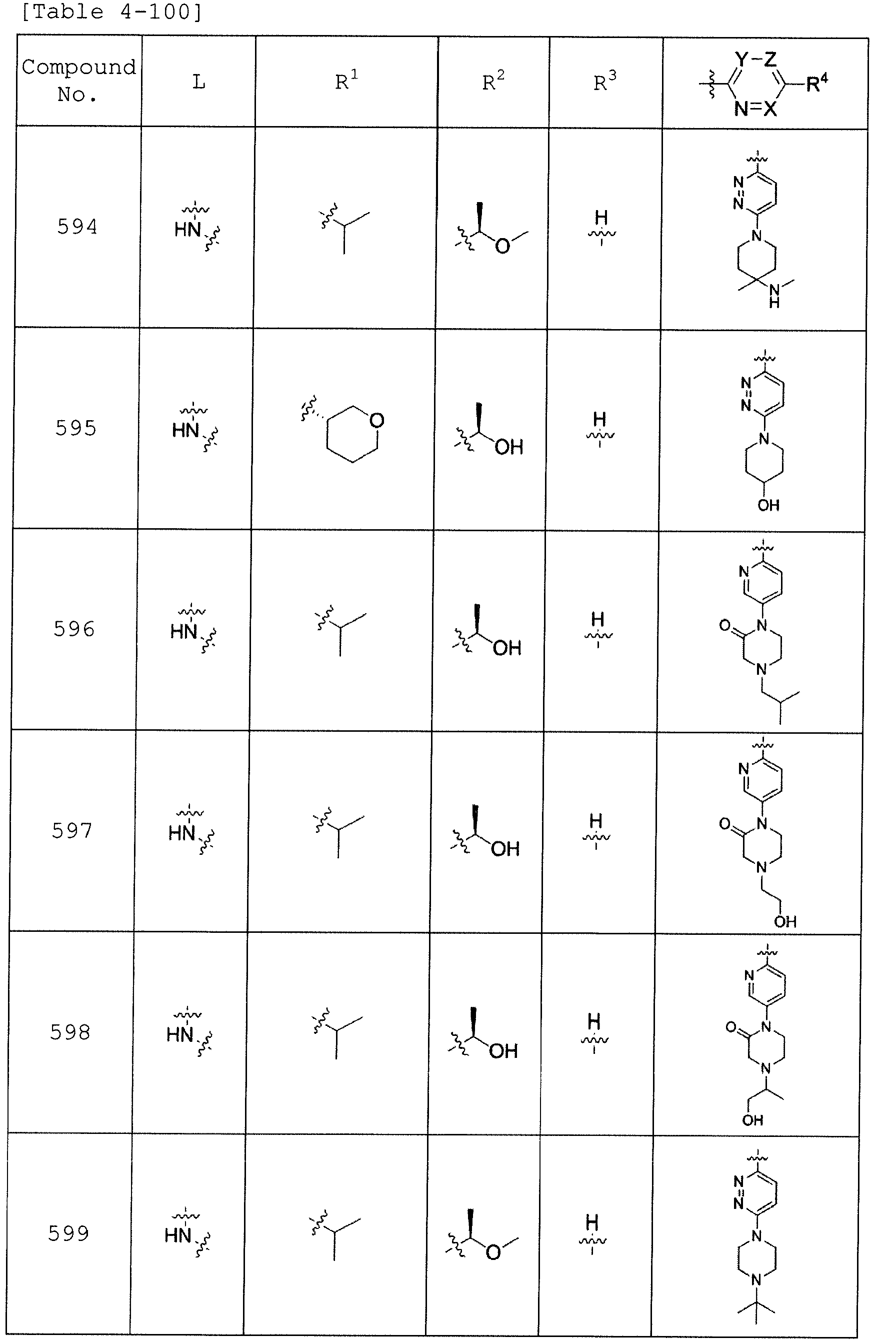

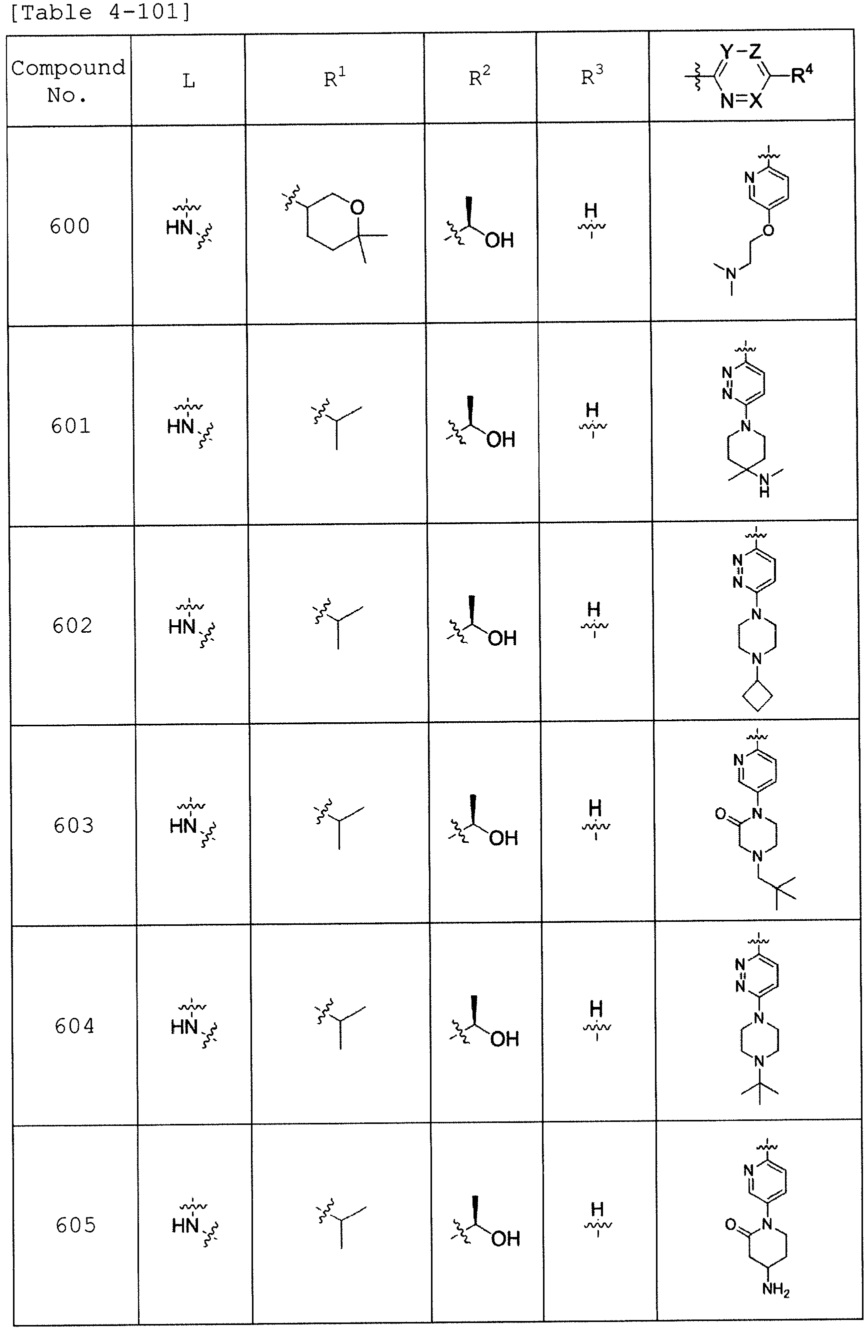

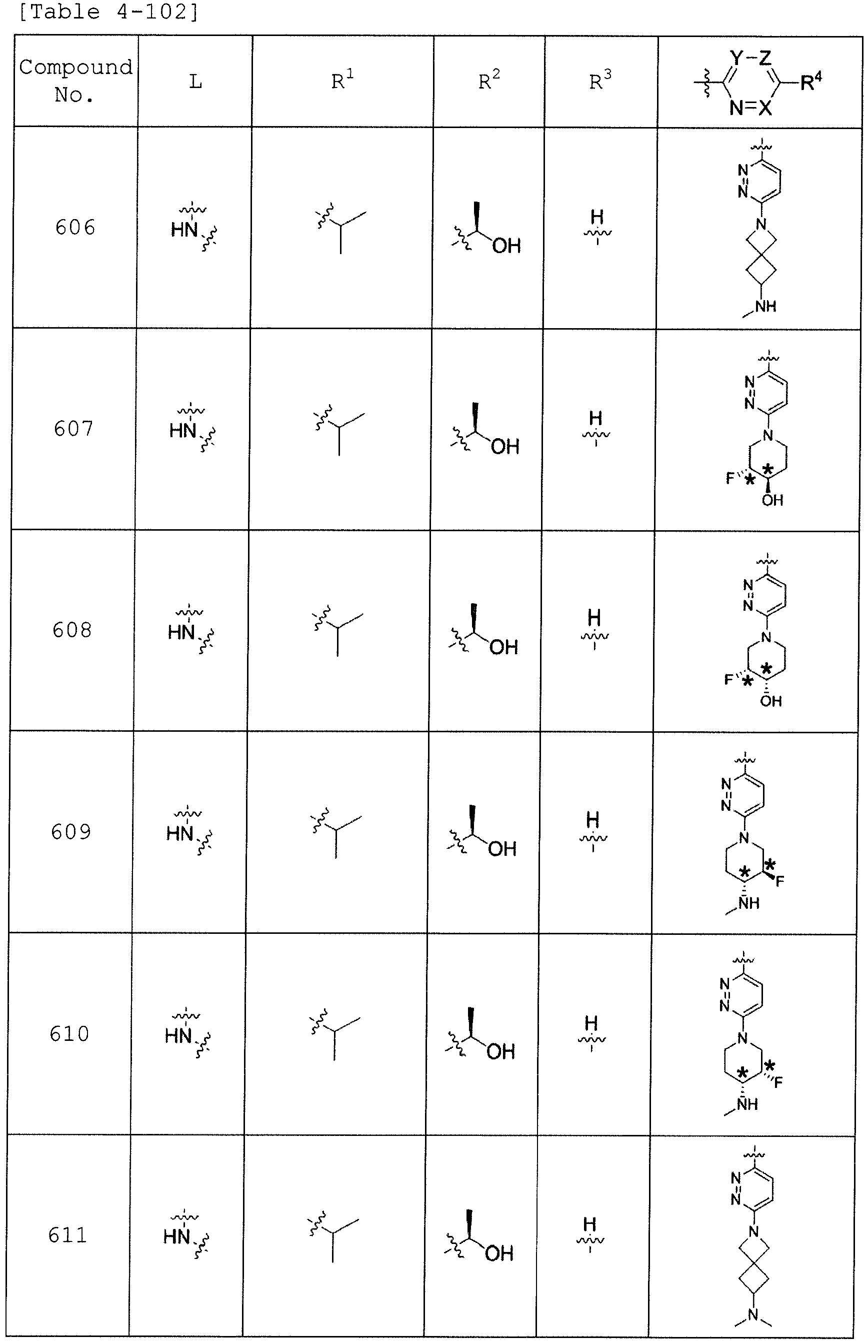

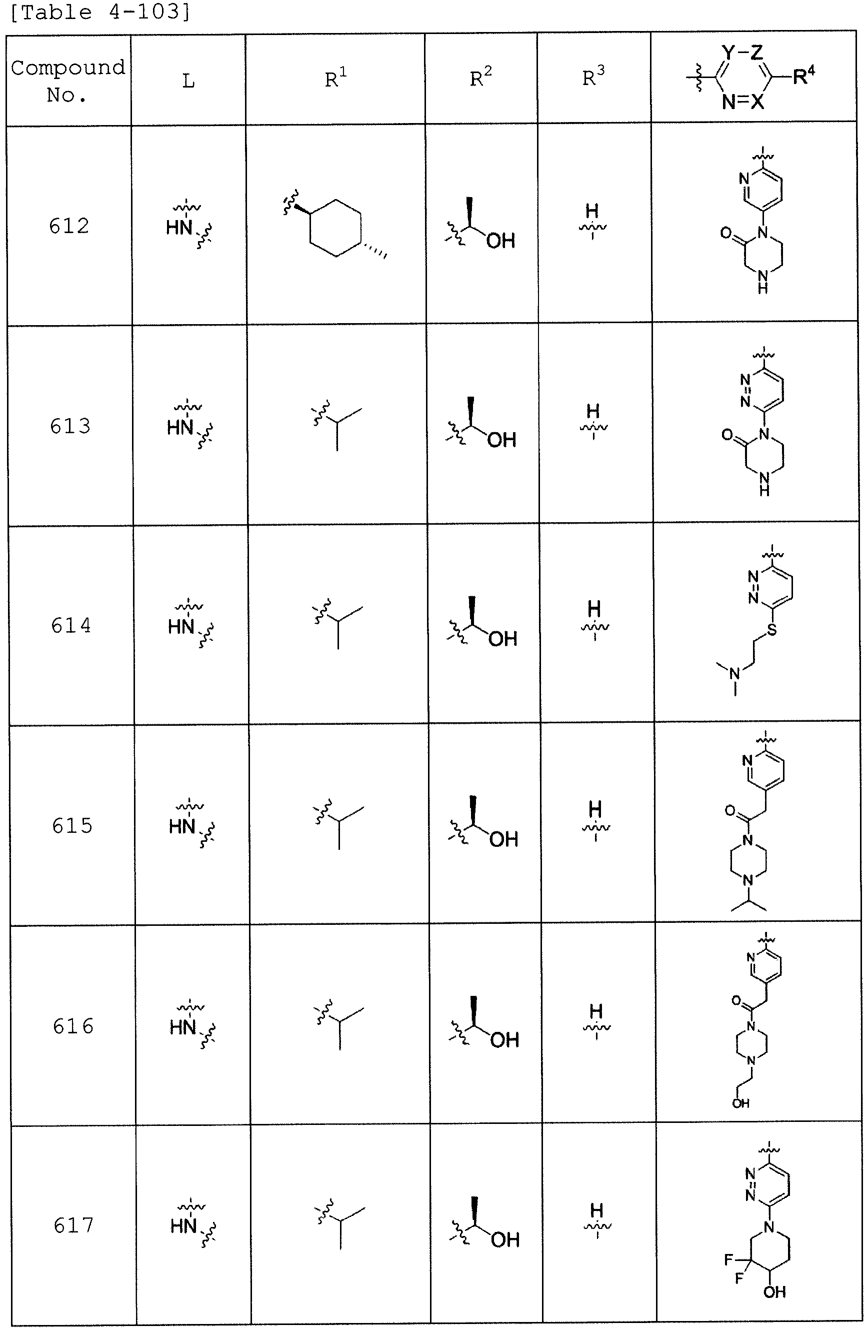

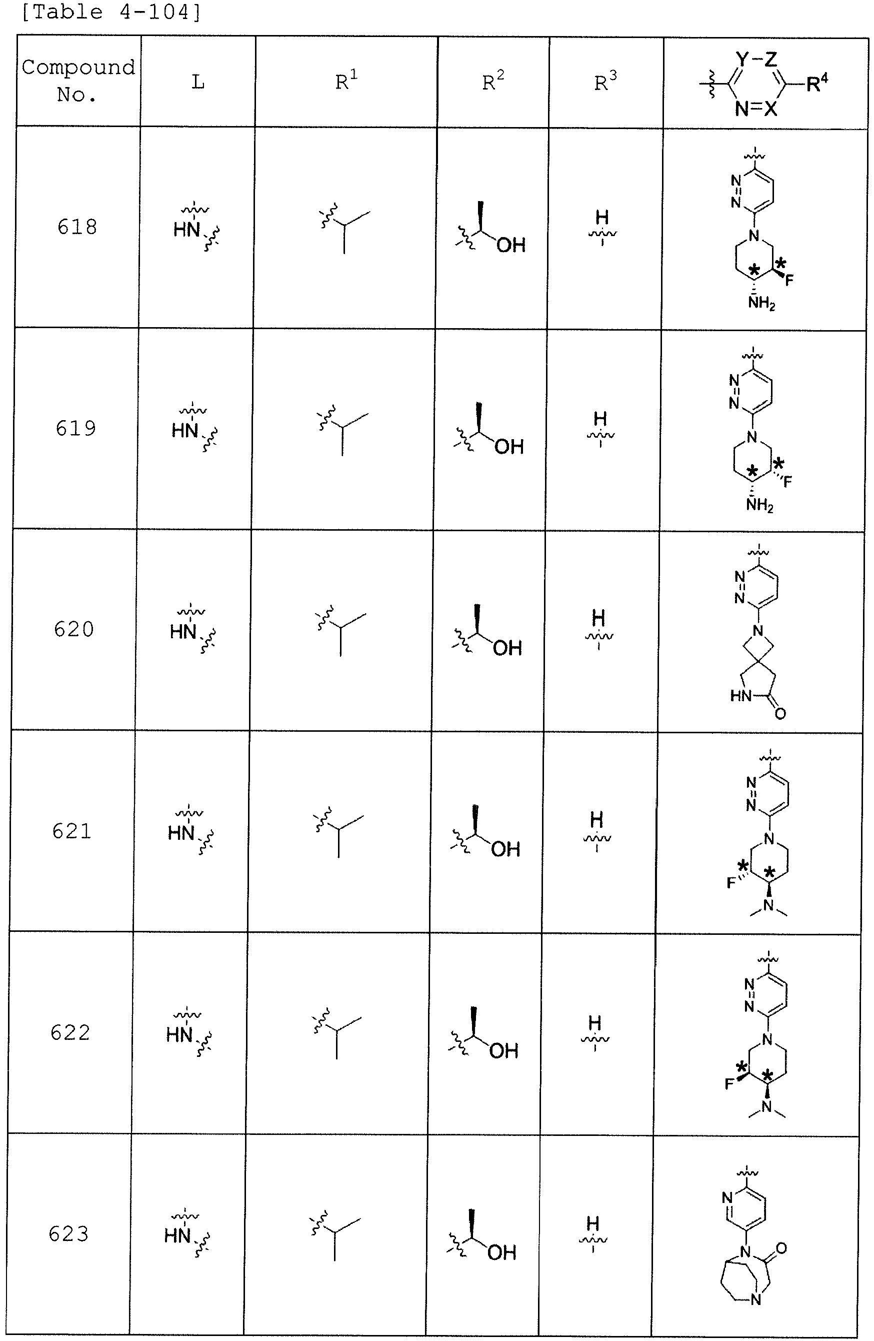

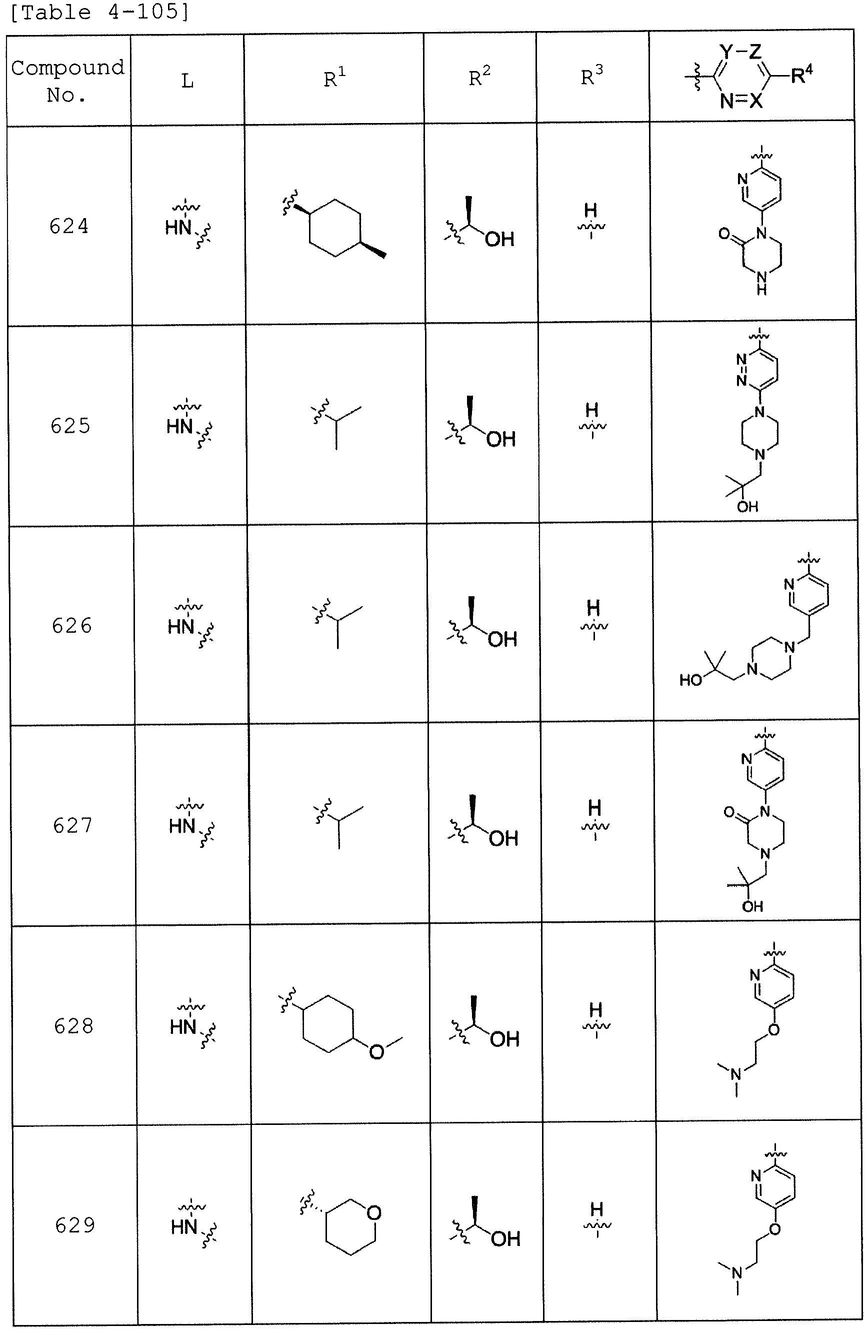

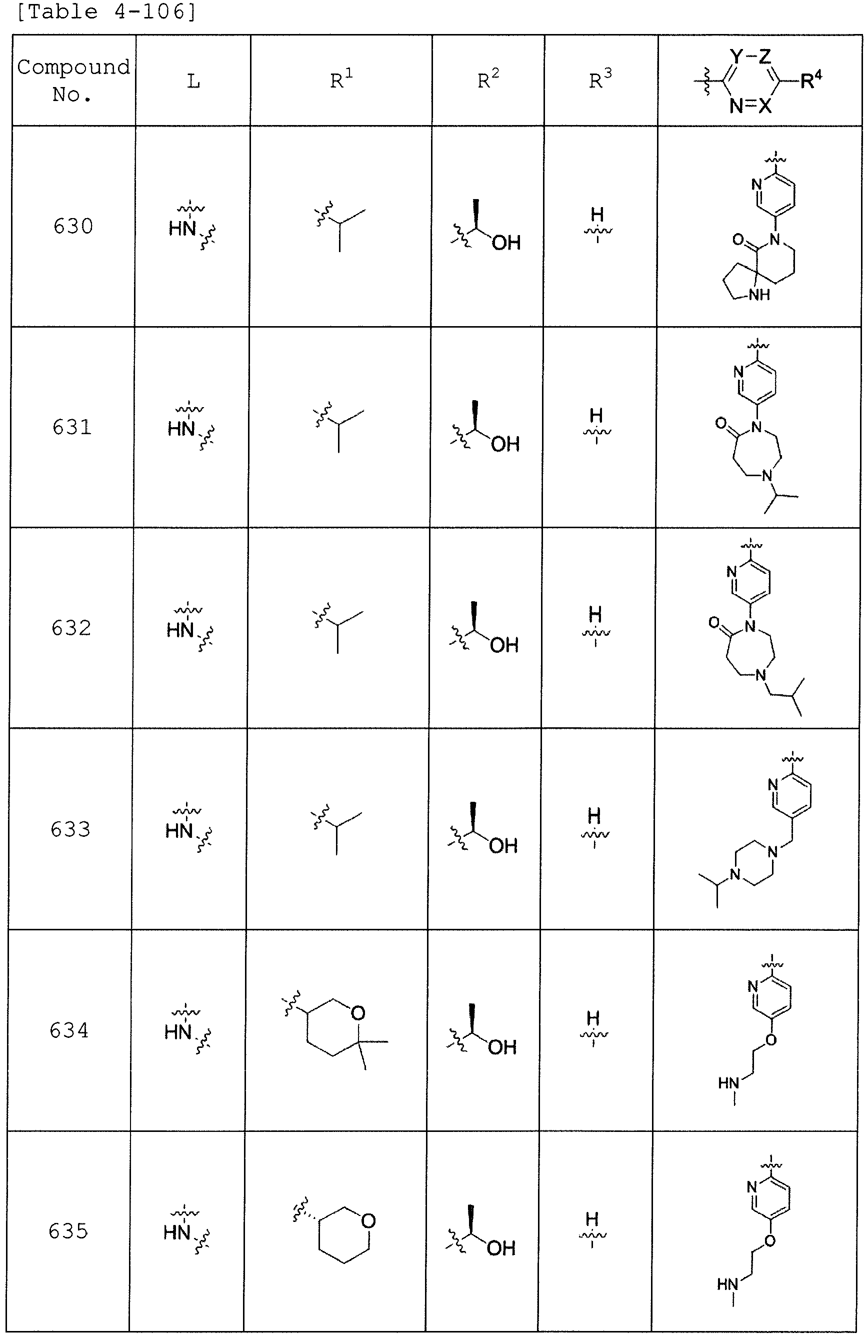

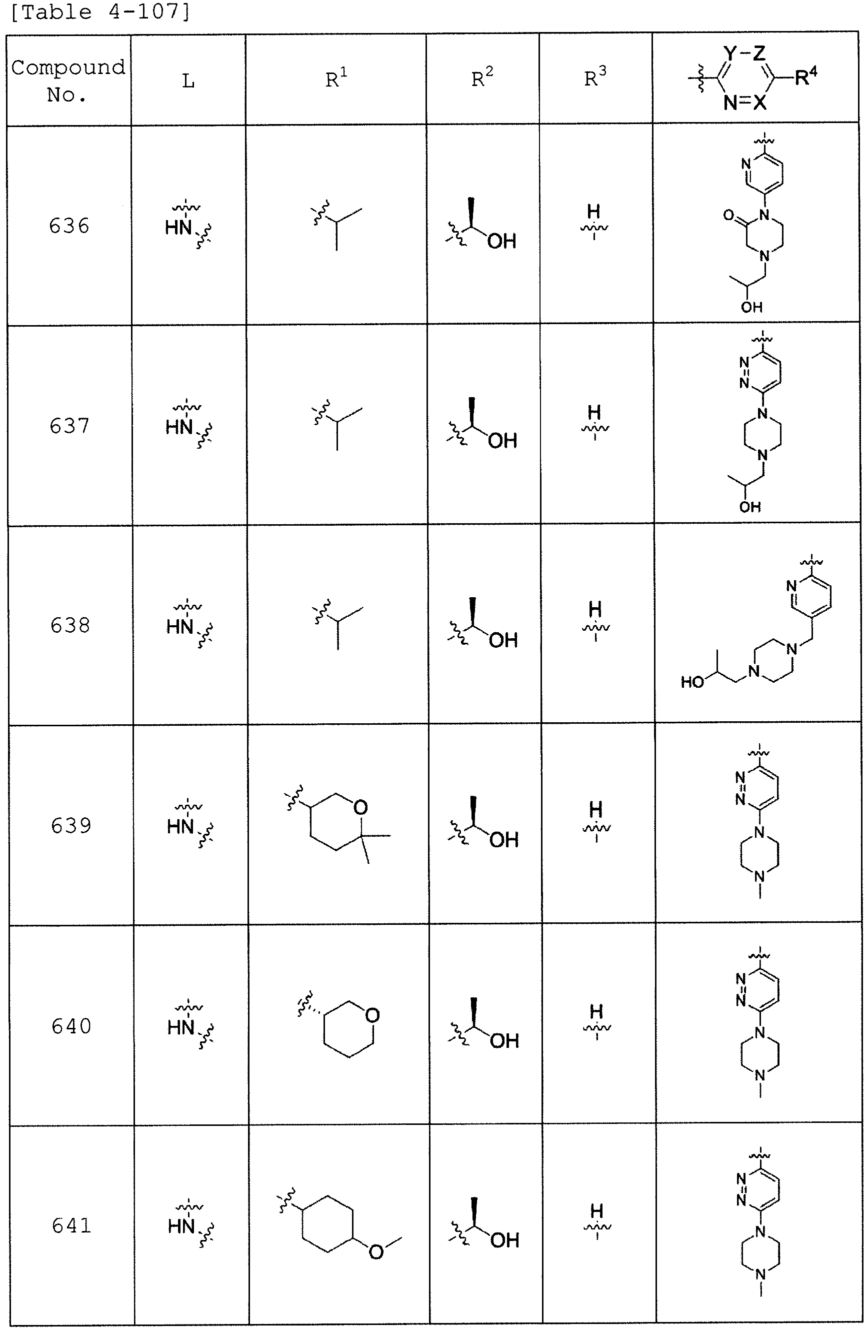

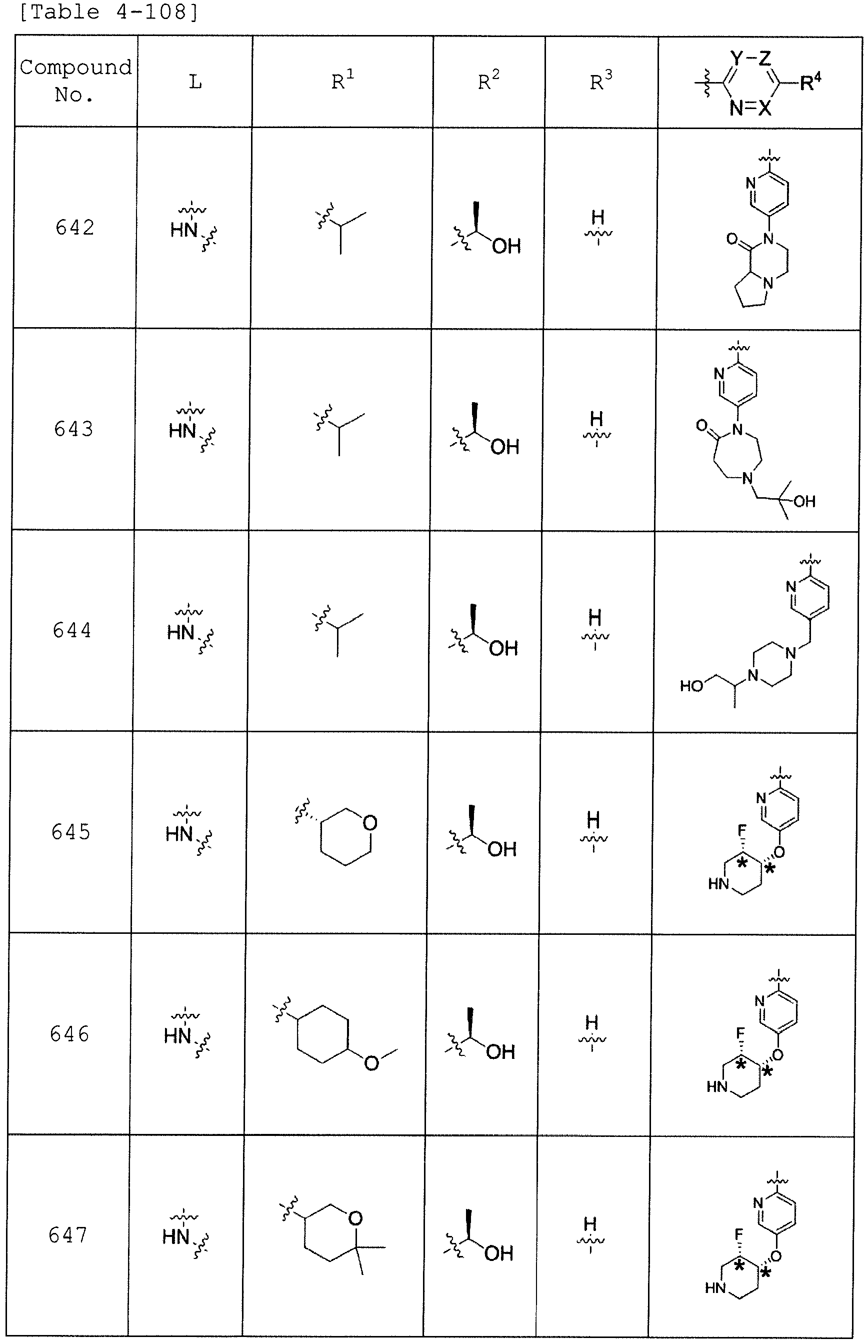

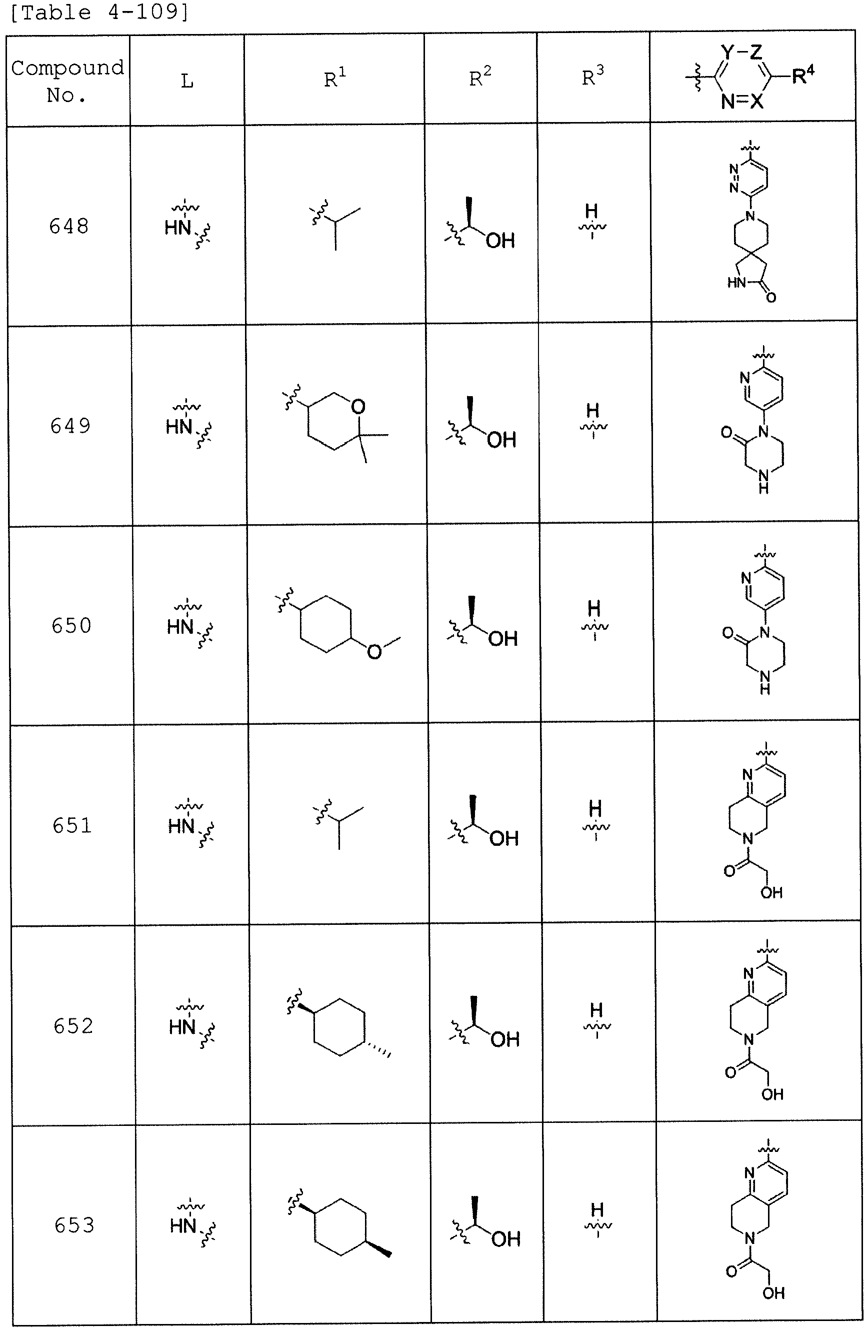

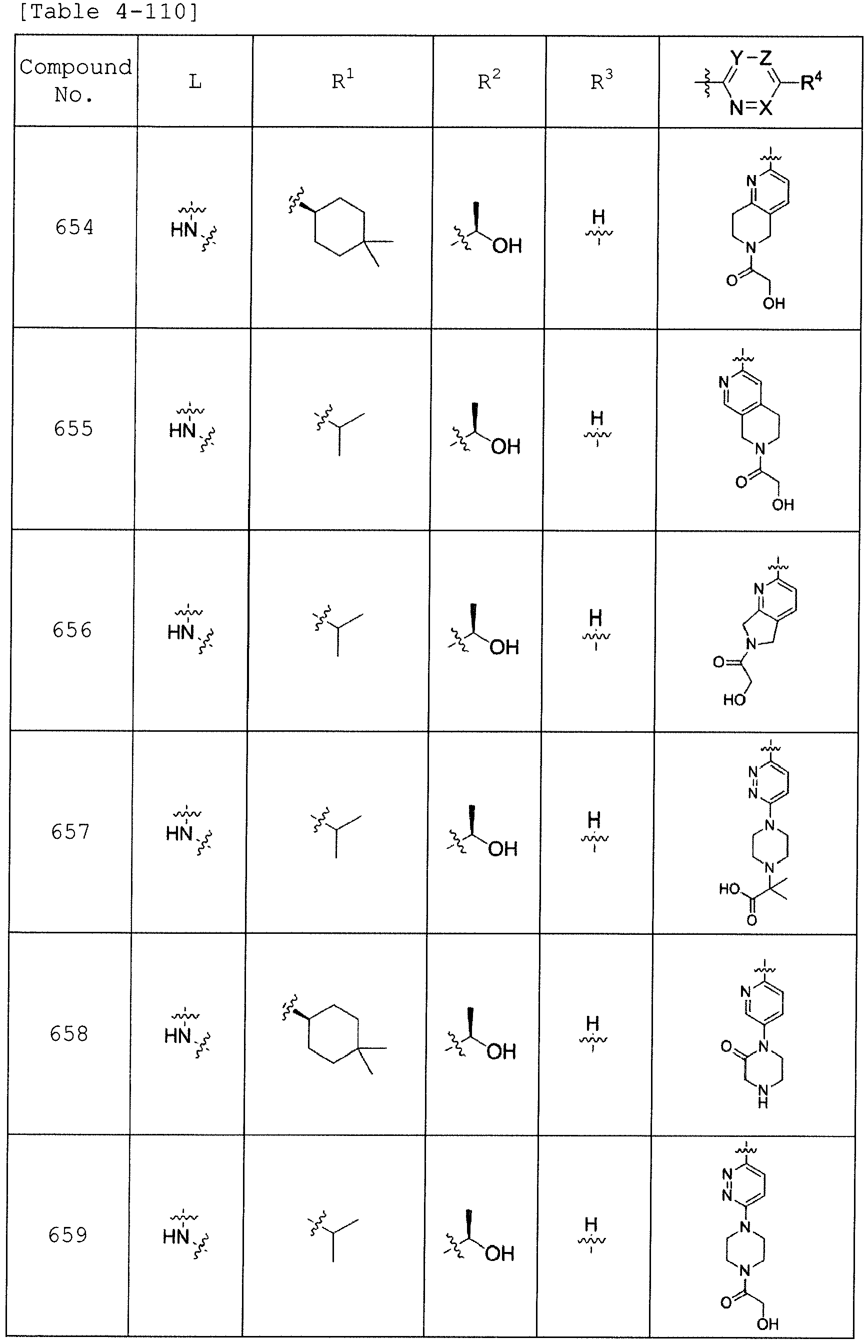

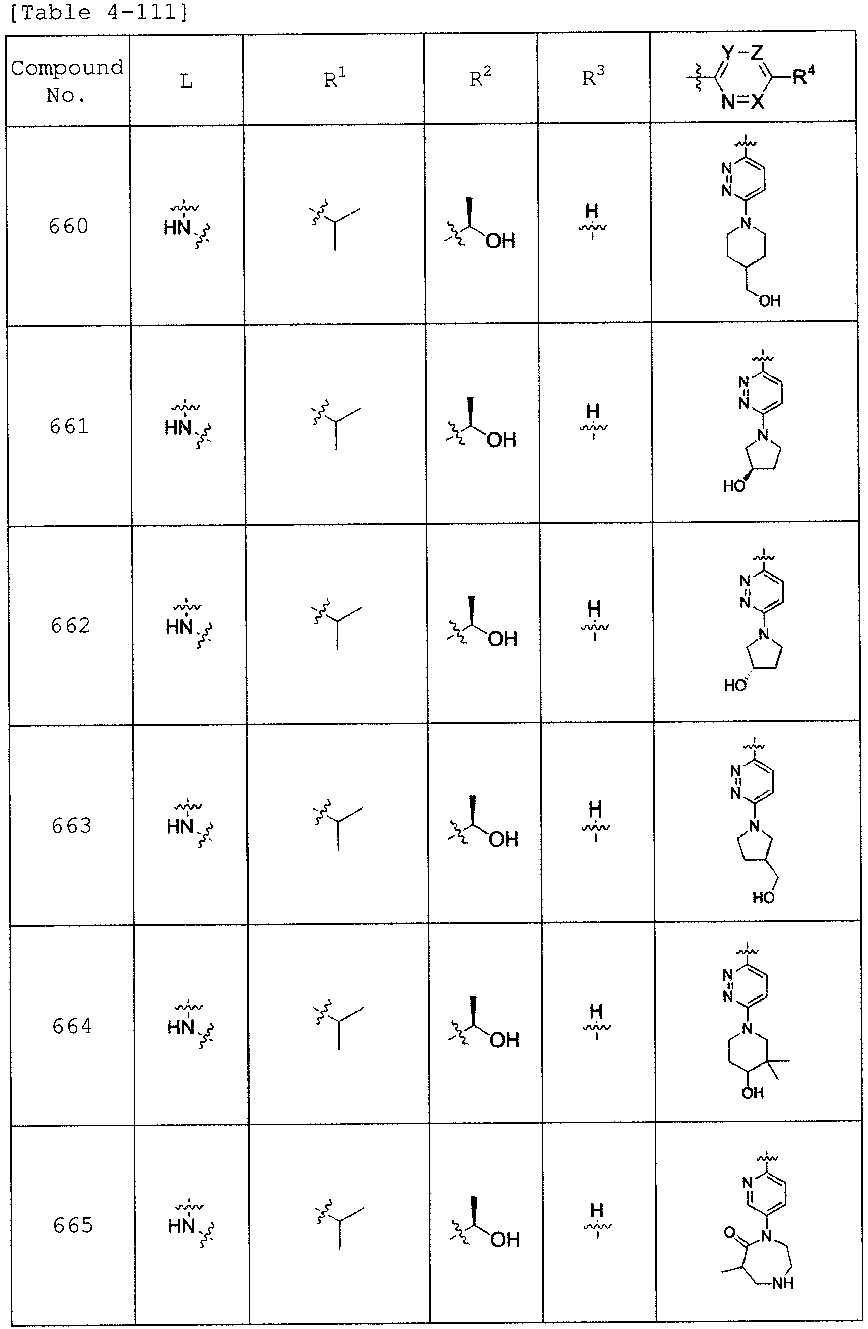

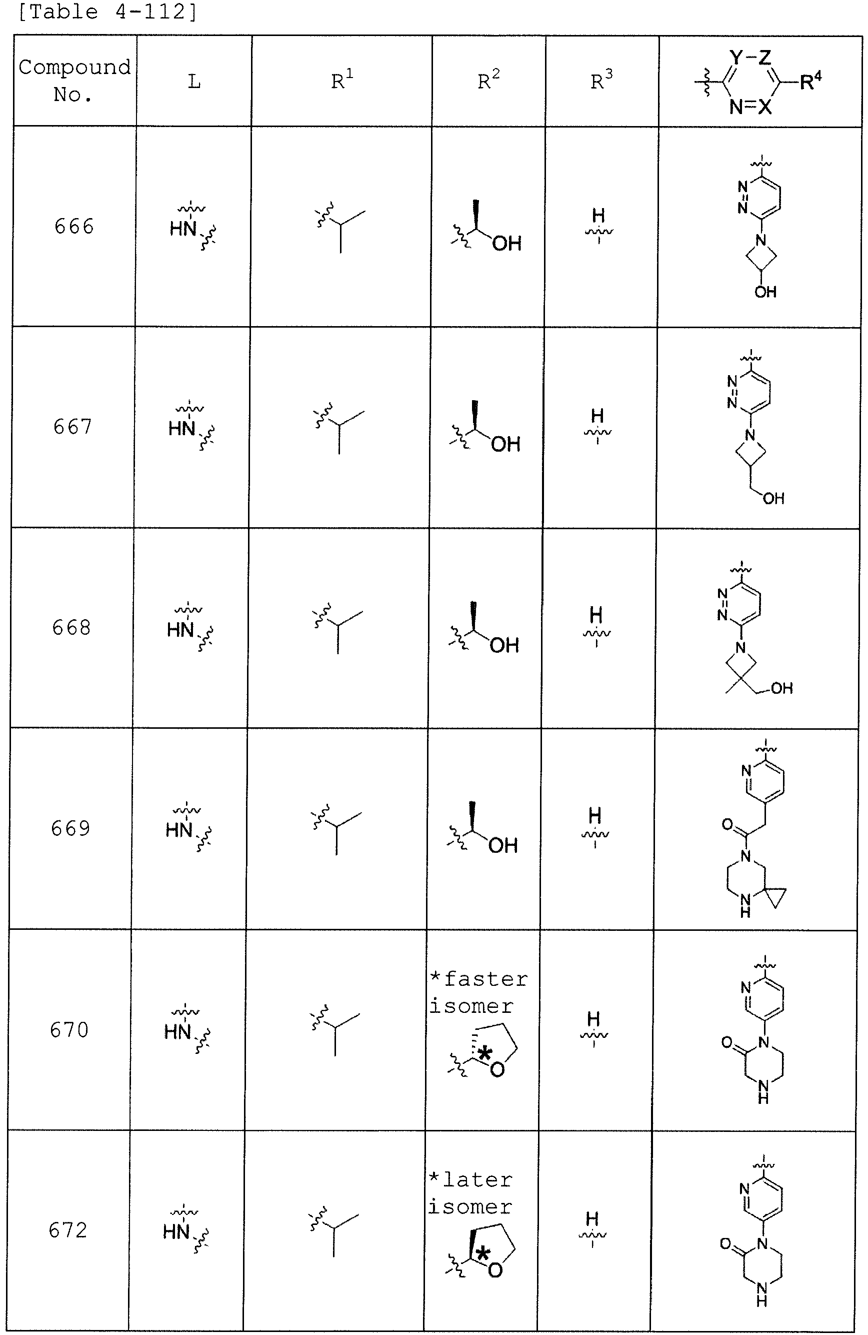

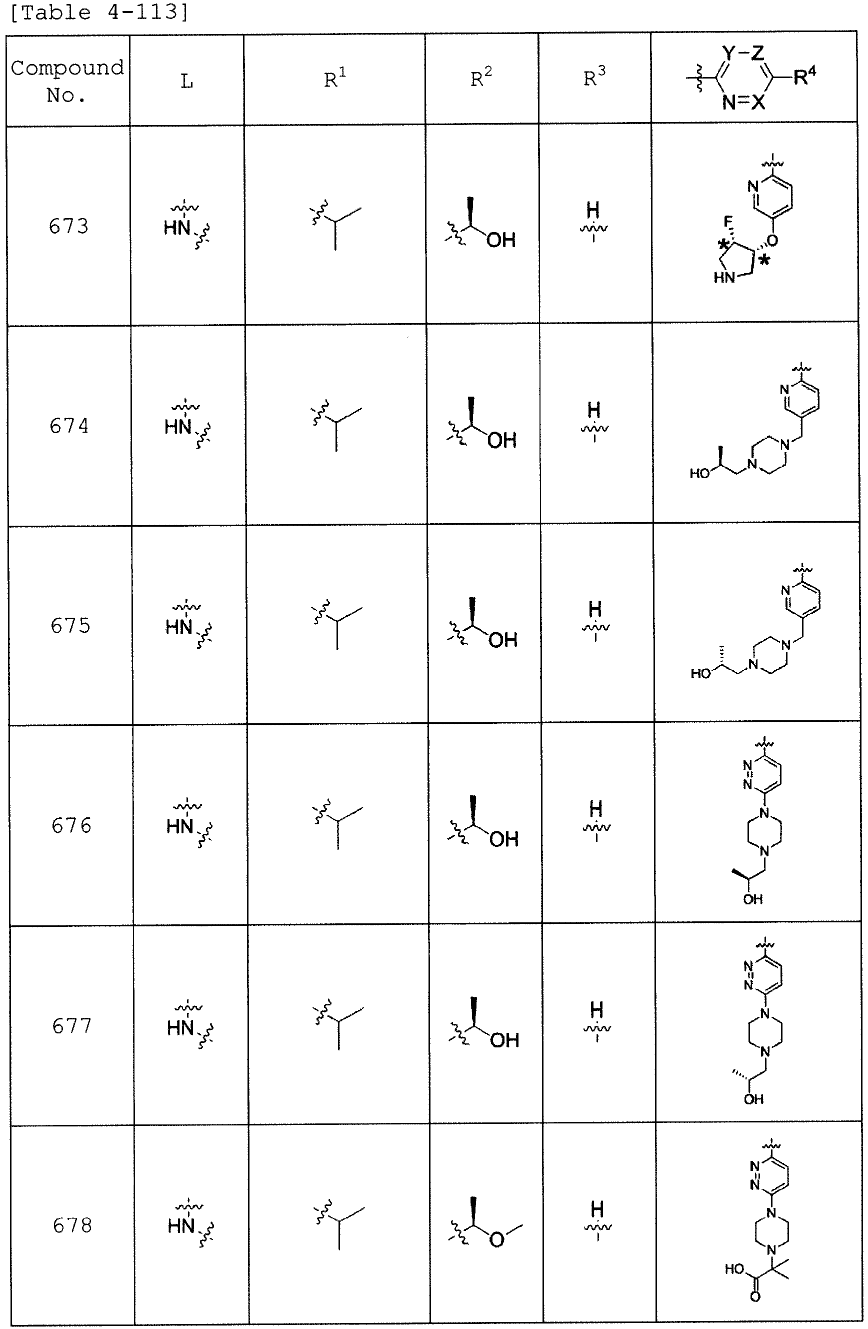

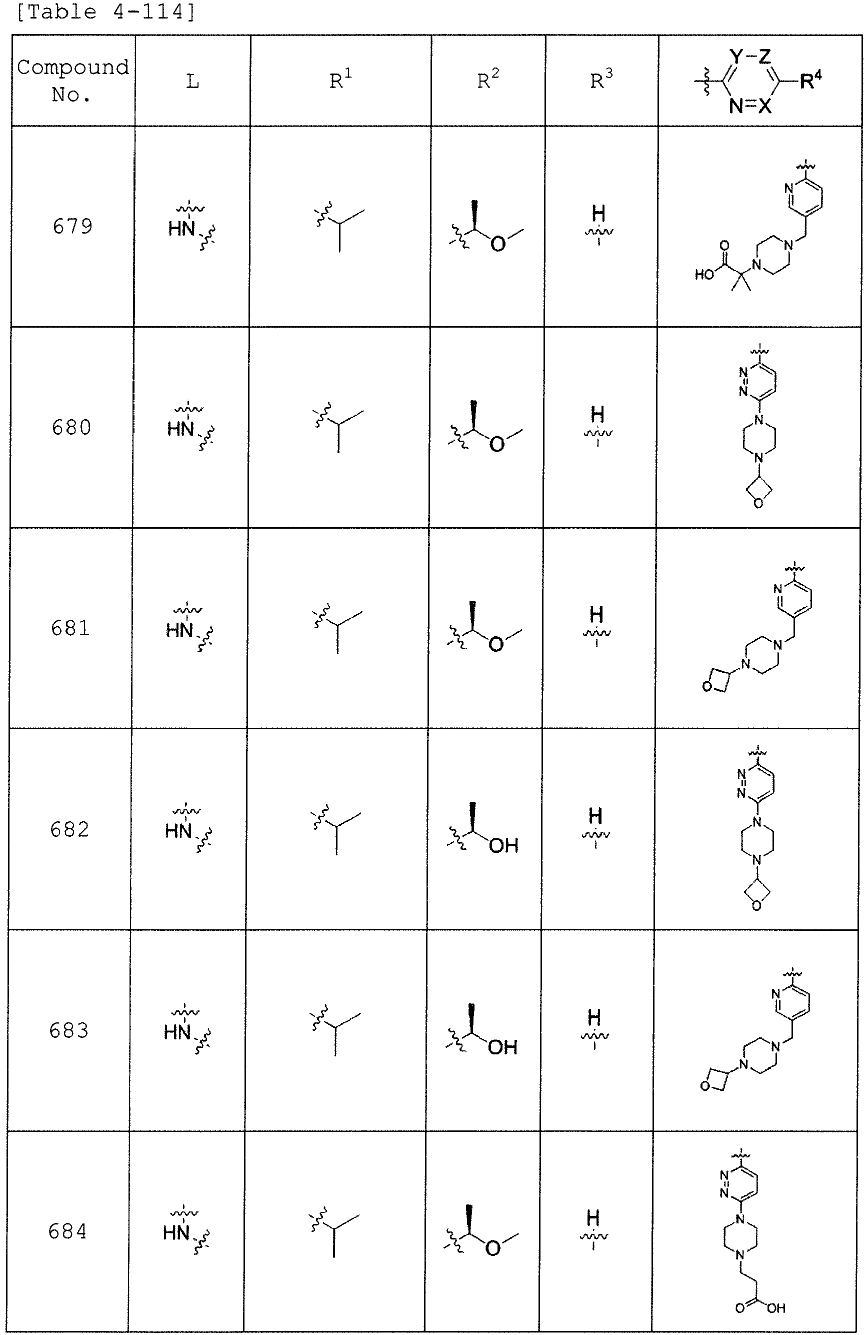

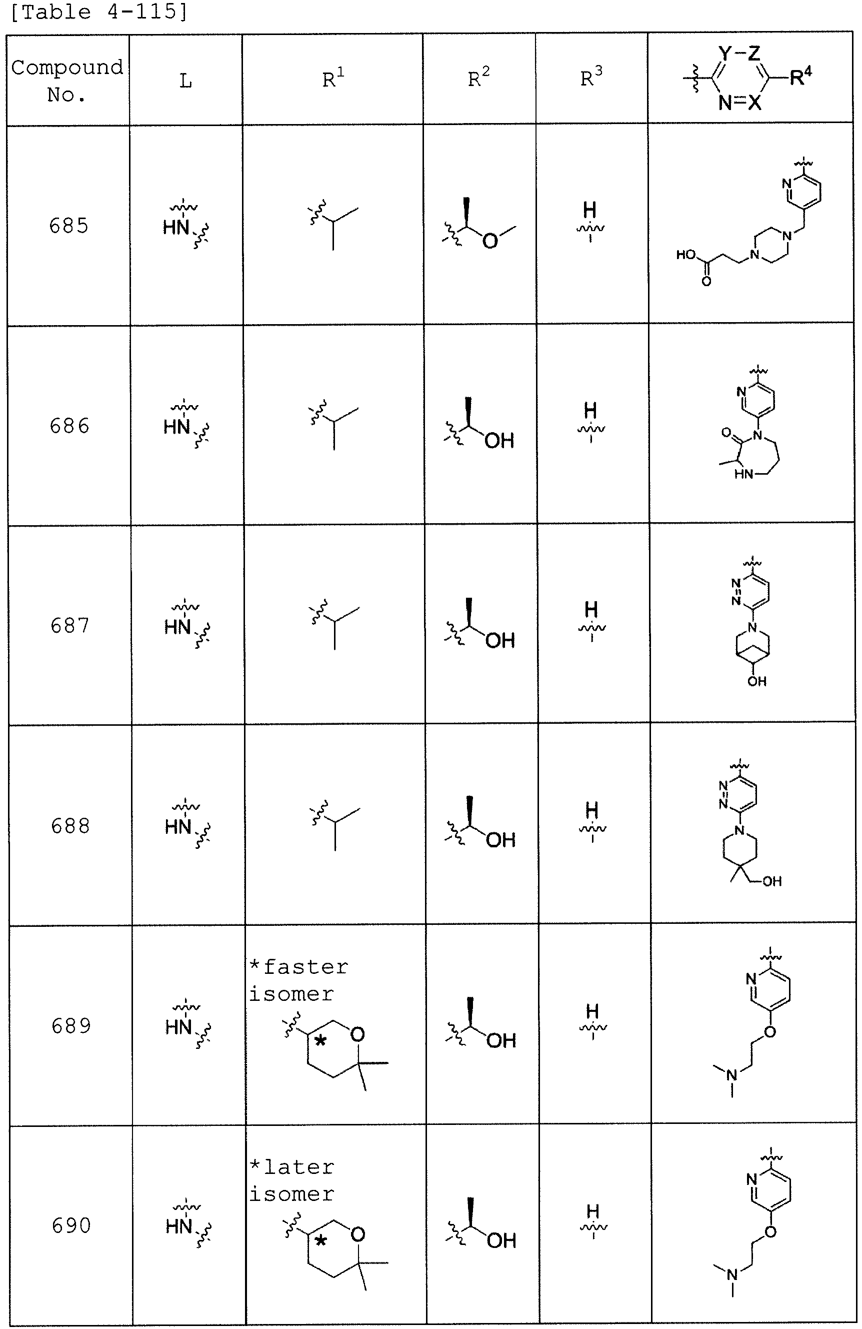

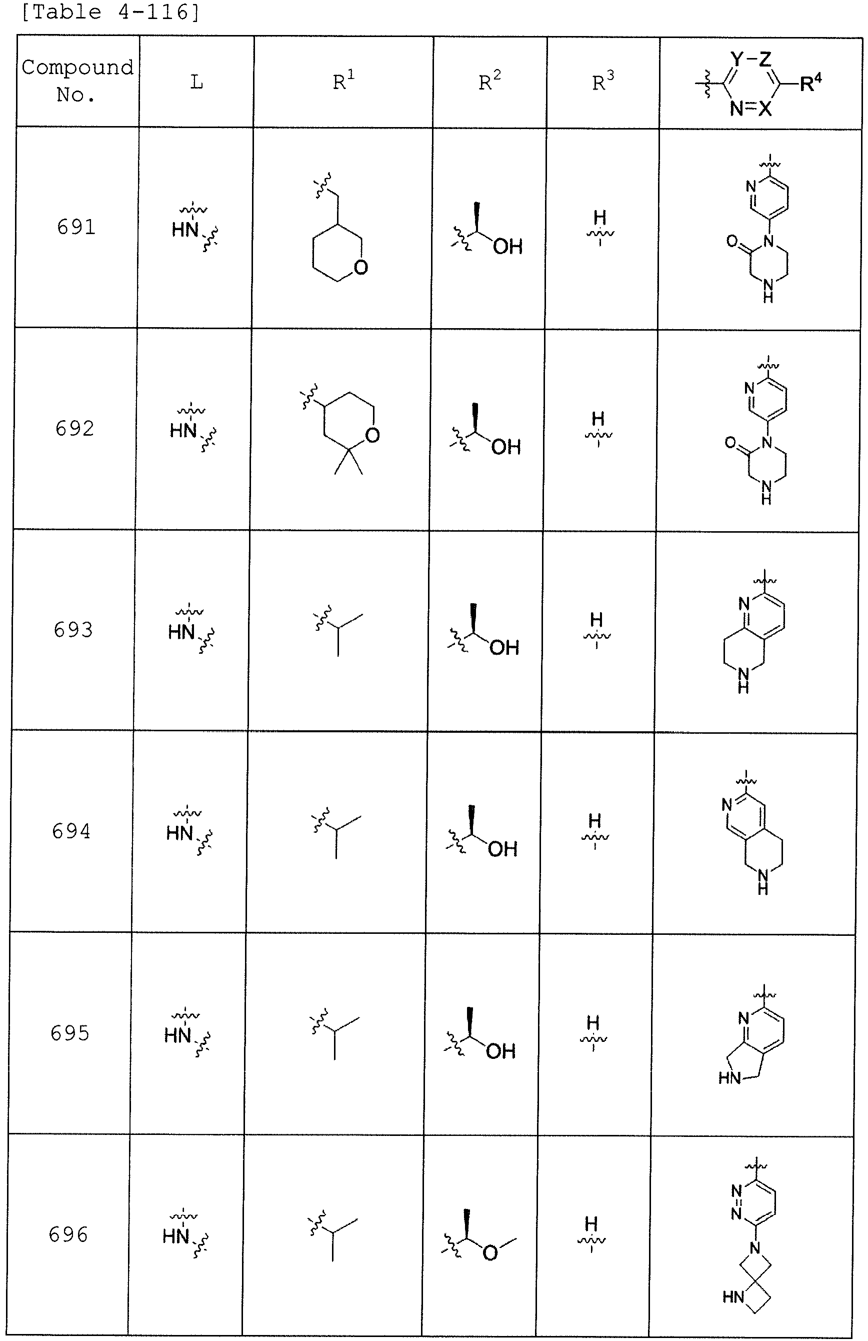

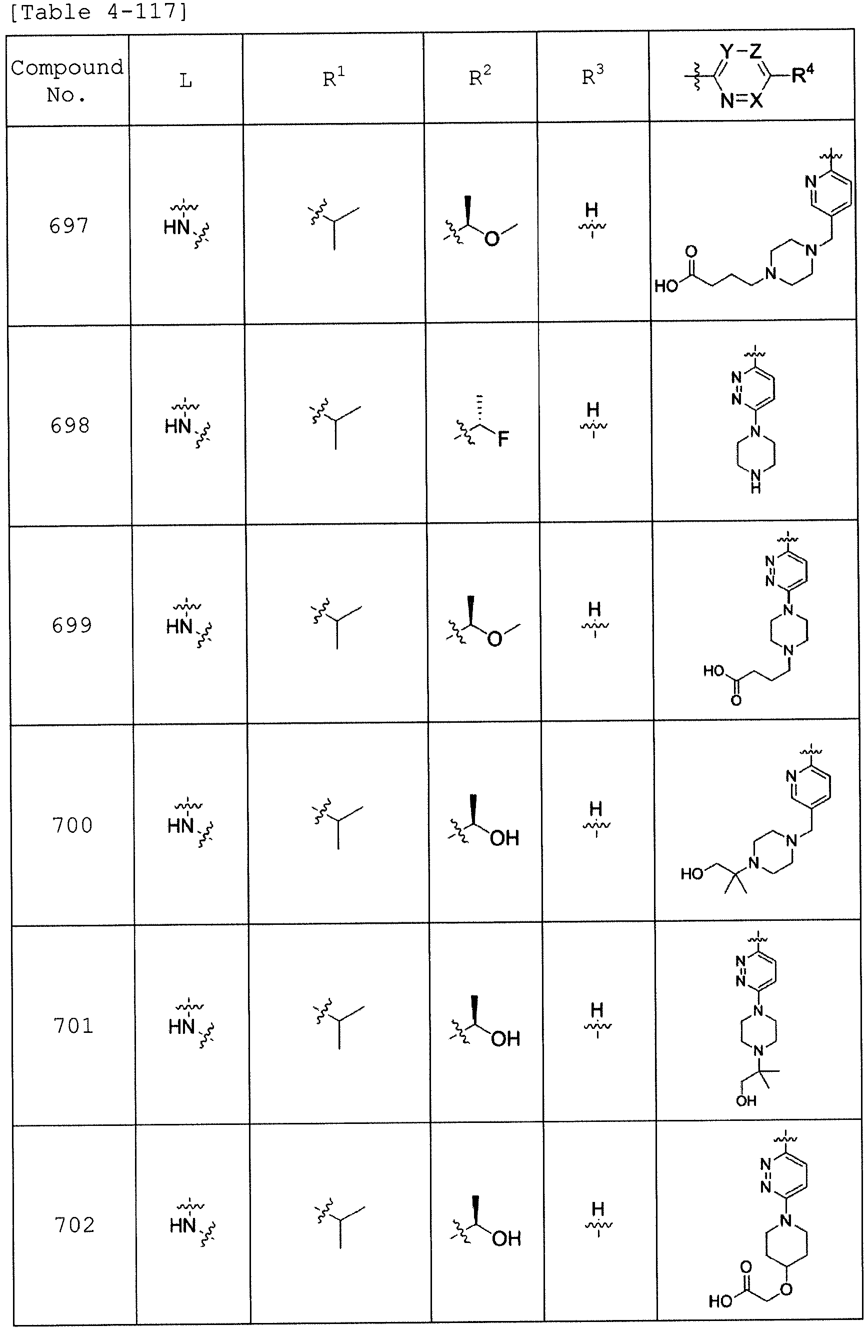

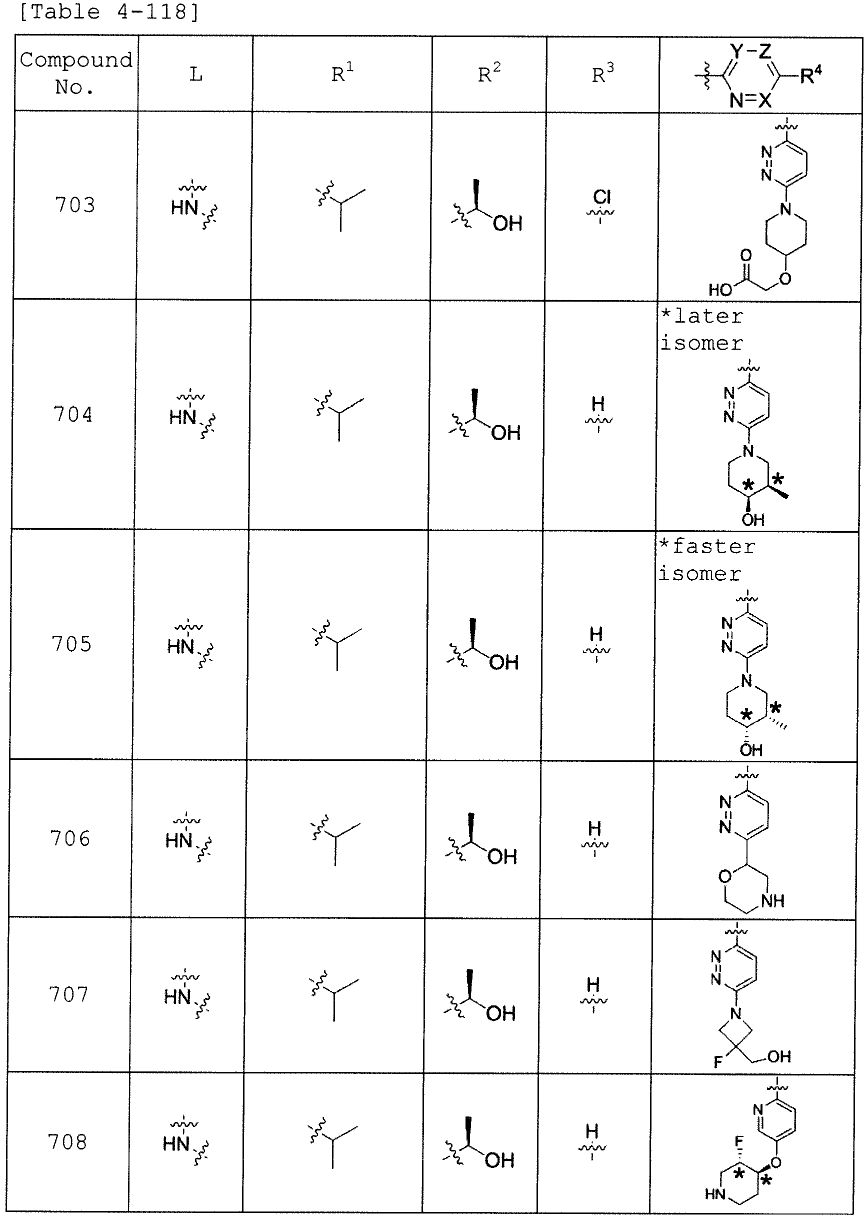

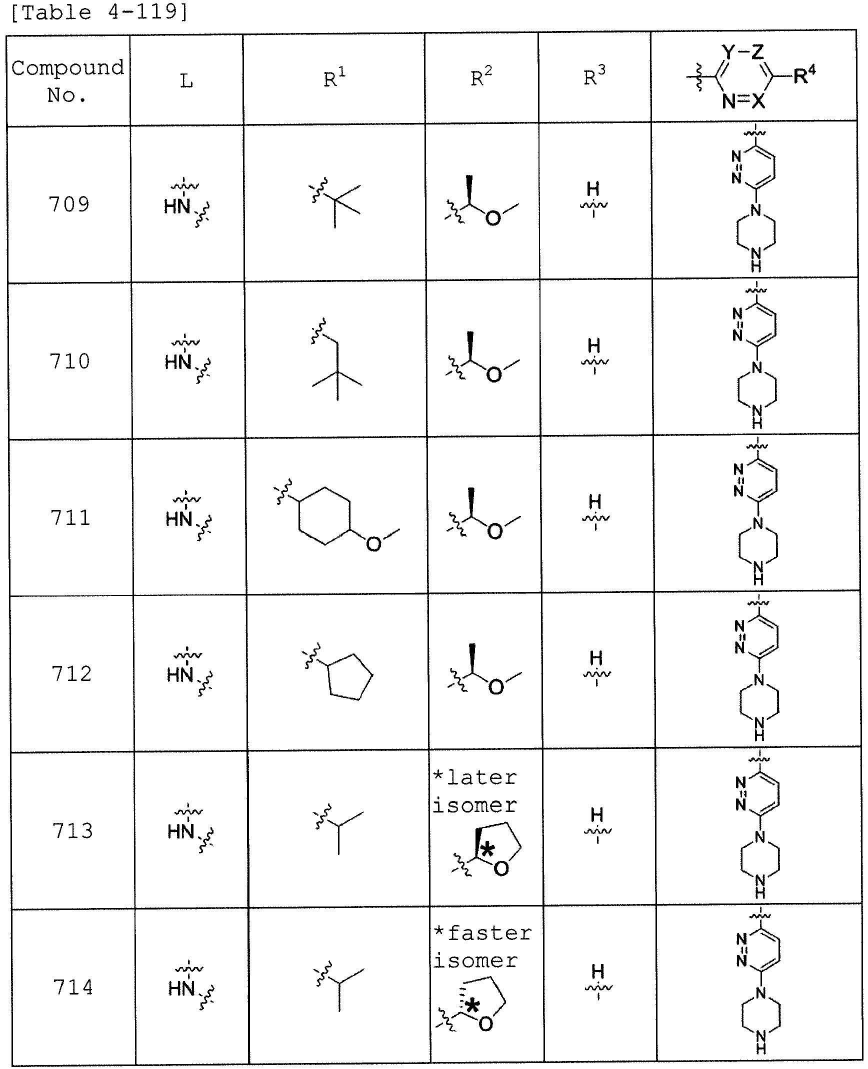

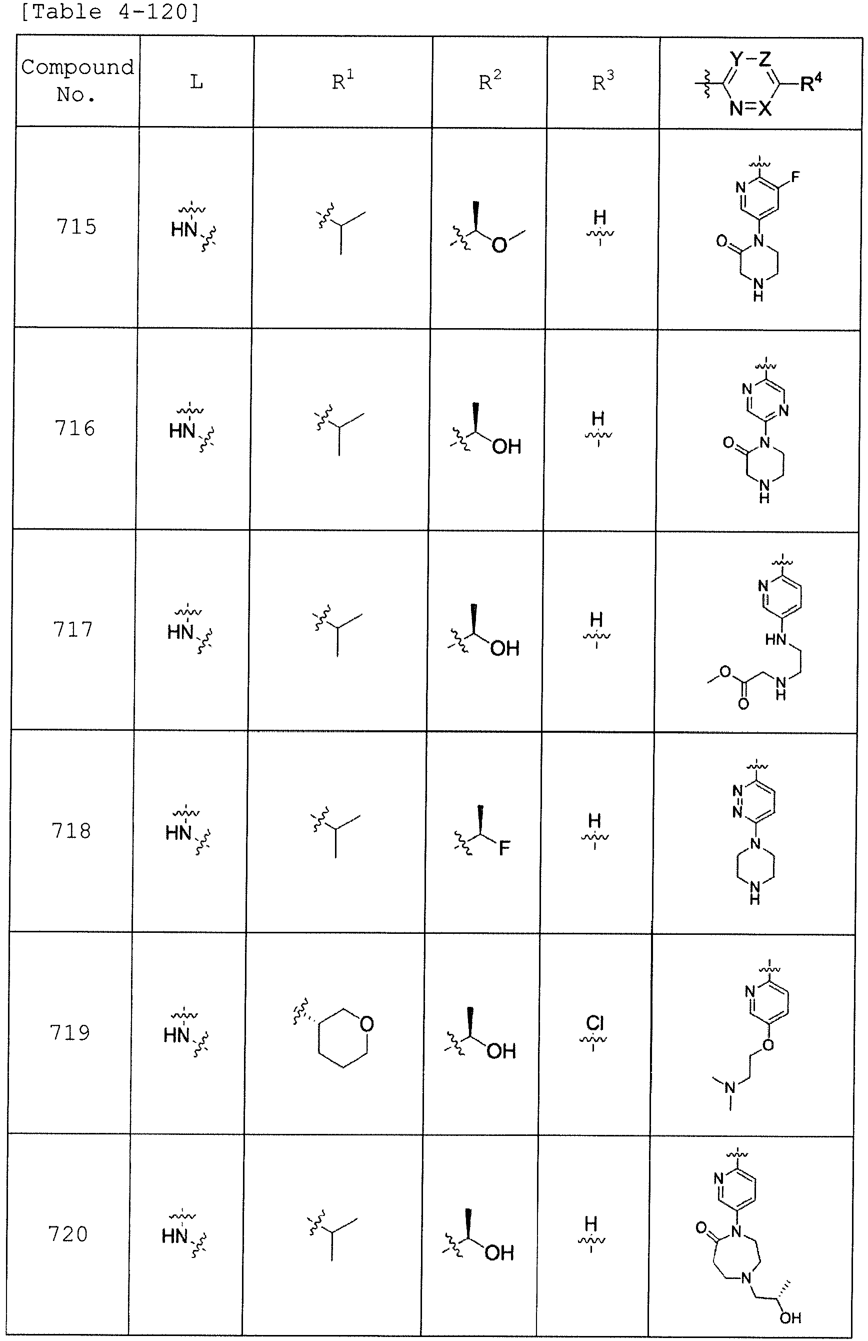

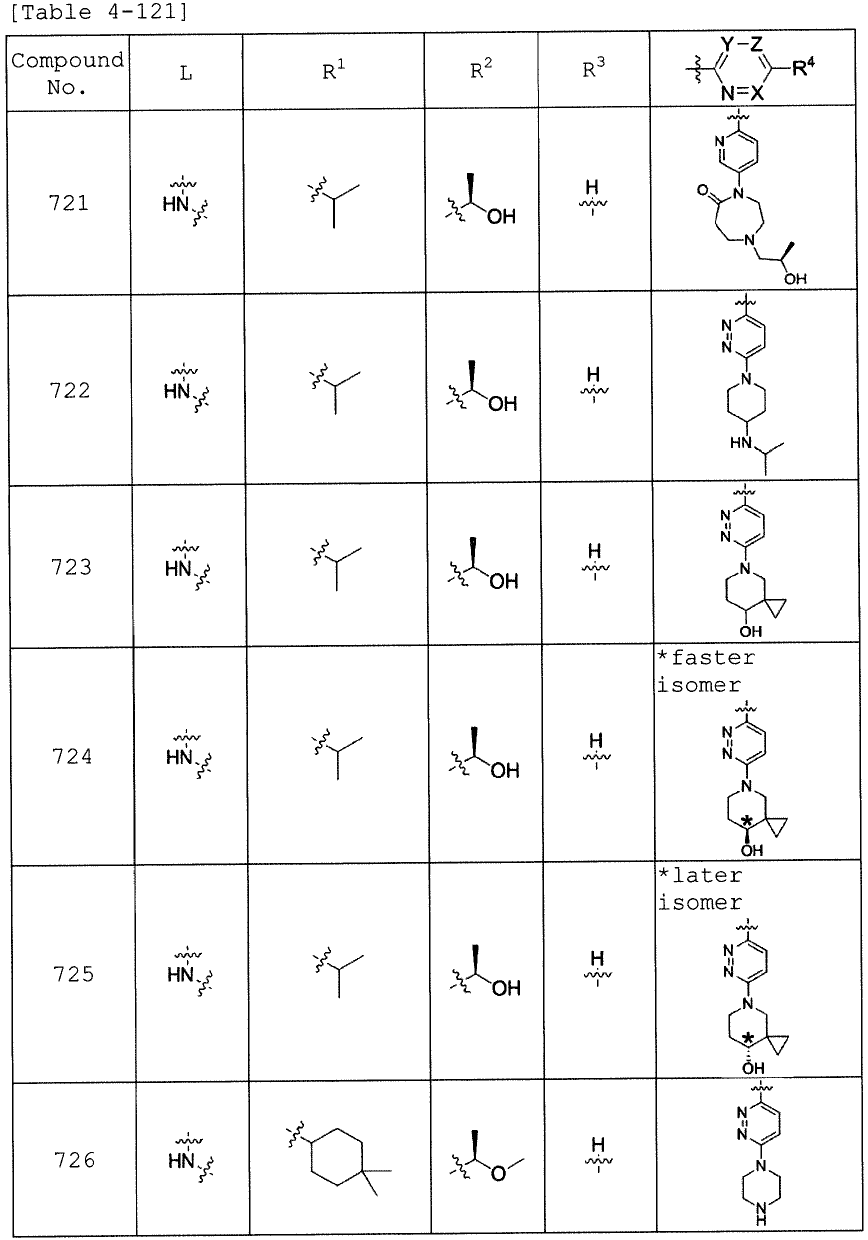

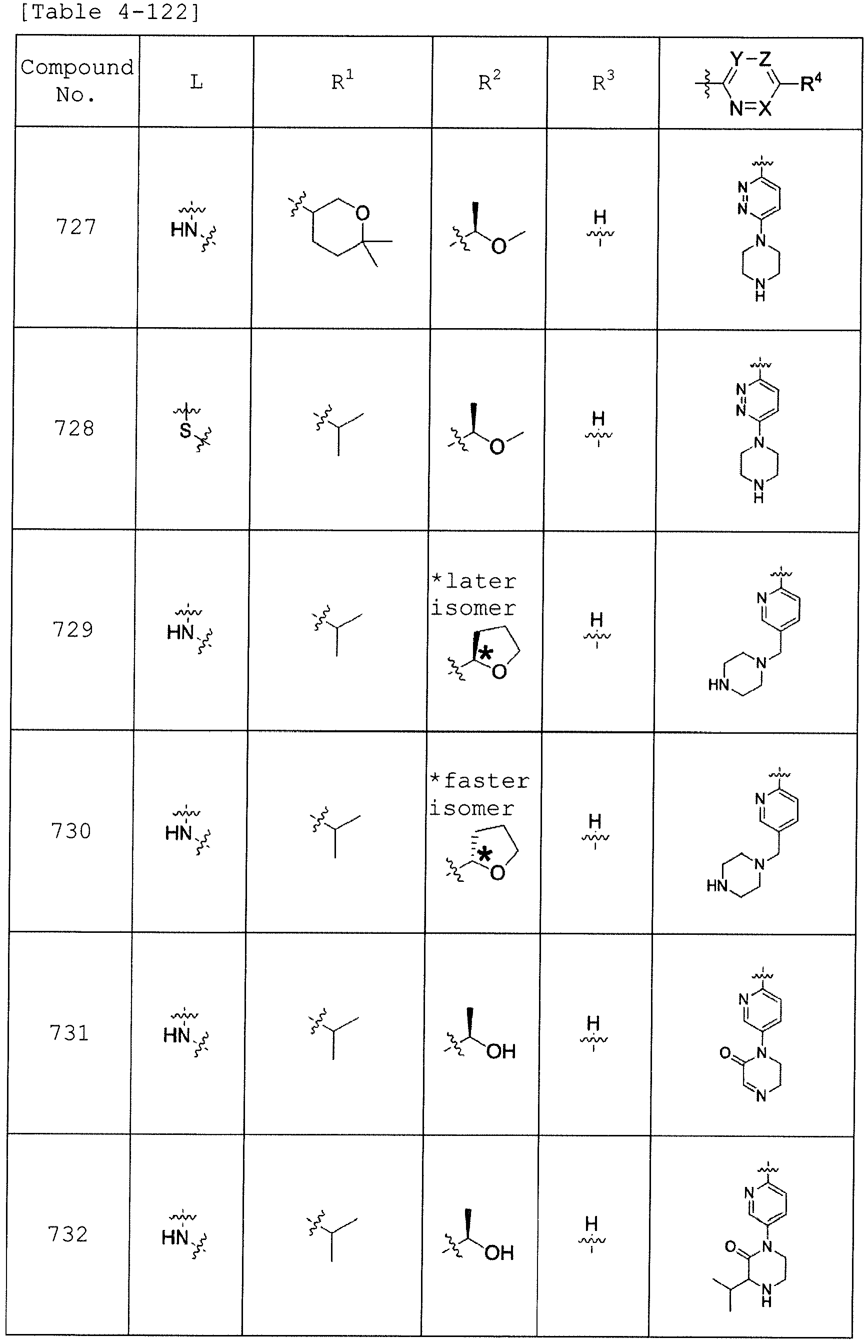

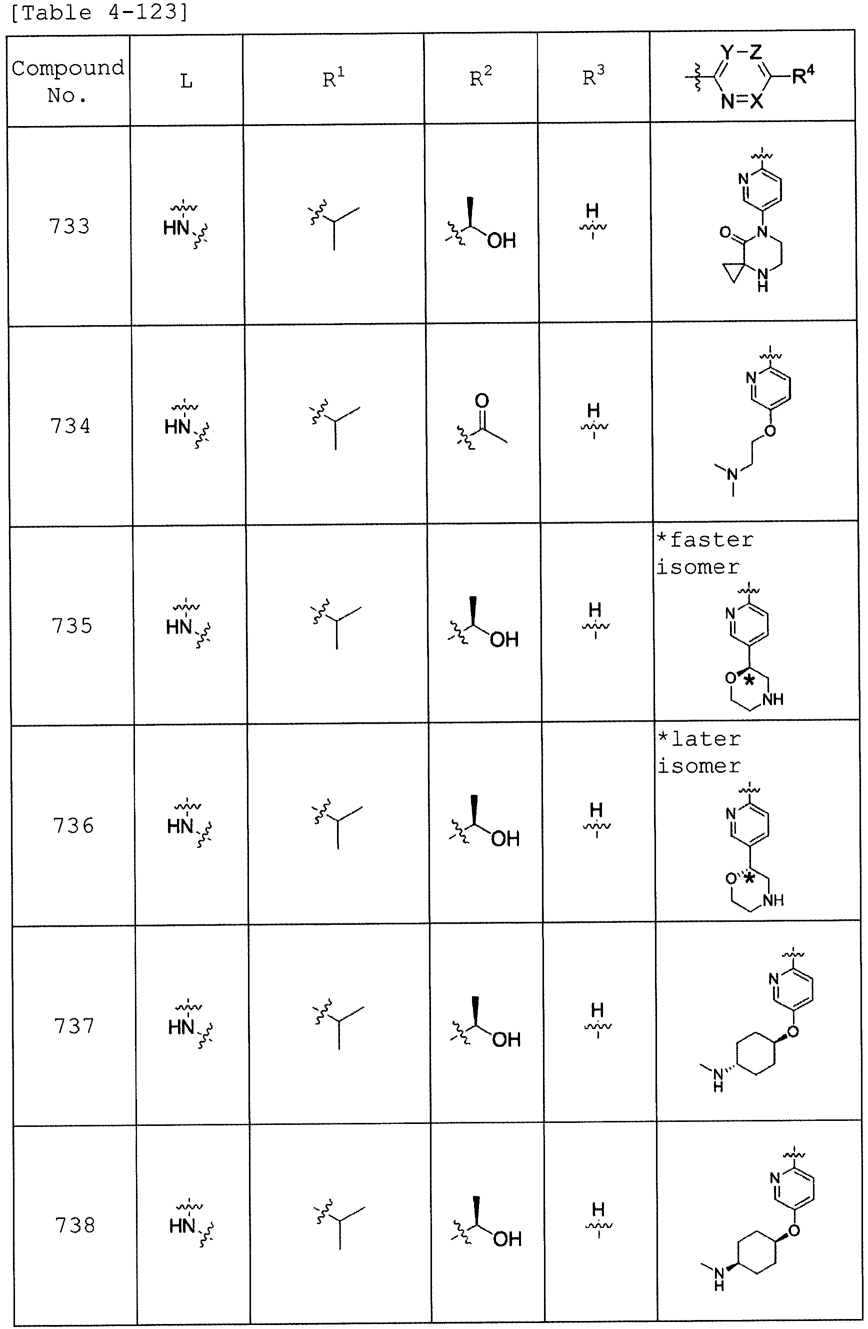

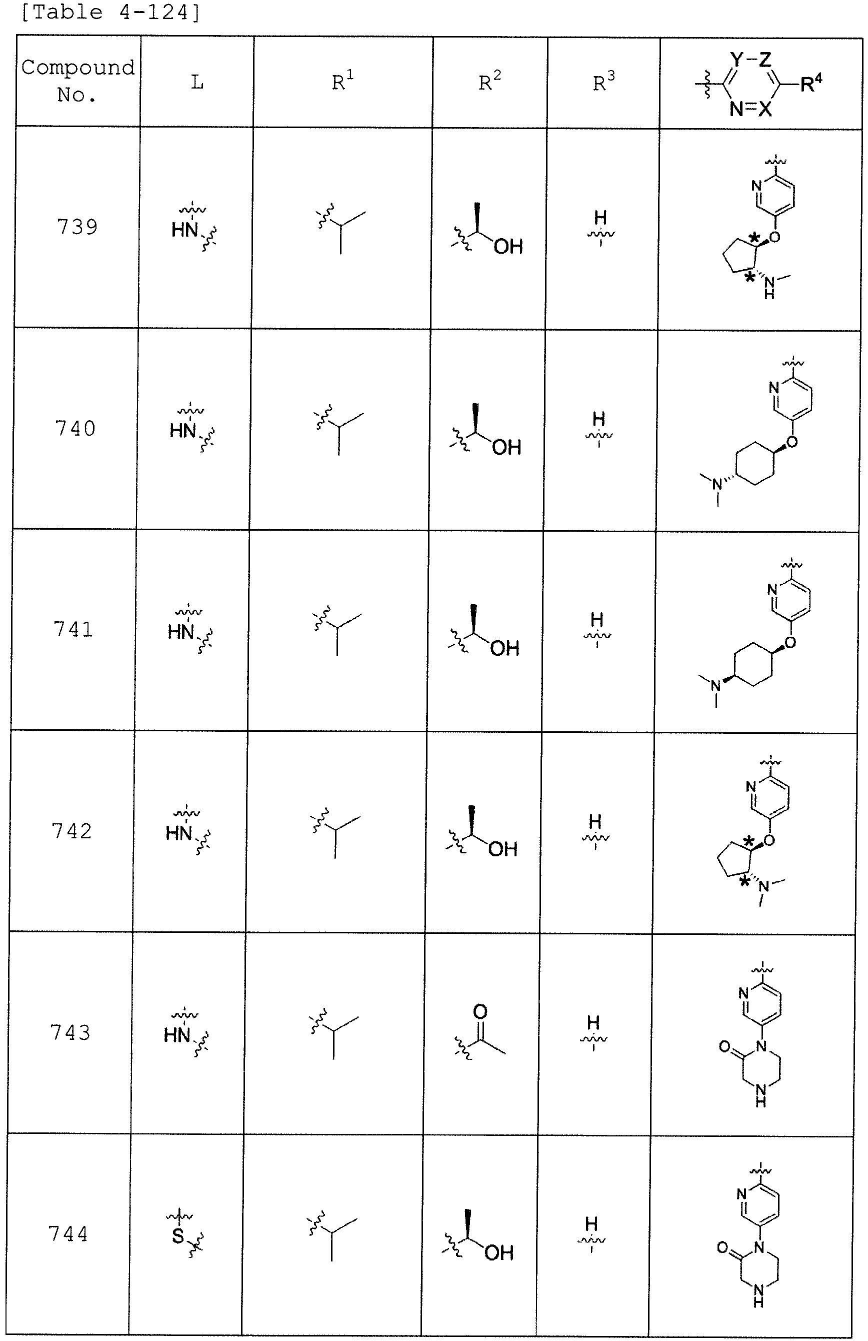

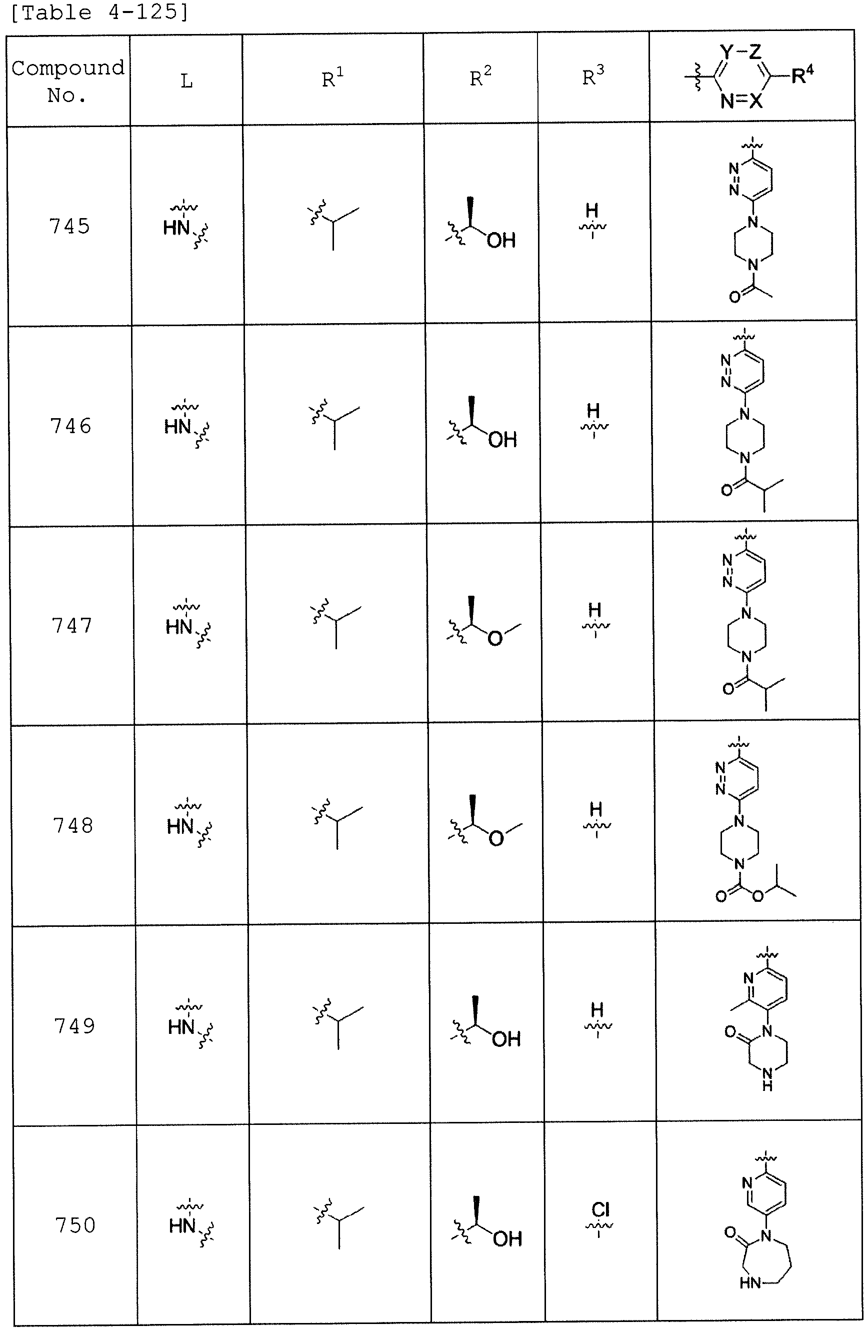

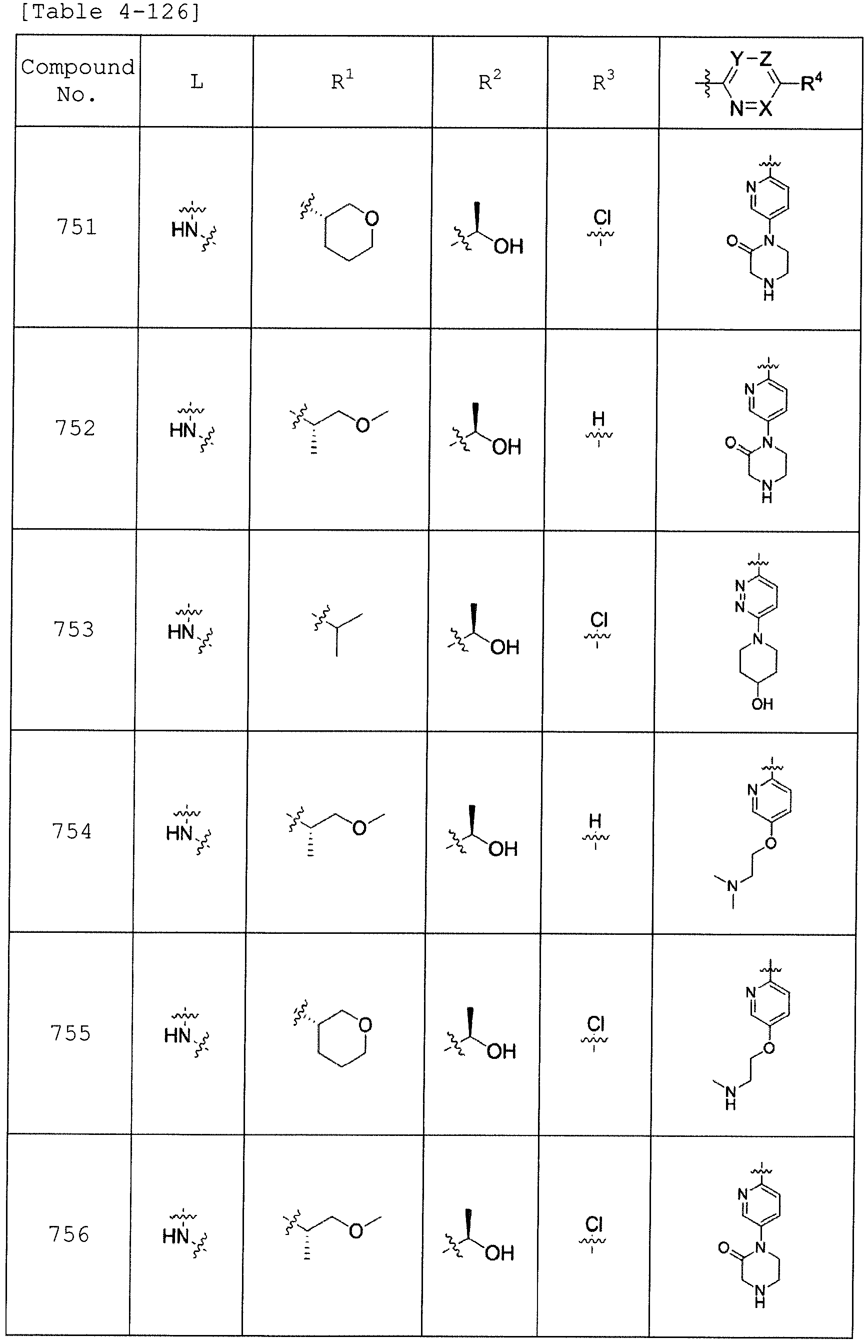

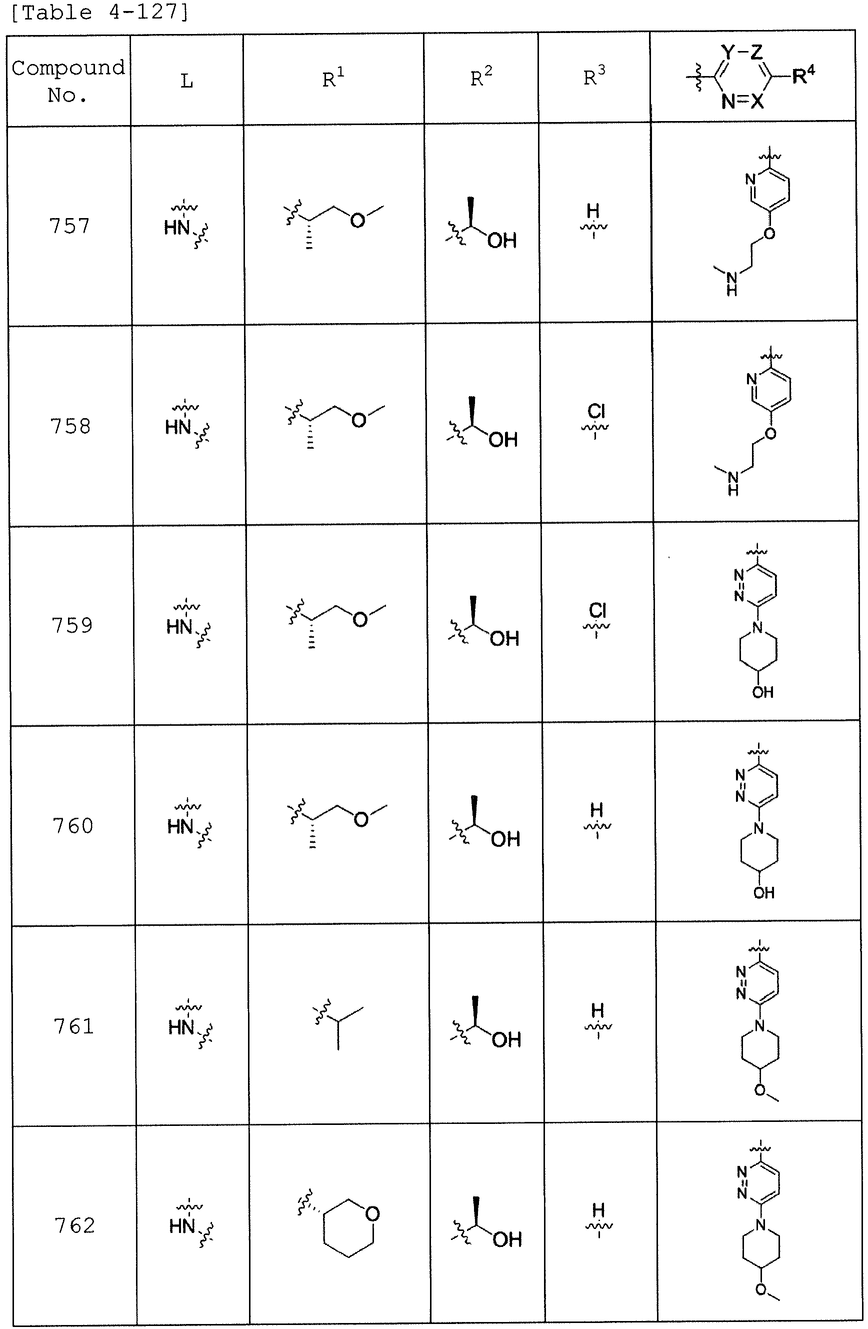

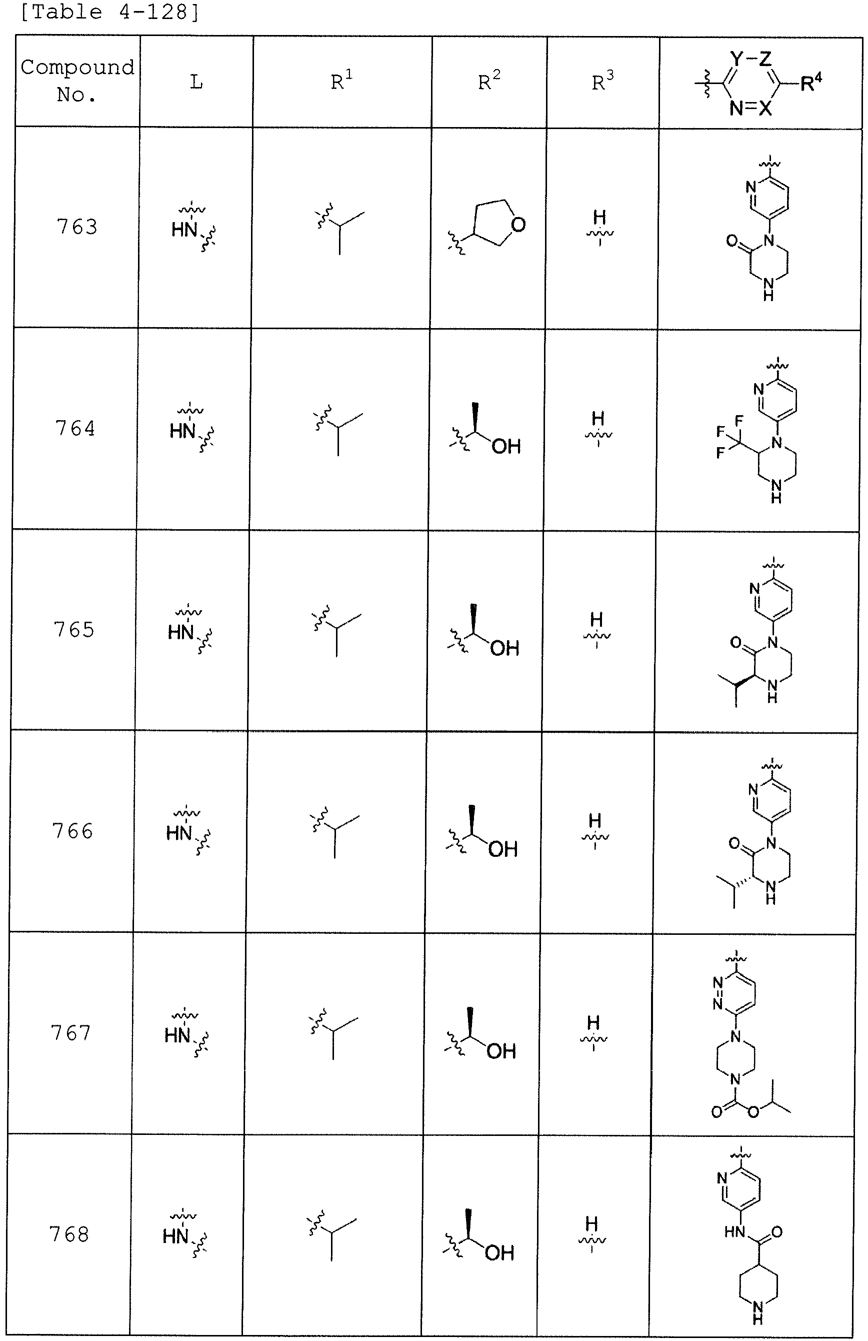

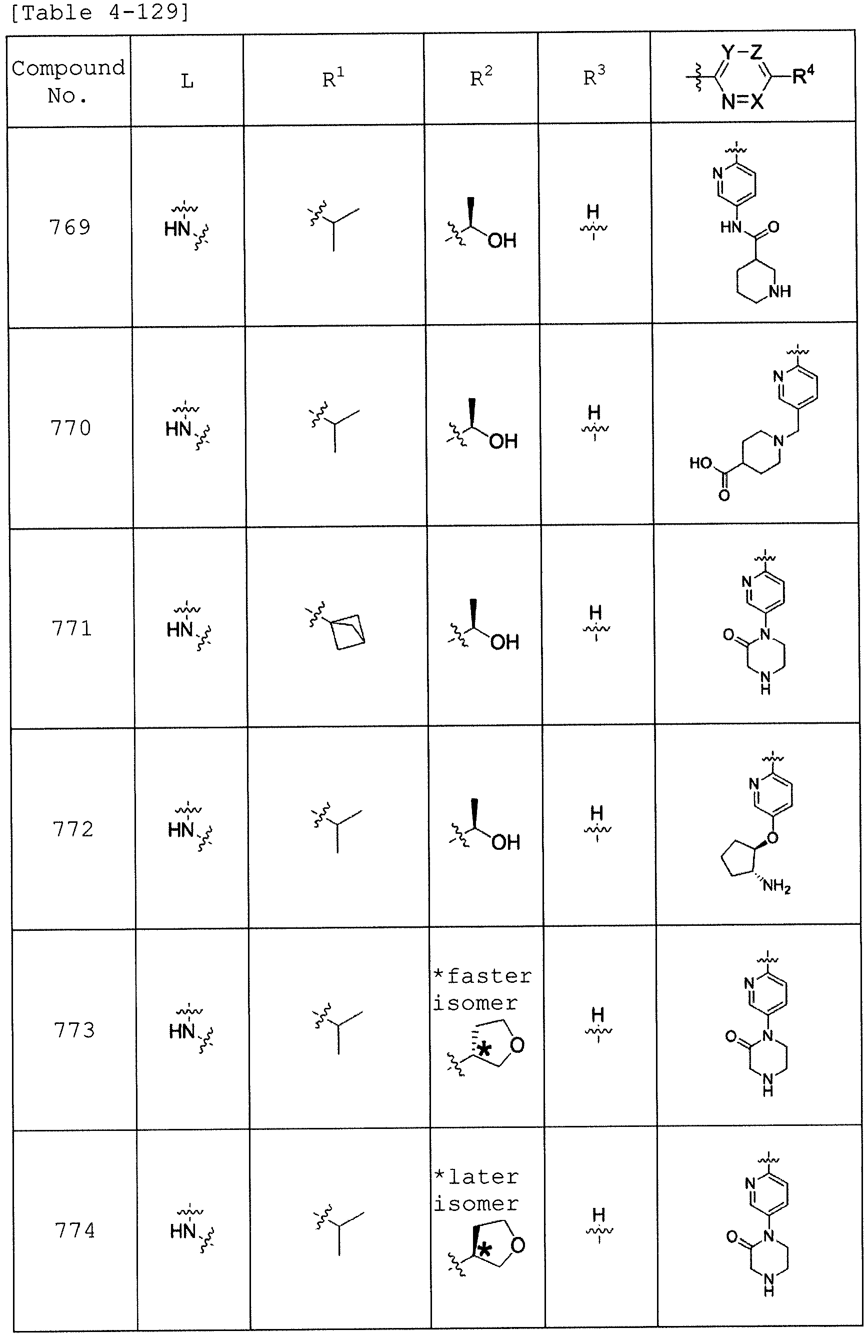

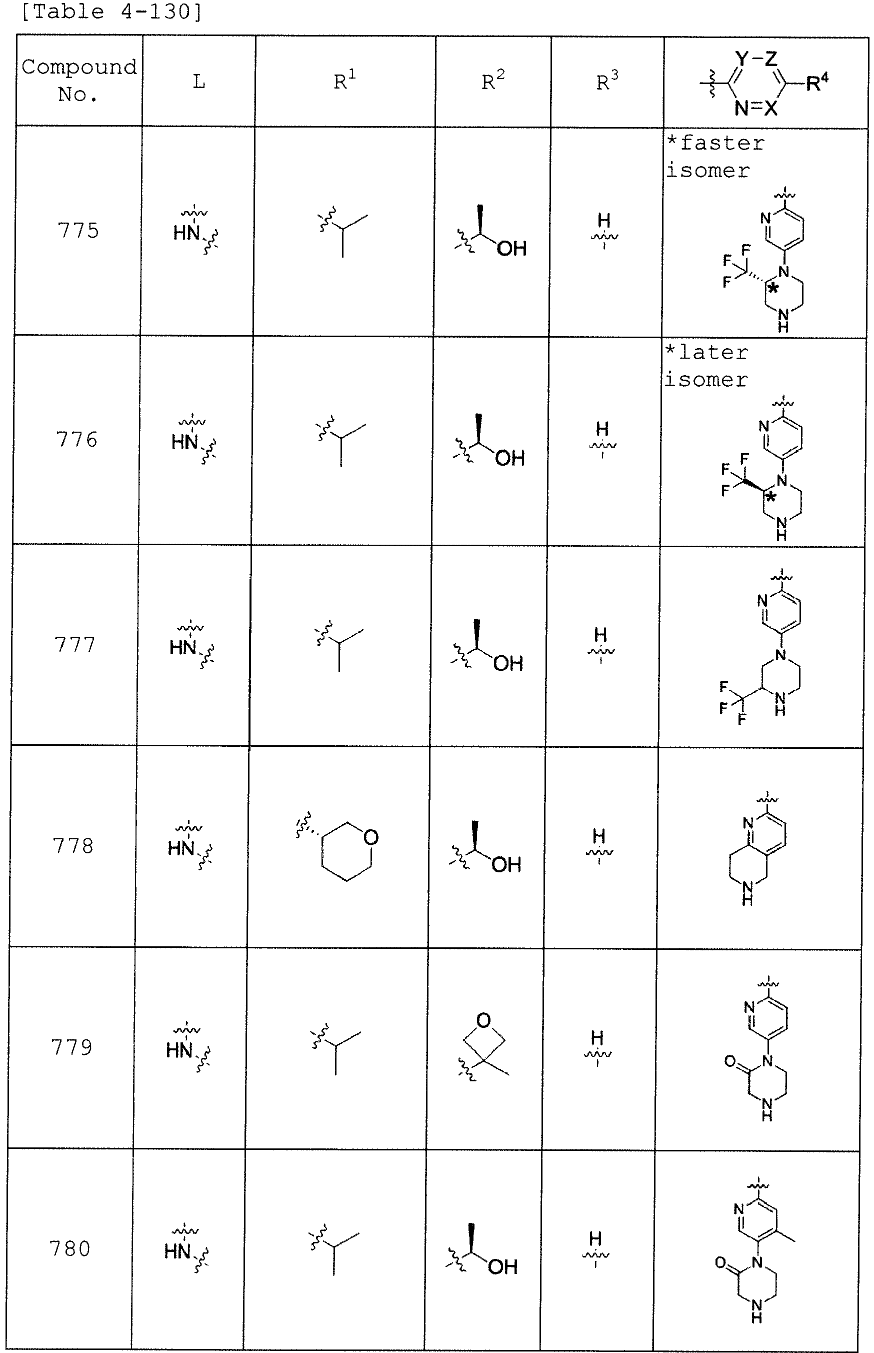

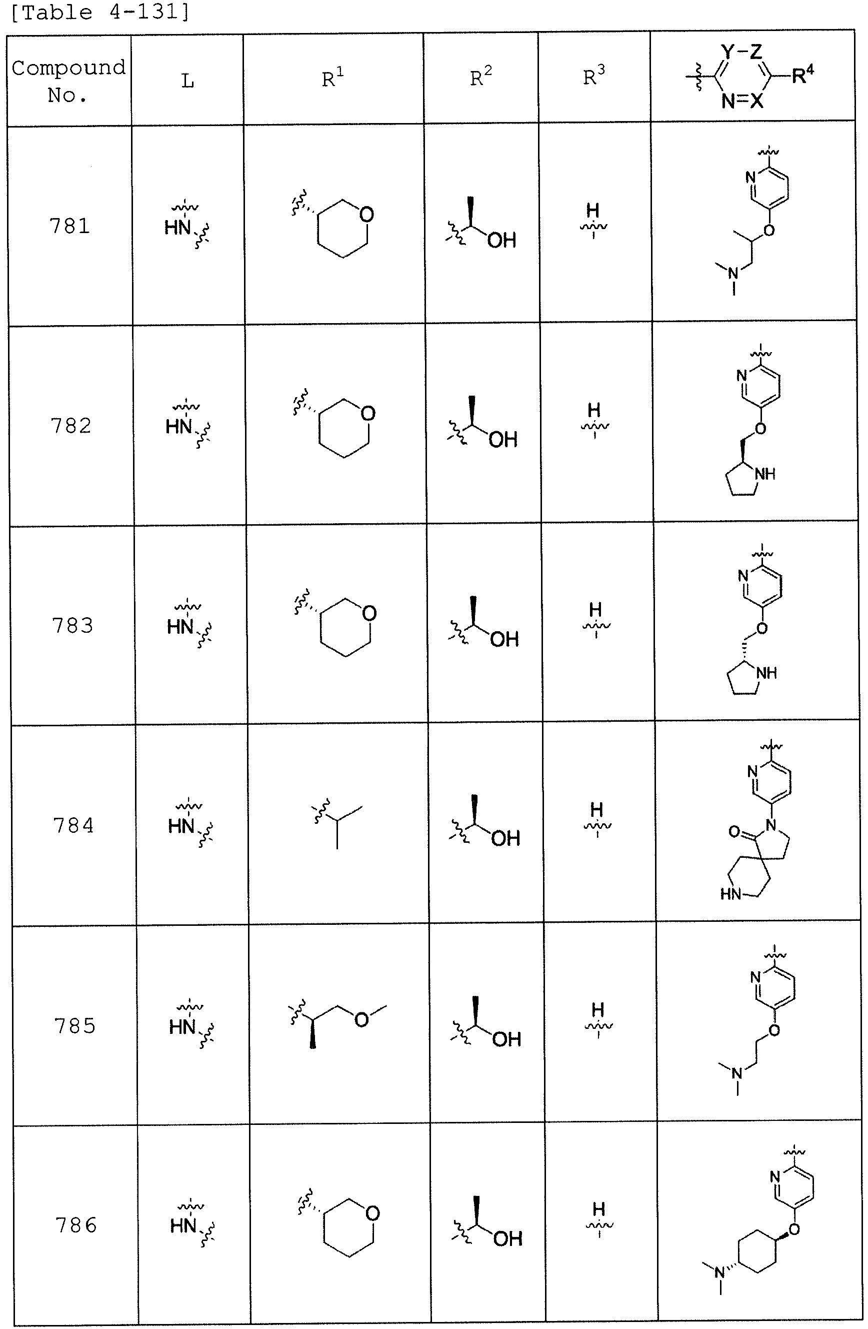

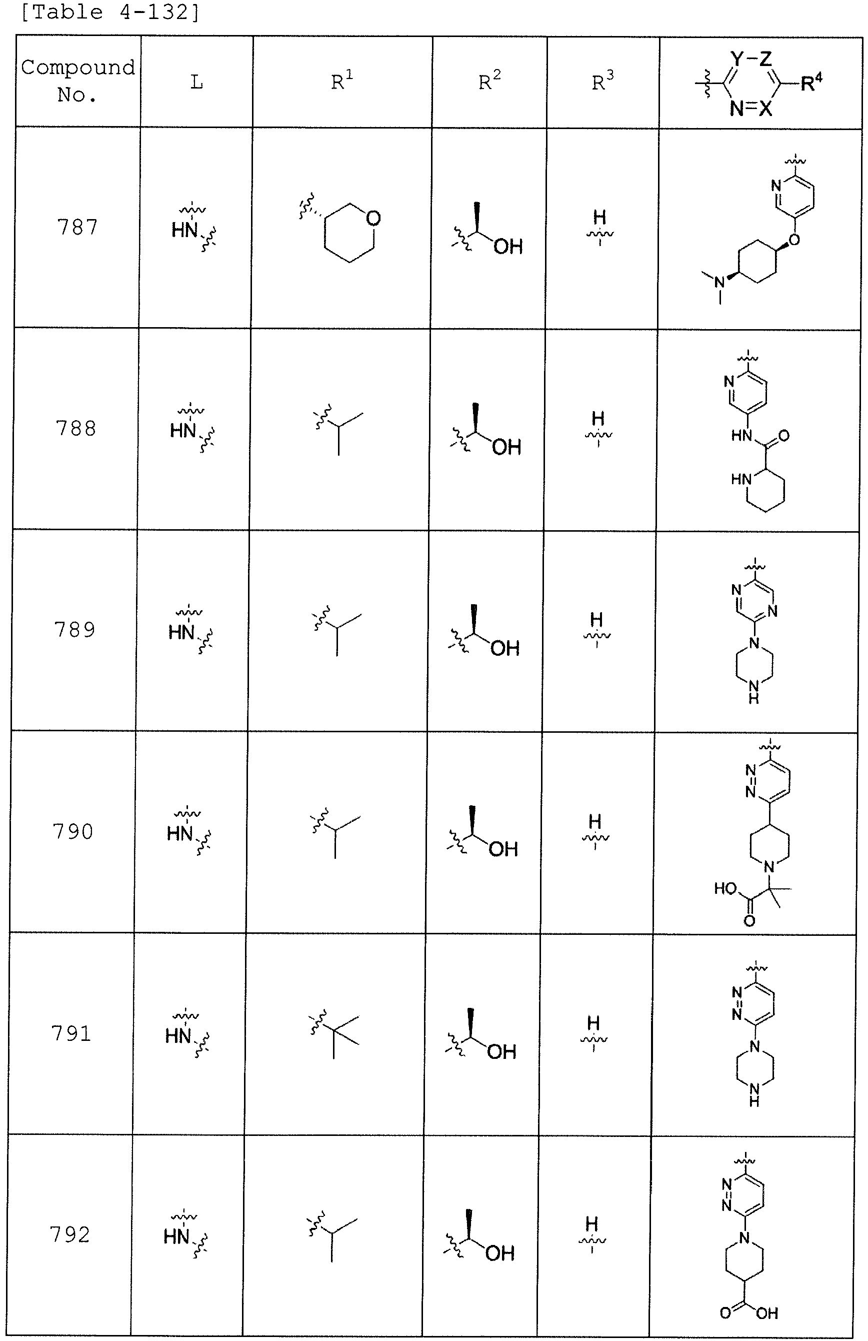

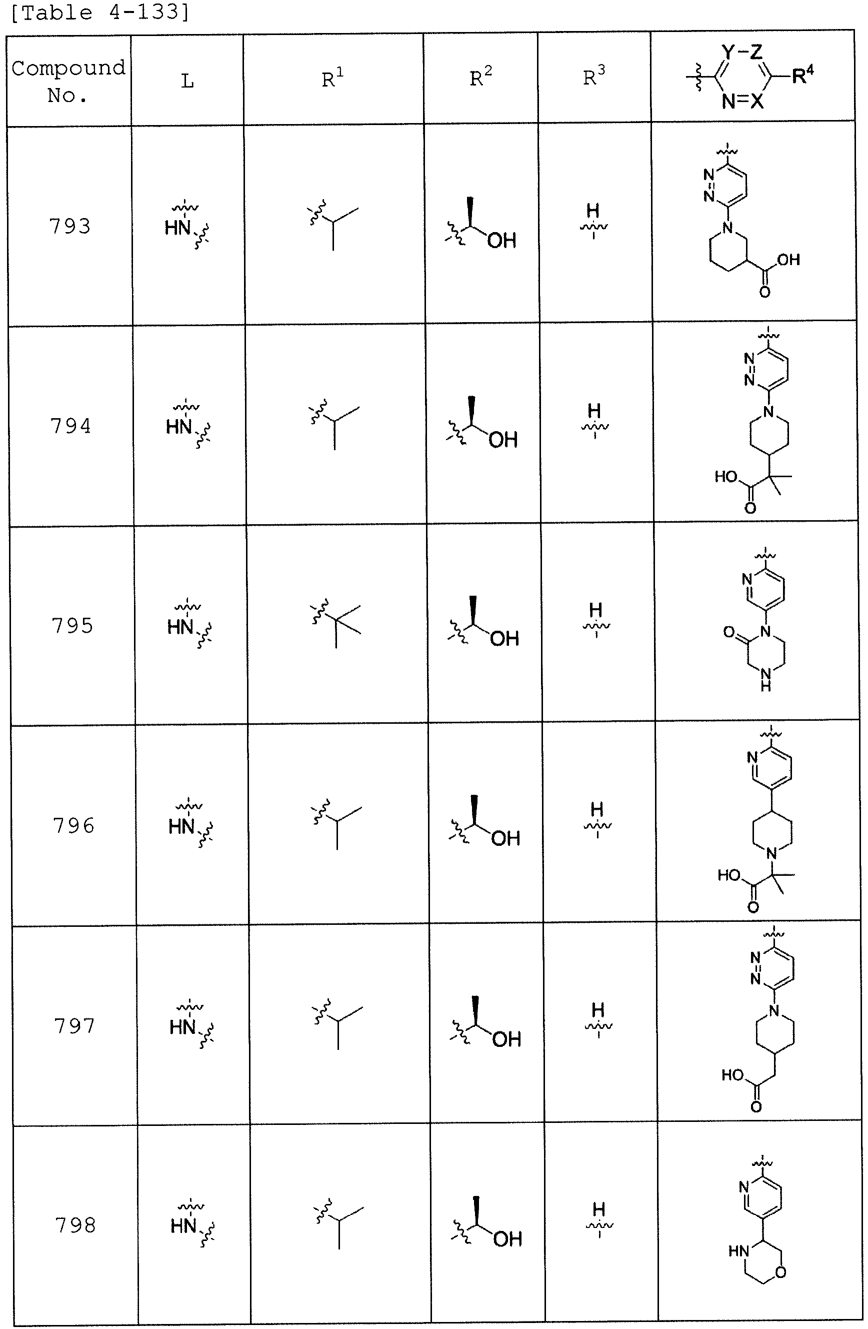

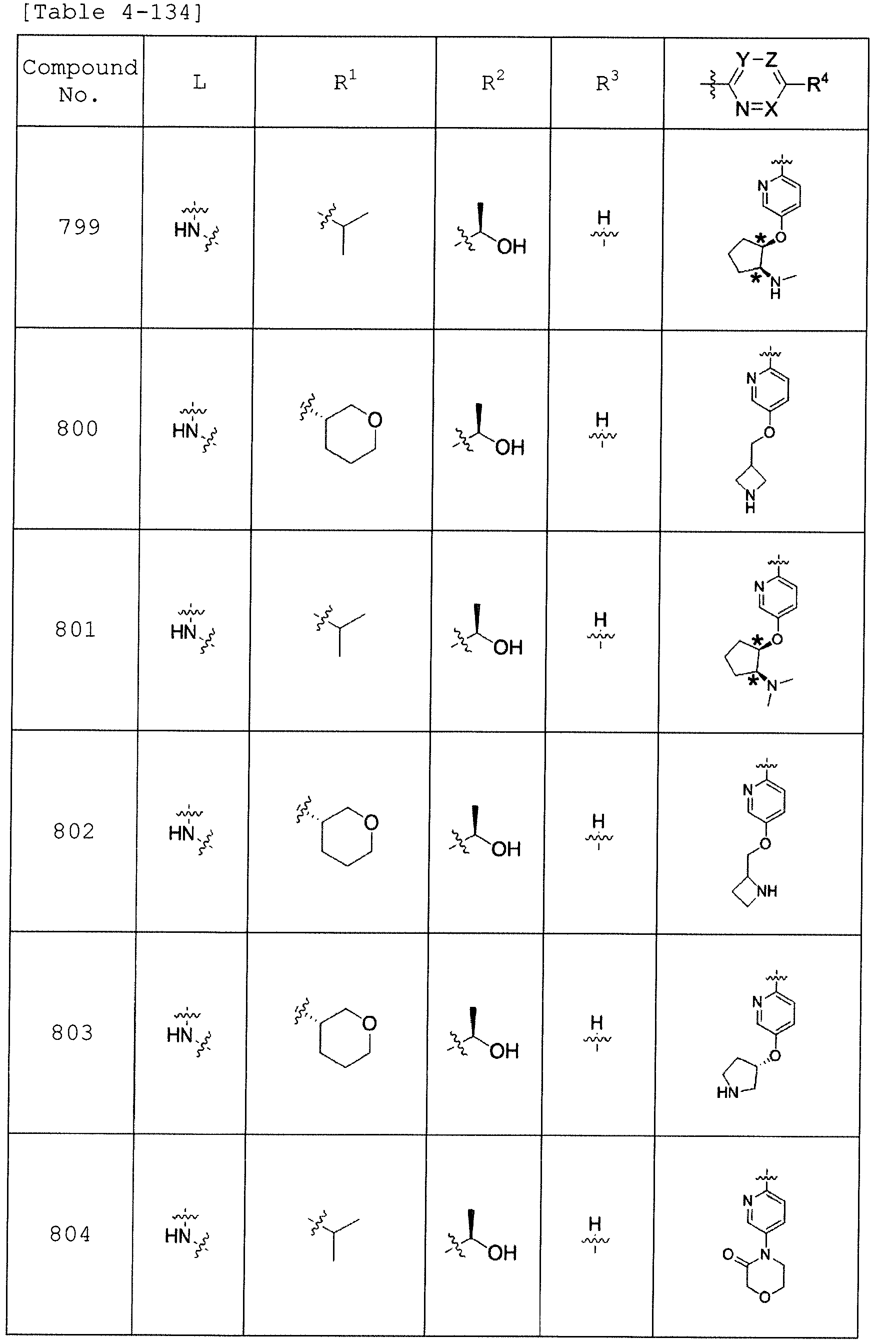

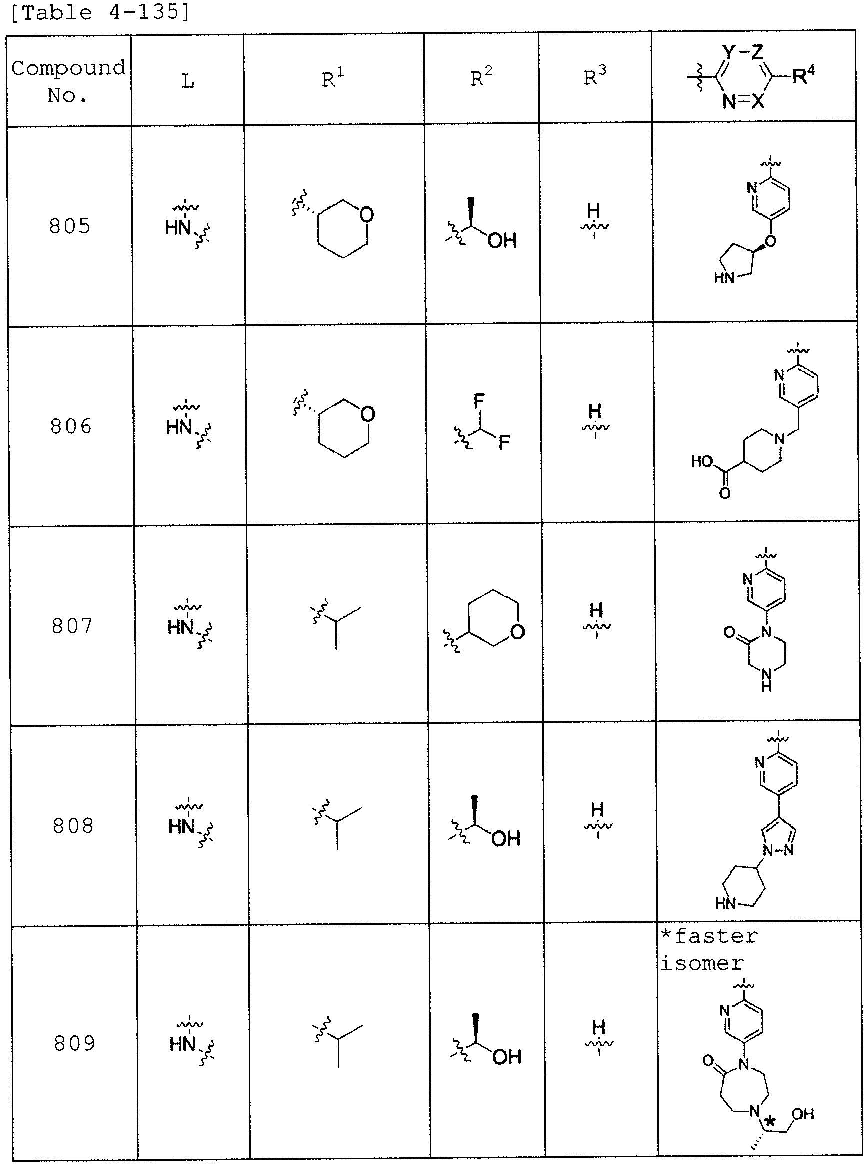

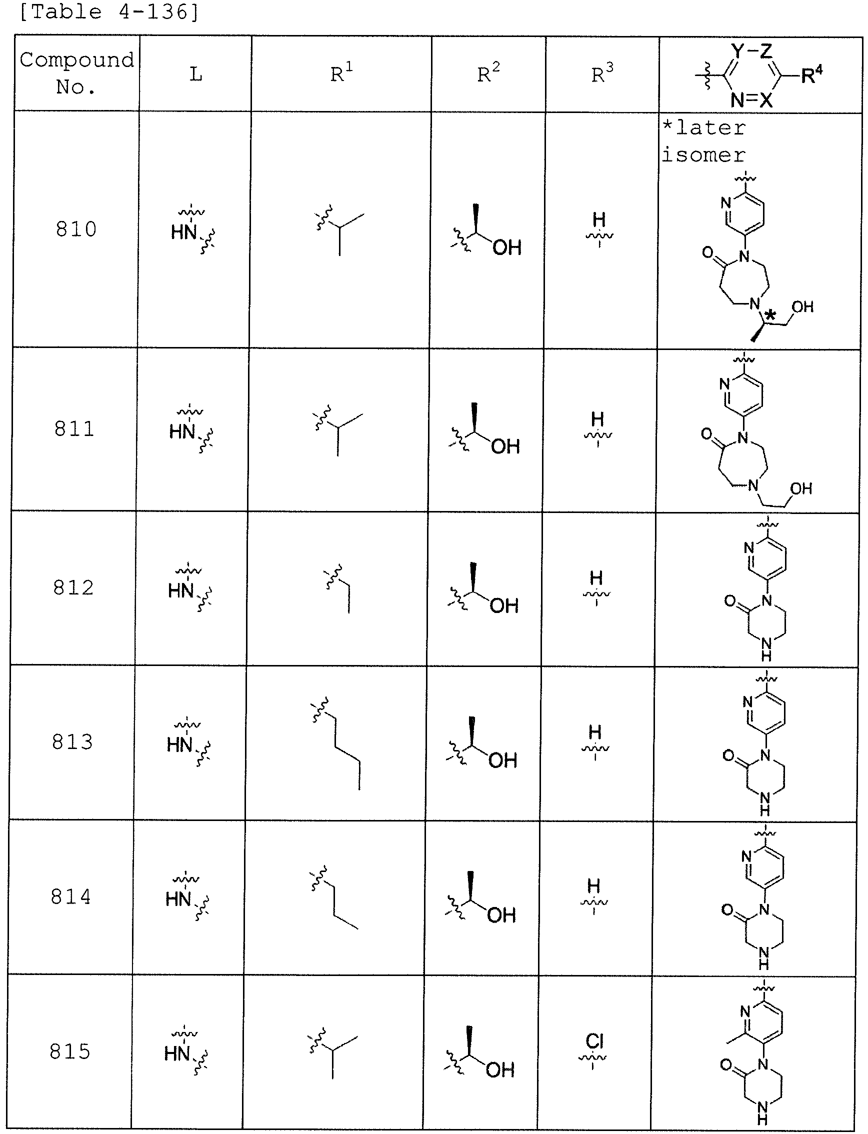

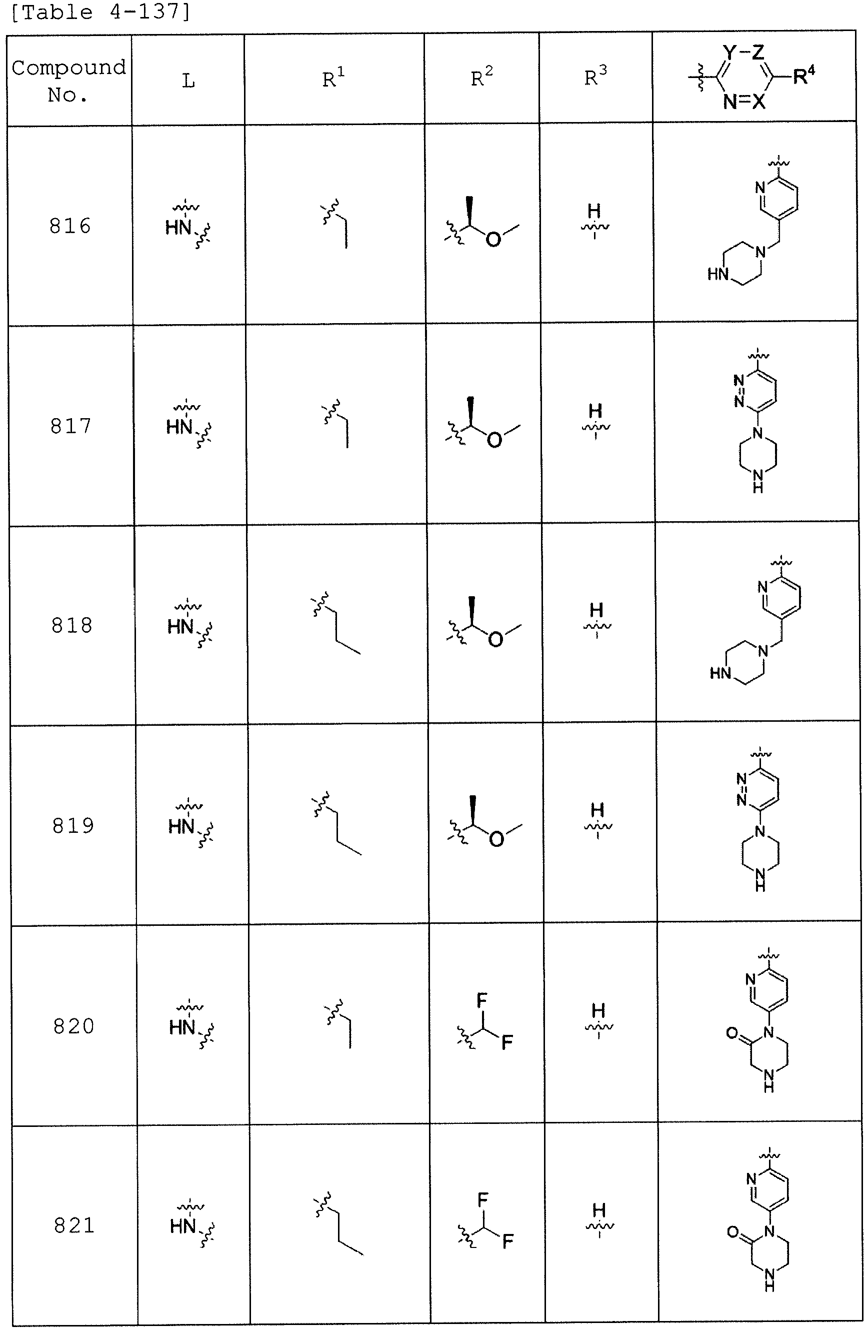

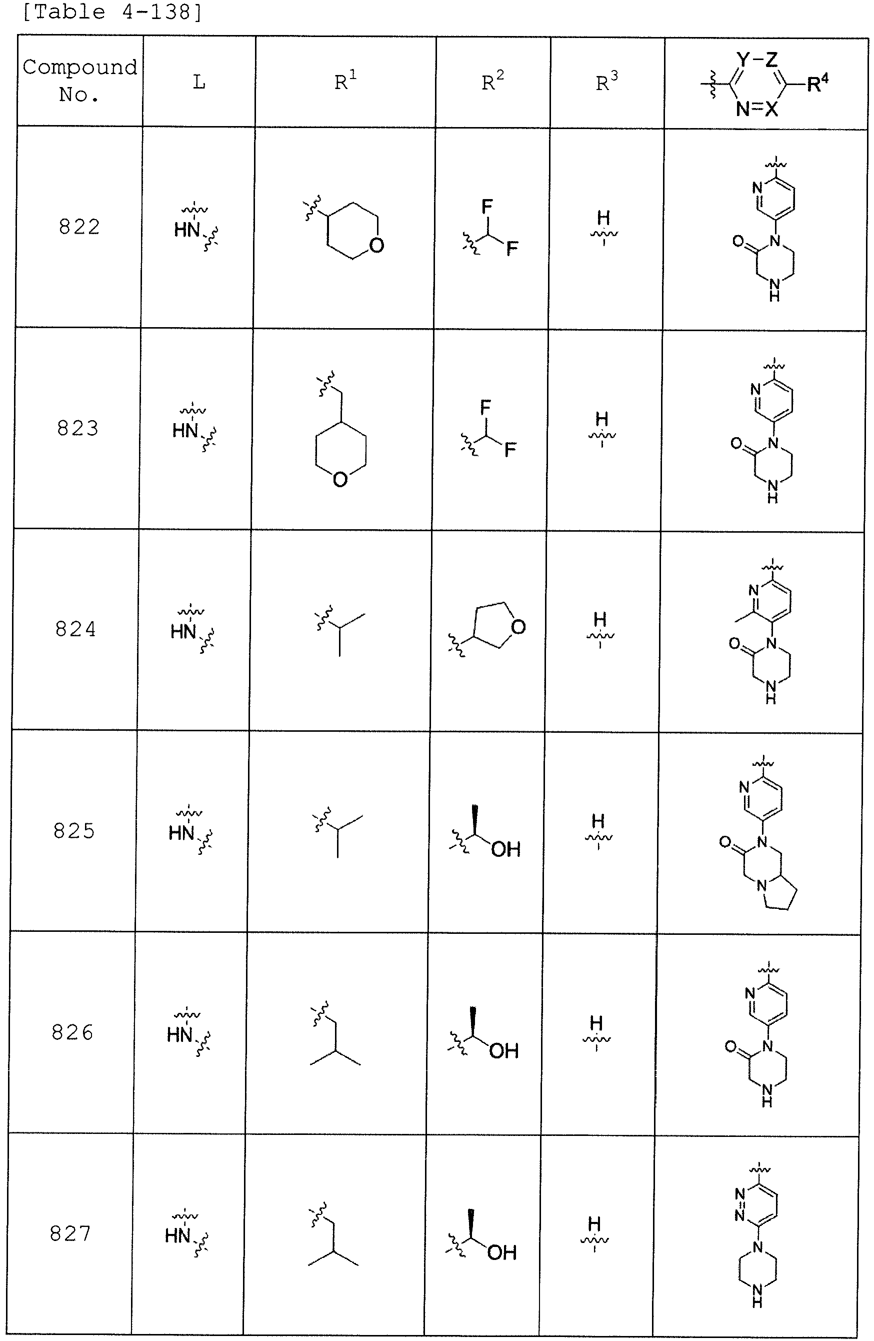

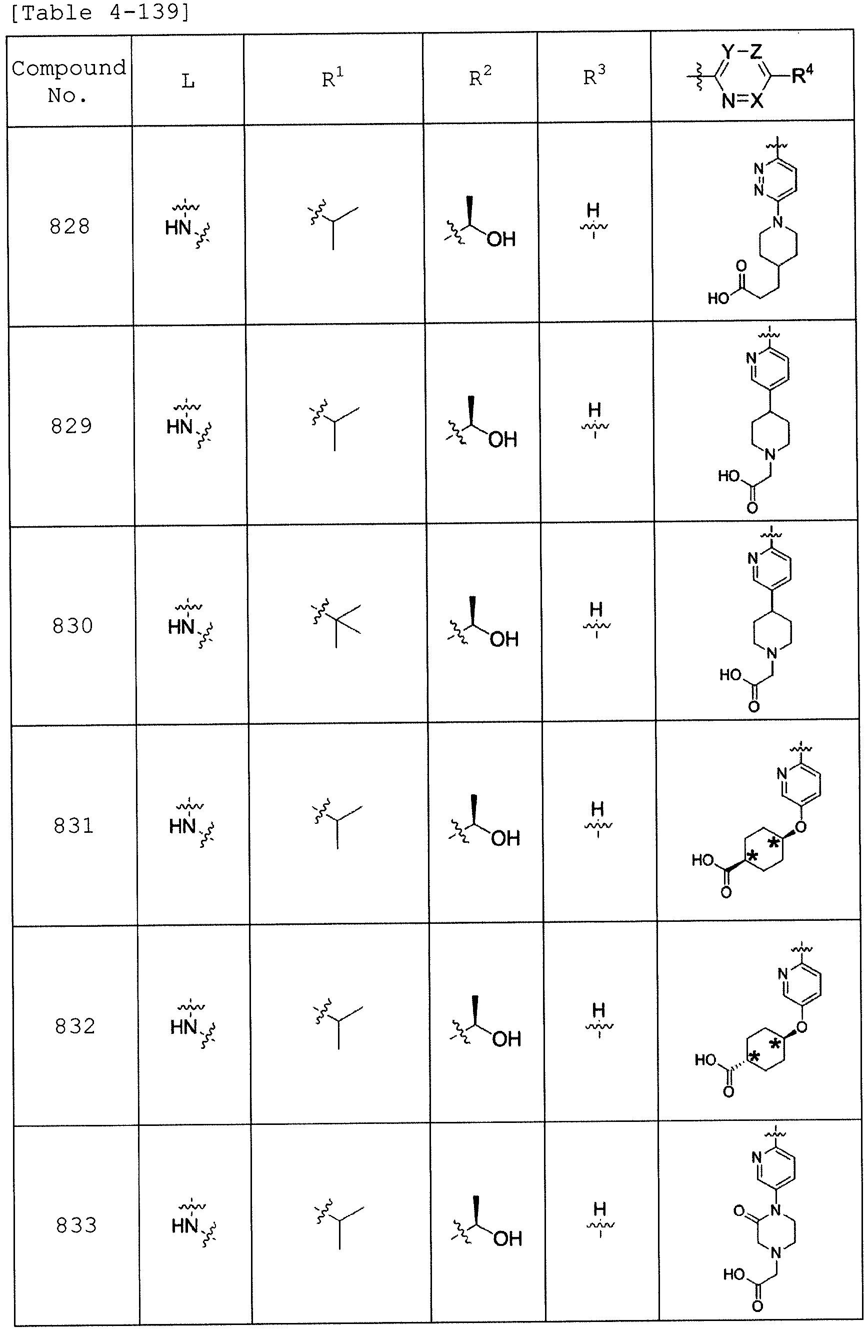

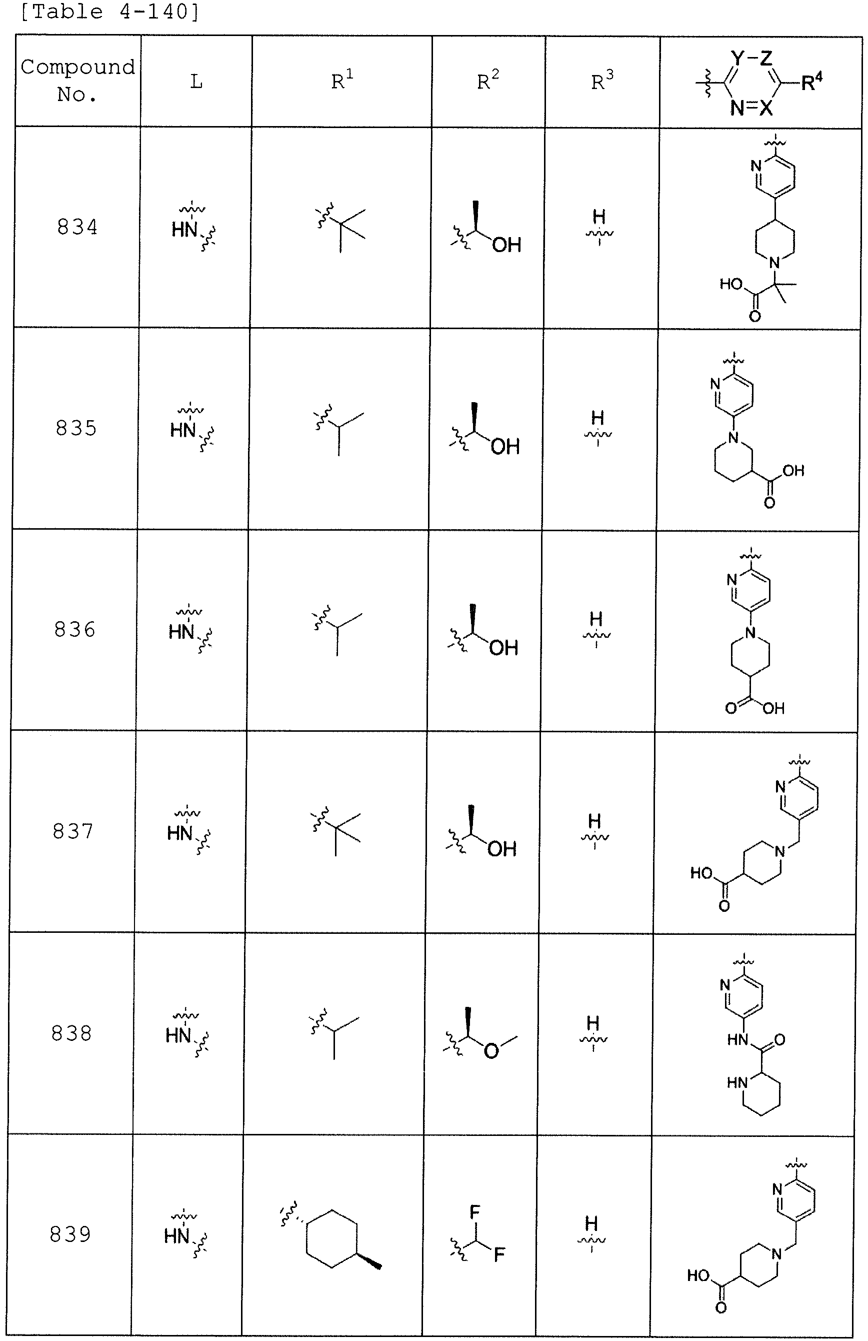

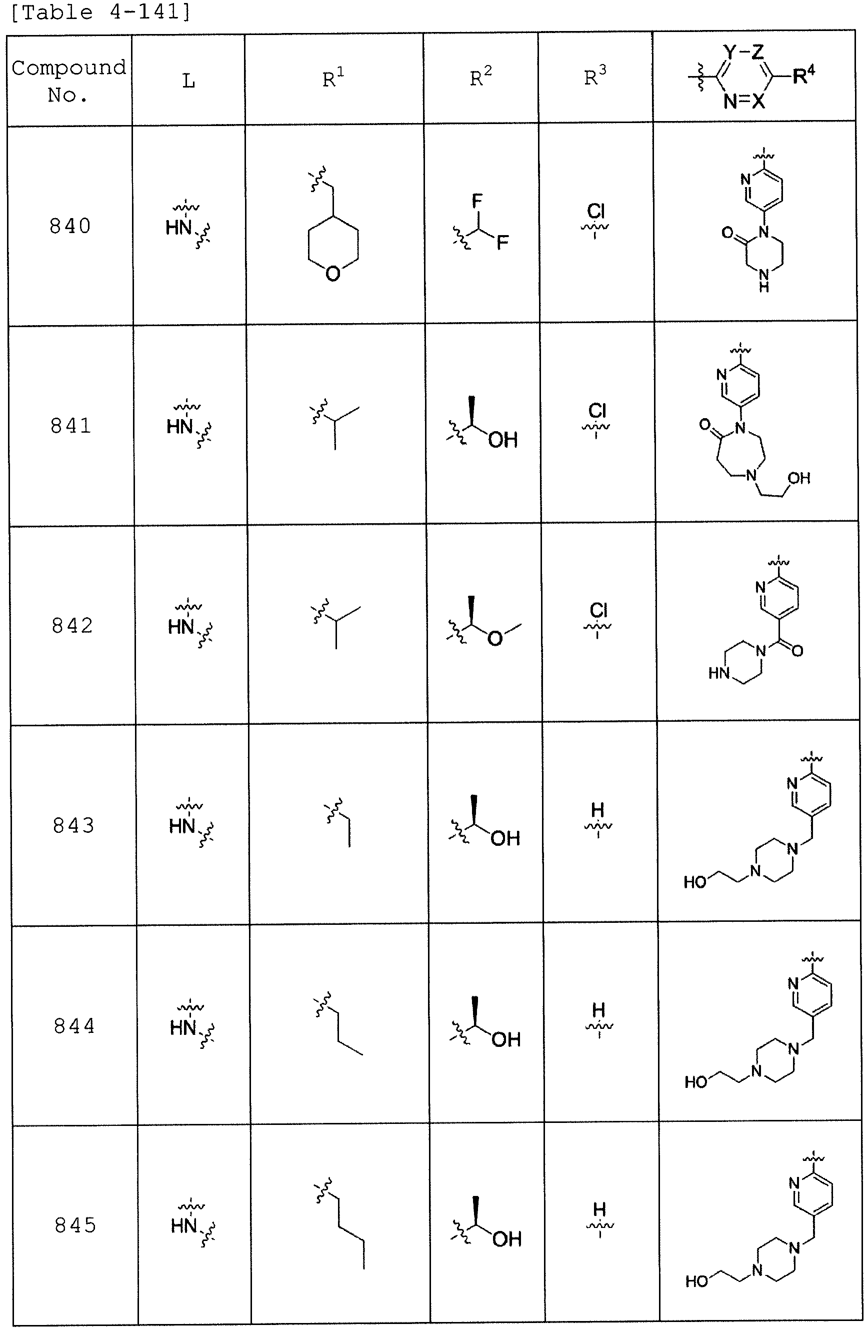

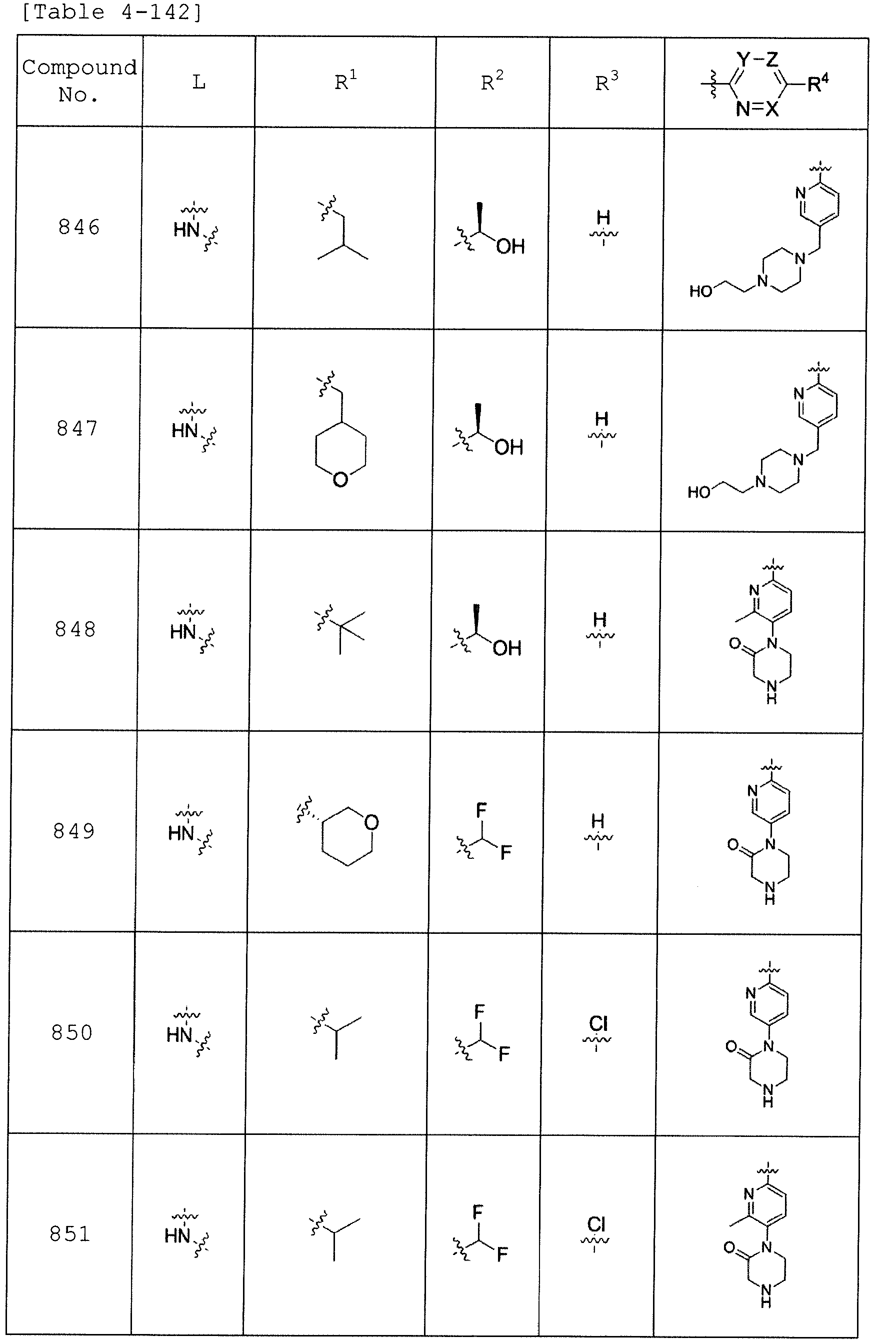

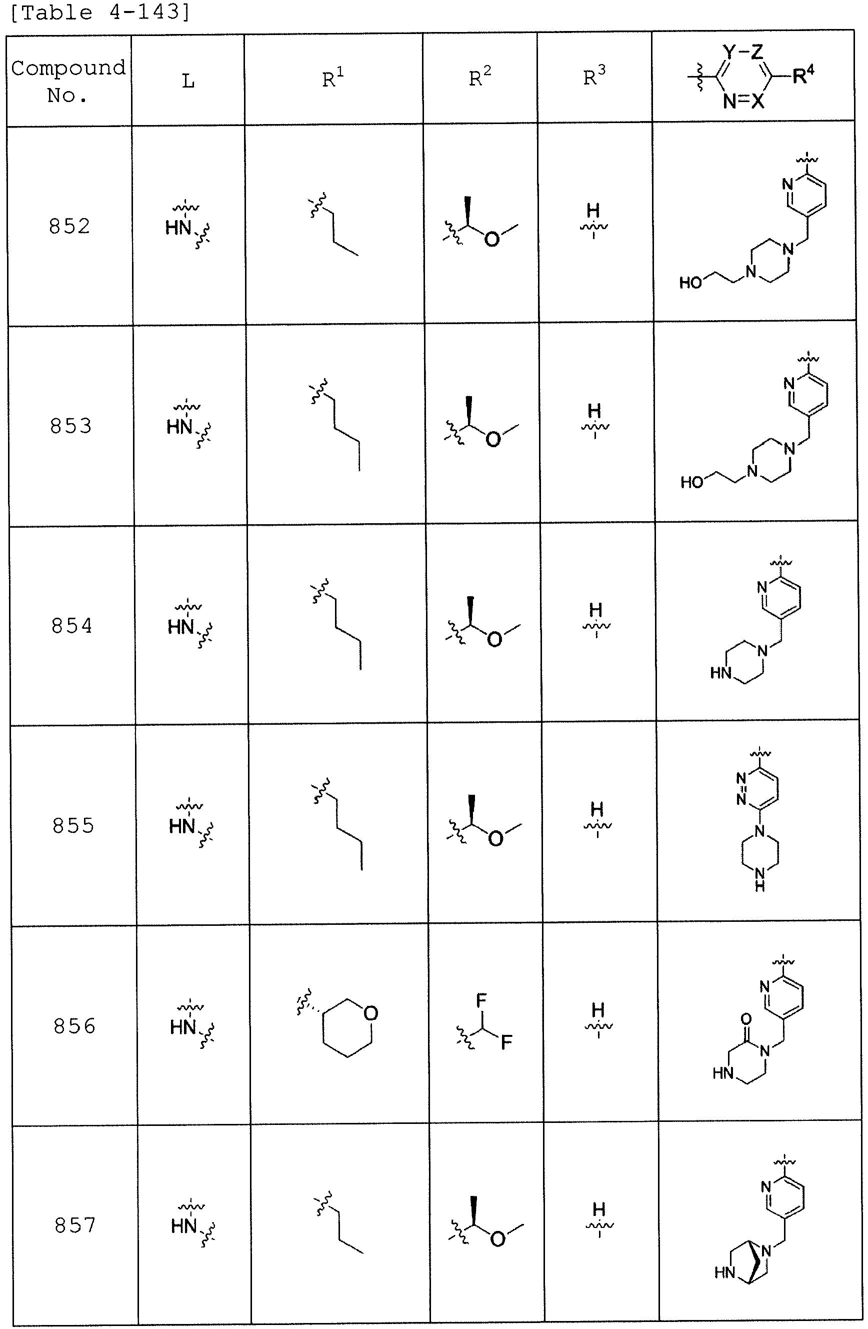

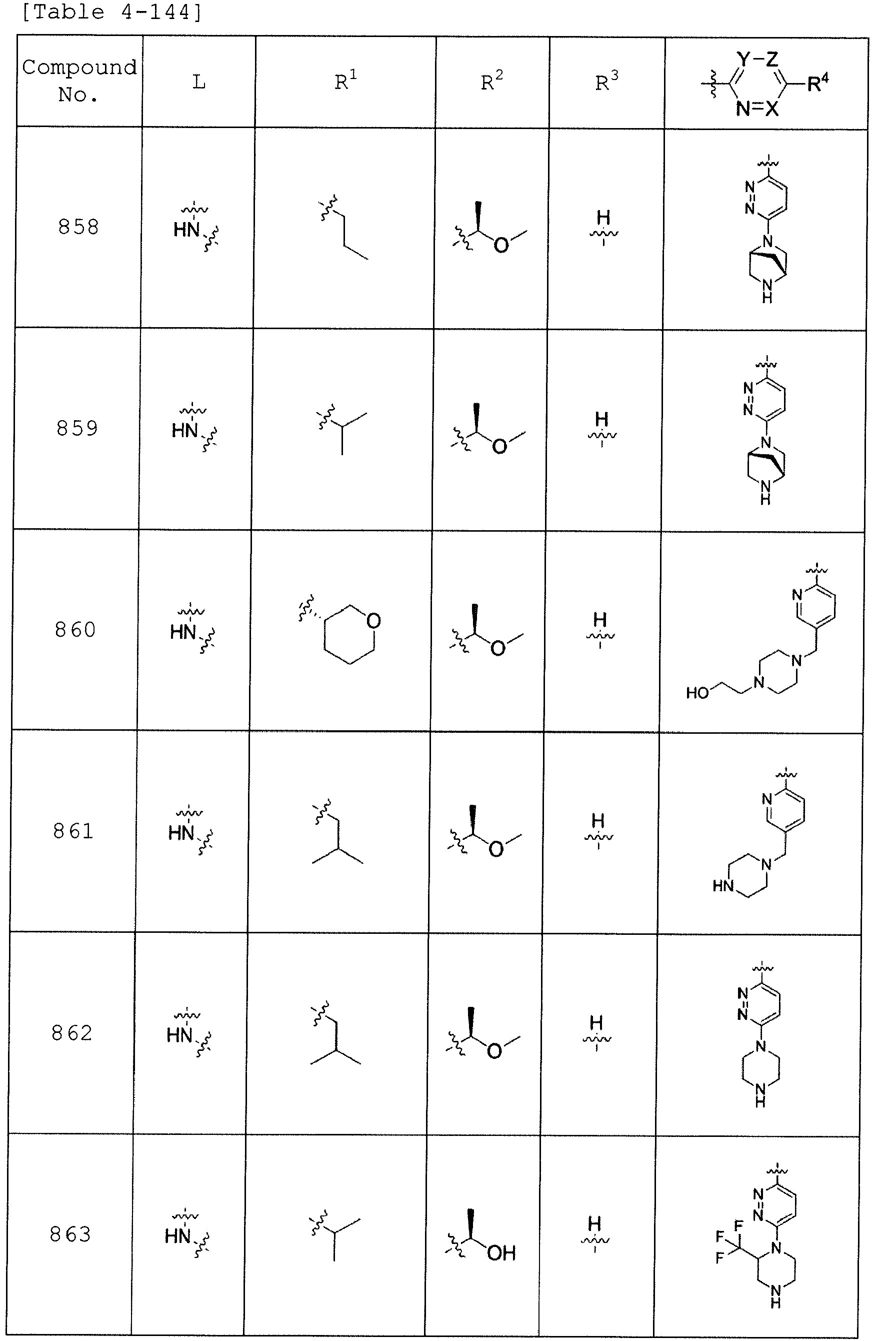

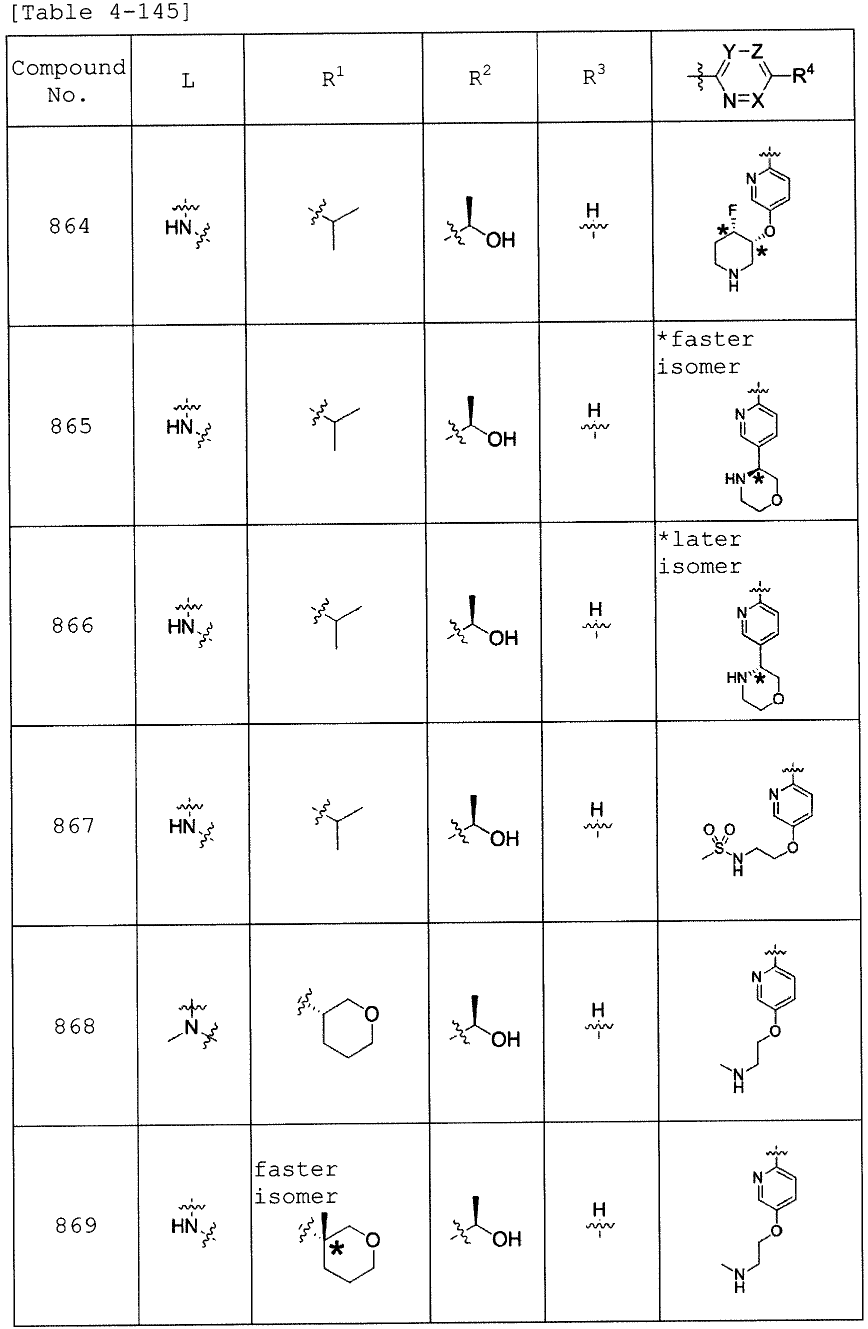

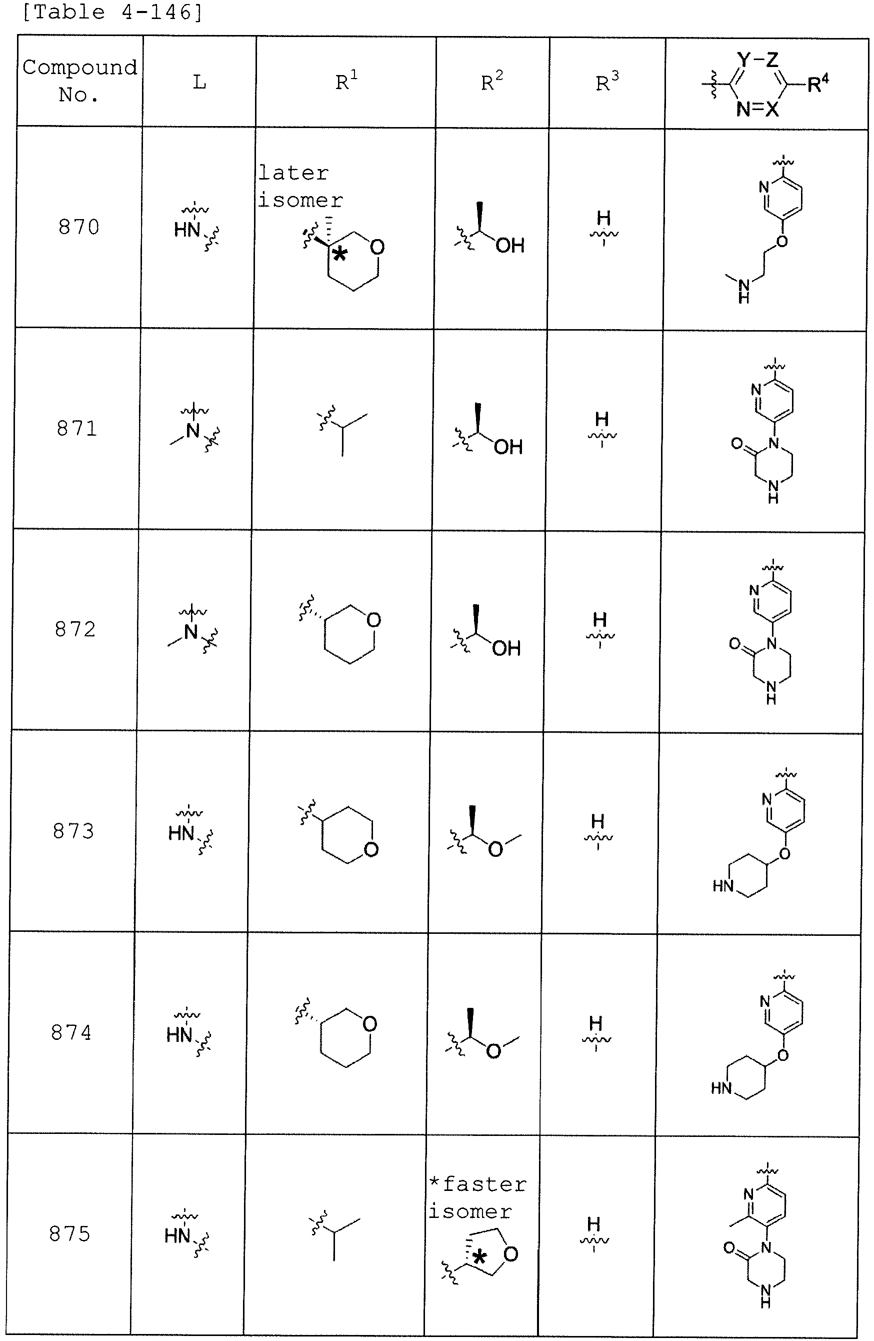

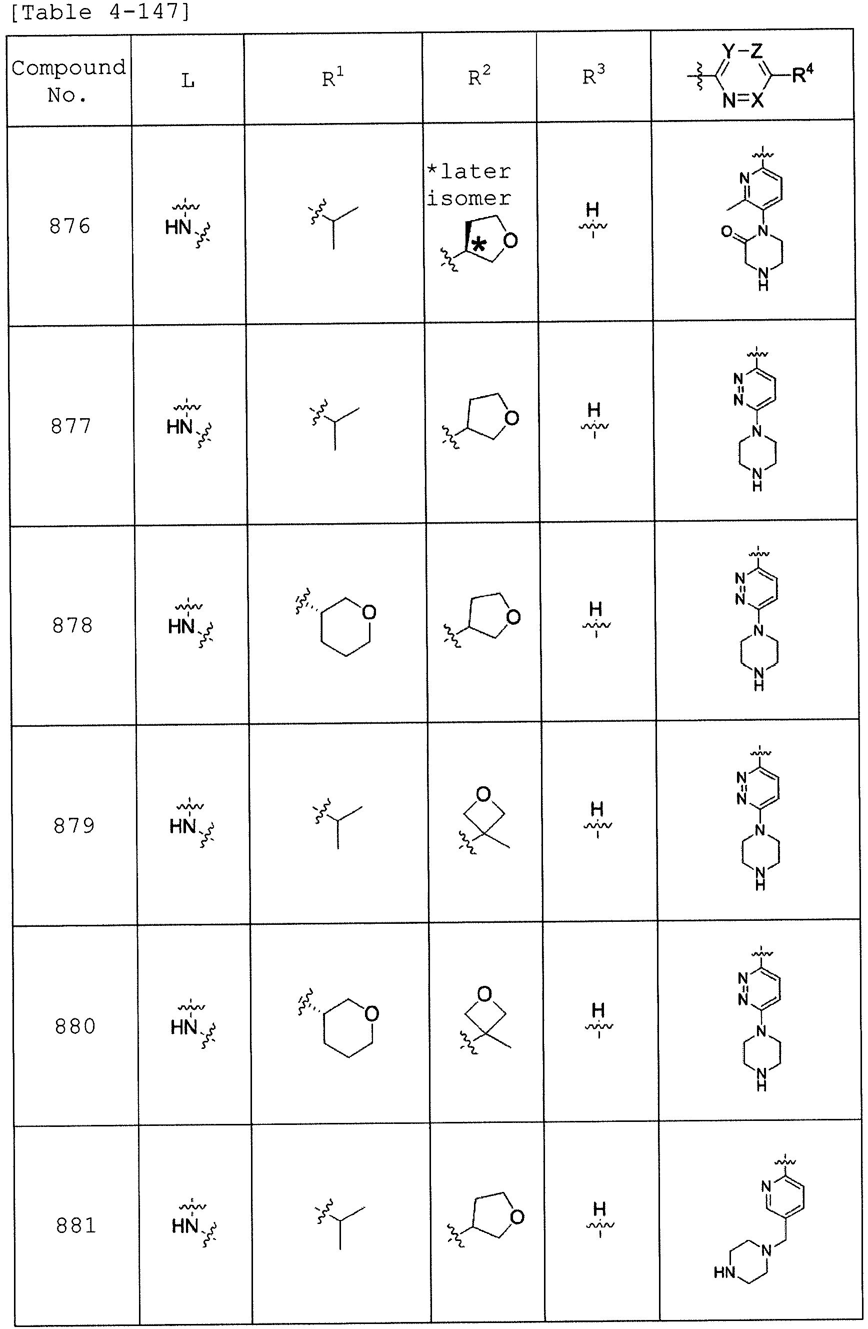

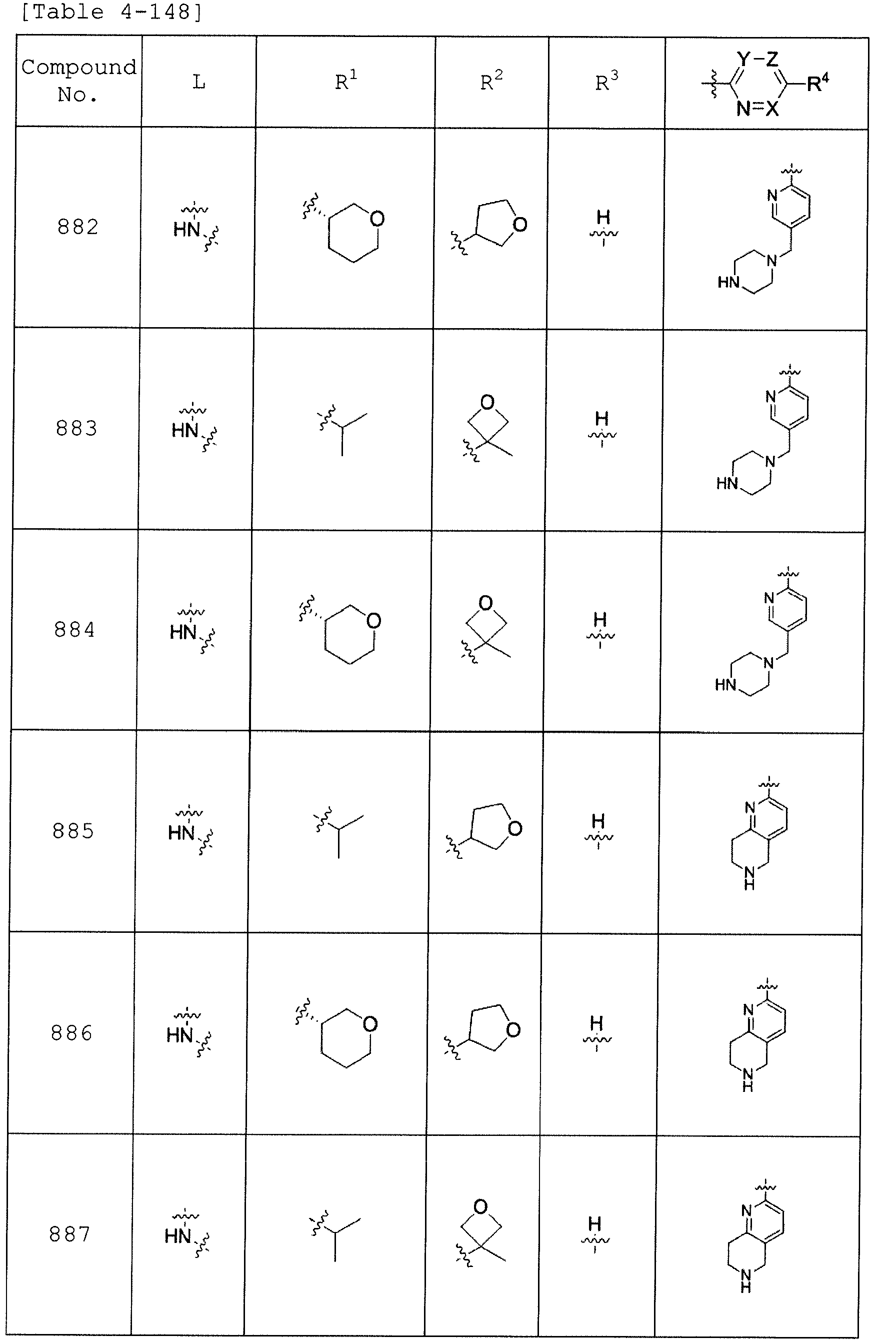

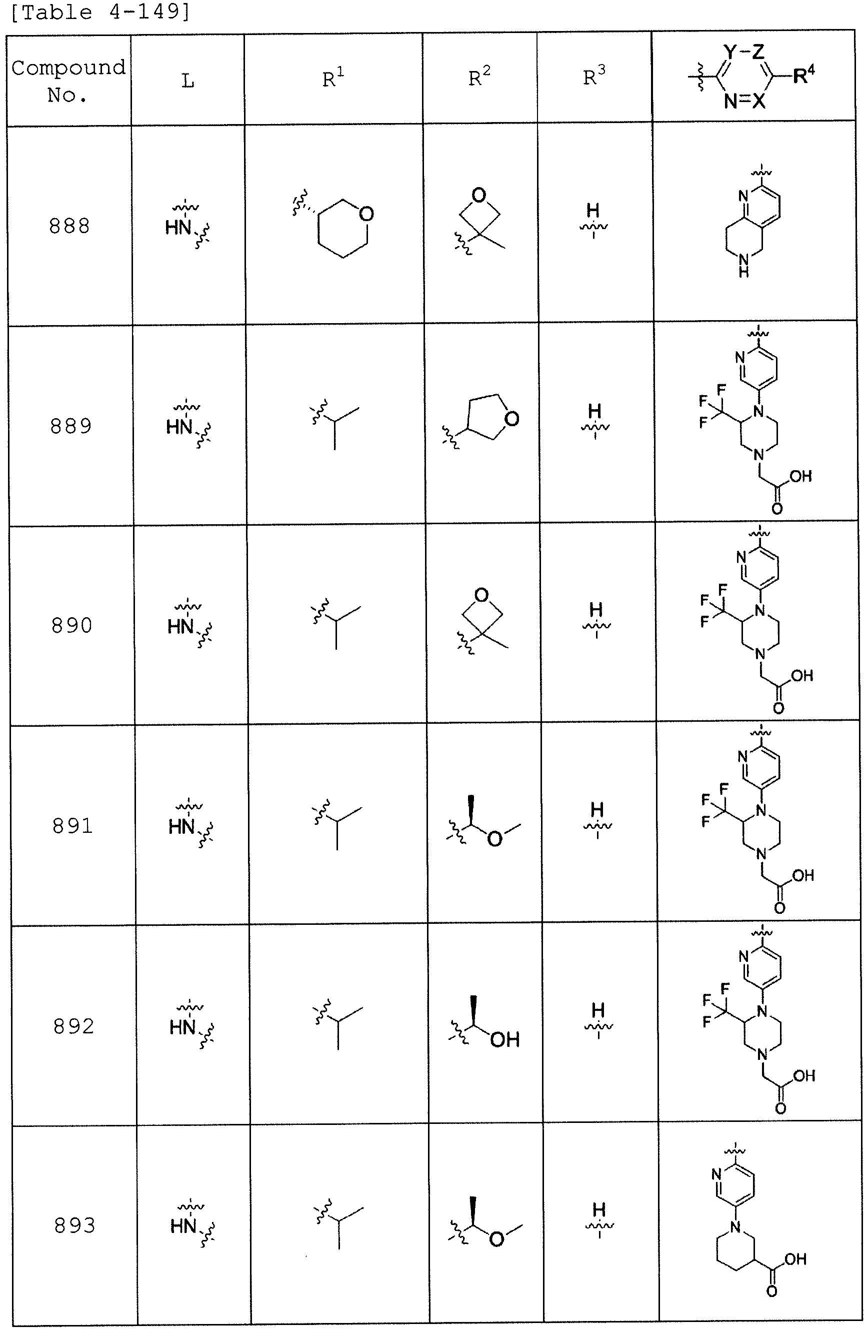

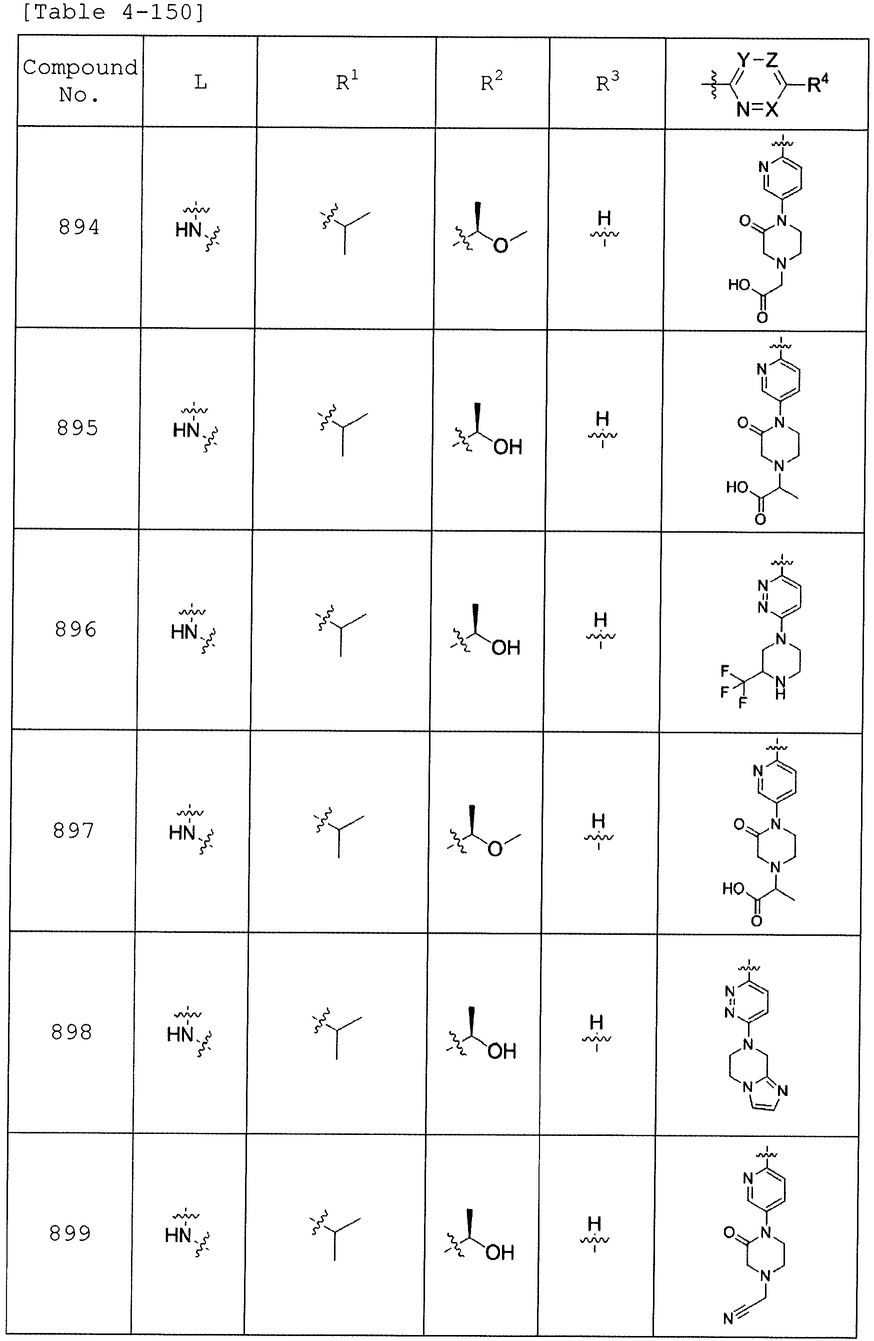

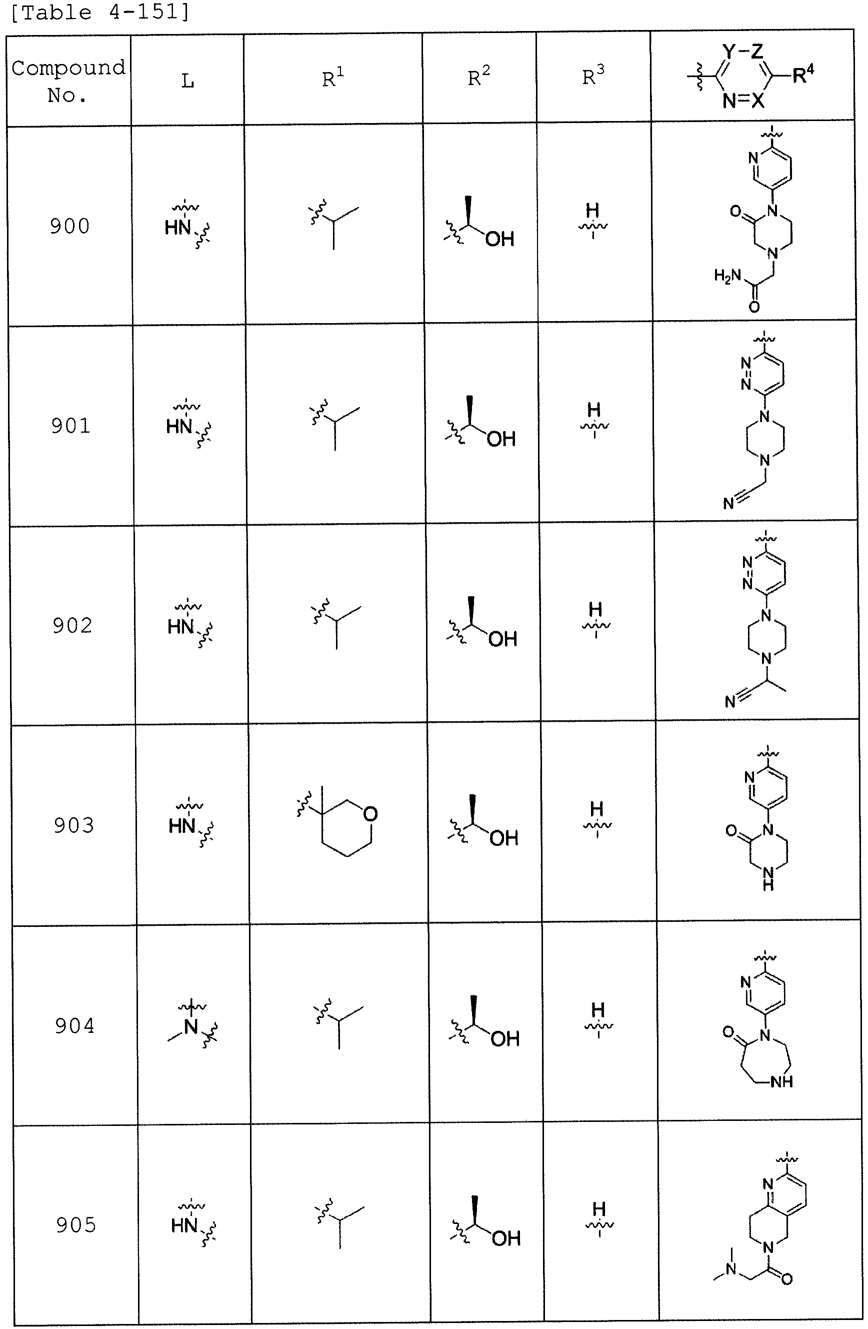

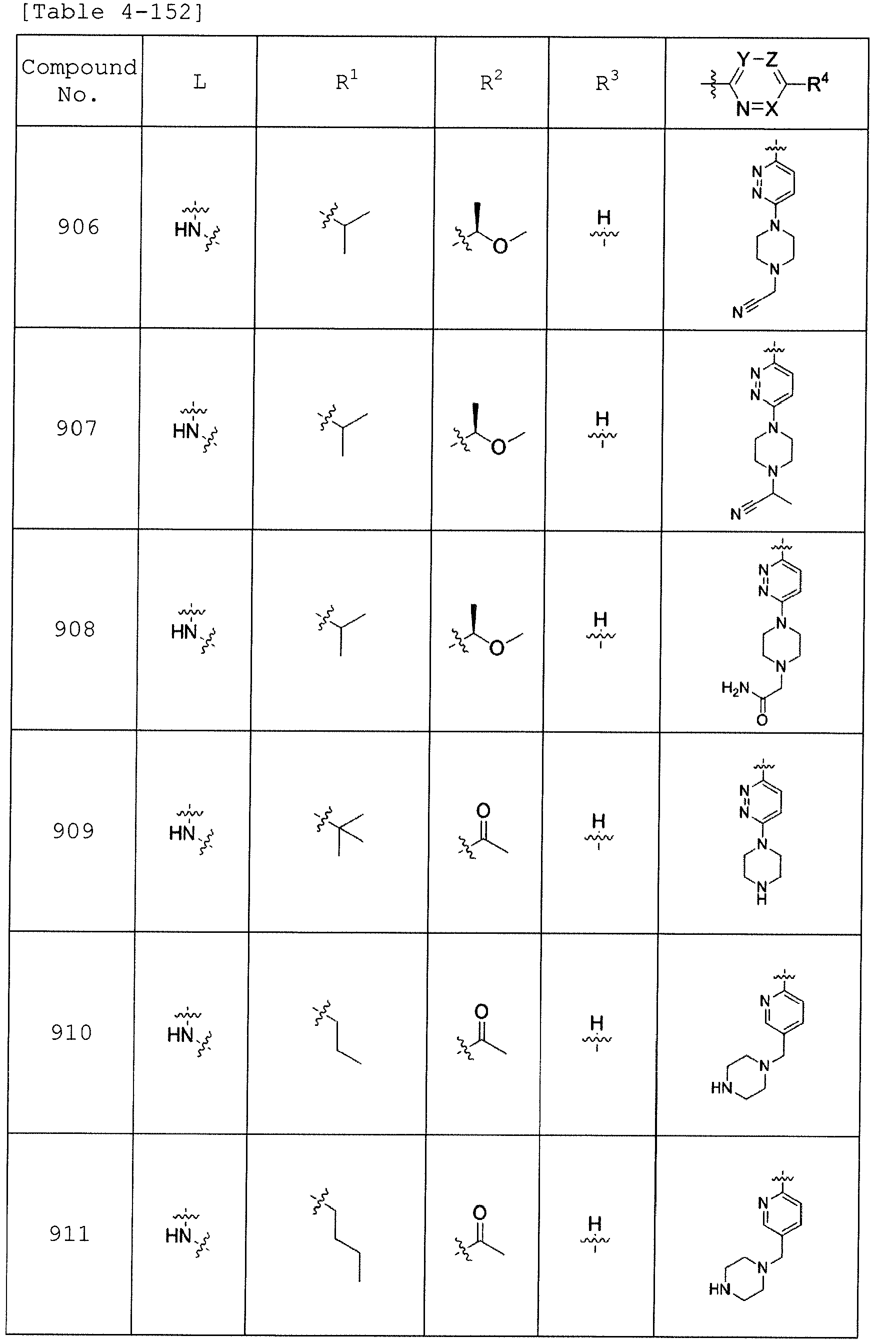

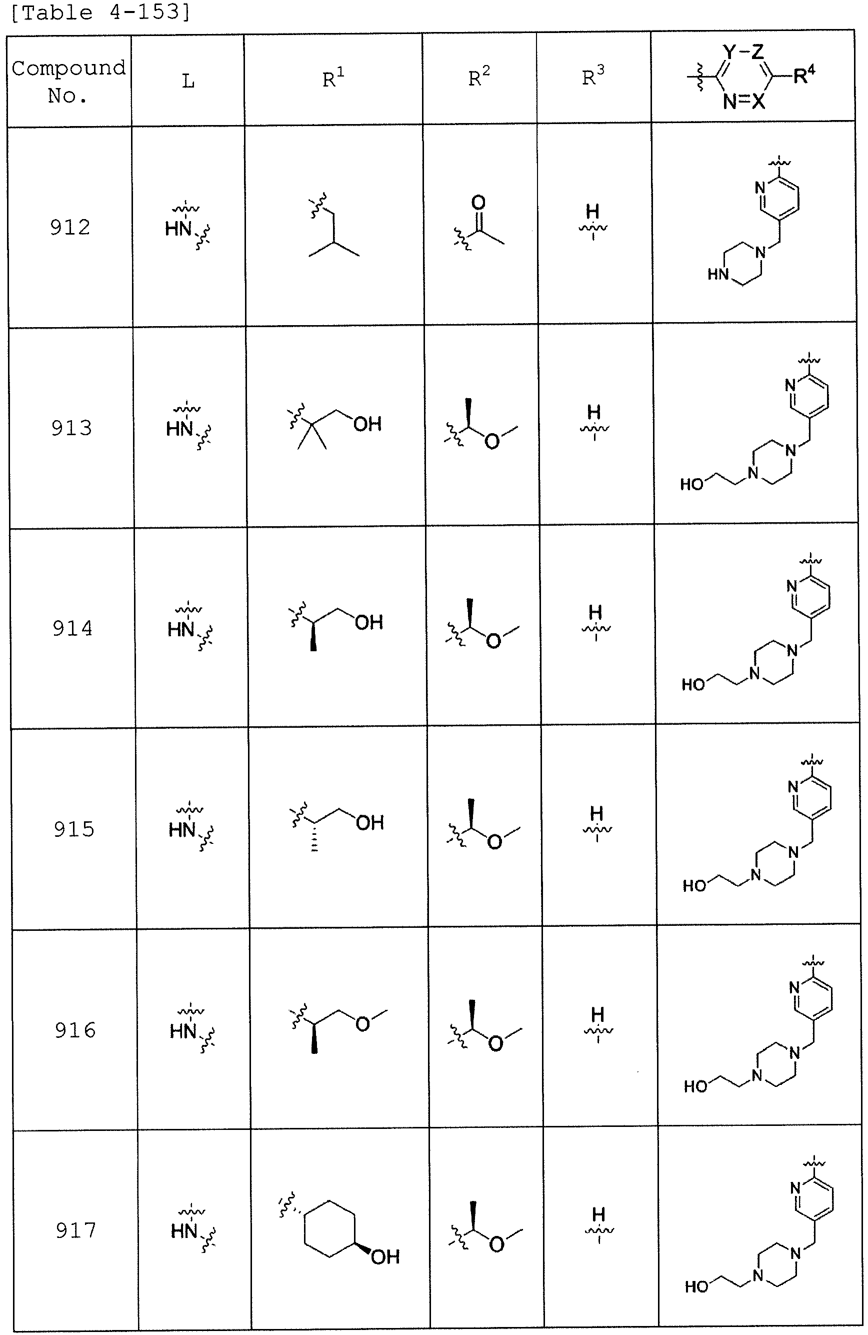

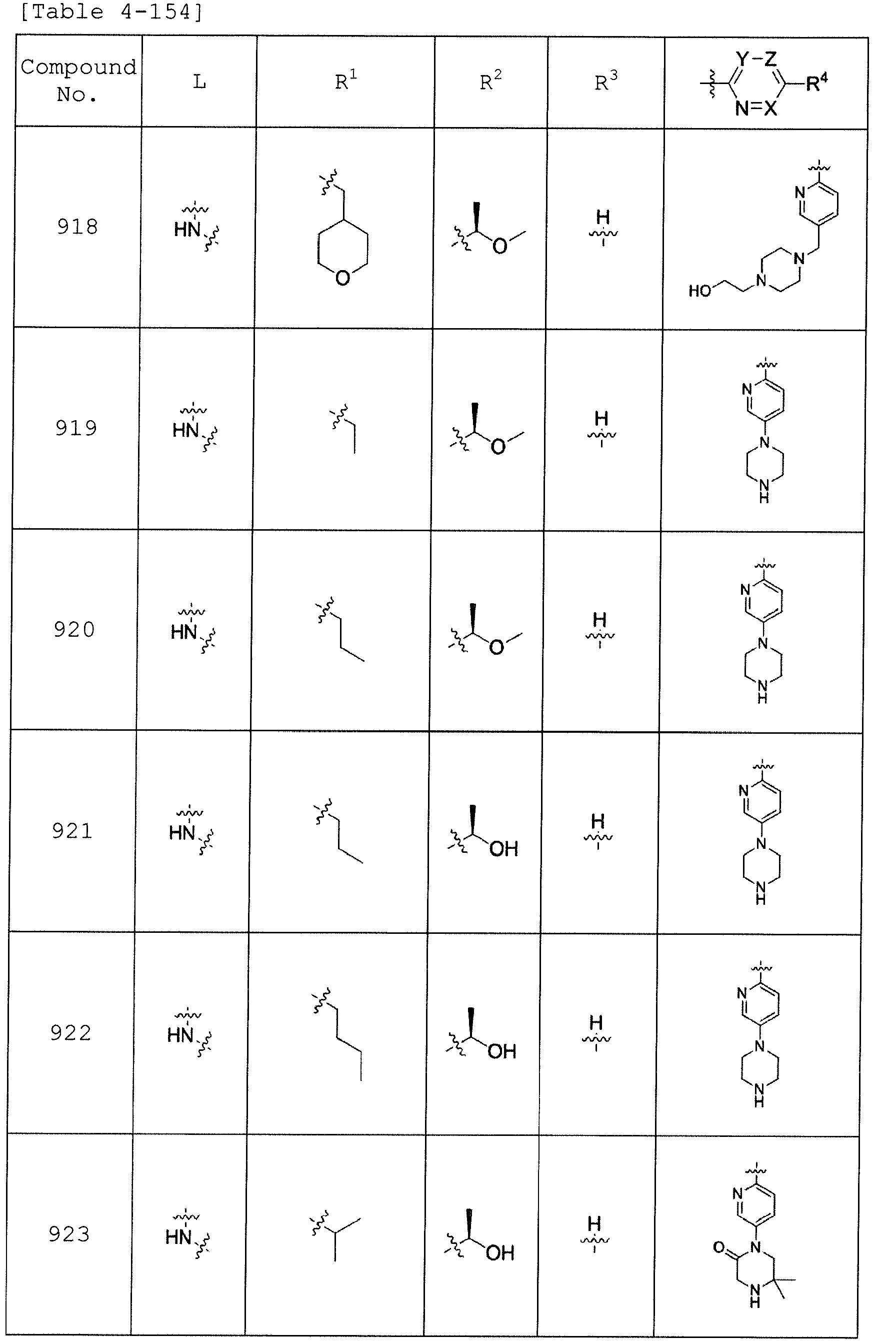

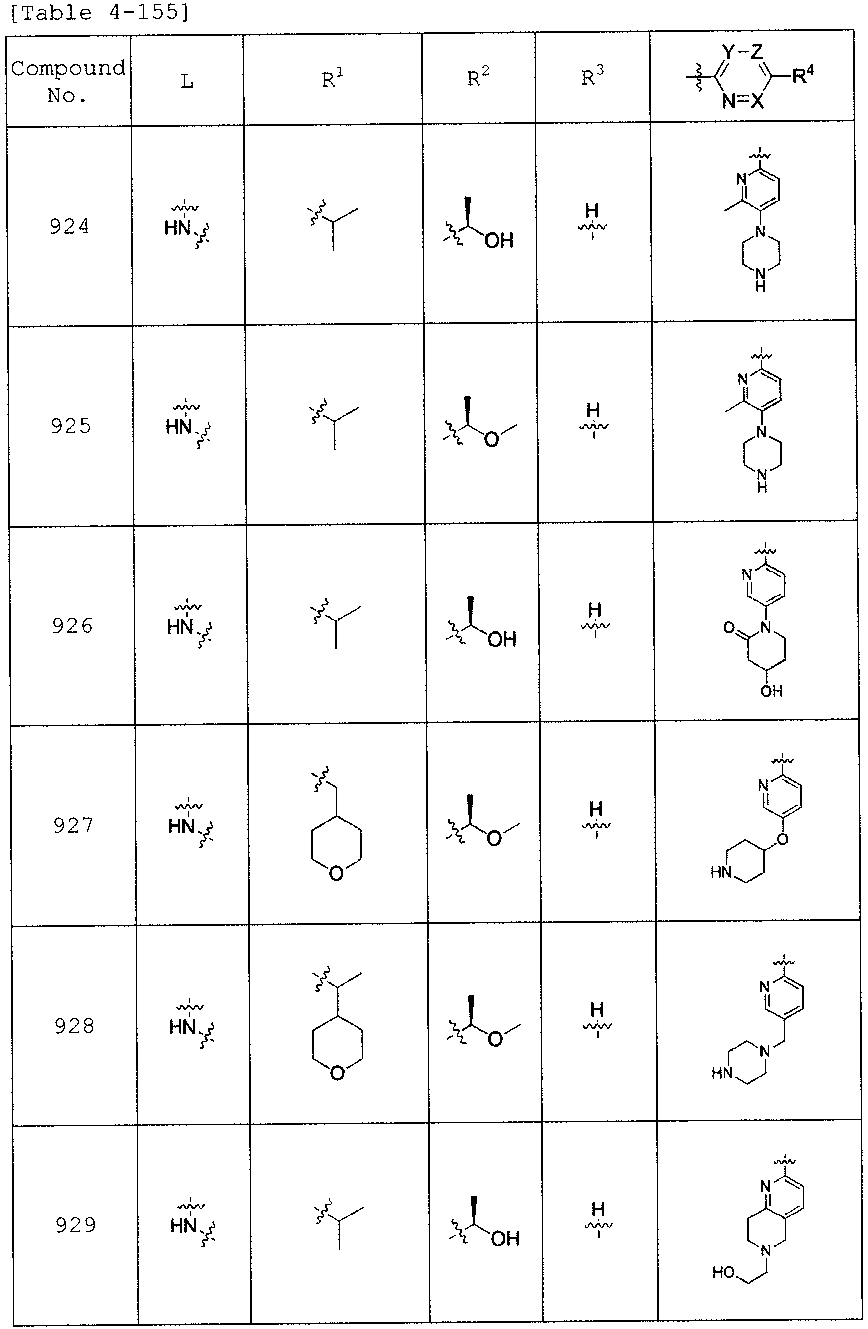

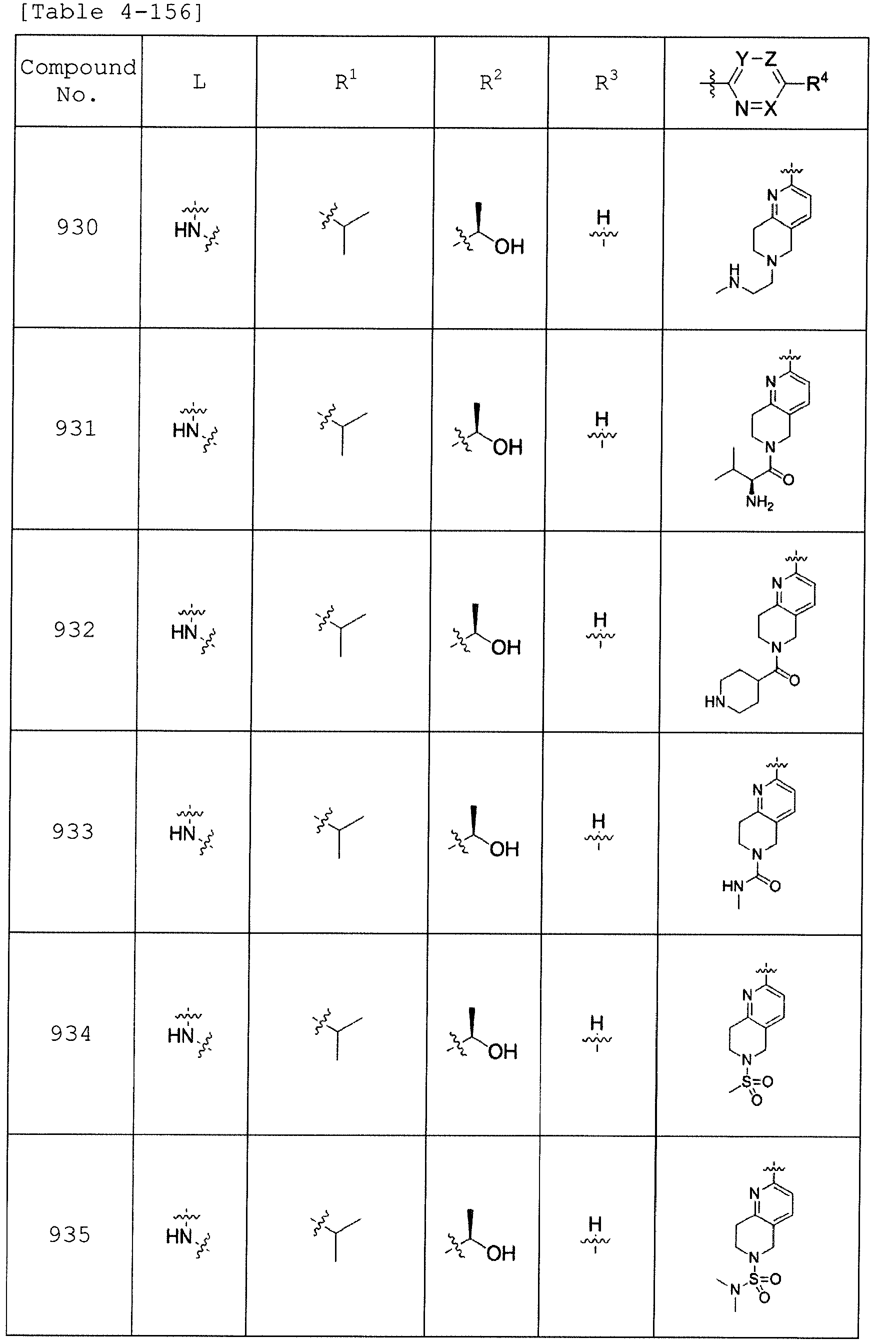

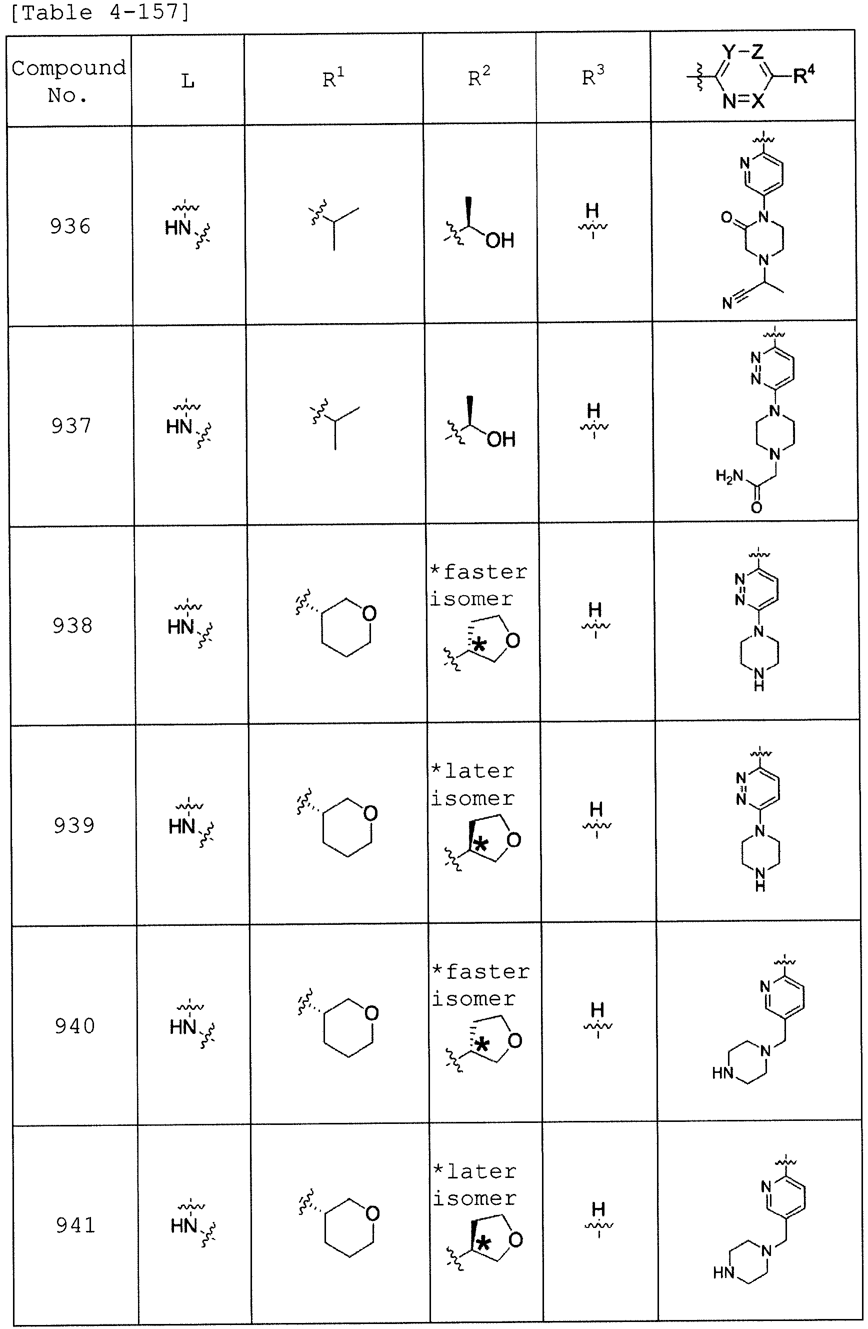

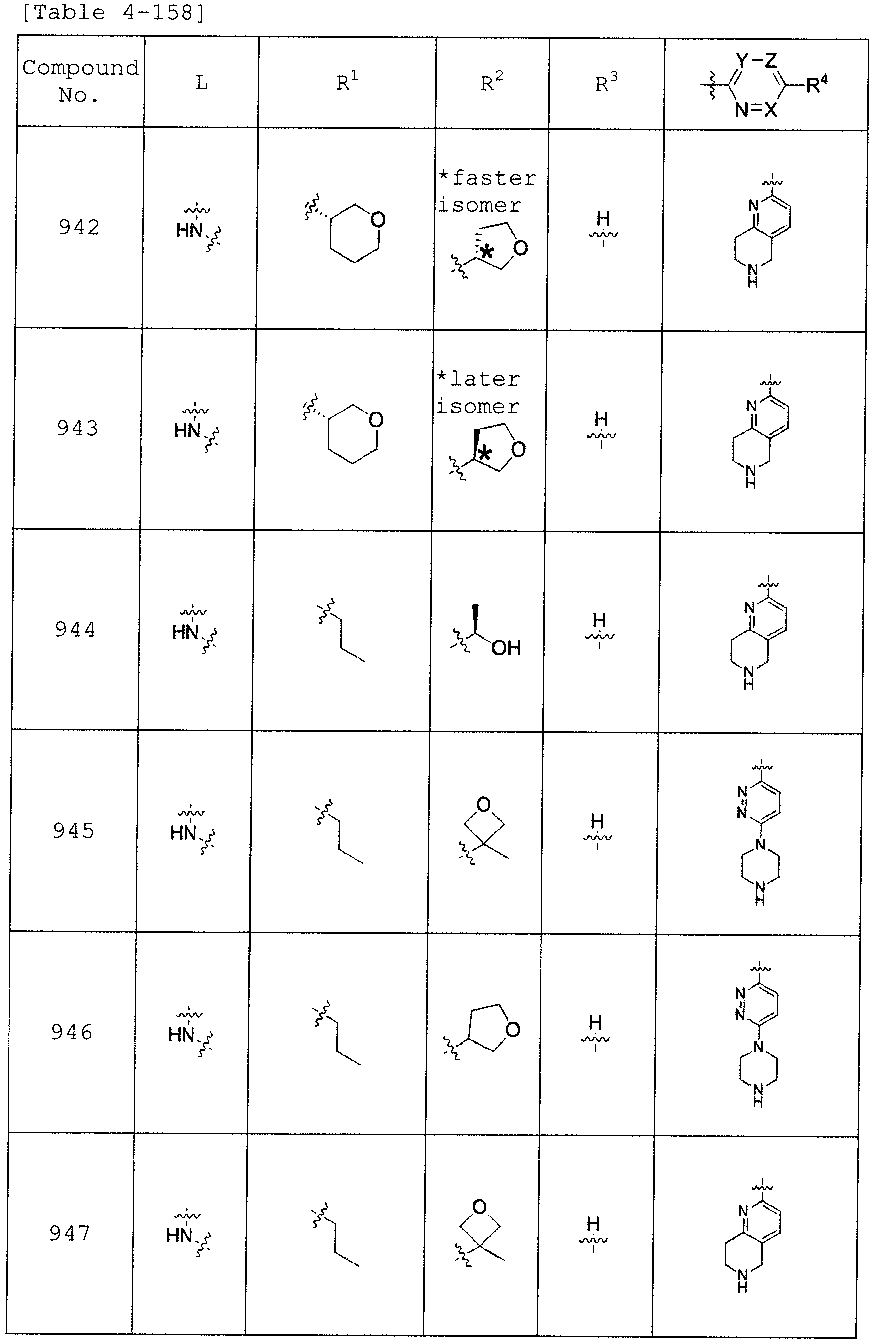

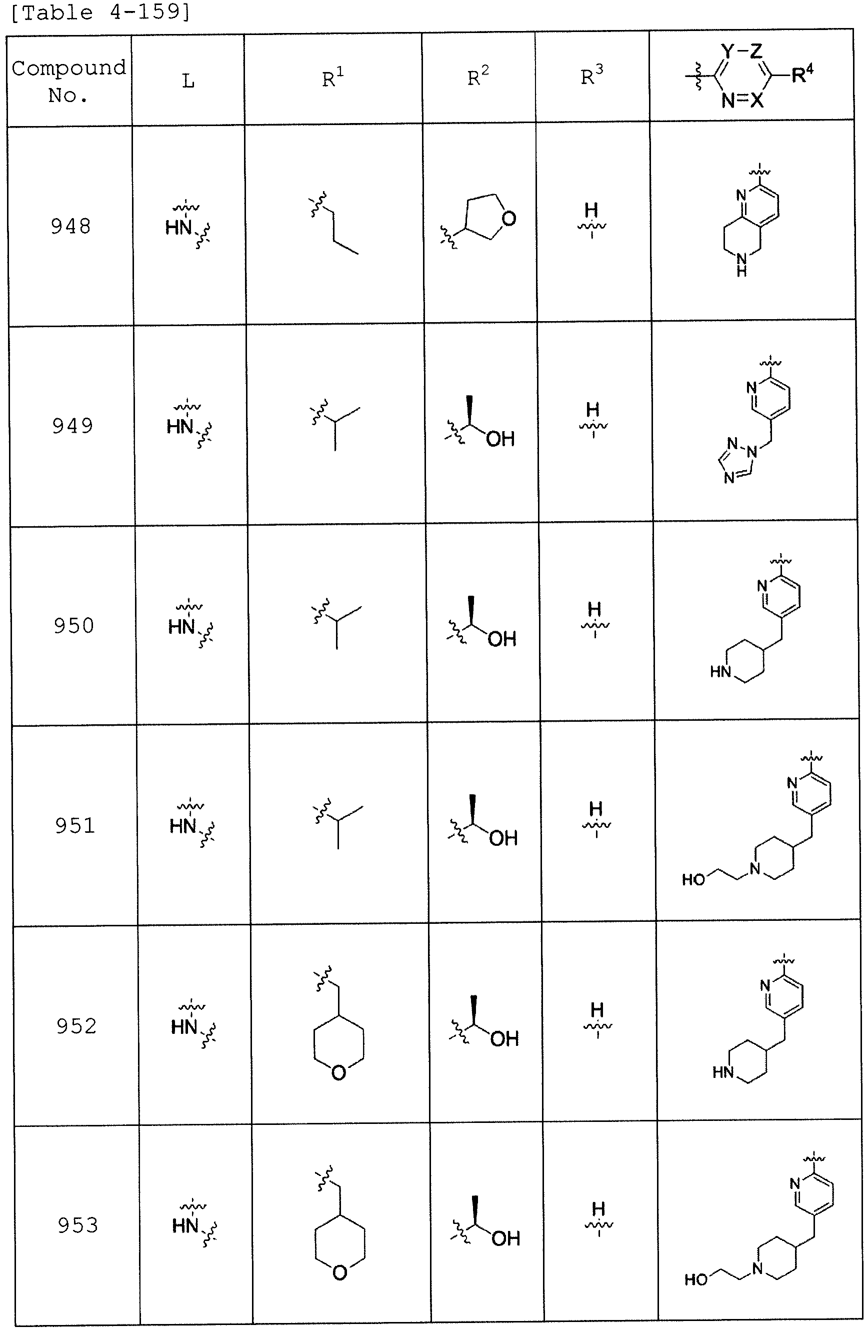

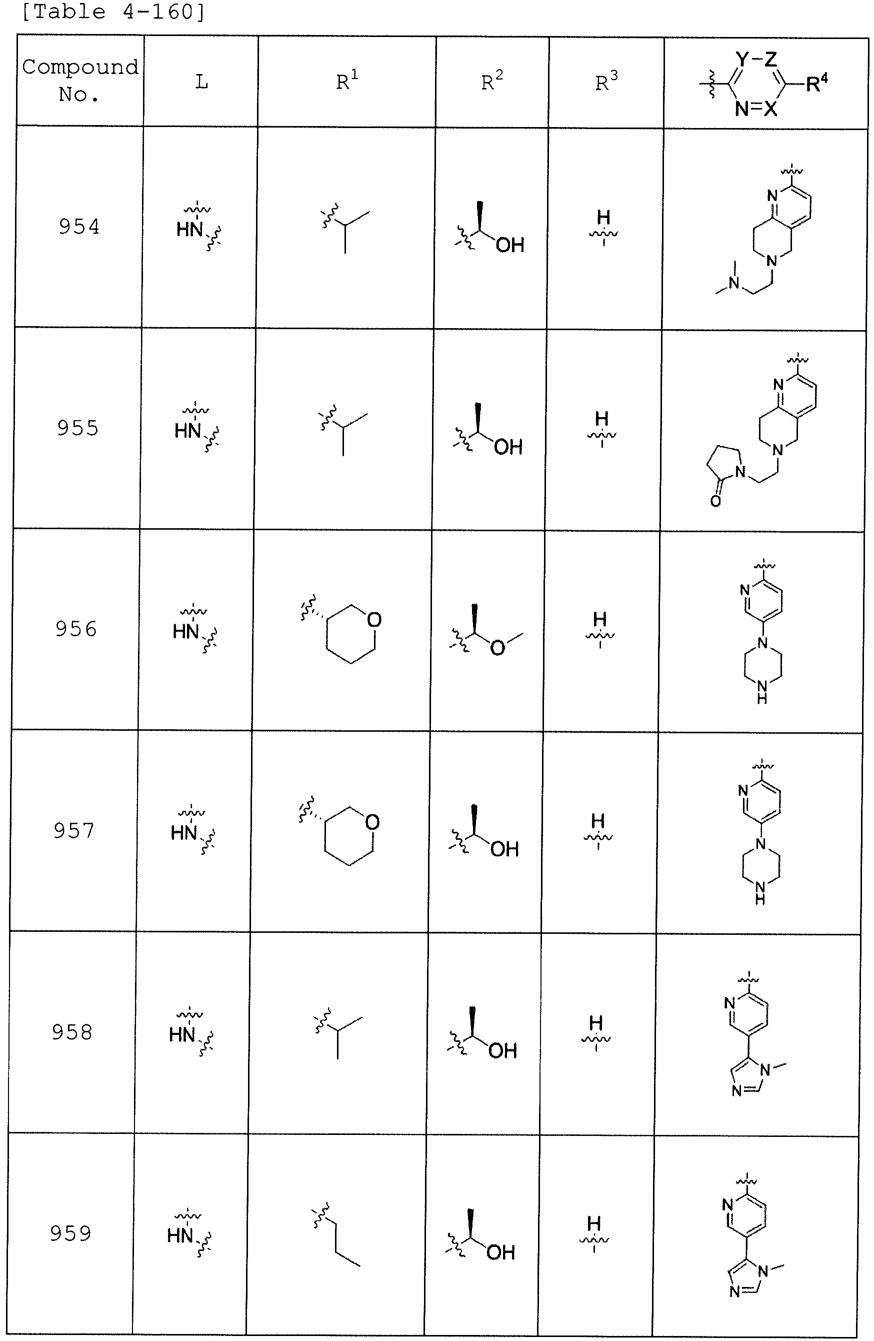

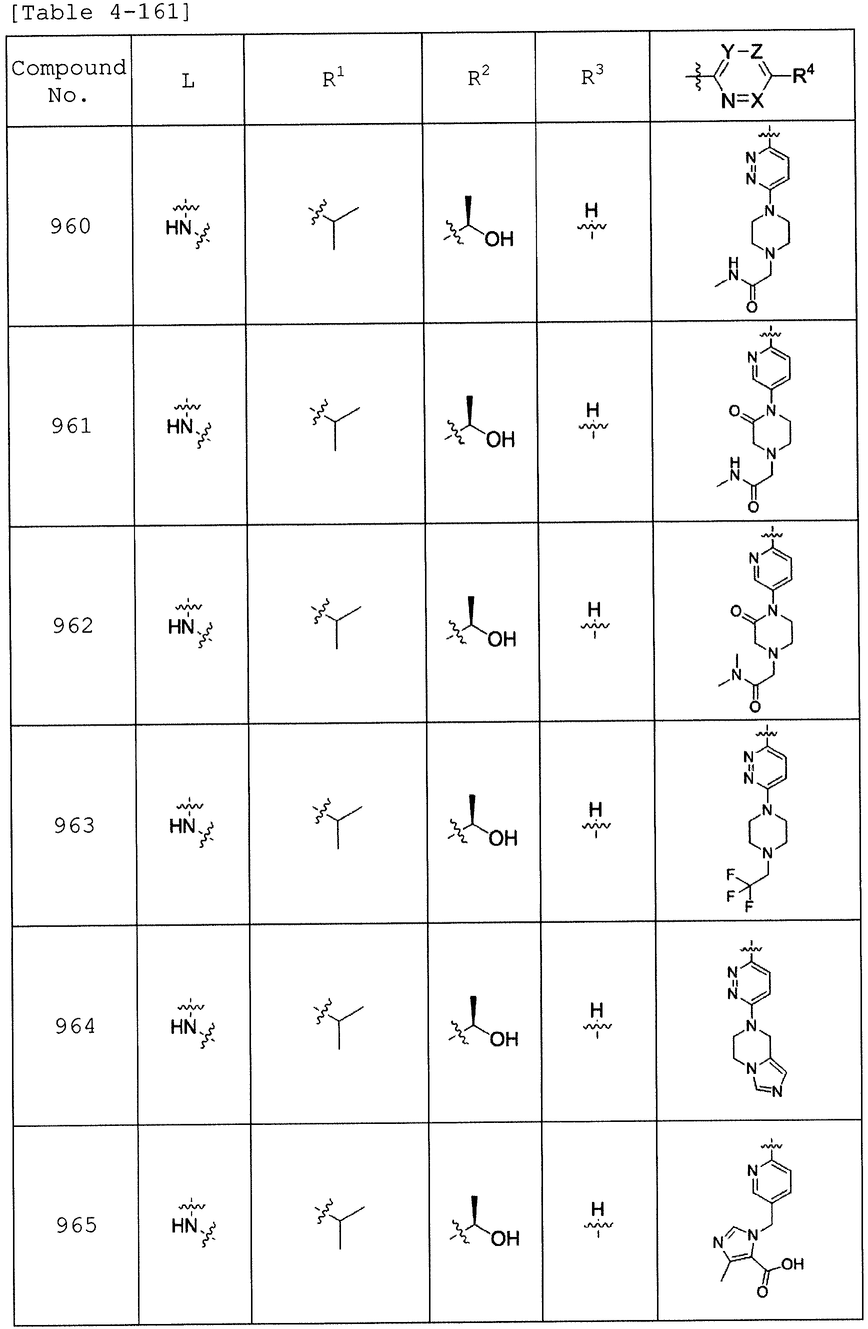

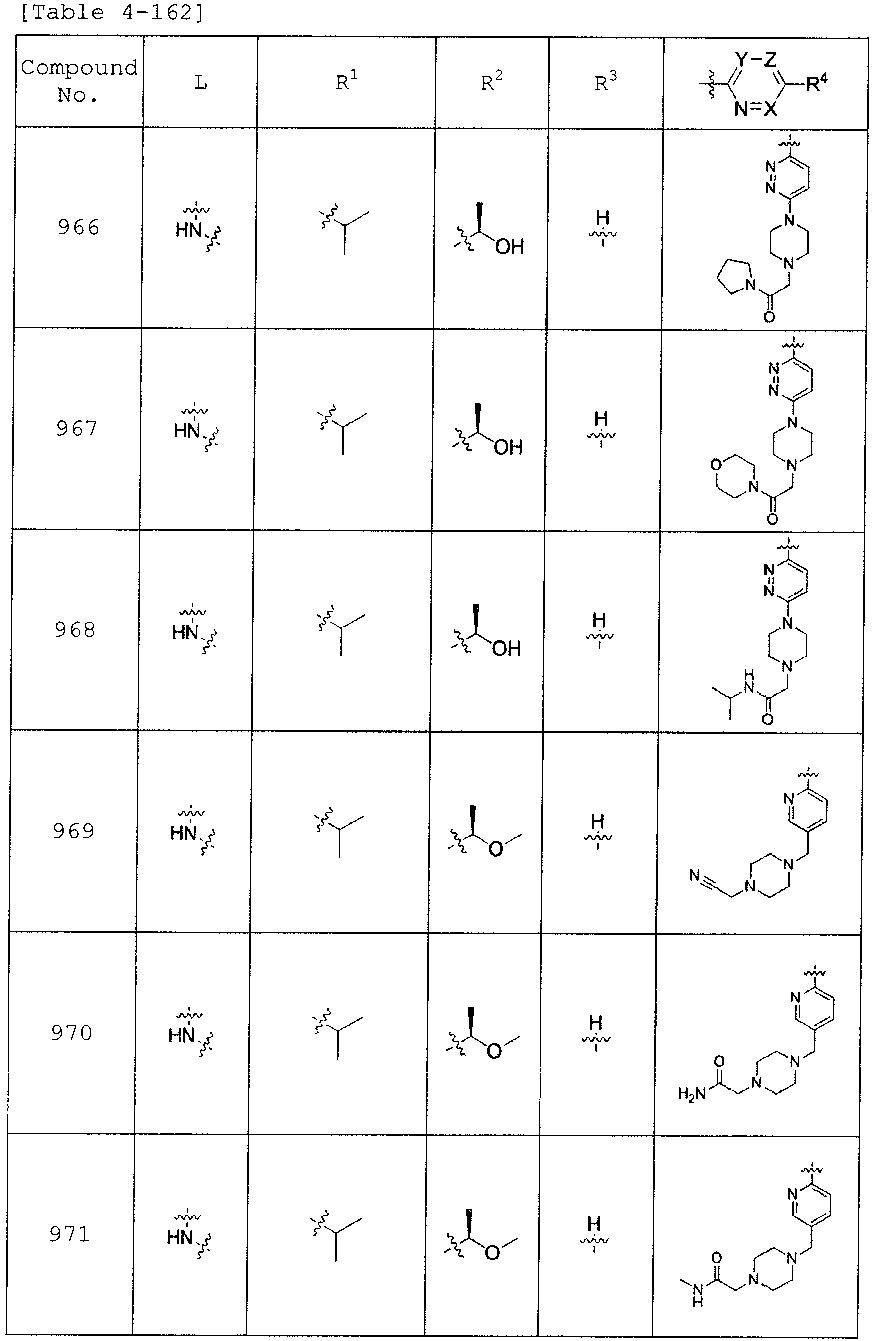

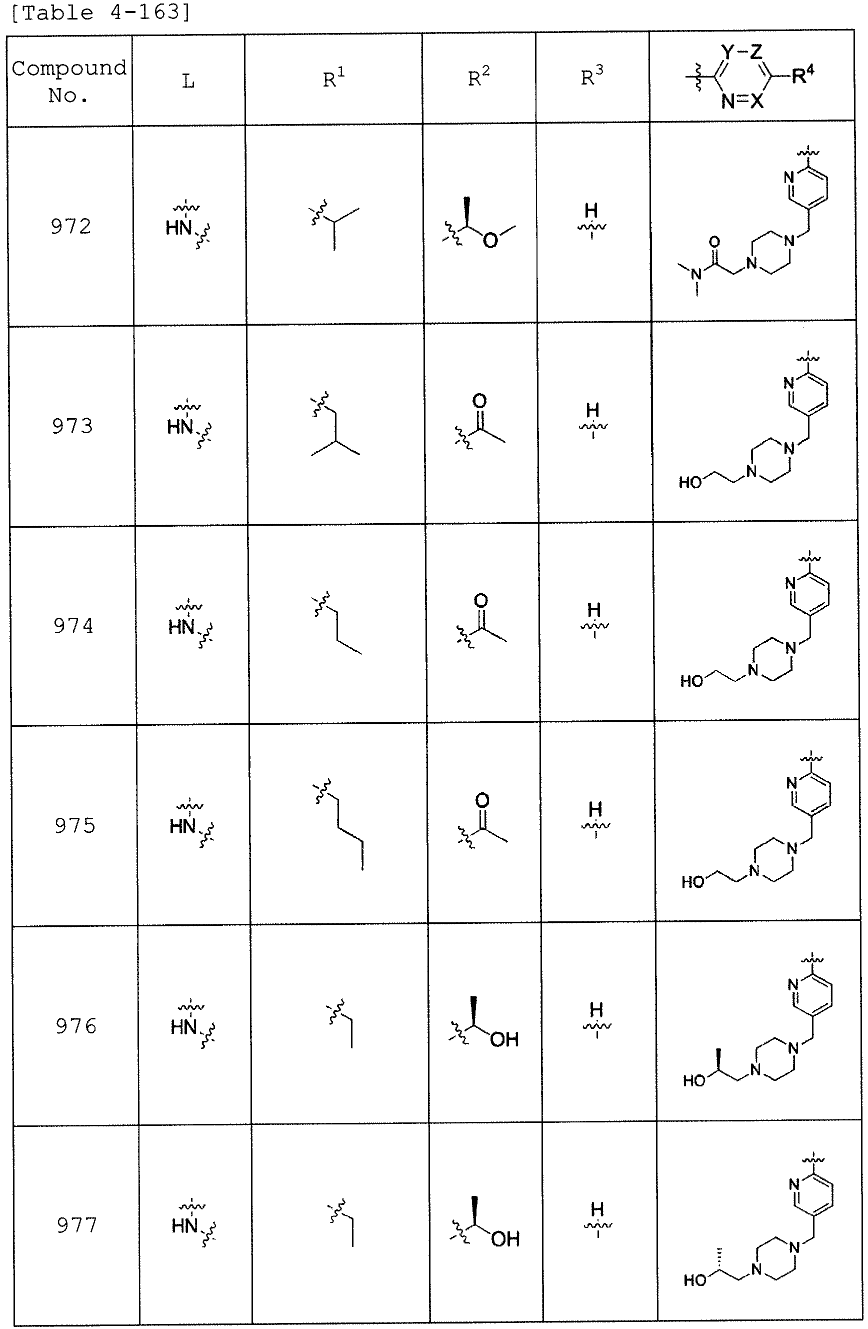

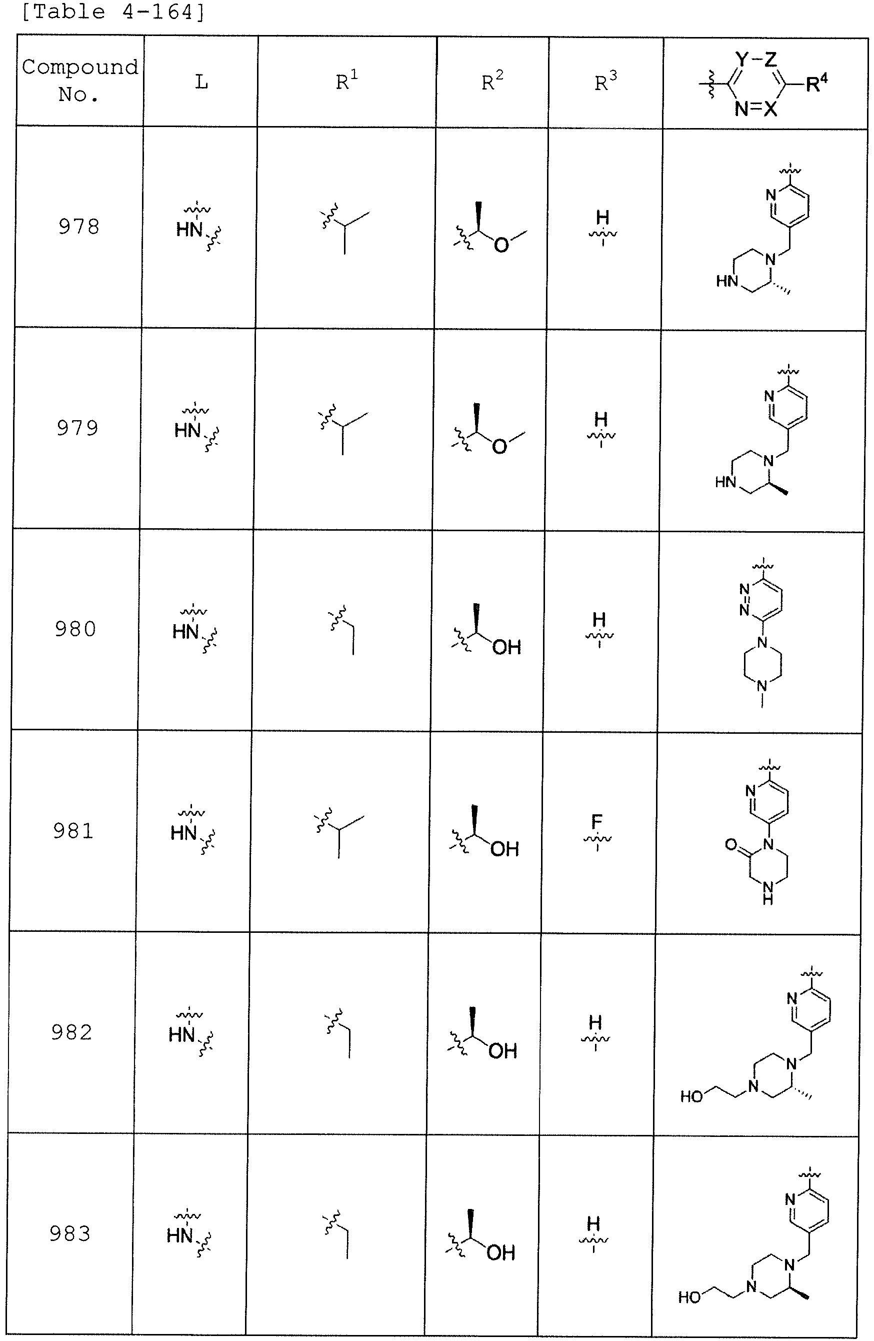

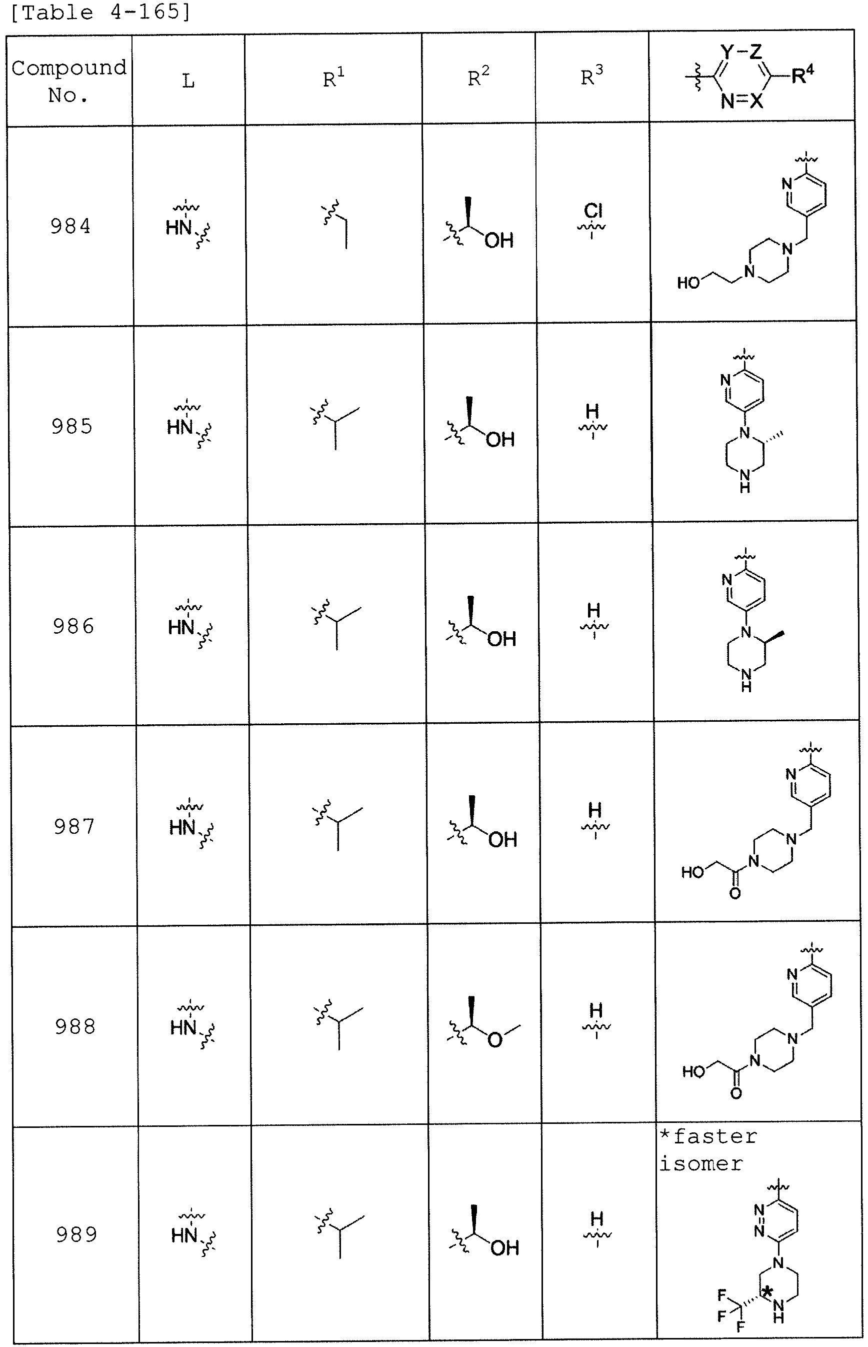

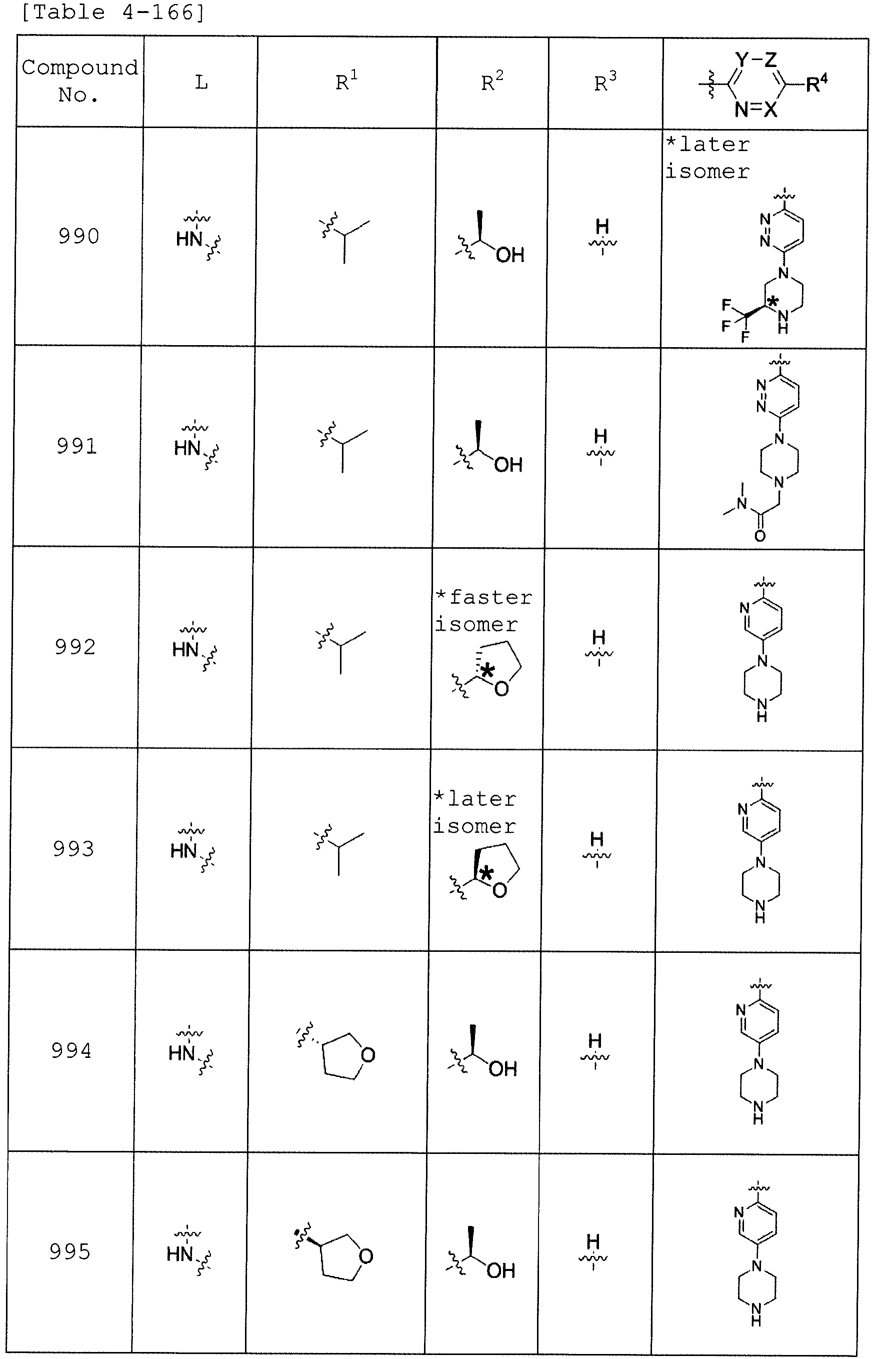

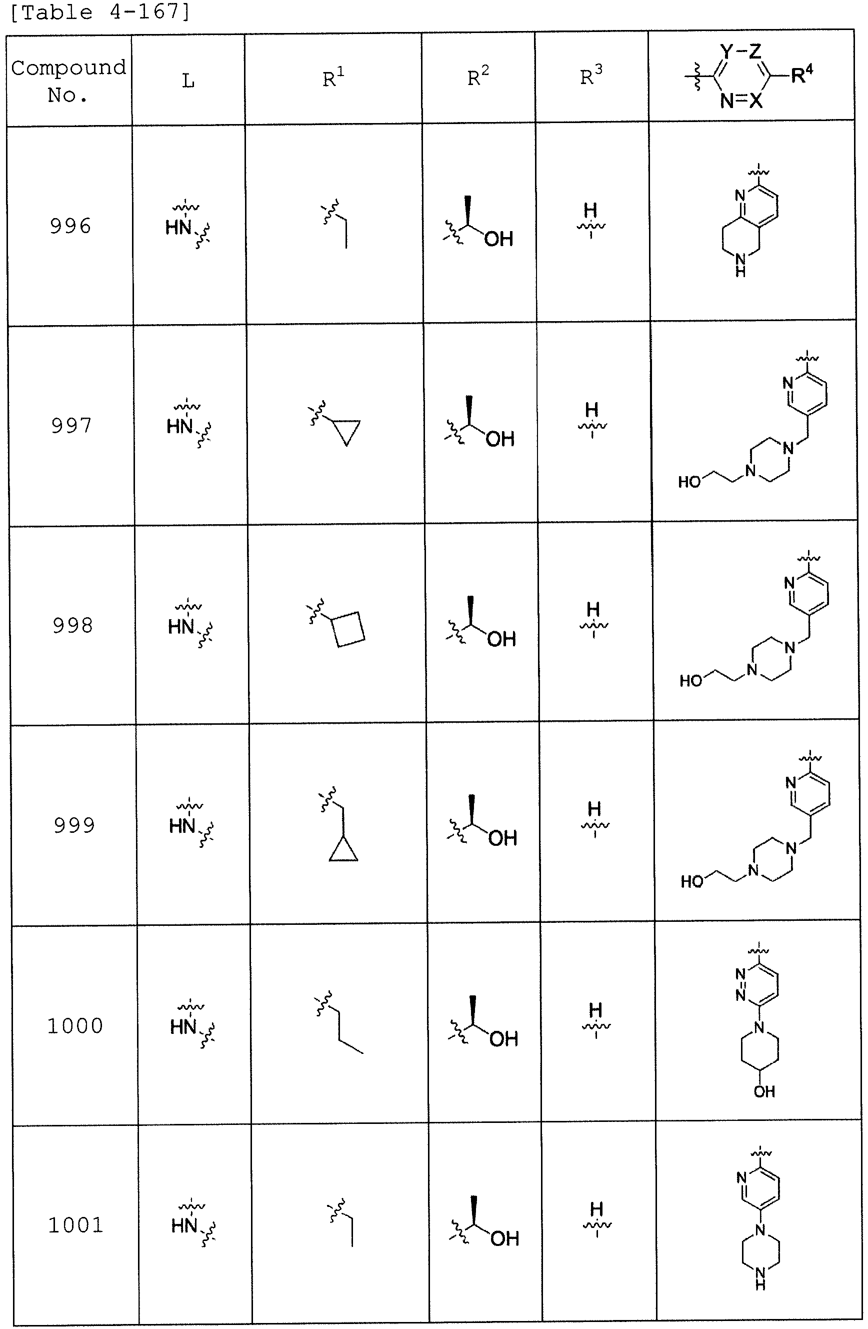

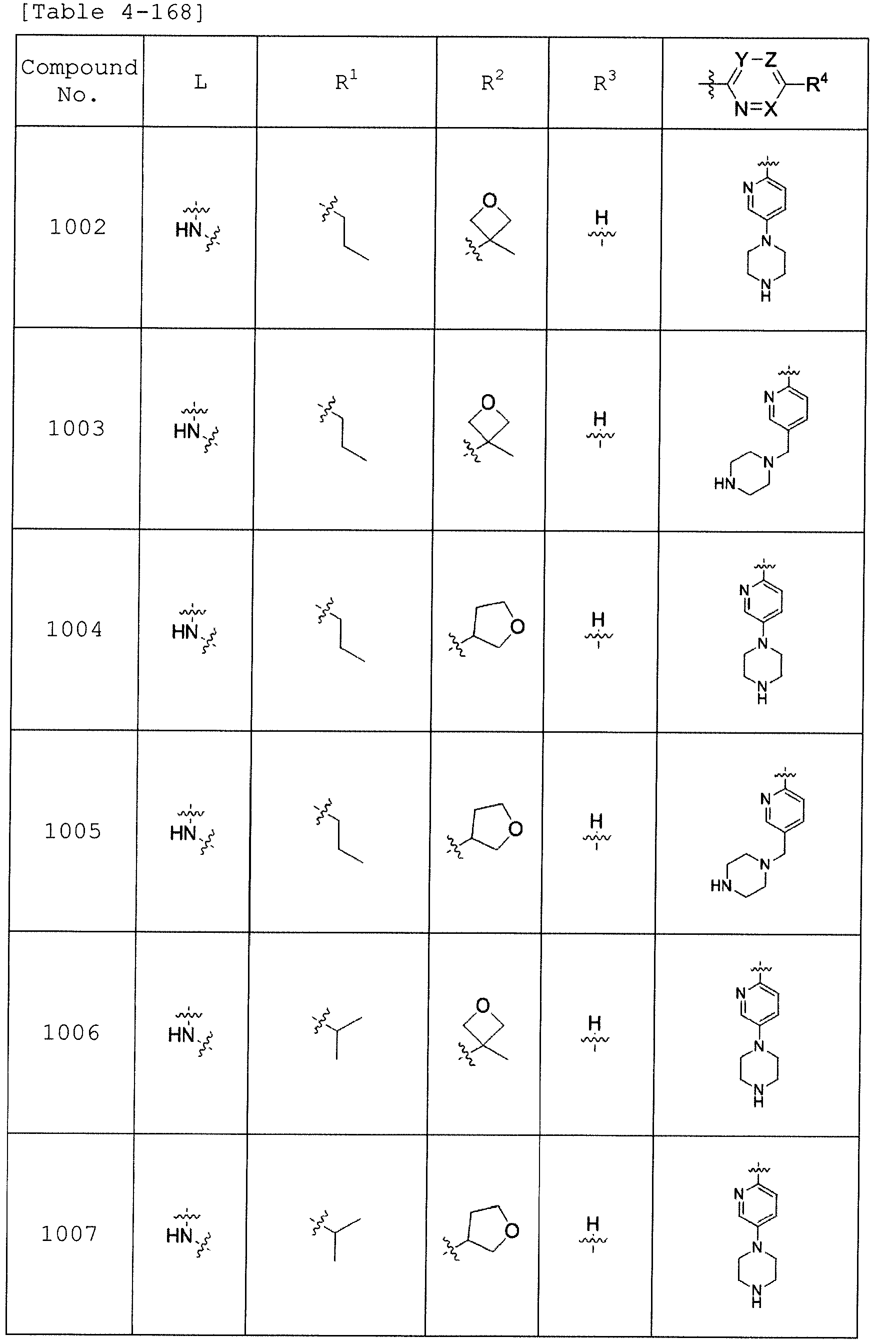

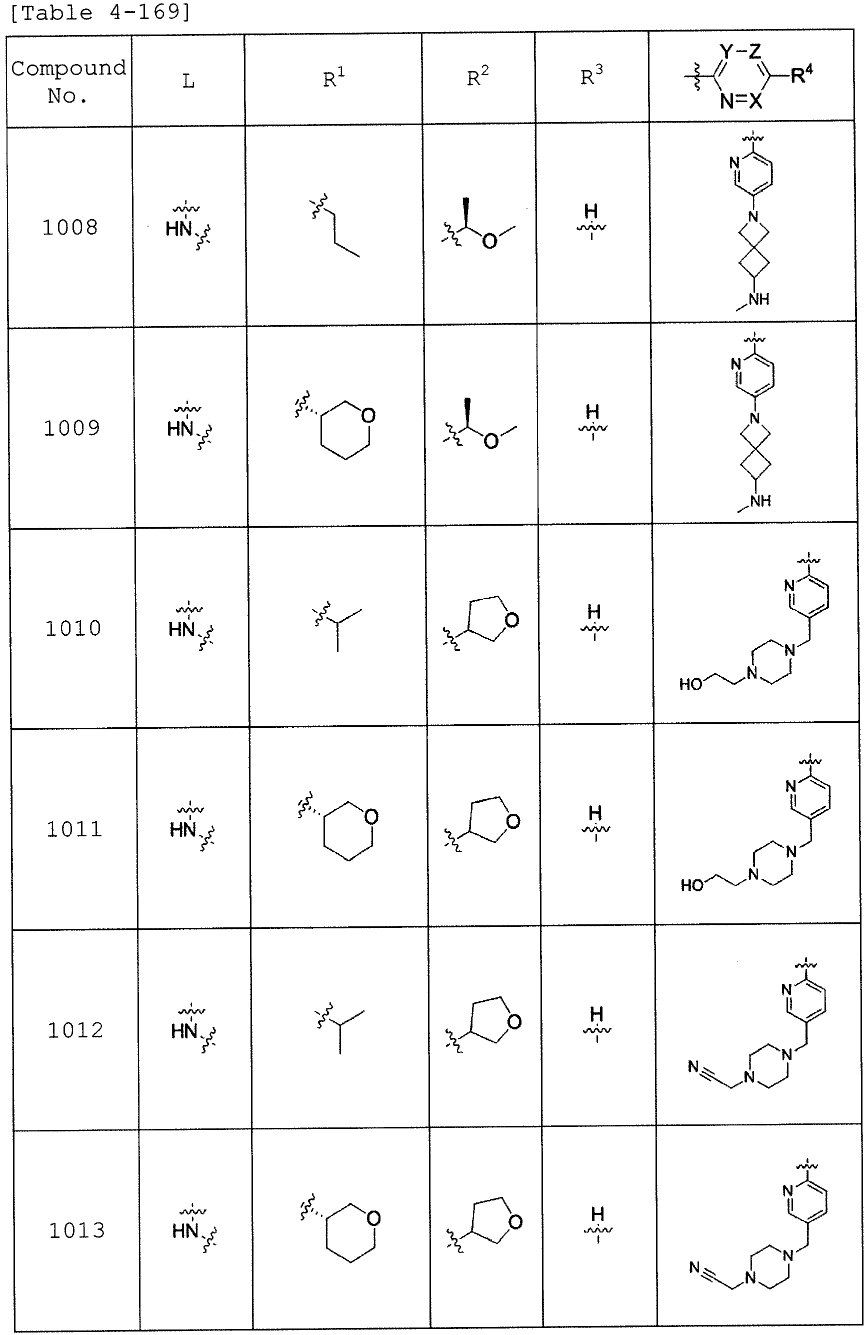

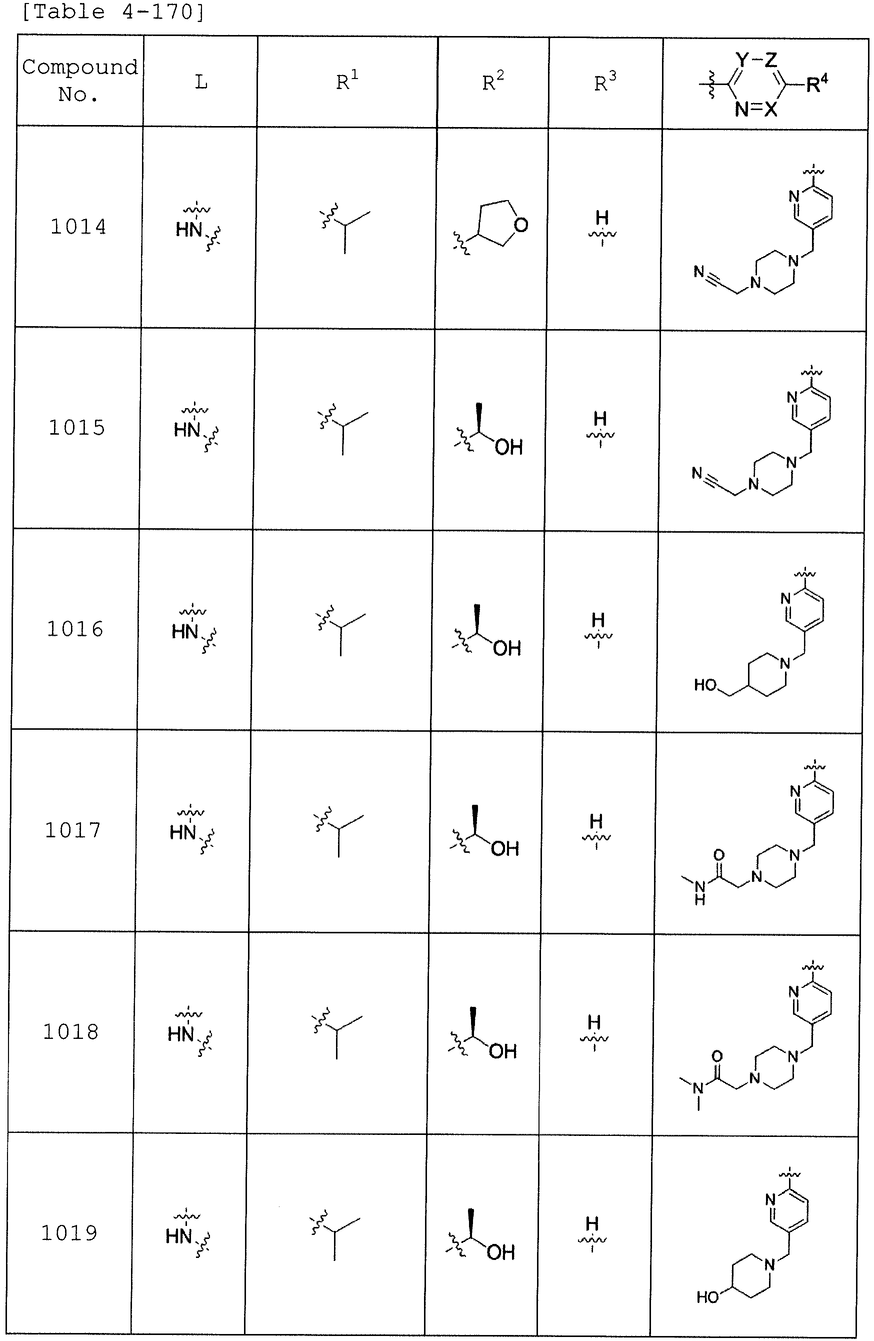

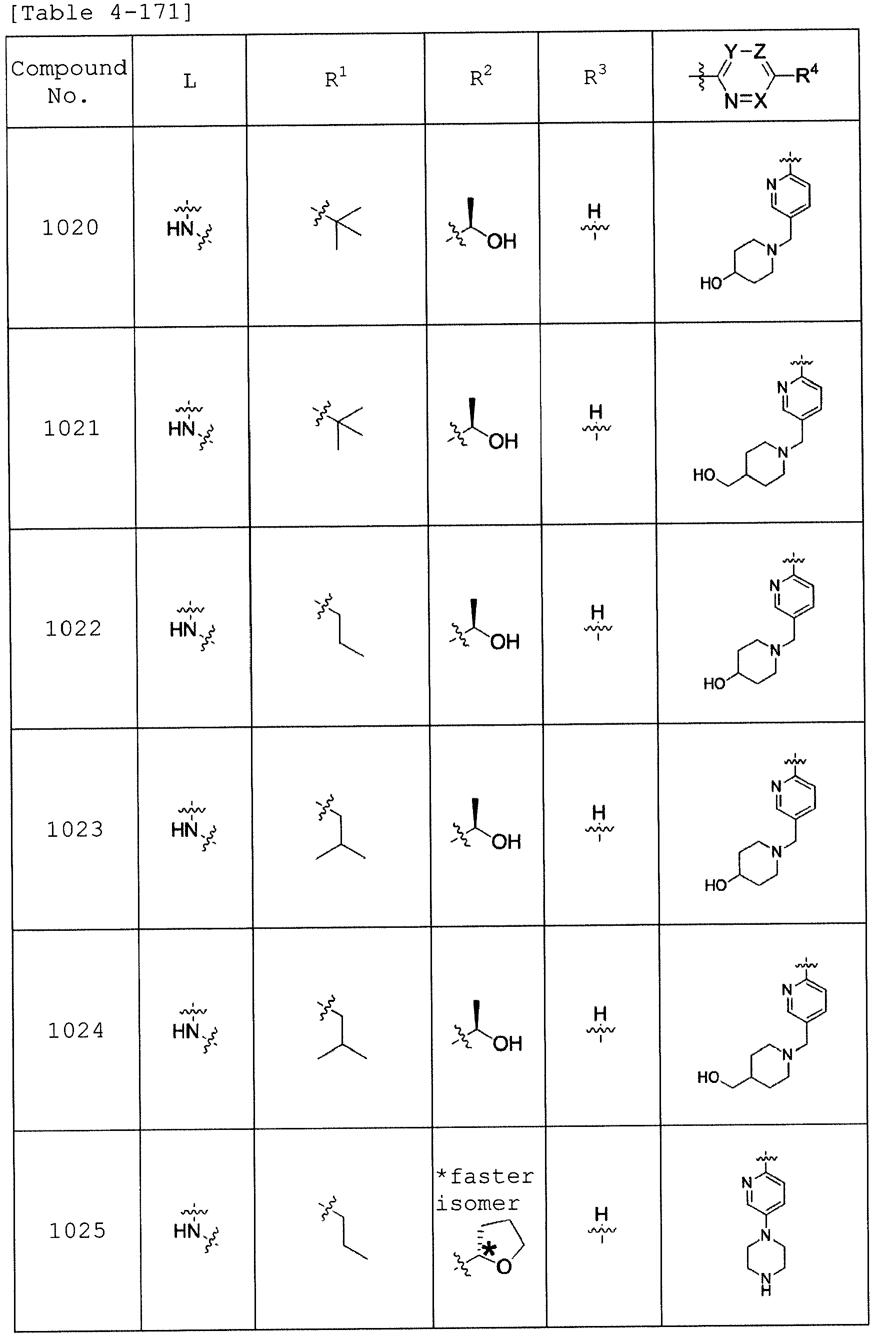

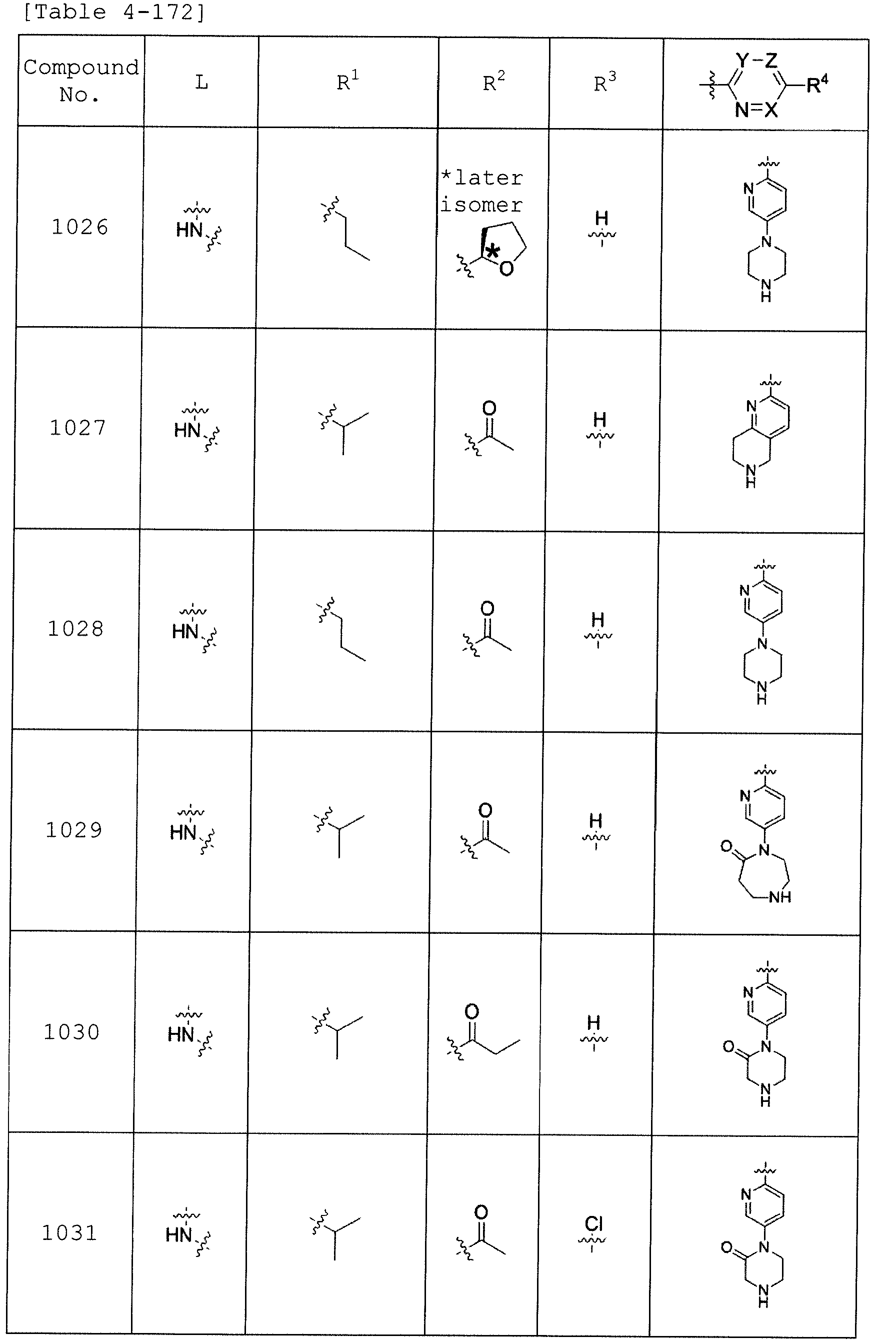

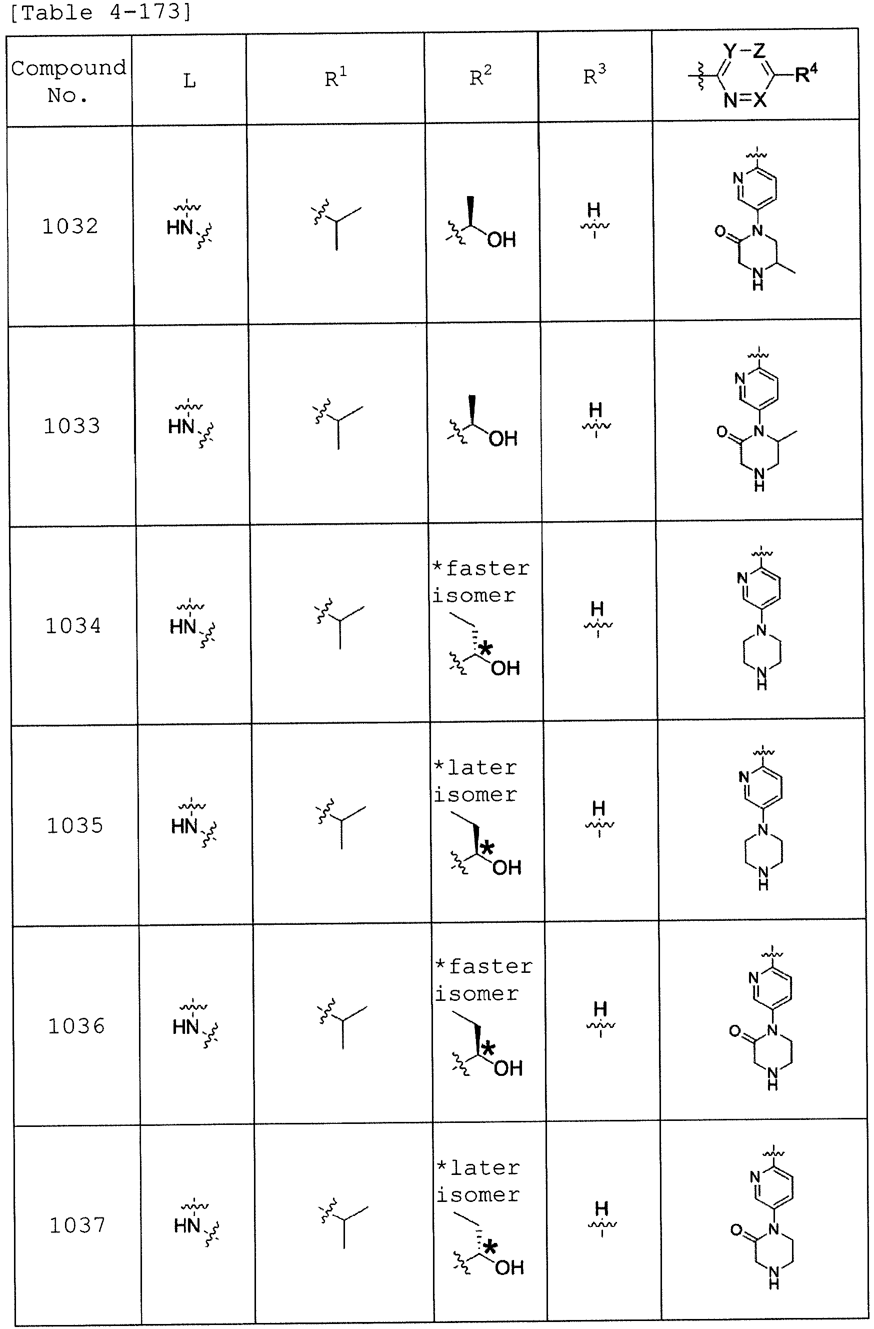

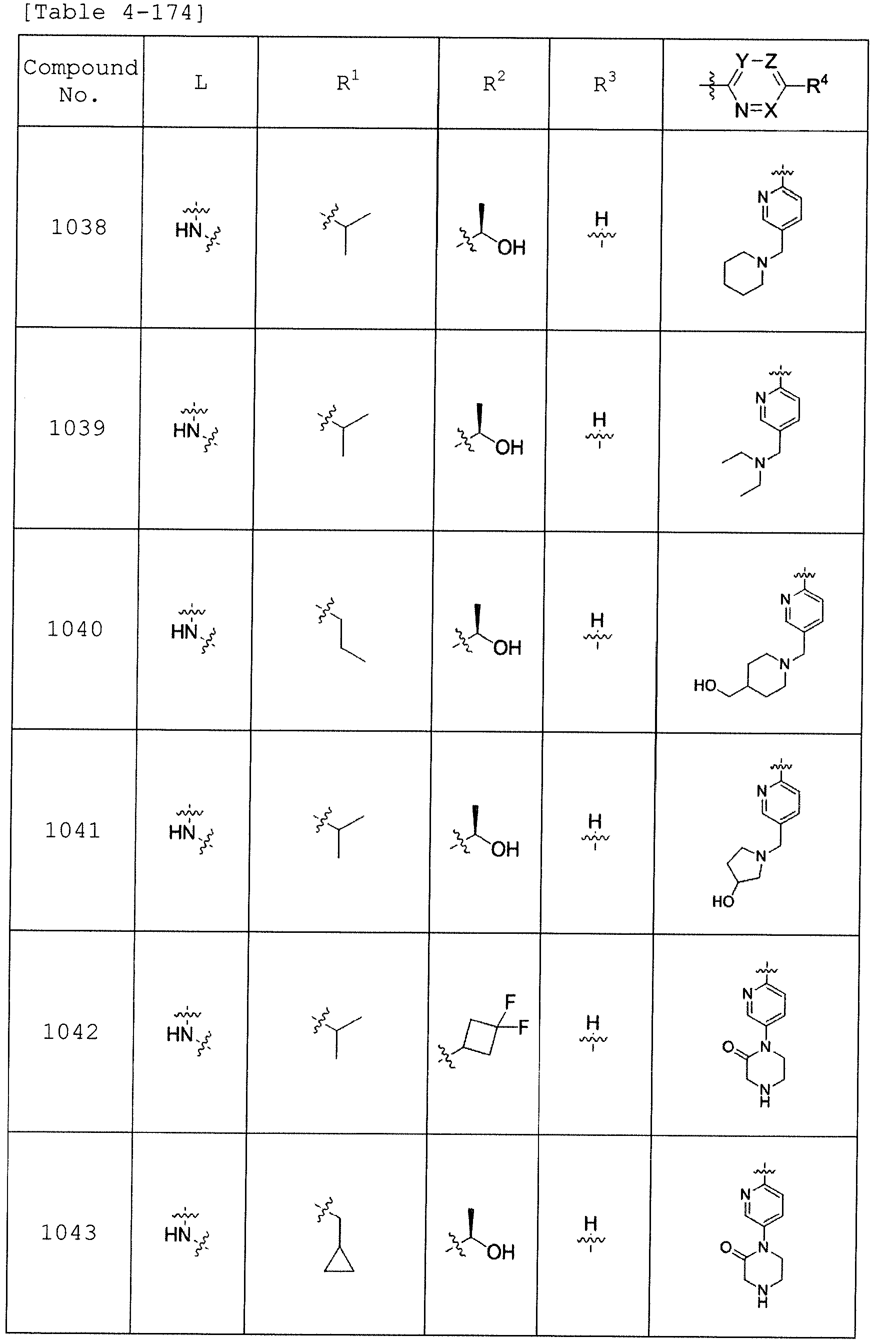

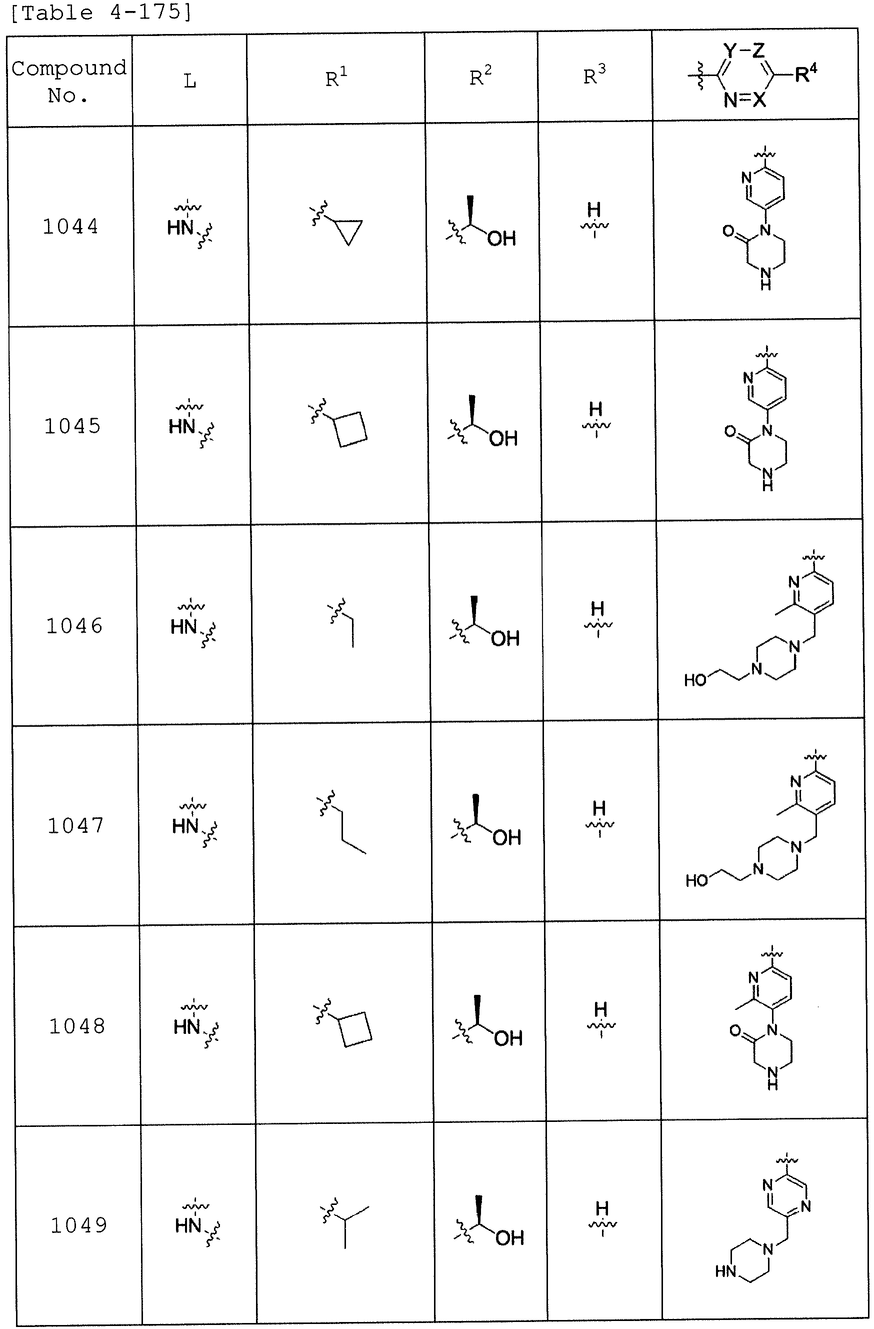

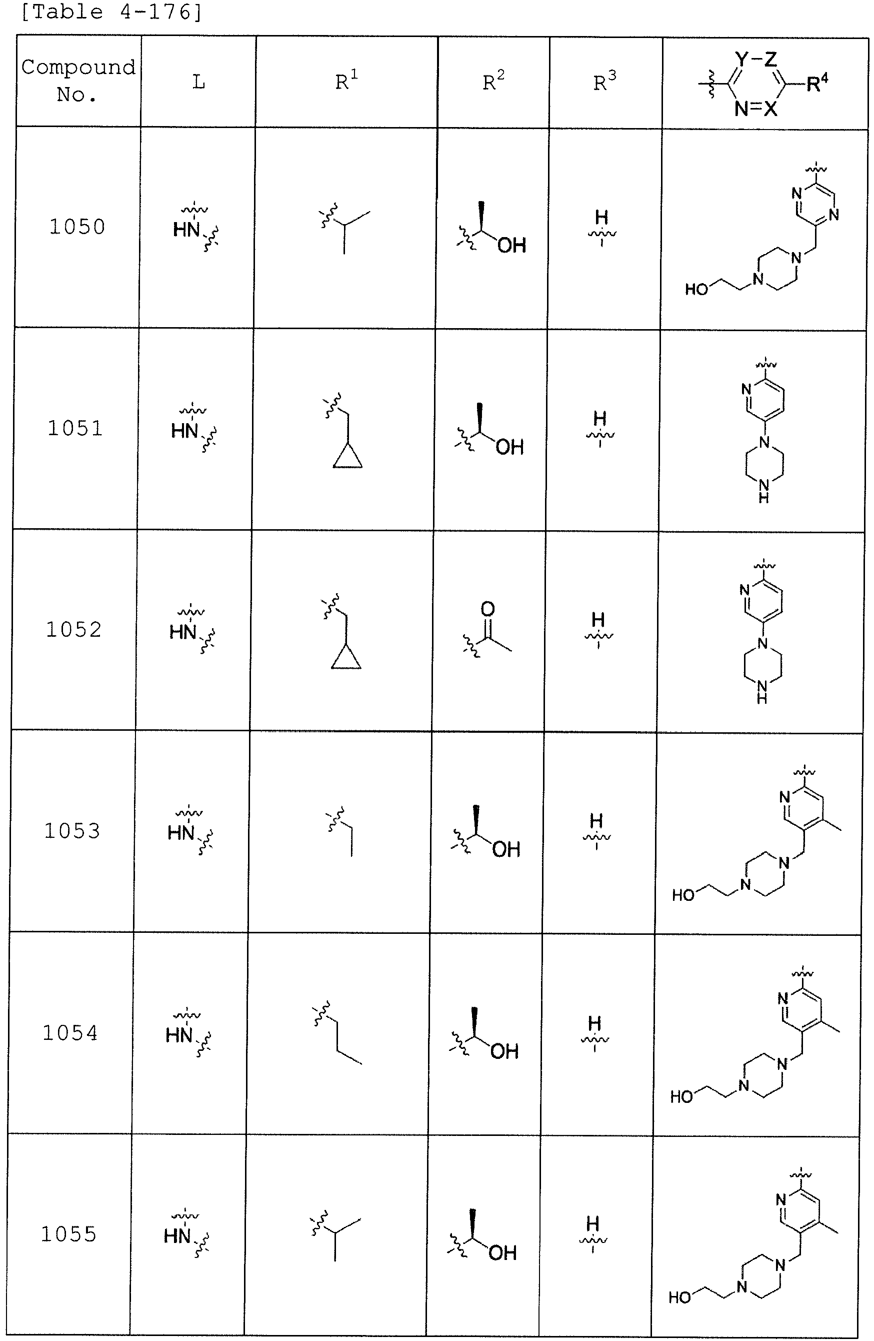

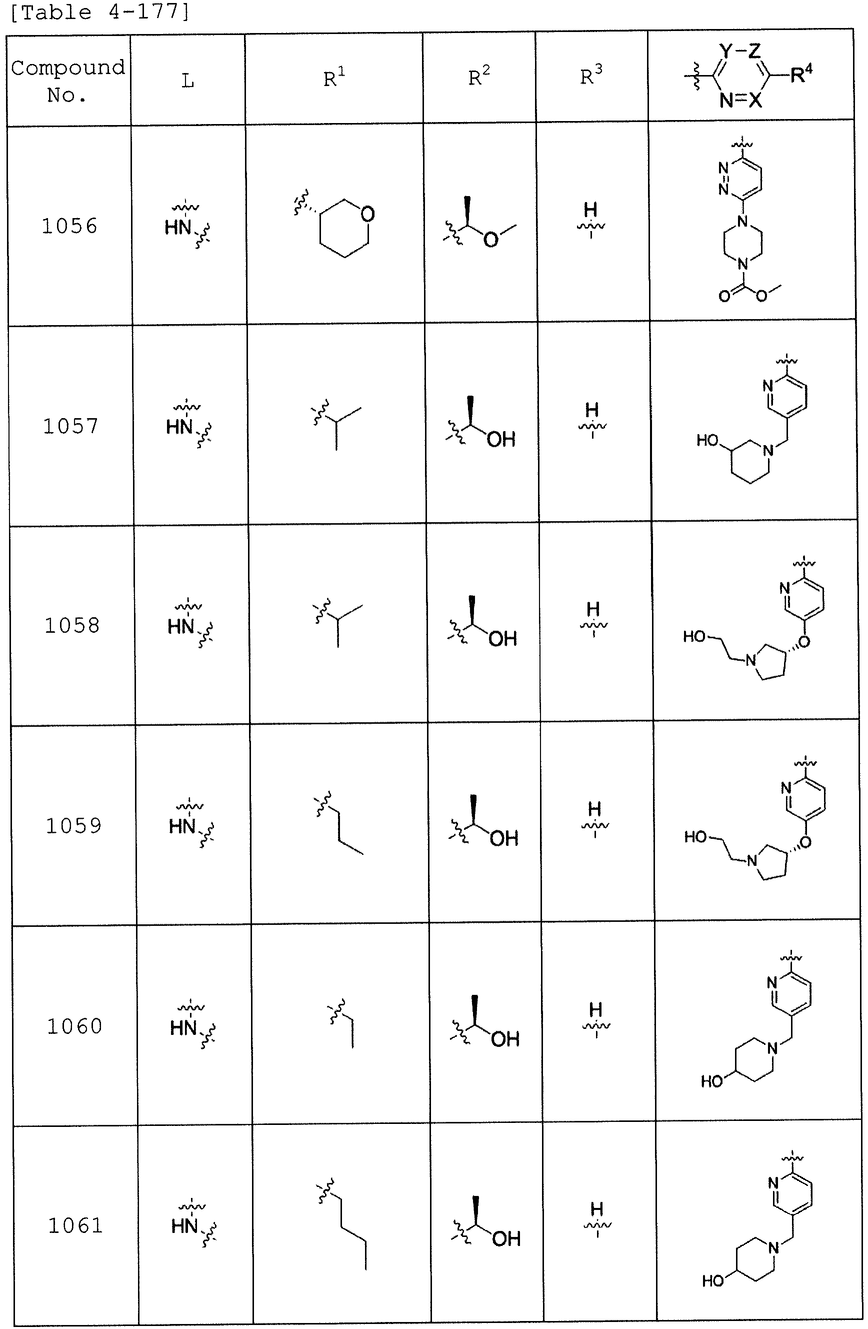

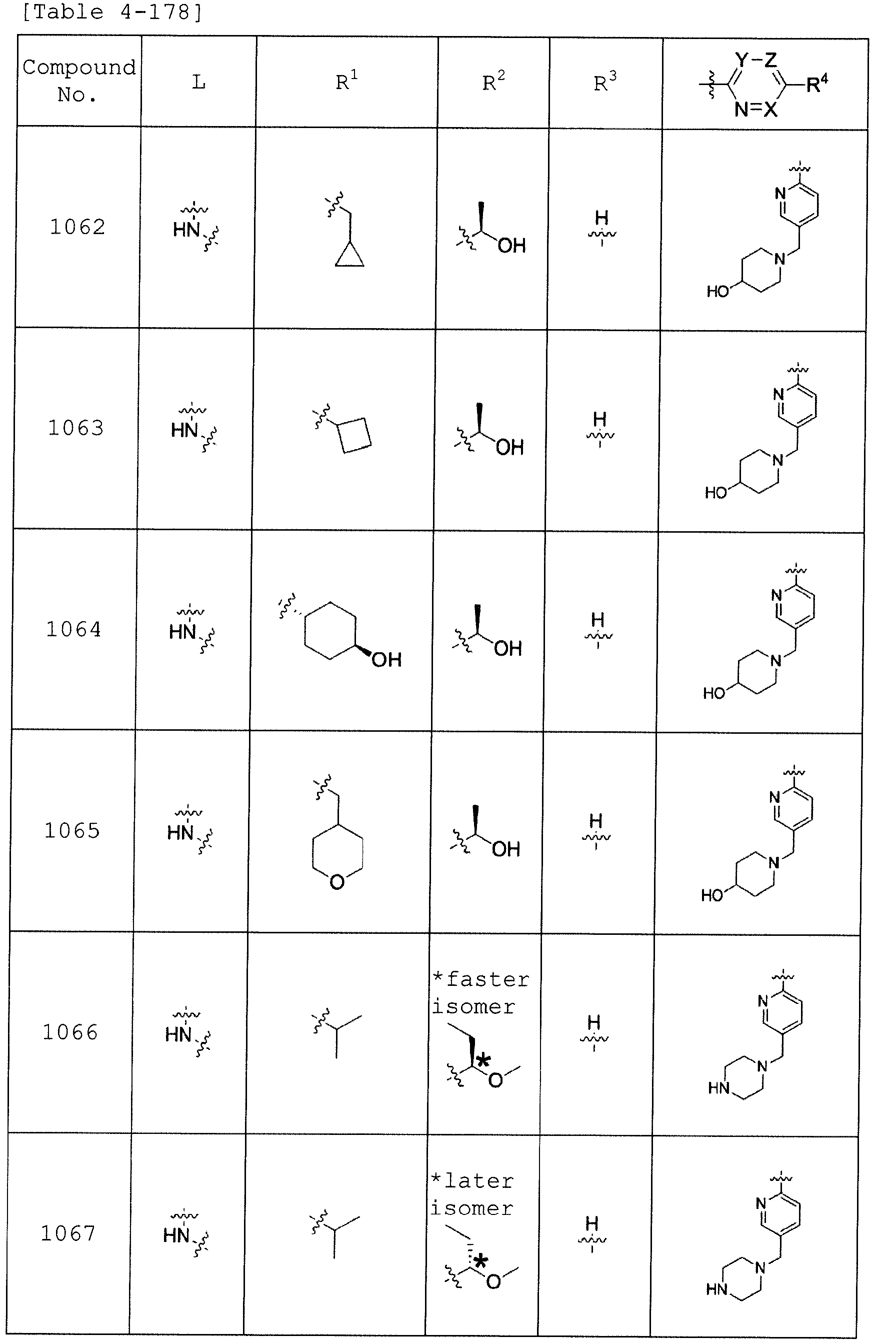

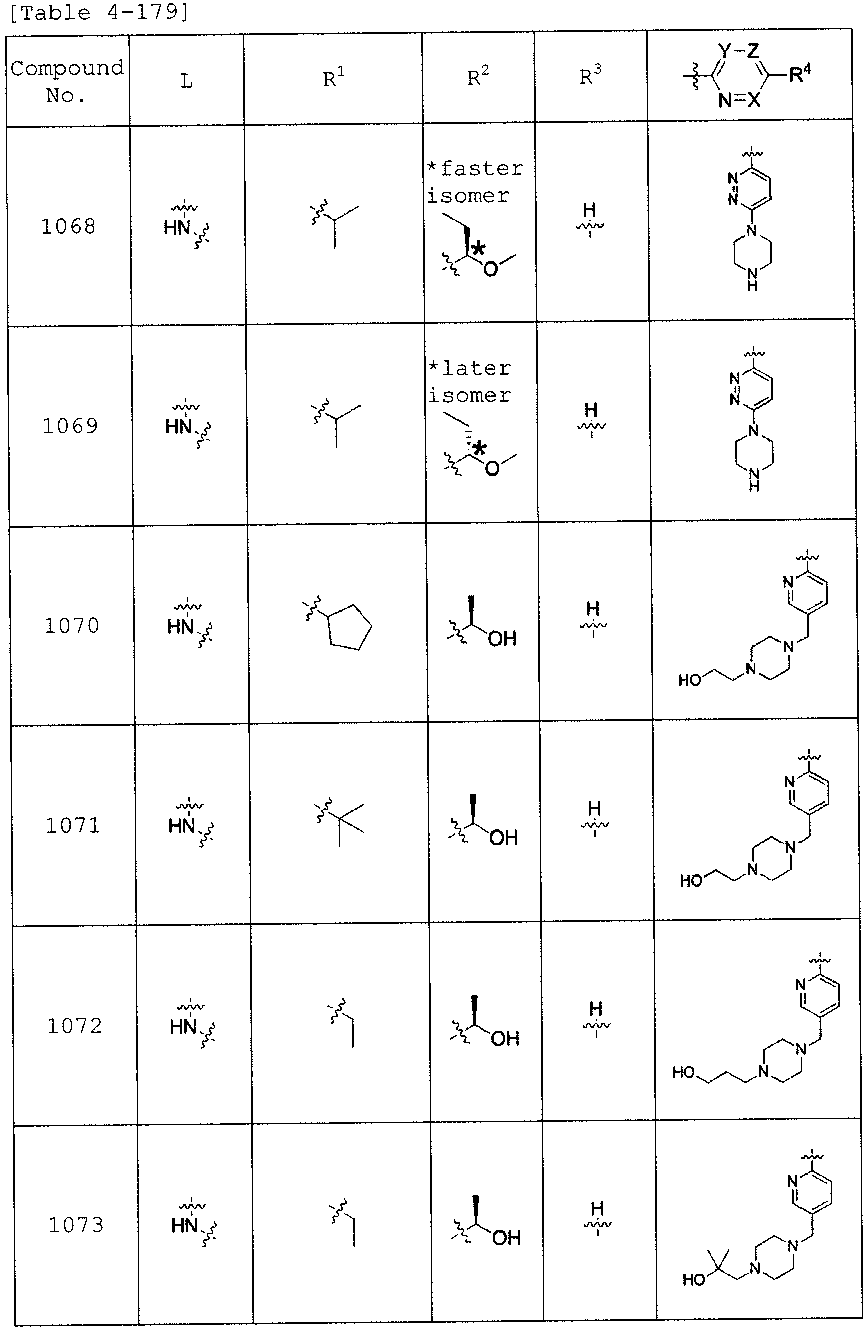

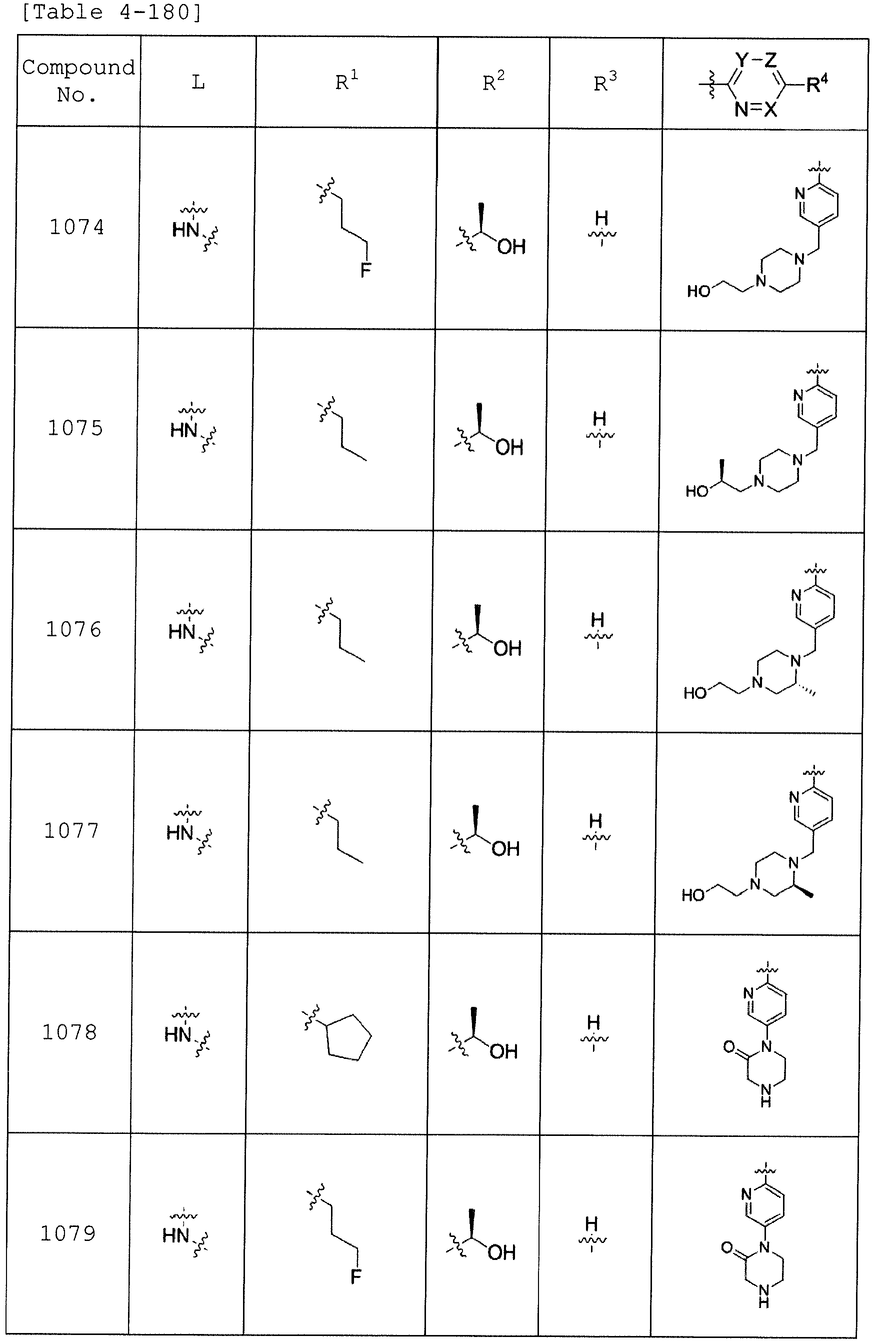

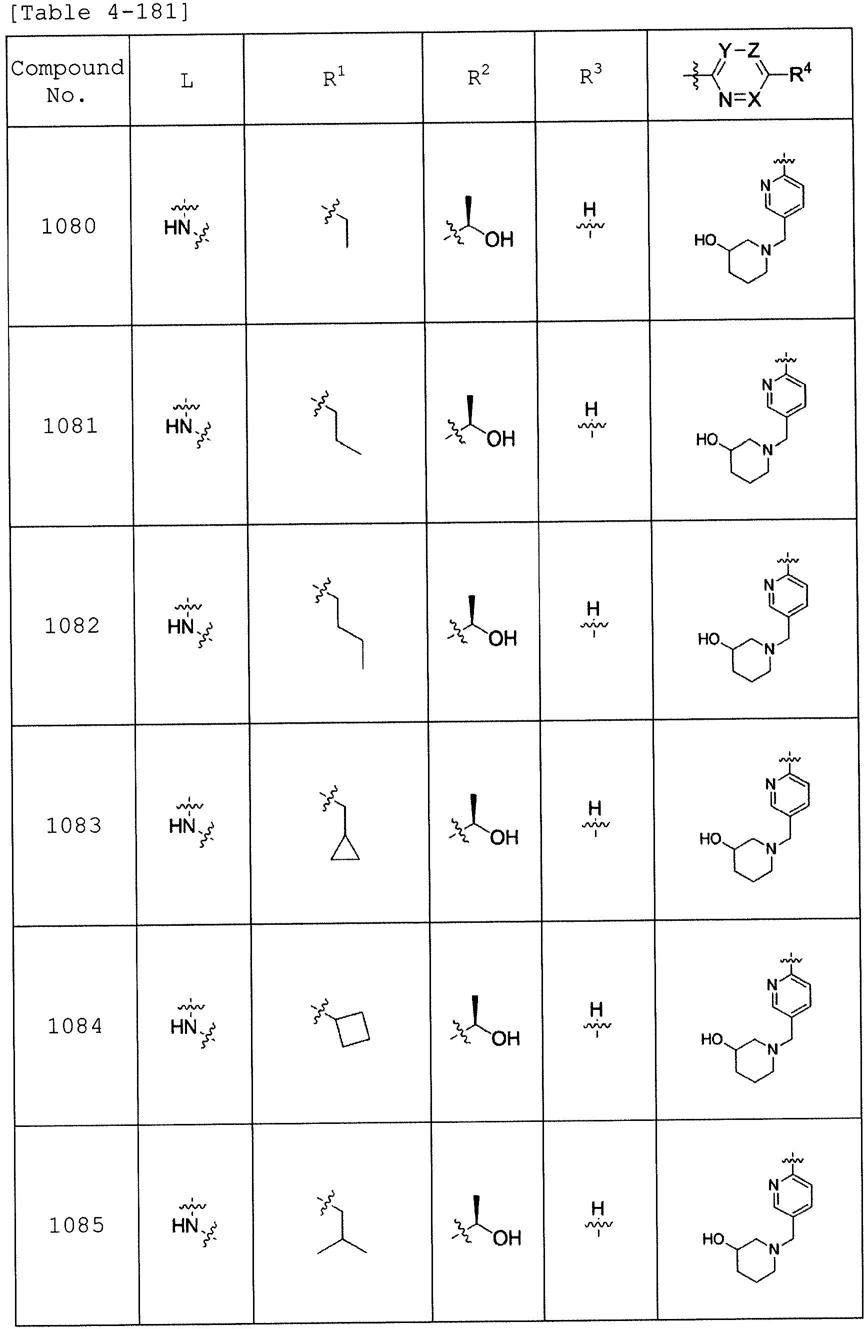

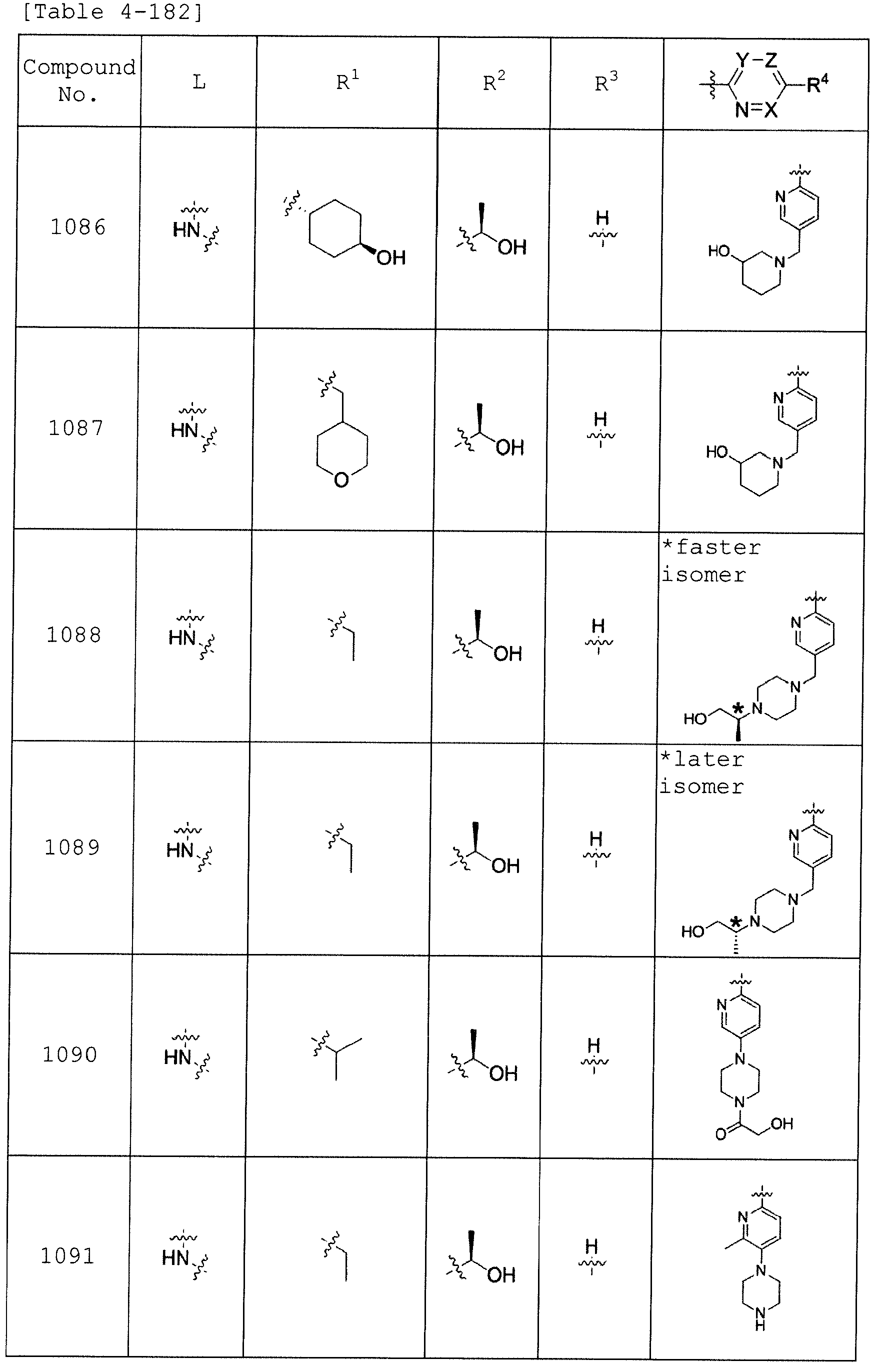

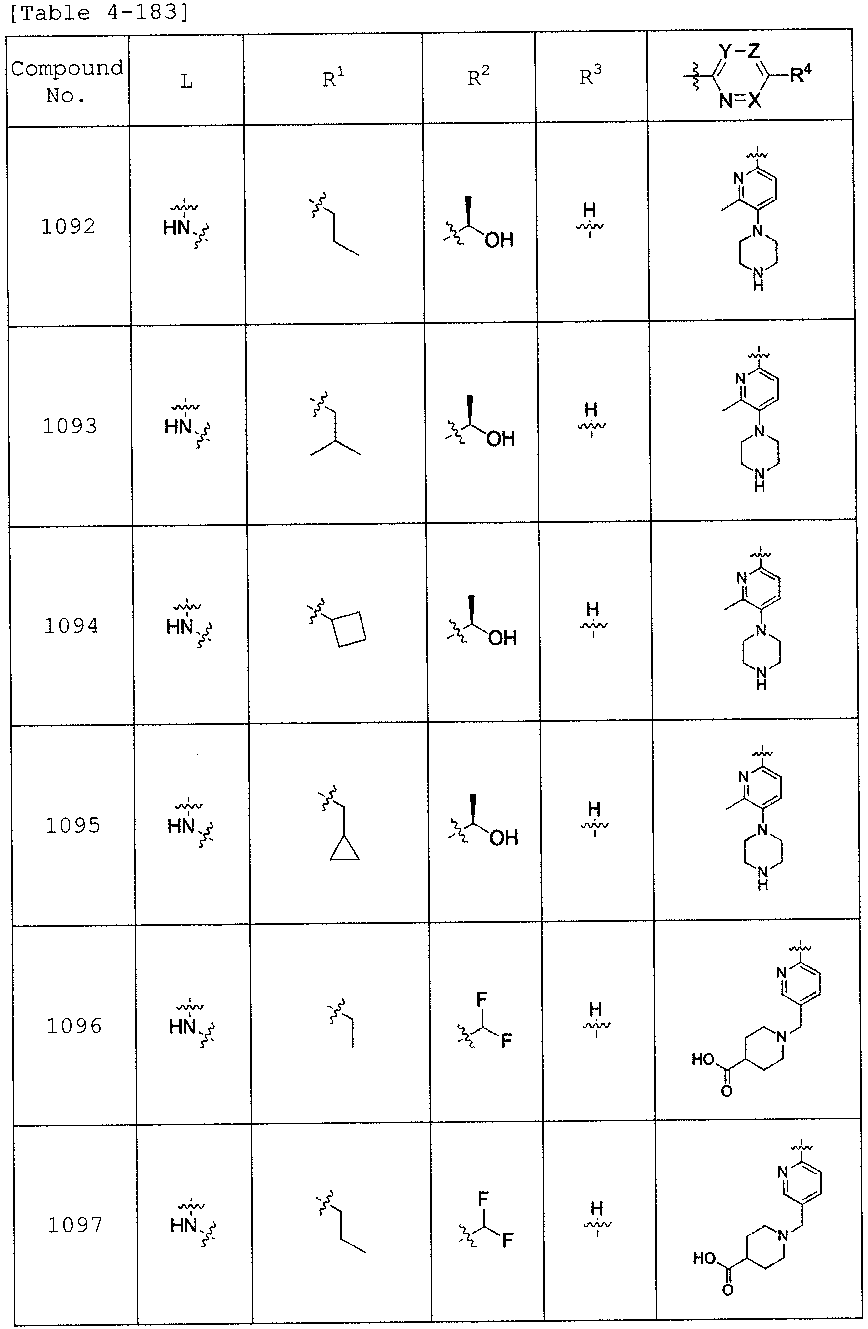

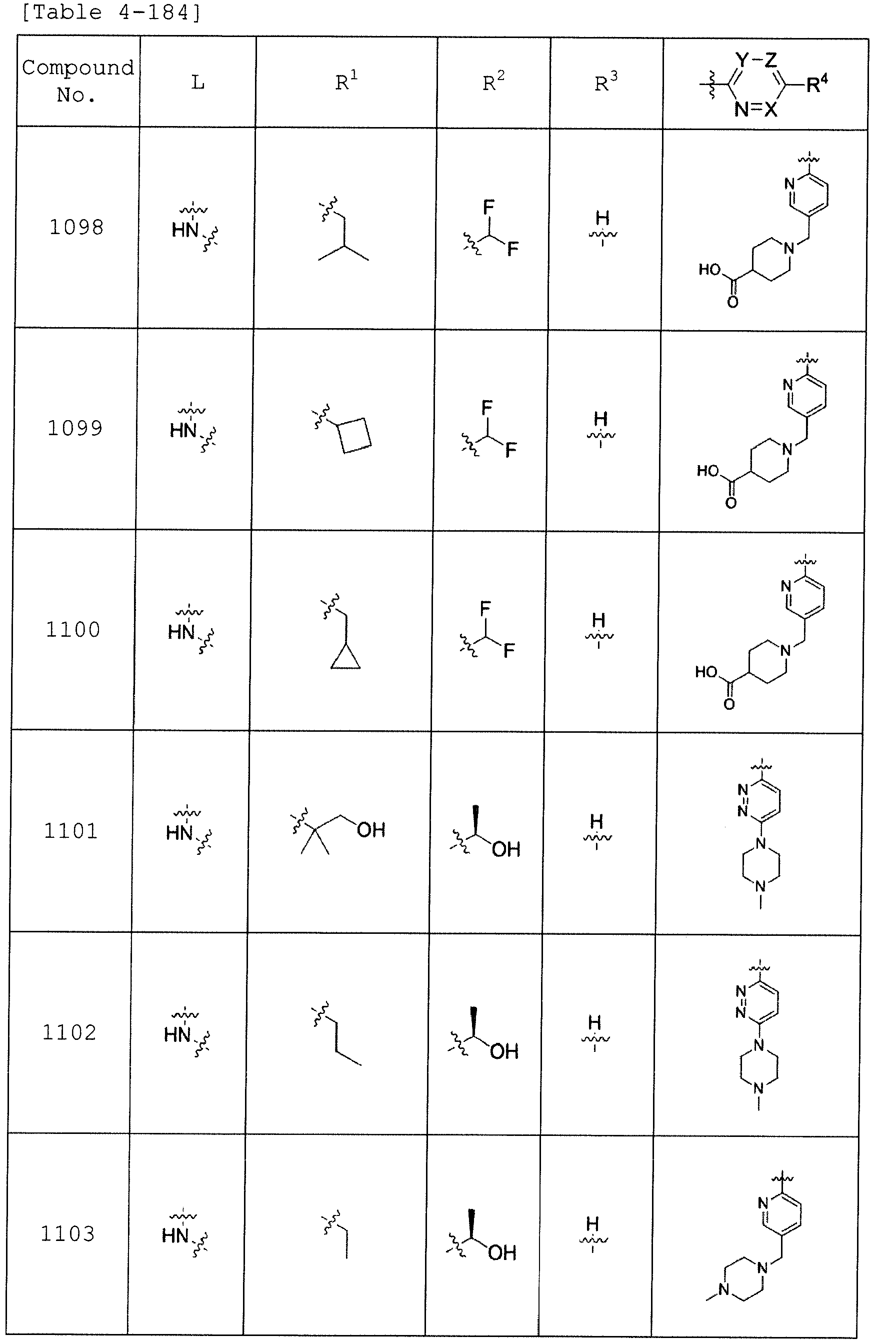

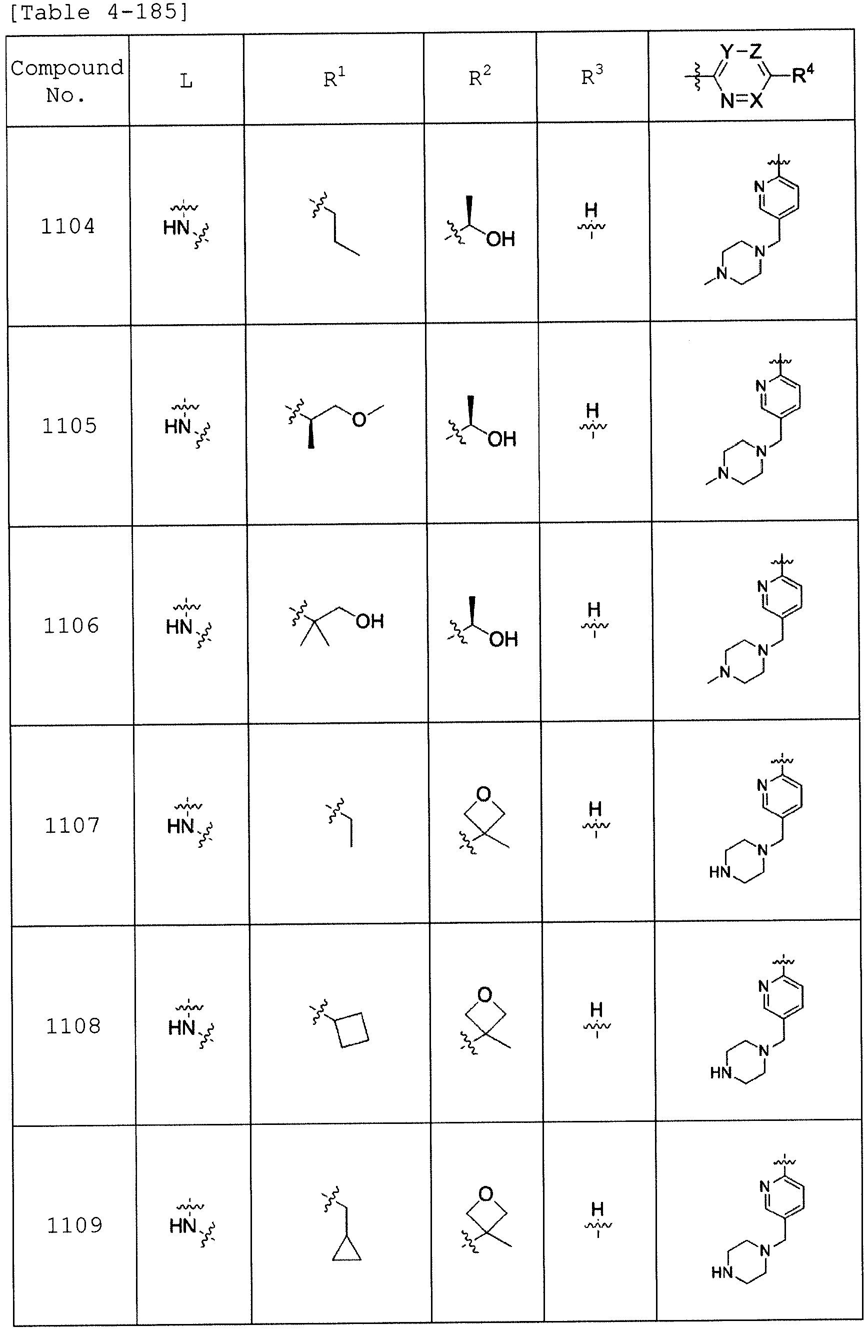

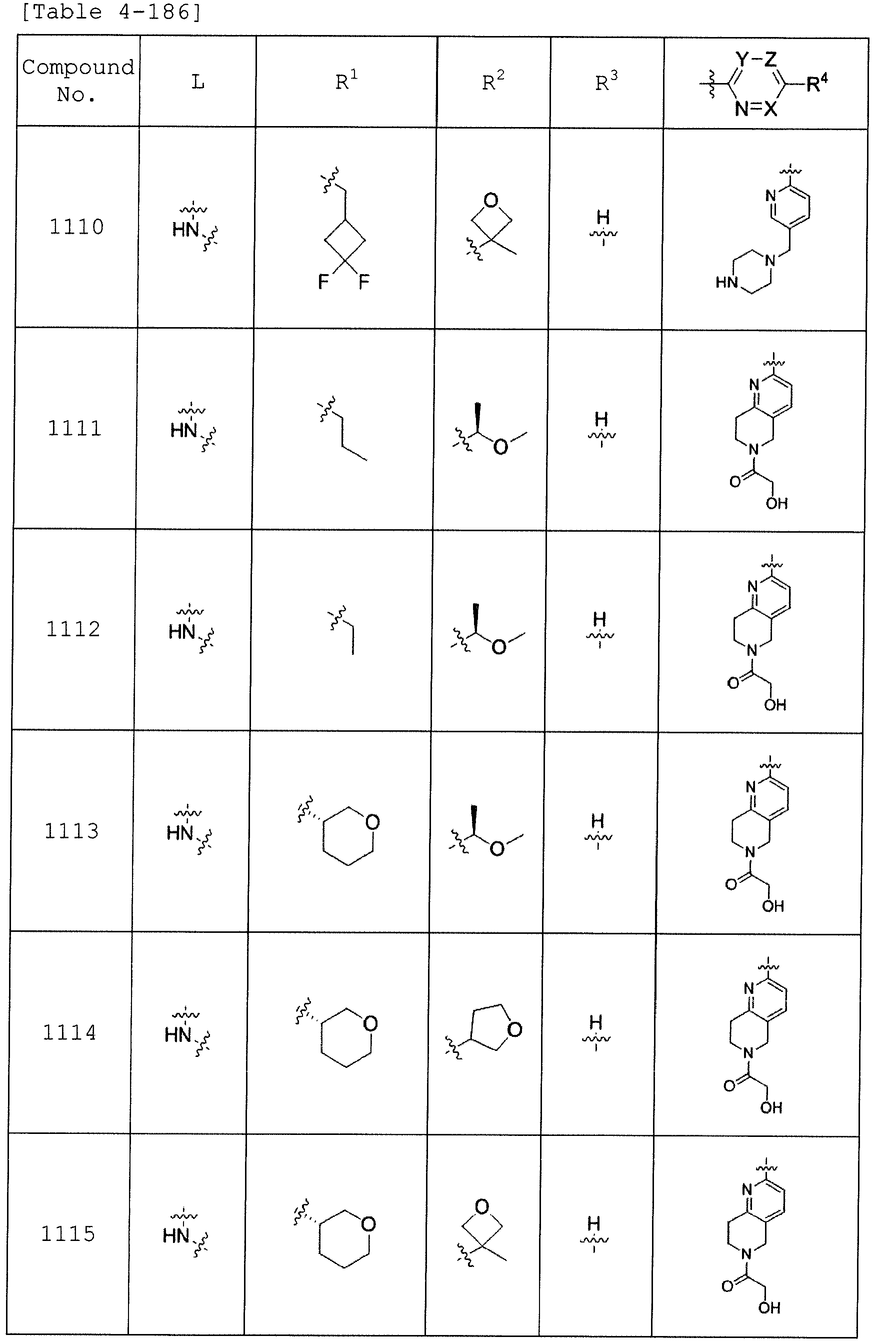

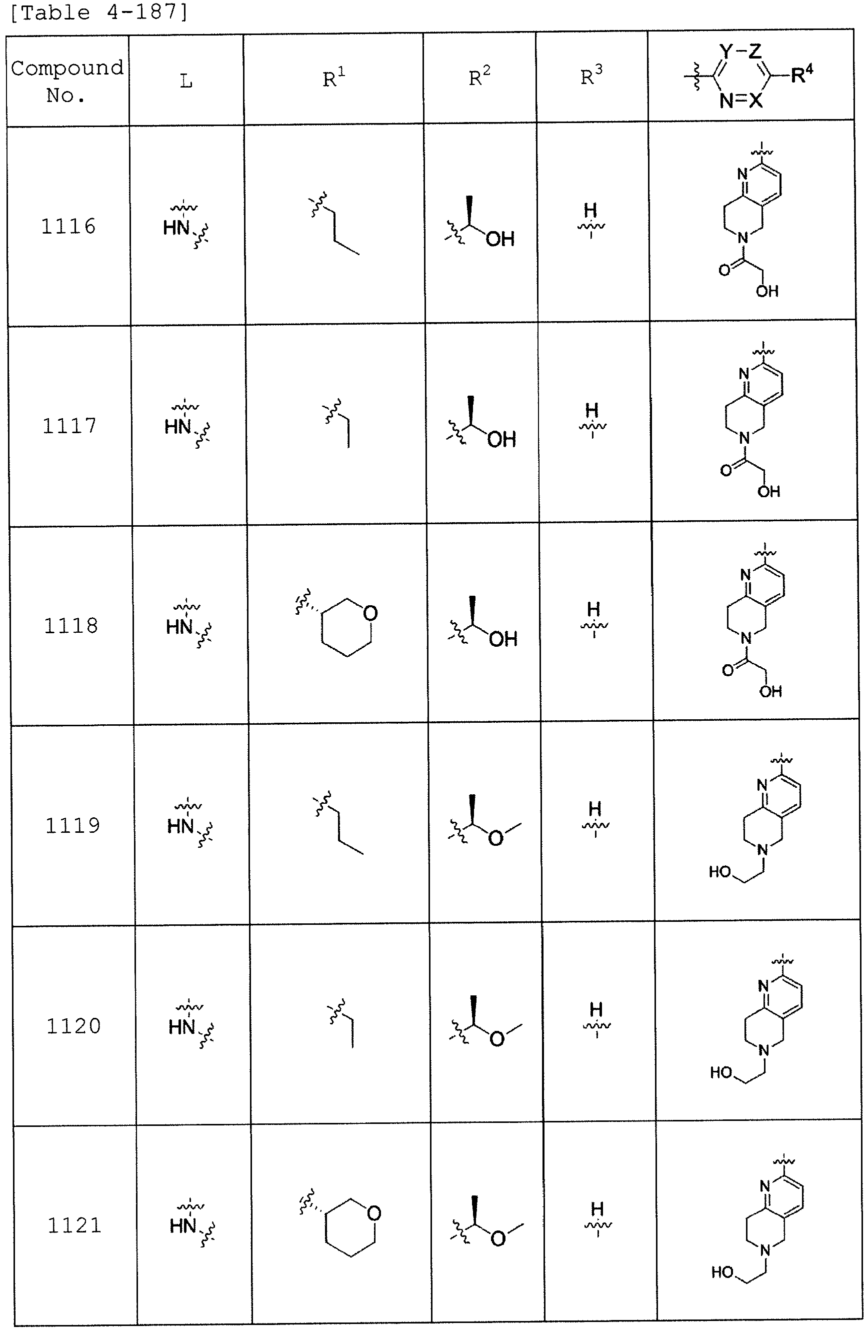

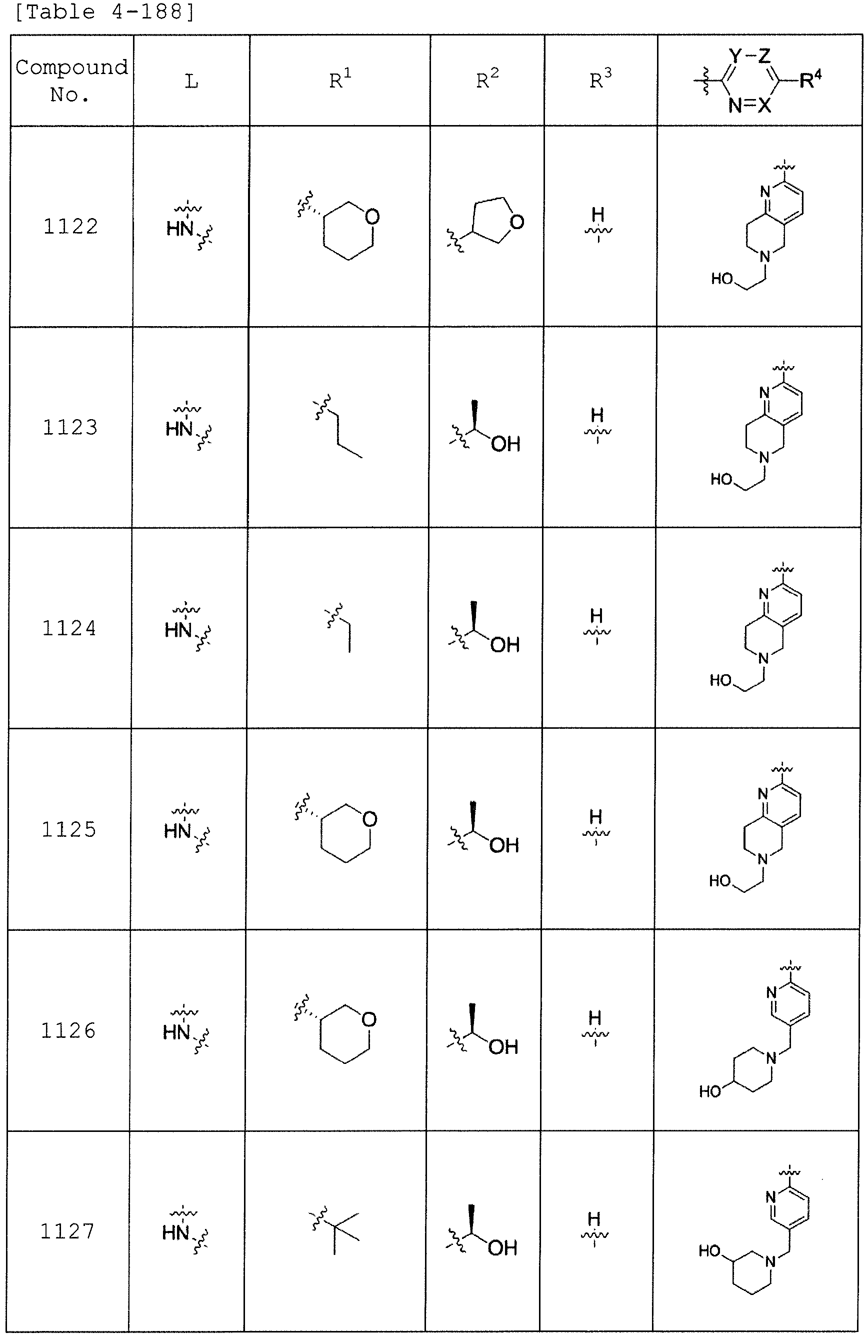

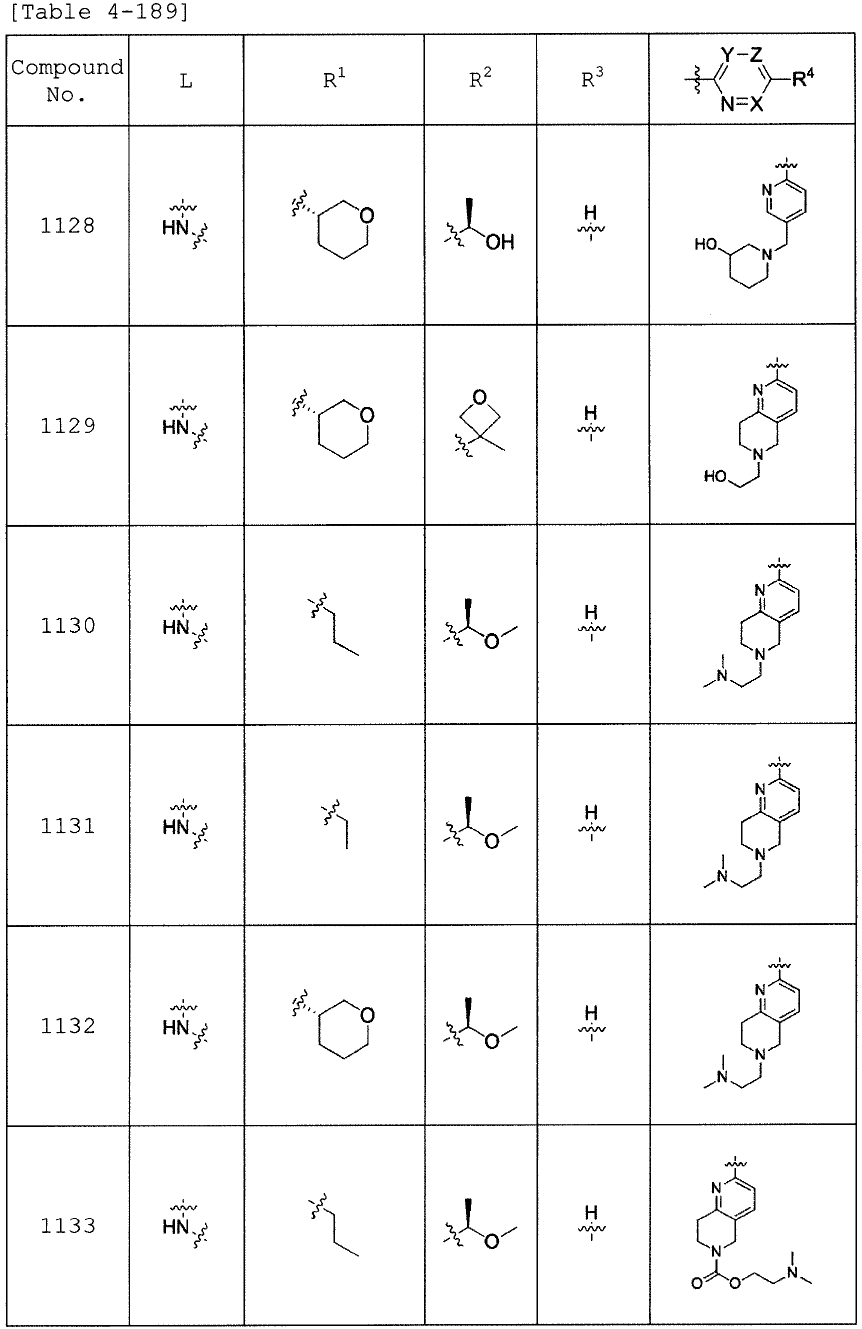

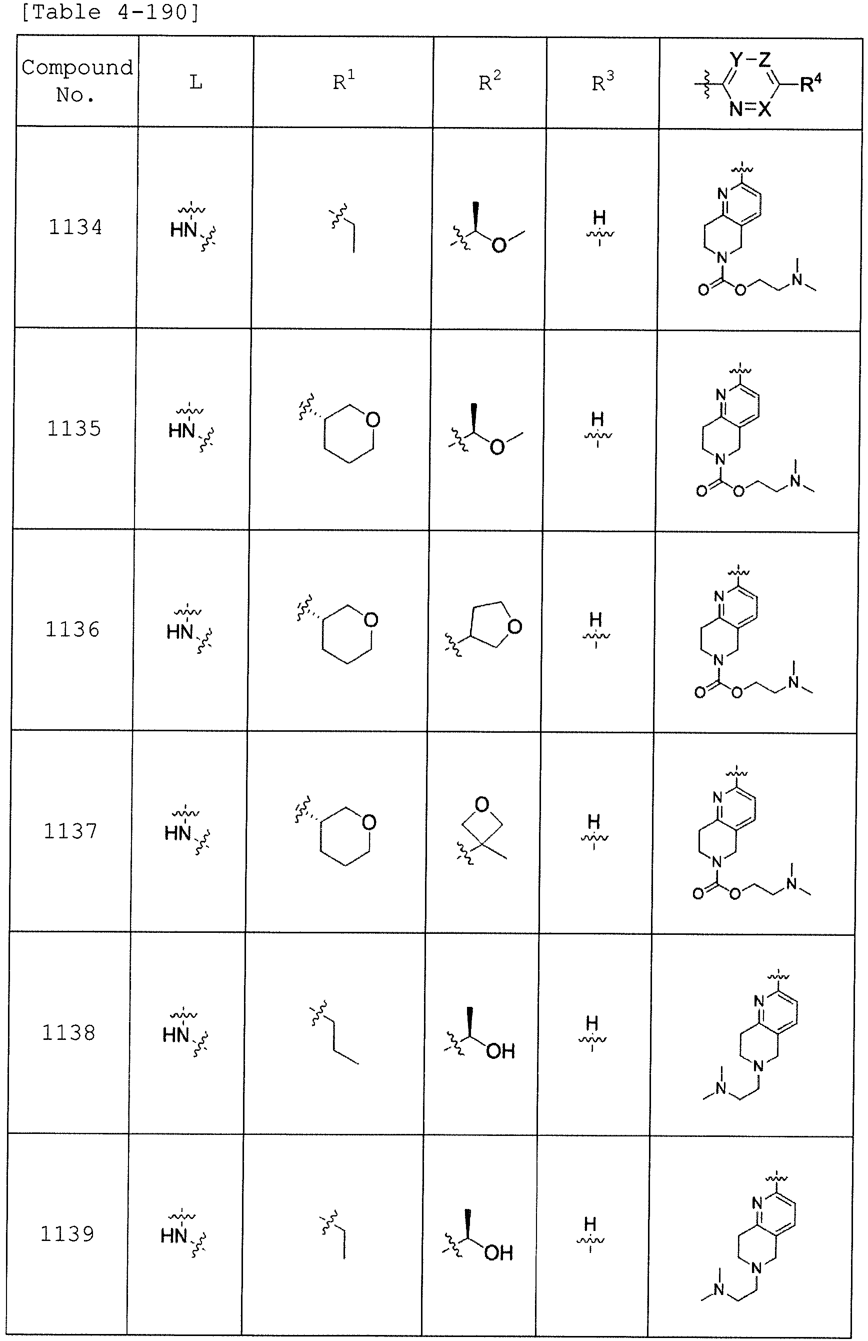

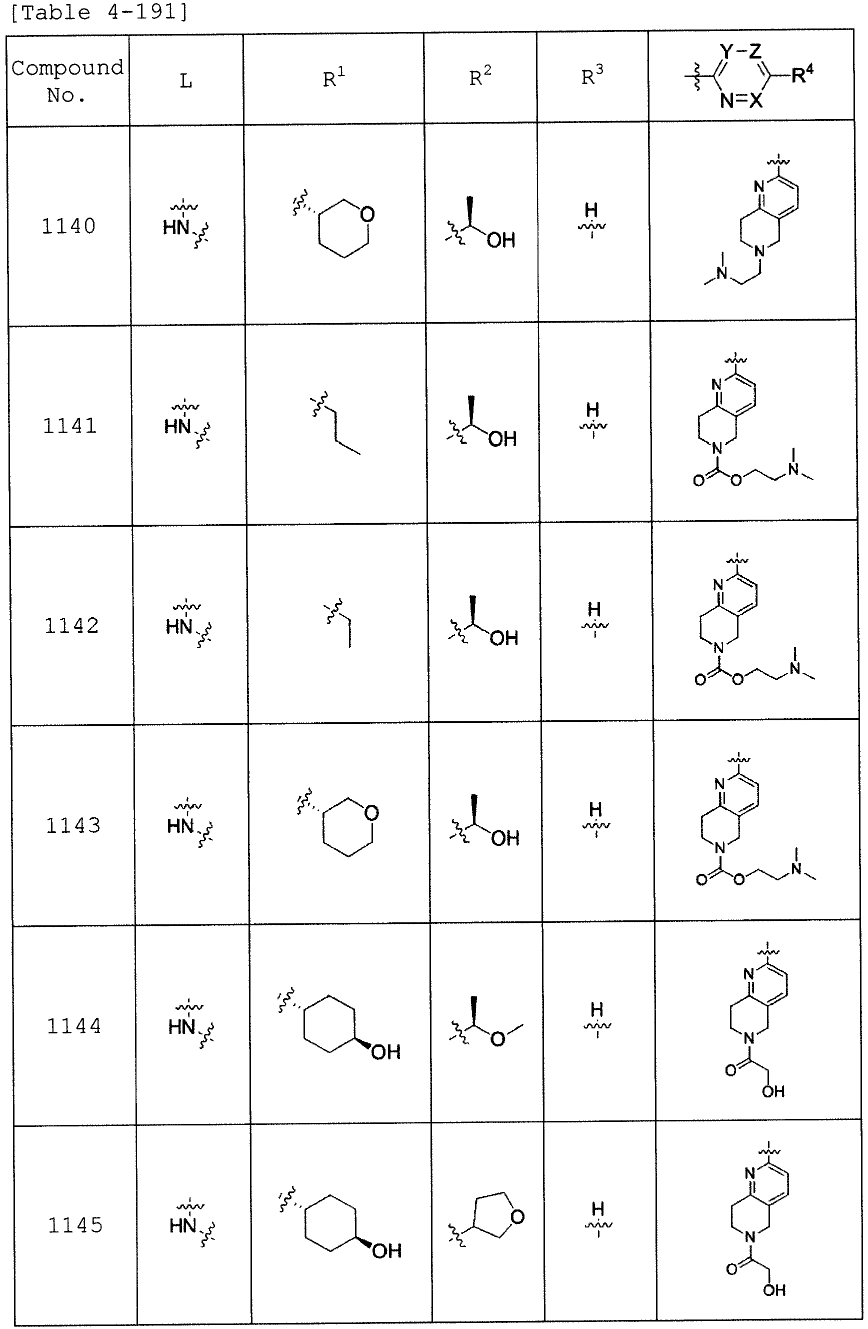

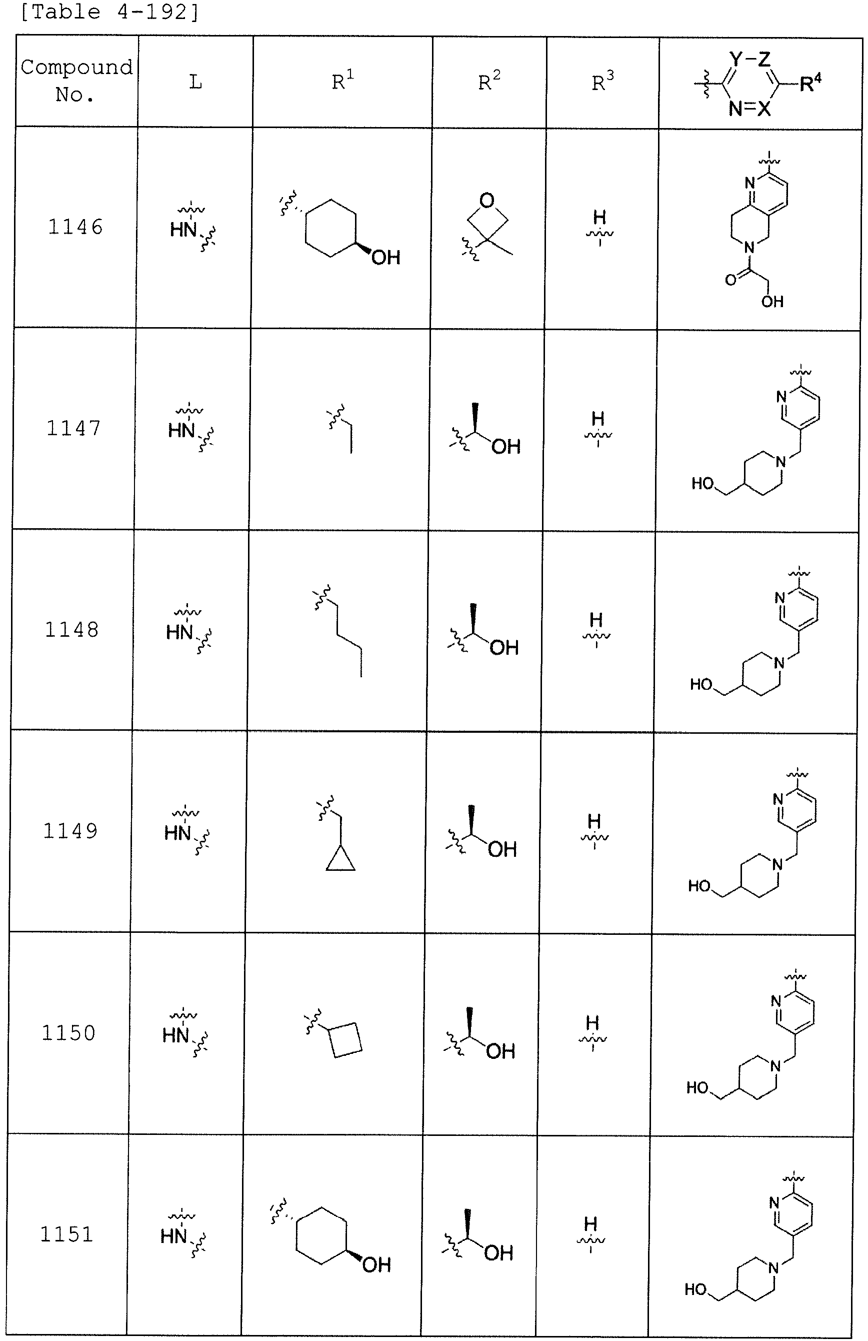

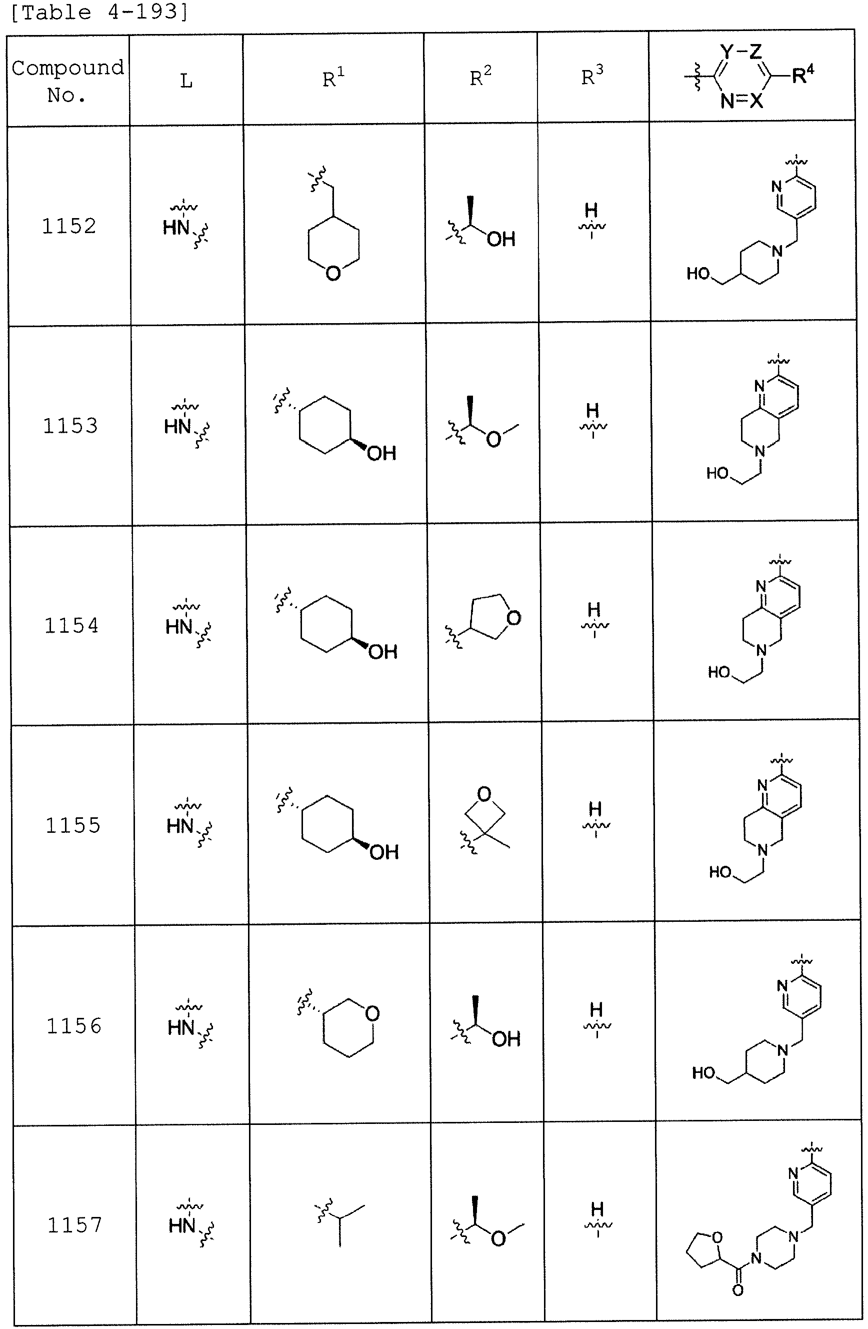

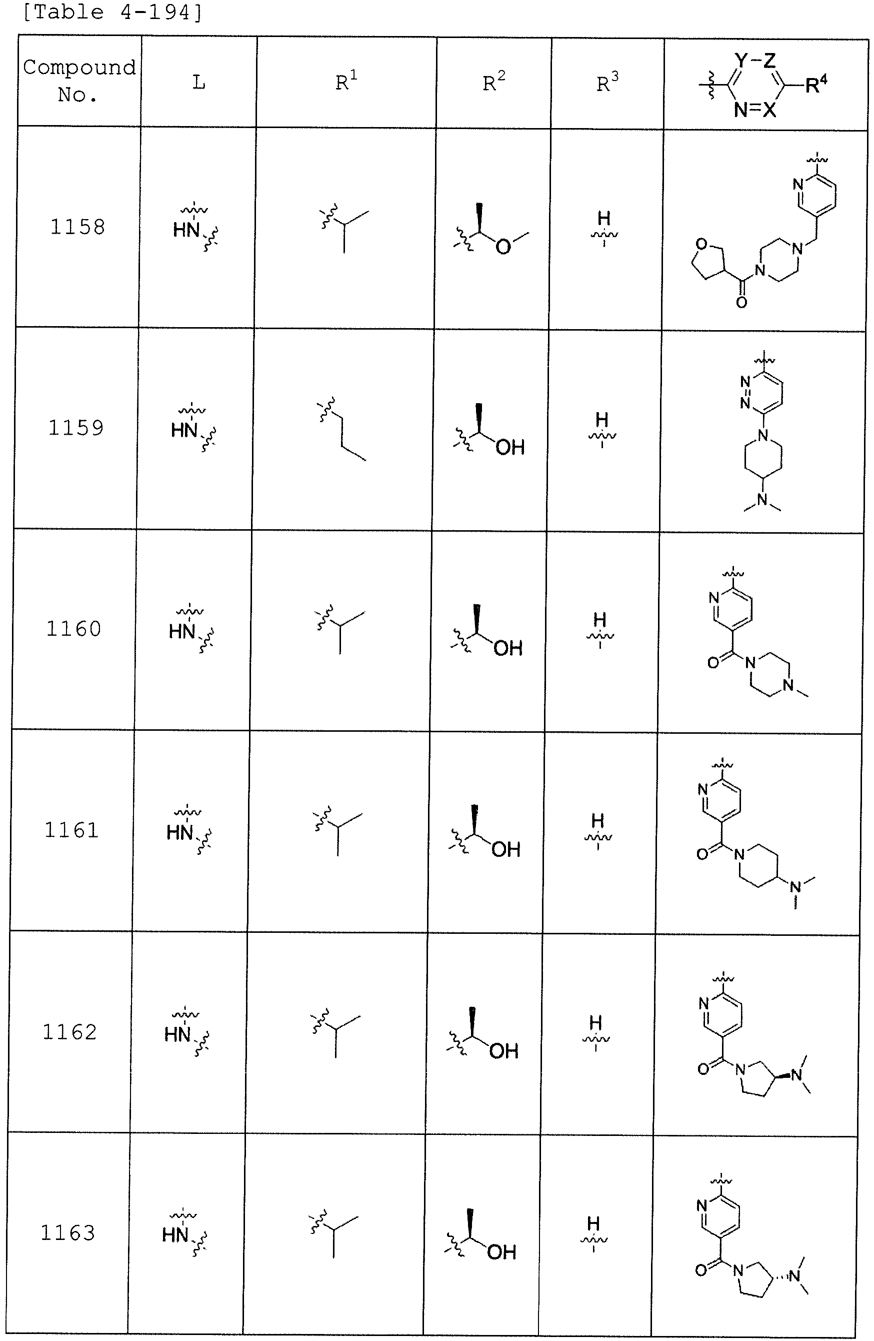

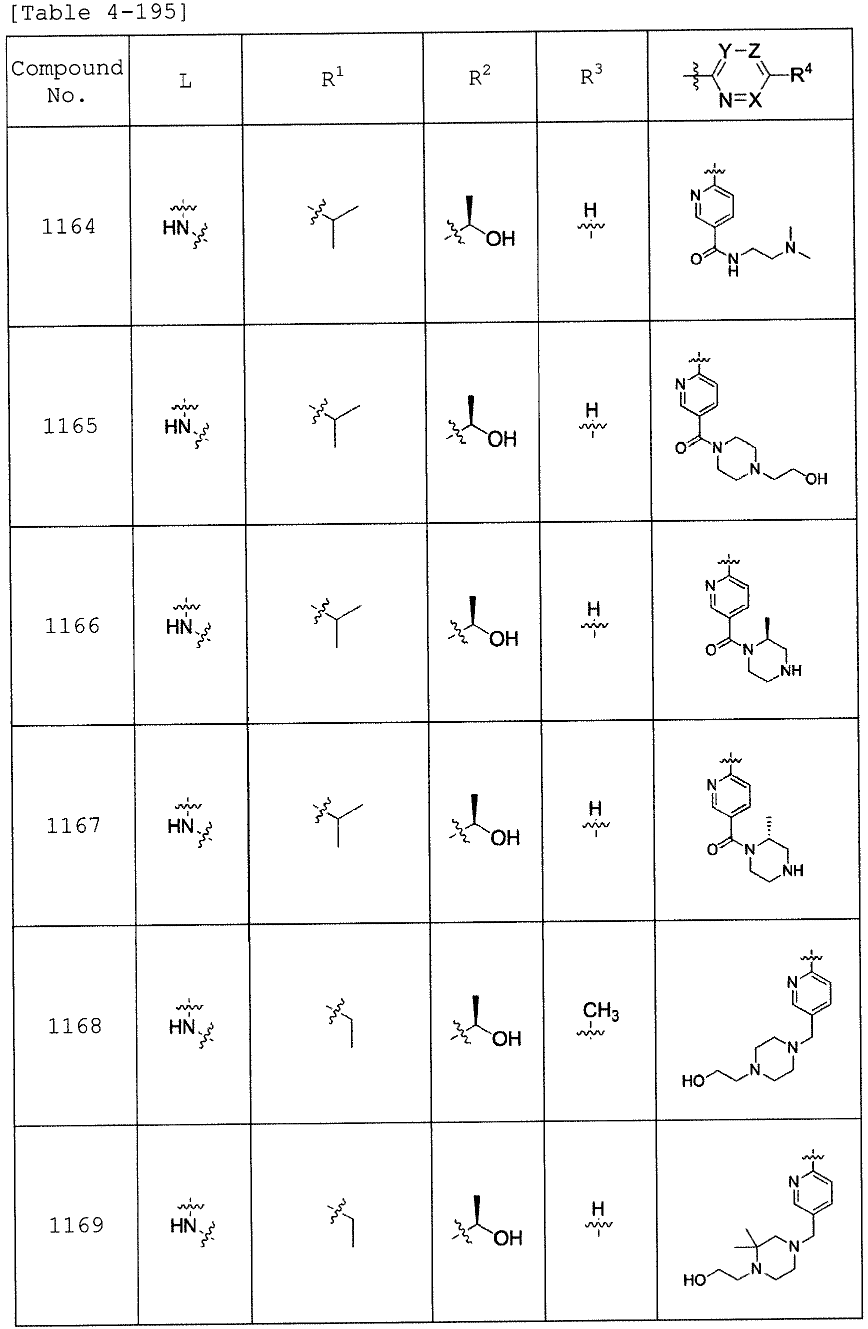

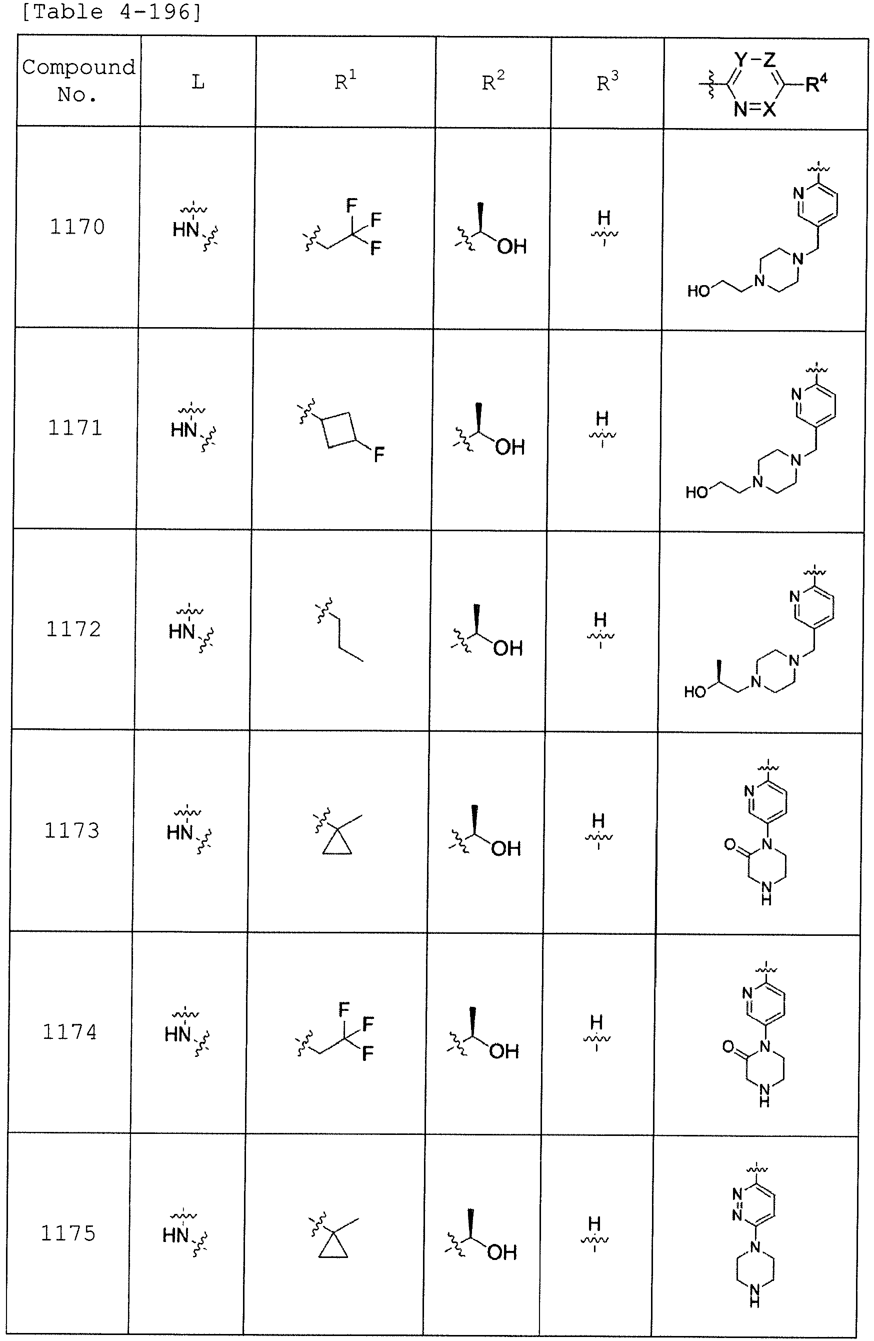

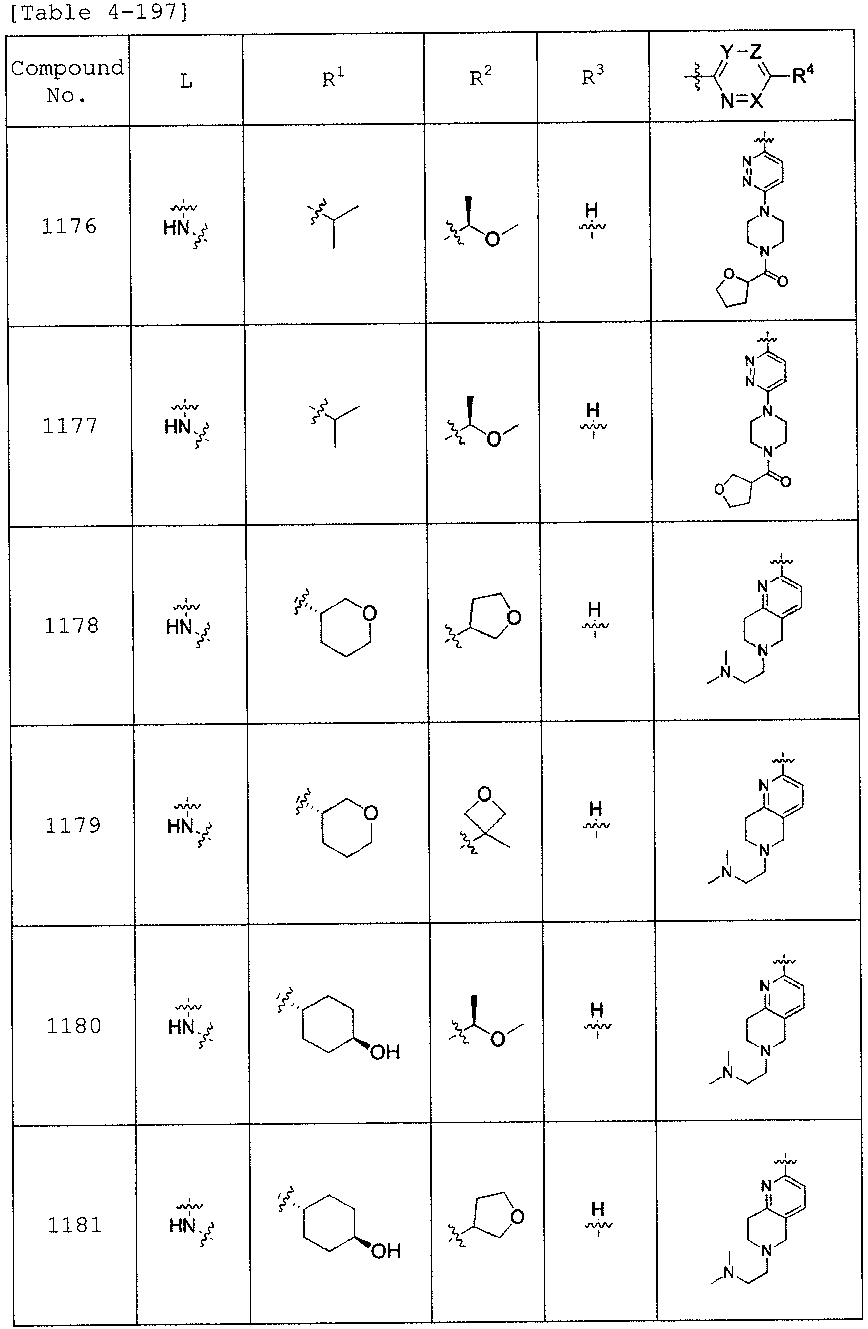

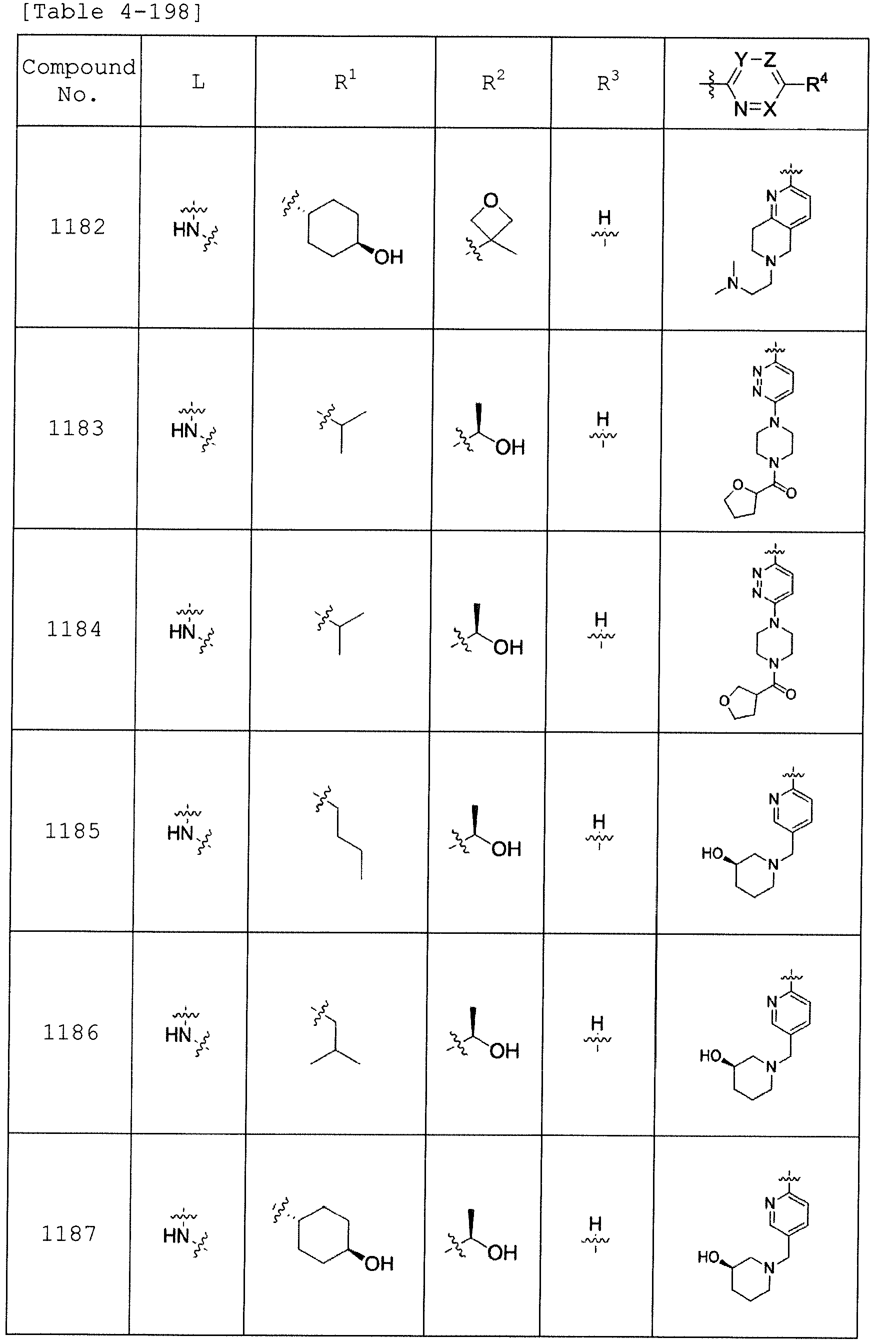

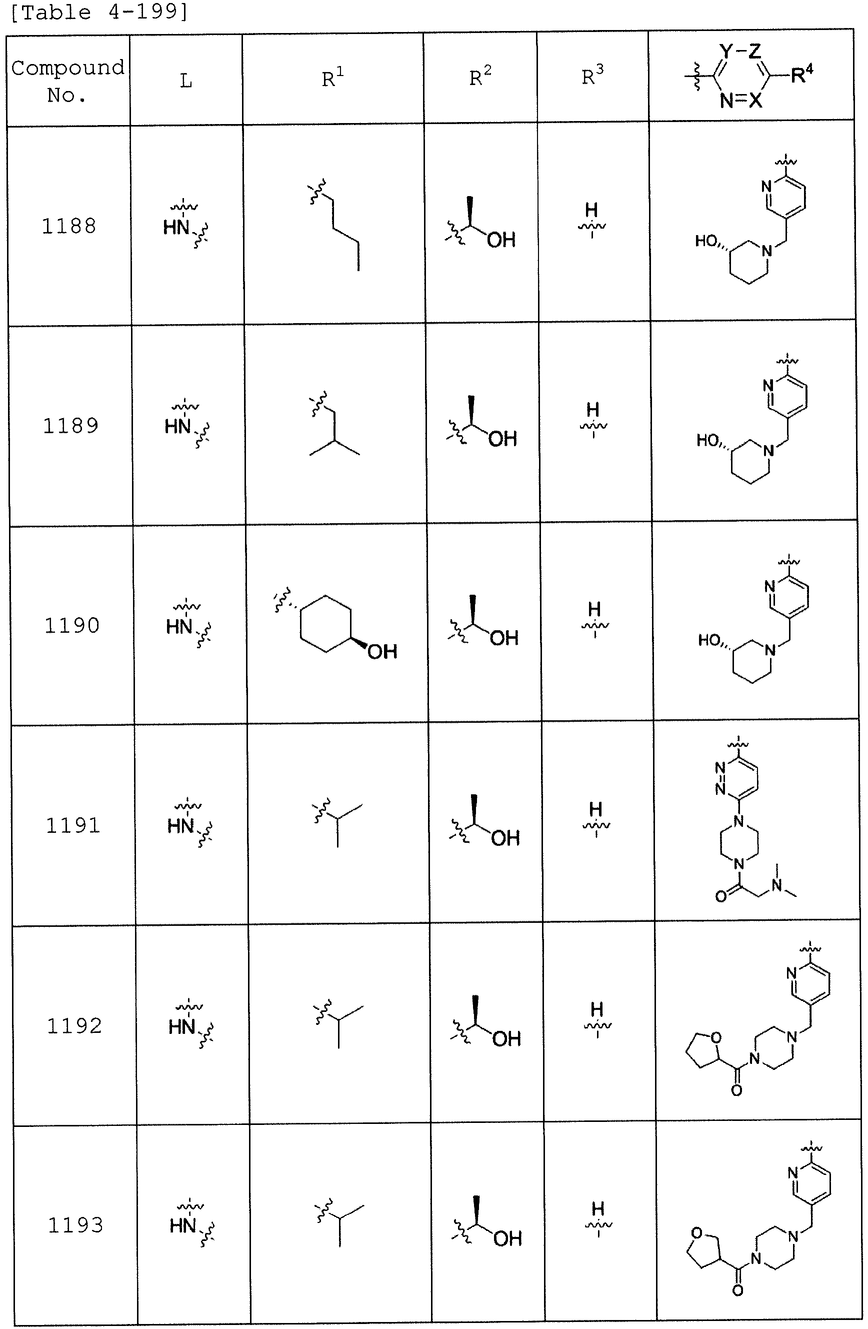

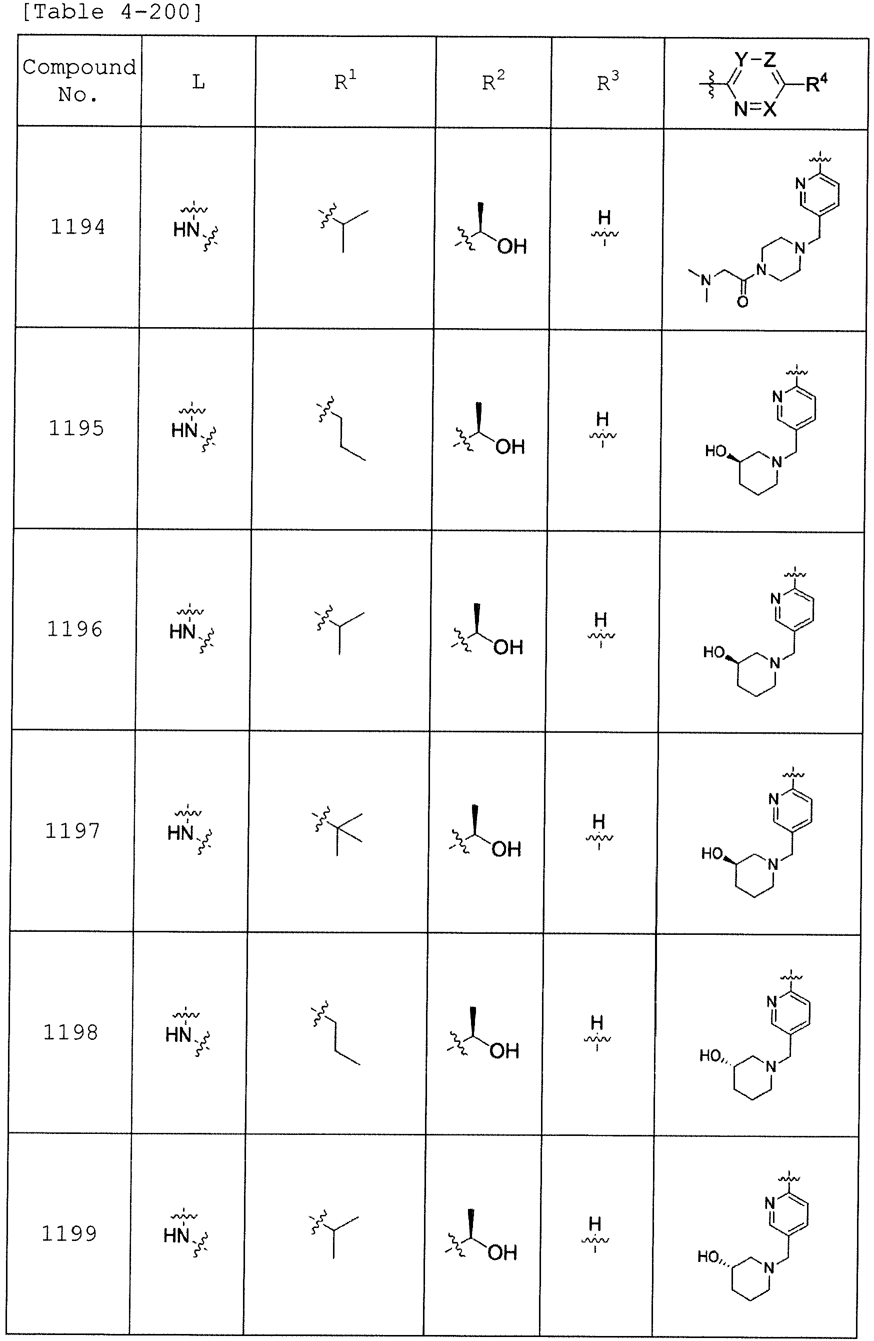

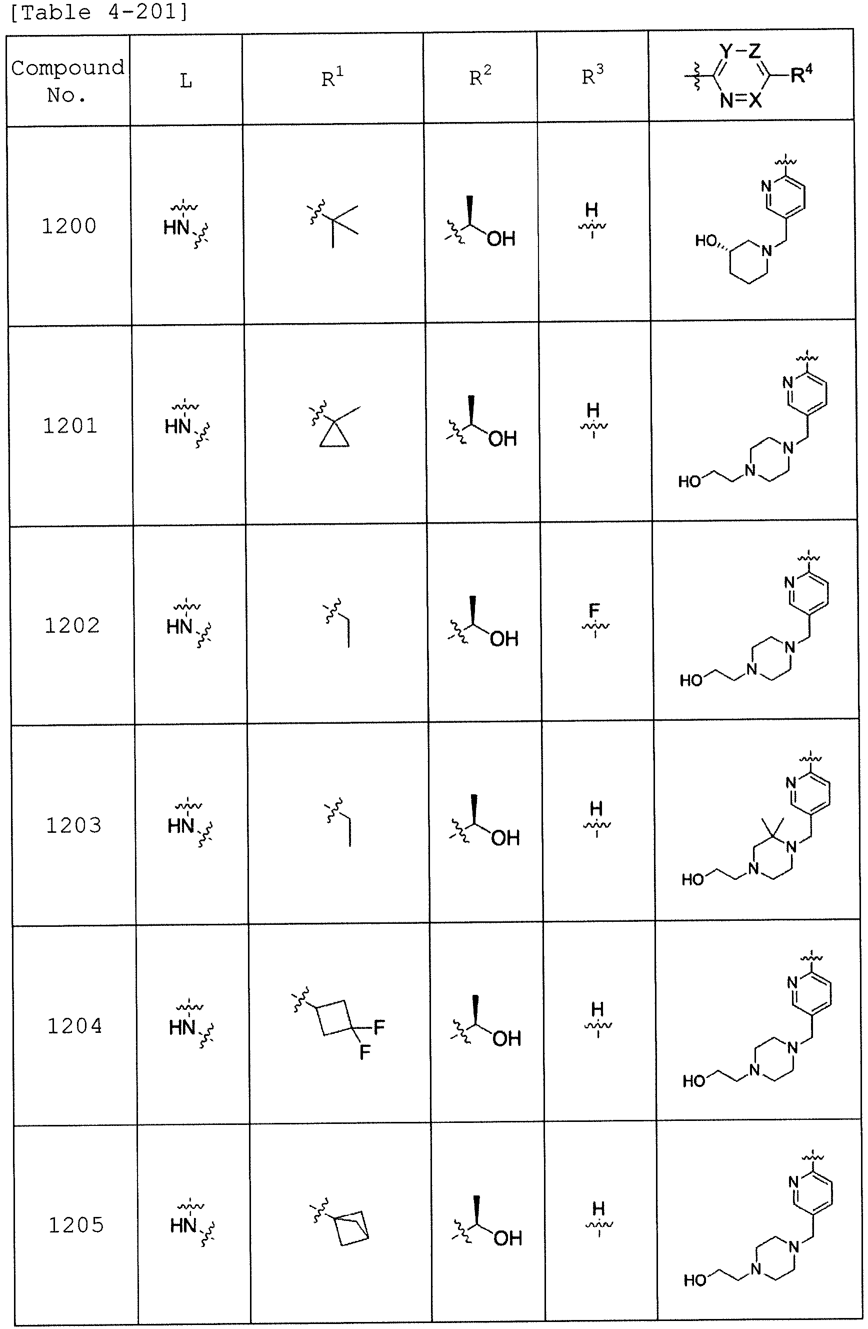

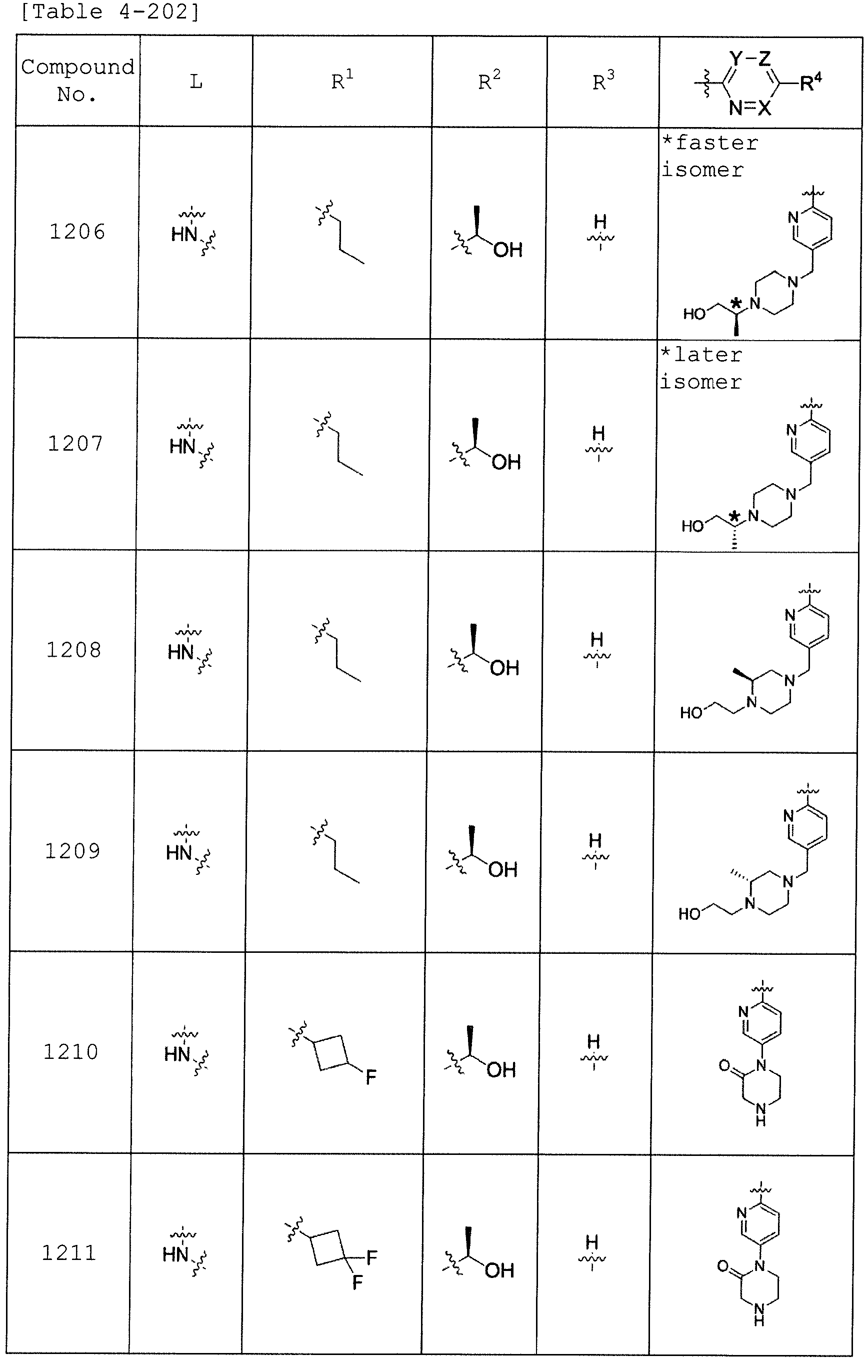

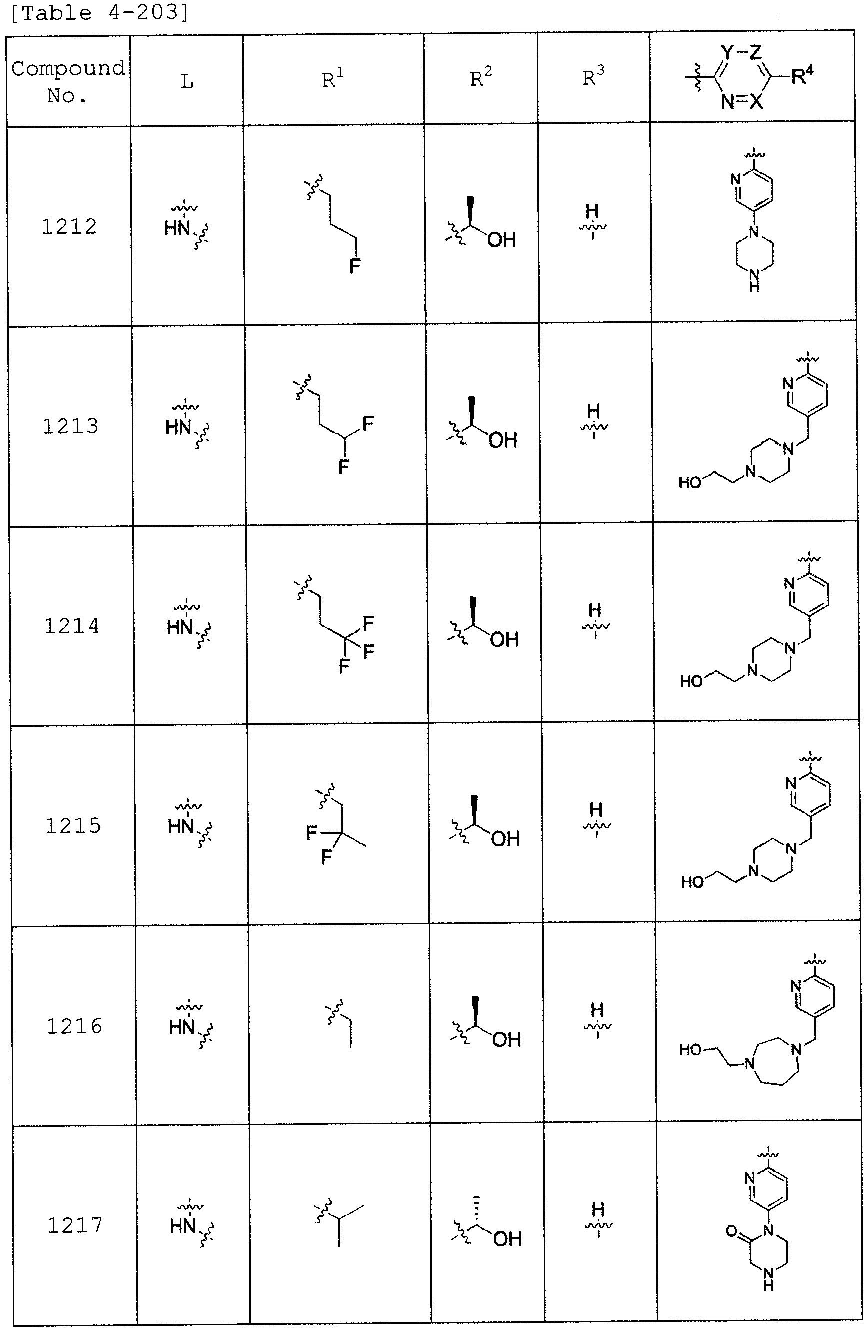

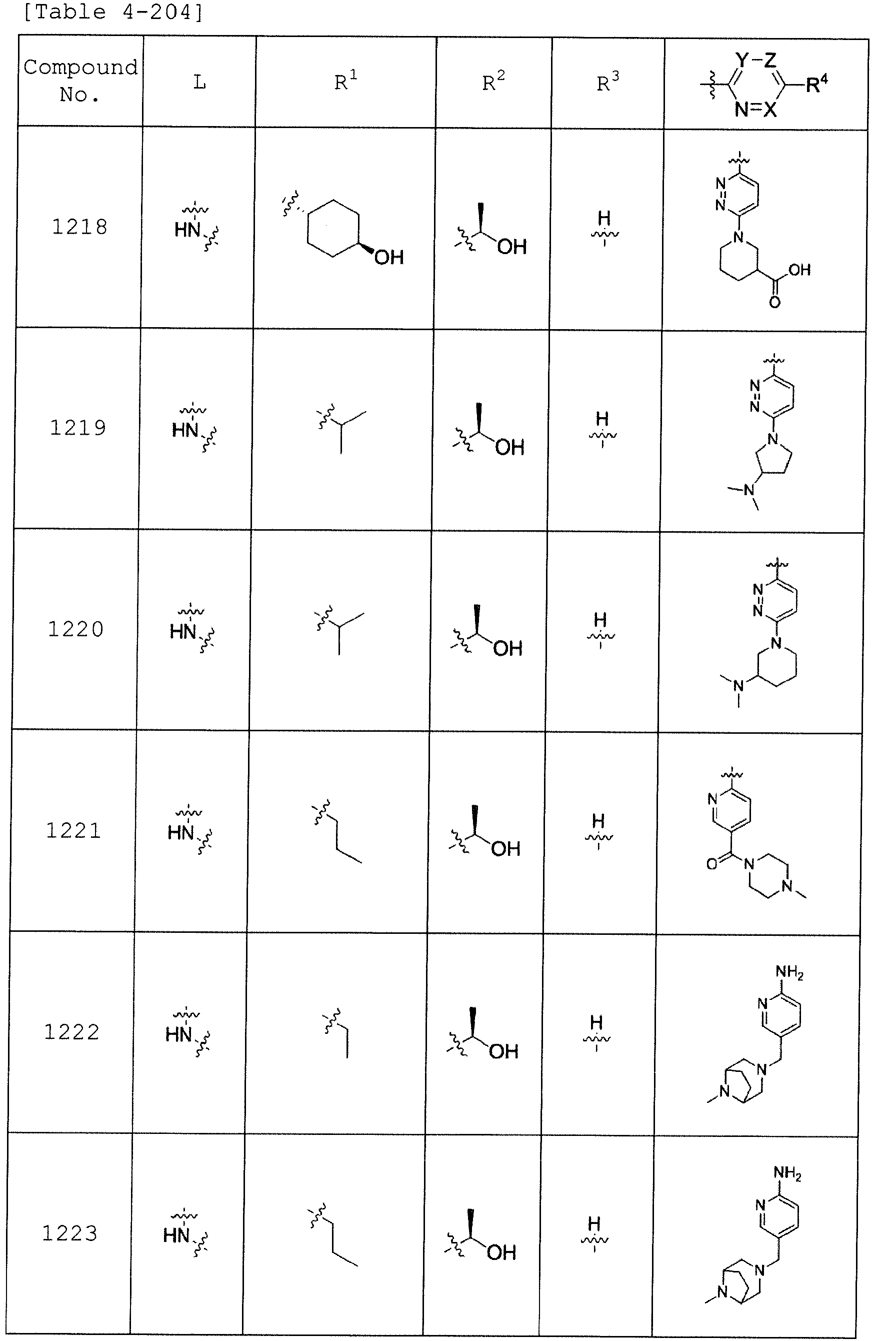

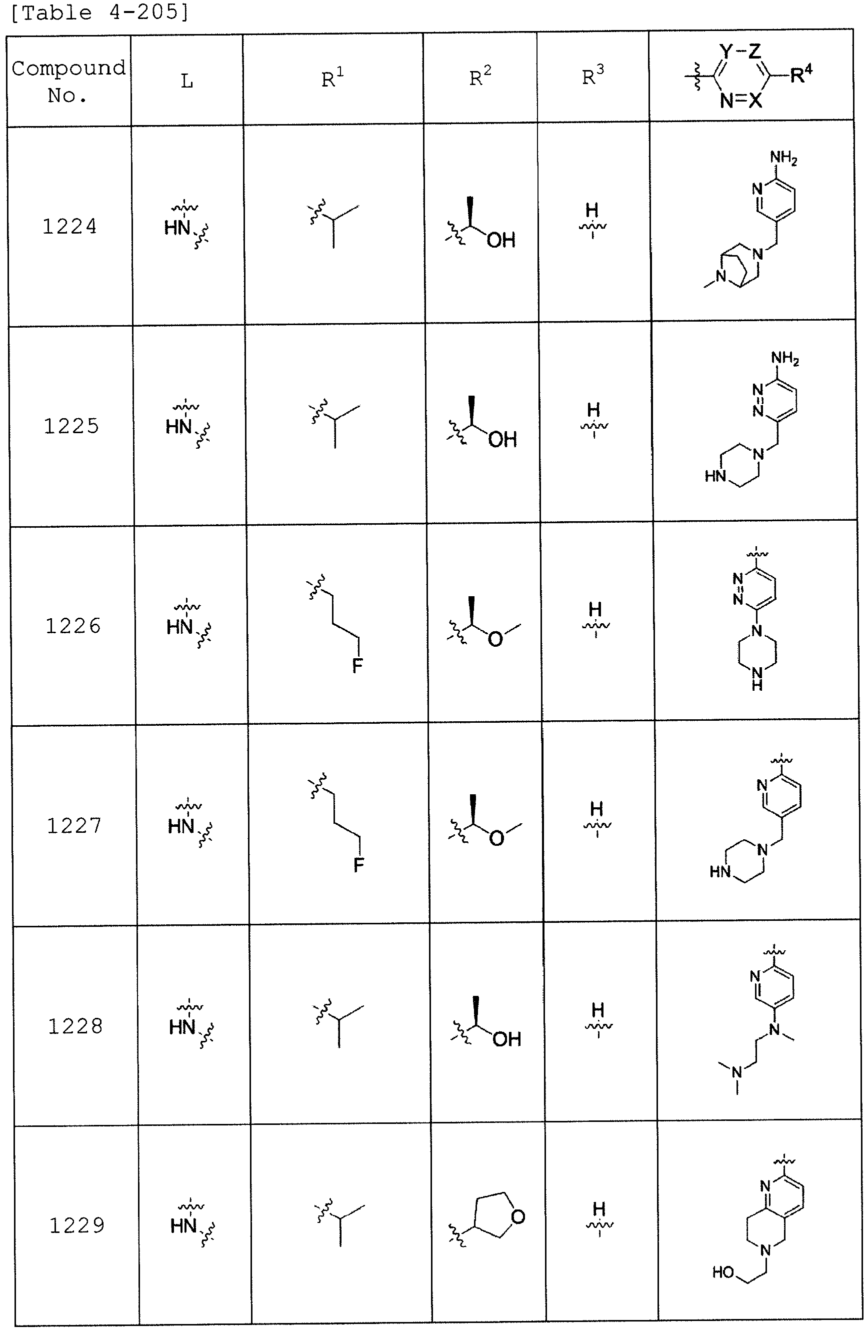

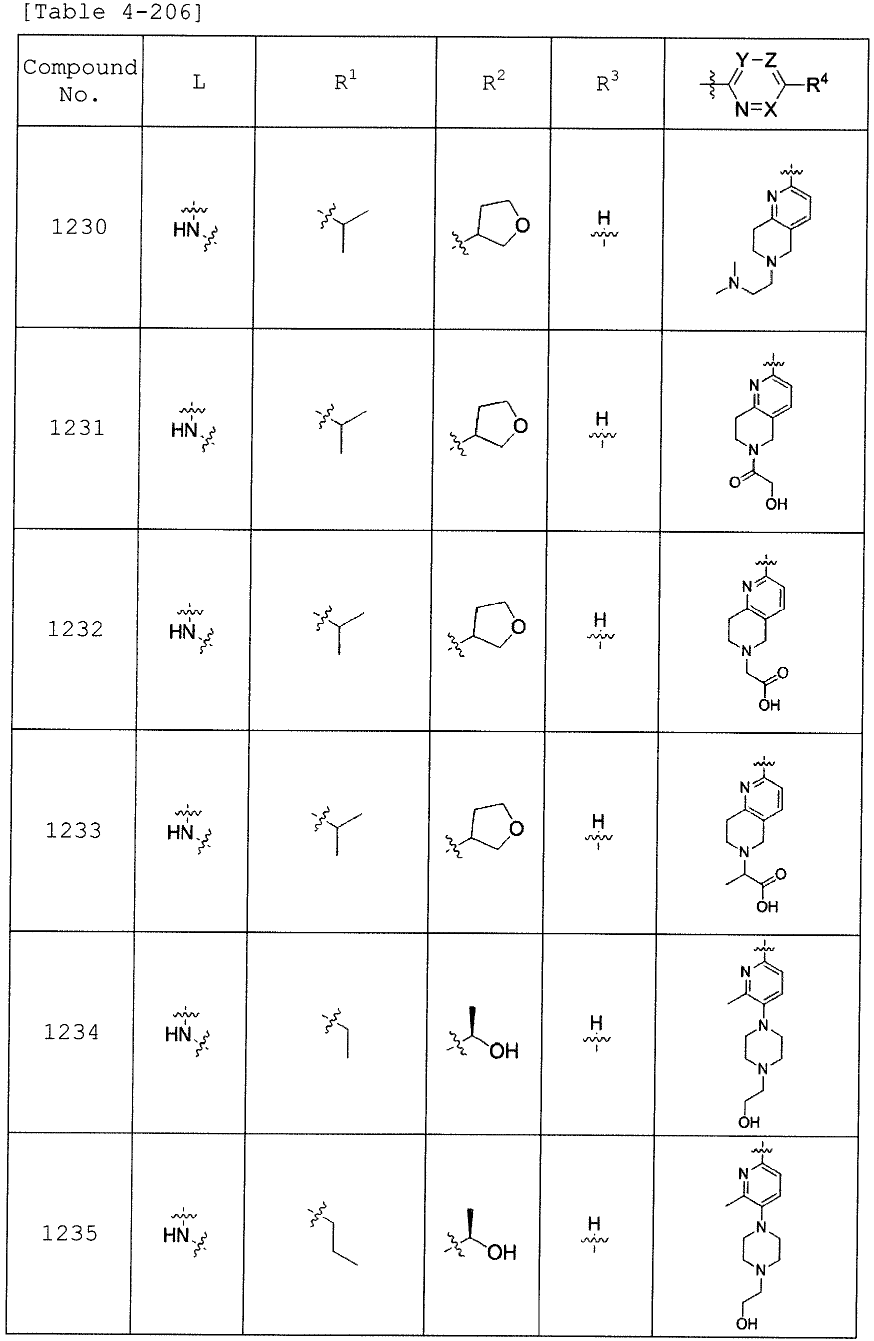

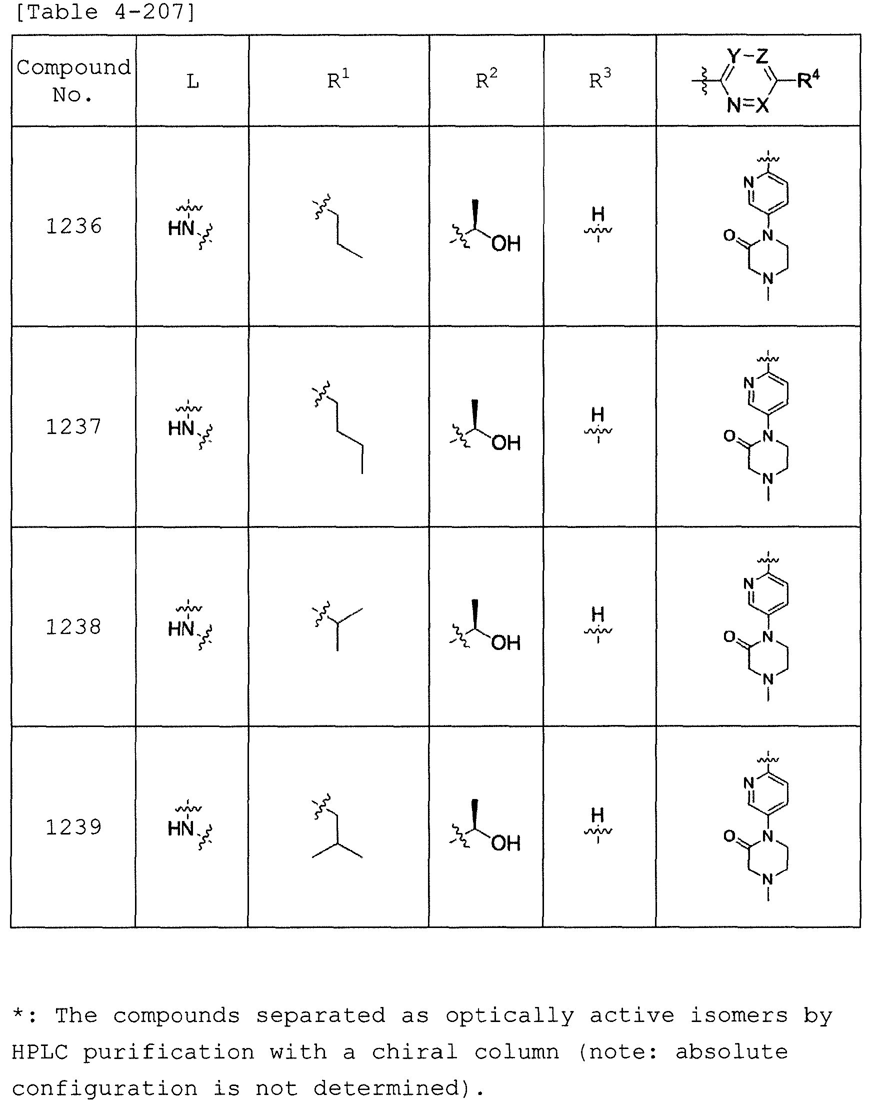

### Abbrebiations:

DEA: diethylamine, TEA = triethylamine, DCM: dichloromethane, IPA: isopropylalcohol
(1) Compound 581: DAICEL CHIRALPAK AD 5µm, n-hexane/MeOH/IPA, RT = 12.31 min.: optically active isomer with shorter retention time.
(1) Compound 582: DAICEL CHIRALPAK AD 5µm, n-hexane/MeOH/IPA, RT = 15.35 min.: optically active isomer with longer retention time.
(2) Compound 671: CHIRALPAK IA-3µm, 100%EtOH, RT= 4.388min.: optically active isomer with shorter retention time.
(2) Compound 672: CHIRALPAK IA-3µm, 100%EtOH, RT= 6.490 min.: optically active isomer with longer retention time.
(3) Compound 689: DAICEL CHIRALPAK AD-H 5µm, n-hexane/EtOH, RT = 17.61 min.: optically active isomer with shorter retention time.
(3) Compound 690: DAICEL CHIRALPAK AD-H 5µm, n-hexane/EtOH, RT = 20.71 min.: optically active isomer with longer retention time.
(4) Compound 704: CHIRALPAK AD-3µm, Hexane (0.2%TEA) /EtOH=50:50, RT = 6.446 min.: optically active isomer with longer retention time.
(4) Compound 705: CHIRALPAK AD-3µm, Hexane (0.2%TEA)/EtOH=50:50, RT = 4.679 min.: optically active isomer with shorter retention time.
(5) Compound 713: CHIRALPAK IA-3µm, 100%MeOH (0.1%DEA), RT = 3.628 min.: optically active isomer with longer retention time.
(5) Compound 714: CHIRALPAK IA-3µm, 100%MeOH (0.1%DEA), RT = 2.533 min.: optically active isomer with shorter retention time.
(6) Compound 724: CHIRALPAK AD-3, Hexane (0.1%DEA)/EtOH = 50:50, RT = 4.32 min.: optically active isomer with shorter retention time.
(6) Compound 725: CHIRALPAK AD-3, Hexane (0.1%DEA)/EtOH = 50:50, RT = 5.06 min.: optically active isomer with longer retention time.
(7) Compound 729: CHIRALPAK IA-3, 0.46*5cm; 3µm, Hexane (0.1%IPA) /EtOH = 50:50, RT = 4.01 min.: optically active isomer with longer retention time.
(7) Compound 730: CHIRALPAK IA-3, 0.46*5cm; 3µm, Hexane (0.1%IPA) /EtOH = 50:50, RT = 1.68 min.: optically active isomer with shorter retention time.
(8) Compound 735: DAICEL CHIRALPAK AD-H 5µm, Hexane/IPA = 90/10, RT = 9.18 min.: optically active isomer with shorter retention time.
(8) Compound 736: DAICEL CHIRALPAK AD-H 5µm, Hexane/IPA = 90/10, RT = 10.65 min.: optically active isomer with longer retention time.
(9) Compound 773: DAICEL CHIRALPAK IA 5µm, MeOH/IPA = 6/4, RT = 17.90 min.: optically active isomer with shorter retention time.
(9) Compound774: DAICEL CHIRALPAK IA 5µm, MeOH/IPA = 6/4, RT = 21.84 min.: optically active isomer with longer retention time.
(10) Compound 775: DAICEL CHIRALPAK IA 5µm, Hexane/MeOH/IPA = 6/1/1, RT = 10.25 min.: optically active isomer with shorter retention time.
(10) Compound776: DAICEL CHIRALPAK IA 5µm, Hexane/MeOH/IPA = 6/1/1, RT = 14.71 min.: optically active isomer with longer retention time.
(11) Compound 809: DAICEL CHIRALPAK AD-H 5µm, 4.6*250mm, n-Hexane/EtOH/MeOH = 70/15/15, RT = 21.62 min.: optically active isomer with shorter retention time.
(11) Compound 810: DAICEL CHIRALPAK AD-H 5µm, 4.6*250mm, n-Hexane/EtOH/MeOH = 70/15/15, RT = 25.87 min.: optically active isomer with longer retention time.
(12) Compound 865: CHIRALPAK IA-3; 0.46*5cm; 3µm; Hexane (0.1%DEA) /EtOH = 70:30, RT = 4.81 min.: optically active isomer with shorter retention time.
(12) Compound 866: CHIRALPAK IA-3; 0.46*5cm; 3µm; Hexane (0.1%DEA)/EtOH = 70:30, RT = 5.48 min.: optically active isomer with longer retention time.
(13) Compound 869: CHIRALPAK IA-3; 0.46*5cm; 3µm; Hexane (0.1%DEA) /EtOH = 60:40, RT = 2.27 min.: optically active isomer with shorter retention time.
(13) Compound 870: CHIRALPAK IA-3; 0.46*5cm; 3µm; Hexane (0.1%DEA) /EtOH = 60:40, RT = 2.93 min.: optically active isomer with longer retention time.
(14) Compound 875: DAICEL CHIRALPAK AD-H 5µm, Hexane/MeOH/EtOH = 80/10/10, RT = 22.30 min.: optically active isomer with shorter retention time.
(14) Compound 876: DAICEL CHIRALPAK AD-H 5µm, Hexane/MeOH/EtOH = 80/10/10, RT = 29.24 min.: optically active isomer with longer retention time.
(15) Compound 938: DAICEL CHIRALPAK AD-H 5µm, (100%EtOH+0.1%DEA), RT = 33.58 min.: optically active isomer with shorter retention time.
(15) Compound939: DAICEL CHIRALPAK AD-H 5µm, (100%EtOH+0.1%DEA), RT = 42.68 min.: optically active isomer with longer retention time.
(16) Compound 940: DAICEL CHIRALPAK AD-H 5µm, (Hexane/MeOH/EtOH +0.1%DEA = 6/2/2), RT = 20.23 min.: optically active isomer with shorter retention time.
(16) Compound 941: DAICEL CHIRALPAK AD-H 5µm, (Hexane/MeOH/EtOH +0.1%DEA = 6/2/2), RT = 22.07 min.: optically active isomer with longer retention time.
(17) Compound 942: DAICEL CHIRALPAK AD-H 5µm, (Hexane/MeOH/EtOH +0.1%DEA = 6/2/2), RT = 13.99 min.: optically active isomer with shorter retention time.
(17) Compound 943: DAICEL CHIRALPAK AD-H 5µm, (Hexane/MeOH/EtOH +0.1%DEA = 6/2/2), RT = 15.66 min.: optically active isomer with longer retention time.
(18) Compound 989: CHIRALPAK IA, n-Hexane/EtOH = 70/30, RT = 13.32 min.: optically active isomer with shorter retention time.
(18) Compound 990: CHIRALPAK IA, n-Hexane/EtOH = 70/30, RT = 16.69 min.: optically active isomer with longer retention time.
(19) Compound 992: CHIRALPAK IA-3: 0.46*5cm; 3µm, Hexane (0.1%DEA) /EtOH = 50:50, 1.5ml/min, RT = 1.87 min.: optically active isomer with shorter retention time.
(19) Compound 993: CHIRALPAK IA-3: 0.46*5cm; 3µm, Hexane (0.1%DEA) /EtOH = 50:50, 1.5ml/min, RT = 3.56 min.: optically active isomer with longer retention time.
(20) Compound 1025: CHIRALPAK IA, 0.46^{*}25cm; 5µm, MeOH (0.1%DEA)/DCM = 75:25, 1.0mL/min., RT = 6.597 min.: optically active isomer with shorter retention time.
(20) Compound 1026: CHIRALPAK IA, 0.46^{*}25cm; 5µm, MeOH (0.1%DEA)/DCM = 75:25, 1.0mL/min., RT = 8.199 min.: optically active isomer with longer retention time.
(21) Compound 1034: CHIRALPAK AS-3, 0.46*10cm; 3µm, Hexane (0.1%DEA) /(MeOH/EtOH = 1:1) = 70:30, 1.0mL/min., RT = 6.23 min.: optically active isomer with shorter retention time.
(21) Compound 1035: CHIRALPAK AS-3, 0.46*10cm; 3µm, Hexane (0.1%DEA) / (MeOH/EtOH = 1:1) = 70:30, 1.0mL/min., RT = 9.94 min.: optically active isomer with longer retention time.
(22) Compound 1036: CHIRALPAK IC-3, 0.46*10cm; 3µm, DCM (0.1%DEA) /MeOH = 20:80, 1.0mL/min., RT = 5.10 min.: optically active isomer with shorter retention time.
(22) Compound 1037: CHIRALPAK IC-3, 0.46*10cm; 3µm, DCM (0.1%DEA)/MeOH = 20:80, 1.0mL/min., RT = 5.95 min.: optically active isomer with longer retention time.
(23) Compound 1066: CHIRALPAK IC-3, 0.46*10cm; 3µm, MeOH (0.1%DEA)/DCM = 95:5, 1.0ml/min., RT = 5.421 min.: optically active isomer with shorter retention time.
(23) Compound 1067: CHIRALPAK IC-3, 0.46*10cm; 3µm, MeOH (0.1%DEA)/DCM = 95:5, 1.0ml/min., RT = 6.00 min.: optically active isomer with longer retention time.
(24) Compound 1068: CHIRALPAK IC, 0.46^{*}25cm; 5µm, MeOH (0.1%DEA)/DCM = 90:10, 1.0ml/min., RT = 17.429 min.: optically active isomer with shorter retention time.
(24) Compound 1069: CHIRALPAK IC, 0.46*25cm; 5µm, MeOH (0.1%DEA)/DCM = 90:10, 1.0ml/min., RT = 20.57 min.: optically active isomer with longer retention time.
(25) Compound 1088: CHIRALPAK IA, n-Hexane/EtOH/MeOH = 50/25/25, 1.0 mL/min., RT = 15.21 min.: optically active isomer with shorter retention time.
(25) Compound 1089: CHIRALPAK IA, n-Hexane/EtOH/MeOH = 50/25/25, 1.0 mL/min., RT = 18.59 min.: optically active isomer with longer retention time.

*: Compound separated as diastereomers by reversed phase HPLC purification (note: absolute configuration is not determined).
(1) Compound 831: C-18: RT = 1.67 min.: diastereomer with shorter retention time.
(1) Compound 832: C-18: RT = 1.71 min.: diastereomer with longer retention time.

*: Compound represented its relative configurations of two substituents.
Compound#: 577, 607, 608, 609, 610, 618, 619, 621, 622, 645, 646, 647, 673, 708, 739, 742, 799, 801, and 864.

**[Table 5-1]**

| Compound No. | NMR | LC/MS (M+H) ⁺ | Exact Mass |
|---|---|---|---|
| 1 | 1H-NMR (DMSO-D6) δ: 10.33 (1H, s), 9.32 (1H, s), 8.74 (1H, br s), 8.09-8.11 (2H, m), 7.59-7.62 (1H, m), 7.22 (1H, s), 6.96 (1H, t, J = 51.1 Hz), 6.67-6.94 (2H,m), 4.38-4.43 (1H, m), 3.36-3.37 (4H, br m), 3.28 (4H, br s), 2.05-2.10 (2H, m), 1.60-1.67 (2H, m), 1.71-1.76 (2H, m), 1.60-1.67 (4H, m) . | 441.42 | 440.22 |
| 2 | 1H-NMR (CD3OD) δ: 9.45 (1H, s), 8.14(1H, dd, J = 9.60, 2.68 Hz), 8.00 (1H, d, J = 2.76Hz), 7.72 (1H, d, J = 23.5Hz), 7.39 (1H, s), 6.64 (1H, t, J = 55.6 Hz), 4.57 (1H, br m), 3.69 (1H, dd, J = 12.2, 3.44 Hz), 3.51-3.54 (3H, m), 3.35-3.47 (6H, m), 3.08-3.15 (2H, m), 2.14-2.21 (2H, br m), 1.94-1.96 (2H, br m). | 456.38 | 455.24 |
| 3 | 1H-NMR (CD3OD) δ: 9.37 (1H, s), 8.16 (1H, d, J = 9.1 Hz), 7.92 (1H, br, s), 7.63 (1H, d, J = 9.1 Hz), 7.24 (1H, s), 6.58 (1H, t, J = 55.7 Hz), 4.41-4.45 (1H, m), 3.52 (4H, br s), 3.44 (4H, br, s), 2.65 (2H, s), 1.35 (6H, d, J = 6.5 Hz). | 415.36 | 414.21 |
| 4 | 1H-NMR (CD3OD) δ: 9.41 (1H, s), 8.22(1H, dd, J = 9.44, 2.72 Hz), 7.94 (1H, d, J = 2.68Hz), 7.60 (1H, d, J = 9.56 Hz), 7.30 (1H, s), 6.60 (1H, t, J = 55.7 Hz), 4.36-4.41 (1H, m), 3.94-4.04 (2H, br m), 3.58-3.64 (2H, m), 3.53-3.58 (4H, m), 3.39-3.45 (4H, m), 2.10-2.13 (2H, br m), 1.69-1.79 (2H, m), 1.14 (2H, d, J = 6.04Hz). | 457.39 | 456.22 |
| 6 | 1H-NMR (CD3OD) δ: 9.05 (1H, s), 8.10 (1H, d, J = 9.1 Hz), 8.00 (1H, d, 2.9 Hz), 7.51 (1H, dd, J = 9.1, 2.9 Hz), 6.90 (1H, s), 4.59 (2H, s), 4.48-4.52 (1H, m), 3.15-3.16 (4H, m), 3.01-3.04 (4H, m), 2.11-2.14 (2H, m), 1.80-1.83 (2H, m), 1.70-1.73 (2H, m), 1.61-1.64 (2H, m). | 421.2 | 420.24 |
| 7 | | 416.22 | 415.19 |

**[Table 5-2]**

| Compound No. | NMR | LC/MS (M+H) ⁺ | Exact Mass |
|---|---|---|---|
| 8 | 1H-NMR (CD3OD) δ: 9.06 (1H, s), 8.15 (1H, d, J = 9.1 Hz), 8.02 (1H, d, 2.9 Hz), 7.55 (1H, dd, J = 9.1, 2.9 Hz), 6.91 (1H, s), 4.58 (5H, s), 4.48-4.50 (1H, m),3.20-3.22 (4H, m), 2.13-2.15 (2H, m), 1.92 (3H, s), 1.62-1.82 (6H, m), 1.50 (3H, d, J = 6.6 Hz). | 435.21 | 434.25 |
| 9 | 1H-NMR (CD3OD) δ: 9.05 (1H, s), 8.14(1H, br s), 8.01 (1H,br s), 7.53 (1H, d, J = 9.0 Hz), 6.90 (1H, s), 4.72-4.77 (1H, m), 4.35-4.42 (1H, m), 3.18-3.20 (4H, br m), 3.07 (4H, br s), 1.49 (3H, d, J = 6.52 Hz), 1.33 (6H, dd, J = 6.48, 2.24 Hz) | 409.35 | 408.24 |
| 12 | 1H-NMR (CD3OD) δ: 9.17 (1H, s), 7.98-8.01(2H, br m), 7.78-7.80(1H, br m), 7.01 (1H, s), 4.77-4.86 (1H, m), 4.36 (1H, br m), 4.00-4.03 (2H, br m), 3.63 (2H, apparent t, J = 11.3 Hz), 3.43-3.44 (8H, br m), 2.11-2.14 (2H, br m), 1.70-1.73 (2H, br m), 1.50 (3H, d, J = 6.52 Hz) | 451.31 | 450.25 |
| 13 | 1H-NMR (CD3OD) δ: 9.36 (1H, s), 8.12(1H, dd, J = 9.52, 2.84 Hz), 7.93 (1H, d, J = 2.72Hz), 7.67 (1H, d, J = 9.48 Hz), 7.26 (1H, s), 6.60 (1H, t, J = 55.76 Hz), 3.43-3.52 (8H, m), 1.59 (9H, s) . | 429.32 | 428.22 |
| 15 | 1H-NMR (CD3OD) δ: 9.40 (1H, s), 8.16(1H, dd, J = 9.48, 2.84 Hz), 7.97 (1H, d, J = 2.88Hz), 7.66 (1H, d, J = 9.52 Hz), 7.30 (1H, s), 6.61 (1H, t, J = 55.72 Hz), 3.83-3.85 (2H, m), 3.69-3.72 (2H, m), 3.42-3.53 (8H, m), 3.42 (3H, s). | 431.27 | 430.20 |
| 16 | 1H-NMR (CD3OD) δ: 9.05 (1H, s), 8.14(1H, br s), 8.01 (1H,br s), 7.53 (1H, d, J = 9.0 Hz), 6.90 (1H, s), 4.72-4.77 (1H, m), 4.35-4.42 (1H, m), 3.18-3.20 (4H, br m), 3.07 (4H, br s), 1.49 (3H, d, J = 6.52 Hz), 1.33 (6H, dd, J = 6.48, 2.24 Hz) | | |

**[Table 5-3]**

| Compound No. | NMR | LC/MS (M+H) ⁺ | Exact Mass |
|---|---|---|---|
| 17 | 1H-NMR (CD3OD) δ: 9.05 (1H, s), 8.14(1H, br s), 8.01 (1H,br s), 7.53 (1H, d, J = 9.0 Hz), 6.90 (1H, s), 4.72-4.77 (1H, m), 4.35-4.42 (1H, m), 3.18-3.20 (4H, br m), 3.07 (4H, br s), 1.49 (3H, d, J = 6.52 Hz), 1.33 (6H, dd, J = 6.48, 2.24 Hz) | | |
| 18 | 1H-NMR (CD3OD) δ: 9.42 (1H, s), 8.20(1H, dd, J = 9.64, 2.68 Hz), 7.96 (1H, d, J = 2.48Hz), 7.60 (1H, d, J = 9.56 Hz), 7.36 (1H, s), 6.66 (1H, t, J = 55.64 Hz), 3.43-3.55 (8H, m), 2.98-3.00(1H, m), 0.90-0.93(2H, m), 0.70-0.72(2H, m). | 413.32 | 412.19 |
| 19 | 1H-NMR (DMSO) δ: 9.98 (1H, s), 9.26 (1H, s), 8.28(1H, d, J = 9.04 Hz), 8.04 (1H, d, J = 2.84Hz), 7.43 (1H, dd, J = 9.08, 2.88 Hz), 7.16-7.18 (2H, br m), 6.81 (1H, t, J = 55.64 Hz), 3.05-3.06 (7H, m), 2.85-2.87 (4H, m), 1.23(1H, s). | 387.22 | 386.18 |
| 20 | 1H-NMR (DMSO) δ: 9.99 (1H, s), 9.26 (1H, s), 8.21 (1H, d, J = 9.08 Hz), 8.03 (1H, d, J = 2.80Hz), 7.45 (1H, dd, J = 9.04, 2.92 Hz), 7.17 (1H, s), 7.07 (1H, t, J = 5.72), 6.79 (1H, t, J = 55.56 Hz), 3.56(1H, q, J =6.6 Hz), 3.05-3.07 (4H, m), 2.85-2.87 (4H, m), 1.25(3H, t, 7.08 Hz). | 401.32 | 400.19 |
| 21 | 1H-NMR (CD3OD) δ: 9.07 (1H, s), 8.13 (1H, d, J = 9.08 Hz), 7.99 (1H, d, J = 2.84Hz), 7.54 (1H, dd, J = 9.08, 2.96 Hz), 6.98 (1H, s), 4.79-4.82 (1H, m), 3.15-3.21 (4H, m), 3.02-3.04 (3H, m), 2.93-2.95 (1H, m), 2.86-2.89 (1H, m), 1.51 (1H, d, J = 6.52 Hz), 0.85-0.88 (2H, m), 0.62 (2H, br s). | 407.37 | 406.22 |
| 22 | 1H-NMR (CD3OD) δ: 9.07 (1H, s), 8.30 (1H, d, J = 9.16 Hz), 8.03-8.09 (2H, br m), 7.95 (1H, d, J = 7.48 Hz), 7.58-7.59 (1H, br m), 7.50-7.54 (1H, br m), 6.91 (1H, s), 4.75-4.79 (1H, m), 3.30 (8H, br s), 3.12 (3H, s), 1.50 (3H, d, J = 6.48 Hz). | 381.24 | 380.21 |

**[Table 5-4]**

| Compound No. | NMR | LC/MS (M+H) ⁺ | Exact Mass |
|---|---|---|---|
| 23 | 1H-NMR (DMSO-d6) δ: 9.73 (1H, s), 9.19 (1H, s), 8.24 (1H, d, J = 9.04 Hz), 8.03 (1H, d, J = 2.72 Hz), 7.45 (1H, dd, J = 9.12, 2.84 Hz), 6.91 (1H, s), 6.79 (1H, t, J = 5.64 Hz), 5.16 (1H, Br m), 4.60-4.62 (1H, m), 3.54-3.57 (2H, m), 3.08-3.10 (4H, m), 2.91-2.93 (4H, m), 1.38 (3H, d, 6.48 Hz), 1.25 (3H, t, J = 7.08 Hz). | 395.33 | I 394.22 |
| 24 | 1H-NMR (DMSO-d6) δ: 9.86 (1H, s), 9.17 (1H, s), 8.01-8.06 (2H, m), 7.45 (1H, d, J = 9.24 Hz), 6.95 (1H, s), 6.40 (1H, s), 5.17 (1H, d, J = 4.72 Hz), 4.62-4.64 (1H, m), 3.05-3.06 (4H, m), 2.87 (4H, br s), 1.53 (9H, s), 1.40 (3H, d, 6.40 Hz), 1.05 (2H, br s) . | 423.3 | 422.25 |
| 25 | 1H-NMR (CD3OD) δ: 8.81 (1H, s), 8.37 (1H, d, J = 8.28 Hz), 7.98 (1H, br s), 7.46 (1H, d, J = 6.68 Hz), 6.29 (1H, br s), 4.67 (1H, br s), 4.41-4.51 (2H, br m), 4.28 (1H, br s), 3.75 (2H, br s), 3.12 (4H, br s), 2.99 (4H, br s), 1.50 (3H, br s). | 423.4 | 422.22 |
| 26 | 1H-NMR (DMSO-d6) δ: 9.97 (1H, s), 9.33 (1H, s), 8.79 (1H, s), 8.24 (1H, d, J = 7.6 Hz), 7.99-8.21 (3H, m), 7.55 (1H, dd, J = 12.4, 4.0 Hz), 7.36-7.42 (2H, m), 7.25 (1H, s), 7.00-7.05 (1H, m), 5.35 (1H, d, J = 6.0 Hz), 4.70-4.76 (1H, m), 3.06-3.09 (4H, m), 2.85-2.95 (4H, m), 1.47 (3H, d, J = 8.8 Hz). | 443.2 | 442.22 |
| 27 | 1H-NMR (DMSO-d6) δ: 12.35 (1H, s), 10.05 (1H, s), 9.33 (1H, s), 8.79 (1H, s), 8.07 (2H, br s), 7.73 (1H, s), 7.50 (1H, d, J = 5.6 Hz), 7.22 (1H, s), 7.00 (1H, s), 5.33 (1H, s), 4.75 (1H, br s), 3.236-3.26 (4H, m), 2.97 (4H, br s), 1.48 (3H, d, J = 8.8 Hz). | 433.2 | 432.21 |

**[Table 5-5]**

| Compound No. | NMR | LC/MS (M+H) ⁺ | Exact Mass |
|---|---|---|---|
| 28 | 1H-NMR (DMSO-d6) δ: 9.84 (1H, s), 9.20 (1H, s), 8.02-8.10 (2H, m), 7.42 (1H, d, J = 12 Hz), 6.98 (1H, s), 6.56 (1H, t, J = 10.4 Hz), 5.18 (1H, t, J = 5.6 Hz), 4.60-4.63 (1H, m), 4.28-4.35 (1H, m), 3.51-3.57 (2H, m), 3.33-3.37 (3H, m), 3.03-3.06 (4H, m), 2.84-2.87 (4H, m), 1.44 (3H, d, J = 8.8 Hz), 1.30 (3H, d, J = 8.8 Hz). | 439.3 | 438.25 |
| 29 | 1H-NMR (DMSO-d6) δ: 9.89 (1H, s), 9.18 (1H, s), 8.03-8.08 (2H, m), 7.41 (1H, dd, J = 12.0, 3.6 Hz), 6.97 (1H, s), 6.67 (1H, s), 5.18 (1H, d, J = 6.0 Hz), 4.58-4.64 (1H, m), 3.57-3.60 (2H, m), 3.28-3.38 (3H, m), 3.02-3.06 (4H, m), 2.84-2.87 (4H, m), 1.51 (6H,s), 1.39 (3H, d, J = 8.4 Hz). | 453.4 | 452.26 |
| 30 | 1H-NMR (DMSO-d6) δ: 10.15 (1H, s), 9.29 (1H, s), 8.05-8.08 (2H, m), 7.44 (1H, dd, J = 12.0, 3.6 Hz), 7.21 (1H, s), 6.62-6.98 (2H, m), 4.34-4.38 (1H, m), 3.55-3.58 (2H, m), 3.22-3.40 (3H, m), 3.12-3.16 (4H, m), 2.91-2.97 (4H, m),1.28 (3H, d, J = 8.8 Hz). | 445.2 | 444.22 |
| 31 | 1H-NMR (DMSO-d6) δ: 10.17 (1H, s), 9.26 (1H, s), 8.00-8.06 (2H, m), 7.42 (1H, dd, J = 12.0, 3.6 Hz), 7.19 (1H, s), 6.62-6.99 (2H, m), 3.56 (2H, s), 3.29-3.38 (3H, m), 3.04-3.07 (4H, m), 2.84-2.87 (4H, m), 1.51 (6H, s). | 459.15 | 458.24 |
| 32 | 1H-NMR (DMSO-d6) δ: 12.56 (1H, br s), 9.69 (1H, s), 9.25 (1H, s), 8.78 (1H, s), 8.25-8.28 (2H, m), 8.02-8.03 (1H, m), 7.50 (1H, d, J = 5.6 Hz), 7.09 (1H, s), 5.26 (1H, d, J = 6 Hz), 4.75 (1H, br s), 3.05-3.08 (4H, m), 2.86-2.89 (4H, m), 1.45 (3H, d, J = 8.4 Hz). | 433.1 | 432.21 |

**[Table 5-6]**

| Compound No. | NMR | LC/MS (M+H)⁺ | Exact Mass |
|---|---|---|---|
| 33 | 1H-NMR (CD3OD) δ: 9.45 (1H, s), 8.82 (1H, d, J = 9.56 Hz), 8.17-8.20 (2H, m), 7.35 (1H, s), 6.60 (1H, t, J = 55.56 Hz), 5.47 (1H, dd, J = 9.80, 2.24 Hz), 5.09 (1H, t, J = 12.64 Hz), 4.89-4.90 (1H, m), 4.29 (1H, dd, J = 12.00, 3.08 Hz), 3.86 (1H, dd, J = 12.04, 1.72 Hz), 3.30-3.34 (4H, m), 3.07-3.10 (4H, m). | 429.25 | 428.19 |
| 34 | 1H-NMR (DMSO-d6) δ: 9.96 (1H, s), 9.18 (1H, s), 8.04 (1H, d, J = 9.2 Hz), 7.98 (1H, d, J = 2.8 Hz), 7.43 (1H, dd, J = 8.8, 2.8 Hz), 7.046 (1H, s), 6.47 (1H, d, J = 7.6 Hz), 4.16-4.21 (1H, m), 3.09 (6H, br s), 2.99 (4H, br s), 2.93 (9H, br s), 1.23 (6H, d, J = 6.8 Hz). | 436.2 | 435.25 |
| 36 | 1H-NMR (CD3OD) δ: 9.14 (1H, s), 8.25 (1H, d, J = 2.9 Hz), 7.59 (1H, dd, J = 8.9, 3.0 Hz), 7.49 (1H, d, J = 8.9 Hz), 7.09 (1H, s), 6.21-6.48 (2H, m), 4.91-4.96 (1H, m), 4.67-4.75 (2H, m), 3.50-3571 (4H, m), 3.43-3.46 (4H, m), 1.56 (1H, d, J = 6.5 Hz). | 431.35 | 430.20 |
| 37 | 1H-NMR (DMSO-d6) δ: 9.92 (1H, s), 9.20 (1H, s), 8.64 (1H, s), 8.15 (1H, d, J = 9.3 Hz), 8.08 (1H, d, J = 2.9 Hz), 7.53 (1H, dd, J = 9.3, 2.9 Hz), 6.87 (1H, s), 6.38 (1H, d, J = 7.8 Hz), 4.25 (2H, td, J = 12.4, 6.5 Hz), 3.31 (10H, s), 3.26 (4H, s), 3.23 (4H, d, J = 5.4 Hz), 1.38 (3H, d, J = 6.3 Hz), 1.29 (6H, dd, J = 6.3, 3.4 Hz). | 423.2 | 422.25 |
| 38 | 1H-NMR (DMSO-d6) δ: 9.92 (1H, s), 9.20 (1H, s), 8.64 (1H, s), 8.15 (1H, d, J = 9.3 Hz), 8.08 (1H, d, J = 2.9 Hz), 7.53 (1H, dd, J = 9.3, 2.9 Hz), 6.87 (1H, s), 6.38 (1H, d, J = 7.8 Hz), 4.25 (2H, td, J = 12.4, 6.5 Hz), 3.31 (10H, s), 3.26 (4H, s), 3.23 (4H, d, J = 5.4 Hz), 1.38 (3H, d, J = 6.3 Hz), 1.29 (6H, dd, J = 6.3, 3.4 Hz) . | | |

**[Table 5-7]**

| Compound No. | NMR | LC/MS (M+H) ⁺ | Exact Mass |
|---|---|---|---|
| 39 | 1H-NMR (DMSO-d6) δ: 9.92 (1.0H, s), 9.20 (1.0H, s), 8.64 (1.1H, s), 8.15 (1.2H, d, J = 9.3 Hz), 8.08 (1.1H, d, J = 2.9 Hz), 7.53 (1.0H, dd, J = 9.3, 2.9 Hz), 6.87 (1.0H, s), 6.38 (1.0H, d, J = 7.8 Hz), 4.25 (2.1H, td, J = 12.4, 6.5 Hz), 3.31 (9.8H, s), 3.26 (3.6H, s), 3.23 (4.5H, d, J = 5.4 Hz), 1.38 (3.1H, d, J = 6.3 Hz), 1.29 (6.7H, dd, J = 6.3, 3.4 Hz). | 423.25 | 422.25 |
| 40 | 1H-NMR (DMSO-d6) δ: 9.80 (1H, s), 9.26 (1H, s), 8.26 (1H, d, J = 12.0 Hz), 8.03 (1H, d, J = 4.0 Hz), 7.40 (1H, dd, J = 12.0, 4.0 Hz), 7.20 (1H, d, J = 9.2 Hz), 7.12 (1H, s), 5.34 (1H, d, J = 6.4 Hz), 4.62-4.66 (1H, m), 4.38-4.45 (2H, br m), 3.03-3.06 (4H, m), 2.84-2.87 (4H, m), 1.38 (3H, d, J = 8.8 Hz). | 449.2 | 448.19 |
| 41 | 1H-NMR (DMSO-d6) δ: 9.82 (1H, s), 9.19 (1H, s), 8.13 (1H, d, J = 12.4 Hz), 8.01 (1H, d, J = 4.0 Hz), 7.42 (1H, dd, J = 12.4, 4.0 Hz), 6.97 (1H, s), 6.58 (1H, t, J = 10.4 Hz), 5.17 (1H, t, J = 6.4 Hz), 4.97-5.00 (1H, m), 4.59-4.62 (1H, m), 4.17-4.19 (1H, br m), 3.57-3.60 (1H, m), 3.03-3.06 (4H, m), 2.84-2.87 (4H, m), 1.38 (3H, dd, J = 8.4, 1.6 Hz), 1.25 (3H, d, J = 4.8 Hz). | 425.4 | 424.23 |
| 42 | 1H-NMR (DMSO-d6) δ: 10.05 (1H, s), 9.27 (1H, s), 8.04-8.10 (2H, m), 7.49 (1H, dd, J = 12.0, 4.0 Hz), 7.19 (1H, s), 6.79 (1H, t, J = 62.8 Hz), 6.61 (1H, s), 4.21-4.32 (1H, m), 3.14-3.17 (1H, m), 3.03-3.06 (4H, m), 2.46-2.50 (4H, m), 2.20 (3H, s), 1.30 (6H, d, J = 8.4 Hz). | 429.15 | 428.22 |
| 43 | 1H-NMR (DMSO-d6) δ: 10.08 (1H, s), 9.28 (1H, s), 8.06-8.12 (2H, m), 7.50 (1H, dd, J = 12.0, 4.4 Hz), 7.19 (1H, s), 6.79 (1H, t, J = 74.4 Hz), 6.61 (1H, s), 4.21-4.32 (1H, m), 3.75-3.79 (4H, m), 3.10-3.15 (4H, m), 1.30 (6H, d, J = 8.0 Hz). | 416 | 415.19 |

**[Table 5-8]**

| Compound No. | NMR | LC/MS (M+H) ⁺ | Exact Mass |
|---|---|---|---|
| 44 | 1H-NMR (DMSO-d6) δ: 9.82 (1H, s), 9.19 (1H, s), 8.08 (1H, d, J = 12.4 Hz), 8.01 (1H, d, J = 4.0 Hz), 7.46 (1H, dd, J = 12.8, 4.4 Hz), 6.88 (1H, s), 6.61 (1H, d, J = 10.0 Hz), 4.38 (2H, s), 4.25 (1H, br m), 3.40 (3H, s), 3.03-3.06 (4H, m), 2.84-2.87 (4H, m), 1.28 (6H, d, J = 8.8 Hz). | 409.2 | 408.24 |
| 45 | 1H-NMR (DMSO-d6) δ: 9.87 (1H, s), 9.17 (1H, s), 8.10 (1H, d, J = 12.4 Hz), 8.02 (1H, d, J = 4.0 Hz), 7.46 (1H, dd, J = 12.8, 4.4 Hz), 6.95 (1H, s), 6.87 (1H, s), 5.39 (1H, br m), 5.17 (1H, d, J = 6.0 Hz), 4.62 (1H, br m), 3.57 (1H, d, J = 6.4 Hz), 3.08-3.10 (4H, m), 2.93 (4H, br m), 1.46 (6H, s), 1.38 (3H, d, J = 8.8 Hz). | 439.2 | 438.25 |
| 46 | 1H-NMR (DMSO-d6) δ: 10.27 (1H, s), 9.36 (1H, s), 8.24-8.26 (2H, m), 7.76 (1H, m), 7.25 (1H, s), 6.72 (1H, t, J = 42.0 Hz), 6.71 (1H, m), 4.25-4.35 (1H, m), 3.17-3.20 (2H, m), 2.73-2.76 (3H, m), 1.81 (2H, m), 1.67 (2H, m), 1.34 (6H, d, J = 6.4 Hz). | 414.3 | 413.21 |
| 47 | 1H-NMR (DMSO-d6) δ: 10.32 (1H, s), 9.37 (1H, s), 8.25-8.30 (2H, m), 7.80 (1H, dd, J = 8.4, 2.4 Hz), 7.25 (1H, s), 6.74 (1H, t, J = 40.0 Hz), 6.70 (1H, m), 4.25-4.35 (1H, m), 3.48 (2H, s), 2.78 (4H, br m), 2.35-2.38 (4H, br m), 1.34 (6H, d, J = 6.4 Hz). | 429.4 | 428.22 |
| 48 | 1H-NMR (DMSO-d6) δ: 9.75 (1H, s), 9.22 (1H, s), 8.20 (1H, d, J = 9.2 Hz), 8.04 (1H, d, J = 2.8 Hz), 7.50 (1H, dd, J = 9.2, 3.2 Hz), 7.00 (1H, s), 6.75 (1H, d, J = 7.6 Hz), 5.19 (1H, d, J = 4.4 Hz), 4.60-4.66 (1H, br m), 3.06-3.09 (4H, m), 2.87-2.90 (4H, m), 2.36-2.41 (2H, m), 2.08-2.15 (2H, m), 1.76-1.80 (2H, m), 1.38 (3H, d, J = 8.8 Hz). | 421.1 | 420.24 |

**[Table 5-9]**

| Compound No. | NMR | LC/MS (M+H) ⁺ | Exact Mass |
|---|---|---|---|
| 49 | 1H-NMR (DMSO-d6) δ: 10.04 (1H, s), 9.27 (1H, s), 8.04-8.09 (2H, m), 7.49 (1H, d, J = 4.0 Hz), 7.18 (1H, s), 6.61 (1H, t, J = 70.8 Hz), 6.58 (1H, m), 4.41-4.45 (1H, m), 4.25-4.35 (1H, m), 3.53-3.57 (2H, m), 3.13-3.16 (4H, m), 2.56-2.60 (4H, m), 2.42-2.46 (2H, m), 1.29 (6H, d, J = 8.8 Hz). | 459.2 | 458.24 |
| 50 | 1H-NMR (DMSO-d6) δ: 10.01 (1H, s), 9.27 (1H, s), 8.04-8.07 (2H, m), 7.48 (1H, dd, J = 12.0, 4.8 Hz), 7.18 (1H, s), 6.79 (1H, t, J = 74.0 Hz), 6.58 (1H, m), 4.68 (1H, d, J = 5.6 Hz), 4.23-4.31 (1H, m), 3.63-3.64 (1H, m), 3.49-3.55 (2H, m), 2.86-2.90 (2H, m), 1.82-1.85 (2H, m), 1.49-1.52 (2H, m), 1.29 (6H, d, J = 8.8 Hz). | 430.2 | 429.21 |
| 51 | 1H-NMR (DMSO-d6) δ: 10.02 (1H, s), 9.26 (1H, s), 8.03-8.07 (2H, m), 7.47 (1H, dd, J = 12.4, 4.4 Hz), 7.18 (1H, s), 6.79 (1H, t, J = 74.4 Hz), 6.58 (1H, m), 4.35-4.39 (1H, m), 4.23-4.28 (1H, m), 3.64-3.68 (2H, m), 3.31-3.36 (2H, m), 2.60-2.72 (2H, m), 1.74-1.78 (2H, m), 1.47-1.49 (2H, m), 1.23-1.28 (11H, m). | 472.3 | 471.26 |
| 52 | 1H-NMR (DMSO-d6) δ: 9.88 (1H, s), 9.23 (1H, s), 8.00 (1H, d, J = 11.6 Hz), 7.69 (1H, d, J = 4.0 Hz), 7.06 (1H, dd, J = 12.0, 4.0 Hz), 6.78 (1H, t, J = 74.0 Hz), 6.56 (1H, d, 10.4 Hz), 4.25-4.27 (1H, m), 3.67-3.69 (1H, m), 3.43-3.47 (3H, m), 2.98-3.02 (1H, m), 2.11-2.17 (1H, m), 1.82-1.85 (1H, m), 1.29 (6H, d, J = 8.8 Hz). | 415.05 | 414.21 |

**[Table 5-10]**

| Compound No. | NMR | LC/MS (M+H)⁺ | Exact Mass |
|---|---|---|---|
| 53 | 1H-NMR (DMSO-d6) δ: 9.87 (1H, s), 9.23 (1H, s), 7.98 (1H, d, J = 9.2 Hz), 7.69 (1H, d, J = 2.8 Hz), 7.17 (1H, s), 7.06 (1H, dd, J = 9.2, 3.2 Hz), 6.78 (1H, t, J = 55.2 Hz), 6.56 (1H, d, 7.6 Hz), 4.98 (1H, d, 4.0 Hz), 4.42 (1H, br m), 4.26-4.27-3.69 (1H, m), 3.44-3.47 (1H, m), 3.31-3.36 (2H, m), 3.10-3.13 (1H, m), 2.06-2.08 (1H, m), 1.91-1.93 (1H, m), 1.30 (6H, d, J = 6.4 Hz). | 416.05 | 415.19 |
| 54 | 1H-NMR (DMSO-d6) δ: 9.81 (1H, d, J = 5.6 Hz), 9.28 (1H, s), 8.07 (1H, d, J = 12.4 Hz), 8.01 (1H, d, J = 3.6 Hz), 7.44 (1H, dd, J = 11.6, 3.6 Hz), 7.12 (1H, d, J = 5.6 Hz), 6.62 (1H, d, J = 12.8 Hz), 5.24 (1H, dd, J = 10.8, 6.0 Hz), 4.64 (1H, br m), 3.03-3.06 (4H, m), 2.84-2.87 (4H, m), 1.47 (3H, d, J = 9.2 Hz), 1.37-1.40 (3H, m). | 463.2 | 462.21 |
| 55 | 1H-NMR (DMSO-d6) δ: 10.33 (1H, s), 9.39 (1H, s), 8.02-8.05 (2H, m), 7.47 (1H, dd, J = 11.6, 4.0 Hz), 7.03 (1H, t, J = 71.6 Hz), 6.46 (1H, d, J = 10.8 Hz), 4.19-4.25 (1H, m), 3.05-3.08 (4H, m), 2.84-2.87 (4H, m), 1.29 (3H, d, J = 8.4 Hz). | 433.3 | 432.20 |
| 56 | 1H-NMR (DMSO-d6) δ: 10.19 (1H, s), 9.36 (1H, s), 8.12 (1H, d, J = 12 Hz), 8.03 (1H, d, J = 3.6 Hz), 7.63 (1H, s), 7.48 (1H, dd, J = 12.0, 4.0 Hz), 6.64 (1H, d, J = 8.8 Hz), 4.23 (1H, br m), 4.23 (1H, br m), 3.92-3.96 (2H, m), 3.49-3.56 (2H, m), 3.06-3.09 (4H, m), 2.86-2.88 (4H, m), 2.52 (3H ,s), 2.06-2.10 (2H, m), 1.69-1.72 (2H, m). | 449.1 | 448.23 |
| 57 | 1H-NMR (DMSO-d6) δ: 10.16 (1H, s), 9.35 (1H, s), 8.08 (1H, d, J = 12 Hz), 8.03 (1H, d, J = 3.6 Hz), 7.60 (1H, s), 7.46 (1H, dd, J = 11.6, 3.6 Hz), 6.63 (1H, d, J = 9.6 Hz), 4.42-4.44 (1H, br m), 3.05-3.08 (4H, m), 2.86-2.88 (4H, m), 2.61 (3H ,s), 2.16 (2H, br m), 1.63-1.77 (6H, m). | 433.15, | 432.24 |

**[Table 5-11]**

| Compound No. | NMR | LC/MS (M+H) ⁺ | Exact Mass |
|---|---|---|---|
| 58 | 1H-NMR (DMSO-d6) δ: 10.10 (1H, s), 9.33 (1H, s), 8.23 (1H, d, J = 12 Hz), 8.04 (1H, d, J = 3.6 Hz), 7.59 (1H, s), 7.45 (1H, dd, J = 12.0, 4.0 Hz), 7.01 (1H, m), 3.62-3.66 (2H, m), 3.05-3.08 (4H, m), 2.84-2.88 (4H, m), 1.30(3H, t, 9.6 Hz). | 393.3 | 392.21 |
| 59 | 1H-NMR (DMSO-d6) δ: 10.28 (1H, s), 9.34 (1H, s), 8.03 (2H, m), 7.60 (1H, s), 7.45 (1H, dd, J = 12.0, 4.0 Hz), 6.54 (1H, m), 3.04-3.07 (4H, m), 2.83-2.87 (4H, m), 1.58(9H, s). | 421.1 | 420.24 |
| 60 | 1H-NMR (DMSO-d6) δ: 10.06 (1H, s), 9.43 (1H, s), 8.03-8.09 (2H, m), 7.47 (1H, dd, J = 12.4, 4.4 Hz), 6.80 (1H, t, J = 72.8 Hz), 6.35 (1H, d, J = 9.6 Hz), 4.22-4.31 (1H, m), 3.06-3.09 (4H, m), 2.87-2.90 (4H, m), 2.50 (3H, s), 1.30 (6H, d, J = 8.4 Hz). | 429.1 | 428.22 |
| 61 | 1H-NMR (DMSO-d6) δ: 9.75 (1H, s), 9.18 (1H, s), 8.08 (1H, d, J = 12.0 Hz), 8.02 (1H, d, J = 4.0 Hz), 7.45 (1H, dd, J = 9.12, 2.84 Hz), 6.96 (1H, s), 6.35 (1H, m), 5.16 (1H, d, J = 6.0 Hz), 4.69-4.70 (1H, m), 4.69-4.70 (1H, m), 4.68-4.56 (1H, m), 4.18-4.31 (1H, m), 3.58-3.62 (1H, m), 3.44-3.52 (2H, m), 2.81-2.88 (2H, m), 1.82-1.88 (2H, m), 1.45-1.54 (2H, m), 1.38 (3H, d, J = 8.40 Hz), 1.29 (6H, dd, J = 8.8, 1.6 Hz). | 424.1 | 423.24 |
| 62 | 1H-NMR (DMSO-d6) δ: 9.87 (1H, s), 9.37 (1H, s), 8.11 (1H, d, J = 9.08 Hz), 8.03 (1H, d, J = 2.84 Hz), 7.49 (1H, dd, J = 9.08, 2.92 Hz), 6.25 (1H, d, J = 7.96 Hz), 4.84 (1H, br s), 4.51 (2H, s), 4.27-4.32 (1H, m), 3.13-3.15 (4H, m), 2.97-2.99 (4H, m), 2.41 (3H, s), 1.29 (6H, d, J = 6.44 Hz). | 437.4 | 408.24 |

**[Table 5-12]**

| Compound No. | NMR | LC/MS (M+H)⁺ | Exact Mass |
|---|---|---|---|
| 63 | 1H-NMR (DMSO-d6) δ: 10.01 (1H, s), 9.32 (1H, s), 8.09 (1H, d, J = 9.2 Hz), 8.03 (1H, d, J = 3.2 Hz), 7.47 (1H, dd, J = 9.2, 3.2 Hz), 6.25 (1H, d, J = 7.6 Hz), 4.99 (1H, m), 4.92 (1H, d, J = 5.2 Hz), 4.27-4.29 (1H, m), 3.05-3.08 (4H, m), 2.85-2.87 (4H, m), 1.42 (3H, d, J = 6.4 Hz), 1.31 (6H, apparent t, J = 6.0 Hz). | 427.1 | 426.23 |
| 64 | 1H-NMR (DMSO-d6) δ: 10.01 (1H, s), 9.32 (1H, s), 8.09 (1H, d, J = 9.2 Hz), 8.03 (1H, d, J = 3.2 Hz), 7.47 (1H, dd, J = 9.2, 3.2 Hz), 6.25 (1H, d, J = 7.6 Hz), 4.99 (1H, m), 4.92 (1H, d, J = 5.2 Hz), 4.27-4.29 (1H, m), 3.05-3.08 (4H, m), 2.85-2.87 (4H, m), 1.42 (3H, d, J = 6.4 Hz), 1.31 (6H, apparent t, J = 6.0 Hz). | 427.2 | 426.23 |
| 65 | 1H-NMR (DMSO-d6) δ: 9.82 (1H, s), 9.36 (1H, s), 8.09 (1H, d, J = 12.0 Hz), 8.01 (1H, d, J = 4.0 Hz), 7.46 (1H, dd, J = 12.0, 4.0 Hz), 6.28 (1H, d, J = 10.0 Hz), 4.94-4.99 (1H, m), 4.73 (1H, d, J = 9.2 Hz), 4.25-4.32 (1H, m), 3.03-3.06 (4H, m), 2.83-2.87 (4H, m), 2.41 (3H, s), 2.27 (1H, br s), 1.29-1.36 (9H, m). | 423.2 | 422.25 |
| 66 | 1H-NMR (DMSO-d6) δ: 9.82 (1H, s), 9.36 (1H, s), 8.09 (1H, d, J = 12.0 Hz), 8.01 (1H, d, J = 4.0 Hz), 7.46 (1H, dd, J = 12.0, 4.0 Hz), 6.28 (1H, d, J = 10.0 Hz), 4.94-4.99 (1H, m), 4.73 (1H, d, J = 9.2 Hz), 4.25-4.32 (1H, m), 3.03-3.06 (4H, m), 2.83-2.87 (4H, m), 2.41 (3H, s), 2.27 (1H, br s), 1.29-1.36 (9H, m). | 423.2 | 422.25 |
| 67 | 1H-NMR (DMSO-d6) δ: 10.40 (1H, s), 9.32 (1H, s), 7.62 (1H, dd, J = 14.8, 11.6 Hz), 7.22 (1H, s), 6.81 (1H, t, J = 74.0 Hz), 6.62 (1H, d, J = 2.8 Hz), 4.24-4.35 (1H, m), 2.89-3.09 (8H, m), 1.31 (6H, d, J = 8.4 Hz). | 433.1 | 432.20 |

**[Table 5-13]**

| Compound No. | NMR | LC/MS (M+H)⁺ | Exact Mass |
|---|---|---|---|
| 68 | 1H-NMR (DMSO-d6) δ: 10.16 (1H, d, J = 15.6 Hz), 9.52 (1H, d, J = 15.6 Hz), 8.09 (1H, d, J = 12 Hz), 8.04 (1H, d, J = 4.0 Hz), 7.47 (1H, dd, J = 12.0, 3.6 Hz), 6.37 (1H, d, J = 9.6 Hz), 4.19-4.28 (1H, m), 3.05-3.19 (4H, m), 2.84-2.87 (4H, m), 2.63 (3H ,s), 2.59 (3H ,s), 1.33 (6H, d, J = 8.4 Hz). | 421.1 | 420.24 |
| 69 | 1H-NMR (DMSO-d6) δ: 9.72 (1H, s), 9.20 (1H, s), 8.26 (1H, d, J = 12.0 Hz), 8.00 (1H, d, J = 4.0 Hz), 7.31-7.42 (6H, m), 7.19-7.28 (1H, m) , 6.98 (1H, s), 5.13 (1H, d, J = 6.0 Hz), 4.76 (1H, d, J = 8.0 Hz), 4.55-4.59 (1H, m), 3.01-3.05 (4H, m), 2.83-2.87 (4H, m), 1.31 (3H, d, J = 8.8 Hz). | 457.1 | 456.24 |
| 71 | 1H-NMR (DMSO-d6) δ: 9.78 (1H, s), 9.19 (1H, s), 8.10 (1H, d, J = 12.4 Hz), 8.02 (1H, d, J = 3.6 Hz), 7.50 (1H, dd, J = 12.4, 3.6 Hz), 6.96 (1H, s), 6.32 (1H, d, J = 10.0 Hz), 5.17 (1H, d, J = 6.0 Hz), 4.55-4.59 (1H, m), 4.18-4.31 (1H, m), 3.12-3.21 (4H, m), 2.45-2.49 (4H, m), 2.23 (3H, s), 1.38 (3H, d, J = 8.4 Hz), 1.29 (6H, dd, J = 8.4, 1.6 Hz). | 423.4 | 422.25 |
| 72 | 1H-NMR (DMSO-d6) δ: 9.79 (1H, s), 9.20 (1H, s), 8.13 (1H, d, J = 12.4 Hz), 8.04 (1H, d, J = 4.0 Hz), 7.49 (1H, dd, J = 12.0, 4.0 Hz), 6.97 (1H, s), 6.34 (1H, d, J = 10.0 Hz), 5.17 (1H, d, J = 6.0 Hz), 4.58-4.66 (1H, m), 4.21-4.31 (1H, m), 3.75-3.88 (4H, m), 3.11-3.25 (4H, m), 1.48 (3H, d, J = 8.4 Hz), 1.38 (6H, d, J = 8.8 Hz). | 410.3 | 409.22 |

**[Table 5-14]**

| Compound No. | NMR | LC/MS (M+H)⁺ | Exact Mass |
|---|---|---|---|
| 74 | 1H-NMR (DMSO-d6) δ: 9.78 (1H, s), 9.19 (1H, s), 8.11 (1H, d, J = 12.0 Hz), 8.03 (1H, d, J = 4.0 Hz), 7.48 (1H, dd, J = 12.0, 4.0 Hz), 6.96 (1H, s), 6.33 (1H, d, J = 10.0 Hz), 5.17 (1H, d, J = 6.0 Hz), 4.61 (1H, m), 4.46 (1H, m), 4.42-4.44 (1H, m), 3.33-3.57 (2H, m), 3.12-3.15 (4H, m), 2.57-2.60 (4H, m), 2.42-2.49 (2H, m), 1.38 (3H, d, J = 8.4 Hz), 1.30 (6H, dd, J = 8.4, 1.6 Hz). | 453.6 | 452.26 |
| 75 | 1H-NMR (DMSO-d6) δ: 9.76 (1H, s), 9.19 (1H, s), 8.05 (1H, d, J = 12.0 Hz), 8.02 (1H, d, J = 4.0 Hz), 7.47 (1H, dd, J = 12.0, 4.0 Hz), 6.96 (1H, s), 6.34 (1H, d, J = 10.0 Hz), 5.17 (1H, d, J = 6.0 Hz), 4.61 (1H, m), 4.38 (1H, m), 4.36-4..38 (1H, m), 3.61-3.64 (2H, m), 3.37-3.40 (2H, m), 2.52-2.56 (2H, m), 1.71-1.78 (2H, m), 1.40-1.42 (2H, m), 1.38 (3H, d, J = 8.8 Hz), 1.29 (6H, dd, J = 8.8, 1.6 Hz). | 466.6 | 465.29 |
| 76 | 1H-NMR (DMSO-d6) δ: 9.57 (1H, s), 9.15 (1H, s), 8.03 (1H, d, J = 12.0 Hz), 7.66 (1H, d, J = 3.6 Hz), 7.03 (1H, dd, J = 12.0, 4.0 Hz), 6.94 (1H, s), 6.31 (1H, d, J = 10.0 Hz), 5.15 (1H, d, J = 6.0 Hz), 4.60-4.62 (1H, m), 4.21-4.28 (1H, m), 3.58-3.62 (1H, m), 3.41-3.46 (2H, m), 3.23-3.24 (1H, m), 2.89-2.94 (1H, m), 2.06-2.13 (2H, m), 1.72-1.76 (1H, m), 1.38 (3H, d, J = 8.8 Hz), 1.30 (6H, dd, J = 8.8, 1.2 Hz). | 409.4 | 408.24 |

**[Table 5-15]**

| Compound No. | NMR | LC/MS (M+H)⁺ | Exact Mass |
|---|---|---|---|
| 77 | 1H-NMR (DMSO-d6) δ: 9.58 (1H, s), 9.15 (1H, s), 8.02 (1H, d, J = 12.0 Hz), 7.67 (1H, d, J = 4.0 Hz), 7.06 (1H, dd, J = 12.0, 4.0 Hz), 6.94 (1H, s), 6.30 (1H, d, J = 10.0 Hz), 5.15 (1H, d, J = 6.0 Hz), 4.98 (1H, d, J = 5.2 Hz), 4.60-4.62 (1H, m), 4.42 (1H, br s), 4.21-4.28 (1H, m), 3.40-3.48 (1H, m), 3.09-3.12 (1H, m), 2.00-2.08 (1H, m), 1.88-1.93 (1H, m), 1.38 (3H, d, J = 8.4 Hz), 1.29 (6H, dd, J = 8.8, 1.2 Hz). | 410.2 | 409.22 |
| 78 | 1H-NMR (DMSO-d6) δ: 9.94 (1H, s), 9.23 (1H, s), 8.22 (1H, d, J = 11.2 Hz), 8.18 (1H, d, J = 2.8 Hz), 7.71 (1H, d J = 3.6 Hz), 6.99 (1H, s), 6.40 (1H, d, J = 9.6 Hz), 5.18 (1H, d, J = 6.4 Hz), 4.58-4.66 (1H, m), 4.20-4.31 (1H, m), 3.02-3.06 (2H, m), 2.51-2.59 (3H, m), 1.69 (2H, br m), 1.55-1.56 (2H, m), 1.39 (3H, d, J = 8.4 Hz), 1.30 (6H, dd, J = 8.8, 2.0 Hz). | 408.4 | 407.24 |
| 79 | 1H-NMR (DMSO-d6) δ: 10.03 (1H, s), 9.25 (1H, s), 8.27 (1H, d, J = 11.6 Hz), 8.19 (1H, d, J = 2.4 Hz), 7.74 (1H, dd, J = 11.6, 2.4 Hz), 6.99 (1H, s), 6.42 (1H, d, J = 10.0 Hz), 5.19 (1H, d, J = 6.0 Hz), 4.61-4.66 (1H, m), 4.22-4.31 (1H, m), 3.54 (2H, s), 2.69-2.77 (4H, m), 2.27-2.31 (4H, m), 1.39 (3H, d, J = 8.4 Hz), 1.30 (6H, dd, J = 8.8, 1.6 Hz). | 423.1 | 422.25 |
| 90 | | 458.53 | 457.20 |

**[Table 5-16]**

| Compound No. | NMR | LC/MS (M+H)⁺ | Exact Mass |
|---|---|---|---|
| 92 | 1H-NMR (DMSO-d6) δ: 9.78 (1H, s), 9.22 (1H, s), 8.13 (1H, d, J = 11.6 Hz), 8.00 (1H, d, J = 4.0 Hz), 7.45 (1H, dd, J = 12.0, 4.0 Hz), 6.65 (1H, s), 6.31 (1H, d, J = 8.0 Hz), 5.19 (1H, d, J = 3.2 Hz), 4.61-4.64 (2H, m), 3.88-4.01 (2H, m), 3.75-3.80 (1H, m), 3.64-3.69 (1H, m), 3.03-3.06 (4H, m), 2.84-2.87 (4H, m), 2.26-2.36 (1H, m), 1.98-2.02 (1H, m), 1.29 (3H, dd, J = 8.8, 2.8 Hz). | 437.2 | 436.23 |
| 94 | 1H-NMR (DMSO-d6) δ: 10.32 (1H, s), 9.34 (1H, s), 8.27 (1H, d, J = 8.4 Hz), 8.24 (1H, d, J = 1.6 Hz), 7.78 (1H, dd, J = 8.4, 1.6 Hz), 7.22 (1H, s), 6.81 (1H, t, J = 55.6 Hz), 6.68 (1H, d, J = 9.6 Hz), 4.27-4.32 (1H, m), 3.57-3.59 (4H, m), 3.65 (2H, s), 2.38 (4H, br s), 1.31 (3H, d, J = 6.4 Hz). | 430.2 | 429.21 |
| 99 | 1H-NMR (DMSO-d6) δ: 10.49 (1H, s), 9.38 (1H, s), 8.69 (1H, d, J = 2.8 Hz), 8.38 (1H, d, J = 8.8 Hz), 8.18 (1H, dd, J = 8.4, 2.4 Hz), 7.75 (2H, d, J = 7.2 Hz), 7.51 (1H, apparent t, J = 7.2 Hz), 7.49 (2H, apparent t, J = 8.0 Hz), 7.25 (1H, s), 6.83 (1H, t, J = 55.2 Hz), 6.74 (1H, d, J = 8.0 Hz), 4.30-4.33 (1H, m), 1.33 (6H, d, J = 6.4 Hz). | 407.1 | 406.17 |
| 100 | 1H-NMR (DMSO-d6) δ: 10.63 (1H, s), 9.40 (1H, s), 8.45 (1H, s), 8.65 (2H, apparent d, J = 6.8 Hz), 8.44 (1H, d, J = 12.0 Hz), 8.32 (1H, d, J = 11.2 Hz), 7.81 (2H, apparent d, J = 7.2 Hz), 7.25 (1H, s), 6.82 (1H, t, J = 73.6 Hz), 6.75 (1H, d, J = 10.4 Hz), 4.30-4.33 (1H, m), 1.33 (6H, d, J = 8.8 Hz). | 408.1 | 407.17 |
| 101 | | 449.20 | 448.17 |

**[Table 5-17]**

| Compound No. | NMR | LC/MS (M+H)⁺ | Exact Mass |
|---|---|---|---|
| 103 | 1H-NMR (DMSO-D6) δ: 10.17 (1H, s), 9.29 (1H, s), 8.15 (1H, d, J = 9.3 Hz), 8.07 (1H, d, J = 3.4 Hz), 7.52 (1H, dd, J = 8.8, 2.9 Hz), 7.19 (1H, s), 6.78 (1H, t, J = 55.6 Hz), 6.61 (1H, d, J = 7.8 Hz), 4.27 (1H, dd, J = 13.4, 6.6 Hz), 4.16-4.15 (2H, m), 3.55 (2H, t, J = 5.4 Hz), 3.46 (2H, t, J = 7.1 Hz), 2.22 (3H, t, J = 8.0 Hz), 1.96-1.88 (3H, m), 1.56-1.33 (3H, m), 1.29 (6H, d, J = 6.3 Hz), 1.20 (2H, dd, J = 33.4, 6.6 Hz), 0.87 (1H, t, J = 7.1 Hz). | 458.44 | 457.20 |
| 104 | 1H-NMR (DMSO-D6) δ: 10.17 (1H, s), 9.32 (1H, s), 8.03 (1H, d, J = 8.3 Hz), 7.72 (1H, t, J = 7.8 Hz), 7.20 (1H, s), 6.90-6.69 (3H, m), 4.31-4.23 (1H, m), 2.43 (3H, s), 1.30 (6H, d, J = 6.3 Hz). | 345.15 | 344.16 |
| 105 | 1H-NMR (DMSO-d6) δ: 12.78 (1H, s), 9.86 (1H, s), 9.20 (1H, s), 8.20 (1H, br s), 8.00 (1H, d, J = 3.6 Hz), 7.74 (1H, br s), 7.45 (1H, d, J = 11.6 Hz), 7.23 (1H, br m), 7.00 (1H, d, J = 7.6 Hz), 6.30 (1H, br s), 5.32 (1H, br s), 5.17-5.21 (1H, m), 4.61-4.64 (1H, m), 3.04-3.06 (4H, m), 2.85-2.88 (4H, m), 1.58 (3H, d, J = 8.0 Hz), 1.29 (3H, dd, J = 15.2, 8.8 Hz) . | 461.2 | 460.24 |
| 106 | 1H-NMR (DMSO-d6) δ: 12.72 (1H, s), 9.82 (1H, d, J = 4.0 Hz), 9.20 (1H, s), 8.03 (1H, dd, J = 12.0, 3.6 Hz), 7.99 (1H, d, J = 4.0 Hz), 7.78 (1H, br s), 7.56 (1H, br m), 7.37 (1H, m), 6.98 (1H, d, J = 3.2 Hz), 6.56 (1H, t, J = 10.4 Hz), 5.29-5.34 (1H, m), 5.19 (1H, t, J = 6.4 Hz), 4.62-4.66 (1H, m), 3.01-3.04 (4H, m), 2.83-2.87 (4H, m), 1.59 (3H, d, J = 8.8 Hz), 1.40-1.42 (3H, m). | 461.2 | 460.24 |

**[Table 5-18]**

| Compound No. | NMR | LC/MS (M+H)⁺ | Exact Mass |
|---|---|---|---|
| 107 | 1H-NMR (DMSO-d6) δ: 10.20 (1H, s), 9.33 (1H, s), 8.21 (1H, dd, J = 7.6, 4.0 Hz), 7.74 (1H, d, J = 9.2 Hz), 7.21 (1H, s), 6.81 (1H, t, J = 74.0 Hz), 6.68 (1H, d, J = 10.4 Hz), 4.39 (1H, d, J = 4.4 Hz), 4.24-4.35 (1H, m), 3.91 (1H, br s), 2.58-2.59 (1H, m), 1.74-1.98 (4H, m), 1.50-1.60 (4H, m), 1.31 (6H, d, J = 8.4 Hz). | 429.2 | 428.21 |
| 108 | 1H-NMR (DMSO-d6) δ: 10.21 (1H, s), 9.33 (1H, s), 8.18-8.21 (2H, m), 7.69 (1H, dd, J = 11.2, 3.2 Hz), 7.21 (1H, s), 6.81 (1H, t, J = 74.0 Hz), 6.68 (1H, d, J = 10.4 Hz), 4.39 (1H, t, J = 6.8 Hz), 4.25-4.32 (1H, m), 3.3.40-3.46 (2H, m), 2.56-2.61 (2H, m), 1.60-1.65 (2H, m), 1.43-1.50 (2H, m), 1.31 (6H, d, J = 8.8 Hz). | 403.1 | 402.20 |
| 109 | 1H-NMR (DMSO-d6) δ: 10.74 (1H, br s), 9.34 (1H, s), 8.82 (1H, d, J = 2.8 Hz), 8.53 (1H, t, J = 6.8 Hz), 8.38 (1H, d, J = 12.0 Hz), 8.29 (1H, dd, J = 12.0, 0.8 Hz), 7.25 (1H, s), 6.82 (1H, t, J = 73.6 Hz), 6.77 (1H, d, J = 10.8 Hz), 4.76 (1H, t, J = 7.2 Hz), 4.28-4.37 (1H, m), 3.52-3.56 (2H, m), 3.31-3.33 (2H, m), 1.32 (6H, d, J = 8.8 Hz). | 418.2 | 417.17 |
| 110 | 1H-NMR (DMSO-d6) δ: 10.40 (1H, s), 9.60 (1H, s), 9.36 (1H, s), 8.59 (1H, d, J = 2.4 Hz), 8.32 (1H, d, J = 8.8 Hz), 7.56 (2H, d, J = 8.8 Hz), 7.24 (1H, s), 6.82 (1H, t, J = 55.6 Hz), 6.88 (1H, d, J = 8.4 Hz), 6.70-6.72 (1H, m), 4.30 (1H, q, J = 6.4 Hz), 1.32 (6H, d, J = 6.4 Hz). | 423.1 | 422.17 |
| 112 | | 453.20 | 452.14 |

**[Table 5-19]**

| Compound No. | NMR | LC/MS (M+H)⁺ | Exact Mass |
|---|---|---|---|
| 113 | 1H-NMR (DMSO-D6) δ: 10.49 (1H, br s), 9.35 (1H, s), 8.31-8.28 (1H, br m), 8.24 (1H, d, J = 8.8 Hz), 7.85 (1H, d, J = 8.3 Hz), 7.23 (1H, s), 6.94-6.66 (2H, m), 4.31-4.28 (1H, m), 3.74 (2H, br s), 2.96 (4H, br s), 1.31 (6H, d, J = 6.3 Hz), 1.24-1.17 (2H, m). | 478.1 | 477.18 |
| 114 | 1H-NMR (DMSO-D6) δ: 10.32 (1H, br s), 9.34 (1H, s), 8.28-8.26 (2H, m), 7.79 (1H, d, J = 7.8 Hz), 7.21 (1H, s), 6.82 (1H, t, J = 45.9 Hz), 6.67 (1H, d, J = 11.2 Hz), 4.31-4.24 (1H, m), 3.53 (1H, br s), 3.31 (4H, br s), 3.16 (6H, s), 2.75 (6H, s), 1.30 (6H, d, J = 6.3 Hz), 1.26-1.16 (2H, m). | 536.2 | 535.23 |
| 115 | 1H-NMR (DMSO-D6) δ: 10.43 (1H, br s), 9.35 (1H, s), 8.30 (2H, br s), 7.84 (1H, br s), 7.22 (1H, s), 6.94-6.67 (2H, m), 4.33-4.25 (1H, m), 3.63-3.59 (1H, m), 3.47-3.36 (4H, br m), 3.16-3.12 (4H, m), 2.91 (3H, br s), 1.31 (6H, d, J = 6.8 Hz), 1.26-1.24 (3H, m). | 507.1 | 506.20 |
| 116 | 1H-NMR (DMSO-D6) δ: 10.35 (1H, br s), 9.34 (1H, s), 8.28-8.26 (2H, br m), 7.79 (1H, d, J = 6.8 Hz), 7.21 (1H, s), 6.94-6.66 (2H, m), 4.30-4.27 (1H, m), 3.31 (4H, br s), 3.19-3.17 (3H, br m), 2.70-2.67 (4H, br m), 1.30 (6H, d, J = 6.3 Hz), 1.23-1.15 (2H, m). | 511.2 | 510.23 |
| 117 | 1H-NMR (DMSO-D6) δ: 10.32 (1H, s), 9.33 (1H, s), 8.28-8.19 (2H, m), 7.78 (1H, dd, J = 8.5, 2.2 Hz), 7.21 (1H, s), 6.94-6.66 (2H, m), 4.33-4.24 (1H, m), 3.42-3.40 (5H, br m), 2.39-2.37 (2H, m), 2.32-2.31 (2H, m), 1.97 (3H, s), 1.30 (6H, d, J = 6.3 Hz). | 471.43 | 470.24 |

**[Table 5-20]**

| Compound No. | NMR | LC/MS (M+H) ⁺ | Exact Mass |
|---|---|---|---|
| 118 | 1H-NMR (DMSO-D6) δ: 10.35 (1H, s), 9.34 (1H, s), 8.28-8.24 (2H, m), 7.79 (1H, dd, J = 8.5, 2.2 Hz), 7.21 (1H, s), 6.94-6.66 (2H, m), 4.33-4.24 (1H, m), 4.08 (1H, br s), 3.56 (2H, s), 2.98-2.95 (2H, br m), 2.03-2.00 (2H, br m), 1.68-1.66 (2H, br m), 1.30 (6H, d, J = 6.3 Hz), 1.23-1.17 (4H, m), 1.01 (6H, s). | 486.48 | 485.27 |
| 119 | | 445.46 | 444.21 |
| 120 | | 445.41 | 444.21 |
| 124 | 1H-NMR (DMSO-D6) δ: 10.31 (1H, s), 9.33 (1H, s), 8.37 (1H, t, J = 5.6 Hz), 8.23 (2H, t, J = 4.1 Hz), 7.73 (1H, dd, J = 8.4, 2.6 Hz), 7.21 (1H, s), 6.94-6.66 (2H, m), 4.32-4.22 (3H, m), 1.86 (3H, s), 1.30 (6H, d, J = 6.3 Hz). | | |
| 125 | 1H-NMR (DMSO-D6) δ: 10.28 (1H, s), 9.33 (1H, s), 8.25 (1H, d, J = 8.3 Hz), 8.21 (1H, d, J = 2.0 Hz), 7.76 (1H, dd, J = 8.3, 2.0 Hz), 7.21 (1H, s), 7.19 (1H, br s), 6.93-6.66 (3H, m), 4.31-4.24 (1H, m), 3.43 (2H, s), 3.33 (2H, br s), 2.84-2.82 (2H, m), 2.06-2.03 (1H, m), 1.93-1.90 (2H, m), 1.67-1.64 (2H, m), 1.56-1.50 (2H, m), 1.30 (6H, d, J = 6.3 Hz). | 471.43 | 470.24 |
| 126 | | 416.34 | 415.19 |
| 127 | 1H-NMR (DMSO-D6) δ: 10.27 (1H, s), 9.33 (1H, s), 8.27-8.22 (3H, m), 7.75 (1H, dd, J = 8.5, 2.2 Hz), 7.21 (1H, s), 6.93-6.66 (2H, m), 4.31-4.24 (1H, m), 3.47-3.38 (2H, m), 3.29-3.25 (1H, m), 3.18-3.14 (1H, m), 2.86-2.84 (1H, m), 2.74-2.71 (1H, m), 1.91-1.88 (1H, m), 1.66-1.59 (5H, br m), 1.45-1.42 (1H, br m), 1.30 (6H, d, J = 6.3 Hz). | 458.4 | 457.24 |

**[Table 5-21]**

| Compound No. | NMR | LC/MS (M+H)⁺ | Exact Mass |
|---|---|---|---|
| 128 | 1H-NMR (DMSO-D6) δ: 10.29 (1H, s), 9.33 (1H, s), 8.26-8.21 (3H, m), 7.75 (1H, dd, J = 8.5, 2.2 Hz), 7.21 (1H, s), 6.93-6.66 (2H, m), 4.33-4.24 (1H, m), 3.51-3.39 (3H, m), 2.80-2.78 (1H, m), 2.66-2.64 (1H, m), 1.88-1.86 (1H, m), 1.79-1.69 (2H, m), 1.62-1.59 (1H, m), 1.43-1.37 (1H, m), 1.30 (6H, d, J = 6.8 Hz), 1.15-1.00 (2H, m). | 444.42 | 443.22 |
| 132 | 1H-NMR (DMSO-d6) δ: 10.26 (1H, br s), 9.33 (1H, s), 8.23 (1H, d, J = 11.7 Hz), 8.20 (1H, d, J = 2.8 Hz), 7.74 (1H, dd, J = 11.6, 3.2 Hz), 7.52 (1H, br s), 7.22 (1H, s), 6.94 (1H, br s), 6.81 (1H, t, J = 74.0 Hz), 6.69 (1H, d, J = 10.4 Hz), 4.23-4.33 (1H, m), 3.40 (2H, s), 1.31 (6H, d, J = 8.8 Hz). | 388.3 | 387.16 |
| 133 | 1H-NMR (DMSO-d6) δ: 10.16 (1H, br s), 9.32 (1H, s), 8.16-8.19 (2H, m), 7.74 (1H, br m), 7.21 (1H, s), 6.80 (1H, t, J = 74.0 Hz), 6.68 (1H, br m), 4.23-4.33 (1H, m), 3.39 (2H, s), 1.30 (6H, d, J = 8.8 Hz). | 389.1 | 388.15 |
| 134 | 1H-NMR (DMSO-d6) δ: 10.19 (1H, br s), 9.33 (1H, s), 8.17-8.20 (2H, m), 7.71-7.74 (1H, br m), 7.21 (1H, s), 6.80 (1H, t, J = 74.0 Hz), 6.67 (1H, d, J = 10.4 Hz), 4.68 (1H, t, J = 7.2 Hz), 4.23-4.33 (1H, m), 3.60-3.66 (2H, m), 2.72 (2H, t, J = 8.4 Hz), 1.31 (6H, d, J = 8.4 Hz). | 375.1 | 374.17 |
| 135 | 1H-NMR (DMSO-d6) δ: 10.21 (1H, s), 9.33 (1H, s), 8.18-8.22 (2H, m), 7.69-7.71 (1H, br m), 7.27 (1H, br s), 7.21 (1H, s), 6.80 (1H, t, J = 74.4 Hz), 6.67-6.72 (2H, br m), 4.28-4.32 (1H, m), 2.59-2.73 (2H, m), 2.09 (2H, t, J = 10.0 Hz), 1.78-1.83 (2H, m), 1.31 (6H, d, J = 8.4 Hz). | 416.2 | 415.19 |

**[Table 5-22]**

| Compound No. | NMR | LC/MS (M+H)⁺ | Exact Mass |
|---|---|---|---|
| 136 | 1H-NMR (DMSO-d6) δ: 10.22 (1H, s), 9.33 (1H, s), 8.18-8.21 (2H, m), 7.68-7.71 (1H, br m), 7.21 (1H, s), 6.80 (1H, t, J = 74.0 Hz), 6.67-6.72 (1H, br m), 4.25-4.27 (1H, m), 2.51-2.73 (2H, m), 2.12 (2H, br m), 1.80-1.87 (2H, m), 1.30 (6H, d, J = 8.4 Hz). | 417.3 | 416.18 |
| 138 | 1H-NMR (DMSO-D6) δ: 10.30 (1H, s), 9.32 (1H, s), 8.23 (2H, d, J = 8.8 Hz), 7.73 (1H, dd, J = 8.5, 2.4 Hz), 7.21 (1H, s), 6.94-6.66 (2H, m), 6.46 (1H, t, J = 6.0 Hz), 5.56 (2H, s), 4.34-4.24 (1H, m), 4.16 (2H, d, J = 6.1 Hz), 1.30 (6H, d, J = 6.3 Hz). | | |
| 139 | 1H-NMR (DMSO-D6) δ: 10.33 (1H, s), 9.33 (1H, s), 8.29-8.27 (1H, m), 8.23-8.23 (2H, m), 7.72-7.72 (1H, m), 7.21 (3H, s), 6.78-6.73 (2H, m), 4.47 (2H, s), 4.31-4.29 (1H, m), 2.94 (3H, s), 2.05 (3H, s), 1.30 (6H, d, J = 6.6 Hz). | | |
| 140 | 1H-NMR (DMSO-D6) δ: 10.37 (1H, s), 9.34 (1H, s), 8.29-8.27 (2H, m), 7.82 (1H, d, J = 8.8 Hz), 7.57 (1H, t, J = 6.1 Hz), 7.21 (1H, s), 6.94-6.66 (2H, m), 4.30-4.26 (1H, m), 4.16 (2H, d, J = 6.1 Hz), 2.91 (3H, s), 1.30 (6H, d, J = 6.3 Hz). | | |
| 141 | 1H-NMR (DMSO-D6) δ: 10.41 (1H, s), 9.34 (1H, s), 8.33-8.29 (2H, m), 7.82 (1H, dd, J = 8.7, 2.3 Hz), 7.22 (1H, s), 6.94-6.66 (2H, m), 4.33-4.26 (1H, m), 4.24 (2H, s), 3.32 (2H, s), 2.97 (3H, s), 2.69 (3H, s), 1.31 (6H, d, J = 6.6 Hz). | | |

**[Table 5-23]**

| Compound No. | NMR | LC/MS (M+H)⁺ | Exact Mass |
|---|---|---|---|
| 142 | 1H-NMR (DMSO-D6) δ: 10.28 (1H, s), 9.33 (1H, s), 8.25 (1H, d, J = 8.8 Hz), 8.21 (1H, d, J = 2.4 Hz), 8.15 (1H, s), 7.76 (1H, dd, J = 8.8, 2.4 Hz), 7.21 (1H, s), 6.93-6.66 (2H, m), 4.31-4.24 (1H, m), 3.33 (1H, br s), 3.16 (2H, s), 2.69-2.66 (2H, br m), 2.09-2.04 (2H, br m), 1.71-1.69 (2H, br m), 1.42-1.33 (2H, m), 1.30 (6H, d, J = 6.8 Hz). | 44.38 | 443.22 |
| 143 | 1H-NMR (DMSO-D6) δ: 10.39 (1H, s), 9.34 (1H, s), 8.49 (1H, br s), 8.33 (1H, s), 8.28 (1H, d, J = 8.8 Hz), 7.86 (1H, dd, J = 8.8, 2.4 Hz), 7.22 (1H, s), 6.94-6.66 (2H, m), 4.34 (2H, s), 4.30-4.24 (1H, m), 3.76-3.73 (2H, br m), 3.17-3.08 (2H, m), 3.02 (2H, d, J = 7.3 Hz), 2.96 (3H, s), 1.68-1.66 (1H, br m), 1.46-1.44 (2H, br m), 1.30 (6H, d, J = 6.3 Hz), 1.23-1.16 (2H, m), 1.06-0.97 (2H, m). | 536.32 | 535.22 |
| 144 | 1H-NMR (DMSO-D6) δ: 10.41 (0.4H, s), 10.33 (0.6H, s), 9.34 (0.4H, s), 9.33 (0.6H, s), 8.28-8.23 (2.0H, m), 7.72 (1.0H, td, J = 9.0, 2.3 Hz), 7.21 (1.0H, s), 6.93-6.66 (2.0H, m), 4.57 (0.8H, s), 4.49 (1.3H, s), 4.32-4.22 (1.0H, m), 3.86-3.80 (2.0H, m), 3.26-3.16 (4.0H, m), 2.08 (3.0H, s), 1.92-1.87 (1.0H, m), 1.47-1.43 (2.0H, m), 1.30 (6.0H, d, J = 6.3 Hz), 1.25-1.11 (3.OH, m). | 500.33 | 499.25 |
| 145 | 1H-NMR (DMSO-D6) δ: 10.38 (0.3H, s), 10.31 (0.6H, s), 9.34 (0.3H, s), 9.33 (0.6H, s), 8.28-8.22 (2.1H, m), 7.72-7.69 (1.0H, m), 7.21 (1.0H, s), 6.94-6.66 (2.0H, m), 4.66-4.53 (2.0H, m), 4.31-4.24 (1.0H, m), 4.04-3.98 (1.0H, br m), 3.80-3.74 (1.0H, m), 3.68-3.60 (1.0H, m), 3.54-3.50 (0.3H, m), 3.39-3.25 (7.5H, m), 3.12 (0.3H, dd, J = 13.7, 7.8 Hz), 2.09 (2.0H, s), 2.07 (1.0H, s), 1.95-1.75 (3.4H, m), 1.48-1.41 (1.0H, m), 1.30 (6.0H, d, J = 6.3 Hz). | 486.31 | 485.24 |

**[Table 5-24]**

| Compound No. | NMR | LC/MS (M+H)⁺ | Exact Mass |
|---|---|---|---|
| 146 | 1H-NMR (DMSO-D6) δ: 10.39 (0.4H, s), 10.31 (0.6H, s), 9.34 (0.4H, s), 9.33 (0.7H, s), 8.28-8.22 (2.0H, m), 7.71 (1.0H, d, J = 8.8 Hz), 7.21 (1.0H, s), 6.94-6.66 (2.0H, m), 4.66-4.53 (2.0H, m), 4.31-4.25 (1.0H, m), 4.04-4.02 (0.8H, br m), 3.80-3.74 (1.1H, m), 3.68-3.60 (1.1H, m), 3.53-3.51 (0.3H, m), 3.36-3.27 (10.0H, m), 3.14-3.10 (0.3H, m), 2.09 (2.0H, s), 2.07 (1.0H, s), 1.95-1.78 (2.0H, m), 1.48-1.41 (0.8H, m), 1.30 (6.0H, d, J = 6.3 Hz). | 486.31 | 485.24 |
| 147 | 1H-NMR (DMSO-D6) δ: 10.44 (1H, s), 9.34 (1H, s), 8.32-8.28 (2H, m), 7.82 (1H, d, J = 8.3 Hz), 7.22 (1H, s), 6.94-6.67 (2H, m), 4.32-4.24 (1H, m), 3.74 (2H, s), 3.24 (2H, d, J = 6.3 Hz), 3.03-3.00 (2H, br m), 2.30-2.24 (2H, br m), 1.70-1.67 (2H, br m), 1.43 (1H, br s), 1.30 (6H, d, J = 6.3 Hz), 1.25-1.16 (3H, m). | 458.36 | 457.24 |
| 148 | 1H-NMR (DMSO-D6) δ: 10.41 (1H, s), 9.34 (1H, s), 8.31-8.28 (2H, m), 7.85-7.82 (1H, m), 7.21 (1H, s), 6.94-6.67 (2H, m), 4.42 (2H, dd, J = 43.9, 15.6 Hz), 4.30-4.25 (1H, m), 4.04-4.01 (1H, m), 3.71 (1H, q, J = 7.2 Hz), 3.65-3.60 (1H, m), 3.21-3.09 (2H, m), 3.03 (3H, s), 1.83-1.72 (3H, m), 1.48-1.41 (1H, m), 1.30 (6H, d, J = 6.3 Hz). | 522.3 | 521.20 |
| 149 | | 522.3 | 521.20 |
| 150 | | 416.15 | 415.19 |
| 151 | | 452.10 | 451.16 |
| 152 | | 430.15 | 429.21 |
| 153 | | 466.15 | 465.18 |

**[Table 5-25]**

| Compound No. | NMR | LC/MS (M+H)⁺ | Exact Mass |
|---|---|---|---|
| 157 | 1H-NMR (DMSO-D6) δ: 10.21 (1H, s), 9.31 (1H, s), 8.48 (1H, s), 8.17-8.15 (2H, m), 7.67 (1H, dd, J = 8.5, 2.7 Hz), 7.20 (1H, s), 6.93-6.60 (2H, m), 4.30-4.24 (1H, m), 2.27 (3H, s), 1.30 (6H, d, J = 6.3 Hz). | 345.26 | 344.16 |
| 158 | 1H-NMR (DMSO-D6) δ: 9.56 (1H, s), 8.36 (1H, s), 8.21 (1H, t, J = 5.9 Hz), 7.95 (1H, d, J = 2.0 Hz), 7.44 (1H, dd, J = 8.5, 2.2 Hz), 7.35 (1H, s), 7.21 (1H, d, J = 7.8 Hz), 6.84 (1H, t, J = 55.4 Hz), 6.39 (1H, d, J = 8.3 Hz), 5.84 (2H, s), 4.46 (2H, d, J = 5.9 Hz), 4.36-4.29 (1H, m), 2.21 (3H, s), 1.26 (6H, d, J = 6.3 Hz). | 441.33 | 440.20 |
| 159 | 1H-NMR (DMSO-d6) δ: 10.31 (1H, s), 9.34 (1H, s), 8.23-8.25 (2H, m), 7.79 (1H, dd, J = 11.6, 2.8 Hz), 7.22 (1H, s), 6.81 (1H, t, J = 74.0 Hz), 6.68 (1H, d, J = 10 Hz), 4.26-4.33 (1H, m), 3.30-3.32 (2H, m), 3.12-3.16 (2H, m), 2.99 (1H, br m), 2.13-2.15 (4H, br s), 1.31 (6H, d, J = 8.8 Hz). | 463.3 | 462.16 |
| 160 | 1H-NMR (DMSO-d6) δ: 10.24 (1H, s), 9.33 (1H, s), 8.25 (1H, d, J = 2.8 Hz), 8.21 (1H, d, J = 11.6), 7.76 (1H, dd, J = 11.6, 3.2 Hz), 7.22 (1H, s), 6.81 (1H, t, J = 74.0 Hz), 6.68 (1H, d, J = 10.4 Hz), 4.23-4.35 (1H, m), 3.95-3.99 (2H, m), 3.38-3.95 (2H, m), 2.73-2.87 (1H, m), 1.64-1.74 (4H, m), 1.31 (6H, d, J = 8.8 Hz). | 415.2 | 414.20 |
| 162 | 1H-NMR (DMSO-D6) δ: 10.33 (1H, s), 9.33 (1H, s), 8.27 (2H, t, J = 7.1 Hz), 7.82 (1H, dd, J = 8.5, 2.2 Hz), 7.21 (1H, s), 6.94-6.66 (2H, m), 4.46-4.26 (3H, m), 3.88-3.83 (1H, m), 3.70-3.66 (1H, m), 3.60-3.50 (2H, m), 3.03 (3H, s), 2.22-2.12 (1H, br m), 1.89-1.82 (1H, m), 1.30 (6H, d, J = 6.3 Hz). | 508.24 | 507.19 |

**[Table 5-26]**

| Compound No. | NMR | LC/MS (M+H)⁺ | Exact Mass |
|---|---|---|---|
| 163 | 1H-NMR (DMSO-d6) δ: 10.42 (1H, s), 9.28 (1H, s), 8.39 (2H, s), 7.19 (1H, s), 6.80 (1H, t, J = 74.0 Hz), 6.71 (1H, d, J = 10.8 Hz), 4.20-4.31 (1H, m), 3.04-3.11 (4H, m), 2.84-2.87 (4H, m), 1.28 (6H, d, J = 8.4 Hz). | 416.2 | 415.20 |
| 165 | 1H-NMR (DMSO-D6) δ: 10.31 (1H, s), 9.33 (1H, s), 8.58 (1H, t, J = 5.6 Hz), 8.25-8.23 (2H, m), 7.74-7.72 (1H, m), 7.21 (1H, s), 6.86-6.71 (2H, m), 4.32-4.25 (3H, m), 2.53 (4H, d, J = 1.2 Hz), 1.60-1.55 (1H, m), 1.30 (6H, d, J = 6.3 Hz), 0.68-0.66 (4H, m). | | 427.19 |
| 166 | 1H-NMR (DMSO-D6) δ: 10.37 (1H, s), 9.34 (1H, s), 8.28-8.27 (2H, m), 7.76 (1H, dd, J = 8.5, 2.4 Hz), 7.21 (1H, s), 6.94-6.66 (2H, m), 4.54 (2H, s), 4.30-4.27 (1H, m), 4.10 (2H, s), 3.81 (2H, t, J = 5.1 Hz), 3.31-3.29 (2H, m), 2.53 (1H, d, J = 0.5 Hz), 1.30 (6H, d, J = 6.6 Hz). | | 443.19 |
| 167 | | 456.20 | 455.22 |
| 168 | | 472.2 | 471.22 |
| 169 | | 444.05 | 443.19 |
| 170 | | 506.2 | 505.21 |
| 171 | 1H-NMR (DMSO-D6) δ: 10.44 (1H, s), 9.27 (1H, s), 8.12 (1H, d, J = 9.8 Hz), 7.41 (1H, d, J = 9.8 Hz), 7.18 (1H, s), 6.78 (1H, t, J = 55.6 Hz), 6.58 (1H, d, J = 7.8 Hz), 4.29-4.21 (1H, m), 4.02-3.99 (2H, m), 3.74-3.70 (1H, m), 3.18-3.11 (2H, m), 1.83-1.79 (2H, m), 1.45-1.36 (2H, m), 1.27 (6H, d, J = 6.3 Hz). | | |

**[Table 5-27]**

| Compound No. | NMR | LC/MS (M+H)⁺ | Exact Mass |
|---|---|---|---|
| 177 | 1H-NMR (DMSO-D6) δ: 10.30 (1H, s), 9.33 (1H, s), 8.26-8.22 (2H, m), 7.76 (1H, d, J = 7.8 Hz), 7.21 (1H, s), 6.93-6.66 (2H, m), 4.29-4.27 (1H, br m), 3.46 (2H, br s), 2.37 (4H, br s), 1.50 (4H, br s), 1.39 (2H, br s), 1.30 (6H, d, J = 6.3 Hz). | | |
| 178 | 1H-NMR (DMSO-d6) δ: 10.28 (1H, s), 9.33 (1H, s), 8.20-8.25 (2H, m), 7.75 (1H, dd, J = 11.2, 2.8 Hz), 7.22 (1H, s), 6.81 (1H, t, J = 74.0 Hz), 6.68 (1H, d, J = 10.4 Hz), 4.53-4.57 (1H, m), 4.26-4.32 (1H, m), 3.92-3.97 (1H, m), 3.09-3.17 (1H, m), 2.72-2.81 (2H, m), 2.04 (3H, s), 1.46-1.85 (4H, m), 1.31 (6H, d, J = 8.8 Hz). | 456.1 | 455.22 |
| 179 | 1H-NMR (DMSO-d6) δ: 10.34 (1H, s), 9.40 (1H, s), 8.33 (1H, d J = 2.8 Hz), 8.29 (1H, d, J = 11.6), 7.82 (1H, dd, J = 11.6, 2.8 Hz), 7.29 (1H, s), 6.88 (1H, t, J = 74.0 Hz), 6.73 (1H, d, J = 10 Hz), 4.36-4.48 (1H, m), 3.72-3.79 (2H, m), 3.92-3.97 (1H, m), 2.98 (3H, s), 2.76-2.90 (3H, m), 1.72-1.98 (4H, m), 1.38 (6H, d, J = 8.8 Hz). | 492.3 | 491.19 |
| 181 | 1H-NMR (DMSO-D6) δ: 10.47 (1H, br s), 9.28 (1H, s), 8.31 (1H, s), 8.15 (1H, d, J = 9.8 Hz), 7.42 (1H, d, J = 10.2 Hz), 7.18 (1H, s), 6.78 (1H, t, J = 55.4 Hz), 6.59 (1H, d, J = 7.8 Hz), 4.29-4.21 (1H, m), 3.62-3.60 (2H, br m), 3.44 (2H, s), 3.04-3.03 (2H, br m), 1.27 (6H, d, J = 6.8 Hz), 1.20 (6H, s). | | |
| 185 | 1H-NMR (DMSO-D6) δ: 10.31 (1H, s), 9.33 (1H, s), 8.48 (1H, br s), 8.26 (1H, d, J = 8.5 Hz), 8.21 (1H, d, J = 2.0 Hz), 7.71 (1H, dd, J = 8.7, 2.3 Hz), 7.21 (1H, s), 6.94-6.66 (2H, m), 5.98 (2H, s), 4.38 (2H, s), 4.31-4.26 (1H, m), 2.76 (3H, s), 1.30 (6H, d, J = 6.6 Hz). | | |

**[Table 5-28]**

| Compound No. | NMR | LC/MS (M+H)⁺ | Exact Mass |
|---|---|---|---|
| 186 | 1H-NMR (DMSO-D6) δ: 10.28 (1H, s), 9.32 (1H, s), 8.50 (3H, s), 8.23-8.21 (2H, m), 7.73-7.72 (1H, m), 7.21 (1H, s), 6.94-6.66 (2H, m), 6.40 (1H, t, J = 6.1 Hz), 5.98 (1H, t, J = 5.7 Hz), 4.29-4.27 (1H, m), 4.18 (2H, d, J = 5.9 Hz), 3.03-2.98 (2H, m), 1.30 (6H, d, J = 6.6 Hz), 0.98 (3H, t, J = 7.2 Hz). | | |
| 187 | 1H-NMR (DMSO-D6) δ: 10.32 (1H, s), 9.33 (1H, s), 8.44 (2H, br s), 8.28-8.24 (2H, m), 7.82 (1H, dd, J = 8.8, 2.4 Hz), 7.21 (1H, s), 7.10 (1H, t, J = 6.2 Hz), 6.87-6.73 (4H, m), 4.30-4.28 (1H, m), 4.07 (2H, d, J = 6.3 Hz), 1.30 (7H, d, J = 6.3 Hz). | | |
| 188 | 1H-NMR (DMSO-D6) δ: 10.34 (1H, s), 9.33 (1H, s), 8.58 (1H, d, J = 2.2 Hz), 8.44 (1H, br s), 8.27 (1H, d, J = 8.8 Hz), 8.20 (1H, s), 8.01 (1H, dd, J = 8.8, 2.4 Hz), 7.92 (1H, s), 7.21 (1H, s), 6.94-6.66 (2H, m), 4.31-4.27 (1H, m), 3.87 (3H, s), 1.31 (7H, d, J = 6.3 Hz). | | |
| 189 | 1H-NMR (DMSO-D6) δ: 10.52 (1H, s), 9.33 (1H, s), 8.38 (1H, d, J = 2.0 Hz), 8.31 (1H, d, J = 8.8 Hz), 7.93 (1H, dd, J = 8.5, 2.2 Hz), 7.19 (1H, s), 6.91-6.63 (2H, m), 4.35-4.20 (2H, m), 4.13-4.10 (1H, br m), 3.40-3.37 (1H, br m), 3.13-3.09 (1H, br m), 2.49 (2H, s), 2.40-2.31 (1H, m), 1.99-1.91 (2H, m), 1.84-1.77 (1H, m), 1.27 (6H, d, J = 6.3 Hz). | 458.2 | 457.20 |
| 190 | 1H-NMR (DMSO-D6) δ: 10.63 (1H, s), 9.38 (1H, s), 8.45 (1H, d, J = 1.5 Hz), 8.38 (1H, d, J = 8.3 Hz), 8.00 (1H, dd, J = 8.8, 2.0 Hz), 7.24 (1H, s), 6.95-6.67 (2H, m), 4.40 (2H, br s), 4.33-4.25 (1H, m), 3.63-3.16 (4H, br m), 2.39-2.05 (3H, br m), 1.31 (6H, d, J = 6.3 Hz). | 458.2 | 457.20 |

**[Table 5-29]**

| Compound No. | NMR | LC/MS (M+H)⁺ | Exact Mass |
|---|---|---|---|
| 191 | 1H-NMR (DMSO-D6) δ: 10.42 (1H, s), 9.27 (1H, s), 8.11 (1H, d, J = 9.8 Hz), 7.40 (1H, d, J = 9.8 Hz), 7.18 (1H, s), 6.78 (1H, t, J = 55.6 Hz), 6.58 (1H, d, J = 7.8 Hz), 4.38 (2H, d, J = 13.7 Hz), 4.28-4.22 (1H, m), 3.33 (3H, br s), 2.76 (2H, t, J = 12.0 Hz), 1.79 (3H, d, J = 11.7 Hz), 1.45 (1H, t, J = 12.2 Hz), 1.28 (6H, d, J = 6.3 Hz), 1.05 (6H, s). | 458.2 | 472.25 |
| 192 | | 480.2 | 479.26 |
| 193 | | 510.25 | 509.28 |
| 194 | 1H-NMR (DMSO-D6) δ: 10.34 (1H, s), 9.34 (1H, s), 8.28 (1H, d, J = 8.8 Hz), 8.25 (1H, d, J = 1.5 Hz), 7.81 (1H, dd, J = 8.3, 2.0 Hz), 7.21 (1H, s), 6.94-6.66 (2H, m), 4.33-4.24 (1H, m), 3.88 (1H, d, J = 13.7 Hz), 3.56 (1H, d, J = 13.7 Hz), 3.15-3.06 (1H, m), 2.93-2.88 (1H, br m), 2.29-2.27 (1H, br m), 1.81 (1H, br s), 1.74-1.69 (1H, br m), 1.50 (3H, br s), 1.37-1.35 (1H, br m), 1.30 (6H, d, J = 6.3 Hz), 1.25-1.22 (1H, br m). | 472.38 | 471.22 |
| 195 | 1H-NMR (DMSO-d6) δ: 9.89 (1H, s), 9.18 (1H, s), 8.06 (1H, d J = 12.4 Hz), 8.04 (1H, d, J = 4.0 Hz), 7.41 (1H, dd, J = 12.4, 4.0 Hz), 6.96 (1H, s), 6.67 (1H, s), 5.18 (1H, d, J = 6.0 Hz), 4.60-4.64 (1H, m), 3.57 (2H, s), 3.38 (3H, s), 3.02-3.05 (4H, m), 2.83-2.86 (4H, m), 1.51 (3H, s), 1.39 (6H, d, J = 8.8 Hz). | 453.2 | 452.26 |

**[Table 5-30]**

| Compound No. | NMR | LC/MS (M+H)⁺ | Exact Mass |
|---|---|---|---|
| 196 | 1H-NMR (DMSO-D6) δ: 10.23 (1H, s), 9.21 (1H, s), 8.24-8.19 (2H, m), 7.74 (1H, d, J = 7.3 Hz), 6.96 (1H, s), 6.66 (1H, s) , 5.17 (1H, d, J = 4.4 Hz), 4.59 (1H, t, J = 5.6 Hz), 4.09 (1H, br s), 3.53 (2H, br s), 3.34 (3H, s), 3.28 (4H, br s), 3.01 (2H, br s), 2.49 (3H, d, J = 1.0 Hz), 1.68-1.66 (2H, br m), 1.47 (6H, s), 1.36 (3H, d, J = 6.3 Hz), 0.98 (6H, s) . | 524.4 | 523.33 |
| 197 | 1H-NMR (DMSO-D6) δ: 12.31 (1H, br s), 10.16 (1H, s), 9.23 (1H, s), 8.21-8.19 (2H, m), 7.71 (1H, dd, J = 8.5, 2.2 Hz), 6.99 (1H, s), 6.70 (1H, s), 5.20 (1H, br s), 4.62 (1H, q, J = 6.3 Hz), 3.56 (2H, s), 3.44 (2H, s), 3.38 (3H, s), 3.33-3.29 (2H, m), 2.77-2.74 (2H, br m), 2.22-2.13 (1H, m), 2.01-1.97 (2H, m), 1.79-1.76 (2H, br m), 1.50 (6H, s), 1.39 (3H, d, J = 6.3 Hz). | 510.25 | 509.28 |
| 198 | 1H-NMR (DMSO-D6) δ: 11.69 (1H, s), 10.28 (1H, s), 9.24 (1H, s), 8.27-8.23 (3H, m), 7.72 (1H, dd, J = 8.5, 2.2 Hz), 7.00 (1H, s), 6.72 (1H, s), 5.36 (1H, dd, J = 6.0, 2.1 Hz), 5.21 (1H, d, J = 4.6 Hz), 4.65-4.59 (1H, m), 4.53 (3H, s), 4.08 (3H, s), 3.79-3.76 (4H, m), 3.38 (4H, s), 3.32 (3H, s), 3.30-3.29 (2H, m), 1.99-1.62 (4H, m), 1.50 (6H, s), 1.39 (3H, d, J = 6.6 Hz). | | |
| 199 | 1H-NMR (DMSO-D6) δ: 11.69 (1H, s), 10.15 (1H, s), 9.25 (1H, s), 8.29-8.20 (3H, m), 7.74 (1H, dd, J = 8.7, 2.3 Hz), 6.89 (1H, s), 6.48 (1H, d, J = 7.6 Hz), 5.36 (1H, dd, J = 6.1, 2.0 Hz), 4.53 (2H, s), 4.28-4.25 (2H, m), 4.10 (3H, s), 3.77 (5H, tt, J = 12.4, 5.1 Hz), 3.27 (4H, s), 1.99-1.62 (4H, m), 1.38 (4H, d, J = 6.6 Hz), 1.30 (8H, dd, J = 6.3, 3.7 Hz). | | |

**[Table 5-31]**

| Compound No. | NMR | LC/MS (M+H)⁺ | Exact Mass |
|---|---|---|---|
| 200 | 1H-NMR (DMSO-D6) δ: 10.09 (1H, s), 9.25 (1H, s), 8.26 (1H, d, J = 8.3 Hz), 8.19 (1H, d, J = 1.5 Hz), 8.15 (1H, s), 7.74 (1H, dd, J = 8.5, 2.2 Hz), 6.89 (1H, s), 6.46 (1H, d, J = 7.8 Hz), 4.29-4.22 (2H, m), 3.44 (2H, s) , 3.27 (3H, s) , 2.89 (2H, d, J = 11.7 Hz), 1.88 (2H, t, J = 11.0 Hz), 1.64 (2H, d, J = 11.2 Hz), 1.38 (3H, d, J = 6.3 Hz), 1.30 (6H, dd, J = 6.3, 3.4 Hz), 1.24-1.14 (4H, m), 1.01 (6H, s). | 494.3 | 493.32 |
| 201 | | 439.2 | 438.25 |
| 202 | | 469.3 | 468.26 |
| 203 | 1H-NMR (DMSO-d6) δ: 10.61 (1H, s), 9.39 (1H, s), 8.63 (1H, d J = 3.2 Hz), 8.35 (1H, d J = 11.6 Hz), 8.14 (1H, dd, J = 12.0, 3.2 Hz), 7.49 (1H, s), 7.24 (1H, s), 6.82 (1H, t, J = 74.0 Hz), 6.72 (1H, d J = 10.4 Hz), 6.25 (1H, s), 4.28-4.34 (1H, m), 3.36-3.41 (2H, m), 2.73-2.78 (2H, m), 1.32 (6H, d, J = 8.4 Hz). | 426.3 | 425.18 |
| 209 | 1H-NMR (DMSO-D6) δ: 10.34 (1H, br s), 9.33 (1H, s), 8.63 (1H, d, J = 1.7 Hz), 8.27 (1H, d, J = 9.3 Hz), 8.13 (3H, s), 8.06 (1H, dd, J = 8.8, 2.4 Hz), 7.21 (1H, s), 6.94-6.66 (2H, m), 4.32-4.27 (1H, m), 2.53 (1H, s), 1.31 (5H, d, J = 6.3 Hz). | | |
| 210 | 1H-NMR (DMSO-D6) δ: 10.57 (1H, s), 9.36 (1H, s), 8.83 (1H, d, J = 2.7 Hz), 8.54 (1H, d, J = 2.4 Hz), 8.42 (1H, d, J = 9.0 Hz), 8.29 (1H, dd, J = 8.8, 2.7 Hz), 7.78 (1H, d, J = 1.7 Hz), 7.23 (1H, s), 6.95-6.67 (2H, m), 6.58 (1H, t, J = 1.8 Hz), 4.32-4.27 (1H, m), 1.32 (7H, d, J = 6.6 Hz). | | |

**[Table 5-32]**

| Compound No. | NMR | LC/MS (M+H)⁺ | Exact Mass |
|---|---|---|---|
| 211 | 1H-NMR (DMSO-D6) δ: 9.47 (1H, s), 8.14 (1H, s), 7.62 (1H, d, J = 9.0 Hz), 7.32 (1H, s), 6.98-6.69 (2H, m), 6.58-6.53 (1H, m), 6.47-6.40 (4H, m), 4.26-4.22 (2H, m), 1.23 (6H, d, J = 6.3 Hz). | | |
| 212 | 1H-NMR (DMSO-D6) δ: 10.73 (1H, br s), 9.25 (1H, s), 8.88 (2H, br s), 8.17 (1H, d, J = 9.8 Hz), 8.13 (1H, s), 7.58 (1H, d, J = 9.8 Hz), 7.01 (1H, s), 6.64 (1H, br s), 4.62 (1H, q, J = 6.3 Hz), 3.76-3.75 (4H, br m), 3.57 (2H, s), 3.33 (3H, s), 3.26 (4H, br s), 1.48 (6H, s), 1.39 (3H, d, J = 6.3 Hz). | 454.20 | 453.26 |
| 213 | 1H-NMR (DMSO-d6) δ: 10.36 (1H, s), 9.32 (1H, s), 8.25-8.31 (2H, m), 7.74 (1H, d, J = 6.8 Hz), 7.23 (1H, s), 6.81 (1H, t, J = 55.6 Hz), 6.70 (1H, d J = 8.0 Hz), 6.47 (1H, s), 4.25-4.37 (3H, m), 3.21 (4H, s), 1.32 (6H, d, J = 6.4 Hz). | 429.2 | 428.19 |
| 214 | 1H-NMR (DMSO-d6) δ: 9.86 (1H, s), 9.21 (1H, s), 8.13 (1H, d, J = 8.8 Hz), 8.05 (1H, d, J = 2.8 Hz), 7.50 (1H, dd, J = 9.2, 2.8 Hz), 6.88 (1H, s), 6.38 (1H, d J = 7.6 Hz), 4.24-4.29 (2H, m), 3.76-3.78 (4H, m), 3.28 (3H, s), 3.11-3.14 (4H, m), 1.39 (3H, d, J = 6.8 Hz), 1.31 (6H, apparent t, J = 3.2 Hz). | 424.2 | 423.24 |
| 215 | 1H-NMR (DMSO-d6) δ: 10.07 (1H, s), 9.21 (1H, s), 8.15-8.21 (2H, m), 7.46-7.68 (1H,m), 6.99 (1H, s), 6.70 (1H, s), 5.19 (1H, d, J = 6.4 Hz), 4.60-4.64 (1H, m), 3.56 (2H, s), 3.39 (3H, s), 3.03-3.07 (2H, m), 2.56-2.72 (3H, m), 1.70-1.74 (2H, m), 1.51-1.55 (8H, m), 1.39 (6H, d, J = 8.4 Hz). | 452.3 | 451.27 |

**[Table 5-33]**

| Compound No. | NMR | LC/MS (M+H)⁺ | Exact Mass |
|---|---|---|---|
| 216 | 1H-NMR (DMSO-D6) δ: 10.36 (1H, br s), 8.90 (1H, s), 7.92 (1H, d, J = 9.8 Hz), 7.20 (1H, d, J = 8.8 Hz), 6.77 (1H, s), 6.54 (1H, s), 5.07 (1H, br s), 4.55-4.54 (1H, br m), 3.73-3.72 (4H, br m), 3.52 (2H, s), 3.39-3.38 (4H, br m), 3.30 (3H, s), 1.44 (6H, s), 1.35 (3H, d, J = 6.8 Hz). | 455.20 | 454.24 |
| 217 | 1H-NMR (CDC13) δ: 9.00 (1H, s), 8.38 (1H, d, J = 9.8 Hz), 8.16 (1H, brs), 7.07 (1H, d, J = 9.8 Hz), 6.83 (1H, s), 6.09 (1H, d, J = 7.3 Hz), 4.46-4.35 (1H, m), 4.32 (1H, q, J = 6.5 Hz), 3.58 (4H, t, J = 5.1 Hz), 3.05 (4H, t, J = 4.9 Hz), 1.50 (3H, d, J = 6.3 Hz), 1.34 (6H, dd, J = 6.3, 5.4 Hz). | 424.25 | 423.25 |
| 218 | 1H-NMR (DMSO-d6) δ: 10.28 (1H, s), 9.38 (1H, s), 8.22-8.28 (2H, m), 7.80 (1H, dd, J = 11.6, 3.2 Hz), 7.60 (1H, s), 7.22 (1H, s), 6.81 (1H, t, J = 74.0 Hz), 6.69 (1H, d J = 10.4 Hz), 6.47 (1H, s), 4.24-4.35 (1H, m), 3.28-3.02 (2H, m), 3.06-3.22 (2H, m), 2.31-2.39 (2H, m), 1.82-1.90 (2H, m), 1.31 (6H, d, J = 8.4 Hz). | 428.2 | 427.19 |
| 219 | 1H-NMR (DMSO-d6) δ: 10.31 (1H, s), 9.34 (1H, s), 8.26 (1H, d, J = 8.4 Hz), 8.24 (1H, d, J = 1.6 Hz), 7.74 (1H, dd, J = 8.4, 2.0 Hz), 7.22 (1H, s), 6.81 (1H, t, J = 55.6 Hz), 6.72 (1H, d J = 8.0 Hz),4.42 (1H, s), 4.27-4.32 (1H, m), 3.13-3.18 (4H, m), 1.76-1.82 (2H, m), 1.31 (6H, d, J = 6.4 Hz). | 443.2 | 442.20 |
| 220 | 1H-NMR (CDC13) δ: 9.04 (1H, s), 8.35 (1H, d, J = 8.8 Hz), 8.27 (2H, d, J = 2.4 Hz), 7.75 (1H, dd, J = 8.5, 2.2 Hz), 6.84 (1H, s), 6.14 (1H, d, J = 7.3 Hz), 4.47-4.36 (1H, m), 4.34 (1H, q, J = 6.5 Hz), 3.49 (2H, s), 3.41 (3H, s), 2.91 (4H, t, J = 4.6 Hz), 2.46 (4H, brs), 1.51 (3H, d, J = 6.3 Hz), 1.36 (6H, t, J = 5.9 Hz). | 437.35 | 436.27 |

**[Table 5-34]**

| Compound No. | NMR | LC/MS (M+H)⁺ | Exact Mass |
|---|---|---|---|
| 221 | 1H-NMR (CDC13) δ: 9.00 (1H, s), 8.36 (1H, d, J = 8.8 Hz), 8.26 (2H, d, J = 3.7 Hz), 7.73 (1H, dd, J = 8.5, 2.2 Hz), 6.91 (1H, s), 6.71 (1H, s), 4.81 (1H, q, J = 6.3 Hz), 3.62 (2H, dd, J = 10.5, 9.0 Hz), 3.50 (3H, s), 3.49 (2H, s), 2.90 (4H, t, J = 4.9 Hz), 2.44 (4H, brs), 1.60 (6H, s), 1.54 (3H, d, J = 6.3 Hz). | 467.35 | 466.28 |
| 222 | 1H-NMR (DMSO-d6) δ: 10.13 (1H, s), 9.23 (1H, s), 8.16-8.20 (2H, m), 7.74 (1H, m), 7.50 (1H, br s), 7.00 (1H, s), 6.92 (1H, s), 6.71 (1H, s), 5.20 (1H, d, J = 6.4 Hz), 4.62-4.69 (1H, m), 3.57 (2H, s), 3.39 (5H, s), 1.51 (6H, s), 1.40 (3H, d, J = 8.4 Hz). | 426.2 | 425.22 |
| 223 | 1H-NMR (DMSO-d6) δ: 10.00 (1H, s), 9.25 (1H, s), 8.18-8.22 (2H, m), 7.70 (1H, m), 6.89 (1H, s), 6.44 (1H, d, J = 6.8 Hz), 4.69 (1H, br s), 4.25-4.26 (2H, br m), 3.61-3.63 (2H, br m), 2.71(2H, br s), 1.39 (3H, d, J = 6.0 Hz), 1.31 (6H, br s). | 383.2 | 382.21 |
| 224 | 1H-NMR (DMSO-d6) δ: 10.03 (1H, s), 9.25 (1H, s), 8.22-8.24 (2H, m), 7.74 (1H, dd, J = 8.8, 2.4 Hz), 6.90 (1H, s), 6.44 (1H, d, J = 8.0 Hz), 4.53-4.57 (1H, br m), 4.23-4.29 (2H, br m), 3.92-3.96 (1H, br m), 3.31(3H, s), 3.10-3.16 (1H, m), 2.77-2.81 (1H, m), 2.51-2.67 (1H, m), 2.04 (3H, s), 1.77-1.86 (1H, m), 1.62-1.66 (1H, m), 1.46-1.50 (1H, m), 1.39 (3H, d, J = 6.4 Hz), 1.32 (6H, m). | 464.3 | 463.27 |
| 225 | 1H-NMR (DMSO-d6) δ: 10.10 (1H, s), 9.23 (1H, s), 8.24 (1H, d, J = 2.4 Hz), 8.16 (1H, d, J = 11.2 Hz), 7.72 (1H, m), 7.00 (1H, s), 6.69 (1H, s), 5.20 (1H, 6.4 Hz), 4.50-4.68 (2H, br m), 3.92-3.96 (1H, br m), 3.58 (2H, s), 3.38 (3H, s), 3.09-3.16 (1H, m), 2.71-2.81 (1H, m), 2.61-2.67 (1H, m), 2.04 (3H, s), 1.77-1.86 (2H, m), 1.51-1.62 (1H, m), 1.51 (6H, s), 1.40 (3H, d, J = 8.4 Hz). | 494.2 | 493.28 |

**[Table 5-35]**

| Compound No. | NMR | LC/MS (M+H)⁺ | Exact Mass |
|---|---|---|---|
| 226 | 1H-NMR (DMSO-D6) δ: 10.41 (1H, s), 9.27 (1H, s), 8.11 (1H, d, J = 9.8 Hz), 7.37 (1H, d, J = 10.2 Hz), 7.18 (1H, s), 6.78 (1H, t, J = 55.4 Hz), 6.58 (1H, d, J = 7.8 Hz), 4.87 (1H, d, J = 4.4 Hz), 4.28-4.23 (1H, m), 4.15-4.11 (1H, m), 3.95-3.92 (1H, m), 3.56-3.55 (1H, m), 3.05-3.02 (1H, m), 2.84-2.81 (1H, m), 1.89-1.87 (1H, br m), 1.76-1.73 (1H, br m), 1.49-1.38 (2H, m), 1.28 (6H, d, J = 6.8 Hz). | | |
| 227 | 1H-NMR (DMSO-D6) δ: 9.96 (1H, s), 9.21 (1H, d, J = 1.5 Hz), 8.17 (1H, d, J = 9.3 Hz), 8.06 (1H, d, J = 2.4 Hz), 7.51 (1H, dd, J = 9.3, 2.9 Hz), 6.87 (1H, s), 6.39 (1H, d, J = 7.3 Hz), 4.29-4.23 (2H, m), 4.15 (2H, t, J = 5.4 Hz), 3.27 (3H, d, J = 1.5 Hz), 2.77 (2H, t, J = 5.4 Hz), 2.32 (6H, s), 1.38 (3H, d, J = 6.3 Hz), 1.29 (6H, dd, J = 6.3, 3.4 Hz). | 426.3 | 425.25 |
| 228 | 1H-NMR (DMSO-d6) δ: 10.52 (1H, s), 9.24 (1H, s), 8.19 (1H, s), 8.13 (1H, d, J = 8.0 Hz), 7.71 (1H, d, J = 8.8 Hz), 7.00 (1H, s), 4.60-4.65 (1H, br m), 3.57-3.63 (4H, m), 3.37 (3H, s), 2.67-2.74 (2H, m), 1.51 (6H, s), 1.40 (3H, d, J = 6.8 Hz). | 413.2 | 412.22 |
| 229 | 1H-NMR (DMSO-D6) δ: 9.95 (1H, s), 9.23 (1H, s), 8.20 (1H, d, J = 8.3 Hz), 8.18 (1H, d, J = 2.0 Hz), 7.70 (1H, dd, J = 8.5, 2.2 Hz), 6.98 (1H, s), 6.39 (1H, d, J = 7.3 Hz), 5.18 (1H, d, J = 4.4 Hz), 4.64-4.58 (1H, m), 4.26 (1H, td, J = 13.3, 6.2 Hz), 4.00 (2H, s), 3.79 (2H, t, J = 5.1 Hz), 3.38-3.32 (4H, m), 2.55 (2H, t, J = 7.6 Hz), 1.85-1.78 (2H, m), 1.38 (3H, d, J = 6.8 Hz), 1.29 (6H, dd, J = 6.6, 1.7 Hz). | 466.25 | 465.25 |

**[Table 5-36]**

| Compound No. | NMR | LC/MS (M+H)⁺ | Exact Mass |
|---|---|---|---|
| 230 | 1H-NMR (DMSO-D6) δ: 10.17 (1H, s), 9.26 (1H, s), 8.33-8.26 (2H, m), 7.80 (1H, dd, J = 8.8, 2.4 Hz), 6.99 (1H, s), 6.42 (1H, d, J = 7.3 Hz), 5.19 (1H, d, J = 4.4 Hz), 4.68-4.55 (1H, m), 4.31-4.21 (1H, m), 3.63 (2H, t, J = 5.4 Hz), 3.40 (2H, s), 3.03 (2H, t, J = 5.1 Hz), 1.39 (3H, d, J = 6.3 Hz), 1.30 (6H, dd, J = 6.3, 2.0 Hz). | 423.25 | 422.22 |
| 231 | 1H-NMR (DMSO-d6) δ: 9.25 (1H, s), 8.23 (1H, d, J = 8.4 Hz), 8.19 (1H, d, J = 2.0 Hz), 7.72 (1H, dd, J = 8.8, 2.4 Hz), 6.90 (1H, s), 6.51 (1H, d, J = 6.8 Hz), 4.24-4.30 (3H, m), 3.39 (2H, s), 3.23 (3H, s), 1.39 (6H, d, J = 6.4 Hz), 1.24-1.32 (3H, m). | 396.2 | 395.21 |
| 232 | 1H-NMR (DMSO-d6) δ: 10.00 (1H, s), 9.24 (1H, s), 8.21 (1H, d, J = 8.4 Hz), 8.19 (1H, d, J = 2.0 Hz), 7.70 (1H, dd, J = 8.4, 2.4 Hz), 6.89 (1H, s), 6.45 (1H, d, J = 7.6 Hz), 4.25-4.28 (2H, m), 3.30 (3H, s), 3.02-3.04 (2H, m), 2.52-2.61(3H, m), 1.69-1.72 (2H, m), 1.52-1.55 (2H, m), 1.39 (3H, d, J = 6.8 Hz), 1.30-1.32 (6H, m). | 422.2 | 421.26 |
| 233 | 1H-NMR (DMSO-D6) δ: 10.09 (1H, br s), 9.26 (1H, s), 8.25-8.17 (2H, m), 7.78 (1H, br s), 6.99 (1H, s), 6.43 (1H, d, J = 7.8 Hz), 5.21 (1H, d, J = 4.9 Hz), 4.61 (1H, t, J = 5.4 Hz), 4.28-4.25 (1H, m), 3.58-3.45 (4H, m), 2.37 (3H, br s), 1.38 (3H, d, J = 6.8 Hz), 1.30-1.29 (6H, m). | | |
| 234 | 1H-NMR (DMSO-D6) δ: 10.07 (1H, s), 9.25 (1H, s), 8.28-8.24 (2H, m), 7.77-7.75 (2H, m), 6.99 (1H, s), 6.42 (1H, d, J = 6.8 Hz), 5.18 (1H, s), 4.61 (1H, t, J = 5.4 Hz), 4.29-4.22 (1H, m), 3.53 (2H, s), 3.14 (2H, s), 2.93 (2H, s), 2.56 (3H, t, J = 4.9 Hz), 1.38 (4H, d, J = 6.8 Hz), 1.30 (6H, d, J = 4.9 Hz). | | |

**[Table 5-37]**

| Compound No. | NMR | LC/MS (M+H)⁺ | Exact Mass |
|---|---|---|---|
| 235 | 1H-NMR (DMSO-D6) δ: 10.08 (1H, s), 9.15 (1H, s), 7.94 (1H, d, J = 9.3 Hz), 7.00-6.93 (2H, m), 6.66 (1H, t, J = 5.6 Hz), 6.58 (1H, s), 5.16 (1H, d, J = 4.4 Hz), 4.77 (1H, t, J = 5.4 Hz), 4.63-4.57 (1H, m), 3.58 (2H, q, J = 5.4 Hz), 3.51 (2H, s), 3.40 (2H, q, J = 5.9 Hz), 3.34 (3H, s), 1.47 (6H, s), 1.38 (3H, d, J = 6.8 Hz). | 429.25 | 428.23 |
| 236 | 1H-NMR (DMSO-D6) δ: 10.33 (1H, s), 9.34 (1H, s), 8.28-8.24 (2H, m), 7.79 (1H, dd, J = 8.3, 2.0 Hz), 7.21 (1H, s), 6.94-6.66 (2H, m), 4.31-4.24 (1H, m), 3.57 (2H, br s), 3.11 (3H, s), 2.91-2.89 (2H, br m), 2.23-2.20 (1H, br m), 2.09-2.06 (2H, br m), 1.77-1.74 (2H, br m), 1.57-1.55 (2H, br m), 1.31 (6H, d, J = 6.3 Hz). | 549.62 | 548.21 |
| 237 | | 451.61 | 450.25 |
| 238 | 1H-NMR (DMSO-D6) δ: 10.31 (1H, s), 9.35 (1H, s), 8.26-8.17 (2H, m), 7.75 (1H, dd, J = 8.3, 2.0 Hz), 6.62 (1H, d, J = 7.8 Hz), 5.13-5.07 (1H, m), 4.79 (1H, d, J = 6.8 Hz), 4.45-4.30 (1H, m), 3.41 (2H, s), 2.67 (4H, t, J = 4.4 Hz), 2.29 (4H, brs), 1.38 (3H, d, J = 6.3 Hz), 1.31 (6H, t, J = 6.6 Hz). | 457.20 | 456.22 |
| 239 | 1H-NMR (DMSO-D6) δ: 10.12 (1H, s), 9.25 (1H, s), 8.27 (1H, d, J = 8.8 Hz), 8.21-8.18 (1H, m), 7.74 (1H, d, J = 8.8 Hz), 6.89 (1H, s), 6.45 (1H, d, J = 7.3 Hz), 4.29-4.22 (2H, m), 3.47 (2H, s), 3.27 (3H, s), 2.50-2.36 (8H, m), 1.45-1.42 (2H, m), 1.38 (3H, d, J = 6.3 Hz), 1.30 (6H, t, J = 4.9 Hz), 0.82 (3H, t, J = 7.3 Hz). | | |

**[Table 5-38]**

| Compound No. | NMR | LC/MS (M+H)⁺ | Exact Mass |
|---|---|---|---|
| 240 | 1H-NMR (DMSO-D6) δ: 10.09 (1H, s), 9.25 (1H, s), 8.27 (1H, d, J = 8.8 Hz), 8.18 (1H, d, J = 12.2 Hz), 7.74 (1H, d, J = 8.8 Hz), 6.89 (1H, s), 6.46 (1H, d, J = 7.8 Hz), 4.30-4.22 (2H, m), 3.45 (2H, s), 3.27 (3H, s), 2.42-2.34 (10H, m), 1.38 (3H, d, J = 6.3 Hz), 1.30 (6H, dd, J = 6.1, 3.7 Hz), 0.98 (3H, t, J = 7.1 Hz). | | |
| 241 | 1H-NMR (DMSO-D6) δ: 10.28 (1H, s), 9.28 (1H, s), 8.37-8.35 (2H, m), 7.94 (1H, dd, J = 8.8, 2.0 Hz), 7.01 (1H, s), 6.44 (1H, d, J = 6.8 Hz), 5.22 (1H, d, J = 3.9 Hz), 4.62 (1H, t, J = 5.4 Hz), 4.28-4.25 (1H, m), 4.13 (2H, s), 3.58 (2H, t, J = 5.4 Hz), 3.30 (6H, s), 3.09 (2H, t, J = 4.9 Hz), 1.39 (3H, d, J = 6.8 Hz), 1.30 (6H, dd, J = 6.8, 2.0 Hz). | | |
| 242 | 1H-NMR (DMSO-D6) δ: 10.51 (1H, s), 9.29 (1H, s), 8.93 (2H, s), 8.42 (1H, s), 8.28 (1H, d, J = 8.8 Hz), 7.95 (1H, d, J = 6.8 Hz), 7.03 (1H, s), 6.72 (1H, s), 4.64 (1H, d, J = 5.9 Hz), 4.18 (2H, s), 3.59 (4H, s), 3.40 (3H, s), 3.31 (3H, s), 3.13 (2H, s), 1.51 (6H, s), 1.40 (3H, d, J = 5.9 Hz). | | |
| 243 | 1H-NMR (DMSO-D6) δ: 10.20 (1H, s), 9.23 (1H, s), 8.19 (2H, t, J = 12.9 Hz), 7.71 (1H, dd, J = 8.8, 2.0 Hz), 6.99 (1H, s), 6.70 (1H, s), 4.62 (1H, q, J = 6.5 Hz), 3.56 (2H, s), 3.38 (3H, s), 3.16 (2H, s), 2.49-2.44 (8H, br m), 2.32 (2H, t, J = 7.6 Hz), 1.50 (6H, s), 1.45-1.39 (5H, m), 0.82 (3H, t, J = 7.3 Hz). | | |

**[Table 5-39]**

| Compound No. | NMR | LC/MS (M+H)⁺ | Exact Mass |
|---|---|---|---|
| 244 | 1H-NMR (DMSO-D6) δ: 10.17 (1H, s), 9.23 (1H, d, J = 4.9 Hz), 8.19 (2H, t, J = 12.4 Hz), 7.71 (1H, dd, J = 8.5, 1.7 Hz), 6.99 (1H, s), 6.70 (1H, s), 4.62 (1H, q, J = 6.3 Hz), 3.56 (2H, s), 3.46 (2H, s), 3.38 (3H, s), 2.40-2.35 (8H, br m), 1.50 (6H, s), 1.40 (3H, d, J = 6.3 Hz), 0.99 (3H, t, J = 7.3 Hz). | | |
| 245 | 1H-NMR (DMSO-D6) δ: 9.89 (1H, s), 9.20 (1H, d, J = 1.0 Hz), 8.16 (2H, dd, J = 5.1, 4.1 Hz), 8.04 (1H, d, J = 2.9 Hz), 7.50 (1H, dd, J = 9.3, 2.9 Hz), 6.96 (1H, s), 6.35 (1H, d, J = 7.8 Hz), 4.60 (1H, q, J = 6.3 Hz), 4.27-4.22 (1H, m), 4.12 (2H, t, J = 5.9 Hz), 2.65 (2H, t, J = 5.6 Hz), 2.24 (6H, s), 1.38 (3H, d, J = 6.3 Hz), 1.29 (6H, d, J = 6.3 Hz). | | |
| 246 | 1H-NMR (DMSO-D6) δ: 10.71 (1H, brs), 9.28 (1H, s), 8.53 (1H, d, J = 9.3 Hz), 7.70 (1H, d, J = 9.3 Hz), 6.91 (1H, s), 6.50 (1H, d, J = 7.3 Hz), 4.30-4.23 (2H, m), 3.68 (2H, s), 3.28 (3H, s), 2.68 (4H, t, J = 4.6 Hz), 2.34 (4H, s), 1.39 (3H, d, J = 6.3 Hz), 1.30 (6H, dd, J = 6.3, 3.9 Hz). | 438.30 | 437.27 |
| 247 | 1H-NMR (DMSO-D6) δ: 10.45 (1H, s), 9.36 (1H, s), 8.37-8.25 (2H, m), 7.82 (1H, dd, J = 8.8, 2.4 Hz), 6.64 (1H, d, J = 7.3 Hz), 5.14-5.07 (1H, m), 4.80 (1H, d, J = 6.3 Hz), 4.42-4.30 (1H, m), 3.64 (2H, t, J = 5.4 Hz), 3.41 (2H, s), 3.03 (2H, t, J = 5.4 Hz), 1.38 (3H, d, J = 6.3 Hz), 1.31 (6H, t, J = 6.6 Hz). | 457.20 | 456.18 |

**[Table 5-40]**

| Compound No. | NMR | LC/MS (M+H)⁺ | Exact Mass |
|---|---|---|---|
| 249 | 1H-NMR (DMSO-D6) δ: 10.22 (1H, s), 9.20 (1H, s), 8.17 (1H, d, J = 9.8 Hz), 7.39 (1H, d, J = 9.8 Hz), 6.87 (1H, s), 6.36 (1H, d, J = 7.3 Hz), 4.43 (1H, t, J = 5.4 Hz), 4.28-4.17 (2H, m), 3.55 (2H, t, J = 5.9 Hz), 3.50 (4H, t, J = 5.1 Hz), 3.26 (3H, s), 2.55 (4H, t, J = 4.9 Hz), 2.44 (2H, t, J = 6.3 Hz), 1.37 (3H, d, J = 6.3 Hz), 1.27 (6H, dd, J = 6.3, 3.4 Hz). | 468.25 | 467.28 |
| 250 | 1H-NMR (CDC13) δ: 9.02 (1H, s), 8.34 (1H, d, J = 9.8 Hz), 8.28 (1H, br s), 7.07 (1H, d, J = 10.2 Hz), 6.74 (1H, s), 6.30 (1H, d, J = 7.8 Hz), 4.80 (1H, q, J = 6.3 Hz), 4.38-4.27 (1H, m), 4.08-4.02 (2H, m), 3.67-3.57 (6H, m), 3.07-3.02 (4H, m), 2.18-2.12 (2H, m), 1.74-1.63 (2H, m), 1.53 (3H, d, J = 6.3 Hz). | | |
| 251 | 1H-NMR (CDC13) δ: 9.03 (1H, s), 8.33 (1H, d, J = 10.2 Hz), 8.27 (1H, br s), 7.07 (1H, d, J = 9.8 Hz), 6.77 (1H, s), 6.46 (1H, d, J = 6.8 Hz), 4.84-4.77 (2H, m), 4.12-4.03 (2H, m), 3.96-3.84 (2H, m), 3.61-3.57 (4H, m), 3.06-3.03 (4H, m), 2.49-2.39 (1H, m), 2.07-1.98 (1H, m), 1.54 (3H, d, J = 6.8 Hz). | | |
| 252 | 1H-NMR (CDC13) δ: 9.02 (1H, s), 8.34 (1H, d, J = 10.2 Hz), 8.27 (1H, br s), 7.07 (1H, d, J = 9.8 Hz), 6.76 (1H, s), 6.46 (1H, d, J = 6.8 Hz), 4.84-4.76 (2H, m), 4.12-4.03 (2H, m), 3.96-3.84 (2H, m), 3.62-3.58 (4H, m), 3.07-3.04 (4H, m), 2.48-2.39 (1H, m), 2.07-1.97 (1H, m), 1.53 (3H, d, J = 6.3 Hz). | | |

**[Table 5-41]**

| Compound No. | NMR | LC/MS (M+H)⁺ | Exact Mass |
|---|---|---|---|
| 253 | 1H-NMR (CDC13) δ: 9.02 (1H, s), 8.40 (1H, d, J = 9.8 Hz), 8.30 (1H, br s), 7.07 (1H, d, J = 10.2 Hz), 6.73 (1H, s), 6.65 (1H, d, J = 8.3 Hz), 4.79 (1H, q, J = 6.3 Hz), 4.36-4.29 (1H, m), 4.01 (1H, dd, J = 11.2, 2.9 Hz), 3.78-3.74 (2H, m), 3.66-3.58 (5H, m), 3.08-3.04 (4H, m), 2.09-2.01 (1H, m), 1.93-1.84 (2H, m), 1.73-1.66 (1H, m), 1.53 (3H, d, J = 6.3 Hz). | | |
| 254 | 1H-NMR (DMSO-D6) δ: 10.22 (1H, s), 9.20 (1H, s), 8.17 (1H, d, J = 9.8 Hz), 7.39 (1H, d, J = 9.8 Hz), 6.87 (1H, s), 6.36 (1H, d, J = 7.3 Hz), 4.43 (1H, t, J = 5.4 Hz), 4.28-4.17 (2H, m), 3.55 (2H, t, J = 5.9 Hz), 3.50 (4H, t, J = 5.1 Hz), 3.26 (3H, s), 2.55 (4H, t, J = 4.9 Hz), 2.44 (2H, t, J = 6.3 Hz), 1.37 (3H, d, J = 6.3 Hz), 1.27 (6H, dd, J = 6.3, 3.4 Hz). | | |
| 255 | 1H-NMR (CDC13) δ: 9.07 (1H, s), 8.48 (1H, br s), 8.33 (1H, d, J = 8.8 Hz), 8.30 (1H, d, J = 2.4 Hz), 7.73 (1H, dd, J = 8.8, 2.4 Hz), 6.76 (1H, s), 6.36 (1H, d, J = 7.3 Hz), 4.82 (1H, q, J = 6.3 Hz), 4.39-4.29 (1H, m), 4.10-4.03 (2H, m), 3.69-3.61 (2H, m), 3.54 (2H, s), 3.09-3.04 (4H, m), 2.65-2.57 (4H, m), 2.21-2.15 (2H, m), 1.76-1.65 (2H, m), 1.54 (3H, d, J = 6.3 Hz). | | |
| 256 | 1H-NMR (CDC13) δ: 9.04 (1H, s), 8.38 (1H, d, J = 8.8 Hz), 8.28-8.24 (2H, m), 7.71 (1H, dd, J = 8.5, 2.2 Hz), 6.75 (1H, s), 6.70 (1H, d, J = 8.3 Hz), 4.80 (1H, q, J = 6.2 Hz), 4.39-4.31 (1H, m), 4.02 (1H, dd, J = 11.2, 2.9 Hz), 3.80-3.75 (2H, m), 3.65 (1H, dd, J = 11.0, 5.6 Hz), 3.56 (2H, s), 3.19-3.10 (4H, m), 2.74-2.64 (4H, m), 2.09-2.02 (1H, m), 1.95-1.85 (2H, m), 1.76-1.67 (1H, m), 1.53 (3H, d, J = 6.3 Hz). | 465.35 | 464.26 |

**[Table 5-42]**

| Compound No. | NMR | LC/MS (M+H)⁺ | Exact Mass |
|---|---|---|---|
| 257 | 1H-NMR (CDC13) δ: 9.06 (1H, s), 8.44 (1H, br s), 8.37 (1H, d, J = 8.3 Hz), 8.29 (1H, d, J = 2.0 Hz), 7.73 (1H, dd, J = 8.3, 2.4 Hz), 6.74 (1H, s), 6.69 (1H, d, J = 8.3 Hz), 4.80 (1H, q, J = 6.3 Hz), 4.38-4.31 (1H, m), 4.03 (1H, dd, J = 11.2, 2.9 Hz), 3.80-3.75 (2H, m), 3.64 (1H, dd, J = 11.2, 5.9 Hz), 3.52 (2H, s), 3.04-3.00 (4H, m), 2.62-2.51 (4H, m), 2.11-2.03 (1H, m), 1.95-1.84 (2H, m), 1.76-1.68 (1H, m), 1.53 (3H, d, J = 6.8 Hz). | | |
| 258 | 1H-NMR (CDC13) δ: 9.06 (1H, s), 8.39 (1H, br s), 8.31 (1H, d, J = 8.8 Hz), 8.28 (1H, d, J = 2.0 Hz), 7.74 (1H, dd, J = 8.5, 2.2 Hz), 6.79 (1H, s), 6.50 (1H, d, J = 6.8 Hz), 4.86-4.78 (2H, m), 4.14-4.05 (2H, m), 3.97-3.85 (2H, m), 3.51 (2H, s), 2.99-2.95 (4H, m), 2.55-2.41 (5H, m), 2.08-2.00 (1H, m), 1.54 (3H, d, J = 6.3 Hz). | | |
| 259 | 1H-NMR (CDC13) δ: 9.07 (1H, s), 8.42 (1H, br s), 8.31 (1H, d, J = 8.8 Hz), 8.29 (1H, d, J = 2.4 Hz), 7.75 (1H, dd, J = 8.5, 2.2 Hz), 6.78 (1H, s), 6.50 (1H, d, J = 6.8 Hz), 4.85-4.78 (2H, m), 4.14-4.05 (2H, m), 3.98-3.86 (2H, m), 3.51 (2H, s), 2.98-2.93 (4H, m), 2.55-2.41 (5H, m), 2.08-1.99 (1H, m), 1.54 (3H, d, J = 6.3 Hz). | | |
| 260 | 1H-NMR (DMSO-D6) δ: 10.14 (1H, s), 9.19 (1H, s), 8.14 (1H, t, J = 4.9 Hz), 7.40 (1H, d, J = 9.8 Hz), 6.96 (1H, s), 6.31 (1H, d, J = 7.8 Hz), 4.60 (1H, q, J = 6.5 Hz), 4.23 (1H, dd, J = 13.4, 6.6 Hz), 4.02-3.99 (2H, br m), 3.72-3.70 (1H, br m), 3.17-3.10 (2H, m), 1.83-1.80 (2H, br m), 1.43 (2H, dd, J = 16.1, 6.8 Hz), 1.37 (3H, d, J = 6.3 Hz), 1.27 (6H, d, J = 6.3 Hz). | | |

**[Table 5-43]**

| Compound No. | NMR | LC/MS (M+H) ⁺ | Exact Mass |
|---|---|---|---|
| 261 | 1H-NMR (DMSO-D6) δ: 10.15 (1H, brs), 9.17 (1H, s), 8.14 (1H, d, J = 9.8 Hz), 7.34 (1H, d, J = 9.8 Hz), 6.94 (1H, s), 6.31 (1H, d, J = 7.8 Hz), 5.15 (1H, d, J = 3.4 Hz), 4.60 (1H, dt, J = 11.1, 4.6 Hz), 4.27-4.18 (1H, m), 3.42 (4H, t, J = 5.1 Hz), 2.81 (4H, brs), 2.29 (1H, s), 1.37 (3H, d, J = 6.3 Hz), 1.27 (6H, d, J = 6.3 Hz). | 410.25 | 409.23 |
| 263 | 1H-NMR (DMSO-D6) δ: 10.05 (1H, s), 9.24 (1H, s), 8.29-8.25 (3H, m), 7.83 (1H, d, J = 8.8 Hz), 6.99 (1H, s), 6.40 (1H, d, J = 6.8 Hz), 4.61 (1H, q, J = 6.2 Hz), 4.29-4.25 (1H, m), 2.87 (2H, s), 2.35 (4H, s), 1.38 (3H, d, J = 6.8 Hz), 1.34 (6H, s), 1.30 (8H, d, J = 5.9 Hz). | | |
| 264 | 1H-NMR (DMSO-D6) δ: 10.18 (1H, s), 9.23 (1H, s), 8.33 (1H, d, J = 2.0 Hz), 8.26 (1H, s), 8.19 (1H, d, J = 8.8 Hz), 7.79 (1H, dd, J = 8.8, 2.0 Hz), 6.99 (1H, s), 6.72 (1H, s), 4.62 (1H, q, J = 6.2 Hz), 3.55 (3H, s), 2.77 (2H, s), 2.30 (3H, s), 1.51 (6H, s), 1.39 (3H, d, J = 6.8 Hz), 1.31 (6H, s). | | |
| 265 | 1H-NMR (DMSO-D6) δ: 10.22 (1H, s), 9.23 (1H, s), 8.23-8.21 (3H, m), 7.72 (1H, d, J = 8.8 Hz), 6.99 (1H, s), 6.71 (1H, s), 5.23 (1H, s), 4.63-4.61 (1H, m), 3.58-3.56 (4H, m), 3.38 (3H, s), 2.88-2.86 (4H, m), 1.50 (6H, s), 1.39 (3H, d, J = 6.8 Hz), 1.33 (3H, d, J = 6.8 Hz). | | |
| 266 | 1H-NMR (DMSO-D6) δ: 10.11 (1H, s), 9.25 (1H, s), 8.36 (2H, s), 8.26 (1H, d, J = 8.8 Hz), 8.20 (1H, s), 7.75 (1H, d, J = 8.8 Hz), 6.89 (1H, s), 6.46 (1H, d, J = 6.8 Hz), 4.29-4.22 (3H, m), 3.50 (7H, d, J = 6.8 Hz), 3.27 (6H, s), 2.80 (4H, s), 2.38-2.36 (6H, br m), 1.38 (3H, d, J = 5.9 Hz), 1.33-1.29 (6H, m). | | |

**[Table 5-44]**

| Compound No. | NMR | LC/MS (M+H) ⁺ | Exact Mass |
|---|---|---|---|
| 269 | 1H-NMR (DMSO-D6) δ: 10.38 (1H, s), 9.33 (1H, s), 8.49 (1H, d, J = 9.8 Hz), 7.40-7.33 (2H, m), 5.46-5.39 (1H, m), 5.36 (1H, d, J = 4.4 Hz), 4.72-4.64 (1H, m), 3.43 (4H, dd, J = 5.6, 4.1 Hz), 2.81 (4H, dd, J = 5.9, 3.9 Hz), 1.41 (9H, dd, J = 5.9, 2.4 Hz). | 411.20 | 410.22 |
| 270 | 1H-NMR (DMSO-D6) δ: 10.11 (1H, s), 9.25 (1H, s) , 8.26 (1H, d, J = 8.8 Hz), 8.20 (1H, t, J = 6.3 Hz), 7.75 (1H, dd, J = 8.8, 2.0 Hz), 6.89 (1H, s), 6.46 (1H, d, J = 7.8 Hz), 4.27-4.20 (2H, m), 3.56-3.53 (2H, m), 3.27 (5H, s), 2.70-2.67 (1H, m), 2.59-2.57 (1H, m), 2.44-2.41 (1H, m), 2.33-2.30 (1H, m), 2.01-1.97 (1H, m), 1.57-1.52 (1H, m), 1.38 (3H, d, J = 5.9 Hz), 1.30 (6H, dd, J = 5.9, 3.9 Hz). | | |
| 271 | 1H-NMR (DMSO-D6) δ: 10.12 (1H, s), 9.25 (1H, s), 8.27 (1H, d, J = 8.8 Hz), 8.22 (1H, br s), 7.77 (1H, dd, J = 8.8, 2.0 Hz), 6.89 (1H, s), 6.46 (1H, d, J = 7.8 Hz), 5.27-5.11 (1H, br m), 4.27-4.24 (2H, m), 3.59 (2H, s), 3.27 (3H, s), 2.83-2.74 (2H, m), 2.62-2.57 (1H, m), 2.32-2.30 (1H, m), 2.22-2.06 (1H, m), 1.91-1.83 (1H, m), 1.38 (3H, d, J = 6.8 Hz), 1.29 (6H, dd, J = 6.3, 3.4 Hz). | | |
| 272 | 1H-NMR (DMSO-D6) δ: 10.11 (1H, s), 9.25 (1H, s), 8.28-8.26 (2H, m), 7.79 (1H, d, J = 9.8 Hz), 6.89 (1H, s), 6.46 (1H, d, J = 6.8 Hz), 4.41 (2H, d, J = 5.9 Hz), 4.30-4.22 (2H, m), 3.70 (2H, s), 3.26 (3H, s), 2.97 (2H, d, J = 10.7 Hz), 2.84 (1H, q, J = 6.5 Hz), 2.67 (2H, d, J = 11.7 Hz), 2.25 (1H, d, J = 7.8 Hz), 1.38 (3H, d, J = 5.9 Hz), 1.29 (6H, dd, J = 6.3, 3.4 Hz). | | |

**[Table 5-45]**

| Compound No. | NMR | LC/MS (M+H) ⁺ | Exact Mass |
|---|---|---|---|
| 273 | 1H-NMR (DMSO-D6) δ: 10.11 (1H, s), 9.25 (1H, s), 8.27 (1H, d, J = 8.8 Hz), 8.22 (1H, s), 7.78 (1H, d, J = 8.8 Hz), 6.89 (1H, s), 6.46 (1H, d, J = 7.8 Hz), 4.30-4.22 (2H, m), 3.68 (2H, t, J = 5.9 Hz), 3.62-3.60 (4H, br m), 3.27 (3H, s), 2.64-2.62 (4H, m), 1.83-1.79 (2H, m), 1.38 (3H, d, J = 5.9 Hz), 1.30 (6H, dd, J = 5.9, 3.9 Hz) . | | |
| 274 | 1H-NMR (DMSO-D6) δ: 10.12 (1H, s), 9.25 (1H, s), 8.27 (1H, d, J = 8.8 Hz), 8.17 (1H, d, J = 14.6 Hz), 7.74 (1H, dd, J = 8.8, 2.0 Hz), 6.89 (1H, s), 6.46 (1H, d, J = 7.8 Hz), 4.30-4.22 (2H, m), 3.44 (3H, s), 3.27 (3H, s), 3.17-3.16 (1H, m), 2.66-2.64 (2H, br m), 2.11-2.09 (2H, br m), 1.82-1.80 (2H, br m), 1.43-1.41 (2H, br m), 1.38 (3H, d, J = 5.9 Hz), 1.30 (6H, dd, J = 5.9, 3.9 Hz). | | |
| 276 | 1H-NMR (DMSO-D6) δ: 10.12 (1H, s), 9.25 (1H, s), 8.27 (1H, d, J = 8.8 Hz), 8.19 (1H, d, J = 2.0 Hz), 8.15 (1H, s), 7.74 (1H, dd, J = 8.8, 2.0 Hz), 6.89 (1H, s), 6.46 (1H, d, J = 7.8 Hz), 4.50 (2H, dt, J = 47.8, 4.9 Hz), 4.30-4.22 (2H, m), 3.44 (2H, s), 3.27 (3H, s), 2.66-2.60 (1H, m), 2.56-2.53 (1H, m), 2.41-2.38 (8H, br m), 1.38 (3H, d, J = 6.8 Hz), 1.30 (6H, dd, J = 5.9, 3.9 Hz). | | |
| 277 | 1H-NMR (DMSO-D6) δ: 9.51 (1H, s), 8.19 (1H, s), 7.78 (1H, s), 7.30 (1H, dd, J = 8.8, 2.0 Hz), 7.02 (1H, s), 6.85 (1H, d, J = 7.8 Hz), 6.36 (1H, d, J = 7.8 Hz), 5.79 (1H, s), 4.36-4.26 (2H, m), 3.72 (2H, s), 3.49 (2H, s), 3.30 (3H, s), 2.75-2.74 (2H, m), 2.60 (3H, s), 1.38 (3H, d, J = 5.9 Hz), 1.29 (6H, t, J = 5.9 Hz). | | |

**[Table 5-46]**

| Compound No. | NMR | LC/MS (M+H) ⁺ | Exact Mass |
|---|---|---|---|
| 278 | 1H-NMR (DMSO-D6) δ: 10.11 (1H, s), 9.25 (1H, s), 8.27 (1H, d, J = 7.8 Hz), 8.18 (1H, d, J = 2.0 Hz), 7.74 (1H, dd, J = 8.3, 2.4 Hz), 7.68 (1H, d, J = 4.9 Hz), 6.89 (1H, s), 6.46 (1H, d, J = 7.8 Hz), 4.30-4.22 (2H, m), 3.42 (2H, s), 3.27 (3H, s), 2.82 (2H, d, J = 10.7 Hz), 2.53 (3H, d, J = 4.9 Hz), 2.07-2.01 (1H, m), 1.93-1.87 (2H, m), 1.63-1.50 (4H, m), 1.38 (3H, d, J = 5.9 Hz), 1.30 (6H, dd, J = 6.8, 3.9 Hz). | | |
| 279 | 1H-NMR (DMSO-D6) δ: 10.06 (1H, s), 9.24 (1H, s), 8.25 (1H, d, J = 8.8 Hz), 8.20 (1H, s), 7.74 (1H, dd, J = 8.8, 2.0 Hz), 6.89 (1H, s), 6.47 (1H, d, J = 7.8 Hz), 4.30-4.22 (2H, m), 3.51-3.45 (4H, br m), 3.27 (5H, s), 2.31 (2H, t, J = 4.4 Hz), 1.38 (3H, d, J = 6.8 Hz), 1.30 (6H, dd, J = 6.8, 3.9 Hz), 1.07 (6H, s). | | |
| 280 | 1H-NMR (DMSO-D6) δ: 10.07 (1H, s), 9.24 (1H, s), 8.26-8.25 (2H, m), 7.78 (1H, d, J = 9.8 Hz), 6.89 (1H, s), 6.46 (1H, d, J = 7.8 Hz), 4.30-4.22 (2H, m), 3.89 (1H, d, J = 13.7 Hz), 3.57 (2H, dd, J = 10.7, 2.0 Hz), 3.30-3.24 (6H, m), 2.73-2.69 (2H, m), 1.38 (3H, d, J = 5.9 Hz), 1.30 (6H, dd, J = 6.3, 3.4 Hz), 0.96 (6H, d, J = 6.8 Hz). | | |
| 281 | 1H-NMR (DMSO-D6) δ: 10.12 (1H, s), 9.25 (1H, s), 8.27 (1H, d, J = 7.8 Hz), 8.19 (1H, d, J = 2.0 Hz), 8.15 (1H, s), 7.74 (1H, dd, J = 8.3, 2.4 Hz), 6.89 (1H, s), 6.46 (1H, d, J = 7.8 Hz), 6.11 (1H, tt, J = 55.6, 4.1 Hz), 4.30-4.22 (2H, m), 3.44 (2H, s), 3.27 (3H, s), 2.69 (2H, td, J = 15.9, 4.2 Hz), 2.54-2.52 (2H, br m), 2.40-2.37 (4H, br m), 1.38 (3H, d, J = 5.9 Hz), 1.30 (6H, dd, J = 5.9, 3.9 Hz). | | |

**[Table 5-47]**

| Compound No. | NMR | LC/MS (M+H) ⁺ | Exact Mass |
|---|---|---|---|
| 282 | 1H-NMR (DMSO-d6) δ: 9.93 (1H, s), 9.22 (1H, s), 8.17 (1H, d, J = 12.4 Hz), 8.06 (1H, d, J = 4.0 Hz), 7.51 (1H, dd, J = 12.0, 4.0 Hz), 6.88 (1H, s), 6.39 (1H, d, J = 10.4 Hz), 4.24-4.29 (2H, m), 4.16 (2H, t, J = 15.2 Hz), 3.57-3.60 (4H, m), 3.21(6H, m), 2.69-2.73 (2H, m), 1.38 (3H, d, J = 8.4 Hz), 1.29-1.32 (6H, m). | | |
| 284 | 1H-NMR (DMSO-D6) δ: 10.18 (1H, s), 9.20 (1H, s), 8.28 (1H, d, J = 9.8 Hz), 7.31 (1H, d, J = 10.2 Hz), 6.96 (1H, s), 6.89 (1H, t, J = 5.9 Hz), 5.17 (1H, d, J = 2.9 Hz), 4.62-4.56 (1H, m), 3.81-3.75 (1H, m), 3.72-3.46 (5H, m), 3.45-3.40 (4H, m), 2.85-2.79 (4H, m), 2.69-2.62 (1H, m), 2.00-1.91 (1H, m), 1.71-1.62 (1H, m), 1.37 (3H, d, J = 6.3 Hz). | | |
| 285 | 1H-NMR (DMSO-D6) δ: 10.26 (1H, s), 9.21 (1H, s), 8.20 (1H, d, J = 9.8 Hz), 7.38 (1H, d, J = 10.2 Hz), 6.89 (1H, s), 6.48 (1H, d, J = 7.3 Hz), 4.26-4.11 (2H, m), 3.92-3.86 (2H, m), 3.51-3.42 (6H, m), 3.26 (3H, s), 2.86-2.82 (4H, m), 2.03-1.96 (2H, m), 1.68-1.55 (2H, m), 1.36 (3H, d, J = 6.3 Hz). | | |
| 286 | 1H-NMR (DMSO-D6) δ: 10.20 (1H, s), 9.20 (1H, s), 8.17 (1H, d, J = 13.2 Hz), 7.38 (1H, d, J = 13.2 Hz), 6.87 (1H, s), 6.36 (1H, d, J = 10.0 Hz), 4.87 (1H, d, J = 6.0 Hz), 4.21-4.28 (2H, m), 4.11-4.17 (1H, m), 3.92-3.96 (1H, m), 3.92-3.96 (1H, m), 3.56-3.61 (1H, m), 3.00 (3H, s), 2.98-3.03 (1H, m), 2.79-2.86 (1H, m), 1.91-1.94 (1H, m), 1.75-1.80 (1H, m), 1.40-1.51 (2H, m), 1.38 (3H, d, J = 10.0 Hz), 1.23-1.29 (6H, m). | 439.4 | 438.25 |

**[Table 5-48]**

| Compound No. | NMR | LC/MS (M+H) ⁺ | Exact Mass |
|---|---|---|---|
| 287 | 1H-NMR (DMSO-D6) δ: 9.95 (1H, s), 9.22 (1H, s), 8.16 (1H, d, J = 8.8 Hz), 8.06 (1H, d, J = 2.8 Hz), 7.51 (1H, dd, J = 9.2, 3.2 Hz), 6.88 (1H, s), 6.39 (1H, d, J = 7.6 Hz), 5.31-5.34 (1H, m), 4.24-4.50 (2H, m), 4.15 (2H, br s), 2.83 (2H, br s), 1.96-2.03 (2H, m), 1.71 (4H, br s), 1.39 (3H, d, J = 6.4 Hz), 1.27-1.33 (6H, m). | 452.2 | 451.27 |
| 288 | 1H-NMR (DMSO-D6) δ: 9.94 (1H, s), 9.22 (1H, s), 8.17 (1H, d, J = 8.8 Hz), 8.07 (1H, d, J = 2.8 Hz), 7.51 (1H, dd, J = 9.2, 3.2 Hz), 6.89 (1H, s), 6.39 (1H, d, J = 7.6 Hz), 4.22-4.30 (2H, m), 4.08-4.11 (2H, m), 3.28-3.30 (3H, m), 2.84-2.86 (2H, m), 2.36 (3H, s), 1.39 (3H, d, J = 6.4 Hz), 1.27-1.33 (6H, m). | 412.1 | 411.24 |
| 289 | 1H-NMR (DMSO-D6) δ: 9.93 (1H, s), 9.22 (1H, s), 8.15 (1H, d, J = 9.2 Hz), 8.05 (1H, d, J = 3.2 Hz), 7.52 (1H, dd, J = 9.2, 2.8 Hz), 6.88 (1H, s), 6.39 (1H, d, J = 7.6 Hz), 4.40-4.48 (1H, m), 4.24-4.27 (2H, m), 3.28 (3H, s), 3.0 (2H, br s), 2.52-2.67 (2H, m), 1.93-1.95 (2H, m), 1.48-1.50 (2H, m), 1.39 (3H, d, J = 6.4 Hz), 1.30 (6H, dd, J = 6.4, 3.6 Hz). | 438.2 | 437.25 |
| 290 | 1H-NMR (DMSO-D6) δ: 9.94 (1H, s), 9.22 (1H, s), 8.16 (1H, d, J = 8.8 Hz), 8.03 (1H, d, J = 3.2 Hz), 7.48 (1H, dd, J = 8.8 2.8 Hz), 6.89 (1H, s), 6.41 (1H, d, J = 7.2 Hz), 4.93-4.94 (1H, m), 4.25-4.29 (2H, m), 3.28 (3H, s), 3.11-3.12 (1H, m), 2.92-2.98 (2H, m), 2.85-2.86 (1H, m), 2.02-2.07 (1H, m), 1.83-1.85 (1H, m), 1.39 (3H, d, J = 6.4 Hz), 1.30 (6H, dd, J = 6.4, 3.6 Hz). | 424.2 | 423.24 |

**[Table 5-49]**

| Compound No. | NMR | LC/MS (M+H) ⁺ | Exact Mass |
|---|---|---|---|
| 291 | 1H-NMR (CDC13) δ: 9.05 (1H, s), 8.34-8.28 (3H, m), 7.74 (1H, dd, J = 8.8, 2.4 Hz), 6.88 (1H, s), 6.24 (1H, d, J = 7.8 Hz), 4.42-4.30 (2H, m), 4.09-4.03 (2H, m), 3.70-3.61 (2H, m), 3.50 (2H, s), 3.41 (3H, s), 3.00-2.90 (4H, m), 2.49 (4H, br s), 2.22-2.15 (2H, m), 1.73-1.61 (2H, m), 1.50 (3H, d, J = 6.3 Hz). | | |
| 292 | 1H-NMR (DMSO-D6) δ: 10.27 (1H, s), 9.24 (1H, s), 8.28 (1H, s), 8.23 (1H, d, J = 8.8 Hz), 8.12 (1H, s), 7.77 (1H, d, J = 7.8 Hz), 7.00 (1H, s), 6.70 (1H, s), 5.27-5.20 (2H, m), 4.63-4.61 (1H, m), 3.73 (1H, br s), 3.56 (2H, br s), 3.38 (2H, s), 2.98-2.88 (2H, br m), 2.19-2.13 (1H, m), 1.95-1.89 (1H, m), 1.50 (6H, s), 1.39 (3H, d, J = 5.9 Hz). | | |
| 293 | 1H-NMR (DMSO-D6) δ: 10.21 (1H, s), 9.23 (1H, s), 8.24-8.21 (2H, m), 8.14 (1H, s), 7.75 (1H, dd, J = 8.8, 2.0 Hz), 6.99 (1H, s), 6.71 (1H, s), 5.23 (1H, br s), 4.62 (1H, q, J = 6.5 Hz), 3.68 (2H, t, J = 5.9 Hz), 3.60-3.56 (5H, m), 3.38 (3H, s), 3.15 (1H, s), 2.64-2.62 (4H, m), 1.83-1.77 (2H, m), 1.50 (6H, s), 1.39 (3H, d, J = 6.8 Hz). | | |
| 294 | 1H-NMR (DMSO-D6) δ: 10.21 (1H, s), 9.23 (1H, s), 8.21-8.19 (2H, m), 8.14 (2H, s), 7.72 (1H, d, J = 10.7 Hz), 6.99 (1H, s), 6.71 (1H, s), 5.23 (1H, br s), 4.62 (1H, q, J = 6.2 Hz), 3.51 (3H, d, J = 33.2 Hz), 3.38 (3H, s), 3.18 (5H, d, J = 18.5 Hz), 2.66 (2H, br s), 2.12 (2H, t, J = 9.3 Hz), 1.51-1.49 (6H, m), 1.50 (6H, s), 1.39 (3H, d, J = 6.8 Hz). | | |

**[Table 5-50]**

| Compound No. | NMR | LC/MS (M+H) ⁺ | Exact Mass |
|---|---|---|---|
| 295 | 1H-NMR (DMSO-D6) δ: 10.26 (1H, s), 9.24 (1H, s), 8.30 (1H, d, J = 2.0 Hz), 8.24 (1H, d, J = 8.8 Hz), 7.79-7.78 (1H, m), 7.01 (2H, d, J = 14.6 Hz), 6.82 (1H, s), 6.70 (1H, s), 5.23 (1H, br s), 4.62 (1H, s), 3.94 (2H, t, J = 5.4 Hz), 3.70 (2H, s), 3.57 (3H, d, J = 10.7 Hz), 2.88-2.80 (2H, m), 1.49 (6H, d, J = 7.8 Hz), 1.39 (3H, d, J = 6.8 Hz). | | |
| 296 | 1H-NMR (DMSO-D6) δ: 9.50 (1H, s), 7.80 (1H, s), 7.31 (1H, dd, J = 7.8, 2.0 Hz), 7.15 (1H, s), 6.85 (1H, s), 6.36 (1H, d, J = 7.8 Hz), 5.79 (2H, s), 5.39 (1H, s), 4.66 (1H, d, J = 5.9 Hz), 4.11 (1H, s), 3.72 (2H, s), 3.60-3.46 (4H, m), 3.38 (2H, s), 3.15 (4H, s), 2.73 (2H, d, J = 4.9 Hz), 2.62 (3H, s), 2.49 (7H, s), 1.76-1.73 (1H, m), 1.51 (6H, s), 1.41 (3H, d, J = 5.9 Hz). | | |
| 297 | 1H-NMR (DMSO-D6) δ: 10.25 (1H, s), 9.24 (1H, s), 8.23-8.21 (2H, m), 8.13 (1H, s), 7.75-7.70 (2H, m), 7.00 (1H, s), 6.71 (1H, s), 5.23 (1H, s), 4.63-4.61 (1H, m), 3.57 (4H, d, J = 9.8 Hz), 3.38 (2H, s), 3.15 (1H, s), 2.96-2.91 (2H, m), 2.53 (3H, d, J = 3.9 Hz), 2.08-2.05 (3H, m), 1.65-1.56 (4H, m), 1.50 (6H, s), 1.39 (3H, d, J = 6.8 Hz). | | |
| 298 | 1H-NMR (DMSO-D6) δ: 10.18 (1H, s), 9.22 (1H, s), 8.21-8.19 (2H, m), 8.13 (1H, s), 7.74 (1H, dd, J = 8.3, 2.4 Hz), 6.99 (1H, s), 6.73 (1H, s), 5.23 (1H, br s), 4.62-4.60 (1H, m), 3.54 (1H, s), 3.48 (2H, t, J = 7.3 Hz), 3.40 (2H, s), 3.26 (1H, s), 2.32 (2H, t, J = 4.4 Hz), 1.50 (6H, s), 1.39 (3H, d, J = 5.9 Hz), 1.06 (6H, s). | | |

**[Table 5-51]**

| Compound No. | NMR | LC/MS (M+H) ⁺ | Exact Mass |
|---|---|---|---|
| 299 | 1H-NMR (DMSO-D6) δ: 10.22 (1H, s), 9.23 (1H, s), 8.21-8.20 (2H, m), 8.13 (1H, s), 7.72-7.71 (1H, m), 6.99 (1H, s), 6.71 (1H, s), 6.11 (1H, tt, J = 55.6, 4.1 Hz), 5.23 (1H, s), 4.63-4.61 (1H, m), 3.56 (1H, s), 3.47 (2H, s), 3.38 (3H, s), 3.15 (1H, s), 2.69 (2H, td, J = 15.6, 3.9 Hz), 2.53-2.51 (2H, m), 2.40 (4H, s), 1.50 (6H, s), 1.39 (3H, d, J = 6.8 Hz). | | |
| 300 | 1H-NMR (CDC13) δ: 9.05 (1H, s), 8.38 (1H, d, J = 8.8 Hz), 8.29-8.24 (2H, m), 7.74 (1H, dd, J = 8.5, 2.2 Hz), 6.88 (1H, s), 6.53 (1H, d, J = 7.8 Hz), 4.40-4.29 (2H, m), 4.06 (1H, dd, J = 11.0, 2.7 Hz), 3.81-3.70 (2H, m), 3.58 (1H, dd, J = 11.2, 5.9 Hz), 3.49 (2H, s), 3.41 (3H, s), 2.95-2.87 (4H, m), 2.49-2.42 (4H, br m), 2.10-2.02 (1H, m), 1.96-1.67 (3H, m), 1.49 (3H, d, J = 6.3 Hz). | | |
| 301 | 1H-NMR (CDC13) δ: 9.05 (1H, s), 8.38 (1H, d, J = 8.8 Hz), 8.31 (1H, br s), 8.27 (1H, d, J = 2.0 Hz), 7.74 (1H, dd, J = 8.5, 2.2 Hz), 6.88 (1H, s), 6.55 (1H, d, J = 8.3 Hz), 4.40-4.30 (2H, m), 4.06 (1H, dd, J = 10.7, 2.9 Hz), 3.81-3.72 (2H, m), 3.60 (1H, dd, J = 11.0, 6.1 Hz), 3.49 (2H, s), 3.41 (3H, s), 2.95-2.88 (4H, m), 2.53-2.37 (4H, m), 2.09-2.03 (1H, m), 1.95-1.82 (2H, m), 1.76-1.68 (1H, m), 1.49 (3H, d, J = 6.3 Hz). | | |
| 302 | 1H-NMR (CDC13) δ: 9.07 (1H, s), 8.34-8.26 (3H, m), 7.75 (1H, dd, J = 8.5, 2.2 Hz), 6.91 (1H, s), 6.39 (1H, d, J = 6.8 Hz), 4.86-4.80 (1H, m), 4.34 (1H, q, J = 6.5 Hz), 4.14-4.05 (2H, m), 3.97-3.90 (1H, m), 3.86-3.81 (1H, m), 3.49 (2H, s), 3.41 (3H, s), 2.94-2.89 (4H, m), 2.50-2.40 (5H, m), 2.06-1.76 (2H, m), 1.50 (3H, d, J = 6.3 Hz). | | |

**[Table 5-52]**

| Compound No. | NMR | LC/MS (M+H)⁺ | Exact Mass |
|---|---|---|---|
| 303 | 1H-NMR (CDCl3) δ: 9.07 (1H, s), 8.36 (1H, br s), 8.33 (1H, d, J = 8.3 Hz), 8.28 (1H, d, J = 2.0 Hz), 7.75 (1H, dd, J = 8.5, 2.2 Hz), 6.90 (1H, s), 6.39 (1H, d, J = 6.3 Hz), 4.87-4.81 (1H, m), 4.34 (1H, q, J = 6.3 Hz), 4.15-4.04 (2H, m), 3.97-3.90 (1H, m), 3.85 (1H, dd, J = 9.3, 3.9 Hz), 3.49 (2H, s), 3.41 (3H, s), 2.95-2.90 (4H, m), 2.50-2.39 (5H, m), 2.05-1.86 (2H, m), 1.51 (3H, d, J = 6.3 Hz). | | |
| 304 | 1H-NMR (CDCl3) δ: 9.02 (1H, s), 8.42 (1H, d, J = 9.8 Hz), 8.22 (1H, br s), 7.07 (1H, d, J = 9.8 Hz), 6.86 (1H, s), 6.49 (1H, d, J = 7.8 Hz), 4.38-4.28 (2H, m), 4.03 (1H, dd, J = 11.2, 2.9 Hz), 3.81-3.69 (2H, m), 3.61-3.55 (5H, m), 3.40 (3H, s), 3.08-3.02 (4H, m), 2.09-2.01 (1H, m), 1.91-1.70 (3H, m), 1.48 (3H, d, J = 6.3 Hz). | | |
| 305 | 1H-NMR (CDCl3) δ: 9.02 (1H, s), 8.42 (1H, d, J = 9.8 Hz), 8.24 (1H, br s), 7.07 (1H, d, J = 9.8 Hz), 6.87 (1H, s), 6.51 (1H, d, J = 8.3 Hz), 4.37-4.29 (2H, m), 4.04 (1H, dd, J = 10.7, 2.9 Hz), 3.79-3.71 (2H, m), 3.63-3.55 (5H, m), 3.40 (3H, s), 3.08-3.02 (4H, m), 2.08-1.99 (1H, m), 1.91-1.67 (3H, m), 1.48 (3H, d, J = 6.3 Hz). | | |
| 306 | 1H-NMR (CDCl3) δ: 9.04 (1H, s), 8.36 (1H, d, J = 9.8 Hz), 8.27 (1H, br s), 7.07 (1H, d, J = 9.8 Hz), 6.90 (1H, s), 6.35 (1H, d, J = 6.8 Hz), 4.86-4.79 (1H, m), 4.33 (1H, q, J = 6.3 Hz), 4.12-4.03 (2H, m), 3.96-3.89 (1H, m), 3.83 (1H, dd, J = 9.3, 3.4 Hz), 3.62-3.56 (4H, m), 3.41 (3H, s), 3.08-3.02 (4H, m), 2.48-2.38 (1H, m), 2.04-1.96 (1H, m), 1.49 (3H, d, J = 6.3 Hz). | | |

**[Table 5-53]**

| Compound No. | NMR | LC/MS (M+H)⁺ | Exact Mass |
|---|---|---|---|
| 307 | 1H-NMR (CDCl3) δ: 9.03 (1H, s), 8.36 (1H, d, J = 9.8 Hz), 8.26 (1H, br s), 7.07 (1H, d, J = 9.8 Hz), 6.89 (1H, s), 6.35 (1H, d, J = 6.8 Hz), 4.87-4.80 (1H, m), 4.33 (1H, q, J = 6.5 Hz), 4.13-4.02 (2H, m), 3.95-3.89 (1H, m), 3.84 (1H, dd, J = 9.3, 3.4 Hz), 3.62-3.58 (4H, m), 3.40 (3H, s), 3.08-3.04 (4H, m), 2.47-2.37 (1H, m), 2.04-1.95 (1H, m), 1.50 (3H, d, J = 6.3 Hz). | | |
| 308 | 1H-NMR (DMSO-D6) δ: 10.08 (1.0H, br s), 9.40-9.08 (2.0H, m), 8.94-8.86 (1.0H, br m), 7.46-7.37 (1.0H, m), 6.83-6.78 (1.0H, m), 5.17-5.09 (1.0H, m), 4.58-4.54 (1.0H, br m), 4.20-4.09 (1.0H, br m), 3.44-3.37 (5.8H, m), 2.85-2.79 (2.2H, m), 2.36-2.20 (2.4H, m), 1.71-1.33 (9.6H, m). | | |
| 309 | 1H-NMR (DMSO-D6) δ: 10.10 (1H, br s), 9.33-8.93 (3H, m), 7.40-7.34 (1H, m), 6.84-6.78 (1H, m), 5.16-5.06 (1H, m), 4.59-4.54 (1H, m), 4.29-4.19 (1H, m), 3.41-3.35 (5H, m), 2.86-2.78 (3H, m), 2.39-2.27 (1H, m), 2.14-1.98 (2H, m), 1.82-1.33 (7H, m). | | |
| 310 | 1H-NMR (DMSO-D6) δ: 10.21 (1H, s), 9.20 (1H, s), 8.16 (1H, d, J = 9.8 Hz), 7.36 (1H, d, J = 9.8 Hz), 6.88 (1H, s), 6.35 (1H, d, J = 7.8 Hz), 4.34 (1H, q, J = 6.3 Hz), 4.29-4.16 (1H, m), 3.46 (2H, t, J = 7.1 Hz), 3.43 (4H, t, J = 4.9 Hz), 2.81 (4H, t, J = 4.9 Hz), 1.37 (3H, d, J = 6.3 Hz), 1.27 (6H, dd, J = 6.3, 3.9 Hz), 1.15 (3H, t, J = 7.1 Hz). | 438.30 | 437.27 |

**[Table 5-54]**

| Compound No. | NMR | LC/MS (M+H)⁺ | Exact Mass |
|---|---|---|---|
| 311 | 1H-NMR (DMSO-D6) δ: 10.62 (1H, brs), 9.27 (1H, s), 8.44 (1H, d, J = 9.3 Hz), 7.60 (1H, d, J = 9.3 Hz), 6.90 (1H, s), 6.48 (1H, d, J = 7.3 Hz), 4.32-4.20 (2H, m), 3.27 (3H, s), 3.04 (2H, dt, J = 12.4, 3.0 Hz), 2.92 (1H, tt, J = 12.0, 3.7 Hz), 2.61 (2H, td, J = 12.2, 2.4 Hz), 1.79 (2H, dt, J = 14.0, 1.6 Hz), 1.64 (2H, ddd, J = 24.4, 12.2, 3.9 Hz), 1.38 (3H, d, J = 6.8 Hz), 1.29 (6H, dd, J = 6.6, 3.7 Hz). | 423.30 | 422.25 |
| 312 | 1H-NMR (CDCl3) δ: 9.06 (1H, s), 8.46 (1H, br s), 8.31 (1H, d, J = 8.8 Hz), 8.29 (1H, d, J = 2.0 Hz), 7.74 (1H, dd, J = 8.8, 2.0 Hz), 6.74 (1H, s), 6.55 (1H, t, J = 6.1 Hz), 4.82 (1H, q, J = 6.3 Hz), 4.07-3.98 (2H, m), 3.59 (2H, t, J = 6.6 Hz), 3.50 (2H, s), 3.47-3.38 (2H, m), 2.93-2.87 (4H, m), 2.45 (4H, br s), 2.06-1.96 (1H, m), 1.78-1.73 (2H, m), 1.56-1.44 (5H, m). | | |
| 313 | 1H-NMR (CDCl3) δ: 9.00 (1H, s), 8.34 (1H, d, J = 9.8 Hz), 8.14 (1H, br s), 7.07 (1H, d, J = 9.8 Hz), 6.72 (1H, s), 6.50 (1H, t, J = 8.0 Hz), 4.80 (1H, q, J = 6.3 Hz), 4.03-3.37 (2H, m), 3.60-3.53 (6H, m), 3.45-3.38 (2H, m), 3.07-3.03 (4H, m), 2.04-1.96 (1H, m), 1.76-1.71 (2H, m), 1.57-1.45 (5H, m). | | |
| 314 | 1H-NMR (CDCl3) δ: 9.06 (1H, s), 8.27 (1H, d, J = 2.4 Hz), 8.24 (1H, d, J = 8.8 Hz), 8.12 (1H, br s), 7.78 (1H, dd, J = 8.8, 2.4 Hz), 6.85 (1H, s), 6.37 (1H, d, J = 7.8 Hz), 4.84 (1H, q, J = 6.3 Hz), 4.46-4.40 (1H, m), 3.51 (2H, s), 3.23-3.21 (4H, m), 2.92-2.91 (4H, m), 2.61-2.54 (2H, m), 2.46-2.33 (6H, m), 1.54 (3H, d, J = 6.3 Hz). | | |

**[Table 5-55]**

| Compound No. | NMR | LC/MS (M+H)⁺ | Exact Mass |
|---|---|---|---|
| 315 | 1H-NMR (DMSO-D6) δ: 10.47 (1H, s), 9.18 (1H, s), 8.83 (1H, d, J = 9.8 Hz), 8.41 (1H, br s), 7.37 (1H, d, J = 9.8 Hz), 6.90 (1H, s), 5.14 (1H, br s), 4.64-4.56 (1H, m), 3.76-3.01 (8H, m), 1.67 (6H, s), 1.39 (3H, d, J = 6.3 Hz). | | |
| 316 | 1H-NMR (DMSO-D6) δ: 10.16 (1H, s), 9.10 (1H, s), 8.80 (1H, br s), 8.77 (1H, d, J = 9.8 Hz), 7.29 (1H, d, J = 9.8 Hz), 6.80 (1H, s), 5.12 (1H, d, J = 4.4 Hz), 4.59-4.53 (1H, m), 3.44-3.38 (4H, m), 2.85-2.80 (4H, m), 2.32-2.04 (4H, m), 1.88-1.74 (4H, m), 1.36 (3H, d, J = 6.8 Hz). | | |
| 317 | 1H-NMR (DMSO-D6) δ: 10.60 (1H, brs), 9.28 (1H, s), 8.48 (1H, d, J = 9.3 Hz), 7.61 (1H, d, J = 9.3 Hz), 7.02 (1H, s), 6.55 (1H, d, J = 7.3 Hz), 5.21 (1H, d, J = 4.4 Hz), 4.65-4.55 (1H, m), 4.24-4.12 (1H, m), 3.92-3.89 (2H, m), 3.48 (2H, dq, J = 16.6, 4.2 Hz), 3.04 (2H, dd, J = 10.0, 2.2 Hz), 2.92 (1H, tt, J = 12.0, 3.5 Hz), 2.61 (2H, td, J = 12.1, 2.1 Hz), 2.00 (2H, t, J = 13.7 Hz), 1.79 (2H, d, J = 12.2 Hz), 1.72-1.59 (4H, m), 1.38 (3H, d, J = 6.3 Hz). | 451.30 | 450.25 |
| 318 | 1H-NMR (DMSO-D6) δ: 10.58 (1H, brs), 9.26 (1H, s), 8.44 (1H, d, J = 9.3 Hz), 7.60 (1H, d, J = 9.3 Hz), 7.00 (1H, d, J = 1.0 Hz), 6.43 (1H, d, J = 7.8 Hz), 5.20 (1H, d, J = 4.4 Hz), 4.67-4.57 (1H, m), 4.32-4.20 (1H,m), 3.06-3.03 (2H, m), 2.92 (1H, tt, J = 12.0, 3.6 Hz), 2.60 (2H, td, J = 12.0, 2.3 Hz), 1.79 (2H, dd, J = 12.7, 2.4 Hz), 1.64 (2H, ddd, J = 24.4, 12.2, 3.9 Hz), 1.38 (3H, d, J = 6.3 Hz), 1.29 (6H, dd, J = 6.3, 2.0 Hz). | 409.30 | 408.24 |

**[Table 5-56]**

| Compound No. | NMR | LC/MS (M+H)⁺ | Exact Mass |
|---|---|---|---|
| 319 | 1H-NMR (DMSO-D6) δ: 10.21 (1H, s), 9.26 (1H, s), 8.36-8.28 (2H, m), 8.15 (1H, d, J = 2.9 Hz), 7.82 (1H, dd, J = 9.0, 2.7 Hz), 6.99 (1H, s), 6.42 (1H, d, J = 7.3 Hz), 5.19 (1H, d, J = 4.4 Hz), 4.64-4.59 (1H, m), 4.32-4.13 (3H, m), 3.85-3.70 (4H, m), 1.39 (3H, d, J = 6.3 Hz), 1.29 (6H, dd, J = 6.3, 2.0 Hz). | 451.30 | 450.21 |
| 320 | 1H-NMR (CDCl3) δ: 9.06 (1H, s), 8.39 (1H, br s), 8.36 (1H, d, J = 8.8 Hz), 8.28 (1H, d, J = 2.0 Hz), 7.76 (1H, dd, J = 8.8, 2.0 Hz), 6.88 (1H, s), 6.51 (1H, t, J = 5.4 Hz), 4.34 (1H, q, J = 5.9 Hz), 4.01-3.99 (1H, m), 3.93-3.91 (1H, m), 3.84-3.75 (2H, m), 3.71-3.67 (2H, m), 3.50 (2H, s), 3.41 (3H, s), 2.93-2.89 (4H, br m), 2.79-2.72 (1H, m), 2.46 (4H, br s), 2.19-2.11 (1H, m), 1.86-1.81 (2H, m), 1.50 (3H, d, J = 5.9 Hz). | | |
| 321 | 1H-NMR (CDCl3) δ: 9.07 (1H, s), 8.55 (1H, br s), 8.43 (1H, d, J = 9.8 Hz), 7.10 (1H, d, J = 9.8 Hz), 6.88 (1H, s), 6.48 (1H, t, J = 5.4 Hz), 4.33 (1H, q, J = 6.3 Hz), 4.00-3.98 (1H, m), 3.90-3.88 (1H, m), 3.82-3.77 (2H, m), 3.68-3.66 (2H, m), 3.60-3.58 (4H, m), 3.40 (3H, s), 3.06-3.05 (4H, m), 2.77-2.74 (1H, m), 2.16-2.13 (1H, m), 1.82-1.78 (2H, m), 1.50 (3H, d, J = 6.3 Hz). | | |
| 322 | | 500.3 | 499.21 |
| 323 | 1H-NMR (CDCl3) δ: 9.04 (1H, s), 8.35-8.28 (3H, m), 7.77-7.76 (1H, m), 6.74 (1H, s), 6.65-6.62 (1H, br m), 4.82 (1H, q, J = 5.9 Hz), 4.04-4.00 (1H, m), 3.91-3.88 (1H, m), 3.83-3.79 (2H, m), 3.70-3.68 (2H, m), 3.50-3.49 (2H, m), 2.92-2.91 (4H, m), 2.76 (1H, br s), 2.46 (4H, br s), 2.18-2.15 (1H, m), 1.82-1.79 (2H, m), 1.54 (3H, d, J = 5.9 Hz). | | |

**[Table 5-57]**

| Compound No. | NMR | LC/MS (M+H)⁺ | Exact Mass |
|---|---|---|---|
| 324 | 1H-NMR (DMSO-D6) δ: 10.10-10.02 (0.2H, m), 9.91-9.80 (0.8H, m), 9.49-9.38 (0.8H, m), 9.23-9.12 (1.0H, m), 8.96-8.88 (0.8H, m), 8.51-8.41 (0.2H, m), 8.19-8.10 (1.0H, m), 7.90-7.70 (1.0H, m), 7.33-7.23 (0.2H, m), 6.98-6.82 (1.0H, m), 5.20-5.10 (1.0H, m), 4.64-4.53 (1.0H, m), 4.34-4.21 (1.0H, m), 2.69-1.98 (10.0H, m), 1.87-1.34 (8.0H, m). | | |
| 325 | 1H-NMR (DMSO-D6) δ: 9.94 (1H, br s), 9.13 (1H, s), 8.91 (1H, s), 8.81 (1H, d, J = 8.3 Hz), 8.16 (1H, s), 7.75 (1H, d, J = 8.3 Hz), 6.82 (1H, s), 5.10 (1H, br s), 4.60-4.55 (1H, m), 3.41 (2H, s), 2.72-2.66 (4H, br m), 2.33-2.03 (8H, m), 1.89-1.79 (4H, br m), 1.37 (3H, d, J = 6.3 Hz). | | |
| 326 | 1H-NMR (CDCl3) δ: 9.42 (1H, s), 8.60 (1H, br s), 8.31 (1H, d, J = 2.0 Hz), 8.29 (1H, d, J = 8.3 Hz), 7.77 (1H, dd, J = 8.5, 2.2 Hz), 6.42 (1H, d, J = 7.8 Hz), 5.18 (1H, q, J = 6.3 Hz), 4.34-4.27 (1H, m), 4.10-4.01 (2H, m), 3.69-3.59 (2H, m), 3.51 (2H, s), 2.95-2.86 (4H, m), 2.49-2.42 (4H, br m), 2.22-2.11 (2H, m), 1.78-1.64 (2H, m), 1.47 (3H, d, J = 6.3 Hz). | | |
| 327 | 1H-NMR (DMSO-D6) δ: 10.19 (1H, br s), 9.16 (1H, s), 8.74 (1H, d, J = 9.8 Hz), 8.06 (1H, br s), 7.31 (1H, d, J = 9.8 Hz), 6.92 (1H, s), 4.61 (1H, q, J = 6.3 Hz), 3.46-3.40 (4H, br m), 2.88-2.79 (4H, br m), 2.53-2.42 (2H, m), 1.58 (6H, s), 1.39 (3H, d, J = 6.3 Hz). | | |
| 328 | 1H-NMR (DMSO-D6) δ: 10.63 (1H, s), 9.36 (1H, s), 8.23-8.20 (2H, m), 7.60 (1H, s), 7.41 (1H, d, J = 9.8 Hz), 6.52 (1H, d, J = 7.8 Hz), 4.32-4.29 (1H, m), 3.52 (7H, t, J = 4.9 Hz), 3.15 (1H, s), 2.92 (4H, t, J = 4.9 Hz), 2.59 (3H, s), 1.33 (6H, d, J = 6.8 Hz). | | |

**[Table 5-58]**

| Compound No. | NMR | LC/MS (M+H)⁺ | Exact Mass |
|---|---|---|---|
| 329 | 1H-NMR (DMSO-D6) δ: 10.45 (1H, s), 9.41 (1H, s), 8.28-8.25 (3H, m), 7.79 (1H, t, J = 8.3 Hz), 7.62 (1H, s), 6.62 (1H, d, J = 7.8 Hz), 4.35-4.32 (1H, m), 2.82 (3H, t, J = 3.9 Hz), 2.60 (3H, s), 2.40-2.33 (4H, m), 1.35 (6H, d, J = 6.8 Hz). | | |
| 330 | 1H-NMR (DMSO-D6) δ: 10.65 (1H, s), 9.38 (1H, s), 8.23-8.22 (2H, m), 7.62 (1H, s), 7.41 (1H, d, J = 9.8 Hz), 6.64 (1H, d, J = 7.8 Hz), 4.23-4.21 (1H, m), 3.92 (3H, d, J = 11.7 Hz), 3.51-3.49 (9H, m), 2.91 (5H, t, J = 4.9 Hz), 2.59 (3H, s), 2.05 (2H, d, J = 12.7 Hz), 1.71-1.64 (2H, m). | | |
| 331 | 1H-NMR (DMSO-D6) δ: 10.39 (1H, s), 9.29 (1H, s), 8.97 (1H, s), 8.72 (1H, d, J = 2.0 Hz), 8.57 (1H, d, J = 4.9 Hz), 8.43-8.40 (2H, m), 8.23 (1H, dd, J = 8.8, 2.0 Hz), 8.16 (1H, d, J = 7.8 Hz), 7.50 (1H, dd, J = 7.8, 4.9 Hz), 6.92 (1H, s), 6.50 (1H, d, J = 6.8 Hz), 4.29-4.26 (3H, m), 3.28 (3H, s), 1.39 (3H, d, J = 5.9 Hz), 1.31 (6H, dd, J = 5.9, 3.9 Hz). | | |
| 332 | 1H-NMR (DMSO-D6) δ: 10.09 (1H, s), 9.24 (1H, s), 8.34 (1H, s), 8.23-8.22 (2H, m), 7.74 (1H, d, J = 8.8 Hz), 6.89 (1H, s), 6.45 (1H, d, J = 6.8 Hz), 4.28-4.24 (2H, m), 3.27 (3H, s), 3.12-3.09 (2H, m), 2.77-2.75 (2H, m), 2.67-2.64 (1H, m), 2.54-2.52 (2H, br m), 1.87-1.85 (1H, m), 1.78-1.75 (1H, m), 1.64-1.61 (2H, m), 1.38 (3H, d, J = 6.8 Hz), 1.30 (6H, dd, J = 6.8, 3.9 Hz). | | |

**[Table 5-59]**

| Compound No. | NMR | LC/MS (M+H)⁺ | Exact Mass |
|---|---|---|---|
| 333 | 1H-NMR (DMSO-D6) δ: 10.18 (1H, s), 9.22 (1H, s), 8.28 (1H, s), 8.23 (1H, s), 8.17 (1H, d, J = 8.8 Hz), 7.70 (1H, d, J = 6.8 Hz), 6.99 (1H, s), 6.69 (1H, s), 5.23 (1H, br s), 4.63-4.61 (1H, m), 3.56 (2H, s), 3.38 (2H, s), 3.14-3.10 (2H, m), 2.79-2.77 (2H, m), 2.70-2.68 (1H, m), 2.54-2.52 (3H, m), 1.89-1.86 (1H, m), 1.79-1.76 (1H, m), 1.63-1.57 (2H, m), 1.50 (6H, s), 1.39 (3H, d, J = 6.8 Hz). | | |
| 334 | 1H-NMR (DMSO-D6) δ: 10.09 (1H, s), 9.19 (1H, s), 8.08 (1H, d, J = 12.8 Hz), 7.00 (1H, d, J = 12.8 Hz), 6.86 (1H, s), 6.34 (1H, d, J = 10.0 Hz), 4.99 (1H, d, J = 4.8 Hz), 4.43 (1H, br s), 4.23 (1H, q, J = 8.8 Hz), 3.50-3.59 (3H, m), 3.37-3.40 (1H, m), 3.30 (3H, s), 1.91-2.07 (2H, m), 1.38 (3H, d, J = 8.4 Hz), 1.29 (6H, d, J = 2.8 Hz). | 425.3 | 424.23 |
| 335 | 1H-NMR (DMSO-D6) δ: 10.31 (1H, s), 9.22 (1H, s), 8.23 (1H, d, J = 9.6 Hz), 7.46 (1H, d, J = 9.6 Hz), 6.89 (1H, s), 6.38 (1H, d, J = 7.6 Hz), 4.65 (1H, t, J = 5.6 Hz), 4.23-4.28 (2H, m), 4.16 (2H, d, J = 5.6 Hz), 3.53-3.64 (8H, br m), 3.30 (3H, s), 1.91-2.07 (2H, m), 1.38 (3H, d, J = 6.4 Hz), 1.30 (6H, dd, J = 6.4, 3.2 Hz). | 482.2 | 481.25 |
| 336 | 1H-NMR (DMSO-D6) δ: 9.99 (1H, s), 9.17 (1H, s), 7.96 (1H, d, J = 9.2 Hz), 6.90 (1H, d, J = 9.6 Hz), 6.85 (1H, s), 6.50 (1H, d, J = 7.6 Hz), 6.34 (1H, d, J = 7.6 Hz), 4.19-4.25 (2H, m), 3.80-3.86 (1H, br m), 3.29 (3H, s), 2.93-2.96 (2H, m), 2.53-2.56 (2H, m), 1.90-1.92 (2H, m), 1.37 (3H, d, J = 6.4 Hz), 1.23-1.30 (8H, m). | | |

**[Table 5-60]**

| Compound No. | NMR | LC/MS (M+H)⁺ | Exact Mass |
|---|---|---|---|
| 337 | 1H-NMR (CDCl3) δ: 9.35 (1H, s), 8.39-8.21 (2H, m), 7.07 (1H, d, J = 9.8 Hz), 6.38 (1H, d, J = 7.3 Hz), 5.17 (1H, q, J = 6.3 Hz), 4.33-4.23 (1H, m), 4.10-4.01 (2H, m), 3.70-3.55 (6H, m), 3.10-3.00 (4H, m), 2.21-2.10 (2H, m), 1.73-1.65 (2H, m), 1.46 (3H, d, J = 6.3 Hz). | | |
| 338 | 1H-NMR (DMSO-D6) δ: 10.19 (1H, br s), 9.18 (1H, s), 8.15 (1H, d, J = 9.8 Hz), 7.37 (1H, d, J = 9.8 Hz), 6.94 (1H, s), 6.30 (1H, d, J = 7.8 Hz), 5.19 (1H, br s), 4.64-4.54 (1H, m), 3.92-3.81 (1H, br m), 2.85-2.75 (4H, m), 2.13-1.79 (5H, m), 1.49-1.18 (7H, m). | | |
| 339 | 1H-NMR (DMSO-D6) δ: 10.23 (1H, br s), 9.18 (1H, s), 8.15 (1H, d, J = 9.8 Hz), 7.37 (1H, d, J = 10.2 Hz), 6.85 (1H, s), 6.34 (1H, d, J = 6.8 Hz), 4.27-4.19 (1H, m), 3.91-3.81 (1H, m), 3.45-3.40 (4H, m), 3.26 (3H, s), 2.85-2.77 (4H, m), 2.10-1.84 (5H, m), 1.47-1.20 (7H, m). | | |
| 340 | 1H-NMR (DMSO-D6) δ: 10.19 (1H, s), 9.19 (1H, s), 8.15 (1H, d, J = 9.8 Hz), 7.36 (1H, d, J = 9.8 Hz), 6.95 (1H, s), 6.31 (1H, d, J = 7.3 Hz), 5.21-5.13 (1H, m), 4.63-4.51 (2H, m), 3.94-3.80 (1H, m), 3.52-3.38 (5H, m), 2.84-2.75 (4H, m), 2.32 (1H, br s), 2.12-1.79 (4H, m), 1.45-1.24 (7H, m). | | |
| 341 | 1H-NMR (DMSO-D6) δ: 10.23 (1H, br s), 9.19 (1H, s), 8.15 (1H, d, J = 9.8 Hz), 7.36 (1H, d, J = 10.2 Hz), 6.86 (1H, s), 6.34 (1H, d, J = 7.8 Hz), 4.57 (1H, d, J = 3.9 Hz), 4.23 (1H, q, J = 6.3 Hz), 3.94-3.79 (1H, m), 3.56-3.37 (5H, m), 3.26 (3H, s), 2.85-2.77 (4H, m), 2.32 (1H, br s), 2.11-2.00 (2H, m), 1.89-1.79 (2H, m), 1.44-1.21 (7H, m). | | |

**[Table 5-61]**

| Compound No. | NMR | LC/MS (M+H) ⁺ | Exact Mass |
|---|---|---|---|
| 342 | 1H-NMR (DMSO-D6) δ: 10.02 (1H, s), 9.22 (1H, s), 8.25-8.12 (2H, m), 7.74 (1H, d, J = 8.3 Hz), 6.96 (1H, s), 6.42-6.33 (1H, m), 5.27-5.12 (1H, m), 4.63-4.57 (1H, m), 3.93-3.84 (1H, m), 2.71-2.62 (4H, br m), 2.33-2.21 (4H, br m), 2.11-2.00 (3H, m), 1.91-1.82 (2H, m), 1.48-1.27 (7H, m). | | |
| 343 | 1H-NMR (DMSO-D6) δ: 10.05 (1H, s), 9.23 (1H, s), 8.25-8.18 (2H, m), 7.79-7.71 (1H, m), 6.87 (1H, s), 6.45-6.39 (1H, m), 4.29-4.20 (1H, m), 3.92-3.84 (1H, m), 2.70-2.61 (4H, m), 2.34-2.21 (4H, m), 2.11-2.00 (2H, m), 1.92-1.80 (3H, m), 1.48-1.19 (7H, m). | | |
| 344 | 1H-NMR (DMSO-D6) δ: 10.05 (1H, br s), 9.23 (1H, s), 8.27-8.16 (2H, m), 7.73 (1H, d, J = 8.8 Hz), 6.98 (1H, s), 6.40 (1H, d, J = 7.8 Hz), 5.20 (1H, br s), 4.67-4.52 (2H, m), 3.98-3.83 (1H, m), 3.56-3.38 (3H, m), 3.25-3.16 (1H, m), 2.71-2.61 (3H, br m), 2.31-2.19 (4H, m), 2.14-2.00 (2H, m), 1.92-1.81 (2H, m), 1.52-1.24 (7H, m). | | |
| 345 | 1H-NMR (DMSO-D6) δ: 10.09 (1H, br s), 9.24 (1H, s), 8.26-8.16 (2H, m), 7.73 (1H, d, J = 8.8 Hz), 6.88 (1H, s), 6.44 (1H, d, J = 7.8 Hz), 4.61-4.53 (1H, br m), 4.24 (1H, q, J = 6.3 Hz), 3.95-3.83 (1H, m), 3.55-3.45 (1H, br m), 3.40 (2H, s), 3.27 (3H, s), 2.70-2.63 (4H, m), 2.35-2.21 (4H, br m), 2.11-2.01 (2H, m), 1.91-1.83 (2H, m), 1.49-1.25 (7H, m). | | |
| 346 | 1H-NMR (CDC13) δ: 9.04 (1H, s), 8.47 (1H, d, J = 8.8 Hz), 8.31 (1H, d, J = 2.9 Hz), 8.19 (1H, s), 7.76 (1H, dd, J = 8.8, 2.0 Hz), 6.75 (1H, s), 6.68 (1H, d, J = 7.8 Hz), 4.81 (1H, q, J = 6.2 Hz), 4.35 (1H, s), 4.03 (1H, dd, J = 11.2, 3.4 Hz), 3.86 (1H, s), 3.78-3.73 (7H, m), 3.67-3.61 (2H, m), 3.27 (2H, t, J = 5.4 Hz), 2.08-2.06 (1H, m), 1.90-1.87 (2H, m), 1.53 (2H, d, J = 6.8 Hz), 1.25 (2H, s). | | |

**[Table 5-62]**

| Compound No. | NMR | LC/MS (M+H)⁺ | Exact Mass |
|---|---|---|---|
| 347 | 1H-NMR (CDC13) δ: 9.05 (1H, s), 8.47 (1H, d, J = 8.8 Hz), 8.32 (1H, d, J = 2.9 Hz), 8.27 (1H, s), 7.76 (1H, dd, J = 8.8, 2.9 Hz), 7.26 (6H, s), 6.75 (1H, s), 6.69 (1H, d, J = 7.8 Hz), 4.81 (1H, q, J = 6.5 Hz), 4.35 (1H, s), 4.02 (1H, dd, J = 11.2, 2.4 Hz), 3.78-3.73 (7H, m), 3.65-3.62 (2H, m), 3.27 (2H, t, J = 5.4 Hz), 2.10-2.06 (1H, m), 1.89-1.88 (2H, m), 1.53 (4H, d, J = 6.8 Hz), 1.25 (1H, s). | | |
| 348 | 1H-NMR (CDC13) δ: 9.06 (1H, s), 8.40 (1H, d, J = 8.8 Hz), 8.32 (1H, d, J = 2.9 Hz), 8.27 (1H, s), 7.76 (1H, dd, J = 8.8, 2.9 Hz), 6.78 (1H, s), 6.50 (1H, d, J = 6.8 Hz), 4.83-4.82 (2H, m), 4.11-4.07 (2H, m), 3.95-3.93 (1H, m), 3.87 (1H, dd, J = 9.3, 3.4 Hz), 3.75 (5H, t, J = 5.4 Hz), 3.27 (2H, t, J = 5.4 Hz), 2.47-2.43 (1H, m), 2.06-2.01 (1H, m), 1.54 (3H, d, J = 6.8 Hz). | | |
| 349 | 1H-NMR (CDC13) δ: 9.06 (0H, s), 8.40 (0H, d, J = 8.8 Hz), 8.32 (0H, d, J = 2.0 Hz), 8.21 (0H, s), 7.76 (0H, dd, J = 8.8, 2.9 Hz), 6.79 (0H, s), 6.50 (0H, d, J = 6.8 Hz), 4.83-4.82 (1H, m), 4.11-4.06 (1H, m), 3.93-3.88 (1H, m), 3.74 (2H, t, J = 4.9 Hz), 3.27 (1H, t, J = 5.4 Hz), 2.50-2.03 (1H, m), 1.54 (2H, d, J = 5.9 Hz). | | |
| 350 | 1H-NMR (CDC13) δ: 9.05 (1H, s), 8.41 (1H, d, J = 8.8 Hz), 8.33 (1H, d, J = 2.0 Hz), 8.26 (1H, s), 7.76 (1H, dd, J = 8.8, 2.9 Hz), 6.76 (1H, s), 6.35 (1H, d, J = 7.8 Hz), 4.82 (1H, q, J = 6.5 Hz), 4.34-4.33 (1H, m), 4.07-4.05 (2H, m), 3.75 (4H, t, J = 4.9 Hz), 3.67-3.61 (2H, m), 3.28 (2H, t, J = 5.4 Hz), 2.18-2.16 (2H, m), 1.53 (2H, d, J = 6.8 Hz). | | |

**[Table 5-63]**

| Compound No. | NMR | LC/MS (M+H) ⁺ | Exact Mass |
|---|---|---|---|
| 351 | 1H-NMR (CDC13) δ: 9.07 (1H, s), 8.50 (1H, dd, J = 8.8, 2.0 Hz), 8.42 (1H, d, J = 2.0 Hz), 8.31 (1H, d, J = 2.9 Hz), 8.21 (1H, s), 7.74-7.70 (1H, m), 6.76 (1H, s), 6.70 (1H, d, J = 7.8 Hz), 4.82-4.80 (1H, br m), 4.41 (1H, s), 4.35 (1H, br s), 4.28 (1H, s), 4.04-3.98 (2H, m), 3.86 (2H, s), 3.83-3.77 (4H, m), 3.67-3.64 (1H, m), 2.10-2.02 (1H, m), 1.90-1.89 (2H, m), 1.53 (3H, d, J = 6.8 Hz). | | |
| 352 | 1H-NMR (CDC13) δ: 9.08 (1H, s), 8.60 (1H, d, J = 7.8 Hz), 8.50 (1H, dd, J = 9.3, 2.4 Hz), 8.32 (1H, d, J = 2.9 Hz), 8.21 (1H, s), 7.74-7.70 (1H, m), 6.76 (1H, s), 6.70 (1H, d, J = 8.8 Hz), 4.82-4.80 (1H, m), 4.41 (1H, s), 4.35 (1H, br s), 4.28 (1H, s), 4.03-3.98 (2H, m), 3.86 (3H, s), 3.80-3.77 (3H, m), 3.67-3.64 (1H, m), 2.08-2.05 (1H, m), 1.97-1.80 (2H, m), 1.53 (3H, d, J = 6.8 Hz). | | |
| 353 | 1H-NMR (CDC13) δ: 9.07 (1H, s), 8.43 (1H, dd, J = 8.8, 2.0 Hz), 8.34 (1H, s), 8.30 (1H, d, J = 2.9 Hz), 8.21 (1H, s), 7.75-7.69 (1H, m), 6.79 (1H, s), 6.50 (1H, d, J = 6.8 Hz), 4.82 (2H, br s), 4.41 (1H, s), 4.28 (1H, s), 4.14-4.04 (2H, m), 4.02-3.88 (3H, m), 3.86 (3H, s), 3.50-3.40 (2H, m), 2.50-2.40 (1H, m), 2.15-1.95 (1H, m), 1.54 (4H, d, J = 6.8 Hz). | | |
| 354 | 1H-NMR (CDC13) δ: 9.08 (1H, s), 8.42 (2H, td, J = 9.0, 3.3 Hz), 8.31 (1H, d, J = 2.0 Hz), 8.21 (1H, s), 7.74-7.70 (1H, m), 6.80 (1H, s), 6.50 (1H, d, J = 6.8 Hz), 4.88-4.77 (2H, m), 4.42 (1H, s), 4.28 (1H, s), 4.12-4.05 (2H, m), 4.01-3.88 (3H, m), 3.86 (3H, s), 3.81 (2H, t, J = 5.4 Hz), 2.52-2.40 (1H, m), 2.12-1.97 (1H, m), 1.55 (3H, d, J = 6.8 Hz). | | |

**[Table 5-64]**

| Compound No. | NMR | LC/MS (M+H) ⁺ | Exact Mass |
|---|---|---|---|
| 355 | 1H-NMR (CDC13) δ: 9.09 (1H, s), 8.60 (1H, d, J = 4.9 Hz), 8.45 (1H, dd, J = 8.8, 2.9 Hz), 8.33 (1H, d, J = 2.0 Hz), 8.21 (1H, s), 7.73 (1H, td, J = 6.1, 3.3 Hz), 6.77 (1H, s), 6.34 (1H, d, J = 7.8 Hz), 4.88-4.77 (1H, m), 4.42 (1H, s), 4.40-4.31 (1H, m), 4.29 (1H, s), 4.06 (2H, td, J = 7.6, 3.6 Hz), 4.00 (1H, t, J = 5.4 Hz), 3.91 (1H, s), 3.87 (2H, s), 3.81 (1H, t, J = 5.9 Hz), 3.67-3.61 (2H, m), 2.25-2.10 (2H, m), 1.76-1.67 (2H, m), 1.54 (3H, d, J = 5.9 Hz). | | |
| 356 | 1H-NMR (DMSO-D6) δ: 10.79 (1H, br s), 9.30 (1H, s), 8.55 (1H, d, J = 12.8 Hz), 8.06 (1H, d, J = 12.8 Hz), 6.93 (1H, s), 6.52 (1H, d, J = 10.0 Hz), 4.25-4.29 (2H, br m), 3.95 (2H, t, J = 6.8 Hz), 3.51 (2H, s), 3.28 (3H, s), 3.10 (2H, t, J = 7.2 Hz), 1.39 (3H, d, J = 8.8 Hz), 1.30 (6H, dd, J = 8.8, 3.6). | 438.4 | 437.23 |
| 357 | 1H-NMR (DMSO-D6) δ: 10.01 (1H, br s), 9.19 (1H, s), 8.02 (1H, d, J = 12.8 Hz), 6.93 (1H, d, J = 12.8 Hz), 6.87 (1H, s), 6.80 (1H, d, J = 7.2 Hz), 6.35 (1H, d, J = 10.0 Hz), 4.34-4.38 (1H, br m), 4.19-4.26 (2H, br m), 3.27 (3H, s), 2.90-3.20 (3H, br m), 1.65-2.20 (3H, br m), 1.38 (3H, d, J = 8.4 Hz), 1.27 (6H, dd, J = 8.4, 2.8 Hz) . | 424.3 | 423.25 |
| 358 | 1H-NMR (CDC13) δ: 9.04 (1H, s), 8.38-8.35 (2H, m), 7.07 (1H, d, J = 9.8 Hz), 6.86 (1H, s), 6.39 (1H, t, J = 6.1 Hz), 4.32 (1H, q, J = 6.3 Hz), 4.03-4.00 (2H, m), 3.58-3.55 (6H, m), 3.42-3.38 (5H, m), 3.06-3.05 (4H, m), 2.05-2.03 (2H, m), 2.04-1.95 (2H, m), 1.52-1.45 (5H, m). | | |

**[Table 5-65]**

| Compound No. | NMR | LC/MS (M+H) ⁺ | Exact Mass |
|---|---|---|---|
| 359 | 1H-NMR (CDC13) δ: 9.10 (1H, s), 8.57 (1H, br s), 8.30 (1H, d, J = 10.2 Hz), 7.13 (1H, d, J = 10.2 Hz), 6.94 (1H, s), 6.22 (1H, d, J = 7.8 Hz), 4.47-4.43 (1H, m), 4.32 (1H, q, J = 6.3 Hz), 3.61-3.60 (4H, m), 3.39 (3H, s), 3.22-3.21 (4H, m), 3.06-3.05 (4H, m), 2.58-2.54 (2H, m), 2.36-2.32 (2H, m), 1.49 (3H, d, J = 6.3 Hz). | | |
| 360 | 1H-NMR (CDC13) δ: 9.05 (1H, s), 8.34-8.28 (3H, m), 7.73 (1H, dd, J = 8.5, 4.3 Hz), 6.87 (1H, s), 6.44 (1H, t, J = 6.1 Hz), 4.34 (1H, q, J = 6.5 Hz), 4.04-4.01 (2H, m), 3.60-3.57 (2H, m), 3.49 (2H, br s), 3.44-3.41 (5H, m), 2.93-2.91 (4H, m), 2.45-2.42 (4H, m), 2.01-1.98 (1H, m), 1.76-1.74 (3H, m), 1.52-1.46 (5H, m). | | |
| 361 | 1H-NMR (CDC13) δ: 9.10 (1H, s), 8.44 (1H, s), 8.29-8.27 (2H, m), 7.79-7.77 (1H, m), 6.95 (1H, s), 6.26 (1H, d, J = 7.3 Hz), 4.47-4.31 (2H, m), 3.51 (2H, br s), 3.40 (3H, s), 3.22-3.21 (4H, m), 2.92-2.91 (4H, m), 2.56-2.32 (8H, m), 1.50 (3H, d, J = 6.3 Hz). | | |
| 362 | 1H-NMR (DMSO-D6) δ: 10.30 (1H, s), 9.22 (1H, s), 8.23 (1H, d, J = 12.8 Hz) 8.11 (1H, br s), 7.43 (1H, d, J = 13.2 Hz), 6.88 (1H, s), 6.37 (1H, d, J = 10.8 Hz), 4.34-4.38 (1H, br m), 4.22-4.26 (2H, br m), 4.07 (2H, s), 3.72-3.79 (3H, m), 3.27 (4H, apparent s), 1.38 (3H, d, J = 8.8 Hz), 1.30 (6H, d, J = 5.2 Hz). | 438.4 | 437.23 |
| 363 | 1H-NMR (DMSO-D6) δ: 9.95 (1H, s), 9.19 (1H, s), 8.92 (1H, d, J = 1.2 Hz), 8.06 (1H, d, J = 1.2 Hz), 6.87 (1H, s), 6.37 (1H, d, J = 7.6 Hz), 4.22-4.26 (2H, br m), 3.29-3.41 (4H, m), 3.26 (3H, s), 2.80-2.83 (4H, m), 1.38 (3H, d, J = 6.4 Hz), 1.28 (6H, dd, J = 6.4, 3.2 Hz). | 424.3 | 423.25 |

**[Table 5-66]**

| Compound No. | NMR | LC/MS (M+H) ⁺ | Exact Mass |
|---|---|---|---|
| 364 | 1H-NMR (DMSO-D6) δ: 10.33 (1H, s), 9.34 (1H, s), 8.28 (1H, d, J = 8.8 Hz), 8.21 (1H, s), 7.77 (1H, d, J = 7.8 Hz), 7.24 (1H, s), 6.96-6.65 (2H, m), 4.20 (1H, br s), 3.91 (2H, d, J = 11.7 Hz), 3.49 (2H, t, J = 11.2 Hz), 3.42 (3H, s), 2.68 (4H, t, J = 4.4 Hz), 2.53 (2H, s), 2.29 (4H, s), 2.00 (2H, d, J = 10.7 Hz), 1.77-1.63 (2H, m) . | | |
| 365 | 1H-NMR (DMSO-D6) δ: 10.53 (1H, s), 9.30 (1H, s), 8.26-8.18 (2H, m), 7.41 (1H, d, J = 9.8 Hz), 7.22 (1H, s), 6.95-6.64 (2H, m), 4.17 (1H, br s), 3.89 (2H, d, J = 11.7 Hz), 3.57-3.50 (3H, m), 3.49-3.42 (2H, m), 2.98-2.87 (4H, m), 2.55-2.50 (3H, m), 1.98 (2H, d, J = 10.7 Hz), 1.70-1.55 (2H, m). | | |
| 366 | 1H-NMR (DMSO-D6) δ: 10.21 (1H, s), 9.30 (1H, s), 8.25-8.00 (3H, m), 7.52 (1H, d, J = 7.8 Hz), 7.22 (1H, s), 6.97-6.63 (2H, m), 4.30-4.07 (3H, br m), 3.90 (2H, d, J = 9.8 Hz), 3.48 (1H, t, J = 10.7 Hz), 2.70-2.64 (2H, br m), 2.25 (6H, s), 2.05-1.96 (2H, m), 1.75-1.58 (2H, m). | | |
| 367 | 1H-NMR (CDC13) δ: 9.34 (1H, s), 8.36 (1H, d, J = 9.8 Hz), 8.28 (1H, br s), 7.06 (1H, d, J = 9.8 Hz), 6.76 (1H, d, J = 7.3 Hz), 5.16 (1H, q, J = 6.3 Hz), 4.34-4.22 (1H, m), 3.96 (1H, dd, J = 11.2, 2.9 Hz), 3.84-3.56 (7H, m), 3.08-3.01 (4H, m), 2.09-1.59 (4H, m), 1.45 (3H, d, J = 6.3 Hz). | | |
| 368 | 1H-NMR (CDC13) δ: 9.34 (1H, s), 8.31 (1H, d, J = 9.8 Hz), 8.22 (1H, br s), 7.07 (1H, d, J = 9.8 Hz), 6.30 (1H, d, J = 7.3 Hz), 5.16 (1H, q, J = 6.2 Hz), 4.08-3.97 (1H, m), 3.79-3.70 (1H, m), 3.63-3.52 (4H, m), 3.07-2.98 (4H, m), 2.32-2.01 (4H, m), 1.60-1.36 (7H, m). | | |

**[Table 5-67]**

| Compound No. | NMR | LC/MS (M+H) ⁺ | Exact Mass |
|---|---|---|---|
| 369 | 1H-NMR (CDC13) δ: 9.40 (1H, s), 8.50 (1H, br s), 8.33 (1H, d, J = 8.3 Hz), 8.30 (1H, d, J = 2.4 Hz), 7.76 (1H, dd, J = 8.5, 2.2 Hz), 6.80 (1H, d, J = 7.8 Hz), 5.17 (1H, q, J = 6.3 Hz), 4.34-4.28 (1H, br m), 3.98 (1H, dd, J = 11.2, 2.9 Hz), 3.84-3.63 (3H, m), 3.50 (2H, s), 2.94-2.86 (4H, m), 2.50-2.39 (4H, br m), 2.10-1.84 (3H, m), 1.46 (3H, d, J = 6.3 Hz). | | |
| 370 | 1H-NMR (CDC13) δ: 9.40 (1H, s), 8.43 (1H, br s), 8.31-8.24 (2H, m), 7.80-7.72 (1H, m), 6.33 (1H, d, J = 7.8 Hz), 5.17 (1H, q, J = 6.2 Hz), 4.09-3.99 (1H, m), 3.82-3.72 (1H, m), 3.50 (2H, s), 2.95-2.86 (4H, m), 2.57-2.20 (6H, m), 2.16-2.01 (2H, m), 1.59-1.40 (7H, m). | | |
| 371 | 1H-NMR (DMSO-D6) δ: 10.17 (1H, s), 9.19 (1H, s), 8.13 (1H, d, J = 9.8 Hz), 7.30 (1H, d, J = 9.8 Hz), 6.86 (1H, s), 6.35 (1H, d, J = 7.3 Hz), 4.35-4.29 (1H, m), 4.29-4.16 (2H, m), 3.89 (1H, dd, J = 12.9, 3.7 Hz), 3.26 (3H, s), 2.96 (2H, dt, J = 18.2, 6.5 Hz), 2.88 (1H, dd, J = 12.2, 3.4 Hz), 2.80 (1H, d, J = 11.7 Hz), 2.66 (1H, dt, J = 20.2, 8.3 Hz), 1.37 (3H, d, J = 6.8 Hz), 1.27 (6H, dd, J = 6.3, 3.4 Hz), 1.13 (3H, d, J = 6.8 Hz). | 438.35 | 437.27 |
| 372 | 1H-NMR (DMSO-D6) δ: 10.17 (1H, s), 9.19 (1H, s), 8.13 (1H, d, J = 10.2 Hz), 7.30 (1H, d, J = 9.8 Hz), 6.86 (1H, s), 6.35 (1H, d, J = 7.3 Hz), 4.33 (1H, dt, J = 11.7, 5.0 Hz), 4.23 (2H, td, J = 13.2, 6.8 Hz), 3.90 (1H, dd, J = 13.2, 4.4 Hz), 3.26 (3H, s), 2.99-2.92 (2H, m), 2.88 (1H, dd, J = 12.0, 3.7 Hz), 2.80 (1H, d, J = 11.7 Hz), 2.64 (1H, td, J = 12.1, 3.6 Hz), 1.37 (3H, d, J = 6.3 Hz), 1.27 (6H, dd, J = 6.3, 2.9 Hz), 1.13 (3H, d, J = 6.8 Hz). | 438.35 | 437.27 |

**[Table 5-68]**

| Compound No. | NMR | LC/MS (M+H) ⁺ | Exact Mass |
|---|---|---|---|
| 373 | 1H-NMR (DMSO-D6) δ: 10.02 (1H, s), 9.17 (1H, s), 8.97 (1H, d, J = 1.2 Hz), 8.08 (1H, d, J = 1.2 Hz), 6.96 (1H, s), 6.76 (1H, s), 5.18 (1H, d, J = 4.4 Hz), 4.60-4.63 (1H, m), 3.50 (2H, s), 3.43 (3H, s), 3.38-3.41 (4H, m), 2.79-2.81 (4H, m), 1.49 (6H, s), 1.39 (3H, d, J = 6.4 Hz). | 454.3 | 453.26 |
| 374 | 1H-NMR (DMSO-D6) δ: 10.19 (1H, s), 9.20 (1H, s), 8.37 (2H, s), 6.86 (1H, s), 6.51 (1H, d, 7.8 Hz), 4.20-4.30 (2H, m), 3.30 (3H, s), 3.01-3.11 (4H, m), 2.86-2.89 (4H, m), 1.38 (3H, d, J = 8.8 Hz), 1.27 (6H, dd, J = 8.8, 3.2 Hz). | 424.4 | 423.25 |
| 375 | 1H-NMR (DMSO-D6) δ: 10.24 (1H, s), 9.17 (1H, s), 8.38 (2H, s), 6.95 (1H, s), 6.78 (1H, s), 5.18 (1H, d, J = 4.4 Hz), 4.60-4.62 (1H, m), 3.57 (2H, s), 3.30 (3H, s), 3.11-3.12 (4H, m), 2.91-2.92 (4H, m), 1.47 (6H, s), 1.39 (3H, d, J = 6.4 Hz). | 454.2 | 453.26 |
| 376 | 1H-NMR (CDC13) δ: 9.06 (1H, s), 8.52 (1H, brs), 8.42 (1H, d, J = 9.8 Hz), 7.04 (1H, d, J = 9.8 Hz), 6.88 (1H, s), 6.49 (1H, d, J = 7.8 Hz), 4.33 (2H, dq, J = 19.1, 5.0 Hz), 4.04 (1H, dd, J = 11.2, 2.9 Hz), 3.75 (2H, ddt, J = 21.5, 12.1, 3.8 Hz), 3.57 (3H, dq, J = 15.5, 4.2 Hz), 3.48 (2H, d, J = 2.4 Hz), 3.40 (3H, s), 3.14 (2H, dd, J = 6.1, 4.1 Hz), 2.04 (1H, td, J = 8.8, 4.6 Hz), 1.91-1.83 (3H, m), 1.74-1.68 (1H, m), 1.48 (3H, d, J = 6.3 Hz), 0.68 (4H, dt, J = 21.6, 5.5 Hz). | 492.40 | 491.28 |

**[Table 5-69]**

| Compound No. | NMR | LC/MS (M+H) ⁺ | Exact Mass |
|---|---|---|---|
| 377 | 1H-NMR (CDC13) δ: 9.09 (1H, s), 8.51-8.50 (2H, m), 8.24 (1H, d, J = 2.9 Hz), 7.59 (1H, dd, J = 8.8, 2.9 Hz), 6.88 (1H, s), 6.13 (1H, d, J = 7.8 Hz), 4.47-4.33 (2H, m), 3.42 (3H, s), 3.28 (3H, s), 3.06-3.02 (2H, br m), 2.42-2.39 (3H, br m), 1.77-1.71 (4H, m), 1.51 (3H, d, J = 6.3 Hz), 1.37 (3H, d, J = 6.3 Hz), 1.37 (3H, d, J = 6.3 Hz). | | |
| 378 | 1H-NMR (CDC13) δ: 9.09 (1H, s), 8.62 (1H, brs), 8.39 (1H, d, J = 8.3 Hz), 8.29 (1H, d, J = 2.0 Hz), 7.76 (1H, dd, J = 8.5, 2.2 Hz), 6.89 (1H, s), 6.53 (1H, d, J = 7.8 Hz), 4.40-4.30 (2H, m), 4.06 (1H, dd, J = 10.7, 2.9 Hz), 3.82-3.71 (2H, m), 3.58 (1H, dd, J = 11.0, 6.1 Hz), 3.52 (2H, s), 3.41 (3H, s), 3.04 (2H, t, J = 4.9 Hz), 2.55 (3H, brs), 2.31 (2H, s), 2.08 (1H, td, J = 9.5, 4.6 Hz), 1.88 (2H, dtd, J = 28.8, 8.5, 4.2 Hz), 1.74 (1H, tt, J = 11.7, 3.7 Hz), 1.49 (3H, d, J = 6.3 Hz), 0.69 (2H, t, J=5.1 Hz), 0.49 (2H, t, J = 5.6 Hz). | 505.40 | 504.30 |
| 379 | 1H-NMR (CDC13) δ: 8.99 (1H, s), 8.35 (1H, d, J = 9.8 Hz), 8.17 (1H, brs), 7.04 (1H, d, J = 9.8 Hz), 6.68 (1H, s), 6.21 (1H, d, J = 7.8 Hz), 4.80 (1H, q, J = 6.5 Hz), 4.48-4.32 (1H, m), 4.05 (1H, brs), 3.55 (2H, t, J = 5.1 Hz), 3.48 (2H, s), 3.14 (2H, t, J = 5.1 Hz), 1.53 (3H, d, J = 6.8 Hz), 1. 37 (6H, d, J = 6.3 Hz), 0.67 (4H, dt, J = 20.2, 5.6 Hz). | 436.35 | 435.25 |
| 380 | 1H-NMR (DMSO-D6) δ: 10.46 (1H, s), 9.35 (1H, s), 8.31-8.22 (3H, m), 7.78-7.72 (1H, m), 7.25 (1H, s), 6.98-6.66 (2H, m), 4.25-4.10 (1H, m), 3.90 (1H, dd, J = 10.7, 2.9 Hz), 3.72-3.64 (1H, m), 3.61-3.53 (1H, m), 3.52-3.42 (3H, m), 3.40-3.33 (1H, m), 2.92-2.85 (3H, m), 2.48-2.25 (4H, m), 2.04-1.94 (1H, m), 1.90-1.70 (2H, m), 1.67-1.55 (1H, m). | | |

**[Table 5-70]**

| Compound No. | NMR | LC/MS (M+H) ⁺ | Exact Mass |
|---|---|---|---|
| 381 | 1H-NMR (DMSO-D6) δ: 10.56 (1H, s), 9.30 (1H, s), 8.26 (1H, s), 8.20-8.10 (1H, m), 7.37 (1H, d, J = 9.8 Hz), 7.23 (1H, s), 6.95-6.63 (2H, m), 4.19-4.08 (1H, m), 3.88 (1H, dd, J = 10.7, 2.9 Hz), 3.71-3.63 (1H, m), 3.58-3.47 (3H, m), 3.45-3.36 (1H, m), 2.93-2.85 (3H, m), 2.03-1.90 (1H, m), 1.83-1.69 (2H, m), 1.66-1.53 (1H, m). | | |
| 382 | 1H-NMR (DMSO-D6) δ: 10.29 (1H, s), 9.31 (1H, s), 8.19-8.05 (3H, m), 7.47 (1H, dd, J = 9.8, 2.9 Hz), 7.23 (1H, s), 6.97-6.65 (2H, m), 4.13 (3H, t, J = 5.4 Hz), 3.93-3.86 (1H, m), 3.73-3.64 (1H, m), 3.61-3.53 (1H, m), 3.48-3.40 (1H, m), 2.67 (2H, t, J = 5.9 Hz), 2.25 (6H, s), 2.05-1.92 (1H, m), 1.86-1.69 (2H, m), 1.65-1.53 (1H, m). | | |
| 383 | | 513.88 | 512.25 |
| 384 | 1H-NMR (DMSO-D6) δ: 10.03 (1H, s), 9.26 (1H, s), 8.10 (1H, d, J = 9.8 Hz), 7.32 (1H, d, J = 8.8 Hz), 7.19 (1H, s), 6.89-6.50 (2H, m), 3.95 (1H, br s), 3.53-3.45 (4H, m), 2.91-2.83 (3H, m), 2.35-2.22 (2H, m), 2.18-2.10 (2H, m), 2.04-1.94 (2H, m), 1.58-1.37 (4H, m). | | |
| 385 | 1H-NMR (DMSO-D6) δ: 9.88 (1H, s), 9.27 (1H, s), 8.11-8.05 (2H, m), 7.50 (1H, dd, J = 9.3, 3.4 Hz), 7.19 (1H, s), 6.90-6.57 (2H, m), 4.13 (2H, t, J = 5.9 Hz), 4.00-3.91 (1H, m), 2.65 (2H, t, J = 5.9 Hz), 2.24 (6H, s), 2.18-2.10 (2H, m), 2.04-1.95 (2H, m), 1.58-1.37 (4H, m). | | |

**[Table 5-71]**

| Compound No. | NMR | LC/MS (M+H) ⁺ | Exact Mass |
|---|---|---|---|
| 386 | 1H-NMR (DMSO-D6) δ: 10.02 (1H, s), 9.31 (1H, s), 8.28-8.15 (3H, m), 7.99 (1H, s), 7.77 (1H, dd, J = 8.3, 2.4 Hz), 7.21 (1H, s), 6.92-6.60 (2H, m), 5.70 (1H, s), 4.04-3.90 (1H, m), 3.58-3.50 (3H, m), 3.46 (1H, s), 3.42-3.34 (3H, m), 2.76 (1H, t, J = 4.9 Hz), 2.41 (2H, t, J = 4.9 Hz), 2.39-2.33 (3H, m), 2.13-2.05 (2H, m), 1.93-1.84 (2H, m), 1.52-1.27 (5H, m). | | |
| 387 | 1H-NMR (DMSO-D6) δ: 10.49 (1H, s), 9.27 (1H, s), 8.13 (1H, d, J = 9.8 Hz), 7.37 (1H, d, J = 9.8 Hz), 7.18 (1H, s), 6.79 (1H, t, J = 55.6 Hz), 6.57 (1H, d, J = 7.8 Hz), 4.60 (1H, br s), 3.89 (1H, br s), 3.61-3.40 (5H, m), 2.81 (4H, s), 2.56-2.50 (2H, m), 2.08-1.98 (2H, br m), 1.90-1.81 (2H, br m), 1.47-1.12 (5H, m). | | |
| 388 | 1H-NMR (DMSO-D6) δ: 10.21 (1H, s), 9.29 (1H, s), 8.20 (1H, s), 8.12 (1H, d, J = 9.8 Hz), 8.07 (1H, d, J = 2.9 Hz), 7.51 (1H, dd, J = 8.8, 2.9 Hz), 7.19 (1H, s), 6.80 (1H, t, J = 55.6 Hz), 6.60 (1H, d, J = 7.8 Hz), 4.12 (2H, t, J = 5.9 Hz), 3.95-3.85 (1H, m), 3.53-3.48 (1H, m), 2.64 (2H, t, J = 5.4 Hz), 2.23 (6H, s), 2.05 (2H, d, J = 9.8 Hz), 1.87 (2H, d, J = 10.7 Hz), 1.50-1.25 (4H, m). | | |
| 389 | 1H-NMR (CDC13) δ: 9.10 (1H, s), 8.77 (1H, s), 8.49-8.45 (2H, m), 7.88-7.87 (1H, m), 6.87 (1H, s), 6.14 (1H, d, J = 7.8 Hz), 4.42-4.35 (2H, m), 3.66 (4H, br s), 3.42 (3H, s), 2.93 (4H, br s), 1.51 (3H, d, J = 5.9 Hz), 1.37 (3H, d, J = 5.4 Hz), 1.36 (3H, d, J = 5.4 Hz). | | |

**[Table 5-72]**

| Compound No. | NMR | LC/MS (M+H) ⁺ | Exact Mass |
|---|---|---|---|
| 390 | 1H-NMR (CDC13) δ: 9.09 (1H, s), 8.68 (1H, s), 8.35-8.32 (2H, m), 7.73 (1H, dd, J = 8.5, 2.2 Hz), 6.86 (1H, s), 6.41 (1H, t, J = 5.9 Hz), 4.33 (1H, q, J = 6.5 Hz), 3.68 (3H, s), 3.54-3.50 (4H, m), 3.41 (3H, s), 2.92-2.90 (4H, m), 2.46 (4H, br s), 2.32-2.29 (1H, m), 2.07-1.97 (4H, m), 1.81-1.70 (2H, m), 1.52-1.46 (5H, m), 1.18-1.12 (2H, m). | | |
| 391 | 1H-NMR (CDC13) δ: 9.06 (1H, s), 8.41 (1H, d, J = 8.8 Hz), 8.23 (1H, d, J = 2.0 Hz), 8.10 (1H, s), 7.71 (1H, dd, J = 8.5, 2.2 Hz), 6.99 (1H, s), 5.44-5.37 (1H, m), 4.36 (1H, q, J = 6.3 Hz), 3.48 (2H, s), 3.41 (3H, s), 3.17 (3H, s), 2.90 (4H, t, J = 4.9 Hz), 2.44 (4H, s), 1.48 (3H, t, J = 9.8 Hz), 1.30 (6H, t, J = 7.1 Hz). | | |
| 392 | 1H-NMR (CDC13) δ: 9.04 (1H, s), 8.36 (1H, d, J = 8.3 Hz), 8.27-8.24 (2H, m), 7.73 (1H, dd, J = 8.3, 2.2 Hz), 6.84 (1H, s), 6.43 (1H, t, J = 5.9 Hz), 4.34 (1H, q, J = 6.5 Hz), 3.51-3.47 (4H, m), 3.41 (3H, s), 2.93-2.89 (4H, m), 2.45 (4H, br s), 2.07-1.98 (1H, m), 1.50 (3H, d, J = 6.3 Hz), 1. 07 (6H, d, J = 6.3 Hz). | | |
| 393 | 1H-NMR (CDC13) δ: 9.03 (1H, s), 8.43 (1H, d, J = 8.8 Hz), 8.32 (1H, s), 8.22 (1H, d, J = 2.4 Hz), 7.92 (1H, dd, J = 8.5, 2.2 Hz), 6.87 (1H, s), 6.65 (1H, t, J = 5.1 Hz), 4.75 (1H, s), 4.33 (1H, q, J = 6.3 Hz), 3.82-3.77 (2H, m), 3.63-3.58 (2H, m), 3.57-3.52 (2H, m), 3.49 (2H, s), 3.40-3.35 (5H, m), 2.91-2.89 (4H, m), 2.46 (4H, br s), 1.51 (3H, d, J = 6.8 Hz) . | | |

**[Table 5-73]**

| Compound No. | NMR | LC/MS (M+H) ⁺ | Exact Mass |
|---|---|---|---|
| 394 | 1H-NMR (CDC13) δ: 9.03 (1H, s), 8.42 (1H, d, J = 8.3 Hz), 8.26 (1H, d, J = 2.0 Hz), 8.21 (1H, s), 7.73 (1H, dd, J = 8.5, 2.0 Hz), 6.84 (1H, s), 6.74 (1H, t, J = 5.4 Hz), 4.32 (1H, q, J = 6.3 Hz), 3.70-3.68 (2H, m), 3.48 (2H, s), 3.40 (3H, s), 3.34 (3H, s), 2.92-2.87 (4H, m), 2.44 (4H, br s), 1.50 (3H, d, J = 6.8 Hz), 1.30 (6H, d, J = 2.4 Hz). | | |
| 395 | 1H-NMR (CDC13) δ: 9.04 (1H, s), 8.33 (1H, d, J = 8.8 Hz), 8.26 (2H, d, J = 1.5 Hz), 7.74 (1H, dd, J = 8.5, 2.2 Hz), 6.86 (1H, s), 6.18 (1H, d, J = 7.3 Hz), 4.34 (1H, q, J = 6.3 Hz), 4.15-4.07 (1H, m), 3.49 (2H, s), 3.42 (3H, s), 3.41 (3H, s), 3.31-3.24 (1H, m), 2.93-2.88 (4H, m), 2.45 (4H, br s), 2.32-2.26 (2H, m), 2.16-2.10 (2H, m), 1.54-1.33 (8H, m). | | |
| 397 | 1H-NMR (CDC13) δ: 9.05 (1H, s), 8.60 (1H, s), 8.45 (1H, d, J = 8.5 Hz), 8.27 (1H, d, J = 2.0 Hz), 7.75 (1H, dd, J = 8.5, 2.0 Hz), 6.89-6.82 (2H, m), 4.35-4.29 (2H, m), 4.02 (1H, br s), 3.48 (3H, s), 3.41 (3H, d, J = 7.3 Hz), 2.91-2.87 (4H, m), 2.45 (4H, br s), 2.33-2.28 (1H, m), 1.93-1.85 (3H, m), 1.65-1.47 (7H, m). | | |
| 398 | 1H-NMR (CDC13) δ: 9.03 (1H, s), 8.40 (1H, d, J = 8.3 Hz), 8.26 (2H, d, J = 1.5 Hz), 7.72 (1H, dd, J = 8.5, 2.2 Hz), 6.85 (1H, s), 6.79 (1H, s), 4.33 (1H, q, J = 6.3 Hz), 4.15-4.08 (1H, m), 3.97-3.88 (1H, m), 3.69-3.61 (1H, m), 3.55 (1H, td, J = 11.5, 5.7 Hz), 3.49-3.41 (3H, m), 3.40 (3H, d, J = 1.5 Hz), 2.90-2.88 (4H, m), 2.44 (4H, br s), 1.93-1.87 (1H, br m), 1.73-1.43 (8H, m). | | |

**[Table 5-74]**

| Compounc No. | NMR | LC/MS (M+H) ⁺ | Exact Mass |
|---|---|---|---|
| 399 | 1H-NMR (CDC13) δ: 9.06 (1H, d, J = 5.9 Hz), 8.40 (1H, s), 8.35 (1H, d, J = 8.8 Hz), 8.28 (1H, d, J = 2.0 Hz), 7.74 (1H, dd, J = 8.8, 2.0 Hz), 6.86 (1H, s), 6.26 (1H, d, J = 7.8 Hz), 4.36-4.21 (2H, m), 3.50 (2H, s), 3.41 (3H, s), 3.19-3.15 (2H, m), 2.92-2.83 (6H, m), 2.45 (4H, br s), 2.22-2.16 (2H, m), 1.60-1.49 (5H, m). | | |
| 400 | 1H-NMR (CDC13) δ: 9.12 (1H, d, J = 6.1 Hz), 8.91 (1H, s), 8.34 (2H, t, J = 6.1 Hz), 7.75 (1H, dd, J = 8.5, 2.2 Hz), 6.87 (1H, s), 6.39 (1H, t, J = 6.1 Hz), 4.34 (1H, q, J = 6.5 Hz), 4.03-3.99 (1H, m), 3.91-3.86 (1H, m), 3.64-3.33 (9H, m), 2.91 (4H, t, J = 4.6 Hz), 2.46 (4H, s), 2.00-1.95 (1H, m), 1.74-1.61 (2H, m), 1.51-1.37 (5H, m). | | |
| 401 | 1H-NMR (CDC13) δ: 9.23 (1H, s), 8.89 (1H, s), 8.80 (1H, s), 8.37 (1H, s), 8.36 (1H, d, J = 8.8 Hz), 8.08 (2H, d, J = 8.8 Hz), 7.84 (1H, dd, J = 8.8, 2.2 Hz), 7.69 (2H, d, J = 8.8 Hz), 7.22 (1H, s), 4.49 (1H, q, J = 6.3 Hz), 3.53 (2H, s), 3.45 (3H, s), 2.94-2.90 (4H, m), 2.48 (4H, br s), 1.59 (3H, d, J = 6.3 Hz). | | |
| 402 | 1H-NMR (CDC13) δ: 9.04 (1H, s), 8.33 (1H, d, J = 8.3 Hz), 8.25 (1H, d, J = 2.0 Hz), 8.21 (1H, s), 7.78 (1H, dd, J = 8.3, 2.0 Hz), 6.85 (1H, s), 6.48 (1H, t, J = 6.1 Hz), 4.33 (1H, q, J = 6.3 Hz), 3.69-3.45 (5H, m), 3.40 (3H, d, J = 1.6 Hz), 2.92-2.88 (4H, m), 2.46 (4H, br s), 2.19-2.13 (1H, m), 2.02-1.97 (1H, m), 1.88-1.80 (3H, m), 1.50 (3H, d, J = 6.3 Hz), 1.39-1.00 (5H, m). | | |

**[Table 5-75]**

| Compound No. | NMR | LC/MS (M+H) ⁺ | Exact Mass |
|---|---|---|---|
| 403 | 1H-NMR (CDC13) δ: 9.12 (1H, s), 9.09 (1H, s), 8.28 (1H, d, J = 8.8 Hz), 8.25 (1H, d, J = 1.5 Hz), 7.78 (1H, dd, J = 8.8, 1.5 Hz), 7.00 (1H, d, J = 20.8 Hz), 6.91 (1H, d, J = 4.4 Hz), 6.57 (1H, t, J = 5.4 Hz), 4.35 (1H, q, J = 6.3 Hz), 4.14-4.05 (1H, m), 3.85-3.67 (2H, m), 3.43 (2H, s), 3.41 (3H, s), 2.91-0.00 (4H, m), 2.44-2.27 (7H, m), 2.03-1.91 (1H, m), 1.52 (3H, d, J = 6.3 Hz). | | |
| 404 | 1H-NMR (CDC13) δ: 9.10 (1H, d, J = 3.9 Hz), 8.79 (1H, s), 8.39 (1H, dd, J = 20.7, 8.5 Hz), 8.32 (1H, s), 7.79-7.73 (1H, m), 6.86-6.86 (1H, m), 6.47-6.29 (1H, m), 4.49-4.30 (2H, m), 4.04-3.88 (3H, m), 3.83-3.74 (1H, m), 3.50 (2H, br s), 3.41 (3H, t, J = 2.7 Hz), 2.94-2.88 (4H, m), 2.62-2.44 (5H, m), 2.19-2.03 (1H, m), 1.95-1.78 (1H, m), 1.50 (3H, dd, J = 6.6, 3.7 Hz), 1.37-1.30 (3H, m). | | |
| 405 | 1H-NMR (CDC13) δ: 9.21-8.98 (2H, m), 8.48-8.30 (2H, m), 7.80-7.73 (1H, m), 7.14 (1H, d, J = 12.7 Hz), 6.96-6.87 (1H, m), 4.41-4.23 (3H, m), 4.00-3.97 (1H, m), 3.79-3.61 (1H, m), 3.51-3.20 (6H, m), 2.90 (4H, br s), 2.46 (4H, br s), 2.18-1.74 (8H, m), 1.53-1.48 (3H, m). | | |
| 406 | 1H-NMR (CDC13) δ: 9.06 (1H, s), 8.30-8.24 (3H, m), 7.79-7.77 (1H, m), 7.28 (1H, s), 6.93-6.91 (1H, m), 6.49-6.47 (1H, m), 5.61 (1H, br s), 4.36-4.30 (1H, m), 3.84-3.71 (2H, m), 3.59 (1H, t, J = 8.8 Hz), 3.51-3.48 (2H, m), 3.42-3.40 (3H, m), 3.34-3.31 (1H, m), 3.05-2.90 (5H, m), 2.59-2.44 (5H, m), 2.32-2.26 (1H, m), 1.52-1.45 (3H, m). | | |
| 407 | | 492.4 | 491.31 |

**[Table 5-76]**

| Compound No. | NMR | LC/MS (M+H) ⁺ | Exact Mass |
|---|---|---|---|
| 408 | 1H-NMR (CDC13) δ: 9.07 (1H, s), 8.58 (1H, s), 8.41 (1H, d, J = 8.8 Hz), 8.29 (1H, d, J = 1.5 Hz), 7.75 (1H, dd, J = 8.8, 1.5 Hz), 6.86 (1H, s), 6.70 (1H, t, J = 5.6 Hz), 4.33 (1H, q, J = 6.5 Hz), 4.25-4.21 (1H, m), 4.02-3.97 (1H, m), 3.93-3.82 (2H, m), 3.72-3.62 (1H, m), 3.50 (2H, s), 3.40 (3H, s), 2.94-2.90 (4H, m), 2.46 (4H, br s), 2.10-1.92 (4H, m), 1.77-1.67 (1H, m), 1.50 (3H, d, J = 6.3 Hz). | | |
| 409 | 1H-NMR (CDC13) δ: 9.11 (1H, s), 8.94-8.91 (1H, m), 8.42-8.40 (1H, m), 8.33 (1H, s), 7.76-7.72 (1H, m), 6.85 (1H, d, J = 5.9 Hz), 6.62-6.59 (1H, m), 4.54-4.45 (1H, m), 4.33 (1H, q, J = 6.3 Hz), 4.14-3.96 (2H, m), 3.91-3.81 (1H, m), 3.49 (2H, s), 3.41-3.41 (3H, m), 2.90 (4H, d, J = 4.9 Hz), 2.46 (4H, s), 2.05-1.71 (5H, m), 1.50 (3H, d, J = 6.8 Hz), 1.41-1.31 (3H, m). | | |
| 410 | 1H-NMR (CDC13) δ: 9.12 (1H, s), 8.94 (1H, s), 8.35 (2H, d, J = 8.8 Hz), 7.76 (1H, dd, J = 8.5, 2.9 Hz), 6.89 (1H, d, J = 2.9 Hz), 6.10 (1H, d, J = 7.3 Hz), 4.56-4.46 (1H, m), 4.34 (1H, q, J = 6.3 Hz), 3.89-3.86 (2H, m), 3.51 (2H, s), 3.43 (3H, s), 2.94-2.90 (4H, m), 2.47 (4H, br s), 2.22-2.10 (3H, m), 1.57-1.33 (11H, m). | | |
| 411 | 1H-NMR (CDC13) δ: 9.03 (1H, s), 8.39 (1H, d, J = 8.3 Hz), 8.25 (1H, d, J = 2.0 Hz), 8.19 (1H, s), 7.75 (1H, dd, J = 8.3, 2.0 Hz), 6.86 (1H, s), 6.69 (1H, t, J = 5.6 Hz), 4.33 (1H, q, J = 6.3 Hz), 4.26-4.22 (1H, m), 4.00-3.98 (1H, m), 3.90-3.82 (2H, m), 3.72-3.62 (1H, m), 3.49 (2H, s), 3.40 (3H, s), 2.91-2.88 (4H, m), 2.45 (4H, br s), 2.06-2.01 (1H, m), 1.97-1.93 (2H, m), 1.77-1.68 (1H, m), 1.50 (3H, d, J = 6.3 Hz). | | |

**[Table 5-77]**

| Compound No. | NMR | LC/MS (M+H) ⁺ | Exact Mass |
|---|---|---|---|
| 412 | 1H-NMR (CDC13) δ: 9.18 (1H, s), 8.65 (1H, s), 8.51-8.48 (2H, m), 8.36-8.33 (2H, m), 8.05 (1H, dd, J = 7.8, 1.5 Hz), 7.84 (1H, dd, J = 8.3, 2.0 Hz), 7.49 (1H, t, J = 7.8 Hz), 7.35 (1H, d, J = 7.8 Hz), 7.19 (1H, s), 4.49 (1H, q, J = 6.3 Hz), 3.53 (2H, s), 3.47 (3H, s), 2.93-2.90 (4H, m), 2.47 (4H, br s), 1.59 (3H, d, J = 6.3 Hz). | | |
| 413 | 1H-NMR (CDC13) δ: 9.07 (1H, s), 8.50 (1H, br s), 8.31-8.29 (2H, m), 7.72 (1H, d, J = 8.8 Hz), 6.88 (1H, s), 6.71 (1H, s), 4.31 (1H, q, J = 6.5 Hz), 3.83 (2H, s), 3.49 (2H, s), 3.39 (3H, s), 2.96-2.88 (4H, m), 2.47 (4H, br s), 2.10 (3H, t, J = 6.6 Hz), 1.96-1.93 (4H, m), 1.49 (3H, d, J = 6.5 Hz), 1.24-1.16 (1H, m). | | |
| 414 | 1H-NMR (CDC13) δ: 9.11 (1H, s), 8.93 (1H, br s), 8.44 (1H, d, J = 8.8 Hz), 8.32 (1H, d, J = 2.0 Hz), 7.90 (1H, dd, J = 8.8, 2.0 Hz), 6.89 (1H, s), 6.57 (1H, t, J = 5.4 Hz), 4.34 (1H, q, J = 6.3 Hz), 3.86-3.81 (2H, m), 3.71-3.67 (2H, m), 3.54-3.47 (4H, m), 3.40 (3H, s), 2.91-2.87 (4H, m), 2.47-2.36 (6H, m), 2.05-1.98 (2H, m), 1.51 (3H, d, J = 6.3 Hz). | | |
| 416 | 1H-NMR (CDC13) δ: 9.09 (1H, s), 8.64 (1H, s), 8.29 (2H, d, J = 8.8 Hz), 7.73 (1H, d, J = 9.8 Hz), 7.62 (1H, d, J = 9.2 Hz), 7.46 (1H, s), 6.92 (1H, s), 6.58-6.56 (2H, m), 4.63-4.61 (2H, m), 4.33 (1H, q, J = 6.3 Hz), 3.48 (2H, s), 3.39 (3H, s), 2.90-2.87 (4H, m), 2.44 (4H, br s), 1.48 (3H, d, J = 5.9 Hz), 1.26 (1H, s). | | |

**[Table 5-78]**

| Compound No. | NMR | LC/MS (M+H) ⁺ | Exact Mass |
|---|---|---|---|
| 417 | 1H-NMR (CDC13) δ: 9.03 (1H, s), 8.34 (1H, d, J = 8.8 Hz), 8.28-8.24 (2H, m), 7.74 (1H, dd, J = 8.5, 2.2 Hz), 6.84 (1H, s), 6.29 (1H, d, J = 6.8 Hz), 4.58-4.48 (1H, m), 4.34 (1H, q, J = 6.5 Hz), 3.49 (2H, s), 3.41 (3H, s), 2.93-2.87 (4H, m), 2.49-2.40 (4H, m), 2.24-2.13 (2H, m), 1.86-1.55 (6H, m), 1.51 (3H, d, J = 6.3 Hz) . | | |
| 418 | 1H-NMR (CDC13) δ: 9.05 (1H, s), 8.38 (1H, d, J = 8.8 Hz), 8.34 (1H, s), 8.28 (1H, d, J = 2.0 Hz), 7.70 (1H, dd, J = 8.5, 2.2 Hz), 6.84 (1H, s), 6.51 (1H, t, J = 6.1 Hz), 4.33 (1H, q, J = 6.3 Hz), 3.56-3.42 (4H, m), 3.40 (3H, s), 2.93-2.87 (4H, m), 2.49-2.39 (4H, br m), 1.50 (3H, d, J = 6.3 Hz), 1.07 (9H, s). | | |
| 419 | 1H-NMR (CDC13) δ: 9.09 (1H, s), 8.41 (1H, br s), 8.32-8.25 (2H, m), 7.76 (1H, dd, J = 8.3, 2.4 Hz), 6.98-6.93 (1H, m), 6.46-6.41 (1H, m), 5.88 (1H, br s), 5.03-4.96 (1H, m), 4.37-4.29 (1H, m), 4.01-3.94 (1H, m), 3.51-3.37 (6H, m), 2.98-2.84 (5H, m), 2.51-2.38 (5H, m), 1.52-1.47 (3H, m). | | |
| 420 | 1H-NMR (CDC13) δ: 9.05 (1H, s), 8.42 (1H, br s), 8.36-8.27 (2H, m), 7.69 (1H, dd, J = 8.8, 2.0 Hz), 6.86 (1H, s), 6.40 (1H, s), 4.32 (1H, q, J = 6.3 Hz), 3.85-3.77 (4H, m), 3.49 (2H, s), 3.40 (3H, s), 2.94-2.87 (4H, m), 2.48-2.33 (6H, m), 1.94-1.87 (2H, m), 1.66 (3H, s), 1.48 (3H, d, J = 6.3 Hz). | | |
| 421 | 1H-NMR (CDC13) δ: 9.11-9.07 (1.0H, m), 8.53 (1.0H, br s), 8.30-8.25 (2.0H, m), 7.80-7.74 (1.0H, m), 6.95-6.90 (1.OH, m), 6.67-6.43 (1.6H, m), 6.00 (0.4H, br s), 4.65 (0.4H, br s), 4.38-4.31 (1.0H, m), 4.07 (0.6H, br s), 3.85-3.65 (1.6H, m), 3.49-3.39 (5.4H, m), 2.93-2.87 (4.0H, m), 2.64-1.88 (8.0H, m), 1.54-1.48 (3.0H, m). | | |

**[Table 5-79]**

| Compound No. | NMR | LC/MS (M+H)⁺ | Exact Mass |
|---|---|---|---|
| 422 | 1H-NMR (CDCl3) δ: 9.08 (1H, s), 8.43 (1H, s), 8.32-8.26 (2H, m), 7.75 (1H, dd, J = 8.3, 2.0 Hz), 6.93 (1H, s), 6.29 (1H, d, J = 6.8 Hz), 6.08 (1H, s), 4.63 (1H, br s), 4.33 (1H, q, J = 6.2 Hz), 3.54-3.45 (4H, m), 3.40 (3H, s), 3.04-2.87 (5H, m), 2.57-2.30 (6H, m), 2.08-1.97 (1H, m), 1.50 (3H, d, J = 6.3 Hz). | | |
| 423 | 1H-NMR (CDCl3) δ: 9.04 (1H, s), 8.38-8.33 (2H, m), 8.27 (1H, d, J = 2.0 Hz), 7.73 (1H, dd, J = 8.5, 2.2 Hz), 6.83 (1H, s), 6.18 (1H, d, J = 6.8 Hz), 4.36-4.24 (2H, m), 3.49 (2H, s), 3.41 (3H, s), 2.94-2.88 (4H, m), 2.45 (4H, br s), 1.78-1.66 (2H, m), 1.53-1.48 (3H, m), 1.35-1.29 (3H, m), 1.07-1.00 (3H, m). | | |
| 424 | 1H-NMR (DMSO-D6) δ: 10.46 (1H, s), 9.32 (1H, s), 8.26-8.17 (2H, m), 7.74 (1H, d, J = 7.8 Hz), 7.20 (1H, s), 6.95-6.60 (2H, m), 4.59 (1H, s), 4.09 (1H, s), 3.68 (1H, s), 3.43 (4H, s), 3.15 (1H, s), 2.72 (4H, s), 2.32 (4H, s), 1.90-1.50 (9H, m). | | |
| 425 | 1H-NMR (DMSO-D6) δ: 10.61 (1H, s), 9.28 (1H, d, J = 5.9 Hz), 8.15 (1H, dd, J = 9.8, 4.9 Hz), 7.41 (1H, d, J = 9.8 Hz), 7.18 (1H, d, J = 5.9 Hz), 6.92-6.63 (2H, m), 4.70-4.53 (1H, m), 4.08 (1H, br s), 3.80-3.45 (7H, m), 3.16 (1H, d, J = 3.9 Hz), 2.96 (2H, br s), 2.85-2.75 (1H, m), 2.64-2.55 (1H, m), 1.95-1.45 (11H, m). | | |
| 426 | 1H-NMR (DMSO-D6) δ: 9.16 (1H, s), 8.17 (1H, s), 8.06 (1H, s), 7.94 (1H, s), 7.44 (1H, s), 7.06 (1H, s), 6.69 (1H, s), 6.56 (1H, s), 4.64 (1H, s), 4.32 (2H, s), 4.00 (1H, s), 3.67 (1H, s), 2.88 (2H, s), 2.78-2.60 (10H, m), 1.85-1.20 (12H, m). | | |

**[Table 5-80]**

| Compound No. | NMR | LC/MS (M+H)⁺ | Exact Mass |
|---|---|---|---|
| 427 | 1H-NMR (CDCl3) δ: 9.03 (1H, s), 8.40 (1H, dd, J = 8.5, 3.7 Hz), 8.34 (1H, br s), 8.25 (1H, s), 7.74 (1H, d, J = 8.5 Hz), 7.06 (1H, d, J = 8.5 Hz), 6.85 (1H, s), 4.62-4.56 (1H, m), 4.50 (1H, br s), 4.36-4.32 (1H, m), 3.48 (2H, s), 3.41 (3H, s), 2.92-2.87 (4H, m), 2.62 (1H, s), 2.45 (4H, br s), 2.28-2.21 (2H, m), 2.12-2.05 (1H, m), 1.99-1.85 (4H, m), 1.50 (3H, d, J = 6.3 Hz). | | |
| 428 | 1H-NMR (CDCl3) δ: 9.08 (1H, s), 8.55 (1H, s), 8.35 (1H, d, J = 8.3 Hz), 8.30 (1H, d, J = 2.4 Hz), 7.75 (1H, dd, J = 8.3, 2.4 Hz), 6.87 (1H, s), 6.28 (1H, t, J = 5.6 Hz), 4.33 (1H, q, J = 6.5 Hz), 4.01-3.97 (2H, m), 3.73-3.68 (2H, m), 3.50 (2H, s), 3.43-3.37 (5H, m), 2.93-2.88 (4H, m), 2.46 (4H, br s), 1.77-1.69 (4H, m), 1.50 (3H, d, J = 6.3 Hz), 1.43 (3H, dd, J = 19.0, 5.9 Hz). | | |
| 429 | 1H-NMR (CDCl3) δ: 9.05 (1H, s), 8.45-8.43 (2H, m), 8.27 (1H, d, J = 2.0 Hz), 7.75 (1H, dd, J = 8.5, 2.0 Hz), 6.89 (1H, br s), 6.84 (1H, s), 4.34 (1H, q, J = 6.3 Hz), 4.08-4.02 (1H, m), 3.96-3.66 (4H, m), 3.49 (2H, s), 3.41 (3H, s), 2.91-2.87 (4H, m), 2.45 (4H, br s), 2.09-2.03 (2H, m), 1.96-1.86 (3H, m), 1.66-1.55 (1H, m), 1.51 (3H, d, J = 6.3 Hz). | | |
| 430 | 1H-NMR (CDCl3) δ: 9.05 (1H, s), 8.57 (1H, s), 8.43 (1H, d, J = 8.8 Hz), 8.29 (1H, d, J = 2.0 Hz), 7.76 (1H, dd, J = 8.8, 2.0 Hz), 6.86-6.83 (2H, m), 4.34 (1H, q, J = 6.2 Hz), 4.02-3.97 (1H, m), 3.87-3.77 (1H, m), 3.70-3.62 (1H, m), 3.49-3.43 (4H, m), 3.41 (3H, s), 2.91-2.87 (4H, m), 2.45 (4H, br s), 1.94-1.82 (3H, m), 1.67-1.38 (8H, m). | | |

**[Table 5-81]**

| Compound No. | NMR | LC/MS (M+H)⁺ | Exact Mass |
|---|---|---|---|
| 431 | 1H-NMR (CDCl3) δ: 9.10 (1H, s), 8.72 (1H, s), 8.35 (1H, d, J = 8.8 Hz), 8.31 (1H, d, J = 2.4 Hz), 7.77 (1H, dd, J = 8.8, 2.4 Hz), 6.87 (1H, s), 6.29 (1H, t, J = 5.6 Hz), 4.34 (1H, q, J = 6.3 Hz), 3.98-3.89 (2H, m), 3.73-3.64 (2H, m), 3.50 (2H, s), 3.44-3.38 (4H, m), 3.19 (1H, t, J = 10.5 Hz), 2.92-2.89 (4H, m), 2.46 (4H, br s), 2.01 (1H, t, J = 6.6 Hz), 1.69-1.55 (5H, m), 1.51 (3H, d, J = 6.3 Hz), 1.32-1.22 (1H, m). | | |
| 432 | 1H-NMR (CDCl3) δ: 9.10 (1H, s), 8.73 (1H, s), 8.42 (1H, d, J = 8.3 Hz), 8.32 (1H, d, J = 2.4 Hz), 7.76 (1H, dd, J = 8.3, 2.4 Hz), 6.87 (1H, s), 6.67 (1H, t, J = 7.8 Hz), 4.35-4.30 (2H, m), 4.11-4.06 (1H, m), 3.70-3.64 (1H, m), 3.49 (2H, s), 3.40 (3H, s), 2.93-2.88 (4H, m), 2.45 (4H, br s), 2.06-2.00 (1H, m), 1.94-1.75 (2H, m), 1.62-1.54 (1H, m), 1.50-1.46 (3H, m), 1.33 (6H, s). | | |
| 433 | 1H-NMR (CDCl3) δ: 9.09 (1H, s), 8.50 (1H, s), 8.32-8.29 (2H, m), 7.72 (1H, dd, J = 8.5, 2.7 Hz), 6.92 (1H, s), 6.81 (1H, s), 4.33-4.28 (1H, m), 3.92-3.70 (6H, m), 3.49 (2H, s), 3.39 (3H, s), 2.92-2.89 (4H, m), 2.46 (4H, br s), 2.17-2.02 (5H, m), 1.51-1.47 (3H, m). | | |
| 434 | 1H-NMR (CDCl3) δ: 9.14 (1H, s), 9.14 (1H, s), 8.38-8.35 (2H, m), 7.71 (1H, dd, J = 8.8, 2.0 Hz), 6.89 (1H, s), 6.82 (1H, s), 4.32 (1H, q, J = 6.3 Hz), 3.80 (2H, s), 3.51 (2H, s), 3.40 (3H, s), 2.93-2.88 (4H, m), 2.46 (4H, br s), 2.16-2.11 (2H, m), 1.89 (2H, br s), 1.67-1.41 (9H, m). | | |
| 435 | | 535.4 | 534.31 |

**[Table 5-82]**

| Compound No. | NMR | LC/MS (M+H)⁺ | Exact Mass |
|---|---|---|---|
| 436 | 1H-NMR (DMSO-D6) δ: 8.93 (1H, s), 8.08-7.93 (2H, m), 7.47 (1H, d, J = 8.3 Hz), 6.68-6.62 (2H, m), 4.19-4.02 (2H, m), 3.22 (3H, s), 3.16 (2H, s), 2.68-2.62 (3H, m), 2.29-2.20 (4H, br m), 1.95-1.77 (5H, m), 1.59-1.50 (2H, br m), 1.37-1.24 (5H, m), 1.03 (3H, s). | | |
| 437 | 1H-NMR (DMSO-D6) δ: 8.79-8.66 (1.0H, m), 7.98-7.87 (1.0H, m), 7.77-7.60 (0.5H, m), 7.52-7.20 (1.5H, m), 6.60-6.46 (1.0H, m), 6.27-6.01 (1.0H, m), 4.15-4.07 (1.0H, m), 3.83-3.57 (1.0H, m), 3.28-3.14 (5.0H, m), 2.64 (2.0H, br s), 2.37-2.02 (6.0H, m), 1.92-1.77 (2.0H, br m), 1.52-1.19 (5.0H, m), 1.14-0.85 (5.0H, m). | 535.4 | 534.31 |
| 438 | 1H-NMR (CDCl3) δ: 9.11 (1H, s), 8.57 (1H, s), 8.39 (1H, d, J = 8.8 Hz), 8.33 (1H, d, J = 2.4 Hz), 7.75 (1H, dd, J = 9.0, 2.7 Hz), 7.10 (1H, s), 6.54 (1H, t, J = 56.1 Hz), 6.27 (1H, d, J = 7.8 Hz), 4.43 (1H, td, J = 13.4, 6.5 Hz), 3.77 (2H, t, J = 5.4 Hz), 3.29 (2H, t, J = 5.4 Hz), 1.51 (6H, s), 1.36 (6H, d, J = 6.8 Hz). | | |
| 439 | 1H-NMR (CDCl3) δ: 9.13 (1H, s), 8.61 (1H, s), 8.37 (1H, d, J = 8.8 Hz), 8.34 (1H, d, J = 2.4 Hz), 7.75 (1H, dd, J = 9.0, 2.7 Hz), 7.26 (1H, s), 7.13 (1H, s), 6.54 (1H, t, J = 55.9 Hz), 6.36 (1H, d, J = 7.8 Hz), 4.36 (1H, tt, J = 14.1, 5.2 Hz), 4.05 (2H, td, J = 7.6, 4.1 Hz), 3.77 (2H, t, J = 5.4 Hz), 3.64 (2H, td, J = 11.3, 2.1 Hz), 3.30 (2H, t, J = 5.4 Hz), 2.17 (2H, dd, J = 12.9, 2.7 Hz), 1.71 (2H, dd, J = 10.7, 4.4 Hz), 1.52 (6H, s). | | |

**[Table 5-83]**

| Compound No. | NMR | LC/MS (M+H)⁺ | Exact Mass |
|---|---|---|---|
| 440 | 1H-NMR (CDCl3) δ: 9.03 (1H, s), 8.39 (1H, d, J = 9.3 Hz), 8.28 (2H, d, J = 2.4 Hz), 7.74 (1H, dd, J = 9.0, 2.7 Hz), 7.26 (3H, s), 6.70 (1H, s), 6.24 (1H, d, J = 7.8 Hz), 4.81 (1H, q, J = 6.3 Hz), 4.41 (1H, td, J = 13.3, 6.7 Hz), 3.76 (2H, t, J = 5.6 Hz), 3.29 (2H, t, J = 5.4 Hz), 1.53 (4H, d, J = 6.3 Hz), 1.51 (6H, s), 1.38 (6H, d, J = 6.3 Hz). | | |
| 441 | 1H-NMR (DMSO-D6) δ: 10.22 (1H, s), 9.27 (1H, s), 8.34 (1H, d, J = 8.8 Hz), 8.24 (1H, s), 7.77 (1H, d, J = 7.8 Hz), 7.02 (1H, s), 6.53 (1H, d, J = 7.8 Hz), 5.24 (0H, s), 4.61 (1H, d, J = 6.8 Hz), 4.14 (5H, s), 3.90 (2H, d, J = 10.7 Hz), 3.65 (2H, t, J = 4.9 Hz), 3.50 (4H, d, J = 11.7 Hz), 3.15 (12H, s), 3.06 (2H, d, J = 4.9 Hz), 2.01 (2H, t, J = 13.2 Hz), 1.37 (3H, d, J = 5.9 Hz), 1.30 (6H, s), 1.22 (4H, s). | | |
| 442 | 1H-NMR (DMSO-D6) δ: 10.22 (0H, s), 9.27 (0H, s), 8.34 (OH, d, J = 8.8 Hz), 8.23 (0H, s), 7.77 (0H, d, J = 8.8 Hz), 7.01 (1H, s), 6.52 (0H, d, J = 7.8 Hz), 5.25 (0H, s), 4.61 (1H, d, J = 5.9 Hz), 4.14 (2H, s), 3.90 (1H, d, J = 8.8 Hz), 3.65 (1H, t, J = 4.9 Hz), 3.55-3.44 (2H, m), 3.15 (6H, s), 3.05 (2H, d, J = 4.9 Hz), 2.01 (1H, t, J = 11.7 Hz), 1.37 (2H, d, J = 5.9 Hz), 1.30 (3H, s), 1.22 (2H, s). | | |
| 443 | 1H-NMR (DMSO-D6) δ: 10.47 (1H, s), 9.34 (1H, s), 8.31-8.25 (2H, m), 7.79 (1H, dd, J = 8.8, 2.9 Hz), 7.22 (1H, s), 6.96-6.62 (2H, m), 4.62 (1H, d, J = 4.9 Hz), 3.92 (1H, t, J = 3.9 Hz), 3.65 (2H, t, J = 5.4 Hz), 3.48 (1H, t, J = 6.8 Hz), 3.06 (2H, t, J = 4.9 Hz), 2.05 (2H, d, J = 9.8 Hz), 1.87 (2H, d, J = 9.8 Hz), 1.45 (2H, q, J = 11.4 Hz), 1.33 (9H, d, J = 18.5 Hz). | | |

**[Table 5-84]**

| Compound No. | NMR | LC/MS (M+H)⁺ | Exact Mass |
|---|---|---|---|
| 444 | 1H-NMR (DMSO-D6) δ: 10.24 (0H, s), 9.26 (0H, s), 8.31 (1H, d, J = 8.8 Hz), 8.23 (1H, s), 7.77 (0H, dd, J = 8.8, 2.0 Hz), 6.90 (0H, s), 6.45 (0H, d, J = 7.8 Hz), 4.29-4.22 (1H, m), 3.65 (1H, t, J = 5.4 Hz), 3.27 (2H, s), 3.15 (1H, d, J = 3.9 Hz), 3.05 (1H, d, J = 5.9 Hz), 2.63 (1H, d, J = 22.4 Hz), 1.38 (2H, d, J = 5.9 Hz), 1.29 (6H, d, J = 6.8 Hz), 1.25 (3H, d, J = 26.3 Hz). | | |
| 445 | 1H-NMR (CDCl3) δ: 9.05 (1H, s), 8.54 (1H, s), 8.37 (1H, d, J = 8.3 Hz), 8.30 (1H, s), 7.73 (1H, dd, J = 8.8, 2.0 Hz), 6.83 (1H, s), 6.33 (1H, d, J = 7.8 Hz), 4.37-4.31 (2H, m), 3.55 (2H, s), 3.42 (3H, s), 2.93-2.88 (4H, m), 2.47 (4H, br s), 2.11-2.06 (2H, m), 1.92-1.87 (2H, m), 1.70-1.65 (8H, m), 1.51 (3H, d, J = 6.3 Hz). | | |
| 446 | 1H-NMR (CDCl3) δ: 9.16 (1H, s), 8.64 (1H, s), 8.51 (1H, s), 8.39 (1H, d, J = 8.3 Hz), 8.34 (1H, d, J = 2.0 Hz), 7.95-7.90 (2H, m), 7.82 (1H, dd, J = 8.3, 2.0 Hz), 7.15-7.06 (3H, m), 4.45 (1H, q, J = 6.3 Hz), 3.52 (2H, s), 3.44 (3H, s), 2.93-2.90 (4H, m), 2.47 (4H, br s), 1.57 (3H, d, J = 6.3 Hz). | | |
| 447 | 1H-NMR (CDCl3) δ: 9.14 (1H, s), 8.63 (1H, s), 8.37 (1H, d, J = 8.3 Hz), 8.30 (1H, s), 8.23 (1H, s), 8.13-8.10 (1H, m), 7.85-7.82 (1H, m), 7.45 (1H, d, J = 8.3 Hz), 7.36-7.34 (1H, m), 7.14 (1H, s), 6.80-6.74 (1H, m), 4.48 (1H, q, J = 6.3 Hz), 3.52 (2H, s), 3.46 (3H, s), 2.93-2.89 (4H, m), 2.47 (4H, br s), 1.59 (3H, d, J = 6.3 Hz). | | |
| 448 | | 515.3 | 514.24 |
| 449 | | 515.3 | 514.24 |

**[Table 5-85]**

| Compound No. | NMR | LC/MS (M+H)⁺ | Exact Mass |
|---|---|---|---|
| 450 | 1H-NMR (CDCl3) δ: 9.15 (1H, s), 8.83 (1H, s), 8.43-8.35 (3H, m), 7.88 (2H, d, J = 8.8 Hz), 7.81 (1H, dd, J = 8.8, 2.2 Hz), 7.05 (1H, s), 6.97 (2H, d, J = 8.8 Hz), 4.44 (1H, q, J = 6.3 Hz), 3.86 (3H, s), 3.52 (2H, s), 3.45 (3H, s), 2.93-2.89 (4H, m), 2.47 (4H, br s), 1.57 (3H, d, J = 6.3 Hz). | | |
| 451 | 1H-NMR (CDCl3) δ: 9.13 (1H, s), 8.58 (1H, s), 8.39 (1H, d, J = 8.8 Hz), 8.34-8.30 (2H, m), 8.02 (1H, d, J = 2.0 Hz), 7.82 (1H, dd, J = 8.8, 2.0 Hz), 7.32-7.25 (2H, m), 7.10 (1H, s), 6.65 (1H, d, J = 7.8 Hz), 4.47 (1H, q, J = 6.3 Hz), 3.90 (3H, s), 3.52 (2H, s), 3.44 (3H, s), 2.93-2.89 (4H, m), 2.47 (4H, br s), 1.59 (3H, d, J = 6.3 Hz). | | |
| 452 | 1H-NMR (CDCl3) δ: 9.03 (1H, s), 8.37-8.33 (2H, m), 7.10 (1H, d, J = 10.2 Hz), 6.83 (1H, s), 6.09 (1H, d, J = 7.3 Hz), 4.44-4.24 (4H, m), 3.41 (3H, s), 3.08-3.01 (2H, m), 2.66-2.64 (1H, m), 2.49 (3H, s), 2.04-2.00 (2H, m), 1.58-1.25 (11H, m). | | |
| 453 | 1H-NMR (CDCl3) δ: 8.96 (1H, s), 8.15 (1H, d, J = 8.8 Hz), 7.93-7.87 (2H, m), 7.13 (1H, dd, J = 8.8, 3.2 Hz), 6.80 (1H, s), 6.14 (1H, d, J = 7.8 Hz), 4.44-4.30 (2H, m), 3.63-3.56 (4H, m), 3.40 (3H, s), 3.08 (2H, t, J = 5.1 Hz), 2.87 (2H, t, J = 5.1 Hz), 1. 98-1.92 (2H, m), 1.50 (3H, d, J = 6.8 Hz), 1.35 (6H, t, J = 5.9 Hz). | | |
| 454 | 1H-NMR (CDCl3) δ: 9.06 (1H, d, J = 2.9 Hz), 8.51 (1H, s), 8.38-8.34 (1H, m), 8.29 (1H, s), 7.72 (1H, d, J = 8.3 Hz), 6.84-6.79 (1H, m), 6.55-6.45 (1H, m), 4.40-3.99 (3H, m), 3.49-3.12 (6H, m), 2.92-2.89 (4H, m), 2.46 (4H, br s), 1.96-1.84 (2H, m), 1.59-1.51 (4H, m), 1.25-0.95 (14H, m). | | |

**[Table 5-86]**

| Compound No. | NMR | LC/MS (M+H)⁺ | Exact Mass |
|---|---|---|---|
| 455 | 1H-NMR (CDCl3) δ: 9.05 (1H, s), 8.65 (1H, s), 8.35-8.29 (2H, m), 7.74 (1H, d, J = 6.8 Hz), 6.85 (1H, s), 6.33 (1H, s), 4.33 (1H, q, J = 6.3 Hz), 3.49 (2H, s), 3.42 (3H, s), 2.93-2.89 (4H, m), 2.46-2.17 (6H, m), 1.80-1.65 (13H, m), 1.51 (3H, d, J = 6.3 Hz). | | |
| 456 | 1H-NMR (CDCl3) δ: 9.31 (1H, br s), 9.28 (1H, s), 8.80 (1H, d, J = 8.3 Hz), 8.37 (1H, d, J = 2.0 Hz), 7.88 (1H, dd, J = 8.3, 2.0 Hz), 7.33 (1H, s), 4.52 (1H, q, J = 6.3 Hz), 4.25-4.19 (1H, m), 3.52 (2H, s), 3.42 (3H, s), 2.93-2.89 (4H, m), 2.47 (4H, br s), 1.56-1.52 (9H, m). | | |
| 457 | 1H-NMR (CDCl3) δ: 9.01 (1H, s), 8.29 (1H, d, J = 9.3 Hz), 8.18 (1H, s), 8.07 (1H, d, J = 2.9 Hz), 7.35 (1H, dd, J = 8.8, 2.9 Hz), 6.83 (1H, s), 6.12 (1H, d, J = 7.3 Hz), 4.45-4.29 (2H, m), 3.91 (2H, s), 3.41 (3H, s), 1.50 (3H, d, J = 6.3 Hz), 1.38-1.32 (6H, m), 0.81-0.76 (2H, m), 0.69-0.65 (2H, m). | | |
| 458 | 1H-NMR (CDCl3) δ: 9.04 (1H, s), 8.61 (1H, br s), 8.29 (1H, d, J = 8.8 Hz), 8.17 (1H, d, J = 2.4 Hz), 7.36 (1H, dd, J = 9.3, 2.9 Hz), 6.83 (1H, s), 6.12 (1H, d, J = 7.3 Hz), 4.45-4.29 (2H, m), 3.84 (2H, s), 3.41 (3H, s), 1.51 (3H, d, J = 6.3 Hz), 1.39-1.21 (12H, m). | | |
| 459 | 1H-NMR (CDCl3) δ: 9.02 (1.0H, s), 8.33-8.25 (2.0H, m), 8.13 (1.0H, d, J = 2.4 Hz), 7.42 (1.0H, dd, J = 8.8, 2.9 Hz), 6.83 (1.0H, s), 6.13 (1.0H, d, J = 7.8 Hz), 4.69-4.63 (0.5H, m), 4.57-4.51 (0.5H, m), 4.45-4.31 (3.0H, m), 3.44-3.34 (4.0H, m), 3.12-3.04 (1.0H, m), 2.92-2.84 (1.0H, m), 2.75-2.68 (1.0H, m), 2.20-2.11 (1.0H, m), 1.78-1.68 (1.0H, m), 1.51 (3.0H, d, J = 6.8 Hz), 1.38-1.34 (6.0H, m). | | |

**[Table 5-87]**

| Compound No. | NMR | LC/MS (M+H)⁺ | Exact Mass |
|---|---|---|---|
| 460 | 1H-NMR (CDCl3) δ: 9.03 (1.0H, s), 8.31 (1.0H, d, J = 9.3 Hz), 8.26 (1.0H, br s), 8.14 (1.0H, d, J = 2.9 Hz), 7.43 (1.0H, dd, J = 9.3, 2.9 Hz), 6.84 (1.0H, s), 6.12 (1.0H, d, J = 7.8 Hz), 4.90-4.86 (0.5H, m), 4.78-4.73 (0.5H, m), 4.46-4.24 (3.0H, m), 3.44-3.34 (4.0H, m), 3.22-3.15 (1.0H, m), 2.93-2.67 (2.0H, m), 2.03-1.92 (2.0H, m), 1.51 (3.0H, d, J = 6.8 Hz), 1.38-1.33 (6.0H, m). | | |
| 461 | 1H-NMR (CDCl3) δ: 8.97 (1H, s), 8.15 (1H, d, J = 9.3 Hz), 8.07 (1H, s), 8.03 (1H, d, J = 3.4 Hz), 7.31 (1H, dd, J = 9.0, 3.2 Hz), 6.80 (1H, s), 6.15 (1H, d, J = 7.8 Hz), 4.45-4.29 (2H, m), 3.77 (2H, s), 3.40 (3H, s), 3.08 (3H, s), 1.50 (3H, d, J = 6.8 Hz), 1.38-1.33 (6H, m), 1.01-0.94 (4H, m). | | |
| 462 | 1H-NMR (CDCl3) δ: 9.06 (1H, s), 8.45 (2H, t, J = 8.8 Hz), 8.32 (1H, d, J = 2.9 Hz), 7.75 (1H, dd, J = 9.3, 2.4 Hz), 6.85 (1H, s), 6.13 (1H, d, J = 6.8 Hz), 4.42 (1H, td, J = 13.4, 6.5 Hz), 4.34 (1H, q, J = 6.5 Hz), 3.90 (1H, td, J = 10.5, 4.2 Hz), 3.76 (1H, q, J = 6.8 Hz), 3.64-3.61 (1H, m), 3.41 (3H, s), 3.37-3.23 (2H, m), 1.47 (7H, t, J = 17.1 Hz), 1.35 (7H, t, J = 5.9 Hz). | | |
| 463 | 1H-NMR (CDCl3) δ: 9.06 (1H, s), 8.48-8.37 (2H, m), 8.31 (1H, s), 7.75 (1H, dd, J = 8.8, 2.9 Hz), 6.85 (1H, s), 6.13 (1H, d, J = 7.8 Hz), 4.42 (1H, td, J = 13.7, 6.8 Hz), 4.34 (1H, q, J = 6.5 Hz), 3.93-3.87 (1H, m), 3.76 (1H, q, J = 6.8 Hz), 3.63 (1H, td, J = 7.3, 4.2 Hz), 3.41 (3H, s), 3.36-3.23 (2H, m), 1.51 (6H, d, J = 6.8 Hz), 1.35 (7H, t, J = 5.9 Hz). | | |

**[Table 5-88]**

| Compound No. | NMR | LC/MS (M+H)⁺ | Exact Mass |
|---|---|---|---|
| 464 | 1H-NMR (CDCl3) δ: 9.04 (1H, s), 8.37-8.32 (1H, m), 8.29-8.24 (2H, br m), 7.76-7.70 (1H, m), 6.86 (1H, s), 6.15 (1H, d, J = 7.3 Hz), 4.39-4.31 (1H, m), 4.15-4.03 (1H, br m), 3.79-3.71 (1H, m), 3.52 (2H, s), 3.46-3.39 (4H, br m), 3.05-2.98 (4H, br m), 2.61-2.52 (4H, br m), 2.34-2.26 (2H, m), 2.09-2.02 (2H, m), 1.58-1.46 (5H, m), 1.45-1.31 (2H, m), 1.18 (6H, d, J = 5.9 Hz). | | |
| 465 | 1H-NMR (CDCl3) δ: 9.07 (1H, s), 8.37-8.26 (3H, m), 7.75 (1H, dd, J = 8.5, 2.2 Hz), 6.90 (1H, s), 6.40 (1H, t, J = 6.1 Hz), 4.33 (1H, q, J = 6.5 Hz), 3.80 (2H, t, J = 6.3 Hz), 3.51 (2H, s), 3.40 (3H, s), 2.97-2.91 (4H, m), 2.79-2.38 (9H, m), 1.50 (3H, d, J = 6.3 Hz). | | |
| 466 | 1H-NMR (CDCl3) δ: 8.99 (1H, s), 8.24 (1H, d, J = 9.3 Hz), 8.08-8.00 (2H, m), 7.40 (1H, dd, J = 9.3, 2.9 Hz), 6.82 (1H, s), 6.13 (1H, d, J = 7.3 Hz), 4.83-4.66 (1H, m), 4.45-4.29 (2H, m), 3.87-3.79 (1H, m), 3.59-3.53 (1H, m), 3.41 (3H, s), 3.11-2.87 (3H, m), 1.99-1.85 (2H, m), 1.62-1.47 (5H, m), 1.38-1.32 (6H, m). | | |
| 467 | 1H-NMR (CDCl3) δ: 9.00 (1H, s), 8.26 (1H, d, J = 9.3 Hz), 8.08-8.03 (2H, m), 7.38 (1H, dd, J = 9.0, 3.2 Hz), 6.82 (1H, s), 6.13 (1H, d, J = 7.3 Hz), 4.46-4.28 (3H, m), 3.87-3.79 (1H, m), 3.56-3.49 (1H, m), 3.41 (3H, s), 3.01-2.75 (3H, m), 2.09-2.00 (1H, m), 1.68-1.49 (6H, m), 1.39-1.31 (6H, m). | | |
| 468 | | 493.4 | 492.30 |

**[Table 5-89]**

| Compound No. | NMR | LC/MS (M+H)⁺ | Exact Mass |
|---|---|---|---|
| 469 | 1H-NMR (CDCl3) δ: 8.99 (1H, s), 8.24 (1H, d, J = 8.8 Hz), 8.06-8.06 (2H, m), 7.40 (1H, dd, J = 8.8, 3.2 Hz), 6.82 (1H, s), 6.15 (1H, d, J = 7.8 Hz), 4.91-4.77 (1H, m), 4.41-4.30 (2H, m), 3.55 (3H, s), 3.39-3.31 (2H, m), 3.20-3.14 (2H, m), 2.11-2.07 (4H, m), 1.50 (3H, d, J = 6.3 Hz), 1.35 (6H, d, J = 6.1 Hz). | | |
| 470 | 1H-NMR (CDCl3) δ: 9.00 (1H, s), 8.27 (1H, d, J = 8.8 Hz), 8.19 (1H, s), 8.07 (1H, d, J = 2.9 Hz), 7.41 (1H, dd, J = 8.8, 2.9 Hz), 6.82 (1H, s), 6.14 (1H, d, J = 7.8 Hz), 4.45-4.31 (2H, m), 3.41 (3H, s), 3.34-3.26 (4H, m), 2.21-2.11 (4H, m), 1.50 (3H, d, J = 6.3 Hz), 1.36 (6H, dd, J = 6.6, 5.1 Hz). | | |
| 471 | 1H-NMR (CDCl3) δ: 9.08 (1H, s), 8.95 (1H, br s), 8.54 (1H, d, J = 9.8 Hz), 7.15 (1H, d, J = 9.8 Hz), 6.85 (1H, s), 6.15 (1H, d, J = 7.3 Hz), 4.59 (2H, t, J = 5.4 Hz), 4.37-4.33 (2H, m), 3.41 (3H, s), 2.82 (2H, t, J = 5.4 Hz), 2.39 (6H, s), 1.51 (3H, d, J = 6.3 Hz), 1.34 (3H, d, J = 5.9 Hz), 1.33 (3H, d, J = 5.9 Hz). | | |
| 472 | 1H-NMR (CDCl3) δ: 9.07 (1H, s), 8.58 (1H, s), 8.36 (1H, d, J = 8.8 Hz), 8.29 (1H, d, J = 1.5 Hz), 7.74 (1H, dd, J = 8.8, 1.5 Hz), 6.85 (1H, s), 6.14 (1H, d, J = 7.8 Hz), 4.76-4.63 (1H, m), 4.46-4.31 (2H, m), 3.51 (2H, s), 3.42 (3H, s), 2.64-2.58 (2H, m), 2.46-2.40 (2H, m), 1.98-1.86 (4H, m), 1.51 (3H, d, J = 6.3 Hz), 1.36 (3H, d, J = 6.1 Hz), 1.35 (3H, d, J = 5.1 Hz). | | |

**[Table 5-90]**

| Compound No. | NMR | LC/MS (M+H)⁺ | Exact Mass |
|---|---|---|---|
| 473 | 1H-NMR (CDCl3) δ: 9.03 (1H, s), 8.32-8.30 (2H, m), 8.16 (1H, d, J = 2.9 Hz), 7.44 (1H, dd, J = 8.8, 2.9 Hz), 6.84 (1H, s), 6.12 (1H, d, J = 7.3 Hz), 4.48-4.31 (3H, m), 3.41-3.29 (5H, m), 3.11-2.98 (2H, m), 2.82 (1H, d, J = 14.1 Hz), 2.08-2.04 (2H, m), 1.51 (3H, d, J = 6.3 Hz), 1.36 (6H, t, J = 5.9 Hz). | | |
| 474 | 1H-NMR (CDCl3) δ: 9.10 (1H, s), 8.91 (1H, s), 8.49 (1H, d, J = 9.8 Hz), 7.12 (1H, d, J = 9.8 Hz), 6.73 (1H, s), 6.19 (1H, d, J = 7.3 Hz), 4.81 (1H, d, J = 5.4 Hz), 4.60 (2H, t, J = 5.6 Hz), 4.42-4.32 (1H, m), 4.14 (1H, s), 2.79 (2H, t, J = 5.6 Hz), 2.36 (6H, s), 1.53 (3H, d, J = 6.3 Hz), 1.36 (3H, d, J = 6.3 Hz), 1.35 (3H, d, J = 6.3 Hz). | | |
| 475 | 1H-NMR (CD3OD) δ: 9.07 (1H, s), 8.28-8.21 (2H, m), 7.81 (1H, dd, J = 8.5, 2.2 Hz), 6.84 (1H, s), 4.32 (1H, q, J = 6.3 Hz), 3.53 (2H, s), 3.37 (3H, s), 2.87-2.80 (4H, m), 2.51-2.41 (4H, br m), 2.21-2.14 (6H, m), 1.99-1.92 (6H, m), 1.47 (3H, d, J = 6.3 Hz). | | |
| 476 | 1H-NMR (DMSO-D6) δ: 9.87 (1H, s), 9.12 (1H, s), 8.02 (1H, d, J = 8.3 Hz), 7.69-6.75 (4H, m), 6.38-6.22 (1H, br m), 4.22-3.89 (10H, m), 3.32 (3H, s), 1.41-1.14 (9H, m). | | |
| 477 | 1H-NMR (CDCl3) δ: 8.96 (1H, s), 8.16 (1H, d, J = 8.8 Hz), 7.95 (1H, s), 7.64 (1H, d, J = 2.9 Hz), 6.90 (1H, dd, J = 8.8, 2.9 Hz), 6.80 (1H, s), 6.13 (1H, d, J = 7.8 Hz), 4.44-4.30 (2H, m), 3.69 (2H, d, J = 6.8 Hz), 3.59 (2H, d, J = 6.8 Hz), 3.40 (3H, s), 2.98 (2H, s), 2.81-2.75 (2H, m), 1.83-1.76 (2H, m), 1.59-1.47 (5H, m), 1.38-1.31 (6H, m). | | |

**[Table 5-91]**

| Compound No. | NMR | LC/MS (M+H)⁺ | Exact Mass |
|---|---|---|---|
| 478 | 1H-NMR (CDCl3) δ: 8.98 (1H, s), 8.17 (1H, d, J = 8.8 Hz), 8.03 (1H, s), 7.65 (1H, d, J = 2.9 Hz), 6.92 (1H, dd, J = 8.8, 2.9 Hz), 6.80 (1H, s), 6.12 (1H, d, J = 7.3 Hz), 4.45-4.28 (2H, m), 4.15 (2H, d, J = 7.8 Hz), 3.85 (2H, d, J = 8.3 Hz), 3.54 (2H, t, J = 7.2 Hz), 3.40 (3H, s), 2.62 (2H, t, J = 7.1 Hz), 1.50 (3H, d, J = 6.3 Hz), 1.39-1.31 (6H, m). | | |
| 479 | 1H-NMR (CDCl3) δ: 8.98 (1H, s), 8.18 (1H, d, J = 9.3 Hz), 8.03 (1H, s), 7.66 (1H, d, J = 2.9 Hz), 6.93 (1H, dd, J = 8.8, 2.9 Hz), 6.80 (1H, s), 6.13 (1H, d, J = 7.8 Hz), 4.44-4.29 (2H, m), 3.90-3.82 (4H, m), 3.40 (3H, s), 3.16 (2H, s), 3.01 (2H, t, J = 7.1 Hz), 2.07 (2H, t, J = 7.1 Hz), 1.50 (3H, d, J = 6.3 Hz), 1.38-1.31 (6H, m). | | |
| 480 | 1H-NMR (CDCl3) δ: 8.96 (1H, s), 8.15 (1H, d, J = 8.8 Hz), 7.94 (1H, s), 7.62 (1H, d, J = 2.4 Hz), 6.89 (1H, dd, J = 9.3, 2.9 Hz), 6.80 (1H, s), 6.13 (1H, d, J = 7.3 Hz), 4.45-4.28 (2H, m), 3.88 (4H, s), 3.40 (3H, s), 2.24 (4H, t, J = 7.6 Hz), 1.94-1.85 (2H, m), 1.50 (3H, d, J = 6.3 Hz), 1.39-1.31 (6H, m). | | |
| 481 | 1H-NMR (CDCl3) δ: 9.00 (1H, s), 8.24 (1H, d, J = 8.8 Hz), 8.11 (1H, s), 8.05 (1H, d, J = 2.9 Hz), 7.38 (1H, dd, J = 9.3, 2.9 Hz), 6.82 (1H, s), 6.13 (1H, d, J = 7.3 Hz), 4.50 (4H, s), 4.45-4.29 (2H, m), 3.41 (3H, s), 3.09 (4H, t, J = 5.6 Hz), 2.05 (4H, t, J = 5.4 Hz), 1.50 (3H, d, J = 6.3 Hz), 1.38-1.32 (6H, m). | | |

**[Table 5-92]**

| Compound No. | NMR | LC/MS (M+H)⁺ | Exact Mass |
|---|---|---|---|
| 482 | 1H-NMR (CDCl3) δ: 8.97 (1H, s), 8.17 (1H, d, J = 8.8 Hz), 7.97 (1H, s), 7.64 (1H, d, J = 2.4 Hz), 6.91 (1H, dd, J = 8.8, 2.9 Hz), 6.80 (1H, s), 6.13 (1H, d, J = 7.8 Hz), 4.45-4.28 (2H, m), 3.67 (4H, s), 3.40 (3H, s), 2.84 (4H, t, J = 5.1 Hz), 1.81 (4H, t, J = 5.4 Hz), 1.50 (3H, d, J = 6.8 Hz), 1.38-1.32 (6H, m). | | |
| 483 | 1H-NMR (CDCl3) δ: 9.01 (1H, s), 8.25 (1H, d, J = 8.8 Hz), 8.18 (1H, s), 7.95 (1H, d, J = 2.9 Hz), 7.23 (1H, dd, J = 8.8, 2.9 Hz), 6.82 (1H, s), 6.12 (1H, d, J = 7.3 Hz), 4.61-4.54 (1H, m), 4.45-4.29 (2H, m), 3.72 (2H, s), 3.67 (2H, s), 3.41 (3H, s), 2.79-2.71 (2H, m), 2.36-2.29 (2H, m), 1.50 (3H, d, J = 6.3 Hz), 1.38-1.32 (6H, m). | | |
| 484 | 1H-NMR (CDCl3) δ: 8.96 (1H, s), 8.16 (1H, d, J = 8.8 Hz), 7.96 (1H, s), 7.61 (1H, d, J = 2.0 Hz), 6.89 (1H, dd, J = 8.3, 2.4 Hz), 6.80 (1H, s), 6.13 (1H, d, J = 6.8 Hz), 4.45-4.28 (2H, m), 3.91 (2H, s), 3.84 (2H, s), 3.47-3.37 (4H, m), 2.63-2.54 (2H, m), 1.97-1.88 (2H, m), 1.50 (3H, d, J = 6.8 Hz), 1.37-1.32 (6H, m). | | |
| 485 | 1H-NMR (CDCl3) δ: 8.97 (1H, s), 8.13 (1H, d, J = 9.3 Hz), 7.97 (1H, s), 7.73 (1H, d, J = 2.4 Hz), 6.98 (1H, dd, J = 8.8, 2.9 Hz), 6.80 (1H, s), 6.13 (1H, d, J = 7.3 Hz), 4.44-4.29 (2H, m), 3.83-3.60 (5H, m), 3.40 (3H, s), 2.77-2.69 (2H, m), 2.04-1.95 (2H, m), 1.50 (3H, d, J = 6.3 Hz), 1.38-1.32 (6H, m). | | |

**[Table 5-93]**

| Compound No. | NMR | LC/MS (M+H)⁺ | Exact Mass |
|---|---|---|---|
| 486 | 1H-NMR (CDCl3) δ: 8.97 (1H, s), 8.17 (1H, d, J = 8.8 Hz), 7.99 (1H, s), 7.66 (1H, d, J = 2.9 Hz), 6.93 (1H, dd, J = 9.0, 2.7 Hz), 6.80 (1H, s), 6.13 (1H, d, J = 7.8 Hz), 4.44-4.29 (2H, m), 3.96 (2H, d, J = 6.8 Hz), 3.75 (2H, d, J = 6.8 Hz), 3.40 (3H, s), 3.04 (2H, t, J = 6.8 Hz), 2.10 (2H, t, J = 7.3 Hz), 1.90-1.82 (2H, m), 1.50 (3H, d, J = 6.3 Hz), 1.38-1.32 (6H, m). | | |
| 487 | 1H-NMR (CDCl3) δ: 9.01 (1H, s), 8.30 (1H, d, J = 8.8 Hz), 8.10 (2H, t, J = 2.7 Hz), 7.39 (1H, dd, J = 9.0, 3.2 Hz), 6.83 (1H, s), 6.12 (1H, d, J = 7.8 Hz), 5.05-4.90 (1H, m), 4.45-4.16 (4H, m), 3.41 (3H, s), 2.82-2.64 (2H, m), 2.36 (6H, s), 1.50 (3H, d, J = 6.3 Hz), 1.36 (3H, d, J = 6.3 Hz), 1.35 (3H, d, J = 6.3 Hz). | | |
| 488 | 1H-NMR (CDCl3) δ: 9.04 (1H, s), 8.41 (1H, s), 8.31 (1H, d, J = 9.8 Hz), 6.91 (1H, d, J = 9.8 Hz), 6.83 (1H, s), 6.10 (1H, d, J = 7.8 Hz), 4.44-4.30 (2H, m), 3.93-3.90 (2H, m), 3.73 (2H, t, J = 6.1 Hz), 3.41 (3H, s), 2.76 (2H, t, J = 4.6 Hz), 2.61 (2H, t, J = 5.6 Hz), 2.40 (3H, s), 2.10-2.04 (2H, m), 1.50 (3H, d, J = 6.3 Hz), 1.36 (3H, d, J = 6.3 Hz), 1.35 (3H, d, J = 6.3 Hz). | | |
| 489 | 1H-NMR (CDCl3) δ: 9.07 (1H, s), 8.61 (1H, s), 8.37 (1H, d, J = 9.8 Hz), 7.10 (1H, d, J = 9.8 Hz), 6.84 (1H, s), 6.09 (1H, d, J = 7.3 Hz), 4.37 (4H, tt, J = 22.0, 7.6 Hz), 3.41 (3H, s), 2.97 (2H, t, J = 12.4 Hz), 2.50-2.31 (7H, m), 1.98 (2H, d, J = 11.7 Hz), 1.66-1.55 (2H, m), 1.50 (3H, d, J = 6.3 Hz), 1.36 (3H, d, J = 6.3 Hz), 1.35 (3H, d, J = 6.3 Hz). | | |

**[Table 5-94]**

| Compound No. | NMR | LC/MS (M+H)⁺ | Exact Mass |
|---|---|---|---|
| 490 | 1H-NMR (CDCl3) δ: 8.99 (1H, s), 8.28 (1H, d, J = 9.8 Hz), 8.17 (1H, s), 6.91 (1H, d, J = 9.8 Hz), 6.68 (1H, s), 6.21 (1H, d, J = 7.3 Hz), 4.80 (1H, q, J = 6.3 Hz), 4.43-4.35 (1H, m), 4.12 (1H, br s), 3.91 (2H, t, J = 4.6 Hz), 3.73 (2H, t, J = 6.3 Hz), 2.76 (2H, t, J = 4.6 Hz), 2.61 (2H, t, J = 5.1 Hz), 2.40 (3H, s), 2.10-2.04 (2H, m), 1.53 (3H, d, J = 6.3 Hz), 1.37 (6H, d, J = 6.3 Hz). | | |
| 491 | 1H-NMR (CDCl3) δ: 9.04 (1H, s), 8.49 (1H, br s), 8.33 (1H, d, J = 9.8 Hz), 7.10 (1H, d, J = 9.8 Hz), 6.70 (1H, s), 6.20 (1H, d, J = 6.8 Hz), 4.80 (1H, q, J = 6.5 Hz), 4.40-4.36 (2H, m), 4.13 (1H, br s), 2.96 (2H, t, J = 12.7 Hz), 2.42-2.26 (8H, m), 1.97 (2H, d, J = 12.2 Hz), 1.65-1.55 (2H, m), 1.53 (3H, d, J = 6.3 Hz), 1.37 (6H, d, J = 6.3 Hz). | | |
| 492 | 1H-NMR (CDCl3) δ: 9.13-9.10 (2H, m), 8.45 (1H, d, J = 8.8 Hz), 8.34 (1H, d, J = 2.4 Hz), 7.68 (1H, dd, J = 8.8, 2.4 Hz), 6.85 (1H, s), 6.13 (1H, d, J = 7.3 Hz), 4.46-4.31 (2H, m), 3.91 (2H, d, J = 7.8 Hz), 3.79 (2H, s), 3.42 (3H, s), 3.22-3.19 (2H, m), 2.05-2.00 (2H, br m), 1.51 (3H, d, J = 6.3 Hz), 1.35 (6H, t, J = 5.6 Hz). | | |
| 493 | 1H-NMR (CDCl3) δ: 9.07 (1H, s), 8.64 (1H, s), 8.37-8.32 (2H, m), 7.77 (1H, dd, J = 8.5, 2.2 Hz), 6.84 (1H, s), 6.15 (1H, d, J = 7.8 Hz), 4.45-4.31 (2H, m), 3.66 (2H, s), 3.41 (3H, s), 3.03-2.93 (4H, m), 2.76-2.70 (4H, m), 1.85-1.78 (2H, m), 1.51 (3H, d, J = 6.3 Hz), 1.36 (6H, t, J = 5.6 Hz). | | |

**[Table 5-95]**

| Compound No. | NMR | LC/MS (M+H)⁺ | Exact Mass |
|---|---|---|---|
| 494 | 1H-NMR (CDCl3) δ: 9.10 (1H, s), 8.87 (1H, s), 8.38 (1H, d, J = 8.3 Hz), 8.32 (1H, d, J = 2.0 Hz), 7.75 (1H, dd, J = 8.3, 2.0 Hz), 6.85 (1H, s), 6.15 (1H, d, J = 7.3 Hz), 4.48-4.32 (2H, m), 3.76-3.72 (1H, m), 3.52 (2H, s), 3.42 (3H, s), 2.82-2.77 (2H, m), 2.25-2.20 (2H, m), 1.94-1.89 (2H, m), 1.67-1.57 (2H, m), 1.51 (3H, d, J = 6.3 Hz), 1.36 (6H, t, J = 5.9 Hz). | | |
| 495 | 1H-NMR (CDCl3) δ: 9.02 (1H, s), 8.37 (1H, s), 8.30 (1H, d, J = 9.8 Hz), 6.92 (1H, d, J = 9.8 Hz), 6.82 (1H, d, J = 1.5 Hz), 6.09 (1H, d, J = 7.3 Hz), 4.44-4.30 (2H, m), 3.85-3.78 (4H, m), 3.41 (3H, s), 3.10 (2H, t, J = 5.1 Hz), 2.89 (2H, t, J = 5.9 Hz), 1.98-1.92 (2H, m), 1.50 (3H, d, J = 6.3 Hz), 1.35 (6H, t, J = 5.6 Hz). | | |
| 496 | 1H-NMR (CDCl3) δ: 8.98 (1H, s), 8.28 (1H, d, J = 9.8 Hz), 8.09 (1H, s), 6.93 (1H, d, J = 9.8 Hz), 6.67 (1H, s), 6.22 (1H, d, J = 7.3 Hz), 4.80 (1H, q, J = 6.3 Hz), 4.43-4.35 (1H, m), 3.85-3.80 (4H, m), 3.10 (2H, t, J = 5.4 Hz), 2.89 (2H, t, J = 5.9 Hz), 1.98-1.92 (2H, m), 1.53 (3H, d, J = 6.3 Hz), 1.37 (6H, d, J = 6.3 Hz). | | |
| 497 | 1H-NMR (CDCl3) δ: 9.02 (1H, s), 8.39 (1H, s), 8.33 (1H, d, J = 9.8 Hz), 7.10 (1H, d, J = 10.2 Hz), 6.69 (1H, s), 6.21 (1H, d, J = 7.8 Hz), 4.80 (1H, q, J = 6.3 Hz), 4.44-4.34 (1H, m), 4.29-4.23 (2H, m), 3.08-3.01 (2H, m), 2.69-2.61 (1H, m), 2.49 (3H, s), 2.04 (2H, dd, J = 12.7, 2.4 Hz), 1.53 (3H, d, J = 6.3 Hz), 1.49-1.40 (2H, m), 1.36 (6H, d, J = 6.3 Hz). | | |

**[Table 5-96]**

| Compound No. | NMR | LC/MS (M+H)⁺ | Exact Mass |
|---|---|---|---|
| 500 | 1H-NMR (DMSO-D6) δ: 10.91 (1H, s), 9.32 (1H, s), 9.25-9.16 (1H, m), 8.91 (1H, s), 8.00 (1H, d, J = 2.4 Hz), 7.90 (1H, d, J = 9.3 Hz), 7.80 (1H, dd, J = 9.3, 2.4 Hz), 7.20 (1H, d, J = 51.2 Hz), 7.05-6.80 (3H, m), 4.45 (2H, s), 4.29 (2H, q, J = 6.2 Hz), 3.28 (4H, t, J = 5.9 Hz), 3.20-3.07 (5H, m), 2.08 (5H, dd, J = 18.3, 10.0 Hz), 1.39 (3H, d, J = 6.3 Hz), 1.31 (6H, dd, J = 6.3, 3.9 Hz), 1.24 (2H, dd, J = 12.2, 5.9 Hz), 1.16 (2H, d, J = 6.3 Hz). | | |
| 501 | 1H-NMR (CDCl3) δ: 9.01 (1H, s), 8.23 (2H, d, J = 8.8 Hz), 8.01 (1H, s), 6.81 (1H, s), 6.12 (1H, d, J = 6.8 Hz), 4.39 (1H, d, J = 4.9 Hz), 4.32 (1H, q, J = 6.5 Hz), 3.86 (2H, s), 3.40 (6H, s), 3.16 (3H, d, J = 8.8 Hz), 2.93 (2H, d, J = 30.2 Hz), 2.01 (5H, s), 1.50 (3H, d, J = 5.9 Hz), 1.34 (7H, s). | | |
| 502 | 1H-NMR (CDCl3) δ: 9.13 (1H, br s), 8.98 (1H, d, J = 2.9 Hz), 8.24 (2H, td, J = 12.4, 7.2 Hz), 8.04 (3H, t, J = 12.2 Hz), 7.91 (1H, d, J = 6.8 Hz), 7.21 (1H, d, J = 6.8 Hz), 6.81 (1H, d, J = 4.9 Hz), 6.14 (1H, d, J = 6.8 Hz), 4.45-4.30 (6H, m), 3.75-3.58 (3H, m), 3.38 (5H, d, J = 24.4 Hz), 2.50 (1H, t, J = 8.3 Hz), 2.31-2.23 (1H, m), 2.11 (0H, t, J = 25.4 Hz), 1.78 (2H, dd, J = 41.0, 18.5 Hz), 1.50 (3H, d, J = 6.8 Hz), 1.37-1.26 (7H, m). | | |
| 503 | 1H-NMR (CDCl3) δ: 9.06 (1H, s), 8.84 (1H, s), 8.23 (1H, d, J = 8.8 Hz), 8.02 (1H, d, J = 6.8 Hz), 7.20 (1H, d, J = 6.8 Hz), 6.81 (1H, s), 6.14 (1H, d, J = 6.8 Hz), 4.44-4.15 (4H, m), 3.40 (3H, s), 3.13 (1H, s), 2.36 (4H, s), 2.11 (3H, s), 1.81 (4H, s), 1.68 (2H, s), 1.50 (3H, d, J = 5.9 Hz), 1.35 (7H, t, J = 5.4 Hz). | | |

**[Table 5-97]**

| Compound No. | NMR | LC/MS (M+H)⁺ | Exact Mass |
|---|---|---|---|
| 504 | 1H-NMR (DMSO-D6) δ: 9.23 (1H, s), 8.71 (1H, s), 8.04 (1H, s), 7.94 (0H, s), 7.57 (3H, s), 7.20 (1H, d, J = 51.7 Hz), 6.94 (1H, d, J = 56.6 Hz), 4.68 (1H, s), 4.24 (2H, s), 3.89 (2H, s), 3.20 (9H, d, J = 36.6 Hz), 2.30-1.80 (7H, m), 1.35 (4H, s), 1.24 (9H, d, J = 14.6 Hz). | | |
| 505 | 1H-NMR (CDCl3) δ: 4.44-4.37 (0H, m), 4.33 (0H, q, J = 6.5 Hz), 3.83 (0H, s), 3.40 (1H, s), 2.92 (0H, d, J = 29.3 Hz), 2.22-2.13 (1H, m), 2.02-1.84 (1H, m), 1.50 (1H, d, J = 6.3 Hz), 1.35 (1H, t, J = 5.6 Hz). | | |
| 506 | 1H-NMR (CDCl3) δ: 9.07 (1H, s), 8.69 (1H, s), 8.41 (1H, d, J = 8.8 Hz), 8.34 (1H, s), 7.75 (1H, dd, J = 9.3, 2.4 Hz), 7.27 (1H, s), 6.70 (1H, s), 6.24 (1H, d, J = 7.8 Hz), 4.81 (1H, q, J = 6.2 Hz), 4.40 (1H, td, J = 13.4, 6.5 Hz), 3.90 (1H, td, J = 10.7, 4.2 Hz), 3.76 (1H, q, J = 6.8 Hz), 3.63 (1H, td, J = 7.3, 4.2 Hz), 3.30 (2H, dtd, J = 29.8, 9.8, 4.1 Hz), 1.53 (3H, d, J = 6.8 Hz), 1.50 (3H, d, J = 6.8 Hz), 1.37 (6H, d, J = 5.9 Hz). | | |
| 507 | 1H-NMR (DMSO-D6) δ: 10.50 (1H, s), 9.24 (1H, s), 8.33 (2H, d, J = 26.8 Hz), 7.77 (1H, s), 7.53 (2H, s), 7.29 (1H, s), 7.17 (1H, s), 7.04 (1H, s), 6.95 (1H, s), 5.19 (1H, s), 4.62 (1H, s), 4.19 (2H, s), 3.97 (0H, s), 3.81 (1H, s), 3.50 (1H, s), 3.33 (3H, s), 3.16 (2H, s), 1.47 (2H, s), 1.37 (2H, d, J = 5.4 Hz), 1.26 (6H, d, J = 3.9 Hz). | | |
| 508 | 1H-NMR (CDCl3) δ: 9.04 (1H, s), 8.34-8.30 (3H, m), 7.74 (1H, d, J = 5.9 Hz), 6.84 (1H, s), 6.14 (1H, br s), 4.41-4.32 (2H, m), 3.65 (2H, br s), 3.53 (2H, br s), 3.41 (3H, s), 2.65-2.60 (10H, br m), 1.51 (3H, d, J = 5.9 Hz), 1.37 (6H, t, J = 4.4 Hz). | | |

**[Table 5-98]**

| Compound No. | NMR | LC/MS (M+H)⁺ | Exact Mass |
|---|---|---|---|
| 509 | 1H-NMR (CDCl3) δ: 9.04 (1H, s), 8.52 (1H, s), 8.31 (1H, d, J = 8.8 Hz), 8.14 (1H, d, J = 2.0 Hz), 7.38 (1H, dd, J = 8.8, 2.0 Hz), 6.83 (1H, s), 6.12 (1H, d, J = 6.8 Hz), 5.07-4.94 (1H, m), 4.43-4.22 (4H, m), 3.41 (3H, s), 3.04-2.93 (2H, m), 2.53 (3H, s), 1.51 (3H, d, J = 6.3 Hz), 1.35 (6H, t, J = 5.6 Hz). | | |
| 510 | 1H-NMR (CDCl3) δ: 9.01 (1H, s), 8.38 (1H, d, J = 9.8 Hz), 8.29 (1H, br s), 7.08 (1H, d, J = 9.8 Hz), 6.69 (1H, s), 6.21 (1H, d, J = 7.3 Hz), 4.80 (1H, q, J = 5.6 Hz), 4.44-4.35 (1H, m), 4.09 (1H, d, J = 4.9 Hz), 3.72-3.61 (6H, m), 2.78 (1H, br s), 2.71-2.62 (6H, m), 1.53 (3H, d, J = 6.8 Hz), 1.37 (6H, d, J = 6.3 Hz). | | |
| 511 | 1H-NMR (CDCl3) δ: 9.05 (1H, s), 8.48 (1H, d, J = 2.4 Hz), 8.43-8.38 (2H, m), 8.23 (1H, dd, J = 9.3, 2.4 Hz), 6.85 (1H, s), 6.13 (1H, d, J = 7.3 Hz), 4.46-4.31 (2H, m), 4.13 (2H, s), 3.79 (2H, d, J = 7.8 Hz), 3.70 (2H, d, J = 7.8 Hz), 3.41 (3H, s), 2.87 (2H, s), 1.51 (3H, d, J = 6.8 Hz), 1.39-1.33 (6H, m). | | |
| 512 | 1H-NMR (CDCl3) δ: 8.97 (1H, s), 8.16 (1H, d, J = 9.3 Hz), 7.99 (1H, s), 7.62 (1H, d, J = 2.9 Hz), 6.89 (1H, dd, J = 8.8, 2.9 Hz), 6.80 (1H, s), 6.13 (1H, d, J = 7.3 Hz), 4.44-4.29 (2H, m), 3.93 (2H, s), 3.85 (2H, s), 3.40 (3H, s), 3.22-3.13 (1H, m), 2.57-2.50 (2H, m), 2.36 (3H, s), 1.99-1.92 (2H, m), 1.50 (3H, d, J = 6.8 Hz), 1.38-1.32 (6H, m). | | |
| 513 | 1H-NMR (CD3OD) δ: 9.10 (1H, s), 8.31 (1H, d, J = 8.3 Hz), 8.23 (1H, d, J = 1.5 Hz), 7.83 (1H, dd, J = 8.5, 2.2 Hz), 6.88 (1H, s), 4.60-4.51 (1H, m), 4.32 (1H, q, J = 6.3 Hz), 3.53 (2H, s), 3.35 (3H, s), 2.97-2.82 (5H, m), 2.72-2.21 (9H, m), 2.17-1.96 (3H, m), 1.45 (3H, d, J = 6.3 Hz). | | |

**[Table 5-99]**

| Compound No. | NMR | LC/MS (M+H)⁺ | Exact Mass |
|---|---|---|---|
| 518 | 1H-NMR (CDCl3) δ: 9.05 (1H, s), 8.45 (1H, s), 8.39 (1H, d, J = 8.8 Hz), 8.23 (1H, d, J = 2.0 Hz), 7.72 (1H, dd, J = 8.8, 2.4 Hz), 6.85 (1H, s), 6.13 (1H, d, J = 7.8 Hz), 4.46-4.31 (2H, m), 3.71 (2H, s), 3.67-3.61 (2H, m), 3.53-3.47 (2H, m), 3.41 (3H, s), 2.86-2.78 (4H, m), 1.51 (3H, d, J = 6.8 Hz), 1.36 (6H, t, J = 5.9 Hz). | | |
| 519 | 1H-NMR (CDCl3) δ: 9.05 (1H, s), 8.52 (1H, s), 8.36 (1H, d, J = 8.3 Hz), 8.24 (1H, d, J = 2.0 Hz), 7.73 (1H, dd, J = 8.3, 2.0 Hz), 6.70 (1H, s), 6.24 (1H, d, J = 7.3 Hz), 4.81 (1H, q, J = 6.3 Hz), 4.45-4.34 (1H, m), 4.11 (1H, br s), 3.71 (2H, s), 3.64 (2H, t, J = 4.9 Hz), 3.50 (2H, t, J = 4.9 Hz), 2.87-2.75 (4H, m), 1.53 (3H, d, J = 6.8 Hz), 1.37 (6H, d, J = 6.8 Hz). | | |
| 520 | 1H-NMR (CDCl3) δ: 9.00 (1H, s), 8.34 (1H, d, J = 9.8 Hz), 8.22 (1H, s), 7.10 (1H, d, J = 10.2 Hz), 6.68 (1H, s), 6.21 (1H, d, J = 7.3 Hz), 4.82-4.75 (1H, m), 4.41-4.33 (3H, m), 4.07 (1H, d, J = 4.4 Hz), 3.76-3.71 (4H, m), 2.97 (2H, t, J = 12.4 Hz), 2.63-2.56 (4H, m), 2.46 (1H, t, J = 11.0 Hz), 1.99 (2H, d, J = 12.7 Hz), 1.65-1.55 (2H, m), 1.53 (3H, d, J = 6.3 Hz), 1.37 (6H, d, J = 6.3 Hz). | | |
| 521 | 1H-NMR (CDCl3) δ: 9.07 (1H, s), 8.72 (1H, s), 8.41 (1H, d, J = 8.8 Hz), 8.30 (1H, d, J = 2.9 Hz), 7.67 (1H, dd, J = 8.8, 2.9 Hz), 6.70 (1H, s), 6.24 (1H, d, J = 7.8 Hz), 4.81 (1H, q, J = 6.3 Hz), 4.45-4.35 (1H, m), 4.10 (1H, br s), 3.92-3.87 (2H, m), 3.78 (2H, s), 3.22-3.17 (2H, m), 1.98-1.93 (2H, m), 1.54 (3H, d, J = 6.3 Hz), 1.37 (6H, d, J = 6.3 Hz). | | |

**[Table 5-100]**

| Compound No. | NMR | LC/MS (M+H) ⁺ | Exact Mass |
|---|---|---|---|
| 522 | 1H-NMR (CDCl3) δ: 9.09 (1H, s), 8.92 (1H, s), 8.41 (1H, d, J = 8.8 Hz), 8.30 (1H, s), 7.66 (1H, d, J = 8.8 Hz), 6.71 (1H, s), 6.23 (1H, d, J = 7.3 Hz), 4.85-4.76 (1H, m), 4.45-4.35 (1H, m), 4.13 (1H, br s), 3.87 (2H, d, J = 6.8 Hz), 3.19-3.12 (4H, m), 2.92-2.86 (2H, m), 1.88 (1H, br s), 1.54 (3H, d, J = 6.3 Hz), 1.38 (6H, d, J = 6.3 Hz). | | |
| 523 | 1H-NMR (CDCl3) δ: 9.00 (1H, s), 8.39 (1H, d, J = 9.3 Hz), 6.83 (1H, s), 6.72 (1H, d, J = 9.8 Hz), 6.16 (1H, d, J = 7.3 Hz), 4.45-4.26 (3H, m), 4.13 (2H, s), 4.10 (2H, s), 3.41 (3H, s), 2.70-2.63 (2H, m), 2.25-2.17 (2H, m), 1.50 (3H, d, J = 6.3 Hz), 1.37-1.31 (6H, m). | | |
| 524 | 1H-NMR (CDCl3) δ: 8.99 (1H, s), 8.33 (1H, d, J = 9.3 Hz), 6.71-6.66 (2H, m), 6.24 (1H, d, J = 7.3 Hz), 4.79 (1H, q, J = 6.5 Hz), 4.43-4.27 (2H, m), 4.13 (2H, s), 4.10 (2H, s), 2.70-2.63 (2H, m), 2.24-2.17 (2H, m), 1.52 (3H, d, J = 6.3 Hz), 1.36 (6H, d, J = 6.8 Hz). | | |
| 525 | 1H-NMR (CDCl3) δ: 8.99 (1H, s), 8.21-8.10 (2H, m), 7.77 (1H, d, J = 2.9 Hz), 7.00 (1H, dd, J = 8.8, 2.9 Hz), 6.80 (1H, s), 6.13 (1H, d, J = 7.8 Hz), 4.45-4.28 (3H, m), 3.99-3.92 (1H, br m), 3.71 (1H, dd, J = 9.0, 2.2 Hz), 3.40 (3H, s), 3.23-3.08 (3H, m), 2.05 (1H, d, J = 9.8 Hz), 1.95 (1H, d, J = 9.8 Hz), 1.50 (3H, d, J = 6.3 Hz), 1.38-1.32 (6H, m). | | |

**[Table 5-101]**

| Compound No. | NMR | LC/MS (M+H)⁺ | Exact Mass |
|---|---|---|---|
| 526 | 1H-NMR (CDCl3) δ: 9.03 (1H, s), 8.43 (1H, s), 8.28 (1H, d, J = 9.3 Hz), 8.09 (1H, d, J = 2.4 Hz), 7.35 (1H, dd, J = 8.8, 2.9 Hz), 6.83 (1H, s), 6.13 (1H, d, J = 7.3 Hz), 4.44-4.31 (2H, m), 4.12 (2H, t, J = 6.1 Hz), 3.41 (3H, s), 2.83 (2H, t, J = 6.1 Hz), 2.50 (3H, s), 2.08-1.91 (2H, m), 1.51 (3H, d, J = 6.3 Hz), 1.36 (3H, d, J = 6.3 Hz), 1.34 (3H, d, J = 6.3 Hz). | | |
| 527 | 1H-NMR (CDCl3) δ: 9.08 (1H, s), 8.63 (1H, br s), 8.53 (1H, d, J = 9.3 Hz), 7.06 (1H, d, J = 9.3 Hz), 6.86 (1H, s), 6.09 (1H, d, J = 7.3 Hz), 4.57 (2H, t, J = 6.3 Hz), 4.46-4.31 (2H, m), 3.41 (3H, s), 2.80 (2H, t, J = 7.1 Hz), 2.48 (3H, s), 2.08-2.01 (2H, m), 1.62 (1H, s), 1.50 (3H, d, J = 6.8 Hz), 1.34 (6H, t, J = 6.3 Hz). | | |
| 528 | 1H-NMR (CDCl3) δ: 8.99 (1H, s), 8.25 (1H, d, J = 8.8 Hz), 8.06 (1H, d, J = 2.9 Hz), 8.02 (1H, s), 7.35 (1H, dd, J = 8.8, 2.9 Hz), 6.68 (1H, s), 6.24 (1H, d, J = 7.8 Hz), 4.80 (1H, q, J = 6.2 Hz), 4.42-4.35 (1H, m), 4.12 (2H, t, J = 6.1 Hz), 2.82 (2H, t, J = 6.8 Hz), 2.49 (3H, s), 2.05-1.99 (2H, m), 1.53 (3H, d, J = 6.8 Hz), 1.38 (6H, d, J = 6.3 Hz). | | |
| 529 | 1H-NMR (CDCl3) δ: 8.99 (1H, s), 8.25 (1H, d, J = 9.3 Hz), 8.07 (1H, d, J = 2.9 Hz), 8.02 (1H, s), 7.36 (1H, dd, J = 9.3, 2.9 Hz), 6.68 (1H, s), 6.24 (1H, d, J = 7.8 Hz), 4.80 (1H, q, J = 6.2 Hz), 4.42-4.35 (1H, m), 4.15 (2H, t, J = 5.1 Hz), 3.03 (2H, t, J = 5.1 Hz), 2.55 (3H, s), 1.53 (3H, d, J = 6.3 Hz), 1.38 (6H, d, J = 6.8 Hz). | | |

**[Table 5-102]**

| Compound No. | NMR | LC/MS (M+H)⁺ | Exact Mass |
|---|---|---|---|
| 530 | 1H-NMR (CDCl3) δ: 9.05 (1H, s), 8.53-8.48 (2H, m), 7.06 (1H, d, J = 9.3 Hz), 6.71 (1H, s), 6.21 (1H, d, J = 7.3 Hz), 4.81 (1H, q, J = 6.3 Hz), 4.57 (2H, t, J = 6.3 Hz), 4.44-4.35 (1H, m), 2.80 (2H, t, J = 7.1 Hz), 2.49 (3H, s), 2.09-2.02 (2H, m), 1.53 (3H, d, J = 6.3 Hz), 1.37 (6H, d, J = 6.3 Hz). | | |
| 531 | 1H-NMR (CDCl3) δ: 9.02 (1H, s), 8.40 (1H, d, J = 9.8 Hz), 8.33 (1H, s), 7.09 (1H, d, J = 9.8 Hz), 6.83 (1H, s), 6.10 (1H, d, J = 7.3 Hz), 4.44-4.30 (2H, m), 3.66-3.64 (4H, m), 3.41 (3H, s), 2.59 (4H, t, J = 4.9 Hz), 2.38 (3H, s), 1.50 (3H, d, J = 6.3 Hz), 1.34 (6H, t, J = 6.3 Hz). | | |
| 532 | 1H-NMR (CDCl3) δ: 9.10 (1H, s), 8.94 (1H, s), 8.36 (1H, d, J = 9.8 Hz), 7.08 (1H, d, J = 9.8 Hz), 6.71 (1H, s), 6.18 (1H, d, J = 7.8 Hz), 4.80 (1H, q, J = 6.2 Hz), 4.44-4.32 (1H, m), 4.21 (1H, s), 3.67-3.62 (4H, m), 2.61-2.56 (4H, m), 2.37 (3H, s), 1.53 (3H, d, J = 6.8 Hz), 1.36 (6H, d, J = 6.8 Hz). | | |
| 538 | 1H-NMR (DMSO-D6) δ: 10.44 (0H, s), 9.28 (1H, s), 8.41-8.33 (1H, m), 7.99 (1H, br s), 7.88 (1H, d, J = 6.8 Hz), 7.49 (1H, br s), 7.00 (1H, s), 6.45 (1H, s), 5.21 (1H, s), 4.61 (1H, s), 4.30-4.23 (1H, m), 4.10-4.03 (1H, m), 3.52 (1H, s), 3.23 (3H, s), 1.38 (3H, d, J = 5.9 Hz), 1.28 (6H, d, J = 5.9 Hz). | | |
| 539 | 1H-NMR (DMSO-D6) δ: 9.69 (0H, s), 9.16 (1H, s), 7.90 (2H, d, J = 4.9 Hz), 7.67 (1H, s), 7.48 (2H, s), 7.29 (1H, s), 7.09 (4H, d, J = 49.8 Hz), 6.94 (1H, s), 6.38 (1H, d, J = 10.7 Hz), 6.03 (0H, s), 5.16 (1H, s), 4.60 (1H, d, J = 5.9 Hz), 4.25-4.19 (1H, m), 3.66 (2H, s), 2.63 (3H, t, J = 12.2 Hz), 1.37 (3H, d, J = 6.8 Hz), 1.27 (6H, d, J = 6.8 Hz). | | |

**[Table 5-103]**

| Compound No. | NMR | LC/MS (M+H) ⁺ | Exact Mass |
|---|---|---|---|
| 548 | 1H-NMR (CDCl3) δ: 9.10 (1H, s), 9.01 (1H, s), 8.37-8.32 (2H, m), 7.75 (1H, dd, J = 8.5, 1.7 Hz), 6.71 (1H, s), 6.25 (1H, d, J = 7.3 Hz), 4.81 (1H, q, J = 6.3 Hz), 4.45-4.37 (1H, m), 3.52 (2H, s), 2.46 (8H, br s), 2.30 (3H, s), 1.54 (3H, d, J = 6.3 Hz), 1.38 (6H, d, J = 6.3 Hz). | | |
| 549 | 1H-NMR (CDCl3) δ: 9.06 (1H, s), 8.66 (1H, s), 8.34 (1H, d, J = 8.8 Hz), 8.29 (1H, d, J = 2.0 Hz), 7.76 (1H, dd, J = 8.8, 2.0 Hz), 6.70 (1H, s), 6.26 (1H, d, J = 7.3 Hz), 4.81 (1H, q, J = 6.3 Hz), 4.45-4.35 (1H, m), 3.50 (2H, s), 3.01 (2H, d, J = 11.2 Hz), 1.99-1.93 (2H, m), 1.74 (2H, d, J = 11.7 Hz), 1.54 (3H, d, J = 6.3 Hz), 1.42-1.23 (11H, m), 1.18 (6H, s). | | |
| 550 | 1H-NMR (CDCl3) δ: 9.06-9.02 (1H, m), 8.68 (1H, br s), 8.28 (1H, d, J = 9.3 Hz), 8.12-8.06 (1H, m), 7.34 (1H, dd, J = 9.0, 3.2 Hz), 6.69 (1H, s), 6.24 (1H, d, J = 6.8 Hz), 4.81 (1H, q, J = 6.5 Hz), 4.44-4.35 (1H, m), 4.26 (2H, t, J = 5.1 Hz), 3.80 (2H, t, J = 5.1 Hz), 3.61-3.55 (4H, m), 3.11 (2H, t, J = 5.1 Hz), 1.53 (3H, d, J = 6.3 Hz), 1.38 (6H, d, J = 6.3 Hz). | | |
| 551 | 1H-NMR (CDCl3) δ: 9.07 (1H, s), 8.63 (1H, s), 8.54 (1H, d, J = 9.3 Hz), 7.10 (1H, d, J = 9.3 Hz), 6.85 (1H, s), 6.09 (1H, d, J = 7.3 Hz), 4.65-4.60 (2H, br m), 4.41-4.32 (2H, m), 3.41 (3H, s), 3.08 (2H, br s), 2.55 (3H, s), 1.50 (3H, d, J = 6.3 Hz), 1.34 (6H, t, J = 5.6 Hz). | | |
| 552 | 1H-NMR (CDCl3) δ: 9.08 (1H, s), 8.72 (1H, s), 8.51 (1H, d, J = 9.3 Hz), 7.10 (1H, d, J = 9.3 Hz), 6.72 (1H, s), 6.21 (1H, s), 4.83-4.78 (1H, m), 4.63-4.58 (2H, m), 4.43-4.36 (1H, m), 3.09-3.03 (2H, m), 2.54 (3H, s), 1.54 (3H, d, J = 5.9 Hz), 1.36 (6H, d, J = 4.4 Hz). | | |

**[Table 5-104]**

| Compound No. | NMR | LC/MS (M+H) ⁺ | Exact Mass |
|---|---|---|---|
| 553 | 1H-NMR (CDCl3) δ: 9.06 (1H, s), 8.48 (1H, s), 8.43 (1H, d, J = 8.8 Hz), 8.24 (1H, s), 7.65 (1H, d, J = 8.8 Hz), 6.85 (1H, s), 6.13 (1H, d, J = 7.3 Hz), 4.44-4.32 (2H, m), 3.98-3.86 (2H, m), 3.67-3.62 (1H, m), 3.42 (3H, s), 2.02-1.80 (6H, m), 1.75-1.69 (2H, m), 1.51 (3H, d, J = 6.3 Hz), 1.36 (6H, t, J = 5.7 Hz). | | |
| 554 | 1H-NMR (CDCl3) δ: 9.06 (1H, s), 8.55 (1H, s), 8.41 (1H, d, J = 9.8 Hz), 8.25 (1H, s), 7.65 (1H, dd, J = 9.8, 2.4 Hz), 6.71 (1H, s), 6.24 (1H, d, J = 7.3 Hz), 4.82 (1H, q, J = 6.3 Hz), 4.45-4.38 (1H, m), 3.98-3.85 (2H, m), 3.65-3.60 (1H, m), 2.08-1.71 (6H, m), 1.54 (3H, d, J = 6.3 Hz), 1.38 (6H, d, J = 6.3 Hz). | | |
| 555 | 1H-NMR (CDCl3) δ: 9.07 (1H, s), 8.71-8.63 (1H, br m), 8.35 (1H, d, J = 8.3 Hz), 8.31 (1H, s), 7.76 (1H, d, J = 8.3 Hz), 6.71 (1H, s), 6.29-6.23 (1H, m), 4.85-4.80 (1H, m), 4.45-4.37 (1H, m), 3.65-3.59 (2H, m), 3.54-3.50 (2H, br m), 2.58-2.57 (10H, m), 1.54 (3H, d, J = 6.3 Hz), 1.39 (6H, d, J = 6.3 Hz). | | |
| 556 | 1H-NMR (CDCl3) δ: 9.08 (1H, s), 8.76-8.67 (1H, br m), 8.53 (1H, d, J = 2.4 Hz), 8.42 (1H, d, J = 9.3 Hz), 8.34 (1H, d, J = 8.8 Hz), 6.85 (1H, s), 6.13 (1H, d, J = 7.3 Hz), 4.46-4.30 (2H, m), 4.16 (1H, t, J = 8.8 Hz), 4.07 (1H, d, J = 8.3 Hz), 3.62 (1H, d, J = 9.8 Hz), 3.42 (3H, s), 3.13-3.06 (2H, m), 2.94-2.89 (1H, m), 2.28-2.17 (1H, m), 1.78-1.74 (2H, m), 1.51 (3H, d, J = 6.3 Hz), 1.36 (6H, t, J = 5.9 Hz). | | |

**[Table 5-105]**

| Compound No. | NMR | LC/MS (M+H) ⁺ | Exact Mass |
|---|---|---|---|
| 557 | 1H-NMR (CDCl3) δ: 9.06 (1H, s), 8.60 (1H, s), 8.32 (1H, d, J = 8.8 Hz), 8.16 (1H, d, J = 2.9 Hz), 7.44 (1H, dd, J = 8.8, 2.9 Hz), 6.84 (1H, s), 6.12 (1H, d, J = 7.3 Hz), 4.76 (2H, s), 4.45-4.31 (2H, m), 3.66-3.53 (4H, m), 3.41 (3H, s), 2.92-2.85 (4H, m), 1.51 (3H, d, J = 6.3 Hz), 1.36 (6H, t, J = 5.9 Hz). | | |
| 558 | 1H-NMR (CDCl3) δ: 9.03 (1H, s), 8.47 (1H, d, J = 2.4 Hz), 8.40-8.32 (2H, m), 8.28 (1H, s), 6.70 (1H, s), 6.24 (1H, d, J = 7.3 Hz), 4.84-4.76 (1H, br m), 4.44-4.39 (1H, m), 4.18-4.05 (3H, m), 3.64-3.58 (1H, m), 3.15-3.03 (2H, m), 2.95-2.88 (1H, m), 2.27-2.16 (1H, m), 1.80-1.72 (1H, m), 1.53 (3H, d, J = 6.3 Hz), 1.38 (6H, d, J = 6.3 Hz). | | |
| 559 | 1H-NMR (CDCl3) δ: 9.03 (1H, s), 8.50 (1H, s), 8.29 (1H, d, J = 8.8 Hz), 8.14 (1H, d, J = 2.9 Hz), 7.44 (1H, dd, J = 8.8, 2.9 Hz), 6.69 (1H, d, J = 1.0 Hz), 6.23 (1H, d, J = 7.3 Hz), 4.82-4.72 (3H, m), 4.44-4.34 (1H, m), 3.67-3.54 (4H, m), 2.92-2.85 (4H, m), 1.53 (3H, d, J = 6.3 Hz), 1.38 (6H, d, J = 6.3 Hz). | | |
| 560 | 1H-NMR (CDCl3) δ: 9.03 (1H, s), 8.37-8.33 (2H, m), 7.10 (1H, d, J = 9.8 Hz), 6.83 (1H, s), 6.09 (1H, d, J = 7.3 Hz), 4.44-4.30 (2H, m), 3.70-3.62 (4H, m), 3.41 (3H, s), 1.74-1.67 (2H, m), 1.61-1.55 (2H, m), 1.50 (3H, d, J = 6.3 Hz), 1.35 (6H, t, J = 5.6 Hz), 1.22 (3H, s). | | |
| 561 | 1H-NMR (CDCl3) δ: 9.04 (1H, s), 8.58 (1H, s), 8.35-8.33 (1H, m), 7.16-7.08 (1H, m), 6.76-6.69 (1H, m), 6.22 (1H, br s), 4.86-4.79 (1H, m), 4.43-4.37 (1H, m), 3.72-3.67 (4H, m), 3.53-3.49 (1H, m), 1.74-1.68 (2H, m), 1.60-1.52 (5H, m), 1.39 (6H, d, J = 6.3 Hz), 1.22 (3H, s). | | |

**[Table 5-106]**

| Compound No. | NMR | LC/MS (M+H)⁺ | Exact Mass |
|---|---|---|---|
| 562 | 1H-NMR (CDCl3) δ: 8.99 (1H, s), 8.34 (1H, d, J = 9.8 Hz), 8.21 (1H, s), 6.73-6.66 (2H, m), 6.20 (1H, d, J = 6.8 Hz), 4.79 (1H, q, J = 6.5 Hz), 4.43-4.34 (1H, m), 4.24 (4H, s), 3.86 (4H, s), 1.52 (3H, d, J = 6.8 Hz), 1.36 (6H, d, J = 6.8 Hz). | | |
| 563 | 1H-NMR (CDCl3) δ: 9.02 (1H, s), 8.56 (1H, d, J = 9.8 Hz), 8.18 (1H, s), 7.14 (1H, d, J = 9.8 Hz), 6.70 (1H, s), 6.22 (1H, d, J = 7.8 Hz), 5.52-5.37 (1H, m), 5.10-4.95 (1H, m), 4.84-4.77 (1H, m), 4.45-4.34 (1H, m), 3.47-3.32 (1H, m), 3.27-3.14 (1H, m), 3.01-2.74 (2H, m), 2.09-2.00 (2H, m), 1.53 (3H, d, J = 6.8 Hz), 1.37 (6H, d, J = 6.8 Hz). | | |
| 564 | 1H-NMR (CDC13) δ: 9.02 (1H, s), 8.55 (1H, d, J = 8.8 Hz), 8.22 (1H, s), 7.11 (1H, d, J = 9.8 Hz), 6.71 (1H, s), 6.22 (1H, d, J = 7.8 Hz), 5.53-5.42 (1H, m), 4.84-4.57 (2H, m), 4.45-4.35 (1H, m), 3.44-3.35 (1H, m), 3.10-3.03 (1H, m), 2.93-2.76 (2H, m), 2.48-2.38 (1H, m), 1.71-1.62 (1H, m), 1.53 (3H, d, J = 6.8 Hz), 1.37 (6H, d, J = 6.8 Hz). | | |
| 565 | 1H-NMR (CDCl3) δ: 9.01 (1H, s), 8.28-8.21 (2H, m), 8.10 (1H, d, J = 2.9 Hz), 7.37 (1H, dd, J = 8.8, 2.9 Hz), 6.68 (1H, s), 6.24 (1H, d, J = 7.8 Hz), 4.80 (1H, q, J = 6.2 Hz), 4.45-4.24 (2H, m), 3.25-3.19 (1H, m), 2.95-2.75 (3H, m), 2.10-2.01 (1H, m), 1.90-1.72 (2H, m), 1.61-1.50 (4H, m), 1.38 (6H, d, J = 6.8 Hz). | | |
| 566 | 1H-NMR (CDCl3) δ: 9.01 (1H, s), 8.28-8.22 (2H, m), 8.04 (1H, d, J = 2.9 Hz), 7.31 (1H, dd, J = 8.8, 2.9 Hz), 6.68 (1H, s), 6.24 (1H, d, J = 7.8 Hz), 4.90-4.77 (2H, m), 4.44-4.35 (1H, m), 3.27-3.18 (2H, m), 3.07 (1H, dd, J = 12.7, 4.0 Hz), 2.99-2.91 (1H, m), 2.19-1.95 (2H, m), 1.53 (3H, d, J = 6.8 Hz), 1.38 (6H, d, J = 5.9 Hz). | | |

**[Table 5-107]**

| Compound No. | NMR | LC/MS (M+H)⁺ | Exact Mass |
|---|---|---|---|
| 567 | 1H-NMR (CDC13) δ: 9.01 (1H, s), 8.32-8.21 (2H, m), 8.13 (1H, d, J = 2.9 Hz), 7.42 (1H, dd, J = 9.3, 2.4 Hz), 6.69 (1H, s), 6.24 (1H, d, J = 7.8 Hz), 4.85-4.53 (2H, m), 4.45-4.33 (2H, m), 3.47-3.35 (1H, m), 3.15-3.05 (1H, br m), 2.99-2.89 (1H, m), 2.82-2.73 (1H, m), 2.24-2.12 (1H, br m), 1.82-1.71 (1H, m), 1.53 (3H, d, J = 6.8 Hz), 1.38 (6H, d, J = 6.8 Hz). | | |
| 568 | 1H-NMR (CDCl3) δ: 9.00 (1H, s), 8.25 (1H, d, J = 8.8 Hz), 8.15 (1H, s), 7.92 (1H, d, J = 2.9 Hz), 7.22 (1H, dd, J = 9.3, 2.9 Hz), 6.68 (1H, s), 6.24 (1H, d, J = 7.3 Hz), 5.07-5.00 (1H, m), 4.80 (1H, q, J = 6.3 Hz), 4.45-4.34 (1H, m), 3.99-3.93 (2H, m), 3.87-3.81 (2H, m), 1.53 (3H, d, J = 6.3 Hz), 1.40-1.34 (6H, m). | | |
| 569 | 1H-NMR (CDCl3) δ: 9.02 (1H, s), 8.35-8.27 (2H, m), 8.15 (1H, d, J = 2.9 Hz), 7.43 (1H, dd, J = 8.8, 2.9 Hz), 6.69 (1H, s), 6.24 (1H, d, J = 6.8 Hz), 4.95-4.76 (2H, m), 4.45-4.33 (2H, m), 3.47-3.37 (1H, m), 3.26-3.18 (1H, m), 3.02-2.87 (1H, m), 2.84-2.74 (1H, m), 2.10-1.95 (2H, m), 1.53 (3H, d, J = 6.8 Hz), 1.38 (6H, d, J = 5.9 Hz). | | |
| 570 | 1H-NMR (CDCl3) δ: 9.00 (1H, s) , 8.27 (1H, d, J = 9.3 Hz), 8.15-8.06 (2H, m), 7.37 (1H, dd, J = 8.8, 2.9 Hz), 6.68 (1H, s), 6.24 (1H, d, J = 7.3 Hz), 4.80 (1H, q, J = 6.2 Hz), 4.45-4.34 (1H, m), 4.22-4.16 (2H, m), 3.92-3.82 (2H, br m), 3.68-3.58 (2H, br m), 3.25-3.14 (1H, br m), 1.53 (3H, d, J = 6.3 Hz), 1.38 (6H, d, J = 6.3 Hz). | | |

**[Table 5-108]**

| Compound No. | NMR | LC/MS (M+H)⁺ | Exact Mass |
|---|---|---|---|
| 571 | 1H-NMR (CDCl3) δ: 9.01 (1H, s), 8.35 (1H, s), 8.26 (1H, d, J = 8.8 Hz), 8.11 (1H, d, J = 2.9 Hz), 7.37 (1H, dd, J = 9.3, 2.9 Hz), 6.68 (1H, s), 6.24 (1H, d, J = 7.8 Hz), 4.80 (1H, q, J = 6.3 Hz), 4.44-4.24 (2H, m), 3.26-3.20 (1H, m), 2.95-2.76 (3H, m), 2.09-2.00 (1H, m), 1.91-1.72 (2H, m), 1.62-1.51 (4H, m), 1.38 (6H, d, J = 6.3 Hz). | | |
| 572 | 1H-NMR (CDCl3) δ: 9.01 (1H, s), 8.33-8.24 (2H, m), 8.05 (1H, d, J = 2.9 Hz), 7.32 (1H, dd, J = 8.8, 2.9 Hz), 6.68 (1H, s), 6.24 (1H, d, J = 7.8 Hz), 4.93-4.76 (2H, m), 4.44-4.35 (1H, m), 3.32-3.21 (2H, m), 3.17-3.10 (1H, m), 3.08-2.98 (1H, m), 2.21-1.99 (2H, m), 1.53 (3H, d, J = 5.9 Hz), 1.38 (6H, d, J = 5.9 Hz). | | |
| 573 | 1H-NMR (DMSO-D6) δ: 10.27 (1H, s), 9.20 (1H, s), 8.79 (1H, br s), 8.22 (1H, d, J = 9.8 Hz), 7.05 (1H, d, J = 9.8 Hz), 6.96 (1H, s), 6.30 (1H, d, J = 7.8 Hz), 5.22-5.15 (1H, br m), 4.64-4.56 (1H, br m), 4.40 (2H, d, J = 9.8 Hz), 4.29-4.18 (3H, m), 3.75 (2H, t, J = 8.3 Hz), 2.69 (2H, t, J = 8.3 Hz), 1.37 (3H, d, J = 5.9 Hz), 1.26 (6H, d, J = 5.9 Hz). | | |
| 574 | 1H-NMR (DMSO-D6) δ: 10.20 (1H, s), 9.19 (1H, s), 8.17 (1H, d, J = 9.8 Hz), 6.97-6.92 (2H, m), 6.30 (1H, d, J = 7.8 Hz), 5.19 (1H, br s), 4.59 (1H, q, J = 6.5 Hz), 4.28-4.17 (1H, m), 4.02-3.92 (4H, m), 3.19 (2H, s), 3.02 (2H, t, J = 6.8 Hz), 2.09 (2H, t, J = 6.8 Hz), 1.37 (3H, d, J = 6.8 Hz), 1.26 (6H, d, J = 6.8 Hz). | | |

**[Table 5-109]**

| Compound No. | NMR | LC/MS (M+H)⁺ | Exact Mass |
|---|---|---|---|
| 575 | 1H-NMR (DMSO-D6) δ: 10.82 (1H, s), 9.27 (1H, s), 8.59-8.50 (2H, br m), 8.25 (1H, d, J = 9.8 Hz), 7.25 (1H, d, J = 8.8 Hz), 7.01 (1H, s), 6.58-6.49 (1H, br m), 4.62 (1H, q, J = 6.5 Hz), 4.31-4.20 (1H, m), 3.95 (4H, s), 3.12-3.04 (4H, br m), 2.02-1.92 (4H, br m), 1.38 (3H, d, J = 6.8 Hz), 1.27 (6H, d, J = 6.8 Hz). | | |
| 576 | 1H-NMR (CDCl3) δ: 8.98 (1H, s), 8.31 (1H, d, J = 9.3 Hz), 8.17 (1H, br s), 6.69-6.63 (2H, m), 6.20 (1H, d, J = 7.3 Hz), 4.79 (1H, q, J = 6.3 Hz), 4.44-4.33 (1H, m), 4.13 (2H, s), 4.05 (2H, s), 3.49-3.37 (1H, m), 2.65-2.57 (2H, m), 2.00-1.92 (2H, m), 1.52 (3H, d, J = 6.3 Hz), 1.36 (6H, d, J = 6.3 Hz). | | |
| 577 | 1H-NMR (CDCl3) δ: 9.02 (1H, s), 8.37 (1H, s), 8.28 (1H, d, J = 8.8 Hz), 8.17 (1H, d, J = 2.9 Hz), 7.44 (1H, dd, J = 8.8, 2.9 Hz), 6.68 (1H, s), 6.24 (1H, d, J = 7.8 Hz), 4.84-4.63 (2H, m), 4.45-4.34 (1H, m), 4.28-4.18 (1H, m), 3.43-3.34 (1H, m), 3.15-3.07 (1H, m), 2.82-2.68 (2H, m), 2.29-2.14 (1H, m), 1.88-1.74 (1H, m), 1.53 (3H, d, J = 5.9 Hz), 1.38 (6H, d, J = 6.8 Hz). | | |
| 578 | 1H-NMR (CDCl3) δ: 9.00 (1H, s), 8.29 (1H, d, J = 8.8 Hz), 8.18 (1H, s), 8.12 (1H, d, J = 2.9 Hz), 7.41 (1H, dd, J = 9.0, 3.2 Hz), 6.68 (1H, s), 6.24 (1H, d, J = 7.8 Hz), 4.80 (1H, q, J = 6.5 Hz), 4.45-4.32 (3H, m), 4.03-3.94 (2H, m), 3.76-3.68 (2H, m), 1.53 (3H, d, J = 6.3 Hz), 1.38 (6H, d, J = 5.9 Hz). | | |

**[Table 5-110]**

| Compound No. | NMR | LC/MS (M+H)⁺ | Exact Mass |
|---|---|---|---|
| 581 | 1H-NMR (CDCl3) δ: 9.01 (1H, s), 8.46 (1H, br s), 8.36 (1H, d, J = 9.8 Hz), 7.13 (1H, d, J = 9.8 Hz), 6.68 (1H, s), 6.21 (1H, d, J = 7.8 Hz), 4.80 (1H, q, J = 6.2 Hz), 4.44-4.34 (1H, m), 4.26-3.82 (3H, m), 3.69-3.43 (3H, m), 2.01-1.89 (2H, m), 1.81-1.51 (6H, m), 1.37 (6H, d, J = 5.9 Hz). | | |
| 582 | 1H-NMR (CDC13) δ: 9.01 (1H, s), 8.46 (1H, br s), 8.36 (1H, d, J = 9.8 Hz), 7.14 (1H, d, J = 9.8 Hz), 6.69 (1H, s), 6.22 (1H, d, J = 7.8 Hz), 4.80 (1H, q, J = 6.2 Hz), 4.45-4.34 (1H, m), 4.21-3.81 (3H, m), 3.69-3.44 (3H, m), 2.01-1.89 (2H, m), 1.77-1.51 (6H, m), 1.37 (6H, d, J = 5.9 Hz). | | |
| 583 | 1H-NMR (CDCl3) δ: 9.03 (1H, s), 8.49 (1H, d, J = 2.9 Hz), 8.40 (1H, d, J = 8.8 Hz), 8.32-8.27 (2H, m), 6.70 (1H, s), 6.25 (1H, d, J = 7.3 Hz), 4.81 (1H, q, J = 6.3 Hz), 4.43-4.37 (1H, m), 3.85-3.73 (3H, m), 2.68-2.61 (1H, m), 2.03-1.88 (1H, m), 1.54 (3H, d, J = 6.3 Hz), 1.38 (6H, d, J = 6.3 Hz). | | |
| 584 | 1H-NMR (CDC13) δ: 9.04 (1H, s), 8.44-8.39 (2H, m), 8.33 (1H, d, J = 2.4 Hz), 7.77 (1H, dd, J = 9.0, 2.4 Hz), 6.70 (1H, s), 6.25 (1H, d, J = 7.3 Hz), 4.83-4.77 (1H, m), 4.44-4.35 (1H, m), 4.11 (1H, br s), 3.74 (2H, t, J = 5.4 Hz), 3.45 (2H, s), 2.92-2.77 (3H, m), 1.53 (3H, d, J = 6.3 Hz), 1.37 (6H, d, J = 6.3 Hz), 1.13 (6H, d, J = 6.3 Hz). | | |
| 585 | 1H-NMR (CDC13) δ: 9.02 (1H, s), 8.37-8.30 (2H, m), 7.08 (1H, d, J = 9.8 Hz), 6.69 (1H, s), 6.21 (1H, d, J = 7.8 Hz), 4.80 (1H, q, J = 6.5 Hz), 4.42-4.33 (1H, m), 4.10 (1H, br s), 3.64 (4H, t, J = 4.9 Hz), 2.80-2.66 (5H, m), 1.53 (3H, d, J = 5.9 Hz), 1.37 (6H, d, J = 5.9 Hz), 1.11 (6H, d, J = 6.8 Hz). | | |

**[Table 5-111]**

| Compound No. | NMR | LC/MS (M+H)⁺ | Exact Mass |
|---|---|---|---|
| 586 | 1H-NMR (CDC13) δ: 9.01 (1H, s), 8.36-8.33 (2H, m), 7.07 (1H, d, J = 9.8 Hz), 6.69 (1H, s), 6.21 (1H, d, J = 7.8 Hz), 4.80 (1H, q, J = 6.8 Hz), 4.44-4.32 (1H, m), 4.10 (1H, br s), 3.62 (4H, t, J = 4.9 Hz), 2.56 (4H, t, J = 4.9 Hz), 2.16 (2H, d, J = 6.8 Hz), 1.88-1.78 (1H, m), 1.53 (3H, d, J = 6.8 Hz), 1.37 (6H, d, J = 6.8 Hz), 0.94 (6H, d, J = 5.9 Hz). | | |
| 587 | 1H-NMR (CDCl3) δ: 9.00 (1H, s), 8.34 (1H, d, J = 9.8 Hz), 8.25 (1H, s), 7.06 (1H, d, J = 9.8 Hz), 6.68 (1H, s), 6.21 (1H, d, J = 6.8 Hz), 4.79 (1H, q, J = 3.9 Hz), 4.43-4.33 (1H, m), 4.09 (1H, d, J = 4.0 Hz), 3.59 (4H, t, J = 4.4 Hz), 2.68 (4H, t, J = 4.4 Hz), 2.14 (2H, s), 1.53 (3H, d, J = 5.9 Hz), 1.37 (6H, d, J = 5.9 Hz), 0.92 (9H, s). | | |
| 588 | 1H-NMR (CDCl3) δ: 9.01 (1H, s), 8.38 (1H, d, J = 9.8 Hz), 8.30 (1H, s), 7.08 (1H, d, J = 9.8 Hz), 6.69 (1H, s), 6.21 (1H, d, J = 7.8 Hz), 4.80 (1H, q, J = 6.5 Hz), 4.44-4.33 (1H, m), 4.16-4.05 (1H, br m), 3.70-3.59 (4H, m), 3.51-3.46 (1H, m), 3.40 (1H, t, J = 10.7 Hz), 2.95-2.89 (1H, m), 2.87-2.81 (2H, m), 2.62-2.57 (2H, m), 1.53 (3H, d, J = 5.9 Hz), 1.37 (6H, d, J = 6.8 Hz), 0.96 (3H, d, J = 5.9 Hz). | | |
| 589 | 1H-NMR (CDCl3) δ: 9.00 (1H, s), 8.41 (1H, d, J = 9.8 Hz), 8.17 (1H, s), 7.11 (1H, d, J = 10.7 Hz), 6.76 (1H, d, J = 6.8 Hz), 6.71 (1H, d, J = 2.9 Hz), 4.82-4.75 (1H, m), 4.27 (1H, br s), 4.10-4.04 (3H, m), 3.99-3.95 (1H, m), 3.86 (1H, t, J = 5.4 Hz), 3.68 (1H, dd, J = 11.7, 4.9 Hz), 3.49 (1H, d, J = 5.9 Hz), 3.27 (2H, t, J = 11.7 Hz), 2.05-1.94 (5H, m), 1.77-1.50 (6H, m), 1.32 (3H, s), 1.31 (3H, s). | | |

**[Table 5-112]**

| Compound No. | NMR | LC/MS (M+H)⁺ | Exact Mass |
|---|---|---|---|
| 590 | 1H-NMR (CDCl3) δ: 8.99 (1H, s), 8.33 (1H, d, J = 9.8 Hz), 8.13 (1H, s), 7.10 (1H, d, J = 9.8 Hz), 6.70 (1H, s), 6.25 (1H, d, J = 7.8 Hz), 4.84-4.76 (1H, m), 4.10-4.07 (3H, m), 3.99 (2H, d, J = 4.9 Hz), 3.39 (3H, s), 3.32-3.26 (3H, m), 2.34-2.23 (2H, br m), 2.18-2.11 (2H, br m), 2.07-2.00 (2H, m), 1.71-1.34 (9H, m). | | |
| 591 | 1H-NMR (CDCl3) δ: 9.09 (1H, s), 8.69 (1H, s), 8.44 (1H, d, J = 8.8 Hz), 8.32 (1H, s), 7.71 (1H, d, J = 8.8 Hz), 6.85 (1H, s), 6.12 (1H, d, J = 6.8 Hz), 4.46-4.30 (2H, m), 3.82-3.73 (1H, m), 3.69-3.63 (1H, m), 3.62-3.57 (1H, m), 3.41 (3H, s), 2.36 (1H, s), 2.08-2.06 (3H, m), 1.51 (3H, d, J = 6.8 Hz), 1.35 (6H, t, J = 5.4 Hz). | | |
| 592 | 1H-NMR (CDCl3) δ: 9.03 (1H, s), 8.82 (1H, s), 8.24 (1H, d, J = 8.8 Hz), 8.14 (1H, d, J = 2.9 Hz), 7.32 (1H, dd, J = 8.8, 2.9 Hz), 6.70 (1H, s), 6.27 (1H, d, J = 7.8 Hz), 4.80 (1H, q, J = 6.2 Hz), 4.17-4.03 (3H, m), 3.40 (3H, s), 3.28 (1H, br s), 3.02 (2H, t, J = 5.4 Hz), 2.55 (3H, s), 2.31-2.25 (2H, m), 2.16-2.10 (2H, m), 1.54-1.38 (8H, m). | | |
| 593 | 1H-NMR (CDCl3) δ: 9.05 (1H, s), 8.47 (1H, s), 8.41 (1H, d, J = 8.8 Hz), 8.29 (1H, d, J = 2.0 Hz), 7.72 (1H, dd, J = 8.8, 2.0 Hz), 6.70 (1H, s), 6.24 (1H, d, J = 7.8 Hz), 4.81 (1H, q, J = 6.5 Hz), 4.45-4.35 (1H, m), 3.82-3.75 (1H, m), 3.70-3.65 (1H, m), 3.58 (1H, dd, J = 10.7, 5.9 Hz), 2.41-2.34 (1H, m), 2.13-2.00 (3H, m), 1.85-1.73 (3H, m), 1.54 (3H, d, J = 5.9 Hz), 1.37 (6H, d, J = 5.9 Hz). | | |

**[Table 5-113]**

| Compound No. | NMR | LC/MS (M+H)⁺ | Exact Mass |
|---|---|---|---|
| 594 | 1H-NMR (CDC13) δ: 9.03 (1H, s), 8.41 (1H, s), 8.35 (1H, d, J = 9.8 Hz), 7.09 (1H, d, J = 9.8 Hz), 6.83 (1H, s), 6.09 (1H, d, J = 6.8 Hz), 4.44-4.30 (2H, m), 3.68-3.58 (4H, m), 3.40 (3H, s), 2.37 (3H, s), 1.72-1.62 (4H, m), 1.50 (3H, d, J = 5.9 Hz), 1.34 (6H, t, J = 5.9 Hz), 1.17 (3H, s). | | |
| 595 | 1H-NMR (CDCl3) δ: 9.00 (1H, s), 8.38 (1H, d, J = 9.8 Hz), 8.14 (1H, s), 7.10 (1H, d, J = 10.7 Hz), 6.73 (1H, s), 6.65 (1H, d, J = 7.8 Hz), 4.83-4.75 (1H, m), 4.37-4.30 (1H, m), 4.13-3.85 (5H, m), 3.84-3.70 (3H, m), 3.64 (1H, dd, J = 10.7, 5.9 Hz), 3.33-3.23 (2H, m), 2.08-2.00 (4H, m), 1.92-1.83 (2H, m), 1.74-1.62 (2H, m), 1.53 (3H, d, J = 6.8 Hz). | | |
| 596 | 1H-NMR (CDC13) δ: 9.06 (1H, s), 8.58 (1H, s), 8.43 (1H, d, J = 8.8 Hz), 8.36 (1H, d, J = 2.9 Hz), 7.78 (1H, dd, J = 8.8, 2.9 Hz), 6.70 (1H, s), 6.24 (1H, d, J = 7.8 Hz), 4.81 (1H, q, J = 5.9 Hz), 4.46-4.34 (1H, m), 4.11 (1H, s), 3.75 (2H, t, J = 5.4 Hz), 3.31 (2H, s), 2.82 (2H, t, J = 5.4 Hz), 2.24 (2H, d, J = 6.8 Hz), 1.89-1.79 (1H, m), 1.54 (3H, d, J = 5.9 Hz), 1.37 (6H, d, J = 5.9 Hz), 0.96 (6H, d, J = 6.8 Hz). | | |
| 597 | 1H-NMR (CDC13) δ: 9.06 (1H, s), 8.62 (1H, s), 8.43 (1H, d, J = 8.8 Hz), 8.35 (1H, d, J = 2.9 Hz), 7.76 (1H, dd, J = 8.8, 2.9 Hz), 6.70 (1H, s), 6.24 (1H, d, J = 7.8 Hz), 4.81 (1H, q, J = 5.9 Hz), 4.44-4.37 (1H, m), 4.10 (1H, br s), 3.79-3.72 (4H, m), 3.44 (2H, s), 2.97 (2H, t, J = 5.4 Hz), 2.73 (2H, t, J = 5.4 Hz), 2.43 (1H, s), 1.54 (3H, d, J = 5.9 Hz), 1.37 (6H, d, J = 5.9 Hz). | | |

**[Table 5-114]**

| Compound No. | NMR | LC/MS (M+H)⁺ | Exact Mass |
|---|---|---|---|
| 598 | 1H-NMR (CDCl3) δ: 9.07 (1H, s), 8.69 (1H, s), 8.44 (1H, d, J = 8.8 Hz), 8.36 (1H, d, J = 2.9 Hz), 7.76 (1H, dd, J = 8.8, 2.9 Hz), 6.70 (1H, s), 6.24 (1H, d, J = 7.8 Hz), 4.81 (1H, q, J = 5.9 Hz), 4.44-4.36 (1H, m), 4.11 (1H, br s), 3.76 (2H, t, J = 5.4 Hz), 3.59-3.42 (4H, m), 3.09-3.04 (1H, m), 3.00-0.00 (1H, m), 2.88-2.82 (2H, m), 1.54 (3H, d, J = 5.9 Hz), 1.37 (6H, d, J = 5.9 Hz), 1.04 (3H, d, J = 5.9 Hz). | | |
| 599 | 1H-NMR (CDC13) δ: 9.04 (1H, s), 8.39-8.36 (2H, m), 7.07 (1H, d, J = 9.8 Hz), 6.83 (1H, s), 6.09 (1H, d, J = 7.8 Hz), 4.44-4.30 (2H, m), 3.64-3.60 (4H, m), 3.41 (3H, s), 2.77-2.71 (4H, m), 1.50 (3H, d, J = 6.8 Hz), 1.34 (6H, t, J = 5.9 Hz), 1.13 (9H, s). | | |
| 600 | 1H-NMR (CDCl3) δ: 9.06 (1H, s), 8.80 (1H, br s), 8.34 (1H, d, J = 8.8 Hz), 8.16 (1H, d, J = 2.9 Hz), 7.38 (1H, dd, J = 8.8, 2.9 Hz), 6.80 (1H, dd, J = 7.8, 2.9 Hz), 6.71 (1H, d, J = 2.9 Hz), 4.79 (1H, q, J = 6.5 Hz), 4.27 (1H, br s), 4.13 (2H, t, J = 5.9 Hz), 4.06 (1H, dd, J = 11.7, 2.9 Hz), 3.69 (1H, d, J = 11.7 Hz), 2.77 (2H, t, J = 5.4 Hz), 2.37 (6H, s), 2.07-1.91 (2H, m), 1.81-1.72 (1H, m), 1.60-1.50 (4H, m), 1.33 (6H, s). | | |
| 601 | 1H-NMR (CDCl3) δ: 9.05 (1H, s), 8.59 (1H, s), 8.32 (1H, d, J = 9.8 Hz), 7.09 (1H, d, J = 9.8 Hz), 6.70 (1H, s), 6.20 (1H, d, J = 6.8 Hz), 4.80 (1H, q, J = 6.2 Hz), 4.42-4.35 (1H, m), 3.71-3.59 (4H, m), 2.37 (3H, s), 1.73-1.60 (4H, m), 1.53 (3H, d, J = 5.9 Hz), 1.36 (6H, d, J = 6.8 Hz), 1.17 (3H, s). | | |

**[Table 5-115]**

| Compound No. | NMR | LC/MS (M+H)⁺ | Exact Mass |
|---|---|---|---|
| 602 | 1H-NMR (CDCl3) δ: 9.03 (1H, s), 8.42 (1H, s), 8.36 (1H, d, J = 9.8 Hz), 7.07 (1H, d, J = 9.8 Hz), 6.69 (1H, s), 6.21 (1H, d, J = 6.8 Hz), 4.83-4.75 (1H, m), 4.43-4.33 (1H, m), 4.11 (1H, d, J = 4.9 Hz), 3.64 (4H, t, J = 4.9 Hz), 2.83-2.75 (1H, m), 2.49 (4H, t, J = 4.9 Hz), 2.12-2.04 (2H, m), 1.99-1.88 (2H, m), 1.80-1.68 (2H, m), 1.53 (3H, d, J = 5.9 Hz), 1.37 (6H, d, J = 6.8 Hz). | | |
| 603 | 1H-NMR (CDCl3) δ: 9.06 (1H, s), 8.53 (1H, s), 8.43 (1H, d, J = 8.8 Hz), 8.36 (1H, d, J = 2.9 Hz), 7.79 (1H, dd, J = 8.8, 2.9 Hz), 6.70 (1H, s), 6.24 (1H, d, J = 7.8 Hz), 4.84-4.76 (1H, m), 4.45-4.34 (1H, m), 4.10 (1H, d, J = 4.9 Hz), 3.73 (2H, t, J = 5.4 Hz), 3.47 (2H, s), 2.94 (2H, t, J = 5.4 Hz), 2.24 (2H, s), 1.54 (3H, d, J = 5.9 Hz), 1.37 (6H, d, J = 5.9 Hz), 0.95 (9H, s). | | |
| 604 | 1H-NMR (CDCl3) δ: 9.03 (1H, s), 8.40 (1H, s), 8.35 (1H, d, J = 9.8 Hz), 7.07 (1H, d, J = 9.8 Hz), 6.69 (1H, s), 6.21 (1H, d, J = 6.8 Hz), 4.80 (1H, q, J = 6.2 Hz), 4.38-4.34 (1H, m), 4.12 (1H, br s), 3.62 (4H, t, J = 4.9 Hz), 2.74 (4H, t, J = 4.9 Hz), 1.53 (3H, d, J = 6.8 Hz), 1.37 (6H, d, J = 6.8 Hz), 1.13 (9H, s). | | |
| 605 | 1H-NMR (DMSO-D6) δ: 11.69 (1H, s), 10.28 (1H, s), 9.24 (1H, s), 8.30 (2H, t, J = 9.3 Hz), 8.16 (2H, s), 7.76 (1H, d, J = 7.8 Hz), 7.52 (2H, s), 6.96 (1H, s), 6.39 (1H, d, J = 6.8 Hz), 5.36 (0H, d, J = 5.9 Hz), 5.19 (1H, d, J = 3.9 Hz), 4.60 (1H, t, J = 4.9 Hz), 4.32-4.15 (1H, m), 3.81-3.59 (3H, m), 2.83 (1H, d, J = 13.7 Hz), 2.29 (1H, d, J = 19.5 Hz), 1.37 (3H, d, J = 5.9 Hz), 1.27 (6H, d, J = 4.9 Hz). | | |

**[Table 5-116]**

| Compound No. | NMR | LC/MS (M+H)⁺ | Exact Mass |
|---|---|---|---|
| 606 | 1H-NMR (CDCl3) δ: 9.00 (1H, s), 8.34-8.24 (2H, m), 6.70-6.64 (2H, m), 6.20 (1H, d, J = 6.8 Hz), 4.79 (1H, q, J = 6.5 Hz), 4.43-4.34 (1H, m), 4.15 (2H, s), 4.06 (2H, s), 3.24-3.14 (1H, m), 2.60-2.51 (2H, m), 2.36 (3H, s), 2.02-1.93 (2H, m), 1.53 (3H, d, J = 5.9 Hz), 1.36 (6H, d, J = 6.8 Hz). | | |
| 607 | 1H-NMR (CDCl3) δ: 9.01 (1H, s), 8.41 (1H, d, J = 9.8 Hz), 8.27 (1H, s), 7.13 (1H, d, J = 9.8 Hz), 6.69 (1H, s), 6.22 (1H, d, J = 7.8 Hz), 4.86-4.75 (1H, m), 4.61-4.33 (3H, m), 4.16-3.89 (3H, m), 3.25-3.11 (2H, m), 2.45 (1H, br s), 2.23-2.13 (1H, m), 1.79-1.50 (4H, m), 1.37 (6H, d, J = 5.9 Hz). | | |
| 608 | 1H-NMR (CDC13) δ: 9.00 (1H, s), 8.38 (1H, d, J = 9.8 Hz), 8.21 (1H, s), 7.13 (1H, d, J = 9.8 Hz), 6.69 (1H, s), 6.22 (1H, d, J = 7.8 Hz), 4.90-4.71 (2H, m), 4.45-4.33 (1H, m), 4.13-3.90 (4H, m), 3.83-3.70 (1H, m), 3.48-3.39 (1H, m), 2.19-1.88 (3H, m), 1.53 (3H, d, J = 6.8 Hz), 1.37 (6H, d, J = 6.8 Hz). | | |
| 609 | 1H-NMR (CDC13) δ: 9.00 (1H, s), 8.39 (1H, d, J = 9.8 Hz), 8.22 (1H, s), 7.11 (1H, d, J = 9.8 Hz), 6.69 (1H, s), 6.21 (1H, d, J = 7.8 Hz), 4.80 (1H, q, J = 6.5 Hz), 4.59-4.34 (3H, m), 4.17 (1H, d, J = 13.7 Hz), 3.16-3.04 (2H, m), 2.87-2.75 (1H, m), 2.51 (3H, s), 2.20-2.12 (1H, m), 1.55-1.35 (10H, m). | | |
| 610 | 1H-NMR (CDCl3) δ: 9.00 (1H, s), 8.36 (1H, d, J = 9.8 Hz), 8.20 (1H, s), 7.14 (1H, d, J = 10.7 Hz), 6.68 (1H, s), 6.21 (1H, d, J = 7.8 Hz), 5.04-4.88 (1H, m), 4.80 (1H, q, J = 6.5 Hz), 4.59-4.31 (3H, m), 3.25 (1H, dd, J = 35.1, 13.7 Hz), 3.11-3.02 (1H, m), 2.80-2.65 (1H, m), 2.53 (3H, s), 2.00-1.77 (2H, m), 1.53 (3H, d, J = 5.9 Hz), 1.37 (6H, d, J = 6.8 Hz). | | |

**[Table 5-117]**

| Compound No. | NMR | LC/MS (M+H)⁺ | Exact Mass |
|---|---|---|---|
| 611 | 1H-NMR (CDC13) δ: 9.00 (1H, s), 8.31 (1H, d, J = 9.8 Hz), 8.25 (1H, s), 6.69-6.64 (2H, m), 6.20 (1H, d, J = 7.8 Hz), 4.79 (1H, q, J = 6.2 Hz), 4.43-4.33 (1H, m), 4.16 (2H, s), 4.02 (2H, s), 2.64-2.53 (1H, m), 2.45-2.37 (2H, m), 2.17-2.06 (8H, m), 1.52 (3H, d, J = 6.8 Hz), 1.36 (6H, d, J = 6.8 Hz). | | |
| 612 | 1H-NMR (CDCl3) δ: 9.04 (1H, s), 8.51-8.38 (2H, m), 8.33 (1H, d, J = 2.0 Hz), 7.76 (1H, dd, J = 9.3, 2.4 Hz), 6.68 (1H, s), 6.25 (1H, d, J = 7.8 Hz), 4.80 (1H, q, J = 6.2 Hz), 4.06-3.95 (1H, m), 3.80-3.70 (4H, m), 3.31-3.25 (2H, m), 2.27-2.19 (2H, m), 1.81 (2H, d, J = 11.7 Hz), 1.56-1.09 (8H, m), 0.96 (3H, d, J = 6.8 Hz). | | |
| 615 | 1H-NMR (CDCl3) δ: 9.03 (1H, s), 8.37-8.32 (2H, m), 8.22 (1H, d, J = 2.9 Hz), 7.72 (1H, dd, J = 8.8, 2.9 Hz), 6.70 (1H, s), 6.24 (1H, d, J = 7.8 Hz), 4.81 (1H, br s), 4.45-4.35 (1H, m), 4.12 (1H, s), 3.71 (2H, s), 3.67 (2H, t, J = 5.4 Hz), 3.53 (2H, t, J = 5.4 Hz), 2.73-2.66 (1H, m), 2.52-2.44 (4H, m), 1.53 (3H, d, J = 6.8 Hz), 1.38 (6H, d, J = 6.8 Hz), 1.03 (6H, d, J = 6.8 Hz). | | |
| 616 | 1H-NMR (CDCl3) δ: 9.06 (1H, s), 8.63 (1H, s), 8.37 (1H, d, J = 8.8 Hz), 8.25 (1H, d, J = 2.0 Hz), 7.72 (1H, dd, J = 8.8, 2.0 Hz), 6.70 (1H, s), 6.24 (1H, d, J = 6.8 Hz), 4.81 (1H, q, J = 6.5 Hz), 4.45-4.35 (1H, m), 4.12 (1H, s), 3.74-3.55 (8H, m), 2.58-2.44 (6H, m), 1.54 (3H, d, J = 5.9 Hz), 1.38 (6H, d, J = 5.9 Hz). | | |

**[Table 5-118]**

| Compound No. | NMR | LC/MS (M+H)⁺ | Exact Mass |
|---|---|---|---|
| 617 | 1H-NMR (CDC13) δ: 9.00 (1H, s), 8.42 (1H, d, J = 9.8 Hz), 8.20 (1H, s), 7.14 (1H, d, J = 9.8 Hz), 6.69 (1H, s), 6.22 (1H, d, J = 7.8 Hz), 4.84-4.76 (1H, m), 4.46-4.34 (1H, m), 4.13-3.89 (4H, m), 3.81-3.69 (2H, m), 2.30-2.13 (2H, m), 2.08-1.90 (1H, m), 1.53 (3H, d, J = 6.8 Hz), 1.37 (6H, d, J = 5.9 Hz). | | |
| 618 | 1H-NMR (CDCl3) δ: 9.00 (1H, s), 8.40 (1H, d, J = 9.8 Hz), 8.22 (1H, s), 7.11 (1H, d, J = 9.8 Hz), 6.69 (1H, s), 6.21 (1H, d, J = 7.8 Hz), 4.80 (1H, q, J = 6.5 Hz), 4.59-4.50 (1H, m), 4.45-4.31 (1H, m), 4.28-4.15 (1H, m), 3.13-2.97 (3H, m), 2.10-2.01 (1H, m), 1.69-1.50 (7H, m), 1.37 (6H, d, J = 6.8 Hz). | | |
| 619 | 1H-NMR (CDCl3) δ: 8.99 (1H, s), 8.36 (1H, d, J = 9.8 Hz), 8.18 (1H, s), 7.13 (1H, d, J = 9.8 Hz), 6.68 (1H, s), 6.21 (1H, d, J = 7.8 Hz), 4.84-4.67 (2H, m), 4.53-4.27 (3H, m), 3.36-3.22 (1H, m), 3.14-2.97 (2H, m), 1.94-1.80 (2H, m), 1.67-1.50 (5H, m), 1.37 (6H, d, J = 5.9 Hz). | | |
| 620 | 1H-NMR (CDCl3) δ: 8.99 (1H, s), 8.40 (1H, d, J = 9.8 Hz), 8.19 (1H, s), 6.74 (1H, d, J = 9.8 Hz), 6.69 (1H, s), 6.21 (1H, d, J = 7.8 Hz), 5.64 (1H, s), 4.84-4.76 (1H, m), 4.43-4.37 (1H, m), 4.19-4.10 (4H, m), 4.05 (1H, br s), 3.71 (2H, s), 2.69 (2H, s), 1.53 (3H, d, J = 6.8 Hz), 1.37 (6H, d, J = 5.9 Hz). | | |

**[Table 5-119]**

| Compound No. | NMR | LC/MS (M+H)⁺ | Exact Mass |
|---|---|---|---|
| 621 | 1H-NMR (CDC13) δ: 9.01 (1H, s), 8.40 (1H, d, J = 9.8 Hz), 8.21 (1H, s), 7.11 (1H, d, J = 9.8 Hz), 6.69 (1H, s), 6.21 (1H, d, J = 6.8 Hz), 4.84-4.62 (2H, m), 4.57-4.47 (1H, m), 4.45-4.35 (1H, m), 4.24 (1H, d, J = 12.7 Hz), 4.06 (1H, br s), 3.14-2.98 (2H, m), 2.83-2.72 (1H, m), 2.43 (6H, s), 2.02-1.93 (1H, m), 1.72-1.61 (1H, m), 1.53 (3H, d, J = 5.9 Hz), 1.37 (6H, d, J = 6.8 Hz). | | |
| 622 | 1H-NMR (CDCl3) δ: 8.99 (1H, s), 8.36 (1H, d, J = 9.8 Hz), 8.15 (1H, s), 7.15 (1H, d, J = 9.8 Hz), 6.68 (1H, s), 6.21 (1H, d, J = 6.8 Hz), 5.17-5.02 (1H, m), 4.84-4.76 (1H, m), 4.65-4.49 (2H, m), 4.44-4.35 (1H, m), 4.08 (1H, br s), 3.18-2.92 (2H, m), 2.48-2.32 (7H, m), 2.18-2.05 (1H, m), 1.90 (1H, d, J = 9.8 Hz), 1.53 (3H, d, J = 6.8 Hz), 1.37 (6H, d, J = 5.9 Hz). | | |
| 623 | 1H-NMR (CDCl3) δ: 9.07 (1H, s), 8.71 (1H, s), 8.43 (1H, d, J = 8.8 Hz), 8.25 (1H, d, J = 2.9 Hz), 7.63 (1H, dd, J = 8.8, 2.0 Hz), 7.27 (1H, s), 6.71 (1H, s), 6.24 (1H, d, J = 6.8 Hz), 4.81 (1H, dd, J = 12.7, 6.8 Hz), 4.41 (1H, td, J = 13.2, 6.5 Hz), 3.93 (3H, s), 3.84 (1H, t, J = 2.4 Hz), 3.19 (5H, dd, J = 14.1, 6.3 Hz), 2.92 (1H, d, J = 29.3 Hz), 2.40 (2H, t, J = 6.3 Hz), 2.10 (3H, ddd, J = 26.6, 17.8, 11.0 Hz), 1.54 (3H, d, J = 6.8 Hz), 1.38 (6H, d, J = 6.8 Hz). | | |
| 625 | 1H-NMR (CDCl3) δ: 9.01 (1H, s), 8.37 (1H, d, J = 8.8 Hz), 8.30 (1H, s), 7.08 (1H, d, J = 9.8 Hz), 6.69 (1H, s), 6.21 (1H, d, J = 7.8 Hz), 4.80 (1H, q, J = 5.9 Hz), 4.40 (1H, dd, J = 13.2, 6.3 Hz), 4.08 (1H, s), 3.63 (4H, br s), 2.82 (4H, br s), 2.42 (2H, s), 1.70 (1H, br s), 1.53 (3H, d, J = 5.9 Hz), 1.37 (6H, d, J = 5.9 Hz), 1.22 (6H, s). | | |

**[Table 5-120]**

| Compound No. | NMR | LC/MS (M+H)⁺ | Exact Mass |
|---|---|---|---|
| 626 | 1H-NMR (CDCl3) δ: 9.07 (1H, s), 8.64 (1H, s), 8.35 (1H, d, J = 8.8 Hz), 8.30 (1H, d, J = 2.0 Hz), 7.75 (1H, dd, J = 8.8, 2.0 Hz), 6.70 (1H, s), 6.26 (1H, d, J = 7.8 Hz), 4.81 (1H, q, J = 6.2 Hz), 4.41 (1H, td, J = 13.4, 6.5 Hz), 4.13 (1H, s), 3.51 (2H, s), 2.68 (4H, br s), 2.51 (4H, br s), 2.34 (2H, s), 1.54 (3H, d, J = 5.9 Hz), 1.39 (6H, d, J = 6.8 Hz), 1.16 (6H, s). | | |
| 627 | 1H-NMR (CDCl3) δ: 9.03 (1H, s), 8.43 (1H, d, J = 8.8 Hz), 8.32 (1H, d, J = 2.9 Hz), 8.24 (1H, s), 7.78 (1H, dd, J = 8.8, 2.9 Hz), 6.70 (1H, s), 6.24 (1H, d, J = 7.8 Hz), 4.81 (1H, s), 4.41 (1H, q, J = 5.9 Hz), 4.10 (1H, s), 3.76 (2H, t, J = 5.4 Hz), 3.55 (2H, s), 3.08 (2H, t, J = 5.4 Hz), 2.50 (2H, s), 2.43 (1H, s), 1.53 (3H, d, J = 5.9 Hz), 1.37 (6H, d, J = 5.9 Hz), 1.26 (6H, s). | | |
| 628 | 1H-NMR (CDCl3) δ: 9.02 (1H, s), 8.37 (1H, s), 8.24 (1H, d, J = 8.8 Hz), 8.12 (1H, d, J = 2.9 Hz), 7.36 (1H, dd, J = 8.8, 2.9 Hz), 6.70 (1H, s), 6.29 (1H, d, J = 7.8 Hz), 4.80 (1H, q, J = 6.5 Hz), 4.16-4.03 (4H, m), 3.40 (3H, s), 3.30-3.22 (1H, m), 2.77 (2H, t, J = 5.9 Hz), 2.37 (6H, s), 2.29 (2H, d, J = 8.8 Hz), 2.14 (2H, t, J = 5.9 Hz), 1.54-1.35 (7H, m). | | |
| 629 | 1H-NMR (CDC13) δ: 9.04 (1H, s), 8.54 (1H, s), 8.31 (1H, d, J = 8.8 Hz), 8.13 (1H, d, J = 2.9 Hz), 7.37 (1H, dd, J = 8.8, 2.9 Hz), 6.73 (1H, s), 6.69 (1H, d, J = 8.8 Hz), 4.80 (1H, q, J = 6.5 Hz), 4.37-4.31 (1H, m), 4.13 (2H, t, J = 5.9 Hz), 4.01 (1H, dd, J = 11.7, 2.9 Hz), 3.77 (2H, t, J = 4.9 Hz), 3.64 (1H, dd, J = 11.7, 5.9 Hz), 2.77 (2H, t, J = 5.4 Hz), 2.37 (6H, s), 2.07-2.03 (1H, m), 1.95-1.85 (2H, m), 1.75-1.65 (1H, m), 1.53 (3H, d, J = 6.8 Hz). | | |

**[Table 5-121]**

| Compound No. | NMR | LC/MS (M+H)⁺ | Exact Mass |
|---|---|---|---|
| 630 | 1H-NMR (CDCl3) δ: 9.09 (1H, s), 8.98 (1H, s), 8.41 (1H, d, J = 8.8 Hz), 8.32 (1H, d, J = 2.9 Hz), 7.70 (1H, dd, J = 8.8, 2.9 Hz), 6.70 (1H, s), 6.22 (1H, d, J = 7.8 Hz), 4.81 (1H, q, J = 6.5 Hz), 4.45-4.35 (1H, m), 3.83-3.77 (1H, m), 3.70-3.65 (1H, m), 3.30-3.25 (1H, m), 2.97-2.87 (1H, m), 2.23-2.16 (1H, m), 2.12-1.96 (4H, m), 1.96-1.82 (3H, m), 1.54 (3H, d, J = 6.8 Hz), 1.39 (6H, d, J = 6.0 Hz). | | |
| 631 | 1H-NMR (CDCl3) δ: 9.06 (1H, s), 8.55 (1H, s), 8.40 (1H, d, J = 8.8 Hz), 8.26 (1H, d, J = 2.9 Hz), 7.66 (1H, dd, J = 8.8, 2.9 Hz), 6.70 (1H, s), 6.24 (1H, d, J = 7.8 Hz), 4.81 (1H, q, J = 6.2 Hz), 4.45-4.36 (1H, m), 4.11 (1H, br s), 3.91 (1H, br s), 3.04 (1H, s), 2.90 (6H, br s), 1.70 (1H, s), 1.53 (3H, d, J = 6.8 Hz), 1.38 (6H, d, J = 5.9 Hz), 1.13 (6H, br s). | | |
| 632 | 1H-NMR (CDCl3) δ: 9.04 (1H, s), 8.39 (1H, d, J = 8.8 Hz), 8.35 (1H, s), 8.23 (1H, d, J = 2.0 Hz), 7.66 (1H, dd, J = 8.8, 2.0 Hz), 6.70 (1H, s), 6.24 (1H, d, J = 6.8 Hz), 4.81 (1H, q, J = 5.9 Hz), 4.45-4.35 (1H, m), 4.10 (1H, s), 3.87-3.84 (2H, m), 2.90-2.85 (2H, m), 2.78-2.70 (4H, m), 2.22 (2H, d, J = 7.8 Hz), 1.84-1.77 (1H, m), 1.53 (3H, d, J = 5.9 Hz), 1.38 (6H, d, J = 6.8 Hz), 0.93 (6H, d, J = 6.8 Hz). | | |
| 633 | 1H-NMR (CDC13) δ: 9.05 (1H, s), 8.50 (1H, s), 8.33 (1H, d, J = 8.8 Hz), 8.29 (1H, d, J = 2.0 Hz), 7.75 (1H, dd, J = 8.8, 2.0 Hz), 6.70 (1H, s), 6.26 (1H, d, J = 7.8 Hz), 4.81 (1H, q, J = 6.5 Hz), 4.45-4.35 (1H, m), 4.13 (1H, s), 3.52 (2H, s), 2.70-2.50 (9H, m), 1.54 (3H, d, J = 6.8 Hz), 1.38 (6H, d, J = 5.9 Hz), 1.07 (6H, d, J = 6.8 Hz). | | |

**[Table 5-122]**

| Compound No. | NMR | LC/MS (M+H)⁺ | Exact Mass |
|---|---|---|---|
| 634 | 1H-NMR (CDCl3) δ: 9.04 (1H, s), 8.58 (1H, s), 8.34 (1H, d, J = 8.8 Hz), 8.12 (1H, d, J = 2.9 Hz), 7.36 (1H, dd, J = 8.8, 2.9 Hz), 6.81 (1H, d, J = 5.9 Hz), 6.71 (1H, d, J = 2.0 Hz), 4.79 (1H, q, J = 6.2 Hz), 4.28 (1H, br s), 4.13 (2H, t, J = 5.4 Hz), 4.07 (1H, dd, J = 11.7, 2.9 Hz), 3.69 (1H, t, J = 5.9 Hz), 3.01 (2H, t, J = 5.4 Hz), 2.54 (3H, s), 2.05-1.98 (1H, m), 1.97-1.90 (1H, m), 1.81-1.73 (1H, m), 1.58-1.51 (4H, m), 1.33 (6H, s). | | |
| 635 | 1H-NMR (CDCl3) δ: 9.04 (1H, s), 8.68 (1H, s), 8.30 (1H, d, J = 8.8 Hz), 8.12 (1H, d, J = 2.9 Hz), 7.33 (1H, dd, J = 8.8, 2.9 Hz), 6.73 (1H, s), 6.67 (1H, d, J = 7.8 Hz), 4.80 (1H, q, J = 6.5 Hz), 4.33 (1H, br s), 4.14 (2H, t, J = 5.4 Hz), 4.02 (1H, dd, J = 10.7, 2.9 Hz), 3.77 (2H, t, J = 4.9 Hz), 3.64 (1H, dd, J = 11.2, 5.4 Hz), 3.01 (2H, t, J = 4.9 Hz), 2.54 (3H, s), 2.08-2.04 (1H, m), 1.93-1.86 (2H, m), 1.73-1.68 (1H, m), 1.53 (3H, d, J = 5.9 Hz). | | |
| 636 | 1H-NMR (CDCl3) δ: 9.05 (1H, s), 8.44 (2H, d, J = 8.8 Hz), 8.33 (1H, d, J = 2.0 Hz), 7.77 (1H, dd, J = 8.8, 2.0 Hz), 6.71 (1H, s), 6.25 (1H, d, J = 7.8 Hz), 4.81 (1H, q, J = 5.9 Hz), 4.45-4.35 (1H, m), 3.98-3.91 (1H, m), 3.83-3.81 (2H, m), 3.54-3.48 (1H, m), 3.11-3.05 (2H, m), 2.90-2.84 (1H, m), 2.50-2.43 (2H, m), 1.54 (3H, d, J = 6.8 Hz), 1.38 (6H, d, J = 6.8 Hz), 1.21 (3H, d, J = 5.9 Hz). | | |

**[Table 5-123]**

| Compound No. | NMR | LC/MS (M+H)⁺ | Exact Mass |
|---|---|---|---|
| 637 | 1H-NMR (CDCl3) δ: 9.00 (1H, s), 8.38 (1H, d, J = 9.8 Hz), 8.21 (1H, s), 7.08 (1H, d, J = 9.8 Hz), 6.69 (1H, s), 6.22 (1H, d, J = 7.8 Hz), 4.80 (1H, q, J = 6.2 Hz), 4.44-4.35 (1H, m), 3.95-3.90 (1H, m), 3.69-3.59 (4H, m), 2.87-2.82 (2H, m), 2.61-2.55 (2H, m), 2.43-2.32 (2H, m), 1.53 (3H, d, J = 6.8 Hz), 1.37 (6H, d, J = 6.8 Hz), 1.18 (3H, d, J = 6.8 Hz). | | |
| 638 | 1H-NMR (CDCl3) δ: 9.05 (1H, s), 8.50 (1H, s), 8.34 (1H, d, J = 8.8 Hz), 8.28 (1H, s), 7.75 (1H, dd, J = 8.8, 2.0 Hz), 6.70 (1H, s), 6.26 (1H, d, J = 7.8 Hz), 4.81 (1H, q, J = 6.5 Hz), 4.45-4.35 (1H, m), 4.12 (1H, br s), 3.86-3.81 (1H, m), 3.51 (2H, s), 2.72 (2H, br s), 2.51 (5H, s), 2.35-2.22 (3H, m), 1.54 (3H, d, J = 6.8 Hz), 1.38 (6H, d, J = 6.8 Hz), 1.13 (3H, d, J = 5.9 Hz). | | |
| 639 | 1H-NMR (CDC13) δ: 9.07 (1H, s), 8.62 (1H, s), 8.43 (1H, d, J = 9.8 Hz), 7.09 (1H, d, J = 9.8 Hz), 6.75 (2H, t, J = 6.8 Hz), 4.79 (1H, q, J = 6.2 Hz), 4.26 (1H, br s), 4.06 (1H, td, J = 7.3, 3.9 Hz), 3.92 (1H, br s), 3.68-3.65 (5H, m), 2.58 (4H, t, J = 4.9 Hz), 2.37 (3H, s), 2.05-1.84 (2H, m), 1.77-1.70 (1H, m), 1.57-1.51 (4H, m), 1.32 (3H, s), 1.31 (3H, s). | | |
| 640 | 1H-NMR (CDCl3) δ: 9.07 (1H, s), 8.65 (1H, s), 8.40 (1H, d, J = 9.8 Hz), 7.07 (1H, d, J = 9.8 Hz), 6.75 (1H, s), 6.63 (1H, d, J = 7.8 Hz), 4.79 (1H, q, J = 6.2 Hz), 4.32 (1H, br s), 4.01 (1H, dd, J = 10.7, 2.9 Hz), 3.76 (2H, t, J = 4.9 Hz), 3.65-3.61 (5H, m), 2.58 (4H, t, J = 4.9 Hz), 2.37 (3H, s), 2.07-2.01 (1H, m), 1.91-1.85 (2H, m), 1.72-1.65 (1H, m), 1.53 (3H, d, J = 6.8 Hz). | | |

**[Table 5-124]**

| Compound No. | NMR | LC/MS (M+H)⁺ | Exact Mass |
|---|---|---|---|
| 641 | 1H-NMR (CDCl3) δ: 9.06 (1H, s), 8.59 (1H, s), 8.35 (1H, d, J = 9.8 Hz), 7.07 (1H, d, J = 9.8 Hz), 6.72 (1H, s), 6.24 (1H, d, J = 7.8 Hz), 4.80 (1H, q, J = 6.5 Hz), 4.08-4.03 (2H, m), 3.64 (4H, t, J = 4.9 Hz), 3.39 (3H, s), 3.29-3.21 (1H, m), 2.58 (4H, t, J = 4.9 Hz), 2.38 (3H, s), 2.30-2.25 (2H, m), 2.15-2.12 (2H, m), 1.54-1.34 (7H, m). | | |
| 642 | 1H-NMR (CDCl3) δ: 9.32 (1H, s), 9.08 (1H, s), 8.40-8.35 (2H, m), 7.72 (1H, d, J = 7.8 Hz), 6.68 (1H, s), 6.17 (1H, d, J = 6.8 Hz), 4.80 (1H, q, J = 6.8 Hz), 4.40-4.35 (1H, m), 3.96-3.92 (1H, m), 3.89-3.80 (1H, m), 3.70-3.60 (1H, m), 3.25-2.94 (5H, m), 2.39-2.32 (1H, m), 2.19-2.10 (1H, m), 2.04-1.90 (2H, m), 1.53 (3H, d, J = 6.8 Hz), 1.36 (6H, d, J = 5.9 Hz). | | |
| 643 | 1H-NMR (CDCl3) δ: 9.05 (1H, s), 8.49 (1H, s), 8.40 (1H, d, J = 9.8 Hz), 8.24 (1H, d, J = 2.0 Hz), 7.65 (1H, dd, J = 9.8, 2.0 Hz), 6.70 (1H, s), 6.24 (1H, d, J = 6.8 Hz), 4.81 (1H, q, J = 6.2 Hz), 4.45-4.36 (1H, m), 4.10 (1H, s), 3.88 (2H, br s), 3.00 (4H, d, J = 14.6 Hz), 2.91 (2H, br s), 2.51 (2H, s), 1.54 (3H, d, J = 5.9 Hz), 1.38 (6H, d, J = 6.8 Hz), 1.22 (6H, s). | | |
| 644 | 1H-NMR (CDCl3) δ: 9.08 (1H, s), 8.84 (1H, s), 8.35 (1H, d, J = 8.8 Hz), 8.33 (1H, d, J = 2.0 Hz), 7.75 (1H, dd, J = 8.8, 2.0 Hz), 6.71 (1H, s), 6.25 (1H, d, J = 7.8 Hz), 4.81 (1H, q, J = 4.9 Hz), 4.45-4.34 (1H, m), 4.14 (1H, br s), 3.52 (2H, s), 3.40 (1H, br s), 3.31 (1H, t, J = 9.8 Hz), 2.85-2.80 (1H, m) , 2.73-2.65 (2H, m), 2.57-2.42 (5H, m), 1.54 (3H, d, J = 6.8 Hz), 1.39 (6H, d, J = 6.8 Hz), 0.92 (3H, d, J = 6.8 Hz). | | |

**[Table 5-125]**

| Compound No. | NMR | LC/MS (M+H)⁺ | Exact Mass |
|---|---|---|---|
| 645 | 1H-NMR (CDCl3) δ: 9.02 (1H, s), 8.34 (1H, d, J = 9.8 Hz), 8.18-8.11 (2H, m), 7.43 (1H, dd, J = 8.8, 2.9 Hz), 6.75-6.67 (2H, m), 4.96-4.76 (2H, m), 4.48-4.29 (2H, m), 4.07-3.98 (1H, m), 3.83-3.73 (2H, m), 3.68-3.61 (1H, m), 3.47-3.38 (1H, m), 3.28-3.18 (1H, m), 3.06-2.75 (2H, m), 2.15-1.64 (8H, m), 1.53 (3H, d, J = 6.8 Hz). | | |
| 646 | 1H-NMR (CDCl3) δ: 9.02 (1H, s), 8.30-8.23 (2H, m), 8.14 (1H, d, J = 2.9 Hz), 7.42 (1H, dd, J = 8.8, 2.9 Hz), 6.70 (1H, s), 6.29 (1H, d, J = 7.8 Hz), 4.90-4.73 (2H, m), 4.42-4.28 (1H, m), 4.18-4.01 (1H, m), 3.49-3.15 (7H, m), 2.88 (1H, dd, J = 29.8, 14.1 Hz), 2.80-2.66 (1H, m), 2.35-2.24 (2H, m), 2.20-2.08 (2H, m), 2.07-1.91 (2H, m), 1.89-1.35 (8H, m). | | |
| 647 | 1H-NMR (CDCl3) δ: 9.03 (1H, s), 8.41-8.30 (2H, m), 8.15 (1H, d, J = 2.9 Hz), 7.44 (1H, dd, J = 8.8, 2.9 Hz), 6.83 (1H, d, J = 6.8 Hz), 6.74-6.70 (1H, m), 4.90-4.72 (2H, m), 4.39-4.25 (2H, m), 4.10-4.03 (1H, m), 3.73-3.67 (1H, m), 3.43-3.34 (1H, m), 3.23-3.15 (1H, m), 2.93-2.79 (1H, m), 2.76-2.67 (1H, m), 2.08-1.50 (11H, m), 1.33 (6H, s). | | |
| 648 | 1H-NMR (CDCl3) δ: 9.00 (1H, s), 8.38 (1H, d, J = 9.8 Hz), 8.28 (1H, br s), 7.11 (1H, d, J = 9.8 Hz), 6.69 (1H, s), 6.22 (1H, d, J = 7.8 Hz), 5.73 (1H, s), 4.80 (1H, q, J = 6.2 Hz), 4.46-4.34 (1H, m), 4.07 (1H, br s), 3.78-3.70 (2H, m), 3.58-3.49 (2H, m), 3.29 (2H, s), 2.33 (2H, s), 1.88-1.79 (4H, m), 1.53 (3H, d, J = 6.8 Hz), 1. 37 (6H, d, J = 6.8 Hz). | | |

**[Table 5-126]**

| Compound No. | NMR | LC/MS (M+H) ⁺ | Exact Mass |
|---|---|---|---|
| 649 | 1H-NMR (CDCl3) δ: 9.06 (1H, s), 8.52-8.44 (2H, m), 8.35 (1H, d, J = 2.0 Hz), 7.75 (1H, dd, J = 8.8, 2.9 Hz), 6.79-6.71 (2H, m), 4.80 (1H, q, J = 6.5 Hz), 4.27 (1H, br s), 4.10-4.03 (1H, m), 3.79-3.64 (5H, m), 3.32-3.23 (2H, m), 2.08-1.49 (7H, m), 1.33 (6H, s). | | |
| 650 | 1H-NMR (CDCl3) δ: 9.05 (1H, s), 8.52-8.30 (3H, m), 7.80-7.71 (1H, m), 6.71 (1H, s), 6.27 (1H, d, J = 6.8 Hz), 4.81 (1H, q, J = 6.5 Hz), 4.16-4.01 (1H, m), 3.82-3.70 (4H, m), 3.40 (3H, s), 3.35-3.21 (3H, m), 2.32-2.23 (2H, m), 2.19-2.09 (2H, m), 1.56-1.34 (7H, m). | | |
| 651 | 1H-NMR (CDCl3) δ: 9.05-9.00 (1H, m), 8.29-8.08 (2H, m), 7.58-7.47 (1H, m), 6.70 (1H, s), 6.26 (1H, d, J = 7.8 Hz), 4.85-4.26 (6H, m), 4.02 (1H, t, J = 5.9 Hz), 3.63 (2H, t, J = 6.3 Hz), 3.05-2.94 (2H, m), 1.54 (3H, d, J = 5.9 Hz), 1.38 (6H, d, J = 5.9 Hz). | | |
| 652 | 1H-NMR (CDCl3) δ: 9.03-8.99 (1H, m), 8.27-8.20 (1H, m), 8.15-8.04 (1H, m), 7.57-7.46 (1H, m), 6.68 (1H, s), 6.26 (1H, d, J = 7.8 Hz), 4.86-3.94 (7H, m), 3.72-3.60 (2H, m), 3.03-2.94 (2H, m), 2.30-2.18 (2H, m), 1.86-1.77 (2H, m), 1.56-1.41 (4H, m), 1.40-1.10 (4H, m), 0.97 (3H, d, J = 5.9 Hz). | | |
| 653 | 1H-NMR (CDCl3) δ: 9.04-9.00 (1H, m), 8.31 (1H, d, J = 8.8 Hz), 8.20-8.09 (1H, m), 7.53-7.43 (1H, m), 6.81-6.66 (2H, m), 4.88-4.27 (6H, m), 4.02 (1H, t, J = 5.9 Hz), 3.72-3.59 (2H, m), 3.06-2.94 (2H, m), 2.01-1.90 (2H, m), 1.84-1.50 (8H, m), 1.37-1.20 (2H, m), 1.01 (3H, d, J = 5.9 Hz). | | |

**[Table 5-127]**

| Compound No. | NMR | LC/MS (M+H) ⁺ | Exact Mass |
|---|---|---|---|
| 654 | 1H-NMR (CDCl3) δ: 9.04-9.00 (1H, m), 8.25 (1H, d, J = 8.8 Hz), 8.18-8.10 (1H, m), 7.57-7.47 (1H, m), 6.69 (1H, s), 6.47-6.38 (1H, br m), 4.84-4.27 (6H, m), 4.13-3.98 (2H, m), 3.75-3.58 (2H, m), 3.06-2.94 (2H, m), 2.08-1.95 (2H, m), 1.75-1.39 (9H, m), 1.04-0.97 (6H, m). | | |
| 655 | 1H-NMR (CDCl3) δ: 9.06 (1H, s), 8.55-8.46 (1H, m), 8.31-8.16 (2H, m), 6.72 (1H, s), 6.24 (1H, d, J = 7.8 Hz), 4.90-4.76 (2H, m), 4.55-4.24 (4H, m), 4.11-3.93 (2H, m), 3.72-3.54 (2H, m), 3.05-2.92 (2H, m), 1.54 (3H, d, J = 6.8 Hz), 1.39 (6H, d, J = 6.8 Hz). | | |
| 656 | 1H-NMR (CDCl3) δ: 9.05-9.00 (1H, m), 8.40-8.06 (2H, m), 7.76-7.64 (1H, m), 6.72 (1H, s), 6.34-6.24 (1H, m), 4.95-4.60 (5H, m), 4.49-4.21 (3H, m), 3.85-3.31 (2H, m), 1.54 (3H, d, J = 5.9 Hz), 1.39 (6H, d, J = 6.8 Hz). | | |
| 657 | 1H-NMR (CD3OD) δ: 9.06 (1H, s), 8.31 (1H, d, J = 9.8 Hz), 7.39 (1H, d, J = 9.8 Hz), 6.90 (1H, s), 4.75 (1H, q, J = 6.2 Hz), 4.41-4.34 (1H, m), 3.64 (4H, br s), 2.89 (4H, br s), 1.49 (3H, d, J = 5.9 Hz), 1.33-1.32 (12H, m). | | |
| 672 | 1H-NMR (DMSO-d6) δ: 10.21 (1H, s), 9.23 (1H, s), 8.32-8.27 (2H, m), 7.81-7.78 (1H, m), 6.91 (1H, s), 6.45 (1H, d, J = 7.6 Hz), 4.82 (1H, m), 4.24 (1H, m), 4.00 (1H, m), 3.85 (1H, m), 3.69 (2H, m), 3.50 (1H, s), 3.13 (2H, m), 2.25 (1H, m), 1.96-1.88 (3H, m), 1.29 (6H, d, J = 6.4Hz). | | |

**[Table 5-128]**

| Compound No. | NMR | LC/MS (M+H) ⁺ | Exact Mass |
|---|---|---|---|
| 674 | 1H-NMR (CDCl3) δ: 9.04 (1H, s), 8.42 (1H, s), 8.34 (1H, d, J = 8.8 Hz), 8.28 (1H, d, J = 2.0 Hz), 7.75 (1H, dd, J = 8.8, 2.0 Hz), 6.70 (1H, s), 6.26 (1H, d, J = 7.8 Hz), 4.81 (1H, q, J = 6.5 Hz), 4.45-4.35 (1H, m), 3.88-3.83 (1H, m), 3.52 (2H, s), 2.72 (2H, br s), 2.53 (5H, br s), 2.37-2.25 (3H, m), 1.54 (3H, d, J = 6.8 Hz), 1.39 (6H, d, J = 6.8 Hz), 1.25 (1H, s), 1.14 (3H, d, J = 6.8 Hz). | | |
| 675 | 1H-NMR (CDCl3) δ: 9.04 (1H, s), 8.35-8.33 (2H, m), 8.27 (1H, s), 7.74 (1H, d, J = 8.8 Hz), 6.70 (1H, s), 6.26 (1H, d, J = 7.8 Hz), 4.81 (1H, q, J = 6.5 Hz), 4.45-4.36 (1H, m), 3.93-3.82 (1H, m), 3.53 (2H, s), 2.77 (2H, br s), 2.55 (5H, br s), 2.40-2.30 (3H, m), 1.54 (3H, d, J = 6.8 Hz), 1.39 (6H, d, J = 6.8 Hz), 1.14 (3H, d, J = 5.9 Hz). | | |
| 676 | 1H-NMR (CDCl3) δ: 9.04 (1H, s), 8.52 (1H, s), 8.37 (1H, d, J = 9.8 Hz), 7.08 (1H, d, J = 9.8 Hz), 6.70 (1H, s), 6.20 (1H, d, J = 7.8 Hz), 4.80 (1H, q, J = 6.5 Hz), 4.43-4.33 (1H, m), 4.11 (1H, br s), 3.96-3.88 (1H, m), 3.69-3.58 (4H, m), 2.86-2.81 (2H, m), 2.59-2.54 (2H, m), 2.41-2.31 (2H, m), 1.53 (3H, d, J = 5.9 Hz), 1.36 (6H, d, J = 5.9 Hz), 1.25 (1H, s), 1.18 (3H, d, J = 5.9 Hz). | | |
| 677 | 1H-NMR (CDCl3) δ: 9.03 (1H, s), 8.43 (1H, s), 8.38 (1H, d, J = 9.8 Hz), 7.08 (1H, d, J = 9.8 Hz), 6.70 (1H, s), 6.21 (1H, d, J = 6.8 Hz), 4.80 (1H, q, J = 6.5 Hz), 4.45-4.35 (1H, m), 4.11 (1H, br s), 3.97-3.88 (1H, m), 3.69-3.58 (4H, m), 2.86-2.81 (2H, m), 2.59-2.54 (2H, m), 2.42-2.31 (2H, m), 1.81 (1H, br s), 1.53 (3H, d, J = 6.8 Hz), 1.35 (6H, d, J = 6.8 Hz), 1.18 (3H, d, J = 5.9 Hz). | | |
| 678 | | 510.4 | 509.29 |

**[Table 5-129]**

| Compound No. | NMR | LC/MS (M+H) ⁺ | Exact Mass |
|---|---|---|---|
| 679 | | 523.4 | 522.31 |
| 680 | 1H-NMR (CDCl3) δ: 9.03 (1H, s), 8.41 (1H, d, J = 9.8 Hz), 8.33 (1H, s), 7.08 (1H, d, J = 9.8 Hz), 6.84 (1H, s), 6.09 (1H, d, J = 6.8 Hz), 4.74-4.66 (4H, m), 4.45-4.30 (2H, m), 3.67 (4H, t, J = 4.9 Hz), 3.60-3.53 (1H, m), 3.41 (3H, s), 2.50 (4H, t, J = 4.9 Hz), 1.50 (3H, d, J = 6.8 Hz), 1.35 (3H, d, J = 6.8 Hz), 1.34 (3H, d, J = 6.8 Hz). | | |
| 681 | 1H-NMR (CDCl3) δ: 9.08 (1H, s), 8.66 (1H, s), 8.37 (1H, d, J = 8.8 Hz), 8.31 (1H, d, J = 2.0 Hz), 7.74 (1H, dd, J = 8.8, 2.0 Hz), 6.85 (1H, s), 6.14 (1H, d, J = 7.8 Hz), 4.68-4.61 (4H, m), 4.46-4.32 (2H, m), 3.54-3.50 (3H, m), 3.42 (3H, s), 2.56 (4H, br s), 2.39 (4H, br s), 1.51 (3H, d, J = 5.9 Hz), 1.37 (3H, d, J = 5.9 Hz), 1.37 (3H, d, J = 5.9 Hz). | | |
| 682 | 1H-NMR (CDCl3) δ: 9.01 (1H, s), 8.38 (1H, d, J = 9.8 Hz), 8.30 (1H, s), 7.08 (1H, d, J = 9.8 Hz), 6.69 (1H, s), 6.21 (1H, d, J = 6.8 Hz), 4.83-4.76 (1H, m), 4.72-4.66 (4H, m), 4.44-4.35 (1H, m), 4.08 (1H, br s), 3.67 (4H, t, J = 4.9 Hz), 3.59-3.53 (1H, m), 2.50 (4H, t, J = 4.9 Hz), 1.53 (3H, d, J = 5.9 Hz), 1.37 (6H, d, J = 6.8 Hz). | | |
| 683 | 1H-NMR (CDCl3) δ: 9.05 (1H, s), 8.51 (1H, s), 8.34 (1H, d, J = 8.8 Hz), 8.29 (1H, d, J = 2.0 Hz), 7.74 (1H, dd, J = 8.8, 2.0 Hz), 6.70 (1H, s), 6.25 (1H, d, J = 7.8 Hz), 4.82 (1H, q, J = 5.9 Hz), 4.68-4.57 (4H, m), 4.45-4.36 (1H, m), 4.11 (1H, br s), 3.54-3.48 (3H, m), 2.55 (4H, br s), 2.39 (4H, br s), 1.54 (3H, d, J = 5.9 Hz), 1.38 (6H, d, J = 6.8 Hz). | | |
| 684 | | 496.4 | 495.27 |

**[Table 5-130]**

| Compound No. | NMR | LC/MS (M+H) ⁺ | Exact Mass |
|---|---|---|---|
| 685 | | 509.4 | 508.29 |
| 689 | 1H-NMR (CDCl3) δ: 9.01 (1H, s), 8.33 (1H, d, J = 8.8 Hz), 8.24 (1H, s), 8.10 (1H, d, J = 2.9 Hz), 7.38 (1H, dd, J = 9.3, 2.9 Hz), 6.81 (1H, d, J = 7.8 Hz), 6.70 (1H, s), 4.79 (1H, q, J = 6.3 Hz), 4.32-4.24 (1H, br m), 4.15-4.03 (3H, m), 3.92 (1H, s), 3.69 (1H, dd, J = 11.7, 4.9 Hz), 2.76 (2H, t, J = 5.4 Hz), 2.36 (6H, s), 2.08-1.87 (2H, m), 1.82-1.48 (5H, m), 1.33 (6H, s). | | |
| 690 | 1H-NMR (CDCl3) δ: 9.01 (1H, s), 8.33 (1H, d, J = 9.3 Hz), 8.19 (1H, s), 8.09 (1H, d, J = 2.9 Hz), 7.38 (1H, dd, J = 9.3, 2.9 Hz), 6.81 (1H, d, J = 7.8 Hz), 6.71 (1H, s), 4.79 (1H, q, J = 6.3 Hz), 4.32-4.22 (1H, br m), 4.17-4.02 (3H, m), 3.69 (1H, dd, J = 12.2, 3.9 Hz), 2.81 (2H, t, J = 5.4 Hz), 2.41 (6H, s), 2.08-1.72 (3H, m), 1.60-1.49 (4H, m), 1.33 (6H, s). | | |
| 691 | 1H-NMR (CDCl3) δ: 9.07 (1H, s), 8.55 (1H, s), 8.46-8.31 (2H, m), 7.76 (1H, dd, J = 8.8, 2.0 Hz), 6.73 (1H, s), 6.56-6.45 (1H, br m), 4.86-4.77 (1H, m), 4.08-3.23 (12H, m), 2.16-1.91 (2H, m), 1.81-1.22 (6H, m). | | |
| 692 | 1H-NMR (CDCl3) δ: 9.07 (1H, s), 8.51 (1H, br s), 8.43-8.33 (2H, m), 7.76 (1H, dd, J = 8.8, 2.9 Hz), 6.75 (1H, s), 6.20 (1H, d, J = 7.8 Hz), 4.82 (1H, q, J = 5.5 Hz), 4.55-4.40 (1H, m), 3.96-3.72 (6H, m), 3.28 (2H, t, J = 5.4 Hz), 2.21-2.05 (2H, m), 1.80-1.24 (11H, m). | | |
| 693 | 1H-NMR (CDCl3) δ: 9.31-8.87 (1H, m), 8.10 (1H, d, J = 8.8 Hz), 7.41-7.19 (1H, m), 6.79-6.65 (1H, m), 6.19 (1H, br s), 4.81 (1H, q, J = 6.2 Hz), 4.45-4.34 (1H, m), 3.99 (2H, br s), 3.42-3.11 (2H, m), 3.06-2.79 (2H, m), 1.58-1.31 (9H, m). | | |

**[Table 5-131]**

| Compound No. | NMR | LC/MS (M+H) ⁺ | Exact Mass |
|---|---|---|---|
| 694 | 1H-NMR (CDCl3) δ: 9.03 (1H, s), 8.42 (1H, br s), 8.15 (1H, s), 8.06 (1H, s), 6.68 (1H, s), 6.26 (1H, d, J = 7.8 Hz), 4.81 (1H, q, J = 6.2 Hz), 4.45-4.33 (1H, m), 4.03 (2H, s), 3.20 (2H, t, J = 5.9 Hz), 2.88 (2H, t, J = 5.9 Hz), 1.53 (3H, d, J = 5.9 Hz), 1.39 (6H, d, J = 5.9 Hz). | | |
| 695 | 1H-NMR (CDCl3) δ: 9.22 (1H, br s), 8.16 (1H, d, J = 8.8 Hz), 7.45 (1H, br s), 6.77 (1H, s), 6.12 (1H, br s), 4.85 (1H, q, J = 6.2 Hz), 4.44-4.16 (5H, m), 1.56-1.32 (9H, m). | | |
| 696 | 1H-NMR (CDCl3) δ: 8.99 (1H, s), 8.35 (1H, d, J = 9.8 Hz), 8.24 (1H, br s), 6.82 (1H, s), 6.71 (1H, d, J = 8.8 Hz), 6.08 (1H, d, J = 6.8 Hz), 4.47-4.26 (4H, m), 4.19 (2H, d, J = 7.8 Hz), 3.60 (2H, t, J = 6.8 Hz), 3.40 (3H, s), 2.65 (2H, t, J = 6.8 Hz), 1.49 (3H, d, J = 6.8 Hz), 1.37-1.30 (6H, m). | | |
| 697 | | 523.4 | 522.31 |
| 698 | | 412.3 | 411.23 |
| 699 | | 510.4 | 509.29 |
| 700 | | 485.4 | 494.31 |
| 701 | | 482.4 | 481.29 |
| 702 | | 483.3 | 482.24 |
| 703 | | 517.3 | 516.20 |

**[Table 5-132]**

| Compound No. | NMR | LC/MS (M+H) ⁺ | Exact Mass |
|---|---|---|---|
| 704 | 1H-NMR (300 MHz, DMSO-d6, ppm) δ: 10.11 (1H, s), 9.16 (1H, s), 8.12(1H, d, J = 9.6Hz), 7.39 (1H, d, J =9.9Hz), 6.93 (1H, s), 6.29 (1H, d, J = 7.8Hz), 5.14 (1H, d, J = 4.5Hz), 4.64 (1H, d, J = 5.4Hz), 4.59-4.55 (1H, m), 4.24-4.11 (3H, m), 3.19-3.13 (1H, m), 2.92-2.84 (1H, m), 2.57-2.52 (1H, m), 1.86-1.81 (1H, m), 1.42 - 1.39(1H, m), 1.35 (3H, d, J = 6.6Hz), 1.25 (6H, d, J = 6.6Hz), 0.95-0.91 (3H, m). | 439.25 | 438.2 |
| 705 | 1H-NMR (300 MHz, DMSO-d6, ppm) δ: 10.11 (1H, s), 9.16 (1H, s), 8.12(1H, d, J = 9.6Hz), 7.39 (1H, d, J =9.9Hz), 6.93 (1H, s), 6.29 (1H, d, J = 7.8Hz), 5.14(1H, d, J = 4.5Hz), 4.64 (1H, d, J = 5.4Hz), 4.59-4.55 (1H, m), 4.24-4.11 (3H, m), 3.19-3.13 (1H, m), 2.92-2.84 (1H, m), 2.57-2.52 (1H, m), 1.86-1.81 (1H, m), 1.42 - 1.39(1H, m), 1.35 (3H, d, J = 6.6Hz), 1.25 (6H, d, J = 6.6Hz), 0.95-0.91 (3H, m). | 439.25 | 438.2 |
| 706 | 1H NMR (300 MHz, DMSO-d6, ppm) δ: 10.08 (1H, s), 9.26 (1H, s), 8.29 - 8.27 (2H, m), 7.81 - 7.78 (1H, m), 7.00 (1H, s), 6.43 (1H, d, J = 7.5 Hz), 5.20 (1H, d, J = 4.5 Hz), 4.64 - 4.61 (1H, m), 4.46 (1H, d, J = 10.2 Hz), 4.31 - 4.24 (1H, m), 3.93 (1H, d, J = 11.4 Hz), 3.68 - 3.62 (1H, m), 3.01 (1H, d, J = 12.0 Hz), 2.81 (2H, d, J = 5.4 H z), 2.68 - 2.50 (1H, m), 2.50 - 1.30 (9H, m). | 410.24 | 409.2 |
| 707 | 1H NMR (300 MHz, DMSO-d6, ppm) δ: 10.19 (1H, s), 9.17 (1H, s), 8.20 (1H, d, J = 9.3 Hz), 7.02 - 6.93 (2H, m), 6.30 (1H, d, J = 7.5 Hz), 5.32 - 5.28 (1H, m), 5.14 (1H, d, J = 4.2 Hz), 4.59 - 4.55 (1H, m), 4.21 - 3.98 (5H, m), 3.83 - 3.62 (2H, m), 1.36 (3H, d, J = 6.3 Hz), 1.25 - 1.02 (6H, m). | 429.25 | 428.2 |

**[Table 5-133]**

| Compound No. | NMR | LC/MS (M+H)⁺ | Exact Mass |
|---|---|---|---|
| 708 | 1H-NMR (300 MHz, DMSO-d6, ppm) δ: 9.93 (1H, s), 9.21 (1H, s), 8.18 (1H, d, J = 8.7Hz), 8.09 (1H, s), 7.56 (1H, d, J = 8.7HZ), 6.97 (1H, s), 6.36 (1H, d, J = 6.9Hz), 5.17 (1H, d, J = 4.8Hz), 4.75 (1H, br), 4.61 (1H, br), 4.26 (2H, br), 3.23 (1H, br), 2.90 (1H, br), 2.35 (2H, br), 2.09 (2H, br), 1.62 (1H, br), 1.38 (3H, d, J = 6.3Hz), 1.30 (6H, d, J = 5.7Hz) | 442.1 | 441.2 |
| 709 | 1H-NMR (300 MHz, DMSO-d6, ppm) δ: 10.33 (1H, s), 9.19 (1H, s), 8.14 (1H, d, J = 9.9Hz), 7.41 (1H, br), 6.87 (1H, s), 6.38 (1H, s), 4.25 (1H, br), 3.51 (4H, br), 3.28 (3H, s), 3.92 (4H, br), 1.52 (9H, s), 1.39 (3H, d, J = 9.3Hz) | 438.1 | 437.3 |
| 710 | 1H-NMR (300 MHz, DMSO-d6, ppm) δ: 10.31 (1H, s), 9.21 (1H, s), 8.17 (1H, d, J = 9.9Hz), 7.31 (1H, d, J = 9.6Hz), 6.86 (1H, s), 6.44 (1H, br), 4.26-4.19 (1H, m), 3.48-3.46 (4H, m), 3.40-3.31(2H, m), 3.26 (3H, s), 2.88 (4H, br), 1.38 (3H, d, J = 6.3Hz), 0.97 (9H, s) | 452.1 | 451.3 |
| 711 | 1H-NMR (400 MHz, DMSO-d6, ppm) δ: 10.24 (1H, s), 9.21 (1H, s), 8.16 (1H, d, J = 9.6Hz), 7.36 (1H, d, J = 10Hz), 6.88 (1H, s), 6.40 (1H, d, J = 7.6Hz), 4.27-4.22 (1H, m), 3.94-3.91(1H, br), 3.45-3.43 (4H, m), 3.32 (6H, s), 3.22-3.19 (1H, m), 2.84-2.82 (4H, m), 2.12-2.01 (4H, br), 1.44-1.41 (6H, m) | 494.2 | 493.3 |
| 712 | 1H-NMR (300 MHz, DMSO-d6, ppm) δ: 10.21 (1H, s), 9.21 (1H, s), 8.17 (1H, d, J = 9.6Hz), 7.36 (1H, d, J = 9.9Hz), 6.88 (1H, s), 6.49 (1H, d, J = 7.2Hz), 4.39-4.33 (1H, m), 4.28-4.22 (1H, m), 3.45-3.42(4H, m), 3.31 (3H, s), 2.84-2.81 (4H, m), 2.082.02 (2H, br), 1.74-1.72 (2H, br), 1.68-1.64 (4H, br), 1.62 (3H, d, J = 5.4Hz) | 450.2 | 449.3 |

**[Table 5-134]**

| Compound No. | NMR | LC/MS (M+H)⁺ | Exact Mass |
|---|---|---|---|
| 713 | 1H-NMR (400 MHz, DMSO-d6, ppm) δ: 10.21 (1H, s), 9.17 (1H, s), 8.16 (1H, d, J = 10Hz), 7.36 (1H, d, J = 10Hz), 6.87 (1H, s), 6.35 (1H, d, J = 7.6Hz), 6.68 (1H, br), 4.82-4.78 (1H, m), 4.22-4.20 (1H, m), 4.01-4.00 (1H, m), 3.84-3.82 (1H, m), 3.44-3.41 (4H, m), 2.83-2.80 (4H, m), 2.24 (1H, br), 1.93-1.88 (3H, m), 1.27 (6H, d, J = 6.8Hz) | 436.2 | 435.2 |
| 714 | 1H-NMR (400 MHz, DMSO-d6, ppm) δ: 10.20 (1H, s), 9.17 (1H, s), 8.16 (1H, d, J = 9.6Hz), 7.36 (1H, d, J = 10Hz), 6.87 (1H, s), 6.35 (1H, d, J = 7.6Hz), 6.68 (1H, br), 4.82-4.78 (1H, m), 4.00 (1H, br), 3.84 (1H, br), 3.43-3.41 (4H, m), 2.82-2.80 (4H, m), 2.26 (1H, br), 1.92-1.88 (3H, m), 1.27 (6H, d, J = 6.4Hz) | 436.2 | 435.2 |
| 715 | | 441.3 | 440.24 |
| 716 | | 424.3 | 423.21 |
| 717 | | 456.3 | 455.24 |
| 718 | | 412.3 | 411.23 |
| 719 | 1H-NMR (CDCl3) δ: 9.38 (1H, s), 8.56 (1H, s), 8.28 (1H, d, J = 9.8 Hz), 8.13 (1H, d, J = 2.9 Hz), 7.37 (1H, dd, J = 9.8, 2.9 Hz), 6.80 (1H, d, J = 7.8 Hz), 5.16 (1H, q, J = 6.2 Hz), 4.30 (1H, s), 4.14 (2H, t, J = 5.9 Hz), 3.97 (1H, dd, J = 11.2, 2.4 Hz), 3.81-3.66 (3H, m), 2.77 (2H, t, J = 5.4 Hz), 2.37 (6H, s), 2.02 (2H, dd, J = 8.8, 4.9 Hz), 1.90 (2H, t, J = 5.4 Hz), 1.45 (3H, d, J = 6.8 Hz) . | 488.3 | 487.21 |

**[Table 5-135]**

| Compound No. | NMR | LC/MS (M+H)⁺ | Exact Mass |
|---|---|---|---|
| 720 | 1H-NMR (CDCl3) δ: 9.09 (1H, s), 8.93 (1H, s), 8.41 (1H, d, J = 8.8 Hz), 8.29 (1H, d, J = 2.9 Hz), 7.66 (1H, dd, J = 8.8, 2.0 Hz), 6.71 (1H, s), 6.23 (1H, d, J = 7.8 Hz), 4.81 (1H, q, J = 6.5 Hz), 4.41 (1H, td, J = 13.4, 6.5 Hz), 3.96-3.85 (3H, m), 3.05-2.73 (6H, m), 2.55 (1H, dd, J = 12.7, 2.9 Hz), 2.36 (1H, t, J = 11.2 Hz), 1.54 (3H, d, J = 5.9 Hz), 1.38 (6H, d, J = 6.8 Hz), 1.18 (3H, d, J = 6.8 Hz). | 495.3 | 494.28 |
| 721 | 1H-NMR (CDCl3) δ: 9.07 (1H, s), 8.78 (1H, s), 8.41 (1H, d, J = 8.8 Hz), 8.27 (1H, d, J = 2.9 Hz), 7.66 (1H, dd, J = 8.8, 2.9 Hz), 6.71 (1H, s), 6.23 (1H, d, J = 7.8 Hz), 4.81 (1H, q, J = 6.5 Hz), 4.41 (1H, td, J = 13.4, 6.5 Hz), 4.12 (1H, q, J = 7.2 Hz), 3.96-3.85 (3H, m), 3.05-2.73 (6H, m), 2.54 (1H, dd, J = 12.7, 2.9 Hz), 2.36 (1H, t, J = 11.2 Hz), 1.54 (3H, d, J = 6.8 Hz), 1.38 (6H, d, J = 5.9 Hz), 1.18 (3H, d, J = 5.9 Hz). | 495.3 | 494.28 |
| 722 | 1H NMR (300 MHz, DMSO-d6, ppm) δ: 10.21 (1H, s), 9.21 (1H, s), 8.22 (1H, d, J = 9.6 Hz), 7.49 (1H, d, J = 9.9 Hz), 6.98 (1H, s), 6.33 (1H, d, J = 7.2 Hz), 5.17 (1H, d, J = 4.5 Hz), 4.63 - 4.60 (1H, m), 4.39 - 4.20 (3H, m), 3.41 (2H, s), 3.03 - 2.95 (2H, m), 2.04 - 1.91 (2H, m), 1.68 - 1.55 (2H, m), 1.53 - 1.38 (3H, m), 1.30 - 1.22 (12H, m). | 466.2 | 465.30 |
| 723 | 1H-NMR (400 MHz, DMSO-d6, ppm) δ: 10.20 (1H, s), 9.26 (1H, s), 8.35 (1H, d, J = 8.8Hz), 8.14 (1H, d, J = 2.4HZ), 7.70 (1H, dd, J1 = 8.8 Hz, J2 = 2.4 Hz), 6.99 (1H, s), 6.44 (1H, d, J = 8.4 Hz), 5.20 (1H, d, J = 4.8 Hz), 4.61(1H, br), 4.25 (1H, br), 3.91 (1H, br), 3.38 (2H, d, J = 9.6 Hz), 3.31 (1H, br), 2.66 (1H, br), 1.39 (3H, d, J = 6.4 Hz), 1.30-1.28 (6H, m), 1.04 (3H, d, J = 6.4Hz) | 451.1 | 450.25 |

**[Table 5-136]**

| Compound No. | NMR | LC/MS (M+H)⁺ | Exact Mass |
|---|---|---|---|
| 724 | 1H-NMR (300 MHz, DMSO-d6, ppm) δ: 10.49 (1H, bar), 9.24 (1H, s), 8.10 (1H, d, J = 9.9Hz), 7.58 (1H, m), 7.00 (1H, s), 6.46 (1H, bar), 5.20 (1H, bar), 4.71-4.59 (2H, m), 4.29-4.22 (1H, m), 3.80-3.74 (1H, m), 3.66-3.59 (3H, m), 1.92-1.85 (1H, m), 1.66-1.58 (1H, m), 1.40-1.38 (4H, m), 1.29-1.24 (6H, m), 0.52-0.47 (2H, m), 0.40-0.36 (2H, m) | 451.4 | 450.25 |
| 725 | 1H-NMR (300 MHz, DMSO-d6, ppm) δ: 10.47 (1H, bar), 9.24 (1H, s), 8.10 (1H, d, J = 9.9Hz), 7.58 (1H, m), 7.00 (1H, s), 6.46 (1H, bar), 5.20 (1H, bar), 4.71-4.61 (2H, m), 4.29-4.22 (1H, m), 3.80-3.74 (1H, m), 3.73-3.59 (3H, m), 1.89-1.85 (1H, m), 1.66-1.63 (1H, m), 1.40-1.38 (4H, m), 1.29-1.27 (6H, m), 0.52-0.47 (2H, m), 0.40-0.36 (2H, m) | 451.1 | 450.25 |
| 726 | 1H-NMR (300 MHz, DMSO-d6, ppm) δ: 10.20 (1H, s), 9.20 (1H, s), 8.16 (1H, d, J = 9.6Hz), 7.34 (1H, d, J = 9.9Hz), 6.86 (1H, s), 6.52 (1H, d, J = 7.8Hz), 4.26-4.19 (1H, m), 3.94-3.91 (1H, br), 3.45-3.34(4H, m), 2.66 (3H, s), 2.84-2.81 (4H, m), 2.01 (2H, br), 1.89 (1H, br), 1.63-1.38 (7, m), 1.32-1.28 (6H, m) | 492.3 | 491.31 |
| 727 | 1H-NMR (300 MHz, DMSO-d6, ppm) δ: 10.27 (1H, s), 9.22 (1H, s), 8.18 (1H, d, J = 9.9Hz), 7.31 (1H, d, J = 9.6Hz), 6.89 (1H, s), 6.68 (1H, br), 4.25-4.22 (1H, m), 4.07 (1H, br), 3.87-3.84(1H, m), 3.51-3.48 (1H, m), 3.44-3.41 (4H, m), 3.26 (3H, s), 2.83-2.80 (4H, m), 1.89 (2H, br), 1.62 (1H, br), 1.52 (1H, br), 1.38 (3H, d, J = 6.6Hz), 1.21 (6H, s) | 494.2 | 493.29 |

**[Table 5-137]**

| Compound No. | NMR | LC/MS (M+H)⁺ | Exact Mass |
|---|---|---|---|
| 728 | 1H-NMR (300 MHz, DMSO-d6, ppm) δ: 10.62 (1H, s), 9.42 (1H, s), 8.56 (1H, d, J = 6.6Hz), 7.48 (1H, s), 7.44 (1H, s), 4.48-4.46 (1H, m), 4.09-4.05 (1H, m), 3.48-3.45 (4H, br), 3.30 (3H, s), 2.83 (4H, br), 1.47-1.43 (9H, m) | 441.1 | 440.21 |
| 729 | 1H-NMR (400 MHz, DMSO-d6, ppm) δ: 10.04 (1H, s), 9.22 (1H, s), 8.25 (1H, d, J = 8.4Hz), 8.18 (1H, d, J = 2Hz), 7.75-7.72 (1H, m), 6.90 (1H, s), 6.44 (1H, d, J = 7.2Hz), 4.83-4.80 (1H, m), 4.24-4.23 (1H, m), 4.02-4.00 (1H, m), 3.84-3.82(1H, m), 3.43 (2H, s), 2.76-2.74 (4H, br), 2.34 (4H, br), 2.31-2.25 (2H, m), 1.94-1.90 (3H, m), 1.29 (6H, d, J = 6.4Hz) | 449.2 | 448.27 |
| 730 | 1H-NMR (400 MHz, DMSO-d6, ppm) δ: 10.04 (1H, s), 9.22 (1H, s), 8.25 (1H, d, J = 8.4Hz), 8.18 (1H, s), 7.75-7.72 (1H, m), 6.89 (1H, s), 6.44 (1H, d, J = 7.2Hz), 4.83-4.80 (1H, m), 4.24-4.23 (1H, m), 4.02-4.00 (1H, m), 3.84-3.82(1H, m), 3.42 (2H, s), 2.74-.2.72 (4H, br), 2.32 (4H, br), 2.31-2.20 (2H, m), 1.94-1.85 (3H, m), 1.29 (6H, d, J = 6.4Hz) | 449.2 | 448.27 |
| 731 | 1H-NMR (300 MHz, DMSO-d6, ppm) δ: 10.22 (1H, s), 9.27 (1H, s), 8.43-8.34 (2H, m), 7.94-7.90 (1H, m), 7.74 (1H, s), 7.01 (1H, s), 6.44 (1H, d, J = 7.5 Hz), 5.20 (1H, d, J = 3.6 Hz), 4.65-4.61 (1H, m), 4.29-4.27 (1H, m), 3.89-3.86 (3H, m), 1.47-1.39 (3H, m), 1.36-1.30 (6H, m) | 421.1 | 420.20 |

**[Table 5-138]**

| Compound No. | NMR | LC/MS (M+H)⁺ | Exact Mass |
|---|---|---|---|
| 732 | 1H-NMR (CDCl3) δ: 9.07 (1H, s), 8.71 (1H, s), 8.43 (1H, d, J = 8.8 Hz), 8.25 (1H, d, J = 2.9 Hz), 7.63 (1H, dd, J = 8.8, 2.0 Hz), 6.71 (1H, s), 6.24 (1H, d, J = 6.8 Hz), 4.81 (1H, t, J = 12.7 Hz), 4.48-4.35 (1H, m), 3.93 (2H, s), 3.84 (1H, t, J = 2.4 Hz), 3.19 (4H, dd, J = 14.1, 6.3 Hz), 2.92 (1H, d, J = 29.3 Hz), 2.40 (2H, t, J = 6.3 Hz), 2.17-2.03 (2H, m), 2.00-1.80 (2H, br m), 1.54 (3H, d, J = 6.8 Hz), 1.38 (6H, d, J = 6.8 Hz). | | 464.26 |
| 733 | 1H-NMR (DMSO-D6) δ: 10.24 (1H, s), 9.31 (1H, s), 8.36 (2H, dd, J = 9.8, 5.9 Hz), 7.86 (1H, dd, J = 8.8, 2.9 Hz), 7.05 (1H, s), 6.47 (1H, d, J = 6.8 Hz), 5.26 (1H, d, J = 3.9 Hz), 4.70-4.64 (1H, m), 4.38-4.28 (1H, m), 3.86 (2H, t, J = 4.9 Hz), 3.16 (2H, t, J = 5.4 Hz), 1.44 (3H, d, J = 6.8 Hz), 1.35 (6H, dd, J = 5.9, 2.0 Hz), 1.23 (2H, q, J = 3.3 Hz), 0.84 (2H, dd, J = 6.8, 3.9 Hz). | | 448.23 |
| 734 | 1H-NMR (300 MHz, DMSO-d6, ppm) δ: 10.28 (1H, s), 9.38 (1H, s), 8.17 (1H, d, J = 9.3Hz), 8.09 (1H, d, J = 3Hz), 7.61 (1H, s), 7.55-7.51(1H, m), 6.54 (1H, d, J = 6.9Hz), 4.37-4.30 (1H, m), 4.16-4.12 (2H, m), 2.64(2H, br),2.61 (3H, s), 2.24 (6H, s), 1.36 (3H, d, J = 6.6Hz) | 410.1 | 409.22 |
| 735 | | 410.2 | 409.22 |
| 736 | | 410.3 | 409.22 |
| 737 | 1H-NMR (CDCl3) δ: 9.02 (1H, s), 8.40 (1H, s), 8.26 (1H, d, J = 8.8 Hz), 8.09 (1H, d, J = 2.4 Hz), 7.34 (1H, dd, J = 8.8, 2.9 Hz), 6.68 (1H, s), 6.24 (1H, d, J = 7.8 Hz), 4.80 (1H, q, J = 6.5 Hz), 4.44-4.35 (1H, m), 4.25-4.16 (1H, m), 2.57-2.46 (4H, m), 2.24-2.04 (4H, m), 1.61-1.47 (5H, m), 1.40-1.21 (8H, m). | 452.3 | 451.27 |

**[Table 5-139]**

| Compound No. | NMR | LC/MS (M+H)⁺ | Exact Mass |
|---|---|---|---|
| 738 | 1H-NMR (CDCl3) δ: 9.01 (1H, s), 8.31 (1H, s), 8.25 (1H, d, J = 9.3 Hz), 8.09 (1H, d, J = 2.9 Hz), 7.35 (1H, dd, J = 9.3, 2.9 Hz), 6.68 (1H, s), 6.24 (1H, d, J = 7.3 Hz), 4.80 (1H, q, J = 6.3 Hz), 4.49 (1H, s), 4.44-4.34 (1H, m), 2.58-2.47 (4H, m), 2.13-2.03 (2H, m), 1.82-1.60 (6H, m), 1.53 (3H, d, J = 6.3 Hz), 1.38 (6H, d, J = 6.3 Hz). | 452.3 | 451.27 |
| 739 | 1H-NMR (CDCl3) δ: 9.00 (1H, s), 8.27-8.22 (2H, m), 8.07 (1H, d, J = 2.9 Hz), 7.34 (1H, dd, J = 9.3, 2.9 Hz), 6.68 (1H, s), 6.24 (1H, d, J = 7.3 Hz), 4.80 (1H, q, J = 6.3 Hz), 4.52-4.46 (1H, m), 4.44-4.34 (1H, m), 3.23-3.17 (1H, m), 2.50 (3H, s), 2.17-2.06 (2H, m), 1.86-1.75 (3H, m), 1.55-1.42 (4H, m), 1.38 (6H, d, J = 6.3 Hz). | 438.3 | 437.25 |
| 740 | 1H-NMR (CDCl3) δ: 9.01 (1H, s), 8.33 (1H, s), 8.25 (1H, d, J = 9.3 Hz), 8.08 (1H, d, J = 2.9 Hz), 7.34 (1H, dd, J = 9.3, 2.9 Hz), 6.68 (1H, s), 6.24 (1H, d, J = 7.8 Hz), 4.80 (1H, q, J = 6.3 Hz), 4.45-4.35 (1H, m), 4.20-4.09 (2H, m), 2.32-2.18 (9H, m), 2.04-1.96 (2H, m), 1.55-1.45 (5H, m), 1.42-1.31 (8H, m). | 466.3 | 465.29 |
| 741 | 1H-NMR (CDCl3) δ: 9.00 (1H, s), 8.25 (1H, d, J = 9.3 Hz), 8.19 (1H, s), 8.08 (1H, d, J = 2.9 Hz), 7.36 (1H, dd, J = 9.3, 2.9 Hz), 6.68 (1H, s), 6.25 (1H, d, J = 7.3 Hz), 4.80 (1H, q, J = 6.3 Hz), 4.49 (1H, br s), 4.45-4.34 (1H, m), 4.13 (1H, s), 2.34-2.19 (7H, m), 2.18-2.10 (2H, m), 1.78-1.52 (9H, m), 1.38 (6H, d, J = 6.3 Hz). | 466.3 | 465.29 |

**[Table 5-140]**

| Compound No. | NMR | LC/MS (M+H)⁺ | Exact Mass |
|---|---|---|---|
| 742 | 1H-NMR (CDCl3) δ: 8.99 (1H, s), 8.24 (1H, d, J = 9.3 Hz), 8.14 (1H, s), 8.05 (1H, d, J = 2.9 Hz), 7.33 (1H, dd, J = 9.3, 2.9 Hz), 6.67 (1H, s), 6.25 (1H, d, J = 7.8 Hz), 4.80 (1H, br s), 4.61-4.55 (1H, m), 4.44-4.34 (1H, m), 4.13 (1H, br s), 2.85-2.77 (1H, m), 2.31 (6H, s), 2.11-1.98 (2H, m), 1.86-1.71 (3H, m), 1.60-1.51 (4H, m), 1.38 (6H, d, J = 6.3 Hz). | 452.3 | 451.27 |
| 743 | 1H-NMR (300 MHz, DMSO-d6, ppm) δ: 10.53 (1H, s), 9.43 (1H, s), 8.34 (2H, dr), 7.85 (1H, d, J = 8.1Hz), 7.63 (1H, s), 6.63 (1H, d, J = 6.9Hz), 4.38-4.32 (1H, m), 3.67 (2H, br),3.44 (2H, s), 3.06 (2H, br), 2.61 (3H, s), 1.37 (6H, d, J = 6.3Hz) | 421.1 | 420.20 |
| 744 | 1H-NMR (300 MHz, DMSO-d6, ppm) δ: 10.54 (1H, s), 9.47 (1H, s), 8.69 (1H, d, J = 8.7Hz), 8.34 (1H, d, J = 2.1Hz), 7.90-7.86 (1H, m), 7.59 (1H, s), 5.48 (1H, d, J = 4.8Hz), 4.86-4.83 (1H, m), 4.12-4.07 (1H, m), 3.69-3.66 (2H, m), 3.42(2H, s), 3.06-3.02 (2H, m), 1.48-1.45 (9H, m) | 440.4 | 439.18 |
| 745 | 1H-NMR (CDCl3) δ: 9.00 (1H, s), 8.43 (1H, d, J = 9.8 Hz), 8.13 (1H, s), 7.11 (1H, d, J = 9.8 Hz), 6.69 (1H, s), 6.22 (1H, d, J = 7.8 Hz), 4.83-4.77 (1H, m), 4.44-4.36 (1H, m), 4.05-4.04 (1H, m), 3.82-3.79 (2H, m), 3.75-3.73 (2H, m), 3.66-3.64 (2H, m), 3.55-3.53 (2H, m), 2.17 (3H, s), 1.53 (3H, d, J = 5.9 Hz), 1.37 (6H, d, J = 6.8 Hz). | 452.3 | 451.24 |
| 746 | 1H-NMR (CDCl3) δ: 9.00 (1H, s), 8.43 (1H, d, J = 9.8 Hz), 8.14 (1H, s), 7.11 (1H, d, J = 9.8 Hz), 6.69 (1H, s), 6.22 (1H, d, J = 7.8 Hz), 4.84-4.76 (1H, m), 4.45-4.35 (1H, m), 4.05 (1H, d, J = 4.9 Hz), 3.81 (2H, br s), 3.77-3.67 (4H, m), 3.53 (2H, br s), 2.92-2.81 (1H, m), 1.53 (3H, d, J = 5.9 Hz), 1.37 (6H, d, J = 5.9 Hz), 1.18 (6H, d, J = 6.8 Hz). | 480.3 | 479.28 |

**[Table 5-141]**

| Compound No. | NMR | LC/MS (M+H) ⁺ | Exact Mass |
|---|---|---|---|
| 747 | 1H-NMR (CDCl3) δ: 9.00 (1H, s),8.45 (1H, d, J = 9.8 Hz), 8.12 (1H, s), 7.11 (1H, d, J = 9.8 Hz), 6.84 (1H, s), 6.09 (1H, d, J = 7.8 Hz), 4.45-4.38 (1H, m), 4.37-4.30 (1H, m), 3.81 (2H, br s), 3.76-3.68 (4H, br m), 3.53 (2H, s), 3.41 (3H, s), 2.90-2.83 (1H, m), 1.50 (3H, d, J = 5.9 Hz), 1.35 (6H, t, J = 5.9 Hz), 1.18 (6H, d, J = 6.8 Hz). | 494.3 | 493.29 |
| 748 | 1H-NMR (CDCl3) δ: 9.00 (1H, s), 8.43 (1H, d, J = 9.8 Hz), 8.10 (1H, s), 7.09 (1H, d, J = 9.8 Hz), 6.83 (1H, s), 6.09 (1H, d, J = 7.8 Hz), 5.00-4.94 (1H, m), 4.45-4.37 (1H, m), 4.36-4.29 (1H, m), 3.68-3.56 (8H, m), 3.41 (3H, s), 1.50 (3H, d, J = 6.8 Hz), 1.35 (6H, t, J = 5.9 Hz), 1.28 (6H, d, J = 5.9 Hz). | 510.3 | 509.29 |
| 749 | 1H-NMR (CDCl3) δ: 9.07 (1H, s), 8.56 (1H, d, J = 9.8 Hz), 8.26 (1H, d, J = 7.8 Hz), 7.55 (1H, d, J = 8.8 Hz), 6.69 (1H, s), 6.23 (1H, d, J = 7.8 Hz), 4.81 (1H, q, J = 6.2 Hz), 4.39 (1H, td, J = 13.2, 6.2 Hz), 3.79-3.49 (5H,m), 3.26 (2H, dd, J = 9.8, 4.9 Hz) , 2.42 (3H, s), 1.53 (3H, d, J = 5.9 Hz), 1.37 (6H, d, J = 6.8 Hz). | 437.3 | 436.23 |
| 750 | 1H-NMR (CDCl3) δ: 9.47 (1H, s), 9.15 (1H, s), 8.36 (2H, q, J = 8.8 Hz), 7.69 (1H, d, J = 8.8 Hz), 6.29 (1H, d, J = 6.8 Hz), 5.16 (1H, d, J = 5.9 Hz), 4.36 (1H, d, J = 5.9 Hz), 3.96-3.75 (4H, m), 3.21 (2H, s), 1.96 (3H, s), 1.46 (3H, d, J = 5.9 Hz), 1.38 (6H, d, J = 4.9 Hz). | 471.2 | 470.19 |
| 751 | 1H-NMR (CDCl3) δ: 9.43 (1H, s), 8.71 (1H, s), 8.44 (1H, d, J = 8.8 Hz), 8.36 (1H, d, J = 2.0 Hz), 7.77 (1H, dd, J = 8.8, 2.0 Hz), 6.80 (1H, d, J = 7.8 Hz), 5.17 (1H, q, J = 6.5 Hz), 4.31 (1H, s), 3.97 (1H, dd, J = 11.2, 2.4 Hz), 3.82-3.65 (8H, m), 3.28 (2H, t, J = 5.4 Hz), 2.04-1.86 (5H, m), 1.46 (3H, d, J = 6.8 Hz). | 499.3 | 498.19 |

**[Table 5-142]**

| Compound No. | NMR | LC/MS (M+H)⁺ | Exact Mass |
|---|---|---|---|
| 752 | 1H-NMR (CDCl3) δ: 9.06 (1H, s), 8.58 (1H, s), 8.47 (1H, d, J = 8.8 Hz), 8.34 (1H, d, J = 2.0 Hz), 7.75 (1H, dd, J = 8.8, 2.0 Hz), 6.72 (1H, s), 6.67 (1H, d, J = 7.8 Hz), 4.81 (1H, q, J = 6.5 Hz), 4.49 (1H, t, J = 7.3 Hz), 3.80-3.71 (4H, m), 3.59 (2H, d, J = 3.9 Hz), 3.47 (3H, s), 3.27 (2H, t, J = 5.4 Hz), 1.54 (3H, d, J = 5.9 Hz), 1.40 (3H, d, J = 6.8 Hz). | 453.3 | 452.23 |
| 753 | 1H-NMR (CDCl3) δ: 9.33 (1H, s), 8.32 (2H, d, J = 9.8 Hz), 7.11 (1H, d, J = 9.8 Hz), 6.30 (1H, d, J = 6.8 Hz), 5.15 (1H, s), 4.45-4.25 (2H, m), 4.15-4.05 (2H, m), 3.99 (1H, s), 3.29 (2H, t, J = 10.2 Hz), 2.04 (2H, d, J = 9.8 Hz), 1.67 (2H, d, J = 8.8 Hz), 1.46 (3H, d, J = 5.9 Hz), 1.37 (6H, d, J = 5.9 Hz). | 459.2 | 458.19 |
| 754 | 1H-NMR (CDCl3) δ: 8.99 (1H, s), 8.30 (1H, d, J = 8.8 Hz), 8.09-8.01 (2H, m), 7.36 (1H, dd, J = 8.8, 2.9 Hz), 6.70-6.65 (2H, m), 4.79 (1H, q, J = 6.2 Hz), 4.50-4.44 (1H, m), 4.14 (2H, t, J = 5.4 Hz), 3.59 (2H, d, J = 4.9 Hz), 3.48 (3H, s), 2.79 (2H, t, J = 5.4 Hz), 2.39 (6H, s), 1.53 (3H, d, J = 6.8 Hz), 1.40 (3H, d, J = 6.8 Hz). | 442.3 | 441.25 |
| 755 | 1H-NMR (CDCl3) δ: 9.42 (1H, s), 9.04 (1H, s), 8.26 (1H, d, J = 8.8 Hz), 8.12 (1H, d, J = 2.0 Hz), 7.32 (1H, dd, J = 8.8, 2.0 Hz), 6.78 (1H, d, J = 7.8 Hz), 5.16 (1H, q, J = 6.2 Hz), 4.30 (1H, s), 4.13 (2H, d, J = 3.9 Hz), 3.97 (1H, d, J = 9.8 Hz), 3.85-3.62 (3H, m), 3.02 (2H, t, J = 4.4 Hz), 2.55 (3H, s), 2.09-1.95 (1H, m), 1.94-1.83 (3H, m), 1.69 (1H, s), 1.45 (3H, d, J = 5.9 Hz). | 474.2 | 473.19 |

**[Table 5-143]**

| Compound No. | NMR | LC/MS (M+H)⁺ | Exact Mass |
|---|---|---|---|
| 756 | 1H-NMR (CDCl3) δ: 9.44 (1H, s), 8.85 (1H, s), 8.44 (1H, d, J = 9.8 Hz), 8.38 (1H, d, J = 2.9 Hz), 7.77 (1H, dd, J = 9.8, 2.9 Hz), 6.75 (1H, d, J = 7.8 Hz), 5.17 (1H, q, J = 6.2 Hz), 4.49-4.38 (1H, m), 3.79-3.71 (4H, m), 3.59 (2H, d, J = 5.9 Hz), 3.47 (3H, s), 3.28 (2H, t, J = 5.4 Hz), 1.47 (3H, d, J = 5.9 Hz), 1.40 (3H, d, J = 6.8 Hz). | 487.3 | 486.19 |
| 757 | 1H-NMR (CDC13) δ: 8.99 (1H, s), 8.30 (1H, d, J = 8.8 Hz), 8.16 (1H, s), 8.08 (1H, d, J = 2.9 Hz), 7.34 (1H, dd, J = 8.8, 2.9 Hz), 6.72-6.63 (2H, m), 4.79 (1H, q, J = 6.5 Hz), 4.50-4.44 (1H, m), 4.14 (2H, t, J = 4.9 Hz), 3.59 (2H, d, J = 4.9 Hz), 3.47 (3H, s), 3.01 (2H, t, J = 5.4 Hz), 2.54 (3H, s), 1.53 (3H, d, J = 6.8 Hz), 1.40 (3H, d, J = 6.8 Hz). | 428.3 | 427.23 |
| 758 | 1H-NMR (CDCl3) δ: 9.38 (1H, s), 8.55 (1H, s), 8.28 (1H, d, J = 9.8 Hz), 8.12 (1H, s), 7.34 (1H, d, J = 8.8 Hz), 6.75 (1H, d, J = 6.8 Hz), 5.16 (1H, d, J = 6.8 Hz), 4.43 (1H, br s), 4.15 (2H, br s), 3.59 (2H, d, J = 3.9 Hz), 3.47 (3H, s), 3.02 (2H, br s), 2.54 (3H, s), 1.46 (3H, d, J = 6.8 Hz), 1.40 (3H, d, J = 5.9 Hz). | 462.3 | 461.19 |
| 759 | 1H-NMR (CDCl3) δ: 9.33 (1H, s), 8.34 (2H, d, J = 10.7 Hz), 7.10 (1H, d, J = 9.8 Hz), 6.69 (1H, d, J = 7.8 Hz), 5.16 (1H, d, J = 5.9 Hz), 4.46-4.41 (1H, m), 4.24 (1H, br s), 4.13-4.04 (2H, m), 4.02-3.95 (1H, m), 3.58 (2H, d, J = 3.9 Hz), 3.45 (3H, s), 3.34-3.23 (2H, m), 2.08-2.00 (2H, m), 1.78-1.60 (3H, m), 1.46 (3H, d, J = 6.8 Hz), 1.39 (3H, d, J = 6.8 Hz). | 489.3 | 488.21 |

**[Table 5-144]**

| Compound No. | NMR | LC/MS (M+H)⁺ | Exact Mass |
|---|---|---|---|
| 760 | 1H-NMR (CDCl3) δ: 8.99 (1H, s), 8.39 (1H, d, J = 9.8 Hz), 8.22 (1H, s), 7.10 (1H, d, J = 9.8 Hz), 6.71 (1H, s), 6.61 (1H, d, J = 7.8 Hz), 4.83-4.76 (1H, m), 4.52-4.45 (1H, m), 4.12-4.03 (2H, m), 4.01-3.94 (2H, m), 3.58 (2H, dd, J = 4.4, 1.5 Hz), 3.46 (3H, s), 3.32-3.23 (2H, m), 2.08-2.00 (2H, m), 1.73-1.62 (2H, m), 1.53 (3H, d, J = 6.3 Hz), 1.39 (3H, d, J = 6.8 Hz). | 455.3 | 454.24 |
| 761 | 1H-NMR (CDCl3) δ: 9.02 (1H, s), 8.38 (1H, s), 8.34 (1H, d, J = 9.8 Hz), 7.10 (1H, d, J = 9.8 Hz), 6.69 (1H, s), 6.21 (1H, d, J = 7.3 Hz), 4.80 (1H, d, J = 6.3 Hz), 4.43-4.34 (1H, m), 4.14-4.06 (1H, m), 4.04-3.95 (2H, m), 3.51-3.44 (1H, m), 3.41 (3H, s), 3.38-3.28 (2H, m), 2.06-1.97 (2H, m), 1.76-1.64 (2H, m), 1.53 (3H, d, J = 6.3 Hz), 1.37 (6H, d, J = 6.8 Hz). | 439.3 | 438.25 |
| 762 | 1H-NMR (CDCl3) δ: 9.04 (1H, s), 8.51 (1H, s), 8.37 (1H, d, J = 9.8 Hz), 7.10 (1H, d, J = 9.8 Hz), 6.74 (1H, d, J = 1.0 Hz), 6.64 (1H, d, J = 8.3 Hz), 4.79 (1H, q, J = 6.5 Hz), 4.37-4.29 (1H, m), 4.03-3.97 (3H, m), 3.76 (2H, t, J = 5.1 Hz), 3.67-3.60 (1H, m), 3.51-3.44 (1H, m), 3.41 (3H, s), 3.36-3.29 (2H, m), 2.05-1.98 (2H, m), 1.92-1.83 (2H, m), 1.75-1.65 (4H, m), 1.53 (3H, d, J = 6.3 Hz). | 481.3 | 480.26 |
| 763 | 1H-NMR (CDCl3) δ: 9.01 (1H, s), 8.48 (1H, s), 8.44 (1H, d, J = 9.3 Hz), 8.34 (1H, d, J = 2.4 Hz), 7.76 (1H, dd, J = 9.0, 2.7 Hz), 6.66 (1H, s), 6.12 (1H, d, J = 7.3 Hz), 4.43-4.33 (1H, m), 4.19 (1H, t, J = 8.0 Hz), 4.12-4.05 (1H, m), 4.03-3.90 (2H, m), 3.77-3.72 (4H, m), 3.54-3.44 (1H, m), 3.27 (2H, t, J = 5.1 Hz), 2.37-2.22 (2H, m), 1.35 (6H, d, J = 6.3 Hz). | 449.3 | 448.23 |

**[Table 5-145]**

| Compound No. | NMR | LC/MS (M+H)⁺ | Exact Mass |
|---|---|---|---|
| 764 | 1H-NMR (CDCl3) δ: 9.00 (1H, s), 8.26 (1H, d, J = 8.8 Hz), 8.20 (1H, br s), 8.07 (1H, d, J = 2.9 Hz), 7.36 (1H, dd, J = 8.8, 2.9 Hz), 6.68 (1H, s), 6.24 (1H, d, J = 7.8 Hz), 4.80 (1H, q, J = 6.5 Hz), 4.45-4.35 (1H, m), 4.12-4.01 (1H, m), 3.50-3.37 (2H, m), 3.30-3.15 (3H, m), 3.05-2.95 (1H, m), 1.53 (3H, d, J = 6.8 Hz), 1.38 (6H, d, J = 6.3 Hz). | 477.2 | 476.23 |
| 765 | 1H-NMR (DMSO-D6) δ: 10.21 (1H, s), 9.26 (1H, s), 8.32 (1H, d, J = 8.8 Hz), 8.25 (1H, s), 7.77 (1H, t, J = 4.4 Hz), 6.99 (1H, s), 6.42 (1H, d, J = 7.8 Hz), 5.21 (1H, d, J = 4.9 Hz), 4.61 (1H, t, J = 5.4 Hz), 4.32-4.20 (1H, m), 3.80-3.70 (1H, br m), 3.60-3.50 (1H, br m), 3.21-3.15 (2H, br m), 3.12-3.00 (3H, br m), 2.88 (1H, s), 2.72 (1H, s), 2.40-2.30 (2H, br m), 1.38 (4H, d, J = 6.8 Hz), 1.29 (7H, d, J = 4.9 Hz), 0.99 (3H, d, J = 6.8 Hz), 0.91 (3H, d, J = 6.8 Hz). | | 464.26 |
| 766 | 1H-NMR (DMSO-D6) δ: 10.29 (1H, s), 9.26 (1H, s), 8.35 (2H, d, J = 37.1 Hz), 8.26 (0H, s), 7.94 (1H, s), 7.78 (1H, d, J = 7.8 Hz), 7.53 (1H, br s), 6.99 (2H, s), 6.42 (1H, d, J = 6.8 Hz), 5.21 (1H, s), 4.61 (1H, s), 4.30-4.20 (1H, m), 3.90-3.77 (1H, br m), 3.70-3.60 (2H, br m), 2.88 (2H, s), 2.72 (2H, s), 2.46-2.35 (1H, br m), 1.38 (3H, d, J = 5.9 Hz), 1.29 (6H, d, J = 4.9 Hz), 1.01 (3H, d, J = 6.8 Hz), 0.96 (3H, d, J = 5.9 Hz). | | 464.26 |
| 767 | 1H-NMR (CDCl3) δ: 8.99 (1H, s), 8.41 (1H, d, J = 9.8 Hz), 8.10 (1H, s), 7.09 (1H, d, J = 9.8 Hz), 6.69 (1H, s), 6.22 (1H, d, J = 7.3 Hz), 5.00-4.94 (1H, m), 4.83-4.77 (1H, m), 4.44-4.36 (1H, m), 4.04 (1H, br s), 3.63 (8H, d, J = 7.8 Hz), 1.53 (3H, d, J = 6.8 Hz), 1.37 (6H, d, J = 6.3 Hz), 1.28 (6H, d, J = 6.3 Hz). | 496.3 | 495.27 |
| 768 | | 451.3 | 450.25 |

**[Table 5-146]**

| Compound No. | NMR | LC/MS (M+H)⁺ | Exact Mass |
|---|---|---|---|
| 769 | | 451.3 | 450.25 |
| 770 | | 466.4 | 465.25 |
| 771 | 1H-NMR (400 MHz, DMSO-d6, ppm) δ: 10.14 (1H, s), 9.27 (1H, s), 8.38 (1H, d, J = 8.8Hz), 8.27 (1H, d, J = 2.8Hz), 7.79-7.74 (1H, m), 7.21 (1H, s), 7.07 (1H, s), 5.21 (1H, d, J = 4.8Hz), 4.63 (1H, br), 3.72-3.70 (2H, m), 3.57 (2H, s), 3.20(2H, br), 2.52 (1H, s), 2.21 (6H, s), 1.41 (3H, d, J = 6.4Hz) | 447.4 | 446.22 |
| 772 | 1H-NMR (400 MHz, DMSO-d6, ppm) δ: 9.86 (1H, s), 9.19 (1H, s), 8.14 (1H, d, J =9.2Hz), 8.04 (1H, d, J = 2.8Hz), 7.51-7.48 (1H, m), 6.96 (1H, s), 6.34 (1H, d, J = 7.2Hz), 5.16 (1H, d, J = 4.4Hz), 4.63-4.55 (1H, m),4.48-4.45 (1H, m), 4.28-4.21(1H, m), 3.29-3.25 (1H, m), 1.96-1.91 (5H, m), 1.83-1.70 (3H, m), 1.68-1.44(3H, m), 1.38-1.27 (6H, m) | 424.1 | 423.24 |
| 773 | 1H-NMR (CDCl3) δ: 8.99 (1H, s), 8.44 (1H, d, J = 8.8 Hz), 8.39 (1H, s), 8.32 (1H, d, J = 2.4 Hz), 7.75 (1H, dd, J = 8.8, 2.4 Hz), 6.66 (1H, s), 6.11 (1H, d, J = 7.3 Hz), 4.43-4.32 (1H, m), 4.19 (1H, t, J = 8.0 Hz), 4.12-3.89 (3H, m), 3.78-3.70 (4H, m), 3.54-3.44 (1H, m), 3.27 (2H, t, J = 5.1 Hz), 2.36-2.23 (2H, m), 1.35 (6H, d, J = 6.8 Hz). | 449.2 | 448.23 |
| 774 | 1H-NMR (CDCl3) δ: 8.99 (1H, s), 8.44 (1H, d, J = 8.8 Hz), 8.35 (1H, s), 8.32 (1H, d, J = 2.4 Hz), 7.75 (1H, dd, J = 9.0, 2.2 Hz), 6.66 (1H, s), 6.12 (1H, d, J = 6.8 Hz), 4.44-4.32 (1H, m), 4.19 (1H, t, J = 7.8 Hz), 4.12-3.90 (3H, m), 3.78-3.70 (4H, m), 3.54-3.44 (1H, m), 3.27 (2H, t, J = 5.4 Hz), 2.37-2.22 (2H, m), 1.35 (6H, d, J = 6.3 Hz). | 449.3 | 448.23 |

**[Table 5-147]**

| Compound No. | NMR | LC/MS (M+H)⁺ | Exact Mass |
|---|---|---|---|
| 775 | 1H-NMR (CDCl3) δ: 8.99 (1H, s), 8.25 (1H, d, J = 8.8 Hz), 8.10 (1H, s), 8.06 (1H, d, J = 2.9 Hz), 7.36 (1H, dd, J = 9.0, 3.2 Hz), 6.67 (1H, s), 6.24 (1H, d, J = 7.3 Hz), 4.84-4.76 (1H, m), 4.45-4.35 (1H, m), 4.15-4.01 (2H, m), 3.50-3.37 (2H, m), 3.30-3.15 (3H, m), 3.05-2.94 (1H, m), 1.53 (3H, d, J = 6.8 Hz), 1.38 (6H, d, J = 6.3 Hz). | 477.2 | 476.23 |
| 776 | 1H-NMR (CDCl3) δ: 9.00 (1H, s), 8.26 (1H, d, J = 9.3 Hz), 8.18 (1H, s), 8.07 (1H, d, J = 2.9 Hz), 7.36 (1H, dd, J = 9.0, 2.7 Hz), 6.68 (1H, s), 6.24 (1H, d, J = 7.3 Hz), 4.84-4.76 (1H, m), 4.45-4.35 (1H, m), 4.15-4.01 (2H, m), 3.50-3.36 (2H, m), 3.30-3.15 (3H, m), 3.05-2.95 (1H, m), 1.53 (3H, d, J = 6.3 Hz), 1.38 (6H, dd, J = 6.3, 1.5 Hz). | 477.2 | 476.23 |
| 777 | 1H-NMR (CDCl3) δ: 9.01 (1H, s), 8.30-8.24 (2H, m), 8.10 (1H, d, J = 2.9 Hz), 7.40 (1H, dd, J = 9.3, 2.9 Hz), 6.68 (1H, s), 6.24 (1H, d, J = 7.8 Hz), 4.84-4.76 (1H, m), 4.46-4.35 (1H, m), 4.12 (1H, d, J = 4.9 Hz), 3.65-3.41 (3H, m), 3.25 (1H, d, J = 11.7 Hz), 3.09 (1H, td, J = 11.5, 2.9 Hz), 2.93-2.82 (2H, m), 1.93 (1H, br s), 1.53 (3H, d, J = 6.3 Hz), 1.38 (6H, d, J = 6.3 Hz). | 477.2 | 476.23 |
| 778 | 1H-NMR (CDCl3) δ: 9.13 (1H, s), 8.13 (1H, d, J = 8.3 Hz), 7.34-7.24 (1H, m), 6.76 (1H, s), 6.68-6.55 (1H, br m), 4.80 (1H, q, J = 6.3 Hz), 4.38-4.28 (1H, br m), 4.05-3.91 (3H, m), 3.82-3.72 (2H, m), 3.67-3.55 (1H, m), 3.35-3.15 (2H, m), 2.99-2.82 (2H, m), 2.17-1.59 (4H, m), 1.51 (3H, d, J = 6.3 Hz). | 422.2 | 421.22 |

**[Table 5-148]**

| Compound No. | NMR | LC/MS (M+H)⁺ | Exact Mass |
|---|---|---|---|
| 779 | 1H-NMR (CDCl3) δ: 9.03 (1H, s), 8.47-8.39 (2H, m), 8.33 (1H, d, J = 2.4 Hz), 7.76 (1H, dd, J = 9.0, 2.7 Hz), 6.64 (1H, s), 6.15 (1H, d, J = 7.3 Hz), 5.17 (2H, d, J = 5.4 Hz), 4.58 (2H, d, J = 5.4 Hz), 4.48-4.37 (1H, m), 3.78-3.71 (4H, m), 3.27 (2H, t, J = 5.4 Hz), 1.80 (3H, s), 1.37 (6H, d, J = 6.3 Hz). | 449.3 | 448.23 |
| 780 | 1H-NMR (CDCl3) δ: 9.07 (1H, s), 8.65 (1H, s), 8.40 (1H, s), 8.18 (1H, s), 6.71 (1H, s), 6.27 (1H, d, J = 6.8 Hz), 4.82 (1H, q, J = 6.5 Hz), 4.45-4.34 (1H, m), 3.78-3.65 (3H, m), 3.55-3.47 (1H, m), 3.34-3.20 (2H, m), 2.34 (3H, s), 1.54 (3H, d, J = 5.9 Hz), 1.39 (6H, d, J = 6.8 Hz). | 437.3 | 436.23 |
| 781 | 1H-NMR (CDC13) δ: 9.01 (1H, s), 8.29 (1H, d, J = 9.3 Hz), 8.14 (1H, s), 8.08 (1H, d, J = 2.9 Hz), 7.38 (1H, dd, J = 9.3, 2.9 Hz), 6.73 (1H, s), 6.69 (1H, d, J = 7.8 Hz), 4.80 (1H, q, J = 6.3 Hz), 4.57-4.50 (1H, m), 4.34 (1H, br s), 4.02 (1H, dd, J = 11.2, 2.9 Hz), 3.77 (2H, t, J = 5.1 Hz), 3.66-3.60 (1H, m), 2.71 (1H, dd, J = 13.2, 6.8 Hz), 2.50-2.43 (1H, m), 2.36 (6H, s), 2.08-2.01 (1H, m), 1.93-1.84 (3H, m), 1.53 (3H, d, J = 6.3 Hz), 1.33 (3H, d, J = 6.3 Hz). | 468.3 | 467.26 |
| 782 | 1H-NMR (CDCl3) δ: 9.02 (1H, s), 8.47 (1H, s), 8.29 (1H, d, J = 9.8 Hz), 8.09 (1H, d, J = 2.9 Hz), 7.33 (1H, dd, J = 8.8, 2.9 Hz), 6.72 (1H, s), 6.67 (1H, d, J = 7.8 Hz), 4.80 (1H, q, J = 6.5 Hz), 4.33 (1H, s), 4.06-3.90 (3H, m), 3.77 (2H, t, J = 4.9 Hz), 3.64 (1H, dd, J = 10.7, 5.9 Hz), 3.59-3.51 (1H, m), 3.11-2.95 (2H, m), 2.09-1.50 (13H, m). | 466.3 | 465.25 |

**[Table 5-149]**

| Compound No. | NMR | LC/MS (M+H)⁺ | Exact Mass |
|---|---|---|---|
| 783 | 1H-NMR (CDCl3) δ: 9.01 (1H, s), 8.32 (1H, s), 8.29 (1H, d, J = 8.8 Hz), 8.08 (1H, d, J = 2.9 Hz), 7.34 (1H, dd, J = 9.8, 2.9 Hz), 6.72 (1H, s), 6.68 (1H, d, J = 7.8 Hz), 4.80 (1H, q, J = 6.5 Hz), 4.34 (1H, br s), 4.05-3.90 (3H, m), 3.77 (2H, t, J = 5.4 Hz), 3.64 (1H, dd, J = 11.7, 5.9 Hz), 3.59-3.51 (1H, m), 3.09-2.95 (2H, m), 2.09-1.50 (13H, m). | 466.3 | 465.25 |
| 784 | | 477.3 | 476.26 |
| 785 | 1H-NMR (CDCl3) δ: 9.00 (1H, s), 8.31 (1H, d, J = 9.3 Hz), 8.17 (1H, s), 8.09 (1H, d, J = 2.9 Hz), 7.37 (1H, dd, J = 9.3, 2.9 Hz), 6.70 (1H, s), 6.67 (1H, d, J = 7.8 Hz), 4.81 (1H, q, J = 6.3 Hz), 4.51-4.45 (1H, m), 4.15 (2H, t, J = 5.6 Hz), 3.60 (2H, d, J = 4.4 Hz), 3.48 (3H, s), 2.80 (2H, t, J = 5.4 Hz), 2.39 (6H, s), 1.53 (3H, d, J = 6.3 Hz), 1.39 (3H, d, J = 6.3 Hz), 1.25 (1H, s). | 442.3 | 441.25 |
| 786 | 1H-NMR (CDCl3) δ: 9.02 (1H, s), 8.31-8.27 (2H, m), 8.07 (1H, d, J = 2.9 Hz), 7.34 (1H, dd, J = 9.3, 2.9 Hz), 6.74-6.66 (2H, m), 4.80 (1H, q, J = 6.5 Hz), 4.37-4.29 (1H, br m), 4.20-4.09 (1H, m), 4.02 (1H, dd, J = 11.2, 2.9 Hz), 3.95-3.86 (1H, br m), 3.80-3.73 (2H, m), 3.64 (1H, dd, J = 11.2, 5.9 Hz), 2.08 (14H, dtt, J = 95.6, 32.1, 11.2 Hz), 1.71-1.32 (8H, m). | 508.4 | 507.30 |
| 787 | 1H-NMR (CDCl3) δ: 9.02 (1H, s), 8.29 (1H, d, J = 9.3 Hz), 8.21 (1H, s), 8.08 (1H, d, J = 2.9 Hz), 7.35 (1H, dd, J = 9.0, 2.7 Hz), 6.74-6.66 (2H, m), 4.84-4.76 (1H, m), 4.52-4.46 (1H, br m), 4.38-4.29 (1H, br m), 4.07-3.98 (1H, m), 3.93-3.85 (1H, br m), 3.81-3.73 (2H, m), 3.63 (1H, dd, J = 11.0, 5.6 Hz), 2.35-2.01 (10H, m), 1.94-1.83 (2H, m), 1.72-1.50 (10H, m). | 508.5 | 507.30 |

**[Table 5-150]**

| Compound No. | NMR | LC/MS (M+H)⁺ | Exact Mass |
|---|---|---|---|
| 788 | 1H-NMR (DMSO-D6) δ: 10.01 (1H, s), 9.75 (1H, s), 9.22 (1H, s), 8.56 (1H, d, J = 4.1 Hz), 8.23 (1H, d, J = 8.8 Hz), 8.11 (1H, dd, J = 9.3, 2.4 Hz), 6.97 (1H, s), 6.40 (1H, d, J = 7.3 Hz), 5.19 (1H, d, J = 4.4 Hz), 4.64-4.58 (1H, m), 4.25 (1H, td, J = 13.4, 6.8 Hz), 3.26-3.21 (1H, m), 2.97 (1H, d, J = 13.2 Hz), 2.57 (1H, t, J = 11.5 Hz), 1.79 (2H, d, J = 18.0 Hz), 1.51-1.33 (8H, m), 1.29 (7H, dd, J = 6.3, 2.0 Hz). | 451.3 | 450.25 |
| 789 | 1H-NMR (CDCl3) δ: 9.16 (1H, d, J = 1.5 Hz), 8.97 (1H, s), 7.89 (1H, d, J = 1.5 Hz), 7.74 (1H, s), 6.67 (1H, s), 6.26 (1H, d, J = 7.8 Hz), 4.80 (1H, q, J = 6.3 Hz), 4.43-4.36 (1H, m), 3.53 (4H, t, J = 5.1 Hz), 3.05 (4H, t, J = 5.1 Hz), 1.53 (3H, d, J = 6.3 Hz), 1.38 (6H, d, J = 6.3 Hz). | 410.3 | 409.23 |
| 790 | | 495.4 | 494.28 |
| 791 | 1H-NMR (DMSO-D6) δ: 10.28 (1H, s), 9.18 (1H, d, J = 2.0 Hz), 8.11 (1H, d, J = 9.8 Hz), 7.37 (1H, d, J = 9.8 Hz), 6.95 (1H, s), 6.34 (1H, s), 5.19 (1H, s), 4.61 (1H, d, J = 5.9 Hz), 3.42 (4H, t, J = 4.1 Hz), 2.81 (4H, t, J = 4.1 Hz), 1.53 (9H, s), 1.38 (3H, d, J = 7.0 Hz). | 424.3 | 423.25 |
| 792 | | 453.3 | 452.23 |
| 793 | | 453.3 | 452.23 |
| 794 | | 495.3 | 494.28 |
| 795 | 1H-NMR (DMSO-D6) δ: 10.30 (1H, s), 9.24 (1H, s), 8.29 (1H, d, J = 2.4 Hz), 8.24 (1H, d, J = 9.3 Hz), 7.79 (1H, dd, J = 8.8, 2.4 Hz), 6.99 (1H, s), 6.43 (1H, s), 5.21 (1H, d, J = 4.4 Hz), 4.63 (1H, t, J = 5.4 Hz), 3.63 (2H, t, J = 5.4 Hz), 3.40 (2H, s), 3.03 (2H, t, J = 5.4 Hz), 1.53 (9H, s), 1.39 (3H, d, J = 9.0 Hz). | 437.3 | 436.23 |
| 796 | | 494.3 | 493.28 |

**[Table 5-151]**

| Compound No. | NMR | LC/MS (M+H)⁺ | Exact Mass |
|---|---|---|---|
| 797 | 1H-NMR (DMSO-D6) δ: 10.16 (1H, s), 9.18 (1H, s), 8.14 (1H, d, J = 9.8 Hz), 7.38 (1H, d, J = 9.8 Hz), 6.95 (1H, s), 6.32 (1H, d, J = 7.8 Hz), 4.60 (1H, q, J = 6.3 Hz), 4.27-4.19 (4H, m), 2.85 (2H, t, J = 11.7 Hz), 2.14 (2H, d, J = 7.3 Hz), 2.02-1.86 (1H, m), 1.75 (2H, d, J = 11.7 Hz), 1.38 (3H, t, J = 6.8 Hz), 1.27-1.17 (10H, m). | 467.3 | 466.24 |
| 798 | 1H-NMR (300 MHz, DMSO-d6, ppm) δ: 10.04 (1H, s), 9.25 (1H, s), 8.31-8.24 (2H, m), 7.86-7.83 (1H, m), 6.99 (1H, s), 6.40 (1H, d, J = 7.8Hz), 6.07-6.04 (1H, m), 5.20-5.18 (1H, m), 4.66-4.61 (1H, m), 4.31-4.24 (1H, m), 3.83-3.69(3H, m), 3.51-3.43 (1H, m), 3.26-3.19 (1H, m), 2.87 (2, br), 1.40-1.36 (3H, m), 1.31 (3H, d, J = 6.6Hz) | 410.3 | 409.22 |
| 799 | 1H-NMR (400 MHz, DMSO-d6, ppm) δ: 9.87 (1H, s), 9.19 (1H, s), 8.15 (1H, d, J =9.2Hz), 8.04 (1H, d, J = 2.8Hz), 7.66-7.53 (1H, m), 6.96 (1H, s), 6.34 (1H, d, J = 7.6Hz), 5.16 (1H, d, J = 4.4Hz), 4.72-4.68 (1H, m),4.63-4.57 (1H, m), 4.28-4.19 (1H, m), 3.92-3.89 (1H, m), 2.30 (3H, s), 1.96-1.80 (2H, m), 1.79-1.62 (2H, m), 1.61-1.46 (2H, m), 1.38-1.36 (3H, m), 1.29-1.27 (6H, m) | 438.4 | 437.25 |
| 800 | 1H-NMR (400 MHz, DMSO-d6, ppm) δ: 9.99 (1H, s), 9.23 (1H, s), 8.18 (1H, d, J =9.2Hz), 8.07 (1H, d, J = 2.8Hz ), 7.49-7.46 (1H, m), 7.01 (1H, s), 6.57 (1H, d, J = 7.6Hz), 5.21 (1H, d, J = 4.0Hz), 4.62-4.60 (1H, m), 4.20-4.13 (3H, m), 3.96-3.93 (2H, m), 3.72-3.60 (2H, m), 3.58-3.41 (3H, m), 3.40-3.38 (2H, m), 3.03-2.99 (1H, m), 2.02-2.00 (1H, m), 1.80-1.76 (2H, m), 1.66-1.61 (1H, m), 1.39-1.37 (3H, m) | 452.1 | 451.23 |

**[Table 5-152]**

| Compound No. | NMR | LC/MS (M+H)⁺ | Exact Mass |
|---|---|---|---|
| 801 | 1H-NMR (400 MHz, DMSO-d6, ppm) δ: 9.84 (1H, s), 9.19 (1H, s), 8.14 (1H, d, J =9.2Hz), 8.00 (1H, d, J = 3.2Hz), 7.49-7.46 (1H, m), 6.95 (1H, s), 6.35 (1H, d, J = 8.0Hz), 5.15 (1H, d, J = 4.8Hz), 4.77-4.75 (1H, m), 4.61-4.59 (1H, m), 4.25-4.23 (1H, m), 2.34 (1H, br), 2.19 (6H, s), 1.88-1.86 (2H, m), 1.75-1.72 (3H, m), 1.68-1.53 (1H, m), 1.38-1.36 (3H, m), 1.29-1.28 (6H, m) | 452.0 | 451.27 |
| 802 | 1H-NMR (400 MHz, DMSO-d6, ppm) δ: 9.99 (1H, s), 9.22 (1H, s), 8.16-8.04 (2H, m), 7.47-7.44 (1H, m), 7.00 (1H, s), 6.57-6.55 (1H, m), 5.19 (1H, d, J = 4.4Hz), 4.60-4.59 (1H, m), 4.10-4.01 (5H, m), 3.99-3.91 (1H, m), 3.68-3.48 (2H, m), 3.41-3.37 (1H, m), 3.26-3.24 (2H, m), 2.32-2.22 (1H, m), 2.17-1.94 (2H, m), 1.78-1.73 (2H, m), 1.68-1.56 (1H, m), 1.37-1.35 (3H, m) | 452.0 | 451.23 |
| 803 | 1H-NMR (400 MHz, DMSO-d6, ppm) δ: 9.95 (1H, s), 9.22 (1H, s), 8.14 (1H, d, J = 9.6Hz), 8.00 (1H, d, J = 2.8Hz), 7.42-7.40 (1H, m), 6.99 (1H, s), 6.57 (1H, d, J = 8.0Hz), 5.19 (1H, d, J = 4.0Hz), 4.89 (1H, br), 4.60-4.58 (1H, m), 4.13-4.12 (1H, m), 3.94-3.91 (1H, m), 3.68-3.56 (1H, m), 3.56-3.54 (1H, m), 3.42-3.37 (2H, m), 3.04-3.01 (1H, m), 2.89-2.87 (2H, m), 2.01-1.97 (2H, m), 1.78-1.73 (3H, m), 1.69-1.56 (1H, m), 1.37-1.35 (3H, m) | 452.0 | 451.23 |
| 804 | 1H-NMR (400 MHz, DMSO-d6, ppm) δ: 10.22 (1H, s), 9.26 (1H, s), 8.35-8.32 (2H, m), 7.88-7.85 (1H, m), 6.99 (1H, s), 6.42 (1H, d, J = 7.6Hz), 5.19 (1H, br), 4.62-4.60 (1H, m), 4.28-4.22 (3H, m), 4.00-3.97 (2H, m), 3.78-3.76 (2H, m), 1.39-1.37 (3H, m), 1.30-1.28 (6H, m) | 424.3 | 423.20 |

**[Table 5-153]**

| Compound No. | NMR | LC/MS (M+H)⁺ | Exact Mass |
|---|---|---|---|
| 805 | 1H-NMR (400 MHz, DMSO-d6, ppm) δ: 9.99 (1H, s), 9.23 (1H, s), 8.20-8.14 (1H, m), 8.07-8.02 (1H, m), 7.53-7.42 (1H, m), 7.01 (1H, s), 6.59 (1H, d, J = 8.0Hz), 5.22 (1H, br), 5.05 (1H, br), 4.62 (1H, br), 4.14 (1H, br), 3.93 (1H, m), 3.69 (1H, br), 3.55-3.43 (2H, m), 3.07-3.03 (1H, m), 2.90-2.85 (2H, m), 2.77 (1H, m), 2.08-1.99 (3H, m), 1.79-1.63 (3H, m), 1.57 (1H, br), 1.44-1.37 (3H, m) | 452.1 | 451.23 |
| 806 | 1H-NMR (DMSO-D6) δ: 10.40 (1H, s), 9.35 (1H, s), 8.23 (2H, dd, J = 7.8, 5.4 Hz), 7.73 (1H, dd, J = 8.3, 2.4 Hz), 7.25 (1H, s), 6.95-6.67 (2H, m), 4.16 (1H, dq, J = 14.6, 3.7 Hz), 3.90 (1H, dd, J = 10.7, 2.9 Hz), 3.71-3.66 (1H, m), 3.59-3.53 (1H, m), 3.46 (4H, dd, J = 10.7, 6.3 Hz), 2.75 (2H, dd, J = 10.2, 2.9 Hz), 2.17 (1H, tt, J = 11.0, 4.2 Hz), 2.02-1.96 (3H, m), 1.86-1.75 (4H, m), 1.64-1.48 (3H, m). | 514.4 | 513.23 |
| 807 | 1H-NMR (DMSO-D6) δ: 10.21 (1H, s), 9.19 (1H, s), 8.31 (1H, d, J = 8.8 Hz), 8.28 (1H, d, J = 2.4 Hz), 7.80 (1H, dd, J = 9.0, 2.7 Hz), 6.77 (1H, s), 6.43 (1H, d, J = 7.3 Hz), 4.26 (1H, td, J = 13.2, 6.3 Hz), 4.01 (1H, dd, J = 11.0, 3.2 Hz), 3.87 (1H, d, J = 11.2 Hz), 3.63 (2H, t, J = 5.4 Hz), 3.45 (1H, t, J = 10.7 Hz), 3.40 (2H, s), 3.02 (2H, t, J = 5.4 Hz), 2.83-2.76 (2H, m), 2.01 (1H, d, J = 12.2 Hz), 1.88-1.80 (1H, m), 1.68-1.62 (2H, m), 1.30 (6H, d, J = 6.3 Hz). | 463.3 | 462.25 |

**[Table 5-154]**

| Compound No. | NMR | LC/MS (M+H)⁺ | Exact Mass |
|---|---|---|---|
| 808 | 1H-NMR (DMSO-D6) δ: 10.10 (1H, s), 9.25 (1H, s), 8.58 (1H, d, J = 2.4 Hz), 8.30-8.26 (2H, m), 8.01 (1H, dd, J = 8.8, 2.4 Hz), 7.92 (1H, s), 6.99 (1H, s), 6.42 (1H, d, J = 7.8 Hz), 5.20 (1H, s), 4.62 (1H, q, J = 6.3 Hz), 4.30-4.15 (2H, m), 3.07-3.00 (2H, m), 2.58 (2H, t, J = 11.2 Hz), 1.97 (2H, d, J = 11.7 Hz), 1.80 (2H, ddd, J = 23.9, 11.7, 3.9 Hz), 1.39 (3H, d, J = 6.3 Hz), 1.30 (6H, dd, J = 6.3, 2.0 Hz). | 474.3 | 473.27 |
| 809 | | 495.3 | 494.28 |
| 810 | | 495.3 | 494.28 |
| 811 | 1H-NMR (CDCl3) δ: 9.59 (1H, s), 9.12 (1H, s), 8.39 (1H, d, J = 9.3 Hz), 8.35 (1H, d, J = 2.9 Hz), 7.64 (1H, dd, J = 9.3, 2.9 Hz), 6.70 (1H, s), 6.18 (1H, d, J = 7.3 Hz), 4.80 (1H, q, J = 6.3 Hz), 4.43-4.32 (1H, m), 3.90 (2H, dd, J = 5.4, 3.4 Hz), 3.70 (2H, t, J = 5.1 Hz), 2.96-2.84 (6H, m), 2.73 (2H, t, J = 5.1 Hz), 1.53 (3H, d, J = 6.3 Hz), 1.37 (6H, d, J = 6.3 Hz). | 481.3 | 480.26 |
| 812 | 1H-NMR (CDCl3) δ: 9.03 (1H, s), 8.42 (1H, d, J = 8.8 Hz), 8.31 (1H, d, J = 2.9 Hz), 8.17 (1H, s), 7.77 (1H, dd, J = 8.8, 2.4 Hz), 6.71 (1H, s), 6.37 (1H, s), 4.82 (1H, d, J = 6.3 Hz), 4.11 (1H, br s), 3.76-3.64 (6H, m), 3.27 (2H, t, J = 5.4 Hz), 1.55 (3H, d, J = 6.4 Hz), 1.38 (3H, t, J = 7.1 Hz). | 409.3 | 408.20 |
| 813 | 1H-NMR (CDCl3) δ: 9.04 (1H, s), 8.42 (1H, d, J = 8.8 Hz), 8.32 (2H, d, J = 2.4 Hz), 7.76 (1H, dd, J = 8.8, 2.4 Hz), 6.70 (1H, s), 6.42 (1H, t, J = 5.6 Hz), 4.81 (1H, q, J = 6.5 Hz), 3.77-3.72 (3H, m), 3.68-3.61 (3H, m), 3.30-3.24 (2H, m), 1.78-1.70 (2H, m), 1.56-1.46 (5H, m), 1.02 (3H, t, J = 7.6 Hz). | 437.3 | 436.23 |

**[Table 5-155]**

| Compound No. | NMR | LC/MS (M+H)⁺ | Exact Mass |
|---|---|---|---|
| 814 | 1H-NMR (CDCl3) δ: 9.06 (1H, s), 8.67 (1H, s), 8.42 (1H, d, J = 8.8 Hz), 8.35 (1H, d, J = 2.4 Hz), 7.76 (1H, dd, J = 8.8, 2.4 Hz), 6.71 (1H, s), 6.44 (1H, t, J = 5.6 Hz), 4.81 (1H, q, J = 6.3 Hz), 3.79-3.72 (4H, m), 3.65-3.58 (3H, m), 3.27 (2H, t, J = 5.4 Hz), 1.83-1.72 (2H, m), 1.54 (3H, d, J = 6.3 Hz), 1.07 (3H, t, J = 7.3 Hz) . | 423.3 | 422.22 |
| 815 | 1H-NMR (CDCl3) δ: 9.40 (1H, s), 8.33 (1H, d, J = 11.2 Hz), 8.23 (1H, d, J = 8.8 Hz), 7.56 (1H, d, J = 8.3 Hz), 6.30 (1H, d, J = 7.8 Hz), 5.16 (1H, s), 4.39-4.31 (2H, m), 3.74 (2H, s), 3.67-3.61 (1H, m), 3.58-3.50 (1H, m), 3.27 (2H, q, J = 5.0 Hz), 2.42 (3H, s), 1.46 (3H, d, J = 6.3 Hz), 1.37 (6H, d, J = 6.3 Hz). | 471.3 | 470.19 |
| 816 | 1H-NMR (CDCl3) δ: 9.10 (1H, s), 8.81 (1H, s), 8.37 (1H, d, J = 8.3 Hz), 8.32 (1H, d, J = 2.0 Hz), 7.77 (1H, dd, J = 8.3, 2.0 Hz), 6.86 (1H, s), 6.27 (1H, t, J = 5.4 Hz), 4.35 (1H, q, J = 6.3 Hz), 3.71-3.65 (2H, m), 3.50 (2H, s), 3.42 (3H, s), 2.91 (4H, br s), 2.46 (4H, br s), 1.51 (3H, d, J = 6.3 Hz), 1.37 (3H, t, J = 7.3 Hz) . | 423.3 | 422.25 |
| 817 | 1H-NMR (CDCl3) δ: 9.10 (1H, s), 8.83 (1H, s), 8.40 (1H, d, J = 10.2 Hz), 7.10 (1H, d, J = 9.8 Hz), 6.86 (1H, s), 6.22 (1H, t, J = 5.1 Hz), 4.33 (1H, q, J = 6.5 Hz), 3.65 (2H, t, J = 6.8 Hz), 3.63-3.55 (4H, m), 3.41 (3H, s), 3.09-3.01 (4H, m), 1.51 (3H, d, J = 6.3 Hz), 1.34 (3H, t, J = 7.1 Hz), 1.25 (1H, s). | 410.3 | 409.23 |

**[Table 5-156**

| Compound No. | NMR | LC/MS (M+H)⁺ | Exact Mass |
|---|---|---|---|
| 818 | 1H-NMR (CDCl3) δ: 9.12 (1H, s), 8.98 (1H, br s), 8.38 (1H, d, J = 8.8 Hz), 8.34 (1H, d, J = 2.0 Hz), 7.75 (1H, dd, J = 8.8, 2.0 Hz), 6.86 (1H, s), 6.36 (1H, t, J = 5.6 Hz), 4.35 (1H, q, J = 6.5 Hz), 3.65-3.58 (2H, m), 3.50 (2H, s), 3.42 (3H, s), 2.94-2.88 (4H, m), 2.46 (4H, br s), 1.80-1.73 (2H, m), 1.51 (3H, d, J = 6.3 Hz), 1.08 (3H, t, J = 7.6 Hz). | 437.3 | 436.27 |
| 819 | 1H-NMR (CDCl3) δ: 9.05 (1H, s), 8.47 (1H, br s), 8.39 (1H, d, J = 10.2 Hz), 7.08 (1H, d, J = 9.8 Hz), 6.85 (1H, s), 6.29 (1H, t, J = 5.6 Hz), 4.33 (1H, q, J = 6.5 Hz), 3.64-3.55 (6H, m), 3.41 (3H, s), 3.05 (4H, t, J = 5.1 Hz), 1.79-1.70 (2H, m), 1.50 (3H, d, J = 6.3 Hz), 1.05 (3H, t, J = 7.6 Hz) . | 424.3 | 423.25 |
| 820 | 1H-NMR (CDCl3) δ: 9.10 (1H, s), 8.41 (1H, d, J = 8.8 Hz), 8.33 (2H, d, J = 2.9 Hz), 7.79 (1H, dd, J = 9.3, 2.4 Hz), 7.11 (1H, s), 6.70-6.38 (2H, m), 3.78-3.63 (6H, m), 3.27 (2H, t, J = 5.4 Hz), 1.36 (3H, t, J = 7.3 Hz). | 415.2 | 414.17 |
| 821 | 1H-NMR (CDCl3) δ: 9.15 (1H, s), 9.00 (1H, s), 8.43-8.36 (2H, m), 7.76 (1H, dd, J = 8.8, 2.0 Hz), 7.09 (1H, s), 6.69-6.40 (2H, m), 3.78-3.72 (4H, m), 3.65-3.56 (2H, m), 3.28 (2H, t, J = 5.4 Hz), 1.80-1.70 (2H, m), 1.07 (3H, t, J = 7.3 Hz). | 429.3 | 428.19 |
| 822 | 1H-NMR (CDCl3) δ: 9.14 (1H, s), 8.66 (1H, s), 8.43-8.36 (2H, m) , 7.78 (1H, dd, J = 8.8, 2.9 Hz), 7.14 (1H, s), 6.54 (1H, t, J = 56.1 Hz), 6.37 (1H, d, J = 7.8 Hz), 4.39-4.32 (1H, m), 4.09-4.01 (2H, m), 3.79-3.72 (4H, m), 3.68-3.62 (2H, m), 3.28 (2H, t, J = 5.4 Hz), 2.21-2.13 (2H, m), 1.72-1.63 (2H, m). | 471.3 | 470.20 |

**[Table 5-157]**

| Compound No. | NMR | LC/MS (M+H)⁺ | Exact Mass |
|---|---|---|---|
| 823 | 1H-NMR (CDCl3) δ: 9.13 (1H, s), 8.56 (1H, s), 8.42-8.33 (2H, m), 7.76 (1H, dd, J = 8.8, 2.9 Hz), 7.13 (1H, s), 6.69-6.41 (2H, m), 4.03 (2H, dd, J = 11.2, 3.4 Hz), 3.79-3.72 (4H, m), 3.59 (2H, t, J = 6.3 Hz), 3.43 (2H, td, J = 11.7, 2.0 Hz), 3.28 (2H, t, J = 5.4 Hz), 2.04-1.97 (1H, m), 1.76-1.70 (1H, m), 1.54-1.42 (3H, m). | 485.3 | 484.21 |
| 824 | 1H-NMR (CDCl3) δ: 8.98 (1H, s), 8.26 (1H, d, J = 8.8 Hz), 8.15 (1H, s), 7.54 (1H, d, J = 8.8 Hz), 6.64 (1H, s), 6.11 (1H, d, J = 7.8 Hz), 4.42-4.32 (1H, m), 4.21-3.90 (4H, m), 3.75-3.44 (5H, m), 3.29-3.22 (2H, m), 2.42-2.21 (5H, m), 1.35 (6H, d, J = 6.3 Hz) . | 463.4 | 462.25 |
| 825 | 1H-NMR (DMSO-D6) δ: 10.21 (1H, s), 9.26 (1H, s), 8.33-8.26 (2H, m), 7.79 (1H, d, J = 8.0 Hz), 6.99 (1H, s), 6.42 (1H, d, J = 7.3 Hz), 5.20 (1H, d, J = 4.9 Hz), 4.65-4.58 (1H, m), 4.29-4.22 (1H, m), 3.72-3.54 (3H, m), 3.10-2.97 (2H, m), 2.62-2.55 (1H, m), 2.15 (1H, q, J = 8.8 Hz), 1.99-1.90 (1H, m), 1.87-1.77 (2H, m), 1.51-1.42 (1H, m) , 1.38 (3H, d, J = 6.3 Hz), 1.29 (6H, d, J = 6.3 Hz). | 463.3 | 462.25 |
| 826 | 1H-NMR (DMSO-D6) δ: 10.19 (1H, s), 9.25 (1H, s), 8.40 (1H, d, J = 8.8 Hz), 8.29 (1H, d, J = 2.4 Hz), 7.80-7.76 (1H, m), 6.98 (1H, s), 6.84 (1H, t, J = 5.9 Hz), 5.18 (1H, d, J = 4.4 Hz), 4.60 (1H, t, J = 5.1 Hz), 3.64 (2H, t, J = 5.4 Hz), 3.45-3.24 (4H, m), 3.03 (2H, t, J = 5.4 Hz), 2.06-1.96 (1H, m) , 1.38 (3H, d, J = 6.3 Hz), 0.95 (6H, d, J = 8.0 Hz). | 437.3 | 436.23 |

**[Table 5-158]**

| Compound No. | NMR | LC/MS (M+H)⁺ | Exact Mass |
|---|---|---|---|
| 827 | 1H-NMR (DMSO-D6) δ: 10.19 (1H, s), 9.19 (1H, s), 8.23 (1H, d, J = 9.8 Hz), 7.31 (1H, d, J = 10.2 Hz), 6.94 (1H, s), 6.72 (1H, t, J = 6.1 Hz), 5.16 (1H, s), 4.58 (1H, q, J = 6.3 Hz), 3.45-3.27 (7H, m), 2.81 (4H, t, J = 4.9 Hz), 2.01-1.95 (1H, m), 1.36 (3H, d, J = 6.3 Hz), 0.93 (6H, d, J = 9.8 Hz). | 424.3 | 423.25 |
| 828 | | 481.3 | 480.26 |
| 829 | | 466.3 | 465.25 |
| 830 | | 480.3 | 479.26 |
| 831 | | 467.3 | 466.23 |
| 832 | | 467.2 | 466.23 |
| 833 | 1H-NMR (DMSO-D6) δ: 10.19 (1H, s), 9.25 (1H, s), 8.33-8.28 (2H, m), 7.82 (1H, dd, J = 8.8, 2.9 Hz), 6.99 (1H, s), 6.42 (1H, d, J = 7.3 Hz), 4.61 (1H, q, J = 6.3 Hz), 4.31-4.22 (1H, m), 3.67 (2H, t, J = 5.4 Hz), 3.37 (2H, s), 3.11 (2H, s), 2.95 (2H, t, J = 5.1 Hz), 1.38 (3H, d, J = 6.8 Hz), 1.29 (6H, dd, J = 6.6, 2.2 Hz) . | 481.3 | 480.22 |
| 834 | | 508.3 | 507.30 |
| 835 | 1H-NMR (DMSO-D6) δ: 9.94 (1H, s), 9.21 (1H, s), 8.13 (1H, d, J = 9.3 Hz), 8.08 (1H, d, J = 2.9 Hz), 7.48 (1H, dd, J = 9.3, 2.9 Hz), 6.96 (1H, s), 6.33 (1H, d, J = 7.8 Hz), 4.61 (1H, q, J = 6.5 Hz), 4.25 (1H, td, J = 13.4, 6.5 Hz), 3.63 (1H, d, J = 8.0 Hz), 3.45 (1H, d, J = 12.2 Hz), 2.89 (1H, t, J = 11.0 Hz), 2.75 (1H, t, J = 11.0 Hz), 2.56-2.50 (1H, m), 1.94-1.88 (1H, m), 1.77-1.71 (1H, m), 1.63-1.50 (2H, m), 1.38 (3H, d, J = 6.3 Hz), 1.29 (6H, dd, J = 6.3, 2.1 Hz) . | 453.3 | 451.23 |

**[Table 5-159]**

| Compound No. | NMR | LC/MS (M+H)⁺ | Exact Mass |
|---|---|---|---|
| 836 | 1H-NMR (DMSO-D6) δ: 9.80 (1H, s), 9.18 (1H, s), 8.10 (1H, d, J = 9.3 Hz), 8.03 (1H, d, J = 2.9 Hz), 7.48 (1H, dd, J = 9.3, 2.9 Hz), 6.95 (1H, s), 6.32 (1H, d, J = 7.8 Hz), 4.60 (1H, q, J = 6.5 Hz), 4.25 (1H, td, J = 13.2, 6.3 Hz), 3.61-3.55 (2H, m) , 2.73 (2H, t, J = 11.2 Hz), 2.35-2.28 (1H, m) , 1.91 (2H, d, J = 10.2 Hz) , 1.73-1.61 (2H, m) , 1.38 (3H, d, J = 6.3 Hz), 1.29 (6H, dd, J = 6.3, 2.2 Hz) . | 452.2 | 451.23 |
| 837 | | 480.4 | 479.26 |
| 838 | 1H-NMR (DMSO-D6) δ: 10.05 (1H, s), 9.76 (OH, s), 9.23 (1H, s), 8.57 (1H, d, J = 2.0 Hz), 8.23 (1H, d, J = 9.3 Hz), 8.13-8.09 (1H, m), 6.88 (1H, s), 6.45 (1H, d, J = 7.8 Hz), 4.25 (2H, dd, J = 10.7, 6.3 Hz), 3.32 (5H, s), 3.27-3.21 (5H, m) , 2.97 (1H, d, J = 12.7 Hz), 2.56 (1H, t, J = 11.0 Hz), 1.79 (2H, d, J = 25.1 Hz), 1.52-1.35 (8H, m), 1.34-1.25 (7H, m). | 465.3 | 464.26 |
| 839 | 1H-NMR (DMSO-D6) δ: 9.62 (1H, s), 9.12 (1H, s), 8.69 (2H, s), 6.83 (1H, s), 6.26 (1H, d, J = 7.8 Hz), 4.21 (2H, q, J = 6.3 Hz), 3.61 (4H, t, J = 4.9 Hz), 3.25 (3H, s), 2.73 (4H, t, J = 4.9 Hz), 1.36 (3H, d, J = 6.3 Hz), 1.25 (6H, dd, J = 6.3, 3.4 Hz). | 424.3 | 525.27 |
| 840 | | 519.2 | 518.18 |
| 841 | 1H-NMR (CDCl3) δ: 9.48 (1H, s), 9.32 (1H, s), 8.41-8.33 (2H, m), 7.68 (1H, dd, J = 8.8, 2.9 Hz), 6.29 (1H, d, J = 7.8 Hz), 5.16 (1H, d, J = 5.9 Hz), 4.37 (2H, td, J = 13.4, 6.5 Hz), 3.92 (2H, br s), 3.70 (2H, t, J = 5.4 Hz), 2.96-2.88 (6H, m), 2.74 (2H, t, J = 4.9 Hz), 1.47 (3H, d, J = 6.8 Hz), 1.38 (6H, d, J = 5.9 Hz). | 515.3 | 514.22 |

**[Table 5-160]**

| Compound No. | NMR | LC/MS (M+H) ⁺ | Exact Mass |
|---|---|---|---|
| 842 | 1H-NMR (CDCl3) δ: 9.48 (1H, s), 8.88 (1H, s), 8.50 (1H, s), 8.44 (1H, d, J = 8.8 Hz), 7.88 (1H, dd, J = 8.8, 2.0 Hz), 6.16 (1H, d, J = 6.8 Hz), 5.00 (1H, q, J = 6.2 Hz), 4.51-4.42 (1H, m) , 3.69 (4H, br s), 3.32 (3H, s), 2.94 (4H, br s), 1.53 (3H, d, J = 6.8 Hz), 1.36 (6H, t, J = 6.3 Hz). | 485.3 | 484.21 |
| 843 | 1H-NMR (CDC13) δ: 9.10 (1H, s), 9.09 (1H, s), 8.39-8.32 (2H, m), 7.76 (1H, dd, J = 8.3, 2.4 Hz), 6.72 (1H, s), 6.36 (1H, t, J = 5.9 Hz), 4.82 (1H, q, J = 6.2 Hz), 3.72-3.61 (4H, m) , 3.52 (2H, s), 2.63-2.49 (10H, m), 1.54 (3H, d, J = 6.8 Hz), 1.38 (3H, t, J = 6.8 Hz) . | 453.3 | 452.26 |
| 844 | 1H-NMR (CDCl3) δ: 9.18 (1H, s), 9.11 (1H, s), 8.35 (2H, t, J = 3.9 Hz), 7.74 (1H, d, J = 8.8 Hz), 6.71 (1H, s), 6.44 (1H, t, J = 5.4 Hz), 4.81 (1H, q, J = 6.2 Hz), 3.66-3.57 (4H, m), 3.52 (2H, s), 2.65-2.47 (11H, br m) , 1.84-1.72 (2H, m), 1.54 (3H, d, J = 6.8 Hz), 1.08 (3H, t, J = 7.3 Hz). | 467.3 | 466.28 |
| 845 | 1H-NMR (CDCl3) δ: 9.17 (1H, s), 9.11 (1H, s), 8.35 (2H, t, J = 3.9 Hz), 7.74 (1H, d, J = 8.8 Hz), 6.71 (1H, s), 6.42 (1H, t, J = 5.9 Hz), 4.82 (1H, q, J = 6.5 Hz), 3.69-3.60 (4H, m), 3.52 (2H, s), 2.64-2.47 (11H, br m), 1.78-1.71 (2H, m), 1.58-1.46 (5H, m), 1.03 (3H, t, J = 7.3 Hz). | 481.4 | 480.30 |
| 846 | 1H-NMR (CDCl3) δ: 9.09 (1H, s), 8.87 (1H, s), 8.36 (1H, d, J = 8.8 Hz), 8.33 (1H, d, J = 2.9 Hz), 7.73 (1H, dd, J = 8.3, 2.4 Hz), 6.71 (1H, s), 6.53 (1H, t, J = 5.9 Hz), 4.81 (1H, q, J = 6.2 Hz) , 3. 62 (2H, t, J = 5.4 Hz), 3.54-3.45 (4H, m), 2.62-2.50 (11H, br m) , 2.11-2.01 (1H, m), 1.54 (3H, d, J = 5.9 Hz), 1.08 (6H, d, J = 6.8 Hz) . | 481.4 | 480.30 |

**[Table 5-161]**

| Compound No. | NMR | LC/MS (M+H) ⁺ | Exact Mass |
|---|---|---|---|
| 847 | 1H-NMR (CDCl3) δ: 9.10 (1H, s), 8.82 (1H, s), 8.36-8.31 (2H, m), 7.74 (1H, dd, J = 8.8, 2.0 Hz), 6.74 (1H, s), 6.55 (1H, t, J = 5.9 Hz), 4.82 (1H, q, J = 6.5 Hz), 4.03 (2H, dd, J = 11.2, 3.4 Hz), 3.66-3.55 (4H, m), 3.52 (2H, s), 3.43 (2H, td, J = 11.7, 2.0 Hz), 2.63-2.50 (11H, br m), 2.07-1.96 (1H, m), 1.79-1.71 (2H, m), 1.57-1.43 (5H, m). | 523.4 | 522.31 |
| 848 | 1H-NMR (CDC13) δ: 9.04 (1H, s), 8.35 (1H, d, J = 8.8 Hz), 8.26 (1H, d, J = 8.8 Hz), 7.53 (1H, d, J = 7.8 Hz), 6.69 (1H, s), 6.49 (1H, s), 4.81 (1H, q, J = 6.5 Hz), 3.74 (2H, s), 3.67-3.61 (1H, m), 3.56-3.46 (1H, m), 3.29-3.23 (2H, m), 2.42 (3H, s), 1.59 (9H, s), 1.53 (3H, d, J = 5.9 Hz). | 451.3 | 450.25 |
| 849 | 1H-NMR (CDCl3) δ: 9.14 (1H, s) , 8.67 (1H, s), 8.46 (1H, d, J = 9.8 Hz), 8.37 (1H, d, J = 2.0 Hz), 7.77 (1H, dd, J = 9.8, 2.0 Hz), 7.14 (1H, s), 6.74 (1H, d, J = 8.8 Hz), 6.53 (1H, t, J = 55.6 Hz), 4.37 (1H, br s), 4.00 (1H, dd, J = 10.7, 2.9 Hz), 3.81-3.72 (6H, m), 3.64 (1H, dd, J = 11.2, 5.4 Hz) , 3.28 (2H, t, J = 5.9 Hz), 2.06-2.00 (1H, m), 1.94-1.84 (2H, m), 1.73-1.63 (1H, m). | 471.3 | 470.20 |
| 850 | | 463.2 | 462.15 |
| 851 | 1H-NMR (CDCl3) δ: 9.44 (1H, s), 8.33 (1H, s), 8.23 (1H, d, J = 8.8 Hz), 7.57 (1H, d, J = 7.8 Hz), 6.95 (1H, t, J = 54.2 Hz), 6.28 (1H, d, J = 7.8 Hz), 4.46-4.38 (1H, m), 3.74 (2H, s), 3.67-3.61 (1H, br m), 3.56-3.47 (1H, br m), 3.29-3.23 (2H, m), 2.42 (3H, s), 1.35 (6H, d, J = 6.8 Hz). | 477.2 | 476.17 |

**[Table 5-162**

| Compound No. | NMR | LC/MS (M+H) ⁺ | Exact Mass |
|---|---|---|---|
| 852 | 1H-NMR (CDCl3) δ: 9.03 (1H, s), 8.36 (1H, d, J = 8.8 Hz), 8.25 (1H, s), 8.14 (1H, s), 7.73 (1H, d, J = 8.8 Hz), 6.85 (1H, s), 6.34 (1H, s), 4.34 (1H, d, J = 6.8 Hz), 3.69-3.54 (7H, m) , 3.41 (3H, s), 2.70-2.56 (10H, br m), 1.77 (2H, d, J = 6.8 Hz), 1.51 (3H, d, J = 5.9 Hz), 1.08 (3H, t, J = 7.3 Hz). | 481.4 | 480.30 |
| 853 | 1H-NMR (CDC13) δ: 9.04 (1H, s), 8.35 (1H, d, J = 8.8 Hz), 8.29-8.22 (2H, m) , 7.73 (1H, d, J = 7.8 Hz), 6.85 (1H, s), 6.31 (1H, s), 4.37-4.30 (1H, m), 3.69-3.61 (4H, br m), 3.53 (2H, s), 3.41 (3H, s), 2.69-2.53 (10H, br m), 1.76-1.70 (2H, m), 1.55-1.48 (5H, m) , 1.25 (1H, s), 1.02 (3H, t, J = 7.3 Hz). | 495.4 | 494.31 |
| 854 | 1H-NMR (CDCl3) δ: 9.12 (1H, s), 9.04 (1H, s), 8.39-8.33 (2H, m), 7.74 (1H, dd, J = 8.8, 2.0 Hz), 6.86 (1H, s) , 6.32 (1H, t, J = 5.4 Hz), 4.34 (1H, q, J = 6.5 Hz), 3.68-3.61 (2H, m) , 3.50 (2H, s), 3.42 (3H, s), 2.92 (4H, br s), 2.47 (4H, br s), 2.12 (1H, br s), 1.72 (2H, td, J = 14.4, 6.8 Hz), 1.56-1.46 (5H, m), 1.02 (3H, t, J = 7.3 Hz). | 451.4 | 450.29 |
| 855 | 1H-NMR (CDCl3) δ: 9.07 (1H, s), 8.57 (1H, s), 8.39 (1H, d, J = 9.8 Hz), 7.08 (1H, d, J = 9.8 Hz), 6.85 (1H, s), 6.25 (1H, t, J = 5.4 Hz), 4.33 (1H, q, J = 6.2 Hz), 3.66-3.57 (6H, m), 3.41 (3H, s), 3.08-3.02 (4H, m), 1.87 (1H, br s), 1.75-1.67 (2H, m), 1.54-1.43 (5H, m), 1.01 (3H, t, J = 7.3 Hz) . | 438.3 | 437.27 |

**[Table 5-163]**

| Compound No. | NMR | LC/MS (M+H) ⁺ | Exact Mass |
|---|---|---|---|
| 856 | 1H-NMR (CDCl3) δ: 9.14 (1H, s), 8.61 (1H, s), 8.38 (1H, d, J = 7.8 Hz), 8.33 (1H, d, J = 2.0 Hz), 7.77 (1H, dd, J = 8.8, 2.9 Hz), 7.14 (1H, s), 6.70 (1H, d, J = 7.8 Hz), 6.53 (1H, t, J = 55.6 Hz), 4.66-4.56 (2H, m), 4.41-4.33 (1H, m), 4.02 (1H, dd, J = 11.7, 2.9 Hz), 3.77 (2H, t, J = 4.9 Hz), 3.61 (3H, t, J = 8.3 Hz), 3.29 (2H, t, J = 5.4 Hz), 3.07 (2H, t, J = 5.4 Hz), 2.10-2.01 (1H, m), 1.93-1.84 (2H, m), 1.77-1.71 (1H, m). | 485.3 | 484.21 |
| 857 | 1H-NMR (CDCl3) δ: 9.08 (1H, s), 8.85 (1H, s), 8.35 (2H, d, J = 8.8 Hz), 7.76 (1H, d, J = 7.8 Hz), 6.84 (1H, s), 6.33 (1H, s), 4.34 (1H, q, J = 6.2 Hz), 3.78-3.56 (5H, m), 3.41 (4H, br s), 3.30-3.22 (1H, m), 2.94-2.85 (2H, m), 2.53 (1H, d, J = 8.8 Hz), 1.90-1.63 (5H, m), 1.50 (3H, d, J = 6.8 Hz), 1.06 (3H, t, J = 7.3 Hz). | 449.3 | 448.27 |
| 858 | 1H-NMR (CDCl3) δ: 9.02 (1H, s), 8.34 (1H, s), 8.31 (1H, s), 6.83 (1H, s), 6.77 (1H, d, J = 9.8 Hz), 6.28 (1H, s), 4.84 (1H, s), 4.33 (1H, q, J = 6.5 Hz), 3.90 (1H, s), 3.70 (1H, d, J = 8.8 Hz), 3.62-3.55 (2H, m), 3.40 (3H, s), 3.33 (1H, d, J = 8.8 Hz), 3.15 (2H, br s), 2.01-1.86 (2H, m), 1.74 (2H, td, J = 14.4, 7.2 Hz), 1.50 (3H, d, J = 6.8 Hz), 1.05 (3H, t, J = 7.8 Hz). | 436.3 | 435.25 |
| 859 | 1H-NMR (CDCl3) δ: 9.02 (1H, s), 8.41-8.30 (2H, m), 6.83 (1H, s), 6.77 (1H, d, J = 9.8 Hz), 6.09 (1H, d, J = 7.8 Hz), 4.84 (1H, s), 4.44-4.28 (2H, m), 3.89 (1H, s), 3.73-3.68 (1H, m), 3.41 (3H, s), 3.32 (1H, d, J = 8.8 Hz), 3.15 (2H, t, J = 10.7 Hz), 1.99-1.85 (3H, m), 1.50 (3H, d, J = 6.8 Hz), 1.34 (6H, t, J = 5.4 Hz). | 436.3 | 435.25 |

**[Table 5-164]**

| Compound No. | NMR | LC/MS (M+H) ⁺ | Exact Mass |
|---|---|---|---|
| 860 | 1H-NMR (CDCl3) δ: 9.05 (1H, s) , 8.38 (1H, d, J = 8.8 Hz), 8.27 (2H, br s), 7.72 (1H, d, J = 8.8 Hz), 6.88 (1H, s), 6.53 (1H, d, J = 6.8 Hz), 4.38-4.30 (2H, m), 4.05 (1H, d, J = 10.7 Hz), 3.80-3.69 (4H, m), 3.61-3.52 (3H, m), 3.40 (3H, s), 2.77-2.62 (8H, m), 2.10-2.02 (2H, m), 1.91-1.84 (2H, m), 1.77-1.70 (2H, m), 1.48 (3H, d, J = 5.9 Hz). | 523.4 | 522.31 |
| 861 | 1H-NMR (CDCl3) δ: 9.12 (2H, br s), 8.40-8.34 (2H, m), 7.73 (1H, d, J = 8.8 Hz), 6.85 (1H, s), 6.43 (1H, t, J = 5.4 Hz), 4.34 (1H, q, J = 6.5 Hz), 3.55-3.46 (4H, m), 3.41 (3H, s), 2.93 (4H, br s), 2.48 (4H, br s), 2.30 (1H, s), 2.08-1.98 (1H, m), 1.51 (3H, d, J = 5.9 Hz), 1.07 (6H, d, J = 6.8 Hz) . | 451.4 | 450.29 |
| 862 | 1H-NMR (CDCl3) δ: 9.08 (1H, s), 8.62 (1H, s), 8.40 (1H, d, J = 9.8 Hz), 7.06 (1H, d, J = 9.8 Hz), 6.85 (1H, s), 6.36 (1H, t, J = 5.4 Hz), 4.33 (1H, q, J = 6.5 Hz), 3.59 (4H, br s), 3.51-3.44 (2H, m), 3.41 (3H, s), 3.06 (4H, br s), 2.07-1.97 (1H, m), 1.50 (3H, d, J = 5.9 Hz), 1.05 (6H, d, J = 6.8 Hz) . | 438.3 | 437.27 |
| 863 | | 478.3 | 477.22 |
| 864 | 1H-NMR (300 MHz, DMSO-d6, ppm) δ: 9.93 (1H, s), 9.21 (1H, s), 8.18 (1H, d, J = 8.7Hz), 8.09 (1H, s), 7.56 (1H, d, J = 8.7HZ), 6.97 (1H, s), 6.36 (1H, d, J = 6.9Hz), 5.17 (1H, d, J = 4.8Hz), 4.75 (1H, br), 4.61 (1H, br), 4.26 (2H, br), 3.23 (1H, br), 2.90 (1H, br), 2.35 (2H, br), 2.09 (2H, br), 1.62 (1H, br), 1.38 (3H, d, J = 6.3Hz), 1.30 (6H, d, J = 5.7Hz) | 442.1 | 441.23 |

**[Table 5-165]**

| Compound No. | NMR | LC/MS (M+H) ⁺ | Exact Mass |
|---|---|---|---|
| 865 | 1H-NMR (300 MHz, DMSO-d6, ppm) δ: 10.04 (1H, s), 9.25 (1H, s), 8.31-8.24 (2H, m), 7.86-7.83 (1H, m), 6.99 (1H, s), 6.40 (1H, d, J = 7.8Hz), 6.07-6.04 (1H, m), 5.20-5.18 (1H, m), 4.66-4.61 (1H, m), 4.31-4.24 (1H, m), 3.83-3.69(3H, m), 3.51-3.43 (1H, m), 3.26-3.19 (1H, m), 2.87 (2, br), 1.40-1.36 (3H, m), 1.31 (3H, d, J = 6.6Hz) | 410.3 | 409.22 |
| 866 | 1H-NMR (300 MHz, DMSO-d6, ppm) δ: 10.04 (1H, s), 9.25 (1H, s), 8.31-8.24 (2H, m), 7.86-7.83 (1H, m), 6.99 (1H, s), 6.40 (1H, d, J = 7.8Hz), 6.07-6.04 (1H, m), 5.20-5.18 (1H, m), 4.66-4.61 (1H, m), 4.31-4.24 (1H, m), 3.83-3.69(3H, m), 3.51-3.43 (1H, m), 3.26-3.19 (1H, m), 2.87 (2, br), 1.40-1.36 (3H, m), 1.31 (3H, d, J = 6.6Hz) | 410.3 | 409.22 |
| 867 | 1H-NMR (400 MHz, DMSO-d6, ppm) δ: 9.93 (1H, s), 9.22 (1H, s), 8.19 (1H, d, J =8.8Hz), 8.07 (1H, d, J = 3.2Hz), 7.56-7.51 (1H, m), 7.37-7.25 (1H, m), 6.98 (1H, s), 6.39-6.31 (1H, m), 5.18 (1H, d, J = 4.8Hz ), 4.66-4.59 (1H, m), 4.38-4.17(1H, m), 4.13-4.10 (2H, m), 3.36-3.35 (2H, m), 3.30 (1H, m), 2.97 (3H, s), 1.40-1.38 (3H, m), 1.31-1.29 (6H, m) | 462.0 | 461.18 |
| 868 | 1H-NMR (400 MHz, DMSO-d6, ppm) δ: 9.88 (1H, s), 9.28 (1H, s), 8.10-8.06 (2H, m),8.49-8.41 (1H, m), 7.19 (1H, s), 5.26-5.25 (1H, m), 4.96-4.88 (1H, m), 4.69-4.60 (1H, m), 4.08-4.05 (2H, m), 3.88-3.82 (1H, m), 3.81-3.73 (1H, m), 3.55-3.48 (1H, m), 3.29-3.20 (1H, m), 3.14 (3H, s), 2.84-2.83 (2H, m), 2.34 (3H, m), 1.95-1.94 (2H, m) , 1.71-1.66 (1H, m), 1.52-1.42 (1H, m), 1.40-1.38 (3H, m) | 454.1 | 453.25 |

**[Table 5-166]**

| Compound No. | NMR | LC/MS (M+H) ⁺ | Exact Mass |
|---|---|---|---|
| 869 | 1H-NMR (400 MHz, DMSO-d6, ppm) δ: 10.09 (1H, s), 9.22 (1H, s), 8.19 (1H, d, J =9.2Hz ),8.07 (1H, d, J =3.2Hz ), 7.41-7.38 (1H, m), 7.00 (1H, s), 6.72 (1H, s), 5.19 (1H, d, J = 4.8Hz), 4.62-4.59 (1H, m), 4.11-4.08 (2H, m), 4.01-3.97 (1H, m), 3.81-3.78 (1H, m), 3.44-3.41 (2H, m), 2.89-2.86 (2H, m), 2.37 (3H, s), 1.60-1.43 (9H, m), 1.39-1.38 (2H, m) | 454.1 | 453.25 |
| 870 | 1H-NMR (400 MHz, DMSO-d6, ppm) δ: 10.09 (1H, s), 9.22 (1H, s), 8.19 (1H, d, J =9.2Hz ), 8.07 (1H, d, J = 3.2Hz ), 7.41-7.38 (1H, m), 7.00 (1H, s), 6.72 (1H, s), 5.20 (1H, d, J = 4.8Hz), 4.62-4.59 (1H, m), 4.12-4.09 (2H, m), 4.01-3.97 (1H, m), 3.81-3.78 (1H, m), 3.44-3.41 (2H, m) , 2.90 (2H, m), 2.38 (3H, s), 1.58-1.51 (6H, m), 1.50-1.41 (3H, m), 1.39-1.38 (2H, m) | 454.1 | 453.25 |
| 871 | 1H-NMR (400 MHz, DMSO-d6, ppm) δ: 10.18 (1H, s), 9.31 (1H, s), 8.30-8.23 (2H, m), 7.77-7.75 (1H, m), 7.17 (1H, s), 5.45-5.38 (1H, m), 5.25 (1H, d, J = 4.4Hz), 4.65-4.62 (1H, m), 3.65-3.63 (2H, m), 3.40 (2H, s), 3.07-3.03 (5H, m), 1.41-1.39 (3H, m), 1.24-1.18 (6H, m) | 437.3 | 436.23 |
| 872 | 1H-NMR (400 MHz, DMSO-d6, ppm) δ: 10.18 (1H, s), 9.33 (1H, s), 8.30 (1H, d, J = 2.8Hz), 8.23 (1H, d, J = 8.4Hz), 7.78-7.76 (1H, m), 7.22 (1H, s), 5.28 (1H, d, J = 4.8Hz), 4.94-'4.93 (1H, m), 4.67-4.63 (1H, m), 3.92-3.88 (1H, m), 3.77-3.75 (1H, m), 3.66-3.62 (2H, m), 3.56-3.51 (1H, m), 3.41 (2H, m), 3.26-3.23 (1H, m), 3.17 (3H, s), 3.03-3.02 (2H, m), 1.96-1.94 (2H, m), 1.73-1.67 (1H, m), 1.54-1.51 (1H, m), 1.45-1.39 (3H, m) | 479.1 | 478.24 |

**[Table 5-167]**

| Compound No. | NMR | LC/MS (M+H) ⁺ | Exact Mass |
|---|---|---|---|
| 873 | 1H-NMR (400 MHz, DMSO-d6, ppm) δ: 9.93 (1H, s), 9.21 (1H, s), 8.16 (1H, d, J = 9.2Hz), 8.0 (1H, d, J = 2.8Hz), 7.52-7.49 (1H, m), 6.89 (1H, s), 6.51 (1H, d, J = 7.6Hz), 4.23 (1H, d, J = 6.4Hz), 4.20 (2H, br), 3.91-3.88 (2H, br), 3.51-3.46 (2H, m), 3.25 (3H, s), 2.95-2.92 (2H, br), 2.57-2.50 (2H, m), 2.02-1.99 (2H, br), 1.92-1.98 (2H, br), 1.64 (2H, br), 1.46-1.43 (2H, m), 1.36 (3H, d, J = 6.4Hz) | 480.2 | 479.26 |
| 874 | 1H-NMR (400 MHz, DMSO-d6, ppm) δ: 9.95 (1H, s), 9.17 (1H, s), 8.07 (1H, d, J = 8.8Hz), 7.99 (1H, d, J = 3.2Hz), 7.42-7.39 (1H, m), 6.85 (1H, s), 6.56 (1H, d, J = 8.4Hz), 4.35-4.33 (1H, m), 4.18-4.17 (1H, m) , 4.08-4.07 (1H, br), 3.88-3.84 (1H, m), 3.63-3.48 (1H, m), 3.37-3.34 (1H, m), 3.20 (3H, s), 2.91-2.86 (2H, m), 2.52-2.43 (2H, m), 1.93-1.92 (1H, br), 1.87-1.83 (2H, m), 1.74-1.68 (2H, br), 1.58-1.52 (1H, br), 1.42-1.35 (2H, m) , 1.32 (3H, d, J = 6.4Hz) | 480.2 | 479.26 |
| 875 | 1H-NMR (CDC13) δ: 8.98 (1H, s), 8.27 (1H, d, J = 8.8 Hz), 8.16 (1H, s), 7.54 (1H, d, J = 8.3 Hz), 6.64 (1H, s), 6.11 (1H, d, J = 7.3 Hz), 4.42-4.32 (1H, m), 4.19 (1H, t, J = 8.0 Hz), 4.11-4.04 (1H, m), 4.02-3.89 (2H, m), 3.74 (2H, s), 3.69-3.60 (1H, m), 3.57-3.43 (2H, m), 3.30-3.22 (2H, m), 2.41 (3H, s), 2.36-2.22 (2H, m), 1.35 (6H, d, J = 6.3 Hz) . | 463.3 | 462.25 |
| 876 | 1H-NMR (CDC13) δ: 8.98 (1H, s), 8.26 (1H, d, J = 8.3 Hz), 8.16 (1H, s), 7.54 (1H, d, J = 8.8 Hz), 6.64 (1H, s), 6.11 (1H, d, J = 7.3 Hz), 4.43-4.32 (1H, m), 4.19 (1H, t, J = 7.8 Hz), 4.12-4.04 (1H, m), 4.02-3.89 (2H, m), 3.74 (2H, s), 3.69-3.60 (1H, m), 3.57-3.44 (2H, m), 3.29-3.22 (2H, m), 2.41 (3H, s), 2.36-2.22 (2H, m), 1.35 (6H, d, J = 6.3 Hz). | 463.3 | 462.25 |

**[Table 5-168]**

| Compound No. | NMR | LC/MS (M+H) ⁺ | Exact Mass |
|---|---|---|---|
| 877 | 1H-NMR (CDCl3) δ: 8.95 (1H, s), 8.38 (1H, d, J = 9.8 Hz), 8.23 (1H, br s), 7.08 (1H, d, J = 9.8 Hz), 6.64 (1H, s), 6.08 (1H, d, J = 7.3 Hz), 4.42-4.32 (1H, m), 4.18 (1H, t, J = 8.0 Hz), 4.12-4.04 (1H, m), 4.02-3.89 (2H, m), 3.63-3.56 (4H, m), 3.53-3.43 (1H, m), 3.10-3.03 (4H, m), 2.36-2.22 (2H, m), 1.35 (6H, d, J = 6.3 Hz). | 436.3 | 435.25 |
| 878 | 1H-NMR (CDCl3) δ: 8.97 (1H, s), 8.41 (1H, d, J = 9.8 Hz), 8.27 (1H, s), 7.07 (1H, d, J = 9.8 Hz), 6.67 (1H, s), 6.48 (1H, t, J = 9.3 Hz), 4.36-4.26 (1H, br m), 4.17 (1H, t, J = 8.0 Hz), 4.11-3.93 (3H, m), 3.89 (1H, t, J = 8.0 Hz), 3.82-3.68 (2H, m), 3.65-3.42 (6H, m), 3.07-3.01 (4H, m), 2.31-2.23 (2H, m), 2.09-1.98 (1H, m), 1.92-1.79 (2H, m), 1.75-1.63 (1H, m). | 478.3 | 477.26 |
| 879 | 1H-NMR (CDCl3) δ: 8.98 (1H, s), 8.38 (1H, d, J = 9.8 Hz), 8.22 (1H, s), 7.08 (1H, d, J = 9.8 Hz), 6.62 (1H, s), 6.11 (1H, d, J = 7.3 Hz), 5.16 (2H, d, J = 5.4 Hz), 4.58 (2H, d, J = 5.4 Hz), 4.47-4.36 (1H, m), 3.61-3.56 (4H, m), 3.07-3.02 (4H, m), 1.79 (3H, s), 1.36 (6H, d, J = 6.3 Hz). | 436.3 | 435.25 |
| 880 | 1H-NMR (CDCl3) δ: 9.00 (1H, s), 8.42 (1H, d, J = 9.8 Hz), 8.26 (1H, s), 7.07 (1H, d, J = 9.8 Hz), 6.65 (1H, s), 6.53 (1H, d, J = 7.8 Hz), 5.14-5.10 (2H, m), 4.60-4.56 (2H, m), 4.40-4.30 (1H, br m), 4.06-4.01 (1H, m), 3.80-3.71 (2H, br m), 3.67-3.55 (5H, m), 3.08-3.01 (4H, m), 2.09-1.98 (1H, m), 1.94-1.83 (2H, m), 1.78 (3H, s), 1.75-1.61 (1H, m). | 478.3 | 477.26 |

**[Table 5-169]**

| Compound No. | NMR | LC/MS (M+H)⁺ | Exact Mass |
|---|---|---|---|
| 881 | 1H-NMR (CDCl3) δ: 8.98 (1H, s), 8.34 (1H, d, J = 8.8 Hz), 8.29 (1H, s), 8.26 (1H, d, J = 2.0 Hz), 7.75 (1H, dd, J = 8.8, 2.4 Hz), 6.65 (1H, s), 6.13 (1H, d, J = 7.3 Hz), 4.44-4.33 (1H, m), 4.19 (1H, t, J = 7.8 Hz), 4.12-4.05 (1H, m), 4.03-3.90 (2H, m), 3.54-3.44 (3H, m), 2.93-2.88 (4H, m), 2.51-2.22 (6H, m), 1.36 (6H, d, J = 6.8 Hz). | 449.4 | 448.27 |
| 882 | 1H-NMR (CDCl3) δ: 9.01 (1H, s), 8.40-8.35 (2H, m), 8.28 (1H, d, J = 2.0 Hz), 7.74 (1H, dd, J = 8.8, 2.4 Hz), 6.69 (1H, s), 6.56-6.49 (1H, m), 4.37-4.29 (1H, br m), 4.18 (1H, t, J = 8.0 Hz), 4.11-3.94 (3H, m), 3.90 (1H, t, J = 7.8 Hz), 3.82-3.71 (2H, m), 3.65-3.56 (1H, m), 3.53-3.44 (3H, m), 2.93-2.87 (4H, m), 2.50-2.39 (4H, br m), 2.33-2.24 (2H, m), 2.12-2.01 (1H, m), 1.95-1.80 (2H, m), 1.78-1.64 (1H, m). | 491.4 | 490.28 |
| 883 | 1H-NMR (CDCl3) δ: 9.01 (1H, s), 8.38-8.32 (2H, m), 8.27 (1H, d, J = 2.0 Hz), 7.76 (1H, dd, J = 8.5, 2.2 Hz), 6.63 (1H, s), 6.16 (1H, d, J = 7.3 Hz), 5.17 (2H, d, J = 4.9 Hz), 4.58 (2H, d, J = 5.4 Hz), 4.48-4.38 (1H, m), 3.49 (2H, s), 2.93-2.88 (4H, m), 2.50-2.40 (4H, br m), 1.80 (3H, s), 1.38 (6H, d, J = 6.8 Hz). | 449.3 | 448.27 |
| 884 | 1H-NMR (CDC13) δ: 9.03 (1H, s), 8.40-8.35 (2H, m), 8.28 (1H, d, J = 2.0 Hz), 7.75 (1H, dd, J = 8.5, 2.2 Hz), 6.66 (1H, s), 6.57 (1H, d, J = 7.8 Hz), 5.15-5.11 (2H, m), 4.59 (2H, d, J = 5.4 Hz), 4.41-4.32 (1H, m), 4.06 (1H, dd, J = 11.0, 3.2 Hz), 3.83-3.71 (2H, m), 3.63 (1H, dd, J = 11.0, 6.1 Hz), 3.49 (2H, s), 2.94-2.88 (4H, m), 2.50-2.41 (4H, br m), 2.13-2.02 (1H, m), 1.96-1.83 (2H, m), 1.81-1.65 (4H, m). | 491.4 | 490.28 |

**[Table 5-170]**

| Compound No. | NMR | LC/MS (M+H)⁺ | Exact Mass |
|---|---|---|---|
| 885 | 1H-NMR (CDCl3) δ: 8.95 (1H, s), 8.13 (1H, d, J = 8.3 Hz), 8.03 (1H, s), 7.40 (1H, d, J = 8.8 Hz), 6.63 (1H, s), 6.12 (1H, d, J = 7.3 Hz), 4.42-4.31 (1H, m), 4.19 (1H, t, J = 8.0 Hz), 4.12-4.04 (1H, m), 4.03-3.89 (4H, m), 3.53-3.43 (1H, m), 3.24 (2H, t, J = 6.1 Hz), 2.87 (2H, t, J = 6.1 Hz), 2.37-2.21 (2H, m), 1.35 (6H, d, J = 6.3 Hz). | 406.3 | 405.23 |
| 886 | 1H-NMR (CDCl3) δ: 8.96 (1H, s), 8.17 (1H, d, J = 8.3 Hz), 8.05 (1H, s), 7.40 (1H, d, J = 8.8 Hz), 6.67 (1H, s), 6.58-6.49 (1H, m), 4.35-4.27 (1H, br m), 4.17 (1H, t, J = 8.0 Hz), 4.10-3.93 (5H, m), 3.89 (1H, t, J = 7.8 Hz), 3.81-3.69 (2H, m), 3.65-3.56 (1H, m), 3.52-3.43 (1H, m), 3.24 (2H, t, J = 6.1 Hz), 2.87 (2H, t, J = 6.1 Hz), 2.32-2.22 (2H, m), 2.10-1.99 (1H, m), 1.93-1.79 (2H, m), 1.77-1.61 (1H, m). | 448.3 | 447.24 |
| 887 | 1H-NMR (CDCl3) δ: 8.97 (1H, s), 8.14 (1H, d, J = 8.3 Hz), 8.03 (1H, s), 7.41 (1H, d, J = 8.3 Hz), 6.61 (1H, s), 6.16 (1H, d, J = 7.3 Hz), 5.16 (2H, d, J = 5.4 Hz), 4.58 (2H, d, J = 5.4 Hz), 4.46-4.36 (1H, m), 4.01 (2H, s), 3.24 (2H, t, J = 6.1 Hz), 2.88 (2H, t, J = 6.1 Hz), 1.80 (3H, s), 1.36 (6H, d, J = 6.8 Hz). | 406.3 | 405.23 |
| 888 | 1H-NMR (CDCl3) δ: 8.99 (1H, s), 8.18 (1H, d, J = 8.3 Hz), 8.06 (1H, s), 7.41 (1H, d, J = 8.8 Hz), 6.64 (1H, s), 6.58 (1H, d, J = 7.8 Hz), 5.15-5.10 (2H, m), 4.58 (2H, d, J = 5.4 Hz), 4.39-4.31 (1H, m), 4.06-3.99 (3H, m), 3.76 (2H, t, J = 5.1 Hz), 3.63 (1H, dd, J = 11.0, 5.6 Hz), 3.25 (2H, t, J = 6.1 Hz), 2.88 (2H, t, J = 5.9 Hz), 2.11-2.01 (1H, m), 1.95-1.81 (2H, m), 1.80-1.63 (4H, m). | 448.3 | 447.24 |

**[Table 5-171]**

| Compound No. | NMR | LC/MS (M+H)⁺ | Exact Mass |
|---|---|---|---|
| 889 | 1H-NMR (CD3OD) δ: 9.00 (1H, s), 8.15 (1H, d, J = 8.8 Hz), 8.04 (1H, d, J = 2.4 Hz), 7.61 (1H, dd, J = 9.0, 2.7 Hz), 6.72 (1H, s), 4.59-4.49 (1H, br m), 4.40-4.29 (1H, m), 4.17-4.01 (2H, m), 3.99-3.85 (2H, m), 3.55-3.15 (6H, m), 3.04-2.80 (2H, m), 2.68-2.59 (1H, m), 2.35-2.21 (2H, m), 1.38-1.29 (6H, m). | 561.3 | 560.25 |
| 890 | 1H-NMR (CD3OD) δ: 9.05 (1H, s), 8.17 (1H, d, J = 9.3 Hz), 8.06 (1H, d, J = 2.4 Hz), 7.63 (1H, dd, J = 9.3, 2.9 Hz), 6.74 (1H, s), 5.16 (2H, d, J = 5.4 Hz), 4.58 (2H, d, J = 5.9 Hz), 4.53-4.35 (2H, m), 3.51-3.42 (1H, m), 3.38-3.20 (2H, m), 3.13-3.03 (2H, m), 2.97-2.91 (1H, m), 2.87-2.73 (1H, m), 2.59-2.50 (1H, m), 1.78 (3H, s), 1.36 (6H, d, J = 6.3 Hz). | 561.3 | 560.25 |
| 891 | 1H-NMR (CD3OD) δ: 9.07 (1H, s), 8.15 (1H, d, J = 9.3 Hz), 8.05 (1H, d, J = 2.9 Hz), 7.62 (1H, dd, J = 9.3, 2.9 Hz), 6.85 (1H, s), 4.63-4.53 (1H, m), 4.43-4.28 (2H, m), 3.57-3.48 (1H, m), 3.44-3.21 (7H, m), 3.06-2.98 (1H, m), 2.92-2.85 (1H, m), 2.72-2.63 (1H, m), 1.46 (3H, d, J = 6.3 Hz), 1.36-1.30 (6H, m). | 549.3 | 548.25 |
| 892 | | 535.3 | 534.23 |
| 893 | 1H-NMR (DMSO-D6) δ: 9.92 (1H, s), 9.20 (1H, s), 8.12 (1H, d, J = 8.8 Hz), 8.06 (1H, d, J = 2.9 Hz), 7.49 (1H, dd, J = 9.3, 2.9 Hz), 6.86 (1H, s), 6.37 (1H, d, J = 7.8 Hz), 4.27-4.20 (2H, m), 3.62 (1H, d, J = 9.3 Hz), 3.44 (2H, d, J = 12.2 Hz), 3.26 (3H, s), 2.90 (1H, t, J = 10.0 Hz), 2.77 (1H, t, J = 9.5 Hz), 2.57-2.51 (1H, m), 1.93-1.88 (1H, m), 1.78-1.72 (1H, m), 1.63-1.50 (2H, m), 1.38 (3H, d, J = 6.8 Hz), 1.29 (6H, dd, J = 6.3, 3.4 Hz). | 466.3 | 465.25 |
| 894 | | 495.3 | 494.24 |

**[Table 5-172]**

| Compound No. | NMR | LC/MS (M+H)⁺ | Exact Mass |
|---|---|---|---|
| 895 | 1H-NMR (DMSO-D6) δ: 10.19 (1H, s), 9.25 (1H, s), 8.32-8.28 (2H, m), 7.82 (1H, dd, J = 8.8, 2.4 Hz), 6.99 (1H, s), 6.42 (1H, d, J = 7.8 Hz), 4.61 (1H, q, J = 6.3 Hz), 4.26 (1H, td, J = 13.3, 6.7 Hz), 3.67-3.60 (2H, m), 3.44 (2H, dd, J = 22.7, 16.8 Hz), 3.21 (1H, q, J = 7.2 Hz), 3.04-2.91 (2H, m), 1.38 (3H, d, J = 6.3 Hz), 1.29 (6H, dd, J = 6.3, 2.0 Hz), 1.20 (3H, d, J = 6.8 Hz). | 495.3 | 494.24 |
| 896 | 1H-NMR (DMSO-D6) δ: 10.28 (1H, s), 9.20 (1H, s), 8.22 (1H, d, J = 9.8 Hz), 7.46 (1H, d, J = 9.8 Hz), 6.96 (1H, s), 6.33 (1H, d, J = 6.8 Hz), 5.19 (1H, d, J = 4.9 Hz), 4.63-4.57 (1H, m), 4.32-4.20 (2H, m), 3.90 (1H, d, J = 11.7 Hz), 3.50 (1H, br s), 3.04-2.96 (4H, m), 2.80 (1H, d, J = 10.7 Hz), 1.35 (3H, dd, J = 18.1, 6.3 Hz), 1.27 (6H, d, J = 4.9 Hz). | 478.2 | 477.22 |
| 897 | | 509.3 | 508.25 |
| 898 | 1H-NMR (DMSO-D6) δ: 10.32 (1H, s), 9.21 (1H, s), 8.27 (1H, d, J = 9.8 Hz), 7.60 (1H, d, J = 10.7 Hz), 7.13 (1H, s), 6.97 (1H, s), 6.91 (1H, s), 6.34 (1H, d, J = 7.8 Hz), 5.19 (1H, s), 4.76 (2H, s), 4.63-4.57 (1H, m), 4.28-4.19 (1H, m), 4.15-4.10 (2H, m), 4.09-4.04 (2H, m), 1.37 (3H, d, J = 6.3 Hz), 1.27 (6H, d, J = 6.3 Hz). | 447.3 | 446.23 |
| 899 | 1H-NMR (DMSO-D6) δ: 10.24 (1H, s), 9.26 (1H, s), 8.35-8.29 (2H, m), 7.83 (1H, dd, J = 8.8, 2.9 Hz), 7.00 (1H, s), 6.43 (1H, d, J = 7.8 Hz), 5.21 (1H, d, J = 3.9 Hz), 4.65-4.57 (1H, m), 4.30-4.21 (1H, m), 3.92 (2H, s), 3.73 (2H, t, J = 5.4 Hz), 3.31 (2H, s), 2.92 (2H, t, J = 5.4 Hz), 1.38 (3H, d, J = 6.8 Hz), 1.29 (6H, dd, J = 6.8, 2.0 Hz). | 462.3 | 461.23 |

**[Table 5-173]**

| Compound No. | NMR | LC/MS (M+H)⁺ | Exact Mass |
|---|---|---|---|
| 900 | 1H-NMR (DMSO-D6) δ: 10.23 (1H, s), 9.26 (1H, s), 8.35-8.29 (2H, m), 7.81 (1H, dd, J = 8.8, 2.0 Hz), 7.41 (1H, s), 7.19 (1H, s), 6.99 (1H, s), 6.43 (1H, d, J = 7.8 Hz), 5.21 (1H, d, J = 4.9 Hz), 4.64-4.58 (1H, m), 4.31-4.21 (1H, m), 3.72 (2H, t, J = 5.4 Hz), 3.30 (2H, s), 3.06 (2H, s), 2.88 (2H, t, J = 4.9 Hz), 1.38 (3H, d, J = 6.8 Hz), 1.29 (6H, dd, J = 5.9, 2.0 Hz). | 480.3 | 479.24 |
| 901 | 1H-NMR (DMSO-D6) δ: 10.26 (1H, s), 9.20 (1H, s), 8.22 (1H, d, J = 9.8 Hz), 7.43 (1H, d, J = 9.8 Hz), 6.96 (1H, s), 6.33 (1H, d, J = 7.8 Hz), 5.19 (1H, d, J = 4.9 Hz), 4.63-4.57 (1H, m), 4.23 (1H, td, J = 13.4, 6.5 Hz), 3.81 (2H, s), 3.57 (4H, t, J = 4.4 Hz), 2.61 (4H, t, J = 4.4 Hz), 1.37 (3H, d, J = 6.3 Hz), 1.27 (6H, d, J = 5.9 Hz). | 449.3 | 448.24 |
| 902 | 1H-NMR (DMSO-D6) δ: 10.25 (1H, s), 9.20 (1H, s), 8.22 (1H, d, J = 9.8 Hz), 7.44 (1H, d, J = 9.8 Hz), 6.96 (1H, s), 6.33 (1H, d, J = 7.8 Hz), 5.19 (1H, d, J = 3.9 Hz), 4.63-4.57 (1H, m), 4.28-4.19 (1H, m), 4.06 (1H, q, J = 7.2 Hz), 3.64-3.52 (4H, m), 2.74-2.68 (2H, m), 2.56-2.51 (2H, m), 1.43-1.35 (6H, m), 1.27 (6H, d, J = 6.8 Hz). | 463.3 | 462.26 |
| 903 | 1H-NMR (400 MHz, DMSO-d6, ppm) δ: 10.37 (1H, s), 9.26 (1H, s), 8.33-8.28 (2H, m), 7.72-7.69 (1H, m), 7.02 (1H, s), 6.74 (1H, s), 5.22-5.20 (1H, m), 4.63-4.59 (1H, m), 3.98 (1H, d, J = 10.8Hz), 3.78 (1H, d, J = 11.6Hz), 3.63-3.61 (2H, m), 3.43-3.39 (4H, m), 3.03-3.01 (2H, m), 2.86-2.58 (1H, m), 1.65-1.37 (9H, m) | 479.3 | 478.24 |

**[Table 5-174]**

| Compound No. | NMR | LC/MS (M+H)⁺ | Exact Mass |
|---|---|---|---|
| 904 | 1H-NMR (400 MHz, DMSO-d6, ppm) δ: 10.12 (1H, s), 9.28 (1H, s), 8.21-8.16 (2H, m), 7.65-7.62 (1H, m), 7.15 (1H, s), 5.39-5.36 (1H, m), 5.23 (1H, d, J = 4.8Hz), 4.64 (1H, m), 3.74-3.72 (2H, m), 3.07 (3H, s), 2.93-2.87 (4H, m), 2.69-2.66 (2H, m), 1.39-1.37 (3H, m), 1.22-1.21 (3H, m), 1.18-1.13 (3H, m) | 451.3 | 450.25 |
| 905 | 1H-NMR (300 MHz, DMSO-d6, ppm) δ: 10.04 (1H, d, J = 8.8Hz), 9.24 (1H, s), 8.14-8.07 (1H, m), 7.67-763 (1H,m), 6.99 (1H, s), 6.38 (1H, d, J = 7.5Hz), 5.21 (1H, d, J = 4.5Hz), 4.76-4.61 (3H, m), 4.29-4.22 (1H, m), 3.88-3.77 (2H, m), 3.19 (1H, d, J = 5.1Hz), 2.92-2.88 (1H, m), 2.79 (1H, br), 2.21-2.19 (6H, m), 1.40 (3H, d, J = 5.7Hz), 1.31-1.28 (6H, m) | 465.1 | 464.26 |
| 906 | 1H-NMR (DMSO-D6) δ: 10.30 (1H, s), 9.20 (1H, s), 8.21 (1H, d, J = 9.8 Hz), 7.43 (1H, d, J = 9.8 Hz), 6.87 (1H, s), 6.37 (1H, d, J = 7.8 Hz), 4.23 (2H, q, J = 6.5 Hz), 3.81 (2H, s), 3.59-3.54 (4H, m), 3.26 (3H, s), 2.64-2.58 (4H, m), 1.37 (3H, d, J = 5.9 Hz), 1.27 (6H, dd, J = 5.9, 2.9 Hz). | 463.3 | 462.26 |
| 907 | 1H-NMR (DMSO-D6) δ: 10.29 (1H, s), 9.20 (1H, s), 8.21 (1H, d, J = 9.8 Hz), 7.44 (1H, d, J = 9.8 Hz), 6.87 (1H, s), 6.37 (1H, d, J = 6.8 Hz), 4.23 (2H, td, J = 12.9, 6.8 Hz), 4.06 (1H, q, J = 7.2 Hz), 3.64-3.50 (4H, m), 3.26 (3H, s), 2.74-2.68 (2H, m), 2.56-2.50 (2H, m), 1.42-1.35 (6H, m), 1.30-1.24 (6H, m). | 477.3 | 476.28 |

**[Table 5-175]**

| Compound No. | NMR | LC/MS (M+H)⁺ | Exact Mass |
|---|---|---|---|
| 908 | 1H-NMR (DMSO-D6) δ: 10.28 (1H, s), 9.20 (1H, s), 8.19 (1H, d, J = 9.8 Hz), 7.41 (1H, d, J = 9.8 Hz), 7.26 (1H, s), 7.17 (1H, s), 6.86 (1H, s), 6.37 (1H, d, J = 7.8 Hz), 4.26-4.20 (2H, m), 3.56 (4H, t, J = 4.4 Hz), 3.26 (3H, s), 2.92 (2H, s), 2.57 (4H, t, J = 4.9 Hz), 1.37 (3H, d, J = 5.9 Hz), 1.27 (6H, dd, J = 6.3, 3.4 Hz). | 481.3 | 480.27 |
| 909 | 1H-NMR (CDCl3) δ: 9.12 (1H, s), 8.46 (1H, s), 8.37 (1H, d, J = 9.8 Hz), 7.61 (1H, s), 7.07 (1H, d, J = 9.8 Hz), 6.40 (1H, s), 3.63-3.58 (4H, m), 3.22 (1H, s), 3.08-3.03 (4H, m), 2.72 (3H, s), 1.63 (9H, s). | 422.3 | 421.23 |
| 910 | 1H-NMR (CDCl3) δ: 9.71 (1H, s), 9.27 (1H, s), 8.40 (1H, d, J = 2.0 Hz), 8.36 (1H, d, J = 8.8 Hz), 7.77 (1H, dd, J = 8.8, 2.0 Hz), 7.62 (1H, s), 6.39 (1H, t, J = 5.9 Hz), 3.65 (2H, dd, J = 13.7, 6.8 Hz), 3.51 (2H, s), 3.24 (1H, s), 2.95-2.89 (4H, m), 2.71 (3H, s), 2.47 (4H, br s), 1.85-1.76 (2H, m), 1.10 (3H, t, J = 7.3 Hz). | 421.3 | 420.24 |
| 911 | 1H-NMR (CDCl3) δ: 9.45 (1H, s), 9.26 (1H, s), 8.41-8.34 (2H, m), 7.78 (1H, dd, J = 8.3, 2.4 Hz), 7.63 (1H, s), 6.39 (1H, t, J = 5.9 Hz), 3.70 (2H, q, J = 6.5 Hz), 3.54-3.51 (2H, m), 3.24 (1H, s), 2.92 (4H, t, J = 4.9 Hz), 2.72 (3H, s), 2.47 (4H, br s), 1.82-1.70 (2H, m), 1.56-1.49 (2H, m), 1.07-1.00 (3H, m). | 435.3 | 434.25 |
| 912 | 1H-NMR (CDCl3) δ: 9.48 (1H, s), 9.26 (1H, s), 8.42-8.34 (2H, m), 7.80-7.74 (1H, m), 7.63 (1H, s), 6.50 (1H, t, J = 5.9 Hz), 3.57-3.51 (4H, m), 3.24 (1H, s), 2.94-2.89 (4H, m), 2.72 (3H, s), 2.47 (4H, br s), 2.17-2.00 (1H, m), 1.12-1.04 (6H, m). | 435.3 | 434.25 |

**[Table 5-176]**

| Compound No. | NMR | LC/MS (M+H)⁺ | Exact Mass |
|---|---|---|---|
| 913 | 1H-NMR (CDCl3) δ: 9.08 (1H, s), 8.60 (1H, s), 8.34-8.28 (2H, m), 7.74 (1H, dd, J = 8.8, 2.0 Hz), 7.43 (1H, br s), 6.88 (1H, s), 6.62 (1H, s), 4.33 (1H, q, J = 6.5 Hz), 3.76 (2H, s), 3.62 (2H, t, J = 5.4 Hz), 3.52 (2H, s), 3.40 (3H, s), 2.63-2.48 (11H, m), 1.53-1.47 (9H, m). | 511.4 | 510.31 |
| 914 | 1H-NMR (CDCl3) δ: 9.12 (1H, s), 8.82 (1H, s), 8.36-8.31 (2H, m), 7.74 (1H, dd, J = 8.8, 2.0 Hz), 6.92 (1H, s), 6.48 (1H, d, J = 4.9 Hz), 4.35-4.23 (2H, m), 3.89 (1H, dd, J = 11.2, 2.0 Hz), 3.75 (1H, dd, J = 11.2, 7.3 Hz), 3.63 (2H, t, J = 5.4 Hz), 3.52 (2H, s), 3.40 (3H, s), 2.63-2.50 (11H, m), 1.50 (3H, d, J = 6.8 Hz), 1.43 (3H, d, J = 6.8 Hz). | 497.4 | 496.29 |
| 915 | 1H-NMR (CDCl3) δ: 9.11 (1H, s), 8.78 (1H, s), 8.36-8.31 (2H, m), 7.74 (1H, dd, J = 8.8, 2.0 Hz), 6.90 (1H, s), 6.48 (1H, d, J = 4.9 Hz), 4.35-4.27 (2H, m), 3.88 (1H, dd, J = 11.2, 2.4 Hz), 3.74 (1H, dd, J = 11.2, 7.8 Hz), 3.63 (2H, t, J = 5.4 Hz), 3.52 (2H, s), 3.38 (3H, s), 2.58 (11H, t, J = 5.4 Hz), 1.49 (3H, d, J = 6.8 Hz), 1.43 (3H, d, J = 6.8 Hz). | 497.4 | 496.29 |
| 916 | 1H-NMR (CDCl3) δ: 9.08 (1H, s), 8.72 (1H, s), 8.40 (1H, d, J = 8.8 Hz), 8.31 (1H, s), 7.72 (1H, d, J = 8.8 Hz), 6.86 (1H, s), 6.55 (1H, d, J = 7.8 Hz), 4.54-4.46 (1H, m), 4.33 (1H, q, J = 6.2 Hz), 3.64-3.57 (4H, m), 3.52 (2H, s), 3.48 (3H, s), 3.41 (3H, s), 2.76 (1H, br s), 2.59 (10H, s), 1.50 (3H, d, J = 5.9 Hz), 1.38 (3H, d, J = 6.8 Hz). | 511.4 | 510.31 |

**[Table 5-177]**

| Compound No. | NMR | LC/MS (M+H)⁺ | Exact Mass |
|---|---|---|---|
| 917 | 1H-NMR (CDCl3) δ: 9.09 (1H, s), 8.63 (1H, s), 8.35 (1H, d, J = 8.8 Hz), 8.30 (1H, d, J = 2.0 Hz), 7.74 (1H, dd, J = 8.3, 2.4 Hz), 6.87 (1H, s), 6.15 (1H, d, J = 7.8 Hz), 4.34 (1H, q, J = 6.5 Hz), 4.15-4.06 (1H, m), 3.82-3.72 (1H, m), 3.63 (2H, t, J = 5.4 Hz), 3.52 (2H, s), 3.42 (3H, s), 2.57 (10H, t, J = 4.9 Hz), 2.33-2.26 (3H, m), 2.12-2.05 (3H, m), 1.61-1.49 (5H, m), 1.46-1.35 (2H, m). | 537.4 | 536.32 |
| 918 | 1H-NMR (CDCl3) δ: 9.11 (1H, s), 8.88 (1H, br s), 8.38-8.32 (2H, m), 7.72 (1H, d, J = 8.8 Hz), 6.87 (1H, s), 6.43 (1H, s), 4.34 (1H, q, J = 6.5 Hz), 4.02 (2H, dd, J = 10.7, 2.9 Hz), 3.65-3.52 (6H, m), 3.45-3.38 (5H, m), 2.67-2.50 (11H, br m), 2.00 (1H, br s), 1.78-1.71 (2H, m), 1.53-1.43 (5H, m). | 537.4 | 536.32 |
| 919 | 1H-NMR (CDCl3) δ: 9.04 (1H, s), 8.52 (1H, s), 8.26 (1H, d, J = 8.8 Hz), 8.11 (1H, s), 7.39 (1H, d, J = 6.8 Hz), 6.83 (1H, s), 6.25 (1H, s), 4.34 (1H, d, J = 5.9 Hz), 3.71-3.62 (2H, m), 3.41 (3H, s), 3.19-3.03 (8H, m), 1.51 (3H, d, J = 5.9 Hz), 1.35 (3H, t, J = 6.3 Hz). | 409.3 | 408.24 |
| 920 | 1H-NMR (CDCl3) δ: 9.04 (1H, br s), 8.81 (1H, br s), 8.27 (1H, br s), 8.15 (1H, br s), 7.36 (1H, br s), 6.81 (1H, br s), 6.31 (1H, br s), 4.37-4.30 (1H, m), 3.64-3.54 (2H, m), 3.41 (3H, s), 3.23-3.03 (8H, m), 1.79-1.70 (2H, m), 1.50 (3H, br s), 1.09-1.00 (3H, m). | 423.3 | 422.25 |

**[Table 5-178]**

| Compound No. | NMR | LC/MS (M+H)⁺ | Exact Mass |
|---|---|---|---|
| 921 | 1H-NMR (CDCl3) δ: 9.03 (1H, s), 8.81 (1H, s), 8.24 (1H, d, J = 8.8 Hz), 8.14 (1H, d, J = 2.0 Hz), 7.37 (1H, dd, J = 8.8, 2.9 Hz), 6.68 (1H, s), 6.43 (1H, t, J = 5.4 Hz), 4.80 (1H, q, J = 6.2 Hz), 3.60 (2H, q, J = 6.5 Hz), 3.19-3.12 (4H, m), 3.11-3.05 (4H, m), 1.77 (2H, td, J = 14.4, 7.2 Hz), 1.53 (3H, d, J = 5.9 Hz), 1.07 (3H, t, J = 7.3 Hz). | 423.3 | 408.24 |
| 922 | 1H-NMR (CDCl3) δ: 8.99 (1H, s), 8.23 (1H, d, J = 8.8 Hz), 8.20 (1H, s), 8.07 (1H, d, J = 2.9 Hz), 7.37 (1H, dd, J = 8.8, 2.9 Hz), 6.67 (1H, s), 6.42 (1H, t, J = 5.4 Hz), 4.80 (1H, q, J = 6.5 Hz), 3.64 (2H, q, J = 6.5 Hz), 3.18-3.13 (4H, m), 3.10-3.06 (4H, m), 1.77-1.70 (2H, m), 1.56-1.45 (5H, m), 1.02 (3H, t, J = 7.3 Hz). | 451.3 | 422.25 |
| 923 | 1H-NMR (CDCl3) δ: 9.05 (1H, s), 8.41 (2H, t, J = 7.3 Hz), 8.29 (1H, d, J = 2.9 Hz), 7.73 (1H, dd, J = 8.8, 2.9 Hz), 6.70 (1H, s), 6.24 (1H, d, J = 7.8 Hz), 4.81 (1H, q, J = 6.2 Hz), 4.46-4.36 (1H, m), 3.75 (2H, s), 3.56 (2H, s), 1.54 (3H, d, J = 5.9 Hz), 1.41-1.34 (12H, m). | 423.3 | 450.25 |
| 924 | 1H-NMR (CDCl3) δ: 9.02 (1H, s), 8.19 (1H, s), 8.10 (1H, d, J = 8.8 Hz), 7.43 (1H, d, J = 8.8 Hz), 6.67 (1H, s), 6.25 (1H, d, J = 7.8 Hz), 4.80 (1H, q, J = 6.2 Hz), 4.45-4.35 (1H, m), 3.09-3.04 (4H, m), 2.92-2.87 (4H, m), 2.51 (3H, s), 1.53 (3H, d, J = 5.9 Hz), 1.38 (6H, d, J = 5.9 Hz). | 437.3 | 422.25 |
| 925 | 1H-NMR (CDCl3) δ: 9.01 (1H, s), 8.12 (1H, d, J = 8.8 Hz), 8.05 (1H, s), 7.44 (1H, d, J = 8.8 Hz), 6.81 (1H, s), 6.14 (1H, d, J = 7.8 Hz), 4.46-4.30 (2H, m), 3.40 (3H, s), 3.07 (4H, t, J = 4.9 Hz), 2.90 (4H, t, J = 4.9 Hz), 2.50 (3H, s), 1.50 (3H, d, J = 6.8 Hz), 1.36 (6H, t, J = 5.9 Hz). | | 436.27 |

**[Table 5-179]**

| Compound No. | NMR | LC/MS (M+H)⁺ | Exact Mass |
|---|---|---|---|
| 926 | 1H-NMR (400 MHz, DMSO-d6, ppm) δ: 10.16 (1H, s), 9.26 (1H, s), 8.31 (1H, d, J = 8.8Hz), 8.23 (1H, d, J = 2.4Hz), 7.77-7.74 (1H, m), 7.00 (1H, s), 6.42 (1H, d, J = 7.6Hz), 5.20 (1H, d, J = 4.4Hz), 5.11 (1H, d, J = 3.6Hz), 4.64-4.61 (1H, m), 4.30-4.24 (1H, m), 4.11 (1H, br), 3.79-3.74 (1H, m), 3.61-3.56 (1H, m), 2.68-2.63 (1H, m), 2.36-2.30 (1H, m), 2.07-2.04 (1H, br), 1.87-1.82 (1H, br), 1.39 (3H, d, J = 6.8Hz), 1.31-1.29 (6H, m) | 438.2 | 437.22 |
| 927 | 1H-NMR (400 MHz, DMSO-d6, ppm) δ: 9.93 (1H, s), 9.22 (1H, s), 8.25 (1H, d, J = 9.2Hz), 8.05 (1H, m), 7.47 (1H, d, J = 7.6Hz), 6.88 (2H, s), 4.42 (1H, br), 4.25 (1H, br), 3.88-3.86 (2H, br), 3.47 (2H, br), 3.27 (5H, s), 3.00-2.97 (2H, br), 2.63-2.58 (2H, m), 1.96 (3H, br), 1.64-1.61 (2H, br), 1.51-1.48 (2H, br), 1.39 (2H, d, J = 5.6Hz), 1.33-1.30 (5H, m) | 494.2 | 493.28 |
| 928 | 1H-NMR (400 MHz, DMSO-d6, ppm) δ: 10.11 (1H, s), 9.24 (1H, s), 8.23-8.19 (2H, m), 7.70 (1H, d, J = 8.4Hz), 6.88 (1H, s), 6.53-6.50 (1H, m), 4.24-4.22 (1H, m), 4.12-4.10 (1H, m), 3.90-3.87 (2H, m), 3.40 (2H, s), 3.31-3.24 (5H, m), 2.67-2.65 (4H, m), 2.28 (4H, br), 1.86-1.83 (1H, br), 1.68-1.59 (2H, m), 1.38-1.32 (5H, m), 1.24-1.22 (3H, m) | 507.5 | 506.31 |
| 929 | 1H-NMR (300 MHz, DMSO-d6, ppm) δ: 9.91 (1H, s), 9.22 (1H, s), 8.03 (1H, d, J = 8.4Hz), 7.51 (1H, d, J = 8.1Hz), 6.98 (1H, s), 6.38 (1H, d, J = 8.4Hz), 5.19 (1H, d, J = 4.2Hz), 4.64-4.60 (1H, m), 4.51-4.47 (1H, m), 4.28-4.22 (1H, m), 3.59 (4H, s), 2.82 (4H, s), 2.61-2.57 (2H, m), 1.39 (1H, d, J = 6.3Hz), 1.29 (1H, d, J = 6.3Hz) | 424.2 | 423.24 |

**[Table 5-180]**

| Compound No. | NMR | LC/MS (M+H)⁺ | Exact Mass |
|---|---|---|---|
| 930 | 1H-NMR (400 MHz, DMSO-d6, ppm) δ: 9.89 (1H, s), 9.21 (1H, s), 8.01 (1H, d, J = 8.4Hz), 7.50 (1H, d, J = 8.4Hz), 6.96 (1H, s), 5.17 (1H, d, J = 4.8Hz), 4.63-4.57 (1H, m), 4.28-4.19 (1H, m), 3.55 (2H, s), 2.82-2.75 (4H, m), 2.65-2.62 (2H, m), 2.57-2.54 (2H, m), 2.29 (3H, s), 1.37 (1H, d, J = 6.4Hz), 1.29-1.27 (1H, m) | 437.2 | 436.27 |
| 931 | 1H-NMR (300 MHz, DMSO-d6, ppm) δ: 9.93 (1H, d, J = 5.4Hz), 9.17 (1H, s), 8.06-8.00 (1H, m), 7.60 (1H, d, J = 6.4Hz), 6.92 (1H, s), 6.33 (1H, d, J = 6.9Hz), 5.14 (1H, d, J = 2.7Hz), 4.70-4.44 (3H, m), 4.22-4.16 (1H, m), 3.79-3.72 (2H, m), 3.48 (1H, d, J = 4.8Hz), 2.86-2.72 (2H, m), 1.69-1.51 (3H, m), 1.32 (1H, d, J = 6.6Hz), 1.23 (1H, d, J = 6.3Hz) | 479.2 | 478.28 |
| 932 | 1H-NMR (400 MHz, DMSO-d6, ppm) δ: 9.99 (1H, d, J = 8Hz), 9.22 (1H, s), 8.09-8.06 (1H, m), 7.67-7.62 (1H, m), 6.97 (1H, s), 6.37 (1H, d, J = 6.4Hz), 5.18 (1H, d, J = 4.4Hz), 4.70 (1H, s), 4.62-4.58 (2H, m), 4.27-4.22 (1H, m), 3.82-3.77 (2H, m), 2.90 (3H, br), 2.76 (2H, br), 2.54 (2H, br), 1.56-1.27 (14H, m) | 491.1 | 490.28 |
| 933 | 1H-NMR (300 MHz, DMSO-d6, ppm) δ: 9.97 (1H, s), 9.23 (1H, s), 8.07 (1H, d, J = 8.1Hz), 7.57 (1H, d, J = 8.4Hz), 6.98 (1H, s), 6.58 (1H, br), 6.38 (1H, d, J = 7.8Hz), 5.19 (1H, d, J = 4.2Hz), 4.63 (1H, br), 4.48 (2H, s), 4.25 (1H, br), 3.64 (2H, br), 2.79 (2H, br), 1.61 (1H, d, J = 3.9Hz), 1.39 (1H, d, J = 6.3Hz), 1.29 (1H, d, J = 6.3Hz) | 437.1 | 436.23 |

**[Table 5-181]**

| Compound No. | NMR | LC/MS (M+H)⁺ | Exact Mass |
|---|---|---|---|
| 934 | 1H-NMR (400 MHz, DMSO-d6, ppm) δ: 10.05 (1H, s), 9.23 (1H, s), 8.09 (1H, d, J = 8.8Hz), 7.64 (1H, d, J = 8.4Hz), 6.98 (1H, s), 6.39 (1H, d, J = 7.6Hz), 5.19 (1H, d, J = 4.4Hz), 4.60 (1H, m), 4.36 (3H, m), 3.54-3.51 (2H, m), 2.97-2.91 (5H, m), 1.38-1.37 (3H, m), 1.29-1.28 (6H, m) | 458.1 | 457.19 |
| 935 | 1H-NMR (400 MHz, DMSO-d6, ppm) δ: 10.02 (1H, s), 9.22 (1H, s), 8.08 (1H, d, J = 8.4Hz), 7.64 (1H, d, J = 8.4Hz), 6.97 (1H, s), 6.38 (1H, d, J = 7.6Hz), 5.19 (1H, d, J = 4.8Hz), 4.62-4.59 (1H, m), 4.37 (2H, s), 4.25-4.23 (1H, m), 3.59-3.56 (2H, m), 2.90-2.87 (2H, m), 2.78 (6H, s), 1.38-1.37 (3H, m), 1.29-1.27 (6H, m) | 487.1 | 486.22 |
| 936 | 1H-NMR (DMSO-D6) δ: 10.23 (1H, s), 9.26 (1H, s), 8.34-8.30 (2H, m), 7.84 (1H, dd, J = 8.8, 2.9 Hz), 7.00 (1H, s), 6.42 (1H, d, J = 7.8 Hz), 5.21 (1H, d, J = 3.9 Hz), 4.65-4.58 (1H, m), 4.31-4.15 (2H, m), 3.79-3.67 (2H, m), 3.37 (1H, d, J = 16.6 Hz), 3.26 (1H, d, J = 15.6 Hz), 3.04-2.98 (1H, m), 2.87-2.81 (1H, m), 1.43 (3H, d, J = 6.8 Hz), 1.38 (3H, d, J = 6.8 Hz), 1.29 (6H, dd, J = 5.9, 2.0 Hz). | 476.3 | 475.24 |
| 937 | 1H-NMR (DMSO-D6) δ: 10.23 (1H, s), 9.20 (1H, s), 8.19 (1H, d, J = 9.8 Hz), 7.41 (1H, d, J = 9.8 Hz), 7.26 (1H, s), 7.17 (1H, s), 6.96 (1H, s), 6.32 (1H, d, J = 7.8 Hz), 5.19 (1H, d, J = 4.9 Hz), 4.64-4.55 (1H, m), 4.28-4.17 (1H, m), 3.55 (4H, t, J = 4.9 Hz), 2.92 (2H, s), 2.57 (4H, t, J = 4.4 Hz), 1.37 (3H, d, J = 6.8 Hz), 1.27 (6H, d, J = 6.8 Hz). | 467.3 | 466.26 |

**[Table 5-182]**

| Compound No. | NMR | LC/MS (M+H)⁺ | Exact Mass |
|---|---|---|---|
| 938 | 1H-NMR (CDCl3) δ: 8.97 (1H, s), 8.41 (1H, d, J = 9.8 Hz), 8.22 (1H, s), 7.07 (1H, d, J = 9.8 Hz), 6.68 (1H, s), 6.50 (1H, d, J = 7.8 Hz), 4.35-4.26 (1H, br m), 4.17 (1H, t, J = 8.3 Hz), 4.10-3.94 (3H, m), 3.89 (1H, t, J = 7.8 Hz), 3.79-3.71 (2H, br m), 3.66-3.54 (5H, m), 3.53-3.43 (1H, m), 3.07-3.01 (4H, m), 2.31-2.24 (2H, m), 2.08-1.97 (1H, m), 1.94-1.81 (2H, m), 1.76-1.61 (1H, m). | 478.3 | 477.26 |
| 939 | 1H-NMR (CDCl3) δ: 8.96 (1H, s), 8.41 (1H, d, J = 9.8 Hz), 8.21 (1H, s), 7.07 (1H, d, J = 9.8 Hz), 6.68 (1H, s), 6.47 (1H, d, J = 7.8 Hz), 4.37-4.26 (1H, br m), 4.17 (1H, t, J = 7.8 Hz), 4.11-3.94 (3H, m), 3.89 (1H, t, J = 7.8 Hz), 3.82-3.68 (2H, m), 3.67-3.54 (5H, m), 3.52-3.43 (1H, m), 3.09-3.01 (4H, m), 2.35-2.24 (2H, m), 2.11-1.99 (1H, m), 1.94-1.80 (2H, m), 1.79-1.60 (1H, m). | 478.3 | 477.26 |
| 940 | 1H-NMR (CDCl3) δ: 9.00 (1H, s), 8.37 (1H, d, J = 8.8 Hz), 8.31 (1H, s), 8.27 (1H, d, J = 2.0 Hz), 7.74 (1H, dd, J = 8.8, 2.9 Hz), 6.69 (1H, s), 6.51 (1H, d, J = 7.8 Hz), 4.38-4.28 (1H, m), 4.18 (1H, t, J = 8.3 Hz), 4.11-4.03 (2H, m), 3.98 (1H, q, J = 7.5 Hz), 3.90 (1H, t, J = 7.8 Hz), 3.84-3.69 (2H, m), 3.59 (1H, dd, J = 11.2, 6.3 Hz), 3.54-3.43 (3H, m), 2.93-2.87 (4H, m), 2.51-2.38 (4H, br m), 2.34-2.24 (2H, m), 2.13-2.02 (1H, m), 1.95-1.80 (2H, m), 1.78-1.62 (1H, m). | 491.4 | 490.28 |

**[Table 5-183]**

| Compound No. | NMR | LC/MS (M+H)⁺ | Exact Mass |
|---|---|---|---|
| 941 | 1H-NMR (CDCl3) δ: 9.00 (1H, s), 8.37 (1H, d, J = 8.8 Hz), 8.33 (1H, s), 8.27 (1H, d, J = 2.0 Hz), 7.75 (1H, dd, J = 8.3, 2.4 Hz), 6.69 (1H, s), 6.54 (1H, d, J = 7.8 Hz), 4.37-4.28 (1H, m), 4.18 (1H, t, J = 7.8 Hz), 4.11-4.02 (2H, m), 3.98 (1H, q, J = 7.5 Hz), 3.90 (1H, t, J = 7.8 Hz), 3.82-3.71 (2H, m), 3.61 (1H, dd, J = 10.7, 5.9 Hz), 3.54-3.44 (3H, m), 2.90 (4H, t, J = 4.9 Hz), 2.52-2.38 (4H, br m), 2.28 (2H, q, J = 7.5 Hz), 2.12-2.02 (1H, m), 1.96-1.81 (2H, m), 1.77-1.59 (1H, m). | 491.4 | 490.28 |
| 942 | | 448.3 | 447.24 |
| 943 | | 448.3 | 447.24 |
| 944 | 1H-NMR (CDCl3) δ: 9.14 (1H, br s), 8.10 (1H, d, J = 8.8 Hz), 7.37-7.23 (1H, br m), 6.73 (1H, br s), 6.39 (1H, br s), 4.81 (1H, q, J = 6.2 Hz), 4.04-3.89 (2H, m), 3.66-3.55 (2H, m), 3.37-3.13 (2H, m), 3.03-2.83 (2H, m), 1.85-1.73 (2H, m), 1.52 (3H, d, J = 6.8 Hz), 1.10 (3H, t, J = 7.8 Hz). | 380.3 | 379.21 |
| 945 | 1H-NMR (CDCl3) δ: 8.99 (1H, s), 8.39 (1H, d, J = 9.8 Hz), 8.28 (1H, s), 7.09 (1H, d, J = 9.8 Hz), 6.62 (1H, s), 6.32 (1H, t, J = 5.9 Hz), 5.16 (2H, d, J = 4.9 Hz), 4.58 (2H, d, J = 4.9 Hz), 3.65-3.57 (6H, m), 3.10-3.04 (4H, m), 1.94 (1H, br s), 1.81-1.70 (5H, m), 1.05 (3H, t, J = 7.3 Hz). | 436.3 | 435.25 |
| 946 | 1H-NMR (CDCl3) δ: 8.96 (1H, s), 8.38 (1H, d, J = 9.8 Hz), 8.22 (1H, s), 7.08 (1H, d, J = 9.8 Hz), 6.65 (1H, s), 6.29 (1H, t, J = 5.9 Hz), 4.18 (1H, t, J = 7.8 Hz), 4.12-4.05 (1H, m), 4.02-3.90 (2H, m), 3.63-3.54 (6H, m), 3.53-3.44 (1H, m), 3.10-3.04 (4H, m), 2.36-2.22 (2H, m), 1.90 (1H, br s), 1.80-1.68 (2H, m), 1.04 (3H, t, J = 7.8 Hz). | 436.3 | 435.25 |

**[Table 5-184]**

| Compound No. | NMR | LC/MS (M+H)⁺ | Exact Mass |
|---|---|---|---|
| 947 | 1H-NMR (CDCl3) δ: 8.98 (1H, s), 8.15 (1H, d, J = 7.8 Hz), 8.03 (1H, s), 7.41 (1H, d, J = 7.8 Hz), 6.61 (1H, s), 6.36 (1H, t, J = 5.9 Hz), 5.17 (2H, d, J = 5.9 Hz), 4.58 (2H, d, J = 4.9 Hz), 4.02 (2H, s), 3.66-3.58 (2H, m), 3.25 (2H, t, J = 6.3 Hz), 2.88 (2H, t, J = 5.9 Hz), 1.82-1.71 (5H, m), 1.06 (3H, t, J = 7.3 Hz). | 406.3 | 405.23 |
| 948 | 1H-NMR (CDCl3) δ: 8.96 (1H, s), 8.14 (1H, d, J = 7.8 Hz), 8.04 (1H, s), 7.40 (1H, d, J = 8.8 Hz), 6.64 (1H, s), 6.33 (1H, t, J = 5.9 Hz), 4.19 (1H, t, J = 7.8 Hz), 4.12-4.05 (1H, m), 4.03-3.90 (4H, m), 3.62-3.55 (2H, m), 3.54-3.44 (1H, m), 3.25 (2H, t, J = 5.9 Hz), 2.88 (2H, t, J = 5.9 Hz), 2.36-2.22 (2H, m), 1.80-1.68 (2H, m), 1.06 (3H, t, J = 7.3 Hz). | 406.3 | 405.23 |
| 949 | 1H-NMR (DMSO-D6) δ: 10.23 (1H, s), 9.25 (1H, s), 8.68 (1H, s), 8.34 (1H, d, J = 2.0 Hz), 8.30 (1H, d, J = 7.8 Hz), 7.99 (1H, s), 7.79 (1H, dd, J = 8.8, 2.0 Hz), 6.99 (1H, s), 6.43 (1H, d, J = 7.8 Hz), 5.41 (2H, s), 5.21 (1H, d, J = 3.9 Hz), 4.64-4.58 (1H, m), 4.29-4.22 (1H, m), 1.38 (3H, d, J = 6.8 Hz), 1.28 (6H, dd, J = 6.8, 2.0 Hz). | 406.3 | 405.20 |
| 950 | 1H-NMR (400 MHz, DMSO-d6, ppm) δ: 9.94 (1H, s), 9.24 (1H, s), 8.21 (1H, d, J = 8.4Hz), 8.11 (1H, d, J = 2Hz), 7.65 (1H, d, J = 2Hz), 6.99 (1H, s), 6.40 (1H, d, J = 7.6Hz), 5.19 (1H, d, J = 4.4Hz), 4.70-4.62 (1H, m), 4.30-4.22 (1H, m), 2.92-2.89 (2H, br), 2.48-2.39 (4H, m), 1.53-1.50 (3H, br), 1.39 (3H, d, J = 6.8Hz), 1.31-1.29 (6H, m), 1.06 (2H, br) | 422.4 | 421.26 |

**[Table 5-185]**

| Compound No. | NMR | LC/MS (M+H)⁺ | Exact Mass |
|---|---|---|---|
| 951 | 1H-NMR (400 MHz, DMSO-d6, ppm) δ: 9.94 (1H, s), 9.24 (1H, s), 8.21 (1H, d, J = 8.4Hz), 8.11 (1H, d, J = 2Hz), 7.68-7.63 (1H, m), 6.99 (1H, s), 6.40 (1H, d, J = 8Hz), 5.19 (1H, d, J = 8.4Hz), 4.62 (1, br), 4.30-4.29 (2H, m), 4.46-4.45 (2H, m), 2.81 (2H, br), 2.48 (1H, s), 2.34-2.31 (3H, m), 1.87 (2H, br), 1.52 (3H, br), 1.39 (3H, d, J = 6.8Hz), 1.31-1.29 (6H, m), 1.28-1.11 (2H, br) | 466.4 | 465.29 |
| 952 | 1H-NMR (400 MHz, DMSO-d6, ppm) δ: 9.92 (1H, s), 9.23 (1H, s), 8.29 (1H, d, J = 8.4Hz), 8.10 (1H, d, J = 1.6Hz), 7.65-7.59 (1H, m), 6.98 (1H, s), 6.90-6.82 (1H, m), 5.189 (1H, s), 4.60 (1H, br), 3.90-3.82 (2H, m), 3.55-3.40 (2H, m), 3.28-3.26 (4H, m), 2.92-85 (2H, m), 2.46 (1H, s), 2.42-2.31 (2H, m), 1.97 (1H, br), 1.62 (2H, br), 1.52 (2H, br), 1.40-1.21 (5H, m), 1.12-0.98 (2H, br) | 478.5 | 477.29 |
| 953 | 1H-NMR (400 MHz, DMSO-d6, ppm) δ: 9.92 (1H, s), 9.23 (1H, s), 8.30 (1H, d, J = 8.4Hz), 8.11 (1H, d, J = 2.0Hz), 7.61-7.49 (1H, dd, J1 = 8.4Hz, J2 = 2.0Hz), 6.98 (1H, s), 6.87-6.86 (1H, m), 5.17 (1H, d, J = 4.8Hz), 4.63-4.59 (1H, m), 3.87-3.3.85 (2H, m), 3.51-3.45 (4H, m), 3.31 (2H, s), 3.28-3.25 (2H, m), 2.99-2.70 (2H, br), 1.97-1.96 (2H, br), 1.65-1.55 (6H, m), 1.39 (3H, 3H, d, J = 6.4Hz), 1.33-1.26 (9H, m) | 520.3 | 521.31 |
| 954 | 1H-NMR (300 MHz, DMSO-d6, ppm) δ: 9.93 (1H, s), 9.22 (1H, s), 8.03 (1H, d, J = 8.4Hz), 7.51 (1H, d, J = 8.4Hz), 6.98(1H, s), 6.38 (1H, d, J = 7.5Hz), 5.20 (1H, d, J = 4.5Hz), 4.64-4.60 (1H, m), 4.26-4.24 (1H, m), 3.58 (2H, s), 2.80-2.78 (4H, m), 2.61-2.56 (2H, m), 2.46-2.42 (2H, m), 2.17 (6H, s), 1.38 (1H, d, J = 6.6Hz), 1.29 (1H, d, J = 6.3Hz) | 541.4 | 450.29 |

**[Table 5-186]**

| Compound No. | NMR | LC/MS (M+H)⁺ | Exact Mass |
|---|---|---|---|
| 955 | 1H-NMR (300 MHz, DMSO-d6, ppm) δ: 9.91 (1H, s), 9.22 (1H, s), 8.02 (1H, d, J = 8.4Hz), 7.51 (1H, d, J = 8.1Hz), 6.98 (1H, s), 6.37 (1H, d, J = 7.2Hz), 5.18 (1H, d, J = 3.6Hz), 4.62 (1H, br), 4.25 (1H, br), 3.60 (2H, s), 3.40 (4H, br), 2.80 (4H, s), 2.62 (2H, br), 2.22-2.17 (2H, m), 1.91 (2H, br), 1.39 (1H, d, J = 6Hz), 1.29 (1H, d, J = 6Hz) | 491.2 | 490.28 |
| 956 | 1H-NMR (400 MHz, DMSO-d6, ppm) δ: 9.91 (1H, s), 9.22 (1H, s), 8.10 (1H, d, J = 9.2Hz), 8.02 (1H, d, J = 2.8Hz), 7.44-7.41 (1H, m), 6.91 (1H, s), 6.61 (1H, d, J = 8.0Hz), 4.27-4.22 (1H, m), 4.15-4.13 (1H, m), 3.95-3.92 (1H, m), 3.71-3.68 (1H, m), 3.58-3.56 (1H, m), 3.43-3.39 (1H, m), 3.27 (3H, s), 3.06-3.03 (4H, m), 2.87-2.84 (4H, m), 2.03-2.00 (1H, m), 1.82-1.76 (2H, m), 1.65-1.62 (1H, m), 1.39-1.37 (3H, m) | 465.4 | 464.26 |
| 957 | 1H-NMR (400 MHz, DMSO-d6, ppm) δ: 9.84 (1H, s), 9.21 (1H, s), 8.10 (1H, d, J = 9.2Hz), 8.02 (1H, d, J = 2.8Hz), 7.44-7.41 (1H, m), 7.00 (1H, s), 6.56 (1H, d, J = 8.0Hz), 5.19 (1H, d, J = 4.4Hz), 4.62-4.60 (1H, m), 4.16-4.12 (1H, m), 3.96-3.93 (1H, m), 3.72-3.68 (1H, m), 3.58-3.53 (1H, m), 3.43-3.81 (1H, s), 3.06-3.03 (4H, m), 2.87-2.84 (4H, m), 2.01-2.02 (1H, m), 1.80-1.75 (2H, m), 1.65-1.61 (1H, m), 1.39-1.37 (3H, m) | 451.1 | 450.25 |
| 958 | 1H-NMR (DMSO-D6) δ: 10.29 (1H, s), 9.28 (1H, s), 8.44 (1H, d, J = 2.0 Hz), 8.36 (1H, d, J = 8.8 Hz), 7.96 (1H, dd, J = 8.3, 2.4 Hz), 7.73 (1H, s), 7.10 (1H, s), 7.00 (1H, s), 6.44 (1H, d, J = 7.8 Hz), 5.22 (1H, d, J = 3.9 Hz), 4.64-4.58 (1H, m), 4.30-4.23 (1H, m), 3.70 (3H, s), 1.39 (3H, d, J = 6.8 Hz), 1.30 (6H, dd, J = 6.8, 2.0 Hz). | 405.3 | 404.21 |
| 959 | | 405.3 | 404.21 |

**[Table 5-187]**

| Compound No. | NMR | LC/MS (M+H)⁺ | Exact Mass |
|---|---|---|---|
| 960 | 1H-NMR (DMSO-D6) δ: 10.23 (1H, s), 9.20 (1H, s), 8.19 (1H, d, J = 9.8 Hz), 7.76 (1H, d, J = 4.9 Hz), 7.41 (1H, d, J = 9.8 Hz), 6.96 (1H, s), 6.32 (1H, d, J = 7.8 Hz), 5.19 (1H, d, J = 4.9 Hz), 4.63-4.57 (1H, m), 4.27-4.18 (1H, m), 3.56 (4H, t, J = 4.4 Hz), 2.96 (2H, s), 2.63 (3H, d, J = 4.9 Hz), 2.55 (4H, t, J = 4.9 Hz), 1.37 (3H, d, J = 6.8 Hz), 1.27 (6H, d, J = 6.8 Hz). | 481.3 | 480.27 |
| 961 | 1H-NMR (DMSO-D6) δ: 10.23 (1H, s), 9.26 (1H, s), 8.33 (1H, d, J = 9.8 Hz), 8.30 (1H, d, J = 2.0 Hz), 7.89 (1H, d, J = 3.9 Hz), 7.82 (1H, dd, J = 8.8, 2.9 Hz), 7.00 (1H, s), 6.42 (1H, d, J = 7.8 Hz), 5.21 (1H, d, J = 3.9 Hz), 4.64-4.58 (1H, m), 4.29-4.21 (1H, m), 3.72 (2H, t, J = 5.4 Hz), 3.28 (2H, s), 3.09 (2H, s), 2.87 (2H, t, J = 5.4 Hz), 2.63 (3H, d, J = 3.9 Hz), 1.38 (3H, d, J = 6.8 Hz), 1.29 (6H, d, J = 6.8 Hz). | 494.3 | 493.25 |
| 962 | 1H-NMR (DMSO-D6) δ: 10.22 (1H, s), 9.26 (1H, s), 8.33-8.29 (2H, m), 7.83 (1H, dd, J = 8.8, 2.9 Hz), 6.99 (1H, s), 6.42 (1H, d, J = 7.8 Hz), 5.21 (1H, d, J = 4.9 Hz), 4.64-4.58 (1H, m), 4.30-4.22 (1H, m), 3.69 (2H, t, J = 5.4 Hz), 3.36 (2H, s), 3.33 (2H, s), 3.00 (3H, s), 2.93 (2H, t, J = 4.9 Hz), 2.82 (3H, s), 1.38 (3H, d, J = 6.8 Hz), 1.29 (6H, dd, J = 5.9, 2.0 Hz). | 508.3 | 507.27 |
| 963 | 1H-NMR (DMSO-D6) δ: 10.24 (1H, s), 9.20 (1H, s), 8.20 (1H, d, J = 9.8 Hz), 7.41 (1H, d, J = 9.8 Hz), 6.96 (1H, s), 6.33 (1H, d, J = 7.8 Hz), 5.19 (1H, d, J = 3.9 Hz), 4.63-4.57 (1H, m), 4.23 (1H, td, J = 13.4, 6.5 Hz), 3.53 (4H, t, J = 4.9 Hz), 3.25 (2H, q, J = 10.1 Hz), 2.75 (4H, t, J = 4.9 Hz), 1.37 (3H, d, J = 6.8 Hz), 1.27 (6H, d, J = 4.9 Hz). | 492.3 | 491.24 |
| 964 | | 447.3 | 446.23 |

**[Table 5-188]**

| Compound No. | NMR | LC/MS (M+H)⁺ | Exact Mass |
|---|---|---|---|
| 965 | | 463.2 | 462.21 |
| 966 | 1H-NMR (DMSO-D6) δ: 10.23 (1H, s), 9.20 (1H, s), 8.19 (1H, d, J = 9.8 Hz), 7.40 (1H, d, J = 9.8 Hz), 6.96 (1H, s), 6.32 (1H, d, J = 7.8 Hz), 5.19 (1H, d, J = 4.9 Hz), 4.63-4.57 (1H, m), 4.23 (1H, td, J = 13.2, 6.8 Hz), 3.52 (4H, s), 3.47 (2H, t, J = 6.8 Hz), 3.28 (2H, t, J = 6.8 Hz), 3.17 (2H, s), 2.62 (4H, s), 1.88-1.82 (2H, m), 1.78-1.71 (2H, m), 1.37 (3H, d, J = 6.8 Hz), 1.27 (6H, d, J = 4.9 Hz). | 521.3 | 520.30 |
| 967 | 1H-NMR (DMSO-D6) δ: 10.24 (1H, s), 9.20 (1H, s), 8.20 (1H, d, J = 9.8 Hz), 7.40 (1H, d, J = 9.8 Hz), 6.96 (1H, s), 6.32 (1H, d, J = 7.8 Hz), 5.18 (1H, d, J = 4.9 Hz), 4.63-4.56 (1H, m), 4.26-4.19 (1H, m), 3.58 (4H, s), 3.55-3.50 (6H, m), 3.44 (2H, t, J = 4.4 Hz), 3.22 (2H, s), 2.56 (4H, t, J = 4.9 Hz), 1.37 (3H, d, J = 6.8 Hz), 1.27 (6H, t, J = 3.9 Hz). | 537.3 | 536.30 |
| 968 | 1H-NMR (DMSO-D6) δ: 10.24 (1H, s), 9.20 (1H, s), 8.20 (1H, d, J = 9.8 Hz), 7.55 (1H, d, J = 7.8 Hz), 7.41 (1H, d, J = 9.8 Hz), 6.96 (1H, s), 6.32 (1H, d, J = 7.8 Hz), 5.19 (1H, d, J = 4.9 Hz), 4.63-4.57 (1H, m), 4.27-4.18 (1H, m), 3.94-3.86 (1H, m), 3.55 (4H, s), 2.95 (2H, s), 2.57 (4H, s), 1.37 (3H, d, J = 6.8 Hz), 1.27 (6H, d, J = 6.8 Hz), 1.07 (6H, d, J = 6.8 Hz). | 509.3 | 508.30 |
| 969 | 1H-NMR (DMSO-D6) δ: 10.13 (1H, s), 9.25 (1H, s), 8.28 (1H, d, J = 8.8 Hz), 8.20 (1H, s), 7.76 (1H, dd, J = 8.3, 2.4 Hz), 6.89 (1H, s), 6.46 (1H, d, J = 7.8 Hz), 4.29-4.22 (2H, m), 3.71 (2H, s), 3.45 (2H, s), 3.27 (3H, s), 1.38 (3H, d, J = 6.8 Hz), 1.30 (6H, dd, J = 5.9, 3.9 Hz). | 476.3 | 475.28 |

**[Table 5-189]**

| Compound No. | NMR | LC/MS (M+H)⁺ | Exact Mass |
|---|---|---|---|
| 970 | 1H-NMR (DMSO-D6) δ: 10.11 (1H, s), 9.25 (1H, s), 8.27 (1H, d, J = 7.8 Hz), 8.20 (1H, d, J = 2.0 Hz), 7.74 (1H, dd, J = 8.8, 2.0 Hz), 7.10 (2H, s), 6.89 (1H, s), 6.46 (1H, d, J = 7.8 Hz), 4.28-4.21 (2H, m), 3.45 (2H, s), 3.27 (3H, s), 2.82 (2H, s), 2.42 (8H, s), 1.38 (3H, d, J = 6.8 Hz), 1.30 (6H, dd, J = 6.8, 3.9 Hz). | 494.3 | 493.29 |
| 971 | 1H-NMR (DMSO-D6) δ: 10.12 (1H, s), 9.25 (1H, s), 8.27 (1H, d, J = 8.8 Hz), 8.20 (1H, d, J = 2.0 Hz), 7.74 (1H, dd, J = 8.3, 2.4 Hz), 7.60 (1H, d, J = 4.9 Hz), 6.89 (1H, s), 6.46 (1H, d, J = 7.8 Hz), 4.28-4.22 (2H, m), 3.46 (2H, s), 3.27 (3H, s), 2.86 (2H, s), 2.58 (3H, d, J = 4.9 Hz), 2.42 (8H, s), 1.38 (3H, d, J = 6.8 Hz), 1.30 (6H, dd, J = 6.8, 3.9 Hz). | 508.3 | 507.31 |
| 972 | 1H-NMR (DMSO-D6) δ: 10.11 (1H, s), 9.25 (1H, s), 8.27 (1H, d, J = 8.8 Hz), 8.19 (1H, d, J = 2.0 Hz), 7.73 (1H, dd, J = 8.3, 2.4 Hz), 6.89 (1H, s), 6.46 (1H, d, J = 7.8 Hz), 4.28-4.22 (2H, m), 3.43 (2H, s), 3.27 (3H, s), 3.09 (2H, s), 2.99 (3H, s), 2.78 (3H, s), 2.41 (8H, s), 1.38 (3H, d, J = 6.8 Hz), 1.30 (6H, dd, J = 6.8, 3.9 Hz). | 522.3 | 521.32 |
| 973 | | 479.3 | 478.28 |
| 974 | 1H-NMR (CDCl3) δ: 9.16 (1H, s), 8.39-8.29 (3H, m), 7.77 (1H, d, J = 7.8 Hz), 7.63 (1H, s), 6.45-6.41 (1H, m), 3.71-3.63 (4H, m), 3.55 (2H, s), 3.23 (1H, s), 2.68 (13H, t, J = 16.1 Hz), 1.85-1.75 (2H, m), 1.10 (3H, t, J = 7.3 Hz). | 465.3 | 464.26 |
| 975 | 1H-NMR (CDCl3) δ: 9.20 (1H, s), 8.83 (1H, s), 8.39-8.32 (2H, m), 7.77 (1H, dd, J = 8.8, 2.0 Hz), 7.63 (1H, s), 6.40 (1H, t, J = 5.9 Hz), 3.73-3.62 (4H, m), 3.54 (2H, s), 3.23 (1H, s), 2.72 (3H, s), 2.63-2.52 (9H, m), 1.81-1.73 (2H, m), 1.58-1.48 (2H, m), 1.04 (3H, t, J = 7.3 Hz). | 479.4 | 478.28 |

**[Table 5-190]**

| Compound No. | NMR | LC/MS (M+H)⁺ | Exact Mass |
|---|---|---|---|
| 976 | 1H-NMR (CDCl3) δ: 9.06 (1H, s), 8.54 (1H, s), 8.34 (1H, d, J = 7.8 Hz), 8.29 (1H, d, J = 2.0 Hz), 7.76 (1H, dd, J = 8.8, 2.0 Hz), 6.71 (1H, s), 6.37 (1H, t, J = 5.4 Hz), 4.82 (1H, q, J = 6.5 Hz), 3.87-3.82 (1H, m), 3.69 (2H, dt, J = 14.3, 6.3 Hz), 3.52 (2H, s), 2.72 (2H, br s), 2.59-2.42 (6H, br m), 2.36-2.24 (3H, m), 1.54 (3H, d, J = 5.9 Hz), 1.38 (3H, t, J = 7.3 Hz), 1.13 (3H, d, J = 6.8 Hz). | 467.4 | 466.28 |
| 977 | 1H-NMR (CDCl3) δ: 9.03 (1H, s), 8.33 (1H, d, J = 8.8 Hz), 8.25 (1H, s), 8.21 (1H, s), 7.76 (1H, dd, J = 8.3, 2.4 Hz), 6.71 (1H, s), 6.37 (1H, d, J = 5.9 Hz), 4.82 (1H, q, J = 6.2 Hz), 3.85-3.80 (1H, m), 3.72-3.65 (2H, m), 3.51 (2H, s), 2.74-2.66 (2H, br m), 2.57-2.44 (5H, m), 2.34-2.21 (3H, m), 1.54 (3H, d, J = 5.9 Hz), 1.39 (3H, t, J = 7.3 Hz), 1.13 (3H, d, J = 5.9 Hz). | 467.4 | 466.28 |
| 978 | 1H-NMR (CDCl3) δ: 9.06 (1H, s), 8.36-8.30 (2H, m), 7.71 (1H, d, J = 7.8 Hz), 6.84 (1H, s), 6.12 (1H, s), 4.44-4.30 (2H, m), 4.03 (1H, d, J = 12.7 Hz), 3.41 (3H, s), 3.17 (1H, d, J = 11.7 Hz), 2.96-2.49 (6H, m), 2.19 (1H, br s), 1.50 (3H, d, J = 5.9 Hz), 1.36 (6H, t, J = 5.4 Hz), 1.18 (3H, br s). | 451.3 | 450.29 |
| 979 | 1H-NMR (CDCl3) δ: 9.08 (1H, s), 8.86 (1H, br s), 8.38-8.32 (2H, m), 7.71 (1H, d, J = 7.8 Hz), 6.83 (1H, s), 6.13 (1H, d, J = 6.8 Hz), 4.44-4.31 (2H, m), 4.03 (1H, d, J = 11.7 Hz), 3.41 (3H, s), 3.18 (1H, d, J = 11.7 Hz), 2.95-2.48 (7H, m), 2.17 (1H, br s), 1.50 (3H, d, J = 5.9 Hz), 1.35 (6H, t, J = 5.4 Hz), 1.18 (3H, s). | 451.3 | 450.29 |

**[Table 5-191]**

| Compound No. | NMR | LC/MS (M+H)⁺ | Exact Mass |
|---|---|---|---|
| 980 | 1H-NMR (CDCl3) δ: 9.03 (1H, s), 8.40 (1H, s), 8.37 (1H, d, J = 9.8 Hz), 7.10 (1H, d, J = 9.8 Hz), 6.70 (1H, s), 6.32 (1H, t, J = 5.4 Hz), 4.80 (1H, q, J = 6.2 Hz), 3.69-3.63 (6H, m), 2.59 (4H, t, J = 4.9 Hz), 2.38 (3H, s), 1.53 (3H, d, J = 6.8 Hz), 1.36 (3H, t, J = 7.3 Hz). | 410.3 | 409.23 |
| 981 | | 441.3 | 440.21 |
| 982 | | 467.4 | 466.28 |
| 983 | 1H-NMR (CDCl3) δ: 9.03 (1H, s), 8.34 (1H, d, J = 8.8 Hz), 8.26 (1H, s), 8.23 (1H, s), 7.75 (1H, d, J = 7.8 Hz), 6.71 (1H, s), 6.38 (1H, t, J = 5.4 Hz), 4.82 (1H, q, J = 6.5 Hz), 4.08-4.02 (1H, m), 3.73-3.65 (4H, m), 3.22 (1H, d, J = 12.7 Hz), 2.93-2.61 (6H, m), 2.43-2.20 (4H, m), 1.54 (3H, d, J = 6.8 Hz), 1.39 (3H, t, J = 7.3 Hz), 1.23 (3H, d, J = 5.9 Hz). | 467.4 | 466.28 |
| 984 | 1H-NMR (CDCl3) δ: 9.41 (1H, s), 8.57 (1H, s), 8.33-8.29 (2H, m), 7.79-7.74 (1H, m), 6.43 (1H, t, J = 5.4 Hz), 5.17 (1H, br s), 4.38 (1H, br s), 3.71-3.62 (4H, m), 3.53 (2H, s), 2.60 (11H, t, J = 5.4 Hz), 1.47 (3H, d, J = 6.8 Hz), 1.39 (3H, t, J = 6.8 Hz). | 487.3 | 486.23 |
| 985 | 1H-NMR (CDCl3) δ: 9.04 (1H, s), 8.67 (1H, s), 8.27 (1H, d, J = 8.8 Hz), 8.14 (1H, d, J = 2.0 Hz), 7.42 (1H, dd, J = 9.3, 2.4 Hz), 6.69 (1H, s), 6.25 (1H, d, J = 7.8 Hz), 4.81 (1H, q, J = 6.5 Hz), 4.44-4.35 (1H, m), 3.61-3.54 (1H, m), 3.19-2.98 (5H, m), 2.84 (1H, dd, J = 12.2, 5.4 Hz), 1.54 (3H, d, J = 5.9 Hz), 1.38 (6H, d, J = 6.8 Hz), 1.05 (3H, d, J = 6.8 Hz). | 423.3 | 422.25 |

**[Table 5-192]**

| Compound No. | NMR | LC/MS (M+H)⁺ | Exact Mass |
|---|---|---|---|
| 986 | 1H-NMR (CDCl3) δ: 9.02 (1H, s), 8.48 (1H, s), 8.26 (1H, d, J = 8.8 Hz), 8.12 (1H, d, J = 2.0 Hz), 7.42 (1H, dd, J = 8.8, 2.9 Hz), 6.68 (1H, s), 6.25 (1H, d, J = 7.8 Hz), 4.80 (1H, q, J = 6.2 Hz), 4.45-4.35 (1H, m), 3.59-3.54 (1H, m), 3.19-2.99 (5H, m), 2.84 (1H, dd, J = 12.2, 5.4 Hz), 1.53 (3H, d, J = 6.8 Hz), 1.38 (3H, d, J = 6.3 Hz), 1.37 (3H, d, J = 6.3 Hz), 1.05 (3H, d, J = 6.8 Hz). | 423.3 | 422.25 |
| 987 | 1H-NMR (CDCl3) δ: 9.06 (1H, s), 8.51 (1H, s), 8.36 (1H, d, J = 8.8 Hz), 8.29 (1H, d, J = 2.0 Hz), 7.74 (1H, dd, J = 8.3, 2.4 Hz), 6.71 (1H, s), 6.25 (1H, d, J = 7.8 Hz), 4.82 (1H, s), 4.46-4.36 (1H, m), 4.16 (2H, s), 4.09 (1H, d, J = 3.9 Hz), 3.70 (2H, t, J = 4.9 Hz), 3.65 (1H, br s), 3.54 (2H, s), 3.29 (2H, t, J = 4.9 Hz), 2.53-2.45 (4H, m), 1.54 (3H, d, J = 5.9 Hz), 1.38 (6H, d, J = 5.9 Hz). | 481.3 | 480.26 |
| 988 | 1H-NMR (CDCl3) δ: 9.12 (1H, s), 8.95 (1H, s), 8.40 (1H, d, J = 8.8 Hz), 8.33 (1H, d, J = 2.0 Hz), 7.74 (1H, dd, J = 8.3, 2.4 Hz), 6.86 (1H, s), 6.14 (1H, d, J = 7.8 Hz), 4.47-4.30 (2H, m), 4.17 (2H, s), 3.70 (2H, t, J = 4.9 Hz), 3.54 (2H, s), 3.42 (3H, s), 3.30 (2H, t, J = 4.9 Hz), 3.04 (1H, br s), 2.56-2.47 (4H, m), 1.51 (3H, d, J = 6.8 Hz), 1.36 (6H, t, J = 5.9 Hz). | 495.4 | 494.28 |
| 989 | 1H-NMR (DMSO-D6) δ: 10.28 (1H, s), 9.20 (1H, s), 8.22 (1H, d, J = 9.8 Hz), 7.46 (1H, d, J = 9.8 Hz), 6.96 (1H, s), 6.33 (1H, d, J = 6.8 Hz), 5.19 (1H, d, J = 4.9 Hz), 4.63-4.57 (1H, m), 4.32-4.20 (2H, m), 3.90 (1H, d, J = 11.7 Hz), 3.50 (1H, br s), 3.04-2.96 (4H, m), 2.80 (1H, d, J = 10.7 Hz), 1.35 (3H, dd, J = 18.1, 6.3 Hz), 1.27 (6H, d, J = 4.9 Hz). | 478.2 | 477.22 |

**[Table 5-193]**

| Compound No. | NMR | LC/MS (M+H)⁺ | Exact Mass |
|---|---|---|---|
| 990 | 1H-NMR (DMSO-D6) δ: 10.28 (1H, s), 9.20 (1H, s), 8.22 (1H, d, J = 9.8 Hz), 7.46 (1H, d, J = 9.8 Hz), 6.96 (1H, s), 6.33 (1H, d, J = 6.8 Hz), 5.19 (1H, d, J = 4.9 Hz), 4.63-4.57 (1H, m), 4.32-4.20 (2H, m), 3.90 (1H, d, J = 11.7 Hz), 3.50 (1H, br s), 3.04-2.96 (4H, m), 2.80 (1H, d, J = 10.7 Hz), 1.35 (3H, dd, J = 18.1, 6.3 Hz), 1.27 (6H, d, J = 4.9 Hz). | 478.2 | 477.22 |
| 991 | | 495.3 | 494.29 |
| 992 | 1H-NMR (400 MHz, DMSO-d6, ppm) δ: 9.77 (1H, s), 9.14 (1H, s), 8.20 (1H, d, J = 8.8Hz), 8.00 (1H, d, J = 2.4Hz), 7.43-7.40 (1H, m), 6.85-6.81 (2H, m), 4.80-4.78 (1H, m), 4.01-3.99 (1H, m), 3.83-3.82 (1H, m), 3.49-3.45 (2H, m), 3.04-3.01 (4H, m), 2.85-2.83 (4H, m), 2.24-2.23 (1H, m), 1.93-1.88 (3H, m), 1.68-1.63 (2H, m), 0.96-0.92 (3H, m) | 435.2 | 434.25 |
| 993 | 1H-NMR (400 MHz, DMSO-d6, ppm) δ: 9.77 (1H, s), 9.14 (1H, s), 8.20 (1H, d, J = 8.8Hz), 8.00 (1H, d, J = 2.4Hz), 7.43-7.40 (1H, m), 6.85-6.81 (2H, m), 4.80-4.78 (1H, m), 4.01-3.99 (1H, m), 3.83-3.82 (1H, m), 3.49-3.45 (2H, m), 3.04-3.01 (4H, m), 2.85-2.83 (4H, m), 2.24-2.23 (1H, m), 1.93-1.88 (3H, m), 1.68-1.63 (2H, m), 0.96-0.92 (3H, m) | 435.2 | 434.25 |
| 994 | 1H-NMR (400 MHz, DMSO-d6, ppm) δ: 9.73 (1H, s), 9.15 (1H, s), 8.07 (1H, d, J = 8.8Hz), 7.94 (1H, d, J = 2.8Hz), 7.39-7.37 (1H, m), 6.96 (1H, s), 6.57 (1H, d, J = 6.4Hz), 5.14 (1H, d, J = 4.4Hz), 4.56-4.53 (2H, m), 3.94-3.85 (2H, m), 3.73-3.71 (1H, m), 3.61-3.58 (1H, m), 2.98-2.96 (4H, m), 2.80-2.77 (4H, s), 2.43 (1H, m), 1.96-1.88 (1H, m), 1.32-1.31 (3H, m) | 437.2 | 436.23 |

**[Table 5-194]**

| Compound No. | NMR | LC/MS (M+H)⁺ | Exact Mass |
|---|---|---|---|
| 995 | 1H-NMR (400 MHz, DMSO-d6, ppm) δ: 9.80 (1H, s), 9.22 (1H, s), 8.13 (1H, d, J = 9.2Hz), 8.01 (1H, d, J = 2.4Hz), 7.47-7.44 (1H, m), 7.02 (1H, s), 6.63 (1H, d, J = 6.4Hz), 5.19 (1H, d, J = 4.4Hz), 4.64-4.61 (2H, m), 4.01-3.89 (2H, m), 3.82-3.66 (2H, m), 3.04 (4H, m), 2.94-2.85 (4H, m), 2.36-2.28 (2H, m), 2.14-1.99 (1H, m), 1.42-1.37 (3H, m) | 437.2 | 436.23 |
| 996 | 1H-NMR (400 MHz, DMSO-d6, ppm) δ: 9.80 (1H, s), 9.21 (1H, s), 8.16 (1H, d, J = 8.4Hz), 7.47 (1H, d, J = 8.4Hz), 6.97 (1H, s), 6.83-6.81 (1H, s), 5.19 (1H, d, J = 4.4Hz), 4.64-4.59 (1H, m), 3.82 (2H, s), 3.59-3.52 (2H, m), 3.04-3.01 (2H, m), 2.73-2.67 (2H, m), 1.40-1.38 (3H, m), 1.26-1.22 (3H, m) | 366.2 | 365.20 |
| 997 | 1H-NMR (CDCl3) δ: 9.08 (1H, s), 8.86 (1H, s), 8.33-8.27 (2H, m), 7.74 (1H, dd, J = 8.5, 2.1 Hz), 6.76 (1H, s), 6.51 (1H, s), 4.83 (1H, q, J = 6.4 Hz), 3.61 (2H, t, J = 5.3 Hz), 3.50 (2H, s), 2.97-2.90 (1H, m), 2.63-2.48 (11H, m), 1.54 (3H, d, J = 6.4 Hz), 0.95-0.89 (2H, m), 0.68-0.62 (2H, m). | 465.3 | 464.26 |
| 998 | 1H-NMR (CDCl3) δ: 9.07 (1H, s), 8.95 (1H, s), 8.38-8.30 (2H, m), 7.75 (1H, d, J = 8.7 Hz), 6.70 (1H, s), 6.47 (1H, d, J = 6.9 Hz), 4.78 (1H, q, J = 6.4 Hz), 4.71-4.61 (1H, m), 3.61 (2H, t, J = 4.6 Hz), 3.51 (2H, s), 2.62-2.44 (12H, m), 2.08-1.96 (2H, m), 1.92-1.80 (2H, m), 1.51 (3H, d, J = 5.5 Hz). | 479.4 | 478.28 |

**[Table 5-195]**

| Compound No. | NMR | LC/MS (M+H)⁺ | Exact Mass |
|---|---|---|---|
| 999 | 1H-NMR (CDCl3) δ: 9.08 (1H, s), 9.06 (1H, s), 8.36 (1H, d, J = 8.2 Hz), 8.32 (1H, s), 7.72 (1H, dd, J = 8.7, 1.8 Hz), 6.70 (1H, s), 6.50 (1H, t, J = 5.3 Hz), 4.79 (1H, q, J = 6.4 Hz), 3.61 (2H, t, J = 5.3 Hz), 3.52-3.44 (5H, m), 2.62-2.45 (11H, m), 1.52 (3H, d, J = 6.4 Hz), 1.24-1.18 (1H, m), 0.63-0.57 (2H, m), 0.37-0.32 (2H, m) . | 479.4 | 478.28 |
| 1000 | | 425.3 | 424.23 |
| 1001 | 1H-NMR (CDCl3) δ: 9.00 (1H, s), 8.50 (1H, s), 8.22 (1H, d, J = 9.1 Hz), 8.09 (1H, d, J = 3.2 Hz), 7.37 (1H, dd, J = 8.9, 2.5 Hz), 6.67 (1H, s), 6.33 (1H, t, J = 5.5 Hz), 4.79 (1H, q, J = 6.4 Hz), 3.69-3.62 (2H, m), 3.16-3.11 (4H, m), 3.08-3.04 (4H, m), 1.52 (3H, d, J = 6.4 Hz), 1.36 (3H, t, J = 7.1 Hz). | 395.3 | 394.22 |
| 1002 | 1H-NMR (CDCl3) δ: 8.97 (1H, s), 8.25 (1H, d, J = 9.8 Hz), 8.13 (1H, s), 8.07 (1H, d, J = 2.9 Hz), 7.38 (1H, dd, J = 8.8, 2.9 Hz), 6.61 (1H, s), 6.36 (1H, t, J = 5.9 Hz), 5.17 (2H, d, J = 4.9 Hz), 4.57 (2H, d, J = 5.9 Hz), 3.66-3.59 (2H, m), 3.18-3.05 (8H, m), 1.82-1.71 (5H, m), 1.06 (3H, t, J = 7.8 Hz). | 435.3 | 434.25 |
| 1003 | 1H-NMR (CDCl3) δ: 9.01 (1H, s), 8.35 (1H, d, J = 8.8 Hz), 8.31 (1H, s), 8.27 (1H, d, J = 2.0 Hz), 7.76 (1H, dd, J = 8.3, 2.4 Hz), 6.63 (1H, s), 6.38 (1H, t, J = 5.9 Hz), 5.17 (2H, d, J = 5.9 Hz), 4.58 (2H, d, J = 4.9 Hz), 3.67-3.60 (2H, m), 3.49 (2H, s), 2.95-2.86 (4H, m), 2.53-2.38 (4H, m), 1.83-1.72 (5H, m), 1.07 (3H, t, J = 7.3 Hz). | 449.3 | 448.27 |

**[Table 5-196]**

| Compound No. | NMR | LC/MS (M+H) ⁺ | Exact Mass |
|---|---|---|---|
| 1004 | 1H-NMR (CDCl3) δ: 8.94 (1H, s), 8.24 (1H, d, J = 9.1 Hz), 8.17-8.02 (2H, m), 7.41-7.32 (1H, br m), 6.63 (1H, s), 6.38-6.27 (1H, br m), 4.18 (1H, t, J = 8.0 Hz), 4.12-4.04 (1H, m), 4.02-3.89 (2H, m), 3.59 (2H, q, J = 6.6 Hz), 3.53-3.43 (1H, m), 3.25-3.01 (8H, m), 2.36-2.22 (2H, m), 1.79-1.72 (2H, m), 1.05 (3H, t, J = 7.3 Hz). | 435.3 | 434.25 |
| 1005 | 1H-NMR (CDCl3) δ: 8.99 (1H, s), 8.38-8.31 (2H, m), 8.27 (1H, d, J = 2.3 Hz), 7.74 (1H, dd, J = 8.7, 2.3 Hz), 6.66 (1H, s), 6.34 (1H, t, J = 5.7 Hz), 4.19 (1H, t, J = 8.0 Hz), 4.12-4.05 (1H, m) , 4.02-3.90 (2H, m), 3.60 (2H, q, J = 6.6 Hz), 3.54-3.45 (3H, m), 2.93-2.87 (4H, m), 2.51-2.39 (4H, br m), 2.37-2.22 (2H, m), 1.81-1.71 (2H, m), 1.06 (3H, t, J = 7.3 Hz). | 449.3 | 448.27 |
| 1006 | 1H-NMR (CDCl3) δ: 8.97 (1H, s), 8.25 (1H, d, J = 8.8 Hz), 8.14 (1H, s), 8.07 (1H, d, J = 2.9 Hz), 7.38 (1H, dd, J = 8.8, 2.9 Hz), 6.61 (1H, s), 6.16 (1H, d, J = 7.8 Hz), 5.17 (2H, d, J = 4.9 Hz), 4.58 (2H, d, J = 4.9 Hz), 4.47-4.37 (1H, m), 3.22-3.03 (8H, m), 1.80 (3H, s), 1.37 (6H, d, J = 6.8 Hz). | 435.3 | 434.25 |
| 1007 | 1H-NMR (CDCl3) δ: 8.94 (1H, s), 8.23 (1H, d, J = 9.1 Hz), 8.16-8.01 (2H, m), 7.40-7.34 (1H, m), 6.62 (1H, s), 6.12 (1H, d, J = 6.9 Hz), 4.42-4.32 (1H, m), 4.19 (1H, t, J = 8.0 Hz), 4.08 (1H, q, J = 7.2 Hz), 4.02-3.90 (2H, m), 3.52-3.43 (1H, m), 3.21-3.03 (8H, m), 2.36-2.21 (2H, m), 1.35 (6H, d, J = 6.4 Hz). | 435.3 | 434.25 |

**[Table 5-197]**

| Compound No. | NMR | LC/MS (M+H) ⁺ | Exact Mass |
|---|---|---|---|
| 1008 | 1H-NMR (CDCl3) δ: 8.97 (1H, s), 8.18-7.89 (2H, m), 7.63 (1H, s), 6.88 (1H, d, J = 7.3 Hz), 6.80 (1H, s), 6.37-6.25 (1H, br m), 4.32 (1H, q, J = 6.4 Hz), 3.92 (2H, s), 3.85 (2H, s), 3.65-3.51 (2H, m), 3.40 (3H, s), 3.26-3.12 (1H, m), 2.59-2.49 (2H, m), 2.35 (3H, s), 2.06-1.92 (2H, br m), 1.78-1.68 (2H, m), 1.50 (3H, d, J = 6.9 Hz), 1.05 (3H, t, J = 7.3 Hz). | 463.4 | 462.29 |
| 1009 | 1H-NMR (CDCl3) δ: 8.97 (1H, s), 8.19 (1H, d, J = 9.1 Hz), 7.64 (1H, br s), 6.92-6.78 (2H, m), 6.54-6.42 (1H, br m), 4.41-4.24 (2H, m), 4.11-3.63 (7H, m), 3.62-3.49 (1H, m), 3.40 (3H, s), 3.32-3.14 (1H, m), 2.64-2.48 (2H, br m), 2.36 (3H, s), 2.24-1.57 (8H, m), 1.47 (3H, d, J = 6.4 Hz). | 505.4 | 504.30 |
| 1010 | 1H-NMR (CDCl3) δ: 8.98 (1H, s), 8.34 (1H, d, J = 8.7 Hz), 8.30 (1H, s), 8.27 (1H, d, J = 1.8 Hz), 7.74 (1H, dd, J = 8.7, 2.3 Hz), 6.65 (1H, s), 6.13 (1H, d, J = 7.3 Hz), 4.44-4.33 (1H, m), 4.19 (1H, t, J = 8.0 Hz), 4.12-4.05 (1H, m), 4.03-3.90 (2H, m), 3.61 (2H, t, J = 5.5 Hz), 3.54-3.44 (3H, m), 2.80-2.21 (12H, m), 1.36 (6H, d, J = 6.4 Hz). | 493.4 | 492.30 |
| 1011 | 1H-NMR (CDCl3) δ: 9.00 (1H, s), 8.37 (1H, d, J = 8.7 Hz), 8.30 (1H, s), 8.27 (1H, d, J = 1.8 Hz), 7.73 (1H, dd, J = 8.7, 2.3 Hz), 6.69 (1H, s), 6.58-6.46 (1H, m), 4.38-4.27 (1H, br m), 4.18 (1H, t, J = 7.8 Hz), 4.11-3.87 (4H, m), 3.83-3.44 (8H, m), 2.92-2.21 (12H, m), 2.13-1.59 (4H, m). | 535.4 | 534.31 |

**[Table 5-198]**

| Compound No. | NMR | LC/MS (M+H) ⁺ | Exact Mass |
|---|---|---|---|
| 1012 | 1H-NMR (CDCl3) δ: 8.99 (1H, s), 8.39-8.32 (2H, m), 8.26 (1H, d, J = 1.8 Hz), 7.75 (1H, dd, J = 8.2, 2.3 Hz), 6.66 (1H, s), 6.13 (1H, d, J = 7.3 Hz), 4.43-4.34 (1H, m), 4.19 (1H, t, J = 8.0 Hz), 4.12-4.05 (1H, m), 4.03-3.90 (2H, m), 3.56-3.43 (5H, m), 2.75-2.40 (8H, m), 2.38-2.22 (2H, m), 1.37 (6H, d, J = 6.4 Hz). | 488.4 | 487.28 |
| 1013 | 1H-NMR (CDCl3) δ: 9.00 (1H, s), 8.38 (1H, d, J = 8.7 Hz), 8.30 (1H, s), 8.26 (1H, d, J = 1.8 Hz), 7.75 (1H, dd, J = 8.7, 2.3 Hz), 6.69 (1H, s), 6.56-6.48 (1H, m), 4.37-4.27 (1H, br m), 4.18 (1H, t, J = 7.8 Hz), 4.12-4.02 (2H, m), 3.98 (1H, q, J = 7.5 Hz), 3.90 (1H, t, J = 8.0 Hz), 3.83-3.70 (2H, m), 3.65-3.43 (6H, m), 2.74-2.40 (8H, m), 2.34-2.23 (2H, m), 2.14-2.02 (1H, m), 1.96-1.80 (2H, m), 1.79-1.67 (1H, m). | 530.4 | 529.29 |
| 1014 | 1H-NMR (CDCl3) δ: 9.01 (1H, s), 8.36 (1H, d, J = 8.7 Hz), 8.30 (1H, s), 8.26 (1H, d, J = 2.3 Hz), 7.76 (1H, dd, J = 8.5, 2.1 Hz), 6.63 (1H, s), 6.16 (1H, d, J = 7.3 Hz), 5.17 (2H, d, J = 5.5 Hz), 4.58 (2H, d, J = 5.0 Hz), 4.48-4.38 (1H, m), 3.53 (2H, s), 3.52 (2H, s), 2.73-2.40 (8H, m), 1.80 (3H, s), 1.38 (6H, d, J = 6.4 Hz). | 488.3 | 487.28 |
| 1015 | 1H-NMR (DMSO-D6) δ: 10.07 (1H, s), 9.24 (1H, s), 8.27 (1H, d, J = 8.7 Hz), 8.20 (1H, d, J = 1.8 Hz), 7.75 (1H, dd, J = 8.2, 2.3 Hz), 6.99 (1H, d, J = 0.9 Hz), 6.41 (1H, d, J = 7.8 Hz), 5.20 (1H, d, J = 4.6 Hz), 4.64-4.58 (1H, m), 4.26 (1H, td, J = 13.5, 7.0 Hz), 3.71 (2H, s), 3.45 (2H, s), 2.44 (8H, s), 1.38 (3H, d, J = 6.4 Hz), 1.29 (6H, dd, J = 6.4, 2.3 Hz). | 462.2 | 461.27 |

**[Table 5-199]**

| Compound No. | NMR | LC/MS (M+H) ⁺ | Exact Mass |
|---|---|---|---|
| 1016 | 1H-NMR (DMSO-D6) δ: 10.03 (1H, s), 9.24 (1H, s), 8.26 (1H, d, J = 8.7 Hz), 8.18 (1H, d, J = 1.8 Hz), 7.73 (1H, dd, J = 8.7, 2.3 Hz), 6.98 (1H, s), 6.41 (1H, d, J = 7.3 Hz), 5.20 (1H, d, J = 4.6 Hz), 4.64-4.57 (1H, m), 4.40 (1H, t, J = 5.3 Hz), 4.29-4.21 (1H, m), 3.41 (2H, s), 3.22 (2H, t, J = 5.7 Hz), 2.81 (2H, d, J = 11.4 Hz), 1.89 (2H, t, J = 10.5 Hz), 1.61 (2H, d, J = 11.0 Hz), 1.38 (3H, d, J = 6.4 Hz), 1.36-1.27 (7H, m), 1.15-1.06 (2H, m). | 452.3 | 451.27 |
| 1017 | 1H-NMR (DMSO-D6) δ: 10.05 (1H, s), 9.24 (1H, s), 8.27 (1H, d, J = 8.7 Hz), 8.19 (1H, d, J = 1.8 Hz), 7.74 (1H, dd, J = 8.7, 2.3 Hz), 7.59 (1H, d, J = 4.6 Hz), 6.99 (1H, d, J = 0.9 Hz), 6.41 (1H, d, J = 7.3 Hz), 5.19 (1H, d, J = 4.6 Hz), 4.64-4.58 (1H, m), 4.31-4.22 (1H, m), 3.46 (2H, s), 2.86 (2H, s), 2.59 (3H, d, J = 5.0 Hz), 2.42 (8H, s), 1.38 (3H, d, J = 6.4 Hz), 1.29 (6H, dd, J = 6.6, 2.1 Hz). | 494.3 | 493.29 |
| 1018 | 1H-NMR (DMSO-D6) δ: 10.06 (1H, s), 9.24 (1H, s), 8.27 (1H, d, J = 8.7 Hz), 8.19 (1H, d, J = 2.3 Hz), 7.73 (1H, dd, J = 8.7, 2.3 Hz), 6.99 (1H, s), 6.41 (1H, d, J = 7.8 Hz), 5.19 (1H, d, J = 4.6 Hz), 4.65-4.57 (1H, m), 4.30-4.22 (1H, m), 3.43 (2H, s), 3.09 (2H, s), 2.99 (3H, s), 2.78 (3H, s), 2.41 (8H, s), 1.38 (3H, d, J = 6.4 Hz), 1.29 (6H, dd, J = 6.4, 2.3 Hz). | 508.3 | 507.31 |

**[Table 5-200]**

| Compound No. | NMR | LC/MS (M+H) ⁺ | Exact Mass |
|---|---|---|---|
| 1019 | 1H-NMR (DMSO-D6) δ: 10.04 (1H, s), 9.24 (1H, s), 8.26 (1H, d, J = 8.7 Hz), 8.18 (1H, d, J = 1.8 Hz), 7.73 (1H, dd, J = 8.7, 2.3 Hz), 6.98 (1H, d, J = 0.9 Hz), 6.41 (1H, d, J = 7.3 Hz), 5.19 (1H, d, J = 4.6 Hz), 4.64-4.58 (1H, m), 4.53 (1H, d, J = 4.1 Hz), 4.26 (1H, td, J = 13.5, 6.7 Hz), 3.47-3.40 (3H, m), 2.69-2.63 (2H, m), 2.03 (2H, t, J = 9.8 Hz), 1.72-1.67 (2H, m), 1.41-1.34 (5H, m), 1.29 (6H, dd, J = 6.3, 2.0 Hz). | 438.3 | 437.25 |
| 1020 | 1H-NMR (DMSO-D6) δ: 10.15 (1H, s), 9.23 (1H, s), 8.20-8.16 (2H, m), 7.70 (1H, dd, J = 8.7, 2.3 Hz), 6.98 (1H, d, J = 0.9 Hz), 6.42 (1H, s), 5.20 (1H, d, J = 4.6 Hz), 4.66-4.59 (1H, m), 4.53 (1H, d, J = 4.1 Hz), 3.47-3.39 (3H, m), 2.68-2.63 (2H, m), 2.02 (2H, t, J = 9.8 Hz), 1.71-1.66 (2H, m), 1.53 (9H, s), 1.42-1.32 (5H, m). | 452.2 | 451.27 |
| 1021 | 1H-NMR (DMSO-D6) δ: 10.16 (1H, s), 9.23 (1H, s), 8.20-8.16 (2H, m), 7.70 (1H, dd, J = 8.2, 2.3 Hz), 6.98 (1H, d, J = 0.9 Hz), 6.42 (1H, s), 5.21 (1H, d, J = 4.6 Hz), 4.65-4.59 (1H, m), 4.40 (1H, t, J = 5.3 Hz), 3.41 (2H, s), 3.22 (2H, t, J = 5.7 Hz), 2.80 (2H, d, J = 11.4 Hz), 1.88 (2H, t, J = 10.5 Hz), 1.61 (2H, d, J = 11.0 Hz), 1.53 (9H, s), 1.39 (3H, d, J = 6.9 Hz), 1.32 (1H, s), 1.16-1.06 (2H, m). | 466.2 | 465.29 |
| 1022 | 1H-NMR (DMSO-D6) δ: 10.00 (1H, s), 9.23 (1H, s), 8.37 (1H, d, J = 8.7 Hz), 8.18 (1H, d, J = 2.3 Hz), 7.73 (1H, dd, J = 8.2, 2.3 Hz), 6.97 (1H, s), 6.87 (1H, t, J = 5.9 Hz), 5.17 (1H, d, J = 4.6 Hz), 4.64-4.57 (1H, m), 4.53 (1H, d, J = 4.1 Hz), 3.54-3.40 (5H, m), 2.69-2.64 (2H, m), 2.03 (2H, t, J = 10.1 Hz), 1.72-1.63 (4H, m), 1.41-1.33 (5H, m), 0.95 (3H, t, J = 7.5 Hz). | 438.2 | 437.25 |

**[Table 5-201]**

| Compound No. | NMR | LC/MS (M+H) ⁺ | Exact Mass |
|---|---|---|---|
| 1023 | 1H-NMR (DMSO-D6) δ: 10.03 (1H, s), 9.23 (1H, s), 8.34 (1H, d, J = 8.7 Hz), 8.18 (1H, d, J = 1.8 Hz), 7.71 (1H, dd, J = 8.2, 2.3 Hz), 6.97 (1H, d, J = 0.9 Hz), 6.83 (1H, t, J = 6.2 Hz), 5.18 (1H, d, J = 4.1 Hz), 4.62-4.52 (2H, m), 3.47-3.34 (5H, m), 2.69-2.63 (2H, m), 2.09-1.98 (3H, m), 1.72-1.65 (2H, m), 1.42-1.33 (5H, m), 0.95 (6H, d, J = 6.3 Hz). | 452.3 | 451.27 |
| 1024 | 1H-NMR (DMSO-D6) δ: 10.03 (1H, s), 9.23 (1H, s), 8.34 (1H, d, J = 8.7 Hz), 8.18 (1H, d, J = 1.8 Hz), 7.71 (1H, dd, J = 8.2, 2.3 Hz), 6.97 (1H, d, J = 0.9 Hz), 6.83 (1H, t, J = 5.9 Hz), 5.17 (1H, d, J = 4.6 Hz), 4.63-4.56 (1H, m), 4.39 (1H, t, J = 5.3 Hz), 3.46-3.33 (4H, m), 3.22 (2H, t, J = 5.7 Hz), 2.81 (2H, d, J = 11.4 Hz), 2.05-1.98 (1H, m), 1.89 (2H, t, J = 10.5 Hz), 1.62 (2H, d, J = 10.5 Hz), 1.39-1.25 (4H, m), 1.17-1.05 (2H, m), 0.95 (6H, d, J = 6.9 Hz). | 466.2 | 465.29 |
| 1025 | 1H-NMR (400 MHz, DMSO-d6, ppm) δ: 9.81 (1H, s), 9.16 (1H, s), 8.09 (1H, d, J = 8.4Hz), 8.00 (1H, s), 7.45 (1H, d, J = 7.2Hz), 6.86 (1H, s), 6.36 (1H, d, J = 6.0Hz), 4.80 (1H, br), 4.22-4.21 (1H, m), 4.00 (1H, d, J = 5.6Hz), 3.84-3.82 (1H, m), 3.15-3.04 (4H, m), 2.86 (4H, m), 2.24 (1H, m), 1.91-1.89 (3H, m), 1.29-1.28 (6H, m) | 435.2 | 434.25 |
| 1026 | 1H-NMR (400 MHz, DMSO-d6, ppm) δ: 9.81 (1H, s), 9.16 (1H, s), 8.09 (1H, d, J = 8.8Hz), 8.00 (1H, d, J = 4.8Hz), 7.47-7.44 (1H, m), 6.86 (1H, s), 6.36 (1H, d, J = 7.6Hz), 4.82-4.79 (1H, m), 4.24-4.19 (1H, m), 4.03-4.00 (1H, m), 3.85-3.82 (1H, m), 3.05-3.04 (4H, m), 2.87 (4H, m), 2.27-2.22 (1H, m), 1.97-1.88 (3H, m), 1.29-1.27 (6H, m) | 435.2 | 434.25 |

**[Table 5-202]**

| Compound No. | NMR | LC/MS (M+H) ⁺ | Exact Mass |
|---|---|---|---|
| 1027 | 1H-NMR (400 MHz, DMSO-d6, ppm) δ: 10.26 (1H, s), 9.38 (1H, s), 8.01 (1H, d, J = 8.0Hz), 7.63 (1H, d, J = 9.2Hz), 7.51 (1H, d, J = 8.4Hz), 6.56 (1H, d, J = 7.2Hz), 4.35-4.30 (1H, m), 3.83 (2H, s), 3.03-3.02 (2H, m), 2.73-2.69 (2H, m), 2.67-2.61 (3H, m), 1.41-1.35 (6H, m) | 378.1 | 377.20 |
| 1028 | 1H-NMR (400 MHz, DMSO-d6, ppm) δ: 10.13 (1H, s), 9.34 (1H, s), 8.22 (1H, d, J = 9.2Hz), 8.05 (1H, d, J = 2.8Hz), 7.59 (1H, s), 7.46-7.43 (1H, m), 7.02-6.99 (1H, m), 3.60-3.55 (2H, m), 3.24-3.05 (4H, m), 2.87-2.85 (4H, m), 2.60-2.56 (3H, m), 1.77-1.71 (2H, m), 1.23-0.97 (3H, m) | 407.2 | 406.22 |
| 1029 | 1H-NMR (400 MHz, DMSO-d6, ppm) δ: 10.51 (1H, s), 9.41 (1H, s), 8.29 (1H, d, J = 8.8Hz), 8.20 (1H, d, J = 2.4Hz), 7.72-7.69 (1H, m), 7.62 (1H, s), 6.60 (1H, d, J = 7.6Hz), 4.36-4.31 (1H, m), 3.78-3.76 (2H, m), 2.96-2.90 (4H, m), 2.71-2.68 (2H, m), 2.60 (3H, s), 1.36-1.30 (6H, m) | 435.0 | 434.22 |
| 1030 | 1H-NMR (400 MHz, DMSO-d6, ppm) δ: 10.53 (1H, s), 9.43 (1H, s), 8.35-8.33 (2H, mz), 7.86 (1H, dd, J1 = 9.2Hz, J2 = 2.4Hz), 7.64 (1H, s), 6.64 (1H, d, J = 7.6 Hz), 4.33-4.39 (1H, br), 3.68-3.66-4.23 (2H, m), 3.42-3.3.40 (2H, m), 3.17-3.15 (2H, m), 3.05-3.04 (2H, m), 1.37-1.358 (6H, d, J = 6.4Hz), 1.13-1.09 (3H, m) | 435.2 | 434.22 |
| 1031 | 1H-NMR (400 MHz, DMSO-d6, ppm) δ: 10.73 (1H, s), 9.51 (1H, s), 36-8.29 (2H, m), 7.85 (1H, d, J = 8.0Hz), 6.77 (1H, d, J = 7.2Hz), 4.29-4.26 (1H, m), 3.67 (2H, s), 3.49-3.46 (3H, m), 3.08 (1H, s), 2.59 (3H, s), 1.34-1.32 (6H, d, J = 6.0Hz) | 445.0 | 454.16 |

**[Table 5-203]**

| Compound No. | NMR | LC/MS (M+H) ⁺ | Exact Mass |
|---|---|---|---|
| 1032 | 1H-NMR (400 MHz, DMSO-d6, ppm) δ: 10.21 (1H, s), 9.28 (1H, s), 8.35-8.31 (2H, m), 7.83 (1H, d, J = 8.4HZ), 7.02 (1H, s), 6.45 (1H, d, J = 6.8Hz), 5.23 (1H, d, J = 3.2 Hz), 4.64 (1H, br), 4.30 (1H, br), 3.57-3.41 (3H, m), 3.18 (1H, br), 2.68 (1H, br), 1.42 (3H, d, J = 6.0Hz), 1.33 (6H, d, J = 5.2Hz), 1.12 (3H, d, J = 6.0Hz) | 420.0 | 436.23 |
| 1033 | 1H-NMR (400 MHz, DMSO-d6, ppm) δ: 10.20 (1H, s), 9.26 (1H, s), 8.35 (1H, d, J = 8.8Hz), 8.14 (1H, d, J = 2.4HZ), 7.70 (1H, dd, J1 = 8.8 Hz, J2 = 2.4 Hz), 6.99 (1H, s), 6.44 (1H, d, J = 8.4 Hz), 5.20 (1H, d, J = 4.8 Hz), 4.61 (1H, br), 4.25 (1H, br), 3.91 (1H, br), 3.38 (2H, d, J = 9.6 Hz), 3.31 (1H, br), 2.66 (1H, br), 1.39 (3H, d, J = 6.4 Hz), 1.30-1.28 (6H, m), 1.04 (3H, d, J = 6.4Hz) | 437.2 | 436.23 |
| 1034 | 1H-NMR (400 MHz, DMSO-d6, ppm) δ: 9.85 (1H, s), 9.20 (1H, s), 8.14 (1H, d, J = 9.2Hz), 7.05 (1H, d, J = 3.2Hz), 7.51 (1H, dd, J1 = 9.2Hz, J2 = 3.2Hz), 6.95 (1H, s), 6.34 (1H, d, J = 7.6 Hz), 5.11 (1H, d, J = 8.8Hz), 4.43-4.39 (1H, m), 4.28-4.23 (1H, m), 3.17-3.14 (4H, m), 3.02-2.99 (4H, m), 1.91-1.85 (1H, m), 1.65-1.58 (1H, m), 1.31-1.28 (6H, m), 0.90-0.86 (3H, m) | 423.1 | 422.25 |
| 1035 | 1H-NMR (400 MHz, DMSO-d6, ppm) δ: 9.85 (1H, s), 9.20 (1H, s), 8.14 (1H, d, J = 9.2Hz), 7.05 (1H, d, J = 3.2Hz), 7.51 (1H, dd, J1 = 9.2Hz, J2 = 3.2Hz), 6.95 (1H, s), 6.34 (1H, d, J = 7.6 Hz), 5.11 (1H, d, J = 8.8Hz), 4.43-4.39 (1H, m), 4.28-4.23 (1H, m), 3.17-3.14 (4H, m), 3.02-2.99 (4H, m), 1.91-1.85 (1H, m), 1.65-1.58 (1H, m), 1.31-1.28 (6H, m), 0.90-0.86 (3H, m) | 423.1 | 422.25 |

**[Table 5-204]**

| Compound No. | NMR | LC/MS (M+H) ⁺ | Exact Mass |
|---|---|---|---|
| 1036 | 1H-NMR (400 MHz, DMSO-d6, ppm) δ: 10.21 (1H, s), 9.27 (1H, s), 8.34-8.29 (2H, m), 7.82-7.80 (1H, m), 6.99 (1H, s), 6.43 (1H, d, J = 7.68Hz), 5.14 (1H, d, J = 4.8 Hz), 4.44-4.41 (1H, m), 4.30-4.25 (1H, m), 3.68-3.66 (2H, m), 3.47 (2H, s), 3.12-3.09 (1H, m), 1.91-1.85 (1H, m), 1.31-1.29 (6H, m), 0.91-0.87 (3H, m) | 437.1 | 436.23 |
| 1037 | 1H-NMR (400 MHz, DMSO-d6, ppm) δ: 10.21 (1H, s), 9.27 (1H, s), 8.34-8.29 (2H, m), 7.82-7.80 (1H, m), 6.99 (1H, s), 6.43 (1H, d, J = 7.68Hz), 5.14 (1H, d, J = 4.8 Hz), 4.44-4.41 (1H, m), 4.30-4.25 (1H, m), 3.68-3.66 (2H, m), 3.47 (2H, s), 3.12-3.09 (1H, m), 1.91-1.85 (1H, m), 1.31-1.29 (6H, m), 0.91-0.87 (3H, m) | 437.0 | 436.23 |
| 1038 | 1H-NMR (DMSO-D6) δ: 10.05 (1H, s), 9.24 (1H, s), 8.26 (1H, d, J = 8.7 Hz), 8.18 (1H, d, J = 2.3 Hz), 7.73 (1H, dd, J = 8.2, 2.3 Hz), 6.98 (1H, d, J = 0.9 Hz), 6.41 (1H, d, J = 7.8 Hz), 5.20 (1H, d, J = 4.6 Hz), 4.61 (1H, dt, J = 11.7, 5.7 Hz), 4.30-4.21 (1H, m), 3.39 (2H, s), 2.32 (4H, s), 1.51-1.44 (4H, m), 1.40-1.33 (5H, m), 1.29 (6H, dd, J = 6.4, 2.3 Hz). | 422.2 | 421.26 |
| 1039 | 1H-NMR (DMSO-D6) δ: 10.08 (1H, s), 9.29 (1H, s), 8.30 (1H, d, J = 8.7 Hz), 8.25 (1H, d, J = 1.8 Hz), 7.78 (1H, dd, J = 8.7, 2.3 Hz), 7.03 (1H, s), 6.45 (1H, d, J = 7.8 Hz), 5.24 (1H, d, J = 4.6 Hz), 4.68-4.62 (1H, m), 4.34-4.25 (1H, m), 3.55 (2H, s), 2.50 (4H, q, J = 6.6 Hz), 1.42 (3H, d, J = 6.2 Hz), 1.33 (6H, dd, J = 6.2, 2.0 Hz), 1.02 (6H, t, J = 7.1 Hz). | 410.3 | 409.26 |

**[Table 5-205]**

| Compound No. | NMR | LC/MS (M+H)⁺ | Exact Mass |
|---|---|---|---|
| 1040 | 1H-NMR (DMSO-D6) δ**:** 10.03 (1H, s), 9.23 (1H, s), 8.38 (1H, d, J = 8.7 Hz), 8.18 (1H, d, J = 1.8 Hz), 7.73 (1H, dd, J = 8.7, 2.3 Hz), 6.97 (1H, s), 6.88 (1H, t, J = 5.9 Hz), 5.18 (1H, d, J = 4.6 Hz), 4.64-4.57 (1H, m), 4.40 (1H, t, J = 5.3 Hz), 3.55-3.44 (2H, m), 3.41 (2H, s), 3.22 (2H, t, J = 5.7 Hz), 2.81 (2H, d, J = 11.4 Hz), 1.89 (2H, t, J = 10.5 Hz), 1.71-1.58 (4H, m), 1.40-1.26 (4H, m), 1.15-1.06 (2H, m), 0.94 (3H, t, J = 7.5 Hz). | 452.3 | 451.27 |
| 1041 | 1H-NMR (DMSO-D6) δ**:** 10.08 (1H, s), 9.25 (1H, s), 8.26 (1H, d, J = 8.7 Hz), 8.21 (1H, d, J = 1.8 Hz), 7.74 (1H, dd, J = 8.7, 2.3 Hz), 6.99 (1H, s), 6.41 (1H, d, J = 7.8 Hz), 5.20 (1H, d, J = 4.6 Hz), 4.69 (1H, d, J = 4.6 Hz), 4.65-4.57 (1H, m), 4.30-4.15 (2H, m), 3.53 (2H, q, J = 11.2 Hz), 2.67 (1H, dd, J = 9.6, 5.9 Hz), 2.57 (1H, q, J = 7.8 Hz), 2.41 (1H, dd, J = 14.0, 8.0 Hz), 2.31 (1H, dd, J = 9.6, 3.7 Hz), 1.98 (1H, td, J = 13.7, 7.2 Hz), 1.57-1.49 (1H, m), 1.38 (3H, d, J = 6.4 Hz), 1.29 (6H, dd, J = 6.4, 1.8 Hz). | 424.2 | 423.24 |
| 1042 | 1H-NMR (CDCl3) δ**:** 9.00 (1H, s), 8.47-8.39 (2H, m), 8.34 (1H, d, J = 2.3 Hz), 7.76 (1H, dd, J = 8.9, 2.5 Hz), 6.63 (1H, s), 6.17 (1H, d, J = 7.3 Hz), 4.48-4.37 (1H, m), 3.79-3.70 (4H, m), 3.41-3.23 (3H, m), 3.11-2.94 (2H, m), 2.91-2.77 (2H, m), 1.38 (6H, d, J = 6.4 Hz). | 469.3 | 468.22 |

**[Table 5-206]**

| Compound No. | NMR | LC/MS (M+H)⁺ | Exact Mass |
|---|---|---|---|
| 1043 | 1H-NMR (CDCl3) δ**:** 9.05 (1H, s), 8.64 (1H, s), 8.44 (1H, d, J = 9.1 Hz), 8.34 (1H, d, J = 2.3 Hz), 7.74 (1H, dd, J = 8.7, 2.7 Hz), 6.70 (1H, s), 6.50 (1H, t, J = 5.3 Hz), 4.79 (1H, q, J = 6.3 Hz), 3.76-3.70 (4H, m), 3.50-3.46 (2H, m), 3.28-3.22 (2H, m), 1.68 (2H, br s), 1.52 (3H, d, J = 6.4 Hz), 1.24-1.18 (1H, m), 0.63-0.58 (2H, m), 0.38-0.32 (2H, m). | 435.3 | 434.22 |
| 1044 | 1H-NMR (CDCl3) δ**:** 9.07 (1H, s), 8.76 (1H, s), 8.39-8.32 (2H, m), 7.74 (1H, dd, J = 9.1, 2.7 Hz), 6.75 (1H, s), 6.49 (1H, s), 4.83 (1H, q, J = 6.4 Hz), 3.76-3.70 (4H, m), 3.26 (2H, t, J = 5.5 Hz), 2.96-2.90 (1H, m), 1.54 (3H, d, J = 6.4 Hz), 1.24 (1H, s), 0.94-0.89 (2H, m), 0.67-0.63 (2H, m). | 421.3 | 420.20 |
| 1045 | 1H-NMR (CDCl3) δ**:** 9.07 (1H, s), 9.01 (1H, s), 8.42 (1H, d, J = 8.7 Hz), 8.37 (1H, d, J = 2.3 Hz), 7.76 (1H, dd, J = 8.7, 2.7 Hz), 6.69 (1H, d, J = 0.9 Hz), 6.44 (1H, d, J = 6.9 Hz), 4.78 (1H, q, J = 6.4 Hz), 4.69-4.61 (1H, m), 3.76-3.71 (4H, m), 3.26 (2H, t, J = 5.3 Hz), 2.56-2.48 (2H, m), 2.07-1.97 (2H, m), 1.89-1.80 (3H, m), 1.51 (3H, d, J = 6.4 Hz). | 435.3 | 434.22 |
| 1046 | 1H-NMR (CDCl3) δ**:** 9.08 (1H, s), 8.51 (1H, br s), 8.11 (1H, d, J = 8.2 Hz), 7.61 (1H, d, J = 8.7 Hz), 6.68 (1H, s), 6.34 (1H, t, J = 5.7 Hz), 4.80 (1H, q, J = 6.4 Hz), 3.70-3.60 (4H, m), 3.45 (2H, s), 2.58-2.46 (13H, m), 1.52 (3H, d, J = 6.4 Hz), 1.36 (3H, t, J = 7.1 Hz). | 467.4 | 466.28 |
| 1047 | 1H-NMR (CDCl3) **δ:** 9.04 (1H, s), 8.28 (1H, s), 8.11 (1H, d, J = 8.2 Hz), 7.60 (1H, d, J = 8.2 Hz), 6.68 (1H, s), 6.44 (1H, t, J = 5.7 Hz), 4.80 (1H, q, J = 6.4 Hz), 3.63-3.57 (4H, m), 3.46 (2H, s), 2.57-2.48 (13H, m), 1.81-1.73 (2H, m), 1.52 (3H, d, J = 6.4 Hz), 1.07 (3H, t, J = 7.3 Hz). | 481.4 | 480.30 |

**[Table 5-207]**

| Compound No. | NMR | LC/MS (M+H)⁺ | Exact Mass |
|---|---|---|---|
| 1048 | 1H-NMR (CDCl3) δ: 8.99 (1H, s), 8.25 (1H, d, J = 8.7 Hz), 8.04 (1H, s), 7.55 (1H, d, J = 8.7 Hz), 6.69 (1H, s), 6.48 (1H, d, J = 6.9 Hz), 4.78 (1H, t, J = 5.7 Hz), 4.70-4.62 (1H, m), 4.13 (1H, d, J = 5.5 Hz), 3.73 (2H, s), 3.64-3.48 (3H, m), 3.28-3.22 (2H, m), 2.56-2.48 (2H, m), 2.40 (3H, s), 2.01 (2H, dd, J = 17.8, 10.1 Hz), 1.89-1.81 (2H, m), 1.51 (3H, d, J = 6.4 Hz). | 449.3 | 448.23 |
| 1049 | 1H-NMR (CDCl3) δ: 9.65 (1H, s), 9.03 (1H, s), 8.32 (1H, s), 8.04 (1H, s), 6.70 (1H, s), 6.24 (1H, d, J = 7.8 Hz), 4.80 (1H, q, J = 6.6 Hz), 4.46-4.39 (1H, m), 3.67 (2H, s), 2.95 (4H, t, J = 4.8 Hz), 2.54 (4H, br s), 1.52 (3H, d, J = 6.4 Hz), 1.37 (6H, d, J = 6.4 Hz), 1.24 (1H, s). | 424.3 | 423.25 |
| 1050 | 1H-NMR (CDCl3) δ: 9.66 (1H, d, J = 0.9 Hz), 9.05 (1H, s), 8.32 (1H, s), 8.14 (1H, s), 6.71 (1H, s), 6.24 (1H, d, J = 7.8 Hz), 4.80 (1H, q, J = 6.4 Hz), 4.46-4.39 (1H, m), 3.71 (2H, s), 3.66 (2H, t, J = 5.0 Hz), 2.73-2.55 (10H, br m), 1.52 (3H, d, J = 6.4 Hz), 1.37 (6H, d, J = 6.4 Hz), 1.24 (1H, s). | 468.3 | 467.28 |
| 1051 | 1H-NMR (CDCl3) δ: 8.99 (1H, s), 8.25 (2H, d, J = 8.7 Hz), 8.07 (1H, d, J = 2.7 Hz), 7.36 (1H, dd, J = 9.1, 3.2 Hz), 6.67 (1H, s), 6.51 (1H, t, J = 5.3 Hz), 4.78 (1H, q, J = 6.4 Hz), 3.48 (2H, t, J = 6.4 Hz), 3.16-3.11 (4H, m), 3.08-3.04 (4H, m), 1.51 (3H, d, J = 6.4 Hz), 1.26-1.16 (2H, m), 0.64-0.57 (2H, m), 0.38-0.32 (2H, m). | 421.3 | 420.24 |

**[Table 5-208]**

| Compound No. | NMR | LC/MS (M+H)⁺ | Exact Mass |
|---|---|---|---|
| 1052 | 1H-NMR (CDCl3) δ: 9.32 (1H, s), 9.20 (1H, s), 8.29 (1H, d, J = 9.1 Hz), 8.18 (1H, d, J = 3.2 Hz), 7.61 (1H, s), 7.37 (1H, dd, J = 8.9, 3.0 Hz), 6.46 (1H, t, J = 5.5 Hz), 3.57-3.51 (2H, m), 3.19-3.15 (4H, m), 3.11-3.07 (4H, m), 2.71 (3H, s), 1.70 (1H, s), 1.29-1.22 (1H, m), 0.65-0.59 (2H, m), 0.42-0.36 (2H, m). | 419.3 | 418.22 |
| 1053 | 1H-NMR (CDCl3) δ: 9.06 (1H, s), 8.75 (1H, s), 8.24 (1H, s), 8.16 (1H, s), 6.70 (1H, d, J = 0.9 Hz), 6.32 (1H, t, J = 5.3 Hz), 4.80 (1H, q, J = 6.4 Hz), 3.69-3.58 (4H, m), 3.46 (3H, d, J = 5.5 Hz), 2.54-2.46 (13H, m), 1.52 (3H, d, J = 6.4 Hz), 1.39 (3H, t, J = 7.3 Hz). | 467.3 | 466.28 |
| 1054 | 1H-NMR (CDCl3) δ: 9.03 (1H, s), 8.36 (1H, s), 8.22 (1H, s), 8.12 (1H, s), 6.69 (1H, s), 6.43 (1H, t, J = 5.5 Hz), 4.80 (1H, q, J = 6.3 Hz), 3.62-3.57 (4H, m), 3.47 (3H, d, J = 6.9 Hz), 2.55-2.46 (14H, m), 1.79 (2H, td, J = 14.6, 7.3 Hz), 1.52 (3H, d, J = 6.4 Hz), 1.10 (3H, t, J = 7.5 Hz). | 481.4 | 480.30 |
| 1055 | 1H-NMR (CDCl3) δ: 9.05 (1H, s), 8.64 (1H, s), 8.26 (1H, s), 8.15 (1H, s), 6.69 (1H, s), 6.28 (1H, d, J = 7.3 Hz), 4.80 (1H, q, J = 6.4 Hz), 4.42-4.35 (1H, m), 3.60 (2H, t, J = 5.5 Hz), 3.48-3.44 (3H, m), 2.51 (14H, dd, J = 18.5, 13.5 Hz), 1.52 (3H, d, J = 6.4 Hz), 1.38 (6H, d, J = 6.4 Hz) . | 481.4 | 480.30 |
| 1056 | 1H-NMR (CDCl3) δ: 9.03 (1H, s), 8.47 (1H, d, J = 10.1 Hz), 8.23 (1H, br s), 7.09 (1H, d, J = 9.6 Hz), 6.87 (1H, s), 6.50 (1H, d, J = 8.2 Hz), 4.33-4.30 (2H, m), 4.03 (1H, dd, J = 11.0, 3.2 Hz), 3.77-3.73 (5H, m), 3.65-3.55 (9H, m), 3.40 (3H, s), 2.05-2.03 1H, m), 1.89-1.87 (2H, m), 1.72-1.69 (1H, m), 1.48 (3H, d, J = 6.4 Hz). | 524.2 | 523.27 |

**[Table 5-209]**

| Compound No. | NMR | LC/MS (M+H)⁺ | Exact Mass |
|---|---|---|---|
| 1057 | 1H-NMR (DMSO-D6) δ: 10.06 (1H, s), 9.24 (1H, s), 8.26 (1H, d, J = 8.7 Hz), 8.19 (1H, d, J = 1.8 Hz), 7.73 (1H, dd, J = 8.2, 2.3 Hz), 6.99 (1H, d, J = 0.9 Hz), 6.41 (1H, d, J = 7.8 Hz), 5.20 (1H, d, J = 4.6 Hz), 4.64-4.56 (2H, m), 4.30-4.22 (1H, m), 3.52-3.36 (3H, m), 2.79 (1H, d, J = 6.9 Hz), 2.65 (1H, d, J = 11.0 Hz), 1.86 (1H, t, J = 10.1 Hz), 1.78 (1H, d, J = 8.7 Hz), 1.69 (1H, t, J = 10.1 Hz), 1.63-1.57 (1H, m), 1.45-1.35 (4H, m), 1.29 (6H, dd, J = 6.4, 2.3 Hz), 1.10-1.00 (1H, m). | 438.25 | 437.25 |
| 1058 | | 454.2 | 453.25 |
| 1059 | | 454.2 | 453.25 |
| 1060 | 1H-NMR (DMSO-D6) δ: 10.01 (1H, s), 9.23 (1H, s), 8.41 (1H, d, J = 8.7 Hz), 8.20 (1H, s), 7.75 (1H, d, J = 8.2 Hz), 6.97 (1H, s), 6.90 (1H, t, J = 5.5 Hz), 5.18 (1H, d, J = 4.1 Hz), 4.64-4.52 (2H, m), 3.59-3.52 (2H, m), 3.45 (2H, br s), 3.32 (2H, s), 2.69 (2H, s), 2.08 (1H, br s), 1.69 (2H, br s), 1.43-1.33 (5H, m), 1.24 (3H, t, J = 7.1 Hz). | 424.2 | 423.24 |
| 1061 | 1H-NMR (DMSO-D6) δ: 10.03 (1H, s), 9.23 (1H, s), 8.37 (1H, d, J = 8.2 Hz), 8.20 (1H, s), 7.74 (1H, d, J = 8.2 Hz), 6.96 (1H, s), 6.84 (1H, t, J = 5.9 Hz), 5.17 (1H, d, J = 4.6 Hz), 4.63-4.54 (2H, m), 3.58-3.41 (5H, m), 3.32 (2H, s), 2.69 (2H, s), 2.06 (1H, br s), 1.73-1.60 (4H, m), 1.43-1.34 (6H, m), 0.93 (3H, t, J = 7.3 Hz). | 452.3 | 451.27 |
| 1062 | 1H-NMR (DMSO-D6) δ: 10.07 (1H, s), 9.24 (1H, s), 8.37 (1H, d, J = 8.7 Hz), 8.20 (1H, s), 7.75 (1H, d, J = 8.2 Hz), 6.98 (1H, s), 6.91 (1H, t, J = 5.9 Hz), 5.19 (1H, d, J = 4.6 Hz), 4.63-4.55 (2H, m), 3.50-3.38 (2H, m), 2.69 (2H, br s), 2.07 (1H, br s), 1.69 (2H, br s), 1.44-1.35 (5H, m), 1.26-1.18 (1H, m), 0.49-0.43 (2H, m), 0.35-0.30 (2H, m). | 450.3 | 449.25 |

**[Table 5-210]**

| Compound No. | NMR | LC/MS (M+H)⁺ | Exact Mass |
|---|---|---|---|
| 1063 | 1H-NMR (DMSO-D6) δ: 10.01 (1H, s), 9.24 (1H, s), 8.34 (1H, d, J = 8.7 Hz), 8.19 (1H, s), 7.76 (1H, d, J = 8.7 Hz), 7.00 (1H, d, J = 0.9 Hz), 6.83 (1H, d, J = 7.8 Hz), 5.19 (1H, d, J = 4.6 Hz), 4.64-4.54 (3H, m), 3.48-3.41 (2H, m), 2.39-2.32 (2H, m), 2.16-2.00 (4H, m), 1.78-1.65 (4H, m), 1.43-1.34 (5H, m). | 450.3 | 449.25 |
| 1064 | 1H-NMR (DMSO-D6) δ: 10.06 (1H, s), 9.24 (1H, s), 8.24 (1H, d, J = 8.2 Hz), 8.18 (1H, s), 7.73 (1H, dd, J = 8.5, 2.1 Hz), 6.98 (1H, s), 6.40 (1H, d, J = 7.8 Hz), 5.19 (1H, d, J = 4.6 Hz), 4.64-4.53 (3H, m), 3.95-3.86 (1H, m), 3.53-3.40 (4H, m), 2.66 (2H, s), 2.11-2.00 (4H, m), 1.90-1.83 (2H, m), 1.73-1.66 (2H, m), 1.46-1.27 (9H, m). | 494.3 | 493.28 |
| 1065 | 1H-NMR (DMSO-D6) δ: 10.04 (1H, s), 9.23 (1H, s), 8.36 (1H, d, J = 8.7 Hz), 8.18 (1H, s), 7.72 (1H, dd, J = 8.7, 2.3 Hz), 6.97 (1H, d, J = 0.9 Hz), 6.89 (1H, t, J = 6.2 Hz), 5.19 (1H, d, J = 4.6 Hz), 4.64-4.53 (2H, m), 3.85 (2H, dd, J = 11.2, 2.5 Hz), 3.53-3.38 (5H, m), 3.26 (2H, td, J = 11.7, 1.7 Hz), 2.66 (2H, br s), 2.10-1.90 (3H, m), 1.70 (2H, d, J = 9.6 Hz), 1.62 (2H, d, J = 12.8 Hz), 1.42-1.24 (7H, m). | 494.2 | 493.28 |
| 1066 | 1H-NMR (400 MHz, DMSO-d6, ppm) δ: 10.06 (1H, s), 9.24 (1H, s), 8.25 (1H, d, J = 8.4 Hz), 8.18 (1H, d, J = 2.0 Hz), 7.75-7.72 (1H, m), 6.86 (1H, s), 6.44 (1H, d, J = 7.6 Hz), 4.28-4.23 (1H, m), 4.05-4.02 (1H, m), 3.40 (2H, s), 3.25 (3H, s), 2.67-2.65 (4H, m), 2.28 (4H, m), 1.85-1.82 (1H, m), 1.80-1.68 (1H, m), 1.30-1.27 (6H, m), 0.86-0.79 (3H, m) | 451.3 | 450.29 |

**[Table 5-211]**

| Compound No. | NMR | LC/MS (M+H)⁺ | Exact Mass |
|---|---|---|---|
| 1067 | 1H-NMR (400 MHz, DMSO-d6, ppm) δ: 10.07 (1H, s), 9.24 (1H, s), 8.25 (1H, d, J = 8.4 Hz), 8.18 (1H, d, J = 2.0 Hz), 7.75-7.72(1H, m), 6.87 (1H, s), 6.44 (1H, d, J = 7.6 Hz), 4.29-4.21 (1H, m), 4.05-4.02 (1H, m), 3.43 (2H, s), 3.40 (3H, s), 2.69-2.66 (4H, m), 2.29 (4H, m), 1.85-1.82 (1H, m), 1.80-1.68 (1H, m), 1.30-1.27 (6H, m), 0.86-0.79 (3H, m) | 451.3 | 450.29 |
| 1068 | 1H-NMR (400 MHz, DMSO-d6, ppm) δ: 10.24 (1H, s), 9.20 (1H, s), 8.16 (1H, d, J = 10.0 Hz), 7.36 (1H, d, J = 10 Hz), 6.84 (1H, s), 6.34 (1H, d, J = 7.6 Hz), 4.26-4.18 (1H, m), 4.04-4.01 (1H, m), 3.43-3.41 (4H, m), 3.24 (3H, s), 2.82-2.80 (4H, m), 1.84-1.76 (1H, m), 1.74-1.67 (1H, m), 1.28-1.26 (6H, m), 0.86-0.79 (3H, m) | 438.3 | 437.27 |
| 1069 | 1H-NMR (400 MHz, DMSO-d6, ppm) δ: 10.24 (1H, s), 9.20 (1H, s), 8.16 (1H, d, J = 10.0 Hz), 7.36 (1H, d, J = 9.6 Hz), 6.84 (1H, s), 6.34 (1H, d, J = 7.6 Hz), 4.26-4.18 (1H, m), 4.04-4.01 (1H, m), 3.43-3.41 (4H, m), 3.24 (3H, s), 2.82-2.80 (4H, m), 1.84-1.76 (1H, m), 1.74-1.67 (1H, m), 1.28-1.26 (6H, m), 0.86-0.79 (3H, m) | 438.3 | 437.27 |
| 1070 | 1H-NMR (400 MHz, DMSO-d6, ppm) δ: 10.06 (1H, s), 9.27 (1H, s), 8.28-8.22 (2H, m), 7.76 (1H, bar), 7.02 (1H, s), 6.57 (1H, s), 5.20 (1H, s), 4.65 (1H, bar), 4.41-4.37 (2H, m), 3.46 (4H, s), 2.34-2.29 (10H, m), 2.09 (2H, m), 1.77-1.67 (6H, m), 1.25 (3H, m) | 493.3 | 492.30 |
| 1071 | 1H-NMR (400 MHz, DMSO-d6, ppm) δ: 10.05 (1H, s), 9.16 (1H, s), 8.14 (2H, m), 8.73-8.59 (1H, m), 6.92 (1H, s), 6.37 (1H, s), 5.11 (1H, d, J = 3.6 Hz), 4.61-4.53 (1H, m), 4.26 (1H, m), 3.41-3.47 (4H, m), 2.31-3.29 (9H, m), 1.47 (9H, s), 1.34 (3H, d, J = 6.0 Hz) | 481.2 | 480.30 |

**[Table 5-212]**

| Compound No. | NMR | LC/MS (M+H)⁺ | Exact Mass |
|---|---|---|---|
| 1072 | 1H-NMR (400 MHz, DMSO-d6, ppm) δ: 9.96 (1H, s), 9.24 (1H, s), 8.41 (1H, d, J = 8.4 Hz), 8.18 (1H, s), 7.76 (1H, d, J = 8.4 Hz), 6.98 (1H, s), 6.92-6.89 (1H, m), 5.18 (1H, d, J = 4.8 Hz), 4.65-4.61 (1H, m),4.44-4.42 (1H, m), 3.61-3.54 (2H, m), 3.50-3.40 (4H, m), 2.38-2.30 (10H, m), 1.58-1.52 (2H, m), 1.40-1.39 (3H, m), 1.27-1.23 (3H, m) | 467.1 | 466.28 |
| 1073 | 1H-NMR (400 MHz, DMSO-d6, ppm) δ: 9.95 (1H, s), 9.23 (1H, s), 8.41 (1H, d, J = 8.4 Hz), 8.19 (1H, d, J = 9.2 Hz), 7.76-7.74 (1H, m), 6.98 (1H, s), 6.91-6.89 (1H, s), 5.17 (1H, d, J = 4.4 Hz), 4.64-4.61 (1H, m), 4.02 (1H, s), 3.59-3.56 (2H, m), 3.44 (2H, s), 2.50 (4H, m), 2.39-2.33 (4H, m), 2.17 (2H, s), 1.39 (3H, d, J = 4.8 Hz), 1.27-1.23 (3H, m), 1.07 (6H, s) | 525.0 | 480.30 |
| 1074 | 1H-NMR (400 MHz, DMSO-d6, ppm) δ: 9.98 (1H, s), 9.25 (1H, s), 8.42 (1H, d, J = 8.4 Hz), 8.19 (1H, s), 7.73 (1H, d, J = 8.4Hz), 7.01 (2H, s), 5.18 (1H, d, J = 4.4 Hz), 4.68-4.62 (3H, m), 4.35-4.33 (1H, m), 3.70-3.68 (2H, m), 3.67-3.63 (4H, m), 2.39-2.33 (10H, m), 2.12-2.09 (2H, m), 1.40 (3H, d, J = 6.4 Hz) | 485.2 | 484.27 |
| 1075 | 1H-NMR (400 MHz, DMSO-d6, ppm) δ: 9.97 (1H, s), 9.24 (1H, s), 8.38 (1H, d, J = 8.8Hz), 8.19 (1H, s), 7.74 (1H, d, J=8.8Hz), 6.98 (1H, s), 6.89-6.86 (1H, br), 5.17 (1H, d, J = 4.4Hz), 4.63-4.60 (1, m), 4.21 (1H, d, 3.2Hz), 3.73-3.71 (1H, br), 3.54-3.52 (2H, m), 3.44 (2H, s), 2.40-2.32 (8H, m), 2.25-2.20 (1H, m), 2.16-2.11 (1H, m), 1.71-1.66 (2H, m), 1.39 (3H, d, J=6.0Hz), 1.03 (3H, d, J=6.0Hz), 0.97-0.95 (3H, m) | 481.3 | 480.30 |

**[Table 5-213]**

| Compound No. | NMR | LC/MS (M+H)⁺ | Exact Mass |
|---|---|---|---|
| 1076 | 1H-NMR (400 MHz, DMSO-d6, ppm) δ: 9.98 (1H, s), 9.22 (1H, s), 8.38 (1H, d, J = 8.8Hz), 8.17 (1H, d, J = 2Hz), 7.74-7.71 (1H, m), 6.96 (1H, m), 6.90-6.88 (1H, m), 5.18 (1H, d, J = 4.4Hz), 4.61-4.59 (1H, m), 4.34-4.32 (1H, m), 3.92 (1H, d, J = 13.6Hz), 3.49-3.42 (4H, m), 3.12 (1H, d, J = 13.6Hz), 2.65 (1H, br), 2.57-2.54 (2H, m), 2.32-2.29 (3H, m), 2.15-2.05 (2H, m), 1.95-1.85 (1H, m), 1.68-1.66 (2H, m), 1.37 (3H, d, J = 6.4Hz), 1.08 (3H, d, J = 6.4Hz), 0.96-0.92 (3H, m) | 481.2 | 480.30 |
| 1077 | 1H-NMR (400 MHz, DMSO-d6, ppm) δ: 9.96 (1H, s), 9.23 (1H, s), 8.39 (1H, d, J = 8.8Hz), 8.19 (1H, s), 7.78-7.74 (1H, m), 6.98 (1H, s), 6.86 (1H, br), 5.17 (1H, d, J = 4.4Hz), 4.60 (1H, br), 4.31 (1H, br), 3.92 (1H, d, J = 13.6Hz), 3.51-3.49 (4H, m), 3.16 (1H, d, J = 13.6Hz), 2.65 (2H, br), 2.40 (4H, br), 2.18-1.92 (3H, m), 1.69-1.64 (2H, m), 1.39 (3H, d, J = 6.4Hz), 1.11 (3H, d, J = 4.8Hz), 0.98-0.94 (3H, m) | 481.2 | 480.30 |
| 1078 | 1H-NMR (400 MHz, DMSO-d6, ppm) δ: 10.22 (1H, s), 9.27 (1H, s), 8.32 (1H, d, J = 9.2Hz), 8.29 (1H, d, J = 2.4Hz), 7.82-7.79 (1H, m), 7.01 (1H, s), 6.55 (1H, d, J = 7.6Hz), 5.22 (1H, d, J = 4.8Hz), 4.64-4.61 (1H, m), 4.41-4.38 (1H, m), 3.66-3.63 (2H, m), 3.41 (2H, s), 3.05-3.02 (2H, m), 2.79 (1H, br), 2.06 (2H, br), 1.74 (2H, br), 1.66-1.56 (4H, m), 1.39 (3H, d, J = 6.4Hz) | 449.0 | 448.23 |

**[Table 5-214]**

| Compound No. | NMR | LC/MS (M+H)⁺ | Exact Mass |
|---|---|---|---|
| 1079 | 1H-NMR (400 MHz, DMSO-d6, ppm) δ: 10.14 (1H, s), 9.25 (1H, s), 8.45 (1H, d, J = 8.8Hz), 8.27 (1H, d, J = 2.4Hz), 7.78-7.75 (1H, m), 7.01-6.99 (2H, m), 5.19 (1H, d, J = 4.4Hz), 4.65-4.60 (2H, m), 4.55-4.52 (1H, m), 3.66-3.62 (4H, m), 3.40 (2H, s), 3.04-3.01 (2H, m), 2.78 (1H, br), 1.38 (3H, d, J = 6.4Hz) | 441.1 | 440.21 |
| 1080 | 1H-NMR (DMSO-D6) δ: 10.03 (1H, s), 9.23 (1H, s), 8.42 (1H, d, J = 8.2 Hz), 8.19 (1H, s), 7.75 (1H, d, J = 8.2 Hz), 6.97 (1H, s), 6.91 (1H, t, J = 5.7 Hz), 5.19 (1H, d, J = 4.6 Hz), 4.65-4.57 (2H, m), 3.62-3.38 (5H, m), 2.84-2.78 (1H, m), 2.69-2.64 (1H, m), 1.89-1.56 (4H, m), 1.45-1.36 (4H, m), 1.24 (3H, t, J = 7.1 Hz), 1.12-1.00 (1H, m). | 424.2 | 423.24 |
| 1081 | 1H-NMR (DMSO-D6) δ: 10.03 (1H, s), 9.23 (1H, s), 8.39 (1H, d, J = 8.7 Hz), 8.18 (1H, s), 7.74 (1H, dd, J = 8.7, 2.2 Hz), 6.97 (1H, d, J = 0.9 Hz), 6.88 (1H, t, J = 5.9 Hz), 5.19 (1H, d, J = 4.6 Hz), 4.63-4.57 (2H, m), 3.56-3.37 (5H, m), 2.80 (1H, d, J = 6.9 Hz), 2.66 (1H, d, J = 6.9 Hz), 1.90-1.57 (6H, m), 1.44-1.35 (4H, m), 1.11-1.00 (1H, m), 0.95 (3H, t, J = 7.3 Hz). | 438.2 | 437.25 |
| 1082 | 1H-NMR (DMSO-D6) δ: 10.03 (1H, s), 9.23 (1H, s), 8.38 (1H, d, J = 8.7 Hz), 8.18 (1H, s), 7.73 (1H, d, J = 6.9 Hz), 6.96 (1H, d, J = 0.9 Hz), 6.86 (1H, t, J = 5.7 Hz), 5.19 (1H, d, J = 4.6 Hz), 4.64-4.56 (2H, m), 3.61-3.37 (5H, m), 2.80 (1H, d, J = 7.8 Hz), 2.66 (1H, d, J = 7.8 Hz), 1.89-1.57 (6H, m), 1.46-1.34 (6H, m), 1.09-1.00 (1H, m), 0.94 (3H, t, J = 7.3 Hz). | 452.3 | 451.27 |

**[Table 5-215]**

| Compound No. | NMR | LC/MS (M+H)⁺ | Exact Mass |
|---|---|---|---|
| 1083 | 1H-NMR (DMSO-D6) δ: 10.07 (1H, s), 9.24 (1H, s), 8.37 (1H, d, J = 8.7 Hz), 8.20 (1H, s), 7.75 (1H, d, J = 7.8 Hz), 6.98 (1H, s), 6.91 (1H, t, J = 5.7 Hz), 5.19 (1H, d, J = 4.6 Hz), 4.64-4.57 (2H, m), 3.54-3.34 (5H, m), 2.80 (1H, br s), 2.66 (1H, br s), 1.90-1.57 (4H, m), 1.46-1.35 (4H, m), 1.26-1.19 (1H, m), 1.10-1.00 (1H, m), 0.48-0.44 (2H, m), 0.35-0.31 (2H, m). | 450.2 | 449.25 |
| 1084 | 1H-NMR (DMSO-D6) δ: 10.04 (1H, s), 9.25 (1H, s), 8.35 (1H, d, J = 8.7 Hz), 8.20 (1H, s), 7.76 (1H, d, J = 6.9 Hz), 7.00 (1H, s), 6.84 (1H, d, J = 7.3 Hz), 5.20 (1H, d, J = 4.6 Hz), 4.65-4.55 (3H, m), 3.53-3.38 (3H, m), 2.80 (1H, d, J = 8.2 Hz), 2.66 (1H, d, J = 8.2 Hz), 2.39-2.30 (2H, m), 2.11 (2H, td, J = 20.0, 10.7 Hz), 1.93-1.56 (6H, m), 1.48-1.34 (4H, m), 1.12-1.00 (1H, m). | 450.2 | 449.25 |
| 1085 | 1H-NMR (DMSO-D6) δ: 10.07 (1H, s), 9.24 (1H, s), 8.35 (1H, d, J = 8.7 Hz), 8.19 (1H, d, J = 1.4 Hz), 7.71 (1H, dd, J = 8.5, 2.1 Hz), 6.97 (1H, s), 6.84 (1H, t, J = 5.9 Hz), 5.18 (1H, d, J = 4.6 Hz), 4.63-4.55 (2H, m), 3.51-3.33 (5H, m), 2.80 (1H, d, J = 7.3 Hz), 2.65 (1H, d, J = 10.5 Hz), 2.07-1.97 (1H, m), 1.90-1.57 (4H, m), 1.45-1.34 (4H, m), 1.09-1.00 (1H, m), 0.95 (6H, d, J = 6.9 Hz). | 452.3 | 451.27 |
| 1086 | 1H-NMR (DMSO-D6) δ: 10.08 (1H, s), 9.24 (1H, s), 8.25 (1H, d, J = 8.7 Hz), 8.19 (1H, d, J = 1.8 Hz), 7.73 (1H, dd, J = 8.5, 2.1 Hz), 6.98 (1H, s), 6.40 (1H, d, J = 7.8 Hz), 5.20 (1H, d, J = 4.6 Hz), 4.64-4.56 (3H, m), 3.96-3.87 (1H, m), 3.55-3.37 (4H, m), 2.79 (1H, d, J = 7.3 Hz), 2.65 (1H, d, J = 11.4 Hz), 2.13-2.00 (2H, m), 1.93-1.57 (6H, m), 1.49-1.28 (8H, m), 1.12-1.00 (1H, m). | 494.3 | 493.28 |

**[Table 5-216]**

| Compound No. | NMR | LC/MS (M+H)⁺ | Exact Mass |
|---|---|---|---|
| 1087 | 1H-NMR (DMSO-D6) δ: 10.04 (1H, s), 9.24 (1H, s), 8.36 (1H, d, J = 8.7 Hz), 8.19 (1H, s), 7.72 (1H, d, J = 8.2 Hz), 6.97 (1H, s), 6.90 (1H, t, J = 6.2 Hz), 5.19 (1H, d, J = 4.6 Hz), 4.64-4.56 (2H, m), 3.85 (2H, dd, J = 11.4, 2.7 Hz), 3.55-3.38 (5H, m), 3.26 (2H, t, J = 11.4 Hz), 2.80 (1H, d, J = 7.8 Hz), 2.65 (1H, br s), 2.01-1.57 (7H, m), 1.46-1.24 (6H, m), 1.11-0.99 (1H, m). | 494.2 | 493.28 |
| 1088 | 1H-NMR (CDC13) δ: 9.04 (1H, s), 8.47 (1H, s), 8.33 (1H, d, J = 8.7 Hz), 8.27 (1H, s), 7.74 (1H, dd, J = 8.2, 2.3 Hz), 6.70 (1H, s), 6.35 (1H, t, J = 5.7 Hz), 4.80 (1H, q, J = 6.4 Hz), 3.73-3.64 (4H, m), 3.51 (2H, s), 3.46-3.40 (1H, m), 3.31 (1H, t, J = 10.3 Hz), 2.88-2.80 (1H, m), 2.75-2.68 (2H, m), 2.58-2.46 (6H, m), 1.52 (3H, d, J = 6.4 Hz), 1.37 (3H, t, J = 7.1 Hz), 1.23 (3H, t, J = 7.1 Hz). | 467.4 | 466.28 |
| 1089 | 1H-NMR (CDC13) δ: 9.02 (1H, d, J = 3.2 Hz), 8.32 (1H, d, J = 8.7 Hz), 8.25 (1H, d, J = 1.8 Hz), 8.21 (1H, s), 7.74 (1H, dd, J = 8.2, 2.3 Hz), 6.70 (1H, d, J = 0.9 Hz), 6.36 (1H, t, J = 5.5 Hz), 4.80 (1H, q, J = 6.4 Hz), 3.74-3.64 (4H, m), 3.51 (2H, s), 3.33 (1H, t, J = 10.7 Hz), 2.87 (1H, br s), 2.73 (2H, br s), 2.53 (5H, br s), 1.53 (3H, d, J = 6.4 Hz), 1.37 (3H, t, J = 7.1 Hz), 1.23 (3H, t, J = 6.9 Hz). | 467.3 | 466.28 |

**[Table 5-217]**

| Compound No. | NMR | LC/MS (M+H)⁺ | Exact Mass |
|---|---|---|---|
| 1090 | 1H-NMR (CDCl3) δ: 8.98 (1H, s), 8.26 (1H, d, J = 9.1 Hz), 8.09 (1H, s), 8.05 (1H, d, J = 2.7 Hz), 7.38 (1H, dd, J = 8.9, 3.0 Hz), 6.67 (1H, d, J = 0.9 Hz), 6.23 (1H, d, J = 7.3 Hz), 4.82-4.75 (1H, m), 4.43-4.34 (1H, m), 4.23 (2H, d, J = 3.7 Hz), 4.09 (1H, d, J = 4.6 Hz), 3.87 (2H, t, J = 5.0 Hz), 3.60 (1H, t, J = 4.3 Hz), 3.47 (2H, t, J = 5.0 Hz), 3.19-3.15 (4H, m), 1.52 (3H, d, J = 6.4 Hz), 1.36 (6H, dd, J = 6.4, 0.9 Hz). | 467.3 | 466.24 |
| 1091 | 1H-NMR (CDCl3) δ: 8.96 (1H, s), 8.09 (1H, d, J = 8.7 Hz), 7.90 (1H, s), 7.44 (1H, d, J = 8.7 Hz), 6.67 (1H, s), 6.36 (1H, d, J = 5.9 Hz), 4.79 (1H, q, J = 6.3 Hz), 3.70-3.63 (2H, m), 3.05 (4H, t, J = 4.8 Hz), 2.88 (4H, t, J = 4.6 Hz), 2.49 (3H, s), 1.52 (4H, d, J = 6.4 Hz), 1.37 (4H, t, J = 7.3 Hz). | 409.3 | 408.24 |
| 1092 | 1H-NMR (CDCl3) δ: 8.97 (1H, s), 8.09 (1H, d, J = 8.7 Hz), 7.94 (1H, s), 7.42 (1H, d, J = 8.7 Hz), 6.66 (1H, s), 6.44 (1H, t, J = 5.5 Hz), 4.79 (1H, q, J = 6.4 Hz), 3.61-3.58 (2H, m), 3.04 (4H, t, J = 4.8 Hz), 2.88 (4H, t, J = 4.6 Hz), 2.49 (3H, s), 1.81-1.73 (2H, m), 1.51 (3H, d, J = 6.4 Hz), 1.06 (3H, t, J = 7.3 Hz). | 423.3 | 422.25 |
| 1093 | | 437.3 | 436.27 |
| 1094 | | 435.3 | 434.25 |
| 1095 | | 435.3 | 434.25 |
| 1096 | 1H-NMR (DMSO-D6) δ: 10.23 (1H, s), 9.29 (1H, s), 8.37 (1H, d, J = 8.7 Hz), 8.17 (1H, d, J = 2.3 Hz), 7.74 (1H, dd, J = 8.7, 2.3 Hz), 7.18-7.14 (2H, m), 6.77 (1H, t, J = 55.6 Hz), 3.58-3.52 (2H, m), 3.42 (2H, s), 2.77-2.70 (2H, m), 2.19-2.13 (1H, m), 1.97 (2H, t, J = 10.5 Hz), 1.78-1.72 (2H, m), 1.55-1.46 (2H, m), 1.22 (3H, t, J = 6.9 Hz). | 458.3 | 457.20 |
| 1097 | | 472.3 | 471.22 |
| 1098 | | 486.3 | 485.24 |

**[Table 5-218]**

| Compound No. | NMR | LC/MS (M+H)⁺ | Exact Mass |
|---|---|---|---|
| 1099 | | 484.3 | 483.22 |
| 1100 | | 484.3 | 483.22 |
| 1101 | 1H-NMR (CDCl3) δ: 8.99 (1H, s), 8.45 (1H, br s), 8.30 (1H, d, J = 10.1 Hz), 7.01 (1H, d, J = 9.6 Hz), 6.86 (1H, d, J = 0.9 Hz), 6.55 (1H, s), 4.82 (1H, q, J = 6.4 Hz), 3.77 (2H, s), 3.61 (4H, t, J = 4.8 Hz), 2.57 (4H, t, J = 5.0 Hz), 2.36 (3H, s), 1.54 (3H, d, J = 6.4 Hz), 1.49 (6H, s) . | 454.3 | 453.26 |
| 1102 | 1H-NMR (CDCl3) δ: 9.02 (1H, s), 8.43 (1H, s), 8.35 (1H, d, J = 9.6 Hz), 7.06 (1H, d, J = 10.1 Hz), 6.68 (1H, d, J = 0.9 Hz), 6.37 (1H, t, J = 5.7 Hz), 4.78 (1H, q, J = 6.4 Hz), 3.63 (4H, t, J = 5.0 Hz), 3.60-3.54 (2H, m), 2.57 (4H, t, J = 5.3 Hz), 2.36 (3H, s), 1.75 (2H, td, J = 14.4, 7.3 Hz), 1.51 (3H, d, J = 6.4 Hz), 1.03 (3H, q, J = 7.6 Hz). | 424.3 | 423.25 |
| 1103 | 1H-NMR (CDCl3) δ: 9.23-9.16 (1H, br m), 9.09 (1H, s), 8.32 (2H, d, J = 8.7 Hz), 7.74 (1H, dd, J = 8.7, 2.3 Hz), 6.69 (1H, s), 6.33 (1H, t, J = 5.7 Hz), 4.79 (1H, q, J = 6.4 Hz), 3.69-3.62 (2H, m), 3.49 (2H, s), 2.48 (8H, br s), 2.28 (3H, s), 1.52 (3H, d, J = 6.4 Hz), 1.36 (3H, t, J = 7.3 Hz). | 423.3 | 422.25 |
| 1104 | 1H-NMR (CDCl3) δ: 9.08 (1H, s), 9.01 (1H, s), 8.34-8.31 (2H, m), 7.73 (1H, dd, J = 8.7, 1.8 Hz), 6.69 (1H, s), 6.42 (1H, t, J = 5.7 Hz), 4.79 (1H, q, J = 6.3 Hz), 3.59 (2H, dd, J = 13.5, 6.6 Hz), 3.50 (2H, s), 2.48 (8H, br s), 2.28 (3H, s), 1.76 (2H, td, J = 14.5, 7.3 Hz), 1.52 (3H, d, J = 6.4 Hz), 1.06 (3H, t, J = 7.3 Hz). | 437.3 | 436.27 |

**[Table 5-219]**

| Compound No. | NMR | LC/MS (M+H)⁺ | Exact Mass |
|---|---|---|---|
| 1105 | 1H-NMR (CDCl3) δ: 9.06 (1H, s), 8.76 (1H, s), 8.36 (1H, d, J = 8.7 Hz), 8.30 (1H, d, J = 1.8 Hz), 7.71 (1H, dd, J = 8.7, 2.3 Hz), 6.71 (1H, d, J = 0.9 Hz), 6.65 (1H, d, J = 7.8 Hz), 4.80 (1H, q, J = 6.4 Hz), 4.50-4.44 (1H, m), 3.59 (2H, d, J = 4.1 Hz), 3.50 (2H, s), 3.46 (3H, s), 2.50 (8H, br s), 2.29 (3H, s), 1.51 (3H, d, J = 6.9 Hz), 1.38 (3H, d, J = 6.4 Hz). | 467.4 | 466.28 |
| 1106 | 1H-NMR (CDCl3) δ: 9.02 (1H, s), 8.49 (1H, s), 8.28-8.25 (2H, m), 7.70 (1H, dd, J = 8.7, 2.3 Hz), 6.87 (1H, s), 6.63 (1H, s), 4.83 (1H, q, J = 6.4 Hz), 3.77 (2H, s), 3.49 (2H, s), 2.51 (8H, br s), 2.29 (3H, s), 1.55 (3H, d, J = 6.4 Hz), 1.50 (6H, s). | 467.5 | 466.28 |
| 1107 | 1H-NMR (CDCl3) δ: 9.02 (1H, s), 8.38-8.33 (2H, m), 8.28 (1H, d, J = 1.8 Hz), 7.77 (1H, dd, J = 8.7, 2.3 Hz), 6.64 (1H, s), 6.30 (1H, t, J = 5.7 Hz), 5.17 (2H, d, J = 5.5 Hz), 4.58 (2H, d, J = 5.0 Hz), 3.74-3.65 (2H, m), 3.49 (2H, s), 2.94-2.87 (4H, m), 2.52-2.38 (4H, br m), 1.81 (3H, s), 1. 37 (3H, t, J = 7.1 Hz). | 435.3 | 434.25 |
| 1108 | 1H-NMR (CDCl3) δ: 9.01 (1H, s), 8.37 (1H, d, J = 8.2 Hz), 8.33 (1H, s), 8.28 (1H, d, J = 1.8 Hz), 7.78 (1H, dd, J = 8.2, 2.3 Hz), 6.65 (1H, s), 6.41 (1H, d, J = 7.3 Hz), 5.16 (2H, d, J = 5.5 Hz), 4.78-4.66 (1H, m), 4.57 (2H, d, J = 5.5 Hz), 3.50 (2H, s), 2.94-2.87 (4H, m), 2.59-2.36 (6H, m), 2.11-1.98 (2H, m), 1.92-1.78 (5H, m) . | 461.3 | 460.27 |

**[Table 5-220]**

| Compound No. | NMR | LC/MS (M+H)⁺ | Exact Mass |
|---|---|---|---|
| 1109 | 1H-NMR (CDCl3) δ: 9.02 (1H, s), 8.38 (1H, d, J = 8.7 Hz), 8.34 (1H, s), 8.28 (1H, d, J = 1.8 Hz), 7.75 (1H, dd, J = 8.7, 2.3 Hz), 6.64 (1H, s), 6.46 (1H, t, J = 5.5 Hz), 5.16 (2H, d, J = 5.5 Hz), 4.58 (2H, d, J = 5.5 Hz), 3.55-3.47 (4H, m), 2.93-2.87 (4H, m), 2.52-2.38 (4H, br m), 1.80 (3H, s), 1.29-1.17 (1H, m), 0.64-0.57 (2H, m), 0.41-0.34 (2H, m). | 461.4 | 460.27 |
| 1110 | 1H-NMR (CDCl3) δ: 9.04 (1H, s), 8.33-8.27 (3H, m), 7.76 (1H, dd, J = 8.7, 2.3 Hz), 6.69 (1H, s), 6.44 (1H, t, J = 5.9 Hz), 5.14 (2H, d, J = 5.5 Hz), 4.59 (2H, d, J = 5.5 Hz), 3.82 (2H, t, J = 6.4 Hz), 3.50 (2H, s), 2.93-2.87 (4H, m), 2.82-2.37 (9H, m), 1.80 (3H, s). | 511.4 | 510.27 |
| 1111 | 1H-NMR (CDCl3) δ: 9.05-9.03 (1.0H, m), 8.27 (1.0H, d, J = 8.2 Hz), 8.15-8.08 (1.0H, m), 7.56-7.46 (1.0H, m), 6.85 (1.0H, s), 6.32 (1.0H, t, J = 5.5 Hz), 4.80 (1.3H, s), 4.44 (0.7H, s), 4.37-4.27 (3.0H, m), 4.02 (0.7H, t, J = 5.9 Hz), 3.70-3.55 (4.3H, m), 3.41 (3.0H, s), 3.02-2.95 (2.0H, m), 1.81-1.71 (2.0H, m), 1.50 (3.0H, d, J = 6.4 Hz), 1.07 (3.0H, t, J = 7.3 Hz) . | 452.3 | 451.23 |
| 1112 | 1H-NMR (CDCl3) δ: 9.05-9.03 (1.0H, m), 8.26 (1.0H, d, J = 8.2 Hz), 8.16-8.10 (1.0H, m), 7.55 (0.6H, d, J = 8.7 Hz), 7.49 (0.4H, d, J = 8.7 Hz), 6.86 (1.0H, s), 6.23 (1.0H, t, J = 5.3 Hz), 4.80 (1.3H, s), 4.44 (0.7H, s), 4.38-4.27 (3.0H, m), 4.02 (0.7H, t, J = 6.2 Hz), 3.72-3.60 (4.3H, m), 3.41 (3.0H, s), 3.02-2.95 (2.0H, m), 1.51 (3.0H, d, J = 6.9 Hz), 1.36 (3.0H, t, J = 7.3 Hz). | 438.3 | 437.22 |

**[Table 5-221]**

| Compound No. | NMR | LC/MS (M+H)⁺ | Exact Mass |
|---|---|---|---|
| 1113 | 1H-NMR (CDCl3) δ: 9.07-9.03 (1.0H, m), 8.33-8.27 (1.0H, m), 8.17-8.08 (1.0H, m), 7.56-7.46 (1.0H, m), 6.88 (1.0H, s), 6.57 (1.0H, t, J = 8.9 Hz), 4.86-4.74 (1.3H, m), 4.44 (0.7H, s), 4.40-4.25 (4.0H, m), 4.07-3.98 (1.7H, m), 3.81-3.72 (2.0H, m), 3.69-3.56 (3.3H, m), 3.40 (3.0H, s), 3.03-2.94 (2.0H, m), 2.11-1.99 (1.0H, m), 1.96-1.80 (2.0H, m), 1.78-1.65 (1.0H, m), 1.48 (3.0H, d, J = 6.4 Hz). | 494.3 | 493.24 |
| 1114 | 1H-NMR (CDCl3) δ: 9.00-8.97 (1.0H, m), 8.32-8.24 (1.0H, m), 8.13-8.04 (1.0H, m), 7.55-7.46 (1.0H, m), 6.69-6.67 (1.0H, m), 6.56 (1.0H, q, J = 9.6 Hz), 4.85-4.74 (1.3H, m), 4.44 (0.7H, s), 4.37-4.25 (3.0H, m), 4.17 (1.0H, t, J = 8.0 Hz), 4.11-3.94 (3.7H, m), 3.90 (1.0H, t, J = 8.0 Hz), 3.82-3.71 (2.0H, m), 3.70-3.58 (3.3H, m), 3.53-3.43 (1.0H, m), 3.02-2.95 (2.0H, m), 2.34-2.21 (2.0H, m), 2.11-1.98 (1.0H, m), 1.96-1.80 (2.0H, m), 1.76-1.64 (1.0H, m). | 506.3 | 505.24 |
| 1115 | 1H-NMR (CDCl3) δ: 9.03-8.99 (1.0H, m), 8.33-8.25 (1.0H, m), 8.18-8.05 (1.0H, m), 7.57-7.46 (1.0H, m), 6.70-6.54 (2.0H, m), 5.16-5.09 (2.0H, m), 4.86-4.74 (1.3H, m), 4.62-4.55 (2.0H, m), 4.44 (0.7H, s), 4.41-4.25 (3.0H, m), 4.07-3.97 (1.7H, m), 3.82-3.58 (5.3H, m), 3.03-2.95 (2.0H, m), 2.11-1.98 (1.0H, m), 1.96-1.83 (2.0H, m), 1.82-1.59 (4.OH, m). | 506.3 | 505.24 |
| 1116 | 1H-NMR (CDCl3) δ: 9.05-9.01 (1.0H, m), 8.28-8.21 (1.0H, m), 8.16-8.06 (1.0H, m), 7.57-7.46 (1.0H, m), 6.73-6.68 (1.0H, m), 6.43 (1.0H, t, J = 5.5 Hz), 4.86-4.76 (2.3H, m), 4.45 (0.7H, s), 4.34-4.26 (2.0H, m), 4.15-4.07 (1.0H, m), 4.02 (0.7H, t, J = 5.9 Hz), 3.69-3.57 (4.3H, m), 3.03-2.95 (2.0H, m), 1.83-1.72 (2.0H, m), 1.53 (3.0H, d, J = 6.4 Hz), 1.08 (3.0H, t, J = 7.5 Hz). | 438.3 | 437.22 |

**[Table 5-222]**

| Compound No. | NMR | LC/MS (M+H)⁺ | Exact Mass |
|---|---|---|---|
| 1117 | 1H-NMR (CDCl3) δ: 9.04-9.01 (1.0H, m), 8.27-8.20 (1.0H, m), 8.13-8.02 (1.0H, m), 7.58-7.47 (1.0H, m), 6.71 (1.0H, s), 6.35 (1.0H, t, J = 5.3 Hz), 4.86-4.76 (2.3H, m), 4.45 (0.7H, s), 4.34-4.26 (2.0H, m), 4.13-4.06 (1.0H, m), 4.02 (0.7H, t, J = 6.2 Hz), 3.73-3.59 (4.3H, m), 3.03-2.94 (2.OH, m), 1.54 (3.0H, d, J = 6.4 Hz), 1.38 (3.0H, t, J = 7.3 Hz). | 424.3 | 423.20 |
| 1118 | 1H-NMR (CDCl3) δ: 9.06-9.01 (1.0H, m), 8.32-8.23 (1.0H, m), 8.18-8.08 (1.0H, m), 7.56-7.45 (1.0H, m), 6.78-6.66 (2.0H, m), 4.87-4.72 (2.3H, m), 4.44 (0.7H, s), 4.39-4.24 (3.0H, m), 4.06-3.95 (1.7H, m), 3.87-3.71 (3.0H, m), 3.71-3.58 (3.3H, m), 3.03-2.94 (2.0H, m), 2.11-1.98 (1.0H, m), 1.97-1.83 (2.0H, m), 1.77-1.64 (1.0H, m), 1.53 (3.0H, d, J = 6.4 Hz). | 480.3 | 479.23 |
| 1119 | 1H-NMR (CDCl3) δ: 9.02 (1H, s), 8.16 (1H, d, J = 8.7 Hz), 8.07 (1H, s), 7.41 (1H, d, J = 8.2 Hz), 6.84 (1H, s), 6.33 (1H, t, J = 5.5 Hz), 4.33 (1H, q, J = 6.6 Hz), 3.78-3.69 (4H, m), 3.66-3.55 (2H, m), 3.41 (3H, s), 3.02-2.90 (4H, m), 2.78 (2H, t, J = 5.3 Hz), 1.81-1.70 (2H, m), 1.50 (3H, d, J = 6.4 Hz), 1.07 (3H, t, J = 7.5 Hz). | 438.3 | 437.25 |
| 1120 | 1H-NMR (CDCl3) δ: 9.03 (1H, s), 8.15 (1H, d, J = 8.7 Hz), 8.11 (1H, s), 7.42 (1H, d, J = 8.7 Hz), 6.84 (1H, s), 6.24 (1H, t, J = 5.5 Hz), 4.34 (1H, q, J = 6.4 Hz), 3.78-3.62 (6H, m), 3.41 (3H, s), 3.02-2.90 (4H, m), 2.78 (2H, t, J = 5.5 Hz), 1.51 (3H, d, J = 6.9 Hz), 1.35 (3H, t, J = 7.3 Hz). | 424.3 | 423.24 |

**[Table 5-223]**

| Compound No. | NMR | LC/MS (M+H)⁺ | Exact Mass |
|---|---|---|---|
| 1121 | 1H-NMR (CDCl3) δ: 9.02 (1H, s), 8.19 (1H, d, J = 8.7 Hz), 8.09 (1H, s), 7.41 (1H, d, J = 8.2 Hz), 6.86 (1H, s), 6.56 (1H, d, J = 7.8 Hz), 4.40-4.28 (2H, m), 4.03 (1H, dd, J = 11.0, 2.7 Hz), 3.81-3.69 (6H, m), 3.63-3.55 (1H, m), 3.40 (3H, s), 3.04-2.91 (4H, m), 2.78 (2H, t, J = 5.5 Hz), 2.12-2.00 (1H, m), 1.96-1.64 (3H, m), 1.48 (3H, d, J = 6.4 Hz). | 480.4 | 479.26 |
| 1122 | 1H-NMR (CDCl3) δ: 8.96 (1H, s), 8.18 (1H, d, J = 8.7 Hz), 8.09 (1H, s), 7.41 (1H, d, J = 8.2 Hz), 6.67 (1H, s), 6.61-6.49 (1H, m), 4.37-4.26 (1H, m), 4.17 (1H, t, J = 8.0 Hz), 4.11-3.87 (4H, m), 3.81-3.57 (7H, m), 3.53-3.43 (1H, m), 3.04-2.90 (4H, m), 2.78 (2H, t, J = 5.3 Hz), 2.34-2.21 (2H, m), 2.11-1.99 (1H, m), 1.96-1.78 (2H, m), 1.76-1.64 (1H, m). | 492.3 | 491.26 |
| 1123 | | 424.3 | 423.24 |
| 1124 | 1H-NMR (CDCl3) δ: 9.21 (1H, s), 8.07 (1H, d, J = 8.7 Hz), 7.30 (1H, d, J = 5.0 Hz), 6.73 (1H, s), 6.35-6.21 (1H, br m), 4.82 (1H, q, J = 6.6 Hz), 3.90-3.50 (6H, m), 3.22-2.68 (6H, m), 1.52 (3H, d, J = 6.4 Hz), 1.40 (3H, t, J = 7.3 Hz). | 410.3 | 409.22 |
| 1125 | | 466.3 | 465.25 |
| 1126 | 1H-NMR (DMSO-D6) δ: 10.15 (1H, s), 9.26 (1H, s), 8.26 (1H, d, J = 8.7 Hz), 8.20 (1H, s), 7.72 (1H, d, J = 8.2 Hz), 7.02 (1H, s), 6.62 (1H, d, J = 7.8 Hz), 5.21 (1H, d, J = 4.6 Hz), 4.64-4.53 (2H, m), 4.19-4.10 (1H, m), 3.93 (1H, dd, J = 10.7, 3.0 Hz), 3.72-3.65 (1H, m), 3.57-3.38 (5H, m), 2.68 (2H, br s), 2.10-1.95 (3H, m), 1.84-1.57 (5H, m), 1.44-1.33 (5H, m). | 480.2 | 479.26 |

**[Table 5-224]**

| Compound No. | NMR | LC/MS (M+H)⁺ | Exact Mass |
|---|---|---|---|
| 1127 | 1H-NMR (DMSO-D6) δ: 10.17 (1H, s), 9.23 (1H, s), 8.24-8.16 (2H, m), 7.72 (1H, d, J = 8.2 Hz), 6.98 (1H, s), 6.43 (1H, s), 5.20 (1H, d, J = 4.6 Hz), 4.65-4.56 (2H, m), 3.55-3.38 (3H, m), 2.79 (1H, d, J = 7.8 Hz), 2.66 (1H, br s), 1.93-1.50 (13H, m), 1.46-1.35 (4H, m), 1.11-0.99 (1H, m). | 452.3 | 451.27 |
| 1128 | 1H-NMR (DMSO-D6) δ: 10.16 (1H, s), 9.26 (1H, s), 8.27 (1H, d, J = 8.2 Hz), 8.20 (1H, s), 7.72 (1H, d, J = 8.2 Hz), 7.02 (1H, s), 6.62 (1H, d, J = 7.8 Hz), 5.22 (1H, d, J = 4.6 Hz), 4.64-4.57 (2H, m), 4.19-4.11 (1H, m), 3.93 (1H, dd, J = 10.7, 3.0 Hz), 3.73-3.66 (1H, m), 3.57-3.38 (5H, m), 2.80 (1H, d, J = 8.2 Hz), 2.66 (1H, d, J = 8.2 Hz), 2.05-1.55 (9H, m), 1.46-1.34 (4H, m), 1.11-1.00 (1H, m). | 480.2 | 479.26 |
| 1129 | 1H-NMR (CDCl3) δ: 8.99 (1H, s), 8.19 (1H, d, J = 8.7 Hz), 8.05 (1H, s), 7.42 (1H, d, J = 8.2 Hz), 6.64 (1H, s), 6.59 (1H, d, J = 7.8 Hz), 5.12 (2H, t, J = 5.0 Hz), 4.58 (2H, d, J = 5.5 Hz), 4.42-4.29 (1H, br m), 4.03 (1H, dd, J = 11.0, 2.7 Hz), 3.91-3.59 (7H, m), 3.16-2.68 (6H, m), 2.13-1.99 (1H, m), 1.98-1.62 (6H, m). | 492.3 | 491.26 |
| 1130 | 1H-NMR (CDCl3) δ: 9.00 (1H, s), 8.14 (1H, d, J = 8.7 Hz), 7.98 (1H, s), 7.40 (1H, d, J = 8.2 Hz), 6.83 (1H, s), 6.33 (1H, t, J = 5.5 Hz), 4.33 (1H, q, J = 6.4 Hz), 3.67 (2H, s), 3.64-3.56 (2H, m), 3.40 (3H, s), 3.02-2.84 (4H, m), 2.79-2.58 (4H, m), 2.37 (6H, s), 1.81-1.69 (2H, m), 1.50 (3H, d, J = 6.4 Hz), 1.06 (3H, t, J = 7.5 Hz). | 465.4 | 464.30 |

**[Table 5-225]**

| Compound No. | NMR | LC/MS (M+H)⁺ | Exact Mass |
|---|---|---|---|
| 1131 | 1H-NMR (CDCl3) δ: 9.00 (1H, s), 8.14 (1H, d, J = 8.2 Hz), 7.98 (1H, s), 7.41 (1H, d, J = 8.7 Hz), 6.83 (1H, s), 6.24 (1H, t, J = 5.3 Hz), 4.34 (1H, q, J = 6.4 Hz), 3.71-3.61 (4H, m), 3.40 (3H, s), 3.01-2.85 (4H, m), 2.81-2.60 (4H, m), 2.39 (6H, s), 1.50 (3H, d, J = 6.4 Hz), 1.35 (3H, t, J = 7.1 Hz). | 451.4 | 450.29 |
| 1132 | 1H-NMR (CDCl3) δ: 9.00 (1H, s), 8.16 (1H, d, J = 8.2 Hz), 8.11-7.96 (1H, br m), 7.40 (1H, d, J = 8.7 Hz), 6.85 (1H, s), 6.54 (1H, d, J = 6.9 Hz), 4.41-4.24 (2H, m), 4.07-3.99 (1H, m), 3.86-3.53 (5H, m), 3.40 (3H, s), 3.06-2.58 (8H, m), 2.40 (6H, s), 2.19-1.64 (4H, m), 1.48 (3H, d, J = 6.4 Hz). | 507.4 | 506.31 |
| 1133 | 1H-NMR (CDCl3) δ: 9.02 (1H, s), 8.22 (1H, d, J = 8.2 Hz), 8.05 (1H, s), 7.48 (1H, d, J = 7.8 Hz), 6.84 (1H, s), 6.33 (1H, t, J = 5.5 Hz), 4.63 (2H, s), 4.34 (1H, q, J = 6.6 Hz), 4.27 (2H, t, J = 5.9 Hz), 3.82 (2H, t, J = 5.9 Hz), 3.65-3.55 (2H, m), 3.41 (3H, s), 2.93 (2H, t, J = 5.5 Hz), 2.63 (2H, t, J = 5.9 Hz), 2.32 (6H, s), 1.81-1.71 (2H, m), 1.50 (3H, d, J = 6.4 Hz), 1.07 (3H, t, J = 7.3 Hz). | 509.4 | 508.29 |
| 1134 | 1H-NMR (CDCl3) δ: 9.02 (1H, s), 8.21 (1H, d, J = 8.7 Hz), 8.04 (1H, s), 7.49 (1H, d, J = 8.2 Hz), 6.85 (1H, s), 6.24 (1H, t, J = 5.5 Hz), 4.64 (2H, s), 4.34 (1H, q, J = 6.4 Hz), 4.27 (2H, t, J = 5.7 Hz), 3.82 (2H, t, J = 5.7 Hz), 3.71-3.63 (2H, m), 3.41 (3H, s), 2.93 (2H, t, J = 5.5 Hz), 2.64 (2H, t, J = 5.7 Hz), 2.32 (6H, s), 1.50 (3H, d, J = 6.4 Hz), 1.35 (3H, t, J = 7.1 Hz). | 495.4 | 494.28 |

**[Table 5-226]**

| Compound No. | NMR | LC/MS (M+H)⁺ | Exact Mass |
|---|---|---|---|
| 1135 | 1H-NMR (CDCl3) δ: 9.03 (1H, s), 8.25 (1H, d, J = 8.7 Hz), 8.06 (1H, s), 7.48 (1H, d, J = 8.2 Hz), 6.87 (1H, s), 6.55 (1H, d, J = 6.9 Hz), 4.63 (2H, s), 4.42-4.24 (4H, m), 4.03 (1H, dd, J = 11.0, 2.7 Hz), 3.91-3.73 (4H, m), 3.66-3.54 (1H, m), 3.40 (3H, s), 2.93 (2H, t, J = 5.3 Hz), 2.64 (2H, t, J = 5.7 Hz), 2.32 (6H, s), 2.13-2.01 (1H, m), 1.98-1.80 (2H, m), 1.79-1.58 (1H, m), 1.48 (3H, d, J = 6.9 Hz). | 551.4 | 550.30 |
| 1136 | 1H-NMR (CDCl3) δ: 8.97 (1H, s), 8.24 (1H, d, J = 8.2 Hz), 8.04 (1H, s), 7.48 (1H, d, J = 8.7 Hz), 6.67 (1H, s), 6.54 (1H, t, J = 9.6 Hz), 4.63 (2H, s), 4.37-4.24 (3H, m), 4.17 (1H, t, J = 8.0 Hz), 4.11-3.94 (3H, m), 3.90 (1H, t, J = 8.0 Hz), 3.86-3.72 (4H, m), 3.66-3.56 (1H, m), 3.53-3.43 (1H, m), 2.93 (2H, t, J = 5.3 Hz), 2.64 (2H, t, J = 5.7 Hz), 2.34-2.22 (8H, m), 2.11-1.98 (1H, m), 1.95-1.79 (2H, m), 1.78-1.60 (1H, m). | 563.4 | 562.30 |
| 1137 | 1H-NMR (CDCl3) δ: 9.00 (1H, s), 8.24 (1H, d, J = 8.7 Hz), 8.04 (1H, s), 7.48 (1H, d, J = 8.7 Hz), 6.65 (1H, s), 6.59 (1H, d, J = 7.8 Hz), 5.12 (2H, t, J = 4.8 Hz), 4.64 (2H, s), 4.58 (2H, d, J = 5.9 Hz), 4.39-4.31 (1H, m), 4.27 (2H, t, J = 5.7 Hz), 4.03 (1H, dd, J = 11.2, 3.0 Hz), 3.87-3.73 (4H, m), 3.68-3.59 (1H, m), 2.93 (2H, t, J = 5.5 Hz), 2.63 (2H, t, J = 5.7 Hz), 2.32 (6H, s), 2.11-2.00 (1H, m), 1.97-1.82 (2H, m), 1.81-1.62 (4H, m). | 563.4 | 562.30 |

**[Table 5-227]**

| Compound No. | NMR | LC/MS (M+H)⁺ | Exact Mass |
|---|---|---|---|
| 1138 | 1H-NMR (CDCl3) δ: 9.00 (1H, s), 8.12 (1H, d, J = 8.2 Hz), 8.01 (1H, s), 7.40 (1H, d, J = 8.2 Hz), 6.68 (1H, s), 6.44 (1H, t, J = 5.7 Hz), 4.80 (1H, q, J = 6.4 Hz), 3.68 (2H, s), 3.64-3.57 (2H, m), 3.01-2.86 (4H, m), 2.77-2.70 (2H, m), 2.65-2.58 (2H, m), 2.35 (6H, s), 1.83-1.72 (2H, m), 1.53 (3H, d, J = 6.4 Hz), 1.08 (3H, t, J = 7.3 Hz). | 451.4 | 450.29 |
| 1139 | 1H-NMR (CDCl3) δ: 9.01 (1H, s), 8.14-7.99 (2H, m), 7.40 (1H, d, J = 8.2 Hz), 6.69 (1H, s), 6.34 (1H, t, J = 5.5 Hz), 4.81 (1H, q, J = 6.4 Hz), 3.72-3.62 (4H, m), 3.02-2.81 (4H, m), 2.78-2.68 (2H, m), 2.64-2.55 (2H, m), 2.34 (6H, s), 1.53 (3H, d, J = 6.4 Hz), 1.37 (3H, t, J = 7.1 Hz). | 437.3 | 436.27 |
| 1140 | 1H-NMR (CDCl3) δ: 9.01 (1H, s), 8.15 (1H, d, J = 8.2 Hz), 8.06 (1H, s), 7.40 (1H, d, J = 8.2 Hz), 6.75-6.65 (2H, m), 4.79 (1H, q, J = 6.4 Hz), 4.39-4.27 (1H, m), 4.01 (1H, dd, J = 11.0, 2.7 Hz), 3.81-3.59 (5H, m), 3.05-2.84 (4H, m), 2.80-2.56 (4H, m), 2.35 (6H, s), 2.12-1.81 (3H, m), 1.78-1.64 (1H, m), 1.52 (3H, d, J = 6.4 Hz). | 493.4 | 492.30 |
| 1141 | 1H-NMR (CDCl3) δ: 9.01 (1H, s), 8.20 (1H, d, J = 8.7 Hz), 8.03 (1H, s), 7.49 (1H, d, J = 8.2 Hz), 6.69 (1H, s), 6.44 (1H, t, J = 5.5 Hz), 4.81 (1H, q, J = 6.4 Hz), 4.64 (2H, s), 4.29 (2H, t, J = 5.7 Hz), 3.82 (2H, t, J = 5.7 Hz), 3.65-3.57 (2H, m), 3.01-2.86 (2H, br m), 2.75-2.60 (2H, br m), 2.35 (6H, s), 1.84-1.72 (2H, m), 1.53 (3H, d, J = 6.4 Hz), 1.08 (3H, t, J = 7.3 Hz). | 495.4 | 494.28 |

**[Table 5-228]**

| Compound No. | NMR | LC/MS (M+H)⁺ | Exact Mass |
|---|---|---|---|
| 1142 | 1H-NMR (CDCl3) δ: 9.02 (1H, s), 8.19 (1H, d, J = 8.7 Hz), 8.08 (1H, s), 7.50 (1H, d, J = 7.3 Hz), 6.70 (1H, s), 6.35 (1H, t, J = 5.3 Hz), 4.81 (1H, q, J = 6.4 Hz), 4.64 (2H, s), 4.27 (2H, t, J = 5.9 Hz), 3.82 (2H, t, J = 5.7 Hz), 3.73-3.63 (2H, m), 2.93 (2H, t, J = 5.3 Hz), 2.63 (2H, t, J = 5.7 Hz), 2.32 (6H, s), 1.53 (3H, d, J = 6.4 Hz), 1.37 (3H, t, J = 7.3 Hz). | 481.3 | 480.26 |
| 1143 | 1H-NMR (CDCl3) δ: 9.03 (1H, s), 8.23 (1H, d, J = 8.2 Hz), 8.10 (1H, s), 7.48 (1H, d, J = 8.7 Hz), 6.75-6.66 (2H, m), 4.80 (1H, q, J = 6.4 Hz), 4.69-4.58 (2H, m), 4.38-4.30 (1H, m), 4.27 (2H, t, J = 5.9 Hz), 4.00 (1H, dd, J = 11.2, 3.0 Hz), 3.89-3.74 (4H, m), 3.68-3.60 (1H, m), 2.93 (2H, t, J = 5.5 Hz), 2.63 (2H, t, J = 5.9 Hz), 2.32 (6H, s), 2.11-1.99 (1H, m), 1.97-1.82 (2H, m), 1.79-1.61 (1H, m), 1.53 (3H, d, J = 6.4 Hz). | 537.4 | 536.29 |
| 1144 | 1H-NMR (CDCl3) δ: 9.06-9.01 (1.0H, m), 8.26-8.20 (1.0H, m), 8.13-8.04 (1.0H, m), 7.56-7.45 (1.0H, m), 6.86 (1.0H, s), 6.13 (1.0H, d, J = 7.3 Hz), 4.80 (1.3H, s), 4.44 (0.7H, s), 4.37-4.25 (3.0H, m), 4.15-3.98 (1.7H, m), 3.83-3.71 (1.0H, m), 3.70-3.58 (2.3H, m), 3.42 (3.0H, s), 3.03-2.93 (2.0H, m), 2.34-2.24 (2.0H, m), 2.13-2.02 (2.0H, m), 1.72-1.47 (5.0H, m), 1.46-1.31 (2.0H, m). | 508.3 | 507.26 |

**[Table 5-229]**

| Compound No. | NMR | LC/MS (M+H)⁺ | Exact Mass |
|---|---|---|---|
| 1145 | 1H-NMR (CDCl3) δ: 8.99-8.95 (1.0H, m), 8.26-8.18 (1.0H, m), 8.07-7.98 (1.0H, m), 7.55-7.45 (1.0H, m), 6.66 (1.0H, s), 6.13 (1.0H, d, J = 7.3 Hz), 4.80 (1.3H, s), 4.44 (0.7H, s), 4.33-4.25 (2.0H, m), 4.19 (1.0H, t, J = 8.0 Hz), 4.11-3.96 (3.7H, m), 3.90 (1.0H, t, J = 8.0 Hz), 3.82-3.70 (1.0H, m), 3.68-3.59 (2.3H, m), 3.54-3.45 (1.0H, m), 3.02-2.94 (2.0H, m), 2.33-2.24 (4.0H, m), 2.13-2.03 (2.0H, m), 1.62-1.33 (4.0H, m). | 520.4 | 519.26 |
| 1146 | 1H-NMR (CDCl3) δ: 9.02-8.97 (1.0H, m), 8.26-8.20 (1.0H, m), 8.08 (0.3H, s), 8.03 (0.6H, s), 7.54 (0.6H, d, J = 8.2 Hz), 7.49 (0.3H, d, J = 8.7 Hz), 6.65 (1.0H, s), 6.16 (1.0H, d, J = 7.3 Hz), 5.14 (2.0H, d, J = 5.5 Hz), 4.80 (1.3H, s), 4.59 (2.0H, d, J = 5.5 Hz), 4.44 (0.7H, s), 4.34-4.25 (2.0H, m), 4.14-3.98 (1.7H, m), 3.82-3.71 (1.0H, m), 3.69-3.59 (2.3H, m), 3.03-2.94 (2.0H, m), 2.36-2.25 (2.0H, m), 2.14-2.04 (2.0H, m), 1.79 (3.0H, s), 1.65-1.35 (4.0H, m). | 520.3 | 519.26 |
| 1147 | 1H-NMR (DMSO-D6) δ: 10.05 (1H, s), 9.24 (1H, s), 8.41 (1H, d, J = 8.2 Hz), 8.19 (1H, s), 7.75 (1H, dd, J = 8.7, 2.3 Hz), 6.97 (1H, s), 6.91 (1H, t, J = 5.7 Hz), 5.19 (1H, d, J = 4.1 Hz), 4.64-4.58 (1H, m), 4.41 (1H, t, J = 5.3 Hz), 3.60-3.52 (2H, m), 3.42 (2H, s), 3.22 (2H, t, J = 5.7 Hz), 2.82 (2H, d, J = 10.1 Hz), 1.90 (2H, t, J = 10.0 Hz), 1.62 (2H, d, J = 11.0 Hz), 1.39 (3H, d, J = 6.4 Hz), 1.36-1.27 (1H, m), 1.24 (3H, t, J = 7.1 Hz), 1.16-1.05 (2H, m). | 438.3 | 437.25 |

**[Table 5-230]**

| Compound No. | NMR | LC/MS (M+H)⁺ | Exact Mass |
|---|---|---|---|
| 1148 | 1H-NMR (DMSO-D6) δ: 10.03 (1H, s), 9.23 (1H, s), 8.37 (1H, d, J = 8.2 Hz), 8.18 (1H, s), 7.73 (1H, dd, J = 8.5, 2.1 Hz), 6.97 (1H, s), 6.85 (1H, t, J = 5.7 Hz), 5.18 (1H, d, J = 4.6 Hz), 4.64-4.57 (1H, m), 4.40 (1H, t, J = 5.3 Hz), 3.58-3.49 (2H, m), 3.42 (2H, s), 3.22 (2H, t, J = 5.7 Hz), 2.81 (2H, d, J = 11.0 Hz), 1.89 (2H, t, J = 10.7 Hz), 1.68-1.58 (4H, m), 1.44-1.25 (6H, m), 1.16-1.05 (2H, m), 0.94 (3H, t, J = 7.3 Hz). | 466.3 | 465.29 |
| 1149 | 1H-NMR (DMSO-D6) δ: 10.06 (1H, s), 9.24 (1H, s), 8.37 (1H, d, J = 8.2 Hz), 8.18 (1H, s), 7.74 (1H, dd, J = 8.7, 2.3 Hz), 6.98 (1H, d, J = 0.9 Hz), 6.91 (1H, t, J = 5.7 Hz), 5.19 (1H, d, J = 4.6 Hz), 4.63-4.57 (1H, m), 4.40 (1H, t, J = 5.3 Hz), 3.49-3.35 (4H, m), 3.22 (2H, t, J = 5.7 Hz), 2.81 (2H, d, J = 10.5 Hz), 1.89 (2H, t, J = 10.5 Hz), 1.62 (2H, d, J = 11.0 Hz), 1.41-1.28 (4H, m), 1.26-1.18 (1H, m), 1.17-1.05 (2H, m), 0.49-0.43 (2H, m), 0.35-0.30 (2H, m). | 464.3 | 463.27 |
| 1150 | 1H-NMR (DMSO-D6) δ: 10.03 (1H, s), 9.25 (1H, s), 8.35 (1H, d, J = 8.2 Hz), 8.19 (1H, d, J = 1.8 Hz), 7.76 (1H, dd, J = 8.7, 2.3 Hz), 7.00 (1H, d, J = 0.9 Hz), 6.83 (1H, d, J = 7.8 Hz), 5.20 (1H, d, J = 4.1 Hz), 4.65-4.55 (2H, m), 4.40 (1H, t, J = 5.3 Hz), 3.42 (2H, s), 3.22 (2H, t, J = 5.7 Hz), 2.81 (2H, d, J = 11.0 Hz), 2.39-2.31 (2H, m), 2.11 (2H, td, J = 20.0, 10.7 Hz), 1.89 (2H, t, J = 11.0 Hz), 1.77-1.69 (2H, m), 1.62 (2H, d, J = 11.0 Hz), 1.39-1.28 (4H, m), 1.17-1.05 (2H, m). | 464.3 | 463.27 |

**[Table 5-231]**

| Compound No. | NMR | LC/MS (M+H)⁺ | Exact Mass |
|---|---|---|---|
| 1151 | 1H-NMR (DMSO-D6) δ: 10.06 (1H, s), 9.24 (1H, s), 8.24 (1H, d, J = 8.7 Hz), 8.18 (1H, d, J = 1.8 Hz), 7.73 (1H, dd, J = 8.2, 2.3 Hz), 6.98 (1H, s), 6.40 (1H, d, J = 7.8 Hz), 5.20 (1H, d, J = 4.6 Hz), 4.64-4.56 (2H, m), 4.40 (1H, t, J = 5.3 Hz), 3.96-3.86 (1H, m), 3.55-3.46 (1H, m), 3.41 (2H, s), 3.22 (2H, t, J = 5.7 Hz), 2.80 (2H, d, J = 11.0 Hz), 2.07 (2H, t, J = 14.0 Hz), 1.93-1.84 (4H, m), 1.61 (2H, d, J = 11.0 Hz), 1.50-1.27 (8H, m), 1.16-1.05 (2H, m). | 508.3 | 507.30 |
| 1152 | 1H-NMR (DMSO-D6) δ: 10.04 (1H, s), 9.23 (1H, s), 8.36 (1H, d, J = 8.7 Hz), 8.18 (1H, d, J = 2.3 Hz), 7.72 (1H, dd, J = 8.2, 2.3 Hz), 6.97 (1H, d, J = 0. 9 Hz), 6.90 (1H, t, J = 5.9 Hz), 5.19 (1H, d, J = 4.6 Hz), 4.63-4.57 (1H, m), 4.41 (1H, t, J = 5.3 Hz), 3.85 (2H, dd, J = 11.2, 2.5 Hz), 3.55-3.40 (4H, m), 3.30-3.20 (4H, m), 2.81 (2H, d, J = 11.0 Hz), 2.02-1.85 (3H, m), 1.62 (4H, d, J = 12.3 Hz), 1.40-1.24 (6H, m), 1.15-1.05 (2H, m). | 508.3 | 507.30 |
| 1153 | 1H-NMR (CDCl3) δ: 9.02 (1H, s), 8.13 (1H, d, J = 8.7 Hz), 8.04 (1H, s), 7.41 (1H, d, J = 8.7 Hz), 6.84 (1H, s), 6.15 (1H, d, J = 7.8 Hz), 4.33 (1H, q, J = 6.4 Hz), 4.14-4.03 (1H, m), 3.81-3.69 (5H, m), 3.41 (3H, s), 3.02-2.91 (4H, m), 2.78 (2H, t, J = 5.3 Hz), 2.34-2.23 (2H, m), 2.12-2.02 (2H, m), 1.62-1.47 (5H, m), 1.46-1.32 (2H, m). | 494.4 | 493.28 |

**[Table 5-232]**

| Compound No. | NMR | LC/MS (M+H)⁺ | Exact Mass |
|---|---|---|---|
| 1154 | 1H-NMR (CDCl3) δ: 8.96 (1H, s), 8.12 (1H, d, J = 8.2 Hz), 8.01 (1H, s), 7.41 (1H, d, J = 8.2 Hz), 6.65 (1H, s), 6.14 (1H, d, J = 7.3 Hz), 4.19 (1H, t, J = 8.0 Hz), 4.11-3.96 (3H, m), 3.89 (1H, t, J = 8.0 Hz), 3.81-3.68 (5H, m), 3.53-3.44 (1H, m), 3.02-2.89 (4H, m), 2.78 (2H, t, J = 5.3 Hz), 2.33-2.24 (4H, m), 2.12-2.03 (2H, m), 1.60-1.47 (2H, m), 1.46-1.34 (2H, m). | 506.4 | 505.28 |
| 1155 | 1H-NMR (CDCl3) δ: 8.98 (1H, s), 8.12 (1H, d, J = 8.2 Hz), 8.02 (1H, s), 7.42 (1H, d, J = 8.7 Hz), 6.63 (1H, s), 6.17 (1H, d, J = 7.8 Hz), 5.14 (2H, d, J = 5.5 Hz), 4.58 (2H, d, J = 5.5 Hz), 4.14-4.01 (1H, m), 3.82-3.68 (5H, m), 3.02-2.90 (4H, m), 2.78 (2H, t, J = 5.3 Hz), 2.35-2.24 (2H, m), 2.13-2.03 (2H, m), 1.79 (3H, s), 1.62-1.48 (2H, m), 1.48-1.35 (2H, m). | 506.4 | 505.28 |
| 1156 | 1H-NMR (DMSO-D6) δ: 10.17 (1H, s), 9.26 (1H, s), 8.26 (1H, d, J = 8.7 Hz), 8.19 (1H, d, J = 1.8 Hz), 7.71 (1H, dd, J = 8.5, 2.1 Hz), 7.02 (1H, s), 6.62 (1H, d, J = 8.2 Hz), 5.23 (1H, d, J = 4.6 Hz), 4.64-4.58 (1H, m), 4.41 (1H, t, J = 5.0 Hz), 4.18-4.10 (1H, m), 3.93 (1H, dd, J = 10.7, 3.0 Hz), 3.72-3.65 (1H, m), 3.56-3.50 (1H, m), 3.45-3.39 (3H, m), 3.22 (2H, t, J = 5.7 Hz), 2.81 (2H, d, J = 10.5 Hz), 2.02-1.71 (5H, m), 1.65-1.58 (3H, m), 1.39-1.27 (4H, m), 1.16-1.05 (2H, m). | 494.3 | 493.28 |

**[Table 5-233]**

| Compound No. | NMR | LC/MS (M+H)⁺ | Exact Mass |
|---|---|---|---|
| 1157 | 1H-NMR (DMSO-D6) δ: 10.15 (1H, s), 9.25 (1H, s), 8.29 (1H, d, J = 8.7 Hz), 8.21 (1H, d, J = 1.8 Hz), 7.76 (1H, dd, J = 8.2, 2.3 Hz), 6.89 (1H, s), 6.47 (1H, d, J = 7.8 Hz), 4.62 (1H, dd, J = 7.3, 5.9 Hz), 4.29-4.22 (2H, m), 3.77-3.67 (2H, m), 3.55-3.40 (6H, m), 3.27 (3H, s), 2.40-2.30 (4H, m), 2.02-1.91 (2H, m), 1.85-1.74 (2H, m), 1.38 (3H, d, J = 6.4 Hz), 1.30 (6H, dd, J = 6.6, 3.9 Hz). | 535.3 | 534.31 |
| 1158 | 1H-NMR (DMSO-D6) δ: 10.15 (1H, s), 9.25 (1H, s), 8.29 (1H, d, J = 8.7 Hz), 8.21 (1H, d, J = 1.8 Hz), 7.76 (1H, dd, J = 8.7, 2.3 Hz), 6.89 (1H, s), 6.47 (1H, d, J = 7.8 Hz), 4.29-4.22 (2H, m), 3.83 (1H, t, J = 8.0 Hz), 3.71-3.61 (3H, m), 3.51-3.44 (6H, m), 3.35-3.29 (1H, m), 3.27 (3H, s), 2.40-2.30 (4H, m), 2.03-1.91 (2H, m), 1.38 (3H, d, J = 6.4 Hz), 1.30 (6H, dd, J = 6.4, 3.7 Hz). | 535.3 | 534.31 |
| 1159 | 1H-NMR (CDCl3) δ: 9.01 (1H, d, J = 2.3 Hz), 8.48-8.28 (2H, m), 7.08 (1H, d, J = 10.1 Hz), 6.68 (1H, s), 6.37 (1H, s), 4.78 (1H, q, J = 6.4 Hz), 4.33 (2H, d, J = 13.3 Hz), 3.57 (2H, q, J = 6.6 Hz), 2.94 (2H, t, J = 11.7 Hz), 2.44-2.36 (1H, m), 2.33 (6H, d, J = 15.1 Hz), 1.96 (2H, d, J = 12.8 Hz), 1.79-1.70 (2H, m), 1.63-1.49 (5H, m), 1.04 (3H, t, J = 7.3 Hz). | 452.4 | 451.28 |
| 1160 | 1H-NMR (CDCl3) δ: 9.06 (1H, s), 8.58 (1H, d, J = 15.6 Hz), 8.48-8.40 (2H, m), 7.86 (1H, dd, J = 8.2, 2.3 Hz), 6.71 (1H, s), 6.24 (1H, d, J = 7.3 Hz), 4.80 (1H, q, J = 6.4 Hz), 4.40 (1H, td, J = 13.5, 6.7 Hz), 4.04 (1H, br s), 3.67 (4H, br s), 2.48 (4H, s), 2.35 (3H, br s), 1.53 (3H, d, J = 6.4 Hz), 1.37 (6H, dd, J = 6.4, 0.9 Hz). | 451.3 | 450.25 |

**[Table 5-234]**

| Compound No. | NMR | LC/MS (M+H)⁺ | Exact Mass |
|---|---|---|---|
| 1161 | 1H-NMR (CDCl3) δ: 9.05 (1H, s), 8.45-8.40 (3H, m), 7.85 (1H, dd, J = 8.7, 2.3 Hz), 6.71 (1H, d, J = 0.9 Hz), 6.24 (1H, d, J = 7.8 Hz), 4.80 (1H, q, J = 6.4 Hz), 4.40 (1H, td, J = 13.5, 6.7 Hz), 4.02 (1H, br s), 3.10-2.80 (2H, m), 2.55-2.44 (2H, m), 2.36 (6H, s), 2.01-1.91 (4H, m), 1.52 (3H, d, J = 6.4 Hz), 1.37 (6H, dd, J = 6.4, 0.9 Hz). | 479.4 | 478.28 |
| 1162 | | 465.4 | 464.26 |
| 1163 | 1H-NMR (CDCl3) δ: 9.07 (1H, s), 8.65-8.58 (2H, m), 8.42 (1H, d, J = 8.7 Hz), 7.98 (1H, dd, J = 8.7, 2.3 Hz), 6.71 (1H, d, J = 0.9 Hz), 6.24 (1H, d, J = 7.8 Hz), 4.80 (1H, q, J = 6.4 Hz), 4.40 (1H, td, J = 13.4, 6.9 Hz), 4.05-3.53 (5H, m), 2.93-2.72 (1H, m), 2.38 (3H, br s), 2.28 (3H, br s), 2.01-1.92 (1H, m), 1.53 (3H, d, J = 6.4 Hz), 1.37 (6H, dd, J = 6.4, 1.4 Hz). | 465.4 | 464.26 |
| 1164 | | 439.3 | 438.25 |
| 1165 | 1H-NMR (CDCl3) δ: 9.06 (1H, s), 8.51-8.41 (3H, m), 7.86 (1H, dd, J = 8.7, 2.3 Hz), 6.71 (1H, s), 6.24 (1H, d, J = 7.3 Hz), 4.80 (1H, d, J = 6.4 Hz), 4.43-4.37 (1H, m), 3.77-3.65 (6H, m), 2.66-2.58 (8H, m), 1.53 (3H, d, J = 6.4 Hz), 1.37 (6H, d, J = 6.4 Hz). | 481.4 | 480.26 |
| 1166 | 1H-NMR (CDCl3) δ: 9.30 (1H, s), 9.11 (1H, s), 8.51 (1H, d, J = 1.8 Hz), 8.43 (1H, d, J = 8.7 Hz), 7.83 (1H, dd, J = 8.5, 2.1 Hz), 6.71 (1H, s), 6.22 (1H, d, J = 7.8 Hz), 4.80 (1H, q, J = 6.4 Hz), 4.42-4.35 (2H, m), 4.00 (1H, br s), 3.26 (1H, t, J = 13.0 Hz), 3.04-2.96 (2H, m), 2.87-2.74 (2H, m), 1.52 (3H, d, J = 6.4 Hz), 1.39-1.35 (9H, m). | 451.3 | 450.25 |

**[Table 5-235]**

| Compound No. | NMR | LC/MS (M+H)⁺ | Exact Mass |
|---|---|---|---|
| 1167 | 1H-NMR (CDC13) δ: 9.35 (1H, s), 9.11 (1H, s), 8.52 (1H, d, J = 1.8 Hz), 8.43 (1H, d, J = 8.7 Hz), 7.83 (1H, dd, J = 8.7, 2.3 Hz), 6.71 (1H, s), 6.21 (1H, d, J = 7.3 Hz), 4.80 (1H, q, J = 6.4 Hz), 4.43-4.34 (2H, m), 4.01 (1H, br s), 3.26 (1H, t, J = '13.3 Hz), 3.05-2.94 (2H, m), 2.86-2.74 (2H, m), 1.52 (3H, d, J = 6.4 Hz), 1.39-1.35 (9H, m) | 451.3 | 450.25 |
| 1168 | 1H-NMR (400 MHz, DMSO-d6, ppm) δ: 10.06 (1H, s), 9.39 (1H, s), 8.37 (1H, d, J = 28.4 Hz), 8.13 (1H, d, J = 2.0 Hz), 7.76-7.73 (1H, m), 6.85-6.82 (1H, m), 4.97-4.94 (1H, m), 4.77 (1H, d, J = 6.4 Hz), 4.36-4.34 (1H, m), 3.62-3.54 (2H, m), 3.47-3.43 (4H, m), 2.48-2.31 (12H, m), 1.33 (3H, d, J = 6.4 Hz), 1.26-1.22 (3H, m) | 467.2 | 466.28 |
| 1169 | 1H-NMR (400 MHz, DMSO-d6, ppm) δ: 10.02 (1H, s), 9.23 (1H, s), 8.41 (1H, d, J = 8.4 Hz), 8.20 (1H, s), 7.76-7.74 (1H, m), 6.97-6.89 (2H, m), 5.21 (1H, d, J = 4.8 Hz), 4.63-4.58 (1H, m), 3.61-3.44 (6H, m), 2.74-2.11 (8H, m), 1.38-1.23 (12H, m) | 481.3 | 480.30 |
| 1170 | 1H-NMR (400 MHz, DMSO-d6, ppm) δ: 10.09 (1H, s), 9.31 (1H, s), 8.43 (1H, d, J = 8.8 Hz), 8.19 (1H, s), 7.74-7.72 (1H, m), 7.31-7.28 (1H, m), 7.14 (1H, s), 5.27 (1H, d, J = 4.8 Hz ), 4.65-4.62 (1H, m), 4.43-4.33 (3H, m), 3.47-3.44 (4H, m), 2.38-2.33 (10H, m), 1.37 (3H, d, J = 6.4 Hz) | 507.3 | 506.24 |
| 1171 | 1H-NMR (400 MHz, DMSO-d6, ppm) δ: 9.95 (1H, s), 9.25 (1H, s), 8.39 (1H, d, J = 8.8Hz), 8.17 (1H, d, J = 1.6Hz), 7.76-7.73 (1H, m), 7.03-7.01 (2H, m), 5.40-4.80 (1H, m(br)), 5.20 (1H, d, J = 4.4Hz), 4.75 (1H, br), 4.62-4.60 (1H, m), 4.36-4.33 (1H, m), 3.48-3.42 (4H, m), 2.62-2.60(4H, br), 2.48-2.32 (10H, m), 1.38-1.36 (3H, d, J=6.4Hz) | 497.4 | 496.27 |

**[Table 5-236]**

| Compound No. | NMR | LC/MS (M+H)⁺ | Exact Mass |
|---|---|---|---|
| 1172 | 1H-NMR (400 MHz, DMSO-d6, ppm) δ: 10.00 (1H, s), 9.24 (1H, s), 8.38 (1H, d, J = 8.0Hz), 8.19 (1H, d, J = 2Hz), 7.74 (1H, d, J=8.8Hz), 6.98 (1H, s), 6.90-6.87 (1H, br), 5.19 (1H, d, J = 4.8Hz), 4.63-4.60 (1H, m), 4.24-4.20 (1H, m), 3.73-3.71 (1H, br), 3.53-3.47 (2H, m), 3.45 (2H, s), 2.39-2.33 (8H, m), 2.24-2.20 (1H, m), 2.15-2.11 (1h, m),1.71-1.66 (2H, m), 1.36 (3H, d, J=6.4Hz), 1.02 (3H, d, J=6.0Hz), 0.97-0.95 (3H, m) | 481.3 | 480.30 |
| 1173 | 1H-NMR (400 MHz, DMSO-d6, ppm) δ: 10.12 (1H, s), 9.25 (1H, s), 8.34 (1H, d, J = 8.8Hz), 8.27 (1H, d, J = 2.4Hz), 7.82-7.79 (1H, m), 7.04 (1H, s), 6.96 (1H, s), 5.23 (1H, d, J = 4.4Hz), 4.67-4.65 (1H, m), 3.64-3.62 (2H, m), 3.40 (2H, s), 3.02 (2H, br), 2.77 (1H, br), 1.48 (3H, s), 1.41 (3H, d, J = 6.8Hz), 0.81-0.79 (2H, m), 0.70 (2H, s) | 435.1 | 434.22 |
| 1174 | 1H-NMR (400 MHz, DMSO-d6, ppm) δ: 10.22 (1H, s), 9.33 (1H, s), 8.47 (1H, d, J = 8.8Hz), 8.29 (1H, s), 7.76 (1H, d, J = 2.8Hz), 7.34-7.30 (1H, m), 7.15 (1H, s), 5.29 (1H, d, J = 4.8Hz), 4.66-4.61 (1H, m), 4.46-4.35 (2H, m), 3.40 (2H, s), 3.65-3.62 (2H, m), 3.40 (2H, s), 3.04-3.01 (2H, m), 2.78 (1H, br), 1.37 (3H, d, J = 6.4Hz) | 463.1 | 462.17 |
| 1175 | 1H-NMR (400 MHz, DMSO-d6, ppm) δ: 10.58 (1H, s), 9.19 (1H, s), 8.21 (1H, d, J = 10Hz), 7.37-7.35 (1H, d, J = 10Hz), 7.01 (1H, s), 6.84 (1H, s), 5.20 (1H, d, J = 4.4Hz), 4.63-4.63 (1H, m), 3.43- 3.41 (4H, m), 32.82-2.80 (4H, m), 1.46 (3H, s), 1.40 (3H, d, J = 6.4Hz), 0.78-0.76 (2H, m), 0.71-0.69 (2H, m) | 422.1 | 421.23 |

**[Table 5-237]**

| Compound No. | NMR | LC/MS (M+H)⁺ | Exact Mass |
|---|---|---|---|
| 1176 | 1H-NMR (DMSO-D6) δ: 10.34 (1H, s), 9.21 (1H, s), 8.23 (1H, d, J = 9.6 Hz), 7.44 (1H, d, J = 9.6 Hz), 6.87 (1H, s), 6.38 (1H, d, J = 7.8 Hz), 4.73 (1H, dd, J = 7.8, 5.5 Hz), 4.28-4.19 (2H, m), 3.81-3.72 (2H, m), 3.69-3.46 (8H, m), 3.26 (3H, s), 2.11-1.95 (2H, m), 1.88-1.77 (2H, m), 1.37 (3H, d, J = 6.4 Hz), 1.27 (6H, dd, J = 6.4, 3.7 Hz). | 522.2 | 521.29 |
| 1177 | 1H-NMR (DMSO-D6) δ: 10.33 (1H, s), 9.21 (1H, s), 8.23 (1H, d, J = 10.1 Hz), 7.45 (1H, d, J = 9.6 Hz), 6.87 (1H, s), 6.37 (1H, d, J = 7.8 Hz), 4.26-4.18 (2H, m), 3.89 (1H, t, J = 8.0 Hz), 3.75-3.48 (11H, m), 3.47-3.38 (1H, m), 3.26 (3H, s), 2.09-1.98 (2H, m), 1.37 (3H, d, J = 6.4 Hz), 1.27 (6H, dd, J = 6.6, 3.4 Hz). | 522.2 | 521.29 |
| 1178 | 1H-NMR (CDC13) δ: 8.96 (1H, s), 8.18-8.13 (1H, m), 8.00 (1H, s), 7.40 (1H, d, J = 8.7 Hz), 6.66 (1H, s), 6.58-6.47 (1H, m), 4.37-4.26 (1H, m), 4.17 (1H, t, J = 8.0 Hz), 4.11-3.93 (3H, m), 3.89 (1H, t, J = 8.0 Hz), 3.81-3.55 (5H, m), 3.53-3.42 (1H, m), 3.01-2.94 (2H, m), 2.92-2.84 (2H, m), 2.74-2.66 (2H, m), 2.59-2.52 (2H, m), 2.31-2.23 (8H, m), 2.10-1.99 (1H, m), 1.92-1.77 (2H, m), 1.76-1.63 (1H, m). | 519.4 | 518.31 |
| 1179 | 1H-NMR (CDCl3) δ: 8.98 (1H, s), 8.17 (1H, d, J = 8.2 Hz), 8.02 (1H, s), 7.41 (1H, d, J = 8.2 Hz), 6.64 (1H, s), 6.58 (1H, d, J = 8.2 Hz), 5.15-5.10 (2H, m), 4.58 (2H, d, J = 5.0 Hz), 4.40-4.29 (1H, m), 4.03 (1H, dd, J = 11.0, 2.7 Hz), 3.81-3.73 (2H, m), 3.70-3.58 (3H, m), 3.02-2.94 (2H, m), 2.92-2.85 (2H, m), 2.75-2.67 (2H, m), 2.61-2.53 (2H, m), 2.32 (6H, s), 2.11-2.00 (1H, m), 1.96-1.62 (6H, m). | 519.4 | 518.31 |

**[Table 5-238]**

| Compound No. | NMR | LC/MS (M+H)⁺ | Exact Mass |
|---|---|---|---|
| 1180 | 1H-NMR (CDCl3) δ: 9.01 (1H, s), 8.11 (1H, d, J = 8.7 Hz), 8.00 (1H, s), 7.40 (1H, d, J = 8.7 Hz), 6.84 (1H, s), 6.15 (1H, d, J = 7.3 Hz), 4.33 (1H, q, J = 6.4 Hz), 4.14-4.03 (1H, m), 3.81-3.71 (1H, m), 3.67 (2H, s), 3.41 (3H, s), 3.01-2.95 (2H, m), 2.92-2.86 (2H, m), 2.74-2.67 (2H, m), 2.60-2.53 (2H, m), 2.34-2.24 (8H, m), 2.12-2.03 (2H, m), 1.61-1.47 (5H, m), 1.46-1.31 (2H, m). | 521.4 | 520.33 |
| 1181 | 1H-NMR (CDCl3) δ: 8.95 (1H, s), 8.10 (1H, d, J = 8.7 Hz), 7.99 (1H, s), 7.40 (1H, d, J = 8.2 Hz), 6.64 (1H, s), 6.14 (1H, d, J = 7.3 Hz), 4.19 (1H, t, J = 8.0 Hz), 4.11-3.96 (3H, m), 3.89 (1H, t, J = 8.0 Hz), 3.81-3.71 (1H, m), 3.67 (2H, s), 3.53-3.44 (1H, m), 3.01-2.93 (2H, m), 2.92-2.84 (2H, m), 2.74-2.66 (2H, m), 2.60-2.52 (2H, m), 2.34-2.23 (10H, m), 2.12-2.03 (2H, m), 1.60-1.47 (2H, m), 1.46-1.33 (2H, m). | 533.4 | 532.33 |
| 1182 | 1H-NMR (CDCl3) δ: 8.97 (1H, s), 8.11 (1H, d, J = 8.7 Hz), 7.99 (1H, s), 7.41 (1H, d, J = 8.2 Hz), 6.62 (1H, s), 6.17 (1H, d, J = 7.3 Hz), 5.14 (2H, d, J = 5.5 Hz), 4.58 (2H, d, J = 5.5 Hz), 4.14-4.00 (1H, m), 3.81-3.72 (1H, m), 3.67 (2H, s), 3.01-2.94 (2H, m), 2.92-2.85 (2H, m), 2.74-2.66 (2H, m), 2.60-2.52 (2H, m), 2.35-2.24 (8H, m), 2.13-2.03 (2H, m), 1.79 (3H, s), 1.61-1.49 (2H, m), 1.48-1.35 (2H, m). | 533.4 | 532.33 |
| 1183 | 1H-NMR (DMSO-D6) δ: 10.29 (1H, s), 9.21 (1H, s), 8.23 (1H, d, J = 9.6 Hz), 7.44 (1H, d, J = 10.1 Hz), 6.96 (1H, s), 6.33 (1H, d, J = 7.8 Hz), 5.20 (1H, d, J = 4.1 Hz), 4.73 (1H, dd, J = 7.5, 5.7 Hz), 4.63-4.55 (1H, m), 4.28-4.19 (1H, m), 3.81-3.45 (10H, m), 2.11-1.96 (2H, m), 1.89-1.77 (2H, m), 1.37 (3H, d, J = 6.4 Hz), 1.27 (6H, dd, J = 6.6, 1.6 Hz). | 508.2 | 507.27 |

**[Table 5-239]**

| Compound No. | NMR | LC/MS (M+H)⁺ | Exact Mass |
|---|---|---|---|
| 1184 | 1H-NMR (DMSO-D6) δ: 10.30 (1H, s), 9.21 (1H, s), 8.24 (1H, d, J = 9.6 Hz), 7.45 (1H, d, J = 10.1 Hz), 6.96 (1H, s), 6.33 (1H, d, J = 7.8 Hz), 5.20 (1H, d, J = 4.6 Hz), 4.63-4.56 (1H, m), 4.27-4.19 (1H, m), 3.89 (1H, t, J = 8.2 Hz), 3.74-3.60 (7H, m), 3.59-3.54 (2H, m), 3.53-3.47 (2H, m), 3.46-3.38 (1H, m), 2.07-1.98 (2H, m), 1.37 (3H, d, J = 6.4 Hz), 1.27 (6H, dd, J = 6.4, 1.4 Hz). | 508.3 | 507.27 |
| 1185 | 1H-NMR (DMSO-D6) δ: 10.07 (1H, s), 9.24 (1H, s), 8.39 (1H, d, J = 8.7 Hz), 8.19 (1H, d, J = 2.3 Hz), 7.73 (1H, dd, J = 8.7, 2.3 Hz), 6.97 (1H, s), 6.86 (1H, t, J = 5.9 Hz), 5.20 (1H, d, J = 4.6 Hz), 4.63-4.56 (2H, m), 3.59-3.36 (5H, m), 2.80 (1H, d, J = 6.9 Hz), 2.65 (1H, d, J = 11.0 Hz), 1.88-1.76 (2H, m), 1.72-1.58 (4H, m), 1.43-1.33 (6H, m), 1.09-1.00 (1H, m), 0.93 (3H, t, J = 7.5 Hz). | 452.3 | 451.27 |
| 1186 | 1H-NMR (DMSO-D6) δ: 10.09 (1H, s), 9.24 (1H, s), 8.36 (1H, d, J = 8.2 Hz), 8.19 (1H, d, J = 1.8 Hz), 7.71 (1H, dd, J = 8.7, 2.3 Hz), 6.97 (1H, s), 6.84 (1H, t, J = 5.9 Hz), 5.20 (1H, d, J = 4.6 Hz), 4.62-4.57 (2H, m), 3.53-3.35 (5H, m), 2.79 (1H, d, J = 6.9 Hz), 2.65 (1H, d, J = 10.1 Hz), 2.06-1.97 (1H, m), 1.89-1.58 (4H, m), 1.45-1.34 (4H, m), 1.11-0.92 (7H, m). | 452.2 | 451.27 |
| 1187 | 1H-NMR (DMSO-D6) δ: 10.10 (1H, s), 9.24 (1H, s), 8.25 (1H, d, J = 8.7 Hz), 8.19 (1H, s), 7.74 (1H, d, J = 8.7 Hz), 6.98 (1H, s), 6.40 (1H, d, J = 7.8 Hz), 5.21 (1H, d, J = 4.6 Hz), 4.64-4.57 (3H, m), 3.95-3.87 (1H, m), 3.54-3.35 (4H, m), 2.79 (1H, d, J = 8.2 Hz), 2.65 (1H, d, J = 9.1 Hz), 2.11-2.02 (2H, m), 1.90-1.57 (6H, m), 1.49-1.27 (8H, m), 1.09-1.00 (1H, m). | 494.3 | 493.28 |

**[Table 5-240]**

| Compound No. | NMR | LC/MS (M+H)⁺ | Exact Mass |
|---|---|---|---|
| 1188 | 1H-NMR (DMSO-D6) δ: 10.06 (1H, s), 9.23 (1H, s), 8.39 (1H, d, J = 8.7 Hz), 8.19 (1H, s), 7.73 (1H, dd, J = 8.5, 2.1 Hz), 6.97 (1H, s), 6.86 (1H, t, J = 5.7 Hz), 5.20 (1H, d, J = 4.6 Hz), 4.63-4.57 (2H, m), 3.59-3.38 (5H, m), 2.80 (1H, d, J = 7.3 Hz), 2.66 (1H, d, J = 7.3 Hz), 1.89-1.58 (6H, m), 1.43-1.34 (6H, m), 1.09-1.00 (1H, m), 0.94 (3H, t, J = 7.3 Hz). | 452.3 | 451.27 |
| 1189 | 1H-NMR (DMSO-D6) δ: 10.12 (1H, s), 9.24 (1H, s), 8.36 (1H, d, J = 8.7 Hz), 8.20 (1H, s), 7.72 (1H, d, J = 8.2 Hz), 6.97 (1H, s), 6.85 (1H, t, J = 5.7 Hz), 5.20 (1H, d, J = 4.6 Hz), 4.63-4.57 (2H, m), 3.53-3.36 (5H, m), 2.79 (1H, s), 2.66 (1H, s), 2.05-1.98 (1H, m), 1.89-1.57 (4H, m), 1.46-1.34 (4H, m), 1.12-0.92 (7H, m). | 452.3 | 451.27 |
| 1190 | 1H-NMR (DMSO-D6) δ: 10.10 (1H, s), 9.24 (1H, s), 8.25 (1H, d, J = 8.7 Hz), 8.19 (1H, s), 7.74 (1H, d, J = 8.2 Hz), 6.98 (1H, s), 6.40 (1H, d, J = 7.8 Hz), 5.21 (1H, d, J = 4.6 Hz), 4.64-4.57 (3H, m), 3.96-3.87 (1H, m), 3.54-3.38 (4H, m), 2.78 (1H, br s), 2.66 (1H, br s), 2.11-2.03 (2H, m), 1.91-1.57 (6H, m), 1.49-1.28 (8H, m), 1.09-1.00 (1H, m). | 494.3 | 493.28 |
| 1191 | 1H-NMR (DMSO-D6) δ: 10.27 (1H, s), 9.20 (1H, s), 8.23 (1H, d, J = 10.1 Hz), 7.44 (1H, d, J = 9.6 Hz), 6.96 (1H, s), 6.33 (1H, d, J = 7.8 Hz), 5.18 (1H, d, J = 4.6 Hz), 4.63-4.57 (1H, m), 4.27-4.19 (1H, m), 3.70-3.48 (8H, m), 3.15 (2H, s), 2.20 (6H, s), 1.37 (3H, d, J = 6.4 Hz), 1.27 (6H, dd, J = 6.4, 1.8 Hz). | 495.2 | 494.29 |

**[Table 5-241]**

| Compound No. | NMR | LC/MS (M+H) ⁺ | Exact Mass |
|---|---|---|---|
| 1192 | 1H-NMR (DMSO-D6) δ: 10.10 (1H, s), 9.25 (1H, s), 8.29 (1H, d, J = 8.2 Hz), 8.22 (1H, d, J = 1.8 Hz), 7.76 (1H, dd, J = 8.7, 2.3 Hz), 6.99 (1H, s), 6.42 (1H, d, J = 7.8 Hz), 5.20 (1H, d, J = 4.6 Hz), 4.65-4.59 (2H, m), 4.30-4.22 (1H, m), 3.78-3.67 (2H, m), 3.54-3.40 (6H, m), 2.41-2.30 (4H, m), 2.03-1.91 (2H, m), 1.85-1.73 (2H, m), 1.38 (3H, d, J = 6.9 Hz), 1.29 (6H, dd, J = 6.4, 2.3 Hz). | 521.3 | 520.29 |
| 1193 | 1H-NMR (DMSO-D6) δ: 10.09 (1H, s), 9.25 (1H, s), 8.29 (1H, d, J = 8.2 Hz), 8.21 (1H, d, J = 1.8 Hz), 7.76 (1H, dd, J = 8.7, 2.3 Hz), 6.99 (1H, s), 6.42 (1H, d, J = 7.3 Hz), 5.20 (1H, d, J = 4.6 Hz), 4.64-4.58 (1H, m), 4.26 (1H, td, J = 13.5, 6.7 Hz), 3.84 (1H, t, J = 8.2 Hz), 3.70-3.61 (3H, m), 3.51-3.44 (6H, m), 3.35-3.27 (1H, m), 2.39-2.30 (4H, m), 2.00-1.92 (2H, m), 1.38 (3H, d, J = 6.4 Hz), 1.29 (6H, dd, J = 6.4, 2.3 Hz). | 521.2 | 520.29 |
| 1194 | 1H-NMR (DMSO-D6) δ: 10.11 (1H, s), 9.25 (1H, s), 8.28 (1H, d, J = 8.2 Hz), 8.22 (1H, s), 7.76 (1H, d, J = 8.2 Hz), 6.99 (1H, s), 6.41 (1H, d, J = 7.3 Hz), 5.21 (1H, s), 4.62 (1H, s), 4.31-4.22 (1H, m), 3.55-3.39 (6H, m), 3.10 (2H, s), 2.42-2.29 (4H, m), 2.17 (6H, s), 1.38 (3H, d, J = 5.9 Hz), 1.29 (6H, t, J = 3.2 Hz). | 508.3 | 507.31 |
| 1195 | 1H-NMR (DMSO-D6) δ: 10.03 (1H, s), 9.23 (1H, s), 8.39 (1H, d, J = 8.2 Hz), 8.19 (1H, s), 7.74 (1H, dd, J = 8.5, 2.1 Hz), 6.97 (1H, d, J = 0.9 Hz), 6.88 (1H, t, J = 5.9 Hz), 5.18 (1H, d, J = 4.6 Hz), 4.64-4.56 (2H, m), 3.56-3.38 (5H, m), 2.80 (1H, d, J = 7.8 Hz), 2.66 (1H, d, J = 7.8 Hz), 1.90-1.57 (6H, m), 1.46-1.35 (4H, m), 1.10-1.00 (1H, m), 0.95 (3H, t, J = 7.3 Hz). | 438.2 | 437.25 |

**[Table 5-242]**

| Compound No. | NMR | LC/MS (M+H) ⁺ | Exact Mass |
|---|---|---|---|
| 1196 | 1H-NMR (DMSO-D6) δ: 10.06 (1H, s), 9.24 (1H, s), 8.27 (1H, d, J = 8.7 Hz), 8.19 (1H, s), 7.74 (1H, d, J = 8.7 Hz), 6.99 (1H, s), 6.41 (1H, d, J = 7.8 Hz), 5.20 (1H, d, J = 4.6 Hz), 4.64-4.56 (2H, m), 4.26 (1H, td, J = 13.4, 6.9 Hz), 3.51-3.37 (3H, m) , 2.79 (1H, d, J = 7.8 Hz), 2.65 (1H, d, J = 9.1 Hz), 1.90-1.58 (4H, m), 1.46-1.37 (4H, m), 1.29 (6H, dd, J = 6.4, 1.8 Hz), 1.09-1.00 (1H, m). | 438.2 | 437.25 |
| 1197 | 1H-NMR (DMSO-D6) δ: 10.17 (1H, s), 9.23 (1H, s), 8.21-8.17 (2H, m), 7.71 (1H, dd, J = 8.5, 2.5 Hz), 6.98 (1H, d, J = 0.9 Hz), 6.43 (1H, s), 5.20 (1H, d, J = 4.6 Hz), 4.65-4.56 (2H, m), 3.52-3.36 (3H, m), 2.78 (1H, d, J = 6.9 Hz), 2.65 (1H, d, J = 11.4 Hz), 1.90-1.50 (13H, m), 1.43-1.36 (4H, m), 1.09-1.00 (1H, m). | 452.2 | 451.27 |
| 1198 | 1H-NMR (DMSO-D6) δ: 10.03 (1H, s), 9.23 (1H, s), 8.39 (1H, d, J = 8.7 Hz), 8.19 (1H, s), 7.74 (1H, d, J = 7.3 Hz), 6.97 (1H, s), 6.89 (1H, t, J = 5.7 Hz), 5.18 (1H, d, J = 4.6 Hz), 4.63-4.57 (2H, m), 3.54-3.40 (5H, m), 2.80 (1H, d, J = 8.2 Hz), 2.65 (1H, br s), 1.90-1.57 (6H, m), 1.45-1.35 (4H, m), 1.10-1.00 (1H, m), 0.95 (3H, t, J = 7.2 Hz). | 438.2 | 437.25 |
| 1199 | 1H-NMR (DMSO-D6) δ: 10.08 (1H, s), 9.25 (1H, s), 8.27 (1H, d, J = 8.7 Hz), 8.20 (1H, s), 7.74 (1H, d, J = 8.7 Hz), 6.99 (1H, s), 6.41 (1H, d, J = 7.8 Hz), 5.20 (1H, d, J = 4.6 Hz), 4.64-4.57 (2H, m), 4.26 (1H, td, J = 13.5, 7.0 Hz), 3.52-3.38 (3H, m), 2.80 (1H, d, J = 7.8 Hz), 2.65 (1H, d, J = 7.8 Hz), 1.90-1.57 (4H, m), 1.46-1.36 (4H, m), 1.29 (6H, dd, J = 6.4, 1.8 Hz), 1.09-1.00 (1H, m). | 438.2 | 437.25 |

**[Table 5-243]**

| Compound No. | NMR | LC/MS (M+H) ⁺ | Exact Mass |
|---|---|---|---|
| 1200 | 1H-NMR (DMSO-D6) δ: 10.18 (1H, s), 9.23 (1H, s), 8.22-8.17 (2H, m), 7.72 (1H, d, J = 8.2 Hz), 6.98 (1H, s), 6.43 (1H, s), 5.21 (1H, d, J = 4.6 Hz), 4.65-4.56 (2H, m), 3.52-3.38 (3H, m), 2.79 (1H, d, J = 7.3 Hz), 2.65 (1H, br s), 1.92-1.50 (13H, m), 1.43-1.37 (4H, m), 1.09-1.00 (1H, m). | 452.2 | 451.27 |
| 1201 | | 479.2 | 478.28 |
| 1202 | | 471.3 | 470.26 |
| 1203 | | 481.3 | 480.30 |
| 1204 | | 515.2 | 514.26 |
| 1205 | | 491.3 | 490.28 |
| 1206 | | 481.3 | 480.30 |
| 1207 | | 481.3 | 480.30 |
| 1208 | | 481.2 | 480.30 |
| 1209 | | 481.2 | 480.30 |
| 1210 | | 453.2 | 452.21 |
| 1211 | | 471.2 | 470.20 |
| 1212 | | 427.4 | 426.23 |
| 1213 | | 503.4 | 502.26 |
| 1214 | | 521.4 | 520.25 |
| 1215 | | 503.3 | 502.26 |
| 1216 | | 467.4 | 466.28 |
| 1217 | | 423.4 | 422.22 |
| 1218 | | 509.3 | 508.25 |
| 1219 | | 437.3 | 436.27 |
| 1220 | | 451.2 | 450.29 |
| 1221 | 1H-NMR (CDCl3) δ: 9.08 (1H, s), 8.73 (1H, s), 8.48 (1H, d, J = 2.3 Hz), 8.42 (1H, d, J = 8.7 Hz), 7.86 (1H, dd, J = 8.7, 2.3 Hz), 6.71 (1H, s), 6.43 (1H, t, J = 5.7 Hz), 4.81 (1H, q, J = 6.4 Hz), 3.68-3.58 (7H, m), 2.47 (4H, br s), 2.34 (3H, s), 1.81-1.73 (2H, m), 1.53 (3H, d, J = 6.4 Hz), 1.06 (3H, t, J = 7.6 Hz). | 451.3 | 450.25 |

**[Table 5-244]**

| Compound No. | NMR | LC/MS (M+H) ⁺ | Exact Mass |
|---|---|---|---|
| 1222 | 1H-NMR (CDCl3) δ: 9.07 (1H, s), 9.05 (1H, s), 8.32 (1H, d, J = 1.8 Hz), 8.30 (1H, d, J = 8.2 Hz), 7.69 (1H, dd, J = 8.5, 2.1 Hz), 6.69 (1H, s), 6.35 (1H, t, J = 5.5 Hz), 4.80 (1H, q, J = 6.4 Hz), 3.70-3.62 (2H, m), 3.46 (2H, s), 3.07 (2H, s), 2.59 (2H, dd, J = 10.5, 2.7 Hz), 2.36 (2H, d, J = 10.1 Hz), 2.26 (3H, s), 1.94-1.78 (4H, m), 1.52 (3H, d, J = 6.4 Hz), 1.36 (3H, t, J = 7.1 Hz). | 449.3 | 448.27 |
| 1223 | 1H-NMR (CDCl3) δ: 9.13 (1H, s), 9.07 (1H, s), 8.33 (1H, d, J = 1.8 Hz), 8.30 (1H, d, J = 8.7 Hz), 7.68 (1H, dd, J = 8.7, 2.3 Hz), 6.68 (1H, s), 6.43 (1H, t, J = 5.7 Hz), 4.79 (1H, q, J = 6.4 Hz), 3.59 (2H, dd, J = 13.3, 6.9 Hz), 3.46 (2H, s), 3.06 (2H, s), 2.58 (2H, dd, J = 10.7, 3.0 Hz), 2.36 (2H, d, J = 10.1 Hz), 2.26 (3H, s), 1.93-1.89 (2H, m), 1.82-1.73 (4H, m), 1.52 (3H, d, J = 6.4 Hz), 1.06 (3H, t, J = 7.3 Hz). | 463.4 | 462.29 |
| 1224 | 1H-NMR (CDC13) δ: 9.19 (1H, s), 9.07 (1H, s), 8.34 (1H, d, J = 1.8 Hz), 8.29 (1H, d, J = 8.7 Hz), 7.67 (1H, dd, J = 8.2, 2.3 Hz), 6.68 (1H, s), 6.23 (1H, d, J = 7.3 Hz), 4.79 (1H, q, J = 6.4 Hz), 4.41-4.33 (1H, m), 3.46 (2H, s), 3.07 (2H, s), 2.58 (2H, dd, J = 10.5, 2.7 Hz), 2.36 (2H, d, J = 10.1 Hz), 2.26 (3H, s), 1.93-1.78 (4H, m), 1.52 (3H, d, J = 6.4 Hz), 1.36 (6H, d, J = 6.4 Hz). | 463.4 | 462.29 |
| 1225 | 1H-NMR (CDCl3) δ: 9.07 (1H, s), 8.66-8.58 (2H, m), 7.68 (1H, d, J = 9.1 Hz), 6.72 (1H, s), 6.22 (1H, d, J = 7.3 Hz), 4.80 (1H, q, J = 6.4 Hz), 4.45-4.36 (1H, m), 3.82 (2H, s), 2.93 (4H, t, J = 5.0 Hz), 2.53 (4H, s), 1.52 (3H, d, J = 6.4 Hz), 1.37 (6H, d, J = 6.4 Hz). | 424.3 | 423.25 |
| 1226 | | 442.4 | 441.24 |
| 1227 | | 455.3 | 454.26 |
| 1228 | | 425.2 | 424.27 |

**[Table 5-245]**

| Compound No. | NMR | LC/MS (M+H) ⁺ | Exact Mass |
|---|---|---|---|
| 1229 | 1H-NMR (DMSO-D6) δ: 9.93 (1H, s), 9.14 (1H, s), 8.02 (1H, d, J = 8.7 Hz), 7.50 (1H, d, J = 8.2 Hz), 6.79 (1H, s), 6.41 (1H, d, J = 7.3 Hz), 4.50 (1H, t, J = 5.3 Hz), 4.28-4.17 (1H, m), 4.04 (1H, t, J = 7.8 Hz), 3.95-3.88 (1H, m), 3.87-3.79 (1H, m), 3.72 (1H, t, J = 7.8 Hz), 3.63-3.54 (4H, m), 3.48-3.38 (1H, m), 2.86-2.75 (4H, m), 2.58 (2H, t, J = 6.2 Hz), 2.24-2.10 (2H, m), 1.32-1.27 (6H, m). | 450.3 | 449.25 |
| 1230 | 1H-NMR (CDC13) δ: 8.95 (1H, s), 8.12 (1H, d, J = 8.7 Hz), 8.01 (1H, s), 7.41 (1H, d, J = 8.7 Hz), 6.63 (1H, s), 6.12 (1H, d, J = 7.3 Hz), 4.42-4.31 (1H, m), 4.19 (1H, t, J = 8.0 Hz), 4.12-4.04 (1H, m), 4.02-3.89 (2H, m), 3.67 (2H, s), 3.53-3.43 (1H, m), 3.01-2.94 (2H, m), 2.92-2.85 (2H, m), 2.74-2.66 (2H, m), 2.59-2.52 (2H, m), 2.37-2.21 (8H, m), 1.35 (6H, d, J = 6.4 Hz). | 477.4 | 476.30 |
| 1231 | 1H-NMR (CDCl3) δ: 9.00-8.95 (1.0H, m), 8.28-8.21 (1.0H, m), 8.16-8.03 (1.0H, m), 7.56-7.46 (1.0H, m), 6.65 (1.0H, s), 6.11 (1.0H, d, J = 7.3 Hz), 4.80 (1.2H, s), 4.48-4.25 (3.8H, m), 4.19 (1.0H, t, J = 8.0 Hz), 4.12-3.89 (3.8H, m), 3.70-3.59 (2.2H, m), 3.54-3.44 (1.0H, m), 3.03-2.94 (2.0H, m), 2.37-2.20 (2.0H, m), 1.36 (6.0H, d, J = 6.4 Hz). | 464.3 | 463.23 |
| 1232 | 1H-NMR (DMSO-D6) δ: 9.98 (1H, s), 9.16-9.13 (1H, m), 8.03 (1H, d, J = 8.7 Hz), 7.52 (1H, d, J = 8.7 Hz), 6.79 (1H, s), 6.41 (1H, d, J = 7.3 Hz), 4.30-4.16 (1H, m), 4.04 (1H, t, J = 8.0 Hz), 3.96-3.87 (1H, m), 3.87-3.79 (1H, m), 3.78-3.68 (3H, m), 3.48-3.34 (3H, m), 3.00-2.91 (2H, m), 2.89-2.80 (2H, m), 2.24-2.09 (2H, m), 1.29 (6H, d, J = 6.9 Hz). | 464.3 | 463.23 |

**[Table 5-246]**

| Compound No. | NMR | LC/MS (M+H) ⁺ | Exact Mass |
|---|---|---|---|
| 1233 | 1H-NMR (DMSO-D6) δ: 9.95 (1H, s), 9.14 (1H, s), 8.02 (1H, d, J = 8.7 Hz), 7.53 (1H, d, J = 8.2 Hz), 6.79 (1H, s), 6.41 (1H, d, J = 7.3 Hz), 4.28-4.18 (1H, m), 4.04 (1H, t, J = 8.0 Hz), 3.95-3.88 (1H, m), 3.87-3.76 (3H, m), 3.72 (1H, t, J = 7.8 Hz), 3.52-3.37 (2H, m), 3.05-2.96 (1H, m), 2.95-2.77 (3H, m), 2.24-2.10 (2H, m), 1.33-1.25 (9H, m). | 478.3 | 477.25 |
| 1234 | 1H-NMR (CDCl3) δ: 8.99 (1H, s), 8.10 (1H, d, J = 8.7 Hz), 8.06 (1H, s), 7.44 (1H, d, J = 8.7 Hz), 6.67 (1H, s), 6.35 (1H, t, J = 5.5 Hz), 4.79 (1H, q, J = 6.4 Hz), 3.71-3.62 (4H, m), 2.95 (4H, t, J = 4.8 Hz), 2.72 (4H, br s), 2.65 (2H, t, J = 5.5 Hz), 2.48 (3H, s), 1.52 (3H, d, J = 6.4 Hz), 1.36 (3H, t, J = 7.1 Hz). | 453.3 | 452.26 |
| 1235 | 1H-NMR (CDCl3) δ: 8.99 (1H, s), 8.10 (1H, d, J = 8.7 Hz), 8.05 (1H, s), 7.43 (1H, d, J = 8.7 Hz), 6.66 (1H, s), 6.43 (1H, t, J = 5.7 Hz), 4.79 (1H, q, J = 6.4 Hz), 3.68 (2H, t, J = 5.3 Hz), 3.59 (2H, q, J = 6.7 Hz), 2.99-2.93 (4H, m), 2.72 (4H, br s), 2.66 (2H, t, J = 5.3 Hz), 2.48 (3H, s), 1.81-1.73 (2H, m), 1.51 (3H, d, J = 6.4 Hz), 1.24 (1H, s), 1.06 (3H, t, J = 7.3 Hz). | 467.4 | 466.28 |
| 1236 | | 437.2 | 436.23 |
| 1237 | | 451.2 | 450.25 |
| 1238 | | 437.2 | 436.23 |
| 1239 | | 451.2 | 450.25 |

### Example 20

### Human CDK4/cyclin D3 inhibitory activity

Each compound was analyzed for CDK4/cyclin D3 inhibitory activity with an assay kit (QS S Assist CDK4/Cyclin D3_FP Kit, available from Carna Biosciences, Inc.). This assay kit determines kinase activity on the basis of the IMAP technology by Molecular Devices. Specifically, the kinase activity is determined through quantification of a variation in fluorescent polarization caused by binding of a kinase-phosphorylated fluorescent substance to an IMAP-binding reagent.

Each solution was prepared with the 10× assay buffer attached to the kit or a separately prepared assay buffer having the same composition as the assay buffer attached to the kit. An assay buffer was prepared by 10-fold dilution of the 10× assay buffer with distilled water. The assay buffer contains 20mM HEPES (pH 7.4), 0.01% Tween20, and 2mM dithiothreitol. A test compound solution was prepared by dilution of the test compound with dimethyl sulfoxide (DMSO) to a concentration 100 times higher than the final concentration and then 25-fold dilution with the assay buffer to a concentration four times higher than the final concentration. An ATP/substrate/Metal solution was prepared by five-fold dilution of the 5× ATP/substrate/Metal solution attached to the kit with the assay buffer. An enzyme solution was prepared by dilution of the CDK4/cyclin D3 attached to the kit with the assay buffer to a concentration twice higher than the final concentration (final concentration of CDK4/cyclin D3: 12.5 to 25 ng/well). A detection reagent was prepared by five-fold dilution of each of 5× IMAP-binding buffer A and 5× IMAP-binding buffer B with distilled water, mixing of IMAP-binding buffer A with IMAP-binding buffer B at a ratio of 85:15, and 400-fold dilution of the IMAP-binding reagent with the mixed buffer.

The test compound solution (5 µL/well) and the ATP/substrate/Metal solution (5 µL/well) were added to a 384-well plate, and the enzyme solution or the assay buffer (10 µL/well) was added to the plate (total amount of the reaction mixture: 20 µL/well) for initiation of enzymatic reaction. The reaction mixture had a composition of 20mM HEPES (pH 7.4), 0.01% Tween 20, 2mM dithiothreitol, 100nM FITC-labeled peptide substrate (the sequence of the substrate peptide is not disclosed by Carna Biosciences, Inc.), 100µM ATP, 1mM magnesium chloride, 1% DMSO, and 12.5 to 25 ng/well CDK4/cyclin D3. The reaction was performed at room temperature for 45 minutes, and the detection reagent (60 µL/well) was then added to the plate, followed by further reaction for 30 minutes at room temperature under light shielding conditions. Subsequently, fluorescent polarization was determined with a microplate reader at an excitation wavelength of 485 nm and an emission wavelength of 535 nm.

The percent inhibition of enzyme activity was calculated for each test compound (note: enzyme activity = 100% in the case of addition of the enzyme solution and addition of DMSO instead of the test compound solution, whereas enzyme activity = 0% in the case of addition of the assay buffer instead of the enzyme solution, and addition of DMSO instead of the test compound solution). The percent inhibition of enzyme activity was fitted to a dose-response curve, to determine a 50% inhibitory concentration against CDK4/cyclin D3.

The inhibitory activity of each compound against CDK4/cyclin D3 was shown in tables described below.

In each table, "+++" corresponds to IC₅₀ < 10 nM, "++" 10 nM ≤ IC₅₀ < 100 nM, and "+" 100 nM ≤ IC₅₀.

### Example 21

### Human CDK2/cyclin A2 inhibitory activity

Each compound was analyzed for CDK2/cyclin A2 inhibitory activity with an assay kit (QS S Assist CDK2/Cyclin A2_FP Kit, available from Carna Biosciences, Inc.). This assay kit determines kinase activity on the basis of the IMAP technology by Molecular Devices. Specifically, the kinase activity is determined through quantification of a variation in fluorescent polarization caused by binding of a kinase-phosphorylated fluorescent substance to an IMAP-binding reagent.

An assay buffer was prepared by 10-fold dilution of the 10× assay buffer attached to the kit with distilled water, and each solution was prepared with the assay buffer. The assay buffer contained 20mM HEPES (pH 7.4), 0.01% Tween 20, and 2mM dithiothreitol. A test compound solution was prepared by dilution of the test compound with dimethyl sulfoxide (DMSO) to a concentration 100 times higher than the final concentration and then 25-fold dilution with the assay buffer to a concentration four times higher than the final concentration. An ATP/substrate/Metal solution was prepared by five-fold dilution of the 5× ATP/substrate/Metal solution attached to the kit with the assay buffer. An enzyme solution was prepared by dilution of the CDK2/cyclin A2 attached to the kit with the assay buffer to a concentration twice higher than the final concentration (final concentration of CDK2/cyclin A2: 2.5 ng/well). A detection reagent was prepared by five-fold dilution of 5× IMAP-binding buffer A with distilled water and 400-fold dilution of the IMAP-binding reagent with the diluted buffer.

The test compound solution (5 µL/well) and the ATP/substrate/Metal solution (5 µL/well) were added to a 384-well plate, and the enzyme solution or the assay buffer (10 µL/well) was added to the plate (total amount of the reaction mixture: 20 µL/well) for initiation of enzymatic reaction. The reaction mixture had a composition of 20mM HEPES (pH 7.4), 0.01% Tween 20, 2mM dithiothreitol, 100nM FITC-labeled peptide substrate (the sequence of the substrate peptide is not disclosed by Carna Biosciences, Inc.), 30µM ATP, 5mM magnesium chloride, 1% DMSO, and 2.5 ng/well CDK2/cyclin A2. The reaction was performed at room temperature for 60 minutes, and the detection reagent (60 µL/well) was then added to the plate, followed by further reaction for 30 minutes at room temperature under light shielding conditions. Subsequently, fluorescent polarization was determined with a microplate reader at an excitation wavelength of 485 nm and an emission wavelength of 535 nm.

The percent inhibition of enzyme activity was calculated for each test compound (note: enzyme activity = 100% in the case of addition of the enzyme solution and addition of DMSO instead of the test compound solution, whereas enzyme activity = 0% in the case of addition of the assay buffer instead of the enzyme solution and addition of DMSO instead of the test compound solution). The percent inhibition of enzyme activity was fitted to a dose-response curve, to determine a 50% inhibitory concentration against CDK2/cyclin A2.

The inhibitory activity of each compound against CDK2/cyclin A2 was shown in tables described below.

In each table, "+++" corresponds to IC₅₀ < 10 nM, "++" 10 nM ≤ IC₅₀ < 100 nM, and "+" 100 nM ≤ IC₅₀.

**[Table 6-1]**

| Compound No. | CDK4 Activity symbol | CDK2 Activity symbol | | Compound No. | CDK4 Activity symbol | CDK2 Activity symbol |
|---|---|---|---|---|---|---|
| 1 | +++ | ++ | | 2 | +++ | ++ |
| 3 | +++ | ++ | | 4 | +++ | ++ |
| 5 | +++ | + | | 6 | +++ | ++ |
| 7 | +++ | + | | 8 | +++ | + |
| 9 | +++ | + | | 10 | +++ | + |
| 11 | ++ | + | | 12 | +++ | + |
| 13 | +++ | ++ | | 14 | +++ | + |
| 15 | +++ | + | | 16 | +++ | + |
| 17 | +++ | + | | 18 | +++ | + |
| 19 | +++ | + | | 20 | +++ | + |
| 21 | +++ | + | | 22 | ++ | + |
| 23 | +++ | + | | 24 | +++ | + |
| 25 | + | + | | 26 | +++ | + |
| 27 | ++ | + | | 28 | +++ | + |
| 29 | +++ | + | | 30 | +++ | ++ |
| 31 | +++ | + | | 32 | +++ | + |
| 33 | + | + | | 34 | ++ | + |
| 35 | +++ | + | | 36 | + | + |
| 37 | +++ | + | | 38 | +++ | + |
| 39 | +++ | + | | 40 | +++ | + |
| 41 | +++ | + | | 42 | +++ | ++ |
| 43 | +++ | ++ | | 44 | +++ | + |
| 45 | +++ | + | | 46 | +++ | ++ |
| 47 | +++ | ++ | | 48 | +++ | + |
| 49 | +++ | ++ | | 50 | +++ | ++ |
| 51 | ++ | + | | 52 | +++ | ++ |
| 53 | +++ | ++ | | 54 | +++ | + |
| 55 | +++ | ++ | | 56 | +++ | + |
| 57 | +++ | + | | 58 | ++ | + |
| 59 | +++ | + | | 60 | +++ | + |
| 61 | +++ | + | | 62 | +++ | + |
| 63 | +++ | + | | 64 | +++ | + |
| 65 | ++ | + | | 66 | +++ | + |
| 67 | +++ | ++ | | 68 | +++ | + |
| 69 | +++ | + | | 70 | +++ | + |

**[Table 6-2]**

| Compound No. | CDK4 Activity symbol | CDK2 Activity symbol | | Compound No. | CDK4 Activity symbol | CDK2 Activity symbol |
|---|---|---|---|---|---|---|
| 71 | +++ | + | | 72 | +++ | + |
| 73 | +++ | ++ | | 74 | +++ | + |
| 75 | +++ | + | | 76 | +++ | + |
| 77 | +++ | + | | 78 | +++ | + |
| 79 | +++ | + | | 80 | +++ | ++ |
| 81 | +++ | ++ | | 82 | ++ | + |
| 83 | +++ | ++ | | 84 | +++ | ++ |
| 85 | ++ | ++ | | 86 | ++ | + |
| 87 | +++ | +++ | | 88 | + | + |
| 89 | +++ | ++ | | 90 | ++ | ++ |
| 91 | +++ | + | | 92 | +++ | + |
| 93 | +++ | + | | 94 | +++ | + |
| 95 | + | ++ | | 96 | ++ | ++ |
| 97 | ++ | + | | 98 | + | + |
| 99 | + | + | | 100 | ++ | + |
| 101 | +++ | + | | 102 | ++ | + |
| 103 | +++ | + | | 104 | ++ | + |
| 105 | +++ | + | | 106 | +++ | + |
| 107 | ++ | + | | 108 | +++ | ++ |
| 109 | ++ | + | | 110 | + | + |
| 111 | ++ | + | | 112 | ++ | ++ |
| 113 | +++ | + | | 114 | ++ | + |
| 115 | +++ | + | | 116 | ++ | + |
| 117 | +++ | + | | 118 | +++ | ++ |
| 119 | ++ | + | | 120 | ++ | + |
| 121 | +++ | ++ | | 122 | ++ | + |
| 123 | ++ | + | | 124 | ++ | + |
| 125 | +++ | ++ | | 126 | + | + |
| 127 | +++ | ++ | | 128 | +++ | ++ |
| 129 | +++ | ++ | | 130 | +++ | ++ |
| 131 | +++ | ++ | | 132 | +++ | ++ |
| 133 | ++ | + | | 134 | +++ | ++ |
| 135 | +++ | + | | 136 | ++ | + |
| 137 | +++ | ++ | | 138 | +++ | ++ |
| 139 | +++ | ++ | | 140 | +++ | + |

**[Table 6-3]**

| Compound No. | CDK4 Activity symbol | CDK2 Activity symbol | | Compound No. | CDK4 Activity symbol | CDK2 Activity symbol |
|---|---|---|---|---|---|---|
| 141 | +++ | + | | 142 | +++ | ++ |
| 143 | ++ | + | | 144 | +++ | + |
| 145 | +++ | ++ | | 146 | +++ | + |
| 147 | +++ | ++ | | 148 | ++ | + |
| 149 | ++ | + | | 150 | +++ | ++ |
| 151 | +++ | ++ | | 152 | +++ | ++ |
| 153 | +++ | ++ | | 154 | +++ | + |
| 155 | +++ | ++ | | 156 | +++ | + |
| 157 | ++ | + | | 158 | ++ | + |
| 159 | +++ | + | | 160 | +++ | |
| 161 | +++ | ++ | | 162 | ++ | + |
| 163 | +++ | + | | 164 | +++ | ++ |
| 165 | +++ | + | | 166 | +++ | ++ |
| 167 | +++ | + | | 168 | +++ | ++ |
| 169 | +++ | ++ | | 170 | +++ | + |
| 171 | +++ | ++ | | 172 | ++ | + |
| 173 | +++ | ++ | | 174 | +++ | ++ |
| 175 | +++ | ++ | | 176 | ++ | + |
| 177 | +++ | ++ | | 178 | +++ | + |
| 179 | ++ | + | | 180 | ++ | + |
| 181 | +++ | ++ | | 182 | ++ | + |
| 183 | +++ | ++ | | 184 | +++ | ++ |
| 185 | +++ | ++ | | 186 | ++ | + |
| 187 | ++ | + | | 188 | ++ | + |
| 189 | +++ | ++ | | 190 | +++ | ++ |
| 191 | +++ | ++ | | 192 | ++ | + |
| 193 | ++ | + | | 194 | +++ | ++ |
| 195 | +++ | + | | 196 | +++ | + |
| 197 | ++ | + | | 198 | ++ | + |
| 199 | ++ | + | | 200 | +++ | + |
| 201 | ++ | + | | 202 | ++ | + |
| 203 | +++ | ++ | | 204 | +++ | + |
| 205 | +++ | ++ | | 206 | +++ | + |
| 207 | +++ | + | | 208 | +++ | + |
| 209 | ++ | + | | 210 | ++ | + |

**[Table 6-4]**

| Compound No. | CDK4 Activity symbol | CDK2 Activity symbol | | Compound No. | CDK4 Activity symbol | CDK2 Activity symbol |
|---|---|---|---|---|---|---|
| 211 | ++ | ++ | | 212 | +++ | + |
| 213 | +++ | ++ | | 214 | +++ | + |
| 215 | +++ | + | | 216 | ++ | + |
| 217 | +++ | + | | 218 | +++ | ++ |
| 219 | +++ | ++ | | 220 | +++ | + |
| 221 | +++ | + | | 222 | ++ | + |
| 223 | +++ | + | | 224 | +++ | + |
| 225 | +++ | + | | 226 | +++ | ++ |
| 227 | +++ | + | | 228 | ++ | + |
| 229 | +++ | + | | 230 | +++ | + |
| 231 | ++ | + | | 232 | +++ | + |
| 233 | +++ | + | | 234 | +++ | + |
| 235 | ++ | + | | 236 | +++ | ++ |
| 237 | ++ | + | | 238 | +++ | + |
| 239 | +++ | + | | 240 | +++ | + |
| 241 | +++ | + | | 242 | ++ | + |
| 243 | +++ | + | | 244 | +++ | + |
| 245 | +++ | + | | 246 | +++ | + |
| 247 | +++ | + | | 248 | ++ | + |
| 249 | +++ | + | | 250 | +++ | + |
| 251 | +++ | + | | 252 | +++ | + |
| 253 | +++ | + | | 254 | +++ | + |
| 255 | +++ | + | | 256 | +++ | + |
| 257 | +++ | + | | 258 | +++ | + |
| 259 | +++ | + | | 260 | +++ | + |
| 261 | +++ | + | | 262 | ++ | + |
| 263 | +++ | + | | 264 | +++ | + |
| 265 | +++ | + | | 266 | +++ | + |
| 267 | +++ | + | | 268 | ++ | + |
| 269 | +++ | + | | 270 | +++ | + |
| 271 | +++ | + | | 272 | +++ | + |
| 273 | +++ | + | | 274 | +++ | + |
| 275 | ++ | + | | 276 | +++ | + |
| 277 | + | + | | 278 | +++ | + |
| 279 | ++ | + | | 280 | + | + |

**[Table 6-5]**

| Compound No. | CDK4 Activity symbol | CDK2 Activity symbol | | Compound No. | CDK4 Activity symbol | CDK2 Activity symbol |
|---|---|---|---|---|---|---|
| 281 | ++ | + | | 282 | +++ | + |
| 283 | +++ | + | | 284 | +++ | + |
| 285 | +++ | + | | 286 | +++ | + |
| 287 | +++ | + | | 288 | +++ | + |
| 289 | +++ | + | | 290 | +++ | + |
| 291 | +++ | + | | 292 | ++ | + |
| 293 | +++ | + | | 294 | +++ | + |
| 295 | ++ | + | | 296 | + | + |
| 297 | +++ | + | | 298 | ++ | + |
| 299 | ++ | + | | 300 | +++ | + |
| 301 | +++ | + | | 302 | +++ | + |
| 303 | +++ | + | | 304 | +++ | + |
| 305 | +++ | + | | 306 | ++ | + |
| 307 | +++ | + | | 308 | + | + |
| 309 | + | + | | 310 | +++ | + |
| 311 | +++ | + | | 312 | +++ | + |
| 313 | +++ | + | | 314 | +++ | + |
| 315 | + | + | | 316 | + | + |
| 317 | +++ | + | | 318 | +++ | + |
| 319 | +++ | + | | 320 | +++ | + |
| 321 | +++ | + | | 322 | ++ | + |
| 323 | +++ | + | | 324 | + | + |
| 325 | ++ | + | | 326 | +++ | + |
| 327 | ++ | + | | 328 | +++ | + |
| 329 | +++ | + | | 330 | +++ | + |
| 331 | + | + | | 332 | +++ | + |
| 333 | +++ | + | | 334 | ++ | + |
| 335 | +++ | + | | 336 | +++ | + |
| 337 | +++ | + | | 338 | +++ | + |
| 339 | ++ | + | | 340 | +++ | + |
| 341 | +++ | + | | 342 | +++ | + |
| 343 | +++ | + | | 344 | +++ | + |
| 345 | +++ | + | | 346 | +++ | + |
| 347 | +++ | + | | 348 | +++ | + |
| 349 | ++ | + | | 350 | +++ | + |

**[Table 6-6]**

| Compound No. | CDK4 Activity symbol | CDK2 Activity symbol | | Compound No. | CDK4 Activity symbol | CDK2 Activity symbol |
|---|---|---|---|---|---|---|
| 351 | +++ | + | | 352 | +++ | + |
| 353 | +++ | + | | 354 | ++ | + |
| 355 | +++ | + | | 356 | ++ | |
| 357 | +++ | | | 358 | +++ | + |
| 359 | ++ | | | 360 | +++ | + |
| 361 | +++ | | | 362 | ++ | |
| 363 | +++ | + | | 364 | +++ | ++ |
| 365 | +++ | + | | 366 | +++ | + |
| 367 | +++ | + | | 368 | +++ | + |
| 369 | +++ | + | | 370 | +++ | + |
| 371 | +++ | + | | 372 | +++ | + |
| 373 | +++ | + | | 374 | ++ | |
| 375 | + | | | 376 | +++ | + |
| 377 | ++ | | | 378 | +++ | + |
| 379 | +++ | + | | 380 | +++ | + |
| 381 | +++ | + | | 382 | +++ | + |
| 383 | ++ | + | | 384 | ++ | + |
| 385 | +++ | + | | 386 | +++ | + |
| 387 | +++ | ++ | | 388 | +++ | + |
| 389 | +++ | ++ | | 390 | +++ | |
| 391 | ++ | | | 392 | +++ | |
| 393 | ++ | | | 394 | ++ | |
| 395 | +++ | + | | 396 | + | |
| 397 | +++ | | | 398 | +++ | |
| 399 | ++ | | | 400 | +++ | + |
| 401 | ++ | | | 402 | +++ | |
| 403 | ++ | | | 404 | +++ | |
| 405 | ++ | | | 406 | ++ | |
| 407 | ++ | | | 408 | ++ | |
| 409 | +++ | | | 410 | +++ | |
| 411 | +++ | | | 412 | +++ | |
| 413 | +++ | | | 414 | ++ | |
| 415 | +++ | | | 416 | ++ | |
| 417 | +++ | | | 418 | +++ | |
| 419 | ++ | | | 420 | +++ | |

**[Table 6-7]**

| Compound No. | CDK4 Activity symbol | CDK2 Activity symbol | | Compound No. | CDK4 Activity symbol | CDK2 Activity symbol |
|---|---|---|---|---|---|---|
| 421 | ++ | | | 422 | ++ | |
| 423 | +++ | | | 424 | +++ | |
| 425 | +++ | | | 426 | +++ | |
| 427 | +++ | | | 428 | +++ | |
| 429 | +++ | | | 430 | +++ | |
| 431 | +++ | | | 432 | +++ | + |
| 433 | ++ | | | 434 | +++ | |
| 435 | ++ | | | 436 | ++ | |
| 437 | + | | | 438 | +++ | |
| 439 | ++ | | | 440 | ++ | |
| 441 | + | | | 442 | + | |
| 443 | ++ | | | 444 | + | |
| 445 | +++ | | | 446 | ++ | |
| 447 | ++ | | | 448 | + | |
| 449 | + | | | 450 | +++ | |
| 451 | +++ | | | 452 | +++ | |
| 453 | +++ | | | 454 | +++ | |
| 455 | +++ | | | 456 | +++ | + |
| 457 | +++ | | | 458 | +++ | |
| 459 | +++ | | | 460 | +++ | |
| 461 | ++ | | | 462 | ++ | |
| 463 | +++ | + | | 464 | +++ | |
| 465 | +++ | | | 466 | +++ | + |
| 467 | +++ | + | | 468 | +++ | |
| 469 | ++ | | | 470 | + | |
| 471 | ++ | | | 472 | ++ | |
| 473 | ++ | | | 474 | +++ | + |
| 475 | ++ | | | 476 | +++ | |
| 477 | +++ | | | 478 | +++ | |
| 479 | +++ | | | 480 | ++ | |
| 481 | ++ | | | 482 | +++ | |
| 483 | +++ | | | 484 | +++ | |
| 485 | +++ | | | 486 | +++ | |
| 487 | +++ | | | 488 | +++ | |
| 489 | +++ | | | 490 | +++ | |

**[Table 6-8]**

| Compound No. | CDK4 Activity symbol | CDK2 Activity symbol | | Compound No. | CDK4 Activity symbol | CDK2 Activity symbol |
|---|---|---|---|---|---|---|
| 491 | +++ | + | | 492 | +++ | ++ |
| 493 | +++ | | | 494 | +++ | |
| 495 | +++ | | | 496 | +++ | |
| 497 | +++ | + | | 498 | +++ | |
| 499 | +++ | ++ | | 500 | +++ | |
| 501 | +++ | + | | 502 | +++ | |
| 503 | +++ | | | 504 | +++ | |
| 505 | ++ | | | 506 | ++ | |
| 507 | +++ | + | | 508 | +++ | + |
| 509 | +++ | + | | 510 | +++ | + |
| 511 | +++ | ++ | | 512 | +++ | + |
| 513 | ++ | | | 514 | ++ | |
| 515 | ++ | | | 516 | ++ | |
| 517 | +++ | | | 518 | +++ | |
| 519 | +++ | + | | 520 | +++ | |
| 521 | +++ | ++ | | 522 | +++ | ++ |
| 523 | +++ | | | 524 | +++ | + |
| 525 | +++ | | | 526 | +++ | + |
| 527 | ++ | | | 528 | +++ | |
| 529 | +++ | + | | 530 | +++ | |
| 531 | +++ | | | 532 | +++ | + |
| 533 | +++ | | | 534 | +++ | |
| 535 | +++ | | | 536 | +++ | |
| 537 | +++ | + | | 538 | +++ | |
| 539 | ++ | | | 540 | +++ | |
| 541 | +++ | | | 542 | ++ | |
| 543 | +++ | | | 544 | +++ | |
| 545 | +++ | | | 546 | +++ | |
| 547 | +++ | | | 548 | +++ | + |
| 549 | +++ | + | | 550 | +++ | + |
| 551 | ++ | | | 552 | +++ | |
| 553 | +++ | + | | 554 | +++ | + |
| 555 | +++ | + | | 556 | +++ | ++ |
| 557 | +++ | + | | 558 | +++ | ++ |
| 559 | +++ | + | | 560 | +++ | + |

**[Table 6-9]**

| Compound No. | CDK4 Activity symbol | CDK2 Activity symbol | | Compound No. | CDK4 Activity symbol | CDK2 Activity symbol |
|---|---|---|---|---|---|---|
| 561 | +++ | + | | 562 | +++ | + |
| 563 | +++ | | | 564 | +++ | |
| 565 | +++ | | | 566 | +++ | + |
| 567 | +++ | + | | 568 | +++ | |
| 569 | +++ | + | | 570 | +++ | |
| 571 | +++ | | | 572 | +++ | + |
| 573 | +++ | + | | 574 | +++ | + |
| 575 | +++ | + | | 576 | +++ | + |
| 577 | +++ | | | 578 | +++ | |
| 579 | +++ | + | | 580 | ++ | |
| 581 | +++ | + | | 582 | +++ | |
| 583 | +++ | + | | 584 | +++ | + |
| 585 | +++ | + | | 586 | +++ | + |
| 587 | +++ | | | 588 | +++ | + |
| 589 | +++ | + | | 590 | +++ | + |
| 591 | +++ | + | | 592 | +++ | + |
| 593 | +++ | + | | 594 | +++ | |
| 595 | +++ | + | | 596 | +++ | + |
| 597 | +++ | + | | 598 | +++ | |
| 599 | ++ | | | 600 | +++ | + |
| 601 | +++ | + | | 602 | +++ | + |
| 603 | +++ | | | 604 | +++ | + |
| 605 | +++ | ++ | | 606 | +++ | + |
| 607 | +++ | | | 608 | +++ | + |
| 609 | +++ | + | | 610 | +++ | + |
| 611 | +++ | + | | 612 | +++ | ++ |
| 613 | ++ | | | 614 | +++ | + |
| 615 | +++ | + | | 616 | +++ | |
| 617 | +++ | | | 618 | +++ | + |
| 619 | +++ | + | | 620 | +++ | |
| 621 | +++ | + | | 622 | +++ | + |
| 623 | +++ | ++ | | 624 | +++ | + |
| 625 | +++ | + | | 626 | +++ | + |
| 627 | +++ | + | | 628 | +++ | + |
| 629 | +++ | + | | 630 | +++ | + |

**[Table 6-10]**

| Compound No. | CDK4 Activity symbol | CDK2 Activity symbol | | Compound No. | CDK4 Activity symbol | CDK2 Activity symbol |
|---|---|---|---|---|---|---|
| 631 | +++ | ++ | | 632 | +++ | + |
| 633 | +++ | + | | 634 | +++ | + |
| 635 | +++ | + | | 636 | +++ | + |
| 637 | +++ | + | | 638 | +++ | + |
| 639 | +++ | + | | 640 | +++ | + |
| 641 | +++ | + | | 642 | +++ | + |
| 643 | +++ | + | | 644 | +++ | + |
| 645 | +++ | + | | 646 | +++ | + |
| 647 | +++ | + | | 648 | +++ | + |
| 649 | +++ | + | | 650 | +++ | + |
| 651 | +++ | + | | 652 | +++ | + |
| 653 | +++ | + | | 654 | +++ | + |
| 655 | ++ | + | | 656 | +++ | + |
| 657 | +++ | + | | 658 | +++ | + |
| 659 | +++ | + | | 660 | +++ | + |
| 661 | +++ | + | | 662 | +++ | + |
| 663 | +++ | + | | 664 | +++ | + |
| 665 | +++ | + | | 666 | +++ | + |
| 667 | +++ | + | | 668 | +++ | + |
| 669 | ++ | + | | 670 | + | + |
| 672 | +++ | + | | 673 | +++ | + |
| 674 | +++ | + | | 675 | +++ | + |
| 676 | +++ | + | | 677 | +++ | + |
| 678 | ++ | + | | 679 | ++ | + |
| 680 | ++ | + | | 681 | ++ | + |
| 682 | +++ | + | | 683 | +++ | + |
| 684 | ++ | | | 685 | ++ | |
| 686 | +++ | + | | 687 | +++ | |
| 688 | +++ | | | 689 | +++ | |
| 690 | +++ | | | 691 | +++ | + |
| 692 | +++ | + | | 693 | +++ | + |
| 694 | ++ | + | | 695 | +++ | + |
| 696 | +++ | + | | 697 | ++ | |
| 698 | +++ | + | | 699 | ++ | |
| 700 | +++ | + | | 701 | +++ | + |

**[Table 6-11]**

| Compound No. | CDK4 Activity symbol | CDK2 Activity symbol | | Compound No. | CDK4 Activity symbol | CDK2 Activity symbol |
|---|---|---|---|---|---|---|
| 702 | ++ | + | | 703 | ++ | + |
| 704 | +++ | | | 705 | +++ | |
| 706 | +++ | + | | 707 | +++ | |
| 708 | +++ | + | | 709 | +++ | + |
| 710 | +++ | + | | 711 | +++ | + |
| 712 | +++ | + | | 713 | +++ | + |
| 714 | + | + | | 715 | ++ | + |
| 716 | ++ | + | | 717 | ++ | + |
| 718 | ++ | + | | 719 | +++ | + |
| 720 | +++ | + | | 721 | +++ | + |
| 722 | +++ | + | | 723 | +++ | + |
| 724 | +++ | + | | 725 | +++ | + |
| 726 | +++ | + | | 727 | +++ | + |
| 728 | +++ | + | | 729 | +++ | + |
| 730 | ++ | + | | 731 | +++ | + |
| 732 | +++ | + | | 733 | ++ | + |
| 734 | +++ | + | | 735 | +++ | + |
| 736 | +++ | + | | 737 | +++ | + |
| 738 | +++ | + | | 739 | +++ | + |
| 740 | +++ | + | | 741 | +++ | + |
| 742 | +++ | + | | 743 | +++ | + |
| 744 | +++ | + | | 745 | +++ | + |
| 746 | +++ | + | | 747 | ++ | + |
| 748 | ++ | + | | 749 | +++ | + |
| 750 | +++ | + | | 751 | +++ | + |
| 752 | ++ | + | | 753 | +++ | + |
| 754 | ++ | + | | 755 | +++ | + |
| 756 | ++ | + | | 757 | +++ | + |
| 758 | ++ | + | | 759 | ++ | + |
| 760 | ++ | + | | 761 | +++ | + |
| 762 | +++ | + | | 763 | +++ | + |
| 764 | +++ | + | | 765 | +++ | + |
| 766 | ++ | + | | 767 | +++ | + |
| 768 | +++ | + | | 769 | +++ | + |
| 770 | ++ | + | | 771 | +++ | + |

**[Table 6-12]**

| Compound No. | CDK4 Activity symbol | CDK2 Activity symbol | | Compound No. | CDK4 Activity symbol | CDK2 Activity symbol |
|---|---|---|---|---|---|---|
| 772 | +++ | + | | 773 | +++ | + |
| 774 | ++ | + | | 775 | +++ | + |
| 776 | +++ | + | | 777 | +++ | + |
| 778 | +++ | + | | 779 | +++ | + |
| 780 | ++ | + | | 781 | +++ | + |
| 782 | +++ | + | | 783 | +++ | + |
| 784 | +++ | + | | 785 | +++ | + |
| 786 | +++ | + | | 787 | +++ | + |
| 788 | +++ | + | | 789 | +++ | + |
| 790 | +++ | + | | 791 | +++ | ++ |
| 792 | ++ | + | | 793 | ++ | + |
| 794 | +++ | + | | 795 | +++ | ++ |
| 796 | +++ | + | | 797 | ++ | + |
| 798 | +++ | + | | 799 | +++ | + |
| 800 | +++ | + | | 801 | +++ | + |
| 802 | +++ | + | | 803 | +++ | + |
| 804 | +++ | + | | 805 | +++ | + |
| 806 | +++ | + | | 807 | ++ | + |
| 808 | +++ | + | | 809 | +++ | ++ |
| 810 | +++ | + | | 811 | +++ | ++ |
| 812 | ++ | + | | 813 | +++ | + |
| 814 | +++ | + | | 815 | +++ | + |
| 816 | +++ | + | | 817 | +++ | + |
| 818 | +++ | + | | 819 | +++ | + |
| 820 | +++ | ++ | | 821 | +++ | ++ |
| 822 | +++ | ++ | | 823 | ++ | + |
| 824 | +++ | + | | 825 | +++ | + |
| 826 | +++ | + | | 827 | +++ | + |
| 828 | ++ | + | | 829 | +++ | + |
| 830 | +++ | + | | 831 | ++ | + |
| 832 | ++ | + | | 833 | +++ | + |
| 834 | +++ | + | | 835 | ++ | + |
| 836 | ++ | + | | 837 | +++ | ++ |
| 838 | +++ | + | | 839 | +++ | + |

**[Table 6-13]**

| Compound No. | CDK4 Activity symbol | CDK2 Activity symbol | | Compound No. | CDK4 Activity symbol | CDK2 Activity symbol |
|---|---|---|---|---|---|---|
| 840 | + | + | | 841 | +++ | + |
| 842 | ++ | + | | 843 | +++ | + |
| 844 | +++ | + | | 845 | +++ | + |
| 846 | +++ | + | | 847 | +++ | + |
| 848 | +++ | ++ | | 849 | +++ | ++ |
| 850 | ++ | ++ | | 851 | ++ | + |
| 852 | +++ | + | | 853 | +++ | + |
| 854 | +++ | + | | 855 | +++ | + |
| 856 | +++ | + | | 857 | +++ | + |
| 858 | +++ | + | | 859 | +++ | + |
| 860 | +++ | + | | 861 | +++ | + |
| 862 | +++ | + | | 863 | +++ | + |
| 864 | +++ | + | | 865 | +++ | + |
| 866 | +++ | + | | 867 | +++ | + |
| 868 | +++ | + | | 869 | +++ | ++ |
| 870 | +++ | + | | 871 | ++ | + |
| 872 | ++ | + | | 873 | +++ | + |
| 874 | +++ | + | | 875 | +++ | + |
| 876 | +++ | + | | 877 | +++ | + |
| 878 | +++ | + | | 879 | +++ | + |
| 880 | +++ | + | | 881 | +++ | + |
| 882 | +++ | + | | 883 | +++ | + |
| 884 | +++ | + | | 885 | +++ | + |
| 886 | +++ | + | | 887 | +++ | + |
| 888 | +++ | + | | 889 | ++ | + |
| 890 | ++ | + | | 891 | ++ | + |
| 892 | ++ | + | | 893 | ++ | + |
| 894 | ++ | + | | 895 | +++ | + |
| 896 | +++ | + | | 897 | ++ | + |
| 898 | +++ | + | | 899 | +++ | + |
| 900 | +++ | + | | 901 | +++ | + |
| 902 | +++ | + | | 903 | +++ | ++ |
| 904 | +++ | + | | 905 | +++ | + |
| 906 | ++ | + | | 907 | +++ | + |

**[Table 6-14]**

| Compound No. | CDK4 Activity symbol | CDK2 Activity symbol | | Compound No. | CDK4 Activity symbol | CDK2 Activity symbol |
|---|---|---|---|---|---|---|
| 908 | +++ | + | | 909 | +++ | + |
| 910 | ++ | + | | 911 | +++ | + |
| 912 | +++ | + | | 913 | +++ | + |
| 914 | +++ | + | | 915 | +++ | + |
| 916 | +++ | + | | 917 | +++ | + |
| 918 | +++ | + | | 919 | +++ | + |
| 920 | +++ | + | | 921 | +++ | + |
| 922 | +++ | + | | 923 | +++ | + |
| 924 | +++ | + | | 925 | +++ | + |
| 926 | +++ | + | | 927 | +++ | + |
| 928 | +++ | + | | 929 | +++ | + |
| 930 | +++ | + | | 931 | +++ | + |
| 932 | +++ | + | | 933 | +++ | + |
| 934 | +++ | + | | 935 | ++ | + |
| 936 | +++ | + | | 937 | +++ | + |
| 938 | +++ | + | | 939 | +++ | + |
| 940 | +++ | + | | 941 | +++ | + |
| 942 | +++ | + | | 943 | ++ | + |
| 944 | +++ | + | | 945 | +++ | + |
| 946 | +++ | + | | 947 | +++ | + |
| 948 | +++ | + | | 949 | +++ | + |
| 950 | +++ | + | | 951 | +++ | + |
| 952 | +++ | + | | 953 | +++ | + |
| 954 | +++ | + | | 955 | +++ | + |
| 956 | +++ | + | | 957 | +++ | + |
| 958 | +++ | + | | 959 | ++ | + |
| 960 | +++ | + | | 961 | +++ | + |
| 962 | +++ | + | | 963 | +++ | + |
| 964 | +++ | + | | 965 | ++ | + |
| 966 | +++ | + | | 967 | +++ | + |
| 968 | +++ | + | | 969 | ++ | + |
| 970 | +++ | + | | 971 | +++ | + |
| 972 | +++ | + | | 973 | ++ | + |
| 974 | ++ | + | | 975 | ++ | + |

**[Table 6-15]**

| Compound No. | CDK4 Activity symbol | CDK2 Activity symbol | | Compound No. | CDK4 Activity symbol | CDK2 Activity symbol |
|---|---|---|---|---|---|---|
| 976 | +++ | + | | 977 | +++ | + |
| 978 | +++ | + | | 979 | +++ | + |
| 980 | +++ | + | | 981 | +++ | ++ |
| 982 | +++ | + | | 983 | +++ | + |
| 984 | +++ | + | | 985 | +++ | + |
| 986 | +++ | + | | 987 | +++ | + |
| 988 | +++ | + | | 989 | +++ | + |
| 990 | +++ | + | | 991 | +++ | + |
| 992 | ++ | + | | 993 | +++ | + |
| 994 | +++ | + | | 995 | +++ | + |
| 996 | +++ | + | | 997 | +++ | + |
| 998 | +++ | + | | 999 | +++ | + |
| 1000 | ++ | + | | 1001 | +++ | + |
| 1002 | +++ | + | | 1003 | +++ | + |
| 1004 | +++ | + | | 1005 | +++ | + |
| 1006 | +++ | + | | 1007 | +++ | + |
| 1008 | +++ | + | | 1009 | +++ | + |
| 1010 | +++ | + | | 1011 | +++ | + |
| 1012 | +++ | + | | 1013 | +++ | + |
| 1014 | +++ | + | | 1015 | +++ | + |
| 1016 | +++ | + | | 1017 | +++ | + |
| 1018 | +++ | + | | 1019 | +++ | + |
| 1020 | +++ | + | | 1021 | +++ | + |
| 1022 | +++ | + | | 1023 | +++ | + |
| 1024 | +++ | + | | 1025 | + | + |
| 1026 | ++ | + | | 1027 | +++ | + |
| 1028 | ++ | + | | 1029 | +++ | + |
| 1030 | ++ | + | | 1031 | +++ | + |
| 1032 | +++ | + | | 1033 | +++ | + |
| 1034 | +++ | + | | 1035 | +++ | + |
| 1036 | +++ | + | | 1037 | ++ | + |
| 1038 | +++ | + | | 1039 | +++ | + |
| 1040 | +++ | + | | 1041 | +++ | + |
| 1042 | ++ | + | | 1043 | +++ | + |

**[Table 6-16]**

| Compound No. | CDK4 Activity symbol | CDK2 Activity symbol | | Compound No. | CDK4 Activity symbol | CDK2 Activity symbol |
|---|---|---|---|---|---|---|
| 1044 | ++ | + | | 1045 | +++ | + |
| 1046 | +++ | + | | 1047 | +++ | + |
| 1048 | +++ | + | | 1049 | +++ | + |
| 1050 | +++ | + | | 1051 | +++ | + |
| 1052 | ++ | + | | 1053 | ++ | + |
| 1054 | ++ | + | | 1055 | ++ | + |
| 1056 | ++ | + | | 1057 | +++ | + |
| 1058 | +++ | + | | 1059 | +++ | + |
| 1060 | +++ | + | | 1061 | +++ | + |
| 1062 | +++ | + | | 1063 | +++ | + |
| 1064 | +++ | + | | 1065 | +++ | + |
| 1066 | ++ | + | | 1067 | +++ | + |
| 1068 | + | + | | 1069 | ++ | + |
| 1070 | +++ | + | | 1071 | +++ | + |
| 1072 | +++ | + | | 1073 | +++ | + |
| 1074 | +++ | + | | 1075 | +++ | + |
| 1076 | +++ | + | | 1077 | +++ | + |
| 1078 | +++ | + | | 1079 | +++ | + |
| 1080 | +++ | + | | 1081 | +++ | + |
| 1082 | +++ | + | | 1083 | +++ | + |
| 1084 | +++ | + | | 1085 | +++ | + |
| 1086 | +++ | + | | 1087 | +++ | + |
| 1088 | +++ | + | | 1089 | +++ | + |
| 1090 | +++ | + | | 1091 | +++ | + |
| 1092 | +++ | + | | 1093 | +++ | + |
| 1094 | +++ | + | | 1095 | +++ | + |
| 1096 | ++ | + | | 1097 | ++ | + |
| 1098 | ++ | + | | 1099 | +++ | + |
| 1100 | ++ | + | | 1101 | +++ | + |
| 1102 | +++ | + | | 1103 | +++ | + |
| 1104 | +++ | + | | 1105 | +++ | + |
| 1106 | +++ | + | | 1107 | +++ | + |
| 1108 | +++ | + | | 1109 | +++ | + |
| 1110 | +++ | + | | 1111 | ++ | + |

**[Table 6-17]**

| Compound No. | CDK4 Activity symbol | CDK2 Activity symbol | | Compound No. | CDK4 Activity symbol | CDK2 Activity symbol |
|---|---|---|---|---|---|---|
| 1112 | ++ | + | | 1113 | ++ | + |
| 1114 | +++ | + | | 1115 | +++ | + |
| 1116 | +++ | + | | 1117 | ++ | + |
| 1118 | +++ | + | | 1119 | +++ | + |
| 1120 | ++ | + | | 1121 | +++ | + |
| 1122 | +++ | + | | 1123 | ++ | + |
| 1124 | ++ | + | | 1125 | +++ | + |
| 1126 | +++ | + | | 1127 | +++ | + |
| 1128 | +++ | + | | 1129 | +++ | + |
| 1130 | ++ | + | | 1131 | ++ | + |
| 1132 | +++ | + | | 1133 | +++ | + |
| 1134 | +++ | + | | 1135 | +++ | + |
| 1136 | +++ | + | | 1137 | +++ | + |
| 1138 | +++ | + | | 1139 | +++ | + |
| 1140 | +++ | + | | 1141 | +++ | + |
| 1142 | +++ | + | | 1143 | +++ | + |
| 1144 | +++ | + | | 1145 | +++ | + |
| 1146 | +++ | + | | 1147 | +++ | + |
| 1148 | +++ | + | | 1149 | +++ | + |
| 1150 | +++ | + | | 1151 | +++ | + |
| 1152 | +++ | + | | 1153 | +++ | + |
| 1154 | +++ | + | | 1155 | +++ | + |
| 1156 | +++ | + | | 1157 | ++ | + |
| 1158 | +++ | + | | 1159 | +++ | + |
| 1160 | +++ | ++ | | 1161 | +++ | ++ |
| 1162 | +++ | ++ | | 1163 | +++ | ++ |
| 1164 | +++ | + | | 1165 | +++ | ++ |
| 1166 | +++ | + | | 1167 | +++ | ++ |
| 1168 | +++ | + | | 1169 | +++ | + |
| 1170 | +++ | + | | 1171 | +++ | + |
| 1172 | +++ | + | | 1173 | +++ | + |
| 1174 | +++ | + | | 1175 | +++ | + |
| 1176 | +++ | + | | 1177 | +++ | + |
| 1178 | +++ | + | | 1179 | +++ | + |

**[Table 6-18]**

| Compound No. | CDK4 Activity symbol | CDK2 Activity symbol | | Compound No. | CDK4 Activity symbol | CDK2 Activity symbol |
|---|---|---|---|---|---|---|
| 1180 | +++ | + | | 1181 | +++ | + |
| 1182 | +++ | + | | 1183 | +++ | + |
| 1184 | +++ | + | | 1185 | +++ | + |
| 1186 | +++ | + | | 1187 | +++ | + |
| 1188 | +++ | + | | 1189 | +++ | + |
| 1190 | +++ | + | | 1191 | +++ | + |
| 1192 | +++ | + | | 1193 | +++ | + |
| 1194 | +++ | + | | 1195 | +++ | + |
| 1196 | +++ | + | | 1197 | +++ | + |
| 1198 | +++ | + | | 1199 | +++ | + |
| 1200 | +++ | + | | 1201 | +++ | + |
| 1202 | +++ | + | | 1203 | ++ | + |
| 1204 | +++ | + | | 1205 | +++ | + |
| 1206 | +++ | + | | 1207 | +++ | + |
| 1208 | +++ | + | | 1209 | +++ | + |
| 1210 | +++ | + | | 1211 | ++ | + |
| 1212 | +++ | + | | 1213 | +++ | + |
| 1214 | +++ | + | | 1215 | +++ | + |
| 1216 | +++ | + | | 1217 | ++ | + |
| 1218 | +++ | + | | 1219 | +++ | + |
| 1220 | +++ | + | | 1221 | +++ | + |
| 1222 | +++ | + | | 1223 | +++ | + |
| 1224 | +++ | + | | 1225 | +++ | + |
| 1226 | +++ | + | | 1227 | +++ | + |
| 1228 | +++ | + | | 1229 | +++ | + |
| 1230 | +++ | + | | 1231 | +++ | + |
| 1232 | +++ | + | | | | |

### Example 22

### Human CDK6/cyclin D3 inhibitory activity

CDK6/cyclin D3 inhibitory activity was determined by the off-chip mobility shift assay (MSA). The MSA separates proteins from one another on the basis of a difference in electrophoretic mobility depending on the molecular weight or electric charge of the proteins. The kinase activity is determined by quantifying the degree of phosphorylation through electrophoretic analysis of a positive to negative change in electric charge of the substrate phosphorylated by the kinase.

Each solution was prepared with an assay buffer containing 20mM HEPES (pH 7.5), 0.01% Triton X-100, and 2mM dithiothreitol. A test compound solution was prepared by dilution of the test compound with dimethyl sulfoxide (DMSO) to a concentration 100 times higher than the final concentration and then 25-fold dilution with the assay buffer to a concentration four times higher than the final concentration. An ATP/substrate/metal solution was prepared to have a concentration four times higher than the final concentration. An enzyme solution was prepared to have a concentration twice higher than the final concentration. The final enzyme concentration was adjusted to an appropriate level on the basis of the enzyme activity signal and the inhibitory activity of a positive control compound.

The test compound solution (5 µL/well) and the ATP/substrate/metal solution (5 µL/well) were added to a 384-well plate, and the enzyme solution or the assay buffer (10 µL/well) was added to the plate (total amount of the reaction mixture: 20 µL/well) for initiation of enzymatic reaction. The reaction mixture had a composition of 20mM HEPES (pH 7.5), 0.01% Triton X-100, 2mM dithiothreitol, 1000nM peptide substrate (DYRKtide-F), 300µM ATP, 5mM magnesium chloride, 1% DMSO, and a predetermined concentration of CDK6/cyclin D3. The reaction was performed at room temperature for five hours, and a termination buffer (QuickScout Screening Assist MSA, manufactured by Carna Biosciences, Inc.) (60 µL/well) was then added to the plate for termination of the reaction. Subsequently, the substrate peptide and the phosphorylated peptide in the reaction mixture were separated from each other and quantified with LabChip 3000 (manufactured by Caliper Lifesciences). The kinase reaction was evaluated by the product ratio (P/(P+S)) calculated from the peak height (S) of the substrate peptide and the peak height (P) of the phosphorylated peptide.

The percent inhibition of enzyme activity was calculated for each test compound (note: enzyme activity = 100% in the case of addition of the enzyme solution and addition of DMSO instead of the test compound solution, whereas enzyme activity = 0% in the case of addition of the assay buffer instead of the enzyme solution and addition of DMSO instead of the test compound solution). The percent inhibition of enzyme activity was fitted to a dose-response curve, to determine a 50% inhibitory concentration against CDK6/cyclin D3.

The inhibitory activity of each compound against CDK6/cyclin D3 was shown in tables described below. In each table, "+++" corresponds to IC₅₀ < 10 nM, "++" 10 nM ≤ IC₅₀ < 100 nM, and "+" 100 nM ≤ IC₅₀.

**[Table 7]**

| Compound No. | CDK6 Activity Symbol | | Compound No. | CDK6 Activity Symbol |
|---|---|---|---|---|
| 3 | +++ | | 16 | +++ |
| 217 | ++ | | 220 | ++ |
| 221 | ++ | | 230 | +++ |
| 300 | ++ | | 304 | ++ |
| 363 | ++ | | 672 | ++ |
| 749 | ++ | | 778 | ++ |
| 779 | +++ | | 818 | ++ |
| 819 | ++ | | 843 | ++ |
| 844 | +++ | | 879 | +++ |
| 883 | +++ | | 904 | +++ |
| 929 | +++ | | 938 | ++ |
| 941 | +++ | | 954 | +++ |
| 1036 | ++ | | | |

### Example 23

A monoclonal antibody cocktail against type II collagen (Arthritogenic MoAb Cocktail (Chondrex #53100), 4.8 mg/mL) was intraperitoneally administered (250 µL/head) to a group of mice with collagen antibodyinduced arthritis (CAIA) (vehicle/+, group of drug administration) (day 1). LPS (LPS Solution (E. coli 0111:B4) (Chondrex #9028), 0.5 mg/mL) was intraperitoneally administered (100 µL/head) on day 4, to induce the disease. The drug was evaluated on the basis of pathological scoring until day 9. The drug was orally administered consecutively from day 4 to day 8 once a day.

In mice of a non-disease-induced group (vehicle/-group), PBS (pH 7.2, Gibco #20012-027) was intraperitoneally administered (250 µL/head) on day 1, and LPS was intraperitoneally administered on day 4.

The drug was evaluated on the basis of the pathological scoring (score 0 to score 4 for each of the extremities, evaluated by the total score). Scoring criteria are as follows:
score 0: no change;
score 1: swelling of only one limb;
score 2: swelling of wrist and ankle or swelling of two or more limbs;
score 3: swelling of wrist and ankle and swelling of one or more limbs; and
score 4: swelling of wrist and ankle and swelling of all the limbs.

Fig. 1 shows the results (scores) on day 9 (final day) .

## Claims

1. A compound represented by Formula (I) or a pharmaceutically acceptable salt thereof: 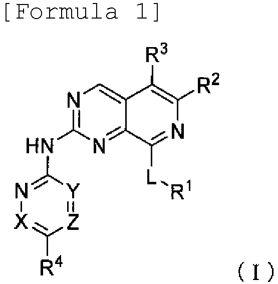 [where
L represents -NR⁵-, -O-, or -S-;
R⁵ represents a hydrogen atom or a C₁₋₆ alkyl group substituted with zero to two -OH groups, zero to two C₁₋₈ alkoxy groups, and zero to six fluorine atoms;
R¹ represents a C₁₋₈ alkyl, C₃₋₁₂ cycloalkyl, (C₃₋₁₂ cycloalkyl)-C₁₋₆ alkyl, 4- to 12-membered heterocyclyl, (4- to 12-membered heterocyclyl) -C₁₋₆ alkyl, C₆₋₁₀ aryl, (C₆₋₁₀ aryl)-C₁₋₆ alkyl, 5- to 10-membered heteroaryl, (5-to 10-membered heteroaryl) -C₁₋₆ alkyl, C₁₋₈ alkylsulfonyl, or C₁₋₈ acyl group;
each of the heteroatom-containing groups represented by R¹ contains one to four heteroatoms selected from oxygen, sulfur, and nitrogen atoms;
R¹ is optionally substituted with one to six substituents selected from the group consisting of a halogen atom, =O, -OH, -CN, -COOH, -COOR⁶, -R⁷, a C₃₋₆ cycloalkyl group substituted with zero to two -OH groups, zero to two C₁₋₈ alkoxy groups, and zero to six fluorine atoms, a 3- to 10-membered heterocyclyl group substituted with zero to two -OH groups, zero to two C₁₋₈ alkoxy groups, and zero to six fluorine atoms, a C₁₋₈ acyl group substituted with zero to two -OH groups, zero to two C₁₋₈ alkoxy groups, and zero to six fluorine atoms, and a C₁₋₈ alkoxy group substituted with zero to two -OH groups, zero to two C₁₋₈ alkoxy groups, and zero to six fluorine atoms;
R⁶ and R⁷ each independently represent a C₁₋₆ alkyl group substituted with zero to two -OH groups, zero to two C₁₋₈ alkoxy groups, and zero to six fluorine atoms;
R² represents a C₁₋₈ alkyl, C₃₋₈ cycloalkyl, 4- to 6-membered heterocyclyl, C₁₋₈ acyl group, -COOR⁸, or -CONR⁹R¹⁰;
each of the C₁₋₈ alkyl and C₃₋₈ cycloalkyl groups represented by R² is substituted with zero or one -OH group, zero to two C₁₋₈ alkoxy groups substituted with zero or one -OH group, zero or one C₁₋₄ alkoxy group, and zero to three fluorine atoms, and zero to five fluorine atoms;
R² is neither an unsubstituted C₁₋₈ alkyl, nor unsubstituted C₃₋₈ cycloalkyl, nor trifluoromethyl group; R⁸, R⁹, and R¹⁰ each independently represent a hydrogen atom or a C₁₋₈ alkyl group;
the 4- to 6-membered heterocyclyl group represented by R² is optionally substituted with one to four substituents selected from the group consisting of a fluorine atom, - OH, and C₁₋₄ alkyl and C₁₋₄ alkoxy groups;
each of the C₁₋₈ acyl group, -COOR⁸, and -CONR⁹R¹⁰ represented by R² is optionally substituted with one to four substituents selected from the group consisting of a fluorine atom, -OH, and a C₁₋₄ alkoxy group;
R⁹ and R¹⁰ of -CONR⁹R¹⁰ represented by R² are optionally bonded via a single bond or -O- to form a ring including the nitrogen atom bonded to R⁹ and R¹⁰;
the heterocyclyl group represented by R² having a 4- or 5-membered ring contains one oxygen heteroatom, and the heterocyclyl group having a 6-membered ring contains one or two oxygen heteroatoms;
R³ represents a hydrogen atom, a C₁₋₈ alkyl group, or a halogen atom;
X represents CR¹¹ or a nitrogen atom;
Y represents CR¹² or a nitrogen atom;
Z represents CR¹³ or a nitrogen atom;
R¹¹ to R¹³ each independently represent a hydrogen, fluorine, or chlorine atom or a C₁₋₆ alkyl or C₁₋₆ alkoxy group;
R⁴ represents -A¹-A²-A³;
A¹ represents a single bond or a C₁₋₈ alkylene, C₂₋₈ alkenylene, or C₂₋₈ alkynylene group;
one or two sp³ carbon atoms at any positions of A¹ are optionally replaced with one or two structures selected from the group consisting of -O-, -NR¹⁴-, -C(=O)-, -C(=O)-O-, -O-C(=O)-, -O-C(=O)-O-, -C(=O)-NR¹⁵-, -O-C(=O)-NR¹⁶-, - NR¹⁷-C(=O)-, -NR¹⁸-C(=O)-O-, -NR¹⁸-C(=O)-NR²⁰-, -S(=O)ₚ-, - S(=O)₂-NR²¹-, -NR²²-S (=O)₂-, and -NR²³-S (=O)₂-NR²⁴-, and a structure of -O-O-, -O-NR¹⁴-, -NR¹⁴-O-, -O-CH₂-O-, -O-CH₂-NR¹⁴-, or -NR¹⁴-CH₂-O- is not formed in the case of replacement of two sp³ carbon atoms;
A² represents a single bond or a C₁₋₇ alkylene, C₃₋₁₂ cycloalkylene, C₃₋₁₂ cycloalkylidene, 4- to 12-membered heterocyclylene, 4- to 12-membered heterocyclylidene, C₆₋₁₀ arylene, or 5- to 10-membered heteroarylene group;
A³ represents a halogen atom, -CN, -NO₂, -R²⁵, -OR²⁶, - NR²⁷R²⁸, -C(=O)R²⁹, -C(=O)-OR³⁰, -O-C(=O)R³¹, -O-C(=O)-NR³²R³³, -C(=O)-NR³⁴R³⁵, -NR³⁶-C (=O)R³⁷, -NR³⁸-C(=O)-OR³⁹, - S (=O)₂-R⁴⁰, -S(=O)₂-NR⁴¹R⁴², or -NR⁴³-S(=O)₂R⁴⁴;
A³ represents -R²⁵, if the A¹ end on the A² side has a structure selected from the group consisting of -O-, - NR¹⁴-, -C(=O)-, -C(=O)-O-, -O-C(=O)-, -O-C(=O)-O-, -C(=O)-NR¹⁵-, -O-C(=O)-NR¹⁶-, -NR¹⁷-C(=O)-, -NR¹⁸-C(=O)-O-, -NR¹⁹-C(=O)-NR²⁰-, -S(=O)ₚ-, -S(=O)₂-NR²¹-, -NR²²-S(=O)₂-, and - NR²³-S (=O)₂-NR²⁴- and A² is a single bond;
R¹⁴, R³², R³⁴, R³⁶, R³⁸, R⁴¹, and R⁴³ each independently represent a hydrogen atom or a C₁₋₈ alkyl, C₁₋₈ acyl, C₁₋₈ alkylsulfonyl, 4- to 12-membered heterocyclyl, C₃₋₁₂ cycloalkyl, C₆₋₁₀ aryl, 5- to 10-membered heteroaryl, (4-to 12-membered heterocyclyl) -C₁₋₃ alkyl, (C₃₋₁₂ cycloalkyl)-C₁₋₃ alkyl, (C₆₋₁₀ aryl) -C₁₋₃ alkyl, or (5- to 10-membered heteroaryl)-C₁₋₃ alkyl group;
R¹⁵ to R³¹, R³³, R³⁵, R³⁷, R³⁹, R⁴⁰, R⁴², and R⁴⁴ each independently represent a hydrogen atom or a C₁₋₈ alkyl, 4- to 12-membered heterocyclyl, C₃₋₁₂ cycloalkyl, C₆₋₁₀ aryl, 5- to 10-membered heteroaryl, (4- to 12-membered heterocyclyl) -C₁₋₃ alkyl, (C₃₋₁₂ cycloalkyl) -C₁₋₃ alkyl, (C₆₋₁₀ aryl)-C₁₋₃ alkyl, or (5- to 10-membered heteroaryl) -C₁₋₃ alkyl group;
A¹, A², A³, and R¹⁴ to R⁴⁴ in A¹ and A³ are each optionally substituted with one to four substituents selected from the group consisting of -OH, =O, -COOH, -SO₃H, -PO₃H₂, - CN, -NO₂, a halogen atom, a C₁₋₈ alkyl group substituted with zero to two -OH groups, zero to two -OR⁴⁵ groups, and zero to six fluorine atoms, a C₃₋₁₂ cycloalkyl group substituted with zero to two -OH groups, zero to two -OR⁴⁶ groups, and zero to six fluorine atoms, a C₁₋₈ alkoxy group substituted with zero to two -OH groups, zero to two -OR⁴⁷ groups, and zero to six fluorine atoms, and a 4-to 12-membered heterocyclyl group substituted with zero to two -OH groups, zero to two -OR⁴⁹ groups, and zero to six fluorine atoms;
R¹⁴ to R⁴⁴ are optionally bonded in A¹ or A³ or between A¹ and A², between A¹ and A³, or between A² and A³ via a single bond, -O-, -NR⁵⁰-, or -S(=O)ₚ- to form a ring;
R¹¹ or R¹³ is optionally bonded to A¹, A², or A³ via a single bond, -O-, -NR⁵¹-, or -S(=O)ₚ- to form a ring;
R⁴⁵, R⁴⁶, R⁴⁷, R⁴⁹, R⁵⁰, and R⁵¹ each represent a hydrogen atom or a C₁₋₄ alkyl group substituted with zero or one - OH group and zero to six fluorine atoms;
p represents an integer of 0 to 2; and
each of the heteroatom-containing groups represented by A¹, A², and A³ contains one to four heteroatoms selected from oxygen, sulfur, and nitrogen atoms].

2. The compound or pharmaceutically acceptable salt thereof according to claim 1, wherein L represents - NH-.

3. The compound or pharmaceutically acceptable salt thereof according to claim 1 or 2, wherein R¹ represents a C₁₋₈ alkyl, C₃₋₁₂ cycloalkyl, (C₃₋₁₂ cycloalkyl)-C₁₋₆ alkyl, 4- to 12-membered heterocyclyl, or (4- to 12-membered heterocyclyl)-C₁₋₆ alkyl group.

4. The compound or pharmaceutically acceptable salt thereof according to any one of claims 1 to 3, wherein R² is one of the following (i) to (iv):
(i) a C₁₋₈ alkyl group substituted with one to four fluorine atoms;
(ii) a C₁₋₈ alkyl group substituted with zero or one -OH group and zero to two C₁₋₈ alkoxy groups substituted with zero or one -OH group, zero or one C₁₋₄ alkoxy group, and zero to three fluorine atoms;
(iii) a 4- to 6-membered heterocyclyl group optionally substituted with one to four substituents selected from the group consisting of a fluorine atom, -OH, and C₁₋₄ alkyl and C₁₋₄ alkoxy groups;
(iv) -COOR⁸, -CONR⁹R¹⁰, or a C₁₋₈ acyl group, optionally each group being substituted with one to four substituents selected from the group consisting of a fluorine atom, -OH, and a C₁₋₄ alkoxy group.

5. The compound or pharmaceutically acceptable salt thereof according to any one of claims 1 to 4, wherein X, Y and Z are defined as in one of the following (i) to (iv):
(i) X represents CR¹¹, Y represents CR¹², and Z represents CR¹³;
(ii) X represents a nitrogen atom, Y represents CR¹², and Z represents CR¹³;
(iii) X represents CR¹¹, Y represents a nitrogen atom, and Z represents CR¹³;
(iv) X represents CR¹¹, Y represents CR¹², and Z represents a nitrogen atom.

6. The compound or pharmaceutically acceptable salt thereof according to any one of claims 1 to 5, wherein A¹ is defined as in one of the following (i) to (v) :
(i) A¹ is a single bond;
(ii) A¹ represents a C₁₋₈ alkylene group, and no sp³ carbon atom in A¹ is replaced with another structure;
(iii) A¹ represents a C₁₋₈ alkylene group, and one sp³ carbon atom at any position of A¹ is replaced with -O-;
(iv) A¹ represents a C₁₋₈ alkylene group, and one sp³ carbon atom at any position of A¹ is replaced with -NR¹⁴-;
(v) A¹ represents a C₁₋₈ alkylene group, one sp³ carbon atom at any position of A¹ is replaced with -NR¹⁴-, and one sp³ carbon atom at any other position of A¹ is optionally replaced with -O-.

7. The compound or pharmaceutically acceptable salt thereof according to any one of claims 1 to 6, wherein A² is defined as in (i) or (ii):
(i) A² represents a 4- to 12-membered heterocyclylene group; and A² is optionally substituted with one to four substituents selected from the group consisting of -OH, - COOH, -SO₃H, -PO₃H₂, -CN, -NO₂, a halogen atom, a C₁₋₈ alkyl group substituted with zero to two -OH groups, zero to two -OR⁴⁵ groups, and zero to six fluorine atoms, a C₃₋₁₂ cycloalkyl group substituted with zero to two -OH groups, zero to two -OR⁴⁶ groups, and zero to six fluorine atoms, a C₁₋₈ alkoxy group substituted with zero to two -OH groups, zero to two -OR⁴⁷ groups, and zero to six fluorine atoms, and a 4- to 12-membered heterocyclyl group substituted with zero to two -OH groups, zero to two -OR⁴⁹ groups, and zero to six fluorine atoms;
(ii) A² represents a 4- to 12-membered heterocyclylene group substituted with =O; and A² is optionally substituted with one to four substituents selected from the group consisting of -OH, =O, -COOH, -SO₃H, -PO₃H₂, - CN, -NO₂, a halogen atom, a C₁₋₈ alkyl group substituted with zero to two -OH groups, zero to two -OR⁴⁵ groups, and zero to six fluorine atoms, a C₃₋₁₂ cycloalkyl group substituted with zero to two -OH groups, zero to two -OR⁴⁶ groups, and zero to six fluorine atoms, a C₁₋₈ alkoxy group substituted with zero to two -OH groups, zero to two -OR⁴⁷ groups, and zero to six fluorine atoms, and a 4-to 12-membered heterocyclyl group substituted with zero to two -OH groups, zero to two -OR⁴⁹ groups, and zero to six fluorine atoms.

8. The compound or pharmaceutically acceptable salt thereof according to any one of claims 1 to 7, wherein X represents CR¹¹, Y represents CR¹², Z represents CR¹³, and R¹¹ or R¹³ is bonded to A¹, A², or A³ via a single bond, -O-, -NR⁵¹-, or -S(=O)ₚ- to form a ring.

9. The compound or pharmaceutically acceptable salt thereof according to any one of claims 1 to 8, wherein A³ is a hydrogen atom; or
wherein A³ is a halogen atom, -CN, -R²⁵, -OR²⁶, -NR²⁷R²⁸, - C(=O)R²⁹, or -C(=O)-OR³⁰, and R²⁵ to R³⁰ each independently represent a hydrogen atom, an optionally substituted C₁₋₈ alkyl group, an optionally substituted 4- to 12-membered heterocyclyl group, an optionally substituted C₃₋₁₂ cycloalkyl group, an optionally substituted (4- to 12-membered heterocyclyl)-C₁₋₃ alkyl group, or an optionally substituted (C₃₋₁₂ cycloalkyl) -C₁₋₃ alkyl group.

10. The compound or pharmaceutically acceptable salt thereof according to any one of claims 1 to 9, wherein R³ is a hydrogen atom; or
wherein R³ represents a C₁₋₄ alkyl group, a fluorine atom, or a chlorine atom.

11. The compound represented by Formula (I) or pharmaceutically acceptable salt thereof according to claim 1, which is selected from:
6-(difluoromethyl)-N8-isopropyl-N2-(5-piperazin-1-yl-2-pyridyl)pyrido[3,4-d]pyrimidine-2,8-diamine;
(1R)-1-[8-(isopropylamino)-2-[(5-piperazin-1-yl-2-pyridyl)amino]pyrido[3,4-d]pyrimidin-6-yl] ethanol;
1-[2-[(5-piperazin-1-yl-2-pyridyl)amino]-8-(tetrahydrofuran-3-ylamino)pyrido[3,4-d]pyrimidin-6-yl]ethanol;
1-[2-[(5-piperazin-1-yl-2-pyridyl)amino]-8-(tetrahydropyran-3-ylamino)pyrido[3,4-d]pyrimidin-6-yl]ethanol;
N8-isopropyl-6-[(1R)-1-methoxyethyl]-N2-(6-piperazin-1-ylpyridazin-3-yl)pyrido[3,4-d]pyrimidine-2,8-diamine;
N8-isopropyl-6-[(1R)-1-methoxyethyl]-N2-[5-(piperazin-1-ylmethyl)-2-pyridyl]pyrido[3,4-d]pyrimidine-2,8-diamine;
1-[6-[[6-[(1R)-1-hydroxyethyl]-8-(isopropylamino)pyrido[3,4-d]pyrimidin-2-yl]amino]-3-pyridyl]piperazin-2-one;
1-[6-[[5-chloro-6-[(1R)-1-hydroxyethyl]-8-(isopropylamino)pyrido[3,4-d]pyrimidin-2-yl]amino]-3-pyridyl]piperazin-2-one;
(1R)-1-[2-[(6-piperazin-1-ylpyridazin-3-yl)amino]-8-(tetrahydropyran-4-ylamino)pyrido[3,4-d]pyrimidin-6-yl]ethanol;
(1R)-1-[2-[(6-piperazin-1-ylpyridazin-3-yl)amino]-8-[[(3S)-tetrahydropyran-3-yl]amino]pyrido[3,4-d]pyrimidin-6-yl]ethanol;
(1R)-1-[2-[(6-piperazin-1-ylpyridazin-3-yl)amino]-8-[[(3R)-tetrahydropyran-3-yl]amino]pyrido[3,4-d]pyrimidin-6-yl]ethanol;
(1R)-1-[2-[[5-(piperazin-1-ylmethyl)-2-pyridyl]amino]-8-(tetrahydropyran-4-ylamino)pyrido[3,4-d]pyrimidin-6-yl]ethanol;
(1R)-1-[2-[[5-(piperazin-1-ylmethyl)-2-pyridyl]amino]-8-[[(3S)-tetrahydropyran-3-yl]amino]pyrido[3,4-d]pyrimidin-6-yl]ethanol;
(1R)-1-[2-[[5-(piperazin-1-ylmethyl)-2-pyridyl]amino]-8-[[(3R)-tetrahydropyran-3-yl]amino]pyrido[3,4-d]pyrimidin-6-yl]ethanol;
1-[6-[[6-[(1R)-1-hydroxyethyl]-8-(isopropylamino)pyrido[3,4-d]pyrimidin-2-yl]amino]pyridazin-3-yl]piperidin-4-ol;
(1R)-1-[8-(isopropylamino)-2-[(6-piperazin-1-ylpyridazin-3-yl)amino]pyrido[3,4-d]pyrimidin-6-yl]ethanol;
1-[[6-[[6-[(1R)-1-hydroxyethyl]-8-(isopropylamino)pyrido[3,4-d]pyrimidin-2-yl]amino]-3-pyridyl]methyl]piperazin-2-one;
6-[(1R)-1-methoxyethyl]-N2-[5-(piperazin-1-ylmethyl)-2-pyridyl]-N8-[(3S)-tetrahydropyran-3-yl]pyrido[3,4-d]pyrimidine-2,8-diamine;
6-[(1R)-1-methoxyethyl]-N2-(6-piperazin-1-ylpyridazin-3-yl)-N8-[(3S)-tetrahydropyran-3-yl]pyrido[3,4-d]pyrimidine-2,8-diamine;
6-[(1R)-1-methoxyethyl]-N2-[5-(piperazin-1-ylmethyl)-2-pyridyl]-N8-(tetrahydropyran-4-ylmethyl)pyrido[3,4-d]pyrimidine-2,8-diamine;
N8-isopropyl-6-[(1R)-1-methoxyethyl]-N2-(5-piperazin-1-ylpyrazin-2-yl)pyrido[3,4-d]pyrimidine-2,8-diamine;
N8-isopropyl-6-[(1R)-1-methoxyethyl]-N2-[6-[(2S)-2-methylpiperazin-1-yl]pyridazin-3-yl]pyrido[3,4-d]pyrimidine-2,8-diamine;
N8-isopropyl-6-[(1R)-1-methoxyethyl]-N2-[6-[(2R)-2-methylpiperazin-1-yl]pyridazin-3-yl]pyrido[3,4-d]pyrimidine-2,8-diamine;
(1R)-1-[2-[[6-(4,7-diazaspiro[2.5]octan-7-yl)pyridazin-3-yl]amino]-8-(isopropylamino)pyrido[3,4-d]pyrimidin-6-yl]ethanol;
(1R)-1-[2-[[5-(4,7-diazaspiro[2.5]octan-7-ylmethyl)-2-pyridyl]amino]-8-(isopropylamino)pyrido[3,4-d]pyrimidin-6-yl]ethanol;
2-[1-[[6-[[6-[(1R)-1-hydroxyethyl]-8-(isopropylamino)pyrido[3,4-d]pyrimidin-2-yl]amino]-3-pyridyl]methyl]-4-piperidyl]propan-2-ol;
(1R)-1-[2-[[5-[[4-(2-hydroxyethyl)piperazin-1-yl]methyl]-2-pyridyl]amino]-8-(isopropylamino)pyrido[3,4-d]pyrimidin-6-yl]ethanol;
(1R)-1-[2-[[5-[2-(dimethylamino)ethoxy]-2-pyridyl]amino]-8-[[(3S)-tetrahydropyran-3-yl]amino]pyrido[3,4-d]pyrimidin-6-yl]ethanol;
(1R)-1-[2-[[6-(4-methylpiperazin-1-yl)pyridazin-3-yl]amino]-8-[[(3S)-tetrahydropyran-3-yl]amino]pyrido[3,4-d]pyrimidin-6-yl]ethanol;
2-hydroxy-1-[4-[6-[[6-[(1R)-1-hydroxyethyl]-8-(isopropylamino)pyrido[3,4-d]pyrimidin-2-yl]amino]pyridazin-3-yl]piperazin-1-yl]ethanone;
1-[6-[[8-(isopropylamino)-6-[(2S)-tetrahydrofuran-2-yl]pyrido[3,4-d]pyrimidin-2-yl]amino]-3-pyridyl]piperazin-2-one;
(1R)-1-[8-(isopropylamino)-2-(5,6,7,8-tetrahydro-1,6-naphthyridin-2-ylamino)pyrido[3,4-d]pyrimidin-6-yl]ethanol;
2-[4-[[6-[[6-[(1R)-1-hydroxyethyl]-8-(isopropylamino)pyrido[3,4-d]pyrimidin-2-yl]amino]-3-pyridyl]methyl]piperazin-1-yl]-2-methyl-propan-1-ol;
4-[6-[[6-[(1R)-1-hydroxyethyl]-8-(isopropylamino)pyrido[3,4-d]pyrimidin-2-yl]amino]-3-pyridyl]-1-[(2S)-2-hydroxypropyl]-1,4-diazepan-5-one;
4-[6-[[6-[(1R)-1-hydroxyethyl]-8-(isopropylamino)pyrido[3,4-d]pyrimidin-2-yl]amino]-3-pyridyl]-1-[(2R)-2-hydroxypropyl]-1,4-diazepan-5-one;
N8-isopropyl-N2-[5-(piperazin-1-ylmethyl)-2-pyridyl]-6-[(2S)-tetrahydrofuran-2-yl]pyrido[3,4-d]pyrimidine-2,8-diamine;
1-[6-[[6-[(1R)-1-hydroxyethyl]-8-(isopropylamino)pyrido[3,4-d]pyrimidin-2-yl]amino]-2-methyl-3-pyridyl]piperazin-2-one;
1-[6-[[8-(isopropylamino)-6-[(3S)-tetrahydrofuran-3-yl]pyrido[3,4-d]pyrimidin-2-yl]amino]-3-pyridyl]piperazin-2-one;
(1R)-1-[2-(5,6,7,8-tetrahydro-1,6-naphthyridin-2-ylamino)-8-[[(3S)-tetrahydropyran-3-yl]amino]pyrido[3,4-d]pyrimidin-6-yl]ethanol;
1-[6-[[8-(isopropylamino)-6-(3-methyloxetan-3-yl)pyrido[3,4-d]pyrimidin-2-yl]amino]-3-pyridyl]piperazin-2-one;
(1R)-1-[2-[[5-[4-(dimethylamino)cyclohexoxy]-2-pyridyl]amino]-8-[[(3S)-tetrahydropyran-3-yl]amino]pyrido[3,4-d]pyrimidin-6-yl] ethanol;
6-[(1R)-1-methoxyethyl]-N2-[5-(piperazin-1-ylmethyl)-2-pyridyl]-N8-propyl-pyrido[3,4-d]pyrimidine-2,8-diamine;
6-[(1R)-1-methoxyethyl]-N2-(6-piperazin-1-ylpyridazin-3-yl)-N8-propyl-pyrido[3,4-d]pyrimidine-2,8-diamine;
1-[[6-[[6-(difluoromethyl)-8-[(4-methylcyclohexyl)amino]pyrido[3,4-d]pyrimidin-2-yl]amino]-3-pyridyl]methyl]piperidine-4-carboxylic acid;
(1R)-1-[8-(ethylamino)-2-[[5-[[4-(2-hydroxyethyl)piperazin-1-yl]methyl]-2-pyridyl]amino]pyrido[3,4-d]pyrimidin-6-yl] ethanol;
(1R)-1-[2-[[5-[[4-(2-hydroxyethyl)piperazin-1-yl]methyl]-2-pyridyl]amino]-8-(propylamino)pyrido[3,4-d]pyrimidin-6-yl]ethanol;
N8-isopropyl-6-(3-methyloxetan-3-yl)-N2-(6-piperazin-1-ylpyridazin-3-yl)pyrido[3,4-d]pyrimidine-2,8-diamine;
N8-isopropyl-6-(3-methyloxetan-3-yl)-N2-[5-(piperazin-1-ylmethyl)-2-pyridyl]pyrido[3,4-d]pyrimidine-2,8-diamine;
6-(3-methyloxetan-3-yl)-N2-[5-(piperazin-1-ylmethyl)-2-pyridyl]-N8-[(3S)-tetrahydropyran-3-yl]pyrido[3,4-d]pyrimidine-2,8-diamine;
4-[6-[[6-[(1R)-1-hydroxyethyl]-8-[isopropyl(methyl)amino]pyrido[3,4-d]pyrimidin-2-yl]amino]-3-pyridyl]-1,4-diazepan-5-one;
(1R)-1-[8-(isopropylamino)-2-[(6-methyl-5-piperazin-1-yl-2-pyridyl)amino]pyrido[3,4-d]pyrimidin-6-yl] ethanol;
(1R)-1-[2-[[6-(2-hydroxyethyl)-7,8-dihydro-5H-1,6-naphthyridin-2-yl]amino]-8-(isopropylamino)pyrido[3,4-d]pyrimidin-6-yl] ethanol;
(1R)-1-[8-(isopropylamino)-2-[[6-[2-(methylamino)ethyl]-7,8-dihydro-5H-1,6-naphthyridin-2-yl]amino]pyrido[3,4-d]pyrimidin-6-yl] ethanol;
N2-(6-piperazin-1-ylpyridazin-3-yl)-6-[ (3S)-tetrahydrofuran-3-yl]-N8-[(3S)-tetrahydropyran-3-yl]pyrido[3,4-d]pyrimidine-2,8-diamine;
N2-[5-(piperazin-1-ylmethyl)-2-pyridyl]-6-[(3R)-tetrahydrofuran-3-yl]-N8-[(3S)-tetrahydropyran-3-yl]pyrido[3,4-d]pyrimidine-2,8-diamine;
(1R)-1-[2-[[6-[2-(dimethylamino)ethyl]-7,8-dihydro-5H-1,6-naphthyridin-2-yl]amino]-8-(isopropylamino)pyrido[3,4-d]pyrimidin-6-yl] ethanol;
(2S)-1-[4-[[6-[[8-(ethylamino)-6-[(1R)-1-hydroxyethyl]pyrido[3,4-d]pyrimidin-2-yl]amino]-3-pyridyl]methyl]piperazin-1-yl]propan-2-ol;
(2R)-1-[4-[[6-[[8-(ethylamino)-6-[(1R)-1-hydroxyethyl]pyrido[3,4-d]pyrimidin-2-yl]amino]-3-pyridyl]methyl]piperazin-1-yl]propan-2-ol;
(1R)-1-[8-(isopropylamino)-2-[[5-[(2R)-2-methylpiperazin-1-yl]-2-pyridyl]amino]pyrido[3,4-d]pyrimidin-6-yl]ethanol;
(1R)-1-[8-(isopropylamino)-2-[[5-[(2S)-2-methylpiperazin-1-yl]-2-pyridyl]amino]pyrido[3,4-d]pyrimidin-6-yl]ethanol;
N8-isopropyl-N2-(5-piperazin-1-yl-2-pyridyl)-6-[(2S)-tetrahydrofuran-2-yl]pyrido[3,4-d]pyrimidine-2,8-diamine;
(1R)-1-[8-(cyclobutylamino)-2-[[5-[[4-(2-hydroxyethyl)piperazin-1-yl]methyl]-2-pyridyl]amino]pyrido[3,4-d]pyrimidin-6-yl]ethanol;
(1R)-1-[8-(cyclopropylmethylamino)-2-[[5-[[4-(2-hydroxyethyl)piperazin-1-yl]methyl]-2-pyridyl]amino]pyrido[3,4-d]pyrimidin-6-yl]ethanol;
6-(3-methyloxetan-3-yl)-N2-(5-piperazin-1-yl-2-pyridyl)-N8-propyl-pyrido[3,4-d]pyrimidine-2,8-diamine;
6-(3-methyloxetan-3-yl)-N2-[5-(piperazin-1-ylmethyl)-2-pyridyl]-N8-propyl-pyrido[3,4-d]pyrimidine-2,8-diamine;
N2-(5-piperazin-1-yl-2-pyridyl)-N8-propyl-6-tetrahydrofuran-3-yl-pyrido[3,4-d]pyrimidine-2,8-diamine;
N2-[5-(piperazin-1-ylmethyl)-2-pyridyl]-N8-propyl-6-tetrahydrofuran-3-yl-pyrido[3,4-d]pyrimidine-2,8-diamine;
N8-isopropyl-6-(3-methyloxetan-3-yl)-N2-(5-piperazin-1-yl-2-pyridyl)pyrido[3,4-d]pyrimidine-2,8-diamine;
N8-isopropyl-N2-(5-piperazin-1-yl-2-pyridyl)-6-tetrahydrofuran-3-yl-pyrido[3,4-d]pyrimidine-2,8-diamine;
2-[4-[[6-[[8-(isopropylamino)-6-tetrahydrofuran-3-yl-pyrido[3,4-d]pyrimidin-2-yl]amino]-3-pyridyl]methyl]piperazin-1-yl]ethanol;
2-[4-[[6-[[6-tetrahydrofuran-3-yl-8-[[(3S)-tetrahydropyran-3-yl]amino]pyrido[3,4-d]pyrimidin-2-yl]amino]-3-pyridyl]methyl]piperazin-1-yl]ethanol;
(1R)-1-[2-[[5-[[4-(hydroxymethyl)-1-piperidyl]methyl]-2-pyridyl]amino]-8-(isopropylamino)pyrido[3,4-d]pyrimidin-6-yl]ethanol;
1-[[6-[[6-[(1R)-1-hydroxyethyl]-8-(isopropylamino)pyrido[3,4-d]pyrimidin-2-yl]amino]-3-pyridyl]methyl]piperidin-4-ol;
1-[[6-[[8-(tert-butylamino)-6-[(1R)-1-hydroxyethyl]pyrido[3,4-d]pyrimidin-2-yl]amino]-3-pyridyl]methyl]piperidin-4-ol;
(1R)-1-[8-(tert-butylamino)-2-[[5-[[4-(hydroxymethyl)-1-piperidyl]methyl]-2-pyridyl]amino]pyrido[3,4-d]pyrimidin-6-yl]ethanol;
1-[[6-[[6-[(1R)-1-hydroxyethyl]-8-(isobutylamino)pyrido[3,4-d]pyrimidin-2-yl]amino]-3-pyridyl]methyl]piperidin-4-ol;
(1R)-1-[2-[[5-[[4-(hydroxymethyl)-1-piperidyl]methyl]-2-pyridyl]amino]-8-(isobutylamino)pyrido[3,4-d]pyrimidin-6-yl]ethanol;
1-[6-[[6-[(1R)-1-hydroxypropyl]-8-(isopropylamino)pyrido[3,4-d]pyrimidin-2-yl]amino]-3-pyridyl]piperazin-2-one;
(1R)-1-[2-[[5-[[4-(2-hydroxyethyl)piperazin-1-yl]methyl]-6-methyl-2-pyridyl]amino]-8-(propylamino)pyrido[3,4-d]pyrimidin-6-yl]ethanol.

12. A pharmaceutical composition comprising the compound or pharmaceutically acceptable salt thereof according to any one of claims 1 to 11 and a pharmaceutically acceptable carrier.

13. A pharmaceutical composition exhibiting a CDK4/6 inhibitory activity, comprising the compound or pharmaceutically acceptable salt thereof according to any one of claims 1 to 11 as an active ingredient.

14. A drug for use in the prevention or treatment of rheumatoid arthritis, arteriosclerosis, pulmonary fibrosis, cerebral infarction, or cancer, the drug comprising the compound or pharmaceutically acceptable salt thereof according to any one of claims 1 to 11 as an active ingredient.

## Patentansprüche

1. Verbindung, dargestellt durch Formel (I), oder ein pharmazeutisch annehmbares Salz davon: 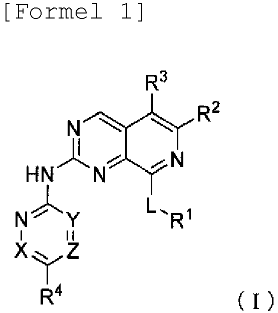 [worin
L -NR⁵-, -O- oder -S- darstellt,
R⁵ ein Wasserstoffatom oder eine C₁₋₆-Alkylgruppe darstellt, die mit null bis zwei -OH-Gruppen, null bis zwei C₁₋₈-Akoxygruppen und null bis sechs Fluoratomen substituiert ist,
R¹ eine C₁₋₈-Alkyl-, C₃₋₁₂-Cycloaklyl-, (C₃₋₁₂-Cycloalkyl)-C₁₋₆-alkyl-, 4- bis 12-glierdige Heterocyclyl-, (4- bis 12-gliedrige Heterocyclyl)-C₁₋₆-alkyl-, C₆₋₁₀-Aryl-, (C₆₋₁₀-Aryl)-C₁₋₆-alkyl, 5- bis 10-gliedrige Heteroaryl-, (5- bis 10-gliedrige Heteroaryl)-C₁₋₆-alkyl-, C₁₋₈-Alkylsulfonyl- oder C₁₋₈-Acylgruppe darstellt,
jede der durch R¹ dargestellten Heteroatom-haltigen Gruppen ein bis vier Heteroatome, ausgewählt aus Sauerstoff-, Schwefel- und Stickstoffatomen, enthält,
R¹ geggebenenfalls mit einem bis sechs Substituenten, ausgewählt aus der Gruppe, bestehend aus einem Halogenatom, =O, -OH, -CN, -COOH, -COOR⁶, -R⁷, einer C₂₋₆-Cycloalkylgruppe, die mit null bis zwei -OH-Gruppen, null bis zwei C₁₋₈-Alkoxygruppen und null bis sechs Fluoratomen substituiert ist, einer 3- bis 10-gliedrigen Heterocyclylgruppe, die mit null bis zwei -OH-Gruppen, null bis zwei C₁₋₈-Alkoxygruppen und null bis sechs Fluoratomen substituiert ist, einer C₁₋₈-Acylgruppe, die mit null bis zwei -OH-Gruppen, null bis zwei C₁₋₈-Alkoxygruppen und null bis sechs Fluoratomen substituiert ist, und einer C₁₋₈-Alkoxygruppe, die mit null bis zwei -OH-Gruppen, null bis zwei C₁₋₈-Alkoxygruppen und null bis sechs Fluoratomen substituiert ist, substituiert ist,
R⁶ und R⁷ jeweils unabhängig ein C₁₋₆-Alkylgruppe, die mit null bis zwei -OH-Gruppen, null bis zwei C₁₋₈-Alkoxygruppen und null bis sechs Fluoratomen substituiert ist, darstellen,
R² eine C₁₋₈-Alkyl-, C₃₋₈-Cycloalkyl-, 4- bis 6-gliedrige Heterocyclyl-, C₁₋₈-Acylgruppe, -COOR⁸ oder -CONR⁹R¹⁰ darstellt,
jede der durch R² dargestellten C₁₋₈-Alkyl- und C₃₋₈-Cycloalkylgruppen mit null oder einer -OH-Gruppe, null bis zwei C₁₋₈-Alkoxygruppen, die mit null oder einer -OH-Gruppe, null oder einer C₁₋₄-Alkoxygruppe und null bis drei Fluoratomen substituiert sind, und null bis fünf Fluoratomen substituiert ist,
R² weder eine unsubstituierte C₁₋₈-Alkyl- noch eine unsubstituierte C₃₋₈-Cycloalkyl- noch eine Trifluormethylgruppe ist,
R⁸, R⁹ und R¹⁰ jeweils unabhängig ein Wasserstoffatom oder eine C₁₋₈-Alkylgruppe darstellt,
die durch R² dargestellte 4- bis 6-gliedrige Heterocyclylgruppe gegebenenfalls mit einem bis vier Substituenten, ausgewählt aus der Gruppe, bestehend aus einem Fluoratom, -OH und C₁₋₄-Alkyl- und C₁₋₄-Alkoxygruppen, substituiert ist,
jede/jedes von der C₁₋₈-Acylgruppe, -COOR⁸ und -CONR⁹R¹⁰, dargestellt durch R², gegebenenfalls mit einem bis vier Substituenten, ausgewählt aus der Gruppe, bestehend aus einem Fluoratom, -OH und einer C₁₋₄-Alkoxygruppe, substituiert ist,
R⁹ und R¹⁰ von -CONR⁹R¹⁰, dargestellt durch R², gegebenenfalls über eine Einfachbindung oder -O- verbunden sind, um einen Ring einschließlich des an R⁹ und R¹⁰ gebundenen Stickstoffatoms zu bilden,
die durch R² dargestellte Heterocyclylgruppe mit einem 4- oder 5-gliedrigen Ring ein Sauerstoffheteroatom enthält und die Heterocyclylgruppe mit einem 6-gliedrigen Ring ein oder zwei Sauerstoffheteroatome enthält,
R³ ein Wasserstoffatom, eine C₁₋₈-Alkylgruppe oder ein Halogenatom darstellt,
X CR¹¹ oder ein Stickstoffatom darstellt,
Y CR¹² oder ein Stickstoffatom darstellt,
Z CR¹³ oder ein Stickstoffatom darstellt,
R¹¹ bis R¹³ jeweils unabhängig ein Wasserstoff-, Fluor- oder Chlortom oder eine C₁₋₆-Alkyl- oder C₁₋₆-Alkoxygruppe darstellen,
R⁴ -A¹-A²-A³ darstellt,
A¹ eine Einfachbindung oder eine C₁₋₈-Alkylen-, C₂₋₈-Alkenylen- oder C₂₋₈-Alkinylengruppe darstellt,
ein oder zwei sp³-Kohlenstoffatome an beliebigen Positionen von A¹ gegebenenfalls durch eine oder zwei Strukturen, ausgewählt aus der Gruppe, bestehend aus -O-, -NR¹⁴-, -C(=O)-, -C(=O)-O-, -O-C(=O)-, -O-C(=O)-O-, -C(=O)-NR¹⁵-, -O-C(=O)-NR¹⁶-, -NR¹⁷-C(=O)-, -NR¹⁸-C(=O)-O-, -NR¹⁹-C(=O)-NR²⁰-, -S(=O)ₚ-, -S(=O)₂-NR²¹-, -NR²²-S(=O)₂- und -NR²³-S(=O)₂-NR²⁴-, ersetzt ist/sind und eine Struktur -O-O-, -O-NR¹⁴-, -NR¹⁴-O-, -O-CH₂-O-, -O-CH₂-NR¹⁴- oder -NR¹⁴-CH₂-O- im Falle des Ersetzens von zwei sp³-Kohlenstoffatomen nicht gebildet wird,
A² eine Einfachbindung oder eine C₁₋₇-Alkylen-, C₃₋₁₂-Cycloalkylen-, C₃₋₁₂-Cycloalkyliden-, 4- bis 12-gliedrige Heterocyclylen-, 4- bis 12-gliedrige Heterocyclyliden-, C₆₋₁₀-Arylen- oder 5- bis 10-gliedrige Heteroarylengruppe darstellt,
A³ ein Halogenatom, -CN, -NO₂, -R²⁵, -OR²⁶, -NR²⁷R²⁸, -C(=O)²⁹, -C(=O)-OR³⁰, -O-C(=O)R³¹, -O-C(=O)-NR³²R³³, -C(=O)-NR³⁴R³⁵, -NR³⁶-C(=O)R³⁷, -NR³⁸-C(=O)-OR³⁹, -S(=O)₂-R⁴⁰, -S(=O)₂-NR⁴¹R⁴² oder -NR⁴³⁻S(=O)₂R⁴⁴ darstellt,
A³ -R²⁵ darstellt, wenn das A¹-Ende an der A²-Seite eine Struktur, ausgewählt aus der Gruppe, bestehend aus -O-, -NR¹⁴-, -C(=O)-, -C(=O)-O-, -O-C(=O)-, -O-C(=O)-O-, -C(=O)-NR¹⁵-, -O-C(=O)-NR¹⁶-, -NR¹⁷-C(=O) -NR¹⁸-C(=O)-O-, -NR¹⁹-C(=O)-NR²⁰-, -S(=O)ₚ-, -S(=O)₂-NR²¹-, -NR²²-S(=O)₂-, and -NR²³-S(=O)₂-NR²⁴-, aufweist und A² eine Einfachbindung ist,
R¹⁴, R³², R³⁴, R³⁵, R³⁸ R⁴¹ und R⁴³ jeweils unabhängig ein Wasserstoffatom oder eine C₁₋₈-Alkyl-, C₁₋₈-Acyl-, C₁₋₈-Alkylsulfonyl-, 4- bis 12-gliedrige Heterocyclyl-, C₃₋₁₂-Cycloalkyl-, C₆₋₁₀-Aryl-, 5- bis 10-gliedrige Heteroaryl-, (4- bis 12-gliedrige Heterocyclyl)-C₁₋₃-alkyl-, (C₃₋₁₂-Cycloalkyl)-C₁₋₃-alkyl-, (C₆₋₁₀-Aryl)-C₁₋₃-alkyl- oder (5- bis 10-gliedrige Heteroaryl)-C₁₋₃-alkylgruppe darstellen,
R¹⁵ bis R³¹, R³³, R³⁵, R³⁷, R³⁹, R⁴⁰, R⁴² und R⁴⁴ jeweils unabhängig ein Wasserstoffatom oder eine C₁₋₈-Alkyl-, 4- bis 12-gliedrige Heterocyclyl-, C₃₋₁₂-Cycloalkyl, C₆₋₁₀-Aryl-, 5- bis 10-gliedrige Heteroaryl-, (4- bis 12-gliedrige Heterocyclyl)-C₁₋₃-aryl-, (C₃₋₁₂-Cycloalkyl)-C₁₋₃-alkyl-, (C₆₋₁₀-Aryl)-C₁₋₃-alkyl- oder (5- bis 10-gliedrige Heteroaryl)-C₁₋₃-alkylgruppe darstellen,
A¹, A², A³ und R¹⁴ bis R⁴⁴ in A¹ und A³ jeweils gegebenenfalls mit einem bis vier Substituenten, ausgewählt aus der Gruppe, bestehend aus -OH, =O, -COOH, -SO₃H, -PO₃H₂, -CN, -NO₂, einem Halogenatom, einer C₁₋₈-Alkylgruppe, substituiert mit null bis zwei -OH-Gruppen, null bis zwei -OR⁴⁵-Gruppe und null bis sechs Fluoratomen, einer C₃₋₁₂-Cycloalkylgruppe, substituiert mit null bis zwei -OH-Gruppen, null bis zwei -OR⁴⁶-Gruppen und null bis sechs Fluoratomen, einer C₁₋₈-Alkoxygruppe, substituiert mit null bis zwei -OH-Gruppen, null bis zwei -OR⁴⁷-Gruppen und null bis sechs Fluoratomen, und einer 4- bis 12-gliedrigen Heterocyclylgruppe, substituiert mit null bis zwei -OH-Gruppen, null bis zwei -OR⁴⁹-Gruppen und null bis sechs Fluoratomen, substituiert sind,
R¹⁴ bis R⁴⁴ gegebenenfalls in A¹ oder A³ oder zwischen A¹ und A², zwischen A¹ und A³ oder zwischen A² und A³ über eine Einfachbindung, -O-, -NR⁵⁰- oder -S(=O)ₚ- verbunden sind, um einen Ring zu bilden,
R¹¹ oder R¹³ gegebenenfalls an A¹, A² oder A³ über eine Einfachbindung, -O-, -NR⁵¹- oder -S(=O)ₚ- verbunden sind, um einen Ring zu bilden,
R⁴⁵, R⁴⁶, R⁴⁷, R⁴⁸ R⁴⁹, R⁵⁰ und R⁵¹ jeweils ein Wasserstoffatom oder eine C₁₋₄-Alkylgruppe, die mit null oder einer -OH-Gruppe und null bis sechs Fluoratomen substituiert ist, darstellen,
p eine ganze Zahl von 0 bis 2 darstellt und
jede der durch A¹, A² und A³ dargestellten Heteroatomhaltigen Gruppen ein bis vier Heteroatome, ausgewählt aus Sauer-, Schwefel- und Stickstoffatomen, enthält].

2. Verbindung oder pharmazeutisch annehmbares Salz davon gemäß Anspruch 1, worin L -NH- darstellt.

3. Verbindung oder pharmazeutisch annehmbares Salz davon gemäß Anspruch 1 oder 2, worin R¹ eine C₁₋₈-Alkyl-, C₃₋₁₂-Cycloalkyl-, (C₃₋₁₂-Cycloalkyl)-C₁₋₆-alkyl-, 4-bis 12-gliedrige Heterocyclyl- oder (4- bis 12-gliedrige Heterocyclyl)-C₁₋₆-alkylgruppe darstellt.

4. Verbindung oder pharmazeutisch annehmbares Salz davon gemäß irgendeinem der Ansprüche 1 bis 3,
worin R² eines der folgenden (i) bis (iv) ist:
(i) eine C₁₋₈-Alkylgruppe, die mit einem bis vier Fluoratomen substituiert ist,
(ii) eine C₁₋₈-Alkylgruppe, substituiert mit null oder einer -OH-Gruppe und null bis zwei C₁₋₈-Alkoxygruppen, die mit null oder einer -OH-Gruppe, null oder einer C₁₋₄-Alkoxygruppe und null bis drei Fluoratomen substituiert ist,
(iii) eine 4- bis 6-gliedrige Heterocyclylgruppe, die gegebenenfalls mit einem bis vier Substituenten, ausgewählt aus der Gruppe, bestehend aus einem Fluoratom, -OH- und C₁₋₄-Alkyl- und C₁₋₄-Alkoxygruppen, substituiert ist,
(iv) -COOR⁸, -CONR⁹R¹⁰ oder eine C₁₋₈-Acylgruppe, wobei jede Gruppe gegebenenfalls mit einem bis vier Substituenten, ausgewählt aus der Gruppe, bestehend aus einem Fluoratom, -OH- und einer C₁₋₄-Alkoxygruppe, substituiert ist.

5. Verbindung oder pharmazeutisch annehmbares Salz davon gemäß irgendeinem der Ansprüche 1 bis 4,
worin X, Y und Z so wie in einem der folgenden (i) bis (iv) definiert sind:
(i) X stellt CR¹¹ dar, Y stellt CR¹² dar und Z stellt CR¹³ dar,
(ii) X stellt ein Stickstoffatom dar, Y stellt CR¹² dar und Z stellt CR¹³ dar,
(iii) X stellt ein CR¹¹ dar, Y stellt ein Stickstoffatom dar und Z stellt CR¹³ dar,
(iv) X stellt ein CR¹¹ dar, Y stellt CR¹² dar und Z stellt ein Stickstoffatom dar.

6. Verbindung oder pharmazeutisch annehmbares Salz davon gemäß irgendeinem der Ansprüche 1 bis 5, worin A¹ so wie in einem der folgenden (i) bis (v) definiert ist:
(i) A¹ ist eine Einfachbindung,
(ii) A¹ ist eine C₁₋₈-Alkylengruppe und kein sp³-Kohlenstoffatom in A¹ ist durch eine andere Struktur ersetzt,
(iii) A¹ stellt eine C₁₋₈-Alkylengruppe dar und ein sp³-Kohlenstoffatom an irgendeiner Position von A¹ ist durch -O- ersetzt,
(iv) A¹ stellt eine C₁₋₈-Alkylengruppe dar und ein sp³-Kohlenstoffatom an irgendeiner Position von A¹ ist durch -NR¹⁴- ersetzt,
(v) A¹ stellt eine C₁₋₈-Alkylengruppe dar, ein sp³-Kohlenstoffatom an irgendeiner Position von A¹ ist durch -NR¹⁴- ersetzt und ein sp³-Kohlenstoffatom an irgendeiner Position von A¹ ist gegebenenfalls durch -O- ersetzt.

7. Verbindung oder pharmazeutisch annehmbares Salz gemäß irgendeinem der Ansprüche 1 bis 6, worin A² so wie in (i) oder (ii) definiert ist:
(i) A² stellt eine 4- bis 12-gliedrige Heterocyclylengruppe dar und A² ist gegebenenfalls mit einem bis vier Substituenten, ausgewählt aus der Gruppe, bestehend aus -OH, -COOH, -SO₃H, -PO₃H₂, -CN, -NO₂, einem Halogenatom, einer C₁₋₈-Alkylgruppe, die mit null bis zwei -OH-Gruppen, null bis zwei -OR⁴⁵-Gruppen und null bis sechs Fluoratomen substituiert ist, einer C₃₋₁₂-Cycloalkylgruppe, die mit null bis zwei -OH-Gruppen, null bis zwei -OR⁴⁶-Gruppen und null bis sechs Fluoratomen subsituiert ist, einer C₁₋₈-Alkoxygruppe, die mit null bis zwei -OH-Gruppen, null bis zwei -OR⁴⁷-Gruppen und null bis sechs Fluoratomen substituiert ist, und einer 4- bis 2-gliedrigen Heterocyclylgruppe, die mit null bis zwei -OH-Gruppen, null bis zwei -OR⁴⁹-Gruppen und null bis sechs Fluoratomen substituiert ist, substituiert ist,
(ii) A² stellt eine 4- bis 12-gliedrige Heterocyclylengruppe dar, die mit =O substituiert ist, und A² ist gegebenenfalls mit einem bis vier Substituenten, ausgewählt aus der Gruppe, bestehend aus-OH, =O, -COOH, -SO₃H, -PO₃H₂, -CN, -NO₂, einem Halogenatom, einer C₁₋₈-Alkylgruppe, die mit null bis zwei -OH-Gruppen, null bis zwei -OR⁴⁵-Gruppen und null bis sechs Fluoratomen substituiert ist, eine C₃₋₁₂-Cycloalkylgruppe, die mit null bis zwei -OH Gruppen null bis zwei -OR⁴⁶-Gruppen und null bis sechs Fluoratomen substituiert ist, einer C₁₋₈-Alkoxygruppe, die mit null bis zwei -OH-Gruppen, null bis zwei -OR⁴⁷-Gruppen und null bis sechs Fluoratomen substituiert ist, und einer 4- bis 12-gliedrigen Heterocyclylgruppe, die mit null bis zwei -OH-Gruppen, null bis zwei -OR⁴⁹-Gruppen und null bis sechs Fluoratomen substituiert ist, substituiert ist.

8. Verbindung oder pharmazeutisch annehmbares Salz davon gemäß irgendeinem der Ansprüche 1 bis 7, worin X -CR¹¹ darstellt, Y -CR¹² darstellt, Z -CR¹³ darstellt und R¹¹ oder R¹³ an A¹, A² oder A³ über eine Einfachbindung -O-, -NR⁵¹- oder -S(=O)ₚ- gebunden ist, um einen Ring zu bilden.

9. Verbindung oder pharmazeutisch annehmbares Salz davon gemäß irgendeinem der Ansprüche 1 bis 8,
worin A³ ein Wasserstoffatom ist oder
worin A³ ein Halogenatom, -CN, -R²⁵, -OR²⁶, -NR²⁷R²⁸, -C(=O)R²⁹ oder -C(=O)-OR³⁰ ist und R²⁵ bis R³⁰ jeweils unabhängig ein Wasserstoffatom, eine gegebenenfalls substituierte C₁₋₈-Alkylgruppe, eine gegebenenfalls substituierte 4- bis 12-gliedrige Heterocyclylgruppe, eine gegebenenfalls substituierte C₃₋₁₂-Cycloalkylgruppe, eine gegebenenfalls substituierte (4-bis 12-gliedrige Heterocyclyl)-C₁₋₃-alkylgruppe oder eine gegebenenfalls substituierte (C₃₋₁₂-Cycloalkyl)-C₁₋₃-alkylgruppe darstellen.

10. Verbindung oder pharmazeutisch annehmbares Salz davon gemäß irgendeinem der Ansprüche 1 bis 9, worin R³ ein Wasserstoffatom ist oder
worin R³ eine C₁₋₄-Alkylgruppe, ein Fluoratom oder ein Chloratom darstellt.

11. Verbindung, dargestellt durch Formel (I), oder ein pharmazeutisch annehmbares Salz davon gemäß Anspruch 1, die ausgewählt ist aus:
6-(Difluormethyl)-N8-isopropyl-N2-(5-piperazin-1-yl-2-pyridyl)pyrido[3,4-d]pyrimidin-2,8-diamin,
(1R)-1-[8-(Isopropylamino)-2-[(5-piperazin-1-yl-2-pyridyl)amino]pyrido[3,4-d]pyrimidin-6-yl]ethanol,
1-[2-[(5-Piperazin-1-yl-2-pyridyl)amino]-8-(tetrahydrofuran-3-ylamino)pyrido[3,4-d]pyrimidin-6-yl]ethanol,
1-[2-[(5-Piperazin-1-yl-2-pyridyl)amino]-8-(tetrahydropyran-3-ylamino)pyrido[3,4-d]pyrimidin-6-yl]ethanol,
N8-Isopropyl-6-[(1R)-1-methoxyethyl]-N2-(6-piperazin-1-ylpyridazin-3-yl)pyrido[3,4-d]pyrimidin-2,8-diamin,
N8-Isopropyl-6-[(1R)-1-methoxyethyl]-N2-[5-(piperazin-1-ylmethyl)-2-pyridyl]pyrido[3,4-d]pyrimidin-2,8-diamin,
1-[6-[[6-[(1R)-1-Hydroxyethyl]-8-(isopropylamino)pyrido[3,4-d]pyrimidin-2-yl]amino]-3-pyridyl]piperazin-2-on,
1-[6-[[5-Chlor-6-[(1R)-1-hydroxyethyl]-8-(isopropylamino)pyrido[3,4-d]pyrimidin-2-yl]amino]-3-pyridyl]piperazin-2-on,
(1R)-1-[2-[(6-Piperazin-1-ylpyridazin-3-yl)amino]-8-(tetrahydropyran-4-ylamino)pyrido[3,4-d]pyrimidin-6-yl]ethanol,
(1R)-1-[2-[(6-Piperazin-1-ylpyridazin-3-yl)amino]-8-[[(3S)-tetrahydropyran-3-yl]amino]pyrido[3,4-d]pyrimidin-6-yl]ethanol,
(1R)-1-[2-[(6-Piperazin-1-ylpyridazin-3-yl)amino]-8-[[(3R)-tetrahydropyran-3-yl]amino]pyrido[3,4-d]pyrimidin-6-yl]ethanol,
(1R)-1-[2-[[5-(Piperazin-1-ylmethyl)-2-pyridyl]amino]-8-(tetrahydropyran-4-ylamino)pyrido[3,4-d]pyrimidin-6-yl]ethanol,
(1R)-1-[2-[[5-(Piperazin-1-ylmethyl)-2-pyridyl]amino]-8-[[(3S)-tetrahydropyran-3-yl]amino]pyrido[3,4-d]pyrimidin-6-yl]ethanol,
(1R)-1-[2-[[5-(Piperazin-1-ylmethyl)-2-pyridyl]amino]-8-[[(3R)-tetrahydropyran-3-yl]amino]pyrido[3,4-d]pyrimidin-6-yl]ethanol,
1-[6-[[6-[(1R)-1-Hydroxyethyl]-8-(isopropylamino)pyrido[3,4-d]pyrimidin-2-yl]amino]pyridazin-3-yl]piperidin-4-ol,
(1R)-1-[8-(Isopropylamino)-2-[(6-piperazin-1-ylpyridazin-3-yl)amino]pyrido[3,4-d]pyrimidin-6-yl]ethanol,
1-[[6-[[6-[(1R)-1-Hydroxyethyl]-8-(isopropylamino)pyrido[3,4-d]pyrimidin-2-yl]amino]-3-pyridyl]methyl]piperazin-2-on,
6-[(1R)-1-Methoxyethyl]-N2-[5-(piperazin-1-ylmethyl)-2-pyridyl]-N8-[(3S)-tetrahydropyran-3-yl]pyrido[3,4-d]pyrimidin-2,8-diamin,
6-[(1R)-1-Methoxyethyl]-N2-(6-piperazin-1-ylpyridazin-3-yl)-N8-[(3S)-tetrahydropyran-3-yl]pyrido[3,4-d]pyrimidin-2,8-diamin,
6-[(lR)-1-Methoxyethyl]-N2-[5-(piperazin-1-ylmethyl)-2-pyridyl]-N8-(tetrahydropyran-4-ylmethyl)pyrido[3,4-d]pyrimidin-2,8-diamin,
N8-Isopropyl-6-[(1R)-1-methoxyethyl]-N2-(5-piperazin-1-ylpyrazin-2-yl)pyrido[3,4-d]pyrimidin-2,8-diamin,
N8-Isopropyl-6-[(1R)-1-methoxyethyl]-N2-[6-[(2S)-2-methylpiperazin-1-yl]pyridazin-3-yl]pyrido[3,4-d]pyrimidin-2,8-diamin,
N8-Isopropyl-6-[(1R)-1-methoxyethyl]-N2-[6-[(2R)-2-methylpiperazin-1-yl]pyridazin-3-yl]pyrido[3,4-d]pyrimidin-2,8-diamin,
(1R)-1-[2-[[6-(4,7-Diazaspiro[2.5]octan-7-yl)pyridazin-3-yl]amino]-8-(isopropylamino)pyrido[3,4-d]pyrimidin-6-yl]ethanol,
(1R)-1-[2-[[5-(4,7-Diazaspiro[2.5]octan-7-ylmethyl)-2-pyridyl]amino]-8-(isopropylamino)pyrido[3,4-d]pyrimidin-6-yl]ethanol,
2-[1-[[6-[[6-[(1R)-1-Hydroxyethyl]-8-(isopropylamino)pyrido[3,4-d]pyrimidin-2-yl]amino]-3-pyridyl]methyl]-4-piperidyl]propan-2-ol,
(1R)-1-[2-[[5-[[4-(2-Hydroxyethyl)piperazin-1-yl]methyl]-2-pyridyl]amino]-8-(isopropylamino)pyrido[3,4-d]pyrimidin-6-yl]ethanol,
(1R)-1-[2-[[5-[2-(Dimethylamino)ethoxy]-2-pyridyl]amino]-8-[[(3S)-tetrahydropyran-3-yl]amino]pyrido[3,4-d]pyrimidin-6-yl]ethanol,
(1R)-1-[2-[[6-(4-Methylpiperazin-1-yl)pyridazin-3-yl]amino]-8-[[(3S)-tetrahydropyran-3-yl]amino]pyrido[3,4-d]pyrimidin-6-yl]ethanol,
2-Hydroxy-1-[4-[6-[[6-[(1R)-1-hydroxyethyl]-8-(isopropylamino)pyrido[3,4-d]pyrimidin-2-yl]amino]pyridazin-3-yl]piperazin-1-yl]ethanon,
1-[6-[[8-(Isopropylamino)-6-[(2S)-tetrahydrofuran-2-yl]pyrido[3,4-d]pyrimidin-2-yl]amino]-3-pyridyl]piperazin-2-on,
(1R)-1-[8-(Isopropylamino)-2-(5,6,7,8-tetrahydro-1,6-naphthyridin-2-ylamino)pyrido[3,4-d]pyrimidin-6-yl]ethanol,
2-[4-[[6-[[6-[(1R)-1-Hydroxyethyl]-8-(isopropylamino)pyrido[3,4-d]pyrimidin-2-yl]amino]-3-pyridyl]methyl]piperazin-1-yl]-2-methyl-propan-1-ol,
4-[6-[[6-[(1R)-1-Hydroxyethyl]-8-(isopropylamino)pyrido[3,4-d]pyrimidin-2-yl]amino]-3-pyridyl]-1-[(2S)-2-hydroxypropyl]-1,4-diazepan-5-on,
4-[6-[[6-[(1R)-1-Hydroxyethyl]-8-(isopropylamino)pyrido[3,4-d]pyrimidin-2-yl]amino]-3-pyridyl]-1-[(2R)-2-hydroxypropyl]-1,4-diazepan-5-on,
N8-Isopropyl-N2-[5-(piperazin-1-ylmethyl)-2-pyridyl]-6-[(2S)-tetrahydrofuran-2-yl]pyrido[3,4-d]pyrimidin-2,8-diamin,
1-[6-[[6-[(1R)-1-Hydroxyethyl]-8-(isopropylamino)pyrido[3,4-d]pyrimidin-2-yl]amino]-2-methyl-3-pyridyl]piperazin-2-on,
1-[6-[[8-(Isopropylamino)-6-[(3S)-tetrahydrofuran-3-yl]pyrido[3,4-d]pyrimidin-2-yl]amino]-3-pyridyl]piperazin-2-on,
(1R)-1-[2-(5,6,7,8-Tetrahydro-1,6-naphthyridin-2-ylamino)-8-[[(3S)-tetrahydropyran-3-yl]amino]pyrido[3,4-d]pyrimidin-6-yl]ethanol,
1-[6-[[8-(Isopropylamino)-6-(3-methyloxetan-3-yl)pyrido[3,4-d]pyrimidin-2-yl]amino]-3-pyridyl]piperazin-2-on,
(1R)-1-[2-[[5-[4-(Dimethylamino)cyclohexoxy]-2-pyridyl]amino]-8-[[(3S)-tetrahydropyran-3-yl]amino]pyrido[3,4-d]pyrimidin-6-yl]ethanol,
6-[(1R)-1-Methoxyethyl]-N2-[5-(piperazin-1-ylmethyl)-2-pyridyl]-N8-propyl-pyrido[3,4-d]pyrimidin-2,8-diamin,
6-[(1R)-1-Methoxyethyl]-N2-(6-piperazin-1-ylpyridazin-3-yl)-N8-propyl-pyrido[3,4-d]pyrimidin-2,8-diamin,
1-[[6-[[6-(Difluormethyl)-8-[(4-methylcyclohexyl)amino]pyrido[3,4-d]pyrimidin-2-yl]amino]-3-pyridyl]methyl]piperidin-4-carbonsäure,
(1R)-1-[8-(Ethylamino)-2-[[5-[[4-(2-hydroxyethyl)piperazin-1-yl]methyl]-2-pyridyl]amino]pyrido[3,4-d]pyrimidin-6-yl]ethanol,
(1R)-1-[2-[[5-[[4-(2-Hydroxyethyl)piperazin-1-yl]methyl]-2-pyridyl]amino]-8-(propylamino)pyrido[3,4-d]pyrimidin-6-yl]ethanol,
N8-Isopropyl-6-(3-methyloxetan-3-yl)-N2-(6-piperazin-1-ylpyridazin-3-yl)pyrido[3,4-d]pyrimidin-2,8-diamin,
N8-Isopropyl-6-(3-methyloxetan-3-yl)-N2-[5-(piperazin-1-ylmethyl)-2-pyridyl]pyrido[3,4-d]pyrimidin-2,8-diamin,
6-(3-Methyloxetan-3-yl)-N2-[5-(piperazin-1-ylmethyl)-2-pyridyl]-N8-[(3S)-tetrahydropyran-3-yl]pyrido[3,4-d]pyrimidin-2,8-diamin,
4-[6-[[6-[(1R)-1-Hydroxyethyl]-8-[isopropyl(methyl)amino]pyrido[3,4-d]pyrimidin-2-yl]amino]-3-pyridyl]-1,4-diazepan-5-on,
(1R)-1-[8-(Isopropylamino)-2-[(6-methyl-5-piperazin-1-yl-2-pyridyl)amino]pyrido[3,4-d]pyrimidin-6-yl]ethanol,
(1R)-1-[2-[[6-(2-Hydroxyethyl)-7,8-dihydro-5H-1,6-naphthyridin-2-yl]amino]-8-(isopropylamino)pyrido[3,4-d]pyrimidin-6-yl]ethanol,
(1R)-1-[8-(Isopropylamino)-2-[[6-[2-(methylamino)ethyl]-7,8-dihydro-5H-1,6-naphthyridin-2-yl]amino]pyrido[3,4-d]pyrimidin-6-yl]ethanol,
N2-(6-Piperazin-1-ylpyridazin-3-yl)-6-[(3S)-tetrahydrofuran-3-yl]-N8-[(3S)-tetrahydropyran-3-yl]pyrido[3,4-d]pyrimidin-2,8-diamin,
N2-[5-(Piperazin-1-ylmethyl)-2-pyridyl]-6-[(3R)-tetrahydrofuran-3-yl]-N8-[(3S)-tetrahydropyran-3-yl]pyrido[3,4-d]pyrimidin-2,8-diamin,
(1R)-1-[2-[[6-[2-(Dimethylamino)ethyl]-7,8-dihydro-5H-1,6-naphthyridin-2-yl]amino]-8-(isopropylamino)pyrido[3,4-d]pyrimidin-6-yl]ethanol,
(2S)-1-[4-[[6-[[8-(Ethylamino)-6-[(1R)-1-hydroxyethyl]pyrido[3,4-d]pyrimidin-2-yl]amino]-3-pyridyl]methyl]piperazin-1-yl]propan-2-ol,
(2R)-1-[4-[[6-[[8-(Ethylamino)-6-[(1R)-1-hydroxyethyl]pyrido[3,4-d]pyrimidin-2-yl]amino]-3-pyridyl]methyl]piperazin-1-yl]propan-2-ol,
(1R)-1-[8-(Isopropylamino)-2-[[5-[(2R)-2-methylpiperazin-1-yl]-2-pyridyl]amino]pyrido[3,4-d]pyrimidin-6-yl]ethanol,
(1R)-1-[8-(Isopropylamino)-2-[[5-[(2S)-2-methylpiperazin-1-yl]-2-pyridyl]amino]pyrido[3,4-d]pyrimidin-6-yl]ethanol,
N8-Isopropyl-N2-(5-piperazin-1-yl-2-pyridyl)-6-[(2S)-tetrahydrofuran-2-yl]pyrido[3,4-d]pyrimidin-2,8-diamin,
(1R)-1-[8-(Cyclobutylamino)-2-[[5-[[4-(2-hydroxyethyl)piperazin-1-yl]methyl]-2-pyridyl]amino]pyrido[3,4-d]pyrimidin-6-yl]ethanol,
(1R)-1-[8-(Cyclopropylmethylamino)-2-[[5-[[4-(2-hydroxyethyl)piperazin-1-yl]methyl]-2-pyridyl]amino]pyrido[3,4-d]pyrimidin-6-yl]ethanol,
6-(3-Methyloxetan-3-yl)-N2-(5-piperazin-1-yl-2-pyridyl)-N8-propyl-pyrido[3,4-d]pyrimidin-2,8-diamin,
6-(3-Methyloxetan-3-yl)-N2-[5-(piperazin-1-ylmethyl)-2-pyridyl]-N8-propyl-pyrido[3,4-d]pyrimidin-2,8-diamin,
N2-(5-Piperazin-1-yl-2-pyridyl)-N8-propyl-6-tetrahydrofuran-3-yl-pyrido[3,4-d]pyrimidin-2,8-diamin,
N2-[5-(Piperazin-1-ylmethyl)-2-pyridyl]-N8-propyl-6-tetrahydrofuran-3-yl-pyrido[3,4-d]pyrimidin-2,8-diamin,
N8-Isopropyl-6-(3-methyloxetan-3-yl)-N2-(5-piperazin-1-yl-2-pyridyl)pyrido[3,4-d]pyrimidin-2,8-diamin,
N8-Isopropyl-N2-(5-piperazin-1-yl-2-pyridyl)-6-tetrahydrofuran-3-yl-pyrido[3,4-d]pyrimidin-2,8-diamin,
2-[4-[[6-[[8-(Isopropylamino)-6-tetrahydrofuran-3-yl-pyrido[3,4-d]pyrimidin-2-yl]amino]-3-pyridyl]methyl]piperazin-1-yl]ethanol,
2-[4-[[6-[[6-Tetrahydrofuran-3-yl-8-[[(3S)-tetrahydropyran-3-yl]amino]pyrido[3,4-d]pyrimidin-2-yl]amino]-3-pyridyl]methyl]piperazin-1-yl]ethanol,
(1R)-1-[2-[[5-[[4-(Hydroxymethyl)-1-piperidyl]methyl]-2-pyridyl]amino]-8-(isopropylamino)pyrido[3,4-d]pyrimidin-6-yl]ethanol,
1-[[6-[[6-[(1R)-1-Hydroxyethyl]-8-(isopropylamino)pyrido[3,4-d]pyrimidin-2-yl]amino]-3-pyridyl]methyl]piperidin-4-ol,
1-[[6-[[8-(tert-Butylamino)-6-[(1R)-1-hydroxyethyl]pyrido[3,4-d]pyrimidin-2-yl]amino]-3-pyridyl]methyl]piperidin-4-ol,
(1R)-1-[8-(tert-Butylamino)-2-[[5-[[4-(hydroxymethyl)-1-piperidyl]methyl]-2-pyridyl]amino]pyrido[3,4-d]pyrimidin-6-yl]ethanol,
1-[[6-[[6-[(1R)-1-Hydroxyethyl]-8-(isobutylamino)pyrido[3,4-d]pyrimidin-2-yl]amino]-3-pyridyl]methyl]piperidin-4-ol,
(1R)-1-[2-[[5-[[4-(Hydroxymethyl)-1-piperidyl]methyl]-2-pyridyl]amino]-8-(isobutylamino)pyrido[3,4-d]pyrimidin-6-yl]ethanol,
1-[6-[[6-[(1R)-1-Hydroxypropyl]-8-(isopropylamino)pyrido[3,4-d]pyrimidin-2-yl]amino]-3-pyridyl]piperazin-2-on,
(1R)-1-[2-[[5-[[4-(2-Hydroxyethyl)piperazin-1-yl]methyl]-6-methyl-2-pyridyl]amino]-8-(propylamino)pyrido[3,4-d]pyrimidin-6-yl]ethanol.

12. Pharmazeutische Zusammensetzung, umfassend die Verbindung oder das pharmazeutisch annehmbare Salz davon gemäß irgendeinem der Ansprüche 1 bis 11 und einen pharmazeutisch annehmbaren Träger.

13. Pharmazeutische Zusammensetzung, die eine CDK4/6-hemmende Aktivität aufweist, umfassend die Verbindung oder das pharmazeutisch annehmbare Salz davon gemäß irgendeinem der Ansprüche 1 bis 11 als Wirkstoff.

14. Arzneimittel zur Verwendung bei der Vorbeugung oder Behandlung von rheumatoider Arthritis, Arteriosklerose, Lungenfibrose, Hirninfarkt oder Krebs, wobei das Arzneimittel die Verbindung oder das pharmazeutisch annehmbare Salz davon gemäß irgendeinem der Ansprüche 1 bis 11 als Wirkstoff umfasst.

## Revendications

1. Composé représenté par la formule (I) ou un sel pharmaceutiquement acceptable de celui-ci : 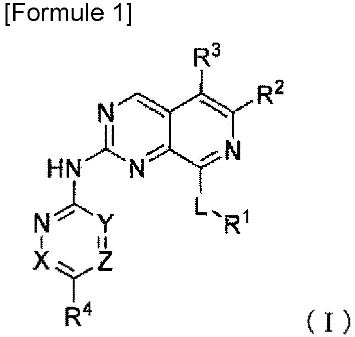 [où
L représente -NR⁵-, -O-, ou -S- ;
R⁵ représente un atome d'hydrogène ou un groupe alkyle en C₁₋₆ substitué par zéro à deux groupes -OH, zéro à deux groupes alcoxy en C₁₋₈, et zéro à six atomes de fluor ;
R¹ représente un groupe alkyle en C₁₋₈, un cycloalkyle en C₃₋₁₂, un (cycloalkyle en C₃₋₁₂)-C₁₋₆alkyle, un hétérocyclyle de 4 à 12 chaînons, un (hétérocyclyle de 4 à 12 chaînons)-C₁₋₆alkyle, un aryle en C₆₋₁₀, un (aryle en C₆₋₁₀)-C₁₋₆alkyle, un hétéroaryle de 5 à 10 chaînons, un (hétéroaryle de 5 à 10 chaînons)-C₁₋₆alkyle, un alkylsulfonyle en C₁₋₈ ou acyle en C₁₋₈ ;
chacun des groupes contenant des hétéroatomes représentés par R¹ contient un à quatre hétéroatomes choisis parmi les atomes d'oxygène, de soufre et d'azote ;
R¹ est optionnellement substitué par un à six substituants choisis dans le groupe constitué par un atome d'halogène, =O, -OH, -CN, -COOH, -COOR⁶, -R⁷, un groupe cycloalkyle en C₃₋₆ substitué par zéro à deux groupes -OH, zéro à deux groupes alcoxy en C₁₋₈, et zéro à six atomes de fluor, un groupe hétérocyclyle à 3 à 10 chaînons substitué par zéro à deux groupes -OH, zéro à deux groupes alcoxy en C₁₋₈, et zéro à six atomes de fluor, un groupe acyle en C₁₋₈ substitué par zéro à deux groupes -OH, zéro à deux groupes alcoxy en C₁₋₈, et zéro à six atomes de fluor, et un groupe alcoxy en C₁₋₈ substitué par zéro à deux groupes -OH, zéro à deux groupes alcoxy en C₁₋₈, et zéro à six atomes de fluor ;
R⁶ et R⁷ représentent chacun indépendamment un groupe alkyle en C₁₋₆ substitué par zéro à deux groupes -OH, zéro à deux groupes alcoxy en C₁₋₈, et zéro à six atomes de fluor ;
R² représente un groupe alkyle en C₁₋₈, un cycloalkyle en C₃₋₈, un hétérocyclyle à 4 ou 6 chaînons, un acyle en C₁₋₈, -COOR⁸ ou -CONR⁹R¹⁰ ;
chacun des groupes alkyle en C₁₋₈ et cycloalkyle en C₃₋₈ représentés par R² est substitué par zéro ou un groupe -OH, zéro à deux groupes alcoxy en C₁₋₈ substitués par zéro ou un groupe -OH, zéro ou un groupe alcoxy en C₁₋₄, et zéro à trois atomes de fluor, et zéro à cinq atomes de fluor ;
R² n'est ni un alkyle en C₁₋₈ non substitué, ni un cycloalkyle en C₃₋₈ non substitué, ni un groupe trifluorométhyle ;
R⁸, R⁹ et R¹⁰ représentent chacun indépendamment un atome d'hydrogène ou un groupe alkyle en C₁₋₈ ;
le groupe hétérocyclyle à 4 à 6 chaînons représenté par R² est optionellement substitué par un à quatre substituants choisis dans le groupe constitué par un atome de fluor, -OH, et des groupes alkyle en C₁₋₄ et alcoxy en C₁₋₄ ;
chacun du groupe acyle en C₁₋₈, -COOR⁸ et -CONR⁹R¹⁰ représentés par R² est optionnellement substitué par un à quatre substituants choisis dans le groupe constitué par un atome de fluor, -OH et un groupe alcoxy en C₁₋₄ ;
R⁹ et R¹⁰ de -CONR⁹R¹⁰ représenté par R² sont optionnellement liés par une liaison simple ou -O- pour former un cycle incluant l'atome d'azote lié à R⁹ et R¹⁰ ;
le groupe hétérocyclyle représenté par R² ayant un cycle à 4 ou 5 chaînons contient un hétéroatome oxygène, et le groupe hétérocyclyle ayant un cycle à 6 chaînons contient un ou deux hétéroatomes oxygène ;
R³ représente un atome d'hydrogène, un groupe alkyle en C₁₋₈, ou un atome d'halogène ;
X représente CR¹¹ ou un atome d'azote ;
Y représente CR¹² ou un atome d'azote ;
Z représente CR¹³ ou un atome d'azote ;
R¹¹ à R¹³ représentent chacun indépendamment un atome d'hydrogène, de fluor ou de chlore ou un groupe alkyle en C₁₋₆ ou alcoxy en C₁₋₆ ;
R⁴ représente -A¹-A²-A³ ;
A¹ représente une liaison simple ou un groupe alkylène en C₁₋₈, alcénylène en C₂₋₈ ou alcynylène en C₂₋₈ ;
un ou deux atomes de carbone sp³ à n'importe quelle position de A¹ sont optionnellement remplacés par une ou deux structures choisies dans le groupe constitué par -O-, -NR¹⁴-, -C(=O)-, -C(=O)-O-, -O-C(=O)-, -O-C(=O)-O-, -C(=O)-NR¹⁵-, -O-C(=O)-NR¹⁶-, -NR¹⁷-C(=O)-, -NR¹⁸-C(=O)-O-, -NR¹⁹-C(=O)-NR²⁰-,-S(=O)ₚ-, -S(=O)₂-NR²¹-, -NR²²-S(=O)₂-, et -NR²³S(=O)₂-NR²⁴-, et une structure -O-O-, -O-NR¹⁴-, -NR¹⁴-O-, -O-CH₂O- , -O-CH₂-NR¹⁴-, ou -NR¹⁴-CH₂O- n'est pas formée dans le cas du remplacement de deux atomes de carbone sp³ ;
A² représente une liaison simple ou un groupe alkylène en C₁₋₇, un cycloalkylène en C₃₋₁₂, un cycloalkylidène en C₃₋₁₂, un hétérocyclylène à 4 à 12 chaînons, un hétérocyclylidène à 4 à 12 chaînons, un arylène en C₆₋₁₀ ou un hétéroarylène à 5 à 10 chaînons ;
A³ représente un atome d'halogène, -CN, -NO₂, -R²⁵, -OR²⁶, -NR²⁷R²⁸, -C(=O)R²⁹,-C(=O)-OR³⁰, -O-C(=O)R³¹, -O-C(=O)-NR³²R³³, -C(=O)-NR³⁴R³⁵, -NR³⁶-C(=O)R³⁷,-NR³⁸-C (=O)-OR³⁹, - S (=O)₂-R⁴⁰, -S (=O)₂-NR⁴¹R⁴², ou -NR⁴³-S(=O)₂R⁴⁴ ;
A³ représente -R²⁵, si l'extrémité A¹ du côté A² a une structure choisie dans le groupe constitué par -O-, -NR¹⁴-, -C(=O)-, -C(=O)-O-, -O-C(=O)-, -O-C(=O)-O-,-C(=O)-NR¹⁵-, -O-C(=O)-NR¹⁶-, -NR¹⁷-C(=O)-, -NR¹⁸-C(=O)-O-, -NR¹⁹-C(=O)-NR²⁰-, -S(=O)ₚ-, -S(=O)₂-NR²¹-, -NR²²-S(=O)₂-, et -NR²³-S(=O)₂-NR²⁴- et A² est une liaison simple ;
R¹⁴, R³², R³⁴, R³⁶, R³⁸, R⁴¹ et R⁴³ représentent chacun indépendamment un atome d'hydrogène ou un groupe alkyle en C₁₋₈, un acyle en C₁₋₈, un alkylsulfonyle en C₁₋₈, un hétérocyclyle de 4 à 12 chaînons, un cycloalkyle en C₃₋₁₂, un aryle en C₆₋₁₀, un hétéroaryle à 5 à 10 chaînons, un (hétérocyclyle à 4 à 12 chaînons)- C₁₋₃ alkyle, un (cycloalkyle en C₃₋₁₂)-C₁₋₃ alkyle, un (aryle en C₆₋₁₀)- C₁₋₃ alkyle, ou un (hétéroaryle à 5 à 10 chaînons)- C₁₋₃ alkyle;
R¹⁵ à R³¹, R³³, R³⁵, R³⁷, R³⁹, R⁴⁰, R⁴² et R⁴⁴ représentent chacun indépendamment un atome d'hydrogène ou un groupe alkyle en C₁₋₈, un hétérocyclyle à 4 à 12 chaînons, un cycloalkyle en C₃₋₁₂, un aryle en C₆₋₁₀, un hétéroaryle à 5 à 10 chaînons, un (hétérocyclyle à 4 à 12 chaînons)- C₁₋₃ alkyle, un (cycloalkyle en C_{3- 12})-C₁₋₃ alkyle, un (aryle en C₆₋₁₀)-C₁₋₃ alkyle, ou un (hétéroaryle à 5 à 10 chaînons)-C₁₋₃ alkyle;
A¹, A², A³, et R¹⁴ à R⁴⁴ dans A¹ et A³ sont chacun optionnellement substitués par un à quatre substituants choisis dans le groupe constitué par -OH, =O, -COOH, -SO₃H, -PO₃H₂, - CN, -NO₂, un atome d'halogène, un groupe alkyle en C₁₋₈ substitué par zéro à deux groupes -OH, zéro à deux groupes -OR⁴⁵, et zéro à six atomes de fluor, un groupe cycloalkyle en C₃₋₁₂ substitué par zéro à deux groupes -OH, zéro à deux groupes -OR⁴⁶, et zéro à six atomes de fluor, un groupe alcoxy en C1-8 substitué par zéro à deux groupes -OH, zéro à deux groupes -OR⁴⁷, et zéro à six atomes de fluor, et un groupe hétérocyclyle à 4 à 12 chaînons substitué par zéro à deux groupes -OH, zéro à deux groupes -OR⁴⁹, et zéro à six atomes de fluor ;
R¹⁴ à R⁴⁴ sont optionnellement liés en A¹ ou A³ ou entre A¹ et A², entre A¹ et A³, ou entre A² et A³ par une liaison simple, -O-, -NR⁵⁰-, ou -S (=O)_{P}- pour former un cycle; R¹¹ ou R¹³ est optionnellement lié à A¹, A² ou A³ par une liaison simple, -O-, -NR⁵¹- ou -S(=O)ₚ- pour former un cycle ;
R⁴⁵, R⁴⁶, R⁴⁷, R⁴⁹, R⁵⁰ et R⁵¹ représentent chacun un atome d'hydrogène ou un groupe alkyle en C₁₋₄ substitué par zéro ou un groupe -OH et zéro à six atomes de fluor ;
p représente un nombre entier de 0 à 2 ; et
chacun des groupes contenant des hétéroatomes représentés par A¹, A² et A³ contient un à quatre hétéroatomes choisis parmi les atomes d'oxygène, de soufre et d'azote].

2. Composé ou sel pharmaceutiquement acceptable de celui-ci selon la revendication 1, dans lequel L représente - NH-.

3. Composé ou sel pharmaceutiquement acceptable de celui-ci selon la revendication 1 ou 2, dans lequel R¹ représente un groupe alkyle en C₁₋₈, un cycloalkyle en C₃₋₁₂, un (cycloalkyle en C₃₋₁₂)-C₁₋₆ alkyle, un hétérocyclyle à 4 à 12 chaînons, ou un (hétérocyclyle à 4 à 12 chaînons)- C₁₋₆ alkyle.

4. Composé ou sel pharmaceutiquement acceptable de celui-ci selon l'une quelconque des revendications 1 à 3, dans lequel R² est l'un des (i) à (iv) suivants
(i) un groupe alkyle en C₁₋₈ substitué par un à quatre atomes de fluor ;
(ii) un groupe alkyle en C₁₋₈ substitué par zéro ou un groupe -OH et zéro à deux groupes alcoxy en C₁₋₈ substitués par zéro ou un groupe -OH, zéro ou un groupe alcoxy en C₁₋₄, et zéro à trois atomes de fluor ;
(iii) un groupe hétérocyclyle de 4 à 6 chaînons optionnellement substitué par un à quatre substituants choisis dans le groupe constitué par un atome de fluor, -OH, et des groupes alkyle en C₁₋₄ et alcoxy en C₁₋₄ ;
(iv) -COOR⁸, -CONR⁹R¹⁰, ou un groupe acyl. en C₁₋₈, chaque groupe étant optionnellement substitué par un à quatre substituants choisis dans le groupe constitué par un atome de fluor, -OH, et un groupe alcoxy en C₁₋₄.

5. Composé ou sel pharmaceutiquement acceptable de celui-ci selon l'une quelconque des revendications 1 à 4, dans lequel X, Y et Z sont définis comme dans l'un des (i) à (iv) suivants :
(i) X représente CR¹¹, Y représente CR¹², et Z représente CR¹³ ;
(ii) X représente un atome d'azote, Y représente CR¹², et Z représente CR¹³ ;
(iii) X représente CR¹¹, Y représente un atome d'azote, et Z représente CR¹³ ;
(iv) X représente CR¹¹, Y représente CR¹², et Z représente un atome d'azote.

6. Composé ou sel pharmaceutiquement acceptable de celui-ci selon l'une quelconque des revendications 1 à 5, dans lequel A¹ est défini comme dans l'une des (i) à (v) suivantes
(i) A¹ est une liaison simple ;
(ii) A¹ représente un groupe alkylène en C₁₋₈, et aucun atome de carbone sp³ dans A¹ n'est remplacé par une autre structure ;
(iii) A¹ représente un groupe alkylène en C₁₋₈, et un atome de carbone sp³ en toute position de A¹ est remplacé par -O- ;
(iv) A¹ représente un groupe alkylène en C₁₋₈, et un atome de carbone sp³ en toute position de A¹ est remplacé par -NR¹⁴- ;
(v) A¹ représente un groupe alkylène en C₁₋₈, un atome de carbone sp³ en toute position de A1 est remplacé par -NR¹⁴-, et un atome de carbone sp³ en toute autre position de A¹ est optionnellement remplacé par -O-.

7. Composé ou sel pharmaceutiquement acceptable de celui-ci selon l'une quelconque des revendications 1 à 6, dans lequel A² est défini comme dans (i) ou (ii) :
(i) A² représente un groupe hétérocyclylène à 4 à 12 chaînons ; et A² est optionnellement substitué par un à quatre substituants choisis dans le groupe constitué par -OH, -COOH, -SO₃H, -PO₃H₂, -CN, -NO₂, un atome d'halogène, un groupe alkyle en C₁₋₈ substitué par zéro à deux groupes -OH, zéro à deux groupes -OR⁴⁵, et zéro à six atomes de fluor, un groupe cycloalkyle en C₃₋₁₂ substitué par zéro à deux groupes -OH, zéro à deux groupes -OR⁴⁶, et zéro à six atomes de fluor, un groupe alcoxy en C₁₋₈ substitué par zéro à deux groupes -OH, zéro à deux groupes -OR⁴⁷, et zéro à six atomes de fluor, et un groupe hétérocyclyle à 4 à 12 chaînons substitué par zéro à deux groupes -OH, zéro à deux groupes -OR⁴⁹, et zéro à six atomes de fluor ;
(ii) A² représente un groupe hétérocyclylène à 4 à 12 chaînons substitué par =O ; et A² est optionnellement substitué par un à quatre substituants choisis dans le groupe constitué par -OH, =O, -COOH, -SO₃H, -PO₃H₂, -CN, -NO₂, un atome d'halogène, un groupe alkyle en C₁₋₈ substitué par zéro à deux groupes -OH, zéro à deux groupes -OR⁴⁵, et zéro à six atomes de fluor, un groupe cycloalkyle en C₃₋₁₂ substitué par zéro à deux groupes -OH, zéro à deux groupes -OR⁴⁶, et zéro à six atomes de fluor, un groupe alcoxy en C₁₋₈ substitué par zéro à deux groupes -OH, zéro à deux groupes -OR⁴⁷, et zéro à six atomes de fluor, et un groupe hétérocyclyle à 4 à 12 chaînons substitué par zéro à deux groupes -OH, zéro à deux groupes - OR⁴⁹, et zéro à six atomes de fluor.

8. Composé ou sel pharmaceutiquement acceptable de celui-ci selon l'une quelconque des revendications 1 à 7, dans lequel X représente CR¹¹, Y représente CR¹², Z représente CR¹³, et R¹¹ ou R¹³ est lié à A¹, A² ou A³ par une liaison simple, -O-, -NR⁵¹- ou -S(=O)ₚ- pour former un cycle.

9. Composé ou sel pharmaceutiquement acceptable de celui-ci selon l'une quelconque des revendications 1 à 8, dans lequel A³ est un atome d'hydrogène ; ou dans lequel A³ est un atome d'halogène, -CN, -R²⁵, -OR²⁶,-NR²⁷R²⁸, -C(=O)R²⁹, ou -C(=O)-OR³⁰, et R²⁵ à R³⁰ représentent chacun indépendamment un atome d'hydrogène, un groupe alkyle en C₁₋₈ optionnellement substitué, un groupe hétérocyclyle à 4 à 12 chainons optionnellement substitué, un groupe cycloalkyle en C₃₋₁₂ optionnellement substitué, un groupe (hétérocyclyle à 4 à 12 chainons)-C₁₋₃ alkyle optionnellement substitué, ou un groupe (cycloalkyle en C₃₋₁₂)-C₁₋₃ alkyle optionnellement substitué.

10. Composé ou sel pharmaceutiquement acceptable de celui-ci selon l'une quelconque des revendications 1 à 9, dans lequel R³ est un atome d'hydrogène ; ou dans lequel R³ représente un groupe alkyle en C₁₋₄, un atome de fluor ou un atome de chlore.

11. Composé représenté par la formule (I) ou sel pharmaceutiquement acceptable de celui-ci selon la revendication 1, qui est choisi parmi :
6-(difluorométhyl)-N8-isopropyl-N2-(5-pipérazin-1-yl-2-pyridyl)pyrido[3,4-d]pyrimidine-2,8-diamine ;
(1R)-1-[8-(isopropylamino)-2-[(5-pipérazin-1-yl-2-pyridyl)amino]pyrido[3,4-d]pyrimidin-6-yl]éthanol ;
1-[2-[(5-pipérazin-1-yl-2-pyridyl)amino]-8-(tétrahydrofuran-3-ylamino)pyrido[3,4-d]pyrimidin-6-yl]éthanol ;
1-[2-[(5-pipérazin-1-yl-2-pyridyl)amino]-8-(tétrahydropyran-3-ylamino)pyrido[3,4-d]pyrimidin-6-yl]éthanol ;
N8-isopropyl-6-[(1R)-1-méthoxyéthyl]-N2-(6-pipérazin-1-ylpyridazin-3-yl)pyrido[3,4-d]pyrimidine-2,8-diamine ;
N8-isopropyl-6-[(1R)-1-méthoxyéthyl]-N2-[5-(pipérazin-1-ylméthyl)-2-pyridyl]pyrido[3,4-d]pyrimidine-2,8-diamine ;
1-[6-[[6-[(1R)-1-hydroxyéthyl]-8-(isopropylamino)pyrido[3,4-d]pyrimidin-2-yl]amino]-3-pyridyl]pipérazin-2-one ;
1-[6-[[5-chloro-6-[(1R)-1-hydroxyéthyl]-8-(isopropylamino)pyrido[3,4-d]pyrimidin-2-yl]amino]-3-pyridyl]pipérazin-2-one ;
(1R)-1-[2-[(6-pipérazin-1-ylpyridazin-3-yl)amino)-8-((tétrahydropyran-4-ylamino)pyrido[3,4-d]pyrimidin-6-yl]éthanol ;
(1R)-1-[2-[(6-pipérazin-1-ylpyridazin-3-yl)amino]-8-[[(3S)-tétrahydropyran-3-yl]amino]pyrido[3,4-d]pyrimidin-6-yl]éthanol ;
(1R)-1-[2-[(6-piperazin-1-ylpyridazin-3-yl)amino]-8-[[(3R)-tetrahydropyran-3-yl]amino]pyrido[3,4-d]pyrimidin-6-yl]ethanol ;
(1R)-1-[2-[[5-(pipérazin-1-ylméthyl)-2-pyridyl]amino]-8-(tétrahydropyran-4-ylamino)pyrido[3,4-d]pyrimidin-6-yl]éthanol ;
(1R)-1-[2-[[5-(pipérazin-1-ylméthyl)-2-pyridyl]amino]-8-[[(3S)-tétrahydropyran-3-yl]amino]pyrido[3,4-d]pyrimidin-6-yl]éthanol ;
(1R)-1-[2-[[5-(pipérazin-1-ylméthyl)-2-pyridyl]amino)-8-[[(3R)-tétrahydropyran-3-yl]amino]pyrido[3,4-d]pyrimidin-6-yl]éthanol ;
1-[6-[[6-[(1R)-1-hydroxyéthyl]-8-(isopropylamino)pyrido[3,4-d]pyrimidin-2-yl]amino]pyridazin-3-yl]pipéridin-4-ol ;
(1R)-1-[8-(isopropylamino)-2-[(6-pipérazin-1-ylpyridazin-3-yl)amino]pyrido[3,4-d]pyrimidin-6-yl]éthanol ;
1-[[6-[[6-[(1R)-1-hydroxyéthyl]-8-(isopropylamino)pyrido[3,4-d]pyrimidin-2-yl]amino]-3-pyridyl]méthyl]pipérazin-2-one ;
6-[(1R)-1-méthoxyéthyl]-N2-[5-(pipérazin-1-ylméthyl)-2-pyridyl]-N8-[(3S)-tétrahydropyran-3-yl]pyrido[3,4- d]pyrimidine-2,8-diamine ;
6-[(1R)-1-méthoxyéthyl]-N2-(6-pipérazin-1-ylpyridazin-3-yl)-N8-[(3S)-tétrahydropyran-3-yl]pyrido[3,4-d]pyrimidine-2,8-diamine ;
6-[(1R)-1-méthoxyéthyl]-N2-[5-(pipérazin-1-ylméthyl)-2-pyridyl]-N8-(tétrahydropyran-4-ylméthyl)pyrido[3,4- d]pyrimidine-2,8-diamine ;
N8-isopropyl-6-[(1R)-1-méthoxyéthyl]-N2-(5-pipérazin-1-ylpyrazin-2-yl)pyrido[3,4-d]pyrimidine-2,8-diamine ;
N8-isopropyl-6-[(1R)-1-méthoxyéthyl]-N2-[6-[(2S)-2-méthylpipérazin-1-yl]pyridazin-3-yl]pyrido[3,4-d]pyrimidine-2,8-diamine ;
N8-isopropyl-6-[(1R)-1-méthoxyéthyl]-N2-[6-[(2R)-2-méthylpipérazin-1-yl]pyridazin-3-yl]pyrido[3,4- d]pyrimidine-2,8-diamine ;
(1R)-1-[2-[[6-(4,7-diazaspiro[2.5]octan-7-yl)pyridazin-3-yl]amino]-8-(isopropylamino)pyrido[3,4-d]pyrimidin-6-yl]éthanol ;
(1R)-1-[2-[[5-(4,7-diazaspiro[2.5]octan-7-ylméthyl)-2-pyridyl]amino]-8-(isopropylamino)pyrido[3,4-d]pyrimidin-6-yl]éthanol ;
2-[1-[[6-[[6-[(1R)-1-hydroxyéthyl]-8-(isopropylamino)pyrido[3,4-d]pyrimidin-2-yl]amino]-3-pyridyl]méthyl]-4-piperidyl]propan-2-ol ;
(1R)-1-[2-[[5-[[4-(2-hydroxyéthyl)piperazin-1-yl]methyl]-2-pyridyl]amino]-8-(isopropylamino)pyrido[3,4-d]pyrimidin-6-yl]éthanol ;
(1R)-1-[2-[[5-[2-(diméthylamino)éthoxy]-2-pyridyl]amino]-8-[[(3S)-tétrahydropyran-3-yl]amino]pyrido[3,4- d]pyrimidin-6-yl]éthanol ;
(1R)-1-[2-[[6-(4-méthylpipérazin-1-yl)pyridazin-3-yl]amino]-8-[[(3S)-tétrahydropyran-3-yl]amino]pyrido[3,4- d]pyrimidin-6-yl]éthanol ;
2-hydroxy-1-[4-[6-[[6-[(1R)-1-hydroxyéthyl]-8-(isopropylamino)pyrido[3,4-d]pyrimidin-2-yl]amino]pyridazin-3-yl]pipérazin-1-yl]éthanone ;
1-[6-[[8-(isopropylamino)-6-[(2S)-tétrahydrofuran-2-yl]pyrido[3,4-d]pyrimidin-2-yl]amino]-3-pyridyl]pipérazin-2-one ;
(1R)-1-[8-(isopropylamino)-2-(5,6,7,8-tétrahydro-1,6-naphtyridin-2-ylamino)pyrido[3,4-d]pyrimidin-6-yl]éthanol ;
2-[4-[[6-[[6-[(1R)-1-hydroxyéthyl]-8-(isopropylamino)pyrido[3,4-d]pyrimidin-2-yl]amino]-3-pyridyl]méthyl]pipérazin-1-yl]-2-méthyl-propan-1-ol ;
4-[6-[[6-[(1R)-1-hydroxyéthyl]-8-(isopropylamino)pyrido[3,4-d]pyrimidin-2-yl]amino]-3-pyridyl]-1-[(2S)-2-hydroxypropyl]-1,4-diazépan-5-one ;
4-[6-[[6-[(1R)-1-hydroxyéthyl]-8-(isopropylamino)pyrido[3,4-d]pyrimidin-2-yl]amino]-3-pyridyl]-1-[(2R)-2-hydroxypropyl]-1,4-diazépan-5-one ;
N8-isopropyl-N2-[5-(pipérazin-1-ylméthyl)-2-pyridyl]-6-[(2S)-tétrahydrofuran-2-yl]pyrido[3,4-d]pyrimidine-2,8- diamine ;
1-[6-[[6-[(1R)-1-hydroxyéthyl]-8-(isopropylamino)pyrido[3,4-d]pyrimidin-2-yl]amino]-2- methyl-3-pyridyl]pipérazin-2-one ;
1-[6-[[8-(isopropylamino)-6-[(3S)-tétrahydrofuran-3-yl]pyrido[3,4-d]pyrimidin-2-yl]amino]-3-pyridyl]pipérazin-2-one ;
(1R)-1-[2-(5,6,7,8-tétrahydro-1,6-naphthyridin-2-ylamino)-8-[[(3S)-tétrahydropyran-3-yl]amino]pyrido[3,4- d]pyrimidin-6-yl]éthanol ;
1-[6-[[8-(isopropylamino)-6-(3-méthyloxetan-3-yl)pyrido[3,4-d]pyrimidin-2-yl]amino]-3-pyridyl]pipérazin-2-one ;
(1R)-1-[2-[[5-[4-(diméthylamino)cyclohexoxy]-2-pyridyl]amino]-8-[[(3S)-tétrahydropyran-3-yl]amino]pyrido[3,4-d]pyrimidin-6-yl]éthanol ;
6-[(1R)-1-méthoxyéthyl]-N2-[5-(pipérazin-1-ylméthyl)-2-pyridyl]-N8-propyl-pyrido[3,4-d]pyrimidine-2,8-diamine ;
6-[(1R)-1-methoxyéthyl]-N2-(6-pipérazin-1-ylpyridazin-3-yl)-N8-propyl-pyrido[3,4-d]pyrimidine-2,8-diamine ;
Acide 1-[[6-[[6-(difluorométhyl)-8-[(4-méthylcyclohexyl)amino]pyrido[3,4-d]pyrimidin-2-yl]amino]-3-pyridyl]méthyl]pipéridine-4-carboxylique ;
(1R)-1-[8-(ethylamino)-2-[[5-[[4-(2-hydroxyéthyl)pipérazin-1-yl]méthyl]-2-pyridyl]amino]pyrido[3,4-d]pyrimidin-6-yl]éthanol ;
((1R)-1-[2-[[5-[[4-(2-hydroxyéthyl)pipérazin-1-yl]méthyl]-2-pyridyl]amino]-8-(propylamino)pyrido[3,4-d]pyrimidin-6- yl]éthanol ;
N8-isopropyl-6-(3-méthyloxétan-3-yl)-N2-(6-pipérazine-1-ylpyridazin-3-yl)pyrido[3,4-d]pyrimidine-2,8-diamine ;
N8-isopropyl-6-(3-méthyloxétan-3-yl)-N2-[5-(pipérazin-1-ylméthyl)-2-pyridyl]pyrido[3,4-d]pyrimidine-2,8-diamine ;
6-(3-méthyloxetan-3-yl)-N2-[5-(pipérazin-1-ylméthyl)-2-pyridyl]-N8-[(3S)-tétrahydropyran-3-yl]pyrido[3,4- d]pyrimidine-2,8-diamine ;
4-[6-[[6-[(1R)-1 -hydroxyéthyl]-8-[isopropyl(méthyl)amino]pyrido[3,4-d]pyrimidin-2-yl]amino]-3-pyridyl]-1,4-diazépan-5-one ;
(1R)-1-[8-(isopropylamino)-2-[(6-méthyl-5-pipérazin-1-yl-2-pyridyl)amino]pyrido[3,4-d]pyrimidin-6-yl]éthanol ;
(1R)-1-[2-[[6-(2-hydroxyéthyl)-7,8-dihydro-5H-1,6-naphthyridin-2-yl]amino]-8-(isopropylamino)pyrido[3,4- d]pyrimidin-6-yl]éthanol ;
(1R)-1-[8-(isopropylamino)-2-[[6-[2-(méthylamino)éthyl]-7,8-dihydro-5H-1,6-naphthyridin-2-yl]amino]pyrido[3,4- d]pyrimidin-6-yl]éthanol ;
N2-(6-pipérazin-1-ylpyridazin-3-yl)-6-[(3S)-tétrahydrofuran-3-yl]-N8-[(3S)-tétrahydropyran-3-yl]pyrido[3,4-d]pyrimidine-2,8-diamine ;
N2-[5-(pipérazin-1-ylméthyl)-2-pyridyl]-6-[(3R)-tétrahydrofuran-3-yl]-N8-[(3S)-tétrahydropyran-3-yl]pyrido[3,4-d]pyrimidine-2,8-diamine ;
(1R)-1-[2-[[6-[2-(diméthylamino)éthyl]-7,8-dihydro-5H-1,6-naphthyridin-2-yl]amino]-8-(isopropylamino)pyrido[3,4-d]pyrimidin-6-yl]éthanol ;
(2S)-1-[4-[[6-[[8-(éthylamino)-6-[(1R)-1-hydroxyéthyl]pyrido[3,4-d]pyrimidin-2-yl]amino]-3-pyridyl]méthyl]pipérazin-1-yl]propan-2-ol ;
(2R)-1-[4-[[6-[[8-(éthylamino)-6-[(1R)-1-hydroxyéthyl]pyrido[3,4-d]pyrimidin-2-yl]amino]-3-pyridyl]méthyl]pipérazin-1-yl]propan-2-ol ;
(1R)-1-[8-(isopropylamino)-2-[[5-[(2R)-2-méthylpipérazin-1-yl]-2-pyridyl]amino]pyrido[3,4-d]pyrimidin-6-yl]éthanol ;
(1R)-1-[8-(isopropylamino)-2-[[5-[(2S)-2-méthylpipérazin-1-yl]-2-pyridyl]amino]pyrido[3,4-d]pyrimidin-6-yl]éthanol ;
N8-isopropyl-N2-(5-pipérazin-1-yl-2-pyridyl)-6-[(2S)-tétrahydrofuran-2-yl]pyrido[3,4-d]pyrimidine-2,8-diamine ;
(1R)-1-[8-(cyclobutylamino)-2-[[5-[[4-(2-hydroxyéthyl)pipérazin-1-yl]méthyl]-2-pyridyl]amino]pyrido[3,4-d]pyrimidin-6-yl]éthanol ;
(1R)-1-[8-(cyclopropylméthylamino)-2-[[5-[[4-(2-hydroxyéthyl)pipérazin-1-yl]méthyl]-2-pyridyl]amino]pyrido[3,4-d]pyrimidin-6-yl]éthanol ;
6-(3-méthyloxetan-3-yl)-N2-(5-pipérazin-1-yl-2-pyridyl)-N8-propyl-pyrido[3,4-d]pyrimidine-2,8-diamine ;
6-(3-méthyloxetan-3-yl)-N2-[5-(pipérazin-1-ylméthyl)-2-pyridyl]-N8-propyl-pyrido[3,4-d]pyrimidine-2,8-diamine ;
N2-(5-pipérazin-1-yl-2-pyridyl)-N8-propyl-6-tétrahydrofuran-3-yl-pyrido[3,4-d]pyrimidine-2,8-diamine ;
N2-[5-(pipérazin-1-ylméthyl)-2-pyridyl]-N8-propyl-6-tétrahydrofuran-3-yl-pyrido[3,4-d]pyrimidine-2,8-diamine ;
N8-isopropyl-6-(3-méthyloxetan-3-yl)-N2-(5-pipérazin-1-yl-2-pyridyl)pyrido[3,4-d]pyrimidine-2,8-diamine ;
N8-isopropyl-N2-(5-pipérazin-1-yl-2-pyridyl)-6-tétrahydrofuran-3-yl-pyrido[3,4-d]pyrimidine-2,8-diamine ;
2-[4-[[6-[[8-(isopropylamino)-6-tétrahydrofuran-3-yl-pyrido[3,4-d]pyrimidin-2-yl]amino]-3-pyridyl]méthyl]pipérazin-1-yl]éthanol ;
2-[4-[[6-[[6-tétrahydrofuran-3-yl-8-[[(3S)-tétrahydropyran-3-yl]amino]pyrido[3,4-d]pyrimidin-2-yl]amino]-3-pyridyl]méthyl]pipérazin-1-yl]éthanol ;
(1R)-1-[2-[[5-[[4-(hydroxyméthyl)-1-pipéridyl]methyl]-2-pyridyl]amino]-8-(isopropylamino)pyrido[3,4-d]pyrimidin-6-yl]éthanol ;
1-[[6-[[6-[(1R)-1-hydroxyéthyl]-8-(isopropylamino)pyrido[3,4-d]pyrimidin-2-yl]amino]-3-pyridyl]méthyl]pipéridin-4-ol ;
1-[[6-[[8-(tert-butylamino)-6-[(1R)-1-hydroxyéthyl]pyrido[3,4-d]pyrimidin-2-yl]amino]-3-pyridyl]méthyl]pipéridin-4-ol ;
(1R)-1-[8-(tert-butylamino)-2-[[5-[[4-(hydroxyméthyl)-1-pipéridyl]méthyl]-2-pyridyl]amino]pyrido[3,4-d]pyrimidin-6-yl]éthanol ;
1-[[6-[[6-[(1R)-1-hydroxyéthyl]-8-(isobutylamino)pyrido[3,4-d]pyrimidin-2-yl]amino]-3-pyridyl]méthyl]pipéridin-4-ol ;
(1R)-1-[2-[[5-[[4-(hydroxyméthyl)-1-piperidyl]méthyl]-2-pyridyl]amino]-8-(isobutylamino)pyrido[3,4-d]pyrimidin-6-yl]éthanol ;
1-[6-[[6-[(1R)-1-hydroxypropyl]-8-(isopropylamino)pyrido[3,4-d]pyrimidin-2-yl]amino]-3-pyridyl]pipérazin-2-one ;
(1R)-1-[2-[[5-[[4-(2-hydroxyéthyl)pipérazin-1-yl]méthyl]-6-méthyl-2-pyridyl]amino]-8-(propylamino)pyrido[3,4- d]pyrimidin-6-yl]éthanol.

12. Composition pharmaceutique comprenant le composé ou son sel pharmaceutiquement acceptable selon l'une quelconque des revendications 1 à 11 et un support pharmaceutiquement acceptable.

13. Composition pharmaceutique présentant une activité inhibitrice de CDK4/6, comprenant le composé ou son sel pharmaceutiquement acceptable selon l'une quelconque des revendications 1 à 11 en tant qu'ingrédient actif.

14. Médicament pour utilisation dans la prévention ou le traitement de la polyarthrite rhumatoïde, de l'artériosclérose, de la fibrose pulmonaire, de l'infarctus cérébral, ou du cancer, le médicament comprenant le composé ou son sel pharmaceutiquement acceptable selon l'une quelconque des revendications 1 à 11 en tant qu'ingrédient actif.
